# EUROPEAN PATENT APPLICATION

(11) **EP 4 063 510 A1**
(43) Date of publication of application: **28.09.2022**
(21) Application number: 22155160.9
(22) Date of filing: 07.11.2019
(51) Int. Cl.: C12N 15/86, C12N 15/113, C12N 15/65, C07K 14/47, C07K 14/705, A61K 48/00, A61P 27/16

(54) **USE OF ADENO-ASSOCIATED VIRAL VECTORS TO CORRECT GENE DEFECTS/ EXPRESS PROTEINS IN HAIR CELLS AND SUPPORTING CELLS IN THE INNER EAR**

(30) Priority: 07.11.2018 US 201862756953 P; 16.08.2019 US 201962888093 P
(62) Divisional of application: 19881503.7
(71) Applicant: Akouos, Inc., Boston, MA 02210 (US)
(72) Inventor: SIMONS, Emmanuel J., Brookline, MA 02446 (US); NG, Robert, Newton, MA 02458 (US); LENZ, Danielle, Brookline, MA 02446 (US); VALERO, Michelle, Revere, MA 02151 (US)
(74) Representative: Pohlman, Sandra M.

(57) **Abstract**

Provided herein are compositions that include at least two different nucleic acid vectors that, when introduced into a primate cell, the at least two different vectors undergo concatamerization or homologous recombination with each other, thereby forming a recombined nucleic acid that encodes a full-length target protein (e.g., a supporting cell target protein or a hair cell target protein). Also provided are compositions that include a single AAV vector that, when introduced into a primate cell, a nucleic acid encoding a full-length target protein (e.g., a supporting cell target protein or a hair cell target protein) is generated at the locus of the supporting cell target gene, and the primate expresses the target protein (e.g., a supporting cell target protein or a hair cell target protein).

## Description

### CROSS-REFERENCE TO RELATED APPLICATIONS

This application claims priority to U.S. Provisional Patent Application Serial No. 62/756,953, filed November 7, 2018, and U.S. Provisional Patent Application Serial No. 62/888,093, filed August 16, 2019; the entire contents of which are herein incorporated by reference.

### TECHNICAL FIELD

The present disclosure relates generally to the use of nucleic acids to treat hearing loss in a primate.

### BACKGROUND OF THE INVENTION

Hearing loss can be conductive (arising from the ear canal or middle ear), sensorineural (arising from the inner ear or auditory nerve), or mixed. Most forms of non-syndromic deafness are associated with permanent hearing loss caused by damage to structures in the inner ear (sensorineural deafness), although some forms may involve changes in the middle ear (conductive hearing loss). The great majority of human sensorineural hearing loss is caused by abnormalities in the hair cells of the organ of Corti in the cochlea (poor hair cell function). The hair cells may be abnormal at birth, or may be damaged during the lifetime of an individual (e.g., as a result of noise trauma or infection).

### SUMMARY

The present invention is based on the discovery that a composition including at least two different nucleic acid vectors, wherein: each of the at least two different adeno-associated virus (AAV) vectors includes a coding sequence that encodes a different portion of a supporting cell target protein, each of the encoded portions being at least 30 amino acid residues in length, wherein the amino acid sequence of each of the encoded portions may optionally partially overlap with the amino acid sequence of a different one of the encoded portions; no single vector of the at least two different vectors encodes the full-length supporting cell target protein; at least one of the coding sequences includes a nucleotide sequence spanning two neighboring exons of the supporting cell target genomic DNA, and lacks an intronic sequence between the two neighboring exons; and when introduced into a primate cell the at least two different vectors undergo concatamerization or homologous recombination with each other, thereby forming a recombined nucleic acid that encodes a full-length supporting cell target protein.

Provided herein are compositions that include at least two different nucleic acid vectors, where: each of the at least two different adeno-associated virus (AAV) vectors includes a coding sequence that encodes a different portion of a supporting cell target protein, each of the encoded portions being at least 30 amino acid residues in length, where the amino acid sequence of each of the encoded portions may optionally partially overlap with the amino acid sequence of a different one of the encoded portions; no single vector of the at least two different vectors encodes the full-length supporting cell target protein; at least one of the coding sequences includes a nucleotide sequence spanning two neighboring exons of the supporting cell target genomic DNA, and lacks an intronic sequence between the two neighboring exons; and when introduced into a primate cell the at least two different vectors undergo concatamerization or homologous recombination with each other, thereby forming a recombined nucleic acid that encodes a full-length supporting cell target protein.

In some embodiments of any of the compositions described herein, the amino acid sequence of one of the encoded portions overlaps with the amino acid sequence of a different one of the encoded portions. In some embodiments of any of the compositions described herein, the amino acid sequence of each of the encoded portions partially overlaps with the amino acid sequence of a different encoded portion. In some embodiments of any of the compositions described herein, the overlapping amino acid sequence is between 30 amino acid residues to about 390 amino acid residues in length.

In some embodiments of any of the compositions described herein, each of the at least two different vectors includes a different segment of an intron, where the intron includes the nucleotide sequence of an intron that is present in a supporting cell target genomic DNA, and where the two different segments overlap in sequence by at least 100 nucleotides. In some embodiments of any of the compositions described herein, the two different segments overlap in sequence by about 30 nucleotides to about 390 nucleotides. In some embodiments of any of the compositions described herein, the nucleotide sequence of each of the at least two different vectors is between about 30 nucleotides to about 390 nucleotides in length. In some embodiments of any of the compositions described herein, the nucleotide sequence of each of the at least two different vectors is between 30 nucleotides to 340 nucleotides in length.

In some embodiments of any of the compositions described herein, the number of different vectors in the composition is two. In some embodiments of any of the compositions described herein, a first of the two different vectors includes a coding sequence that encodes an N-terminal portion of the supporting cell target protein. In some embodiments of any of the compositions described herein, the N-terminal portion of the supporting cell target protein is between about 30 amino acids to about 390 amino acids in length. In some embodiments of any of the compositions described herein, the N-terminal portion of the supporting cell target protein is between about 30 amino acids to about 340 amino acids in length. In some embodiments of any of the compositions described herein, the first vector further includes one or both of a promoter and a Kozak sequence. In some embodiments of any of the compositions described herein, the first vector includes a promoter that is an inducible promoter, a constitutive promoter, or a tissue-specific promoter.

In some embodiments of any of the compositions described herein, the second of the two different vectors includes a coding sequence that encodes a C-terminal portion of the supporting cell target protein. In some embodiments of any of the compositions described herein, the C-terminal portion of the supporting cell target protein is between 30 amino acids to about 390 amino acids in length. In some embodiments of any of the compositions described herein, the C-terminal portion of the supporting cell target protein is between 30 amino acids to about 340 amino acids in length. In some embodiments of any of the compositions described herein, the second vector further includes a poly(dA) sequence.

Also provided herein are compositions that include two different nucleic acid vectors, where: a first nucleic acid vector of the two different nucleic acid vectors includes a promoter, a first coding sequence that encodes an N-terminal portion of a supporting cell target protein positioned 3' of the promoter, and a splicing donor signal sequence positioned at the 3' end of the first coding sequence; and a second nucleic acid vector of the two different nucleic acid vectors includes a splicing acceptor signal sequence, a second coding sequence that encodes a C-terminal portion of a supporting cell target protein positioned at the 3' end of the splicing acceptor signal sequence, and a polyadenylation sequence at the 3' end of the second coding sequence; where each of the encoded portions is at least 30 amino acid residues in length, where the amino acid sequences of the encoded portions do not overlap, where no single vector of the two different vectors encodes the full-length supporting cell target protein, and, when the coding sequences are transcribed in a primate cell, to produce RNA transcripts, splicing occurs between the splicing donor signal sequence on one transcript and the splicing acceptor signal sequence on the other transcript, thereby forming a recombined RNA molecule that encodes a full-length supporting cell target protein.

In some embodiments of any of the compositions described herein, at least one of the coding sequences includes a nucleotide sequence spanning two neighboring exons of a supporting cell target genomic DNA, and lacks an intronic sequence between the neighboring exons.

Also provided herein are compositions that include: a first nucleic acid vector including a promoter, a first coding sequence that encodes an N-terminal portion of a supporting cell target protein positioned 3' of the promoter, a splicing donor signal sequence positioned at the 3' end of the first coding sequence, and a first detectable marker gene positioned 3' of the splicing donor signal sequence; and a second nucleic acid vector, different from the first nucleic acid vector, including a second detectable marker gene, a splicing acceptor signal sequence positioned 3' of the second detectable marker gene, a second coding sequence that encodes a C-terminal portion of a supporting cell target protein positioned at the 3' end of the splicing acceptor signal sequence, and a polyadenylation sequence positioned at the 3' end of the second coding sequence; where each of the encoded portions is at least 30 amino acid residues in length, where the respective amino acid sequences of the encoded portions do not overlap with each other, where no single vector of the two different vectors encodes the full-length supporting cell target protein, and, when the coding sequences are transcribed in a primate cell to produce RNA transcripts, splicing occurs between the splicing donor signal on one transcript and the splicing acceptor signal on the other transcript, thereby forming a recombined RNA molecule that encodes a full-length supporting cell target protein.

In some embodiments of any of the compositions described herein, at least one of the coding sequences includes a nucleotide sequence spanning two neighboring exons of a supporting cell target genomic DNA, and lacks an intronic sequence between the neighboring exons. In some embodiments of any of the compositions described herein, the first or second detectable marker gene is alkaline phosphatase. In some embodiments of any of the compositions described herein, the first and second detectable marker genes are the same.

Also provided herein are compositions that include: a first nucleic acid vector including a promoter, a first coding sequence that encodes an N-terminal portion of a supporting cell target protein positioned 3' to the promoter, a splicing donor signal sequence positioned at the 3' end of the first coding sequence, and a F1 phage recombinogenic region positioned 3' to the splicing donor signal sequence; and a second nucleic acid vector, different from the first nucleic acid vector, including a second F1 phage recombinogenic region, a splicing acceptor signal sequence positioned 3' of the second F1 phage recombinogenic region, a second coding sequence that encodes a C-terminal portion of a supporting cell target protein positioned at the 3' end of the splicing acceptor signal sequence, and a polyadenylation sequence positioned at the 3' end of the second coding sequence; where each of the encoded portions is at least 30 amino acid residues in length, where the respective amino acid sequences of the encoded portions do not overlap with each other, where no single vector of the two different vectors encodes the full-length supporting cell target protein, and, when the coding sequences are transcribed in a primate cell to produce RNA transcripts, splicing occurs between the splicing donor signal one transcript and the splicing acceptor signal on the other transcript, thereby forming a recombined RNA molecule that encodes a full-length supporting cell target protein.

In some embodiments of any of the compositions described herein, at least one of the coding sequences includes a nucleotide sequence spanning two neighboring exons of a supporting cell target genomic DNA, and lacks an intronic sequence between the two neighboring exons.

Also provided herein are compositions that include a single adeno-associated virus (AAV) vector, where the single AAV vector that includes a nucleic acid sequence that encodes a supporting cell target protein; and when introduced into a primate cell, a nucleic acid encoding a full-length supporting cell target protein is generated at the locus of the supporting cell target gene, and the primate expresses the supporting cell target protein.

In some embodiments of any of the compositions described herein, the supporting cell target gene is gap junction protein beta 2 (GJB2). In some embodiments of any of the compositions described herein, the supporting cell target gene is solute carrier family 26 member 4 (SLC26A4).

In some embodiments of any of the compositions described herein, the composition further includes a pharmaceutically acceptable excipient.

In some embodiments of any of the compositions described herein, the second nucleic acid vector further includes a destabilizing sequence.

In some embodiments of any of the compositions described herein, the second nucleic acid vector further includes a FKBP12 destabilizing sequence.

Also provided herein are kits including any of the compositions described herein. In some embodiments of any of the kits described herein, the kit further includes a preloaded syringe containing the composition.

Also provided herein are methods of correcting a supporting cell target gene defect in a supporting cell of an inner ear in a primate that include: administering to the inner ear of the primate a therapeutically effective amount of any of the compositions described herein, where the administering repairs the supporting cell target gene defect in the supporting cell of the inner ear of the primate.

Also provided herein are methods of increasing the expression level of a supporting cell target protein in a supporting cell of an inner ear of a primate that include: administering to the inner ear of the primate a therapeutically effective amount of any of the compositions described herein, where the administering results in an increase in the expression level of the supporting cell target protein in the supporting cell of the inner ear of the primate.

In some embodiments of any of the methods described herein, the primate has previously been determined to have a defective supporting cell target gene.

Also provided herein are methods of treating non-syndromic sensorineural hearing loss in a primate identified as having a defective supporting cell target gene that include: administering to the inner ear of the primate a therapeutically effective amount of any of the compositions described herein.

In some embodiments of any of the methods described herein, the method further includes, prior to the administering step, determining that the primate has a defective supporting cell target gene.

Also provided herein are methods of restoring hearing in a primate identified or diagnosed as having hearing loss that include: administering to the inner ear of the primate a therapeutically effective amount of any composition described herein.

In some embodiments of any of the methods described herein, the method further includes prior to the administering step, determining that the primate has a defective supporting cell target gene.

Also provided herein are methods of restoring synapses and/or preserving spiral ganglion nerves in a primate identified or diagnosed as having an inner ear disorder that include: administering to the inner ear of the primate a therapeutically effective amount of any composition described herein.

In some embodiments of any of the methods described herein, the method further includes prior to the administering step, determining that the primate has a defective supporting cell target gene.

Also provided herein are methods including administering to an inner ear of a primate a therapeutically effective amount of any composition described herein.

In some embodiments of any of the methods described herein, the primate has been previously identified as having a defective supporting cell target gene.

Also provided herein are compositions including at least two different nucleic acid vectors, wherein: each of the at least two different adeno-associated virus (AAV) vectors includes a coding sequence that encodes a different portion of a hair cell target protein, each of the encoded portions being at least 30 amino acid residues in length, wherein the amino acid sequence of each of the encoded portions may optionally partially overlap with the amino acid sequence of a different one of the encoded portions; no single vector of the at least two different vectors encodes the full-length hair cell target protein; at least one of the coding sequences includes a nucleotide sequence spanning two neighboring exons of the hair cell target genomic DNA, and lacks an intronic sequence between the two neighboring exons; and when introduced into a primate cell the at least two different vectors undergo concatamerization or homologous recombination with each other, thereby forming a recombined nucleic acid that encodes a full-length hair cell target protein.

Provided herein are compositions that include at least two different nucleic acid vectors, where: each of the at least two different adeno-associated virus (AAV) vectors includes a coding sequence that encodes a different portion of a hair cell target protein, each of the encoded portions being at least 30 amino acid residues in length, where the amino acid sequence of each of the encoded portions may optionally partially overlap with the amino acid sequence of a different one of the encoded portions; no single vector of the at least two different vectors encodes the full-length protein; at least one of the coding sequences includes a nucleotide sequence spanning two neighboring exons of the hair cell target genomic DNA, and lacks an intronic sequence between the two neighboring exons; and when introduced into a primate cell the at least two different vectors undergo concatamerization or homologous recombination with each other, thereby forming a recombined nucleic acid that encodes a full-length hair cell target protein.

In some embodiments of any of the compositions described herein, the amino acid sequence of one of the encoded portions overlaps with the amino acid sequence of a different one of the encoded portions. In some embodiments of any of the compositions described herein, the amino acid sequence of each of the encoded portions partially overlaps with the amino acid sequence of a different encoded portion. In some embodiments of any of the compositions described herein, the overlapping amino acid sequence is between 30 amino acid residues to about 390 amino acid residues in length.

In some embodiments of any of the compositions described herein, each of the at least two different vectors includes a different segment of an intron, where the intron includes the nucleotide sequence of an intron that is present in a hair cell target genomic DNA, and where the two different segments overlap in sequence by at least 100 nucleotides. In some embodiments of any of the compositions described herein, the two different segments overlap in sequence by about 30 nucleotides to about 390 nucleotides. In some embodiments of any of the compositions described herein, the nucleotide sequence of each of the at least two different vectors is between about 30 nucleotides to about 390 nucleotides in length. In some embodiments of any of the compositions described herein, the nucleotide sequence of each of the at least two different vectors is between 30 nucleotides to 340 nucleotides in length.

In some embodiments of any of the compositions described herein, the number of different vectors in the composition is two. In some embodiments of any of the compositions described herein, a first of the two different vectors includes a coding sequence that encodes an N-terminal portion of the hair cell target protein. In some embodiments of any of the compositions described herein, the N-terminal portion of the hair cell target protein is between about 30 amino acids to about 390 amino acids in length. In some embodiments of any of the compositions described herein, the N-terminal portion of the hair cell target protein is between about 30 amino acids to about 340 amino acids in length. In some embodiments of any of the compositions described herein, the first vector further includes one or both of a promoter and a Kozak sequence. In some embodiments of any of the compositions described herein, the first vector includes a promoter that is an inducible promoter, a constitutive promoter, or a tissue-specific promoter.

In some embodiments of any of the compositions described herein, the second of the two different vectors includes a coding sequence that encodes a C-terminal portion of the hair cell target protein. In some embodiments of any of the compositions described herein, the C-terminal portion of the hair cell target protein is between 30 amino acids to about 390 amino acids in length. In some embodiments of any of the compositions described herein, the C-terminal portion of the hair cell target protein is between 30 amino acids to about 340 amino acids in length. In some embodiments of any of the compositions described herein, the second vector further includes a poly(dA) sequence.

Also provided herein are compositions that include two different nucleic acid vectors, where: a first nucleic acid vector of the two different nucleic acid vectors includes a promoter, a first coding sequence that encodes an N-terminal portion of a hair cell target protein positioned 3' of the promoter, and a splicing donor signal sequence positioned at the 3' end of the first coding sequence; and a second nucleic acid vector of the two different nucleic acid vectors includes a splicing acceptor signal sequence, a second coding sequence that encodes a C-terminal portion of a hair cell target protein positioned at the 3' end of the splicing acceptor signal sequence, and a polyadenylation sequence at the 3' end of the second coding sequence; where each of the encoded portions is at least 30 amino acid residues in length, where the amino acid sequences of the encoded portions do not overlap, where no single vector of the two different vectors encodes the full-length hair cell target protein, and, when the coding sequences are transcribed in a primate cell, to produce RNA transcripts, splicing occurs between the splicing donor signal sequence on one transcript and the splicing acceptor signal sequence on the other transcript, thereby forming a recombined RNA molecule that encodes a full-length hair cell target protein.

In some embodiments of any of the compositions described herein, at least one of the coding sequences includes a nucleotide sequence spanning two neighboring exons of a hair cell target genomic DNA, and lacks an intronic sequence between the neighboring exons.

Also provided herein are compositions that include: a first nucleic acid vector including a promoter, a first coding sequence that encodes an N-terminal portion of a hair cell target protein positioned 3' of the promoter, a splicing donor signal sequence positioned at the 3' end of the first coding sequence, and a first detectable marker gene positioned 3' of the splicing donor signal sequence; and a second nucleic acid vector, different from the first nucleic acid vector, including a second detectable marker gene, a splicing acceptor signal sequence positioned 3' of the second detectable marker gene, a second coding sequence that encodes a C-terminal portion of a hair cell target protein positioned at the 3' end of the splicing acceptor signal sequence, and a polyadenylation sequence positioned at the 3' end of the second coding sequence; where each of the encoded portions is at least 30 amino acid residues in length, where the respective amino acid sequences of the encoded portions do not overlap with each other, where no single vector of the two different vectors encodes the full-length hair cell target protein, and, when the coding sequences are transcribed in a primate cell to produce RNA transcripts, splicing occurs between the splicing donor signal on one transcript and the splicing acceptor signal on the other transcript, thereby forming a recombined RNA molecule that encodes a full-length hair cell target protein.

In some embodiments of any of the compositions described herein, at least one of the coding sequences includes a nucleotide sequence spanning two neighboring exons of a hair cell target genomic DNA, and lacks an intronic sequence between the neighboring exons. In some embodiments of any of the compositions described herein, the first or second detectable marker gene is alkaline phosphatase. In some embodiments of any of the compositions described herein, the first and second detectable marker genes are the same.

Also provided herein are compositions that include: a first nucleic acid vector including a promoter, a first coding sequence that encodes an N-terminal portion of a hair cell target protein positioned 3' to the promoter, a splicing donor signal sequence positioned at the 3' end of the first coding sequence, and a F1 phage recombinogenic region positioned 3' to the splicing donor signal sequence; and a second nucleic acid vector, different from the first nucleic acid vector, including a second F1 phage recombinogenic region, a splicing acceptor signal sequence positioned 3' of the second F1 phage recombinogenic region, a second coding sequence that encodes a C-terminal portion of a hair cell target protein positioned at the 3' end of the splicing acceptor signal sequence, and a polyadenylation sequence positioned at the 3' end of the second coding sequence; where each of the encoded portions is at least 30 amino acid residues in length, where the respective amino acid sequences of the encoded portions do not overlap with each other, where no single vector of the two different vectors encodes the full-length hair cell target protein, and, when the coding sequences are transcribed in a primate cell to produce RNA transcripts, splicing occurs between the splicing donor signal one transcript and the splicing acceptor signal on the other transcript, thereby forming a recombined RNA molecule that encodes a full-length hair cell target protein.

In some embodiments of any of the compositions described herein, at least one of the coding sequences includes a nucleotide sequence spanning two neighboring exons of a hair cell target genomic DNA, and lacks an intronic sequence between the two neighboring exons.

Also provided herein are compositions that include a single adeno-associated virus (AAV) vector, where the single AAV vector that includes a nucleic acid sequence that encodes a hair cell target protein; and when introduced into a primate cell, a nucleic acid encoding a full-length hair cell target protein is generated at the locus of the hair cell target gene, and the primate expresses the hair cell target protein.

In some embodiments of any of the compositions described herein, the composition further includes a pharmaceutically acceptable excipient.

Also provided herein are kits including any of the compositions described herein. In some embodiments of any of the kits described herein, the kit further includes a preloaded syringe containing the composition.

Also provided herein are methods of correcting a hair cell target gene defect in a hair cell of an inner ear in a primate that include: administering to the inner ear of the primate a therapeutically effective amount of any of the compositions described herein, where the administering repairs the hair cell target gene defect in the hair cell of the inner ear of the primate.

Also provided herein are methods of increasing the expression level of a hair cell target protein in a hair cell of an inner ear of a primate that include: administering to the inner ear of the primate a therapeutically effective amount of any of the compositions described herein, where the administering results in an increase in the expression level of the hair cell target protein in the hair cell of the inner ear of the primate.

In some embodiments of any of the methods described herein, the primate has previously been determined to have a defective hair cell target gene.

Also provided herein are methods of treating non-syndromic sensorineural hearing loss in a primate identified as having a defective hair cell target gene that include: administering to the inner ear of the primate a therapeutically effective amount of any of the compositions described herein.

In some embodiments of any of the methods described herein, the method further includes, prior to the administering step, determining that the primate has a defective hair cell target gene.

Also provided herein are methods of restoring hearing in a primate identified or diagnosed as having hearing loss that include: administering to the inner ear of the primate a therapeutically effective amount of any composition described herein.

In some embodiments of any of the methods described herein, the method further includes prior to the administering step, determining that the primate has a defective hair cell target gene.

Also provided herein are methods of restoring synapses and/or preserving spiral ganglion nerves in a primate identified or diagnosed as having an inner ear disorder that include: administering to the inner ear of the primate a therapeutically effective amount of any composition described herein.

In some embodiments of any of the methods described herein, the method further includes prior to the administering step, determining that the primate has a defective hair cell target gene.

Also provided herein are methods including administering to an inner ear of a primate a therapeutically effective amount of any composition described herein.

In some embodiments of any of the methods described herein, the primate has been previously identified as having a defective hair cell target gene.

The term "a" and "an" refers to one or to more than one (i.e., at least one) of the grammatical object of the article. By way of example, "an element" encompasses one element and more than one element.

The term "supporting cell target protein" means a protein that is expressed in a supporting cell in a primate and functionally contributes, at least in part, to the hearing in the primate. Non-limiting examples of supporting cell target proteins include GJB2 and SLC26A4.

The term "mutation in a supporting cell target gene" refers to a modification in a wildtype supporting cell target gene that results in the production of a supporting cell target protein having one or more of: a deletion in one or more amino acids, one or more amino acid substitutions, and one or more amino acid insertions as compared to the wildtype supporting cell target protein, and/or results in a decrease in the expressed level of the encoded supporting cell target protein in a primate cell as compared to the expressed level of the encoded supporting cell target protein in a primate cell not having a mutation. In some embodiments, a mutation can result in the production of a supporting cell target protein having a deletion in one or more amino acids (e.g., 2, 3, 4, 5, 6, 7, 8, 9, 10, 11, 12, 13, 14, 15 16, 17, 18, 19, or 20 amino acids). In some embodiments, the mutation can result in a frameshift in the supporting cell target gene.

The term "hair cell target protein" means a protein that is expressed in a hair cell in a primate and functionally contributes, at least in part, to the hearing in the primate. Non-limiting examples of hair cell target proteins include tectorin alpha (TECTA), unconventional myson-15 (MYO15A), myosin IIIA (MYO3A), myosin VI (MYO6), myosin VIIA (MYO7A), cadherin related 23 (CDH23), collagen type XI alpha 2 chain (COL11A2), lipoxygenase homology domains 1 (LOXHD1), protocadherin related 15 (PCDH15), whirlin (WHRN), an otoferlin (OTOF) gene, a trio rho guanine nucleotide exchange factor (TRIO) and f-actin binding protein (TRIOBP) gene, a stereocilin (STRC) gene, a pejvakin (PJVK) gene, a norrin cysteine knot growth factor (NDP) gene, a clarin 1 (CLRN1) gene, a transmembrane channel-like 1 (TMC1) gene, a vascular endothelial growth factor (VEGF) gene, and a heat shock protein family A (Hsp70) member 1A (HSPA1A) gene.

The term "mutation in a hair cell target gene" refers to a modification in a wildtype hair cell target gene that results in the production of a hair cell target protein having one or more of: a deletion in one or more amino acids, one or more amino acid substitutions, and one or more amino acid insertions as compared to the wildtype hair cell target protein, and/or results in a decrease in the expressed level of the encoded hair cell target protein in a primate cell as compared to the expressed level of the encoded hair cell target protein in a primate cell not having a mutation. In some embodiments, a mutation can result in the production of a hair cell target protein having a deletion in one or more amino acids (e.g., 2, 3, 4, 5, 6, 7, 8, 9, 10, 11, 12, 13, 14, 15 16, 17, 18, 19, or 20 amino acids). In some embodiments, the mutation can result in a frameshift in the hair cell target gene.

The term "frameshift" is known in the art to encompass any mutation in a coding sequence that results in a shift in the reading frame of the coding sequence. In some embodiments, a frameshift can result in a nonfunctional protein. In some embodiments, a point mutation can be a nonsense mutation (i.e., result in a premature stop codon in an exon of the gene). A nonsense mutation can result in the production of a truncated protein (as compared to a corresponding wildtype protein) that may or may not be functional. In some embodiments, the mutation can result in the loss (or a decrease in the level) of expression of hair cell target mRNA or hair cell target protein or both the mRNA and protein. In some embodiments, the mutation can result in the production of an altered hair cell target protein having a loss or decrease in one or more biological activities (functions) as compared to a wildtype hair cell target protein. In some embodiments, the mutation can result in the loss (or a decrease in the level) of expression of supporting cell target mRNA or supporting cell target protein or both the mRNA and protein. In some embodiments, the mutation can result in the production of an altered supporting cell target protein having a loss or decrease in one or more biological activities (functions) as compared to a wildtype supporting cell target protein.

In some embodiments, the mutation is an insertion of one or more nucleotides into a hair cell target gene. In some embodiments, the mutation is in a regulatory sequence of the hair cell target gene, i.e., a portion of the gene that is not coding sequence. In some embodiments, a mutation in a regulatory sequence may be in a promoter or enhancer region and prevent or reduce the proper transcription of the hair cell target gene. In some embodiments, the mutation is an insertion of one or more nucleotides into a supporting cell target gene. In some embodiments, the mutation is in a regulatory sequence of the supporting cell target gene, i.e., a portion of the gene that is not coding sequence. In some embodiments, a mutation in a regulatory sequence may be in a promoter or enhancer region and prevent or reduce the proper transcription of the supporting cell target gene. The term "conservative mutation" refers to a mutation that does not change the amino acid encoded at the site of the mutation (due to codon degeneracy).

Modifications can be introduced into a nucleotide sequence by standard techniques known in the art, such as site-directed mutagenesis and PCR-mediated mutagenesis. Conservative amino acid substitutions are ones in which the amino acid residue is replaced with an amino acid residue having a similar side chain. Families of amino acid residues having similar side chains have been defined in the art. These families include amino acids with basic side chains (e.g., lysine, arginine, and histidine), acidic side chains (e.g., aspartic acid and glutamic acid), uncharged polar side chains (e.g., glycine, asparagine, glutamine, serine, threonine, tyrosine, cysteine, and tryptophan), nonpolar side chains (e.g., alanine, valine, leucine, isoleucine, proline, phenylalanine, and methionine), beta-branched side chains (e.g., threonine, valine, and isoleucine), and aromatic side chains (e.g., tyrosine, phenylalanine, tryptophan, and histidine).

Unless otherwise specified, a "nucleotide sequence encoding an amino acid sequence" includes all nucleotide sequences that are degenerate versions of each other and thus encode the same amino acid sequence.

The term "endogenous" refers to any material originating from within an organism, cell, or tissue.

The term "exogenous" refers to any material introduced from or originating from outside an organism, cell, or tissue that is not produced or does not originate from the same organism, cell, or tissue in which it is being introduced.

The term "isolated" means altered or removed from the natural state. For example, a nucleic acid or a peptide naturally present in a living animal is not "isolated," but the same nucleic acid or peptide partially or completely separated from the coexisting materials of its natural state is "isolated." An isolated nucleic acid or protein can exist in substantially purified form, or can exist in a non-native environment such as, for example, a host cell.

The term "transfected," "transformed," or "transduced" refers to a process by which exogenous nucleic acid is transferred or introduced into a cell. A "transfected," "transformed," or "transduced" primate cell is one that has been transfected, transformed or transduced with exogenous nucleic acid.

The term "expression" refers to the transcription and/or translation of a particular nucleotide sequence encoding a protein.

The term "transient expression" refers to the expression of a non-integrated coding sequence for a short period of time (e.g., hours or days). The coding sequence that is transiently expressed in a cell (e.g., a primate cell) is lost upon multiple rounds of cell division.

The term "primate" is intended to include any primate (e.g., a human, a non-human primate (e.g., simian (e.g., a monkey (e.g., a marmoset, a baboon, or a macaque), or an ape (e.g., a gorilla, a gibbon, an orangutan, or a chimpanzee). In some embodiments, the primate has or is at risk of having hearing loss. In some embodiments, the primate has been previously identified as having a mutation in a hair cell target gene (e.g., an OTOF gene, a TRIOBP gene, a STRC gene, a PJVK gene, a NDP gene, a CLRN1 gene, a TMC1 gene, a VEGF gene, or a HSPA1A gene). In some embodiments, the primate has been previously identified as having a mutation in a supporting cell target gene (e.g., a GJB2 gene or a SLC26A4 gene). In some embodiments, the primate has been previously identified as having a mutation in a GJB2 gene. In some embodiments, the primate has been previously identified as having a mutation in a SLC26A4 gene. In some embodiments, the primate has been identified as having a mutation in a hair cell target gene (e.g., an OTOF gene, a TRIOBP gene, a STRC gene, a PJVK gene, a NDP gene, a CLRN1 gene, a TMC1 gene, a VEGF gene, or a HSPA1A gene) and has been diagnosed with hearing loss. In some embodiments, the primate has been identified as having a mutation in a supporting cell target gene (e.g., a GBJ2 gene or a SLC26A4 gene) and has been diagnosed with hearing loss. In some embodiments, the primate has been identified as having hearing loss.

A treatment is "therapeutically effective" when it results in a reduction in one or more of the number, severity, and frequency of one or more symptoms of a disease state (hearing loss, e.g., non-syndromic sensorineural hearing loss or syndromic sensorineural hearing loss) in a primate. In some embodiments, a therapeutically effective amount of a composition can result in an increase in the expression level of an active target protein (e.g., a supporting cell target protein (e.g., a wildtype, full-length supporting cell target protein or a variant of a supporting cell target protein that has the desired activity) (e.g., as compared to the expression level prior to treatment with the composition) or a hair cell target protein (e.g., a wildtype, full-length hair cell target protein or a variant of a hair cell target protein that has the desired activity) (e.g., as compared to the expression level prior to treatment with the composition)). In some embodiments, a therapeutically effective amount of a composition can result in an increase in the expression level of an active target protein (e.g., an active supporting cell target protein (e.g., a wildtype, full-length supporting cell target protein or active variant) ort an active hair cell target protein (e.g., a wildtype, full-length hair cell target protein or active variant) in a target cell (e.g., a supporting cell or a hair cell). In some embodiments, a therapeutically effective amount of a composition can result in an increase in the expression level of an active target protein (e.g., an active supporting cell target protein (e.g., a wildtype, full-length supporting cell target protein or active variant) or an active hair cell target protein (e.g., a wildtype, full-length hair cell target protein or active variant), and/or an increase in one or more activities of a target protein (e.g., a supporting cell target protein or a hair cell target protein) in a target cell (e.g., as compared to a reference level, such as the level(s) in a primate prior to treatment, the level(s) in a primate having a mutation in a target gene (e.g., a supporting cell target gene or a hair cell target gene), or the level(s) in a primate or a population of primates having hearing loss, e.g., non-syndromic sensorineural hearing loss or syndromic sensorineural hearing loss).

The term "nucleic acid" or "polynucleotide" refers to deoxyribonucleic acid (DNA) or ribonucleic acid (RNA), or a combination thereof, in either single- or doublestranded form. Unless specifically limited, the term encompasses nucleic acids containing known analogues of natural nucleotides that have similar binding properties as the reference nucleotides. Unless otherwise indicated, a particular nucleic acid sequence also implicitly encompasses complementary sequences as well as the sequence explicitly indicated. In some embodiments of any of the nucleic acids described herein, the nucleic acid is DNA. In some embodiments of any of the nucleic acids described herein, the nucleic acid is RNA.

The term "active hair cell target protein" means a protein encoded by DNA that, if substituted for both wildtype alleles encoding full-length hair cell target protein in hair cells of what is otherwise a wildtype primate, and if expressed in the hair cells of that primate, results in that primate's having a level of hearing approximating the normal level of hearing of a similar primate that is entirely wildtype. Non-limiting examples of active hair cell target proteins are full-length hair cell target proteins (e.g., any of the full-length hair cell target proteins described herein).

The term "active supporting cell target protein" means a protein encoded by DNA that, if substituted for both wildtype alleles encoding full-length supporting cell target protein in supporting cells of what is otherwise a wildtype primate, and if expressed in the supporting cells of that primate, results in that primate's having a level of hearing approximating the normal level of hearing of a similar primate that is entirely wildtype. Non-limiting examples of active supporting cell target proteins are full-length supporting cell target proteins (e.g., any of the full-length supporting cell target proteins described herein).

For example, an active target protein (e.g., an active supporting cell target protein or an active hair cell target protein) can include a sequence of a wildtype, full-length target protein (e.g., a supporting cell target protein (e.g., a wildtype, human, full-length supporting cell target protein) or a hair cell target protein (e.g., a wildtype, human, full-length hair cell target protein) including 1 amino acid substitution to about 160 amino acid substitutions, 1 amino acid substitution to about 155 amino acid substitutions, 1 amino acid substitution to about 150 amino acid substitutions, 1 amino acid substitution to about 145 amino acid substitutions, 1 amino acid substitution to about 140 amino acid substitutions, 1 amino acid substitution to about 135 amino acid substitutions, 1 amino acid substitution to about 130 amino acid substitutions, 1 amino acid substitution to about 125 amino acid substitutions, 1 amino acid substitution to about 120 amino acid substitutions, 1 amino acid substitution to about 115 amino acid substitutions, 1 amino acid substitution to about 110 amino acid substitutions, 1 amino acid substitution to about 105 amino acid substitutions, 1 amino acid substitution to about 100 amino acid substitutions, 1 amino acid substitution to about 95 amino acid substitutions, 1 amino acid substitution to about 90 amino acid substitutions, 1 amino acid substitution to about 85 amino acid substitutions, 1 amino acid substitution to about 80 amino acid substitutions, 1 amino acid substitution to about 75 amino acid substitutions, 1 amino acid substitution to about 70 amino acid substitutions, 1 amino acid substitution to about 65 amino acid substitutions, 1 amino acid substitution to about 60 amino acid substitutions, 1 amino acid substitution to about 55 amino acid substitutions, 1 amino acid substitution to about 50 amino acid substitutions, 1 amino acid substitution to about 45 amino acid substitutions, 1 amino acid substitution to about 40 amino acid substitutions, 1 amino acid substitution to about 35 amino acid substitutions, 1 amino acid substitution to about 30 amino acid substitutions, 1 amino acid substitution to about 25 amino acid substitutions, 1 amino acid substitution to about 20 amino acid substitutions, 1 amino acid substitution to about 15 amino acid substitutions, 1 amino acid substitution to about 10 amino acid substitutions, 1 amino acid substitution to about 9 amino acid substitutions, 1 amino acid substitution to about 8 amino acid substitutions, 1 amino acid substitution to about 7 amino acid substitutions, 1 amino acid substitution to about 6 amino acid substitutions, 1 amino acid substitution to about 5 amino acid substitutions, 1 amino acid substitution to about 4 amino acid substitutions, 1 amino acid substitution to about 3 amino acid substitutions, between about 2 amino acid substitutions to about 160 amino acid substitutions, about 2 amino acid substitutions to about 155 amino acid substitutions, about 2 amino acid substitutions to about 150 amino acid substitutions, about 2 amino acid substitutions to about 145 amino acid substitutions, about 2 amino acid substitutions to about 140 amino acid substitutions, about 2 amino acid substitutions to about 135 amino acid substitutions, about 2 amino acid substitutions to about 130 amino acid substitutions, about 2 amino acid substitutions to about 125 amino acid substitutions, about 2 amino acid substitutions to about 120 amino acid substitutions, about 2 amino acid substitutions to about 115 amino acid substitutions, about 2 amino acid substitutions to about 110 amino acid substitutions, about 2 amino acid substitutions to about 105 amino acid substitutions, about 2 amino acid substitutions to about 100 amino acid substitutions, about 2 amino acid substitutions to about 95 amino acid substitutions, about 2 amino acid substitutions to about 90 amino acid substitutions, about 2 amino acid substitutions to about 85 amino acid substitutions, about 2 amino acid substitutions to about 80 amino acid substitutions, about 2 amino acid substitutions to about 75 amino acid substitutions, about 2 amino acid substitutions to about 70 amino acid substitutions, about 2 amino acid substitutions to about 65 amino acid substitutions, about 2 amino acid substitutions to about 60 amino acid substitutions, about 2 amino acid substitutions to about 55 amino acid substitutions, about 2 amino acid substitutions to about 50 amino acid substitutions, about 2 amino acid substitutions to about 45 amino acid substitutions, about 2 amino acid substitutions to about 40 amino acid substitutions, about 2 amino acid substitutions to about 35 amino acid substitutions, about 2 amino acid substitutions to about 30 amino acid substitutions, about 2 amino acid substitutions to about 25 amino acid substitutions, about 2 amino acid substitutions to about 20 amino acid substitutions, about 2 amino acid substitutions to about 15 amino acid substitutions, about 2 amino acid substitutions to about 10 amino acid substitutions, about 2 amino acid substitutions to about 9 amino acid substitutions, about 2 amino acid substitutions to about 8 amino acid substitutions, about 2 amino acid substitutions to about 7 amino acid substitutions, about 2 amino acid substitutions to about 6 amino acid substitutions, about 2 amino acid substitutions to about 5 amino acid substitutions, about 2 amino acid substitutions to about 4 amino acid substitutions, between about 3 amino acid substitutions to about 160 amino acid substitutions, about 3 amino acid substitutions to about 155 amino acid substitutions, about 3 amino acid substitutions to about 150 amino acid substitutions, about 3 amino acid substitutions to about 145 amino acid substitutions, about 3 amino acid substitutions to about 140 amino acid substitutions, about 3 amino acid substitutions to about 135 amino acid substitutions, about 3 amino acid substitutions to about 130 amino acid substitutions, about 3 amino acid substitutions to about 125 amino acid substitutions, about 3 amino acid substitutions to about 120 amino acid substitutions, about 3 amino acid substitutions to about 115 amino acid substitutions, about 3 amino acid substitutions to about 110 amino acid substitutions, about 3 amino acid substitutions to about 105 amino acid substitutions, about 3 amino acid substitutions to about 100 amino acid substitutions, about 3 amino acid substitutions to about 95 amino acid substitutions, about 3 amino acid substitutions to about 90 amino acid substitutions, about 3 amino acid substitutions to about 85 amino acid substitutions, about 3 amino acid substitutions to about 80 amino acid substitutions, about 3 amino acid substitutions to about 75 amino acid substitutions, about 3 amino acid substitutions to about 70 amino acid substitutions, about 3 amino acid substitutions to about 65 amino acid substitutions, about 3 amino acid substitutions to about 60 amino acid substitutions, about 3 amino acid substitutions to about 55 amino acid substitutions, about 3 amino acid substitutions to about 50 amino acid substitutions, about 3 amino acid substitutions to about 45 amino acid substitutions, about 3 amino acid substitutions to about 40 amino acid substitutions, about 3 amino acid substitutions to about 35 amino acid substitutions, about 3 amino acid substitutions to about 30 amino acid substitutions, about 3 amino acid substitutions to about 25 amino acid substitutions, about 3 amino acid substitutions to about 20 amino acid substitutions, about 3 amino acid substitutions to about 15 amino acid substitutions, about 3 amino acid substitutions to about 10 amino acid substitutions, about 3 amino acid substitutions to about 9 amino acid substitutions, about 3 amino acid substitutions to about 8 amino acid substitutions, about 3 amino acid substitutions to about 7 amino acid substitutions, about 3 amino acid substitutions to about 6 amino acid substitutions, about 3 amino acid substitutions to about 5 amino acid substitutions, between about 4 amino acid substitutions to about 160 amino acid substitutions, about 4 amino acid substitutions to about 155 amino acid substitutions, about 4 amino acid substitutions to about 150 amino acid substitutions, about 4 amino acid substitutions to about 145 amino acid substitutions, about 4 amino acid substitutions to about 140 amino acid substitutions, about 4 amino acid substitutions to about 135 amino acid substitutions, about 4 amino acid substitutions to about 130 amino acid substitutions, about 4 amino acid substitutions to about 125 amino acid substitutions, about 4 amino acid substitutions to about 120 amino acid substitutions, about 4 amino acid substitutions to about 115 amino acid substitutions, about 4 amino acid substitutions to about 110 amino acid substitutions, about 4 amino acid substitutions to about 105 amino acid substitutions, about 4 amino acid substitutions to about 100 amino acid substitutions, about 4 amino acid substitutions to about 95 amino acid substitutions, about 4 amino acid substitutions to about 90 amino acid substitutions, about 4 amino acid substitutions to about 85 amino acid substitutions, about 4 amino acid substitutions to about 80 amino acid substitutions, about 4 amino acid substitutions to about 75 amino acid substitutions, about 4 amino acid substitutions to about 70 amino acid substitutions, about 4 amino acid substitutions to about 65 amino acid substitutions, about 4 amino acid substitutions to about 60 amino acid substitutions, about 4 amino acid substitutions to about 55 amino acid substitutions, about 4 amino acid substitutions to about 50 amino acid substitutions, about 4 amino acid substitutions to about 45 amino acid substitutions, about 4 amino acid substitutions to about 40 amino acid substitutions, about 4 amino acid substitutions to about 35 amino acid substitutions, about 4 amino acid substitutions to about 30 amino acid substitutions, about 4 amino acid substitutions to about 25 amino acid substitutions, about 4 amino acid substitutions to about 20 amino acid substitutions, about 4 amino acid substitutions to about 15 amino acid substitutions, about 4 amino acid substitutions to about 10 amino acid substitutions, about 4 amino acid substitutions to about 9 amino acid substitutions, about 4 amino acid substitutions to about 8 amino acid substitutions, about 4 amino acid substitutions to about 7 amino acid substitutions, about 4 amino acid substitutions to about 6 amino acid substitutions, between about 5 amino acid substitutions to about 160 amino acid substitutions, about 5 amino acid substitutions to about 155 amino acid substitutions, about 5 amino acid substitutions to about 150 amino acid substitutions, about 5 amino acid substitutions to about 145 amino acid substitutions, about 5 amino acid substitutions to about 140 amino acid substitutions, about 5 amino acid substitutions to about 135 amino acid substitutions, about 5 amino acid substitutions to about 130 amino acid substitutions, about 5 amino acid substitutions to about 125 amino acid substitutions, about 5 amino acid substitutions to about 120 amino acid substitutions, about 5 amino acid substitutions to about 115 amino acid substitutions, about 5 amino acid substitutions to about 110 amino acid substitutions, about 5 amino acid substitutions to about 105 amino acid substitutions, about 5 amino acid substitutions to about 100 amino acid substitutions, about 5 amino acid substitutions to about 95 amino acid substitutions, about 5 amino acid substitutions to about 90 amino acid substitutions, about 5 amino acid substitutions to about 85 amino acid substitutions, about 5 amino acid substitutions to about 80 amino acid substitutions, about 5 amino acid substitutions to about 75 amino acid substitutions, about 5 amino acid substitutions to about 70 amino acid substitutions, about 5 amino acid substitutions to about 65 amino acid substitutions, about 5 amino acid substitutions to about 60 amino acid substitutions, about 5 amino acid substitutions to about 55 amino acid substitutions, about 5 amino acid substitutions to about 50 amino acid substitutions, about 5 amino acid substitutions to about 45 amino acid substitutions, about 5 amino acid substitutions to about 40 amino acid substitutions, about 5 amino acid substitutions to about 35 amino acid substitutions, about 5 amino acid substitutions to about 30 amino acid substitutions, about 5 amino acid substitutions to about 25 amino acid substitutions, about 5 amino acid substitutions to about 20 amino acid substitutions, about 5 amino acid substitutions to about 15 amino acid substitutions, about 5 amino acid substitutions to about 10 amino acid substitutions, about 5 amino acid substitutions to about 9 amino acid substitutions, about 5 amino acid substitutions to about 8 amino acid substitutions, about 5 amino acid substitutions to about 7 amino acid substitutions, between about 6 amino acid substitutions to about 160 amino acid substitutions, about 6 amino acid substitutions to about 155 amino acid substitutions, about 6 amino acid substitutions to about 150 amino acid substitutions, about 6 amino acid substitutions to about 145 amino acid substitutions, about 6 amino acid substitutions to about 140 amino acid substitutions, about 6 amino acid substitutions to about 135 amino acid substitutions, about 6 amino acid substitutions to about 130 amino acid substitutions, about 6 amino acid substitutions to about 125 amino acid substitutions, about 6 amino acid substitutions to about 120 amino acid substitutions, about 6 amino acid substitutions to about 115 amino acid substitutions, about 6 amino acid substitutions to about 110 amino acid substitutions, about 6 amino acid substitutions to about 105 amino acid substitutions, about 6 amino acid substitutions to about 100 amino acid substitutions, about 6 amino acid substitutions to about 95 amino acid substitutions, about 6 amino acid substitutions to about 90 amino acid substitutions, about 6 amino acid substitutions to about 85 amino acid substitutions, about 6 amino acid substitutions to about 80 amino acid substitutions, about 6 amino acid substitutions to about 75 amino acid substitutions, about 6 amino acid substitutions to about 70 amino acid substitutions, about 6 amino acid substitutions to about 65 amino acid substitutions, about 6 amino acid substitutions to about 60 amino acid substitutions, about 6 amino acid substitutions to about 55 amino acid substitutions, about 6 amino acid substitutions to about 50 amino acid substitutions, about 6 amino acid substitutions to about 45 amino acid substitutions, about 6 amino acid substitutions to about 40 amino acid substitutions, about 6 amino acid substitutions to about 35 amino acid substitutions, about 6 amino acid substitutions to about 30 amino acid substitutions, about 6 amino acid substitutions to about 25 amino acid substitutions, about 6 amino acid substitutions to about 20 amino acid substitutions, about 6 amino acid substitutions to about 15 amino acid substitutions, about 6 amino acid substitutions to about 10 amino acid substitutions, about 6 amino acid substitutions to about 9 amino acid substitutions, about 6 amino acid substitutions to about 8 amino acid substitutions, between about 7 amino acid substitutions to about 160 amino acid substitutions, about 7 amino acid substitutions to about 155 amino acid substitutions, about 7 amino acid substitutions to about 150 amino acid substitutions, about 7 amino acid substitutions to about 145 amino acid substitutions, about 7 amino acid substitutions to about 140 amino acid substitutions, about 7 amino acid substitutions to about 135 amino acid substitutions, about 7 amino acid substitutions to about 130 amino acid substitutions, about 7 amino acid substitutions to about 125 amino acid substitutions, about 7 amino acid substitutions to about 120 amino acid substitutions, about 7 amino acid substitutions to about 115 amino acid substitutions, about 7 amino acid substitutions to about 110 amino acid substitutions, about 7 amino acid substitutions to about 105 amino acid substitutions, about 7 amino acid substitutions to about 100 amino acid substitutions, about 7 amino acid substitutions to about 95 amino acid substitutions, about 7 amino acid substitutions to about 90 amino acid substitutions, about 7 amino acid substitutions to about 85 amino acid substitutions, about 7 amino acid substitutions to about 80 amino acid substitutions, about 7 amino acid substitutions to about 75 amino acid substitutions, about 7 amino acid substitutions to about 70 amino acid substitutions, about 7 amino acid substitutions to about 65 amino acid substitutions, about 7 amino acid substitutions to about 60 amino acid substitutions, about 7 amino acid substitutions to about 55 amino acid substitutions, about 7 amino acid substitutions to about 50 amino acid substitutions, about 7 amino acid substitutions to about 45 amino acid substitutions, about 7 amino acid substitutions to about 40 amino acid substitutions, about 7 amino acid substitutions to about 35 amino acid substitutions, about 7 amino acid substitutions to about 30 amino acid substitutions, about 7 amino acid substitutions to about 25 amino acid substitutions, about 7 amino acid substitutions to about 20 amino acid substitutions, about 7 amino acid substitutions to about 15 amino acid substitutions, about 7 amino acid substitutions to about 10 amino acid substitutions, about 7 amino acid substitutions to about 9 amino acid substitutions, between about 8 amino acid substitutions to about 160 amino acid substitutions, about 8 amino acid substitutions to about 155 amino acid substitutions, about 8 amino acid substitutions to about 150 amino acid substitutions, about 8 amino acid substitutions to about 145 amino acid substitutions, about 8 amino acid substitutions to about 140 amino acid substitutions, about 8 amino acid substitutions to about 135 amino acid substitutions, about 8 amino acid substitutions to about 130 amino acid substitutions, about 8 amino acid substitutions to about 125 amino acid substitutions, about 8 amino acid substitutions to about 120 amino acid substitutions, about 8 amino acid substitutions to about 115 amino acid substitutions, about 8 amino acid substitutions to about 110 amino acid substitutions, about 8 amino acid substitutions to about 105 amino acid substitutions, about 8 amino acid substitutions to about 100 amino acid substitutions, about 8 amino acid substitutions to about 95 amino acid substitutions, about 8 amino acid substitutions to about 90 amino acid substitutions, about 8 amino acid substitutions to about 85 amino acid substitutions, about 8 amino acid substitutions to about 80 amino acid substitutions, about 8 amino acid substitutions to about 75 amino acid substitutions, about 8 amino acid substitutions to about 70 amino acid substitutions, about 8 amino acid substitutions to about 65 amino acid substitutions, about 8 amino acid substitutions to about 60 amino acid substitutions, about 8 amino acid substitutions to about 55 amino acid substitutions, about 8 amino acid substitutions to about 50 amino acid substitutions, about 8 amino acid substitutions to about 45 amino acid substitutions, about 8 amino acid substitutions to about 40 amino acid substitutions, about 8 amino acid substitutions to about 35 amino acid substitutions, about 8 amino acid substitutions to about 30 amino acid substitutions, about 8 amino acid substitutions to about 25 amino acid substitutions, about 8 amino acid substitutions to about 20 amino acid substitutions, about 8 amino acid substitutions to about 15 amino acid substitutions, about 8 amino acid substitutions to about 10 amino acid substitutions, between about 10 amino acid substitutions to about 160 amino acid substitutions, about 10 amino acid substitutions to about 155 amino acid substitutions, about 10 amino acid substitutions to about 150 amino acid substitutions, about 10 amino acid substitutions to about 145 amino acid substitutions, about 10 amino acid substitutions to about 140 amino acid substitutions, about 10 amino acid substitutions to about 135 amino acid substitutions, about 10 amino acid substitutions to about 130 amino acid substitutions, about 10 amino acid substitutions to about 125 amino acid substitutions, about 10 amino acid substitutions to about 120 amino acid substitutions, about 10 amino acid substitutions to about 115 amino acid substitutions, about 10 amino acid substitutions to about 110 amino acid substitutions, about 10 amino acid substitutions to about 105 amino acid substitutions, about 10 amino acid substitutions to about 100 amino acid substitutions, about 10 amino acid substitutions to about 95 amino acid substitutions, about 10 amino acid substitutions to about 90 amino acid substitutions, about 10 amino acid substitutions to about 85 amino acid substitutions, about 10 amino acid substitutions to about 80 amino acid substitutions, about 10 amino acid substitutions to about 75 amino acid substitutions, about 10 amino acid substitutions to about 70 amino acid substitutions, about 10 amino acid substitutions to about 65 amino acid substitutions, about 10 amino acid substitutions to about 60 amino acid substitutions, about 10 amino acid substitutions to about 55 amino acid substitutions, about 10 amino acid substitutions to about 50 amino acid substitutions, about 10 amino acid substitutions to about 45 amino acid substitutions, about 10 amino acid substitutions to about 40 amino acid substitutions, about 10 amino acid substitutions to about 35 amino acid substitutions, about 10 amino acid substitutions to about 30 amino acid substitutions, about 10 amino acid substitutions to about 25 amino acid substitutions, about 10 amino acid substitutions to about 20 amino acid substitutions, about 10 amino acid substitutions to about 15 amino acid substitutions, between about 15 amino acid substitutions to about 160 amino acid substitutions, about 15 amino acid substitutions to about 155 amino acid substitutions, about 15 amino acid substitutions to about 150 amino acid substitutions, about 15 amino acid substitutions to about 145 amino acid substitutions, about 15 amino acid substitutions to about 140 amino acid substitutions, about 15 amino acid substitutions to about 135 amino acid substitutions, about 15 amino acid substitutions to about 130 amino acid substitutions, about 15 amino acid substitutions to about 125 amino acid substitutions, about 15 amino acid substitutions to about 120 amino acid substitutions, about 15 amino acid substitutions to about 115 amino acid substitutions, about 15 amino acid substitutions to about 110 amino acid substitutions, about 15 amino acid substitutions to about 105 amino acid substitutions, about 15 amino acid substitutions to about 100 amino acid substitutions, about 15 amino acid substitutions to about 95 amino acid substitutions, about 15 amino acid substitutions to about 90 amino acid substitutions, about 15 amino acid substitutions to about 85 amino acid substitutions, about 15 amino acid substitutions to about 80 amino acid substitutions, about 15 amino acid substitutions to about 75 amino acid substitutions, about 15 amino acid substitutions to about 70 amino acid substitutions, about 15 amino acid substitutions to about 65 amino acid substitutions, about 15 amino acid substitutions to about 60 amino acid substitutions, about 15 amino acid substitutions to about 55 amino acid substitutions, about 15 amino acid substitutions to about 50 amino acid substitutions, about 15 amino acid substitutions to about 45 amino acid substitutions, about 15 amino acid substitutions to about 40 amino acid substitutions, about 15 amino acid substitutions to about 35 amino acid substitutions, about 15 amino acid substitutions to about 30 amino acid substitutions, about 15 amino acid substitutions to about 25 amino acid substitutions, about 15 amino acid substitutions to about 20 amino acid substitutions, between about 20 amino acid substitutions to about 160 amino acid substitutions, about 20 amino acid substitutions to about 155 amino acid substitutions, about 20 amino acid substitutions to about 150 amino acid substitutions, about 20 amino acid substitutions to about 145 amino acid substitutions, about 20 amino acid substitutions to about 140 amino acid substitutions, about 20 amino acid substitutions to about 135 amino acid substitutions, about 20 amino acid substitutions to about 130 amino acid substitutions, about 20 amino acid substitutions to about 125 amino acid substitutions, about 20 amino acid substitutions to about 120 amino acid substitutions, about 20 amino acid substitutions to about 115 amino acid substitutions, about 20 amino acid substitutions to about 110 amino acid substitutions, about 20 amino acid substitutions to about 105 amino acid substitutions, about 20 amino acid substitutions to about 100 amino acid substitutions, about 20 amino acid substitutions to about 95 amino acid substitutions, about 20 amino acid substitutions to about 90 amino acid substitutions, about 20 amino acid substitutions to about 85 amino acid substitutions, about 20 amino acid substitutions to about 80 amino acid substitutions, about 20 amino acid substitutions to about 75 amino acid substitutions, about 20 amino acid substitutions to about 70 amino acid substitutions, about 20 amino acid substitutions to about 65 amino acid substitutions, about 20 amino acid substitutions to about 60 amino acid substitutions, about 20 amino acid substitutions to about 55 amino acid substitutions, about 20 amino acid substitutions to about 50 amino acid substitutions, about 20 amino acid substitutions to about 45 amino acid substitutions, about 20 amino acid substitutions to about 40 amino acid substitutions, about 20 amino acid substitutions to about 35 amino acid substitutions, about 20 amino acid substitutions to about 30 amino acid substitutions, about 20 amino acid substitutions to about 25 amino acid substitutions, between about 25 amino acid substitutions to about 160 amino acid substitutions, about 25 amino acid substitutions to about 155 amino acid substitutions, about 25 amino acid substitutions to about 150 amino acid substitutions, about 25 amino acid substitutions to about 145 amino acid substitutions, about 25 amino acid substitutions to about 140 amino acid substitutions, about 25 amino acid substitutions to about 135 amino acid substitutions, about 25 amino acid substitutions to about 130 amino acid substitutions, about 25 amino acid substitutions to about 125 amino acid substitutions, about 25 amino acid substitutions to about 120 amino acid substitutions, about 25 amino acid substitutions to about 115 amino acid substitutions, about 25 amino acid substitutions to about 110 amino acid substitutions, about 25 amino acid substitutions to about 105 amino acid substitutions, about 25 amino acid substitutions to about 100 amino acid substitutions, about 25 amino acid substitutions to about 95 amino acid substitutions, about 25 amino acid substitutions to about 90 amino acid substitutions, about 25 amino acid substitutions to about 85 amino acid substitutions, about 25 amino acid substitutions to about 80 amino acid substitutions, about 25 amino acid substitutions to about 75 amino acid substitutions, about 25 amino acid substitutions to about 70 amino acid substitutions, about 25 amino acid substitutions to about 65 amino acid substitutions, about 25 amino acid substitutions to about 60 amino acid substitutions, about 25 amino acid substitutions to about 55 amino acid substitutions, about 25 amino acid substitutions to about 50 amino acid substitutions, about 25 amino acid substitutions to about 45 amino acid substitutions, about 25 amino acid substitutions to about 40 amino acid substitutions, about 25 amino acid substitutions to about 35 amino acid substitutions, about 25 amino acid substitutions to about 30 amino acid substitutions, between about 30 amino acid substitutions to about 160 amino acid substitutions, about 30 amino acid substitutions to about 155 amino acid substitutions, about 30 amino acid substitutions to about 150 amino acid substitutions, about 30 amino acid substitutions to about 145 amino acid substitutions, about 30 amino acid substitutions to about 140 amino acid substitutions, about 30 amino acid substitutions to about 135 amino acid substitutions, about 30 amino acid substitutions to about 130 amino acid substitutions, about 30 amino acid substitutions to about 125 amino acid substitutions, about 30 amino acid substitutions to about 120 amino acid substitutions, about 30 amino acid substitutions to about 115 amino acid substitutions, about 30 amino acid substitutions to about 110 amino acid substitutions, about 30 amino acid substitutions to about 105 amino acid substitutions, about 30 amino acid substitutions to about 100 amino acid substitutions, about 30 amino acid substitutions to about 95 amino acid substitutions, about 30 amino acid substitutions to about 90 amino acid substitutions, about 30 amino acid substitutions to about 85 amino acid substitutions, about 30 amino acid substitutions to about 80 amino acid substitutions, about 30 amino acid substitutions to about 75 amino acid substitutions, about 30 amino acid substitutions to about 70 amino acid substitutions, about 30 amino acid substitutions to about 65 amino acid substitutions, about 30 amino acid substitutions to about 60 amino acid substitutions, about 30 amino acid substitutions to about 55 amino acid substitutions, about 30 amino acid substitutions to about 50 amino acid substitutions, about 30 amino acid substitutions to about 45 amino acid substitutions, about 30 amino acid substitutions to about 40 amino acid substitutions, about 30 amino acid substitutions to about 35 amino acid substitutions, between about 35 amino acid substitutions to about 160 amino acid substitutions, about 35 amino acid substitutions to about 155 amino acid substitutions, about 35 amino acid substitutions to about 150 amino acid substitutions, about 35 amino acid substitutions to about 145 amino acid substitutions, about 35 amino acid substitutions to about 140 amino acid substitutions, about 35 amino acid substitutions to about 135 amino acid substitutions, about 35 amino acid substitutions to about 130 amino acid substitutions, about 35 amino acid substitutions to about 125 amino acid substitutions, about 35 amino acid substitutions to about 120 amino acid substitutions, about 35 amino acid substitutions to about 115 amino acid substitutions, about 35 amino acid substitutions to about 110 amino acid substitutions, about 35 amino acid substitutions to about 105 amino acid substitutions, about 35 amino acid substitutions to about 100 amino acid substitutions, about 35 amino acid substitutions to about 95 amino acid substitutions, about 35 amino acid substitutions to about 90 amino acid substitutions, about 35 amino acid substitutions to about 85 amino acid substitutions, about 35 amino acid substitutions to about 80 amino acid substitutions, about 35 amino acid substitutions to about 75 amino acid substitutions, about 35 amino acid substitutions to about 70 amino acid substitutions, about 35 amino acid substitutions to about 65 amino acid substitutions, about 35 amino acid substitutions to about 60 amino acid substitutions, about 35 amino acid substitutions to about 55 amino acid substitutions, about 35 amino acid substitutions to about 50 amino acid substitutions, about 35 amino acid substitutions to about 45 amino acid substitutions, about 35 amino acid substitutions to about 40 amino acid substitutions, between about 40 amino acid substitutions to about 160 amino acid substitutions, about 40 amino acid substitutions to about 155 amino acid substitutions, about 40 amino acid substitutions to about 150 amino acid substitutions, about 40 amino acid substitutions to about 145 amino acid substitutions, about 40 amino acid substitutions to about 140 amino acid substitutions, about 40 amino acid substitutions to about 135 amino acid substitutions, about 40 amino acid substitutions to about 130 amino acid substitutions, about 40 amino acid substitutions to about 125 amino acid substitutions, about 40 amino acid substitutions to about 120 amino acid substitutions, about 40 amino acid substitutions to about 115 amino acid substitutions, about 40 amino acid substitutions to about 110 amino acid substitutions, about 40 amino acid substitutions to about 105 amino acid substitutions, about 40 amino acid substitutions to about 100 amino acid substitutions, about 40 amino acid substitutions to about 95 amino acid substitutions, about 40 amino acid substitutions to about 90 amino acid substitutions, about 40 amino acid substitutions to about 85 amino acid substitutions, about 40 amino acid substitutions to about 80 amino acid substitutions, about 40 amino acid substitutions to about 75 amino acid substitutions, about 40 amino acid substitutions to about 70 amino acid substitutions, about 40 amino acid substitutions to about 65 amino acid substitutions, about 40 amino acid substitutions to about 60 amino acid substitutions, about 40 amino acid substitutions to about 55 amino acid substitutions, about 40 amino acid substitutions to about 50 amino acid substitutions, about 40 amino acid substitutions to about 45 amino acid substitutions, between about 45 amino acid substitutions to about 160 amino acid substitutions, about 45 amino acid substitutions to about 155 amino acid substitutions, about 45 amino acid substitutions to about 150 amino acid substitutions, about 45 amino acid substitutions to about 145 amino acid substitutions, about 45 amino acid substitutions to about 140 amino acid substitutions, about 45 amino acid substitutions to about 135 amino acid substitutions, about 45 amino acid substitutions to about 130 amino acid substitutions, about 45 amino acid substitutions to about 125 amino acid substitutions, about 45 amino acid substitutions to about 120 amino acid substitutions, about 45 amino acid substitutions to about 115 amino acid substitutions, about 45 amino acid substitutions to about 110 amino acid substitutions, about 45 amino acid substitutions to about 105 amino acid substitutions, about 45 amino acid substitutions to about 100 amino acid substitutions, about 45 amino acid substitutions to about 95 amino acid substitutions, about 45 amino acid substitutions to about 90 amino acid substitutions, about 45 amino acid substitutions to about 85 amino acid substitutions, about 45 amino acid substitutions to about 80 amino acid substitutions, about 45 amino acid substitutions to about 75 amino acid substitutions, about 45 amino acid substitutions to about 70 amino acid substitutions, about 45 amino acid substitutions to about 65 amino acid substitutions, about 45 amino acid substitutions to about 60 amino acid substitutions, about 45 amino acid substitutions to about 55 amino acid substitutions, about 45 amino acid substitutions to about 50 amino acid substitutions, between about 50 amino acid substitutions to about 160 amino acid substitutions, about 50 amino acid substitutions to about 155 amino acid substitutions, about 50 amino acid substitutions to about 150 amino acid substitutions, about 50 amino acid substitutions to about 145 amino acid substitutions, about 50 amino acid substitutions to about 140 amino acid substitutions, about 50 amino acid substitutions to about 135 amino acid substitutions, about 50 amino acid substitutions to about 130 amino acid substitutions, about 50 amino acid substitutions to about 125 amino acid substitutions, about 50 amino acid substitutions to about 120 amino acid substitutions, about 50 amino acid substitutions to about 115 amino acid substitutions, about 50 amino acid substitutions to about 110 amino acid substitutions, about 50 amino acid substitutions to about 105 amino acid substitutions, about 50 amino acid substitutions to about 100 amino acid substitutions, about 50 amino acid substitutions to about 95 amino acid substitutions, about 50 amino acid substitutions to about 90 amino acid substitutions, about 50 amino acid substitutions to about 85 amino acid substitutions, about 50 amino acid substitutions to about 80 amino acid substitutions, about 50 amino acid substitutions to about 75 amino acid substitutions, about 50 amino acid substitutions to about 70 amino acid substitutions, about 50 amino acid substitutions to about 65 amino acid substitutions, about 50 amino acid substitutions to about 60 amino acid substitutions, about 50 amino acid substitutions to about 55 amino acid substitutions, between about 60 amino acid substitutions to about 160 amino acid substitutions, about 60 amino acid substitutions to about 155 amino acid substitutions, about 60 amino acid substitutions to about 150 amino acid substitutions, about 60 amino acid substitutions to about 145 amino acid substitutions, about 60 amino acid substitutions to about 140 amino acid substitutions, about 60 amino acid substitutions to about 135 amino acid substitutions, about 60 amino acid substitutions to about 130 amino acid substitutions, about 60 amino acid substitutions to about 125 amino acid substitutions, about 60 amino acid substitutions to about 120 amino acid substitutions, about 60 amino acid substitutions to about 115 amino acid substitutions, about 60 amino acid substitutions to about 110 amino acid substitutions, about 60 amino acid substitutions to about 105 amino acid substitutions, about 60 amino acid substitutions to about 100 amino acid substitutions, about 60 amino acid substitutions to about 95 amino acid substitutions, about 60 amino acid substitutions to about 90 amino acid substitutions, about 60 amino acid substitutions to about 85 amino acid substitutions, about 60 amino acid substitutions to about 80 amino acid substitutions, about 60 amino acid substitutions to about 75 amino acid substitutions, about 60 amino acid substitutions to about 70 amino acid substitutions, about 60 amino acid substitutions to about 65 amino acid substitutions, between about 70 amino acid substitutions to about 160 amino acid substitutions, about 70 amino acid substitutions to about 155 amino acid substitutions, about 70 amino acid substitutions to about 150 amino acid substitutions, about 70 amino acid substitutions to about 145 amino acid substitutions, about 70 amino acid substitutions to about 140 amino acid substitutions, about 70 amino acid substitutions to about 135 amino acid substitutions, about 70 amino acid substitutions to about 130 amino acid substitutions, about 70 amino acid substitutions to about 125 amino acid substitutions, about 70 amino acid substitutions to about 120 amino acid substitutions, about 70 amino acid substitutions to about 115 amino acid substitutions, about 70 amino acid substitutions to about 110 amino acid substitutions, about 70 amino acid substitutions to about 105 amino acid substitutions, about 70 amino acid substitutions to about 100 amino acid substitutions, about 70 amino acid substitutions to about 95 amino acid substitutions, about 70 amino acid substitutions to about 90 amino acid substitutions, about 70 amino acid substitutions to about 85 amino acid substitutions, about 70 amino acid substitutions to about 80 amino acid substitutions, about 70 amino acid substitutions to about 75 amino acid substitutions, between about 80 amino acid substitutions to about 160 amino acid substitutions, about 80 amino acid substitutions to about 155 amino acid substitutions, about 80 amino acid substitutions to about 150 amino acid substitutions, about 80 amino acid substitutions to about 145 amino acid substitutions, about 80 amino acid substitutions to about 140 amino acid substitutions, about 80 amino acid substitutions to about 135 amino acid substitutions, about 80 amino acid substitutions to about 130 amino acid substitutions, about 80 amino acid substitutions to about 125 amino acid substitutions, about 80 amino acid substitutions to about 120 amino acid substitutions, about 80 amino acid substitutions to about 115 amino acid substitutions, about 80 amino acid substitutions to about 110 amino acid substitutions, about 80 amino acid substitutions to about 105 amino acid substitutions, about 80 amino acid substitutions to about 100 amino acid substitutions, about 80 amino acid substitutions to about 95 amino acid substitutions, about 80 amino acid substitutions to about 90 amino acid substitutions, about 80 amino acid substitutions to about 85 amino acid substitutions, between about 90 amino acid substitutions to about 160 amino acid substitutions, about 90 amino acid substitutions to about 155 amino acid substitutions, about 90 amino acid substitutions to about 150 amino acid substitutions, about 90 amino acid substitutions to about 145 amino acid substitutions, about 90 amino acid substitutions to about 140 amino acid substitutions, about 90 amino acid substitutions to about 135 amino acid substitutions, about 90 amino acid substitutions to about 130 amino acid substitutions, about 90 amino acid substitutions to about 125 amino acid substitutions, about 90 amino acid substitutions to about 120 amino acid substitutions, about 90 amino acid substitutions to about 115 amino acid substitutions, about 90 amino acid substitutions to about 110 amino acid substitutions, about 90 amino acid substitutions to about 105 amino acid substitutions, about 90 amino acid substitutions to about 100 amino acid substitutions, about 90 amino acid substitutions to about 95 amino acid substitutions, between about 100 amino acid substitutions to about 160 amino acid substitutions, about 100 amino acid substitutions to about 155 amino acid substitutions, about 100 amino acid substitutions to about 150 amino acid substitutions, about 100 amino acid substitutions to about 145 amino acid substitutions, about 100 amino acid substitutions to about 140 amino acid substitutions, about 100 amino acid substitutions to about 135 amino acid substitutions, about 100 amino acid substitutions to about 130 amino acid substitutions, about 100 amino acid substitutions to about 125 amino acid substitutions, about 100 amino acid substitutions to about 120 amino acid substitutions, about 100 amino acid substitutions to about 115 amino acid substitutions, about 100 amino acid substitutions to about 110 amino acid substitutions, about 100 amino acid substitutions to about 105 amino acid substitutions, between about 110 amino acid substitutions to about 160 amino acid substitutions, about 110 amino acid substitutions to about 155 amino acid substitutions, about 110 amino acid substitutions to about 150 amino acid substitutions, about 110 amino acid substitutions to about 145 amino acid substitutions, about 110 amino acid substitutions to about 140 amino acid substitutions, about 110 amino acid substitutions to about 135 amino acid substitutions, about 110 amino acid substitutions to about 130 amino acid substitutions, about 110 amino acid substitutions to about 125 amino acid substitutions, about 110 amino acid substitutions to about 120 amino acid substitutions, about 110 amino acid substitutions to about 115 amino acid substitutions, between about 120 amino acid substitutions to about 160 amino acid substitutions, about 120 amino acid substitutions to about 155 amino acid substitutions, about 120 amino acid substitutions to about 150 amino acid substitutions, about 120 amino acid substitutions to about 145 amino acid substitutions, about 120 amino acid substitutions to about 140 amino acid substitutions, about 120 amino acid substitutions to about 135 amino acid substitutions, about 120 amino acid substitutions to about 130 amino acid substitutions, about 120 amino acid substitutions to about 125 amino acid substitutions, between about 130 amino acid substitutions to about 160 amino acid substitutions, about 130 amino acid substitutions to about 155 amino acid substitutions, about 130 amino acid substitutions to about 150 amino acid substitutions, about 130 amino acid substitutions to about 145 amino acid substitutions, about 130 amino acid substitutions to about 140 amino acid substitutions, about 130 amino acid substitutions to about 135 amino acid substitutions, between about 140 amino acid substitutions to about 160 amino acid substitutions, about 140 amino acid substitutions to about 155 amino acid substitutions, about 140 amino acid substitutions to about 150 amino acid substitutions, about 140 amino acid substitutions to about 145 amino acid substitutions, between about 150 amino acid substitutions to about 160 amino acid substitutions, or about 150 amino acid substitutions to about 155 amino acid substitutions. One skilled in the art would appreciate that amino acids that are conserved between wildtype supporting cell target proteins from different species or wildtype hair cell target proteins from different species can be mutated without losing activity, while those amino acids that are not conserved between wildtype supporting cell target proteins from different species or wildtype hair cell target proteins from different species should not be mutated as they are more likely (than amino acids that are not conserved between different species) to be involved in activity.

An active target protein (e.g., a supporting cell target protein or a hair cell target protein) can include, e.g., a sequence of a wildtype, full-length target protein (e.g., a supporting cell target protein (e.g., a wildtype, human, full-length supporting cell target protein) or a hair cell target protein (e.g., a wildtype, human, full-length hair cell target protein) that has 1 amino acid to about 50 amino acids, 1 amino acid to about 45 amino acids, 1 amino acid to about 40 amino acids, 1 amino acid to about 35 amino acids, 1 amino acid to about 30 amino acids, 1 amino acid to about 25 amino acids, 1 amino acid to about 20 amino acids, 1 amino acid to about 15 amino acids, 1 amino acid to about 10 amino acids, 1 amino acid to about 9 amino acids, 1 amino acid to about 8 amino acids, 1 amino acid to about 7 amino acids, 1 amino acid to about 6 amino acids, 1 amino acid to about 5 amino acids, 1 amino acid to about 4 amino acids, 1 amino acid to about 3 amino acids, about 2 amino acids to about 50 amino acids, about 2 amino acids to about 45 amino acids, about 2 amino acids to about 40 amino acids, about 2 amino acids to about 35 amino acids, about 2 amino acids to about 30 amino acids, about 2 amino acids to about 25 amino acids, about 2 amino acids to about 20 amino acids, about 2 amino acids to about 15 amino acids, about 2 amino acids to about 10 amino acids, about 2 amino acids to about 9 amino acids, about 2 amino acids to about 8 amino acids, about 2 amino acids to about 7 amino acids, about 2 amino acids to about 6 amino acids, about 2 amino acids to about 5 amino acids, about 2 amino acids to about 4 amino acids, about 3 amino acids to about 50 amino acids, about 3 amino acids to about 45 amino acids, about 3 amino acids to about 40 amino acids, about 3 amino acids to about 35 amino acids, about 3 amino acids to about 30 amino acids, about 3 amino acids to about 25 amino acids, about 3 amino acids to about 20 amino acids, about 3 amino acids to about 15 amino acids, about 3 amino acids to about 10 amino acids, about 3 amino acids to about 9 amino acids, about 3 amino acids to about 8 amino acids, about 3 amino acids to about 7 amino acids, about 3 amino acids to about 6 amino acids, about 3 amino acids to about 5 amino acids, about 4 amino acids to about 50 amino acids, about 4 amino acids to about 45 amino acids, about 4 amino acids to about 40 amino acids, about 4 amino acids to about 35 amino acids, about 4 amino acids to about 30 amino acids, about 4 amino acids to about 25 amino acids, about 4 amino acids to about 20 amino acids, about 4 amino acids to about 15 amino acids, about 4 amino acids to about 10 amino acids, about 4 amino acids to about 9 amino acids, about 4 amino acids to about 8 amino acids, about 4 amino acids to about 7 amino acids, about 4 amino acids to about 6 amino acids, about 5 amino acids to about 50 amino acids, about 5 amino acids to about 45 amino acids, about 5 amino acids to about 40 amino acids, about 5 amino acids to about 35 amino acids, about 5 amino acids to about 30 amino acids, about 5 amino acids to about 25 amino acids, about 5 amino acids to about 20 amino acids, about 5 amino acids to about 15 amino acids, about 5 amino acids to about 10 amino acids, about 5 amino acids to about 9 amino acids, about 5 amino acids to about 8 amino acids, about 5 amino acids to about 7 amino acids, about 6 amino acids to about 50 amino acids, about 6 amino acids to about 45 amino acids, about 6 amino acids to about 40 amino acids, about 6 amino acids to about 35 amino acids, about 6 amino acids to about 30 amino acids, about 6 amino acids to about 25 amino acids, about 6 amino acids to about 20 amino acids, about 6 amino acids to about 15 amino acids, about 6 amino acids to about 10 amino acids, about 6 amino acids to about 9 amino acids, about 6 amino acids to about 8 amino acids, about 7 amino acids to about 50 amino acids, about 7 amino acids to about 45 amino acids, about 7 amino acids to about 40 amino acids, about 7 amino acids to about 35 amino acids, about 7 amino acids to about 30 amino acids, about 7 amino acids to about 25 amino acids, about 7 amino acids to about 20 amino acids, about 7 amino acids to about 15 amino acids, about 7 amino acids to about 10 amino acids, about 7 amino acids to about 9 amino acids, about 8 amino acids to about 50 amino acids, about 8 amino acids to about 45 amino acids, about 8 amino acids to about 40 amino acids, about 8 amino acids to about 35 amino acids, about 8 amino acids to about 30 amino acids, about 8 amino acids to about 25 amino acids, about 8 amino acids to about 20 amino acids, about 8 amino acids to about 15 amino acids, about 8 amino acids to about 10 amino acids, about 10 amino acids to about 50 amino acids, about 10 amino acids to about 45 amino acids, about 10 amino acids to about 40 amino acids, about 10 amino acids to about 35 amino acids, about 10 amino acids to about 30 amino acids, about 10 amino acids to about 25 amino acids, about 10 amino acids to about 20 amino acids, about 10 amino acids to about 15 amino acids, about 15 amino acids to about 50 amino acids, about 15 amino acids to about 45 amino acids, about 15 amino acids to about 40 amino acids, about 15 amino acids to about 35 amino acids, about 15 amino acids to about 30 amino acids, about 15 amino acids to about 25 amino acids, about 15 amino acids to about 20 amino acids, about 20 amino acids to about 50 amino acids, about 20 amino acids to about 45 amino acids, about 20 amino acids to about 40 amino acids, about 20 amino acids to about 35 amino acids, about 20 amino acids to about 30 amino acids, about 20 amino acids to about 25 amino acids, about 25 amino acids to about 50 amino acids, about 25 amino acids to about 45 amino acids, about 25 amino acids to about 40 amino acids, about 25 amino acids to about 35 amino acids, about 25 amino acids to about 30 amino acids, about 30 amino acids to about 50 amino acids, about 30 amino acids to about 45 amino acids, about 30 amino acids to about 40 amino acids, about 30 amino acids to about 35 amino acids, about 35 amino acids to about 50 amino acids, about 35 amino acids to about 45 amino acids, about 35 amino acids to about 40 amino acids, about 40 amino acids to about 50 amino acids, about 40 amino acids to about 45 amino acids, about 45 amino acids to about 50 amino acids, deleted. In some embodiments where two or more amino acids are deleted from the sequence of a wildtype, full-length hair cell target protein, the two or more deleted amino acids can be contiguous in the sequence of the wildtype, full-length protein. In other examples where two or more amino acids are deleted from the sequence of a wildtype, full-length hair cell target protein, the two or more deleted amino acids are not contiguous in the sequence of the wildtype, full-length protein. One skilled in the art would appreciate that amino acids that are not conserved between wildtype, full-length hair cell target proteins from different species can be deleted without losing activity, while those amino acids that are conserved between wildtype, full-length hair cell target proteins from different species should not be deleted as they are more likely (than amino acids that are not conserved between different species) to be involved in activity.

In some embodiments where two or more amino acids are deleted from the sequence of a wildtype, full-length supporting cell target protein, the two or more deleted amino acids can be contiguous in the sequence of the wildtype, full-length protein. In other examples where two or more amino acids are deleted from the sequence of a wildtype, full-length supporting cell target protein, the two or more deleted amino acids are not contiguous in the sequence of the wildtype, full-length protein. One skilled in the art would appreciate that amino acids that are not conserved between wildtype, full-length supporting cell target proteins from different species can be deleted without losing activity, while those amino acids that are conserved between wildtype, full-length supporting cell target proteins from different species should not be deleted as they are more likely (than amino acids that are not conserved between different species) to be involved in activity.

In some examples, an active target protein (e.g., a supporting cell target protein or a hair cell target protein) can, e.g., include a sequence of a wildtype, full-length target protein (e.g., a wildtype, full-length supporting cell target protein or a wildtype, full-length hair cell target protein) that has between 1 amino acid to about 100 amino acids, 1 amino acid to about 95 amino acids, 1 amino acid to about 90 amino acids, 1 amino acid to about 85 amino acids, 1 amino acid to about 80 amino acids, 1 amino acid to about 75 amino acids, 1 amino acid to about 70 amino acids, 1 amino acid to about 65 amino acids, 1 amino acid to about 60 amino acids, 1 amino acid to about 55 amino acids, 1 amino acid to about 50 amino acids, 1 amino acid to about 45 amino acids, 1 amino acid to about 40 amino acids, 1 amino acid to about 35 amino acids, 1 amino acid to about 30 amino acids, 1 amino acid to about 25 amino acids, 1 amino acid to about 20 amino acids, 1 amino acid to about 15 amino acids, 1 amino acid to about 10 amino acids, 1 amino acid to about 9 amino acids, 1 amino acid to about 8 amino acids, 1 amino acid to about 7 amino acids, 1 amino acid to about 6 amino acids, 1 amino acid to about 5 amino acids, 1 amino acid to about 4 amino acids, 1 amino acid to about 3 amino acids, about 2 amino acids to about 100 amino acids, about 2 amino acid to about 95 amino acids, about 2 amino acids to about 90 amino acids, about 2 amino acids to about 85 amino acids, about 2 amino acids to about 80 amino acids, about 2 amino acids to about 75 amino acids, about 2 amino acids to about 70 amino acids, about 2 amino acids to about 65 amino acids, about 2 amino acids to about 60 amino acids, about 2 amino acids to about 55 amino acids, about 2 amino acids to about 50 amino acids, about 2 amino acids to about 45 amino acids, about 2 amino acids to about 40 amino acids, about 2 amino acids to about 35 amino acids, about 2 amino acids to about 30 amino acids, about 2 amino acids to about 25 amino acids, about 2 amino acids to about 20 amino acids, about 2 amino acids to about 15 amino acids, about 2 amino acids to about 10 amino acids, about 2 amino acids to about 9 amino acids, about 2 amino acids to about 8 amino acids, about 2 amino acids to about 7 amino acids, about 2 amino acids to about 6 amino acids, about 2 amino acids to about 5 amino acids, about 2 amino acids to about 4 amino acids, about 3 amino acids to about 100 amino acids, about 3 amino acid to about 95 amino acids, about 3 amino acids to about 90 amino acids, about 3 amino acids to about 85 amino acids, about 3 amino acids to about 80 amino acids, about 3 amino acids to about 75 amino acids, about 3 amino acids to about 70 amino acids, about 3 amino acids to about 65 amino acids, about 3 amino acids to about 60 amino acids, about 3 amino acids to about 55 amino acids, about 3 amino acids to about 50 amino acids, about 3 amino acids to about 45 amino acids, about 3 amino acids to about 40 amino acids, about 3 amino acids to about 35 amino acids, about 3 amino acids to about 30 amino acids, about 3 amino acids to about 25 amino acids, about 3 amino acids to about 20 amino acids, about 3 amino acids to about 15 amino acids, about 3 amino acids to about 10 amino acids, about 3 amino acids to about 9 amino acids, about 3 amino acids to about 8 amino acids, about 3 amino acids to about 7 amino acids, about 3 amino acids to about 6 amino acids, about 3 amino acids to about 5 amino acids, about 4 amino acids to about 100 amino acids, about 4 amino acid to about 95 amino acids, about 4 amino acids to about 90 amino acids, about 4 amino acids to about 85 amino acids, about 4 amino acids to about 80 amino acids, about 4 amino acids to about 75 amino acids, about 4 amino acids to about 70 amino acids, about 4 amino acids to about 65 amino acids, about 4 amino acids to about 60 amino acids, about 4 amino acids to about 55 amino acids, about 4 amino acids to about 50 amino acids, about 4 amino acids to about 45 amino acids, about 4 amino acids to about 40 amino acids, about 4 amino acids to about 35 amino acids, about 4 amino acids to about 30 amino acids, about 4 amino acids to about 25 amino acids, about 4 amino acids to about 20 amino acids, about 4 amino acids to about 15 amino acids, about 4 amino acids to about 10 amino acids, about 4 amino acids to about 9 amino acids, about 4 amino acids to about 8 amino acids, about 4 amino acids to about 7 amino acids, about 4 amino acids to about 6 amino acids, about 5 amino acids to about 100 amino acids, about 5 amino acid to about 95 amino acids, about 5 amino acids to about 90 amino acids, about 5 amino acids to about 85 amino acids, about 5 amino acids to about 80 amino acids, about 5 amino acids to about 75 amino acids, about 5 amino acids to about 70 amino acids, about 5 amino acids to about 65 amino acids, about 5 amino acids to about 60 amino acids, about 5 amino acids to about 55 amino acids, about 5 amino acids to about 50 amino acids, about 5 amino acids to about 45 amino acids, about 5 amino acids to about 40 amino acids, about 5 amino acids to about 35 amino acids, about 5 amino acids to about 30 amino acids, about 5 amino acids to about 25 amino acids, about 5 amino acids to about 20 amino acids, about 5 amino acids to about 15 amino acids, about 5 amino acids to about 10 amino acids, about 5 amino acids to about 9 amino acids, about 5 amino acids to about 8 amino acids, about 5 amino acids to about 7 amino acids, about 6 amino acids to about 100 amino acids, about 6 amino acid to about 95 amino acids, about 6 amino acids to about 90 amino acids, about 6 amino acids to about 85 amino acids, about 6 amino acids to about 80 amino acids, about 6 amino acids to about 75 amino acids, about 6 amino acids to about 70 amino acids, about 6 amino acids to about 65 amino acids, about 6 amino acids to about 60 amino acids, about 6 amino acids to about 55 amino acids, about 6 amino acids to about 50 amino acids, about 6 amino acids to about 45 amino acids, about 6 amino acids to about 40 amino acids, about 6 amino acids to about 35 amino acids, about 6 amino acids to about 30 amino acids, about 6 amino acids to about 25 amino acids, about 6 amino acids to about 20 amino acids, about 6 amino acids to about 15 amino acids, about 6 amino acids to about 10 amino acids, about 6 amino acids to about 9 amino acids, about 6 amino acids to about 8 amino acids, about 7 amino acids to about 100 amino acids, about 7 amino acid to about 95 amino acids, about 7 amino acids to about 90 amino acids, about 7 amino acids to about 85 amino acids, about 7 amino acids to about 80 amino acids, about 7 amino acids to about 75 amino acids, about 7 amino acids to about 70 amino acids, about 7 amino acids to about 65 amino acids, about 7 amino acids to about 60 amino acids, about 7 amino acids to about 55 amino acids, about 7 amino acids to about 50 amino acids, about 7 amino acids to about 45 amino acids, about 7 amino acids to about 40 amino acids, about 7 amino acids to about 35 amino acids, about 7 amino acids to about 30 amino acids, about 7 amino acids to about 25 amino acids, about 7 amino acids to about 20 amino acids, about 7 amino acids to about 15 amino acids, about 7 amino acids to about 10 amino acids, about 7 amino acids to about 9 amino acids, about 8 amino acids to about 100 amino acids, about 8 amino acid to about 95 amino acids, about 8 amino acids to about 90 amino acids, about 8 amino acids to about 85 amino acids, about 8 amino acids to about 80 amino acids, about 8 amino acids to about 75 amino acids, about 8 amino acids to about 70 amino acids, about 8 amino acids to about 65 amino acids, about 8 amino acids to about 60 amino acids, about 8 amino acids to about 55 amino acids, about 8 amino acids to about 50 amino acids, about 8 amino acids to about 45 amino acids, about 8 amino acids to about 40 amino acids, about 8 amino acids to about 35 amino acids, about 8 amino acids to about 30 amino acids, about 8 amino acids to about 25 amino acids, about 8 amino acids to about 20 amino acids, about 8 amino acids to about 15 amino acids, about 8 amino acids to about 10 amino acids, about 10 amino acids to about 100 amino acids, about 10 amino acid to about 95 amino acids, about 10 amino acids to about 90 amino acids, about 10 amino acids to about 85 amino acids, about 10 amino acids to about 80 amino acids, about 10 amino acids to about 75 amino acids, about 10 amino acids to about 70 amino acids, about 10 amino acids to about 65 amino acids, about 10 amino acids to about 60 amino acids, about 10 amino acids to about 55 amino acids, about 10 amino acids to about 50 amino acids, about 10 amino acids to about 45 amino acids, about 10 amino acids to about 40 amino acids, about 10 amino acids to about 35 amino acids, about 10 amino acids to about 30 amino acids, about 10 amino acids to about 25 amino acids, about 10 amino acids to about 20 amino acids, about 10 amino acids to about 15 amino acids, about 20 amino acids to about 100 amino acids, about 20 amino acid to about 95 amino acids, about 20 amino acids to about 90 amino acids, about 20 amino acids to about 85 amino acids, about 20 amino acids to about 80 amino acids, about 20 amino acids to about 75 amino acids, about 20 amino acids to about 70 amino acids, about 20 amino acids to about 65 amino acids, about 20 amino acids to about 60 amino acids, about 20 amino acids to about 55 amino acids, about 20 amino acids to about 50 amino acids, about 20 amino acids to about 45 amino acids, about 20 amino acids to about 40 amino acids, about 20 amino acids to about 35 amino acids, about 20 amino acids to about 30 amino acids, about 20 amino acids to about 25 amino acids, about 30 amino acids to about 100 amino acids, about 30 amino acid to about 95 amino acids, about 30 amino acids to about 90 amino acids, about 30 amino acids to about 85 amino acids, about 30 amino acids to about 80 amino acids, about 30 amino acids to about 75 amino acids, about 30 amino acids to about 70 amino acids, about 30 amino acids to about 65 amino acids, about 30 amino acids to about 60 amino acids, about 30 amino acids to about 55 amino acids, about 30 amino acids to about 50 amino acids, about 30 amino acids to about 45 amino acids, about 30 amino acids to about 40 amino acids, about 30 amino acids to about 35 amino acids, about 40 amino acids to about 100 amino acids, about 40 amino acid to about 95 amino acids, about 40 amino acids to about 90 amino acids, about 40 amino acids to about 85 amino acids, about 40 amino acids to about 80 amino acids, about 40 amino acids to about 75 amino acids, about 40 amino acids to about 70 amino acids, about 40 amino acids to about 65 amino acids, about 40 amino acids to about 60 amino acids, about 40 amino acids to about 55 amino acids, about 40 amino acids to about 50 amino acids, about 40 amino acids to about 45 amino acids, about 50 amino acids to about 100 amino acids, about 50 amino acid to about 95 amino acids, about 50 amino acids to about 90 amino acids, about 50 amino acids to about 85 amino acids, about 50 amino acids to about 80 amino acids, about 50 amino acids to about 75 amino acids, about 50 amino acids to about 70 amino acids, about 50 amino acids to about 65 amino acids, about 50 amino acids to about 60 amino acids, about 50 amino acids to about 55 amino acids, about 60 amino acids to about 100 amino acids, about 60 amino acid to about 95 amino acids, about 60 amino acids to about 90 amino acids, about 60 amino acids to about 85 amino acids, about 60 amino acids to about 80 amino acids, about 60 amino acids to about 75 amino acids, about 60 amino acids to about 70 amino acids, about 60 amino acids to about 65 amino acids, about 70 amino acids to about 100 amino acids, about 70 amino acid to about 95 amino acids, about 70 amino acids to about 90 amino acids, about 70 amino acids to about 85 amino acids, about 70 amino acids to about 80 amino acids, about 70 amino acids to about 75 amino acids, about 80 amino acids to about 100 amino acids, about 80 amino acid to about 95 amino acids, about 80 amino acids to about 90 amino acids, about 80 amino acids to about 85 amino acids, about 90 amino acids to about 100 amino acids, about 90 amino acids to about 95 amino acids, or about 95 amino acids to about 100 amino acids, removed from its N-terminus and/or 1 amino acid to 100 amino acids (or any of the subranges of this range described herein) removed from its C-terminus.

In some embodiments, an active target protein (e.g., a supporting cell target protein or a hair cell target protein) can, e.g., include the sequence of a wildtype, full-length target protein (e.g., a wildtype, full-length supporting cell target protein or a wildtype, full-length hair cell target protein) where 1 amino acid to 50 amino acids, 1 amino acid to 45 amino acids, 1 amino acid to 40 amino acids, 1 amino acid to 35 amino acids, 1 amino acid to 30 amino acids, 1 amino acid to 25 amino acids, 1 amino acid to 20 amino acids, 1 amino acid to 15 amino acids, 1 amino acid to 10 amino acids, 1 amino acid to 9 amino acids, 1 amino acid to 8 amino acids, 1 amino acid to 7 amino acids, 1 amino acid to 6 amino acids, 1 amino acid to 5 amino acids, 1 amino acid to 4 amino acids, 1 amino acid to 3 amino acids, about 2 amino acids to 50 amino acids, about 2 amino acids to 45 amino acids, about 2 amino acids to 40 amino acids, about 2 amino acids to 35 amino acids, about 2 amino acids to 30 amino acids, about 2 amino acids to 25 amino acids, about 2 amino acids to 20 amino acids, about 2 amino acids to 15 amino acids, about 2 amino acids to 10 amino acids, about 2 amino acids to 9 amino acids, about 2 amino acids to 8 amino acids, about 2 amino acids to 7 amino acids, about 2 amino acids to 6 amino acids, about 2 amino acids to 5 amino acids, about 2 amino acids to 4 amino acids, about 3 amino acids to 50 amino acids, about 3 amino acids to 45 amino acids, about 3 amino acids to 40 amino acids, about 3 amino acids to 35 amino acids, about 3 amino acids to 30 amino acids, about 3 amino acids to 25 amino acids, about 3 amino acids to 20 amino acids, about 3 amino acids to 15 amino acids, about 3 amino acids to 10 amino acids, about 3 amino acids to 9 amino acids, about 3 amino acids to 8 amino acids, about 3 amino acids to 7 amino acids, about 3 amino acids to 6 amino acids, about 3 amino acids to 5 amino acids, about 4 amino acids to 50 amino acids, about 4 amino acids to 45 amino acids, about 4 amino acids to 40 amino acids, about 4 amino acids to 35 amino acids, about 4 amino acids to 30 amino acids, about 4 amino acids to 25 amino acids, about 4 amino acids to 20 amino acids, about 4 amino acids to 15 amino acids, about 4 amino acids to 10 amino acids, about 4 amino acids to 9 amino acids, about 4 amino acids to 8 amino acids, about 4 amino acids to 7 amino acids, about 4 amino acids to 6 amino acids, about 5 amino acids to 50 amino acids, about 5 amino acids to 45 amino acids, about 5 amino acids to 40 amino acids, about 5 amino acids to 35 amino acids, about 5 amino acids to 30 amino acids, about 5 amino acids to 25 amino acids, about 5 amino acids to 20 amino acids, about 5 amino acids to 15 amino acids, about 5 amino acids to 10 amino acids, about 5 amino acids to 9 amino acids, about 5 amino acids to 8 amino acids, about 5 amino acids to 7 amino acids, about 6 amino acids to 50 amino acids, about 6 amino acids to 45 amino acids, about 6 amino acids to 40 amino acids, about 6 amino acids to 35 amino acids, about 6 amino acids to 30 amino acids, about 6 amino acids to 25 amino acids, about 6 amino acids to 20 amino acids, about 6 amino acids to 15 amino acids, about 6 amino acids to 10 amino acids, about 6 amino acids to 9 amino acids, about 6 amino acids to 8 amino acids, about 7 amino acids to 50 amino acids, about 7 amino acids to 45 amino acids, about 7 amino acids to 40 amino acids, about 7 amino acids to 35 amino acids, about 7 amino acids to 30 amino acids, about 7 amino acids to 25 amino acids, about 7 amino acids to 20 amino acids, about 7 amino acids to 15 amino acids, about 7 amino acids to 10 amino acids, about 7 amino acids to 9 amino acids, about 8 amino acids to 50 amino acids, about 8 amino acids to 45 amino acids, about 8 amino acids to 40 amino acids, about 8 amino acids to 35 amino acids, about 8 amino acids to 30 amino acids, about 8 amino acids to 25 amino acids, about 8 amino acids to 20 amino acids, about 8 amino acids to 15 amino acids, about 8 amino acids to 10 amino acids, about 10 amino acids to 50 amino acids, about 10 amino acids to 45 amino acids, about 10 amino acids to 40 amino acids, about 10 amino acids to 35 amino acids, about 10 amino acids to 30 amino acids, about 10 amino acids to 25 amino acids, about 10 amino acids to 20 amino acids, about 10 amino acids to 15 amino acids, about 15 amino acids to 50 amino acids, about 15 amino acids to 45 amino acids, about 15 amino acids to 40 amino acids, about 15 amino acids to 35 amino acids, about 15 amino acids to 30 amino acids, about 15 amino acids to 25 amino acids, about 15 amino acids to 20 amino acids, about 20 amino acids to 50 amino acids, about 20 amino acids to 45 amino acids, about 20 amino acids to 40 amino acids, about 20 amino acids to 35 amino acids, about 20 amino acids to 30 amino acids, about 20 amino acids to 25 amino acids, about 25 amino acids to 50 amino acids, about 25 amino acids to 45 amino acids, about 25 amino acids to 40 amino acids, about 25 amino acids to 35 amino acids, about 25 amino acids to 30 amino acids, about 30 amino acids to 50 amino acids, about 30 amino acids to 45 amino acids, about 30 amino acids to 40 amino acids, about 30 amino acids to 35 amino acids, about 35 amino acids to 50 amino acids, about 35 amino acids to 45 amino acids, about 35 amino acids to 40 amino acids, about 40 amino acids to 50 amino acids, about 40 amino acids to 45 amino acids, or about 45 amino acids to about 50 amino acids, are inserted. In some examples, the 1 amino acid to 50 amino acids (or any subrange thereof) can be inserted as a contiguous sequence into the sequence of a wildtype, full-length protein. In some examples, the 1 amino acid to 50 amino acids (or any subrange thereof) are not inserted as a contiguous sequence into the sequence of a wildtype, full-length protein. As can be appreciated in the art, the 1 amino acid to 50 amino acids can be inserted into a portion of the sequence of a wildtype, full-length protein that is not well-conserved between species.

Unless otherwise defined, all technical and scientific terms used herein have the same meaning as commonly understood by one of ordinary skill in the art to which this invention belongs. Methods and materials are described herein for use in the present invention; other suitable methods and materials known in the art can also be used. The materials, methods, and examples are illustrative only and not intended to be limiting. All publications, patent applications, patents, sequences, database entries, and other references mentioned herein are incorporated by reference in their entirety. In case of conflict, the present specification, including definitions, will control.

### BRIEF DESCRIPTION OF DRAWINGS

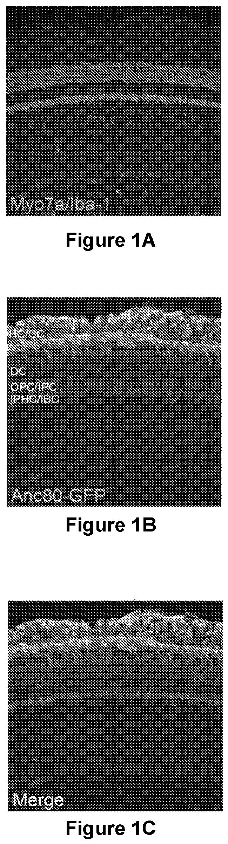

Figure 1A is a representative image of Myo7a/Iba-1 immunofluorescent staining of cochlear tissue of a cynomolgus macaque (non-human primate) following administration of a single Anc80-GFP AAV vector directly into the inner ear through the round window.
Figure 1B is a representative image of Anc80-GFP immunofluorescent staining of the same cochlear tissue of the cynomolgus macaque as in Figure 1A.
Figure 1C is a representative image of a merged immunofluorescent staining of Myo7a/Iba-1 and Anc80-GFP of the same cochlear tissue of the cynomolgus macaque as in Figure 1A.

### DETAILED DESCRIPTION

Provided herein are compositions that include at least two different nucleic acid vectors, where: each of the at least two different adeno-associated virus (AAV) vectors includes a coding sequence that encodes a different portion of a supporting cell target protein, each of the encoded portions being at least 30 amino acid residues in length, where the amino acid sequence of each of the encoded portions may optionally partially overlap with the amino acid sequence of a different one of the encoded portions; no single vector of the at least two different vectors encodes the full-length supporting cell target protein; at least one of the coding sequences includes a nucleotide sequence spanning two neighboring exons of the supporting cell target genomic DNA, and lacks an intronic sequence between the two neighboring exons; and when introduced into a primate cell the at least two different vectors undergo concatamerization or homologous recombination with each other, thereby forming a recombined nucleic acid that encodes a full-length supporting cell target protein.

Provided herein are compositions including two different nucleic acid vectors, where: a first nucleic acid vector of the two different nucleic acid vectors includes a promoter, a first coding sequence that encodes an N-terminal portion of a supporting cell target protein positioned 3' of the promoter, and a splicing donor signal sequence positioned at the 3' end of the first coding sequence; and a second nucleic acid vector of the two different nucleic acid vectors includes a splicing acceptor signal sequence, a second coding sequence that encodes a C-terminal portion of a supporting cell target protein positioned at the 3' end of the splicing acceptor signal sequence, and a polyadenylation sequence at the 3' end of the second coding sequence; where each of the encoded portions is at least 30 amino acid residues in length, where the amino acid sequences of the encoded portions do not overlap, where no single vector of the two different vectors encodes the full-length supporting cell target protein, and, when the coding sequences are transcribed in a primate cell, to produce RNA transcripts, splicing occurs between the splicing donor signal sequence on one transcript and the splicing acceptor signal sequence on the other transcript, thereby forming a recombined RNA molecule that encodes a full-length supporting cell target protein.

Provided herein are compositions including: a first nucleic acid vector including a promoter, a first coding sequence that encodes an N-terminal portion of a supporting cell target protein positioned 3' of the promoter, a splicing donor signal sequence positioned at the 3' end of the first coding sequence, and a first detectable marker gene positioned 3' of the splicing donor signal sequence; and a second nucleic acid vector, different from the first nucleic acid vector, including a second detectable marker gene, a splicing acceptor signal sequence positioned 3' of the second detectable marker gene, a second coding sequence that encodes a C-terminal portion of a supporting cell target protein positioned at the 3' end of the splicing acceptor signal sequence, and a polyadenylation sequence positioned at the 3' end of the second coding sequence; where each of the encoded portions is at least 30 amino acid residues in length, where the respective amino acid sequences of the encoded portions do not overlap with each other, where no single vector of the two different vectors encodes the full-length supporting cell target protein, and, when the coding sequences are transcribed in a primate cell to produce RNA transcripts, splicing occurs between the splicing donor signal on one transcript and the splicing acceptor signal on the other transcript, thereby forming a recombined RNA molecule that encodes a full-length supporting cell target protein.

Provided herein are compositions including: a first nucleic acid vector including a promoter, a first coding sequence that encodes an N-terminal portion of a supporting cell target protein positioned 3' to the promoter, a splicing donor signal sequence positioned at the 3' end of the first coding sequence, and a F1 phage recombinogenic region positioned 3' to the splicing donor signal sequence; and a second nucleic acid vector, different from the first nucleic acid vector, including a second F1 phage recombinogenic region, a splicing acceptor signal sequence positioned 3' of the second F1 phage recombinogenic region, a second coding sequence that encodes a C-terminal portion of a supporting cell target protein positioned at the 3' end of the splicing acceptor signal sequence, and a polyadenylation sequence positioned at the 3' end of the second coding sequence; where each of the encoded portions is at least 30 amino acid residues in length, where the respective amino acid sequences of the encoded portions do not overlap with each other, where no single vector of the two different vectors encodes the full-length supporting cell target protein, and, when the coding sequences are transcribed in a primate cell to produce RNA transcripts, splicing occurs between the splicing donor signal one transcript and the splicing acceptor signal on the other transcript, thereby forming a recombined RNA molecule that encodes a full-length supporting cell target protein.

Provided herein are compositions including a single adeno-associated virus (AAV) vector, where the single AAV vector that includes a nucleic acid sequence that encodes a supporting cell target protein; and when introduced into a primate cell, a nucleic acid encoding a full-length supporting cell target protein is generated at the locus of the supporting cell target gene, and the primate expresses the supporting cell target protein.

Provided herein are compositions that include at least two different nucleic acid vectors, where: each of the at least two different adeno-associated virus (AAV) vectors includes a coding sequence that encodes a different portion of a hair cell target protein, each of the encoded portions being at least 30 amino acid residues in length, where the amino acid sequence of each of the encoded portions may optionally partially overlap with the amino acid sequence of a different one of the encoded portions; no single vector of the at least two different vectors encodes the full-length hair cell target protein; at least one of the coding sequences includes a nucleotide sequence spanning two neighboring exons of the hair cell target genomic DNA, and lacks an intronic sequence between the two neighboring exons; and when introduced into a primate cell the at least two different vectors undergo concatamerization or homologous recombination with each other, thereby forming a recombined nucleic acid that encodes a full-length hair cell target protein.

Provided herein are compositions including two different nucleic acid vectors, where: a first nucleic acid vector of the two different nucleic acid vectors includes a promoter, a first coding sequence that encodes an N-terminal portion of a hair cell target protein positioned 3' of the promoter, and a splicing donor signal sequence positioned at the 3' end of the first coding sequence; and a second nucleic acid vector of the two different nucleic acid vectors includes a splicing acceptor signal sequence, a second coding sequence that encodes a C-terminal portion of a hair cell target protein positioned at the 3' end of the splicing acceptor signal sequence, and a polyadenylation sequence at the 3' end of the second coding sequence; where each of the encoded portions is at least 30 amino acid residues in length, where the amino acid sequences of the encoded portions do not overlap, where no single vector of the two different vectors encodes the full-length hair cell target protein, and, when the coding sequences are transcribed in a primate cell, to produce RNA transcripts, splicing occurs between the splicing donor signal sequence on one transcript and the splicing acceptor signal sequence on the other transcript, thereby forming a recombined RNA molecule that encodes a full-length hair cell target protein.

Provided herein are compositions including: a first nucleic acid vector including a promoter, a first coding sequence that encodes an N-terminal portion of a hair cell target protein positioned 3' of the promoter, a splicing donor signal sequence positioned at the 3' end of the first coding sequence, and a first detectable marker gene positioned 3' of the splicing donor signal sequence; and a second nucleic acid vector, different from the first nucleic acid vector, including a second detectable marker gene, a splicing acceptor signal sequence positioned 3' of the second detectable marker gene, a second coding sequence that encodes a C-terminal portion of a hair cell target protein positioned at the 3' end of the splicing acceptor signal sequence, and a polyadenylation sequence positioned at the 3' end of the second coding sequence; where each of the encoded portions is at least 30 amino acid residues in length, where the respective amino acid sequences of the encoded portions do not overlap with each other, where no single vector of the two different vectors encodes the full-length hair cell target protein, and, when the coding sequences are transcribed in a primate cell to produce RNA transcripts, splicing occurs between the splicing donor signal on one transcript and the splicing acceptor signal on the other transcript, thereby forming a recombined RNA molecule that encodes a full-length hair cell target protein.

Provided herein are compositions including: a first nucleic acid vector including a promoter, a first coding sequence that encodes an N-terminal portion of a hair cell target protein positioned 3' to the promoter, a splicing donor signal sequence positioned at the 3' end of the first coding sequence, and a F1 phage recombinogenic region positioned 3' to the splicing donor signal sequence; and a second nucleic acid vector, different from the first nucleic acid vector, including a second F1 phage recombinogenic region, a splicing acceptor signal sequence positioned 3' of the second F1 phage recombinogenic region, a second coding sequence that encodes a C-terminal portion of a hair cell target protein positioned at the 3' end of the splicing acceptor signal sequence, and a polyadenylation sequence positioned at the 3' end of the second coding sequence; where each of the encoded portions is at least 30 amino acid residues in length, where the respective amino acid sequences of the encoded portions do not overlap with each other, where no single vector of the two different vectors encodes the full-length hair cell target protein, and, when the coding sequences are transcribed in a primate cell to produce RNA transcripts, splicing occurs between the splicing donor signal one transcript and the splicing acceptor signal on the other transcript, thereby forming a recombined RNA molecule that encodes a full-length hair cell target protein.

Also provided herein are compositions including a single adeno-associated virus (AAV) vector, where the single AAV vector that includes a nucleic acid sequence that encodes a hair cell target protein; and when introduced into a primate cell, a nucleic acid encoding a full-length hair cell target protein is generated at the locus of the hair cell target gene, and the primate expresses the hair cell target protein.

Also provided are kits that include any of the compositions described herein. Also provided herein are methods that include introducing into a cochlea of a mammal a therapeutically effective amount of any of the compositions described herein.

Also provided are methods of correcting a supporting cell target gene defect in a supporting cell of an inner ear in a primate that include: administering to the inner ear of the primate a therapeutically effective amount of any of the compositions described herein, where the administering repairs the supporting cell target gene defect in the supporting cell of the inner ear of the primate.

Also provided are methods of correcting a hair cell target gene defect in a hair cell of an inner ear in a primate that include: administering to the inner ear of the primate a therapeutically effective amount of any of the compositions described herein, where the administering repairs the hair cell target gene defect in the hair cell of the inner ear of the primate.

Also provided herein are methods of increasing the expression level of a supporting cell target protein in a supporting cell of an inner ear of a primate that include: administering to the inner ear of the primate a therapeutically effective amount of any of the compositions described herein, where the administering results in an increase in the expression level of the supporting cell target protein in the supporting cell of the inner ear of the primate.

Also provided herein are methods of increasing the expression level of a hair cell target protein in a hair cell of an inner ear of a primate that include: administering to the inner ear of the primate a therapeutically effective amount of any of the compositions described herein, where the administering results in an increase in the expression level of the hair cell target protein in the hair cell of the inner ear of the primate.

Also provided herein are methods of treating hearing loss, e.g., non-syndromic sensorineural hearing loss or syndromic sensorineural hearing loss, in a primate identified as having a defective supporting cell target gene that include: administering to the inner ear of the primate a therapeutically effective amount of any of the compositions described herein.

Also provided herein are methods of treating hearing loss, e.g., non-syndromic sensorineural hearing loss or syndromic sensorineural hearing loss, in a primate identified as having a defective hair cell target gene that include: administering to the inner ear of the primate a therapeutically effective amount of any of the compositions described herein.

Also provided herein are methods of restoring hearing in a primate identified or diagnosed as having hearing loss that include: administering to the inner ear of the primate a therapeutically effective amount of any of the compositions described herein.

Also provided herein are methods of restoring synapses and/or preserving spiral ganglion nerves in a primate identified or diagnosed as having an inner ear disorder that include: administering to the inner ear of the primate a therapeutically effective amount of any of the compositions described herein.

Also provided herein are methods that include administering to an inner ear of a primate a therapeutically effective amount of any of the compositions described herein.

Additional non-limiting aspects of the compositions, kits, and methods are described herein and can be used in any combination without limitation.

### Gap junction protein beta 2 (GJB2)

The GJB2 gene encodes gap junction protein beta 2, a member of the gap junction protein family. Gap junction proteins are specialized proteins, also called connexins, involved in intracellular communication. Mutations in GJB2 have been associated with hearing loss and deafness (Amorini et al., Ann. Hum. Genet. 79(5):341-349, 2015; Qing et al., Genet. Test Mol. Biomarkers 19(1):52-58, 2015).

The human GJB2 gene is located on chromosome 13q12. It contains 2 exons encompassing ~5 kilobases (kb) (NCBI Accession No. NG008358.1). The full-length wildtype GJB2 protein expressed from the human GJB2 gene is 226 amino acids in length.

Various mutations in the GJB2 gene have been associated with hearing loss (e.g., non-syndromic sensorineural hearing loss or syndromic sensorineural hearing loss). For example, the c.35delG allele was found on 65.5% of patients from Eastern Sicily (Amorini et al., Ann. Hum. Genet. 79(5):341-349, 2015).

Additional exemplary mutations in a GJB2 gene detected in subjects having non-syndromic sensorineural hearing loss or syndromic sensorineural hearing loss, and methods of sequencing a nucleic acid encoding GJB2 are described in, e.g., Snoeckx et al., Am. J. Hum. Genet 77: 945-957, 2005; Welch et al., Am. J. Med. Genet A 143: 1567-1573, 2007; and Zelante et al., Hum. Mol. Genet. 6:1605-1609, 1997. Methods of detecting mutations in a gene are well-known in the art. Non-limiting examples of such techniques include: real-time polymerase chain reaction (RT-PCR), PCR, sequencing, Southern blotting, and Northern blotting.

An exemplary human wildtype GJB2 protein is or includes the sequence of SEQ ID NO: 1. Non-limiting examples of nucleic acid encoding a wildtype GJB2 protein are or include SEQ ID NO: 2. As can be appreciated in the art, at least some or all of the codons in SEQ ID NO: 2 can be codon-optimized to allow for optimal expression in a non-human mammal.

In some embodiments of any of the compositions described herein, the GJB2 protein comprises a sequence that is at least 75% (e.g., at least 80%, at least 85%, at least 90%, at least 91%, at least 92%, at least 93%, at least 94%, at least 95%, at least 96%, at least 97%, at least 98%, at least 99%) identical to SEQ ID NO: 1.
**Human Full-length Wildtype GJB2 Protein (SEQ ID NO: 1)**
**Human Wildtype GJB2 cDNA (SEQ ID NO: 2)**

atggattggggcacgctgcagacgatcctgggggtgtgaacaaacactccaccagcattggaaagatctg
gctcaccgtcctcttcattttcgcattatgatcctcgttgtggctgcaaaggaggtgtggggagatgagc
aggccgactttgtctgcaacaccctgcagccaggctgcaagaacgtgtgctacgatcactacttccccatc
tcccacatccggctatgggccctgcagctgatcttcgtgtccacgccagcgctcctagtggccatgcacgt
ggcctaccggagacatgagaagagaggaagttcatcaaggggagataaagagtgaatttaaggacatcg
aggagatcaaaacccagaaggtccgcatcgaaggctccctgtggtggacctacacaagcagcatcttcttc
cgggtcatcttcgaagccgccttcatgtacgtcttctatgtcatgtacgacggcttctccatgcagcggct
ggtgaagtgcaacgcctggccttgtcccaacactgtggactgctttgtgtcccggcccacggagaagactg
tcttcacagtgttcatgattgcagtgtctggaatttgcatcctgctgaatgtcactgaattgtgttatttg
ctaattagatattgttctgggaagtcaaaaaagccagtttaa

A non-limiting example of a human wildtype GJB2 genomic DNA sequence is SEQ ID NO: 3. The exons in SEQ ID NO: 3 are: nucleotide positions 1-156 (exon 1), and nucleotide positions 3336-5469 (exon 2). The intron is located between each of these exons in SEQ ID NO: 3, i.e., at nucleotide positions 157-3335 (intron 1).

**Human Wildtype GJB2 Gene Sequence (SEQ ID NO: 3)** (NCBI Reference Sequence: NG_008358.1)

1 ggggtgcggt taaaaggcgc cacggcggga gacaggtgtt gcggccccgc agcgcccgcg
61 cgctcctctc cccgactcgg agcccctcgg cggcgcccgg cccaggaccc gcctaggagc
121 gcaggagccc cagcgcagag accccaacgc cgagaccccc gccccggccc cgccgcgctt
181 cctcccgacg caggtgagcc cgccggcccc ggactgcccg gccaggaacc tggcgcgggg
241 agggaccgcg agacccagag cggttgcccg gccgcgtggg tctcggggaa ccgggggct
301 ggaccaacac acgtccttgg gccggggggc gggggccgcc ttctggagcg ggcgtttctg
361 cggccgagct ccggagctgg aatggggcgg ccggggaagt ggacgcgatg gcaccgcccg
421 gggtgcgagt ggggccgggc gcgcgcggga ggggaaaaag gcgcgggcga gccgccagcg
481 cgaggtttgt ggtgtcgccg atgtcccttc ggggtactct agcgcagccg cctggctact
541 tgacccactg ccaccaaacg ttttaaattc accgaaagct tagcttcgaa gcaaagctcc
601 gtttcgccgg tgaagcagga agccttcgct gcaggaactg acctttacct cttggagcgg

661 cttctgcaga aaaatccccg ggcagagatt tgggcggagt ttgcctagaa ctaacgcgga
721 gccagccgat cccggcctac cccggggcca agatttcagt ggcttccctt tttcctaaac
781 acttcacgag ggtctgtttc cgggctgtgc tccccgccta gaaggaaaat ttttaggacc
841 cttgttcgcg aagaggtggt gtgcggctga gacccgcgtc ctcaggacgg ttccatcagt
901 gcctcgatcc tgccccactg gaggaggaag gcagcccgaa cagcgctcac ctaactaaca
961 gctgctgaga gctgggttcc gtggccatgc acctgggact gccttgagaa gcgtggtacg
1021 gccgtgtccc catgtgacct tagagtccct ttcgaaactg ctgtgcacag tcggtcacaa
1081 tttcagacac tggtgagaag ggtggaggaa ccctctgggg acagccaggc aaggtcgacc
1141 acccatcacc taagggtgga gaaatttaag gggtgaagag tcccttttgc cttttctgga
1201 tcctggtgat tcacctagtg tcttccctaa ggaactgaac caactcctcc gctggcctct
1261 ggcagccctc caggcggtgc aggatggcgt gggcccggta ggaagctgca tgtaaccgcc
1321 cagggtcggg aggccaggag ggcagctcct cctctgactt gaatattgaa aacaacttcg
1381 tcctgcttct gagcccctct taacccatga ccccctagcc cattggggag taaatcttaa
1441 tttactcctc ttcctgaaaa aggatcttta aaacaggtag cttcaactca agctttataa
1501 aataacaata tagggtttct cggaactgta tttttctcag ctgatggtaa ctggacaggt
1561 ctgtagaagg gtgtatgacc tgggtttggc aggtggaaga gggcaaagga taaacccctc
1621 ctcctgcagc cccatattct tggccaggtg tattgttgta aaccaggaga gagtttactt
1681 cggggagtat cctgttttcc actcagtgag ggccaatgaa gaatgtctaa ttccataaga
1741 tgcttttgtt aaaatcggaa tgttgctgtc ctcggtggtt ctgctgttgg gacgggactg
1801 gcctgagctg tgggtgctgt agcaggacaa ccagctcacc taagggcctc ccagtctgga
1861 ttatcaatgg gtcagtgctg aacctgggct aaaatattgt tttttccaat gatgttgtct
1921 ttcccaagct cagtgaagct aaatgtttca caggcctatg tcaatctgat gtaactttcg
1981 tggccacctc tctcctgtta gcctctgacc aaggtggcac tggatggttt ctgcctgacc
2041 ttggtgcccc gtggcagcga ctgtgggtca tgaaagacat tcactacgag cctgcttctg
2101 gagtccatca gaaaacggga tgcaacttgc ctaaaatgag gagaggagga tgcttttaag
2161 aaaaagaaga aggaggattc actaccagct ctgaagggtg gaaaagagat gattcatccg
2221 gattgtggag agggtggaat cttgtttagg agagcgttgg ttgtggcagg cagggtgtaa
2281 ctatgaatca gtgaagacaa ttcacatcct gggatgaaaa gaaggccatg ggctcacagg
2341 agattatcca ctggcctctc cacatccgct tgcagtaagg agtgtgggac tctcccaagc
2401 ttcagcgctg aactgcaatg cagtgacgtc gcttagctgg gccagtaacc gagggagttg
2461 aattttctgt cattttaaaa taatgtgtct tttaagaaac actttgaaat taaaaccaca
2521 gcccacaatt ataatgcact gttgcagcac ttatcaaaac agatatgcta actgagccat
2581 cagtgccagc ctgacagtga ggccaccaag ccatccacaa agcctacacg aaagtctgtg
2641 ctcacagtgg cttttctcca tgaagagggc attcctaacc tcttcctttc acgtaggagg
2701 aagcaaggtc ctttgtaaaa ttttaactcg gggtgcctca aatgtaaact taaccactgg
2761 taacaacagt ttcactgcta catgccacgt ctgtgaaaat tcattcaaga cattaaggaa
2821 agtggctcag cagagagact agacatctta tcctcacggt tctcctgtac ttggcctctc
2881 agcctttgag caaggttggc ccaagctagt atcggcccca gtggtacagc caaaacttga

2941 gactgcaaat ggatgcagct gttgaacgct gagtaacttc tgcagagtca ggaagaccca
3001 aggaagctct gcagaggatg caggggtacg gtcagaaccc ctgagtgcct ttcagctaac
3061 gaggacttta tgacactccc cagcacagca aatttttatg atgtgtttaa agattgggtg
3121 aattactcag gtgaacaagc tactttttat cagagaacac ctaaaaacac gttcaagagg
3181 gtttgggaac tatacattta atcctatgac aaactaagtt ggttctgtct tcacctgttt
3241 tggtgaggtt gtgtaagagt tggtgtttgc tcaggaagag atttaagcat gcttgcttac
3301 ccagactcag agaagtctcc ctgttctgtc ctagctagtg attcctgtgt tgtgtgcatt
3361 cgtcttttcc agagcaaacc gcccagagta gaagatggat tggggcacgc tgcagacgat
3421 cctggggggt gtgaacaaac actccaccag cattggaaag atctggctca ccgtcctctt
3481 catttttcgc attatgatcc tcgttgtggc tgcaaaggag gtgtggggag atgagcaggc
3541 cgactttgtc tgcaacaccc tgcagccagg ctgcaagaac gtgtgctacg atcactactt
3601 ccccatctcc cacatccggc tatgggccct gcagctgatc ttcgtgtcca cgccagcgct
3661 cctagtggcc atgcacgtgg cctaccggag acatgagaag aagaggaagt tcatcaaggg
3721 ggagataaag agtgaattta aggacatcga ggagatcaaa acccagaagg tccgcatcga
3781 aggctccctg tggtggacct acacaagcag catcttcttc cgggtcatct tcgaagccgc
3841 cttcatgtac gtcttctatg tcatgtacga cggcttctcc atgcagcggc tggtgaagtg
3901 caacgcctgg ccttgtccca acactgtgga ctgctttgtg tcccggccca cggagaagac
3961 tgtcttcaca gtgttcatga ttgcagtgtc tggaatttgc atcctgctga atgtcactga
4021 attgtgttat ttgctaatta gatattgttc tgggaagtca aaaaagccag tttaacgcat
4081 tgcccagttg ttagattaag aaatagacag catgagaggg atgaggcaac ccgtgctcag
4141 ctgtcaaggc tcagtcgcta gcatttccca acacaaagat tctgacctta aatgcaacca
4201 tttgaaaccc ctgtaggcct caggtgaaac tccagatgcc acaatggagc tctgctcccc
4261 taaagcctca aaacaaaggc ctaattctat gcctgtctta attttctttc acttaagtta
4321 gttccactga gaccccaggc tgttaggggt tattggtgta aggtactttc atattttaaa
4381 cagaggatat cggcatttgt ttctttctct gaggacaaga gaaaaaagcc aggttccaca
4441 gaggacacag agaaggtttg ggtgtcctcc tggggttctt tttgccaact ttccccacgt
4501 taaaggtgaa cattggttct ttcatttgct ttggaagttt taatctctaa cagtggacaa
4561 agttaccagt gccttaaact ctgttacact ttttggaagt gaaaactttg tagtatgata
4621 ggttattttg atgtaaagat gttctggata ccattatatg ttccccctgt ttcagaggct
4681 cagattgtaa tatgtaaatg gtatgtcatt cgctactatg atttaatttg aaatatggtc
4741 ttttggttat gaatactttg cagcacagct gagaggctgt ctgttgtatt cattgtggtc
4801 atagcaccta acaacattgt agcctcaatc gagtgagaca gactagaagt tcctagtgat
4861 ggcttatgat agcaaatggc ctcatgtcaa atatttagat gtaattttgt gtaagaaata
4921 cagactggat gtaccaccaa ctactacctg taatgacagg cctgtccaac acatctccct
4981 tttccatgac tgtggtagcc agcatcggaa agaacgctga tttaaagagg tcgcttggga
5041 attttattga cacagtacca tttaatgggg aggacaaaat ggggcagggg agggagaagt
5101 ttctgtcgtt aaaaacagat ttggaaagac tggactctaa agtctgttga ttaaagatga
5161 gctttgtcta cttcaaaagt ttgtttgctt accccttcag cctccaattt tttaagtgaa

5221 aatatagcta ataacatgtg aaaagaatag aagctaaggt ttagataaat attgagcaga

5281 tctataggaa gattgaacct gaatattgcc attatgcttg acatggtttc caaaaaatgg

5341 tactccacat atttcagtga gggtaagtat tttcctgttg tcaagaatag cattgtaaaa

5401 gcattttgta ataataaaga atagctttaa tgatatgctt gtaactaaaa taattttgta

5461 atgtatcaaa tacatttaaa acattaaaat ataatctcta taataattta aaa

### Solute carrier family 26 member 4 (SLC26A4)

The SLC26A4 gene encodes the Pendred protein, and has homology to sulfate transporters. Mutations in SLC26A4 have been associated with hearing loss and deafness (Albert et al., Eur. J. Hum. Genet. 14:773-779, 2006; and Qing et al., Genet. Test Mol. Biomarkers 19(1):52-58, 2015).

The human SLC26A4 gene is located on chromosome 7q22. It contains 21 exons encompassing -57 kilobases (kb) (NCBI Accession No. NG_008489.1). The full-length wildtype SLC26A4 protein expressed from the human SLC26A4 gene is 780 amino acids in length.

Various mutations in the SLC26A4 gene have been associated with hearing loss. For example, point mutations V250A, D266N, E303Q, F345S, D697, K715N, and E737D have been reported in Chinese and U.S. patients with non-syndromic hearing loss (Dai et al., Physiol Genomics 38(3): 281-290, 2015).

Additional exemplary mutations in a SLC26A4 gene detected in subjects having non-syndromic sensorineural hearing loss or syndromic sensorineural hearing loss, and methods of sequencing a nucleic acid encoding SLC26A4 are described in, e.g., Albert et al., Eur. J. Hum. Genet. 14: 773-779, 2006; and Qing et al., Genet. Test Mol. Biomarkers 19(1):52-58, 2015. Methods of detecting mutations in a gene are well-known in the art. Non-limiting examples of such techniques include: real-time polymerase chain reaction (RT-PCR), PCR, sequencing, Southern blotting, and Northern blotting.

An exemplary human wildtype SLC26A4 protein is or includes the sequence of SEQ ID NO: 4. Non-limiting examples of nucleic acid encoding a wildtype SLC26A4 protein are or include SEQ ID NO: 5 or 6. As can be appreciated in the art, at least some or all of the codons in SEQ ID NO: 5 or 6 can be codon-optimized to allow for optimal expression in a non-human mammal.

In some embodiments of any of the compositions described herein, the SLC26A4 protein comprises a sequence that is at least 75% (e.g., at least 80%, at least 85%, at least 90%, at least 91%, at least 92%, at least 93%, at least 94%, at least 95%, at least 96%, at least 97%, at least 98%, at least 99%) identical to SEQ ID NO: 4.
**Human Full-length Wildtype SLC26A4 Protein (SEQ ID NO: 4)**
**Exemplary Human Wildtype SLC26A4 cDNA (SEQ ID NO: 5)** 
atggcagcgccaggcggcaggtcggagccgccgcagctccccgagtacagctgcagctacatggtgtcgcg
gccggtctacagcgagctcgctttccagcaacagcacgagcggcgcctgcaggagcgcaagacgctgcggg
agagcctggccaagtgctgcagttgttcaagaaagagagcctttggtgtgctaaagactcttgtgcccatc
ttggagtggctccccaaataccgagtcaaggaatggctgcttagtgacgtcatttcgggagttagtactgg
gctagtggccacgctgcaagggatggcatatgccctactagctgcagttcctgtcggatatggtctctact
ctgctttttccctatcctgacatactttatctttggaacatcaagacatatctcagttggacctttttcca
gtggtgagtttaatggtgggatctgttgttctgagcatggcccccgacgaacactttctcgtatccagcag
caatggaactgtattaaatactactatgatagacactgcagctagagatacagctagagtcctgattgcca
gtgccctgactctgctggttggaattatacagttgatatttggtggcttgcagattggattcatagtgagg
tacttggcagatcctttggttggtggcttcacaacagctgctgccttccaagtgctggtctcacagctaaa
gattgtcctcaatgtttcaaccaaaaactacaatggagttctctctattatctatacgctggttgagattt
ttcaaaatattggtgataccaatcttgctgatttcactgctggattgctcaccattgtcgtctgtatggca
gttaaggaattaaatgatcggtttagacacaaaatcccagtccctattcctatagaagtaattgtgacgat
aattgctactgccatttcatatggagccaacctggaaaaaaattacaatgctggcattgttaaatccatcc
caaggggggtttttgcctcctgaacttccacctgtgagcttgttctcggagatgctggctgcatcattttcc
atcgctgtggtggcttatgctattgcagtgtcagtaggaaaagtatatgccaccaagtatgattacaccat
cgatgggaaccaggaattcattgcctttgggatcagcaacatcttctcaggattcttctcttgttttgtg
 
gccaccactgctctttcccgcacggccgtccaggagagcactggaggaaagacacaggttgctggcatcat
ctctgctgcgattgtgatgatcgccattcttgccctggggaagcttctggaacccttgcagaagtcggtct
tggcagctgttgtaattgccaacctgaaaggatgtttatgcagctgtgtgacattcctcgtctgtggaga
cagaataagattgatgctgttatctgggtgtttacgtgtatagtgtccatcattctgggctggatctcgg
tttactagctggccttatatttggactgttgactgtggtcctgagagttcagtttccttcttggaatggcc
ttggaagcatccctagcacagatatctacaaaagtaccaagaattacaaaaacattgaagaacctcaagga
gtgaagattcttagattttccagtcctattttctatggcaatgtcgatggttttaaaaatgtatcaagtc
cacagttggatttgatgccattagagtatataataagaggctgaaagcgctgaggaaaatacagaaactaa
taaaaagtggacaattaagagcaacaaagaatggcatcataagtgatgctgtttcaacaaataatgctttt
gagcctgatgaggatattgaagatctggaggaacttgatatcccaaccaaggaaatagagattcaagtgga
ttggaactctgagcttccagtcaaagtgaacgttcccaaagtgccaatccatagccttgtgcttgactgt
ggagctatatctttcctggacgttgttggagtgagatcactgcgggtgattgtcaaagaattccaaagaat
tgatgtgaatgtgtattttgcatcacttcaagattatgtgatagaaaagctggagcaatgcgggttctttg
acgacaacattagaaaggacacattctttttgacggtccatgatgctatactctatctacagaaccaagtg
aaatctcaagagggtcaaggttccattttagaaacgatcactctcattcaggattgtaaagatacccttga
attaatagaaacagagctgacggaagaagaacttgatgtccaggatgaggctatgcgtacacttgcatcct
ga
**Exemplary Human Wildtype SLC26A4 cDNA (SEQ ID NO: 6)**

ctcagccttcccggttcgggaaaggggaagaatgcaggaggggtaggatttctttcctgataggatcggtt
gggaaagaccgcagcctgtgtgtgtctttcccttcgaccaaggtgtctgttgctccgtaaataaaacgtcc
cactgccttctgagagcgctataaaggcagcggaagggtagtccgcggggcattccggcggggcgcgagc
agagacaggtcatggcagcgccaggcggcaggtcggagccgccgcagctccccgagtacagctgcagctac
atggtgtcgcggccggtctacagcgagctcgctttccagcaacagcacgagcggcgcctgcaggagcgcaa
gacgctgcgggagagcctggccaagtgctgcagttgttcaagaaagagagcctttggtgtgctaaagactc
ttgtgcccatcttggagtggctccccaaataccgagtcaaggaatggctgcttagtgacgtcatttcggga
gttagtactgggctagtggccacgctgcaaggatggcatatgccctactagctgcagttcctgtcggata
tggtctctactctgctttttttccctatcctgacatactttatctttggaacatcaagacatatctcagttg
gaccttttccagtggtgagtttaatggtgggatctgttgttctgagcatggcccccgacgaacactttctc
gtatccagcagcaatggaactgtattaaatactactatgatagacactgcagctagagatacagctagagt
cctgattgccagtgccctgactctgctggttggaattatacagttgatatttggtggcttgcagattggat
tcatagtgaggtacttggcagatcctttggttggtggcttcacaacagctgctgccttccaagtgctggtc
tcacagctaaagattgtcctcaatgtttcaaccaaaaactacaatggagttctctctattatctatacgct
ggttgagattttttcaaaatattggtgataccaatcttgctgatttcactgctggattgctcaccattgtcg
tctgtatggcagttaaggaattaaatgatcggtttagacacaaaatcccagtccctattcctatagaagta
attgtgacgataattgctactgccatttcatatggagccaacctggaaaaaaattacaatgctggcattgt
taaatccatcccaaggggggtttttgcctcctgaacttccacctgtgagcttgttctcggagatgctggctg
catcattttccatcgctgtggtggcttatgctattgcagtgtcagtaggaaaagtatatgccaccaagtat

gattacaccatcgatgggaaccaggaattcattgcctttgggatcagcaacatcttctcaggattcttctc
ttgttttgtggccaccactgctctttcccgcacggccgtccaggagagcactggaggaaagacacaggttg
ctggcatcatctctgctgcgattgtgatgatcgccattcttgccctggggaagcttctggaacccttgcag
aagtcggtcttggcagctgttgtaattgccaacctgaaagggatgtttatgcagctgtgtgacattcctcg
tctgtggagacagaataagattgatgctgttatctgggtgtttacgtgtatagtgtccatcattctggggc
tggatctcggtttactagctggccttatatttggactgttgactgtggtcctgagagttcagtttccttct
tggaatggccttggaagcatccctagcacagatatctacaaaagtaccaagaattacaaaaacattgaaga
acctcaaggagtgaagattcttagattttccagtcctattttctatggcaatgtcgatggttttaaaaaat
gtatcaagtccacagttggatttgatgccattagagtatataataagaggctgaaagcgctgaggaaaata
cagaaactaataaaaagtggacaattaagagcaacaaagaatggcatcataagtgatgctgtttcaacaaa
taatgcttttgagcctgatgaggatattgaagatctggaggaacttgatatcccaaccaaggaaatagaga
ttcaagtggattggaactctgagcttccagtcaaagtgaacgttcccaaagtgccaatccatagccttgtg
cttgactgtggagctatatctttcctggacgttgttggagtgagatcactgcgggtgattgtcaaagaatt
ccaaagaattgatgtgaatgtgtattttgcatcacttcaagattatgtgatagaaaagctggagcaatgcg
ggttctttgacgacaacattagaaaggacacattcttttgacggtccatgatgctatactctatctacag
aaccaagtgaaatctcaagagggtcaaggttccatttagaaacgatcactctcattcaggattgtaaaga
tacccttgaattaatagaaacagagctgacggaagaagaacttgatgtccaggatgaggctatgcgtacac
ttgcatcctgaaagtgggttcgggaggtctctatgagcaaggaatacaagacaaaacttcctcaatgcatt
gactatttcttcagactcaaaacactcattctttttctattaagccattgaaagagaagcactaagactg
cttctaggctttatttataaaataaacaccttatccctaacatgggcaaaatggctagaattattcagacg
atttggcagcgtccagggtaagctggtgttataatacgctgctgatctacatcacagatttgctaataatg
ttcacgtgggccctggcatatctctgttcagttagagtgagtgctgacccaacagcctctgtggtcaagcg
agtcacgaatgattaatcataaagaaaaatcagttttgactgacctggatatccatgagctgcactgatc
accatgtaaggtcacatttagtaaatgctgaaataaaatgattaatgcatttatcaataaaagcctttgaa
aatactttggataataaattggagttttaaaaatgcaaatttgcttagtatctaataatgaagtgttatta
catatagccggaattgaggatctctttgatcctggaaatggtttacctaaaagctacagaaccaggccaat
atattttgaaatattgatgcagacaaatgaaataataaagagatttcatggtttataaaaatctttttg
atatgataataatcatgatcacaactgagatcaaaaaatatatgacagattatttgtttaaaaatgcag
ttttaattatcttagtctatagaaatgatcattgcatggaggcatgtataggtatgatctgtgtaaaatct
gacataaaaacagtgctattctgagtgaaatttttttgatgtgcttacataaccatggtgattaaaatga
gtttatatttttttctcaaaaatttagcagtgtgtaaagtaagtaatctttaactgaactctgaccactta
aaaaaaaatctaaaaattgaactacctatagtagtctgtgtttaaagtgaatttttaaagacaaagcattc
taaatgaactcaatataaaaacattcatttggaatgtacatactgaaaaatacaggtttttttgaccaaaa
gtttttatatcttttctttttatttatttttttcctaagtgccaacaattttctagatattatatacaaca
caggctttgatcttggggacttttcccatatatttcacactggagtgaatgaagttgtacttcatttctag
agaaaagttatacccaggtccccaattgagaatgtcttgcttgattgaaaacgacatcatcccttggtata
ctccagggattggtttcaggacccctgcatttaccaaaatttgtgcacactcaagtcctgcagtcacccct
gcctaaagatagaatggcttctctgtttttcttctgaaatacaaccagaaacaatgtgtctatttctgaaa

gaataggattaatgatcatacaaatgggttaatcctgaattctggttgtaaatctggttacagcataacta
ggattataatgctgcctcattttcacagcactacttgcttatattgacaacaaatcatctcgctaaagagt
gaatgtaggccaggcgcggtggctcatgcctgtaatcccagcactttgggaggccgaggcgggtggatcac
gaggtcaggagatcgagaccatcctggctaacatggtaaaaccccgtctctactaaaaatagaaaaaaaga
aattagcctagcgtggtggctggcgggcgcctgtagtcccagctatttgggaggctaaggcaggagaatgg
cgtgaacccgggaggcggagcttgcagtgagccgaggtcgtgccactgcactccagcctgggcgacagagc
aagactccgtctcaaaaaaaaaaaaaaaaaaaaaaaagagtgaatgtaatagtcttgcagaaaatgaatg
aatacctttgttcaataaaggaaatatgcactgctcactttttttgaaggaaatgccaaagttacgttttac
aacaaggctagagtttgtaaattctgggttcatttgtgatgacataagtcagcaaactgcgggaatactgt
ctcttctatgtattttgtgaatagtaagcataattttagttttgtattatcaatgaaaatttcacttgaaa
ttaaagctgccttttgttatatttttaacctataggataagattccagtattgtatatgagttttaacaaa
ttaaaaaatcaaatcatgtacatttgaaaatatttgcacacatttaaaaataaatgtaaagttgtcttta
aactactcggatgtgtcctttctgaacaa

A non-limiting example of a human wildtype SLC26A4 genomic is SEQ ID NO: 7. The exons in SEQ ID NO: 7 are: nucleotide positions 1-221 (exon 1), nucleotide positions 1005-1171 (exon 2), nucleotide positions 2262-2801 (exon 3), nucleotide positions 11504-11614 (exon 4), nucleotide positions 13530-13714 (exon 5), nucleotide positions 14311-14475 (exon 6), nucleotide positions 22568-22720 (exon 7), nucleotide positions 22821-22903 (exon 8), nucleotide positions 28419-28566 (exon 9), nucleotide positions 29490-29603 (exon 10), nucleotide positions 33769-33846 (exon 11), nucleotide positions 33987-34082 (exon 12), nucleotide positions 35299-35405 (exon 13), nucleotide positions 37408-37477 (exon 14), nucleotide positions 39449-39541 (exon 15), nucleotide positions 40467-40562 (exon 16), nucleotide positions 41193-41423 (exon 17), nucleotide positions 43697-43751 (exon 18), nucleotide positions 49420-49565 (exon 19), nucleotide positions 51905-51988 (exon 20) and nucleotide positions 56348-57175 (exon 21). The introns are located between each of these exons in SEQ ID NO: 7, i.e., at nucleotide positions 222-1004 (intron 1), nucleotide positions 1172-2261 (intron 2), nucleotide positions 2802-11503 (intron 3), nucleotide positions 11615-13529 (intron 4), nucleotide positions 13715-14310 (intron 5), nucleotide positions 14476-22567 (intron 6), nucleotide positions 22721-22820 (intron 7), nucleotide positions 22904-28418 (intron 8), nucleotide positions 28567-29489 (intron 9), nucleotide positions 29604-33768 (intron 10), nucleotide positions 33847-33986 (intron 11), nucleotide positions 34083-35298 (intron 12), nucleotide positions 35406-37407 (intron 13), nucleotide positions 37478-39448 (intron 14), nucleotide positions 39542-40466 (intron 15), nucleotide positions 40563-41192 (intron 16), nucleotide positions 41424-43696 (intron 17), nucleotide positions 43752-49419 (intron 18), nucleotide positions 49566-51904 (intron 19), and nucleotide positions 51989-56347 (intron 20).

**Human Wildtype SLC26A4 Gene Sequence (SEQ ID NO: 7) (NCBI Reference Sequence: NG_008489.1)**

1 ctcagccttc ccggttcggg aaaggggaag aatgcaggag gggtaggatt tctttcctga
61 taggatcggt tgggaaagac cgcagcctgt gtgtgtcttt cccttcgacc aaggtgtctg
121 ttgctccgta aataaaacgt cccactgcct tctgagagcg ctataaaggc agcggaaggg
181 tagtccgcgg ggcattccgg gcggggcgcg agcagagaca ggtgagttcg ccctgaagat
241 gcccacaccg cccggcccgg gctccactcc cggggaggcc tcgagggttg cggatgggac
301 tcttaagtgg tcacggatca ggtgggcagg gggcagtaca gctttctttc tgagacgccg
361 agagcgaaca ggctgctcgg aaaacaggac gaggggagag acttgctcaa taagctgaaa
421 gttctgcccc cgagagggct gcgacagctg ctggaatgtg cctgcagcgt ccgcctcttg
481 gggacccgcg gagcgcgccc tgacggttcc acgcctggcc cgggggtctg cacctctcct
541 ccagtgcgca cctggagctg cgtcccgggt caggtgcggg gagggaggga atctcagtgt
601 ccccttccag ccttgcaagc gcctttggcc cctgccccag cccctcggtt tgggggagat
661 ttcagaacgc ggacagcgcc ctggctgcgg gccatagggg actgggtgga actcgggaag
721 cccccagagc aggggcttac tcgcttcaag tttggggaac cccgggcagc gggtgcaggc
781 cacgagaccc gaaggttctc aggtgccccc ctgcaggctg gccgtgcgcg ccgtggggcg
841 cttgtcgcga gcgccgaggg ctgcaggacg cggaccagac tcgcggtgca gggggcctg
901 gctgcagcta acaggtgatc ccgttctttc tgttcctcgc tcttcccctc cgatcgtcct
961 cgcttaccgc gtgtcctccc tcctcgctgt cctctggctc gcaggtcatg gcagcgccag
1021 gcggcaggtc ggagccgccg cagctccccg agtacagctg cagctacatg gtgtcgcggc
1081 cggtctacag cgagctcgct ttccagcaac agcacgagcg gcgcctgcag gagcgcaaga
1141 cgctgcggga gagcctggcc aagtgctgca ggtagcggcc gcgcgggcct gcgtagagag
1201 aagcggagcg gggcgtccac gccttgggga gggaaggcgg tccccagcgg gcgagagtgg
1261 ggtgcgggcg gcggagcccc tgggcgccag ctgcttctcc cagaggcccg actttcggtc
1321 tccggtcctc cacgccgccc ttctggtggg agggtggctc catcagtctc gggcccgaaa
1381 tgaacttacc tgggaaactc gcctttgggg agagtgggtt ctaggagccc cgtctctctt
1441 tttcctctct gaaggaaact tggagtgcct cttggggtac agtgggtccc tgttgccttc
1501 ttgggagctt gtttaaatga aatgaatagg gaaacccagc tcttgaccag gaggagtcct
1561 tgaaacactc aagctaagta ggcgggctac cattcagtta gagaccagga tgcaagctag
1621 aacccagggg agcgcggggt gtgccaagta cttcatcagc aggctgtggg acccctgggg

1681 aaagccaccc tcagtctcta aacccaaaca tgccgtaact agatgtcaca aacataaaga
1741 aattagagtt tctaaaacct ttcattatag aacatttcaa atatatgcat aagtagacgt
1801 agtagtataa tgaactcccc acccccaccg tttttaaccc atcactcggc ctcaactgtg
1861 atcaaatcca agttactctt gtttcttgta tctacaccta cttgctccct ccagtattat
1921 ttttaagcaa atctcatata gcatactgtc tttatgtatt ttactgcata tctttaaaga
1981 ctcttaaaaa atataaccac atttcatgat cacaccttaa tatctaaaaa taacctccta
2041 atatcaaaaa ttgagtcatt attaaaatgt ccaatcgtcc cataaatgcc ttaattaaaa
2101 aacaatttat tcaaatcagg agttatacca cctacttttg atatttttct ttcatctgta
2161 ggtcactgaa tttaaaaact accaagtagc aaggtataag gtatacattc ctcatgctaa
2221 gatttttgta aaaactagct ccaggcttgt attgccaaaa tatactcagt gtgtttatct
2281 tctttaaaga aaataataat aataaggcgc ctggattagg agtctgaaaa gtaatctcca
2341 tttagagact ccacatccat cagtctctgg ctggaccaga aaataggttt ttgtgtagga
2401 atattttttc agaggattaa aaatgtgagc gaggggtggg acacttaatc ctgtgttctc
2461 tagaagagtg catttaaaag gatagataca gttcttggca aaagcatggt aagcacttca
2521 gggttattat tttccaggaa atacttatcc tttttccaaa tagttataaa catcagcaga
2581 atccagttca taactttgtg atttgcaaat tggttgtgac tgagattgga ttgaaaaccc
2641 agttttcttg ctttttgaca gttgttcaag aaagagagcc tttggtgtgc taaagactct
2701 tgtgcccatc ttggagtggc tccccaaata ccgagtcaag gaatggctgc ttagtgacgt
2761 catttcggga gttagtactg ggctagtggc cacgctgcaa ggtaagatgt tggcagattg
2821 agagttctgg tctccagcag gagtttaaca cttctcccca gctaccatag gtctgtgaca
2881 gatggttgct tacccttcaa ggcctgtatc tttcctgtag agccccttag tggagagagt
2941 cacctctctt ctccccttcc ttagagttct cttcctggga aactgctgcc ccactaggtg
3001 cagaggtcca atttagaggc atatactagg cagtggcttc tcaatttttt taaattttat
3061 tttatttgag acgggggctc gctctgtcac ctaggctgga gtgcagtggc gcaatcctgg
3121 ctcactgcaa gctccgtctc ccgggttcac gccattctcc cgcctcagcc tccccagtag
3181 ctgggactac aggcgcccgc cactacgccc ggctaatttt ttgtattttt agtagagacg
3241 gggtttcacc gggttagcca ggatggtatc gacctcctga cctcgtgatc cgcctgcctt
3301 ggcctcccaa aatgctggga ttacaggcgt gagccactgc acccagccag cttctctatt
3361 ttcattgacc acaattcaat aagaaatgtg taaagagttt caagtcaaga tttaaaaaaa
3421 aaaaagaaat gtgttacatc ctgatatata catatatatc taaagttcct gtgaaatatt
3481 tattataaca atgtgctaat ctttcacttt gttctattat gcttaggttt tttaaatgat
3541 gattgcagca cattaaatta ttttatatcc acacgtggat cttttttgtt ggtttgtttt
3601 gctttgcttt ttagagatac agtcttgctc tgtcacccag gctggtgtgc agtggcacga
3661 tcttggctca atgcaacctc tctgcctctc gggttcaagt gattcttgtg cctcagcctt
3721 ctgagtagct ggggctacag gcatgtgcca ccatgtccag ctaattactg tatttttagt
3781 agagatgggg tttcgccatg ttggccaggg tggtctggaa ctcctaacct caagtgatcc
3841 gccctccttg gtatcccaaa gtactgtgat tacaggcatg agccaccatg cccagcctcc
3901 cacacgtgga tcttgaggtg tagtttgaaa ctggcttaag gaaaagtgaa gggagcttcc

3961 tagcctggac tccacatgaa tgttttgcct gcccctcatc cctttttaaa aaaattcatc
4021 ctcttatttt aatgtgtggg aaaatgtgcc aacataggtg gttatttgtt cttttataaa
4081 ctgagttcat accataatat tcccataagc atcttagaaa tctgttgtat agttggtgcc
4141 tagcagcatg gcattctcct ctgtccccta tccccaccat ctcgtttctg ccccaccccc
4201 aacaccctga ccaatttccc aaagaccctg aatcatgaaa acagaaatat cgttatttat
4261 tataatttgt gaaacaccta atatgttcca ggcactaggt atgctgacac gaataagatg
4321 catcctctgc ttccaaggaa ctcatagtcc aatggagaaa gagaaaacaa ttcaatgtga
4381 taagggctgt ggcaggggag tgcaaattac ttgctaaggg agtgcaaatg aggttcactc
4441 aaagcggtct taggggatct tgaatagctt cctggaagtt gaatgtcaaa gctgagtttc
4501 cctgatggct aagtctgagt ttgccaagtg atgggaagta gggtggggtg gggatggctt
4561 gaaaatagac tatttcagaa gctagtaatg aatgttgagc aatattgtga gctaaaattt
4621 ggctatggag ctttcaagat tgctcagatc aggccaggta gatgcccgaa gagaaggagg
4681 atggcctatg aacccagggt ggagcctcag cctgatcaag catgttctta atcacaaagg
4741 tggacgggag agagactagg atgggctgtg tggttcctaa gttaaacaat gccaagtatt
4801 ctacactgtg ctctaagggc tgtgacatgg tgacaccctg tgactcctgg gggacaggaa
4861 ccatgcctca ttcatgtttg ttcagcaggt gcctagcaca gagacaggca cataatagat
4921 ggtatattta gtgaaaaaga ttgaattgca ttcctgataa tgaatcttct ttatatataa
4981 aaaatggtgg ttcatgctcc atgtctcccc agttttcata gacaagtatc ttataagaat
5041 tcattttgtt tataagcatg agacactatg ctaagtaaga atgttgccag ctatctcatt
5101 taatcctcat aacaatcctg ctaggtaggt ctattattat ttcattttca gtaggtgaaa
5161 aaatctatta aatcttgccc agggtcacgt ggctggtaag tggtacagct gggattttaa
5221 tccggtgtct attgtgtagt tccttacttt atgggtaaga tgcttttcta gaaacattat
5281 ctctataaag ttttctgtgt aaggtgaatt tctgggaggt taatctggct cagttacaca
5341 tccatttatc attcattcac ttaataaata tttataaact aattctgtag accaggtatc
5401 cagccatgtg cactcagtgg ttcattaaca ggcacaggtg ctgacttcat ggagctttgt
5461 aaaattattt tgaggggtag ggatgggaat taaataagta gataaataga aaataatta
5521 tagattgcaa tatctattgt ggtggattaa atatactgca atagggatta atttgaaagg
5581 tggatcttta tttttatatt tttttgagac agggtcttgc tctgttgcgc aggctggagt
5641 gcagtggtga gatcacagct caccacagcc ttggcctcct gggcccaggt gatcctccca
5701 tctcagcctc gccagtagct ggaactacag gcatgtgcca ccatgcttgg ctaatttttg
5761 tattttttgt agagacaagg tttcaccatg ttgcccaggc tggtctggaa ctcctaggct
5821 caagttatct gcctgcctcg gcctcccaaa gtgctgggat tagaggtgtg agccattgtg
5881 cccagccagg aagggaggtc tattttagat ataatggtca aggaagaact tactgagaag
5941 gtgaaattta gtaaaagaca agagaatgag aaggagacag ccatgtgata agcagaaaaa
6001 aagagtgtac caggtaaagg tgcaaaggcc ctgaggccag aaaacttagc attttccagg
6061 gaggaagagg aagaattgta gtgtggatga gtgcagtggg ggagggtgag taaaccatga
6121 aataaactga ggcaggactg ggattaggat gaggtaaggg aggcactcac tctcaggatg
6181 cctctttaaa tattgtaccc taggaaactc atttgcctca ccctattcct ggccctgtgg

6241 ggatattgct gtgaattaaa gggagaatgg gtgtgtcagc ttgaaccaga tgatgcaggc
6301 tatgaagaga gctgcaagat aggaatatat tgtgcagcaa gaatcactga cacataatga
6361 tgagtgtgga tgaaaatcac tgatgcctcc aaggttgttg gcttgagcaa ccggatagat
6421 cattacatca tttatctaaa tggcaaagac aggctgagga gcaggtttgg gataggaaat
6481 caagtttcct tttggatatg ctaagtttga gacacagggt gagacatcta agtggagatg
6541 ccacctagag aagtgggagc tactgcaagt gtgaaggtta gaggagaggt ctaggctaga
6601 cataaaaatc aggaaagcat tgctgaagag gtgatattta caagagtggg aatgcctgtg
6661 attacctagg aagaaagggc aggtgtagaa gaaaagagag ccagagtact tagaagttag
6721 gaaggcaagg agaggggcag ccaagaacct gagaaagcct ggcgcaggaa gttgaagaca
6781 aagaacaacc aaaggaatgt gggatcagaa gccaagagag aaggtttaaa aaaaaaaaaa
6841 aaaaaaaaaa agcatagagg ggctgagagt gttgaatttg cctgaggggt ggggtatgat
6901 gaaggattac agactgcttt taagaagttt tgccatgaat cagagggaga taagagatga
6961 agacagagtg aagggagaat ttgtttttct taatatggga gatactagaa cgtatttgtg
7021 aaccgatagg aatgacctag tagaaagaaa gagatctgtg gtgcaagaag tacaagagaa
7081 ataactagaa gcaaagtagt aaaaaggggc aaacgtggag gggtttgaga atggaactag
7141 gagaactact ccttaaaggc aggagggata tttcttccat tttcaaagga agatagccca
7201 aatggtgcag atgcagacgg gtttgaaatt ttgatgaaaa gtaacttggg agcatctcat
7261 gcattctgtg ttttcgatta aatgcagaat atgtccctta actttgagtc tggtggagaa
7321 gggtttgggc agattgaggt aagtagggga ggtttgaata gttgtctcaa ggagaggaaa
7381 ggcacacccc ctgaggaaat gtaactgggt ccccaggcag tgttgagtgc ccatctagca
7441 cttgtggtca cacattttac atgaaattgg ttagcccaga tgtagatgcc ttcagtccag
7501 tttatctagc tgctcaaagg ctctcatgga taaggtagat gtttgaggtc agccaaattg
7561 gggttttgcc agacaagaaa aggaaggaaa gagagagtga aaaaagggag ttaagaatat
7621 ttgcaaaatt ccatggaatc tgtgtgggca agggtggaag tgaagacagg aaggtgtgga
7681 ggatagagag aaagtggatc ggttaattaa ttagaagcct cgtgagcaca aataaatgtt
7741 gcaataagga ttctgcagta gatgagctgg aaggtttgga ggctgtagct ggagagaggc
7801 tcaaaattca gattttggag gtggtggtgt ggtttccagt gatgacaatg tccatgccac
7861 aaccactggc tgagcagctg agtagtagaa caactccgag aagaccagct caggttactg
7921 ggaagccagg aagttgctaa ggatgatggg atctttgtga tttttgttat taaaacaaat
7981 tcccatcaga cagcaaaaga tcgatgcaac ccttgtagtg ctatttttat tcacaacttt
8041 aaggagtctg ctgtctgctt tctccttttg acttttatgt atttgtttgt ttaaacaaca
8101 gaaatttatt tctgcaacat gaaggtgcct gtagattcag tttctggtga cagcccgtca
8161 ccttgctgcg tcctcacatg gtggaaggct ttacctttag aagaaattta aatttaatat
8221 aaatttcaac tgtttattag cacctgcttt gccagtattt gtcaccatgc tataaatcat
8281 gactaccaac agagaaattc tatcatttaa atcaatggct cagatttctt ctaatttttt
8341 ttcttttttg agactctttc tcacacaggc tggagtgtag tggtatgacc ttggctcact
8401 gcaaccactg cctcctgggt tcaaactatc cttgtgcctc agcctcctca gtagctgaaa
8461 ttacaggcat gtaccaacac ccccagctaa tttgtgtatt tttagtagag acgagatttc

8521 accatgttgg ccgggctggt cttgaactcc tggcctcagt gatcttccca cctcagcctc
8581 ccgaaatgct aggattacaa tcatgagcca tcacacctag ccccagattt ctttacaaat
8641 tgtgtaattt taaaactgtt gtaccaagtt tctttataat aaagatgatt ccagtatatt
8701 tgagtagtag tggtccctaa atgggacact ctgtatgaaa actgaaaagt tcccctttc
8761 caggcatatt aagaactgca gagcccagtt tggggaaatg gaagtttgag aaggacttga
8821 aaaatgtcca tatgaaatag acaagttgtg ctttcatccc ctgctccttc ttttcttatc
8881 ttccttctgt ggagtcacct tcctatttta ttccatgctg ttgggactac tgactattgc
8941 aattcctgtt tgtaatttac atattaaaac aattgtacta atagatatat ttttctagaa
9001 tacaaactcc actagggcag agactttgtt ttaacagtgc ctgacacatg gtaggtgcat
9061 aatcaactgt tgttgaatga atgaatgctt attgtagaaa actagaaaat acatataagg
9121 aacatgaaaa aatacctcca ctacaaacta gaagtctcct ctgatcctac catacagata
9181 acccattgat atttcagtaa atatcttact aggcacataa atacttatct atataaaagt
9241 ataatgaaat gtctggaaat aataagcacc aaattcagga taattgttgc cttctgaggg
9301 tagagggagg aaagaggatg tgattgagga agagtatact gagagtttcc gctgcattgg
9361 cagtaattta tttcttaagc tagatgatgg atacatgaaa gtttattata ttattcattg
9421 tgtattgtgt ttcttgtata tgtaaattac ttcatatttt attcaaaata cctccagatt
9481 tttttttttt tttgagatgg agtcttgctc tgttgcccag gctggaatgc agtggcgcaa
9541 tctcggctca ctgaaacctc cgcctcctgg gttcaagcag ttctctgcct cagcctcctg
9601 agtagctggg attacaggtg cctaccccca tgcctggcta atttttgtat ttttagtaga
9661 gacggggttt caccatcttg gccagggtgg tcttgagctc ctgacctcct gatccaccca
9721 cttcggcctc ccaaagtgct gggattacag gtgtgactca ccgcgcccgg tccctccggc
9781 tttttttaat gcgtacactc ccacaggctg gagtgggagt gtatttttta caaaagagga
9841 agcatacata cctatcagtt ttgaatcttg attttactca cttttttccc tcttacttca
9901 catctctcca cgttactgaa tgtacttctg agcatcacat caatttcatt tcatagaatt
9961 acgaagtatg aagcataact tagttaacca cttccctgct tggggacatt catctgataa
10021 gtaatattga gtgtgtgggg gccagcatat tcaggctgtt gccattttta ataaactact
10081 ataaatgtaa ttccttatta atgactcaag agtgaaaagg cttgattttc tctcattgtg
10141 agtgagtgaa gggtgaggag gtaaaacgga gtaagatatg ctggctacct aagtatttaa
10201 ccaaggaaaa gaatgtcatt atcctcttct aaccctcatg taaactagaa tgttgatttc
10261 tctatagcca ggcattaatg ggtctggggg ctgctgccat tgcctagtgg aggagttggt
10321 caacttcatc agggtagagt tgtcttgtct gctgctgagt ataattatca tatttttct
10381 ttgggtctaa cttttcttcc ccaccaaaaa aggagaaagg gcagtgtaac aatgactgtg
10441 tccctcagaa cgcagagaaa tattgagctc agacacaact gcctctgtgc aaaactggtc
10501 tgagcctttg gagtgaattg accaaaactt ttttttttct tttttagaca taaggtcttg
10561 ctctgttgcc caggctggag tgcagtggca cgatcatagc ttgctgtaac ctcaaactcc
10621 tgggctcaaa caatcctcct gcctcagcct ccttaataac taggactaca ggcgtgaacc
10681 aatatgctgg gctcattcat tttattgtag agatggagtc ttgctatgtt ttccaggctg
10741 gtctcaaact cctgggctca actgatactc ctgcctatgg cttcccaaag cactgggatt

10801 ataggtgtag gacaccaggc ctgaccaagt ttttaatcaa catttgactt gattgttttt
10861 tcccactgat ctgattggtg gaagactgaa ttactgatca actgagtcca ttcatttgag
10921 caggtgatgt ttgtagatgc aatcattgtt ccaggtgatt tggatcctgt tctgcatcct
10981 agcatagcca gtctgtatgc aagataagga gaccaactca ggacaggctg atctgttgaa
11041 actgtaaatt gagagtatct gtgtaaaggc ccttctttct gcagtagaaa gacagatgct
11101 cctctacttc tcatgagtta catcctaata acccattgca agttgaacat attttaagtc
11161 aaaaatgcac ttaatacacc taacctatgg aacatcatcg cttaggctat cctacctgaa
11221 atgtgctcag aacacttaca ataacctaca gttgggcaaa ataatctaac gcaagcctat
11281 tttataataa agtattgaat atctcatgta gtttactgaa taatgtactg aaagtgaaaa
11341 acagaatggt tgtatggtta cttaaagtat ggtttctact gaatgcatat tgctttgca
11401 tcatcataaa ggcaaagtca taagtggaac cattgtaagt tgaggacttt ctgcatactg
11461 taactttggt ttgtgaatgt aatcactttg catgtgcttt cagggatggc atatgcccta
11521 ctagctgcag ttcctgtcgg atatggtctc tactctgctt ttttccctat cctgacatac
11581 tttatctttg gaacatcaag acatatctca gttggtaatt ataagtatat tttacaatta
11641 tatttgctca tgtttaaagt gttttggcta tattaagtgc attatacctc tattaggttg
11701 gtgcaaaagt aattgcggtt ttcacaatta tacttttaat tgtgaaaacc gcaattactt
11761 ttgcaccaac ctaatatatc tgtgttaatg ttgtcaggga aatgggattt cagtgttttg
11821 cctgcttttt ctattcactg atgttaggta acttttttaa tgaagtggaa aaataaaaaa
11881 actgaaaatg acagcctact ttaacatttt agcatgtttt gctttttaaa aacgtgatca
11941 atttgactcc atttttggag tcataatgac agggaaaaaa tgacacaagg tctttgaacc
12001 taccatttta ctggattaac ttagaaatta ctaagtatgt tcatcaaata catgttcatg
12061 atttaattaa agagcatact tattttaaat cactaatatt agaagactgg caaaatctta
12121 ccaaaacaat ttagaagcta agcttggctt tctttcttgg tatggcttat aataagtttt
12181 cctgctattc ctaaaatttg aattctcgtt tgttagaatg tcctagtaaa tgagtgtttt
12241 aatgctattt tgtgactttg acatttatac tagagaaaat tttgctccta catccatctg
12301 ctggctagac atgtctacct cgatgatcct cttgaaataa atgcaaccat ctactttatc
12361 cccaacccat gtttttgtcc ttagttgtat ttccttagaa ggagatcaga aagaggtttt
12421 gattgaaaga acttccacac cttggagaat caatcagtgt tgagtgcttg actagatgag
12481 gaacatggaa gctggttttt ctctttggta ttctatacat tgagatattc caggtgactg
12541 gggttgggtt ttgagacctc aaaaccataa agccttccat gatgagaggg tagaatcatc
12601 atcatcgtca tggataaaaa taacagtagt gaacggagca attgctgaaa aaaaaatatt
12661 ttttaaaata taaaatctta cagattgaca tttgatatga aaaaatgttt tgtcttacaa
12721 aaagagaaag aaacttcata ctccctttgc tgttttttta atcctaactt cgaccctgtg
12781 atattgatca aggtactcag atccacagag atcaggacat tggacactct aggaaattaa
12841 tgagattata tgaaataaaa gttgagcgga acaagtaaac actcaatgta tgtgctacct
12901 ttgccagaaa acctttcctg acaactctca tccctgtccc caccacccat acacatgagg
12961 atcacacaca gacaggttag acgacctttt gatatactca cgaagcatcc tgaatgttgt
13021 attctaataa ctacctacgt gtctctctcg cctatttcta ggtcatgaat gcctcgagat

13081 atctgcgttt ttagcttctt tttgttgttg ttgagacaga atctggctct gtcacctagg
13141 ctggagtgcg gtggcgtgat ctcagctcac tgcagcctcc acctccccag ctcaagcaat
13201 ccccccacct cagcctcccg agtagctggg accataggca cgcaccacca cacccagcta
13261 atttttgtct tattttggta gagatggggt tttactatgt tgcttaggct ggtctggaac
13321 tcctgggctc aagcgatctt cccacctcag tctcccaaag tgctgcggtt acagatgtga
13381 gccactgggt ccggctcagc ttctttcgtg aacaaacaat attttcctag tcacagctaa
13441 atcttttata cattttttaa accctatgca gacacattga acatttgtga ttaataactg
13501 attaattgtt agagactttt tttccccagg accttttcca gtggtgagtt taatggtggg
13561 atctgttgtt ctgagcatgg cccccgacga acactttctc gtatccagca gcaatggaac
13621 tgtattaaat actactatga tagacactgc agctagagat acagctagag tcctgattgc
13681 cagtgccctg actctgctgg ttggaattat acaggtaatg aacttacaag taaaatatag
13741 atggatgtaa tttttatttg aaattaactt taaagcatat agacttaaag attctactaa
13801 aaacaaaaca aagtaatttc ctggaaccca aaattatttt ctaaattacg ttgttttagg
13861 tcaggtgcta aaatagtaag caagacccca cttattaagg ctcacttatc atcaaagtca
13921 gagacagaaa aaagcacaga agaacatgtg tgattcaatt gaggatgaga gaagggaaat
13981 atacgggaat ccataaagga gaaaaagtgt tctggtgagc ggagacacag gaccgaaagc
14041 cacataaata agctctgggt ttttgctttt ctaagtacat gtatagaaat acttcaaggt
14101 ttttattaca cttagttttc aaattttaga gtggtggagg aaggggagtg atagggtatt
14161 aagaaattca tatttttttc taccagtatt tttgtgctat aggcaggcta ctagtgtttt
14221 cattggtatt aagcttgatg taatatttcc agagagtagg tttctatctc aggcaaacat
14281 ttaatttttc tttccttttc cttatcgtag ttgatatttg gtggcttgca gattggattc
14341 atagtgaggt acttggcaga tcctttggtt ggtggcttca caacagctgc tgccttccaa
14401 gtgctggtct cacagctaaa gattgtcctc aatgtttcaa ccaaaaacta caatggagtt
14461 ctctctatta tctatgtaag tgttgcttct tgctccaggg atgggtcact gttcattcca
14521 gaaacaattg tattcattct ctgagtctgg gccaggcgtg gtggctcaca cctgtaatcc
14581 cagcactttg gaaggccgag gtgggcagat tgcttgagcc caggagtttg agacgtgaga
14641 cctcatctct taaaaaaaaa aaaaaaaaaa aagaaagaaa gaaaagaaaa gaaaaagaaa
14701 gaaaaaatcc aaaaatccga aaatttgctg ggtgtggtgg tgcacacttg tagtctcagc
14761 tacttgggag gctgaggtgg gaagatctcc tgagccctgg aggttgaggc tgcagtgagc
14821 tgtgatcgcc ccactgcact ccaccctggg tgacagagca agaacctgtt tcaagcaaaa
14881 acaaaatcaa aacaaaacaa aacttgagtc tgggagcacc cacatttctt tctttctttc
14941 tttttttttt tttttttgag acagagtctt actctgtcac ccaggctgga gtgcagtggc
15001 atgatattgg ctcaccacaa cctccacctc ccgggttcaa gcgattctcc tgcctcagcc
15061 tcctgagtag ctgggattac aggcgcccgc caccacaccc agctaatttt tgtatattta
15121 gtagagacag ggtttcacca tttgggccag gctggtcttg aactcctgac cttgtgatcc
15181 acccacatcg gcctcccaaa gtgctgggat tacaggcgtg agccaccgcg cccggcctgg
15241 gagcacccac atttctctat gcatactttg ggagccattg aaaacatttg gttgcttctg
15301 tcagaaatac tattgtactt aaaaacatta atgaaaacaa agtggtctaa tgaagtaaag

15361 gctgctctgg aatgtaacag acttgatttc tagcctcagc tttgtctcta acaggtaagt
15421 gaccctggat acatcatgta gggtgctaat tgaaagctca gttttagaac tggatggacc
15481 ttgattcata ttctggctct actgtctact agccatgtga gccaatcacc atgcctcagt
15541 ttccttctct gcaaaattgg gttagtaata gtatatgcct cattggattg ttaaataaga
15601 atagataagc acttggccca ggggctggta cataacaagt actctaaata aaggagttat
15661 tttgaaatga ttatttcaag ccatcttttc ttttttattg gtgaaatgaa atgattggag
15721 atgtatctct aaaagctttt tctaaccata agagtccgta aaatgcataa tgtaaatgtc
15781 tcaacaatta taaatgaaaa ggaacattag attcagagat gattcaccat gcaaaagaaa
15841 tgcaaaagca ggatgtagat cacactaatt agatttgaaa aaggttttgg atctaaaaat
15901 gtgttctaca taaagcatgt tattgatgct tgaaaaatgg tgataatagt aacatgatgt
15961 catcctagct tggggggaat gataccatac aaaaataacg tgtcaacatt aagaatggag
16021 tttgagtctt taaagaccca cctattagct tctaaaacaa tgtaaggcca tgtacacata
16081 tctaagaatt ggtgagtatg tcaataattg ctcaatttta taacggtgcc ataatcccat
16141 actattacac tccatccatt caacaaatat taccaagtgc ttactacagg ccaggtacta
16201 tgctaagcag tgagaataga cagaggaggc agaataacat agtgaataag atcatgaggc
16261 caggtgtggt ggctcatgcc tgtaatccca gcagtttggg agactgacac aggtggatca
16321 cttgatccca tgagttcaag atcagcttgg gcaacatgac aaaaccttgt ccctactaat
16381 aatacaaaaa taattagaca tgcattgtgg cacgcacctg tagtcccagc tacttgggag
16441 gctgaggtga gaggatcacc tgagctcagg aagttgaggc tgcagtgagc cgagatcatg
16501 ccactgcctt ccagcctggg tgatgggagt gagaccctgt ctcaaacaac aataacagca
16561 acaacaaaca aaaaaccaaa aacaaacaaa aaagatcatg gactttgaac ttcaacagac
16621 ttgagttggc atcttggttc tccacccact acccatgatg tttatactca acttccctaa
16681 gcctcagtgt cctcaattgt aaaatgggag tagtaatagt agctacctct tagagcagtg
16741 aggattgaaa aagaaaatac gtgtaaggca cttagcacaa tatctgtcac atagtaagaa
16801 ttgagtttat tattattatt atcacagatg aatagacctc atgaagctcg ccgttcattt
16861 cctcgggacc cagcctatta ggaaacatta gcaaaagttt ttgattcagc attatatggg
16921 tcattcttga tatgtttttt cctgtcaaat cctcagtttt ctattaaaaa agaatttttt
16981 tttttttttg agacagggtc ttgccccatt gcctgggctg gagtacagta gcacagtcat
17041 agctcactgc gacctccatc tccctgtgtc aagcgattct tccccctcag cctcatgagt
17101 agctggaact acaggtgtgc accaccacac ctggctaatt ttctttttct ttgttgaatt
17161 ttagtagagg caatgtcttg ctatgttgcc caggttagtc tcgaactcct gagttcaagt
17221 gatcctctca ctttggcctc ccaaagttct gggattaaag gagtgagcca ccatgtccag
17281 cccatatttt aaaattaaaa ttaaaatttt aagattttat tttggtagag acaggggttt
17341 tgcaatgtcg cccaggctgg tcttgaaccc ctggcctcaa gcagtcatct tactgcatgc
17401 ctcctaatgt gttgggatta taggtgtggg ccactgcacc tggccaatcc ttagttcttt
17461 ttgagacctc tgactgcctt attttgacag gctctgtaac ttttgcagct cctggcacat
17521 cttgctttta cttactttta cattttctcc atcaaatgtt cactcattta ttccttcatt
17581 ctcattcatt cagtttatct actcaataaa cacatattga atacttacca tgtgccaggc

17641 actgttcact ccctatacaa acccagtgac tcattaacct accaagacat ctcttactga
17701 gtattctcac ctcactgggc aaacattgct aggccttcat ctacacagat cgcaggaggt
17761 ccaaactaat tgttgacctg ggtccatggc catttcagga cccctgcaga acaggagaaa
17821 tagttcttct ggtctcatag gattatgttt aagaagaaac tttctgcaaa tgatttcact
17881 ttgacaggga caaaagtgag caggaaacac tagaccaagg tcaaaaatag tttttaaacc
17941 aagtgcagtc attatgtaac ttacttcctg ttgaatattt ttcttgaaat ctcagtttag
18001 catacagcat agaattttgc aagaaactcc tcacaggcca cccaagttgg aaaaggtatt
18061 tcagtcatcc ctatatggtg gtgaatataa gtttgctctt ctgaaaaaaa aaaaaaatca
18121 gcaggtctga aaacgattgc atagggttaa atgagccgct tagtctctga acagagaatt
18181 ttgcttatga cattaccatt gcaattatac atgaaagaat ccttcaataa atgcagagta
18241 gtagaggatt ctcccagaaa agactgtaag attgttgtaa aatactttac ctggagtgtt
18301 aacttatctt tgttctaagg ttgcactttg gtttgtcctg ccctctcccc caaaaaggtg
18361 ctgacaacta gaagtaacaa atgcaaagaa ataggggaga aaatattaga agaaaaagaa
18421 atcagttaat ttacaaatgt cagagatact ggatgtagga ggcagaaggg agaatgacag
18481 ggtattggca gcaatttaga attagtcaat acccttgatg ctaccatgta gctactcagt
18541 tatctaagta aatcccagtc cacttagggg ttgatttccc accactcaat ttgaatggtt
18601 tgtctcagta gacaagggtg agagctttga aagccaccac taagcttgat tagatttgtt
18661 actttgtacc tatttcagta tgtaaaattt atacattgta ttaaagtaac actttaagga
18721 ttgatctata catgtataca caaaagatat ggaaggaaat gcctgacagc aaaaggtggt
18781 cgtgaagcat ttgtgatatt cgtagagata aaataaacaa aaataaattc agttccaata
18841 gtaaactgat caattttctc atacacatta ttgggcataa ttttttctta cggtttcata
18901 aacacaggta atcctgggca agaacaaaga aattatctaa atgaaattgt cctaaaattt
18961 tccaaacaca gcaggaaata taataaaata ttatctatta ccaaaaagtt cattatcata
19021 atttgaatta tttggaatac taagctaaaa tacagaatta tatgtgtatc catactatta
19081 acatacttta tggtatttta acagtaaact aaattagaag tactgttcag tgtttttgta
19141 gagtctaatt tctatgtcgt taaatataat cttatattat tatataatat aatcttatat
19201 tattatataa tcttatatta ttatattata taatcttata ttatataatc ttatcttata
19261 taatcttata ttatatgctc ttatataata taatcttatt atataatctt atcttatata
19321 atataatctt attatataat ataatcttat tatataatat aatcttatct tattatataa
19381 tataatctta ttatataata taatcttatc ttattatata atataatctt attatataat
19441 atattcttat cttattatat aatcttatct tattatataa tataatctta tcttattata
19501 taatataatc ttatcttatt atataatata atcttatctt attatataat ataatcttat
19561 cttattatat aatataatct tataatataa tcttaactta ttatataata taatcttatt
19621 atataatctt atcttattat ataatataat cttatcttat tatataatat aatcttatat
19681 tattatataa tcttatctta ttatataatg ttatattatt atataatctt atcttattat
19741 ataatgttat attattatat aatcttatct tattatataa tcttatatta ttatataagt
19801 ataatctttt attatattat tacataatat ataagtataa tcttatatta ttatataata
19861 ttattattat acaataataa tgtaaactga ctattgcagt ttacatggtg agtttatttt

19921 ctgcacagat taaactttct atcatagagc tcttcttaaa agattctgaa tgactactca
19981 atatgtaggc aacttattta catactcaga taccttgcat gatgcaattc tgtaatataa
20041 actgattttg gtttttgaac ctcgtgttac atttctaatc caccagacat actttttatt
20101 caaaggtcta attgattctc atggattaaa ctggaatcct gtagtgatta tttttgtaag
20161 gtaaaaatgt ttgtgtgatt gggataatga ttccaaatgt tttgtttttc aagttttaat
20221 ttttgcatct tgagttgaaa agtacacctt taatgaatag acaattcatc aaaatgttat
20281 acctagctcc tgaattaatt agggtcattc atcagaaaac tgattatgta aaggaccaaa
20341 cagcaatgat aaccttcaag cctctgggat aggtagggct gtttgctcct gtgctaaatg
20401 ccctaagttc ttgttatcgt gtctgctggt ggtagttatc ccattttctt tttcactctg
20461 ctgtccctgg catcatcttg tttctgtcag tgtaaatgcc tttagcaatt ttcagtgact
20521 ctgaaaacca gacaaagtga atttgttagt actggatttc aagaaagagc aaaagtaccc
20581 tctgagtgaa agttcaagag tcttcctaca agttaaatgc ctgaactcta gtccaagtgt
20641 ttggtgggag gtattttgca tgcataatag cttctccccc atttccttac tcttcttgaa
20701 tgtgttccag agagctgagg ttgctggcaa agtgactcct agattactta aggttcatta
20761 agtacttaaa gttcattaag ctcatacata attgtcacca gattatcatt ataattattt
20821 taaaagtagt ttatctgtga aaataagtaa aatacatttt ataaatgtta taaactataa
20881 gatgctccat tattttatct tcctataaaa tatgtctata aaaaagctca tttaaagaaa
20941 agttaccttc actgtgagat tgtctcaaag aaatgtctca aagatacctt cttaaatcct
21001 gccttctaca ggactgaagt taatataaca aagatattag tgttcttcct tgggattctt
21061 ccttgctaat ttccttgaaa acacccttga ttcccagaag tctcctattt acaaaacaaa
21121 actagttttt cttgaattga ttcatctgtc tacttaaaga ttttttttgg ccagttgtgg
21181 tagctcatgc ctgtaacccc aacactttga gaggttgggc aggaggattt cttgaggcca
21241 ggagttcaag accagtctgg gcaacaaagt gagacaccat cccctgcccc cacctcctcc
21301 cgccccaatc tctacaaaaa ataaaatagc ctatttattg tactctctac aataaataaa
21361 ataaatatag atgcctgcct gtagctccag ctactctgga gggtgatgtg gaaggattgc
21421 ttgagctcag gagttcgagg ccactgtact ccagcctggg caagagagct aaaaaaaaaa
21481 aaaaaaaaaa aaaaaatttt ttttatgtta tcttagttca agtttgagaa ataaaattaa
21541 gtaccaaatt tattcccaaa aatgtatgct tgtcaggaga tatacctaat gttaaatgac
21601 gagttaatgg gtgcagtaca ccaacatggc acacgtatac atatgtaaca aacctgcacg
21661 ttgtgcacat gtaccctaaa acttaaagta taatttaaaa acaaaaaaca gaaaaaaact
21721 acagattcaa ttcagcttta ggaattaaaa aaaagatttg atgtaattat atcattataa
21781 aatcaaattt tttaaataaa acatgaaatt gttttaattt aaaaaaaagt atgcttggcc
21841 tatatgtcaa ttcaatagct tttctttgcc agaaagaatg aagtcagtct acctgtatcc
21901 acatatgaag tattttagga ttttttgtaa ttttttgtta ttatctatat cattcaggtt
21961 taggaaagaa ctttacctaa aatactggct aattgtgccc caaaaaaaag aaagctagaa
22021 agaaagaaca cacacacaca cacatacaca catatacaca cacaaaaaaa acaaccaact
22081 gatcaactgg gagtttcagg tttatcagcc tttagtctca atgctgtatc actgtagtaa
22141 aaatcaggct acttgatagc acctagaaac ccattttaaa atataaactt gtaaagcatt

22201 tctgtaagta aaagttagta tgtttataat gacatttttt cttttaagac atctttatta
22261 ttatcataaa aaatacagag aagcaaaaat aagaatgtag aaatcaccca gtttttcctt
22321 tccaagtaaa caatcataaa aaacttggtg tatatatata ttgcagcaat tgtatgtatg
22381 taatggtctc tgtatcaacc aacacatttt tatcatttta ctgaaacttt tgagtgttgt
22441 ttgatgctga tatcatggtt tttcatgtgg gaagattcat atgagaattg attgtgtgtg
22501 tgtgcgtgtg tgtgtgctcg tgtgcgtgta gcagcaggaa gtatataaaa ttatttcttt
22561 tttatagacg ctggttgaga tttttcaaaa tattggtgat accaatcttg ctgatttcac
22621 tgctggattg ctcaccattg tcgtctgtat ggcagttaag gaattaaatg atcggtttag
22681 acacaaaatc ccagtcccta ttcctataga agtaattgtg gtaagtagaa tatgtagtta
22741 gaaagttcag cattatttgg ttgacaaaca aggaattatt aaaaccaatg gagtttttaa
22801 catcttttgt tttatttcag acgataattg ctactgccat ttcatatgga gccaacctgg
22861 aaaaaaatta caatgctggc attgttaaat ccatcccaag ggggtgagtg tggtgttcct
22921 cttagtacta atacattaag tcagtaagtc agtctttttt atttaaataa aacctttat
22981 tacaagcttc atttcactga tactccttca atagtcctat ttgtgtgtga tctggaagaa
23041 acaaccataa gacaaacagt atgtgtgtag ataaacgtca agccatttgt ttaaaagcag
23101 cccgtatatg ctcttttttc ttcattttct taaaacatgg cagaacatgt acggttaaca
23161 ccagagtcct gatttaaact tatacataac attataatat aatataaata gaatcatttc
23221 tcagtaacat ctattgcact gtgaacaatc tgcttaagga atcagggacg tctggcctaa
23281 attctctatc atttagttta atcaattttg gataataatt atatcctcgt acccataagt
23341 aaaatgcatt atcttcaagg gaagttttag aaacatttgg tagtttggtg aattagaaga
23401 gtaaaagatc aaggtccttc attcatctct gtcaccttcc agttttgtga cctgaggaga
23461 ttctcctata taagacaagg ataatgaaaa cagccgaagc ctcaactcaa gcttattatg
23521 aaacaccagt gagatctctc atgaaatgaa gagtagtcaa gacaccctac accataattt
23581 tctcatatgg ggttacaaag gaggcctaga cgtttattat atttgtaaca tacactcata
23641 acatttaaga ctagacttgc tgttaatcaa gaagtactag ttttccctaa gactggaaga
23701 gtttggtacc ggtgtttcta ccaggaaagg gagtggaggg gagggaaggg cacggagcca
23761 ctgtttggta ccagtgtttg ttttccatgc tcttaataga aacttcctca ggcaagctta
23821 ttcccagagc ttattctaca gggaactgct gtattcccat ttgatgaatc ttataatttc
23881 ctctaatatc tgttccaaac tatttgaaaa agcacagctg gctacatggg acttaatttg
23941 catgctatcc attacagtgt ttctcaaact tacccttttg taagaatcac atgggatcct
24001 ttttcacaca gattcctggt tcccacccca aacctattga atcaggcttt ccagaggtgg
24061 ggctcaggaa ttaatatttt tcaaaagtgc ctcaagtgat tcttgccctc aaggaaactg
24121 gagaaactct ttaagagagg atcaacaaat ctaatctaac aactagataa tttcattagt
24181 tagaaacaat tacagaaaaa gatactttac agatctcttt tgtcaggctt cctaggatgt
24241 atcaagttat aggtggtcca cagagcaggc cctgtcccat ccatccgtgt tccattagtt
24301 ggtttaggga actcatttct tctgccctaa attatccctc caggtcctct gcagagcttt
24361 cctgtgctct cctcctgaga acagatttct agcctctcgc ccttgagttt aatgaagctg
24421 aatcctttga cagaccacct ggtcagcttg gtcagctaat ttaacctgcc tggccccagg

24481 gactttaagc tactccatga gggcagtgac cccattggtg ttgttcagct ctgtcccaga
24541 gcacagagct catctcacag taagctctca gaaaacactt gttgaaggaa taaaataccc
24601 aagcctatgt cattataaac aagccacaac aaaggccttg gttatctgat ctcacttggt
24661 agcacagaag caattctgat gatattaaca aacctgaaaa agaaagggag tggagagatt
24721 ggaaaaaagc aagcagagtt ttaaagtggc cctgaggtga gcacagtgaa aggaagaggg
24781 acagagagat tgtgatatgc ccccttttga tctcctccct gcttcaatcc tccattaagg
24841 ctgctgctcg tagttccctt tatcctattg agggtccata agttaggatc actcctaacc
24901 taggaccata cgttaggacc ataagttagt ccccaataac aatgccccat tggacagcca
24961 aaaacagtgg ctccatgccc ttccctctac tccctttcct ggtagaaaca tacaactttt
25021 ccttgtccag tcaacaccta agcgccccac taggattcac tcattctccc atcctgggtg
25081 ctttcttaac cagcagacac tatgagggcc agtcatgtgc ctctcttctc atgcaactgg
25141 tccttatctg caactcgtaa tctgcccagt tcctcactca tcctgcccag gcttctcttt
25201 tacctcttct gttatagtgg tgtgtgtgtg tctgtgtgta tgtgtgtgtg tgtgctcacg
25261 cacgtgtatg tgtaaaccca taacaagatt gacagctctg tcctctaaat tttaatccct
25321 attcactctc ttgcctcctg tccttgccag ttccagaggt ccccttgttg tcatgtttgt
25381 tactctcttt tttcttttct tttctttaac agagtctcac tctgtcaccc agcctggaat
25441 gcagtggcat gatctctgtg gtgtgtgtgt gtgggtgtgt gtgtgtgtgt ataaacccat
25501 aacaagattg acggctctgt cctctaaatt ttaatcccta ttcactgtct tgcctcctgt
25561 cttcgtcagt tccagaggtc ctcttgttgt catgtttgtt actctttctt tcattctctt
25621 tctttcttcc cttccttccc tcccttccct ccctttccta ccttccctcc cttccctccc
25681 ttccctccct ttcctacctg cactcccttt cctccccttc ctaccttccc tccctttcct
25741 ccccttcctt ccttccttcc tctctctctt ttttctttct ttctttcttt ctttctttct
25801 ttctttcttt ctttctttct tttctttctt tccttctttc tttctttttc ttttcctttc
25861 tttccttttc tgaaacagaa cctcactctg tcacccagca tggaatgcag tggcatgatc
25921 tcggctcact gcaacctctg cctcccaggt tcaagcaatt ctcctgcctc agcctcctga
25981 gtagctggga caacaggcac acactgccat gcctggctaa tttttagtag agacggggtt
26041 tcaccatgtt ggccaggctg gcctcaaact cctgacctca ggcgatctgc ctgcctcagc
26101 ctcccaaagt gctgggatta caggcactgt tatagacccc ttttgtcagg tttcctagga
26161 tgtatcaagt tataggtggc ccgcagaaca tgccctgtcc catcaatctg ggttccattc
26221 attggtttgg ggaactcagg cctactgttg tttttaaagt atgtgctttg cagttgtact
26281 tgaaagtgtg gcacaggggc aaagccaaca aaaccacagg gtttttttcc tggctgcttt
26341 ctgagggcta ctcttctcct ctagctggag tggtggtgca tttgccaagt gttcagaccc
26401 agtctgaagt actctggtat tctgtttggt attctgttta tgacgctggc ctgtcgtttg
26461 tggaattttg ggcccttcag ggttcacttc tcccggtctt catcttttca gataagtgtg
26521 tgttcacatg ctcactccct tgggctcttc atagtgttgt cttatgatca ggcctgacat
26581 gcttggctgg ggccaaacag gcccactgag caaaaaattt ggagagttat atccacagaa
26641 ggagggcaag ggcaagggca aggacaaaag gcttacttac aggtcattag aagatatcca
26701 gacgggactt caattggttt ggggcaaata attggcacac ttagggttca gcaagacagc

26761 agacatgagt ggcaggagta tgacagactt agattctccc aatatatacc aagaacctac
26821 tatatgtgta gacactaatt acttacttac acaagtgatt cctcattgaa tcatatgaga
26881 agaatatata gtccccattt gacagctgag gaaactggga tgtggtgggt ttaaataaca
26941 tgcctaaagt catttgagag tgattctggt cctttgactt aagtctttgc actttacact
27001 ttgcacacct ggataatcaa ggatcaggtc atatttggag aactaggcag ccaggcaggc
27061 aaagtgcatt aggctgctat caaaactaag ctgtatgtga aactggaaag ttatcctgac
27121 aagtccgagt gacctgaagt agggttgact gggtctcagg agcaagtcag gagcctgttt
27181 tgagaagtgt ggtcccaagt cacatcccaa ctagcaggtg atgtcatgct gagtcgccca
27241 actgttggat cagggtttcc aacatcctgt ctgtctacag atggggttca ttccacagtc
27301 tcattgtggg gtaggatggg atataaggag aaaaggagtc tgaaaattaa cctcactggt
27361 agggagagta aggctacaag ggcagagaac cagacagatc ttgttaggta ggaaatcgtc
27421 taatatctaa catttagacc tggacccact aactgacatc aagcaaatca cttcacgttt
27481 ccaggtctaa gttcctttat caacaagaca agacaagagt ggtggattca acaataccta
27541 ggatgctttc tctatggttt tctctaagaa ccttatttct attttatttt taccactgtt
27601 ctctaattat aggggaaaat gtagtttcct tattaaagaa actttaggca taagtgatgt
27661 aggtcgggga gacctttcat ggctgggggc ctctggaatt ataccatatg gatagtcatc
27721 aagatatagg tccatctccc aaattttcat atttgagaga acctcgtggt ataatgtgta
27781 ggttaatttt tttatatggt ttgtagggta gctctggttt tgtcaaacag tagtcattgg
27841 accccacaat cactgagata ttttagtcac tatggagcgt tgtgataatt acattaattt
27901 aatctttttt gccttaagaa gattttaaat gtcctggctc tcaaaggatt ttccactgag
27961 ctcttttagc ttcatacatt ctttgtctct ttaacagagc tgatacctct tcaaacacag
28021 taaatctcat attatggcta gaatctcttt taaattcaaa gaactttaag atggaattgt
28081 ggaaggagaa acactaacat gtgaaatggc atggatgggg ctgtatgata ttaaagaatc
28141 agtaacagca gatttattca tgtgagagat agagaagagt cagcatggct aggctcaaaa
28201 atagaggaca aagaaatcag ccagtaagat aacaccaact tctgattaaa cattataaaa
28261 gctgctacta tcatgtatgt atttcgtgtg ctttttgttc ttttggatca agtactttca
28321 tgtctaatat gtgactgagc agatatagca tttgatgaga tggggaaaaa ggatggtggt
28381 caaatcttca cagcattttt cacttaaaaa ctcactaggt ttttgcctcc tgaacttcca
28441 cctgtgagct tgttctcgga gatgctggct gcatcatttt ccatcgctgt ggtggcttat
28501 gctattgcag tgtcagtagg aaaagtatat gccaccaagt atgattacac catcgatggg
28561 aaccaggtat gggtgccctt ttgctgaact ggttttatag ggctggaaac aggaaaaaaa
28621 cataaatgga aaagattttg gtgtcagcta aagaaggggt tggattcttt caccagacct
28681 tattgggttg gttttcctct tgtgtttgct aattagaaat tattttggaa tagacacact
28741 gcatcacttt gcttttttaa gaaataagtt gaaataacac taattttaaa agagctaatt
28801 cagtcaggtc ataaataaca ttcaaagtga acatattagg tcagaaagag aattcttcat
28861 gtaacccagt acctcttggc aattattttt aacggcagtt ataatgtgac aaaacagtgc
28921 aacaaacatt aataatagtt gtgaccactt taaattgagg tggttcactg tgcagttaat
28981 atatacaggc aatttttgta ggcatgtgat acattgccag gtaagctgat ttcacactgc

29041 ttgcaaattt gcaagttctg tatctatatg tttctgaaaa tacagtatag caataacagt
29101 agttaaatgg ttagtaatca agcagaataa cagcactgct agtcataata tgcttcatag
29161 tgaatattca accacagaaa aacactttat gtgagagctg cagacattcc attcactagg
29221 cagtgaaaaa gcttagcaaa tgatcaggtg ctatttcttg tcaactttaa aaattatcga
29281 gagcaatgag acctctctca gatggtatgg cgtccaaact cctgatgtcg tacaaggacc
29341 ccaagtacct atcacggtaa aaattaaatt ggaccaccac gcagagtagg catgggagtt
29401 ttcattctta atgtacttcc tgaaatactc agcgaaggtc ttgcaaagat tcaatttgta
29461 ggatcgttgt catccagtct cttccttagg aattcattgc ctttgggatc agcaacatct
29521 tctcaggatt cttctcttgt tttgtggcca ccactgctct ttcccgcacg gccgtccagg
29581 agagcactgg aggaaagaca caggtaggaa caacagcctt atgatatcca tctcagagaa
29641 caagtcgagg aatggcaaca gaggaaggct cgcaccgagc ttagcaggac aatttgcctt
29701 tcagacttgt acttcctaat ctgattcacc tcaggcctat tcctcttgtt ccactccctc
29761 acctgaaatc tcttaaaaaa caacatgtat ggttttctga tacagtgatt ctcaaatcta
29821 tttgtcagtg cttacctgtc atgtagctac acttacctgc tgtggtcaat aacaactgac
29881 aaggaatcag taatgaagga tgctgatttt tgttaatttg ttacctgggg agacagatgg
29941 cctggtaaaa gcgcttcctg gctttaggag ctattctatt ttcaggaaag tgaaaagctc
30001 tattcacatt tcctttagaa ggatcaggaa cttcctccag gggcgtttgg tcccacagaa
30061 caacagtttg ctagccaaga atatttttgt tgtaacattt ttggcatttc ttcctgagta
30121 agatttatgt tgttgaagtt ctgcatgtgg gtttaatctt atcttttaaa tgtggtttgc
30181 agttgttgcc tttacaaggt ggccaaaaag ccagcatcct agttaatctc tggataatcc
30241 ctccttcagt gttcagaaag ctcaggagcc atactcaaag gccactcttt ccagcaggac
30301 acagtcgaag agaccacacc aagcagatgg gcacaagaga cagactcagg aattttgctt
30361 ctgtctctta tgccttgagg tccttatctt cccaacacag aaagaactat tatttaggag
30421 taagaagtgc attgagactc caaagaacaa caaacccaaa ctacatagtg caatacacac
30481 acacacacac acacacacac acacacacat gcacacacac atgcacagtc ttcacagtct
30541 tcaaatgggt tttactaagg cggggacact taaaaaataa atataagtcg ggcacagtgg
30601 ctcatgcttg taatcccagc actttgggag gctgaggtgg gcggatcaat tgaggtcagg
30661 agttcgagac taacatggcc aacatggtga aaccccttct ctactaaaaa tgcaaaaatt
30721 agccaggtgt ggtggtagat gcctgtaatc ccagctactt gggaggctga ggcaggagaa
30781 tcacttgaac ctgggaggtg gaggttgcag tgagccaata tcgtgccact acattccagc
30841 ctgggcgaca agagctagac tctgtgtcaa atatatatat acacacacac acacacacac
30901 acacacacac aaacatatat atatatatat attctcatat atatatacga gaataactga
30961 aaaaactaat gactccaaca gtgacttgcc cagggagggc tttaattgtc cccaatatgc
31021 atgtgatgct cctttcctat ttataatgga tcttgtgaca gacttggaag aattggcctc
31081 ttccagtttt ttggctgctt ttcaacaaaa aattttattt tcagaatttt tcttaatttt
31141 ttattttttt tgcaaatatt tccagctctg agggagcagg taaaataata cagctcatat
31201 ccggaggcat agccagagat ctgtggtcaa ggggaagagg tcagagccaa tttccaaagc
31261 tgtgcacatt tcaggctgca gctgtcagca gcaggaactt ctgctgcttt catttgagac

31321 ggagcactga caagctctcc agagcacatg gtcttcagcg ggtgcaggca aatctcaaac
31381 tgctgatgtc cattctcctg atgttacctc caggctcaaa tgaggcatgg gctggctgtc
31441 ccagtaagta tgcccatgta aagtgacctc cttggcacag ggtttggggg ataatggtgc
31501 tctggtaagc taataccccc tgccacatac tggcatgtat tatatttagg aatgaaggat
31561 gccaggattt cagcattaaa gaatgaaacg agtaaagaaa tggcttcatg tttaattatt
31621 gaaatgtata ctaaattctg gggttaaaag tctaagtgcc tgaactgaaa tccagggact
31681 gatggacaaa taaactggca gggcttctct tgtgtggctt ctcgtgtttt ctcttatgtt
31741 ggaacttcag tttcagcatc tacaaaatgg aaatgataat tgcatccatt tcacagggat
31801 attgtgagga ttaggcaaga aaatgtaaag ccttagcaca tacttgtaaa tgctcattgt
31861 ttcactcctt taggcaagaa tagcatctta gcatctatga tacatatatt gtgcctggca
31921 cataagaagc acctggcata tatttgctga atgaatgaat ggaagaatga gtattggtgg
31981 acacgaactg tttttagcac atctacagat tgtagaagaa ttaaacattg gaacctttc
32041 tttccctcac ccccatctga cttccactga ctcacagaaa agtaattctg attacaaacc
32101 actctttagc tctgattacc ccctccctta acagaataat tttttagtaa ctgaatctgg
32161 ttgtagatat aaagtctaca gagtttctca caaacaagcc ttatcaagta agtagaaata
32221 attgatcact caccaatttt aatatagtgg ccaaaatgag ccttggcctc ccataattgg
32281 agactcatta tgacatataa ttggcaagta ggtggccact ctcctggcat gagtttggca
32341 gctgactgtg gtccttgtcc ccaaatctcc atatctttct ccagttcaca gagctcccca
32401 tccacagtga gatgcaagtg gctcggtaaa tactggttga gtaattgcat gcaaggaagg
32461 aagggaggga taagggaagg aaagaaagaa gaaaatggaa ggaaggacaa gggagaggaa
32521 gggaagggat atgaaggaaa gcgaagagaa gggagggaag taggatatca gcttctctgc
32581 ttagtcagga gaagggaaag agcaccaggg tactgggaat ctaggatggg gataggaaga
32641 gcatagaaaa gccagccaga catacaaagc acgcttttta gtacctcata cacatcatat
32701 tgcctcaggc tgacccaact ataatctaaa tataaatgat caatttgcca ggtagagcaa
32761 gtgctttcta attttctgtt tatatttgct gtgtctgtct cccgtaactc agaccttttg
32821 cactagacag tacttgtgct caacaaaatc ttcccagttg tttcctctct gattgctttg
32881 cttgtagttc cctgtctcct tttcttcttt gtttcagagc ttctaatctt catcttggct
32941 ctgccccagc tgtctcaaca gagaattcat tggtcatcta atcaaacgag agagtagtcc
33001 ccaccccctc agcctctctc ctaactagta tggatcttag gagagctatt tttgccagcg
33061 aaggtaaagt ttctgaagct tccttttacc cctgctattg ccccgttact ccatagtcac
33121 tgtttcaggc tgccttgggg caaagctctg agcctgcctc cgtggaactg tcagaggagg
33181 caggagacac aatcccacct ctcaatgtgc aaatgtggca tttcgaattt gcaataggat
33241 gagtaaattt attttctctg agactttaat gagaccatgt gctacaagta cgaagtgtta
33301 tcagagttgc tattatagct gcagcttcaa taatgcagaa aaatcagcta tgtgccatga
33361 gaatttattt cccaagaata cgacaatgat ctctgttgtt gggaaagggg ggattggtcc
33421 atgtttcctg ccatggtaaa taactagaag cttggcctga atggacgccg aaaccgcagg
33481 tgtgtactat caccaaataa aatccctgtc aaactggctt taatttcata ggatcagtcc
33541 ccatattttc tttaacttct ctgccatagc atatatagat gccatttttg ttcagttttg

33601 tggcttgagc aaataactat cacttttctc gacagtattg agcagaaggg ggagacaggg
33661 aagtatgaag tgtgtctgtg aacaggctgt ctcatacaca catccagtga gctggaagac
33721 acaagggaga aggacgaatc cttttcatag gaggtgtgtg tcttccaggt tgctggcatc
33781 atctctgctg cgattgtgat gatcgccatt cttgccctgg ggaagcttct ggaacccttg
33841 cagaaggtat aaccctgctt ctctgcatac cgattgcata atttcccttc actactctgc
33901 taccagataa ataacaggag atttaacaat catcacatgg aaaaccattc cctgaataac
33961 acagccttct ctgtctctct tggcagtcgg tcttggcagc tgttgtaatt gccaacctga
34021 aagggatgtt tatgcagctg tgtgacattc ctcgtctgtg gagacagaat aagattgatg
34081 ctgtaagtca cctaccacct atatttatct gaaataagat ttggttctta tatgcttcct
34141 gccatatcac tatattcccc ccatccccta agtctcactt gtgctttggg aactccagag
34201 gagaaattag aattgtgggg ataaatcaaa gccatgaagt cttccatgtg aactgcattt
34261 tgtgaagatt ctgtatctct gggaaggtca atttccagca agaatcccat atgaggctca
34321 ttcatacacc tttggccttc taaacacaaa aagcaaaaaa atgtttaaac accctagttt
34381 agaaggacaa acaacataca aaacaatgca aaaacaaaat acaaaatgta aaaacccacg
34441 ctgactttaa ctctgactaa cataaccttt gcaactattc tgagaaacta aaaatcagaa
34501 gctaaatcct tcagagttcc tctctaattt aaaccataaa aagaatgaga agacttccta
34561 catattgata tggaaaattt tccaagacaa atcgttaagt gaaaaagaga ccagaataga
34621 aatgctgggg aaaagcacag acacaggcag aaataaaagc ggatacaagg agggagggaa
34681 gttgggaatg ggtggagaag ggggagggtt gtgagcaaga gttttcccct ttttaatgat
34741 aattccctta tgaaccatac aaatatatta gctattcaaa aatttagaaa taaataaaat
34801 gggacagttg gtcttaattg agaagcccag tttctacagc tacttaaata taaaatgaat
34861 gaagtcacaa tatttttaaa aagctgtgat ttggccaggc acagtggctc aggcctgtaa
34921 tcccagcaat ttgggaggca ggaggatcac ttgagctcag gaatttgaga ccagcctgcg
34981 taacacagtg agaccttgtc tctactacaa ataaaagatt aaaaattagc cgggtttggt
35041 ggtatgtact tgtgggtagt cccagctact tgggaggctg aggcgggagg atcacttgaa
35101 tccagaagat ggaggctgca gtgaactatc atggtgccac tgcactccag cctgggcaat
35161 agagtgtgac cctatctcaa aagaaaaaaa aaaatgtaat ttgtttgtgg atcattgatc
35221 ttatttttat aggtagttat cacatgatgg tacctgatac attaatataa ttcttttcat
35281 ttctattttt ttccctaggt tatctgggtg tttacgtgta tagtgtccat cattctgggg
35341 ctggatctcg gtttactagc tggccttata tttggactgt tgactgtggt cctgagagtt
35401 cagttgtgag taacgtaaaa cccagatttc ctataaacag aacaacacac tctgagcttc
35461 cttataccat tttgataaat atagtgaagc cactttcttt cgttatagtt actgtatatt
35521 gagtgcttct atgcattaag cagacagtgt tttacagaca tacttaatct tcaaaaatag
35581 cctatgaata gttttgatta gtctcattct acaggtgaga aaagagaaat tcagaaaagc
35641 taagaagcat ccctaagatc acacagctag tacgtggcag agctaagatt tgaacctatg
35701 tacctactca tgtcctctac tgctgtgctc tccttttagt tgtggtaaga ctaaaaagta
35761 catagtttag ccaaaggctg aagttgcttt gtagtttctt ctttaaatga gatgaggaat
35821 gtaaggcgct cggccccagg cttgggacaa gacagctgtt atatattata gttcattttc

35881 ttttttaaag ctagtaacca aaagttacat aacccagaac aggaggactt taaaatagct
35941 gatgacagcc aaaattcatt caaatctctc atataaccaa taaggcatat aaccaataaa
36001 ccacacaatt atgtgaagga aacaattagc tcatatgcag agtgaaggca gcaaagacat
36061 gatttaaata atggaaaaca gcctgtgagg gaaggtgagg agcctgaagg tggtttttg
36121 gttggttttg ttttgattct agagaggcct tagttaagtg ccctcctggg cctagagggg
36181 aacagagtct ttctctccct cctcaatgtg tccatcgtct ttccagactt ctggcccctg
36241 ctctgcctgt gcttgggccg tgaaagaaga agacacctgg aaaaacatca agatagatgt
36301 cttcagctta gcatccgctc acctctcctg tcccaccagc cagttctcct tcctgccctc
36361 cttccctacc ttggcatatt ggtgcctacc caaaaggctt gtgttttcta cctcagcatc
36421 ctaaatgatt gcccaagggc ctgagaaggt ttgcaggctc ccaaagttct caatttgctt
36481 ctaggacacg tctcagaaac gctccttaga agatgctgtc tctcatgctg tatatgcctc
36541 agtgtctatt ttttcttgtg tggatgtggt tggaaaagaa gggttttaca gagagagatt
36601 agcttataaa cttttaagtt aaaaatcagc acaaacctgt aagccaagga ataactggtg
36661 gcacatttta gctgctagtt tggtatctag gcttttagcc aacaaaaagc agtatcctcc
36721 acagtattta atttccagta attatcatgc tgatccagag agcacaatgc tgaaagtaca
36781 ctaaacagat actctttctt caggaattgt atttagcatg tctctagtca cacatgaaga
36841 gtcacggaat ttatctgatt gcatcaggaa caagaaatca atagtctggt tgacaggaag
36901 gacttatttc tggtcctcca gtctcctatt tctataaaaa atgactaagt cacaaagaag
36961 cgccaggcac tggaagaccc aggggttaaa gacagaggct ctgctgccct ttacctccag
37021 gccttttctc acttctctta agaagtttag ttaacaataa aaatcaggat aatactgatg
37081 acctttcagg gttatggcag ggcttaaata agatttcaca tgcgtgaaag ctctatgctt
37141 atcctataac gtgttgtgca cgtgcaagta tgatgctcat tatttctctc agccatcaga
37201 agagaggcac agttctcccc tcccctgccg attccacaca aacaccagct gttcatttca
37261 gagttagcta caggaaaatg tcatctgcaa taaagacaga gtccaaaaca ccagaatgat
37321 gggctcttta gtagctgttg tttttaactt tttattccaa aatacggctg ttccaaaaaa
37381 tcttgacctt gatatttttt cttctagtcc ttcttggaat ggccttggaa gcatccctag
37441 cacagatatc tacaaaagta ccaagaatta caaaaacgta agtacctttg tgagacattt
37501 gctggacttg ggtttactag cctgaagttt cagcagctcc attttacgta caaggtagcc
37561 aaagggagaa aatgcctatt gggaaagtct gttagtccac agggagtgtc atgaaaactt
37621 ttgatccagt gcaccttctg acacccatgg cttatgtgaa ttttgtctat gctagctgaa
37681 tgtctttttt tttttttctt tttagatgga gtctcactct tcacccaggc tggagtgcag
37741 aggcaggatc tcagctcact gcaacctctg cctcctgggt tcaagtgatt ctcttgtctc
37801 agcctccgag tggctgggat tacaggcagg tgccaccacg cctggctaat ttttgtattt
37861 ttagtagaga tggggttgca ccatgttgcc caggctggtc tcaaactcct gagctcaagt
37921 gatttgcccg tcttggcctc ccaaagagtt gggattacag gcgtgagcca ctgcacctgg
37981 ctagctgaat gtcattttaa tataaaactg ttgtaagagg aatttaaaaa taataggcaa
38041 tttctagatg ttcatccaaa attttgttta tctggtatat aattcttatc ttctgtaaga
38101 ttttctttat tcagttctaa caagagatta tcaagttctc tatgaccaag accttattat

38161 ttgacacttt gctttatcct tggtattaga agaaataaaa tatgacacag gaaatgaaaa
38221 acaaagaaat taaacatccc attgatggac ttgtgtcacc tgcaaataaa attgcttagt
38281 ggtgactagg ggaaacaaag ataaaaaagc agagaaagta aatcaagaaa atacaaattt
38341 agcaaataat aataataata atatcaaccc taggaagttg tagtctaatt ttaccttaac
38401 attaaaaaaa gttggacctt gaaactggga tttcagtagc tgctacttaa tttatttccg
38461 tgatctttct gttatgttga gccaagtgcc attaagagct aaaatgattt agagattttt
38521 ttggctttgg ggagggagct ctaagctgag ttagggaatt ctgacttcta ggctccatct
38581 tggtgagact ttgcccaagt cattcagcct ctcaggacct cagttttctc atctgaagca
38641 gctagactaa aattgctgat tcctgaagac tagaaaagtc tatgattctg tgattcctgg
38701 gttctatttt gggtgtggcc attgtatgtc agggtgagaa cagatgatat cataggcctc
38761 agttctgcaa atcccattga ttttgctttt tttctttgct acttttaaag aatgttagca
38821 aaatcaggag ggtcagtggt atgaacttca ggcaaatcat ggcactgttc taagcctcag
38881 tttcttttct ggaaatcaga gggttgaata aattgctttc taaggccctt tctaactttg
38941 accttctatg agactgtaga ggtccaagag tttttgtaac ctgttgtctc cctattcaag
39001 aaaagttgta gaggccgggc acggtggttc acacctgtaa tcccagcact ttgggaggct
39061 gaggcgggtg aatcacttga ggtcaggagt ttgagaccaa cctggccaat gtggcaaaac
39121 tctgtctcta ctaaaaatgg aaaaattagc tgggtgtggt ggcagcacct gtaatcccaa
39181 ctacttggga ggctgaggca ggagaatccc ttgaacccag gaggtggagg ttgcagtgag
39241 ccgacattgt gccactgtac tccagcctgc gcaacagagt gaaactccat ctcaaaaaaa
39301 aaaaaaaaag aaaagaaaga aaagttgagt gctgctaccc agctcctctg agcaactgtg
39361 acttgactcc ttgctaagta gccagaaatg taattaaata cttgaggctt gaaattattt
39421 aatcccagac aatttctttt aatgccagat tgaagaacct caaggagtga agattcttag
39481 attttccagt cctattttct atggcaatgt cgatggtttt aaaaaatgta tcaagtccac
39541 agtaagtatt ttatccctag aaatttgttt tctaacctct tttgagactt cattcattct
39601 acaagtattt actggggtcc aatcaggaat aggccctaga ccctcttccc tttgtgtagg
39661 gcaatgagaa ttaaaatata acatccttgc cttcaaataa tttacagtct atttggggat
39721 taaaaaaaca catatgttaa aacccaggta gtaataacta tgccagacaa aaacattaga
39781 gatgtttcta ggcagaattt gagtaagttt caaataagta gtatagacag ttgagagttc
39841 atagaaggaa gagatccagg ttagttggac taacaggctt tgtgcggcag ttggctgaag
39901 gaaagaaatc atgtcaagtg tttgagaact atgtgagctg agagacagtg gaaacatgta
39961 ttggaggtag ggttgcccag ttgcagcaaa aagtaaaata tactagtgga ataaataagt
40021 agaaaatcct ggcaccaaat gccatgctat gctttgaact tggagtttgc ctctaaatca
40081 gtgtttttct aagtgagttc cagggcaaac tgattcagag gaatgttcac agatttgca
40141 ataacaaaag ggctctgtga taccaaggga atttggaaaa cccagactta aactaaagag
40201 ggtattttgg ggtgtgtgtg tgtttctgtt ttgtctttta ctgtcttgga gcctttgata
40261 tgctactgtc ttctcagagc ttctgggtag gagtagggta gcctgggagt agacagagat
40321 ctactccatc agaccttaca atttcttttt tggcaggata gctcaaggaa ttataccctt
40381 tgagaaatag cctttccaga taacagttgc cattaataag ctttaggtgc caggcatttt

40441 aagtaacttg acatttattt ccaaaggttg gatttgatgc cattagagta tataataaga
40501 ggctgaaagc gctgaggaaa atacagaaac taataaaaag tggacaatta agagcaacaa
40561 aggtgagatg acatctttct tttccccctt aaattatttc ctttccctga tgagagcagt
40621 tagagggtct aaaattaaat ctatcctctt tagtatccag atgtgaatga acaaatgaca
40681 tgtacgtatc aaagaacaac tgagctattc ttataggcaa gcgggagtgg aagggaatga
40741 aataaaatca gcagcgctgc ctggaaacac aggatgtgat ttttttcccc accatgaaca
40801 gtgtctgcat tattttctat actttatttt tactgaaagg aacaaaataa gtgcaaattc
40861 aatgccagaa aactacccac catagaaggc agtctaagtc cataaacttt tggagctgtt
40921 aacttttact cagattcttc tggccagtgt atttcttggc aaagttccac aatcatccag
40981 aaaacaaaag tttcctggct cctctgttct cccagttttc ttcctagaca acatcaaagt
41041 ttgggctgag gtgaaaccca tccttaaaaa ttcatctcct tgatgtcttg cttaccaagg
41101 aacagtgtgt aggtcttttg gataatttga tatgaatggt tgaaagattt caaatctttg
41161 acaattaagt tgacagtgtt ttcttcgttt agaatggcat cataagtgat gctgtttcaa
41221 caaataatgc ttttgagcct gatgaggata ttgaagatct ggaggaactt gatatcccaa
41281 ccaaggaaat agagattcaa gtggattgga actctgagct tccagtcaaa gtgaacgttc
41341 ccaaagtgcc aatccatagc cttgtgcttg actgtggagc tatatctttc ctggacgttg
41401 ttggagtgag atcactgcgg gtggtaaggt tctggttttc tgaattatac atttggagct
41461 ttggcaatag taaaatgatg tgggttgtcc agtattgcaa cagggcaaat acatgggctt
41521 tgtaattttt ctaggtgaat gcttttgtaa aaaagtgtaa tattttaaag cataggctct
41581 ggagccagac tacctggggg agatactggc ttcaccactt actagcagta cgaccctggg
41641 caagttgctt aatctgtcta tatctcagtt tcttcatctg taatatggag gtaatgatgg
41701 tatctacctt cacaggttgt tacaaggatt aaataagcta atagatataa ggtgtttaga
41761 agagtgtctg gttcaggctg ggcatggtgg ctcacgcctg taatcccagc acttcgggag
41821 gctgaggcag gtggatcatg aggtcaggag ttcaagacca gcctggccaa tatggtgaaa
41881 ccccgtctct accaaaaata caaaaattag ctgggcatgg tggcgcacac ctgtagtccc
41941 agctcctagg aggctgtggc aggagaatcg cttgaacccg ggaggtggag gttgcagctg
42001 agattgtgcc actgtactcc agcctgggtg acagagtgag acttcatctc aaaaaaaaaa
42061 aaaaaaaaga atatctggtt caaagatact ccccagcaaa ttaattcaca tttattatgt
42121 tttatctttc tgagaatgta taacaagtgg tatatgaaaa gaaaaaaatg ggcaaaagtt
42181 tattaagtat tacatttcta tttgttatgt taacagcaac aggataagga ataccaggtg
42241 tatgttagga ctggaaaagc caggcattat taagaggtta gagtaggaag caggtcagac
42301 atctggaagg tcaaagcaag agttgaggag tatgcaggat agaaacatta atgatatcag
42361 aaaccagtta tgcaagctga agttcattaa ttctgtcaac aaatagatgt gaaatgccta
42421 atgtgtactg agcactctgc caggcacaag agactagtga tgaagtagac acagtttcta
42481 cctacgtggg acttagcagt ctggaggaga aggcaaacat taagtcatct tataaataac
42541 aattgtagta agtgctccaa agaagatgaa taggacatca cctgaagcat caagttcagt
42601 gaagcttcag gtaggtatca aaagggtcag gcagaaaagt aagcaggtca agacaccaac
42661 aatcagatgt aataatataa agctgactcc taactgaggt tccacttagc taaggcttct

42721 ttgctacctc tataagggga ggaaatgatt ccaggatgtg ggtactaggt aggtagggta
42781 gagaagtgat tgtgcttggc aaaagagtat gagaatatgc tgacaggttc tgttctagtt
42841 ctcttagcag attttgttat tagctcattt gaaatcactc ttgttctttg ttgtacattt
42901 cactctaagc attggctgag atccattgta gactatattg gccagagagt tcaatgaatt
42961 acttttgggg acagtcttaa catatttaat gccagaatat ttcccacaca acgaatattt
43021 actttcaaat attataatga cagttatgat agctaaagaa gactccaaac tttatacttg
43081 aacagaggtc ttgatttgtt ttcctccata attctcaaat tctcttttat tattgactat
43141 gggtcttgtg tcttgacttt ttaaaattaa atgtttattt tgacataatt gtaaattcac
43201 atgtagttgt aaggaataat atttagatcc tatatacccc ttacccagtt tctcccaatg
43261 agtaacatct tgtaaaactg tagcataaaa tcacagtagg acactgacat tcaaaataga
43321 ggccttgact tttaggtcaa tagtaacatt ttgaacccga taagcatagc cacacccata
43381 tctcttctcc aatcagcagg gcctatatta agaagcctcc tgtactctct tctttccaca
43441 aatgcttatt tagcacctcc acgctatcaa gtgctgtgct attgagcgct ggatgttgcc
43501 atctcttgag atataatcct aattatacca ttactgtagt ttgaaaacct ccatggtttt
43561 gcaataataa cctttcctta aagtcctgat taaccatgaa agtaataatg tttctcctga
43621 gcaagtaact gaatgctact gaattatggg cagataaggt tgttaattgt tacaaactct
43681 ccttttttat ttttagattg tcaaagaatt ccaaagaatt gatgtgaatg tgtattttgc
43741 atcacttcaa ggtaaataca tatatctaca tatctacctg taagactttc ccgtaagccc
43801 tttctcctat ctgggactgt ggtcacatta tgtctgaagg cctttttttt ttttctttta
43861 aagatctcaa ttgtcattat ttgcagttct ggaatctggc actgcttcat tccataaaac
43921 agaataagtg ttccaaggaa ctaagcagga gttcagtatt atacacagaa aaggccaaag
43981 acagcaaaag caagcaacaa agagtatatt agtccattca aagttacttt cttgtaagtc
44041 agagacagtg agacagaaca ataggaaaat aatggattag ttaacatgag gtcatgtcag
44101 gatacttttt tgtatgagga ttattgcaga ggaagcttta ttattatgcc aactggaaat
44161 ttaaactgtc ctgttttagg aaatttgcta ttacctctct ctcctgattt cttcaagtat
44221 cagataacaa tttagtttag gtttgataac ttgaaacttt agcatgagtg actccatttt
44281 gatttttagt cttgtctgtt gtcgtctagt gcgggagctt agtctaaaac aatggccttt
44341 tatttttatt ttttatttta ctatgtgcct gtgccttgca tgaccccagg atgggcacag
44401 ctgcttggcc cctgagccac cgaaggctgc tctactcctt cgttctggct actaaaagct
44461 gcaaagctct gggagggcaa agctcaagaa gactaaaaca atggccttta ccatttttgt
44521 ttgatgattt tccccgtttt gatcaggctc tcacctaggt aagagtgaac aaaatgtagg
44581 agatcagtgc tactctgtta ccatcatttt gggtttctgg tctcaggagg tcatgtgtag
44641 cttatggtgc cctcctcatt atcatgtatg tctctgagtt tttgttgttt cagagagaga
44701 ccattcgatg tctgacaggt ggctttttgg aaacatttaa aactttgaga gggtataatg
44761 taccaggaag actgctatta tgactatcag ggagataata ccaagggttt atagtatgct
44821 ccttagccag gattctcatg aatcaaacca actaaaattg aatagcctga caaggagact
44881 acctgtttta accaagaagt cttctctgta gtacctgatg tatttatata tgtggaacaa
44941 gaagtgtcac ccaactgcac agatgcttcc ttgttgagtt agcaggtaat ctagcattcc

45001 atgactggtt aaattaaagc agaaagtgta agttacccaa agaagctact cattgtgaag
45061 ttttaactac agcactatcc tgccaagtga aagaggtagg cacaagtaag ggaaaattaa
45121 aagggataag catcttatga tagggagtct tgttctgaca gtcttggagc tgtctacagc
45181 atgaagttga caacttcttg ttttggtttg tagttttatt gtctctagtt gtggcatcca
45241 gcattctggc gaactctcta tgtggcccac agtttaagca tgagattcat ctcttgaaat
45301 ttacactgag ttgctcagct tcagcttaca gagcttcagg aactgagtag ttcttggtct
45361 tcattggagt gttgtagcca gatattagtg aaagctaaaa gaattaagaa tccagctcag
45421 tctacaggta gataataaaa actcataaat attgaacagg gctacagtct aagaacagtt
45481 ttactatgct tttcttttga accatatgtt tttctttcta tagtcacctc catttctatc
45541 aaagacgatc atggtagacc aatttgttta caaaataagt ttggtctcaa acttggcctg
45601 attgattatt tacacagtac agcaagaata actacatagg tgactccttt caaatttgct
45661 ttgccaagcc tggcgaggta ttgcttgcct gtgagcccag ctacttggga ggctgaggtg
45721 agaggattgc ttgagcccag gaattcaagg ctgcagtgca ctatgattgt gcctgtgaat
45781 agccactgca ctatagcctg gacaacacag tgagaccatg tctccaaaaa ataattgctt
45841 tgatagaaat tttgacaagg aatctcagat tggacttttt aaaacgtctt gatgctatga
45901 agtcaaacca aggcagacat taggctttgc ctgatgtatc taatatcttg aagttactgg
45961 gcctcccagg aaggaacgac tttttattca ctcattgtaa ggctagcagc ccttgaagcc
46021 aggaattctg tgcacatttt caaatatgat attctattca aagccttgat aatataacca
46081 atgttttcca attgtattct atttaaaaga acagattcta ttgaactttc atgtaaataa
46141 tcatattgcc ataaaaataa gaatactcac aaagagtttc caaattctgg aaggatcagg
46201 tagagaggaa aagcaaatgt ttcaattttt gtttatgaaa gtatgcttaa caaggctggg
46261 tgcggtggct cacatctgta atcccagcac tttgggaagc caaggcggac aattgctttg
46321 agctcagagt ttgagaccag cctggcaaca tggcaaaacg ccgttactac aaaaaataca
46381 aaaaattagc tgggcatgga gcctgaggct gagaatcact tgagctgggg aagtggaggt
46441 agcagtgagc ccagatggtg ccactgcact ccaacctggg tgacagagtg agaccctgtc
46501 tcaaaaaata aaaaaaaagt atgcttaacc aagtggctgt aaactgcaga tagctttaaa
46561 gaaaaatttt ctttagatct ggaaaacaaa tattaaaaga accagcaatg tttcaaataa
46621 aaaagccata aaacctgtaa ttcttctcca tcagttcatt cagtctcatg taattaattc
46681 ttgctctgtt tgatcttggc tggtagttta attccaacga atggtatgaa ttcaaagtta
46741 ttagaaacct gtatttgtca gagttctttt cattcttccc atataactct ttgaagtcac
46801 agcactttag aattataatg gcttacaaag agctttcaga aaaagtatca gaacaaaaca
46861 attaactgtg gacaacaaga cttaaagtgg ctatatttaa agatctgatg tgagttaccc
46921 aattgacaag gatatttgga tatttctgtg gcacacaaca atttaaaata accaaaatta
46981 tgactggtag catttatacc aagacctatc acatttctag gaatgttata taattttgga
47041 acatattaat aacatatcta taaaaatata gcacaaagaa agttaaacat catttcttat
47101 tttaacagtg cttcccatat aatttaacct atcagataag gccatttggt ttaacatctc
47161 tcttttacag attctttaag aaattccagg gtcctctgga acatcccaaa gttagttcaa
47221 ggtcaaaaag acttaatttt gatttttgag aagtttgtca aataccaaag gtttaaaaca

47281 tttgatcaaa atcggatcat aggtcactat gaaataaaac caaagtgaaa aaagagttca
47341 aaggcaaaaa gcacaagaag agttatattg atgaaacatg aaatctctgt tttctaggcc
47401 agttacctgg aagagaaaat cctctcacaa ttttctatta agagtaaacc aatcctctga
47461 gaaaactcta ttgttccaac acataggccc acactttagc cttccatcag tgtactttaa
47521 tattaatgct caatttttag aaaaacttat aaataattcc cttctacttt tagccaactc
47581 aatcacataa aatttttcat gatatttatc ttctacaaac cttctacaac ttgcttaaac
47641 cttcatttgg tcctatactt ccttttttaa aattggcatt gtaccttagg acaaagattt
47701 actttctttt tctccttatc attttgacca tataaggtta tctcctatac aaaagaaaaa
47761 attactctct tttcaatttt ctttatctct tcatacttgt aaatttcttc tcacatcttt
47821 cctacctagt ggttccttcc tgccttgttt tgatttcctt cataagtcca tatttagaaa
47881 caacctttaa ataacctctg attgccaagc tagagttaag ttttaaataa ataaataggc
47941 cggtcatggc ggctcacatc tgtaatccca gcactttggg aggcaaaggt cagaggatca
48001 cttcaggcca ggagtttgag accagcctga gcaacatggt gagaccccgt ctctattatt
48061 ttttttttaa aaaacaaaaa gcataaaaac tctgagaaca ttttgaaccc agaaagatat
48121 tacttccaat ttgtgccaca gagctggtaa tgtatggaaa tgtgtatctt gacagtgggt
48181 gggggtgggg tgaatattga caatgggggg atgttgttat gaggagagca aagctggaga
48241 ttacttagat tccttagaac aggaggactc tcaagggtgt gagtctctgt gtgtttcttt
48301 atgaatgtat gtgtgtagga aagtgctaag gcttaggttg aattctttag cagcagagcc
48361 caagatgggg attcttgcac aagcgattta ttgagaatac atctgaggaa atttatgaag
48421 cagtgagggg agcaggatag ggaaggggaa gaagctgatc aaagacatgg tttctgaagt
48481 ccagcgtaga tccctgattt cacagggagc accaccacag gaagagtacc agaaggttat
48541 gcctccttga ggcaagagga cctgctttct cccccagtaa tgagtcagtc agtggctgca
48601 ggccagccat gtgtggggat gaaacctccc agttctcttt atttacttat ttatttgaga
48661 cagggtcttg ctctgtcacc caggctggag tgcagtggtg caactgtggc tcactgcagc
48721 ctcaaactcc tgggctcaag tgatcctccc atctcagcct cctgagtagc tgagactata
48781 ggtgcacaac cacacctggc taatttttgt atttttttat aaagatgggg tttcaccata
48841 ttgcccagaa gttctgggtt caaagtgatc tgccctcctc ggcctctcaa agtgctggaa
48901 ttacagaccc agtcctcttt agatgacaca gctcccaacg ggcaagggaa agtctccaga
48961 gaagggcaca gctgtgaaac attggcagcc tggcccacag cagcaggagg atggatacac
49021 tggcttggcc aaagggacgt gatcgtccac aaggttgact acgaccagtt atgggataac
49081 cattctatat actactggaa aagaaactgt catttcaaat ctgggtcaca ttttggataa
49141 gagaaaaata aataaataaa aggaggagct acaaaaactt agtataggtt gatgctttaa
49201 aatttctttt cttagctggg catggtaggg tgtgccctgt agtcctagct aattgggagg
49261 gtgaggtggg gggatcactt gaacttggga cgcggaggtt gcagtgagca atgatgccac
49321 tgcactccag cctgggcaat agaatgagac tctgtctcaa aaacaaacaa aaatttcttt
49381 tcctaggaac taacaaaaca ttgtgtcttt cttttgaaga ttatgtgata gaaaagctgg
49441 agcaatgcgg gttctttgac gacaacatta gaaaggacac attctttttg acggtccatg
49501 atgctatact ctatctacag aaccaagtga aatctcaaga gggtcaaggt tccattttag

49561 aaacggtaaa tattcaacct ttctacagat gtatcttttc taaactatca tgatttctat
49621 aaatggcaaa cattacacaa gtctagtcta gctgttgaat tttaagctac ctatataact
49681 tcatggagcc tcagtttttt catcagtaaa atggaagtaa aaacattaac cttgctaggt
49741 agatatgaag actaaataag atagtttata ggaaattacc tggtagtacc cagtactgaa
49801 tagttctaca atgtgtaggt ttattaataa aagtgggcat tagctataat gccttttaag
49861 aatattgaat cttggatctt ttgtgatctg ggatttgtgt acataaattc cacttaaatt
49921 ctcagcaatc tggaaaacct tgaattataa atgtctttaa acaggatcca ccaggccata
49981 agtgatcttt gaaaaagaaa tttaaaaatc aagccacaaa gaagcccagg gttaattttt
50041 ccctacaaaa tagaataggc cctgatggga ggcctcatgg cacaacagta gcgcatctgg
50101 ctccagaata ggccctgatt tcccatgggt attattttgt taatttttct gtcaaatacc
50161 cagaagaggt gagcattctg gtctggttca ttgggcttgg cctaatggtg ctcctggtgg
50221 tacaagaatc tcatttctca tagtgaattt aggaaactaa agcaaatacc tatcaagata
50281 taataacagt aatgattcca gtgacatcgt ggatatttta aattgcttcc catttttcta
50341 aaagtagaat aagtagatcc ttattgtaga ttggcagttg attgaagaaa actaacttag
50401 gaaatgatat tatattattc tttttaggca ctggtagtgg agagattgtt ctatttgatg
50461 ttccttggta aaatactagt cctaactacc aagctgtgga tcttaaatat aaaaaaggaa
50521 aaaaaaaaaa gagtaaagct acaaatctaa agaaaaaaaa actgtttatc attgaaaatt
50581 cataaacttt ttctatccaa cttaaaattc tctaacccat aggtgataag aaagttggat
50641 catatgaagt atcctttaag agatccacat taaaagtaaa acagaaccaa ggtatcaagt
50701 tctttctcaa catgaaagca gttattttat ttgtttatac ccattcttac taagatttta
50761 ggctatgaat gtcataaaaa ccactaacca agagtgtatc tactaggtta gactcactct
50821 tgtttgcaat cagagaggta ttgtgagtgg ctgggatgga tttttttgga ccttaaacaa
50881 atagactaag cataacggaa tgcttgtatt acatacttac tggtccacta aaagtgggtt
50941 ggacattcct ggtggcctgt gagatcaaga ggcttatatt tctgtgtctt ctcagagtct
51001 agcacaaagc tttatatgtg ataagtaggt gaatgatgat aaactccttc aagagggagt
51061 ccctctgtat caaagatatt gtgactgcaa tgacacaacc ttcttcctat ccctttattt
51121 taccatcaca ggactatctc aggacacaac ttgtagttag acttatcctc tacctttccc
51181 attatgtcat accatttgca cagggactta accctctaat tcattctcat actagctaag
51241 agaattgggc tatttgtgag ttgaaaagta gctaagtagc atttcaaaat gttattttag
51301 cctggatata tatttggagt tgctttgaaa atgtcctttt ccatgcaaaa cacaaagcca
51361 aaattgtcgt tggtttcctc tgtgtagaag attaaattac atgccacctc taaacagtag
51421 agcttttctg aataaccaac ttggtccata gacattggtt tccatctcca atagaattaa
51481 tttccaccca attccatttg tggctgtttt ttgtcatcag tgacaagctc ttcactgtgc
51541 attcattgca cactcaacgc tgtgctaagt gctcttagct tagccattga gagatgcact
51601 attgactgct gaatcattta ggcagagggg gtgacttgtt aagaggcata cactcaagag
51661 gttggggaag agagctgaaa aggagtttac acaatggaga actataccag ttcacctttc
51721 aatgtgcaaa aaaatgatat acaaaaaatt ttagttggga aatataaaag aacaatacag
51781 ctgaagagga ttctgaagta tgtaaagaca gatgagaagc accaggaaag cttcaaatca

51841 ttttcagtgg agcatcaggt gggttgatgc tattctattt ctaccctgtg ttctcttttt
51901 caagatcact ctcattcagg attgtaaaga tacccttgaa ttaatagaaa cagagctgac
51961 ggaagaagaa cttgatgtcc aggatgaggt atgatcattt tcttctgaag aaaatatttg
52021 aattacattt tgaataatta gagtaataca aatagtgaat atatctgatt aagaactgtc
52081 agggaacata attcccccaa atgcagaaaa gatggcttca tagcagggaa aaagagaaaa
52141 taagaattct cccttgaaga cattaagtac ttggggatct tttgtcccag gcatttgggg
52201 atgatcagca actggcaaga gccaacacac tgctcctgat ttgataccta cacctctgca
52261 tactgctctt ctagagatgt aaggccatgg ggtaggaaaa aaatgaatag aggaaacttc
52321 taagctagga actttataag cctcaggaat agtatgtgag gaacagaaac cctatgacct
52381 tccttcatat gactgataaa ggctttaaaa acacactctc cccaccctag actgattctg
52441 tgtatttcag atgctcttgt gacatcagac tattcctagg aaacttgtct gaacttgtag
52501 catctgagta aattttgcag cttcgccact ggataagagg tgaacactga aggaagatca
52561 cagaattgta aaaagttgaa agatttctta gaatcaagta gtctaacatt ctagcccatg
52621 cagaaatcct tcctacacca tccttgatga atgttcattc agcttttatg tgaacatttc
52681 cagacattgg ccactagcgg cttctcaagg gagtttgtta catggttgga agtctctaaa
52741 tgttacttgt gcttacagaa ctcaatttgg cctttctgta gtaaccacct agtgaactga
52801 gatctgcctt ccttagcaac acagaccttc ccactgaaca gcccttcaaa tatttgagaa
52861 tagttctcat gtctccagca gactctcttc cagttgagac tcttctagtt ccttctattc
52921 cttacaaaca agcagtcagt gcttctctgg tcacgatggg gacacaaaac cctaaatgcg
52981 gccaggccag catggagatt attcaaccat gacctacctt gatctcatca agatattcct
53041 attactacag cttaaaaatg tgtttccttt cataatagca acatcagaat tttagcttct
53101 attgagcagg ggccaactaa agactggggc attttcatac aagctatggt ctagtagagt
53161 tccctagtta tatttattta attgagcttt gaccctaata cagaactaca cctatttctg
53221 ctggatttca tcttgttggt tcttgtttac tgacataatt ttgtattatt attagtatta
53281 ttattattat tattatttgt gactgagtct tactctgtca cctggactgg agtgcagtgg
53341 tacaatctca gctcactgca atccctccct cccaggttca agagattctc acgtgtgcct
53401 cagcttccca agtagttggg attacaggcg ctcgccacca cgtccagata attttttttt
53461 tttgtattct tagtagagac agggttttgc catgttgtcc aggctggtct tgaactcctg
53521 acctcaagta atccacctgc cttgccctcc cagattgctg ggattacagg catgagccac
53581 cacattggcc agttttgtgt tttgaatcta acatccagga tattgtctac cccagtaccc
53641 gttacctggt gtcataatca aatctgataa gcctgccttc tctgcctttt tctaggtctt
53701 gaaaaaatgt tgcaaagaaa acattcaacc ccctatagtg aaatcaggca gtaaggatga
53761 caggcagaga tttctgtgga ggaaatacat tgctagagtg aaaatcacag tacctgcatc
53821 ttggcccacc agcttagtga gcccaggaaa gtcattatcg acctgagaag tcctttagc
53881 tccagtagtt tatgactcta tggatatagg gtgccttagt ttgagtaaaa actcatatgt
53941 caacattaaa acttaagact gctatattat aggatattta ttggttgcca cgtaaaataa
54001 tcattcaggg agggtttcta tagatggtaa aacaatatag cacaatagtt tcaagggcat
54061 ggactttaga acaagacaga cctgggtgct agtcctcatt ctgctactta ctggctgtgc

54121 accccagggc aaattactta tcctgtcttc atattagtat cttcatgtaa agtagtaata
54181 ataccacaga gttgtgaaaa ttaaaggata aatgtaaagt aacaagagca gtgccttgta
54241 catagtaagt gctcaataaa tagtaatcat tgctcttaaa taaatccaat taaagtgtga
54301 tgaatacagg ccaggctcag tgactcaagc ctgtaatccc agcactttgg gaggctgagg
54361 cagggggatc acttgaggtc aggagtttga gaccagcctg actaacatgg taaccccccg
54421 accatctcta ctaaaaaaaa aaaaaaaaaa attagctggg tgtggtggca ggcacctgta
54481 atcccagcta ctcgggaggc tgaggcagga gaattgcttg aacctgggag gcagaggttg
54541 cagtgagctg agatcacgcc acagcactcc agcctgggca acagtgagtg agattcagtc
54601 tccaaaaaaa aaaaaaagta taatgaatac gaaatgaagt tttaccctta tttctattgt
54661 gatgatatac acctaagatg agtagcagta agcaatcaat actataaaaa catatttata
54721 aaaaagataa tgcagactta aggagaattc agttgtatca acactttgtt ttccccttgc
54781 ttccacaggc tatgcgtaca cttgcatcct gaaagtgggt tcgggaggtc tctatgagca
54841 aggaatacaa gacaaaactt cctcaatgca ttgactattt cttcagactc aaaacactca
54901 ttcttttttc tattaagcca ttgaaagaga agcactaaga ctgcttctag gctttattta
54961 taaaataaac accttatccc taacatgggc aaaatggcta gaattattca gacgatttgg
55021 cagcgtccag ggtaagctgg tgttataata cgctgctgat ctacatcaca gatttgctaa
55081 taatgttcac gtgggccctg gcatatctct gttcagttag agtgagtgct gacccaacag
55141 cctctgtggt caagcgagtc acgaatgatt aatcataaag aaaaatcagt tttgactga
55201 cctggatatc catgagctgc actgatcacc atgtaaggtc acatttagta aatgctgaaa
55261 taaaatgatt aatgcattta tcaataaaag cctttgaaaa tactttggat aataaattgg
55321 agttttaaaa atgcaaattt gcttagtatc taataatgaa gtgttattac atatagccgg
55381 aattgaggat ctctttgatc ctggaaatgg tttacctaaa agctacagaa ccaggccaat
55441 atattttgaa atattgatgc agacaaatga aataataaag agattttcat ggtttataaa
55501 aatctttttt gatatgataa taatcatgat cacaactgag atcaaaaaaa tatatgacag
55561 attattttgt ttaaaaatgc agttttaatt atcttagtct atagaaatga tcattgcatg
55621 gaggcatgta taggtatgat ctgtgtaaaa tctgacataa aaacagtgct attctgagtg
55681 aaaatttttt tgatgtgctt acataaccat ggtgattaaa atgagtttat atttttctc
55741 aaaaatttta gcagtgtgta aagtaagtaa tctttaactg aactctgacc acttaaaaaa
55801 aaatctaaaa attgaactac ctatagtagt ctgtgtttaa agtgaatttt taaagacaaa
55861 gcattctaaa tgaactcaat ataaaaacat tcatttggaa tgtacatact gaaaaataca
55921 ggtttttttg accaaaagtt tttatatctt ttctttttat ttattttttt cctaagtgcc
55981 aacaattttc tagatattat atacaacaca ggctttgatc ttggggactt ttcccatata
56041 tttcacactg gagtgaatga agttgtactt catttctaga gaaaagttat acccaggtcc
56101 ccaattgaga atgtcttgct tgattgaaaa cgacatcatc ccttggtata ctccagggat
56161 tggtttcagg acccctgcat ttaccaaaat ttgtgcacac tcaagtcctg cagtcacccc
56221 tgcctaaaga tagaatggct tctctgtttt tcttctgaaa tacaaccaga aacaatgtgt
56281 ctatttctga aagaatagga ttaatgatca tacaaatggg ttaatcctga attctggttg
56341 taaatctggt tacagcataa ctaggattat aatgctgcct cattttcaca gcactacttg

56401 cttatattga caacaaatca tctcgctaaa gagtgaatgt aggccaggcg cggtggctca
56461 tgcctgtaat cccagcactt tgggaggccg aggcgggtgg atcacgaggt caggagatcg
56521 agaccatcct ggctaacatg gtaaaacccc gtctctacta aaaatagaaa aaaagaaatt
56581 agcctagcgt ggtggctggc gggcgcctgt agtcccagct atttgggagg ctaaggcagg
56641 agaatggcgt gaacccggga ggcggagctt gcagtgagcc gaggtcgtgc cactgcactc
56701 cagcctgggc gacagagcaa gactccgtct caaaaaaaaa aaaaaaaaaa aaaaaagagt
56761 gaatgtaata gtcttgcaga aaatgaatga atacctttgt tcaataaagg aaatatgcac
56821 tgctcacttt tttgaaggaa atgccaaagt tacgttttac aacaaggcta gagtttgtaa
56881 attctgggtt catttgtgat gacataagtc agcaaactgc gggaatactg tctcttctat
56941 gtattttgtg aatagtaagc ataattttag ttttgtatta tcaatgaaaa tttcacttga
57001 aattaaagct gccttttgtt atatttttaa cctataggat aagattccag tattgtatat
57061 gagttttaac aaattaaaaa atcaaatcat gtacatttga aaatatttgc acacatttaa
57121 aaataaatgt aaagttgtct tttaaactac tcggatgtgt cctttctgaa caaaa

### Otoferlin

To date, several hundred mutations in the human *OTOF* gene have been identified to cause profound prelingual deafness DFNB9. Such mutations are the cause of deafness in 2-8% of people that are born with an autosomal, recessively inherited, non-syndromic deafness in different populations (Rodriguez-Ballesteros et al. (2008) Hum. Mutat. 29 823-831; Choi et al. (2009) Clinical Genetics 75 237-243; Duman et al. (2011) Genet Test Mol Biomarkers 15 29-33; Varga et al. (2006) J Med Genet 43 576-581; Iwasa et al. (2013) BMC Med. Genet. 14 95). For example, two substitutions in exon 15 at positions 490 and 515 in the conserved C2C domain of otoferlin cause DFNB9 (Mirqhomizadeh et al. (2002) Neurobiol. Dis. 10(2): 157-164). Migliosi et al. found a novel mutation Q829X in OTOF in Spanish patients with prelingual non-syndromic hearing loss (Migliosi et al. (2002) J. Med. Genet. 39(7): 502-506).

Additional exemplary mutations in a otoferlin gene detected in subjects having hearing loss and methods of sequencing a nucleic acid encoding otoferlin are described in, e.g., Rodriguez-Ballesteros et al. (2003) Hum Mutat. 22: 451-456; Wang et al. (2010) BMC Med Genet. 11:79; Yildirim-Baylan et al. (2014) Int. J. Pediatr. Otorhinolaryngol 78: 950-953; Choi et al. (2009) Clin. Genet. 75(3): 237-243; and Marlin et al. (2010) Biochem Biophys Res Commun 394: 737-742.

Otoacoustic emissions from DFNB9 patients are normal, at least for the first decade of life, indicating morphological integrity of the inner ear and proper function of outer hair cells. Apart from the lack of synaptic transmission and the subsequent loss of synapses, the morphology and physiology of the inner ear remains preserved in DFNB9, at least during the first decade in life in humans.

Studies in mouse models revealed that synapses are structurally normal, and IHCs preserve normal synapse numbers in mice within the first postnatal week. Between P6 and P15, about half of the synapses get lost (Roux et al. (2006) Cell 127 277-289). Animal models allowed studying the effect of mutations in otoferlin on synaptic transmission by recording the change in plasma membrane capacitance after vesicle fusion and the activity in the auditory nerve. In otoferlin knock-out (Otof-/-) mice, almost no exocytosis could be triggered in IHCs by depolarization induced Ca2+ influx through voltage gated Ca2+ channels (Roux et al. (2006) Cell 127 277-289). In profoundly hearing impaired pachanga (Otof^{Pga/Pga}) mice with a random point mutation in the C2F domain, short (<10ms) depolarizations of the IHCs elicited vesicle fusion of similar size as in wild type mice, however sustained stimulations uncovered a strong deficiency in replenishing vesicles to the readily releasable pool (Pangrsic et al. (2010) Nat. Neurosci. 13 869-876). The p.Ile515Thr mutation, found in human patients with only mildly elevated hearing thresholds but a severe reduction in speech understanding and a temperature-dependent deafening (Varga et al. (2006) J Med Genet 43 576-581), uncovered an intermediate phenotype when studied in a mouse model (Strenzke et al. (2016) EMBO J. 35:2519-2535). These OtofI515T/I515T mice showed a moderate elevation of hearing thresholds when assessed by ABR with a reduction in wave I amplitude, but normal auditory thresholds in behavioral tests and recordings of single auditory nerve units. Exocytosis of the RRP is again intact, but sustained exocytosis is reduced, although not as severe as in OtofPga/Pga. While in wild type mice at room temperature, during a sustained stimulus 750 vesicles can fuse per second at each active zone, this rate drops to 350 vesicles/s/active zone in OtofI515T/I515T mice and to 200 vesicles/s/active zone in OtofPga/Pga IHCs (Pangrsic et al. (2010) Nat. Neurosci. 13 869-876; Strenzke et al. (2016) EMBO J. 35 2519-2535). This correlated with lower otoferlin protein levels at the plasma membrane of IHCs, indicating that the amount of otoferlin scales with exocytosis and hearing (Strenzke et al. (2016) EMBO J. 35 2519-2535).

Methods of detecting mutations in a gene are well-known in the art. Non-limiting examples of such techniques include: real-time polymerase chain reaction (RT-PCR), PCR, sequencing, Southern blotting, and Northern blotting.

The OTOF gene encodes otoferlin, a protein that is involved in synaptic vesicle exocytosis in cochlear hair cells (see, e.g., Johnson and Chapman (2010) J. Cell Biol. 191(1):187-198; and Heidrych et al. (2008) Hum. Mol. Genet. 17:3814-3821).

The human OTOF gene is located on chromosome 2p23.3. It contains 48 exons encompassing ~ 132 kilobases (kb) (NCBI Accession No. NG009937.1). The mRNA encoding the long-form of otoferlin expressed in the brain includes 48 exons (Yasunaga et al., Am. J. Hum. Genet. 67:591-600, 2000). Forward and reverse primers that can be used to amplify each of the 48 exons in the OTOF gene are described in Table 2 of Yasunaga et al., Am. J. Hum. Genet. 67:591-600, 2000. In some examples, the full-length OTOF protein is a full-length wildtype OTOF protein. The full-length wildtype OTOF protein expressed from the human OTOF gene is 1997 residues in length.

An exemplary human wildtype otoferlin protein is or includes the sequence of any one of SEQ ID NOs: 8-12. Isoform e of human otoferlin protein (SEQ ID NO: 12) is encoded by an mRNA that includes exon 48 and does not include exon 47 of the otoferlin gene (Yasunaga et al., Am. J. Hum. Genet. 67:591-600, 2000). In some embodiments, the active otoferlin protein has the sequence of SEQ ID NO: 12, but is missing the 20 amino acids including the RXR motif identified in Strenzke et al., EMBO J. 35(23):2499-2615, 2016). Non-limiting examples of nucleic acids encoding a wildtype otoferlin protein are or include any one of SEQ ID NO: 13-17. As can be appreciated in the art, at least some or all of the codons in SEQ ID NO: 13-17 can be codon-optimized to allow for optimal expression in a non-human mammal or in a human. Orthologs of human otoferlin proteins are known in the art.
**Human canonical (long) isoform sequence (otoferlin protein)** (SEQ ID NO: 8) (also called otoferlin isoform a) (NCBI Accession No. AAD26117.1) 
MIKTEKSYPERRLRGVLEELSCGCCRFLSLADKDQGHSSRTRLDRERLKSCMRELENMGQQARMLRAQVKR
HTVRDKLRLCQNFLQKLRFLADEPQHSIPDIFIWMMSNNKRVAYARVPSKDLLFSIVEEETGKDCAKVKTL
FLKLPGKRGFGSAGWTVQAKVELYLWLGLSKQRKEFLCGLPCGFQEVKAAQGLGLHAFPPVSLVYTKKQAF
QLRAHMYQARSLFAADSSGLSDPFARVFFINQSQCTEVLNETLCPTWDQMLVFDNLELYGEAHELRDDPPI
 
IVIEIYDQDSMGKADFMGRTFAKPLVKMADEAYCPPRFPPQLEYYQIYRGNATAGDPLAAFELLQIGPAGK
ADLPPINGPVDVDRGPIMPVPMGIRPVLSKYRVEVLFWGLRDLKRVNLAQVDRPRVDIECAGKGVQSSLIH
NYKKNPNFNTLVKWFEVDLPENELLHPPLNIRVVDCRAFGRYTLVGSHAVSSLRRFIYRPPDRSAPSWNTT
GEVVVTMEPEVPIKKLETMVKLDATSEAVVKVDVAEEEKEKKKKKKGTAEEPEEEEPDESMLDWWSKYFAS
IDTMKEQLRQQEPSGIDLEEKEEVDNTEGLKGSMKGKEKARAAKEEKKKKTQSSGSGQGSEAPEKKKPKID
ELKVYPKELESEFDNFEDWLHTFNLLRGKTGDDEDGSTEEERIVGRFKGSLCVYKVPLPEDVSREAGYDST
YGMFQGIPSNDPINVLVRVYVVRATDLHPADINGKADPYIAIRLGKTDIRDKENYISKQLNPVFGKSFDIE
ASFPMESMLTVAVYDWDLVGTDDLIGETKIDLENRFYSKHRATCGIAQTYSTHGYNIWRDPMKPSQILTRL
CKDGKVDGPHFGPPGRVKVANRVFTGPSEIEDENGQRKPTDEHVALLALRHWEDIPRAGCRLVPEHVETRP
LLNPDKPGIEQGRLELWVDMFPMDMPAPGTPLDISPRKPKKYELRVIIWNTDEVVLEDDDFFTGEKSSDIF
VRGWLKGQQEDKQDTDVHYHSLTGEGNFNWRYLFPFDYLAAEEKIVISKKESMFSWDETEYKIPARLTLQI
WDADHFSADDFLGAIELDLNRFPRGAKTAKQCTMEMATGEVDVPLVSIFKQKRVKGWWPLLARNENDEFEL
TGKVEAELHLLTAEEAEKNPVGLARNEPDPLEKPNRPDTSFIWFLNPLKSARYFLWHTYRWLLLKLLLLL
LLLLLALFLYSVPGYLVKKILGA
**Human Isoform 2 (short 1) (otoferlin protein)** (SEQ ID NO: 9) (also called otoferlin isoform d) (NCBI Accession No. NP_919304.1) 
MIKTEKSYPERRLRGVLEELSCGCCRFLSLADKDQGHSSRTRLDRERLKSCMRELENMGQQARMLRAQVKR
HTVRDKLRLCQNFLQKLRFLADEPQHSIPDIFIWMMSNNKRVAYARVPSKDLLFSIVEEETGKDCAKVKTL
FLKLPGKRGFGSAGWTVQAKVELYLWLGLSKQRKEFLCGLPCGFQEVKAAQGLGLHAFPPVSLVYTKKQAF
QLRAHMYQARSLFAADSSGLSDPFARVFFINQSQCTEVLNETLCPTWDQMLVFDNLELYGEAHELRDDPPI
IVIEIYDQDSMGKADFMGRTFAKPLVKMADEAYCPPRFPPQLEYYQIYRGNATAGDLLAAFELLQIGPAGK
ADLPPINGPVDVDRGPIMPVPMGIRPVLSKYRVEVLFWGLRDLKRVNLAQVDRPRVDIECAGKGVQSSLIH
NYKKNPNFNTLVKWFEVDLPENELLHPPLNIRVVDCRAFGRYTLVGSHAVSSLRRFIYRPPDRSAPSWNTT
GEVVVTMEPEVPIKKLETMVKLDATSEAVVKVDVAEEEKEKKKKKKGTAEEPEEEEPDESMLDWWSKYFAS
IDTMKEQLRQQEPSGIDLEEKEEVDNTEGLKGSMKGKEKARAAKEEKKKKTQSSGSGQGSEAPEKKKPKID
ELKVYPKELESEFDNFEDWLHTFNLLRGKTGDDEDGSTEEERIVGRFKGSLCVYKVPLPEDVSREAGYDST
YGMFQGIPSNDPINVLVRVYVVRATDLHPADINGKADPYIAIRLGKTDIRDKENYISKQLNPVFGKSFDIE
ASFPMESMLTVAVYDWDLVGTDDLIGETKIDLENRFYSKHRATCGIAQTYSTHGYNIWRDPMKPSQILTRL
CKDGKVDGPHFGPPGRVKVANRVFTGPSEIEDENGQRKPTDEHVALLALRHWEDIPRAGCRLVPEHVETRP
LLNPDKPGIEQGRLELWVDMFPMDMPAPGTPLDISPRKPKKYELRVIIWNTDEVVLEDDDFFTGEKSSDIF
VRGWLKGQQEDKQDTDVHYHSLTGEGNFNWRYLFPFDYLAAEEKIVISKKESMFSWDETEYKIPARLTLQI
WDADHFSADDFLGAIELDLNRFPRGAKTAKQCTMEMATGEVDVPLVSIFKQKRVKGWWPLLARNENDEFEL
TGKVEAELHLLTAEEAEKNPVGLARNEPDPLEKPNRPDTAFVWFLNPLKSIKYLICTRYKWLIIKIVLALL
GLLMLGLFLYSLPGYMVKKLLGA
**Human Isoform 3 (short 2) (otoferlin protein)** (SEQ ID NO: 10) (also called otoferlin isoform c) (NCBI Accession No. NP_919303.1) 
MMTDTQDGPSESSQIMRSLTPLINREEAFGEAGEAGLWPSITHTPDSQEEGLNDIQEMIKTEKSYPERRLR
GVLEELSCGCCRFLSLADKDQGHSSRTRLDRERLKSCMRELENMGQQARMLRAQVKRHTVRDKLRLCQNFL
QKLRFLADEPQHSIPDIFIWMMSNNKRVAYARVPSKDLLFSIVEEETGKDCAKVKTLFLKLPGKRGFGSAG
WTVQAKVELYLWLGLSKQRKEFLCGLPCGFQEVKAAQGLGLHAFPPVSLVYTKKQAFQLRAHMYQARSLFA
ADSSGLSDPFARVFFINQSQCTEVLNETLCPTWDQMLVFDNLELYGEAHELRDDPPIIVIEIYDQDSMGKA
DFMGRTFAKPLVKMADEAYCPPRFPPQLEYYQIYRGNATAGDLLAAFELLQIGPAGKADLPPINGPVDVDR
GPIMPVPMGIRPVLSKYRVEVLFWGLRDLKRVNLAQVDRPRVDIECAGKGVQSSLIHNYKKNPNFNTLVKW
FEVDLPENELLHPPLNIRVVDCRAFGRYTLVGSHAVSSLRRFIYRPPDRSAPSWNTTVRLLRRCRVLCNGG
SSSHSTGEVVVTMEPEVPIKKLETMVKLDATSEAVVKVDVAEEEKEKKKKKKGTAEEPEEEEPDESMLDWW
SKYFASIDTMKEQLRQQEPSGIDLEEKEEVDNTEGLKGSMKGKEKARAAKEEKKKKTQSSGSGQGSEAPEK
KKPKIDELKVYPKELESEFDNFEDWLHTFNLLRGKTGDDEDGSTEEERIVGRFKGSLCVYKVPLPEDVSRE
AGYDSTYGMFQGIPSNDPINVLVRVYVVRATDLHPADINGKADPYIAIRLGKTDIRDKENYISKQLNPVFG
KSFDIEASFPMESMLTVAVYDWDLVGTDDLIGETKIDLENRFYSKHRATCGIAQTYSTHGYNIWRDPMKPS
QILTRLCKDGKVDGPHFGPPGRVKVANRVFTGPSEIEDENGQRKPTDEHVALLALRHWEDIPRAGCRLVPE
HVETRPLLNPDKPGIEQGRLELWVDMFPMDMPAPGTPLDISPRKPKKYELRVIIWNTDEVVLEDDDFFTGE
KSSDIFVRGWLKGQQEDKQDTDVHYHSLTGEGNFNWRYLFPFDYLAAEEKIVISKKESMFSWDETEYKIPA
RLTLQIWDADHFSADDFLGAIELDLNRFPRGAKTAKQCTMEMATGEVDVPLVSIFKQKRVKGWWPLLARNE
NDEFELTGKVEAELHLLTAEEAEKNPVGLARNEPDPLEKPNRPDTSFIWFLNPLKSARYFLWHTYRWLLLK
LLLLLLLLLLLALFLYSVPGYLVKKILGA
**Human Isoform 4 (short 3) (otoferlin protein)** (SEQ ID NO: 11) (also called otoferlin isoform b) (NCBI Accession No. NP_004793.2) 
MIKTEKSYPERRLRGVLEELSCGCCRFLSLADKDQGHSSRTRLDRERLKSCMRELENMGQQARMLRAQVKR
HTVRDKLRLCQNFLQKLRFLADEPQHSIPDIFIWMMSNNKRVAYARVPSKDLLFSIVEEETGKDCAKVKTL
FLKLPGKRGFGSAGWTVQAKVELYLWLGLSKQRKEFLCGLPCGFQEVKAAQGLGLHAFPPVSLVYTKKQAF
QLRAHMYQARSLFAADSSGLSDPFARVFFINQSQCTEVLNETLCPTWDQMLVFDNLELYGEAHELRDDPPI
IVIEIYDQDSMGKADFMGRTFAKPLVKMADEAYCPPRFPPQLEYYQIYRGNATAGDLLAAFELLQIGPAGK
ADLPPINGPVDVDRGPIMPVPMGIRPVLSKYRVEVLFWGLRDLKRVNLAQVDRPRVDIECAGKGVQSSLIH
NYKKNPNFNTLVKWFEVDLPENELLHPPLNIRVVDCRAFGRYTLVGSHAVSSLRRFIYRPPDRSAPSWNTT
GEVVVTMEPEVPIKKLETMVKLDATSEAVVKVDVAEEEKEKKKKKKGTAEEPEEEEPDESMLDWWSKYFAS
IDTMKEQLRQQEPSGIDLEEKEEVDNTEGLKGSMKGKEKARAAKEEKKKKTQSSGSGQGSEAPEKKKPKID
ELKVYPKELESEFDNFEDWLHTFNLLRGKTGDDEDGSTEEERIVGRFKGSLCVYKVPLPEDVSREAGYDST
YGMFQGIPSNDPINVLVRVYVVRATDLHPADINGKADPYIAIRLGKTDIRDKENYISKQLNPVFGKSFDIE
ASFPMESMLTVAVYDWDLVGTDDLIGETKIDLENRFYSKHRATCGIAQTYSTHGYNIWRDPMKPSQILTRL
CKDGKVDGPHFGPPGRVKVANRVFTGPSEIEDENGQRKPTDEHVALLALRHWEDIPRAGCRLVPEHVETRP
 
LLNPDKPGIEQGRLELWVDMFPMDMPAPGTPLDISPRKPKKYELRVIIWNTDEVVLEDDDFFTGEKSSDIF
VRGWLKGQQEDKQDTDVHYHSLTGEGNFNWRYLFPFDYLAAEEKIVISKKESMFSWDETEYKIPARLTLQI
WDADHFSADDFLGAIELDLNRFPRGAKTAKQCTMEMATGEVDVPLVSIFKQKRVKGWWPLLARNENDEFEL
TGKVEAELHLLTAEEAEKNPVGLARNEPDPLEKPNRPDTSFIWFLNPLKSARYFLWHTYRWLLLKLLLLLL
LLLLLALFLYSVPGYLVKKILGA
**Human Isoform 5 (short 4) (otoferlin protein)** (SEQ ID NO: 12) (also called otoferlin isoform e) (NCBI Accession No. NP_001274418.1) 
MALLIHLKTVSELRGRGDRIAKVTFRGQSFYSRVLENCEDVADFDETFRWPVASSIDRNEMLEIQVFNYSK
VFSNKLIGTFRMVLQKVVEESHVEVTDTLIDDNNAIIKTSLCVEVRYQATDGTVGSWDDGDFLGDESLQEE
EKDSQETDGLLPGSRPSSRPPGEKSFRRAGRSVFSAMKLGKNRSHKEEPQRPDEPAVLEMEDLDHLAIRLG
DGLDPDSVSLASVTALTTNVSNKRSKPDIKMEPSAGRPMDYQVSITVIEARQLVGLNMDPVVCVEVGDDKK
YTSMKESTNCPYYNEYFVFDFHVSPDVMFDKIIKISVIHSKNLLRSGTLVGSFKMDVGTVYSQPEHQFHHK
WAILSDPDDISSGLKGYVKCDVAVVGKGDNIKTPHKANETDEDDIEGNLLLPEGVPPERQWARFYVKIYRA
EGLPRMNTSLMANVKKAFIGENKDLVDPYVQVFFAGQKGKTSVQKSSYEPLWNEQVVFTDLFPPLCKRMKV
QIRDSDKVNDVAIGTHFIDLRKISNDGDKGFLPTLGPAWVNMYGSTRNYTLLDEHQDLNEGLGEGVSFRAR
LLLGLAVEIVDTSNPELTSSTEVQVEQATPISESCAGKMEEFFLFGAFLEASMIDRRNGDKPITFEVTIGN
YGNEVDGLSRPQRPRPRKEPGDEEEVDLIQNASDDEAGDAGDLASVSSTPPMRPQVTDRNYFHLPYLERKP
CIYIKSWWPDQRRRLYNANIMDHIADKLEEGLNDIQEMIKTEKSYPERRLRGVLEELSCGCCRFLSLADKD
QGHSSRTRLDRERLKSCMRELENMGQQARMLRAQVKRHTVRDKLRLCQNFLQKLRFLADEPQHSIPDIFIW
MMSNNKRVAYARVPSKDLLFSIVEEETGKDCAKVKTLFLKLPGKRGFGSAGWTVQAKVELYLWLGLSKQRK
EFLCGLPCGFQEVKAAQGLGLHAFPPVSLVYTKKQAFQLRAHMYQARSLFAADSSGLSDPFARVFFINQSQ
CTEVLNETLCPTWDQMLVFDNLELYGEAHELRDDPPIIVIEIYDQDSMGKADFMGRTFAKPLVKMADEAYC
PPRFPPQLEYYQIYRGNATAGDLLAAFELLQIGPAGKADLPPINGPVDVDRGPIMPVPMGIRPVLSKYRVE
VLFWGLRDLKRVNLAQVDRPRVDIECAGKGVQSSLIHNYKKNPNFNTLVKWFEVDLPENELLHPPLNIRVV
DCRAFGRYTLVGSHAVSSLRRFIYRPPDRSAPSWNTTVRLLRRCRVLCNGGSSSHSTGEVVVTMEPEVPIK
KLETMVKLDATSEAVVKVDVAEEEKEKKKKKKGTAEEPEEEEPDESMLDWWSKYFASIDTMKEQLRQQEPS
GIDLEEKEEVDNTEGLKGSMKGKEKARAAKEEKKKKTQSSGSGQGSEAPEKKKPKIDELKVYPKELESEFD
NFEDWLHTFNLLRGKTGDDEDGSTEEERIVGRFKGSLCVYKVPLPEDVSREAGYDSTYGMFQGIPSNDPIN
VLVRVYVVRATDLHPADINGKADPYIAIRLGKTDIRDKENYISKQLNPVFGKSFDIEASFPMESMLTVAVY
DWDLVGTDDLIGETKIDLENRFYSKHRATCGIAQTYSTHGYNIWRDPMKPSQILTRLCKDGKVDGPHFGPP
GRVKVANRVFTGPSEIEDENGQRKPTDEHVALLALRHWEDIPRAGCRLVPEHVETRPLLNPDKPGIEQGRL
ELWVDMFPMDMPAPGTPLDISPRKPKKYELRVIIWNTDEVVLEDDDFFTGEKSSDIFVRGWLKGQQEDKQD
TDVHYHSLTGEGNFNWRYLFPFDYLAAEEKIVISKKESMFSWDETEYKIPARLTLQIWDADHFSADDFLGA
IELDLNRFPRGAKTAKQCTMEMATGEVDVPLVSIFKQKRVKGWWPLLARNENDEFELTGKVEAELHLLTAE
EAEKNPVGLARNEPDPLEKPNRPDTAFVWFLNPLKSIKYLICTRYKWLIIKIVLALLGLLMLGLFLYSLPG
YMVKKLLGA
**Complete cds (otoferlin cDNA)** (SEQ ID NO: 13) (encodes the protein of SEQ ID NO: 8) 
atga tcaaaacgga gaagtcctac cctgagcgtc gcctgcgggg cgtcctggag gagctgagct
gtggctgctg ccgcttcctc tccctcgctg acaaggacca gggccactca tcccgcacca
ggcttgaccg ggagcgcctc aagtcctgca tgagggagct ggaaaacatg gggcagcagg
ccaggatgct gcgggcccag gtgaagcggc acacggtgcg ggacaagctg aggctgtgcc
agaacttcct gcagaagctg cgcttcctgg cggacgagcc ccagcacagc attcccgaca
tcttcatctg gatgatgagc aacaacaagc gtgtcgccta tgcccgtgtg ccctccaagg
acctgctctt ctccatcgtg gaggaggaga ctggcaagga ctgcgccaag gtcaagacgc
tcttccttaa gctgccaggg aagcggggct tcggctcggc aggctggaca gtgcaggcca
aggtggagct gtacctgtgg ctgggcctca gcaaacagcg caaggagttc ctgtgcggcc
tgccctgtgg cttccaggag gtcaaggcag cccagggcct gggcctgcat gccttcccac
ccgtcagcct ggtctacacc aagaagcagg cgttccagct ccgagcgcac atgtaccagg
cccgcagcct ctttgccgcc gacagcagcg gactctcaga cccctttgcc cgcgtcttct
tcatcaatca gagtcagtgc acagaggtgc tgaatgagac cctgtgtccc acctgggacc
agatgctggt gttcgacaac ctggagctct atggtgaagc tcatgagctg agggacgatc
cgcccatcat tgtcattgaa atctatgacc aggattccat gggcaaagct gacttcatgg
gccggacctt cgccaaaccc ctggtgaaga tggcagacga ggcgtactgc ccaccccgct
tcccacctca gctcgagtac taccagatct accgtggcaa cgccacagct ggagacccgc
tggcggcctt cgagctgctg cagattggac cagcagggaa ggctgacctg ccccccatca
atggcccggt ggacgtggac cgaggtccca tcatgcccgt gcccatgggc atccggcccg
tgctcagcaa gtaccgagtg gaggtgctgt tctggggcct acgggaccta aagcgggtga
acctggccca ggtggaccgg ccacgggtgg acatcgagtg tgcagggaag ggggtgcagt
cgtccctgat ccacaattat aagaagaacc ccaacttcaa caccctcgtc aagtggtttg
aagtggacct cccagagaac gagctgctgc acccgccctt gaacatccgt gtggtggact
gccgggcctt cggtcgctac acactggtgg gctcccatgc cgtcagctcc ctgcgacgct
tcatctaccg gcccccagac cgctcggccc ccagctggaa caccacgggg gaggttgtgg
tgactatgga gccagaggta cccatcaaga aactggagac catggtgaag ctggacgcga
cttctgaagc tgttgtcaag gtggatgtgg ctgaggagga gaaggagaag aagaagaaga
agaagggcac tgcggaggag ccagaggagg aggagccaga cgagagcatg ctggactggt
ggtccaagta ctttgcctcc attgacacca tgaaggagca acttcgacaa caagagccct
ctggaattga cttggaggag aaggaggaag tggacaatac cgagggcctg aaggggtcaa
tgaagggcaa ggagaaggca agggctgcca aagaggagaa gaagaagaaa actcagagct
ctggctctgg ccaggggtcc gaggcccccg agaagaagaa acccaagatt gatgagctta
aggtataccc caaagagctg gagtccgagt ttgataactt tgaggactgg ctgcacactt
tcaacttgct tcggggcaag accggggatg atgaggatgg ctccaccgag gaggagcgca
ttgtgggacg cttcaagggc tccctctgcg tgtacaaagt gccactccca gaggacgtgt
cccgggaagc cggctacgac tccacctacg gcatgttcca gggcatcccg agcaatgacc
 
ccatcaatgt gctggtccga gtctatgtgg tccgggccac ggacctgcac cctgctgaca
tcaacggcaa agctgacccc tacatcgcca tccggctagg caagactgac atccgcgaca
aggagaacta catctccaag cagctcaacc ctgtctttgg gaagtccttt gacatcgagg
cctccttccc catggaatcc atgctgacgg tggctgtgta tgactgggac ctggtgggca
ctgatgacct cattggggaa accaagatcg acctggagaa ccgcttctac agcaagcacc
gcgccacctg cggcatcgcc cagacctact ccacacatgg ctacaatatc tggcgggacc
ccatgaagcc cagccagatc ctgacccgcc tctgcaaaga cggcaaagtg gacggccccc
actttgggcc ccctgggaga gtgaaggtgg ccaaccgcgt cttcactggg ccctctgaga
ttgaggacga gaacggtcag aggaagccca cagacgagca tgtggcgctg ttggccctga
ggcactggga ggacatcccc cgcgcaggct gccgcctggt gccagagcat gtggagacga
ggccgctgct caaccccgac aagccgggca tcgagcaggg ccgcctggag ctgtgggtgg
acatgttccc catggacatg ccagcccctg ggacgcctct ggacatctca cctcggaagc
ccaagaagta cgagctgcgg gtcatcatct ggaacacaga tgaggtggtc ttggaggacg
acgacttctt cacaggggag aagtccagtg acatcttcgt gagggggtgg ctgaagggcc
agcaggagga caagcaggac acagacgtcc actaccactc cctcactggc gagggcaact
tcaactggcg ctacctgttc cccttcgact acctggcggc ggaggagaag atcgtcatct
ccaagaagga gtccatgttc tcctgggacg agaccgagta caagatcccc gcgcggctca
ccctgcagat ctgggatgcg gaccacttct ccgctgacga cttcctgggg gccatcgagc
tggacctgaa ccggttcccg cggggcgcaa agacagccaa gcagtgcacc atggagatgg
ccaccgggga ggtggacgtg cccctcgtgt ccatcttcaa gcaaaagcgc gtcaaaggct
ggtggcccct cctggcccgc aatgagaacg atgagtttga gctcacgggc aaggtggagg
ctgagctgca tttactgaca gcagaggagg cagagaagaa cccagtgggc ctggcccgca
atgaacctga ccccctagag aaacccaacc ggcccgacac gagcttcatc tggttcctga
accctctcaa gtcggctcgc tacttcttgt ggcacacgta tcgctggctg ctcctcaaac
tgttgctgct cctgctgctg ctcctcctcc tcgccctgtt cctctactct gtgcctggct
acctggtcaa gaaaatcctc ggggcctga
**Human Otoferlin Transcript Variant 1** (SEQ ID NO: 14) (encodes the protein of SEQ ID NO: 8) 
atggccttgctca tccacctcaa gacagtctcg gagctgcggg gcaggggcga ccggatcgcc
aaagtgactt tccgagggca atccttctac tctcgggtcc tggagaactg tgaggatgtg
gctgactttg atgagacatt tcggtggccg gtggccagca gcatcgacag aaatgagatg
ctggagattc aggttttcaa ctacagcaaa gtcttcagca acaagctcat cgggaccttc
cgcatggtgc tgcagaaggt ggtagaggag agccatgtgg aggtgactga cacgctgatt
gatgacaaca atgctatcat caagaccagc ctgtgcgtgg aggtccggta tcaggccact
gacggcacag tgggctcctg ggacgatggg gacttcctgg gagatgagtc tcttcaagag
gaagagaagg acagccaaga gacggatgga ctgctcccag gctcccggcc cagctcccgg
 
cccccaggag agaagagctt ccggagagcc gggaggagcg tgttctccgc catgaagctc
ggcaaaaacc ggtctcacaa ggaggagccc caaagaccag atgaaccggc ggtgctggag
atggaagacc ttgaccatct ggccattcgg ctaggagatg gactggatcc cgactcggtg
tctctagcct cagtcacagc tctcaccact aatgtctcca acaagcgatc taagccagac
attaagatgg agccaagtgc tgggcggccc atggattacc aggtcagcat cacggtgatc
gaggcccggc agctggtggg cttgaacatg gaccctgtgg tgtgcgtgga ggtgggtgac
gacaagaagt acacatccat gaaggagtcc actaactgcc cctattacaa cgagtacttc
gtcttcgact tccatgtctc tccggatgtc atgtttgaca agatcatcaa gatttcggtg
attcactcca agaacctgct gcgcagtggc accctggtgg gctccttcaa aatggacgtg
ggaaccgtgt actcgcagcc agagcaccag ttccatcaca agtgggccat cctgtctgac
cccgatgaca tctcctcggg gctgaagggc tacgtgaagt gtgacgttgc cgtggtgggc
aaaggggaca acatcaagac gccccacaag gccaatgaga ccgacgaaga tgacattgag
gggaacttgc tgctccccga gggggtgccc cccgaacgcc agtgggcccg gttctatgtg
aaaatttacc gagcagaggg gctgccccgt atgaacacaa gcctcatggc caatgtaaag
aaggctttca tcggtgaaaa caaggacctc gtggacccct acgtgcaagt cttctttgct
ggccagaagg gcaagacttc agtgcagaag agcagctatg agcccctgtg gaatgagcag
gtcgtcttta cagacctctt ccccccactc tgcaaacgca tgaaggtgca gatccgagac
tcggacaagg tcaacgacgt ggccatcggc acccacttca ttgacctgcg caagatttct
aatgacggag acaaaggctt cctgcccaca ctgggcccag cctgggtgaa catgtacggc
tccacacgta actacacgct gctggatgag catcaggacc tgaacgaggg cctgggggag
ggtgtgtcct tccgggcccg gctcctgctg ggcctggctg tggagatcgt agacacctcc
aaccctgagc tcaccagctc cacagaggtg caggtggagc aggccacgcc catctcggag
agctgtgcag gtaaaatgga agaattcttt ctctttggag ccttcctgga ggcctcaatg
atcgaccgga gaaacggaga caagcccatc acctttgagg tcaccatagg caactatggg
aacgaagttg atggcctgtc ccggccccag cggcctcggc cccggaagga gccgggggat
gaggaagaag tagacctgat tcagaacgca agtgatgacg aggccggtga tgccggggac
ctggcctcag tctcctccac tccaccaatg cggccccagg tcaccgacag gaactacttc
catctgccct acctggagcg aaagccctgc atctacatca agagctggtg gccggaccag
cgccgccgcc tctacaatgc caacatcatg gaccacattg ccgacaagct ggaagaaggc
ctgaacgaca tacaggagat gatcaaaacg gagaagtcct accctgagcg tcgcctgcgg
ggcgtcctgg aggagctgag ctgtggctgc tgccgcttcc tctccctcgc tgacaaggac
cagggccact catcccgcac caggcttgac cgggagcgcc tcaagtcctg catgagggag
ctggaaaaca tggggcagca ggccaggatg ctgcgggccc aggtgaagcg gcacacggtg
cgggacaagc tgaggctgtg ccagaacttc ctgcagaagc tgcgcttcct ggcggacgag
ccccagcaca gcattcccga catcttcatc tggatgatga gcaacaacaa gcgtgtcgcc
tatgcccgtg tgccctccaa ggacctgctc ttctccatcg tggaggagga gactggcaag
gactgcgcca aggtcaagac gctcttcctt aagctgccag ggaagcgggg cttcggctcg
gcaggctgga cagtgcaggc caaggtggag ctgtacctgt ggctgggcct cagcaaacag
 
cgcaaggagt tcctgtgcgg cctgccctgt ggcttccagg aggtcaaggc agcccagggc
ctgggcctgc atgccttccc acccgtcagc ctggtctaca ccaagaagca ggcgttccag
ctccgagcgc acatgtacca ggcccgcagc ctctttgccg ccgacagcag cggactctca
gacccctttg cccgcgtctt cttcatcaat cagagtcagt gcacagaggt gctgaatgag
accctgtgtc ccacctggga ccagatgctg gtgttcgaca acctggagct ctatggtgaa
gctcatgagc tgagggacga tccgcccatc attgtcattg aaatctatga ccaggattcc
atgggcaaag ctgacttcat gggccggacc ttcgccaaac ccctggtgaa gatggcagac
gaggcgtact gcccaccccg cttcccacct cagctcgagt actaccagat ctaccgtggc
aacgccacag ctggagacct gctggcggcc ttcgagctgc tgcagattgg accagcaggg
aaggctgacc tgccccccat caatggcccg gtggacgtgg accgaggtcc catcatgccc
gtgcccatgg gcatccggcc cgtgctcagc aagtaccgag tggaggtgct gttctggggc
ctacgggacc taaagcgggt gaacctggcc caggtggacc ggccacgggt ggacatcgag
tgtgcaggga agggggtgca gtcgtccctg atccacaatt ataagaagaa ccccaacttc
aacaccctcg tcaagtggtt tgaagtggac ctcccagaga acgagctgct gcacccgccc
ttgaacatcc gtgtggtgga ctgccgggcc ttcggtcgct acacactggt gggctcccat
gccgtcagct ccctgcgacg cttcatctac cggcccccag accgctcggc ccccagctgg
aacaccacgg tcaggcttct ccggcgctgc cgtgtgctgt gcaatggggg ctcctcctct
cactccacag gggaggttgt ggtgactatg gagccagagg tacccatcaa gaaactggag
accatggtga agctggacgc gacttctgaa gctgttgtca aggtggatgt ggctgaggag
gagaaggaga agaagaagaa gaagaagggc actgcggagg agccagagga ggaggagcca
gacgagagca tgctggactg gtggtccaag tactttgcct ccattgacac catgaaggag
caacttcgac aacaagagcc ctctggaatt gacttggagg agaaggagga agtggacaat
accgagggcc tgaaggggtc aatgaagggc aaggagaagg caagggctgc caaagaggag
aagaagaaga aaactcagag ctctggctct ggccaggggt ccgaggcccc cgagaagaag
aaacccaaga ttgatgagct taaggtatac cccaaagagc tggagtccga gtttgataac
tttgaggact ggctgcacac tttcaacttg cttcggggca agaccgggga tgatgaggat
ggctccaccg aggaggagcg cattgtggga cgcttcaagg gctccctctg cgtgtacaaa
gtgccactcc cagaggacgt gtcccgggaa gccggctacg actccaccta cggcatgttc
cagggcatcc cgagcaatga ccccatcaat gtgctggtcc gagtctatgt ggtccgggcc
acggacctgc accctgctga catcaacggc aaagctgacc cctacatcgc catccggcta
ggcaagactg acatccgcga caaggagaac tacatctcca agcagctcaa ccctgtcttt
gggaagtcct ttgacatcga ggcctccttc cccatggaat ccatgctgac ggtggctgtg
tatgactggg acctggtggg cactgatgac ctcattgggg aaaccaagat cgacctggag
aaccgcttct acagcaagca ccgcgccacc tgcggcatcg cccagaccta ctccacacat
ggctacaata tctggcggga ccccatgaag cccagccaga tcctgacccg cctctgcaaa
gacggcaaag tggacggccc ccactttggg ccccctggga gagtgaaggt ggccaaccgc
gtcttcactg ggccctctga gattgaggac gagaacggtc agaggaagcc cacagacgag
catgtggcgc tgttggccct gaggcactgg gaggacatcc cccgcgcagg ctgccgcctg
 
gtgccagagc atgtggagac gaggccgctg ctcaaccccg acaagccggg catcgagcag
ggccgcctgg agctgtgggt ggacatgttc cccatggaca tgccagcccc tgggacgcct
ctggacatct cacctcggaa gcccaagaag tacgagctgc gggtcatcat ctggaacaca
gatgaggtgg tcttggagga cgacgacttc ttcacagggg agaagtccag tgacatcttc
gtgagggggt ggctgaaggg ccagcaggag gacaagcagg acacagacgt ccactaccac
tccctcactg gcgagggcaa cttcaactgg cgctacctgt tccccttcga ctacctggcg
gcggaggaga agatcgtcat ctccaagaag gagtccatgt tctcctggga cgagaccgag
tacaagatcc ccgcgcggct caccctgcag atctgggatg cggaccactt ctccgctgac
gacttcctgg gggccatcga gctggacctg aaccggttcc cgcggggcgc aaagacagcc
aagcagtgca ccatggagat ggccaccggg gaggtggacg tgcccctcgt gtccatcttc
aagcaaaagc gcgtcaaagg ctggtggccc ctcctggccc gcaatgagaa cgatgagttt
gagctcacgg gcaaggtgga ggctgagctg catttactga cagcagagga ggcagagaag
aacccagtgg gcctggcccg caatgaacct gaccccctag agaaacccaa ccggcccgac
acgagcttca tctggttcct gaaccctctc aagtcggctc gctacttctt gtggcacacg
tatcgctggc tgctcctcaa actgttgctg ctcctgctgc tgctcctcct cctcgccctg
ttcctctact ctgtgcctgg ctacctggtc aagaaaatcc tcggggcctg a
**Human Otoferlin Transcript Variant 2** (SEQ ID NO: 15) (encodes the protein of SEQ ID NO: 11) 
atgatcaaaacgga gaagtcctac cctgagcgtc gcctgcgggg cgtcctggag gagctgagct
gtggctgctg ccgcttcctc tccctcgctg acaaggacca gggccactca tcccgcacca
ggcttgaccg ggagcgcctc aagtcctgca tgagggagct ggaaaacatg gggcagcagg
ccaggatgct gcgggcccag gtgaagcggc acacggtgcg ggacaagctg aggctgtgcc
agaacttcct gcagaagctg cgcttcctgg cggacgagcc ccagcacagc attcccgaca
tcttcatctg gatgatgagc aacaacaagc gtgtcgccta tgcccgtgtg ccctccaagg
acctgctctt ctccatcgtg gaggaggaga ctggcaagga ctgcgccaag gtcaagacgc
tcttccttaa gctgccaggg aagcggggct tcggctcggc aggctggaca gtgcaggcca
aggtggagct gtacctgtgg ctgggcctca gcaaacagcg caaggagttc ctgtgcggcc
tgccctgtgg cttccaggag gtcaaggcag cccagggcct gggcctgcat gccttcccac
ccgtcagcct ggtctacacc aagaagcagg cgttccagct ccgagcgcac atgtaccagg
cccgcagcct ctttgccgcc gacagcagcg gactctcaga cccctttgcc cgcgtcttct
tcatcaatca gagtcagtgc acagaggtgc tgaatgagac cctgtgtccc acctgggacc
agatgctggt gttcgacaac ctggagctct atggtgaagc tcatgagctg agggacgatc
cgcccatcat tgtcattgaa atctatgacc aggattccat gggcaaagct gacttcatgg
gccggacctt cgccaaaccc ctggtgaaga tggcagacga ggcgtactgc ccaccccgct
tcccacctca gctcgagtac taccagatct accgtggcaa cgccacagct ggagacctgc
tggcggcctt cgagctgctg cagattggac cagcagggaa ggctgacctg ccccccatca
 
atggcccggt ggacgtggac cgaggtccca tcatgcccgt gcccatgggc atccggcccg
tgctcagcaa gtaccgagtg gaggtgctgt tctggggcct acgggaccta aagcgggtga
acctggccca ggtggaccgg ccacgggtgg acatcgagtg tgcagggaag ggggtgcagt
cgtccctgat ccacaattat aagaagaacc ccaacttcaa caccctcgtc aagtggtttg
aagtggacct cccagagaac gagctgctgc acccgccctt gaacatccgt gtggtggact
gccgggcctt cggtcgctac acactggtgg gctcccatgc cgtcagctcc ctgcgacgct
tcatctaccg gcccccagac cgctcggccc ccagctggaa caccacgggg gaggttgtgg
tgactatgga gccagaggta cccatcaaga aactggagac catggtgaag ctggacgcga
cttctgaagc tgttgtcaag gtggatgtgg ctgaggagga gaaggagaag aagaagaaga
agaagggcac tgcggaggag ccagaggagg aggagccaga cgagagcatg ctggactggt
ggtccaagta ctttgcctcc attgacacca tgaaggagca acttcgacaa caagagccct
ctggaattga cttggaggag aaggaggaag tggacaatac cgagggcctg aaggggtcaa
tgaagggcaa ggagaaggca agggctgcca aagaggagaa gaagaagaaa actcagagct
ctggctctgg ccaggggtcc gaggcccccg agaagaagaa acccaagatt gatgagctta
aggtataccc caaagagctg gagtccgagt ttgataactt tgaggactgg ctgcacactt
tcaacttgct tcggggcaag accggggatg atgaggatgg ctccaccgag gaggagcgca
ttgtgggacg cttcaagggc tccctctgcg tgtacaaagt gccactccca gaggacgtgt
cccgggaagc cggctacgac tccacctacg gcatgttcca gggcatcccg agcaatgacc
ccatcaatgt gctggtccga gtctatgtgg tccgggccac ggacctgcac cctgctgaca
tcaacggcaa agctgacccc tacatcgcca tccggctagg caagactgac atccgcgaca
aggagaacta catctccaag cagctcaacc ctgtctttgg gaagtccttt gacatcgagg
cctccttccc catggaatcc atgctgacgg tggctgtgta tgactgggac ctggtgggca
ctgatgacct cattggggaa accaagatcg acctggagaa ccgcttctac agcaagcacc
gcgccacctg cggcatcgcc cagacctact ccacacatgg ctacaatatc tggcgggacc
ccatgaagcc cagccagatc ctgacccgcc tctgcaaaga cggcaaagtg gacggccccc
actttggccc ccctgggaga gtgaaggtgg ccaaccgcgt cttcactggg ccctctgaga
ttgaggacga gaacggtcag aggaagccca cagacgagca tgtggcgctg ttggccctga
ggcactggga ggacatcccc cgcgcaggct gccgcctggt gccagagcat gtggagacga
ggccgctgct caaccccgac aagccgggca tcgagcaggg ccgcctggag ctgtgggtgg
acatgttccc catggacatg ccagcccctg ggacgcctct ggacatctca cctcggaagc
ccaagaagta cgagctgcgg gtcatcatct ggaacacaga tgaggtggtc ttggaggacg
acgacttctt cacaggggag aagtccagtg acatcttcgt gagggggtgg ctgaaggccc
agcaggagga caagcaggac acagacgtcc actaccactc cctcactggc gagggcaact
tcaactggcg ctacctgttc cccttcgact acctggcggc ggaggagaag atcgtcatct
ccaagaagga gtccatgttc tcctgggacg agaccgagta caagatcccc gcgcggctca
ccctgcagat ctgggatgcg gaccacttct ccgctgacga cttcctgggg gccatcgagc
tggacctgaa ccggttcccg cggggcgcaa agacagccaa gcagtgcacc atggagatgg
ccaccgggga ggtggacgtg cccctcgtgt ccatcttcaa gcaaaagcgc gtcaaaggct
 
ggtggcccct cctggcccgc aatgagaacg atgagtttga gctcacgggc aaggtggagg
ctgagctgca tttactgaca gcagaggagg cagagaagaa cccagtgggc ctggcccgca
atgaacctga ccccctagag aaacccaacc ggcccgacac gagcttcatc tggttcctga
accctctcaa gtcggctcgc tacttcttgt ggcacacgta tcgctggctg ctcctcaaac
tgttgctgct cctgctgctg ctcctcctcc tcgccctgtt cctctactct gtgcctggct
acctggtcaa gaaaatcctc ggggcctga
**Human Otoferlin Transcript Variant 3** (SEQ ID NO: 16) (encodes the protein of SEQ ID NO: 8) 
atgatgaccga tactcaggat ggccctagcg agagctccca gatcatgagg tccctcactc
ccctgatcaa cagggaggag gcatttgggg aggctgggga ggcggggctg tggcccagca
tcacccacac tcctgattca caggaagaag gcctgaacga catacaggag atgatcaaaa
cggagaagtc ctaccctgag cgtcgcctgc ggggcgtcct ggaggagctg agctgtggct
gctgccgctt cctctccctc gctgacaagg accagggcca ctcatcccgc accaggcttg
accgggagcg cctcaagtcc tgcatgaggg agctggaaaa catggggcag caggccagga
tgctgcgggc ccaggtgaag cggcacacgg tgcgggacaa gctgaggctg tgccagaact
tcctgcagaa gctgcgcttc ctggcggacg agccccagca cagcattccc gacatcttca
tctggatgat gagcaacaac aagcgtgtcg cctatgcccg tgtgccctcc aaggacctgc
tcttctccat cgtggaggag gagactggca aggactgcgc caaggtcaag acgctcttcc
ttaagctgcc agggaagcgg ggcttcggct cggcaggctg gacagtgcag gccaaggtgg
agctgtacct gtggctgggc ctcagcaaac agcgcaagga gttcctgtgc ggcctgccct
gtggcttcca ggaggtcaag gcagcccagg gcctgggcct gcatgccttc ccacccgtca
gcctggtcta caccaagaag caggcgttcc agctccgagc gcacatgtac caggcccgca
gcctctttgc cgccgacagc agcggactct cagacccctt tgcccgcgtc ttcttcatca
atcagagtca gtgcacagag gtgctgaatg agaccctgtg tcccacctgg gaccagatgc
tggtgttcga caacctggag ctctatggtg aagctcatga gctgagggac gatccgccca
tcattgtcat tgaaatctat gaccaggatt ccatgggcaa agctgacttc atgggccgga
ccttcgccaa acccctggtg aagatggcag acgaggcgta ctgcccaccc cgcttcccac
ctcagctcga gtactaccag atctaccgtg gcaacgccac agctggagac ctgctggcgg
ccttcgagct gctgcagatt ggaccagcag ggaaggctga cctgcccccc atcaatggcc
cggtggacgt ggaccgaggt cccatcatgc ccgtgcccat gggcatccgg cccgtgctca
gcaagtaccg agtggaggtg ctgttctggg gcctacggga cctaaagcgg gtgaacctgg
cccaggtgga ccggccacgg gtggacatcg agtgtgcagg gaagggggtg cagtcgtccc
tgatccacaa ttataagaag aaccccaact tcaacaccct cgtcaagtgg tttgaagtgg
acctcccaga gaacgagctg ctgcacccgc ccttgaacat ccgtgtggtg gactgccggg
ccttcggtcg ctacacactg gtgggctccc atgccgtcag ctccctgcga cgcttcatct
accggccccc agaccgctcg gcccccagct ggaacaccac ggtcaggctt ctccggcgct
 
gccgtgtgct gtgcaatggg ggctcctcct ctcactccac aggggaggtt gtggtgacta
tggagccaga ggtacccatc aagaaactgg agaccatggt gaagctggac gcgacttctg
aagctgttgt caaggtggat gtggctgagg aggagaagga gaagaagaag aagaagaagg
gcactgcgga ggagccagag gaggaggagc cagacgagag catgctggac tggtggtcca
agtactttgc ctccattgac accatgaagg agcaacttcg acaacaagag ccctctggaa
ttgacttgga ggagaaggag gaagtggaca ataccgaggg cctgaagggg tcaatgaagg
gcaaggagaa ggcaagggct gccaaagagg agaagaagaa gaaaactcag agctctggct
ctggccaggg gtccgaggcc cccgagaaga agaaacccaa gattgatgag cttaaggtat
accccaaaga gctggagtcc gagtttgata actttgagga ctggctgcac actttcaact
tgcttcgggg caagaccggg gatgatgagg atggctccac cgaggaggag cgcattgtgg
gacgcttcaa gggctccctc tgcgtgtaca aagtgccact cccagaggac gtgtcccggg
aagccggcta cgactccacc tacggcatgt tccagggcat cccgagcaat gaccccatca
atgtgctggt ccgagtctat gtggtccggg ccacggacct gcaccctgct gacatcaacg
gcaaagctga cccctacatc gccatccggc taggcaagac tgacatccgc gacaaggaga
actacatctc caagcagctc aaccctgtct ttgggaagtc ctttgacatc gaggcctcct
tccccatgga atccatgctg acggtggctg tgtatgactg ggacctggtg ggcactgatg
acctcattgg ggaaaccaag atcgacctgg agaaccgctt ctacagcaag caccgcgcca
cctgcggcat cgcccagacc tactccacac atggctacaa tatctggcgg gaccccatga
agcccagcca gatcctgacc cgcctctgca aagacggcaa agtggacggc ccccactttg
ggccccctgg gagagtgaag gtggccaacc gcgtcttcac tgggccctct gagattgagg
acgagaacgg tcagaggaag cccacagacg agcatgtggc gctgttggcc ctgaggcact
gggaggacat cccccgcgca ggctgccgcc tggtgccaga gcatgtggag acgaggccgc
tgctcaaccc cgacaagccg ggcatcgagc agggccgcct ggagctgtgg gtggacatgt
tccccatgga catgccagcc cctgggacgc ctctggacat ctcacctcgg aagcccaaga
agtacgagct gcgggtcatc atctggaaca cagatgaggt ggtcttggag gacgacgact
tcttcacagg ggagaagtcc agtgacatct tcgtgagggg gtggctgaag ggccagcagg
aggacaagca ggacacagac gtccactacc actccctcac tggcgagggc aacttcaact
ggcgctacct gttccccttc gactacctgg cggcggagga gaagatcgtc atctccaaga
aggagtccat gttctcctgg gacgagaccg agtacaagat ccccgcgcgg ctcaccctgc
agatctggga tgcggaccac ttctccgctg acgacttcct gggggccatc gagctggacc
tgaaccggtt cccgcggggc gcaaagacag ccaagcagtg caccatggag atggccaccg
gggaggtgga cgtgcccctc gtgtccatct tcaagcaaaa gcgcgtcaaa ggctggtggc
ccctcctggc ccgcaatgag aacgatgagt ttgagctcac gggcaaggtg gaggctgagc
tgcatttact gacagcagag gaggcagaga agaacccagt gggcctggcc cgcaatgaac
ctgaccccct agagaaaccc aaccggcccg acacgagctt catctggttc ctgaaccctc
tcaagtcggc tcgctacttc ttgtggcaca cgtatcgctg gctgctcctc aaactgttgc
tgctcctgct gctgctcctc ctcctcgccc tgttcctcta ctctgtgcct ggctacctgg
tcaagaaaat cctcggggcc tga
**Human Otoferlin Transcript Variant 4** (SEQ ID NO: 17) (encodes the protein of SEQ ID NO: 9) 
atga tcaaaacgga gaagtcctac cctgagcgtc gcctgcgggg cgtcctggag gagctgagct
gtggctgctg ccgcttcctc tccctcgctg acaaggacca gggccactca tcccgcacca
ggcttgaccg ggagcgcctc aagtcctgca tgagggagct ggaaaacatg gggcagcagg
ccaggatgct gcgggcccag gtgaagcggc acacggtgcg ggacaagctg aggctgtgcc
agaacttcct gcagaagctg cgcttcctgg cggacgagcc ccagcacagc attcccgaca
tcttcatctg gatgatgagc aacaacaagc gtgtcgccta tgcccgtgtg ccctccaagg
acctgctctt ctccatcgtg gaggaggaga ctggcaagga ctgcgccaag gtcaagacgc
tcttccttaa gctgccaggg aagcggggct tcggctcggc aggctggaca gtgcaggcca
aggtggagct gtacctgtgg ctgggcctca gcaaacagcg caaggagttc ctgtgcggcc
tgccctgtgg cttccaggag gtcaaggcag cccagggcct gggcctgcat gccttcccac
ccgtcagcct ggtctacacc aagaagcagg cgttccagct ccgagcgcac atgtaccagg
cccgcagcct ctttgccgcc gacagcagcg gactctcaga cccctttgcc cgcgtcttct
tcatcaatca gagtcagtgc acagaggtgc tgaatgagac cctgtgtccc acctgggacc
agatgctggt gttcgacaac ctggagctct atggtgaagc tcatgagctg agggacgatc
cgcccatcat tgtcattgaa atctatgacc aggattccat gggcaaagct gacttcatgg
gccggacctt cgccaaaccc ctggtgaaga tggcagacga ggcgtactgc ccaccccgct
tcccacctca gctcgagtac taccagatct accgtggcaa cgccacagct ggagacctgc
tggcggcctt cgagctgctg cagattggac cagcagggaa ggctgacctg ccccccatca
atggcccggt ggacgtggac cgaggtccca tcatgcccgt gcccatgggc atccggcccg
tgctcagcaa gtaccgagtg gaggtgctgt tctggggcct acgggaccta aagcgggtga
acctggccca ggtggaccgg ccacgggtgg acatcgagtg tgcagggaag ggggtgcagt
cgtccctgat ccacaattat aagaagaacc ccaacttcaa caccctcgtc aagtggtttg
aagtggacct cccagagaac gagctgctgc acccgccctt gaacatccgt gtggtggact
gccgggcctt cggtcgctac acactggtgg gctcccatgc cgtcagctcc ctgcgacgct
tcatctaccg gcccccagac cgctcggccc ccagctggaa caccacgggg gaggttgtgg
tgactatgga gccagaggta cccatcaaga aactggagac catggtgaag ctggacgcga
cttctgaagc tgttgtcaag gtggatgtgg ctgaggagga gaaggagaag aagaagaaga
agaagggcac tgcggaggag ccagaggagg aggagccaga cgagagcatg ctggactggt
ggtccaagta ctttgcctcc attgacacca tgaaggagca acttcgacaa caagagccct
ctggaattga cttggaggag aaggaggaag tggacaatac cgagggcctg aaggggtcaa
tgaagggcaa ggagaaggca agggctgcca aagaggagaa gaagaagaaa actcagagct
ctggctctgg ccaggggtcc gaggcccccg agaagaagaa acccaagatt gatgagctta
aggtataccc caaagagctg gagtccgagt ttgataactt tgaggactgg ctgcacactt
tcaacttgct tcggggcaag accggggatg atgaggatgg ctccaccgag gaggagcgca
ttgtgggacg cttcaagggc tccctctgcg tgtacaaagt gccactccca gaggacgtgt
cccgggaagc cggctacgac tccacctacg gcatgttcca gggcatcccg agcaatgacc
 
ccatcaatgt gctggtccga gtctatgtgg tccgggccac ggacctgcac cctgctgaca
tcaacggcaa agctgacccc tacatcgcca tccggctagg caagactgac atccgcgaca
aggagaacta catctccaag cagctcaacc ctgtctttgg gaagtccttt gacatcgagg
cctccttccc catggaatcc atgctgacgg tggctgtgta tgactgggac ctggtgggca
ctgatgacct cattggggaa accaagatcg acctggagaa ccgcttctac agcaagcacc
gcgccacctg cggcatcgcc cagacctact ccacacatgg ctacaatatc tggcgggacc
ccatgaagcc cagccagatc ctgacccgcc tctgcaaaga cggcaaagtg gacggccccc
actttgggcc ccctgggaga gtgaaggtgg ccaaccgcgt cttcactggg ccctctgaga
ttgaggacga gaacggtcag aggaagccca cagacgagca tgtggcgctg ttggccctga
ggcactggga ggacatcccc cgcgcaggct gccgcctggt gccagagcat gtggagacga
ggccgctgct caaccccgac aagccgggca tcgagcaggg ccgcctggag ctgtggtgg
acatgttccc catggacatg ccagcccctg ggacgcctct ggacatctca cctcggaagc
ccaagaagta cgagctgcgg gtcatcatct ggaacacaga tgaggtggtc ttggaggacg
acgacttctt cacaggggag aagtccagtg acatcttcgt gagggggtgg ctgaagggcc
agcaggagga caagcaggac acagacgtcc actaccactc cctcactggc gagggcaact
tcaactggcg ctacctgttc cccttcgact acctggcggc ggaggagaag atcgtcatct
ccaagaagga cctggcccgc aatgagaacg atgagtttga gctcacgggc aaggtggagg
ctgagctgca tttactgaca gcagaggagg cagagaagaa cccagtgggc ctggcccgca
atgaacctga ccccctagag aaacccaacc ggcccgacac ggccttcgtc tggttcctca
accctctcaa gtccatcaag tacctcatct gcacccggta caagtggctc atcatcaaga
tcgtgctggc gctgttgggg ctgctcatgt tggggctctt cctctacagc ctccctggct
acatggtcaa aaagctcctt ggggcatga
**Human Otoferlin Transcript Variant 5** (SEQ ID NO: 18) (encodes the protein of SEQ ID NO: 12)

atg gccttgctca tccacctcaa gacagtctcg gagctgcggg gcaggggcga ccggatcgcc
aaagtgactt tccgagggca atccttctac tctcgggtcc tggagaactg tgaggatgtg
gctgactttg atgagacatt tcggtggccg gtggccagca gcatcgacag aaatgagatg
ctggagattc aggttttcaa ctacagcaaa gtcttcagca acaagctcat cgggaccttc
cgcatggtgc tgcagaaggt ggtagaggag agccatgtgg aggtgactga cacgctgatt
gatgacaaca atgctatcat caagaccagc ctgtgcgtgg aggtccggta tcaggccact
gacggcacag tgggctcctg ggacgatggg gacttcctgg gagatgagtc tcttcaagag
gaagagaagg acagccaaga gacggatgga ctgctcccag gctcccggcc cagctcccgg
cccccaggag agaagagctt ccggagagcc gggaggagcg tgttctccgc catgaagctc
ggcaaaaacc ggtctcacaa ggaggagccc caaagaccag atgaaccggc ggtgctggag
atggaagacc ttgaccatct ggccattcgg ctaggagatg gactggatcc cgactcggtg
tctctagcct cagtcacagc tctcaccact aatgtctcca acaagcgatc taagccagac

attaagatgg agccaagtgc tgggcggccc atggattacc aggtcagcat cacggtgatc
gaggcccggc agctggtggg cttgaacatg gaccctgtgg tgtgcgtgga ggtgggtgac
gacaagaagt acacatccat gaaggagtcc actaactgcc cctattacaa cgagtacttc
gtcttcgact tccatgtctc tccggatgtc atgtttgaca agatcatcaa gatttcggtg
attcactcca agaacctgct gcgcagtggc accctggtgg gctccttcaa aatggacgtg
ggaaccgtgt actcgcagcc agagcaccag ttccatcaca agtgggccat cctgtctgac
cccgatgaca tctcctcggg gctgaagggc tacgtgaagt gtgacgttgc cgtggtgggc
aaaggggaca acatcaagac gccccacaag gccaatgaga ccgacgaaga tgacattgag
gggaacttgc tgctccccga gggggtgccc cccgaacgcc agtgggcccg gttctatgtg
aaaatttacc gagcagaggg gctgccccgt atgaacacaa gcctcatggc caatgtaaag
aaggctttca tcggtgaaaa caaggacctc gtggacccct acgtgcaagt cttctttgct
ggccagaagg gcaagacttc agtgcagaag agcagctatg agcccctgtg gaatgagcag
gtcgtcttta cagacctctt ccccccactc tgcaaacgca tgaaggtgca gatccgagac
tcggacaagg tcaacgacgt ggccatcggc acccacttca ttgacctgcg caagatttct
aatgacggag acaaaggctt cctgcccaca ctgggcccag cctgggtgaa catgtacggc
tccacacgta actacacgct gctggatgag catcaggacc tgaacgaggg cctgggggag
ggtgtgtcct tccgggcccg gctcctgctg ggcctggctg tggagatcgt agacacctcc
aaccctgagc tcaccagctc cacagaggtg caggtggagc aggccacgcc catctcggag
agctgtgcag gtaaaatgga agaattcttt ctctttggag ccttcctgga ggcctcaatg
atcgaccgga gaaacggaga caagcccatc acctttgagg tcaccatagg caactatggg
aacgaagttg atggcctgtc ccggccccag cggcctcggc cccggaagga gccgggggat
gaggaagaag tagacctgat tcagaacgca agtgatgacg aggccggtga tgccggggac
ctggcctcag tctcctccac tccaccaatg cggccccagg tcaccgacag gaactacttc
catctgccct acctggagcg aaagccctgc atctacatca agagctggtg gccggaccag
cgccgccgcc tctacaatgc caacatcatg gaccacattg ccgacaagct ggaagaaggc
ctgaacgaca tacaggagat gatcaaaacg gagaagtcct accctgagcg tcgcctgcgg
ggcgtcctgg aggagctgag ctgtggctgc tgccgcttcc tctccctcgc tgacaaggac
cagggccact catcccgcac caggcttgac cgggagcgcc tcaagtcctg catgagggag
ctggaaaaca tggggcagca ggccaggatg ctgcgggccc aggtgaagcg gcacacggtg
cgggacaagc tgaggctgtg ccagaacttc ctgcagaagc tgcgcttcct ggcggacgag
ccccagcaca gcattcccga catcttcatc tggatgatga gcaacaacaa gcgtgtcgcc
tatgcccgtg tgccctccaa ggacctgctc ttctccatcg tggaggagga gactggcaag
gactgcgcca aggtcaagac gctcttcctt aagctgccag ggaagcgggg cttcggctcg
gcaggctgga cagtgcaggc caaggtggag ctgtacctgt ggctgggcct cagcaaacag
cgcaaggagt tcctgtgcgg cctgccctgt ggcttccagg aggtcaaggc agcccagggc
ctgggcctgc atgccttccc acccgtcagc ctggtctaca ccaagaagca ggcgttccag
ctccgagcgc acatgtacca ggcccgcagc ctctttgccg ccgacagcag cggactctca
gacccctttg cccgcgtctt cttcatcaat cagagtcagt gcacagaggt gctgaatgag

accctgtgtc ccacctggga ccagatgctg gtgttcgaca acctggagct ctatggtgaa
gctcatgagc tgagggacga tccgcccatc attgtcattg aaatctatga ccaggattcc
atgggcaaag ctgacttcat gggccggacc ttcgccaaac ccctggtgaa gatggcagac
gaggcgtact gcccaccccg cttcccacct cagctcgagt actaccagat ctaccgtggc
aacgccacag ctggagacct gctggcggcc ttcgagctgc tgcagattgg accagcaggg
aaggctgacc tgccccccat caatggcccg gtggacgtgg accgaggtcc catcatgccc
gtgcccatgg gcatccggcc cgtgctcagc aagtaccgag tggaggtgct gttctggggc
ctacgggacc taaagcgggt gaacctggcc caggtggacc ggccacgggt ggacatcgag
tgtgcaggga agggggtgca gtcgtccctg atccacaatt ataagaagaa ccccaacttc
aacaccctcg tcaagtggtt tgaagtggac ctcccagaga acgagctgct gcacccgccc
ttgaacatcc gtgtggtgga ctgccgggcc ttcggtcgct acacactggt gggctcccat
gccgtcagct ccctgcgacg cttcatctac cggcccccag accgctcggc ccccagctgg
aacaccacgg tcaggcttct ccggcgctgc cgtgtgctgt gcaatggggg ctcctcctct
cactccacag gggaggttgt ggtgactatg gagccagagg tacccatcaa gaaactggag
accatggtga agctggacgc gacttctgaa gctgttgtca aggtggatgt ggctgaggag
gagaaggaga agaagaagaa gaagaagggc actgcggagg agccagagga ggaggagcca
gacgagagca tgctggactg gtggtccaag tactttgcct ccattgacac catgaaggag
caacttcgac aacaagagcc ctctggaatt gacttggagg agaaggagga agtggacaat
accgagggcc tgaaggggtc aatgaagggc aaggagaagg caagggctgc caaagaggag
aagaagaaga aaactcagag ctctggctct ggccaggggt ccgaggcccc cgagaagaag
aaacccaaga ttgatgagct taaggtatac cccaaagagc tggagtccga gtttgataac
tttgaggact ggctgcacac tttcaacttg cttcggggca agaccgggga tgatgaggat
ggctccaccg aggaggagcg cattgtggga cgcttcaagg gctccctctg cgtgtacaaa
gtgccactcc cagaggacgt gtcccgggaa gccggctacg actccaccta cggcatgttc
cagggcatcc cgagcaatga ccccatcaat gtgctggtcc gagtctatgt ggtccgggcc
acggacctgc accctgctga catcaacggc aaagctgacc cctacatcgc catccggcta
ggcaagactg acatccgcga caaggagaac tacatctcca agcagctcaa ccctgtcttt
gggaagtcct ttgacatcga ggcctccttc cccatggaat ccatgctgac ggtggctgtg
tatgactggg acctggtggg cactgatgac ctcattgggg aaaccaagat cgacctggag
aaccgcttct acagcaagca ccgcgccacc tgcggcatcg cccagaccta ctccacacat
ggctacaata tctggcggga ccccatgaag cccagccaga tcctgacccg cctctgcaaa
gacggcaaag tggacggccc ccactttggg cccccctgga gagtgaaggt ggccaaccgc
gtcttcactg ggccctctga gattgaggac gagaacggtc agaggaagcc cacagacgag
catgtggcgc tgttggccct gaggcactgg gaggacatcc cccgcgcagg ctgccgcctg
gtgccagagc atgtggagac gaggccgctg ctcaaccccg acaagccggg catcgagcag
ggccgcctgg agctgtgggt ggacatgttc cccatggaca tgccagcccc tgggacgcct
ctggacatct cacctcggaa gcccaagaag tacgagctgc gggtcatcat ctggaacaca
gatgaggtgg tcttggagga cgacgacttc ttcacagggg agaagtccag tgacatcttc

gtgagggggt ggctgaaggg ccagcaggag gacaagcagg acacagacgt ccactaccac
tccctcactg gcgagggcaa cttcaactgg cgctacctgt tccccttcga ctacctggcg
gcggaggaga agatcgtcat ctccaagaag gagtccatgt tctcctggga cgagaccgag
tacaagatcc ccgcgcggct caccctgcag atctgggatg cggaccactt ctccgctgac
gacttcctgg gggccatcga gctggacctg aaccggttcc cgcggggcgc aaagacagcc
aagcagtgca ccatggagat ggccaccggg gaggtggacg tgcccctcgt gtccatcttc
aagcaaaagc gcgtcaaagg ctggtggccc ctcctggccc gcaatgagaa cgatgagttt
gagctcacgg gcaaggtgga ggctgagctg catttactga cagcagagga ggcagagaag
aacccagtgg gcctggcccg caatgaacct gaccccctag agaaacccaa ccggcccgac
acggccttcg tctggttcct caaccctctc aagtccatca agtacctcat ctgcacccgg
tacaagtggc tcatcatcaa gatcgtgctg gcgctgttgg ggctgctcat gttggggctc
ttcctctaca gcctccctgg ctacatggtc aaaaagctcc ttggggcatg a

A non-limiting example of a human wildtype otoferlin genomic DNA sequence is SEQ ID NO: 19. The exons in SEQ ID NO: 19 are: nucleotide positions 5001-5206 (exon 1), nucleotide positions 25925-25983 (exon 2), nucleotide positions 35779-35867 (exon 3), nucleotide positions 44590-44689 (exon 4), nucleotide positions 47100-47281 (exon 5), nucleotide positions 59854-59927 (exon 6), nucleotide positions 61273-61399 (exon 7), nucleotide positions 61891-61945 (exon 8), nucleotide positions 68626-68757(exon 9), nucleotide positions 73959-74021 (exon 10), nucleotide positions 74404-74488 (exon 11), nucleotide positions 79066-79225 (exon 12), nucleotide positions 80051-80237 (exon 13), nucleotide positions 81107-81293 (exon 14), nucleotide positions 82690-82913 (exon 15), nucleotide positions 83388-83496 (exon 16), nucleotide positions 84046-84226 (exon 17), nucleotide positions 84315-84435 (exon 18), nucleotide positions 85950-86050 (exon 19), nucleotide positions 86193-86283 (exon 20), nucleotide positions 86411-86527 (exon 21), nucleotide positions 86656-86808 (exon 22), nucleotide positions 87382-87571 (exon 23), nucleotide positions 87661-87785 (exon 24), nucleotide positions 88206-88340 (exon 25), nucleotide positions 89025-89186 (exon 26), nucleotide positions 89589-89708 (exon 27), nucleotide positions 90132-90293 (exon 28), nucleotide positions 90405-90567 (exon 29), nucleotide positions 91050-91180 (exon 30), nucleotide positions 92549-92578 (exon 31), nucleotide positions 92978-93106 (exon 32), nucleotide positions 95225-95291 (exon 33), nucleotide positions 96198-96334 (exon 34), nucleotide positions 96466-96600 (exon 35), nucleotide positions 96848-96985 (exon 36), nucleotide positions 97623-97750 (exon 37), nucleotide positions 97857-98027 (exon 38), nucleotide positions 98670-98830 (exon 39), nucleotide positions 99593-99735 (exon 40), nucleotide positions 100128-100216 (exon 41), nucleotide positions 101518-101616 (exon 42), nucleotide positions 101762-102003 (exon 43), nucleotide positions 102669-102847 (exon 44), nucleotide positions 102952-103052 (exon 45), nucleotide positions 103494-103691 (exon 46), nucleotide positions 105479-106496 (exon 47), and exon 48 (the sequence starting with CCGGCCCGAC (SEQ ID NO: 89); see also the description of this exon in Yasunaga et al., Am. J. Hum. Genet. 67:591-600, 2000).

The introns are located between each contiguous pair of exons in SEQ ID NO: 19, i.e., at nucleotide positions 100-5001 (intron 1), nucleotide 5207-25924 (intron 2), nucleotide positions 25984-35778 (intron 3), nucleotide positions 35868-44589 (intron 4), nucleotide positions 44690-47099 (intron 5), nucleotide positions 47282-59853(intron 6), nucleotide positions 59928-61272 (intron 7), nucleotide positions 61400-61890 (intron 8), nucleotide positions 61946-68625 (intron 9), nucleotide positions 68758-73958 (intron 10), nucleotide positions 74022-74403 (intron 11), nucleotide positions 74489-79065 (intron 12), nucleotide positions 79226-80050 (intron 13), nucleotide positions 80238-81106 (intron 14), nucleotide positions 81294-82689 (intron 15), nucleotide positions 82914-83387 (intron 16), nucleotide positions 83497-84045 (intron 17), nucleotide positions 84227-84314 (intron 18), nucleotide positions 84436-85949 (intron 19), nucleotide positions 86051-86192 (intron 20), nucleotide positions 86284-86410 (intron 21), nucleotide positions 86528-86655 (intron 22), nucleotide positions 86809-87381 (intron 23), nucleotide positions 87572-87660 (intron 24), nucleotide positions 87786-88205 (intron 25), nucleotide positions 88341-89024 (intron 26), nucleotide positions 89187-89588 (intron 27), nucleotide positions 89709-90131 (intron 28), nucleotide positions 90294-90404 (intron 29), nucleotide positions 90568-91049 (intron 30), nucleotide positions 91181-92548 (intron 31), nucleotide positions 92579-92977 (intron 32), nucleotide positions 93107-95224 (intron 33), nucleotide positions 95292-96197 (intron 34), nucleotide positions 96335-96465 (intron 35), nucleotide positions 96601-96847 (intron 36), nucleotide positions 96986-97622 (intron 37), nucleotide positions 97751-97856 (intron 38), nucleotide positions 98028-98669 (intron 39), nucleotide positions 98831-99592 (intron 40), nucleotide positions 99736-100127 (intron 41), nucleotide positions 100217-101517 (intron 42), nucleotide positions 101617-101761 (intron 43), nucleotide positions 102004-102668 (intron 44), nucleotide positions 102848-102951 (intron 45), nucleotide positions 103053-103494 (intron 46), nucleotide positions 103692-105478 (intron 47), and nucleotide positions 106497-108496 (intron 48).

**Human Otoferlin Gene Sequence** (SEQ ID NO: 19) (NCBI Accession No. NG_009937.1)

1 gggagagcat gcgggctttt tcacccattt ggagactctg cctgcagaat cctcaagaca
61 ttcatttcat tcattcgttc actctttttt tcagacaagg gttcttagga tcattcattc
121 attctataca gatttgctga tactgtacag gttgcctctc agaatccagt gcctgttctc
181 agtacctatg ctgactggta ggagggataa agacagcctt acaccctggg agaggcggtc
241 cagagtagaa acatcagaca ctgtaggaga aagagccaaa gaacaggagg caacagccag
301 acttgcatgt tagatggttg gagtctgaaa atatccagag taaagaaagg taggtgtaac
361 ctagtatttg aaagtgctaa acagcaagcc agcgctcagg gcaagtggcc aggaagcttt
421 ccaagaaaaa cttctgggag gataaaggga ggtctgtgaa aggaccgacc gcaaagtcgg
481 gcgggtactg cacacatgac ccattaacta cggctcttcc ggccttgcca ggttcttcga
541 gcgggcaccc acccggtatc cttccctcct tcaaccaaca agtactgacc ttgcccgaat
601 gctgggtgct ggccctgtgc gccggacgga agggcctgat tttctcgcat tttctctaag
661 cgctggaggc tgcccgcctg gggcgtctcc aagcactaaa ccccacgtgg ctccatctcc
721 tcccccttcc ccggctacat ccatgcgagg tcccttatgc cctgctggct gtgccccact
781 tgccagggag cgccttcctg tgaaggggaa tctgggaggg cccccgcccg tgcctgccca
841 cttcctactc tgaaaccaca aacctctagg aaggcccctc gccccgtcgg gtggaaaatt
901 ggctcctcgg ggttccttca accccaaagt ccgggggcag ggggctgaat ttcgatcacc
961 gaggagggcg ctagtggaag ggggagggga ggaggcgctc cctcggcctt acccgggcat
1021 gagtccaggg ggcacgtagg cggcattgaa ctcggtcagt agggtgcccg cgctgcggga
1081 ggccatggtg ggtgcgcgag tacagcgagc ctggcgctcc gctccgcccc acggtaaaca
1141 gcggcgggtc acctggcaac ggccacggcg gcgagcctgg caacgccagc tggccccgcg
1201 gtcctcgcgt tccgagacac tgcgcccccc tggcggccac ctcccggagc agccccatag
1261 ggcgcgccca gaaggcgcct cccatttccg cgagctctgc tgggtgaccc ctgtcacttg
1321 ccccggcgcg tcggggctct ctgccttgct gcctgcggga tgtgcagagg ctcagatgcg
1381 ggagggaggc agaacatcgc tggagaggcc tggcgggatc cgtcttgcgg tgacccggct
1441 tggaacgcga cgctaccaca aaggacaggc caggtcctga ctttggaggt cacgcacgaa
1501 gcagctgccc caccgtggct caggcccaga aacctgagtg gccttagggt ccccaggagc

1561 tatgtctctc caaggcttcc cctgcacccc agctccatgg gagttccagg aaggggctct
1621 gtagtgggcc gcgctgggcg aggttgccta ggacgctgtg tgtaattgtg tgtgtgcgtg
1681 cgtgcgtgca tgtgtgcgtg tgtgtgtgcg cgccttgcga agaggagcat tttcattttt
1741 tcttcttaat ctctagctga tggcttcctt ctcacctcat catacctgta aactactcca
1801 caagaggcaa aaagaaaatc atagaacgtc tgggtgtgtg atttacattt gtccccaatg
1861 ctgccgttca ttttggggat ggcatccgca ggggtagcgc ttgccccaga gagcaggcct
1921 gctgaaggcc caccccctgt gtaatcctcc ctgtgactgc ttcagcctcc acatttgagt
1981 ttctccaggg acccttacac cagccttggt gcctagggca ggcacagggc cttcggcttt
2041 tccataaggg gtgagggtat ggggacagct tccacggtgc caggctcttg gtgactcggg
2101 tggaggccct gccaccccag ctccctctgc agcagtcctg gtgtcatgaa gaagcctggc
2161 agttcccact ttgtctccag gtcttgcttc ttcccttcag atgccggatg gagagcagtg
2221 ctcccctcgc acttcggttc tgtaaatggc agtggatgaa gaccgcagac gctactgggt
2281 tcacagaatg tggctgccac cagcattctc caaagacttc ccctggcaaa tgccaatgaa
2341 atgaagggga gccacaggct tcccagaggc tcaggaagca gaaacactga ggcctggttg
2401 gaaagagagc tgtaggcaaa ggaacggctg caaacaaact gggtggaagg aaaatccatc
2461 catccttcag aatgcagtca tgtctgtatt tttctaaatt ctcattgggg aacatgttct
2521 acaataagga aaacatgagt caaagacccc cagccctggt gaggttgacc gcctacttcc
2581 tcccccattc agggctgccc agccagtcct gctggtaaac ccaccaccct gccttccacc
2641 tgcctgtgtt ctagtccatc tgtctcgtct gattgggatc ctgtgccatc catatcatat
2701 tcatccctga atctcagcac ctggctctgg aaagaattca gtaaatgttc attacataaa
2761 ttatatactc agttaccaaa atgggtaaca gaagaatgga aattaaaaag tgggttctgg
2821 ctgggaacgg tggctcatgc ctgtaatccc agcattttgg aaggccgagg caggcggatc
2881 acttgaggtc agcagtttga gaccagccta gccaacatgg tgaaacccca tctctactaa
2941 aaatacaaac attagctggg catggtagca catgcttata gtctgaccta tttgggaggc
3001 tgaggcagga gaatcgcttg aacccgggag gcagaggttg cagtgagcct agatcactcc
3061 actgccctcc agcctgggtg acagagtgag actctctctc aaaaaaaaaa aaaaaaaaag
3121 agtgggatct gtctcttagg ttttcagaaa ggccagctga ctccattgag agctgctgag
3181 tggaattgct accattttat ggatgtggaa agtgagattc agcatagatc aatgccttgg
3241 ctagggcagg atcatgggct ttttcctaaa gatgcacagg gtctataaga ggcctcatga
3301 aattcactcc tttcagggga ggacctccag agaaccaagg aagaagatat tttctgttgt
3361 tgttgttgta ccttgtagtc accaagcctt aaaaacacag atcacctgat gacctcatgg
3421 atggaaaatg ctagtgaatt taggtgtttt tttttttaag acattaaact tatctagctt
3481 ttgaaaattt agcattcata cttgtattca ataaacagaa tactctaaga catttttaaa
3541 ttcaatatga ttagataaat gtgagaaagg taagcaagaa ttggttagga aaaagtgcaa
3601 aggaaaaaaa cgtaagtaga aaaagaaaaa tagggcacag tagcaaggaa atatctagaa
3661 ggaggaaaga aggctcttgg gacatactgt aggttatttg gaatgcagaa ttcaaaagaa
3721 gtaacctctt gttaccatgc tgtgaaaaat tagtatttct ctttttaacc tttagactct
3781 gtttcaggta gatccctgag ctcactcttt tatcctttca acagtaattg ggcatcctac

3841 ggtgcacagg ataccatgtg gggctctggg gtagagggat gctgtgctcc agggccctcc
3901 tgaggtcagg aggggaaaag gcaagggaag cagccccagg actcagtaag atgagaggag
3961 gcaggagcag cacagaggag gaccccaacc cagctggggt gcgggtacag aggcacctcc
4021 aggagcaggc actcaagctg agtccagggt ggggtggagg gcattccacc aagaatgaat
4081 agcaagaaca aggctgcaga tgaccctgca cagattccag catcagggat tctggccccc
4141 agcgtccctt tccttggtgt atgctgctgg aagacaggag ggggcagttc tcttttggtc
4201 cttagaaatt tccctataga catcccttct cctgccccta gcagcttata acagcagaag
4261 gccattcaat tctgctcctc tggctggaaa gcaaacatca gcctggctta ccttcgctag
4321 cctgcctgac ccccagcctt ggggtggggc taggttagtg ccctggatcc ttaaacatgg
4381 cccctggtag cacataagcc tctgctgcct ccacaaccag ccatctccat catgcatggc
4441 ctggtcctga gcctcccctc aggcagcagc cccagcctct ctgcttcctg agattcagaa
4501 acagggatcc caggcagggg ctcagggccc agcctcttgg gggcacagtc tccagcaggg
4561 aagaggtggg cgtaggtgga caaatacatt cccttcttct tgtagtgtgc ccctgtgggt
4621 gggagtgccc cccgccccac atccacccta cctatgctca tgcccactcc tctgtgccca
4681 ctactggtgg cagggcttgc ttagcaggga agcagcagag ccctgcagag gggtgggcag
4741 agcctccctt ccaggagctg ggagaagaca cctgaagctg gacagagctt tatctgggag
4801 ccggcggctg ctgaataatg aattccaact ctgccgcact cccaggctgc acattgggtg
4861 cacacacaca cacacacaca cgcacacacc gatacacaca cgctgggctg acaacttgga
4921 ggggcggggg ctgtgaggct ctgtgagtcg ctgcaggagc agcttagctc ggcttggttg
4981 ccttggtctc tgtgggcagc atcggagggg ggtcgggagg aggaggagga ggcagcggca
5041 gagaagagag aggcgtgtga gccgtgctcc accggctagc tccttcccgc tgctcctgcc
5101 tggcagtgcc aggcagccca caccagcatg gccttgctca tccacctcaa gacagtctcg
5161 gagctgcggg gcaggggcga ccggatcgcc aaagtgactt tccgaggtag ggaagccgct
5221 gtctcagagg gacgccagct ctgagagctg gaccagccca tgctctggag gtggccgggg
5281 ataggacagg gctgggagga tgggtgggaa aagttctagc aggggctggg gcgacgagcc
5341 ttttcacagg tagtcatctg cctgggactg actggccatt gtgatttctg gaaaatcagg
5401 gtcccctgtt cttccatgcc tgtgtctcct tgtgcttgcc aacatgccag ttcccgggc
5461 agggcacaga gccctctgcc tggggaggca gaagtgagga ccacatccct gagctcaggg
5521 agacccagga ccccctcctt ctctctctct ccctctcctg caggttgatt tccttgagca
5581 cagggtgggg gccgggtatg gagcctgggg cacccagggt gactgggaga aaccaggcag
5641 ccagtagctc cctgggcccc agagtgatgg gggacactgt tggcttcttt tagatctatt
5701 catgggcggg aaccccggga gcccctcgcc tctcttctgt ctggtctttt ctcatccttt
5761 tcactcctgt attcagcgac catttattat gatctactgt gtcccaagga taggggtaga
5821 aacacagagc tgagcacaac ccagccccag ccccaaggag cctgcatctg ttgggcaggc
5881 gtggaacaag caggacaggt atgtgagaca gaaggtgggg gtgcagcatg caagctacag
5941 aagcagcacc ctcacccagg ctggtgaccg gtggtcaggg aaggcttccc ggaggaagta
6001 acactcagct aatacccgaa ggaccagcgg gtgggagcta ggagaggagg taaggactga
6061 ggaggcagag gaaacagtgt gcccagagct gagagaggag ctgaaactct ggaactgaaa

6121 gaggttccat gtgtctacag ctcagagagg gtgcaggagc ggaggccaga gagccaagca
6181 gggcaaggag gcgaggctgg acctgacccc ggggacagcg gagcccctga gcattttaag
6241 tggggaaagg cagggccagg ctgactctgt ccttcaccac ccacctctgc ctctccatgt
6301 ggttgctgcc cagccctgag ggttgggggt acttctcact agacatggcc ccttctcccg
6361 agagaggggt ctgagagtgg gatggggacg aggtggtgag aagggggcag agaggacccc
6421 cctatactaa agagcaaagt gagccttggg gcccaggctg gacacagtgt ctgcacctct
6481 gttggaaggg atgggagaga ccattggcag aacattccag caagagccct ggggcaccca
6541 catcacctac ccagaggccc tgggatgccc acattgcagc ttgtgttcat agggacaggc
6601 aggggtggag gtgatggcag tggccctgcc ggcaggcgga ggttaggcag gagggagagg
6661 gaggtgtggg ctgggctgcc ttttgcctcc caccccaacc cactgactgc tttctctgca
6721 gcaccctgga tctccctctg tgtgccacct gcaggctcat gctggcttta gaaaaagcaa
6781 atgtgtcctt tcatctgtga aatgtggccc tgagctccac gggcagcata gtgtggggcg
6841 gttaagctac aggttgccat cctgctgcag ctgtgctgga acggtccagg ctgcaccgga
6901 caggcccact tgcaggccga gaaaccaagg ttcaggggta gaacactgcg cgtggcattc
6961 acggaggagc tgaggcacgg tggtcccagc tctcaggacc tcatcctgag tactcaatct
7021 tgcaagctct ttatctgctc ctgggccacc tgggggccag caccttgtct gacacccatc
7081 ttctccttgg agcagtgaag ggctgggcct ttagcctcac tgaccacaca ggaattagtt
7141 tatttgcaac ccttgctctc tggactgagt ggagaaatcg cttccaagct ccacattggc
7201 aggaggtggc catgggcaga gctcctgggg aagggggctg ggcagcagga ataggcagat
7261 gacaagcagt ttccagaaag tacctttatt acaacgaaag atgcctcagc cgctcacctg
7321 tgtcatctag cagttcacag ttgtaggcgc cccaaaagac caacactgac cacaattcag
7381 agggaatgga cccgctctac aatgttcctt agcatctccc agcacatgca accactgggg
7441 gccaagcaag tcctctgcag ggggaaacaa gaaactggta aacacgcaga gggagacact
7501 ggccccaggc tccccaaggc ttttgagtga aagtcaatgc cgggggaggt ggtgagggag
7561 cccttggtga gctgtgccca ttggcagtgg ccctaggtag gccagagcag ctggttgaaa
7621 tatggcttga ccttctgtgc tgtgccatcc aagacactaa ggaaagagcc ctcctgggct
7681 attgtggaaa aagttctgga tatggagtca gaagccctag atcctacctg ggttctgcca
7741 ctcaccctca gtttcctcat ctgtaaaatg ggcatgataa taacactcac ttcactagat
7801 tgcttcaggg aaaccatagg tatggaagag tttgtgggaa gagtattcaa atggtcgatg
7861 tgtttgtgag gcagccattc ccattctcct tgggagcagc tgctggcccc tgaggtatct
7921 tctctccaga ccagcagagg gccccatgtg agatataaag ggaagaggag agcacgtcta
7981 aggggttcca gtctgccagg agaaggcaga gagcctggac tctgctctgt aggagcctct
8041 gctgagatgt tccgcaggaa cccaggcagg cagacccccg agactcctgt tctcatctgg
8101 ctgggtgtgc accagctttg cccgtggcca cctcttttcc cactgccctc aggccagtcg
8161 gcccctcggt cagtgctccc agccagttct gtatctagct cttagttctt actgcccctg
8221 gctgctccta agctggtgct gccctgttca tgcatttcct gaacatgcta atatttgtgc
8281 acaggcctgg gaccctgagg tgaaggggcc agcctggccc tccaggagtc acaatcccat
8341 tgggtcagac aggagatagg gagtcctggg cgaatctcta ttgtgcctgc aggccacagg

8401 gctcagctgg gtttctcatt aacaaaatgg agacagtggt cctcatttag ctcagaggcc
8461 tgcagttccc aggagtaatc aagagtgggc tatctaagaa ggagccatct tgaccacagg
8521 ttgctactca aagagaggaa gtattagctt caggtatttc ctttatcaac tcctgagata
8581 gttccctaca tcaatcacag agccacagaa cgcagggtgt gaagggtccc gaagttcttc
8641 tagtccaacc ttctgtccag tgctgagcac caaggctggg ggacctggac tgtaagggaa
8701 ccaagtgaca gacagtccag ggccaaccct gatgtctccc ttctctttct atcccctgag
8761 ccttcttgcc aagacatgag gtttggggat tttctgtgtg agataagaac atctaaaggc
8821 tggatctggc ttccacataa ttcactgtgg ctggaaattg gcacaaaggt ttgcagggca
8881 tccccctcca ccttttcatt cttactcttc ttcccaccct ccccctgatt cccaccctgc
8941 tatgttccca ctacattcaa cttccagcca ttcccaaaag ataccacacc cttttacacc
9001 tccaaacttt gagacatcga tcatatttct tctgcctgga atgaccaccc tccaccttga
9061 gaacgcccat taattctgct catccttcaa ggcccagctc atatgctacc tcctccatga
9121 aggagtagta ctttgcacac aaagtactcc tctagcagta ctttgcacac attcttccta
9181 ctgctctgcc aaggtacatt gcatttcaga aactggccac acctctgcat gctgcccaag
9241 cttttgagca tcttcaggac agacatcaga catggtttct gtctttgagg gattcactgt
9301 gtcatgggtt tgagttcttc cactgtggca ctgtcactct ttggctccat ttccccaact
9361 gtgagctctc accccagaac tttctatcca aggaggatca agggactgga agaggcctca
9421 agggccaaga accaagaacc ttggctctta ctccttccat caacttccct aacccagaga
9481 atcttctagg gctccagagg ggcctaacga tttttttttt tttttttttt tttgagtctc
9541 gctctgtcgc caggctggag tgcagtgcag tgcgatctcg gctcactgca acctccagct
9601 cctgggttca agcgattctt ctgcctcagc ctctggagta gctgggacta caggcacacg
9661 ccaccatgcc cgactaattt ttgtattttt agtagagacg gtgtttcacc atatttccca
9721 ggctggtctc aaactcctga cctcatgatc cgcctgcctc ggcctcccaa agtgctggga
9781 ttacaggtgt gagccactgc gcccagtcaa gcctaacaaa tattagcaag ttcagctcca
9841 gcccataggt gggaagcaag agggattgcg tgtgtaagcc agagtcagtt aggggaagac
9901 agaggaagga gaaagggcag acgggaaagg aggtgggagc aggctctgag ttggcccccg
9961 gagctcagaa gggaggcggg agtttttatt gagcagggga aatgggcggt atttaagaag
10021 ccaccgaagg gtcttagttc attggcagga tcaaaggcca tgaaagggcc aggcgtggtg
10081 gctcatgcct ctttccgagc cttactgcga ccacacgtgg ggcaggatct caccatctga
10141 cagaggagaa gaaacataca cacagaggtg aaatgagtgc agcatacagg agggccagcg
10201 gtgggggtaa atagcaacaa tgttcagagg atgacagctt acttagagct acagagacga
10261 gggaaaagga gcaggaggag gaagtgccta agctgggtct tgaaaaactg cccctgtttg
10321 tacctatgga gatgcaggaa gtgaggaatt ggtgatggag gaaggtgaag tctttttggg
10381 tgagggaccg accagaccag aggccagagg aggagaattg cagccaagtt ccatgtcggg
10441 gaagccacac catgtagctg gagcagaggc tataggaagt agaagaggga gaccagctag
10501 ggagggagca gtgggcctgc tgagtgattg tgcagggaag ggttgcagct gaggcactgg
10561 ggacaagatg tccccctact gttggctgca ggctccacct ctgactccac attacattta
10621 ttttccccag taaaatgagg gagccctccc tcttgatgat aaatatagct actatttgga

10681 aacttatctg ccgcccctgg taaaggaggc agaataccca gtgcacccac ccctgaaatg
10741 ctcctgcaat gcctaggcct gtctgtgggc tgtggtccag cctagcaccc cctctcttgg
10801 agaatgtgag gttggcattg tccctgctga aggcagcaat agaggggacc cttacgacga
10861 ccttggcagc cggtggctgg ctgtggacac agccctgcgc ttttgtttta gtcctaacgg
10921 atatgccccc tgcatcctcc attcttcaca gcacactcag tccccctcac cacacctgag
10981 ggaaatcttc tctgcctaat gccccacgta gtgaatgttc tcccctcctc cctcccccag
11041 agcaagccat tgcacaccag cgaagggcag acagagcaca cagaaaattc cccagacttc
11101 tgctttgata ggaagtgtat tttcatgtaa tggaaatgat tgtaaaactg ccttcccctg
11161 caaaagccct caggaatatg ctgctggaaa tgtgcttgag aacagacttg caaatcattt
11221 ctctgaattg ggaagtcttg gttatcttag ccacttttac cgatcatgtg aaagccacat
11281 ctctcgcacc cactcccatc cctgggtctc atttttttct tttttctgtc gcccacgctg
11341 gagtgcagtg gcacaatcat agttcactgc aacctcaacc tcctgggttc aagcgatgct
11401 cccacctcag cctcccgagt agctaggact acaggcacgc accaccatgc tcagctaatt
11461 tttgtatttt tagtagagat ggggtttcac catattgccc aggctggtct tgaactcccg
11521 ggctcatgtg agctgcctgg ctcagcctcc caaagtgctg ggattatagg cgtaagccac
11581 tgtgcctggc cttttttttt tttttttaac tttttatata taaaaaaact ctcaaataca
11641 tacaagagtg gggagaacag tatcatggcc ctatgttgta ccatccaatt tcagctacca
11701 tcaattcatg gccaatcttg cttcatttgc atcccatcct acttcctcac cccaaattat
11761 tttgaagaag atcccaaacc ccataccatt ccatctgtaa atattcagta cacagttata
11821 aaagataaga gctcaaataa ataccacata ctttgtcatt ttaaagaaat taataacaat
11881 tccttagtac tataaactaa tcaggcagtc atcaaattcc taattgtctc atcaatgtca
11941 taattttttt ccaatttatt aaaagcttca ttttaatgtg ttttgtaaat caggtattta
12001 atcatttatc cactcgttct gttgaagggc agccatgtgc cacgcactgg gggatggtgg
12061 ggaatgagac agatgtggtt cctgtcctca aagggggaag atgaccactg cacccatcat
12121 catgaggagc tttatggaag ggaggcacag gtgcccaggc agctcatggt gaggaaaccc
12181 acttagccca ggatgggggg aagccacccc agtgcgtgag ccagagtcag ttaggggaag
12241 actgaggaag gaggaagggc agatgggaag ggagggagga gcaggctcct agttggcccc
12301 tagagcccgg aagtgagtca ggagttttta ttgagcagga ggaatgggcg gtatttaaga
12361 agcccttgaa gggtcgcagc tcattggcag gaacaaaggc catgaaaggg ctgggcatgg
12421 tggctcacgc ctgtaatccc agcactttgg gaggccaagg cgggggggat catttgaggg
12481 caggagtttg agaccagcct ggccaacatg gtgaaaccac atctctacta aaaacacaaa
12541 aattagccgg gcgtggtggt gcacgcctat aatcccagct actcgggagg ctgaggcagg
12601 agaatcgctt gaacccagga ggcagaggct gcagtgagcc gagattgtgc cactgcactc
12661 cagcctgggc gacagagcga gactctgtct caaggggggg aaaaaagcca tgaaaggctc
12721 catcccaggc agaggggcta acactagtgc agacgtggtg gtgtggagat gcatcagggg
12781 agtgtggagg ggagaccgga aggcagggca cgggggacgc agccaaacac ggagggtctt
12841 tggtgtcaca ctcttagcag gagaatggga tgctaagatc ttgttttgga gagaaggctc
12901 tagaggtgag gactgcgggc agggacactg gggaggaagc ccggggcagg cggagggctg

12961 agtgcctcag caggcgccat gagagaggaa ggagcggcgc tgggatcagg ggagtgagtg
13021 acagggcagg gcaagagtca attgtggtcc tggggaccac cgcggaccca gggatttggg
13081 gtacagggcc ttagttgatt ctcatctaga caggtgccag ttgctgacac cctgaattct
13141 ggcaaacatg gagacatcag ggtggaaaca cctggagcca ctgccatcca ggtgggcgcc
13201 ttgagggcca ggccccttgg ctgctttccc agcagcagca tggagcctgg cacagaacag
13261 acctggcata gaagtgggag caaggaacac cgcctgtggg tgcaggagca gtgatcagtt
13321 agcaacaggg gcccccctgc agccagccat ccaggcagca ccagacagtg atccatttct
13381 ctcggtgaca gttctagaaa aggctccgga gacagggact atcatgtgat catttttacc
13441 ctagaccgct tgaccgcttt ctgtctgaac tgatccacct gctagacgct gcctcaacag
13501 tcctcgtttc tattttattt tcttttactt caaagcagaa gggctaatct gctgaggtgg
13561 agacgagttt tcacccactg gctttttttt ttttcagccg aagtttaagg taaagtcagg
13621 actgcagttt ccacaatatt tttaccctat cggagagtaa aagagtcacc ctcccattat
13681 acctcattgt attcccagtg cctggcacag aatgggtact ctacacactt gtggatcact
13741 gggaccacaa gctcctctgt caccgtaaca ggggaatcta ggacccagca gttgatgact
13801 gtgtgggtgg cactgtccaa ggctcaaaga caaataccac aggcccttcc tgcctcagtg
13861 ccagggtctg aaaaatggga accagcgctt tcagtcggtt gcagggcaaa gtcagagttt
13921 gagtgaggac ctggaagaag ggtttgagct gagccagaga gatgggattt atcatccctg
13981 ccctgcccct gcctctcaga tgaatccctg acccaagcct aagtttccat agtgtaaacc
14041 tggatgttag cagggtgggt taaggggaaa tacatgagtg gaggtagaaa tgatatttta
14101 gtacaatttg tggataataa agggttagag ttccatgggg cctccattta gctgagaaat
14161 ggggaaaatg gaaggggtaa ccttttccag tgggaggccc agggactctg cagtatccca
14221 agtctggctc tgtgagtcat tggtgatttt cagagagtat actatgtaag aaacatgagg
14281 aacagctgct ttcttggaca actctttgaa gtgaatttct ttcttctacc atgccatcaa
14341 ctgtcattat aaaattgaag atgtgtttct catggggaaa aaaattgtta ttacaagaga
14401 tatgggcaat tagacaagtc tcaatacatt tcaaaagact gaaatcttac agagtatgtt
14461 ctctgaccat agtggaattc agctaaaaat caataataag aagattatct ttgaaagttc
14521 caaatatttg gaaattaatg cctttcttaa cctatggtca aagaaggaag taaaagagaa
14581 attatgaaac atttttaact gaattataat gaaaatacaa catattaaca tttatcattt
14641 taagtactta cagcagaaaa gaagaaaagt gtaaaattgt aatctaggtt tctgccttaa
14701 aaagatacca aaagaaaagc aaattaaacc caaatcttga aaacattatg ttaaataaaa
14761 gaagccagac acaaaagacc acatactgta taacctcatt tacagtcagg catcgcttag
14821 tgataggagt acattctgaa aaatgcatca ttaagcgatt gtgtcattgt gtgaacatca
14881 tagagtgtac ttacacaaag ctagatggta tagccaacta cacacctagg ctacatggta
14941 tgacctactg ctcctaggcc acaaacgtgt acagaacgta actgtattca atgctgtagg
15001 caatcatagt acaatggtaa gtatttgtgt atctaaacat agaaaagata cagtaaatac
15061 aatattataa tcttatggga ccactgttgt atatccaagt ggtcgttgac caaaacggtg
15121 ttatgtggtg catgaatgta tacgaaatgc tcagaacagg caaatccata cagagaggag
15181 atttgtggtt gtcaagggct gtggggaggg agaatgggga gtgactgctc acgggtatgg

15241 agtctccttc cagggtgatg aaaatattct ggaattaggt agtggtgatg ggtgtacaac
15301 cttgggaata tactaaaaac gactgaactg tgcactttag aaggatgaat gttatggaat
15361 gtgaattata tttcaataaa taaaaatcaa acacccccca ccaaattaaa cccaaggaca
15421 gtataaaagg aaggaaatag aaaatagaca acagagaaat ttaacaaagc caaaagttgg
15481 ttcttcaaaa aaaattaata aaatagataa accctagcaa gacttatcaa aaataaaaag
15541 caagaaaaca caaattacca atatcaggaa taaaagaggg aatatcatta catttaaagg
15601 aaaatatggg aatattacca ataacttacc tcaataaatt ctacaactca gatgaaatga
15661 ataaatcttt tgaaaaacaa cttaccaaaa ctgacagaaa aagaaatgga aaatccaaag
15721 agcctccata tctattaaag aaattgaatt tgtaataaca atgatgatga tgacaataaa
15781 cttccttcca caaagaaaac ttcagatcta gatgatttca ctgctgtatt gtagaaaaca
15841 ttcttttttt ctttttttga gatggagtct cgcactgtca cccaggctgg agtgcagtgg
15901 cgcgatctca gctcactgca acctctgtct ccccagttca agtgattctc ctgcctcagc
15961 ctcccgagta gagcagctga gactacacgc aggtgccacc acgcccggct aatttttgta
16021 tttttagtag agacagggtt tcaccatgtt ggccaggctg gtcttgaact cctgacctca
16081 agtgatctgc ccatatcggc ctctcaaagt gctgggatta caggtgtaag ccaccacacc
16141 cggcctgtag aaaacattga aaggaaaacg taataacaat cttacacaaa ctctttcaga
16201 aaatagagaa ggaagagcac atctcaattc attttttatt atcaaaacct gacaaagacc
16261 ttacacaaaa agaaaattac agcccaatat ctctcatgaa cacagatgta aaaatcctta
16321 acaaaatatt agccaactga atagcaatat atgaaaagaa taacacatca tgacaagtga
16381 gtattatccc aggaatgcaa tgttaattta aaagtagaaa atcaaaatat tttaccatat
16441 taacacagta aaggaaaaaa ttcatgatta tatcaataga tgcagaaaat gcatttgaca
16501 aaattcaaca cccattcatg ataataacta tcagcaaact accaataaaa ggagcttcct
16561 caatctgata aaaatcatcc agctaacatt atacttaatt cagaaatatt gaacattaag
16621 atcatgagtc aggcaaagtt ttctactctc accacttctg ttcaacactg tgctagaatt
16681 ctagccagta caaggtggca aggtaaaaac aactcctaca gattggaaag gaagaagtaa
16741 aactatcttt attcacagaa aacctgattg gttatgtaga aaatcctaag gaatctatta
16801 aaaagctact acaattaatg aatgaattta gcaagttttc aggatatatg attaatacac
16861 aagaaaataa attttaccaa agacaggaaa gacttccacc ctaaaaacaa aacaacacaa
16921 aatgctgaga gaaataaaaa aaaaaaaaaa ccaaacatgt agaaaataga ttatgtttat
16981 ggactgaaga ttcataattg ttaagatgac aattctccac aaaatgatct atagactgaa
17041 tgcaatccta ataaaaatca caacaaacta ttttgtagaa attgaaaact actttaaaat
17101 ttatgtggac atgcaaatga tctagaatag ctaaagcagc cttgaaaatg gagaagagaa
17161 ttggagggtt cacaccacag ctgacccctg aacaacatgg gtccacttat atgtggattt
17221 tttttttttt tctttttgag acagagtttc actcttgttg cccaggctgg agtgcaatgg
17281 agcaatctca gctcactgca acctccacct cccgggttca agcgattctc ctttctcagc
17341 ctcccgagta gctgggatta caggcatgca ccatcactcc cagctaattt ttgtattttc
17401 agtagagacg aggtttcacc atgttggtca ggctggtctc gaactcctga cctcaagtga
17461 tccaccagcc tcggcctcct aaagtgctgg gattacaggc atgagccact tcgcctggcc

17521 atggtttctc ttccttatct tttttcctaa cattttcttt tctctagctt actttattgt
17581 aaaagtacag tacataatac atatacaaaa tatgtgttaa ttggctgttt atgttatcat
17641 taaggcttct ggttaacagt agtctattag tagctaagtt tttggggagt caaaatttat
17701 atgcagcttt tcaactatat gaggggctgg tacctctaat ccctgtgttg ttcaagggtc
17761 aactatattt aattataagt ctaactataa agttaaagga attaagaaag tgtggcattg
17821 gtctaagaat agacatatag accaatggag cagaatagtg tccagaaata aactctgtac
17881 atataaaatc aattgatttt caatcaaggt gttggatatc tagaactagg tatctatatg
17941 gagaaaaaat gaacttcaaa ctcctacctc ataccatcca caaaaattaa tgtaagatgg
18001 attatagaat taaacattaa aaacttataa gcttctagaa gaaaatatag aacatctttt
18061 tttgtattct tgggttaggc aaaggtttct taaagaaaaa ttttaaaaga ctaaacataa
18121 gaaataaatt tcactaaaaa cttgcatttc ttaaaaattg gaaactctct ttggctagtc
18181 acagagtaaa aaaaattgaa aactcctatt cataaaaaaa tcacatttaa gaaggcaaga
18241 gaaggcaagt cacacacagg aagaaaatat tcacatttat ctgacaaagg acttgtagcc
18301 agaatatata aagaatccct ataagtcaat aataataaga caaattatga aatttttat
18361 gtaggcaaaa gacttgaaga gacactccat agaataagat agacgtatgg ccaataaaca
18421 tgtgaaaatg tgatcaacat cctgagtcat tagagaaatg aagatcaaaa ttacaaagag
18481 atattatttc acatgctcta aaatagctaa aatgtaaaag tctgacagta atcaaatgtt
18541 gatgaggatg gaacaactga aatttttttt acattgctga taagagtgtg aaatgatgca
18601 atccccttag aaaactattt ggcgatttct ttcttttctt ttcttttttt tttttttgg
18661 gatggcatct cgttctgtca cccaggctgg agtgcagtgg tgtgatcttg gttcaatgca
18721 acctttgcct cccgggttca agcgattctc ctgcttcagc ctcccgagta gctgggacta
18781 caggttcgca ccaccacgcc tggctaattt tttttttgta tttttagtag agacagggtt
18841 tcaccgtgtt agccatgatg gtctccatct cctgacctca tgatctgcct gcctcagcct
18901 cccaaagtgc tgggattaca ggcatgagcc attgcgcccg gccagcaatt tcttataaag
18961 ctaagggtac ctatcctgtg acataaccat tctgttctta ggaatttcct caagagaaat
19021 aaaattatat tttcagaaaa agactcacat gaatgtttac agcaactttg ctcatatagc
19081 ccaaccctag aagcacccaa aagtttatca tcaacagaga aatggaccaa gaagttgtgg
19141 tataggcatg caacgggcta ttactctaca ataaaaatca atacattact tatatatgca
19201 acaacatgga tgaatcacaa aaacattacc ttgaccaaaa gaagccagac acacaaaaag
19261 tacattctgt aggatacctt ttatatgaag tttaaaacag gcaaactgat ctttggtgct
19321 agaaatcaga gcactggtta ctaaggacct agaaatcgac tggaaaggga catgagagaa
19381 catgtggaac aaatggaaat gttctatgtc ttgattgtgt ggcggtatac agggaaatgc
19441 atttattgaa actcattgaa ttttacactt aagatccatg aatgtaagtt tcacctagtt
19501 ttaaaaaaag aagagagact cacatctaaa atacctaaaa cttgaaaaat attatataag
19561 atgaggaaaa aaattatttc tgattccatg ctactaaaaa taagctacaa atgtgattca
19621 taatagaaga agccattttt atatttagca cataacttaa aggtctaaag gacaagcaag
19681 ctgggcgcag tggcccacgc ctataatcct agcattttgg gaggccaagg caggaggatc
19741 acttaaagtc aggaatttga gaacagcttg gccacatgct ggtgaaaccc tgtctctcta

19801 aaaatacaaa aaaagttagc caggcatggt ggcacacgcc tgtaattcca gctacttagg
19861 aggctaatgc aggagagtca cttgaaccca ggaggtggag gttgcagtga gcggagatta
19921 cgccactgca ctccagcctg ggtgacagat caagactccg tctcaaaaaa ataaattcaa
19981 aacaaaatag aatgaaggag aaacaagact atttagtaga ctgagaatag ctgtttctta
20041 ttgagggata aactgcacca aatgctattg taagcattat tatttccttc attttaaggg
20101 tgaggaaaca agggcaaaga gaagttaatt agcttcatcc agggcacgca aactaggaag
20161 aggcagctgg gatttgaact cagggactct ggcccaagag ccttaacctc tatcccatgc
20221 cccctcgtgc ttgtaaagga cttgatgctt tacttagact ttcccacaac agtttcttgt
20281 catcctcgtg atggtcccag gacacaggct ggtccaggat aatgaccccc ggctaacaga
20341 tggtgagtct gagaccccaa ggggttggat ggccagctcc caggcctcca gaccatgggc
20401 cgttgtgccg ggccgcacac ctgagtgggc ctggcttttt cctgcacgca ggggtgcttg
20461 ggccctgctt cctggtgctc catgggagta tctggccttc aaaaggaaca gatcgatctg
20521 aagtggggag agagaaaata cagcaggacc atcactcaga gagtcgctgt agaggaagag
20581 agggagagga gacgccagga catgggagct ttgtgggagg gaatgggact atttgctgag
20641 ctttgtacag cccagcccag gcagtgcata caacaaggct ttgctgattt taaataacac
20701 aaggaaatag cagccccagg tctgagtcac cagcagagtt ttactctatt tctcagctca
20761 cacagggaca atagcaatgg caggcactgc tgggagatag gctagtgggc tgggccctgg
20821 caaaatcacg acgtccttga aaacagtccc aggaccttct ccttcccctt cggatggccc
20881 accacctcac cctctgccct ttcgggaccc cagcccctct ccagtcttgc ttccacaggc
20941 tgctccctga gggcagacca ttcttgcttt tcccttcacc atcgggactc ccctgtcctg
21001 tgtccagctc cttggaggct ctactctctg ctctacgctt tcaaacattt ccttacactg
21061 ttctctctgc ctggatttcc ctccttctcc ttgcttccca gtctctctga atatccagat
21121 ttcttctggt cttctagcgt caatgcgtgt gcctctgcaa tgcctcctta atccactaca
21181 ttgagagtca ttgctccttt tcctgtgttc tcactttatt tacacttttt gcatgccaat
21241 gacaaaattc tgccttgtat catcggcaga attttccatg tctgatatat ctccctctgg
21301 attgtaatac gctcttaaag gcatcgttca gattcgaatg atcttagtac aactcccctc
21361 tgtgaaggcc aaatggcaca caagccagga gctagaaagc cctggttgcc tgtggaaatg
21421 aatgaacaca catgggctgg cagccgtgtc cctgcacacc tgccttttct cagaggtgtg
21481 gccctagcag ggtaagctgt ctggaagagg gagctatttt gtgcagatgg aacattttgt
21541 ccccttaaat catgtcccat gaaaggccag catggcatcc tgcttctgaa acaagcactg
21601 agctcgtctc catttggaga agatctgtcg tgtgggtatt tttaaatggt gaaaggagaa
21661 atgggtatat ctgataccat ggcacttgtt acactttaag cacagactct gttattcata
21721 ctccatctat tgactcaggg aggagtttat ttcaaaccaa acagctcagc tatcccagga
21781 gcagctgcct cagaaggaag gaggatgctg ttctatttcc cccatcgaac ctccttctca
21841 ctcgatccac aacagccttt ccatgcaagg aagaggctat tttctttcaa gctttattct
21901 ccattttccc tgtggcaaga aaggtgattg gatattggga tttgtaatgg ttttatctga
21961 aagccattta aaatgaagct ggaatgtcaa caagactgac gctgaatgga gtagatgtga
22021 agtggaaatg aacttgaaac aagagatggg gttagtcatc cccctgatca cttggaacag

22081 atgatggagg tcccctgcct ctgctccagt gaacagaaca gaaccacttg aatactgcca
22141 gccactgttt gtacataccc tcctgaaacg gctcttccaa aatgctgaag gacattttgt
22201 tttcttaata atttggcctt tccactgttt ctgtatgatt gatgtgtttc agcaaacctt
22261 ttttaaaaaa gaatatctac aggtaaattt agctcatcat gtttgagaat attcaccatc
22321 tctaaatccc cagggcctaa atcaatgagg ttttagcaca ttttgaaaca gaaccctgat
22381 gtataaattc tgcaatggct gatttcacgt attttacttc ctctctaaca aagctatgga
22441 aaaatgcaca agcaggggag ggtttgatcc tcggtgtttc ctgcagatgt gaatctgaca
22501 ccaccttgga gcagggcccc tccccacctt ctcccaatcc tccgctgggt tgctgcattt
22561 ttcactccaa gagaccccgg aggacagcta tatccgaggc ttattaaaat cttctaggat
22621 gcaaaggcct ttggttattc agagcagcag gtctttaggg gaagccccca tacacaaaca
22681 tgctcagctg attctcaatt tatattgtgt gttacctgcg gggaaaatta aaagctcaaa
22741 cctcaggcac tttccctctc ctcccttgat tgcccggggg cttgttgttg agatatttct
22801 tgttgatttt tttgtttttt atgtctctgg ctggtgctaa acattctttc tttcccatca
22861 tgtttacttc acatctcttt gccgtataga tagcttctgc tttatggttt tttcctcctt
22921 atctcagatg cagccagccc ttccccacct gcccccgccc caaaacacac acacgaacac
22981 acaccctttc agactgcatc cacactctct gtttcagtgg atctgacatc aacgaccccg
23041 ccacccccgg ccctcgtcag aggggagctc tgcattgaac taggactagc tcctgggtca
23101 taaaagttgc taggaccctg gagagctctt actaaacaga tgaggccacc actgacagag
23161 gagagaggtc agttcccgga gggaacgagc ctgctttgct gagccccctt ccttgcccag
23221 cccccagacc catcacaggg aaggttcaga ggagctctga cccctccagt acctccttgg
23281 cctgacgccg ctctagcccg ggctcccagg ttttctgtag ggagaggagc acagtccgct
23341 ccacccatgc catgggcttc atcagtgaca acccaaactc acccccaaat aggaatggct
23401 tcaacctgga gacagccctt caatgaaatg gtttatgctg gtggagattt aaaggtattc
23461 gtctttcggc tgggcgcggt gactcacacc tataatccca gcactttggg aggccgaggt
23521 gggagatcac ttgaggccag gaattcgaga ccagcctgac caacatgcga aaccccatct
23581 ctactaaaag taaaaaaatt agctaggcac agtggcgcat gcctgtaatc ccaactactt
23641 gggaggctga ggcacgagaa tcatttgaac cccggaggca gagtttgcag tgagccaaga
23701 ttacactact gcactccagc ctgggtgaca gagcaagact ctgtctcaag acaaacaaaa
23761 agggtgtttg tctttcaatt gcaaaaatca agcatggtca ctgaaaaaaa attcaaattc
23821 acatttcatt ttgttttgtt ttgttttgtt tgtctttcag ccaggtgcag tggctcacat
23881 caaagtatgc tgctttcaat atcttctcac tctccctccc cagggataac cactttcttt
23941 tttattttat ttatttaact tttttgagat ggagtttcac tagttgccca ggctggagtg
24001 caatggcgca atctcggccc accacaacct ccgcctcctg ggttcaagcg attctcctgc
24061 ctcggcctct tgagtagctg ggattacagg catgtgccac cacacccagc tatttttgta
24121 tttttagtag agacggggtt tctccatgtt ggtcaagcag gtctcgaact cccgacctca
24181 ggtgatccgc ccgcctcggc ctcccaaatt gctgggatta caggcgtgag ccactgcgcc
24241 caggggggta accactttca gtggtttgag atgtttgtct cttcatagaa gaaatatatg
24301 catatagttt gggttttggt atttttttta attaatggag ttctaatata catgctgcct

24361 tttttccctt accatggaat tgacacagga taataaatta attctaagca acaaattttt
24421 ctttaaccca tcctgtctct aggtttctcc tcacagaaat tacaacccct tctcttgtgg
24481 ttccatccca tccctaaaac agtatttatc ctcccttctc tgtaccatta ctgtttcttg
24541 tgcgaaattg taccttggag tatgtactga atggtgttgt actggaggcc tggcgcggtg
24601 actcatgcct gtaatcccag cactttggga gaccaaggtg ggcgggtcat ttgaggtcaa
24661 gagttccaga cgagcctggc caacatggtg aaaccccgtc tctactaaaa atacaaaaat
24721 tagctgggca tggtggcggc gcctgtaatc ccagttactc aggaggctga ggcaggagaa
24781 tcgcttgaac ccaggaggcg gaggttgcag tgagccgaga tcgtgccact gcaatccagc
24841 ctgggaacag agcaagactg catctcaaaa aaaaaaaaaa aatggtgttt cccttgcact
24901 ggtgccctgt tggctcctgg cctcccttct agaccatcag ctacctgtgg gtgggagcct
24961 gtgcctttgt cattggtcca tcctctatcc cgaggccccg ggacatggcc tagaacatca
25021 gagactccag cggctgtttc tcaaagtgaa ctgaactagc tggtgtttcc cacacgatgg
25081 agccccctcg acctgccctc gattctctcc tgacttattt ctccccacat cttgggccag
25141 ttccagctgg actagcaaag agggcagctt tacctctgtg ctcccaacat gccctagtgg
25201 gtggcgtatg gatgggggag cctgggacaa tgtgtgtcca tctcacacac tctctacttc
25261 ccatgccagt ggggaaagtg ggggcaggaa tagagcccgg gagggaactg ccagagactg
25321 tcttctctgc taaggctgac aggaagacgg acaaccatga cagtggtgga aggggaaggc
25381 ccttggccca cggggaatgg ctgtctggaa gctgacttgt tgttgggggc ctggtttggg
25441 caggaacccc tggggtggct gcagaaggag gctgggcttc acagacccct gtcggcaggc
25501 aggggctacg ccccctatac taggcttctg ctcagggcag cagggcccgg agccaccagc
25561 agagctgagg aggctgcaga ggaggctgag agatccatgg aggaagaaca tggtggaaaa
25621 gagtgcctga ggtccgcttc cacggccact ctgaccagcc tgggccttct gtgccttagc
25681 attcccgttc ctaacatgga actcatggcc tttgacccgc ttccctcacg aggctgctga
25741 gagggtcaga gaagaagccg catggggact ggcctcaccc tgggagaggt gagcaagcca
25801 cgaggtccca tgttgcatga aaagtcaaat gctgttagga cgactcccag gatgaggagg
25861 tctgacccca tgctcatcgt ctggacagct ctgaccctag aatttatttc ctctttcccc
25921 acagggcaat ccttctactc tcgggtcctg gagaactgtg aggatgtggc tgactttgat
25981 gaggtaggag gcccaagact cccccctccc tcagccctga gcccagaggg gaacagccac
26041 ccttcgctgc ctcttgcggg cacacactgg ccccaaatcc caggcactgg cctgctctga
26101 gcccctcacc tctcagctgc ttcctgaagg aggtggtaag gagggtgtgc aggaactccc
26161 tcccccatgt tccccccaga aacctctgca caggtgaggc ccgtgtgcaa atgacacggg
26221 gaacagcagc ctcgcccacc tcgacggccc tgctgggtct gggcctgtta ttccctgtgg
26281 agaaagagcc cacaggtgct gtagacctga atgtgagcca ccagctgagg gcccaacgag
26341 gcaggcgagt gttggggacg tcgcaaaggc cagtctgtgt cagtccccag ggttgaaga
26401 gcagggcagc cccgggcact gaggtaggag ccccagtcac cagggaaaca ttgtcaagca
26461 ccccacctga gtgagggcgc catctccaga ccttgagggg aagcataggg aacagaagac
26521 agtctggatt ttcagaaaca gctctggggg ttccatgaga aaagacgtga gctgctggtc
26581 tattgacaac gatttttctc aatcctcact ccagctgggc tgttgtcaca attttatcag

26641 caaaggagca ggcgacacca tcccacaggg gtgcccagga gcagtgagag gacttacaac
26701 gcttccctct ccctgccctt cccctcggta cctggccggg ctgggagccc tatcctcaca
26761 cacacacacc agctcgagca gcctgcggcc tcctccgcct cctcacattc tttcgtctat
26821 gtgtttgctg cttccagaat ctatattccc ctctggctgc tgtaggttct ccagggatga
26881 ctttggagtg agggccagca gtcatccgtc accatcttag cacaaactgg aagccatgta
26941 gtgaccctca aatagtgtac cccctctctc ctggggttgg ctctcccgct tggctctgtg
27001 ggggctggga agggcctcat agcagactgt gcctcttgag gttggctgct ggcgttcggc
27061 tgagcagcca ccaggctgac acttggcacc agctgtctcc ccctcctcca gaaaccaggc
27121 cacacgatgt ggctgtgtcc atcgacccag agctggagac acagggagca aaacctcgct
27181 cctcactcag gcttaggcac agccatcaac catcgcggcc cagcaccaca ccctccccaa
27241 ccacttcagg gcgcagttgc cacgacctcc ctggtacgtc cccactgcta tgcaggacag
27301 gtgaatcact catttaaagt gaatcactag agctaaggaa gtttctgtcc ttaccttctc
27361 cccctcctgg ctgtacaatg acctcagttt ctaaacctgc caccatcttt gtcaaatggg
27421 gttggtgatg ggtgtgaagg tctctgtgaa atgatgaaca aaatgaaggg ttatggtgag
27481 agtggaatca tggctgccca ctgagacagc tgtcatagat gcttcaggct tgccccatta
27541 cacacccggc ctgctccctc ccctccccat ttcctcagtc agggcactgg gctggctggg
27601 agaagatgcc agcagcctgg cccagtttcc catagtccta cctccctggg cttctgccca
27661 ctaatccacc aggcacagca tctgactccc tcctcccaag actcccaggg gatatgtcag
27721 cccaggcgtt cagccagggg gcaacctgat gccccttcca agggcactgc caccctttc
27781 catgcccagc gccttggaca cctgagccac aggtatgcac gacattcaaa gttaatcacc
27841 atgtcgttcc tcttcccaac tccgcccagc ctgcctcagg gggagagtct gtctctgctg
27901 gccctgaaca aggggccaca ttccgagcat ctagtgcatg ccaggccggg cttgagatat
27961 cccagaggca ggaacctggg ctcaacaccc catgatgatt tgtatgggag cttagggatg
28021 gggtagccag agtcagtgtg gagagactgg ggcagggcag aagcagttat ggaagaagca
28081 catgactagg gtcagagagc agggcacgcg atggggagaa gaggctcctg cccggggaaa
28141 gtgctgcccg cccactgggg agacagggaa gtttctggtt gttgcaatga ggagcctact
28201 ggcatttagt ggggtgggga gcaaggatgc agaatgactt gcaatgctca ggccatcccg
28261 cacttcccac ctgggagccc cgtgggaaga tggtggcagc cccagcagca gcactgagct
28321 gcttcccgag ccatgcccct tccactcaat ctccgaaatc atccacagac gaaattaggc
28381 tcagatggta actacccttc cccaggtgac agagctggaa gcggccaagc cagggttctg
28441 aattcatgca tagattagac ctaagccctc tctggataca gtgatagggc ctgactccca
28501 ccacccctgc aaggcccgtg ttggagtctc tccacacccc ctgcctccat ttctccctgg
28561 gcaaagtggc ctcagccctg gccaccaatg gtcagcagtg cacatcccac aaagcaggaa
28621 ggccaccctc ccattccctg ttcaggaacg gcccggaggc ctgggtcagc ccagtcccaa
28681 aggtagcctg gagccatacc ctggctttaa cctttctccc tagacccagt ggacaagttg
28741 taaacttccc tggcttcccc ctgaaagaat ctgcctgcca gtggttccag aatatcctgg
28801 gaaagagccc tttcaaatcc ccctgaacct tcttgacttt ccagaaagcc tcttctctcc
28861 gtcttactct gcccagggcc tgccggagcc agcttagtga gacacactgg cctgggagtc

28921 atgagaccta caccacagtc cttgctgagg aacctctttg ggctttggat tccccttctg
28981 taaaagagag atatgctccc tgctctatct gccccacggg actgtcacaa ggctaaaaaa
29041 agatgaaatg agagacaaga aagcaaatgt ctctcacagc tctactaggt aaggggttcc
29101 aagcaaggga ggggcccctc ctctgctcag gcatgggtga gaggggctgc agcagctggg
29161 cttaggaacc tgagtgggga ggaggatgcc atggaagaaa gacaatgctc tctacaacaa
29221 tgggtggaaa cagagcctgg acatggtgtc tcctttccca gaggctggaa cctgagcccc
29281 tacatttaac ctacaactct ggaaaagcta gggagagaca gagaaagacc cagcctttgt
29341 gacctgggga aacaggagta gccaccttta gccagtttgg tttggctcta acacaggccc
29401 tgtgtggagc aggccccagg ggagaggcca gagtaacaat agtacttgtg ccaatgataa
29461 gaagaactgc tgctatttac tgagcactta gtgtgtggca agatgggctg agagcttgac
29521 acaagcatta gactcactag aacttgctgg attggtagca tcctaggtga taaggagcca
29581 caacaggatt taaacccgag tctatctgat tccaggacct atggtcattt gtaaagtaga
29641 caagactcag ccctgccctc aaggggctca gtctagcggg cagatggacc cactacaaac
29701 aaggagggac cctgggcctt ctgagccaag aggaagaaga ggtctccagc ctgggggatg
29761 acagggcagg ggagtttggc cttatctgcc cctttctgct tgtaccagga agtcatggct
29821 gttacctcct ggaggccaga aacgcacctg tcttgttcgt cattggcctg gcacccagca
29881 tggagttgag aaaatgttgc cgagtgaaag aatgaaaaaa acagtgacgt tcactaggac
29941 tggcacctgt caccgcaggc ctgtgcccag accgagtggg ctcagcaggg ctagtggtac
30001 atcaagccct ttgctgtggc ctggatagaa aggaagcagg caaggcacag atgagatcac
30061 ctaaagggac tttagatcac cttgatctct aaggttagag atcatctagt ttaaccctct
30121 tgtcttgcag ttgaagaaac aggtcttaga gtggggaaga gaattgccca agaagaccag
30181 ctgagatctg agatcctaca tttaacttac aactctaaag aagctgggga aagagacaga
30241 gacaaagaca gaaacagaga gagagagaca gagacacaga gacccagcct ttgtgccctg
30301 ggaaagtgaa atagccacac tcgggatact gagcccaggc ctttaagctt gcaaatccgt
30361 ctctttccat tgtactctaa agcagcagtc cccaaccttt ttggcaccag gggctggttt
30421 cgtggaagac agtttttcca cagactggga tggtggtggc tgggggttat ttgggatgaa
30481 actgttcctc ctcagaacat caggcattag attctcttaa ggagtgcaca gcctagatct
30541 cttgcatatg ttgttcacag tagggttcac actcctatga gaatctaatg ctgtcactga
30601 tctgacatta gatggagctt aggcgggaat gctcgctcac ccgcggctca cctcttgctg
30661 tgtggctctg ctcctaacag gccatggact ggtactggtc tgtggcccgg gatatgggga
30721 cccctgctct aaagctttgt agtgcatctc aatattttcc cccaaaccta tagccctgtc
30781 cctttaagga tgctaccttg ctgatttagg atggttccag gcccactcag cggccaagct
30841 ggacataatc cagatcattc tccaaggtac atcagggaaa ccttttttgc agagtcctag
30901 atcactgggg ctgataggat gcagaaccct gtgccccggc cagcaccagc caggtcaggg
30961 ctaagcctag accaggaaac tgggtcagga ctcagcactc cccgggctcc tgagatcact
31021 ccctccaaca gaggccctct ttccctccct atctgtacta cctaccacct tgcttctgtc
31081 aacagtccta tcccaggctg ctattccagg tctgtgtctg tctccagaac tatccaggac
31141 catccccagg actgagaaac aaccctgcat ccctgcgttc tgcacagcgt catgcacaga

31201 gcctggcaca cagcaagtgc tcagcaaatg ttcgccccca gcatggggct tgctggctcg
31261 tgacatcttg agtgactgtg tttggcttaa ctgctacttg agtcccccag cagtaccagg
31321 cttcaggaga ggattcagtg tagtggatta agccctgagg actgaactcc agactaagca
31381 tcctgagact tgagtttgaa tcctgcctct gccaccaacc agccttgtgc ccgcgggcaa
31441 ctctcgcctc tccactttcc cacttgtaaa aggagcacat ctttgccatg ttgctgtgag
31501 cttggttatg ggtaaatgtg ctctggaagt actaagtcct acccagatga aattcttcct
31561 tcagaagccc ttttccttca gtcttttttt tttttttttt tttttgaggt tggagtctta
31621 ctctgtcacc caggctggag tgcaatggtg ccatctgggc tcactgcaac ctctgcctcc
31681 caggttcaag cgattctcct gcctcagcct cccaagtagc tgagattaca ggcatgcacc
31741 accacacctg gctaattttt gtattttagt agagaccgtg tttcaccatg ttggccaggc
31801 tggtatcaaa ctcctaacct caaatcatct gcccactttg gcctcccaaa atgctgtgtt
31861 tgcaggtatg agccactgcc cccagccccc tttccttcaa tctaatagct cacaagttca
31921 ataccatagt caggagagga gtgtagctcc caaaccctca aaatcctcat ctcgtcttgc
31981 tgtgttctca gcatatcacc ccaaatggag gaaaggacat aaccacaaaa atgaggagac
32041 aatgaaaaat gaaattaggg aagagaagat gcagtgaaag agacacgtga aatccacagg
32101 ccgaggcaga agaggaaagt gatggcggcg aaggggaggg aggtggcatg acaaggagac
32161 aaggcagttg cagacctgca cagagttccc actgggaaga tctaaactaa acacattctg
32221 ccgtgctgtg tcacttacga ctgctgtcac ctgtgagatc ccatttgatt ctctcaatag
32281 ccctctaagg tagatattgt cacccccctc ttttcacaga tggggaagct gaggcacagc
32341 tagtgagtgg cagagccagg gctagaactc aagtctcctg actcaaactc agagtttctg
32401 caacaccacg gagactccca gtctgcgtga gatacaagcc tcggtgttgt cagaagaaca
32461 aaaaagaagc aaagggtggg catgtccttt ggagctctgt gaggtgccca gagctcacag
32521 aggctgcctc agcaaaggaa ggtgtcagca atgggagatg gggctggaaa tgctgtctgg
32581 gcacagcaag gggcctctgg ggtgctagga aggggaatcc ccacctcttt cagggagctg
32641 tagaggagcc actgaaaagt tttaacaaag gagagataaa aagagaggaa aattcaaccc
32701 caaaagtctc cttcttacag cccatgcctc tacttccagg gaggccaaat gggagtaacc
32761 cccagtccca gaccctgagt cacttccctc cagccaagag gcccgtcacc ctccgaggcc
32821 ccacactgat gccaggaccc cacctagagg tgccggcttg atcggtctcc aggctgggcc
32881 ttgggatttt taaaagcttc tggaatggat tgcagttcca gccaagatga agaacccctg
32941 agaaagacag agggagacag agacaaaggg agagatagag acagagagag aaactgatga
33001 aagaaaacag atgtggtgag gagaagaaag cagacgaatg atgcgggagg gaaggaaaag
33061 aaatgagggg aaagcctcgt gtgggtggaa aatgcgggag ggagacaggg gaggaaagaa
33121 aggaaactgt aaggtgggag caagtcagag accgaagaag ctgctgtgac tcagtgagga
33181 ccctgtcttc tccctcaccc ggaaagcctc caagaagaac attccccgct gacataggca
33241 tggactgccc agacccggct gacgaatttc tgaaagggca cctgagccac acacctacac
33301 acgcttcccc tgattattct catctgcagt caggggcccc ggaagccccc tgtctcctgt
33361 gccctccctg tcccccgact ctcactcaac aaactggccc tcgccccgca gccaggcagc
33421 ctccctcccc aggtccctta gcctcagcct cccgccaccg ttcaacaccc cacctccctt

33481 tcacagggcc atctccaaag tcatgtcctt tttctcctga tctttctctc cttccaatgc
33541 caaaatccca cccacagacc ctcaggggga ccaatcaccc ctcccctctt catccccagt
33601 aaaccaaatc tcggtggctc ctgcggcccc ccgactccca cacagcgccc tggctgatga
33661 gctgggcctt cctgggctgc tttatttccc tgcagcccca taaaagcagc ttcattaaga
33721 aaaccacaca ctctgctgag ctgccagagg catccagaag ctggagccgc agccgctgaa
33781 tgaggcctgt gggccgggct gcctccactt cctgggactc tcagcgcacc cctccgccct
33841 gtgcccacct cgcctctgaa gccctcccta ggccctgctc agaaccctcc ccaccccacc
33901 ccgcagaccc ctctcccgct cctctgcttc ttcctccctc tcatgccaag gccaggtcct
33961 cccttgccag tcctgaaggg agatgtcaca aaggccgttt tcctgagaca ggccctgccc
34021 acactgggca gtgacccgcg gtgctggccc agtgtgccca agccctccaa gtccccaggc
34081 tgggagaagg aatctcagct ttgggaatgg caaacgtgca gggtgggctc acagaagcct
34141 cctcccgagg gcctgctcag ccagcaacag agtgctccaa gtcatgcatc tgctctgagg
34201 ccgagttcct tgtttataga gcacagaagc cattacccac ctctccggcc attggaaaga
34261 ctaaatgaat gaatatgcac gtagagcatt cagacctctg cataagaaaa agttccccag
34321 aaatggcagc gattattact attattattt attactggac tattgagcag tctgaatttg
34381 gtagggtata aaaatggaac taaaaaataa aaagtcactg ccttctacca gatctgtcaa
34441 aatcctccct gacacggtct ctccacagcg tcttgagcag acctcagcgt tgtctggtcc
34501 tgtccagaag ggcttcctac tccagtctgg catcctgttc acccctggcc atcctccttc
34561 cctcatgctt gtggctcagg atgtggagtg ggacagggac ccctgctgag gccctccaca
34621 tcctcccctg gtgggcagcc catggaaaaa ctgtcctcat atcactctac cccacttggc
34681 caatggagcg caaatagatg acttgccaca gccaacagtg gcaggctggg ctccttgccc
34741 tgctgtactc tatgggtctc ctgccagtcc actgccctgc agatgccact ggggatttgc
34801 atgccatgct gcccctcttg caggtatcta atcaccatga gctgtttccc caagaagtac
34861 cacaaaaaca ctacgcctag gacagaattc ttcccacctt tcccttgccc agtcttcttt
34921 cccatcccag gaaggtgggc tgggtgatgg ttggaggtaa cagggccttt gaggaagccc
34981 taggaagatg tctggctctg cccattggca gctttttaca gaatggggca ctgggccact
35041 ttagcctctg ttttgagcca ttgttgccac ttctagggca ccaggaaaaa tcccaatcaa
35101 attcctactc atcattaaca ttgagttagg gaggaagcaa cgaagaagtg gtaagggtga
35161 atctagaaac aatgagttgg ggacaggctg tgggcctgac cccaataaca ctaatcagct
35221 caaacctgtc cccagtgtta ccactggcag agtgaggagt ggtttgaact caacacagga
35281 gtctggggca gagcctgtgc cagtcagggt gattctgatg agagagagct ggaagctcac
35341 agctccattc agcacccacc aaaaaccagg ctcacaccca cattccttgc tgtctccctg
35401 ggacccgacc accaccctgg ttgcaccacc ctctcccaac ccccctcagg agctgggagg
35461 ccatggaagc agcgctgtgt agggagtcaa aagtcctggt aggtccaggc tcttcaccta
35521 ccacatgacc ttgggcaggt cctccaacat ctctgagcac catctttgaa agaggagtgg
35581 gcaacaatgc cggctctaca aggtggttgt tggaactaaa tgagattcca ggtgtcaatg
35641 ctttgtccct ggggagcact gggcccaagt gaggggccac tctgactgtt ggagatcccc
35701 acaagagatt cccctcccac accccacggc tcctacctgt tattgccacc gaagccgcag

35761 ttgtccctct ctccccagac atttcggtgg ccggtggcca gcagcatcga cagaaatgag
35821 atgctggaga ttcaggtttt caactacagc aaagtcttca gcaacaagta agtgtgggat
35881 ggggcaggag gggctgggcc tggacgggct gggagcctgc tcttctcctt gggctaccct
35941 ctgtttgggg cttaaaaagg ggacctacac tcccaacctg ccctggattg atcttcaaag
36001 accgaagaaa tcttgggctg gtctttgatc cccattttga tccctcttct tgtcttcaga
36061 atcccccagt tctcccagct gctgtcagcc aagaacactc caccctccta tcacctctgc
36121 tgtctccaac cccatcaaag cttcctgtct taaagactta aagctttaag tctccctcaa
36181 tgccacgctg gggtacagtt acccactgga gaggcgaaag gtggaggggc tgatgatctc
36241 ctccaagaga cactctgtag ccctgagggc aggcttgaca gtttggggtg gtggtttagg
36301 agtcaggcaa gcctgatctc agacactcag gtcagtcatc tactgtgtga ccctggaaag
36361 gttacataac ctctctgatt tcaatgtgct catgtgtgaa aaggacaagt atttacctca
36421 aagggcagct gtgtgaatta aatgagatag atattcactc actcattcaa caaatgttta
36481 ctaagcgtcg attgtgtatc aggtactgaa ttaggtgatg cagagggcgg ggtgcctacc
36541 ttcatggagc ttgcactcca caagtgaagt acacctgcca tacgagcgtt atcactataa
36601 ggtgttcaat aaaagtagtt gtcatccttc tctctgggcc atgagaagaa ttcagaagga
36661 tttgaacacg ctggaacagt ggctaagtaa tatcttgggt taaaaaatca cagccaggtg
36721 tggcacacac ctgtagtcct aggtccttgg gcagataagg cgggaggatc atttgatccc
36781 agtaatttga ggctagcctg ggcaacatag tgagatccca tctctttaaa aaaaaatcgg
36841 ttgctcttat acaagctgga ggtcatggct taactgtaac atgtccaaga aggtatggag
36901 gtgttagctc actgaggact cagaatgact caacagtggg aggagactac aaacaaaaga
36961 aaagtaggcc tcatcctaaa tcatactaat agaattaaag cactcctgat ctgagcagga
37021 gagacaagga gtccactgct ctgatactag tggcagagaa ttcctgtagg caccacccta
37081 tagtttccag gaggaaaggg actttaagtg ttaccttata acaaatggat gagagcctga
37141 aagggaaaat catagaggac atattcatcc attcatccat ccattgatcc attcatccat
37201 ccatccatct atcattcctg ttattcatcc atctgcctat ccatttgtct gtccatcaat
37261 tgatttatcc acccttcctt tctttcatcc atccttccat cctttcatcc atccattcat
37321 caatccatcc ctccatccat ttatcaattc atttatccac ccatttatcc atctatccat
37381 tcatccgtcc atcctttcat tcatcctatc catccactca tccatccatc catccatcca
37441 ttcacccatc cttcctttca tccattcatt catcaaccca atccttccat ctttccatcc
37501 attcatccac cacttaccct ttcatccttt ccattcatcc ttccatccac tcatccatcc
37561 attcatccat ccttccacct tttttctttt cctttttttt tttttttttt ttgagacaga
37621 gtcttgctct gtggcccaca gtggagtgca gtggcctgat ctcggctcac tgcaacctcc
37681 gcctctctgg ttcaagcaat tatcctgtat cagcctcctg agtagctggg attagaggtg
37741 ctcaccaccg tgcccagcta atttttgtat ttttagtaga gatggggttt caccatgttg
37801 gccaggttgg tatcgaactc ctgacctcaa gtgatctacc ctcctcggcc tcccaaagtg
37861 ctgggattac aggcgtgagc caccactttt ggcccatcct tctatctttc cattcactga
37921 tccatctatc gatccatcca ttctttcatt ctttcatcca tccttccatc catcatcctt
37981 ccttccatcc attcatccat cctttcattc ttccatccat tcatccatcc atccatcttt

38041 tcacccaact ttgcagtcac tcatcataca tatagtgaac aaatgctttc tgccagtcag
38101 acactatgct agtcactgga gatactgaga tgaggtattg aggcacagtc cctgccccta
38161 ccctcccaga aggctttttg ccacaggaag aggaaagaaa ggtgtgtacc tagaggcagg
38221 atgaggaaca ggctgctgga agacagcaga caactgaaag atagccagta ttttccagcc
38281 aatgtgctgt cttaggaggt agtgagctcc ttgccactgg gagagggtga gcaaagagtg
38341 gaccacattg tagaggggat ttctgagtca ggaaaggccc caccatatgg gctccaagga
38401 gtctctgatt ctgtgggttt tattaggagc tttcaaaatc tcagctgagt atggggcaag
38461 ggcatttcag aaatgctgtc agggccacag ggaggatgtt tagtgcacaa aggccttccc
38521 tttgcctctc atggcatcac accattcagg cagctgcttt tggcacccac aagcaaggtt
38581 ggtgagacac cccccaccag gggcccccaa ggtgaagcag agccttcttc cctggggtga
38641 gataccatga gggtgtgacc tatgaggaca ggagaaacag gaacaagtgg aaggctcaca
38701 cacagaccaa gtgggtgtga aaaaggaagg agaatcggaa gtcaagcacc agggagggca
38761 cctgagagcc cctgaaggaa cccaaccata ggtgccaaag catccaggag ctttacagct
38821 cagccactac agccctagct gtgcaggagg ggaaaccaag tctcagaggg gaacctgagg
38881 tcacatgggt agacaatggt agagccaggg ctagaatgca tgtctgttaa cttctcttcc
38941 taatccaact cccagaaacc aaaatatttg agcagtgatg gtggaaactg agggcatgga
39001 agacattgtt aaatcattta taatgaaagc caccagccta accactgaaa tgctggaaac
39061 acttgagccc atcaaaagac tgctacaaac aaatcagaag tatttgggaa atcgggaaat
39121 tgactcccat gcagtgaata tgttgaaagt tacagcttct attaggtgtt tttggttttt
39181 gttttgttca gagacagaat ctcactctgt cacccaggct ggagtgcagt ggcctagtct
39241 cggctcattg caacctctgc ctcctgagtt caagtgattc tcatgtcttg gcctcccgag
39301 tagctgggac tacaggtgca tgccaccagg cctggataat ttttgtattt ttagtagaga
39361 cagggttttc cccatgttgg ccaggatggt ctcgaactcc tggcttcagg taatagacct
39421 gcctcagctt cccaaagtgc tgggattaca ggtgccagcc accaaatcca gcctgtatta
39481 gatgttctta agtgccaata agaaggaaga cagaaagggg aatagttcct atcctaactc
39541 tcctcttctt ccctccctgc ccccatccaa gagaaggcag gaaaccccac aaggggaggc
39601 ccaggcctgg tgggagaggt tctggcattt ctttggaagc ctagagaatg cccagggcac
39661 tgctggctgc agagctgaga tgggaaagaa gaggtcccct tgtggagagc acaggcccag
39721 gctgacacag gccagcttgt gggggtcaga gtgggagaga ggtgtttgga atgatgtgag
39781 actcaagtct ccccatcctg gcatttgcct gggccaggcc agaggagcaa tgaggccact
39841 gccctcaacc cccacatggc ccagttcaga ccccaggctc cttctcatgg ggcagctgag
39901 tggaaggggt ctttggggtc tgcactaggc ttagcaatga gcaaagccat ggccactgct
39961 ggggacagtg cctggcacat ggcaggctcc tgattatttt gtggaataaa agaatgaata
40021 aatgcccctg tggtgttagg aggcagaggg taggtataca aggtgggaga gcctttgaga
40081 gggaatcgga gtggcccctc ttctctgaag gactcagggc actcaagggt gacactcagc
40141 atcacatgac cgatgcattt ggggaaattg agctaaaaga ggcatggagg agctgcctgg
40201 gagagtgggt cccaggacga gaaatgagcc tgatcccatc actgccatct cagagagaga
40261 gacttgtcct agaagcagag agtagcttgg aaactggccc ctcacccagt ggttctagag

40321 gctgccagga ggcagagaca tggtatgaag gaaggaggca tcctaggcag aaaccaggag
40381 acctccaggt ctgcttggcc ccgtttggaa caaagacctg tgtccaggaa ccttgggctt
40441 ccccatgaga aagaaatgtt cggttcagaa gattttgtgc atccagctct gcctgtccgg
40501 gacctctgag ggtccatggc ctggcctcca gcctcctgcc tgctaaggat gccagggacc
40561 ctgaataatt caggagccag gacagcagga gaggcctcgc agggcccagg acacctgaca
40621 cgggctccca ggggaaagtg atgaattatt gacaggattc tggtgcccag ttgccgctct
40681 attgtgatca gtctgtgggc ccatctgggc tggaggcctt ttcctgccgg ctaattacgt
40741 ggggatctta gctctgccca gccgcccagc ccctcgtggg gccagacagc ctcctgtgag
40801 gggtgtcctc tttgccatgg tgacctggat ggccctgtaa ggcaagtagt cagaggacat
40861 ggagttagcc ccagatcctt tgcctgacag ccccagggaa actgaggccc agagaggcct
40921 cttgtccagg gtcacacagc aggttcagga cagtcaggtc ttcaccccag gcctcctgac
40981 acccgggcca gtgctccttc ccacagaaca catggcctca caactccatt tctagggaaa
41041 tgactcagct gtctaaggag catatgagcc cacccttcct caatcgtcct gggccccact
41101 ctcccaggca gctcctccat gctgacccat gcagaggagt aagaacctac tatgtgtgca
41161 gccaggatgc acaaaggcca gctgcggcgg gcagggtcgc ctctggggtc accacaaggg
41221 ccagagccag gggcttctcc tacctcttcc tgtcccagct tcttcccaaa gaatatctat
41281 agaagacggg ctgagggatg gcatcctaga agcagtgcca gggcctttgt tttgaaactg
41341 ttttcatggc aagcactgtg tttttgctta aatggtatgt tacagacctg tagaaagagt
41401 caaggtttat aaaactgctt ttctttatac tttacataag gctgagataa tatcaattgt
41461 ccatgtcaaa aaatataatt tttactttta tttgagagag gttggggggt gaactgttag
41521 gagttaaaac cccccaagcc actcttggag gtgtggcaaa ttgattcctt taatcttgca
41581 agctcctagc gtcccagtcc caccctccag gccatagaca agctctggct gtgcgaggct
41641 gtgcacagct tgaagggtgg gggactgcct ggacctgccc ctgaagccca tcctgcaaag
41701 gagactcgcc ctgtgtaggc tctccagatg gccagccctg ctcattatta gggagggagg
41761 aggggctggc atccgagtgc cggtgttggt agaggaattt cacatgagca gtctcctcac
41821 cactgtccgg tggaagtcgg agggctatag cagaaggagc caaaaccaca ctgctgccag
41881 gaggtggaat ctcccttggg tcctgctgag tgagcccaca tctgtctgac tccagagccc
41941 aggctcctca ctgtctacac cgcttccctt tagggatgga attaatctgc atgtggacag
42001 ggcttggacc agggtaggaa gagtagggga tttgcctgga ggccaatctt gctcagcaca
42061 ctgtgtgatt aaggcagtgg ctgaatggac taccagtgac agccccatca ctgtcaccac
42121 caccggagta tctagcaggg acttaaaagc acatgtcagg gaggttgttt cagggattcc
42181 tcctggaagg ggcgctggac aaggtctctc aggttcttcc caagtataag atctgagatt
42241 caggcaggtt aagcctagaa ggcaccagac gcctctgtga gcacttgcag gggaatagc
42301 tccaccctgc cccctgggag tgtcttgtca cattgcagca cctctgcgga tcctggggag
42361 ctgccttggc tgttcttagg catctaattc tggagaaaga tgtgcctgat cgccccacgc
42421 tcaacaggtg ctcgacaggt cctaggggaa tcaaggcagc tgaatagcca tccacaaagg
42481 gtaatagtag agcagggatt ctcaaagtgt ctccccagcc agcagcttca gcatcacctg
42541 gaaacttgtc agaaatacac attctcaggc cctctcccac ccactgaatc agagccctgg

42601 ggtcggggcc agtggtgtga actgaagtcc ggtggaccca gtttggtgac ttcacattag
42661 aagcttgaag ttggccatgg cgggagtatt tatatctcag aaaccagcaa atgctacaga
42721 tgagcacctc tcctctccca ccagtgacac attttccagc atatccacag aagaggatat
42781 cccagccgtg catgttttca caagccctcc aggtgattct gctcatgctg aagtttgaga
42841 accactgatt tagaacaaaa aggggtgagg ctgcaaaaag atgagtgtat tttttcgtgc
42901 ccgtggtcta ctgttagaat gtctacatgg tacggaagct aaaagtcccc ttccctcttc
42961 attttataaa gaccaaattg agactcaggg gcagcagctc cagggctcct ggcccccagc
43021 ccagcacttc tgtctctggg gcactgcctg agctgggaga ggtgcatgca gctatggaag
43081 aaggcttcca tgggctgctc taaggcccca gaagcccagt tcggctttag agtgaaggag
43141 tggcgtggtc agtttcatcc tcagggaggt ggggcacaat ggtggttggg gctctgagct
43201 ccaaagggga gcatttcata tccattcctc atggctgccc ctaccacttc ctcccctcac
43261 acagatggga agatttttaa aaaatcaatg ttcaccctcc acagacctca gggtggtaga
43321 gacatgaagc atgtctgagt cagaattcat ctgatacctc acctcaccca gatctaccat
43381 taattagctg tgtcccttcc ccttccatgg cctcactgtc ttcctctata aatctcaggc
43441 agcatcagat gatgcccaag gtcccttcca gctctcacac gccctggatg cttccctccc
43501 ctttgtgcct tcagctagaa atggtgtgtg tattctaatc cactctatgt ggaagcacca
43561 tgtaagaacg tctgtctgag ttccctgttc tctcccagag caacaaacag ccttctgact
43621 gtggggtgca atggggttga agtcaactcc atcagcattt attgggcacc cactgttgtt
43681 cattaattca gccagcagat attgagatct ctagtgtgct agaaatgtca aacagaggct
43741 agaataaaaa gcaccaccca tgcccttgag aggttcacag tttagaggca gtacatgaat
43801 cacgacaaaa cggactgctt tggagtgctg gctgtgtgct gggggctttg acaagaacac
43861 tcgcagatga tctcattcag tcctccaaca acaactctgt gaagtagggc ttatcagctc
43921 atcttatgag aaaaccgaca ctgagagagc ttaaggaagt tgcccaggaa gcaggtagct
43981 tgctagtcag tgtcagagct gggttcagat ccctgtctct ttgatgccag gactgtgctg
44041 ttaaccaaag cattgcaccg cctctcccct gctgtgcagt tccatgggct ctgccatcac
44101 acaggttcct gccatccagg aacttacagg ctgcagatgt ggaatggatg agtgagtgct
44161 gttggtgtgg gctggaaacc ttggtcatgg ttaggcgggt agaagccgga ggagctactt
44221 gtccatccag gaaactcagg tcctttccca gcctccacac tgagcatctg gaagcttctc
44281 tgcaagggct cagctaaagc tggatttcat gcacctttcc ccgcagtctg tagacatccc
44341 ctaagggtgc tcactgcccc ttggagctct cccaaggaaa tgggcctccc acaaacaacc
44401 atccacagtg ggacatgctg ccctggctgc aaccagtcct ggctcctggg ccaccttctc
44461 cagcccagcc cagagctgga gcaagcccac ctgagtcctc cccaagcagt cacagccctt
44521 gggatgtcag tcttgctcct caccctggtc tcttccatgt taccaccgtg ccccctctgc
44581 catccccagg ctcatcggga ccttccgcat ggtgctgcag aaggtggtag aggagagcca
44641 tgtggaggtg actgacacgc tgattgatga caacaatgct atcatcaagg tgggtatgcc
44701 ccacggagtt cctggacttt cccatatggg gccatctggg ggtctgttct ccctgcctca
44761 cacatggggt ggtctgggga atgcctctca tgcatggcga ggtgtcgcag gaggaggaga
44821 ctcaggtcag actctgctgg aatggctcaa tctgtggcca tggctggagg ccctgtggca

44881 tggcagggtc tgctgcccca cagtaggcac ccctgccctt cctacggtgg gaatgcacca
44941 actggtcctt ccatttggac ccagctcagg agtcactaaa gaggctcctt ttgtcatcca
45001 aaggccagac tgagccctgc tggcactcga gggcatgacc ttcactctgc acatctggct
45061 cgggtcctcc ctgggagggc agggacctca tttcctattg tttgtcacat cccagcactt
45121 ttgcgtgagc acgcgtgcac tagtgatgca cagtctagag gcacctgaca catgttctaa
45181 caaagaagga agcaacccta ccaggacagc ctggccccag gctctgctct gattcttcag
45241 tcctcttgag ctcagccgtg gccactcatg ggcagttggc atgatggtga ggaacaggga
45301 ccctgagtat gaatcttagg ctgctcacta actgtgtgac ttaggcaagc tacatatcac
45361 ctcaaagtga gcacaattac attatccacc tcccaggact attctgagga ttaaaggaca
45421 atacacagaa agtacttagc acagatatgc acaataaatc tataatattg ttagctatta
45481 tttcctgacc tcagtttacc tatgtataaa atgggactgt gccatggtcc cccagtgggg
45541 tcctgtgtaa tatatggccc tatgtagtgg ccaagagaaa cagcctcaga agccaggact
45601 gcagccacag ggagctagaa cagaaggaac cctcttctcc catgggcagg ggtggcaggg
45661 agggcttctt aaagttaccg gggtctcagc caccacagca aggggagaag gggtgcagaa
45721 taagctatgt atggacaccc atgggaaggc gggctgggag gagaacacag aaacctggat
45781 gaagagtgta gtgtgggaag tgagataaga gaattgggga gtgagcaggg atggacagac
45841 agcacagccc atgccctgga ttttcagaat cagacttaag atgtcagcag aggaaaggac
45901 tcacggatct cctagcccac ttgccaccca tctccgctac ccattgttat gaatgggaac
45961 aatgaagtcc tggatgggga atggattcgg ccacagttga cagcagggcc agcacggaga
46021 cccaggactt ccaatgccag agaccaccat tttcccataa caacatactt ggttccacca
46081 agagggtccc atttgttcca gaaaggacat ggggcttgga gttaggcagc ctgggctcag
46141 atcccagctc cagtacttcc tagctgtgtg acttaaagca agtaacttaa cctcctgata
46201 tgctaggtaa ggtaggttgc aatgttgaaa agatcaaatg agtgtacatt aaagcatctt
46261 agcatgatgc ctagcacagt gaggattcag aaaatatgtg cacctttccc ttcccttggg
46321 aaatctaagc ctgggtgctg gagcatccaa gcactgagtg agggccgcct gtctggggca
46381 caggaccaag tcacctctag accagtgagg aagggtgatc cctgtgcact gggggggaaa
46441 ctagagctgc aagaaagtaa tggctccagg acgcagagca ggaagcccaa tcatgactct
46501 ccactgctaa gcctgagccc ggttgagtgc tctacttttt gccagaggaa aaccctaggg
46561 cagctcccaa gattccctcc ctctccaggg taggaagggt gagtgtgagc ctgggccctg
46621 ggctcctctc ccggaggcca ctttcagctc attctgtcca tttccaacaa gccgatttct
46681 ggcgggccgc gcctcatgcc tgcaggcagc ccttgttctg aggagaggag gtggcctgat
46741 catggggatg ctgaacgaca ctctcggtta tgtagacagt ggactcgggg ggtgagtcca
46801 ggctcagttg tcctgggcag gccaggcccc tccaccttga gcaccaggga ggagtggaac
46861 caccagcagc gggacctgga gccaggtacc agtaagctga cccggtctgc acaagtcagc
46921 ctttcagagc ctctgttttc tcatcagcaa aatggggcag aggggatcat accatcctga
46981 cctcctccag tgaggcaagg gtgtggaagc gctgtgtaaa caccaccaag cagccacata
47041 tacggggtgg agattgctgt caccatccca gctgctcacc accgcctccc tcccttcaga
47101 ccagcctgtg cgtggaggtc cggtatcagg ccactgacgg cacagtgggc tcctgggacg

47161 atggggactt cctgggagat gagtctcttc aagaggaaga gaaggacagc caagagacgg
47221 atggactgct cccaggctcc cggcccagct cccggccccc aggagagaag agcttccgga
47281 ggtaacaggc aggacccctc tggctgccca ctgctcccaa gacacgggga agagacctgg
47341 ggcctggtcc tactgacagg cctctctagg cctagctggg ctcagagtac agggctgagc
47401 ttctggagag acatccaagg ggtgggaaga tgtttctgta gccccccacc cagccgtcca
47461 cccccgaggt cactgggcag ggagcaggag actcagatgg agctgcaagg ccaaggcctc
47521 cccagtggcc ctgagggagg tggagctcta acacagggat ggggctagat ctcctgcctt
47581 tctcagagaa ggggagttgg ggataggagg gcccctggcc tctctggttc aagacccagc
47641 agtgtccacc aggtgccggc agcctgacct ctaccctggc ccagtgagga ggcccaagag
47701 agggcagctc ttcctgccag agtgaagcca aagatcctgg ctgggccctg gcctttcttc
47761 ctcttgagaa gggagggtac ccgggaaccc ccagaatcca gcgtcacgcc ctgcaagtgt
47821 cctgccacct atggatcaca ggaagctgga tccaggacag ctcattccac gtgcactatt
47881 tttacaaaac agaaaatgca attgtattac tattagagac caaggggaca gaatctgggc
47941 tagtctagta agatgttgtc catgggttcc taagtcacta ggaattatgt gttaaatgcc
48001 agtaagtcaa gggtgcatct gaaggaccag ggtgtgtttt gagaagggac cctccgtgcc
48061 actggaaaca tatccctgta agctccttcc acgctttcct ttccaagtaa agtttactca
48121 gtccctctgc tgaagaaacc ataggttcta aattagtgta attgaactga catactttta
48181 tgggacaaat atgttttctc ctttgcactt gaatgagttc acaccccctt aagcacccct
48241 cacacacagg tgcatgagtg cacacgtgtg cacatacaca tacacacaca cacacaagct
48301 tcagaaaccc tgggagccag atgagtaata gtaacaaggt caaagtgacc aggcctgcaa
48361 caaacatggc gggactgtct actccaggcc agccacgggc atggggcagc cccaggctag
48421 cttaaagcct gtcccctcag actcctggct ctttagcaga aaacccaggc ctgagtccta
48481 gctctgcctc ttactagtcg tgtgaccctt ggcaagtctc tgaccctctc tcctcacctg
48541 caaagaagga catagcaagg aaatccccct gccctgctgc tgggagagcc ggagggatca
48601 cagaggaaca ggtggcaatg gaggtgctga gcgctgccca tgtccaggct ggtgacagac
48661 aaacaggttt cttgtagcac aacaatgcaa agagtcagga atcgggttgg aaattgtgag
48721 aagaggagca gtgctcagac ggggagtgaa gggatgctga ctcagtgggc accacctggt
48781 cctcctcgac tgtctccatt cagaaaagag gttggcaggg ctggtccaga aaccagaagc
48841 cgggctctaa gaaagagctt ccagagaaca agatcggaag ccacacaagg gccatcagtc
48901 acagggaaag gaatgctgac cccaaatcag gagagctaag gcccattaag tcccatcttg
48961 actgtgccac tcattggctg agtgacactg ggcatgtcac cagcccctgt aggtcagtga
49021 caggaatggg ttcagcaagt tacatctggc tcccctccaa ctctgaggtc ctaagggacc
49081 catggatgct ccggacctgt gggtcacatc catgggaaac aacatcctgg gagcccttag
49141 cagccacgaa ggcaacctcc ctggggtggc gtagcaacac agagccagtt tctcactcag
49201 tcctaagaga gtgggccagg gtgctgatgg gggctctgga gagcttagag tcccagagag
49261 ccctaggcac caggaatctg gagataagag ggctcccccg acccttcctc acagcctggt
49321 ggcttcccca cctctcccca tcttcgtgtg gcccctccca gggtagcagc aagaagcggg
49381 agcaaaagga ggggcttcac cccggactgg gaattgggag agggtgacag ccgaagccag

49441 gcacccccac cctgcattgc aggggcagtg tgcaggagca cagtttccct gctgccactg
49501 gggctgccag cccaggtttg agagcacatt ggggacaggc atcgctgcta caccttctcc
49561 cgccaggcct agccatgcag ccagcaggcc tggttgtgga aaagaggggt tgagaaaaat
49621 tctagagatg ggccatttgc tggtgaggag aaggtggagc gggttcaaga acttggggtt
49681 ttcctgtagc ttaatcctgg ctgcccatat ggggccattg agttctgggt caaggagtgc
49741 tgaggtgagg agggggacct ggaagctggc cccgcagccc tagatgttgc atctcaccct
49801 tgtcacatag tgtgggttct gggaggaagc cagagagctc tccagaaggg acttggccaa
49861 ccttttatca ggaaaacacc tactccttaa agaggacaac accggggaag gggtgtcagt
49921 gcctcacctc tgaccccgtt aggagcccag gcaggcttcc cctgcatctg ctccctgccc
49981 tccatcctct cctgggaagg ccaatcccct tatagatcag ggtattcaat aaccacagtt
50041 ccctcttgtt cctgctttag ccacaatgag caagaggaaa atcccgttgc tggcaagcag
50101 cagtgtctca ccggccatct tgaccgttag ggtccggcgt ggggatcagc cgctccctgg
50161 ctgatggggt gaaatgaagg cctgctgggg gctgcgggga ggtagactga gtgggcagga
50221 aacagcctca gcccagacag gccccgggca gcccaggctg gaatcttcca cggatggagg
50281 ggaggctgat gccaggagat cctctgggca ctcctcacct ctccctccag ggcctgggtc
50341 caccccccag ccctgccctg cctggcctgc ttctctggga cttcacccac attcatcctg
50401 tcctcactct gggggtcaga tggttctaag caggggcagt gtggacctca ggcctcagct
50461 gctgtgacac tcactcctaa gaccctaaga atctgccatg aagttacccc gttcctcctc
50521 agggaatcag tatgggggct ctgcctgaat gaccaccaac cccaccgccc actgactctc
50581 tgccccaacg attccacctc ccctaggccc tggtcagccg catgccctct gagccactct
50641 gcccacttct ctagttcctg ccacgaagcc ctgatttctg ccggcttttt gcacaatgcc
50701 cccggtaatg tgccaggaag ctgtgctgtg tgacagagca caggctccac tcacagctct
50761 gctattggga acccccctcc ccatttgtga gatggggctc aaggatgcct cacagaagtg
50821 ccaggattga gtgaggtgca cctgcgagtc atccacccct gtgcctggca tgtggcaggt
50881 gctcagccag tgttcacttc cttcctcttc actgactcca tgttaccctg atgtcaactt
50941 ctaccccgag cagcacctga ccctcttgtt caggtgctgt ccaagcacct tctcagctct
51001 tgctccccac cctataccaa gggctctgca gaagctgccc acacaggagc cttactggcc
51061 caggggcagg tatagggagc tcatttactc aaagttcccc tcacctcccc aggctctcag
51121 tgttgcagag atgtaatgaa tccctaacct catgggcgct ccactctcag cagcacaaaa
51181 tcagtgttcc agacccttgc atggcagcgt atgcctgccc tagtctctgg agagaaagtg
51241 acttcggccc tgaccacact cacatgcaca cacatgtgta cacaagtgcc catgtgtttg
51301 cacacactca catgcactgc atgacaccag ggaggactct ggattctggg ctctgctgtt
51361 gatgctgtct ttaggagtca catcttagag aggacaaaga aagaaatccc agctttagtc
51421 ttgcagctaa actgtagaga cctgagcctt tcctcctgag tcccagtatt ctgcgactat
51481 ctcctcttct cattttctcc acctggcctg gctctcctgc cctccacgac ccccacctcc
51541 ttctgctgcc caaaccctct tctgctggtc tcctctctgc tgggtctcat ctgggaggag
51601 tttggggcag gagccacttt tctcattctc cttattgatc attcttccac tccagtggag
51661 gcctgcagaa gccaggtaac ttcctcctat gctggggcca gagggcagtg ggcacgtgt

51721 gcatggtggg gctctgagac agaggggcag agagtgggca tgcagaagtg tgtgcatgtg
51781 gggccagtac atgcagcctt accccaagag tgtttttaaa tcggtttcca cagcccctag
51841 agagctagaa aatcctctgc tgccttcctt ctccaggctg agccccagag ttgcagcagc
51901 tcccaaagct gctcagatga gccggggcag ccgggcgcac tgggattttg tgcttgagac
51961 acattcctcc tcctatgctg tgagctcttc tccgcttagt agtctcagct gatgtgggca
52021 gccgaggcct ggctgatgtt aacacctttg aattacagac tgacctgctt gcccaacgta
52081 aaggttagct ggccagcgct ggactgcagc tgtgctgtgt ctaggccttc aggtcttgag
52141 ccatcaggat atgcagtcaa gtctacctgg gcaaggcctg tgccagaaca gggtgtgagg
52201 agaggaataa tttgctaatt gttattcact ttcctgtctt catcctcatt gccacagcac
52261 tgggaaagca ttgagagaac attccagaaa agttttctgg aagcctccct aaagtgagat
52321 aggggggccg tttttccctg ctctgagccc tggagtggta gctcacttac cgccccacct
52381 acacccctag aactgccccc aaccacccgt gtgcactcca tagcaggacc cccagccctg
52441 cctcaccctc acagagcagg cttagcccac tgttccccta gaggccccca gagcctggga
52501 gctcccaccc cattggaggg gagcagggga gcaggggagc cggtgggcag aggagcaggg
52561 tggcccaggc cgggagggtg gccaggctca tctaaccctg tgttccctga ggaagctgcg
52621 acccacattg tggaatcatc agatcttagc ctggtagggg cgatgagctg aggccctgag
52681 gtatcagagc ttggggttgg aatagtggcc acccaggccc agcactgtgg gcccctccca
52741 tctcccccaa ggcagaaccc agggccatgg gacccctttt ctgcagacct aggcttgcta
52801 ccgtgcgaag ctgtggggtg agaggtctgt gggacaagga ggcccccggg gagcgtgcag
52861 gcccgtgggg ccggggctga ggccagtgtc tctggtccac agcccctcac acgtgtcccc
52921 ttgtctcctc atttcttccc cctactcagc aaaggcagag agaagaccaa gggaggcaga
52981 gatgacgagc acaagtaggt gtcccaggcg agggcagacg caacagcgtc tctggggaga
53041 cagaggcctc ctccccacag gctgtggagg gcgggatggg ctcggctgag agggctcagg
53101 agtgggctgt cgtggtagag ctggcacggg tcatggaggc ccagggaggc cttttcacct
53161 ggccacgggg gtgagggatg aggaggggtt agggtcgcgg gggaggtggc cagaccttct
53221 ggtttccact ctgggctctc gggggtgacc atcctcctct ccgtgtcttg ttcctcctcc
53281 actgttcagc ccccatcctg tggccttcgg gcttccctgg gagggagggg acagctaaag
53341 gctaagggga gaggggcatg aggacctcag ggatccctct cccaacaggg gcgaggtagg
53401 gccaggcagc tggaggcata aggaggagga actctgggta ccctttgcag ctgcaggtcg
53461 tgggtggagt tggagacccc cagaaagctt aggagaggcc accatttggg cggccctctc
53521 tatcttcatc tccccaaaag gcttcctgga aaaatgaggt cagggtgctg tctggagagg
53581 acaggagaag ggtcctgcag gtgggagggg ctggtcacaa gggtctggaa agattcgaag
53641 gctgggagtt gtgaatgggt cttgtaggca cccctagaag cacactgaca tctggaggac
53701 tctagcatta cgggatctca taagggcagt ggtagccaag gtggaccata ctggaggagg
53761 cctctggtta acacctgctc ttacagatga caaatctgtc tcagaacagg ggcagagctg
53821 gaaccagaag acagggaagc tgacttctga cccagagctc accatagacc caggaaattt
53881 agagaacttg actgtatcta aatttaatct aattctccaa acataccaat gagagctgaa
53941 aagccaggag tgtggtcttt tttatcttcc agataagcaa cctgagcccc agagatctta

54001 agagatttgc tcaaaactgt ggcctcaata tattaagaat tagttcatct gaagatatca
54061 tggaaatatc aggattgtgt gttgaggggt ctgaactgat caacactggt gtgggcttgg
54121 gatatccact ggcattaaga tcctggggaa ctcacagcct cattcattgc ttcaaaaaaa
54181 ggtatgagtt ctaactcgca tttgttacgc acaagcaaaa gcaatgatga tgcagtgtta
54241 actctgtagt gatggaggaa atggcgctgt gtaggaagag ctccagccca gagacctgaa
54301 ggatggagga gtttgccagg ttcccagtaa gggaagacac gtaggcagag catgtgtcaa
54361 ggctcagagc cagggaactg aatccagggt gggtggcgat gatggggaat gctgagctgg
54421 ggagtcctct tagggagtcc tcaagggttc aggtttctcc atagatgctt agatgtaaag
54481 ggcatatgaa tgagatacaa gcagcctgtc catccccagc ttctctgcct gagatcagcg
54541 atgctctaag gcccttcttg atgcccagct tcccagtctg tcccaggcca gacccacagg
54601 gacacagact ggcctgatcc ctttgactgg ctacagttgg agtaagaggt acctcacagt
54661 gccagcacat agtaggtcca ccaaaatact ggtttcctag cacttctctg tcctaatttc
54721 ttaccatctc taattagcac atgagataaa gtaaactggc atatagtatt gttattatta
54781 ttatgtgtag agcaatagga ctattgaaag aataggaaaa tgagatttct aagaagagat
54841 tgaacaaatt taaactcttt agcctggagg agagaaaact gtggagacag atttcaatct
54901 ctgaaatgtt aatgtgtggc aaagctggtt ggtatctatc tccactgaga agagataaat
54961 ggaaataaat ttaagttgaa gttaagttga agtagagaaa caagattcaa cccacgaaag
55021 aactcatagt gtgtgacatt ctgggatagg ctggtttggg gagatgagaa aacacccatc
55081 atcttccatg atgctgcttg cttgagcctt gcaaagggct gggccgccag caagaactga
55141 agagccagct tccctgacaa tcctggggtc aggacaagtg tgtgtgggtc ccccaagcct
55201 tctggtacaa cgtgggcaca tgtgcaaacg ggccagtttg gagacagggt ggttcttgta
55261 taaagggtca gcctatcctg cccacctatg gaaagctatt caaaaactaa ctgacattca
55321 acacactttc aaagacacct gtaaagacca aaattatgaa attccctgtg ccttggggca
55381 tggaaaattt ttaaaataaa cccatttagg ggcgcttgga taacatgctc cgaggttttg
55441 aaaattgggc tgtttgagca atgcaaagaa ggaccacaga ccctatttct catagacagg
55501 agtctttatc ctgattgatt acctgaggcc tgagctaatg agcctagtag ttccagttga
55561 agccagtcat ggggcagaag ccctgggcag tgaatggact cgacctgcag ctcatttatg
55621 gtcaggtctc tattagcttt cagaataatc cagagacctg aattaatgaa acactcgggt
55681 cactaaattt agtttggtga atgttcccaa gtcattatga atactaagct ctacctagga
55741 ccctgtgtgc accacacagc aacacgcttc taaatctggg ccataaaatg agcggagaaa
55801 tcatcagatt tatagtttac aataagtgaa ctaattttag catttttttc ctctagagat
55861 aaataccagt ctagattgta ggtgcatttc attctgacca tggtgaatgt agccacagga
55921 aactcaaacg gattctaaat cctcagaggg tcctgggata ctggcctccc cttggctctc
55981 acttctcaag ctttaatacg cccaagaata aggagccatt tgattgtaaa gtgagttttc
56041 actgagttga ttattggctt gttttctttg tctgtttctc accatatcct gtttttggta
56101 attgtagaac tcaactagac caaatgcaga attttacttt gaaaacaaaa aaaggccagg
56161 acaacggaca cctcaacagc aaattacatt ttttgaaatc taagtctttc agacaaatag
56221 ataatcttca aaattccctg attaagttga catttatttc aaaactctcc tttacagttt

56281 gccaagagaa gtaggcatac tgctttctgg gaactggtgc tgcaatcagg cagaaaggct
56341 ttttctatag tgaacttctg atgtgttact gatcagaaaa ttacaaaggc tagtggcagt
56401 gatgacatca tgctgtactg atcacaattc agattcattg aagagcaagt ctaggagaat
56461 cagaggtaca gagcctgtag taagaattct ggaaattaca tccttcacta cacatttctg
56521 catttttaat aaagggacaa agacaatatc cttttgagat attcccaggc acacaggagt
56581 gctaccagta ctaccacttc agacagctaa aactatgcag ggttggaagg gagagatttc
56641 tcctttcaat tgcttcaagg tgtagtagag atagttggac aagtatagtt tttgtttgtg
56701 tgtttgtttg agacagagtc tcactctgtc gcccaggctg gagtgcagtg gtgcgatcgt
56761 ggctcactgc aacctccgcc tcctagttca agcgattctc ctgcctcagc ctcccgagta
56821 actgggatta caggcatgtg ccaccacacc cagctaattt tagtagagac ggggtttcac
56881 catgttgacc aggctggtct tgaactcctg acctcaagtg atccacctgc ctcggcctcc
56941 caaagtgctg ggattacagg tgtgagtcac cgcaccggac caggacaagt atagtttttt
57001 aaagcctcaa tcatttcttt caaatctgtt tgtgttaaga aataaagtga ctttcaaatt
57061 caagggccat aagtgcccca gggatgcctc aggtcctttt ccacctgggt gagtccatgg
57121 gaccagcctc tgaaagccac tatttcttgc taccttgcac tccccccggg actcaggcag
57181 cagggccaga tcccatccca gcaggccggg cagccggctt ctccaggcag ggcctgcttc
57241 taacctacca tctcagagca cttcccctct ggggaagtgg ggaaggggca ggggagaggg
57301 aagtctgtgg ctggagcagg ctgcaatggc aaaggctaac tatgtaggtc acacatgcgg
57361 aagggccaag gactccaaga ataacacagc ctaggaatgg ccaagaagta atggtgcaaa
57421 gtcaaacgct gagccctgca ggagactcct gggtaattca ggagagacat agttgagcag
57481 cacggctggg caaaaaggaa tcccaagcac tgagacagga agtgacccac gaggaggccc
57541 acaaaaacta cacttctgaa gctgggccta tggcaggggt caggacagcc agcacccctg
57601 tccttggggc cagcaggtgg ggcaaaatcc cacaggatgc ttcagggcaa gccagccagg
57661 gtggtccttt ccccaccatc acacgattat ttctggtggg ttcattgatc tgttcaatgt
57721 tatgtcacca gcaatttaac atgcacagca gtcccttccc aaatgtttgg gtgctcaggt
57781 tctttttgaa tgagcccctt ctttgtaaac atgggtatga tctgtgagat tccacagatg
57841 acagcatcct gatctgtaaa ctatgctggc acacagacca tacctgtacc tatgggtgcc
57901 agccctgggg ccttttagct aaaatcccaa atggtcagga atggatggga ttcttgccca
57961 cagaaaagtg cagtcctacg gcatcgaggc aaaggacctg agatgcagta aaaatattgc
58021 tggcctagaa cacaggagct ttctgcttca atcccagttc tgccactcac cgtgtgacct
58081 cgcgattatt gtcccctctc tgagcctcag tctttttatt ggttgaatga ggagaagtat
58141 gtggccctca gaggattgcc aggaagatga aatgagcaga catctgcaga cattgcagac
58201 atctagaaag gcctttggcc ctatgtagtc tctttttggc ctgtatgtgt gtgtgtgtgt
58261 gtctgtgtgt gtgtgtgtgt gtgtgtaact cacctccccc atcagaaggg aggaagcttt
58321 tgaaaataat tccaaaggca tcagctttga ttttagagcc tctttctttc cctttcttct
58381 cattgtcgta tttcagatgg tatgctactg aagagtgcag acacatcagc cttcttccat
58441 tcctttttag gaattcaaag aacatgctaa tgttccctaa attgtgagct gctgccacgt
58501 gaaaagggtg attctcaatt gctccctcac tgggatatga acgtgctttg tcctgcatat

58561 gaacagtaaa gaaaggtcct ggggttggga tagggccagg tttggggaat aagattggcc
58621 ccacaatcct gaggagaggg gcttgaggtt tctaggtcat ctgcctgagg acagaaaggt
58681 tttccccaac ctcctattgc ccagcataaa gaacaaggat ctggggtctt agaaaccata
58741 gtcaagcctc cacagggaga tgtgtgaccc aaaaagggtg gactctgctg taagcgagct
58801 ccaggttgga gcctacacct ggctccacct ggacctacag gatgatgggg ccatacccag
58861 gaggagggag aacgggaagt ggagagagtc aggtatccac tcatgcaata tggatggggt
58921 aatggaacac tgcactttac cctctgctga tcctgctcct ggggcttcct ggtgccagga
58981 accacaggct cggctgcagg gacatcactg aaggacagtg aggacccctg cccctttcc
59041 tcagccccgc tgactgcaag gcccttcacc tgggggcaca aggtcgcagc aagccagagg
59101 aagaggtagg aactatggag cctgccataa gttcctgggg ttgggggaaa ggggaatttg
59161 gcagtgagag gggacaggta gaaggggcaa aatccaggac cgaagataga tgggaaaggg
59221 cctatctccc cctttttcat ggggaccaag gagcatccca gaacacggag aggttggaga
59281 aagacaagtc aggctttgag caccagggcc atctgagaac tgaacagctc gtttcaacca
59341 catctcccat ctcctcatcg gcctctgccc ctgagtcctc tctggtccct gccccgccgt
59401 tctgatgctc tgtctccagt cctcgggctt tctctccaac cagctgagcc ggggaggatg
59461 agcgtgggga cactggcagg gctcggagct ctgaggagac tagactgagg ggctgggatc
59521 agactgaaaa gtccaagacc cagaggagct ccagttaaaa cggctctttc cggctcaaga
59581 ccacgttccc tgcttgctgg ggaccccatc cctctcctcc gtgtgtgaaa ggatggcaaa
59641 ggcggaagtg gaggggtctc tcactgccct gattccccct cctggctccc aatttgggaa
59701 gacagatccc gatctgtctc gggaccagta ggtgaggggc cgggtccatc tcccttctct
59761 gatgtgttct ctcatgtttg gctcttctgt gtttgtgtgt tttcctccat gcgtccctct
59821 ccctgcacct cattctggtg gcccccctca cagagccggg aggagcgtgt tctccgccat
59881 gaagctcggc aaaaaccggt ctcacaagga ggagccccaa agaccaggta ggacaggtgc
59941 cgcgtgagcc ccgcagcccc gcgcctcgcg gcgccctccc gccgctttcc agcccctgcc
60001 cggctgccaa aaggccctcc tgggataggt cgtcccaggg ccaggcccca cccagtgatg
60061 cgtggccctc taggaaggag acgccctagg gccgagggag ggaaacgggc agcgccctct
60121 gtcgggggtg cctggttaca aggctcctcc cgccctcgtt gctcccggtt cttccttctt
60181 cacgcagaag cccagtgttt taattagagc agagaatatc catcaagaaa cgctgtttgg
60241 ccaactccct atttctgggg ggaaaccgtt gtcccactag tacggggtcg gcggcaggcc
60301 tcgcccaagc gctctcacca ggctgcagcc tggcctttgg ggaggtcctg gggccgctgg
60361 agggcgcggc agggccgggc ctgggaggct ggagcagcag gtggcactgg gccctcccgg
60421 aggagcgact ggagagggga agacagctgc tgcagaggag gcgggaggcc tggaggaaga
60481 caaggggaga aatggagagg acaggggagc tgggaggggg ccatcttctc catcacgggc
60541 ccaccatcgg ggctcccggc tgccctcccc cagcatcccc tctgtggcct actggccctc
60601 aggctctctc ccccaccctc ttgcccccat gccaattcca gccgtgccct tcaccttctt
60661 aaatggagag agtacagctg ctggctgagg accaaaccca gcatccttgg gtggctgttc
60721 ggaatccaca tgtctgcatc tcattagatg aatacacacc aatgcattag gttctgaatt
60781 ccatcagctg tttgatatgg ataagttact tcctaagcct tcgtttctgt acctgtgaca

60841 tgagaataac gcccatcttg gcagggcaca gctctaagga ctagagatag tcatgtaaag
60901 tcctggtgtt gtgcctggaa cagagagtat ccacccaaca aatggtagct atttattgag
60961 aggtgcaaaa gtatggattc tttattccct accttcttcc ataagagata ttcagtgcct
61021 aagtaagtgg tatacaataa aagagcattc aattaaaata aaaaggcaag accagacata
61081 atggccattc aaatccaaga tcgggattag gtagaaaaac actgcagtta ttcaggccat
61141 aatattctaa gcggtagcaa ctaagtaaac ctcgaattta actctcagct ttctggatgc
61201 cagagtaaaa ggagaaatgg tcactatctc accatcagtc agcccaccta accagtcttt
61261 catcttcccc agatgaaccg gcggtgctgg agatggaaga ccttgaccat ctggccattc
61321 ggctaggaga tggactggat cccgactcgg tgtctctagc ctcagtcaca gctctcacca
61381 ctaatgtctc caacaagcgg tgagtggacg ggagccctgc aaccccagct gcccccaccc
61441 tgtcaggcag gacctggctt gccatgattg gaatttgggt agggaagaat cagggctcat
61501 ggacggcttc ttcctaacct aaccttaccc ttttctgctt ttccttgggt gtttttctca
61561 cctctctccc tgcaaaagca gagcacagca gctgcaggcc tgttctcagg tccttgaata
61621 cctcccagcc caaggaagcc acagacctgc ccaccccata tttgaaccta ttgaccccat
61681 gtctgtctct ctgttacttc tgcctttgat tagttctctg tgttgaggcc tctgaagaca
61741 actaatcctc actattcttg gcagtgcttg agtgtttaaa gaccatgatt ctgtccctct
61801 gcccactccc aggtctaacc actgccagtg tcctcctccc caacaagcag tccccatacc
61861 ccaggcctga tggtacattc tcttcctcag atctaagcca gacattaaga tggagccaag
61921 tgctgggcgg cccatggatt accaggtagg tgggcacctg gaggcctcat gtctttcaga
61981 ctctgctcta gtgggggatt ctagccggat gtgcattatc cactatactg aggcatggat
62041 ccaggggtca tagagaacaa caagctgaat ataagttccc aaagtaagct tgttcctggg
62101 aggcattcct agaagtatca tcagtagaga gatacaaacc caaagaaaag cttgagaact
62161 ctgcctatgc taatcaaagc ccttccagta tttcatgttc agtattggac ttcataaagt
62221 gagaagcacc tagaaaaacc ataaagagac caagaactac aaaaaagtat tcaaacagtt
62281 aaaataaggg cctttgaaga aagttttcct ggcatgattt ttaaattgtt gttttgtatt
62341 atttgattgc tggaagtaat gacgattctg tttatctctg gatggtgaga ctgtgagtga
62401 tttttttctt ctttttgctt ctctgtattt tctgagatta acacaacttt tttatttgtg
62461 tgcttctaag accagagaca aaagactgag acaaagagag gttgacagct gtaagaatgt
62521 gaatgtggcc aacagtgcag gcaggacagg tgatgagcat gtctggtggc tgctgggaca
62581 ccatctctca ggaagggccc agggaccctc cattgctcca gaccctccct tggaggctaa
62641 caggactcag ctataaatca gaagactgct gggctttcct aggattgagt tagaatgctg
62701 gctgctcaga ggggacaggg cttgcaagag atctcacctg gctgccactg cacttctgtc
62761 ctggggatgt tcggtctggg tatcagcacc aggccccaga atcagagtga gccccatcta
62821 ccagggtaga gctttatctc tgatgcctag ttccagaccc caagcacact aacccttctt
62881 tacggacaac cccacctccc ctcactcagg tgatgatgga aaggataaga tggcactttt
62941 ctatgtaaaa ggtacatctg gccatgtttt ctccagaata tacttcatct cccttcatct
63001 ttgttttagt tagtttccat ctactcaggc aactgtcttc ccaggtgcct gaaaaacagc
63061 cccaggcaat gtcaaacgga cctgcccctg tggttttaaa taacttcaag ccattggatt

63121 ccttaggggt ttgagaatgc cggcccctca cctctgccaa aacccaaaca caaagtgtcc
63181 tgagacttcc gagggctttt ccaagggatt gggctcaggg ctttgtttct ctgtgtgctg
63241 agaactggag aattccaaag aacaacgacc ccaactcagg tatcatgtca ctggcctaag
63301 aggtaaggac acatcaaaaa tagagtcatt attgttcatg aaacttattt ttgaaggttt
63361 attctatgcc aggcactgtg ctaggagcta tatatgtatt tccattgaag ttaatgtaat
63421 tcttcaacca cccaaatata ggtgttactg catttatagg taagaaagtg aggccccagg
63481 aaggtcagga acttgtccca ggtcgttgcc actaagttag ccaaggttag agccagggtt
63541 caaggccagg ggtgtctggc catagccata agagcaattg acttctctat atcaagcctt
63601 ctagacagct gctcccttcg ggtccctggt tcatcttcat cttgctactt agtgcttgtt
63661 ttccaaacct tttcaaactg cctcctacca gagactttta ttaactggac cagacattgt
63721 cccctccaaa gactttttaa accgaactga acaaaacggt tgttgtattt aacctggtag
63781 catagtgttt cccgacaagt tcattcacaa ttaaccttgc aaacatttga gtgagagcta
63841 tgaggatgca aatgtgaaat tattttcact tccaatacat acagtgtata atgcaataaa
63901 agcaggccgg gtgcagtgac tcatgcctgt aatcccagca ctttcagagg ccgagatgag
63961 tggattgcaa gaccagcctg gccaacatgg tgaaaccccg tctctactaa aaatacaaaa
64021 attagccagg catggtggtg gcgcatacct gtaatcccag ctacttggga ggctgaggca
64081 ggagaatcac ttgaacccag aaagtggagg ttgtagtgag tcgagaccgc accactgcac
64141 tccaacctgg gcaacagagg gagaccttgt ctcaaaagaa aattaaaaat aaataaataa
64201 ataataaaaa gcaataaaac cagatacgac ttcactctct tggagacttt gggtttgatg
64261 agatataaag accgcaggaa ggacacctcg ttggtgagat gtggggagac ttagctgaac
64321 tcagatccag gaagggttgt gagggctcag gaccattcta aaggttatgg cactgaaaga
64381 agcctggagg taaaaggacc gcggcgtacc catgctcttt gtttcttcca tttagaatgc
64441 acgtcacgtg taaactcttt cctgattatt gccatctcca aggcaagaaa ccacagtcac
64501 ccccctgtga atgtgcagtt tccccaccag agtgagcaac gcttggcact tggcagagat
64561 gttcttggtg gatcaagggg tcttgagtct ctttattacc atctcttcct ggcaccaaga
64621 cttaatacat tgtcacccaa catctctcca gttttcacct ccatttcatg cccctgccac
64681 tgacctgagg tccccagcct caactgcatg gttgtaccta ctttctacct ggtctccctg
64741 ccctcatatt gcctcctcca ctccaaactc catgccacca ctggagcaaa cttctgaaaa
64801 tgcaaatctg gtcatgtcac atctctgcca cctggaaaag tggggagtgg gtaatggatg
64861 ttgcagaagg acagtactaa agggcgcacc aactctcatc tctggctccc agcagatgtt
64921 cctgaagctg gatcaggtcc ctgggaagca ggtcacaaag cattattact ctcgggtttt
64981 cccaggacag gggtatagat ttaccactca gcattattcc ttgtgatcgc tttcatgaag
65041 ccactgggga gtatgtccct tatcttgaaa ggagatggtg gaatagtcag ctggtcagtt
65101 ggtcccagct gtggctccgt ttgcctcttc atcttccacc ctggaaaaat tgagtcctct
65161 gctgatgatt ggagagaagg atggccaagc cctcccccca gaaccctgga gaatgaccag
65221 ccatttttta ttttttattt caagtgcatc atttcccctt cctagagccc caggaggggt
65281 gggaggcagc aggaatggca ttccccttcc cccaacacca tgcgcctggt ggtctttctc
65341 cagcgccctc agcttcctct cggggctgcc cttcaaccag caatgagcac cctctggagc

65401 ttctcttccc tccgttgctc cttcaagcct ctgcccacct tcccctccac cctccgcctc
65461 tactgccaga gcttctgccc aggttgtcac cttcactggc tctgctacat ggaggaaggg
65521 agaaacgtat gaggcctgaa aagggggaca gggagaagta gcctgtctta tggccatgcc
65581 tcgagatgga aaacataaaa accaaccact ctcttggcag tgggaagagg acttgctgct
65641 ggcttatctt cggtagatgc caaaactatg tctgtccgct tcccagtcct gagttataga
65701 aagccttttt tattgttggt gggggcatta gttgtggaaa aattcatggg gtttttaaaa
65761 atctttggca aaaaagggtt cgtggaagtt aaaactctac aaactattgt gcaaagaagg
65821 acttctagtg tccgtttgct ctcttcatag taaacttctc agagctaagt tagttagtcc
65881 tatttccttt ttagctccct gaaggagcag tgaatattga acttctgtga agctccagat
65941 tctaagcttt tacttccgtc cctttgtcac ttcacgttcc aaacggaaat gtttgcttcc
66001 tagttcaagt gtcacttcat ttgtaaatat cccctttcct ggcaatgttc tcattaacat
66061 tggaatttct atttccttaa catgattact tctgatgtgt agatttttaa tcaccctcag
66121 gttatttttc agactttgat ggacactgga tgatcatttt agtggttatt ctttttcatg
66181 gaaagggaag ataattctct caagtaaagt cgtatttaga agttgcatca aagcacaact
66241 aaaatatgaa atctccaaga tgacaggcat gaaaagcaac cttcggagtc gcattagaag
66301 aatgtgccgg gctcagtggc tcacgcctgt aattccagca ctttgggaag ccaaggcagg
66361 tggatcacga gggagactga gaccatcccg gccaacatgg tgaaacctcg tctctactaa
66421 aaatacaaaa attagccgtg cggtggcagg cgcctataat cccagctact cgggaggctg
66481 agtctggaga atagcttgaa cccgggaggt ggaggttgca gtgagcctag atcgtgccac
66541 tgcactccag ccggggcaac agagtgagac tctgtctcaa aaaaaaaaaa aaaaaaaaaa
66601 aaaaaaaaag aagaatgtac agaggtccat gcttattcta gctggggaaa ggcttttctt
66661 gttaaaacac acgtttgaga gaggcaagtg aatcaatcat tctaagtatc atgcacaagc
66721 tccctacttg agagatcaac cgcttagctg aagggagatt ctgttggggt cactcacatt
66781 ttgtagagct ttctgctgtt taggatgcat tttgacacac atgatctcat ttaacctgca
66841 tacaacctta caggtagctg gtgtctctgc ttgtaactga aaaacacaag gctatgtggt
66901 catttgccct cacacctggc aacatgtcag gtctggggtt caatcccaag ttcagcaacc
66961 cccagtacat gcctctgtct atcttaacat ggccaccctg acaggaactg acacctccaa
67021 atccaaatga gacctgatgg cttaaaatga gggttcggga aaagtagtag tgatgtcaaa
67081 aattactggt gctctggctc tctctctctc tgtctctctc tctgtcctta tatcttttta
67141 gcttaaaaag ccaataattg gctggtcaca gtggctcgtg cctgcattcc cagcactttg
67201 ggaggccaag gcaggcagat cacctgaggt caggagttca agaccagcct ggccaacatg
67261 gtaaaaccca atctctacta aaaaaaaaat acaaaaatta gccgggcggg gtggcatatg
67321 cttgtaatcc cagctacttg ggaggctggg gcagaagaat cgcttgaacc caggaggtgg
67381 aggctgcagt gagctgagat catgccactg cactccaggc ctgagtgaca gagagagact
67441 cagtgtgaaa aaaaaaaaaa agccattaat catttgtggt aagctccaca aggaagaatt
67501 tgttgcaatc ccacctgtgc ctggctccag caggtttcca ggaaacattt gttgcatgaa
67561 tgaacaactg aaatagggct tcccaggata taagtaagat tgcagagctg gacgccacat
67621 aaaggacaca gcagagtgca cacaacatac aatcagtagg ctgaaaaagg cggctcaggc

67681 aggcaccaaa gccgcactgg ccatacaaac aaagaaattc agcaaacaat gtggaaaggg
67741 gggggagcca aattccagaa atgcaaagac gtatcagagg cctgcaggct tacatgagaa
67801 ttcctggagc aggaaattaa atgcaagtta atgaagactt tgaaagcttt attcctgagt
67861 gcatgaaacc tgtgacatgg gtttggcagc tctttctgaa gcagagccat cgacgctggg
67921 gaagaacgca gggcacttgg catcacctag gagctttaaa aatacagatt ccagcctggg
67981 cacggtggct catgcctgta attccagcac tttgggaggc cgaggcagac agatcacttg
68041 aggccgggag tttgagaaca gcctggccaa catggcaaaa ccccatctgt ataaaaaaga
68101 caaaaattag cccggtgtgg tagtgcatgc ctgtaatccc agctactcag gaggctgagg
68161 cagaagaatc acttgaaccc aggaggcaga gggttgcagt gagccgagat catgccactg
68221 cactccagcc tgggcaacag agtaagactc catcaaaaaa ataaaaatac agatgcctga
68281 gctgcatgtc caggattgga ttggattggt ctggaggtgg cctgggcatc agtgttttta
68341 atgcctctcc ctcccttcgg cccagcaagt taagtctagt gaacactgac ttagaggtta
68401 gccaactcat actcctctat aaatgaagac acttgaggca tagaaggaga aagagccagg
68461 atcacccagc agatgagagg cagtggtcag aatggagccc aggaggccag tgctggctcc
68521 cgggccctcc ctctgaccct gctgccccag cccctggacc cctgcgggac caggctccta
68581 ggctcaggca gctttccttc tggctccccc ttctcctgcc tgcaggtcag catcacggtg
68641 atcgaggccc ggcagctggt gggcttgaac atggaccctg tggtgtgcgt ggaggtgggt
68701 gacgacaaga agtacacatc catgaaggag tccactaact gcccctatta caacgaggtc
68761 agtggccctg tggggaaagg agcctctgta aatatggcag gagggtggcc ccagccctcc
68821 ccaggagaaa taggagtggc tggaagtcaa ggagcacaca gggctaccag caggggtgat
68881 ccaccctgtt acaaccccca ggacactgac aaacagagcc acatcttctg aatcctctga
68941 accccccagt ctagagcaca ggccgacact ggtctttgaa gcgtacacca cccgggtgct
69001 cctgggcaga ccccagcagt gcctgtgaaa gaaccccccc ccaccccact ccccaagagg
69061 cctggttccc actgtttccc aggccagagc ccaaggacag ggctgcctca ttccaaccca
69121 tggtggcact ggcaccatta gtaagcactt tctaccagcc aggcatgctc ctaaatgctt
69181 ctgaactgga ccatccaaag ttgtcactat caattccctt ttacacaagg ggacagtgag
69241 acttctgaag gcagaccttg tccaacctca cagtgccagc atcgcccttg gcgtgctagt
69301 ggggttctgc cctcttggct cggctccttc atggttgcct aaacttctgc gggcttctct
69361 ccagagcact ggccttgatt ttagggtgtg aaagagaaaa catttggtac ccactcctat
69421 tctttccagc catttgaggc cacatcaagg gtgagggacc tcagagacct tctactttaa
69481 tctccccatc ttacagagga ggatactgag gcctccatat ggaagggcct tgcccacaat
69541 ctcagagagt gttgctgata gagcaggggc caggctgccc acccagcttc cagcccaggg
69601 ctctggccac catcacaaat atggcacctt ggttcccagg ctccagatgg gaccccatca
69661 taatcagctc tggggaggga tgtgagaaat agagaaatac actcactcat caaaacccaa
69721 agaatgactt agaggcatga agaacagcga aagtgagact tttaataacg gtcttgcaag
69781 atggagtgtc tggtaggcag gcacccctgg ggcagtcaca acaggaattt atctcctagt
69841 gtgcaagtcc ctcccccact tccccattgg ttgagcacta tggggttaca atcttccctg
69901 acgtcgccta agtttcattt tcccccttac aaggttatac cccgctcccc ttcgatttcc

69961 ccacttaagt ttcgatttcc caataacaaa actttcttcc ctttcatggg ctgacccctc
70021 ctctacattc tgttcgctta ttgtgtcctt ctaggtgcat gagccgtgtg gtttgttgca
70081 ttcgcaggtt ggatgccagt acttagactt atcctgcctt gaaaatggac cattgaaaat
70141 gttttctcac caggatgccc aggcctttcc cagatgcctg atcacacatc agactaaccc
70201 agaagccaca ggcctgggcc ctgccatccc tttcctggag ggttccccag tctgtttctc
70261 ctggtggctc caggcagtga ggggtgccag gctgtcccaa gtaaggcagc tgagtcccgt
70321 accctgaggt gggtgagggc tgaggtgccc cctgtagctt gggggagggc atttctcagg
70381 cagctctgag agtcctcaga ctgtgcacct gcaggaagaa aacccaggcc cacctctgcc
70441 cttcagccca ggcctggccg aactctcctt tgtgaccaca aattgactct gctctcccag
70501 ctgctttccc acaggccccc cgagctggac aacctgctca ggacccctcc agccccaagg
70561 acattcagga ttgacatctc tggccttacg ttaggacaca tcctgactgg acaagtctcc
70621 gcctagataa gcatctatgt ccccaagaca ggcatggaga caggtccctg tggccccggt
70681 ggttacttga tagcagtgaa gccacttctg ctaacttaag ggatctccct ccaccaggcc
70741 cacacacctt ggctttcatt caaattactt cctctttccc aagggcacag agctgctctc
70801 cctactctgt ggctccccca gaaaactcct actcacccgg cagggcccag ctcaaatgtt
70861 cttgctactg agcagccttg cctgctctgt ctgggtcaga ggtcagcact cccctttctc
70921 tgcagtgtta atgtcctgga gttgaggttg gtcctccgtg tctctgtcct gctggtggac
70981 tggaggctcc cagtctcctg cctcgctatt tgttcagagc caggcatgta ctcggagggg
71041 ttcagtaaca ctcacccaaa ggaagacaca aacatccact gagcatctgc cgcacaccag
71101 acagtggcac gtggggcaac atggaggctg ccgagatgaa ggaggcacag tttccgccct
71161 gcgcaggtcc cctccttgtt tcagcctaga atctttctaa attccctatt tacttcctct
71221 aagactgact attcattctg ctcatctttg gacaatctta aatgtgtttc tgatcacatt
71281 tttgttcttt ttcacaaacc caaatagctc tgattttgtt tctgtttcct ctaaactagg
71341 gtttctcaaa ctcagcacta tggtatcttg gactggataa ttgtggtggg atcgtcctgt
71401 gctgtgtagg atgtgagcag catccctggc ctctacccag tagatgccaa tagcaactcc
71461 ccttagttac agctaccaag tttccagaca tgccaaatgt cccctgaaat cgccctcagt
71521 tgagaccacc attctaaact atcttactat gattccttcc agccccaaat cagcctttcc
71581 agccccaaag ctttgtttcc cttttcttct ctttctatgc cacattcctt ttcaatgaag
71641 gacatcctcc tcgtcgtggg aatgtattat ttctgactaa ttatctgagc tggtaggaac
71701 cttcctttgg ctccattaaa acaacagagg gaggtgggaa caatcaacat gacttaggta
71761 tctaagctat tacaaaagga aaatatgctc aggaaataac aggaatttga ttgcagacag
71821 ctcagagagt tcgggattct ggctctagct cagctagctc gctcccctgg ggctggcaca
71881 tctgcgggca cctggcctga tcaccctgcc tggccctgag cctgcctgtg tgcagggaag
71941 ctcaaaacct ttgcccttcc ggaacaagct ggccattcac aaaccagact aaggctcctt
72001 tcctgtgtgt cctgtgtgtg catctggccc cgccctgggc atcttgggag cgccaccatc
72061 cttcctcctg ctgaaaatcc acctctcccc tttgccttgg atccttctct ttctacagct
72121 caaagaacct ggcattgcac agcccaactc tgtctttgtt tttcttccag ccccttcttc
72181 cttgcctata aggtgttcta ctccagcata tgctaagtac aaccatgaag acccatgtgt

72241 gctcaaggac aggaactagg agtctgtcac ctctctctgg gcctttcctc attcccacac
72301 acttctccag tgacctcctt tttccctcca cccctccaca gtcagaccac ttgggttgtc
72361 ctcacttcct ccccccagtc acccctcaat ctctgcaggg tttctgcctc cagatgtcat
72421 tgacactgct ctcctcaagg tcaccaagga cattgctgtt cctgagtata atgtccatgt
72481 catcctcctc tcccttgatc acccagtttc tttcatcccg ctgcctgctg ctcctcttgg
72541 gggcccctca cgccactctc ctgcttcccc tgtctctcca gctccatctc cccggcctaa
72601 cttcttggct gaaaggctgt gtcttcttgg agccctgacc tgggcctgct tcccctccca
72661 tgctctcccc aggtatctcc tccatctcca aggtgtcagt taccatcttg tgctgacgat
72721 tcccaaatcc atatccccac ccgcctcccc agagcttcag aactctggcc agcacaccct
72781 gggcctgtta gtgtcatgca cagctgtcgc tgcttgctat gtgcttaccg tggtgcacac
72841 ctgtgttaaa gacttgacca gggtgtctca ttgcaacttc acaaccacac cttgaagccc
72901 cccaagtgaa tggcacccct ggagcagttc agcacgacag ccctggatca tatctccaca
72961 ttcataggat aataggtaaa ctgaggctca ggcaagaaag tgagtgatct agcagtaaca
73021 gccaggtggg gctggctctg aagctggctt taccctcagt gctcaagggc cagcccccag
73081 cagccaggag cacgttctct tcggacattt accccaagca cattggaaat atttctccct
73141 tctcccccac cacagttcac caaagcagca gcccctctac tcactgcccc acaccctcat
73201 cctcagcacc tgcacaccca ggccagggga agagatgggg actgcaaatg tgcatggcag
73261 cccacagcac caaaggacag tgcacaccca tggcatttca catcgactgc caactaagtg
73321 cttggcacac catggttcat ctgctctgag gcttgcgtta ttttacagtt tcacctctct
73381 gaatttggga tgtgtctcct aggcagcctt gtggcttcat ggcaatgtcc atgtggctta
73441 ttaaaccgta gtatatctta cagccagtag ccactttaga tttggtaaaa tatgttacac
73501 tgtctcatca gagcctcata acaagactct gaggtgggtg tcattatccc catttcacag
73561 ctcaaaaaac tgaggttcag agaatgtgtt gctcaaggcc acacagttaa gaaggtgcag
73621 agcggggact agaacccagg tctgcttgct ccctggccct gtgcctcttc tgagcacagt
73681 acaagggcat gggctgtccc acctactgtt ccaagttcct ttcccacccc acactagcca
73741 gcaccagccc cacctagggg atggggctct gcttcctacc acccaccacc agggtcaatc
73801 aggatcagat gagttgcctc tgctgctcct ctgaactttg gggctcaggg caaatgtctg
73861 gggtgcagac aggccctgcg aggagggagg ggcccaacac tgcctgggct gttgctgctg
73921 ccctcagtga cagcaggcct ggatccttcc ccctccagta cttcgtcttc gacttccatg
73981 tctctccgga tgtcatgttt gacaagatca tcaagatttc ggtgagtggg gagcagcccc
74041 tggccggcag ggccactccc tcaaactgca cctttgtgca gacttgagag ggcccccctc
74101 tgtctagacc catgataaag aggcagcgcc gtgcctggac acacggctgg gcaagtaggg
74161 acaggctgga ttctggcccc cacccacccc tttcattgga tgcctctgaa tagtgcattc
74221 gcccgtaaac agtggccagc aagaaactgg cgtgggcagg aggagctgat gctgagcctc
74281 agtgagccac cccactctgc caacgccctc ccacttcacc acaaagctcg ggtcccacca
74341 ctgccccatg gaaagcctca cccttgctgg gcctgacggg tgggctgtgg ttctgcctga
74401 aaggtgattc actccaagaa cctgctgcgc agtggcaccc tggtgggctc cttcaaaatg
74461 gacgtgggaa ccgtgtactc gcagccaggt gagtacctgc gcatggccag tcagtcgagg

74521 tgtgcatggc tcagggagcg tggtgcacgg ggcccttccc actgtggcag ttcctgggaa
74581 aaggctggca aggaccacag tctggcgact gttagtacac acagccatct ggcctgcgga
74641 gaagtggcgc gcacagtctg tacacaaatg cagtgagcaa ctgcgcaccc agtctggtgg
74701 gttctcactc ataattaggt gagtaactct gcacagagcc tggaaggagc ttcatcacac
74761 gctctggagt gcatggcagg ctggtgcatc actggccata ggcaggctga ccagtagtgg
74821 tagaggatgc ggtgagggag acccgtcaca gcctgcagag aggctgcgcc cagactcctg
74881 cctcctgggc tggcacctgc tgagcagaaa agaacccctt tccacccctc acaggaccct
74941 gccgtcccct tttacagttg aggctcagag ccagggcttg ccccagccac acagctggcc
75001 gggggtgaag tcaggctgtg tgtccccaga gcctcactga gccacactat ccaatctgaa
75061 agcggtgatg ctggtcaatg ccagatgtag tgcggcggcc tctgagggac tctggtctct
75121 gaaaacactt cttacaagag ccccaggctt ttgggcagcc atgtgtgctc cgtggcgtcc
75181 tgtagggtaa cagcaatggt gttggcaagg gaaccccaag gggtggccca gagggcttgt
75241 gaggtgggcc aggcagagga agtgcctcag cctgccagat gacagctcac cacgtgggca
75301 ggttagggac caccctgtgt ccctgaagtg aatgcactgc tcagagtgct gtaatgaccc
75361 cattaggcgc tttgacagtg agaacataat ttcacccaat tgtcacattg ggccctcaca
75421 aaacagtaat gtaggcagca gcatttcttc ccattttgca catgagataa atgaggctaa
75481 gagaaatgaa ggggcttaga gataccacta aactagtagg ttgaacaaat tggcgatcgc
75541 ttgtggttca acctaagaga cgtccagctg gtgcacatta caactatgac ttagaccagg
75601 gaatctgagt ccaaaaccaa gattatacac ataaaaatca caagatcata gaacactcaa
75661 gctgcccaag acctgaaaaa catctagtcc agcctctgtg aagataaact gaagcccaag
75721 gtgatgaagg ggtttgtcca ccatctagga gtagcacccc tgtctccagc tcccaggcca
75781 gtgcctttgc caccacccca cacatgccat agccctcagc ggagggcagc cctgctgacc
75841 atcagtgcca tggggagcag ggaagagtcg gcctcctgcc atgttgcttt ccaggaagca
75901 tgcatagccg tggactactt gcttggaatg actggcatcc ctgaggataa gctcctccca
75961 gggctgagca gtggccctgg taccactggc aggcctgagg ccgagagcca agagaaagtc
76021 agggggtgga gaaaaaggca gatattagtc taatatggac ctctgggctt ctgggtagta
76081 aagtcctggt gaggaaacca ccagggcatc agtaagtgcc agatgttggg ctaagctcac
76141 tgtcccagca ggcctcaccc tcacgacccc atataggcat cattcccatt tcaccataag
76201 gaaactgagg ctcagcaggc ttaaacagct ggtccagggc cacattgtga gtaagcggca
76261 gaaacaggat gtgggccgag gtctccactt gggtaaccta gctcgagatt tgctgcttct
76321 gctgaatcca gccctgagcc tggtgccctc ccagggacag gagagcctga agtcacatgg
76381 gtcctggagt tgagacccaa tgtctttact ctgatatctc cagtttggcc tgaacttctt
76441 gatgagtcgt tggttccagc cagggcattg ttggacagca tgctcccaga gacactcctg
76501 ggtgctgctg ggaagctttg ggtggtgtcg atatctcttg gaactggaag gggtttcccg
76561 agagaacctc ttctcaccct ggcaggacca cattttgtca tttgtaccac cagatgcaac
76621 atcaggaaac tgtttactgt ttgctactct ctgttggggg tgaggacggg ccatacactc
76681 atcctcatag atcatagagg aagtcagggg catacactca tcctcataga tcataaagga
76741 ggtctgggat gcagcaactt ccccagctgg gctacccttg actggcaggt gtcagcccca

76801 ggctgaaggg aggtgaaatg atgtttcagg ctggtaagca tatagctcag gatctcatgt
76861 ggtccttgta tctctagcct gagatcacac agtattagga ctagaagggc ctcagaagtc
76921 atgagtcaag tgttaacctg atgtagggac ccccacagca ccatctctga ttggtcctct
76981 aaccttgctt agtcctccca gaaatggggc actcactact tcttatttta gatatcttca
77041 ctttctttag gtcatctcaa aatctgtcac cctgtaacct tcacccactt tcacacgtct
77101 ctctttcagg acaaaaagaa aaatagcctt taaatttgaa ggtaaatttt acattcctcc
77161 tgaaccgtat tctctttctt tgctaaatgt ccccaattca tctaaccatg cacacctcta
77221 tacatctacc cctgtatcca tccatccact cacccaccca tctacaaatc catccaccca
77281 cctgtccaca gatccaccca cccatccatg tatccatcca ttcacgcacc aatccatata
77341 tccattggtc cacccaccca cccatccgtg aatccatcca cccacccacc cgcctgtcta
77401 cagatccacc cactcaccca tatatccatc catccaccca cccacccatc catatatcta
77461 cccacccatc tatccacaga tccatccacc tacccaccca tccacagatc cacccaccca
77521 tatatccacc catccaccca cccatccata tatctaccca cccacctgtc cacagattaa
77581 tggctccttc tatccagtaa tccattcacc atctgtccgt ctatctctca gccggccagg
77641 cagctcacca cccatccctc tgcccattta tgcacccatt cattgagctt tctgtccatc
77701 tcctcatccg acacacatct gctctggcaa gacctgaagc tcctgggatt cctgggacat
77761 ggcccctccc tggaggcctg ccagcctggc tgaggttctc cagtgactcc tcatccacgt
77821 gccttcctgc cctctcacct tgcttgtccc tctcttcaga ctctactgtg gcattatctt
77881 ttctgagaca caggtggcct ggctattccc agaaaggcca acctctgtac acagccccta
77941 acatctccgg gagcccacca gggccacagg cttgcaggac acaggcctgg gctacggtag
78001 aaggtggaag aggagggagt gaaggaggga ggtgctgagt ccctgagagt tcagtgccca
78061 cctaacatcc cgggaagcag attcctaagg cacagtgatg cgcttccctt tgcttgccca
78121 ctgggcttaa agtaaacctg aaggggcaaa tccttcctaa agcgcctcct tccctgacat
78181 ccgtcctttc ctccacacag tcataggtca tgggacactg ggatgagact gacaatccct
78241 gtctttagga gactctcagt ctaaaatcag agccaaacaa tacccagatc agcacagtga
78301 gatgggaggt gtgcatggag agcacagaga acaaacacta aagagcccag gatcaactct
78361 tccgaggaag tcagggaggg ctgcatggag gaggagagac ttgagcaaaa tgagtagaag
78421 tttacaaatt gaatagggag aaagacaggt ggaggtggag agaacaggaa tgagggcatt
78481 ccaagctcaa agagcagcag atgcaaaggc atggaggtat aaaagggcat ataaaaccgc
78541 aacacctgtt cagcaaggag gaacacaggg ctgggtgggc tcagcagagt gagtcaggga
78601 gcactaaatc aggagcctgc aggccatgcc ttttgtcttc accccaaggc actgggaagc
78661 cccagagggg ctgaagatgg ggagcaggag catgagcctc cacctgggaa ggccctgtgt
78721 gcagctgtgt ccttcacagc tatgtgaagg ctggctgact ggggtggggc catagaagag
78781 gccacaggca gaaaaaccca attaggagga ggctgtcgtg cttccaagtc acaccactca
78841 tgggatatat gtccatgggc aggccatgca cccactgccc cagctccaga ggacctcaga
78901 ctctatcaca ccctgggcta gggacaggca gagctgtggc cccaggtccc gggactctag
78961 ggaccaagac agcatttggt tctgccaccc cttggcctgc cctgagcact gggagacgct
79021 ctgggtgggg gtgtctggtg cagtgcccac ccctcatcat tgcagagcac cagttccatc

79081 acaagtgggc catcctgtct gaccccgatg acatctcctc ggggctgaag ggctacgtga
79141 agtgtgacgt tgccgtggtg ggcaaagggg acaacatcaa gacgccccac aaggccaatg
79201 agaccgacga agatgacatt gaggggtgag gcccagctac cccagcccct ggagtctgag
79261 ccagcccttc cttcccccag ggagcccccg gtgagggttt gctgagagca cctgccaccc
79321 gtctccctca ccctgctctc gctcactctc ccaagcagcc ccactcttcc gaggcagggc
79381 aggggcaggg ggtgatggag gatggctgag aatatcagtc ctttgacatc ctttctgcca
79441 gtgagaaatg ggttgagaag agtcgatgct ggcagaccag aaccagcagc tgctgggtcc
79501 tgagtatagc agcaggaagt gctgggagag ccaaggtttc ttctggtgtg ccaattccag
79561 gcagctgata gcagctgttg agaaatattc tgtgaccttg tctggagtca gtgggtaaaa
79621 gtgctgggaa tcaatgaatc ctgtcttttg tgagctgggg gagagagagt ggctactgtg
79681 aagtttttgt ctacgaaaca aaagatacgg cacaaaacta taaaacactg acaataatac
79741 taagatcata tcccaatgta caatctacaa agcactcata tgttttagcg tgcttgagcc
79801 gatcatgttc ttgggaggtg ggtattatta cagccacctc aacagtgagg aaacggcagc
79861 tcagtgagat gggaagattt gcccagaatc ccacaagggg caagttccgt gaacacaagt
79921 gtctaatcct agccttgctc cctcagctgt cccttagaga ctgtgccagg acccaggagt
79981 gtgtagatgc caggatgggg gttggggacc tggacctggt gaccatggcc ctggctgtat
80041 gtgctggcag gaacttgctg ctccccgagg gggtgccccc cgaacgccag tgggcccggt
80101 tctatgtgaa aatttaccga gcagaggggc tgccccgtat gaacacaagc ctcatggcca
80161 atgtaaagaa ggctttcatc ggtgaaaaca aggacctcgt ggacccctac gtgcaagtct
80221 tctttgctgg ccagaaggta ctggggtatg aggtacagga ggctggggct gggtgacagg
80281 tatcacaagg gcaggctgat gggtgtgggg cctggggcag ctgggcaggt aagacaaggc
80341 tggaaatcat gttggccagg gacggactta gaggatcctc actgcctcct ggagtcctta
80401 gactgagagc tgcgcagggg gcatgagagg gaggagaggc tgggggtgtg ggaagagtgg
80461 agacaagtcc acacacaggc acgcacacac acatgcaccc actcatacac gcacgcatac
80521 caactcacac acgcacgcat accaactcac acatgcagac acgcacccac tcacccgtgc
80581 acacgtgcac ccacgaatac acacacacat gcacccattc acacacgcac acacacccac
80641 tcacacatgc acacatgcac ccactcacac acgcacacac acccattcac acatgcacac
80701 atgcacccac tcacacacgc acgcataccc actcacacgt gcacacatgc acccacgaat
80761 acatacatgc acccactcac acgcacacat gcacccatta acacacgcac acatgcaacc
80821 actcacacac gcacacatgc atccactcac gcacacatgc acccactcac gcacacatgc
80881 acccactcat acacacacat gcatccactc acacacgcac gcgcacccac tcacacatgc
80941 acccactcac acacgcacac atgcatccac tcacacacgc atgcacaccc actcacacat
81001 gcacacatgt gcccactcac gcacacatgc gcccactcac acacacacac atgcacccac
81061 tcacacacac atgccacgcc ctcacctgtg ctcccccttc caccagggca agacttcagt
81121 gcagaagagc agctatgagc ccctgtggaa tgagcaggtc gtctttacag acctcttccc
81181 cccactctgc aaacgcatga aggtgcagat ccgagactcg gacaaggtca acgacgtggc
81241 catcggcacc cacttcattg acctgcgcaa gatttctaat gacggagaca aaggtcagca
81301 gcgggagacc cggtgtgctg gggggcagag ggagtggcct gaagggaagg aatatgtgag

81361 atgccctctc ttcacaccac cgtcatcagc tctggtcctc agcctcacag gtgcttccag
81421 ggccttgcca gcagcccctg cctcaggggg agggtgagga tttgtggctc aggtcggctc
81481 agagctggcc tgagcccagc tgactggatt tgtcagctag tagttgtgtg acctgttttc
81541 ccatttgtaa agtgaaaata ataatgatta tatgatatga taattatagt atgatatgca
81601 cattatagca gtgtatatac tgcatttgta tataattaca gtgtgtgtgt atatatatat
81661 gatacctaat agtagtgcct atctgcagaa tcgcagggat tgactacctc ctaggatccc
81721 tgccctgtgc tgggttccaa ggacatagag tgagataaga atctattcct ggatcaaagt
81781 gctcactgtg tattgaggag atagatgtgc taccaaatgg tcaaaacata aggcagaaga
81841 cacaaaaggc cacatgttgt ctgatccatg tatgtgaaac atccggcaaa tccacagaga
81901 cagaaagtag attgccggtt tccaggtgct aaagggaggg gattgggact aactgctaaa
81961 taggtgcaga gtttcttttg agggggtgga actgctctgg aagtagatga tggtttatag
82021 cacagtgaat atacgaaaaa ccactgaaat gtacacttta aaagggtgaa ttttatgtca
82081 tgtgaagtat atctaaattt ttaaaatgct ataagtgtgt tttttaaaaa aaggcagaat
82141 aaaaaggaaa tgaaccaaaa aaactggatc atgagacccc tgtcagaggc ctgacaaatc
82201 ttagccataa gagaagggac ttgtgcctag gaattgggaa ggcttcgtga ggaagcagca
82261 tttcacaggc agagatggag agggatggcc ttcagcaagg aggggagggg tctgcactgg
82321 cagggtcacc gtggtgggac agagctgcaa aaatggcaca gggcccaagg tcatgctgcg
82381 gacttggaag gactggctct gtcctacaag tagactgttg acaattcctg ctctaagttc
82441 caggaagcag ggagctagac tttgccttcc cactgcttcc agctcaggcc cccgcaggga
82501 agaaatagac ccaagagagt gctccacccc agcgcagtgt aagaaggctg ccccacacct
82561 gaatggcaca catgaagttc tcagctcagc acccaggagc tggaggccag ggacccccaa
82621 aagaggcccc tcttccccag ggaaactgga tgtgacgctg ccccctcatt tttgttccca
82681 ctgccacagg cttcctgccc acactgggcc cagcctgggt gaacatgtac ggctccacac
82741 gtaactacac gctgctggat gagcatcagg acctgaacga gggcctgggg gagggtgtgt
82801 ccttccgggc ccggctcctg ctgggcctgg ctgtggagat cgtagacacc tccaaccctg
82861 agctcaccag ctccacagag gtgcaggtgg agcaggccac gcccatctcg gaggtgagac
82921 cccaggcact gtgctgtccc ccagggcctc cagctcagcc cccttctccc tgagtcacct
82981 gggtcccagg ctttgcctgt tgctgccctg tctacctgag gagtcatacc tcaggataag
83041 gcttctcccc aggaagcaga ggcccccact cagccccgca acaggactct gccctcgtcc
83101 actgcctgcc tgtgctctgt ctcacaacgg ccgccaggtc tcacgatggt gcctcccctc
83161 tgcccctctt cccagccacg gctgtccctg tggtgtctgc agccagccct gcttccatgg
83221 atatccaggc tggggggccg ttggggtgtg ggctgcctgg ccccaaagcc gggtgggatg
83281 cccccaccat ccacccccac ggcctgtctg tgagacggaa ggaggcctgg cccccgacct
83341 gccaccctta ctcaagctca ggaacgcttt gaactgcctc cccacagagc tgtgcaggta
83401 aaatggaaga attctttctc tttggagcct tcctggaggc ctcaatgatc gaccggagaa
83461 acggagacaa gcccatcacc tttgaggtca ccataggtga gtgctgggcc cacgggggct
83521 tcgggcctag gtgagtgctg ggcccacggg ggcttcaggc ctaggctgca ggtggtaagt
83581 gtgaggctct gcacctcagc cctgtcctca ctcttgtagg gaacgttgac ccctttcccc

83641 tgagaggcct caagcaccca caaaacacct ttgtgtgtgc atggggtgga ccacgcccca
83701 taaaggggcc aaccctcagc tccacctaca gggtgaggct gtgtttggga cctggggcag
83761 agggcaggga gaagcccttc ccggggctgg agaggagaga gtgggaactc aggccacagc
83821 ctgggccagg gctgggcctc gcactcaggc gtcatgagga ctccctgact ctcagcggag
83881 tggggacacg ggctaatgct ctctggctaa tgatgtgtca cgtctgagcc ccattccctg
83941 actctccctc tcccctttcc caaacccaag gcagcaccgg attggacggt gggaggggcc
84001 tggtggggaa tgcactctag gccttctggg cccattctgc tccaggcaac tatgggaacg
84061 aagttgatgg cctgtcccgg ccccagcggc ctcggccccg gaaggagccg ggggatgagg
84121 aagaagtaga cctgattcag aacgcaagtg atgacgaggc cggtgatgcc ggggacctgg
84181 cctcagtctc ctccactcca ccaatgcggc cccaggtcac cgacaggtgg gcccagcctc
84241 ccatcctcct gtggcctggc cctcccacct ccctgggccc ccaagacctc acctcccgcc
84301 tctgcccacc ccaggaacta cttccatctg ccctacctgg agcgaaagcc ctgcatctac
84361 atcaagagct ggtggccgga ccagcgccgc cgcctctaca atgccaacat catggaccac
84421 attgccgaca agctggtcag ggccaggcca ggggccagca ggggtggggt cctgggggag
84481 acggcgctgt cagagggcct tccctggaac acacaaagct ggtctgccca gggggctgca
84541 ggaacggccc cagcaggacc tctgttccca ttctcagccc caggtcccag ggcacagaca
84601 aggacaggag ccacctctag tcactcagcc tccagtgaat cctggggcag gagtgttggg
84661 ttccctgatt tttccagaaa tcttaaaagc aaggctaggg agacaggtgt ggtggctcac
84721 gcctgtaatc ccaacacttt gggaggccaa ggcaggtgga tcacctgagg tcaggagttc
84781 gagaccatcc tggccaacat agtgaaatgc cgtctctact aaaaatgcaa aaattagcgg
84841 ggcttggtgg cgggcacctg taatcccagc tattcgggag gctgaagcag gagaatcgct
84901 tgaacccgtg aggcggagtt tgcagtgagc caagatggcg ccactgcact ccagcctggg
84961 caaccgagca aaacttcatc tcaaaaaata aaataaaata aaataaaaca aaataaaaaa
85021 gcaaggctag ggaagggaaa tggggaaaag ggagaagcga gcagtgtctg aagctcagga
85081 gctgccccct cctcctctgc ccacccctct ccccccatga gccatgggca ctgactgggc
85141 gaggagtgca gctgttggca tgagacctcc ttctccccag cctggggaag cagggaccag
85201 cagataatcc cgcccttccc tgagctgttg ctgttccctg ttgaagttcc ctgaagctca
85261 gccagctcat attttataaa aatgaattaa aacctccaaa aaaatcaagg ctaatatgag
85321 gtctgctccc agccccctgc caatgtcctc cttgtcatcc ctgtcttgtg actataacca
85381 caattaaagc ccatagcctt tgcagtgtga cctgggtctg cagttatctg caaggcttcc
85441 ccctaaggag ctggggtaca agctgacctg tgtttcctgt gggacctggg tctcacagcc
85501 ctcccagtgc tctcagagcc agagccactc cctcttcccc caggcatctg ttcccatccc
85561 ctgccacagg ctccagcatt ctggagtgac gtcaggatct tggcacatcc agtcctaggg
85621 tttccctctg tctctggcca gccctgagcc ccgtagccgt gagttctgcc caggccctgt
85681 gagctcacca gagccacaga ctcacagccc agaggtggct tcttccttca ggaactgaag
85741 aacccccatg aacaccaaca tctccaggtt ctgagaacag aacctgggaa attgatgact
85801 tcctcatgat gaccgatact caggatggcc ctagcgagag ctcccagatc atgaggtccc
85861 tcactcccct gatcaacagg gaggaggcat ttggggaggc tggggaggcg gggctgtggc

85921 ccagcatcac ccacactcct gattcacagg aagaaggcct gaacgacata caggagatga
85981 tcaaaacgga gaagtcctac cctgagcgtc gcctgcgggg cgtcctggag gagctgagct
86041 gtggctgctg gtgagaaggg aggggcggca ggtgggggga ggatgaggcg gaactggaca
86101 aaggggaccc tgccagggct gggcagatga ggaagaatct acagagcccc tgctggtcag
86161 gctcgctaaa ccccggctcg ccctaccccc agccgcttcc tctccctcgc tgacaaggac
86221 cagggccact catcccgcac caggcttgac cgggagcgcc tcaagtcctg catgagggag
86281 ctggtgagga cgcaactgga cggcgggggc tggagggagg gcctggttgt gagaaggtgt
86341 cacaaccccc tttgtcatgg cccgagctgt cccccagttg ctgggttcac tgacaccccc
86401 tccttcgcag gaaaacatgg ggcagcaggc caggatgctg cgggcccagg tgaagcggca
86461 cacggtgcgg gacaagctga ggctgtgcca gaacttcctg cagaagctgc gcttcctggc
86521 ggacgaggtg cggcccaagg ggtcggggct ttgcctcatc cagggctggc tctctggggc
86581 ccctcaggat cagggggctc aatcaggagg ctagaccccc ttacccactg cccctggcca
86641 ccccccaacc cccagcccca gcacagcatt cccgacatct tcatctggat gatgagcaac
86701 aacaagcgtg tcgcctatgc ccgtgtgccc tccaaggacc tgctcttctc catcgtggag
86761 gaggagactg gcaaggactg cgccaaggtc aagacgctct tccttaaggt gctggagggg
86821 gcaggatgga tgggggcctg ggtcctttca ggggagcagc agcccccacc tggggctgga
86881 agcctgagtg acagggtggg gccttcaagc agcaggtgct cagagaagag ccaagaatgg
86941 gaaggacgtg gggaaggggg tggggaagga ggtgggggaa ggggtggggg aaggggtggg
87001 gaaggaggtg gggaaggggg tgggggaagg ggtgggggaa ggggtgggga aggaggtggg
87061 ggaaggggtg gggaaggggg tggggaagga ggtgggggaa ggggtgaggg aaggggtggg
87121 ggaaggggtg gggaaggagg tgggggaagg ggtggggaag gaggtggggg aaggggtggg
87181 gaaggaggtg gggaaggggg tggggaaggg ggcatgacgg ccccctgccc atcctcctgc
87241 ctccaccgga ccccagccca gcgggatggc tgtgaagggg cagggcagct ctgaccaggg
87301 cctctgccgg cccctgcccc ttccccacct cttgggccct ggcagccgtc aggctcctgg
87361 tgaccccatg cccaccccca gctgccaggg aagcggggct tcggctcggc aggctggaca
87421 gtgcaggcca aggtggagct gtacctgtgg ctgggcctca gcaaacagcg caaggagttc
87481 ctgtgcggcc tgccctgtgg cttccaggag gtcaaggcag cccagggcct gggcctgcat
87541 gccttcccac ccgtcagcct ggtctacacc agtgagtgag gacccctcac tcgaagcctc
87601 acctgggagg gggccggagg ggctgcccat cctggaacct caggctgccc ttccccacag
87661 agaagcaggc gttccagctc cgagcgcaca tgtaccaggc ccgcagcctc tttgccgccg
87721 acagcagcgg actctcagac ccctttgccc gcgtcttctt catcaatcag agtcagtgca
87781 cagaggtgag ggcctgggag gagggagtgg ctctggcttt gagagcacca gaagcctgtg
87841 agcctgtggg gactggagcc tgtggggcct gtgagcctgt ggggcctgga gccacaggtc
87901 agccagtgcc ccggtttctc aggggaagcc ctgcagataa ctgcagcaga cgcgggtcac
87961 actggaaagg ctacgggctt tagctgtggt tatattcaca agacagggac gacaaggagg
88021 acattggcag ggggcttggg ccaaacctca tattagcctt gattttaaga tttctggaag
88081 aatcaagaca cctgtcactc ctgcccccag gttccgtgga agctgagtga gtagggtgg
88141 cttctgtttg tgcatctgcc cccccctctg tcacttgtcc cctgtctccc catcccatgc

88201 tgcaggtgct gaatgagacc ctgtgtccca cctgggacca gatgctggtg ttcgacaacc
88261 tggagctcta tggtgaagct catgagctga gggacgatcc gcccatcatt gtcattgaaa
88321 tctatgacca ggattccatg gtatgggtgg gctctggtgc cagggacccc agcagcacca
88381 cacctggccc ttgttccctg cactccacct gcgctgaggc tgtgccctca cctccgccac
88441 ccacctgcag agctccttgg ccaggtgctg gctgggaagc tgggagtggg ggctgacatc
88501 tagctcctcc tgcctgcttg cggcagcctg accccttccc aggccagcac atcctcacct
88561 cctgtctcct acagactgag ccctggaggt gtgccagcaa gtcctgctgt cactagcctc
88621 cccaccccag ggctccccga cccctgccct cacctaccac aaccagtaac agccttgcca
88681 tcctccgtgg aggttctgga gcagggagta agggattggg gtgacccccg agagttagcg
88741 agccaccttc tcacatacca cctcagctgc tgaggtgccc agatagccct ggactagagg
88801 taggaggacc tcatgtctag tcctgagccg ggcatctgcc atttactgac tgcttggcct
88861 tgaacaagtc tttggtgggc cctgagactt ggtttcctca tctgtaaaat ggggtgatca
88921 cacctgtccc ttacaacaac tcatggcgct gtggtaagga ccaaacgaga tcacaggtgt
88981 ggacagcaag caccaagagg cttctgggtt gtctgttgcg tcagggcaaa gctgacttca
89041 tgggccggac cttcgccaaa cccctggtga agatggcaga cgaggcgtac tgcccacccc
89101 gcttcccacc tcagctcgag tactaccaga tctaccgtgg caacgccaca gctggagacc
89161 tgctggcggc cttcgagctg ctgcaggtgg gactgcaggg aagaggggct gaggcctggg
89221 gactggaggc tgggagttgg ggcctagggc tgggggctgg accctgaagg ctggggcctg
89281 gggtctgggg cctgggggtg gggcctgggg gctggaggct ggggcctggg gctgggggct
89341 ggggctgagg gctggggcct ggggcctggg gggttagggc ctgggcgtta ggtcctgggg
89401 gttggggcct ggagctgggg cctgggggct ggggcctagg ggcctctctc ctacccatcc
89461 tgaccaaagc tgggggcgtg gtccttaggg ggtttccctg tctccccaga tcatggcaaa
89521 caactcatgg ggaaagagac cctgtccctg ggctccccag tgtgtgacct gtgaccccca
89581 ttccccagat tggaccagca gggaaggctg acctgccccc catcaatggc ccggtggacg
89641 tggaccgagg tcccatcatg cccgtgccca tgggcatccg gcccgtgctc agcaagtacc
89701 gagtggaggt gcgagggctc tgtgtggccc ttcccttttg gatgaggagt ctgaggaccc
89761 aggaggggaa gtggcttgtc atccccctcc caccaccatc accaggagcc agcagcagac
89821 ccaggctcct gccccagccc agggcctgcc cccatctgca ccaagtcgca ctcccatgtc
89881 ccaggaccat gcccagccct cctgtctgca cccctgaccc tgcacccctt ctccgggcag
89941 ccccaccctc atccctggag aacgcctgcc cctcctctgc tctcctggcc ctgctcccac
90001 cctttgaaat ctggggtgaa ctactgccca tcccactgcc cggttcccta ttggatggtc
90061 cagcaccccc atggctggct cccttgacta aggggctctc ttctctgcac ccaccctcca
90121 acctctccca ggtgctgttc tggggcctac gggacctaaa gcgggtgaac ctggcccagg
90181 tggaccggcc acgggtggac atcgagtgtg cagggaaggg ggtgcagtcg tccctgatcc
90241 acaattataa gaagaacccc aacttcaaca ccctcgtcaa gtggtttgaa gtggtgagtg
90301 caggccctgg cggaggacat cctgtggcca gtgtggctcc actcagccag ccggcttcct
90361 ctgccccttc ttggaggctc aggctcctgt cgccaacacc ccaggacctc ccagagaacg
90421 agctgctgca cccgcccttg aacatccgtg tggtggactg ccgggccttc ggtcgctaca

90481 cactggtggg ctcccatgcc gtcagctccc tgcgacgctt catctaccgg cccccagacc
90541 gctcggcccc cagctggaac accacgggta tggccacatc caccctgcca ccaagcctgg
90601 ctccacgccc cccagggaag ggctccaggt ctccgcccag tccaagccgc gaccagagtt
90661 tgctctttgg ggccgagggg gtgggcatag aagaaggtca ccctggtggc caaggaaggc
90721 tgcccaaggt gacgccagct ctacagccag agagggcaga actggagaca gcctgggggg
90781 gcctgagggc agaaagcccc ttgcgctggt tgatggagaa gacccaggcc tccccgccac
90841 ctccctccca ccggcctccg cagcttcccc ttatctcggg ctctcttctt ccctccccat
90901 gcccatattt tctcccttga ctgcctgtcc ccctctccct cagaaagttc cagaatcagc
90961 tttagttctc cgcaccttcc tgccccgcca atctcactgt tcctcttgct ccttcctctg
91021 cctgtctgtc catctatcca tctgtccagt caggcttctc cggcgctgcc gtgtgctgtg
91081 caatgggggc tcctcctctc actccacagg ggaggttgtg gtgactatgg agccagaggt
91141 acccatcaag aaactggaga ccatggtgaa gctggacgcg gtaaggcggg tggggtcagc
91201 cccctgctgg ctgggaacat gcaacttcgt gtgacacatc ctgtgaacca gactatctgc
91261 cccgagagaa aaggacacta agagcctcct ttggcatgtg actgtcgggg gctgagcttg
91321 attgtgtgtc caaacagctc cctcctaata ctatcctgga atgcacacgc gtgcgtgtgg
91381 tatgtaaatg tgcatgtgtg tgcgcatgtg tgtggtgtgc atatgtgcat gtgtgtgtgc
91441 atgcatgtga tgtatgtgca tttgtgcgtg catgcgtatg tgtggtgtgc atatgtgcat
91501 cgtgcatgta tgcatgtggt gtatgtgcat gtgtgtgtgc atgtatgtgg ggtatgtgca
91561 tatgcacgtg cgtgcgtgtg gtgtgcacat gtgcatgcat gtgtggtgta tgttcatatg
91621 cacgtgcatg catgcatgtg gtatgagcat gtgcatgtat gtgtgcatgc gtatggtgtg
91681 catatgtgca tgtggtgtgt gcatatgtgt gtgcatgtgg tgtgtaaatg catatgtgca
91741 tgtgtgcagt gtgcatgcat gcgggtggta ggtaaatgtg catgagtgtg gtatatatgc
91801 atatatgtgt gcatgtgcgc ttgtgtgcat atgtgcatgt gtgcatgtgg tatgtaaatg
91861 tgcatgtgtg catgtgtgca tgtggtgtat gtgcatatat gtgtgcatgc atgcttgcgg
91921 tgtgcgtatg tgcacatgcg tgcatacatg tgggtgtgtg catgtgcact ggcatgtggt
91981 atgtgtgtat gagagacaga gtgagccatc acagtttatt ataatcacct gctataattt
92041 ttactccaac aaatgacacc ataggtaatg attccttatg agatcatcag tgtcctttcc
92101 aaagagtccc tggttttcca tcccagccat tcacaaatga cacttccctg gcctgggtca
92161 gaggaggatt tccaggtgga agctccttat tcctcctggc tttgggccta ggactgttca
92221 tctgccagag agagaactgc catctggggg ttgggtgtgg aagtggcgta caagctgtag
92281 accctacagg gaagggcaga acgtgggcca gcctggaaca cagcccactg ttcctttggc
92341 cacccaaaga aacatcctca ctcagaccct ttgcagactc accagtcaga gtgctcttat
92401 taatgcaaag ttggccttgc gggcacaggg gctgccagca cacagtaatt tccatctcgg
92461 ctcctccaca caccctgtgc taggccactg cctgccactc caggccccag tggctgattc
92521 tgcccctgtc tccttggttc ctctgcagac ttctgaagct gttgtcaagg tggatgtggt
92581 gagtgtgggg gccatgcagg ggctctgggg tgagagggtc ctttattggc aatgatgtca
92641 ggccaaaggg tccagcatca gggacagagc cagccccagc ccccgcatgc ttgggccagc
92701 acacctcccc taagatgcag tggaaaagag ctgggtgagt gcaggactcg gtgccagtcc

92761 tgcttgggcg agtggatcgc actggcaggt cacaacactc tgtggcctgt ggcaccttct
92821 ccataaaata aggccctgcc ccttggacat gggtggcccc acagagcctc atggagccca
92881 aggcttcctg ctgacagatg gcggaatggg tggaggctct cgggattgtc caggcaggag
92941 gagtccatgt aggactctgg acctggccaa cctgcaggct gaggaggaga aggagaagaa
93001 gaagaagaag aagggcactg cggaggagcc agaggaggag gagccagacg agagcatgct
93061 ggactggtgg tccaagtact ttgcctccat tgacaccatg aaggaggtga gacctcgggt
93121 agggtgaggg gaggtgaccc cctccacact cagaccctcc tgtccagctt ctttcccacg
93181 gcccccaaga gctggatccc aactccattc atgtgagttt gggatggttt tgtctcacat
93241 ccttgaaatc ctagctcaga actcaccttc caagatgatg gtcccatcta ccttccctgc
93301 ttggttttct tttgtcctca ttgactcatt ccacaaatgc ctcttgggta tatgcgatga
93361 gccaggctcc atactaggtg ctatgggagc caaaagatgc cacacctgcc ctgatgggcc
93421 tccggtttac tgggcaaaat ggacatgtaa acagagactt agagaggact atgtgataac
93481 aaaggcaaat catggcttgg gggaatcagg gaaggcgtct cagaggaggt gacctttgtg
93541 gaaaaggaga aacatgttct cagaggccat aagggaacaa ctgcttatca gtagtcctta
93601 cgactcttct tcataatgtt ttattttaca catcttggtc atcggcacgt gaatgattct
93661 tcccctatta cacatatgtg ggttccatct gtttggccag attgcaagtt ccagaaagcg
93721 catttctctg ctttcttgtt tgtgtcctct ccacgctgcc tacctagctc agggtgctct
93781 gactggccac ctgaccagga gggacacatg cctgcagttg tgtttcctca gtggggccag
93841 agctgatcca gccacatcct ctcccaggcc ctttctgtga cccaagctag gtgtgatatt
93901 tattagagca cacattaagc tgctgtagca agttagagtg aagtgaacaa gacagcagtt
93961 tatttctcac ataacatctc agagagtggg ggttaggctg gtatggtggc tcagtggtgt
94021 caaggactaa gatcctgacc tcttgtttgt cacatctgta aggttggagg tgactcaaca
94081 tcatgttcta attcaagtgc acaggaaggg ttaaagagga gatgaggcat atgcttttct
94141 ttttcagggc ccaatccaga agctgcacat atgacttcca ctcacattga tcagaatcta
94201 gtcccttagc tacacttatc tgcaaaggag gctgggaaat gtaatctttc acaaggtggc
94261 tgtgtgctca gcaaaacttg taaaaatagg agaacagatc attgaggaca actggtagtc
94321 tcgtctccca ccatcaagtt ggccaggctg gagctgcaag aggctgcctg ttgtcgtctc
94381 tactcctgca attttcacag actgccacca ggtggcggtg gcgacttagt tgaaacctgg
94441 tttctagttt ctagtaagat ttattctcgt ttccagtaag atggggtgtg ctgagtgaaa
94501 gaaaatatag tacattttct tactgttata cttttgtaca ttaaaacaat ttattaatat
94561 cactaaaagc aacattaaaa tgcactgtac ttttttaagt agaaaatgta aatatttatt
94621 gcaaggtgtt atttcttttt tttttaaatt atactttaag ttctagggta catgtgcaca
94681 atgtgcaggt ttgttacata tgtatgcatg tgccatgttg gtgtgctaca cccattaact
94741 cgtcatttac agtgggtatt gctcctaatg ctatccctcc cctctccccc agcaccacca
94801 caggccccgg tgtgtgatgt tcccctccct gtgtccaagt gttctcattg ttcagttccc
94861 tcccatgagt gagaacatgc agtgtttggt tttctgtcct tgtgacagtt tcttcagaat
94921 gatggtttct ggcttcatcc atgtccctgc aaaggacatg aactcatcct tttttatggc
94981 tgcatagtat tccatggtgt atatgtgcca cagatgcata aacccctaag taccactagt

95041 cacactgtac acattataaa ctttttttaa cccacttaag cagagcacat ggtggacttg
95101 aaatgcagta gggaagattt tctacttcta gtgcattaag actggtctgg gcccacaatt
95161 tggggtcaac agactcctcc ccaggaaacc caccttctgt ccttttcatc tcatcttttg
95221 gcagcaactt cgacaacaag agccctctgg aattgacttg gaggagaagg aggaagtgga
95281 caataccgag ggtgagccct ggaacctgga actctgcctc ctcctccccg cccctgggtg
95341 gtcagctctc ctgcccacct catcacctgc ttctcttttc cctcctgcca gccctcccag
95401 ccttagtagc cagggaagca gcatcagtaa tccatgggca gtactgggca ggctaaggcc
95461 cttgtccagg gaggctttta ctctgaccag ttcagcaccc acagtgatgg gcagacattt
95521 agagccacag aacatcagag ctagaaggga cctcagagct cattccatcc agcattttcc
95581 agactctttt ccatgaagaa aattgatgga tgttctagaa ggaacaaatt agttttggtc
95641 aaattagttt aggaaaagct gattgaaaca gcttgttttg gtgcaggact tttcataccc
95701 tttactgtgc aaatgttatt atacatctct aaaaggaaga cacagtataa aatatttccc
95761 cagcttattg gatcacagta ccctttcttc taaagcatgc cagcagcatc tgcaccaggt
95821 gctccactga atgaggtttg ggaaatgcta atacagagtg ttattattat ggacaagaag
95881 agggaagcta gtgaggaaag tgagtgggca aggaactgcg cagcaacagg cctccacccc
95941 caaacccatg ctccttcctg cccctctctt ccaaagggca tgaagacagt cttcagacac
96001 cttcccgggc ataaacccac tggggccccc agcacctcca tgaggcgggc atgctaggga
96061 gtattatctg catttgacag cacatggaca tccgagagtt aagttagtaa agcaaattag
96121 cgacagagct gggtctcgaa tccaggcctt cctgctctgc tcatttctgc tccacctcct
96181 tttctgcttc tggccaggcc tgaaggggtc aatgaagggc aaggagaagg caaggctgc
96241 caaagaggag aagaagaaga aaactcagag ctctggctct ggccaggggt ccgaggcccc
96301 cgagaagaag aaacccaaga ttgatgagct taaggtgaga gcctaggagc agacccttct
96361 agggtgtgtg ggggcgggaa gaggggggcg cagtgatcct gcaggtgggg caggacaaaa
96421 gccagagacc aggcagccgc cccctaacag cctggtctgt ctcaggtata ccccaaagag
96481 ctggagtccg agtttgataa ctttgaggac tggctgcaca ctttcaactt gcttcggggc
96541 aagaccgggg atgatgagga tggctccacc gaggaggagc gcattgtggg acgcttcaag
96601 gtcaggccag gagcacgggc ccagcctgag cacccgccag cccttgcccc ccccacaggt
96661 tcccccactc acctcccacc actgccccgg ccactgcctc cagaagccct ggactctccc
96721 atgactcagc agttaaaggg tgaaggccct gccacatcag ggtccagccc tcctggtgct
96781 gttagctatt ttgaatgcca cccttgccag gcaaacctac accctgaccc acatttgtct
96841 tgcccagggc tccctctgcg tgtacaaagt gccactccca gaggacgtgt cccgggaagc
96901 cggctacgac tccacctacg gcatgttcca gggcatcccg agcaatgacc ccatcaatgt
96961 gctggtccga gtctatgtgg tccgggtgag actcccgtcg ctttccctcc ttgcaagtct
97021 cctcatcctc ccatctggga gagagcaaag aagctgcata ctggccaggt gcaatggttt
97081 acgcctgtaa tcccagcact ttgggaggcc aaggcaggag gatcacctga ggtcaggagt
97141 tcgagaccag cctggccaac atggttaaac cccgtctcta ctaaaaatac aaaaattagc
97201 caggcacagt gccacgcacc tgtaatccca gctactgagg aggctgaggc aggaaaatcg
97261 cttgaacctg ggaggtggag cttgcagtga gctgagatca cgtcattgca ctccagcctg

97321 ggagacagag cgagactctg tctcaaagga aaaaaaaaag aagaagaagc tgcatactga
97381 cagccccaga ctgggaggca ccagggagat catttggtct agtctttatc ttttctagag
97441 gaagaagtca acgcccagag aggggtagcc acttgcctag ggtcacacag caagcctcct
97501 gaggggtcca gtgctcctgc ccctgccctt cactgtcata ggaccctcag gcagcctggc
97561 aggggcactg aagatgagca gggcatggga gcctgctcag gcagcccttc tccccattcc
97621 aggccacgga cctgcaccct gctgacatca acggcaaagc tgacccctac atcgccatcc
97681 ggctaggcaa gactgacatc cgcgacaagg agaactacat ctccaagcag ctcaaccctg
97741 tctttgggaa gtaaggcttc ttggtgccct cccctgccac acccctactt ctccaaccca
97801 gaacacctcc caccctagcc aatccttaag cccttctcct aactccaccc caccaggtcc
97861 tttgacatcg aggcctcctt ccccatggaa tccatgctga cggtggctgt gtatgactgg
97921 gacctggtgg gcactgatga cctcattggg gaaaccaaga tcgacctgga gaaccgcttc
97981 tacagcaagc accgcgccac ctgcggcatc gcccagacct actccacgta tgtggggcat
98041 ggggcagagg gcgggggcag tgtgcctgct ttgcctccac tctaaaatga gattcagatc
98101 ctggtgattt gagaggttag acattgtggt ttctgtccca ggccaggcct ctgcccctac
98161 agtccctggg cacagtcttc aggggacagg actgcagatc ccaaaacaag gagagggaca
98221 gtggattcca ggacagagga ggcaggacga ggagagacaa gtgggctagg gaaggtgggg
98281 gctagcaaga ggaggacaga aagagtcccc tggggatctg ggccagctct gccacttata
98341 ggctgtgtga ccctggccaa gtcccttcac ctctctggtt ttcagtttcc tcatgggagt
98401 ctctgaggtt ccagaggagt gggcacagtg aggggatgca gtggggctag gctggggaca
98461 ctgatatgtg gtccaaggcc cagggcagga aggttcagcc tccagcagcc ctagtgtctc
98521 aggtgagcct ggggcccagg gcaggaggaa tcaggctcac ttgctgatga gggcaacagc
98581 caactttaga gaactggcca cgaccagctg tcatccagga tggcttagtc ccacccagcc
98641 cctccaagcc tgtgtgtccc ctcccacaga catggctaca atatctggcg ggaccccatg
98701 aagcccagcc agatcctgac ccgcctctgc aaagacggca aagtggacgg cccccacttt
98761 gggccccctg ggagagtgaa ggtggccaac cgcgtcttca ctgggccctc tgagattgag
98821 gacgagaacg gtaatggggc actggatgca gccgcctgcc ccctctccag gggtttcag
98881 aggccaggag gggcccacac ttccctgggg aacctcacag cctagcctgg ggtgtcctca
98941 gcctaaacct ggtcttagtg accctctgct catcacccca gagccccgtt cctgtctgcc
99001 cagcctctgt gcttccaccc ctcctacagg gcagccccac ctgctttctc tctgtccaaa
99061 tcccagtgag gtagagaggc tatctgcatc ccattctgca gataaacaaa ccaagatcct
99121 acttctagtg actcgcccaa ggtcatgtgg ctctttagag gcagagcgag ggcatcggcc
99181 caggtccatc tggctctaat ccttaaactc tccttacttc tctgtccatg aggcacttcc
99241 cagagaagca gagaattggg aggtgtctgt gctggtatct gatcactttc aggtgctggg
99301 acaggaagtg gattctcccc tccctgaccc ttctctcagc ccagcagggc tctccagtca
99361 acttccccaa gcagaggacc aagggcacag acctctcttg gccctgtggg gcagctgaga
99421 ccctgggcaa gccacttctc cttctgtgcc tcagtttccc caacagtgaa aaggaggtgg
99481 ggtagacagg tgatggcata gaggcttctt tgtgctccta agtccctagt cccagcaaag
99541 gtcttctggt tctgctgagc catgtgtgca gctgagccgc cggcacccac aggtcagagg

99601 aagcccacag acgagcatgt ggcgctgttg gccctgaggc actgggagga catcccccgc
99661 gcaggctgcc gcctggtgcc agagcatgtg gagacgaggc cgctgctcaa ccccgacaag
99721 ccgggcatcg agcaggtgac acttgcatgg ccaagactgg ggttcttggg tattggggac
99781 cagggatccc caacctgagt cagtaaaggt agaagccagg cctcacgctg tccgctgcat
99841 tccagaaagc ctggcaggca ccttcccacc aaggcccctc agggtaactt gaatggcaag
99901 ggtctttgct ttgagtgaga attacatcag ctcaggcaga gacctgggtt gtctccctca
99961 gaagctgtga ttggctattg gtgacatcat aggaattcct ggcatccaaa gatggaaact
100021 gatgaactgg gtccttgaca gaacaaaggt taggagggag aggagagctg
atcttcctgg 100081 gcagagggag aaggccctgg gagatctgcc acaaccctct
tcctcagggc cgcctggagc 100141 tgtgggtgga catgttcccc atggacatgc
cagcccctgg gacgcctctg gacatctcac 100201 ctcggaagcc caagaagtga
gcggcctggg gcccagcgct actcccttcc ggcccacccc 100261 acccacttcc
caccacggcc caacccttcc tgcggggggc tcaggtccag atggccttgg 100321
ccagttggca tccctggggc ctgggtgtgg ccactgggcc ttatagggga gaaactgacc
100381 aaccaatcga tagagtttag ggcctgccct ggagagtccc cacgtgctaa
ggcaggacaa 100441 cgccttccac cctgcatcag tgagcgagac aagccccaca
cagcacactc cggtgcattg 100501 tatctacacg gtctacgcgc ccatacacat
gcatctcatg cacacacaca cacacagcat 100561 agcactccca cggacaccca
cacacatgcc cacactgacg cactccttca caacacacag 100621 aacccctggg
agccggccct gcgtcatcct cacatccagg ctagtgtgat gttccgccaa 100681
gttttctttg gagtgcctgc cagtgatgaa tccaggagtg agtgcctgga agaggcagga
100741 ggtggtagag tgaaaagaac ccgggtttcc catccataaa gcggcaggtt
caagtccagc 100801 tgtcagacag tgactggatt cagggccagc ttcagggatg
gaggaagcca cacagggtcc 100861 cactcttgtt tccatgctcc tatgttgcca
tcttgaaatt ctttatgaca agtgaccctg 100921 catttccatt tggcgctggc
cccagcaaat catgcagcaa attctgccag gaatgtcctg 100981 gaaacctgag
gccatgagtc agctgttcca gggcctgtca ttagggggca cccaactcac 101041
tgggccactc tgggctcgca gccctgtctg caatcccagg agagccatgc tcagatcaat
101101 ttttagtgaa tgactcagcc tcactcttaa tgattgaaat gaactacatc
attcataatt 101161 acaattaaac cttgagggga aaatcctctt tggggacagg
gacatacagc ttccatgggc 101221 ttccattgct ggctactggg cttaaatgca
gacttctctt ggaactagca tctgagacag 101281 agggtggctt cctcccctca
ccccaccccc accgcccaca gccatgtgcc tggtgggcct 101341 tctcactcat
gcctctgctc ccccaacccc aaaccactcc tccagcccca gcctggccca 101401
ggccctgtgc tgaccccaag accccagggc ttctctccgc ctgccccatc ccagccctgg
101461 caccctggca taacccccac ctggggctgc taaccagcct tctctccctg
ggcccaggta 101521 cgagctgcgg gtcatcatct ggaacacaga tgaggtggtc
ttggaggacg acgacttctt 101581 cacaggggag aagtccagtg acatcttcgt

gagggggtgg gtgagcagtc cctgcatggg 101641 agggactgag ctgggtgggg
cttgcttccc ggggggcagg agaggaggca gagggaaggt 101701 ggctgtgggt
gggagaggcc ccaccagcct ggctgcgggt ctggagatgt ggcgcccaca 101761
ggtggctgaa gggccagcag gaggacaagc aggacacaga cgtccactac cactccctca
101821 ctggcgaggg caacttcaac tggcgctacc tgttcccctt cgactacctg
gcggcggagg 101881 agaagatcgt catctccaag aaggagtcca tgttctcctg
ggacgagacc gagtacaaga 101941 tccccgcgcg gctcaccctg cagatctggg
atgcggacca cttctccgct gacgacttcc 102001 tgggtgcaga gcagggcagg
gatgggggtt caggatccag aactgagaag aggcaggggc 102061 cgggttggcg
gggacaggga gagccctgcc ttggggggac aaggggtcat tgcttccagt 102121
ccatcagagc caggggtgtc cgacggggtt ccgcccccaa ggacccacct aaccgtccct
102181 aggcagatgt tctcagtgtc ataaccaggc tgacaacaag ctttggacac
accaaggagg 102241 gggcagtcag tgatgatggg ggaggggaga ggctccctaa
gggagtcggg aaacctccct 102301 gggcctcagg ttgcttctct gtaaagcagg
gattctccct aaaactctaa tctgcatctg 102361 caaaagcttg tgggattagc
aggactttga cagaatggat gcatggcagg gagtggcagg 102421 ggagacccca
ggagagaccc tctggggtga aagaaggacc tgagtaaagc atgggggctg 102481
gggctgccta gcaagcccac gaggatgcct gcattgaggc cagttgctgg gagtgaggca
102541 ggaggatgag atgtgggctc agggaggtct gcactcagca aacaaggggc
cgaggagccc 102601 agacccccac ccccaccccg ccccacccca gccctgttca
ctctgagctg acactgaggt 102661 tgccacaggg gccatcgagc tggacctgaa
ccggttcccg cggggcgcaa agacagccaa 102721 gcagtgcacc atggagatgg
ccaccgggga ggtggacgtg cccctcgtgt ccatcttcaa 102781 gcaaaagcgc
gtcaaaggct ggtggcccct cctggcccgc aatgagaacg atgagtttga 102841
gctcacggtg cgcacccctt cctgctcggg cagtaggcag ggctgggact ggctggggtg
102901 ccaagggccc agcacgcagc ctggacgctg ggacctgtct gtcctctgca
gggcaaggtg 102961 gaggctgagc tgcatttact gacagcagag gaggcagaga
agaacccagt gggcctggcc 103021 cgcaatgaac ctgaccccct agagaaaccc
aagtgagtgc cctgccggcc cagcccctcc 103081 ccactctggc tcttggaggc
gctggaacag gaaggagacg atccccagac accccatctc 103141 ttcctgggcc
acagcctcat tacaggggag cctcctccct gccctctgcc aaccctgaat 103201
cccacaacgt ccctttgaac cagaggatgg gctaggaata ctggagagca ggaccttccc
103261 tgggaaaggc cccatctagc ggagagaaac tatgctcttc ccttcctagc
ttttgcagaa 103321 gagccagctc cagtggtggc ctctggggcc atggccagcc
ttgatggcca gaggggtgcc 103381 tagaactgca cacacagccc aggacagctt
ccctcttggt cttggcccct cgccacaccc 103441 aatcccttct cctccctgtc
cctcctctgc gctctctacc cttcatactc cagccggccc 103501 gacacgagct
tcatctggtt cctgaaccct ctcaagtcgg ctcgctactt cttgtggcac 103561

acgtatcgct ggctgctcct caaactgttg ctgctcctgc tgctgctcct cctcctcgcc
103621 ctgttcctct actctgtgcc tggctacctg gtcaagaaaa tcctcggggc
ctgagcccag 103681 tggcctcctg ggtctgatat ttcttacttc ttcctctgga
ccaagggcta ggcaggcgcg 103741 tgcacacaca cacatataca cgcacacatc
cacaaacaca aatatgcata catgtgcaca 103801 caaacacaca caagcatgta
tgcacacatg tgccacgcac aagtgcatac acatgcactc 103861 aagcacacac
aggcacacac ttaagcccac acaaacatgc acagcaactt tcaagctttc 103921
atgacagaat agactctcag aactagaaat gactatttta cacttttcca cattaagcca
103981 aagaggtgaa gtagcttgct caaggttacc cagcaaatta gggtagagcc
gggactcaag 104041 ccaaggtttc caaatttcca atcctgtgtt ctttgcacac
cacctctgct cccagccatg 104101 tttctcccag actgtaaccc aatttttttt
tttttttttt ttttttggag acagagtctc 104161 gctctgtcgc ccaggctaga
gtgcagtggc acaatcttgg ctcactgcca gctccgcctc 104221 ccgggttcac
gccattctcc tgcctcagcc tcctgagtag ctgggactac aggcacccgc 104281
caccacgcct ggataatttt ttgtattttt agtagagacg gggtttcact gtgttagcca
104341 ggatggtctc gatctcctga cctcgtgatc cgcccatctc ggcctcccaa
agtgctggga 104401 ttacaggcgt gagccaccgc gcccagccga ctctaaccca
atttcattgg cagaaagaag 104461 ctgagctatg gacagggaaa agggaagggt
gtggccaggg ttagccaaca ggtctggaaa 104521 tggctctcac ccgtgtgtga
cctcccagtc cagggctgtt gttttctgcc cttcaggata 104581 ataattccat
tcaactcaaa catttcatag ccaccatggc ctgaacaccc aactgcaggt 104641
gctcagagct ccccaggcac tggctcactg tgccattgcg ccaaacccag gaggtaggta
104701 ctgcttgtaa ccccatttta tacatgaaga aaccgaggtt gatagaggtg
cctcacttgc 104761 ctgtgaaaac cattcaaaac caggtgccct gcacccaagc
cccggctctg tgtccccacc 104821 aatactgagc tcagctctgg ggctgcacgc
catgtcctct gggaggcagt agtttgaaaa 104881 tgcatatgca gtactttcac
ttgtatcttg acattttaca aaacctattg tttttataac 104941 acaaacgaca
gatagtgagg ctggccaggt gcttctcggc tgggaggctc tacagaaggt 105001
ggagtgggtc ttcatcctga gagtgggcag gtcggtgagg gcagcttgca ccccacaggg
105061 gctgctcaga ccctcagcag ggcccggtgg gaacagccat ttatgtccat
cagaaaggaa 105121 gaggttgtga agcaccagtc ttaccaagga cgtggggcag
cacatagaac gagctaaccg 105181 caggagcttc ctaacgatga gaactgcctg
cagggccaag gctgccccat gctgcaagta 105241 gctcccatct aggccttagg
aggagattcg ggccccaaag gtgctgcagg gaggatagag 105301 ttttgtaagc
agcaagtcgg gtacttagaa atgggtttaa accctggctc cagcattcac 105361
tgcttttggg gctggggcca tggcagggct tttgggggtc cagaaggaca gaggatgggg
105421 tgcactggga ggcagctcct gcccactggc tgaccggctc ctctcttcca
cttcccagcc 105481 ggcccgacac ggccttcgtc tggttcctca accctctcaa

gtccatcaag tacctcatct 105541 gcacccggta caagtggctc atcatcaaga
tcgtgctggc gctgttgggg ctgctcatgt 105601 tggggctctt cctctacagc
ctccctggct acatggtcaa aaagctcctt ggggcatgaa 105661 ggccgccagc
tcccgccagc cgctccccag ccctgccgca tttcctttca gtggcttgga 105721
ctctttccca tctcccctgg ggagcctgag gagcccagcg tccactcttc atgccttggg
105781 ccgagcctgc ctcctgcttg cgggggccgc ctgtcctcac tgccccaggc
tgcggcttgc 105841 ccagtcccgc ccctctgacc cctgcctgtg ggctggggag
ccttggatgg ggtggggacc 105901 tggaatgggt ctctcttgcc ccacctggct
gaggcgccac ccttcttcag gcccaggctc 105961 cagaggaaga ctcctgaaac
cctccccagg tcttccaagt acaggattga agctttagtg 106021 aaattaacca
aggaccatgg gtcagtgccc agggctttaa aaagaatgaa cgagcaaaag 106081
gtatccccgc cgtgacccct gcagatagca ccggtctttg atccgcagca ggggccagac
106141 cctgcccaca agtcccagcg cggctgcttc tgccactgct gggctccact
tggctcctct 106201 cacttcccag ggggtcgcct gtcctgcctg tgggtttcca
tggcttccca gagctccctc 106261 tgccccagcc agcgcctcca ggcccagctg
aggagctgtg agaagcagca gaggggactc 106321 cccatcccgg gcacaccctg
tcctcccacc cctgccccct tgcccttcca gccctttcag 106381 ctgcagctgg
gagctggccc gtcaagtgct gcccctgcct gtgtctgggt ttctgttggc 106441
tgtttttctt ttcttgagtg gtgatttttc tctaaataaa agaagtcaag cactgagctg
106501 acaggtctgc actggtcaag ctgagaggag cagccgcagg tgcggggcag
aggcagcagt 106561 tctctaggct ggcagattca gtaccagcag ggctcccatg
gcctttcccc ttcaagtggc 106621 ctctggtggc ccagggttca aggcaggcct
caggcacagg gctgcccacc caggctgccc 106681 acaggaggag tcttacacca
tagcccaaag ggcacaccag cctgggccca ggggccccca 106741 aagcaccgaa
agaatccttc aggtctgggg ccagtctgaa ggcttaaggc cccacaaggg 106801
cccaaaagct ctagcctcca cccacagaaa ccagttcccc tgaaagcaag aaaggagccc
106861 cacgctactg gcctcggcct ctgactcaca ccactatctg agcctggggc
aaagtcccct 106921 catgacacag aggtaggggc aagggcctgg tcttgctaga
cctgacctct ttaaagagat 106981 tcagccacaa ctttaaaagg ctttaatcat
aatgcaggac acaggtaact cctctggctc 107041 cttatttcga caggctataa
agacagccct ggaaaatcca gagagcaggc ccaggtgcgg 107101 cccagtgggt
gatatgagct ggctccggca cagacaccag catcaggcca gccctaacac 107161
atcagccttt ggcggtccag ctcattttgt gggtacccgc aacacctgag tgggaggaac
107221 ttgcagttca gagttgatct ggaaaagaaa gggtgaaagt cgttacattt
ttcccagggc 107281 cctccttctt tgccaccctt gcgagccacg ctggctctcc
catgggcctc caaagccctg 107341 cacgcatctc ctctccaaag ctgagagatg
ctgaacaccc acagactgcc tccctgcagg 107401 agacacgcct gagccttctt
gctcagcttc tgctccagct gtgacctctg gtagcgttct 107461 tcatctcccg

cccgtccatt ctctgcagtc ttcccctctc ctctctccac gcacccatgc 107521
catccccaat ctagcctgcc ccacctcttc ctcccccagc ctcttgtata cagtagcatc
107581 tgtggaagcg ggaaccatac cgtccagaat gtctgggtcc tgtcacaggc
caagggtcat 107641 tatgaggcca aggttcacta caacatccca ctttcagctg
tggcctggga cctgggactg 107701 tgctggggcc acgcagcccc tgtggctgga
cttggagctg gcctcagtca cctcacacgt 107761 gtgcaactaa cagctgcgtc
acgaccttcc tggcctacag ccccagctgc aggtgcccac 107821 acccgcagag
caggaaatgt cagcaatgga ctttttcctt cctcctcttg ccaggccatg 107881
gggaagttgt ccacaactat tgcagggttg ggcacagatg ggggccaggg aatatctgcc
107941 ccagcagagc ctccttcccc atgcgaggat gaccctccag tcatatccca
ggcagggaac 108001 cggggctggt gggtggaaag agcccaggct ctggacgagt
tgcaaactct gcaagttgtg 108061 attgtccttt gtgtgaaatg aggagcattg
tttccgccct gtccacctca cacagcgggt 108121 gtgaggatgg aagaagagag
cacagctgga ggctctgtag ctggggagct gctcctggct 108181 gtggcactgc
ttcacacaga gcagtcccag ctggccactg gcaccagaaa ccacagtatc 108241
aaagagacac tgtcacacct gagctgtcct ggaggagact tcaggcaaga tctactcctt
108301 tggccagtag ttgtctcaag gtgccactct tgatttttta catcatatca
caaagctccc 108361 ttgcctctga cggggagggg actcctcgtt ccctaatccc
agctccagtg cccctgcccc 108421 acccgtgtct cccgctgccc ctccttggaa
ggccccgccg cccaccttgg agttcagata 108481 ctgctgcagt agcgcc

### Norrin Cystine Knot Growth Factor (NDP)

The NDP gene encodes "norrin cystine knot growth factor" (NDP). The human ND gene is located on chromosome Xp11.3. It contains 3 exons, encompassing ~ kilobases (kb) (NCBI Accession No. NG_009832.1). NDP encodes secreted norrin protein that plays a role in retina vascularization of Wnt signaling pathway through (FZDA) and (LRP5) coreceptor. Mutations in NDP have been associated with Norrie Disease Pseudoglioma, an X-linked recessive syndromic hearing loss that is characterized by early childhood retinopathy. About one third of individuals with Norrie disease develop progressive hearing loss, and more than half experience developmental delays in motor skills.

Various mutations in the NDP gene have been associated with hearing loss (e.g., Norrie Disease Pseudoglioma). For example, the p.His4ArgfsX21, p.Asp23GlufsX9, p.Arg38Cys, p.Ile48ValfsX55, p.His50Asp, p.Ser57^{∗}, p.Cys93Arg, p.Lys104Gln, p.Gly113Asp, p.Arg121Gln, and p.Cys126Arg mutation have each been associated with Norrie disease pseudoglioma. See, e.g., Musada et al., Mol. Vis. 22:491-502, 2016; Chamney et al., Eye (Lond) 25(12):1658, 2011; Liu et al., J Chin. Med. Assoc. 79(11):633-638, 2016; and Parzefall et al., Audiol. Neurootol. 19(3):203-209, 2014.

Methods of detecting mutations in a gene are well-known in the art. Non-limiting examples of such techniques include: real-time polymerase chain reaction (RT-PCR), PCR, sequencing, Southern blotting, and Northern blotting.

An exemplary human wildtype NDP protein is or includes the sequence of SEQ ID NO: 20, SEQ ID NO: 21, SEQ ID NO: 25, SEQ ID NO: 26, or SEQ ID NO: 27. Non-limiting examples of nucleic acid encoding a wildtype NDP protein are or include SEQ ID NO: 21, SEQ ID NO: 22 or SEQ ID NO: 24. As can be appreciated in the art, at least some or all of the codons in SEQ ID NO: 21, SEQ ID NO: 22 or SEQ ID NO: 24 can be codon-optimized to allow for optimal expression in a non-human mammal.
**Human Mature NDP Protein (SEQ ID NO: 20)** (NCBI Accession No. NP_000257.1) 
MKTDSSFIMDSDPRRCMRHHYVDSISHPLYKCSSKMVLLARCEGHCSQASRSEPLVSFSTVLKQPFRSSCH
CCRPQTSKLKALRLRCSGGMRLTATYRYILSCHCEECNS
**Human Mature NDP cDNA (SEQ ID NO: 21)**

atgaaaacggacagctcattcataatggactcggaccctcgacgctgcatgaggcaccactatgtggattc
tatcagtcacccattgtacaagtgtagctcaaagatggtgctcctggccaggtgcgagggcactgcagcc
aggcgtcacgctccgagcctttggtgtcgttcagcactgtcctcaagcaacccttccgttcctcctgtcac
tgctgccggccccagacttccaagctgaaggcactgcggctgcgatgctcaggggcatgcgactcactgc
cacctaccggtacatcctctcctgtcactgcgaggaatgcaattcc

A non-limiting example of a human wildtype NDP genomic DNA sequence is SEQ ID NO: 22. The exons in SEQ ID NO: 22 are: nucleotide positions 1-87 (exon 1); nucleotide positions 88-468 (exon 2) and nucleotide positions 469-1719 (exon 3).
**Human NDP Gene Sequence (SEQ ID NO: 22)** (NCBI Reference Sequence: NG_009832.1) 
1 aagatgctcc gtggaaggga gccgagcggt gggcagaggc tgagtccccg ataacgagcg

61 cctcacattt ccgtggcatt cccatttgct agtgcgctgc tgcggccgca cgcctgattg

121 atatatgact gcaatggcac ttttccattt gacattctct ctctctctct ccctctctct
 
181 ctctccctct ctctctccct ctctctctct ccctgtgtcg cttaaacaac agtcctaact
241 tttgtgtgtt gcaaatataa aaggcaagcc atgtgacaga gggacagaag aacaaaagca
301 tttggaagta acaggacctc tttctagctc tcagaaaagt ctgagaagaa aggagccctg
361 cgttccccta aggtaagcag gaaaacaagc gctgccggct tgcctaggaa atcgcttctt
421 tccaaaccga gcatttcatc ctcctctcag acaaagagac aacctttaaa taccacttaa
481 atttgcatta ctttaatttg cattaatctg tagcatgcta gcattgtgtt tcttaaatga
541 acgggtcttt gaagtctgtg acagagcagg gagcctccta gaaaagcttg aaaatatctt
601 ttggtttgac ttaaagagat ttttcttttc aaactctctg cgtagttaga tgtaactttg
661 tgatggttct tatagttgac cccgtgaaag ggggctgcga agcagcaact tgccaacatg
721 gattcatgga gcccagtatc ttccccagca ccctcatccc acttacaatg ggggcactat
781 taacgcaggc aagactgcga gtttaaaagc gcactgttta cttttattac tacttatgta
841 tgctgctgat ctcctgccag ggctctgccg gagtgctcag aatgacttca aacaataggc
901 aacatttatt tctagctttc tgtgtcacgc tttgcaggaa tcctgctccc ttgcactctt
961 ctttcagcgt gcaggtcttc gcttccatag atgcattggg gagcaccggg aggagatgag
1021 gatggattta ctgtatgatt ttgattgggc aaaaagtccg aggaaatgca tgctgtttgc
1081 cttggactta ttgcaggggg ttatttttag tcatcagctc gtccctgcag catgatgttc
1141 aaggagtgac cgggaaagga tgctcccacc agcacagaga ccaggacctg taggaatgac
1201 tcctacctgc gggatattcc gaggtttctt tcaaactttg gaatacaagt tctctcttta
1261 aatgaacaat tgagctgtag gaggcctggg cataactctt tcttggggga cgggaagttt
1321 gattgtgtac agaatgttaa tgactcagaa cgcccagtaa ctgaggctca ttgtaataga
1381 caaacccaga taagcctcca tgagaggcag actcccatct ttactgagat tcaggtgcgg
1441 gaaaggaagc tatttggcaa gatgggagtc ccttgtacag ttctttgtaa ctcattaata
1501 acagatatgg ctgctgtgaa acatgtctct tttcatttct aatattagtg tattttaatt
1561 acaaggagct ggcattggca aattacccca acacataaga ttaaatacca ggtgcatttg
1621 aaaacagaga gatgactgaa atggggagtt gctgtgggtg tcaaccaaat acagcgggga
1681 cggcacaatg cttctcaact gagcgtttaa accagatgca aatatctgct catttcttc
1741 tcatcagtct tactaattca aggaaacttt atggtcctgt ttgccagttt tcattgcaaa
1801 ttggtacaca gtttatgcaa aacttttctg attttctctg ataaccagcc caataagcat
1861 cgctgaatga aaatctgata tttgaatagg ttaatttaca actttggggt attcattagt
1921 caagaaagcc ataaaagaca ctatggattc cattgctgta aaattttatt gtaatttttt
1981 tttaaaaaaa ctgcggttag caaagcattt agctgaatca aaacaatttt cttagactat
2041 tattcagatc attacatgag agtatttgaa cacaaaatgc aatgttttgc atcatttatt
2101 ttaaaataca tttggttcag gtaacgggag aactggctga ggccttctta cgactgtgta
2161 ccaagcagct gacacccaca gatggaaatg tgcctgtgga gttttcaatc ttaattcgag
2221 ttccaatgac actgtcaagg gaaaaataaa tcatttttca atacatttaa caatttccaa
2281 ctcatttatt ttcctgggcc cttgaaaaat gtggtgagtt tcttggaatt atagccacca
2341 atggtggcca gcaatcttta ttttaagaac tcacaattca agtactatta ttctctggtt
2401 aattatttat ttcatcaatt tttaaataaa tatttattaa gcattttcta gatgcccagt
 
2461 gctatgctag tcccgatgga tgcaaaggtg aatgagacat tgtcccatac tctaattctc
2521 agtccatatg ccatctctag ttagattttg gaactgaaat gggcattgcc aatgggtaaa
2581 gtgggcaata actttcctgt tataaagaat tatttaaaac agctactggt ttctagattt
2641 acttgaaatg attctcactc atcagtgggt tttctttcca tttatgatta agggattccc
2701 ctttggcctt tttcaccaga tcctgggtac acgtcatttg cacagctaac acggctagat
2761 ctttccagcc tgggtagaag aatcccggaa aaagacagta gtttatcctt tctctgctct
2821 cctgagcacc ctgtggaaag gaccatatgc tgtctctatt ggggatgggt gtgagtcgcc
2881 tactcagtag ctaacatcag ggccacttga tgaatcatcg aattagaaac atgcatatgg
2941 aggaagatgc agtcactatc cctcaccact tacttgtccc acccccagta tcaaaacaaa
3001 tgacctctgg gcctggccta aattcaagag gcccctggga agaagctgtt ggaaactctt
3061 ccccaaaccc aggtctccat catagccaca cagtgttgct ttcaaataac ccagtatggt
3121 gttttatcac gtgtttaggt tactggggga aaaaaaaaac cctgaaaaca gcatcaccag
3181 ggtccttgac ttggtcctta tcagcagata atcccccgtg gttggattaa aatgcctctg
3241 actccctggg gtgcctgact caccctagcc agccctcagc ttcttggcca gcggccttcg
3301 agcccatcct cacctcttcc catgacggcc agtgactgct tctttgccca ggccctctgc
3361 actaggccag cagccagtgg gagcagaggg gatgggaagg caggtggaca gactgtgaag
3421 tgctaaggca gagcggggaa gcagggctgt gagacagggt gggtgagctc cagggcctgg
3481 gttgctgtgg cctgccctgg ctgtgatcag ctgtcatttc ctctttgtgt atgacactgt
3541 ggaacatatt aaaatttctc tgggatggta tgtttggaat tcctctgatg ccccacctca
3601 ccaacttacc cggacaataa aagcaaggtt gtaagccttt gaacagctgg ctggctatat
3661 atttcccttc tttctttcgc ataatctaat tcagaaaaga atccagctag actggcagtg
3721 ccatgtctgg gtacttgttt cctgcaagtc cttcctcctc cacctcctcc cgccagtcct
3781 ggaatcttcc tgggtggcat gtggcttttc ttttcttttg ctcaccactg ctcccagccc
3841 gccccctttt cccttcgcct gagctccttc ctttgtcagc tggctctttg ctctgcctgt
3901 tttccagaca gcttgggtcc tgttccatcc ggggagaaca gcccatggga tctccccatc
3961 tccatagtat tcccaggatg tcattcttgg caccagctct gccgggctga taggactggg
4021 gggctttttg tagtcccgag cctgaatcca ctcacagaga ggagcagcca catggcttag
4081 ctgccctttg ggctgccgag agcggggctg gagtgaccgg gttggcagga acgagcatac
4141 catcacagtg acccgaacat ctccggcaaa ggccttgggg tgggagcgct atgggaatgc
4201 ctggatgcac ttcggcctac attaagagaa atgtggttat tctctgtttc gggggagaga
4261 caagcggctt gaaacaaagc cctctggatc cttggcccct tccctgcccc ctttcccggt
4321 ctgtccttcc cacatccctc cctccccaaa cagaaagaaa gagagacagg acttctaatt
4381 tggccagcac agacccgaat gtctttatga aacccattta gtgaaagaaa gaggggagaa
4441 atatattctg ggttgatccg aactaaaaaa tgcatggggc tttggagatg gggcgggatt
4501 ttttctttct ttctttctgt ctcttaagag cagaacaaca gtttcgttga tgagttgcaa
4561 atggaaggaa gtggacctct caattagaga ttcccattta agagcctcaa tgtaaacctc
4621 cctgtgacat ccttaaaaga tgctgctaat gtccctggga ccaaaaggta ttaaacagca
4681 agccaccagg cggcttccta ttccttttct tttctggggg gctttggtcg tctttttcaa
 
4741 attagctaaa attcattaaa ggacccaaaa ggaaaaattt aaagcagctg aattaaaatg
4801 agtctttccc tgttagcctg acaagaagaa aaaaagaaag gaagaaaggg aaagaaagag
4861 aagaaagaac caattcccct ccactaccac ttcttgggtt ttaatatgaa ttattccata
4921 caagtcccta ggctagagga tctatttaaa gacgcctggc agcatgaagg gagagggtaa
4981 aaatacaacc actcttttgt ccatcaaagg ttttctttgg gaggacatta agagcattcc
5041 tgttttcttg tctgcccctg ccagtggcaa tgttccagct ggtagtatcc actcctcgcc
5101 catcaggtca gcaatcatga acttctgtgt ttaatttcag gagaaaacaa agggtgggaa
5161 atccttccga aacccctatg tgtgtgcagc agggcagggg aggctggaac tggaatcagt
5221 actctcttgg ctgtgcaggt tgcatgctgc aaattcacac acccacacag ttgagttgtt
5281 gaggaagatt gtgccgatgg cattatttct gatgccagct tcccttgcat ctgcaaagct
5341 tttttcttgt gtctctggca gtcatttgct tgtcaccatt catgtgcact gtccccttca
5401 tcagaggttc tcagagcctg atggtcaagg atatgtcact agagtcttac ccccagaccc
5461 attttcaaca gtaagaacgt gaaaccctgg atggggtttg caatctgaag caactacttt
5521 cttgttagga agagtgctga ctcagaaaat tcaaagaatg ggtaacttcc gttggcgaca
5581 tatccccagt gctcataatc aggaattcat ccagttgggg tccatttcac cctcatggca
5641 aaatctggac ataaacattg actagtagct ttaaactggg tataagattt gcaacagata
5701 acatttttgt cttttctttt ttcttttttt tttccacaaa agctaagaat tttcagaca
5761 atttgctctc ttccaaggcc tggatcaatc ccaacttttt cttttaagcc atgatccaac
5821 caattgcagt atcctttaaa aatgaaaggg caacaggcta agacattcaa gaaagttgat
5881 aagctgtcag aaaatgctca agggtggcct ttctacagtg ccacaaacca acaacagccc
5941 taaacgtgga gtcctcttcc atcctttcat cagtagggaa taaaaatagg gagagagcag
6001 gtcaggggag aagtaacaaa agagaagtaa gaaggattat ttttgatgtt caaaatctgc
6061 tgctgccctt tcaatatttt caaatatcac aatttcagaa agggaaggat ccctttattt
6121 cacccactct ccctcccagg aaggtgtttg aagttgctgt ctttacctga aaaggaggtc
6181 aactcctcaa gtgaaaaaac atcccctatc ttcaactcga agtaaaatgg aggtacacac
6241 atttgtacct aattatgtgg gtacaaatat aatttgtatc cacaaactat aaaggatgta
6301 aagaatcaaa gaaatccaat ccttaatcaa agtccaattt ttctttaaaa cttgagagga
6361 tattttaggg ctcattgcat tgctgtgatt tcactgcccc ccaccaaatt atgaaaaatt
6421 actccttttc agaatttaaa tttttctttt gagatgtgga atccaaaggc agcagaatag
6481 tattgttaca gggaagctgg cagtcaaaag acatagacat agctgcagca tgcatggttt
6541 aagcaaatca agacaagcag aaaaccacca ccccaacaag ttcagggtta tcagcaatat
6601 agggacacgt atttttatgt gatttttatg agaaccttgc ccctttgaca tatgtctgag
6661 tctaggagca ttccggattg caacgggatt gaattcagta ttcaaatccc accattgggc
6721 agaaaaggca aattgaatgg ggaatggaaa gactgagttg ctcctattgt ttatgcccca
6781 ttcctaacta agggctctac agctgttagg tgagatcact acccaccatt ccaaagtgtt
6841 gttgcgggtg aggaggttca aacgcagctt tggaaacact ttgctgcact ctttgaagaa
6901 aggccaacat gctcatttgg ctctagggta aaagagagcc tgtaccctga gctaacattg
6961 gtcacctctc ctcctgaggc cataccacag ccagcagtac agcagcagaa caagtgttta
 
7021 gttgtttcag cacaggtatc cccagaacac ctggcacctt ttcttttcca cagggagaat
7081 gtacattcat gcattttggc aagactgagt cctctgagtt gggtgtctcg gaactctcct
7141 attttccttc taaagggtct tatgtaaggt gagcagtaga gtgtgtctca ttcctcataa
7201 aaaatcccct tcctgggagg gagtgggtga aatgaaggga tccttccctg actgaaattg
7261 tgctatttga aaaaattgaa agggcagtgg cagattttga acttcaaaat gaatccttcc
7321 tcctcctcct cctcttttgt tacttctccc atgacctgct ctctccctac tttaattccc
7381 cattgaagaa aggatagaag aggactctgt gtttagggct actgttgatt tgtggcacta
7441 tagaaaggcc acccctaagc attttctgac agctcatccc catctcagtc acagctttta
7501 tttgggtagc tggatttaca gaaataatca aatccctaga ggaaaagact tcaacagaaa
7561 agtacctcct gacacttggg taacctgact tggtcatttc caatttatgc aagatttgta
7621 ttctaaaagt caattagtta tgtcaactgt taaaaactca gaatgagtct ttccattctt
7681 cagaatgact cttttctgat tatctgtggg ccatagaagc ccacagatac tcagcacagg
7741 atccaagtgc cataccaagt ggtcaactct tttagacgta agtctcagcc cagggcttct
7801 ggaatgtacc tcccctagca gagaagctct gtagtagata ctttaccaag cacagggcct
7861 cgctcttgac cccaagacta ctgattctac ttgcagagaa gacaactttt ctaatcaatc
7921 tttctcttct atcctgaacc agagtatcag agattgtccc attagggcac gaggatctgt
7981 ggctcaagct cagttaagaa ataagcgtct ttcaaaagaa aggtgcaaga gggtggaact
8041 cagcctatat tgtcattatc aaatgcaaga agtctaatcc tagcatgatt tattaatatt
8101 agcctttctt ctctccccgt ttatgctttg gtgggtactg gacagaaacc ccacaaattt
8161 taagacagtt ttaagagaaa tagtaactgg ttaaatatcc tcactacttc aaattattat
8221 tattattatt gatattactg ttaacttttg tttttttgag acagggtctc actctgatgc
8281 ccaggctgga gtgcagtgat gcgatcacag cccactgtag gctcaaccac ccatgctcag
8341 gtgatcctcc cacctcaacc tcccaagtag ctgggatgac aagcgtgtac caccatgcct
8401 ggctagtatt tttttattat ttttgtagag atgaagtctc cctgtgttac ccaggatgat
8461 cttgaactcc tgggcttaag tgatcctccc aacactgcct tctaaagtgc tgggtttaca
8521 ggcatgagcc gctgcacttg gcccacttcc tatgattaac aggatattat taatttagaa
8581 actatttagt acatggcaag cactcagtca aatggttatt aacggctgtt tttatttcag
8641 gccaccaata tagcttcatc aaggggcaaa tacttatagc ttgggcttca gtgttggatc
8701 agacaccacc ttagaagttt tttgcaatca ttaactcctt aagtaagaag ggaactcttt
8761 ttgtaggatt gctttgggga atatgtagcc ccaaggcagg ggctgtactc tgagaagctt
8821 gggtagagtt ggagactgtt agaaatggat agacacataa aagcatttag accaatctgt
8881 tcattttgca gatgaggaaa ttgagtccta gaaaaggcaa gtgacttgtt caagatcaca
8941 cagccaatga tgggagggta ggactcaggg cttgaggatc agtttcagaa tgcagtgaaa
9001 ttgttatccc atatttcttt caaatcagta tagggacacg gaacactaac aacataggcg
9061 accaatgggt ctagggagga aaagatgaaa gctgtaggta agtgggagtt tggataacaa
9121 tgagatgttt ttctggagtt cctgacaatg cctgaaaaaa aaagtcagga ttttaagctt
9181 cagtcaggct caaggtcgag tcctcccact ccagtccctc ctcagacagt aaaacttttc
9241 aagccaagga catacatata ggtgggggca tttgacagtt ggctcatctc cccaccctcc
 
9301 aaattattta tatttctagt ccttcagttt agcactccta tttaccccag gattgtaatt
9361 cattgcctct ttccctggcc atattcctcc atcctttttg tttctcccat ctcttttctt
9421 cctccatcac ccccactgct gagcttggct acacttggct agtagtgcag gttgggagaa
9481 cacagagtcc tatcacagac tggaggacta ttatttaatc acaagaatgt tgtctttttt
9541 ctcctaaggt agcactctgg ggacggtcat tgctgtcttt attccctcac ccccccacca
9601 cccctgcact ggattagaca catgaaatgt gcctatctct gagatgaaag gagcaagaac
9661 tgcttcctcc ccactgacct tttgcctgcc catccttgga gatacctgga gttggcaggc
9721 tggaggccta gcagcatttg cagtcagtgc cgctgcctct gggtcagccc aggcttggct
9781 ccttggctgc tactgatgtg ctgccagcta ggaatattaa aaaccattca ttgaacgcac
9841 aggatgcttt ataaaatcca acatgctgtt tacagtttat aaattaactg ccttgggaag
9901 aggcatttga cttattctcc aaggcatcta gttgactaga tccaattgga atcctttgag
9961 ttcctgggaa ggggctactt aacacttcac actttctaaa ttagacctag agtttcttgc
10021 ttttctgatc ctagtactgt atgaagtttt atctagggtt atctagcaag ctttcttcca
10081 cttataaatc tttgatgttt ttgcaatttc tttgatagag gctttatgtt ctactatctt
10141 cactttattt atgcttcata ccataatttt ttaaaaaatt acaataaata tcaagtaatt
10201 ctggctgata tactgactcc attccaaggt cattatattt tatttgctta acttggtctt
10261 cttagaaatg atgcaatgaa tactggatac aaattagatg ttcagagagt ttatcaatgt
10321 gtctaaaaac catccaggaa aacaacaggc caagactctg cctgcagagt cagtgagcta
10381 gaagagggat cgaggatagg aactatattt ttcccctgac cctatctctt atctgctaga
10441 gcaaaaggag acacacagta ggtgctcaat aaatacatgt taaaaaatat ttgttgaaag
10501 aagccacaga agggaatatc agtatacatt agtattgaaa acagcctagg gggaaatatg
10561 gatactgaag caccaccctc ctgtgcctgg caagcaaggc aagaataagg ccattcttgg
10621 cacataccac ttagcctgtt gctgagataa cttgcgtgaa accagcactt taccctctgc
10681 cagccagaag gtcagcccaa gagtgagctg atctgtgggc acaacaccat ctctcaccca
10741 ggaggctggg cccgctgtaa gctctgctat attatttccc tgaggatgac aggtggggaa
10801 atctgggcac cagttgttct tttccactgg tatcatttgg aaggtagctc caaagtcggg
10861 cacgccagcc tacgtgcctc tcctgccggc agaggctcca cattggagct attagaatgt
10921 gggctctgta cgtcggccaa gtccatgtct cagagactgt atccagagca tagcgtggcc
10981 acagggctac tgagttcagc aagagagtca ttctcttgtt caatccatta ttgcctgggc
11041 caaggttcta agtcttcgta ggcagcattt gattttatga cacgtcttaa aaaattaagg
11101 caaactggaa ttaatttagt tttgagaaaa caacagaagc tgctacaagg cagagataat
11161 tctaggctgc cagaaagtgg tgatctcagt ggtgtacatt ttcttaattc ctgattttcc
11221 tctttgtgag gtcttcctcc cactcccata ctttgcacat tctcacctat gcacaccctg
11281 agcaacacaa tgttgacaac ttgacttagc attgctcttt tcttaagctt tgttttcatt
11341 tcttcttcaa tacaacagtg ttggttcatt caatttgaac actcaaggca aaaaaaaaaa
11401 aaaaaatccc aggttatgtt ttcagaatct tacctttttg tttttgtttt gagggttctg
11461 tgagttttgt ggcatattta cattgttgct tacattctta tttcagaatt taagataaaa
11521 actgggcaac aactctcacg ttttctgagt gtgttgaaat gaaaaatctg tttcttagaa
 
11581 ttaatggtaa agcaacattt cctgtcaatc tttcattctt taatattact tctgggttca
11641 gctcaccatc tctaaagaga aagccagatg atcaagttac attgtatgtg acattaacta
11701 tctctttatc tgtgtaaatg tcataatatc tgcttgcaaa gtgttttatt catttttagg
11761 aaaaaatttt aaactgtgct tgcaattcag gatggtgcta ctaggtggca gtaatgtaca
11821 agaactatgt ggtcgccctg gaagagtatt tgactaagag actagcatgc cttcccgtaa
11881 ttataagaaa taaaaatata atatttttaa aatactcaaa ctggcacaaa cagtaaacat
11941 ccattaactt agttaccata tttaagctga gcttttaaaa gagaaagcct tattttttaa
12001 tgaaatttta aataaagtat acattcacaa gctcaaaaat ctaaaaatac gaaaagatat
12061 atagtgaaaa gtctttctct catgcctgtc cctcaactac cacaggtaac taattttgtt
12121 agtttcttgg gtatcctttc agagctttgc tatacaaata tgtgttttta tctctctcca
12181 ttttaactca aatcaataca tagagtttct gcatccttcc atatatagtt ggttagtata
12241 ttgttgtgtg aacattttag aatataatta accagtcccc tattaatgga tatttcatgc
12301 tgtttccaat tttatgctat ttcagccatg tgctcatgtg atttcacatg taggataatt
12361 cacagaagtg gaattgctgg gtctaaatta tatacatttg catttttaaa aggtattgcc
12421 agattggcct ctacagagtt tggaccaatt tatactgaac tttctccaaa atggacttgt
12481 tttcccacag ccttgccaat agggtgcatg atcagacttc ggaatctttg taaatctgat
12541 tggttgaaca atgtatttca aagtacttta attttcattc tcttaaatga gacttttatt
12601 ctcttaaatt ctcttctatc tttaagggcc ctttatattt cctttttctg tggagaaagt
12661 ggaaatgcca ataaatacaa cgatgcatac acatgtatag ggttttgtta ttgatgtagc
12721 ttgtttttgc catcatgctg tacatgatgt tctttaattt gctttgctca tttaaccata
12781 cctcctggcc atggctctga agagaagttg ctggcagtgc agaagaaggg ccaggactgg
12841 aatgtctgct tctctcttca tccttttgcc attgactcct gggggaaata tctgtagacc
12901 gaagcagtga aatctaatca attctctttc tgacactaat taaggcttca aagagacaga
12961 taaccacttg ggataattta gtcacagtga aacttgggaa atggtcagta atatttcatc
13021 ctctgaaagt ggaccccaag aaagttctgg ctctttgagt tgttgtcttg gtaattttca
13081 gggtaacgtg tataggaaaa cccagattat gcagatgaaa agaaaacaaa catgctcttc
13141 tgcatccttt gcagatttga aactttgggc acaggtttga tttatttcaa tgagatgttg
13201 ctattagcat gaacataaag tggtgcaaag ttgtgctgga atatggtctc gtcaactttg
13261 ggctcactgt ctgcatatgg agatgatagt aactgaatcc atagttatca agtggtggca
13321 ttttagaaca caaagggaca gcagatgcca tctctgtcaa ctccttccct ctacagatga
13381 ggaaacttgg gctagaggag gttgaatgac ttgtgtaagg cttccccagc taattaatgg
13441 cagtgccagg actcaagtcc aggtgggttc cctcattact ggtccttgcc cttcctgcaa
13501 tttataggac ttccctgagg cacatgtgct gaggctttgc tattgccagg gattctgtgc
13561 tctgaaacag ccctatctca cacacccctt tccacttagg atcccctacc tcacacttcc
13621 aagacagcta attgaggcta aatgggtctg atgccattct attagtgcaa aataaattgg
13681 cttatgccaa cttgaactcc taatcttgat tttatcaaca ctttgccctg tttattcaga
13741 ctgttcatga gtaggaagtt aaggaaaatg ttgcctatga acaaaatgat caaactttta
13801 tctgaaatac ctttgaaagt tttcccgaag ctttaattat catgttcctt ttgttttgga
 
13861 tgtaatagca ggccctccca attttcctaa atgtttgaaa atatttttga tactttctgt
13921 cttctgttgt ggttaaaaaa aagaaggcac cagtgttggc tatgcctggg gagttagtga
13981 ggtaatctgg agagatctct ggaccccagt cctttgtcca agaggtatgg tcaaaagaac
14041 aaagcaaatt tcagaaaaac cggtataata tgatcctatt ttaatggtaa gtctgttcca
14101 acaactgctt tctttttcga attacaccat gtgcccaggc tacaaaggct ctgcagaaat
14161 gtgactctgt tggcaaatac gcgtgtgata ccttaaccat gctggaggcc agttagcata
14221 atgtgtattt cagatgcagt ttgtcatcag tgctgggtgc tatggggctt aaaacaaaaa
14281 caggatgagc ctagagcaca gaagtggaaa attaggtggg tgggtgagta tggacttgga
14341 aatggaaaat gtgcttccct gtgggacatg gcttgtgttc attagtgctg acctgcctcc
14401 tctctagccc tgtaagctga cgctgacacc aaagcttgct gagaaactga atagaccctc
14461 ccgccccgac caaccaagga caaagatgag accacccaat tcggttacgt tgttgccaga
14521 acaacatgtt taatctttaa catgggttca aactattctt ggccctagga acatggactt
14581 cagcaattaa agtcaacatg tgcttccatt aaaccattgt gtccacctcc aaatggttat
14641 aaatatgtag cataagctat gggagttggg gtggaatgga tgacagcctt tgctaatgac
14701 gctctagaaa ccaatattct cctctcaaaa taacatggaa aaattctact taatacctcc
14761 tgggttccat tagtggttct gggtaaataa ttctggggaa agtaatttct gttttcattc
14821 cagctgtgca gcagatactg tgatgatgga ttgcaagtgc aaagagtaag acaaaactcc
14881 agcacataaa ggacaatgac aaccagaaag cttcagcccg atcctgccct ttccttgaac
14941 gggactggat cctaggaggt gaagccattt ccaatttttt gtcctctgcc tccctctgct
15001 gttcttctag agaagttttt ccttacaaca atgagaaaac atgtactagc tgcatccttt
15061 tctatgctct ccctgctggt gataatggga gatacagaca gtaaaacgga cagctcattc
15121 ataatggact cggaccctcg acgctgcatg aggcaccact atgtggattc tatcagtcac
15181 ccattgtaca agtgtagctc aaaggtaaga ccaaggtctc tgtgaggaga gcatttccct
15241 cagaaacagg caagaagcca tatttctttg tcaggggatg gagaactaag atcatagccc
15301 aaacttaagt gatgtggctg gcccacaggg cttgtgggag aatgaggctt ttagaatttc
15361 cccccgtgct ttgcactgtt atcagtaaaa gtgcctaatc aatcatgtgc acacccatcc
15421 agtgggacag gtgtcctttg tccctggctg tgatgtttat gagtggtagt gtgcttttcc
15481 aaaaaagaaa aatggagttt acaagtcatc ataactctct agctccatca tcaataaaga
15541 ggaagagaat gcaaagaatg cgattgagca gtggaacata cttcttggag ggctctttaa
15601 agagattctt ggtgaaataa ctggctttgt ggtctgagcc tttcttcagg tgctacattt
15661 ctgttcaaat gatttcaaaa gataagatgg caatatgatc atttatcatt tttacattgc
15721 ttatccaaat cgttctcatt tggaaaagga tgcatgtttc ttgcagaggc agtccgtgcc
15781 tcagtggtgt gcaagggaat atcatgctta aaaggccacc aagagtggcc agctacatgg
15841 accacccaga agggaggcac attgtagggt ggaacatacg agatctcacg actgtcactg
15901 cccctaaaca ggcacaatga atgaaatttg tccacacttg gtaaataata atttgtttag
15961 cccaacctca gagattttta catttagttt tgagatgaaa atcacttaaa cattctcgta
16021 tggcaagtaa tttttgaaat taccagtata attaagaatt ttttatagta tcaatcacca
16081 gaattaagac catataagcc aatccctcca ctctccattc aaaaacatta tgacttttgt
 
16141 atgccttgaa aatttccatt aatttctctg attatagctc agtgatgatg attcatttta
16201 gtttaggagg atcagaattc gagttaaatc attttaacca ttatattaat aagatctatg
16261 actgtgggaa aaaatagaga ttttccactt aataaaaact ttctggctct tatttgtgct
16321 tgtagaacca aaaaaaaatg gagtaaatag atgaacataa atttttgcag ggccacaatt
16381 ccttcttagg aaagggaaac taaaacaaat tgagaaaaaa catgagaagt gcttttacaa
16441 gtgtgattgc tcagaaattg tgggcccatg taaggttgac tgcagcaatt attagtttct
16501 ccttccctcc cccttcagaa acagacagca tgaatattta gtgattttat tccatggata
16561 atttctttcc gtattgagtt gtcctccata atcgtgattc ttttttaaaa aaaggaatta
16621 aaattatttc aaagaaagtg tcactatgat gatagaaagg aacaaagaaa taggagcatc
16681 tataaaaact agaaaacaat ctggaaagac tggacaaaat gatggaggag tactttggca
16741 acaatcacat taatctagcg aaggtcaaag aagagacatc atgaactcaa accgaagaaa
16801 ttcacatgtg aattctgagc atgagtctta gaaacagact ttagtgtact gttattcacc
16861 tttgtttctt ccagaaagcc atggcaaata acagcgcaaa attgtgatag caggagaaat
16921 gtttgtaatt tcatgggaac aatctgtttt caaacatccc aaactatccc aaatatagca
16981 atagttttat gtaaatggtg cttgcaatta taaacctttc ttccctggcc ataaaatatg
17041 cctggcaaaa tctcaactac ccagaccttt tctaggttag atttttctct atgtatccag
17101 gagcaaaact gttttcattt ggaaatgttc tataattctt aactcttaca ttctcgggct
17161 gatcttttct ctaatgggtc tcacttaatg aaattgtttg cctttagaga cttggataaa
17221 acatttagcc tatgtctaga aaagaggcaa caacaatcta ccctagcttc tttttacaaa
17281 gaaaagactg ggatagattt ttttgtctct ataaaaatta tcccttgaca tcatgttcaa
17341 gggtcattat ttattaaaca tgtgcctact gtttgcctgc caattgttaa tgctttctga
17401 ctcacattta tccattatct ggccttgtaa aggcaagaaa gggagaatgt gacccaggct
17461 cagggaaggg agtgtacctg cttgaacacc taatatatgt gtcaggcacc tcatattgta
17521 tattataatc attgccaaga aacctgtgat ggcaggcatt attattccca ttatcctggt
17581 aaggaaaact gaagcccaaa ataattagat tgcttgatat catatagcca gggagtggca
17641 atgtctgcct tacagcattc atcttcccac tacttggatc ttcatattgg aggaacacaa
17701 gtagaatgta ggagccatca catagaagtt caggctctgc ttctgatctg ctggcagtaa
17761 taatttatac tcagcaatgt ttctagaaaa aataaataat agtgaaagcc tttctgggag
17821 ctttcaatga gactgttcat cccagccctg aacttgcagc tggccagtcc taggtccctg
17881 ctgcccatct cctagcaatt ccggaaagga ctgagaggga gtcaggcaga aaagactggc
17941 cacagtggcc caccaaagag gacagtgtgg ccacagatta ggatgtgcct gatagtttta
18001 agtggtggca cctcctgggt ttgggtacaa aagatcctgg gagtagcagg ttcatttata
18061 atcttcatgg aagactaagt ttctctaaaa tttggagtaa aggctgtaga gcaaacaggc
18121 aaaagaccag ttgatagtgg aaacagactg cctggtggaa tctctatgag aactgaggca
18181 tgccaggctt tgaagtgaga gagacctggt ttccaattcc agctccatgg cttgccgaat
18241 gagtgacttt gaggaagttc agtaacttcc ttctttgtgc ctcagtttct tcccatgcaa
18301 aatagagctg tctacctgag ggtatgggta tgggtgttgg tgatttgtaa agcatccagc
18361 ggtgtccagc acataatagc catttaatag aagatattat gatcttgatg ataaagttat
 
18421 aaactaagca ggtgtttaga ctcaattttc tatttcagtc acaactgaga tctcaataac
18481 tgctgggatc tcctatagcc ttaaaagatt aggaaaatga accttattgt agttcttgtt
18541 aagcacaggg tggggcagaa gagcaaagta aatgtaagcc actgtgctgg gtgctaacag
18601 ggaggataaa agtaaaagag aaacagcccc gatatctgtc taaccttcct tcccagatga
18661 gtggaggaag gaggggctgg agggggatga gttttggact tgggccaggg aggggatttc
18721 aggaatggag ggtgggaaag caaactggca gggaagacct ggaaagtctc agttgggtga
18781 ggagtggggg atgctggccc caggccaggg catggaacca gggcatgact atctgaaccg
18841 gtaagggcca caggagcagg tggggaactg gaagagcaac aacaaaggat aaggaggaag
18901 aggtttggga gacaaaacct aggaaaagag gacctggtat gctaagggct gagtcctaac
18961 agcttcctaa caggaccccc aagccatctt ctactgctac ccaccaagct catggtgtga
19021 taggagaaat ggagggagtc aggaaaattg tgtttataat acaatagact tcttgagaga
19081 tataatttga tttttttaaa aggaacaaat ttatactgag tgcctaccat gtccaggtgc
19141 tgtggaaggt gctggagact ctaaagtgga aagaaagaaa aaagagacaa tttttttgcc
19201 ttcatgatgc ttacaagaaa atggggagag agggaggagg ggtgttctgt gcacaaagat
19261 cagtttgtat ctgtttgatt catgaccgga gtgaagggac cagtttctag ttggagtgag
19321 ctgagttccc atccaccctt tcaaagcccc caccatctct ctgctgcttt gttccctggt
19381 tgcaattttc ttggtgccct cagcctagca cagatctttt ggaggttgac ctgggtccct
19441 gaagatgagc ggagttatga ggctgcccgt ggggaaagaa tgggagaaga agcatgggtg
19501 aatcttgttc ctgaggttat agagggtgtt tcaagaggtg ccatggaaat aagggtgcat
19561 agtgttcact gctgcgaaaa aggcaaaata cttttccact ctgtcggagt aaaatcgcaa
19621 aaatgctctt ctagggtggg acaggtgggt ctttattctg atgctaatgg tgacatctgg
19681 tcagtcaagg ctcttggaag attcagctct gcataagctt ggggatggtg aagctaatgt
19741 cccaagaagc ccacaagaaa aagttagact tgtatgtcta tggatatggt caggggtttc
19801 tgaggcaaat caatgtagat aaggaagtct gtgagttggc atatgcttga ggaaaacatg
19861 gtatcaggag gcaggtatta tactggttta ggtcagggct ttggtttcag atagaccttg
19921 gtttgattcc agctctgcat tttaccacct atgtgagttt gggcaagtta ctggaactct
19981 ctaaagcttg agtttcctta tctgtcctaa tagtgtagat cataacagta ctttcttgta
20041 gggttgttgt aaggatgaaa taactcacat tgcaagtgct caataaatgt tcttattagc
20101 gaaatgatgg ttatctagtg attcatctag agcacagccc tcaaacttga atgggtccag
20161 agacctgcta ctcaaagtgt gggcaccagc ccagtaggac tgacatcaac tggaatctga
20221 tgggaaatgc agaatctcag gctctaggcc agaactactc aatcagaact tgccttttaa
20281 caagatccta gatgattcac acgcacattc cagtctgaga agctctgatc cggaagagat
20341 caggagatgt tcaagtgctg aaacacaagt aaggcatggc agaagctaaa atgtgaagta
20401 ttgagagtaa accaggagag cagatggtta ttccttccat cccttaagga tcttatcttt
20461 caaacttctg ctttaccact tcaacacaca aaacagagat agatttcctt ctaggagtgt
20521 gtgtgtgtgt gtgtgtgtgt gtgtgtgttg agaacatcaa tggttcaata attgcttcat
20581 agtcttttct tccaggaaag agcatttgaa tttggctaca gagaatagat aggtgggatt
20641 ttattaggca gagcaaggtg gaaaggacat atttgggaga cagaagacat aagcaaaggc
 
20701 aagaggcagg agaattcagg gcactttctt ggactcctaa gtagttaagt ttgactggag
20761 tagggagtgg gtagtagagt gatgagaata gttgtaagta agactggaaa tacatgtgaa
20821 acctgggtca tgaagggcct tgactcctct tcaagatttt ggattttatt ctgaactact
20881 gaaggcattt attgtacatg tctgtgcatt cttcttgcac aaacagtcca gctcccaagg
20941 aagaggggcc cagcccattg gaggtggccc cacgtccttc agtgcctgga gttcttgtcc
21001 ttcagagtgg gctgggggtg gagctggaca tcctgaaaac ttttagtgag attgcaatca
21061 gatgagctca tttacaaatg gccctacgct gttgacttgg tcattttatg gacagtgcta
21121 atggcccagt cagttaggcc tacgtttggt tcacctgtgt atacatcact tggatattat
21181 gtcccatatc attgttggcc cttctgagtt aagttcgttg gcacagattt actttgtcca
21241 cccagactga aactgcacct ctgaagactc catcacggaa ggtgactctg tgaataactg
21301 cttagtcaac ctaatagatg atctagatga gagattagca aatttaacac tactccaagg
21361 tgaggtatca agcagctctt cccacataca gggagatggg gggagaacag actaaggaag
21421 atcttggact ctctctctca ttagagaaat gggggtggga attagggact agcctgtgta
21481 ctactgacct atggcatagt ctctcaaaac ccatttcaga gccaggcaca gtggtacatg
21541 cctgtagtcc tggctacaca ggaggctgag gcaggaggat cccttgagct caggagttta
21601 aggctgtagt gtgctatgat catgcctgtg aataaccact gcactgcagc ctggacaaca
21661 tagcaagact ctgtctctaa aaacaaaaca aaacaaacaa acaatcattt ctgatagttt
21721 actttgcctg tcaaaatgaa gagcaggtag gatagaatca aaagctttaa aaaaatcaaa
21781 acagcttaca gctgctattt tctctctgcc tctgcgatca ttcagagaaa ataaattcac
21841 tggtctagtc caacttattc ttccctaagc cctgctgttt tttttttcta gtatttcata
21901 ttgccttggt ggttgctaag tgattgttgg atttagcccc agtatattct cagataacta
21961 tgccaataat ctataattac cagaattcat cttttctccc ttttggaaag atggactggc
22021 tatttttttc ttgtaaacct tgggtgttta attctgccct acaggatgat gtcagagata
22081 atagctggtg agtctttaat aataactgcg aattcctaca atattgtaag gtaaacgtca
22141 aaaggtttca tgcatatata tgtatctttt aatctttcat ttgaacagtt tcacttttta
22201 tgatcttgct gttggttctg gaattatagg acaaattttt cttttctttt cttttctttt
22261 tgagacaggg ccttgctctg tcactcatgc tggagtacag tggcttgatc atagcttcct
22321 gcagcctcta attcctgggt tcaagtgatc ctcctgcctc agctgccaaa gtagctggga
22381 ctacaggtgc acatcaccac gcctggttaa ctttttattt ttttttttag agacacggtc
22441 atgctatgtt gcccaggctg gtctcaaact cctggggtca agtgatcctc ctgcctcaga
22501 ctccaaagtg ttgggattat aggcatgaga caatacacct ggcccagggg atttttttct
22561 ccatcactta acatgaaatt caccaagcaa aacctcttac ataaccatgg gaccaataat
22621 ctcagattct ccattcccct ccccaccttg atattaggat tatgcagctt aatgagtcaa
22681 gctgtcccac cctacccacc tattactccc actcccagga taccaggagt tttgtatggg
22741 atacataaat gatccttttt tccattccgt taagctgtat gcaaattgtg tgtgtgctta
22801 tgtacatttt tctggggata actttggtag ttttcatcag attcttaaag gtgtctgttt
22861 gctaaagcca gattaagagc tactataaag aacacatagc ctccgttatt ccagcccaat
22921 ctttggaaat agagattatc agaatgggta catcctaata tttgccgcaa atgactctga
 
22981 agatgcggat gcaattacat aaatcccagg ctccttcaac atataaaccc agaagtgtga
23041 tttcagggcc ccagacatgc cctgtctcta cttcattctc ttgtcccaaa ttgcagcacc
23101 cagaatattt cttcctaagc attactcagg ctatcctatt ttcttattca gaaactatat
23161 tcctgttctt tagctatttc ctgcacaaat caatttcttt tttttttttt tttttttggt
23221 agtcatttta atttacttct tggtttcaat ttggattgac tttctgcatt ttgctactgg
23281 tagaattgca atgcattggg ccagtggtag accactacct gtcaggagaa atttcttttg
23341 ttctttggtt tttactcgca catcacagtg atgggcgtca ttcagtggtt ctaaaccctc
23401 agtacactta gaaatcacct aagaagcttt gaaaaccatg aaagcctggt ccctatctcc
23461 aaggatttta attcaatcga tctgaatgag gcctgggtgt tgacttaaaa aaaatgcaac
23521 atgcattgag ccactggtct aatctagcag gaattggcaa cgagtgtgag ggtctttcaa
23581 aagatgctgt tttacctggc taaggttgtg gcatgcccac agagtaacca cctccttttc
23641 tttccccaga tggtgctcct ggccaggtgc gaggggcact gcagccaggc gtcacgctcc
23701 gagcctttgg tgtcgttcag cactgtcctc aagcaaccct tccgttcctc ctgtcactgc
23761 tgccggcccc agacttccaa gctgaaggca ctgcggctgc gatgctcagg gggcatgcga
23821 ctcactgcca cctaccggta catcctctcc tgtcactgcg aggaatgcaa ttcctgaggc
23881 ccgctgctgt gtgtggcttc tggatgggac aactgtagag gcagttcgac cagccaggga
23941 aagactggca agaaaagagt taaggcaaaa aaggatgcaa caattctccc gggactctgc
24001 atattctagt aataaagact ctacatgctt gttgacagag agagatactc tgggaacttc
24061 tttgcagttc ccatctcctt tctctggtac aatttctttt ggttcatttt cagattcagg
24121 cattttcccc cttggctctc aatgctgttt gggtttccaa caattcagca ttagtgggaa
24181 aaagtgggcc ctcatacaca agcgtgtcag gctgtcagtg tttggtgcac gctggggaag
24241 aatttacttt ggaaagtaga aaagcccagc ttttcctggg acatcttctg ttattgttga
24301 tgtttttttt taccttgtca ttttggtcta aggttgccat tgctgctaaa ggttaccgat
24361 ttcaaagtcc agataccaag catgtggata tgtttagcta cgtttactca cagccagcga
24421 actgacatta aaataactaa caaacagatt cttttatgtg atgctggaac tcttgacagc
24481 tataattatt attcagaaat gactttttga aagtaaaagc agcataaaga atttgtcaca
24541 ggaaggctgt ctcagataaa ttatggtaaa attttgtaag ggagcagact tttaaagact
24601 tgcacaaata cggatcctgc actgactctg gaaaaggcat atatgtacta gtggcatgga
24661 gaatgcacca tactcatgca tgcaaattag acaaccaagt atgaatctat ttgtgggtgt
24721 gctatagctt tagccgtgtc acgggcatca ttctctaata tccacttgtc catgtgaaac
24781 atgttgccaa aatggtggcc tggcttgtct tctgaacgtt tggttcaaat gtgttttggt
24841 cctggaggct caaattttga gttattccca cgttttgaaa taaaaagagt atattcaaaa
**Mouse Mature NDP Protein (SEQ ID NO: 23)** (NCBI Accession No. NP_035013.1) 
KTDSSFLMDSQRCMRHHYVDSISHPLYKCSSKMVLLARCEGHCSQASRSEPLVSFSTVLKQPFRSSCHCCR

PQTSKLKALRLRCSGGMRLTATYRYILSCHCEECSS
**Mouse Mature NDP cDNA (SEQ ID NO: 24)** 
aaaacagacagttcatttctgatggactctcaacgctgcatgagacaccattatgtcgattctatcagtca
cccactgtacaaatgtagctcaaagatggtgctcctggccagatgtgagggcactgcagccaggcatcac
gctctgagcccttggtgtccttcagcactgtcctcaagcaacctttccgttcctcctgtcactgctgccga
ccccagacttccaagctgaaggctctgcgtctgcgctgctcagggggcatgcgacttactgccacttaccg
gtacatcctctcctgtcactgtgaggaatgcagctcc
**Rhesus Monkey Mature NDP Protein (SEQ ID NO: 25)** (NCBI Accession No. NP_001253901.1)
**Rat Mature NDP Protein (SEQ ID NO: 26)** (NCBI Accession No. NP_001102284.1) 
KTDSSFLMDSQRCMRHHYVDSISHPLYKCSSKMVLLARCEGHCSQASRSEPLVSFSTVLKQPFRSSCHCCR

PQTSKLKALRLRCSGGMRLTATYRYILSCHCEECSS
**Chimpanzee NDP Protein (SEQ ID NO: 27)** (NCBI Accession No. XP_016799622.1)

KTDSSFVMDSDPRRCMRHHYVDSISHPLYKCSSKMVLLARCEGHCSQASRSEPLVSFSTVLKQPFRSSCHC
CRPQTSKLKALRLRCSGGMRLTATYRYILSCHCEECNS

### Trio Rho Guanine Nucleotide Exchange Factor (TRIO) and F-Actin Binding Protein (TRIOBP)

The TRIOBP gene encodes "trio Rho guanine nucleotide exchange factor (TRIO) and F-actin binding protein" (TRIOBP), a protein that is expressed along the length of the stereocilia actin filaments and in the stereocilia rootlets of the inner hair cell. TRIOBP is important for the stability and rigidity of the stereocilia of specialized hair cells in the inner ear (see, e.g., Kitajiri et al. (2010) Cell 141: 786-798; and Zhao et al. (2015) Curr. Opin. Neurobiol. 34: 172-179).

Stereocilia, which are about 10-50 µm in length, are important for hearing and balance. Stereocilia play a mechanosensing role in hearing. By bending in response to sound, they form a structure for mechanoreception of sound stimulation.

The human TRIOBP gene is located on chromosome 22q13.1. It contains 24 exons encompassing ~ 80 kilobases (kb) (Hirosawa et al. (2001) DNA Res 8:1-9; NCBI Accession No. NG_012857.1). To date, there are three known families of TRIOBP splice isoforms: TRIOBP-5, TRIOBP-4 and TRIOBP-1 (see, e.g., Kitajiri et al. (2010) Cell 141: 786-798). While TRIOBP-1 is ubiquitously expressed, TRIOBP-5 and TRIOBP-4 are expressed predominantly in the inner ear and in the eye. TRIOBP-5 gene (NCBI Accession No. NM_001039141), the longest isoform, contains 24 exons, and the translation start site for the TRIOBP-5 isoform is found in exon 3. The TRIOBP-5 protein has a molecular mass of ~218 kDa. The TRIOBP-4 is gene encoded by exons 1-6 and is expressed from the same promoter as TRIOBP-5. The TRIOBP-4 protein has a molecular mass of ~107kDa. TRIOBP-1 (NCB1 Accession No. NM-007032) is encoded by exons 11a-24, has a molecular mass of ~72 kDa, and is translated from a start site in exon 11a (see, e.g., Siepel et al. (2001) J. Cell. Sci. 114: 389-399).

Various mutations in the TRIOBP genes have been associated with non-syndromic hearing loss (e.g., profound, prelingual deadness DFNB28 (MIM#609761) (see, e.g., Riazuddin et al. (2006) Am J Hum Genet 78: 137-143) and age-related hearing impairment (see, e.g., Hoffmann et al. (2016) PLoS Genet 12(10): e1006371)). DFNB28-causing mutations have been predominantly found in exon 6 of TRIOBP, an exon that is present in both TRIOBP-4 mRNA and TRIOBP-5 mRNA. DFNB28-deafness can be modeled by generating TRIOBP-4/5 deficient mice, in which mouse exon 8, the ortholog of exon 6 of human TRIOBP-4 and TRIOBP-5, is mutated/deleted (see, e.g., Kitajiri et al. (2010) Cell 141: 786-798). Nonsense mutation (c.5014G>T (p.Gly1672^{∗}) in exon 9 of TRIOBP-5) and frameshift mutations (c.2653del (p.Arg885Alafs^{∗}120), c.3460_3461del (p.Leu1154Alafs^{∗}29), and c.3232dup (p.Arg1078Profs^{∗}6) in exon 6)) were found in Dutch families with congenital and moderate hearing impairment (see, e.g., Wesdorp et al. (2017) Hearing Research 347: 56-62). The nonsense mutation found in exon 9 was the first disease-associated mutation that was present only in TRIOBP-5.

Additional exemplary mutations in a TRIOBP gene that have been detected in subjects having hearing loss and methods of sequencing a nucleic acid encoding TRIOBP are described in, e.g., Shahin et al. (2006) Am J Hum Genet 78:144-152; Diaz-Horta et al. (2012) PLoS One 7: e50628 ; Gu et al. (2005) Clin. Genet. 87: 588-593; and Table 1 in Wesdorp et al. (2017) Hearing Research 347: 56-62. Methods of detecting mutations in a gene are well-known in the art. Non-limiting examples of such techniques include: real-time polymerase chain reaction (RT-PCR), PCR, sequencing, Southern blotting, and Northern blotting.

An exemplary human wildtype TRIOBP protein is or includes the sequence of SEQ ID NO: 28, SEQ ID NO: 30, or SEQ ID NO: 31, SEQ ID NO: 32. Non-limiting examples of nucleotide sequences encoding a wildtype TRIOBP protein are or include SEQ ID NO: 29, and SEQ ID NO: 33.

In some embodiments of any of the compositions described herein, the TRIOBP protein comprises a sequence that is at least 75% (e.g., at least 80%, at least 85%, at least 90%, at least 91%, at least 92%, at least 93%, at least 94%, at least 95%, at least 96%, at least 97%, at least 98%, at least 99%) identical to SEQ ID NO: 28.

In some embodiments of any of the compositions described herein, the TRIOBP protein comprises a sequence that is at least 75% (e.g., at least 80%, at least 85%, at least 90%, at least 91%, at least 92%, at least 93%, at least 94%, at least 95%, at least 96%, at least 97%, at least 98%, at least 99%) identical to SEQ ID NO: 30.

In some embodiments of any of the compositions described herein, the TRIOBP protein comprises a sequence that is at least 75% (e.g., at least 80%, at least 85%, at least 90%, at least 91%, at least 92%, at least 93%, at least 94%, at least 95%, at least 96%, at least 97%, at least 98%, at least 99%) identical to SEQ ID NO: 31.

In some embodiments of any of the compositions described herein, the TRIOBP protein comprises a sequence that is at least 75% (e.g., at least 80%, at least 85%, at least 90%, at least 91%, at least 92%, at least 93%, at least 94%, at least 95%, at least 96%, at least 97%, at least 98%, at least 99%) identical to SEQ ID NO: 32.
**Human Full-length Wildtype TRIOBP Protein Isoform 5 (SEQ ID NO: 28)** 
MEEVPGDALCEHFEANILTQNRCQNCFHPEEAHGARYQELRSPSGAEVPYCDLPRCPPAPEDPLSASTSGC
QSVVDPGLRPGPKRGPSPSAGLPEEGPTAAPRSRSRELEAVPYLEGLTTSLCGSCNEDPGSDPTSSPDSAT
PDDTSNSSSVDWDTVERQEEEAPSWDELAVMIPRRPREGPRADSSQRAPSLLTRSPVGGDAAGQKKEDTGG
GGRSAGQHWARLRGESGLSLERHRSTLTQASSMTPHSGPRSTTSQASPAQRDTAQAASTREIPRASSPHRI
TQRDTSRASSTQQEISRASSTQQETSRASSTQEDTPRASSTQEDTPRASSTQWNTPRASSPSRSTQLDNPR
TSSTQQDNPQTSFPTCTPQRENPRTPCVQQDDPRASSPNRTTQRENSRTSCAQRDNPKASRTSSPNRATRD
NPRTSCAQRDNPRASSPSRATRDNPTTSCAQRDNPRASRTSSPNRATRDNPRTSCAQRDNPRASSPSRATR
DNPTTSCAQRDNPRASRTSSPNRATRDNPRTSCAQRDNPRASSPNRAARDNPTTSCAQRDNPRASRTSSPN
 
RATRDNPRTSCAQRDNPRASSPNRATRDNPTTSCAQRDNPRASRTSSPNRATRDNPRTSCAQRDNPRASSP
NRTTQQDSPRTSCARRDDPRASSPNRTIQQENPRTSCALRDNPRASSPSRTIQQENPRTSCAQRDDPRASS
PNRTTQQENPRTSCARRDNPRASSRNRTIQRDNPRTSCAQRDNPRASSPNRTIQQENLRTSCTRQDNPRTS
SPNRATRDNPRTSCAQRDNLRASSPIRATQQDNPRTCIQQNIPRSSSTQQDNPKTSCTKRDNLRPTCTQRD
RTQSFSFQRDNPGTSSSQCCTQKENLRPSSPHRSTQWNNPRNSSPHRTNKDIPWASFPLRPTQSDGPRTSS
PSRSKQSEVPWASIALRPTQGDRPQTSSPSRPAQHDPPQSSFGPTQYNLPSRATSSSHNPGHQSTSRTSSP
VYPAAYGAPLTSPEPSQPPCAVCIGHRDAPRASSPPRYLQHDPFPFFPEPRAPESEPPHHEPPYIPPAVCI
GHRDAPRASSPPRHTQFDPFPFLPDTSDAEHQCQSPQHEPLQLPAPVCIGYRDAPRASSPPRQAPEPSLLF
QDLPRASTESLVPSMDSLHECPHIPTPVCIGHRDAPSFSSPPRQAPEPSLFFQDPPGTSMESLAPSTDSLH
GSPVLIPQVCIGHRDAPRASSPPRHPPSDLAFLAPSPSPGSSGGSRGSAPPGETRHNLEREEYTVLADLPP
PRRLAQRQPGPQAQCSSGGRTHSPGRAEVERLFGQERRKSEAAGAFQAQDEGRSQQPSQGQSQLLRRQSSP
APSRQVTMLPAKQAELTRRSQAEPPHPWSPEKRPEGDRQLQGSPLPPRTSARTPERELRTQRPLESGQAGP
RQPLGVWQSQEEPPGSQGPHRHLERSWSSQEGGLGPGGWWGCGEPSLGAAKAPEGAWGGTSREYKESWGQP
EAWEEKPTHELPRELGKRSPLTSPPENWGGPAESSQSWHSGTPTAVGWGAEGACPYPRGSERRPELDWRDL
LGLLRAPGEGVWARVPSLDWEGLLELLQARLPRKDPAGHRDDLARALGPELGPPGTNDVPEQESHSQPEGW
AEATPVNGHSPALQSQSPVQLPSPACTSTQWPKIKVTRGPATATLAGLEQTGPLGSRSTAKGPSLPELQFQ
PEEPEESEPSRGQDPLTDQKQADSADKRPAEGKAGSPLKGRLVTSWRMPGDRPTLFNPFLLSLGVLRWRRP
DLLNFKKGWMSILDEPGEPPSPSLTTTSTSQWKKHWFVLTDSSLKYYRDSTAEEADELDGEIDLRSCTDVT
EYAVQRNYGFQIHTKDAVYTLSAMTSGIRRNWIEALRKTVRPTSAPDVTKLSDSNKENALHSYSTQKGPLK
AGEQRAGSEVISRGGPRKADGQRQALDYVELSPLTQASPQRARTPARTPDRLAKQEELERDLAQRSEERRK
WFEATDSRTPEVPAGEGPRRGLGAPLTEDQQNRLSEEIEKKWQELEKLPLRENKRVPLTALLNQSRGERRG
PPSDGHEALEKEVQALRAQLEAWRLQGEAPQSALRSQEDGHIPPGYISQEACERSLAEMESSHQQVMEELQ
RHHERELQRLQQEKEWLLAEETAATASAIEAMKKAYQEELSRELSKTRSLQQGPDGLRKQHQSDVEALKRE
LQVLSEQYSQKCLEIGALMRQAEEREHTLRRCQQEGQELLRHNQELHGRLSEEIDQLRGFIASQGMGNGCG
RSNERSSCELEVLLRVKENELQYLKKEVQCLRDELQMMQKDKRFTSGKYQDVYVELSHIKTRSEREIEQLK
EHLRLAMAALQEKESMRNSLAE
**Human Wildtype TRIOBP Isoform 5 cDNA (SEQ ID NO: 29)** 
atggaggag gtgcctgggg atgccctgtg tgaacacttt gaggccaaca tacttaccca
gaaccgctgt caaaactgct tccaccctga ggaggcccat ggagcaagat accaggagct
caggagccct tcaggtgctg aggtgcccta ctgcgacctg cctcgatgtc cacctgcccc
tgaggaccca ctcagcgcct caacctccgg ctgccagtct gtggtggacc caggcctcag
gccagggccc aagaggggcc catccccctc agcagggctc ccagaagagg gtcccacagc
tgcccccagg agcaggagcc gggagcttga ggcagtaccc tatctggagg gcctgaccac
ttccttgtgt ggcagctgca acgaggaccc cggctctgac cccacctcca gccctgactc
cgccacccct gatgatacca gcaactcgtc ctctgtggac tgggacactg ttgagaggca
ggaggaggag gcccccagct gggacgagct cgcagtgatg atcccgagga ggcctcggga
ggggccgaga gctgacagct cccaaagggc tccgtctctc ctcaccaggt cccctgtggg
 
aggagatgct gcaggccaga aaaaggagga caccggcggt gggggccgga gcgcaggaca
gcactgggca aggctccggg gagaaagcgg gttgtccctg gagcggcacc ggtcaacact
gacccaggct tcctccatga caccacacag tggacctcga agcaccacgt ctcaggcttc
tcctgcccaa agggacactg ctcaggctgc ctctacacgt gaaatcccca gagcctcctc
tccccatcga atcacccaaa gggacacctc cagggcctca tccacccaac aggaaatctc
cagggcctca tccacccaac aggaaacctc cagggcctca tccacccaag aggacacccc
tagggcctca tccacccaag aggacacccc cagggcctca tctacacagt ggaacacccc
cagagcttcc tctccctcac gaagcaccca actggataac cccagaacct cttctaccca
gcaggacaac ccccaaactt cttttcctac ttgtactccc cagcgggaaa accccaggac
accctgtgtc cagcaggacg atcccagagc ctcctctccc aacagaacca ctcaacgaga
gaattccaga acatcctgtg cccagcggga caatcccaaa gcctccagaa cctcctctcc
caatagagcc acacgagaca accccagaac atcctgcgcc cagcgggaca atcccagagc
ctcctctccc agtagagcta cacgagacaa ccccacaaca tcctgtgccc agcgggacaa
tcccagagcc tccagaacct cctctcccaa tagagccaca cgagacaacc ccagaacatc
ctgtgcccag cgggacaatc ccagagcctc ctctcccagt agagctacac gagacaaccc
cacaacatcc tgtgcccagc gggacaatcc cagagcctcc agaacctcct ctcccaatag
agccacacga gacaacccca gaacatcctg cgcccagcgg gacaatccca gagcctcctc
tcccaataga gctgcacgag acaaccccac aacatcctgt gcccagcggg acaatcccag
agcctccaga acctcctctc ccaatagagc cacacgagac aaccccagaa catcctgtgc
ccagcgggac aatcccagag cctcctctcc caatagagct acacgagaca accccacaac
atcctgtgcc cagcgggaca atcccagagc ctccagaacc tcctctccca atagagccac
acgagataac cccagaacat cctgtgccca gcgggacaat cccagagcct cctctcccaa
cagaaccacc caacaagaca gccccagaac atcctgtgcc cgacgggacg atcccagagc
ctcctctcct aacagaacca tccaacaaga gaaccccaga acatcctgtg ccctacggga
caatcccaga gcctcctctc ccagcagaac catccaacaa gagaacccca gaacatcctg
tgcccaacgg gacgatccca gagcctcctc tcctaacaga accacccaac aagagaaccc
cagaacatcc tgtgcccgac gggacaatcc cagagcctcc tctcgcaaca gaaccatcca
gcgagacaac cccagaacat cctgtgccca gcgggacaat cccagagcct cctctcctaa
cagaaccatc caacaagaga acctcagaac atcctgtacc cgacaggaca atcccaggac
ctcctctccc aatagagcca cacgagacaa ccccagaaca tcctgtgccc agcgggacaa
tctcagagcc tcctctccca tcagagccac ccaacaggac aaccccagaa cttgtattca
acagaacatc cccagatcat cttctaccca acaagacaac cctaaaacct cttgtaccaa
acgagataac ctcagaccca cttgtacaca gcgggaccgc acacagtcct tttcctttca
acgagacaac cctggaacct cctcatctca atgctgcacc caaaaggaga atctgagacc
atcatctccc caccgctcca ctcaatggaa caatcccagg aattcatctc cccatcgtac
taacaaagac atcccctggg cctcgtttcc cctccggcca actcagagtg atggtccccg
aacctcttcc ccatctcgct ccaagcaaag cgaggttccc tgggcatcca tcgccctccg
gccaacccaa ggtgacaggc ctcagacatc ctctcccagc aggccagccc agcatgaccc
 
accccagtcc tcctttggcc ccacccagta caacttgcca tcccgggcca cctcttcctc
ccataaccca ggccaccaga gcacctcccg aacttcctca cctgtgtacc ccgctgccta
tggggctccc ctgacctctc ctgagccctc ccagcctcca tgtgctgtgt gcattgggca
ccgggatgcc cctcgagcct cttcgccccc tcgctatttg cagcacgacc ccttcccctt
cttcccagag ccccgcgccc ctgagagtga accgccccac cacgagcctc cctatatacc
acctgctgtg tgcattggac accgagatgc cccccgggcg tcctcgcccc cccgccacac
ccaatttgac cccttcccct tcctcccaga cacatcagat gccgagcatc agtgtcagtc
cccccaacac gagccccttc agctccctgc acctgtgtgt attgggtacc gagatgcacc
ccgggcctcc tccccaccac gccaggcccc agagccttcc ctcttattcc aggacctccc
cagggccagc acagagagcc ttgtcccttc catggactct ctgcacgagt gcccccacat
ccccacccct gtgtgcattg ggcaccggga tgcaccctcc ttctcatccc caccacgcca
ggctcctgag ccatccctct tcttccagga tccccctgga actagtatgg agagcctggc
cccctccact gactctctgc atggctcccc agtgctgatc ccccaagtgt gcatcgggca
ccgggatgca ccccgagcct cctccccacc ccgccaccca cccagtgacc tagcgttcct
ggcaccctca ccttcaccgg gcagctctgg gggctcccgg ggctcagcgc ctcccgggga
gaccaggcac aacttggagc gggaggagta cactgtgctg gccgacctgc ccccacccag
gaggctggcc cagagacagc caggccccca ggcgcagtgc agcagcgggg gccgcaccca
cagccctggc cgtgcagagg tggagcgcct cttcgggcaa gagcgcagga agtccgaggc
agcgggggcc ttccaggccc aggacgaggg acggtcacag cagcccagcc aaggccagag
ccaacttctc cgaagacagt ccagccctgc ccccagcagg caggtgacca tgctccctgc
caaacaggca gaactgaccc ggcggagcca agcagagccc cctcatcctt ggagtcctga
gaagagacct gagggagatc ggcagctcca ggggtccccg ctgcccccca ggacatcagc
caggacccct gagagggagc tgcggacaca gagacctctg gagagtggcc aagcaggccc
aagacagcct ctgggggtgt ggcagagtca ggaggaaccg ccagggtccc agggccctca
tagacaccta gaaaggagct ggagcagcca ggagggaggc ctgggccctg ggggctggtg
gggatgtgga gagcccagcc tgggggcagc caaagccccg gagggagcat gggggggcac
ttccagggag tacaaggaga gctgggggca gccagaggcc tgggaggaga agcccactca
tgagctcccc agagaactag gaaagagaag cccactcacg agcccccctg agaactgggg
aggccccgca gagtcctcac aatcctggca ctctgggaca cccactgctg tgggctgggg
ggcagaggga gcgtgtccat acccgcgtgg ctctgagagg cgacccgagc ttgactggag
ggatctgctt ggccttctcc gggcaccagg agagggggtc tgggcccgtg tccccagcct
ggactgggag ggcctcttgg agctcctgca ggccaggctg ccccgcaagg acccagctgg
acacagggat gacctggcca gggctttagg gccagagctg ggtcccccag gcacaaacga
tgtccctgag caggagtcac acagccagcc agaaggctgg gccgaggcca ccccagtcaa
tggacacagc cccgcactgc agtcccagag cccggtccag ctgcccagcc ctgcctgcac
ctccacccag tggccaaaga tcaaagtgac aagaggacca gcgaccgcaa ctctggcagg
cctggagcag acgggccccc tggggagcag gagcactgcg aagggcccca gcttgccaga
gctgcagttc caaccagagg agcctgagga gtcagaacca agcagaggcc aagaccccct
 
gactgaccag aagcaggcag actcggcaga caagaggcca gcagagggca aggctgggag
cccgctcaag ggccgactgg tgacctcatg gcggatgccc ggggaccggc ccacgctgtt
caatccgttc ctgctgtctc tgggggtcct caggtggcga aggcccgatc tgctcaactt
caagaaggga tggatgtcga tcttggacga gcctggagag cctccctccc cctcgctcac
caccacctct acttcgcagt ggaagaaaca ttggtttgtg ctgacagatt caagtctcaa
atattacaga gactccactg ctgaggaggc agatgagctg gatggtgaga tcgacctgcg
ttcctgcacg gatgtcactg agtacgcggt gcagcgcaac tatggcttcc agatccacac
caaggatgct gtctatacct tgtcggccat gacctcaggc atccggcgga actggatcga
ggctctgaga aagaccgtac gtccaacttc agccccagat gtcaccaagc tctcggactc
taacaaggag aacgcgctgc acagctacag cacccagaag ggccccctga aggcagggga
gcagcgggcg ggctctgagg tcatcagccg gggtggccct cggaaggcgg acgggcagcg
tcaggccttg gactacgtgg agctctcgcc gctgacccag gcttccccgc agcgggcccg
caccccagcc cgcactcctg accgcctggc caagcaggag gagctggagc gggacctggc
ccagcgctcc gaggagcggc gcaagtggtt tgaggccaca gacagcagga ccccagaggt
gcctgctggt gaggggccgc gccggggcct gggtgccccc ctgactgagg accagcaaaa
ccggcttagt gaggagatcg agaagaagtg gcaggagctg gagaagctgc ccctgcggga
gaataagcgg gtgcccctca ctgccctgct caaccaaagc cgcggagagc gccgagggcc
cccaagtgac ggccacgagg cactggagaa ggaggttcag gctcttcggg cccagctgga
ggcgtggcgt ctccaagggg aggctcctca gagtgcactg agatcccagg aggatggcca
catccccccg ggctacatct cacaggaggc atgtgagcgc agcctggcag agatggagtc
ctcgcaccag caggtgatgg aggagctgca gcggcaccac gagcgggagc tgcagcgcct
gcagcaggag aaggagtggc tcctggctga ggagacggca gccacggcct cagccattga
agccatgaag aaggcctacc aggaagagct gagccgagag ctgagcaaaa cacggagtct
ccagcagggc ccggatggcc tccggaagca gcaccagtca gatgtggagg cactgaagcg
agagctgcag gtgctatcgg agcagtactc gcagaagtgc ctggagattg gggcactcat
gcggcaggct gaggagcgcg agcacacgct gcgccgctgc cagcaggagg gccaggagct
gctgcgccac aaccaggagc tgcatggccg cctgtcagag gagatagacc agctgcgcgg
cttcattgcc tcgcagggca tgggcaatgg ctgcgggcgc agcaacgagc ggagttcctg
cgagctagag gtgctgcttc gcgtaaaaga aaacgaactc cagtacctaa agaaggaggt
gcagtgcctc cgggacgagc tccagatgat gcagaaggac aagcgcttca cctcgggaaa
gtaccaggac gtctatgtgg agctgagcca catcaagaca cggtctgagc gggagatcga
gcagctgaag gagcacctgc gtcttgccat ggccgccctc caggagaagg agtcgatgcg
caacagcctg gctgagtag
**Human Full-length Wildtype TRIOBP Protein Isoform 1 (SEQ ID NO: 30)** 
MTPDLLNFKKGWMSILDEPGEPPSPSLTTTSTSQWKKHWFVLTDSSLKYYRDSTAEEADELDGEIDLRSCT
DVTEYAVQRNYGFQIHTKDAVYTLSAMTSGIRRNWIEALRKTVRPTSAPDVTKLSDSNKENALHSYSTQKG
PLKAGEQRAGSEVISRGGPRKADGQRQALDYVELSPLTQASPQRARTPARTPDRLAKQEELERDLAQRSEE
 
RRKWFEATDSRTPEVPAGEGPRRGLGAPLTEDQQNRLSEEIEKKWQELEKLPLRENKRVPLTALLNQSRGE
RRGPPSDGHEALEKEVQALRAQLEAWRLQGEAPQSALRSQEDGHIPPGYISQEACERSLAEMESSHQQVME
ELQRHHERELQRLQQEKEWLLAEETAATASAIEAMKKAYQEELSRELSKTRSLQQGPDGLRKQHQSDVEAL
KRELQVLSEQYSQKCLEIGALMRQAEEREHTLRRCQQEGQELLRHNQELHGRLSEEIDQLRGFIASQGMGN
GCGRSNERSSCELEVLLRVKENELQYLKKEVQCLRDELQMMQKDKRFTSGKYQDVYVELSHIKTRSEREIE
QLKEHLRLAMAALQEKESMRNSLAE
**Human Full-length Wildtype TRIOBP Protein Isoform 4 (SEQ ID NO: 31)** 
MIPRRPREGPRADSSQRAPSLLTRSPVGGDAAGQKKEDTGGGGRSAGQHWARLRGESGLSLERHRSTLTQA
SSMTPHSGPRSTTSQASPAQRDTAQAASTREIPRASSPHRITQRDTSRASSTQQEISRASSTQQETSRASS
TQEDTPRASSTQEDTPRASSTQWNTPRASSPSRSTQLDNPRTSSTQQDNPQTSFPTCTPQRENPRTPCVQQ
DDPRASSPNRTTQRENSRTSCAQRDNPKASRTSSPNRATRDNPRTSCAQRDNPRASSPSRATRDNPTTSCA
QRDNPRASRTSSPNRATRDNPRTSCAQRDNPRASSPSRATRDNPTTSCAQRDNPRASRTSSPNRATRDNPR
TSCAQRDNPRASSPNRAARDNPTTSCAQRDNPRASRTSSPNRATRDNPRTSCAQRDNPRASSPNRATRDNP
TTSCAQRDNPRASRTSSPNRATRDNPRTSCAQRDNPRASSPNRTTQQDSPRTSCARRDDPRASSPNRTIQQ
ENPRTSCALRDNPRASSPSRTIQQENPRTSCAQRDDPRASSPNRTTQQENPRTSCARRDNPRASSRNRTIQ
RDNPRTSCAQRDNPRASSPNRTIQQENLRTSCTRQDNPRTSSPNRATRDNPRTSCAQRDNLRASSPIRATQ
QDNPRTCIQQNIPRSSSTQQDNPKTSCTKRDNLRPTCTQRDRTQSFSFQRDNPGTSSSQCCTQKENLRPSS
PHRSTQWNNPRNSSPHRTNKDIPWASFPLRPTQSDGPRTSSPSRSKQSEVPWASIALRPTQGDRPQTSSPS
RPAQHDPPQSSFGPTQYNLPSRATSSSHNPGHQSTSRTSSPVYPAAYGAPLTSPEPSQPPCAVCIGHRDAP
RASSPPRYLQHDPFPFFPEPRAPESEPPHHEPPYIPPAVCIGHRDAPRASSPPRHTQFDPFPFLPDTSDAE
HQCQSPQHEPLQLPAPVCIGYRDAPRASSPPRQAPEPSLLFQDLPRASTESLVPSMDSLHECPHIPTPVCI
GHRDAPSFSSPPRQAPEPSLFFQDPPGTSMESLAPSTDSLHGSPVLIPQVCIGHRDAPRASSPPRHPPSDL
AFLAPSPSPGSSGGSRGSAPPGETRHNLEREEYTVLADLPPPRRLAQRQPGPQAQCSSGGRTHSPGRAEVE
RLFGQERR
**Human Full-length Wildtype TRIOBP Protein Isoform 6 (SEQ ID NO: 32)**

MEEVPGDALCEHFEANILTQNRCQNCFHPEEAHGARYQELRSPSGAEVPYCDLPRCPPAPEDPLSASTSGC
QSVVDPGLRPGPKRGPSPSAGLPEEGPTAAPRSRSRELEAVPYLEGLTTSLCGSCNEDPGSDPTSSPDSAT
PDDTSNSSSVDWDTVERQEEEAPSWDELAVMIPRRPREGPRADSSQRAPSLLTRSPVGGDAAGQKKEDTGG
GGRSAGQHWARLRGESGLSLERHRSTLTQASSMTPHSGPRSTTSQASPAQRDTAQAASTREIPRASSPHRI
TQRDTSRASSTQQEISRASSTQQETSRASSTQEDTPRASSTQEDTPRASSTQWNTPRASSPSRSTQLDNPR
TSSTQQDNPQTSFPTCTPQRENPRTPCVQQDDPRASSPNRTTQRENSRTSCAQRDNPKASRTSSPNRATRD
NPRTSCAQRDNPRASSPSRATRDNPTTSCAQRDNPRASRTSSPNRATRDNPRTSCAQRDNPRASSPSRATR
DNPTTSCAQRDNPRASRTSSPNRATRDNPRTSCAQRDNPRASSPNRAARDNPTTSCAQRDNPRASRTSSPN
RATRDNPRTSCAQRDNPRASSPNRATRDNPTTSCAQRDNPRASRTSSPNRATRDNPRTSCAQRDNPRASSP
NRTTQQDSPRTSCARRDDPRASSPNRTIQQENPRTSCALRDNPRASSPSRTIQQENPRTSCAQRDDPRASS

PNRTTQQENPRTSCARRDNPRASSRNRTIQRDNPRTSCAQRDNPRASSPNRTIQQENLRTSCTRQDNPRTS
SPNRATRDNPRTSCAQRDNLRASSPIRATQQDNPRTCIQQNIPRSSSTQQDNPKTSCTKRDNLRPTCTQRD
RTQSFSFQRDNPGTSSSQCCTQKENLRPSSPHRSTQWNNPRNSSPHRTNKDIPWASFPLRPTQSDGPRTSS
PSRSKQSEVPWASIALRPTQGDRPQTSSPSRPAQHDPPQSSFGPTQYNLPSRATSSSHNPGHQSTSRTSSP
VYPAAYGAPLTSPEPSQPPCAVCIGHRDAPRASSPPRYLQHDPFPFFPEPRAPESEPPHHEPPYIPPAVCI
GHRDAPRASSPPRHTQFDPFPFLPDTSDAEHQCQSPQHEPLQLPAPVCIGYRDAPRASSPPRQAPEPSLLF
QDLPRASTESLVPSMDSLHECPHIPTPVCIGHRDAPSFSSPPRQAPEPSLFFQDPPGTSMESLAPSTDSLH
GSPVLIPQVCIGHRDAPRASSPPRHPPSDLAFLAPSPSPGSSGGSRGSAPPGETRHNLEREEYTVLADLPP
PRRLAQRQPGPQAQCSSGGRTHSPGRAEVERLFGQERRKSEAAGAFQAQDEGRSQQPSQGQSQLLRRQSSP
APSRQVTMLPAKQAELTRRSQAEPPHPWSPEKRPEGDRQLQGSPLPPRTSARTPERELRTQRPLESGQAGP
RQPLGVWQSQEEPPGSQGPHRHLERSWSSQEGGLGPGGWWGCGEPSLGAAKAPEGAWGGTSREYKESWGQP
EAWEEKPTHELPRELGKRSPLTSPPENWGGPAESSQSWHSGTPTAVGWGAEGACPYPRGSERRPELDWRDL
LGLLRAPGEGVWARVPSLDWEGLLELLQARLPRKDPAGHRDDLARALGPELGPPGTNDVPEQESHSQPEGW
AEATPVNGHSPALQSQSPVQLPSPACTSTQWPKIKVTRGPATATLAGLEQTGPLGSRSTAKGPSLPELQFQ
PEEPEESEPSRGQDPLTDQKQADSADKRPAEGKAGSPLKGRLVTSWRMPGDRPTLFNPFLLSLGVLRWRRP
DLLNFKKGWMSILDEPGEPPSPSLTTTSTSQWKKHWFVLTDSSLKYYRDSTAEEADELDGEIDLRSCTDVT
EYAVQRNYGFQIHTKDAVYTLSAMTSGIRRNWIEALRKTVRPTSAPDVTKLSDSNKENALHSYSTQKGPLK
AGEQRAGSEVISRGGPRKADGQRQALDYVELSPLTQASPQRARTPARTPDRLAKQEELERDLAQRSEERRK
WFEATDSRTPEVPAGEGPRRGLGAPLTEDQQNRLSEEIEKKWQELEKLPLRENKRVPLTALLNQSRGERRG
PPSDGHEALEKEVQALRAQLEAWRLQGEAPQSALRSQEDGHIPPGYISQEACERSLAEMESSHQQVMEELQ
RHHERELQRLQQEKEWLLAEETAATASAIEAMKKAYQEELSRELSKTRSLQQGPDGLRKQHQSDVEALKRE
LQVLSEQYSQKCLEIGALMRQAEEREHTLRRCQQEGQELLRHNQELHGRLSEEIDQLRGFIASQGMGNGCG
RSNERSSCELEVLLRVKENELQYLKKEVQCLRDELQMMQKDKRFTSGKYQDVYVELSHIKTRSEREIEQLK
EHLRLAMAALQEKESMRNSLAE

A non-limiting example of a human wildtype TRIOBP genomic DNA sequence is SEQ ID NO: 33. The exons in SEQ ID NO: 33 are: nucleotide positions 1-102 (exon 1), nucleotide positions 601-709 (exon 2), nucleotide positions 4319-4492 (exon 3), nucleotide positions 13440-13579 (exon 4), nucleotide positions 16223-16424 (exon 5), nucleotide positions 18776-18947 (exon 6), nucleotide positions 26198-29516 (exon 7), nucleotide positions 36311-36425 (exon 8), nucleotide positions 37412-38455 (exon 9), nucleotide positions 41655-41732 (exon 10), nucleotide positions 43908-44045 (exon 11), nucleotide positions 54785-54841 (exon 12), nucleotide positions 57890-57997 (exon 13), nucleotide positions 58114-58203 (exon 14), nucleotide positions 58563-58672 (exon 15), nucleotide positions 60626-61151 (exon 16), nucleotide positions 62167-62277 (exon 17), nucleotide positions 68683-68830 (exon 18), nucleotide positions 71087-71189 (exon 19), nucleotide positions 72041-72200 (exon 20), nucleotide positions 72275-72388 (exon 21), nucleotide positions 74663-74749 (exon 22), nucleotide positions 75614-75777 (exon 23), and nucleotide positions 76796-79569 (exon 24). The introns are located between each pair of these exons in SEQ ID NO: 33, i.e., at nucleotide positions 103-600 (intron 1), nucleotide positions 710-4318 (intron 2), nucleotide positions 4493-13439 (intron 3), nucleotide positions 13580-16222 (intron 4), nucleotide positions 16425-18775 (intron 5), nucleotide positions 18948-26197 (intron 6), nucleotide positions 29517-36310 (intron 7), nucleotide positions 36426-37411 (intron 8), nucleotide positions 38456-41654 (intron 9), nucleotide positions 41733-43907 (intron 10), nucleotide positions 44046-54784 (intron 11), nucleotide positions 54842-57889 (intron 12), nucleotide positions 57998-58113 (intron 13), nucleotide positions 58204-58562 (intron 14), nucleotide positions 58673-60625 (intron 15), nucleotide positions 61152-62166 (intron 16), nucleotide positions 62278-6862 (intron 17), nucleotide positions 68831-71086 (intron 18), nucleotide positions 71190-72040 (intron 19), nucleotide positions 72201-72274 (intron 20), nucleotide positions 72389-74662 (intron 21), nucleotide positions 74750-75613 (intron 22), and nucleotide positions 75778-76795 (intron 23).

**Human Wildtype TRIOBP Genomic Sequence (SEQ ID NO: 33)** (NCBI Accession No. NG_012857.1)

1 cttccacctc ccgagcatcg ctctgattca ttgttaaaat aaagaggcca tgaccctggc
61 actcccaccc cggcagcccc tgtgcgtgga aacccagcca aggttagcac tgggcctcag
121 aaaagaggag ctggtagggg gaggcctaca ggaggggtag gggtcgaggg atccggggag
181 gaactggctg aatgcctgga tggagagggt cccgggtcca ttctctgggt ggggcatgtg
241 tagctgctta aggttttgat tattatatcc acatctcaag tgcattttag agaaggattc
301 tgcctattgg ttgtgggtag gggaggaaac gggagcagcg aggggcacgt ggagtagagc
361 cccctaccca cccccaggaa aatgagcggc gttctcctgc taggcgacgc tcctttatgt
421 ggccagacca ggagagaaac gcccaggacg ggcggggccg atgcgcccct cttccaccac
481 caggagtcgg gaggacgacc gcccggggag gatcagagac cctgggccca cggaaggacc
541 agtgggtagc cctctcccca ccgtgccttg ctgtcgcccc caccgcattc ccttctccag
601 gtctgatgat ggaggagcct tggccctggc tgtcccaggg gaggaggtga aattcctcag
661 ctctccacca agattggcca caaaagcctg atcccctgga acacagcagg tgaggatgga

721 tgtgggcagg tgacctgggg gcaccaaaag ctgcaggtgt tttgggatgt tgggttcctc
781 ccacccccca ccctccccct ggggctctgt gtgagtgggg aggccatctt taagtgattc
841 tcctgaccca gctccactcc agggcatgga tgttggtggc cttaggggca cccagaaagg
901 agaacccttg cacaagtaag ataccagggc ggccgggcgc ggtggctcaa gcctgtaatc
961 ccagcacttt gggaggtcga ggtgggtgga tcacaaggtc aggagttcga gaccagcctg
1021 gcccatatgg tgaaacctcg tctctaccaa aaatacaaaa aaattatccg ggcatggtgg
1081 cacatgcctg taatcccagc tatttgggag gctgcggcag gagaattgct tgaatccggg
1141 aggcagaggt tgcagtgagc cgagatcgcg ccactgcact ccagcctggg tgatagaccg
1201 agattctgtc tcaaaaaaaa aaaaaaaaaa agaaagaaag acaccagggt ttcagaaggg
1261 tatgatgctt cacacctgta atcccagcac tttgagggaa ggaggaggat tacttgaggt
1321 caggagttca ggagttcaaa accagcctgg gtaacactgc aagagccttt tttttttttt
1381 tttttttttt tgagacacac tgttgcccag gctggagtgc agcggggcga tcttagctcc
1441 actgcaacct ccacctcctg ggttcaagca attctcttgc cccagcctct tgagtagctg
1501 ggattacagg catgcgcacc acgcccagct aatttttata gttttagtag agacagggtt
1561 tcatcatgtt gggcaggctg gtcttgaatt cctggcctca agtgatctgc ctgcctcagc
1621 ctcccaaagt gctgggatta taggcgtgag ccaccgcacc tggccaagag cccattttta
1681 caaaataaaa aagaaaaaaa ttagctgggc gtggtggcgt acacctgtag tcccagttac
1741 tcagtcccag agactgaggc aggagaatca tttgagcccg ggagtttgag gttgcaagga
1801 gctatgatcg agccactgca ctccagcctg ggtgacagag caagaccctg cccccacctc
1861 cccaaaaaag acattagggg ccggatgcag tggctcacac ctgtaatccc agcattttgg
1921 gaggccgaag ggggcggatc acctgaggtc aggagttcac ctggccaaca tggcgaaacc
1981 ccgttgctac taaaaataca aaaattagcc aggcatgacg gtgcatgcct gtgatcccag
2041 atactactgg ggaggctgag gtagaattgc ttgaacccag gaggcggagg ttgcagtgag
2101 ccgagtgagc caagatcaca tcacggcact ccagcctagg tgacagagag agacttcgtc
2161 tcaaaaaaac aaaacaaaac aaaacgaaaa cacattagga tttctttttg gctaagttct
2221 gccacaaact tgctgtgtga ctttaggctg ttcacgtttg tcaaagacgt cgtcctcaac
2281 tctgaaaata cctggccagc tttttgcctc cctcgctgtg cttcccaagg gacctgtgcg
2341 agttggcagt atgatccagt gctcctcact tccacctccg agagagcagg gacacacaca
2401 gccctggatt tgaatcagcc actgcgacct tcagtgaccg aaggaacctc tctgagcctt
2461 agttgcctga tctaaaattg gcattaattt gatctacaat gcagctataa gacaaggaca
2521 ctcaaacttt ggggcgcact agaatcacct ggggaatcac tttttttttt taagacagag
2581 tctcactctt atcacccagg ctggagtaca gtggtgcaat cttggctcac tgcaacctcc
2641 gcctcctggg ttcaagtgat tctcctgcct cagcctcccg agtaactggg actacaggtg
2701 cataccacca tgcctggcta attttgtatt tttagtagag acagggtttg gcaatattgg
2761 ccaggctggt ctcgaactcc tgacctcagg tgatctgccc gcctcggcct cccaaagtgc
2821 tgggattata ggcgtgagcc actgcgcctg gccaggaatc gcttctttat tttcattttc
2881 atttatttat ttatttattt atttattttg agatggagtc tcgctctgtt gctcagtctg
2941 gagtgaagtg gcgtgctctc cgctcactgc tacctctgcc tcccaggttc aagcgattct

3001 cgtgcctcag cctcccaagt agctgggact acaggtgcgt gctaccacgc ccagctaatt
3061 tttgtattta gtagaggcgg ggtttcacca tgttggccag gctggtcttg aactcctggc
3121 ctcaagtgat ctgcctgcct cagcctccca aagtgctggg attacaggcg tgagccaccg
3181 ccctcggctg gaatcacttc tttaacatgc acagccccag acctcagtct cttggagatt
3241 ctaactgtgt gaattgggag agagaaaggg gaatctagat tttttttttt ttttttgag
3301 atgtggtctt gctatgttgc ctaggctgga gtgctgtaac tattcacagg tgcagttata
3361 gcacactacc acctcaaact cctgggctca agagatcctc ccacctctgc ctcccgagta
3421 gcttggactg caggtgcaca ccatcttacc cagctgaaat ctgctttttt tttttctcag
3481 tgcagtggca cagtcatggc tcactgcagc ctcgacctcc cagactcagg tgatcctccc
3541 aacttcagcc tcctgagtag cagggaccac aggcctgcac caccttgccc agctaatttt
3601 tgtatttttt gtagagacgg agtttcgcca gttgcccagg atggtcttga actcttgggc
3661 tcaaatgatc cgccgcccgc aatgggccca gctggaatct gcattttttt gtttttgtt
3721 ttgagatgaa gtctcactct gtcgcccagg ctggagtgca atggcgcaat cttggctcac
3781 tgcaacctcc gcctcctggg ttcaagccat tctcctgcct caggctccca agtagctggg
3841 attacaggca cgtgccatca cacttggctg atttttgtat ttttagtaga gacagggttt
3901 taccatattg gccaggctgg tctcgaactc ctgacctcaa gtgatcggcc cacctcggcc
3961 tcccaaagtg ctgggattac aggcatcagc cactgtgcct ggcctggtat ctgcattttt
4021 atgaacagcc caggagggtg taatgcagag tccttggcgt gcactttcag caacactgca
4081 gtaaggacta gaaatcgcac atcaaatgct ggcagagtgc ctggtgctta gtaggtgctt
4141 agtgagtgtt tgttgagtga ataaatgaac aggaatggat ggagaacagg aacccaaagg
4201 aatggatgag taaccttatt tccttggtcc ttgtctggaa actggggcaa ctggggctgg
4261 ggttcaggtg agtggccagc cagtcatctg gtctttgcct ccccctcatt tttgccaggc
4321 ctcacataga cggtcagcca ttggatcata ggaactgccc tggcctgact cacccaatat
4381 ggaggaggtg cctggggatg ccctgtgtga acactttgag gccaacatac ttacccagaa
4441 ccgctgtcaa aactgcttcc accctgagga ggcccatgga gcaagatacc aggtgggcca
4501 gttttccacg tggttggggt ggtttgtgat gggggcatgg gtgaaggact gggcctgtgg
4561 tgtccgctaa ctcgcagttg aacccagatg ctggacttcc tcctgtcgag agcctcggtt
4621 ttcccacatt gcagggaagt ggttgaacca tgtgaatgtt tcacaatctt cagtcacttt
4681 cctcctgctt atatgatacg attgttacca tagccttatc taatctgtta cttactttgt
4741 tattctttaa atcaacatct tttccccttt ctattgaaac aaaacttcac atcatcaaca
4801 caagtagaaa actagaatta cttcctgtta agaagaagag gttaagaaga aaggtagagg
4861 ctgggcacgg tggctcacgc ctgtaatccc agcactttgg gaggccgagg tgggtggatc
4921 acccgaggtc gggagtttga aaccagcctg gccaacatgg tgaaaattag ctgggcatgg
4981 tggtgcatgc ctgtaatccc agctacttgg gaggctgagg caggagaatc acttgaacct
5041 gggaggcgga gattgtggtg agccgagatc gcgccattgc actccagcct gggcaacaga
5101 atgaaactgt ctcaaaaaac aaaacaaaac aaaacaaaaa agagaagaaa ggtagaaaaa
5161 gaaaaaacaa aacagaaatg tcgttacacc ctaacttgag agtgttttct tttctttttt
5221 cttttttttg agacagagtc tcactctgtc gcctgggcta gagtgcaatg gcaatggtgt

5281 gatctcagct cactgcaatc tctgcctcct aggttcaagc gatcctcccg cctcagcctc
5341 ccaagtattg ggattacagg tgcctgccac catgcccagc taatttttgt atttttagta
5401 gaaacggggt ttcgccatat tggccaggct ggtcttaaac tcctgacctc aggtgatcca
5461 cccgcctcag cctcccaaag tgttgggatt acaggcgtga gccaccgcgc ctggcctcga
5521 gagtgttttc tatggaaggc tctgagcttg catcctgctg tctctttgtc aaaaagggag
5581 attagcagat gttagagaga tgttaagaat acaccagcat catctagaga ttttctttct
5641 ctctctcttt tttttttttt tttttttttt tggagatagg gtcttgctct gtagcacagg
5701 ctggagtgca gtggtccaac ctatagtaca tggctcactg tagcctcgac ctcccaggct
5761 caaatgatct tcccaccttg gcctcctgag tagcagggac tacaggcaca tgccaccatg
5821 gccagctaat tttcttgtaa agatgggatc tcactatgtt gccagggctg gtctcaaatt
5881 cctgggctca ggtgatcctc ccacctcaac ctctgtaact gcagggatta caggtgtgag
5941 acactgcacc tggtctcaag atttcttctt tcaagttatt gaaggagtga agaagaatcc
6001 tttttttctt ttcttttttt ttcatttgag acggagtctc tctctgtcgc caggctagag
6061 ttcagtggct caatctcagc tcactgcaac ctccgtctcc tgggttcaag caattctcct
6121 gcctcagcct cctgagtacc tgggattaca ggtgcccacc accatgccca gctaattttt
6181 gtatctttag tagagacagg gtttcaccat gttggtcagt ctggtctcga actcctgacc
6241 ttgtgatctg tctgcctcgg cttcccaaag tgctgggatt acaggtgtga gccaccgcgc
6301 ctggccgaga ttttcttctt caagttattg aaggagtgaa gaagagtcct tttctgactt
6361 tgtgattgat gtagtctgct gccaagcccc catgcatgta aacacagagc acctgataca
6421 cacgcaatga gaaacctggg actcacgaac ctccgagggc ccttccaacc ttgaccacca
6481 tggcttcgtc attcccattg gggtctgaag gtcctctttt tttttttttt tttttgagac
6541 agagtctcgt tctgttgccc aggctggagt acagtggtgc gatctgggct cactgcaagc
6601 tcggcctccc aggtttatgc cattctcctg cctcagcctc atgagtagct gggacaacag
6661 gcacccggca ccacgcccag ctaatttttt gtctttttag tagagacagg gtttcaccat
6721 gttagccagg atggcctcga tcttcctgac ctcgttattc gcccacctcg acctcccaaa
6781 gtgctgggat tacaggcatg agccaccgcg cccagcttct accagtcttg atacctccaa
6841 taatagctct cttgtgctca cctgcaagtt caggttcagg gaacagatgc ctgggagtcc
6901 atctttcatg caaatgactt tcttccagtg gttgggaaga tggcaagaat ggaacaggag
6961 agccgtggat tcaggctcgt ctgagttcta gtctcaatcc caccatttcc cacccgagac
7021 cgtgggtcag ctcacaaatt catacgtact gaataggtgc ttaaaatgtg ccaggcatta
7081 tgctagatga tggggaaatc atgataaatc agaaaggcac agccaggcac agtgactcac
7141 tcctgtaatc ccagcacttt gggaggccaa ggtgggtgga tcacttggag tttgagacca
7201 tcctggcaac atggcaaaac cccatctcta caaaaaattt aaaaaattag ctgggcgtag
7261 tgggatgcgg ctatagtctc agttactcag gaggctgagg tgggagaatc acctgagact
7321 gggaggcggg ggcttcagtg agccgtgatc gcattactgt atttcagcct gggtgacaga
7381 gttggaccct gactcagaac agaacagaac agaacagaac agaacagaac agaacagaac
7441 agaacagaac agtacagaaa acagggagaa ggaggggaag ggggagggga agggggaggg
7501 ggagagagag gaggagagga ggagagaggg acacatccct ggcttgtctt catggggctt

7561 atattctagc aagagaaatg cacatgatta tttaatcttg gttgtgataa atgtataaag
7621 gaacagtaag tggcatgaag gtaaaggata gggcattagg gaaagtttct ccaaaaaaaa
7681 aaaaatgaca tttcagctgc ttagtaaagg ctgtgcaggc attagcatta gtgaaattgc
7741 aggaagagga gaggaaggaa ggagaatgtg caaaggccag cctggcacgg cagctcacgc
7801 ctgtaatccc agcactttgg gaggaagtgc ttggattgag gcaccgaagc aggaagatca
7861 cttgaactca ggagtttgag accagcctgg gcagcacagt gagaccccat ttctaccaaa
7921 aaaaaaaaaa aaaaaattag ccaggcagga tggtgcacac ctgtagtccc agctagtcag
7981 aaggctgatg tgggaggatt actggatccc aggagttcaa ggttgcagtg agccatgagc
8041 atgccaatgc actccagcct gggcaacaga atgagacctt gtctcaaaaa caaaacaaac
8101 aaacaggtca ggtgcagtgg ttcatgcctg taatctcagc actttacgag gccgagacgg
8161 gtggatcacc tgaggtcagg agttcaagac caccctggcc aacatggtga aaccccatct
8221 ctattaaaaa tacacaaatt agctacatgt ggtggtgggt gcctgtaatc ccaggtactt
8281 aggaggctga ggcaggagaa tctcttgaac ccaggaggca gaggttgcag tgagccaaga
8341 ttgcaccact gtactccagc ctgggcaaca gagcgagaat cctctcaaaa caaaacaaac
8401 aaacaaagaa tatgcaaagg ccctgaggca gaaaggaggc tgcacatcgg gctggagagg
8461 acaggcaaga ggggggcaca gaggagctat aggacaagga tggagaatgg ggggaggcct
8521 ggtctcactg gccttgtgcg gctttgtgga catgttgagg acttaggatt ctgaggtttt
8581 tttctgtttt gtttttgttt ttttttgaga tggagtctcg ctctgtcgca caggctggag
8641 tgcagaggcg tgatctcggc tcactgcaac ctctgcctcc caggttcaag cgattctcct
8701 gcctcagcct cccaggtagc tgagactaca ggcatgtact accaaaccca gttaattttt
8761 gtatttttag tagagatggg gtttcaccat gccgctcagg ctggtcttga actcctggcc
8821 tcaagcaatc tgccctcctt ggcctcccaa agtgctgaga ttacaagtgt gagccacctc
8881 gcccggccaa gccacggaag atttcaaata agggagtgac gtgctctgat ttgcattttt
8941 cacagtcact ctgattgctg ggcagagaga atggagggca gggtggtgag agtgaatcag
9001 gtgacccgcc aggaaaaggt gcacgtactc atcggtgcca gagatgattg gggctttgag
9061 atggaggcaa atggacacat ttccgaacta attgagcagg cacgtctaca ggatttgggg
9121 gtgagggagg ggagaaaccc atggctgact cctgtaattt cttcaggcag ggtacttggg
9181 ttgggaggtc tgtttggagt tgtctggggc atggtgtggg gtgtcggcag gatacagtgg
9241 agatgagggg gaagttagtg aattggtggg gctggagctc agagccagac catcacagga
9301 gatggaggcc tgcgagtctt caggatctgt cactgaccaa agtcttgctc acggatgagc
9361 ctatcatctg tggaagaaag tgtggaatga gatgagaaca ggcctgggtg gaggccaggg
9421 aatcagcaga gacagccaag aaggccagag gctaggaggc aaaccccaca gtgtgcccct
9481 cctccccagg agccaagacc agagatggtt tcaagagagg gggccaggca caatggctcc
9541 cgcctgtgat cacagcactt taggaggctg aggctaaagg atggcttgaa accaggagtt
9601 taagaccagc ctagttaaca aagtgagatc ccatctctac aaaacaaaaa atgaactggg
9661 tgtagtggtg catgcctgca gtcccagcta cttgggaggc tgaggcagga ggattgtttg
9721 agcccaggag attgaggctg cagtgcactg tgatcatgcc actgccacag caagactatc
9781 ttaaaaaaaa aaaaaaaaga tcagagaggg gcacgacatc aaatcctcca ctttcttgag

9841 cttccagttg ctctgatgat gatcaaggct gtgaaactct cctttggcaa atgcatgagg
9901 ggtgactgtt gtgtctccga gccaggccct gggacccaac tctgggcagg tagcagctcc
9961 ctgtgtatac cagatgtgat agtaaaaaac tgacactggg ccgggcgcgg tggctcacgc
10021 ctgtaatccc agcactttgg gaggccgagg cgggtggatc acttgaggtc aggagtttga
10081 gaccagcctg actagcatgg tgaaatcctg actctactaa aaatacaaaa atacaaaaat
10141 taacagggtg tggtggcgga tgcctgtaat cccagctact cgggaggctg aggcaggaga
10201 atttcttgaa tccgggaggc ggaggttgca gtgagccgag atctggccac cgcactccag
10261 cctgggcgac aagagcgaaa aactccatct caaaaaaaac aaaaaaacct cctgggcccc
10321 agaagatggc aactgcctct tccagcctct tccacctgcc tgcctgtgca ctcctctgcc
10381 actctggacc tattccatgg tgccacgtgg tcaggcattg aaggaatggg tgggtttttt
10441 ttccatcacc ctcagatgct gctgctgggg ttggggcggg tcttccctgg ctgactctgt
10501 ctgcctcacc cactctctcc ccatctctct gtcaccacag gtctccaggg accaaattta
10561 gcccattttt cctccaagga ttctggaccc ttggccatat ttgcctctag ctttaggtca
10621 acagatttct aacatggagt caaaaaatct gtatgtttaa aaaaaaaaaa ttggctggga
10681 gtggtggctc acgtctgtaa tcccagcact ttgggaggcc gaggcaggtg gatcacctga
10741 ggtcaggagt tcgagaccac cctggccaac atggtgaaac cccgtctcta ctaaaaatac
10801 aaaaattagc tgggtatggt ggcggacccc tgtagtccca gctactcagg aggctgaggc
10861 aggacaattg cttgaacccg ggaggcagag gttgcagtga gctgagatcg cgccattgca
10921 ctccagcctg ggtgacagag tgagactctg tctcaaaaaa aagaaaaaaa aaaaaaaggc
10981 cgggcgtggt ggctcacggc tgtaatccca gcactttggg aggccgaggc gggcggatca
11041 cgagatcagg agatcgagac catcctggct aacacggtga aaccctgtct ctactaaaaa
11101 tacaaaaaat tagctgggca tggtggcggg cgtccatagt cccagctact cgggaggctg
11161 aggcaggaga atggcatgaa cccgggaggc ggagcttgca gtgagccaag atcacgccac
11221 tgcactctag cctgagcgac agagtgagac tccgtctcac aaaaaaaaaa aaaaaaaaaa
11281 attgaggcca ggcacagtgg ctcatgcctg tgatcccagc actttggccg aggcaggcag
11341 atcacctgcc atcaggagtt cgagaccagt ctcgccaaca tggcaaaccc ctgtctctac
11401 taaaaataga aaaattagcc gggcatggtg tcgggcacct gtaatcacag ctactggggt
11461 ggctgaggca gaagaattgc ttgaacccag gaggcgaagg ttgcagtgag ccgagactgc
11521 accattgcac tctaacctgg gctataagag cgagactctg tctcaaaaaa aaagcccttt
11581 acaaatatta actcgtttaa ttctcagaac acccctagga ggtgggtcct gctatcctgc
11641 cggttttgta gatgaggtaa ctgaggcaca gagaggataa gtcacgtggc ccaggtcttg
11701 gtcgcttctc agcctccatt tccgagagga gaaaacaatg ctccgaaagg ggaagtcatc
11761 ggcctgaggt caccagccgg gaaccggagg agccaggatg gggcccgagg tccatctgag
11821 gccagagcca gtgctgaccc gccagacagg aaatcagagc tggggactga ccgtgggccc
11881 cactcaagtg ggtggtgtgg ggtggtgacg ggggctgggg tgaaagccag gatgaggtgt
11941 cacattaggc tagggttcct gctgggcctg gcggggcaga gacccctggt tgatgcctgg
12001 caagtctctc ctcatttctg ctgtgctgtc ccctgggtgg ccaaggcgcc aggcccggct
12061 gtggatctga cacctcctgc tgccgagctc tgatcagaat ctggctgcag ggcagggggt

12121 gaaactgaat cccaccaggc ctcccctggc ctggatgtca cggcagacat cccaacccca
12181 cttcttggca ctgcctgacc ccagcagtgg ccttcttgga acctggagag cctgtgccca
12241 ccctgtggtg ccgaccaggc tgctgccttg gccctgggct ctgggctcca gggtccccag
12301 ggatcccagg cccaggctct tgcccctccc ggctctcagg tgagctgctg ggatcgggtt
12361 cctgcggttt catgtctttc attgttcttg gctggggccc ggcttatgaa actgtaatga
12421 ggggcccctg ctcccgaagc cggcccctct aaggtttcca ctgcctcggc ggctgctgcc
12481 accacagccc ggagtcgggg cctgggaggg cagcagtgtg ggggcctgag ccggagcccc
12541 ccggggacga gggtgctgac agtcgacagc caccaccacc accagagccc gcagcccagg
12601 taatccgaga ggaggcgggg cgcagggtgc catggccgct gtctcccacc accaggggag
12661 ggaactgccg ctctccagag gtggggagga attggggggg gccctggagg tgagcagccg
12721 ttcccccctg gtgagcccag caccagacca gccttcacag gtgtggcctt gccaaaggtg
12781 gagggagaga ggagtgggca gctggctcta gcccccgccc ttttcctggg gccttgaccc
12841 tccagccctg gatcccctgc ctctccttta gtggggcaaa tctcttctgt cctccaagac
12901 cttgctttcc catctcctct accatcaaac tgcctcttcc tccctcagac ctctcccttc
12961 tctcagccag cctaatcctt cctcctcgcc cttgtctttt ggccgcaaaa gaccctggtg
13021 gatccttcta tccacctcgt tctggggcgc acgcacatac atgcatgtat acatgaagtt
13081 gcactcatgc aaacgtacag atcggtgtac ccgcgggacc tgcgtagaca cgcatgcgca
13141 agagctcgct ccacctatgt gcacacatgt gcccccaata ccaggccttt atccacacac
13201 atacctgctc caaggcccat atgtgtacac tcacgtgtgc aggcacacac caggtacctc
13261 agccagccct cggcttgagt cccaaggggt gcttctagcc tgatgggcag ctccttgaga
13321 agtgcaggat cccccgagga gatgcctgga gactcccacg ccaccgggag ggctgtgca
13381 gggggagggg agcccccacg tgggcgctga gctgtctcct ctctccaacc ctggcccagg
13441 agctcaggag cccttcaggt gctgaggtgc cctactgcga cctgcctcga tgtccacctg
13501 cccctgagga cccactcagc gcctcaacct ccggctgcca gtctgtggtg gacccaggcc
13561 tcaggccagg gcccaagagg tgggtagagt cccagggccc aggaagggct tcatggggtg
13621 gaatgccctc acccttccca tttgcactcc ttccccaagg cagcacctgt ctctaatggc
13681 tgctggcatg ggtcacgtgg tcagtcctct aaacctaggc acaggtgggt ggtgggcatg
13741 ctccttgggc ccacaggaag aactcagatc acaccaccct gtttgcactt cagtggctga
13801 gcctcccctc cccagtcctg gctgtctctc ccaggggccc tgcccacccc ataacctccc
13861 aagaccgagc tcagctttgc cccagggtgt gtggctgctg tacactcacc ttgcagtggg
13921 catggccaca gcctactcac atttcctggt tccctttgca cagctttaaa agattaaaca
13981 gtccctgcac ccaggggcac ctgctgtcac ccacctacgt gggtgtgcac tgctccaggc
14041 tctggggttc cttccaccct caggcctgag tggcagtccc cctttaaggt catggtacct
14101 tgggaggcca tggtctgaga gtctagaaac ctgggttcca gcttcagatc catccctgat
14161 gggtcttgag caagttactc tgtctttctg tgtctcagtt tccctctctg taaaataaga
14221 gggattgaat taaatcagca ttgtgctggg atcattcatg tgctcctcac aggtaggcat
14281 attaaaacaa acaaacaaac aaaaaaacag cctgggtgca gtggctcatg cctgtaattc
14341 cagaactttg ggaggccaag gtgggcggat cacttgaggt caggaggtcg agaccagcct

14401 gaccaacatg gcgaaaccct gtctctacta actatacaaa aattagccag gcatggtggc
14461 aggtgcctgt aatcccagct gctcgggagg ctgaggcagg agaaccactt gaacccggga
14521 ggcagaggtt gcagtgagtt gagatcgtgc cattacactc cagcctgggt gacacagcga
14581 ggctccgtct caaaaaaaaa aaaaacaaca aaaaaaaaaa acaaaaaaaa acccacaaaa
14641 aaacgaaaaa aaaaacaaag acagctttgt tatcaagtat gtttcagaaa gtctgggata
14701 aatgaagttg aatagctttc ctgcaggact tgtcagagcc tttagcttgc ctgcgtgcat
14761 cgtggctttt gggtaacgga agtagagaag tatgtcattt gactacagag ctctttttta
14821 tggccatcac ttattatcat cgtgtaggcc agtctgtggg tcactctttg gggatactgc
14881 cagcgctaag ccttcttgag cctgtgactt cttttaaagc agagccttct ctgcagctcc
14941 ctccctcagc atctgggcta gtgaacccgt caatgcctgt ggagccatgc tgcctctcac
15001 atcctcgtta gccaagcatg agtctgtctt cttagactta ctgaacttgg ggtcagtaga
15061 ctaagactaa gacttaaagt tggggctggg gcaatagttt tggttcataa cacagctctg
15121 ccctttccta tctcggtgac cttggccagt ctcttgacct ctctgagcct cagtgtcttc
15181 ctctgtgaaa tgggaattat tattattatt attattttga gatggcatct tgctctatca
15241 cccaagctgg agtgcaatga cacgatgttg gctcactgca acctcagcct cccgagtagc
15301 tgggattcca ggcatacacc accacacccg actaattttt gtatttttat ttatttattt
15361 ttgagataga gtctcgtgct gtcacccagg ctggagcgca atggcacgat cttggctcac
15421 tgcaacctcc acctcctggc ttcaagcgat tatcttgcct cagcctcctg agtagctggg
15481 attacaggtg tgtgccacca ccatgcccat ctaatttttt tatttttagt agagacgggg
15541 tttcaccatg ttggacaggc tagtctcgga ctactgacct caggtgatcc acccaccttg
15601 acctcccaaa gtgctaggat ttcaggtgtg agccactgcg cccggcctaa aatgggaatt
15661 attatactta ctttaaaggg atgctgttgc tgggcgcagt ggctcacgcc tgtaatccca
15721 gcactttggg agtccgagac gggcggatca cgaggtcagg agatcgagac catcctggct
15781 aacacggtga aaccccatct ctactaaaaa atacaaaaaa ttagccaggc gtggtggcgg
15841 gcgcctgtag tcccagctac tggggaggct gaggcaggag aatggcatga acctgggagg
15901 cagagattgc agtgagccga gatggtgcca ctgcactcca gcctgggtga cagagcgaga
15961 ctctgtctca aaataaataa ataaataaat aaataaataa ataaataaat aataaaatta
16021 caaaaaaaaa agggatgctg ttttagctca atgaggcaat ccatataaaa agtctgacac
16081 agaggcagct ctcaccattg gctccctttc ccacaccagc gtgtgggccc caggctccct
16141 gtgtgagtga gtgcatatgc ctgggtgggg tgggggatgc tcctaactcc ccattacttt
16201 ttgggtctgt ctcctctccc aggggcccat ccccctcagc agggctccca gaagagggtc
16261 ccacagctgc ccccaggagc aggagccggg agcttgaggc agtaccctat ctggagggcc
16321 tgaccacttc cttgtgtggc agctgcaacg aggaccccgg ctctgacccc acctccagcc
16381 ctgactccgc cacccctgat gataccagca actcgtcctc tgtggtgagc caggagtggg
16441 agtttggggg acaagagtgg ctcacacctc catctgcagc cctgggtccc acctaaacct
16501 ccaggccagg gctgagcctc acaccaggga atccgacgag gtcctctgga ccattagctg
16561 gcagctcggg ccaccccggc gaacccctgg agagtgggga tttctgcaca ggctggccca
16621 gggcagctgg ggtctggctg tgaaaagaca gggaaaggag tttcagccga caggagctct

16681 gggtgaatca ccagcaggct cgggctgcca catgctctca gatcccagag tgttgggttt
16741 ggtgtctagc gcaagagtgg cacagggccc cagggccctg ccctcgacac acagtgctga
16801 gtcccatggc cagccctgga atagatttcc aagagacaga ggaaagggag caggctgcgg
16861 gggtcattta accacgaaaa gagaagagcc agcagtcttt cctccagcat ttgcattttg
16921 aggggctgcc gcagagcaga agagacaggc ttgtcccagg agacccccca aaaggaaaat
16981 gacagggggt ggtgataaga ctcagaataa aacaacccag cctcacacgc ttcatggcca
17041 gctccatgct aagggcttgg tgggtgtggg ctcaggggag actcaccaca gcctcagaag
17101 tggagatttc agtcgcccca tatggggctg aggctcagcg gggggagcaa cttagtggag
17161 gccacacatc tgggaagtag tggggctagg atttgaaccc acttttctct ggcccctggg
17221 gctacctcac acagtcccac atgcggaact ctgggactct gggcaaagct cttggcttca
17281 atggatgtac atgcctccta ggtccgttga agatgagcga cttggcactt agcacctgct
17341 agagcccagc acagtgccag gcaaacagca catgctttta atttaattga ttgattgaag
17401 actgggtgcg gtggctcacg cctgtaatcc cagcactttg ggaggccaag gtgggcggat
17461 cacctgaagt caggagatcg agaccagcct ggccaacatg gtgaaactct gtctctacta
17521 aaaatacaaa aattagcagg gtgtggtggt gggcacctgt aatcccagct acttgggagg
17581 ctgagacagg agaatcgctt gaacccggga ggttgcagtg agcttagatt gcaccattgc
17641 actccagcct gggcaacaag agcaaaactc cgtcttaaat agagaaaaaa aaaaaagttg
17701 attgattgag acagggtctt gctccattgc ccagactgga atgcaatgat gtgaacatgg
17761 ctcactacag ccttgacttc ctgggctcaa gtgatccccc cacctcagcc tcctcagtag
17821 ctgggactat aggcatgcac aaccatgcct agttattatt atttttttta attttgtaga
17881 gacagggtct cactgtgttg cccagcctgg aagtgctttt tactgaggcc atggagcgga
17941 gtctacacca cagaaggtgt ggcaccactc actgtcacct cttggggaca gaacttgaca
18001 gtttaggaag tgctttttat tttttattta attttattta tttttgagat ggagtcttgc
18061 tctgtcacct gggctggagt gcagtgccac aatctcagct cactgcaacc tctgtctccc
18121 aggttcaagc gattcgcctg cctcagcctc tcgagtatct gggattacag gcgcccacca
18181 ctatgcctgg ctagtttttg tatttttaat agagacaggg tttcaccatg ttggccaggc
18241 tggtttcgaa ctcctgccct caggtgatcc acctgcctca gcctcacaaa gtgttgggat
18301 tacaggcgtg agccactgcg ccctgcaggg aagtgctttg tttttttttg ttttgttttt
18361 ctttttgttt tttgagacag agtctcactc tgtcacccag gctggagctc agtggcgcga
18421 tctccactca atgcaagctc cacctcccag gttcacacca ttctcctgcc tcagcttccc
18481 aagtagctgg gactacaggt gcccgccacc acgcccagct aattttttgt attttttagt
18541 agagatgggg tttcaccatg tttcccagga tggtctcgat ctcctgacct cgtgatccac
18601 ccacctcgtc ctcccaaagt gctgggattg caggcgtgag ccacagcgcc tggccaggga
18661 agtgcttttt aatgccaatt cccttccctt ccttctgccc ctctcatttg gagagtatat
18721 gtcctgcagc cttttttctc ccgcaaacat actttctcct ccccttctct ggcaggactg
18781 ggacactgtt gagaggcagg aggaggaggc ccccagctgg gacgagctcg cagtgatgat
18841 cccgaggagg cctcgggagg ggccgagagc tgacagctcc caaagggctc cgtctctcct
18901 caccaggtcc cctgtgggag gagatgctgc aggccagaaa aaggagggtg agtccttctg

18961 ccaggttggt tcccatggtg atggcctggg gccccccaga tagccatctc actggccatt
19021 tgggactctg ggcacggctt actttggtgg cctgagtgtt aataatagta acagtttgca
19081 tgtcatgatg gagcagccac aatgagttat gctacccact ttaattatta ttattattat
19141 ttgggatgga gtcttgctct gttgcccagg ctagagtgca gtggtgtgat ctcggctcac
19201 tgcaaccttt gcctccccag ttcaagcgat tctcctgccc cagcctccca agtagctaag
19261 attacaggct cccgccacca tgcccagcta atttttgtct ttttagtaga catggggttt
19321 caccatgtta gcccagctgg tctcaaactc ccaaccttca gtgatctgcc catcttggcc
19381 tcctaaattg ccgggattac aggcgtgatt acaggcacgc ctagcctgtt attattatta
19441 ttattactat tattattttg agataagagt ctcacactgt tgcccagact ggagtgcagt
19501 ggtgccctct tggctcactg caacctccgc ctcctgggtt caagagattc tcctgcctca
19561 gcctcctaag tacctgggat tacaggcacc cgccaccaca cgtggctaat ttgtgtattt
19621 ttagtagaga cagggtttca ccatgttggc cgggcttgtc tcaaattcct ggctggggat
19681 tacaggagtg agccactatg cctggcctta gccaaggagt ttggacttct ctaaatagta
19741 gggagccacc gaaggttttt gaggaggaaa gtcatgtaat taaacctgtg cctcaggaag
19801 ttccctagat caaagatgca aaggatgaat tggagcaggg aggggttgaa ggatgagaaa
19861 ccagttaaga gactgttgca ggcactgtag caataactgg ttcaggcatg aatcactggc
19921 ggatgcaaaa tccctagggt gataatttgt cagcaaacag gatagtcatg gtttcaaagt
19981 attgtgtccg gaattggtgg gttcttggtc tcactgactt caagaatgaa gcggcagacc
20041 ctcgcgatat tacagttctt aaaggcggcg tgtctggagt ttgttccttc tgatgttcgg
20101 atgtgtttgg agtttcttcc ttctggtggg ttcgcggtct cgctggctca ggagtgaagc
20161 tgcagacctt tgtggtgagt gttacggctc ttaaggagac gcgtccgcag ttgttctttc
20221 ctccctgtgg gttcatggtc tggatggctt caggagtgaa gctgcagacc ttcgtggtga
20281 gcgttacagc tcataaaggc agtgtggacc caaagagtga gcagcagcaa gatttattgc
20341 aaagagcgaa agaacaaagc ttccacagca tggaagagga cccgagcggg ttgccagagc
20401 tggctggggc agcctgcttt tattctcttg tctggcccca cccacatcct gctgattggt
20461 ccattttaca gagagccgag tggtctgttt tgacagggcg ctgattggtg catttacaat
20521 ccctgagctt gacacaaagg ttctccacat ccccactaga ttagctagat acagtgtcca
20581 cacaaaggtt ctccaagtcc ccaacagagt agctagatac agagtgtgga ttggtgcatt
20641 cacaaaccct gagctagaca cagggtgctg attggtgtgt ttacaaacct tgagctagag
20701 acagagtgct gattggtgta tttacaatcc cttagctaga cataaaggtt ctccaagttc
20761 ccactacact caggagccca gctggcttca cccagtggat ctcgttctgg ggctgcaggt
20821 ggagctgcct gccagtccca tgccatgcgc ccacactcct cagcccttgg gtggttgatg
20881 ggactgggcg ctgtggagca ggggcggcgc tcatcgggga ggctcgggct gcacaggagc
20941 ccacggaggc ggggggaagc tcaggcatgg cgggctgcag gtcccgagcc ctgcccggtg
21001 ggaaggcagc taaggcccgg cgagaaatcc agcgcagcgc tggtgggcgg gcactgctgg
21061 gggacccagc acaccctccc cagccgctgg cccaggtgct aagcccctca ttgcccgggg
21121 ccggcagggc cagccggccg ctccgagtgc ggggcccacc aagcccacgc ccacccggaa
21181 ctccagctgg cccacaagcg ccgcgcgcag cctcggttcc cgctcgcgcc tctccctcca

21241 cacctccgtg caagctgagg gagccagctc cggcctcggc cagcccagga aggggctccc
21301 acagtgcagc ggtggcctga agggctcctc aagtgccgcc aaagtgggag cccaggcaga
21361 ggaggcgccg agagtgagcg agggctgtga gggccgccag catgctgtca cctctcagta
21421 tcacccctca gattctgcat cgattactga gagaaaggca cccttaggct ggggagagcg
21481 ggaagatacc actttcacca agaggctgct cagcatcccc agagtggggc aaaccaacat
21541 aatatgcccc ctgatatgat gcatggggtc aggggagggg atgctggggt gggcacagaa
21601 tcccttccct tctaaatcgt cctgctgaaa atgcattgcc tacacctaac cccagtgaaa
21661 catcagacaa acccgaattg aaagacattc tgcaaaatga ctgggctcct cataggacaa
21721 aaagcggcta ggggactctt cttgattaaa agagactaac aggatgagga cgtggaatgc
21781 catgtgtgag cccattggga tctgggacag gaggacacag agctgtgaca ggcattactg
21841 ggacaactgg ggcttgtttt tttttttttt tttttttgag atggagtctt gctctgttcc
21901 ccaggctgga gtgcagtggc gcgatctcag ctcattgcaa cctccacctc ctgggttcaa
21961 gcgattctcc tgcctcagtc tcctgagtag ctgggactat aggtgcctgc caccacgccc
22021 agctgacttt ttgtatttct agtagagaca gggtttcacc atgttgagcc tgatgggatc
22081 cgggacacgc ctatagtccc agttactcgg gatgctgagg caggaggatc gcttgaactt
22141 gggaggtgga ggttgccgtg agctgagatc gccccactgc actcccgcct gggcaacaga
22201 ataagactcc atctcaaata aataaataaa taaataaata aataaataag ggccatggtt
22261 gtggggtgag gaggggacag agaaggagag aggtttagga ggcagacttg atggtacttt
22321 ggacaggagg atacacagtc acagctcctg gcctgggtgt ctgggtagat ggtgtgccct
22381 ttgctgagag caggtggctg gggagcggat gggcccagcg ctggctgtgc tgtgtgggat
22441 ttgcgagccc tgaggaagag gttttgaggc ttcagatgct cagggaagca ggggatggag
22501 atgtggcatc ttcaactgag gagcaatgtt cactcaggca ctcagcagac gtttacttac
22561 tgcccacaag tcaatcatgg ctgaatagat gaggcagcaa tgagaacagc agcagcatta
22621 cagcagtgac ctgttttttt gatgttccag gccttgtgct aaataaatgt cttgcacaga
22681 atcactctgg gctccccgat ccctcactcc tggtctgcct ccttcctcac gggccactcc
22741 acctcgctct cctttgctga ggccgcctcc tctgcttcac ttttaaatat ctgatgcccc
22801 acatcccagt cctgggcccc ttctgtcttc tccctgcaca cttagctgct tggctttcgg
22861 tactgtctgg gaaaaaccat gactccggag tccacatctc tggccttgat ttctcccctg
22921 aacctgccaa tttatacaac ccagccctct actgcgttaa tcacacctca ggccccacac
22981 tgcactcact gtttcactca tccccccccg cccttttttt tttttttttt tttttttttg
23041 agacagagtt tcactcttgt tgcccaggct ggagtacaat ggcgcagtct cggctcactg
23101 caacctccac ctcccaggtt gaagccattc tcctgcctca ggctcccaag tagctgggat
23161 tacaggcatg caccaccacg cccggctaat tttgtatttt tagtagagac ggggtttctc
23221 catgttggtc aggctggtct cgaactcccg acctcaggtg atccgctgat ccgcctgcct
23281 cggcttccca aagtgctggg attacaggcg tgagtgaccg tgcccggccc actgctacaa
23341 tactattaac taagctacag acgtgatggg attcaccagt ttttctacta atatcctttt
23401 tctgttccag gatccattcc aggatcccac attgccttta gtccctctcc tcatctcctc
23461 cgtctcctcc aatctgtgac aggtttccca tctcttccag tttcccctga aggctggggg

23521 cacagcgatc tgctcacctt tgactttaaa aaaagtctcc ctggatgaac aatgacattg
23581 gccccagagt gcccaggctt gggggctact gcaaccatcc aggagagaaa tagtggtggc
23641 ttggagtagg aagtgggtgt agatgagaga aacaggtgaa tatggaatgt atttatttta
23701 tttttcgtag agatgggggc tcactatgtt gcccaggctg gtctccaact cctgggctca
23761 agcaatcctc ctgcctcagc ctcccgaaat gttggaatta caggtgtgag ccactaccag
23821 gcatgaaacg tattttagaa tgccatggga catcagccac cagcaatttt ttttgttttt
23881 tgttttctga gacagagtct cgctctgtca cccaggctgg agtgcagtgg ccagatctca
23941 gctcactgca acctccacct cccgggttca agcaattctc cagcctctgc ctcccgagta
24001 gctgggatta caggcatgca ctactgcatc cggctaattt tttttttttt tttttgtatt
24061 tttagtagag atggggtttc accgtgttag cagggctggt atcgatctcc tgaccttgtg
24121 atccgcccgc ctcggcctcc caaagtgctg ggattacagg catcagccac tgctcccggc
24181 tgcatccagc taatttttgt atttttagta gagactggtt tcaccatgtt ggccaggctg
24241 gtctcgaact cctgacctca gatgatccac ccgccttggc ctcccaaagt gttagcatta
24301 caggcgtgag ccaccgtgcc tggcaacctt gcaaattttg aattccctgc taggtgctgg
24361 gaactcagac tttcaccaac accagcccag agaggctgga cgaggcagtg gctgcaaagg
24421 gagaggacag gggatcttaa gaaggcgtga agggagggga tctgcatgaa ggcaactgtg
24481 gctacttttt tgtttcgttt tttgttttaa gacaatgtct tgctctgttg cccatgctgg
24541 agggcaatgg cacattcttg gctcactgca acctccacct cctgggctca ggcactcctc
24601 cggcctcagc ctcctaggta gctgggacta caggcacgta ccaccaggcc ctgctaagtt
24661 ttaaaaaact tgtagtagag acagggtctc gccatgttgc ctaggctggt ctggaactcc
24721 tgagctcgag tgatcctccc accttggcct ctcaaagtgc tgggattaca ggtgtaagcc
24781 accacaccca tagttgtggc tcctttaaga tgaggccatg atacccttta ctcttcacct
24841 aaagcagtag ccaagggaca tgtacatagg ggtgctcaac acctgcttgg tgaccgaggg
24901 attgagcggg gtggagggtg acacaccaag gtcatggcaa ctctgtcatg aggctaggag
24961 ttgcaaagag ggtcactact gaggacagtt acattaatcc tttaggccag tgcttctgaa
25021 actataaggt gtacaaatca ccagacagtt ttgttaaatg cagatccaga agcagtgcat
25081 ctgggctggg acctgagatt ctgcatttct ggcaaactcc cagctaccag tttataaaag
25141 gttgtccatc cctgcacctt tcctatcctc atggcagccc taggagaagc ctcagggtag
25201 gaacatttgg atacaaaaaa acaggggctt tctagagctg tcgcacagga aacaggctca
25261 ttagaacctg tctctactaa aaacacaaaa atgagctggg catagtggtg catgcctgta
25321 atcccaacta cttgggaggc tgaggcagga gaatcatttg aacccaggag gcttaggttg
25381 cggtgagccg agatcgcgtc attgcactct agcctgggtg acagaacgag actctgtctc
25441 aaaaaaaaaa aaaaaaaggt cgggcatggt ggctcacgcc tgtaatccca gcactttggg
25501 aggccgaggt gagcggatca cctgaggtca ggagttcgag accagcctgg ccaacatggt
25561 gaaaccccgt ctctactaaa aatacaaaaa ttagctgggc atggtggcgc atgcctgtaa
25621 tcccagctac tcgggaggct gaggcaggag aatggcttga acctgggagg cggaggttgc
25681 catgagccga gatcacgcca ctgcactcca gcctgggcga cagagcgaga ctctgactca
25741 aaaacaaaac aaaacaagaa atggggggaa ttccttggat tctgattgaa ggaaatagaa

25801 ctgggaaggg aagactgtca ggtggctgct gttgggccca cctctccctc cctggggtca
25861 cacaggccat tgtcaacact tctctccaca aatttgcttc ttctctgtct tgtccgtgga
25921 tccccagacc ctgtggcctt ccagttcttg aacccatctc ttgagtgagt cagggtctca
25981 atttcaaatt cttgacctca gcttgagaga ggcatccaag cccttgtcta atcagccttg
26041 gtcagagagg tggagtcact tggtacagac agtgagacct gtaggaggag ggtgtggggt
26101 ttgccctaat cactggtcct aagggacaaa gaaaggtaga atatgagagc tgcctggacc
26161 tctcccctta ccttgagccc ctctcttctc tctccagaca ccggcggtgg gggccggagc
26221 gcaggacagc actgggcaag gctccgggga gaaagcgggt tgtccctgga gcggcaccgg
26281 tcaacactga cccaggcttc ctccatgaca ccacacagtg gacctcgaag caccacgtct
26341 caggcttctc ctgcccaaag ggacactgct caggctgcct ctacacgtga aatccccaga
26401 gcctcctctc cccatcgaat cacccaaagg gacacctcca gggcctcatc cacccaacag
26461 gaaatctcca gggcctcatc cacccaacag gaaacctcca gggcctcatc cacccaagag
26521 gacaccccta gggcctcatc cacccaagag gacaccccca gggcctcatc tacacagtgg
26581 aacaccccca gagcttcctc tccctcacga agcacccaac tggataaccc cagaacctct
26641 tctacccagc aggacaaccc ccaaacttct tttcctactt gtactcccca gcgggaaaac
26701 cccaggacac cctgtgtcca gcaggacgat cccagagcct cctctcccaa cagaaccact
26761 caacgagaga attccagaac atcctgtgcc cagcgggaca atcccaaagc ctccagaacc
26821 tcctctccca atagagccac acgagacaac cccagaacat cctgcgccca gcgggacaat
26881 cccagagcct cctctcccag tagagctaca cgagacaacc ccacaacatc ctgtgcccag
26941 cgggacaatc ccagagcctc cagaacctcc tctcccaata gagccacacg agacaacccc
27001 agaacatcct gtgcccagcg ggacaatccc agagcctcct ctcccagtag agctacacga
27061 gacaacccca caacatcctg tgcccagcgg gacaatccca gagcctccag aacctcctct
27121 cccaatagag ccacacgaga caaccccaga acatcctgcg cccagcggga caatcccaga
27181 gcctcctctc ccaatagagc tgcacgagac aaccccacaa catcctgtgc ccagcgggac
27241 aatcccagag cctccagaac ctcctctccc aatagagcca cacgagacaa ccccagaaca
27301 tcctgtgccc agcgggacaa tcccagagcc tcctctccca atagagctac acgagacaac
27361 cccacaacat cctgtgccca gcgggacaat cccagagcct ccagaacctc ctctcccaat
27421 agagccacac gagataaccc cagaacatcc tgtgcccagc gggacaatcc cagagcctcc
27481 tctcccaaca gaaccaccca acaagacagc cccagaacat cctgtgcccg acgggacgat
27541 cccagagcct cctctcctaa cagaaccatc caacaagaga accccagaac atcctgtgcc
27601 ctacgggaca atcccagagc ctcctctccc agcagaacca tccaacaaga gaaccccaga
27661 acatcctgtg cccaacggga cgatcccaga gcctcctctc ctaacagaac cacccaacaa
27721 gagaacccca gaacatcctg tgcccgacgg gacaatccca gagcctcctc tcgcaacaga
27781 accatccagc gagacaaccc cagaacatcc tgtgcccagc gggacaatcc cagagcctcc
27841 tctcctaaca gaaccatcca acaagagaac ctcagaacat cctgtacccg acaggacaat
27901 cccaggacct cctctcccaa tagagccaca cgagacaacc ccagaacatc ctgtgcccag
27961 cgggacaatc tcagagcctc ctctcccatc agagccaccc aacaggacaa ccccagaact
28021 tgtattcaac agaacatccc cagatcatct tctacccaac aagacaaccc taaaacctct

28081 tgtaccaaac gagataacct cagacccact tgtacacagc gggaccgcac acagtccttt
28141 tcctttcaac gagacaaccc tggaacctcc tcatctcaat gctgcaccca aaaggagaat
28201 ctgagaccat catctcccca ccgctccact caatggaaca atcccaggaa ttcatctccc
28261 catcgtacta acaaagacat cccctgggcc tcgtttcccc tccggccaac tcagagtgat
28321 ggtccccgaa cctcttcccc atctcgctcc aagcaaagcg aggttccctg ggcatccatc
28381 gccctccggc caacccaagg tgacaggcct cagacatcct ctcccagcag gccagcccag
28441 catgacccac cccagtcctc ctttggcccc acccagtaca acttgccatc ccgggccacc
28501 tcttcctccc ataacccagg ccaccagagc acctcccgaa cttcctcacc tgtgtacccc
28561 gctgcctatg gggctcccct gacctctcct gagccctccc agcctccatg tgctgtgtgc
28621 attgggcacc gggatgcccc tcgagcctct tcgccccctc gctatttgca gcacgacccc
28681 ttccccttct tcccagagcc ccgcgcccct gagagtgaac cgccccacca cgagcctccc
28741 tatataccac ctgctgtgtg cattggacac cgagatgccc cccgggcgtc ctcgcccccc
28801 cgccacaccc aatttgaccc cttccccttc ctcccagaca catcagatgc cgagcatcag
28861 tgtcagtccc cccaacacga gccccttcag ctccctgcac ctgtgtgtat tgggtaccga
28921 gatgcacccc gggcctcctc cccaccacgc caggccccag agccttccct cttattccag
28981 gacctcccca gggccagcac agagagcctt gtcccttcca tggactctct gcacgagtgc
29041 ccccacatcc ccacccctgt gtgcattggg caccgggatg caccctcctt ctcatcccca
29101 ccacgccagg ctcctgagcc atccctcttc ttccaggatc cccctggaac tagtatggag
29161 agcctggccc cctccactga ctctctgcat ggctccccag tgctgatccc ccaagtgtgc
29221 atcgggcacc gggatgcacc ccgagcctcc tccccacccc gccacccacc cagtgaccta
29281 gcgttcctgg caccctcacc ttcaccgggc agctctgggg gctcccgggg ctcagcgcct
29341 cccggggaga ccaggcacaa cttggagcgg gaggagtaca ctgtgctggc cgacctgccc
29401 ccacccagga ggctggccca gagacagcca gggccccagg cgcagtgcag cagcgggggc
29461 cgcacccaca gccctggccg tgcagaggtg gagcgcctct tcgggcaaga gcgcaggtga
29521 gcccgggggt ggggtcagcc aggtgggctg gggaggaggc tcggcagcag gacaggtgaa
29581 gggttagggt gccaaaagaa agtgttcaaa tccccctggc ttgccattta ccggctgtgt
29641 gaccttgggc aaatcgctcc atttctctgt ttctgcatat tgggagtttt ctgttttgtg
29701 tgtgtgtgtg gttaagtgat gtcatgcaca gcccagtggt taacagcaaa gactgcagtc
29761 aggagggctg agtccaaatc ctgggtcaaa gccacatgtc aactcgtatg acctgatata
29821 gtgagttaac ctacctgagc ctcagtttcc tcatctgtaa gggaggggaa catgagcaca
29881 ttcattcaaa taagggtgaa ctgtcttttt atttttattt ttttgttttt tttttgagg
29941 tggagttttg ctcttgttgc ccaggccgga gcgcaatggt gcaatctcgg ctcactgcaa
30001 cctccacctc ctgggttcaa gcgattctcc tgcctcagcc tcccgagtag ctgggattac
30061 aggcacgtgc caccacaccc ggctaatttt tttgtatttt tactagagac ggggtttcgc
30121 catattggcc aggctggtct cgaactcctg acctcaagtg atccacccgc ctcggcctcg
30181 caaagtgcta ggattacagg agtgagccac cgtgcccggc caaaatgaga gtgaactctc
30241 aaaccctttg atccagactc atgggttttt tttttttttc ttccgtgcat gagggagaag
30301 aagaaagtac ccatgatttt ttcctgcctt ggcaaagaaa actcatgggt ccctatgcat

30361 ttcagaaaac agttatgtca atttaaaatg tatggccagg gccgggcacg gtggctcatg
30421 cctgtaatcc cagcacttgg ggaggccgag gcgggcggat cacaaggtca ggagatcgac
30481 agcagcctgg ctaacacggt gaaaccccat ctctactaaa aatacaaaaa attagctggg
30541 catggtggcg ggcgcctgta gtcccagcaa ctcgggaggc tgaggcagga gaattgcttg
30601 aacctgggag gcggagcttg cagtgagccg agatcgtacc actgcactcc aacctgggtg
30661 acagagtgag actccatcta aaaataaaaa taaaataaaa atgtatggcc agagctacct
30721 attttaagaa agaaagctca cagaattatg gatttttagc agtcaaacat cctgggacac
30781 ctgaagtcca ggtgtctttt gagtctcctt ggtcagcagc actggccccc tggaagggga
30841 gcatttcctc ctgatcaggc tgaggtttct ctccccagaa gttccagccc tcgagttttc
30901 aagaatcaga gctagaattc cattagttag ggtcaagaga acaacagcaa caaaaagcag
30961 tctggaagtc tcaaaacttt ccatgtatat aattcaacga atttttagtt tatttaccaa
31021 gttgtccaac cattgccaca atctaacttt agaacatttt catcacccta aagggtatgg
31081 aatattttgt atctaaaatt cctctttgct gctccttggg aaagttttct cctggatttg
31141 aatctgcatg agaaacctta tcagtcttgg ctgggtgcag tggctccctc ctgtaatccc
31201 agcactggag cttgcagtga gccaagatca tgccactaca ctccagcctg ggtgacagag
31261 tgagactccg tctcaaaaaa aaaaaaaaaa aaaaaaatta gccgggcata gtggcacacg
31321 cctgtagtcc cagctactca ggaggctgag gcagcagtat tgcctgaatt tgggaggtgg
31381 agtttgcagt gagctgagat tgcgtcattg cactccagcc tgggcaacaa gagtgaaact
31441 ccatctcaaa aaaagaaaaa aaagaaactt tatctcttgt tcttcaaatt tccttttcca
31501 gtaagcttga tcatttaata tccagatgac aaccgtgtgg tctccagcat tagtataatt
31561 ctttctttgg ccttaaaaac aaaaacccag agcagtttta ggtttacagc aaaattgagc
31621 agaaagtgca gtgttccatg cacagcctct cccactacca atatcctgta ctagataggt
31681 atgcttgttt ccagctcctg gcctcaaggg atcctccttt cttggcctct caaagtgctg
31741 gtgagccact gtactgggca gtagggtgat acatttgtta caatccatga acctacacta
31801 tgaaatcacc caaagtttag ggttcactct tggtgatgta cagcctatag gcttgaatga
31861 atgtatgaca agtatccacc attgtaacat catggagtag tttcaccctg aaagtcttca
31921 gtgtctttaa cttttttttt tgtttggaga tggagtctcg ctctgtcacc caggctggag
31981 tgcagtagtg caatcttggc tcactgcaac ctctgccttc tgggttcaag cagttctcct
32041 gcctcagcct cccgaggagc tgggactaca ggagcatgcc gccacatccg gctaattttt
32101 ttgtattttt agtagcgaca gggtttcacc ctgttgccca ggctagtctt gaactcctga
32161 gctcaggcaa tctacccacc tcggcctccc aaagtgctgg gattataggc gtgagccacc
32221 gtgcctggtc tgaccttttt tttttttgaa acagggtctt gctttgtcac ccagcagtag
32281 tgcagtggcg tgatcatggc tcactgcggc ctcaaactgc caggctcaag cgattctctc
32341 acctcagcct cctcagtagc tgggactaca ggcgtgtacc actataccca gctaattttt
32401 ctactttttg tagagatgga gtctcacttt gttgcccagg ttggtctcaa actcctggcc
32461 tcaagcaatg ctcccacctt ggcctcccaa agtgtctttg acctttttat gattactatt
32521 tcttgtctaa tgtcaatttt cctaactttg tcttaactaa taactcagca gggacattaa
32581 ggtctttaag ccaaagttaa ttaatttctt ttcctttttt ccaggataat ttcagagaga

32641 taaggtgttt atattaataa tagaaatgag aaatatgttt acacttttat taaccatttg
32701 tttttacacg atcactccta tacatttaat tctactctct ttgaaatagc ttatttacct
32761 tttagggaag ttttcataat tttactgaat aattaaaatt tttcctctta tctggcacct
32821 tttccaagta gaaaaccatt catcctctga atatggaaaa ttttaaaact gtgcagtgtg
32881 tgtgtaaggg acagactgga gcttatgagc gggtggctgt aggagtgtgc ttttattttc
32941 tcctatttcc tcatcgatat gtatttattg agcatctact ctgtccaagc attgtcccga
33001 gtctggaaaa tttttcttaa agggttgtca catcaaagca ttgggagttt tggcttgatc
33061 ttaatttttt tttccttaga tgcctttggc atctcttaga gtcacgtagc atcctgtggt
33121 catttgaatt caaaacctag ccttcactgg atttgctaga cttgatggct ttgctccttt
33181 aattctctct ggaggtcgaa gaacccatct aagcttccga aggaaacgtt tcctttgtca
33241 tgcttggtgc ggccacatca accctcggga acaggtggcc ccgaggagac gctgttgctt
33301 cagcatcgga caagccaggt ctctgttggg gtttcagaat cttcttgagt ccctcactct
33361 tttctctcag ttctacatgg gggaaaaaag gaaggcgagt tctcaggagg aatgctcatc
33421 aagcaagctg gagttcgtat ggaaaatgca tccatccgga agagaacaca caagtgcctt
33481 gcttccctgc accgggtgtt tccactattg tcctcctggt cctccccatt agggaggaac
33541 agcccactgg gtcacgtctg ggctcttgct tcatcgaagc tcctataccc cagttcgggg
33601 gaaaattcac tctaaggcct tcagctctca cagagtaagg aaacgcactc acctccaatt
33661 agattacaaa ttggctgggt gcagtggctc atgcttgtaa tgccaagact ttgggaggtc
33721 aaggcaggtg gattacctga ggtcaggagt tcaaaatgag accagcctgg ccaacatggt
33781 gaaaccccgt ctctactaaa aatataaaaa ttagctgggc atggtggtgg acacctgtaa
33841 tcccagctgc tcagcaggct gaggcaggaa aatcacttaa aaccagaagg cggaggttgc
33901 agtgagctga gattgcacca ctgcactcca gcctgggtga cagagcgaga ctccatctca
33961 aaaaaaaaaa aaaaaaaaaa gttacaaaca gcctgcccag ggagccctca agctcagaaa
34021 cacttcctcc agggaacttt ggttcctctc cgaaccagct gccaataact gctaacaact
34081 aacattattg tgctttatgg ctctgattct aatgacattg gcaaaagaca tgaaacaaca
34141 aaaaactcca accccatact ttttttttga gacaggatct tgctctttca cccaggctgg
34201 aatgcagtgg catgatcatg gctcactgca gcctcaacct cccaggctga agcgatcctc
34261 ccacctcagc ttcccaagta gctgggacta cagccacgta ccaccatgct cggctcatta
34321 aaaaaatttt tttttcctag aacaaggtct cattctgttg ccagggctgt ctcaaactcc
34381 tcgactcaag tgatcctctg acctcagcct ccccaagtgc tggaattaca ggtgtaaaga
34441 cactgtacct ggcctcccaa ccccatatat atatatatat attttttgag acagggtctc
34501 actatgtcac ccaggctgga gtgcagtggc gcgatctcag ctcactgcaa cctccacctc
34561 ctggggtcaa gcgatcctcc tgcctcagcc tcctgagtag ctgggattac aggcacgtgc
34621 caccacgcct ggctaagttt tgtattttta gtagagattg ggtttcacta tgttgaccag
34681 gctggtctcg aactcctgac ctcaagtgat ccacccacct cagcctccca aagtgctgtg
34741 attagaggca tgagccacca cacctgggct aatttttgta tttttagaga ctaggtttca
34801 ctatgttgtc taggctggtc ttgaactcct gagctcaagc catccgcctg cctcagcctc
34861 ccaaagttct gggattacag gcgtgaccca ttgcgccctg cccaatccca tacttttgaa

34921 ggtgtcgttc cagcatggct cctcaggcac aggcccacca cagacagcag ggcctatgga
34981 ggcctgagca gagtttcctc atacttccct ttttgctgcc tcactctggg tccaccacag
35041 ggtggccttt ttcaggcttc tcttcctggt tcagttcatc gaccaaagac atggctccat
35101 ttgctaactg agacccaacc aaaataaaca ttcatggagt ggttgggcct tttgttgtga
35161 ccccttaggt agaggaaggg agagatatgc ccaggttctg tttggtgttt ctacctgtca
35221 atagagatac cacgttaatc attaacagcc atgcctccca gttagtaagc aagacaacta
35281 actggaacta gactttttac acaagttaga gatgatcttc atcggatgcg tcaacaatgc
35341 ctgaagccca gtgtcttaga aacaacactt gcccagggtc aggcgtggtg gctcatgcct
35401 gtaatcccag ctacttggga gattaaggca gagaatcact tgaacccggg agatggaagt
35461 tgcagtgagc cgagatcgcg ccactgcact ccagcctggg tgacagagcg agactccatc
35521 tcaaaaaaaa aaaaaaaaaa aagacacttg cttggacagt ttatgttggc ctctctgaag
35581 ttgtcaggga gaatgtttct ttcttctgat cttgctcaga gctcttttgc caacagctag
35641 gatggcctgg agttttcaag gctaagcgct gggattctgt tggttaacag tgatgggagg
35701 gaaaaaggaa aagaaatcca aaatttacaa tatggacagg aaaaggcaga aagaatggaa
35761 cacgggggca cacagcagtc tggctctgtc gtcccacctc gtgggctgcg atcatgatac
35821 aagtgcttgg cccctggaac ataagaagca ctcaataaat gctttgtcat aatgattatg
35881 tgtgcccagt gcctggtaca cagtaggtgc tcaataaatc ttaattcccg caaagaccag
35941 tctaaccttt gtctgggggt ggctcttggg ctgctggtgg ggtggtggtt agtcatagga
36001 agtactggat gagacaggag agggccatgg ccagggccag atccccctcc ccacccaacc
36061 gcctctactc cccacttttc tcgtcatgtt cgcaagagcc tcatgaggca gaatagagca
36121 gtgttaacca tccccatttt tcagaagggg aaactaaggc cctgagagtc atcctcactt
36181 agggcccttc aacgagcttg cagtgggtcc ctggctgtcc cacatctcct cctgttggag
36241 gtgggagcag aggggctggg gtgctgggag tgggacagtc cctcagagga gtggctgcat
36301 ttgctcatag gaagtccgag gcagcggggg ccttccaggc ccaggacgag ggacggtcac
36361 agcagcccag ccaaggccag agccaacttc tccgaagaca gtccagccct gcccccagca
36421 ggcaggtgag cactgccagc tgtctggggc cccgcacccc aaggggcggc cactgcctct
36481 tccctcccgc tagaagccag gcagggggct tggacaggaa ggtcctctat gaaagggtcg
36541 gagtccaatc atggggatcc agctctcccc tctccccaac ggccagcagc tcttggcaaa
36601 atgcacaaga ccccacatta atcctttgaa gatagcccct ctcccacgct atccccttcc
36661 tttactaagc actgctgtct tttttttttt tttttttttt tgagatggag tcttgcactg
36721 tctcccaggc tggagtgcag tgggacaata ttggctcact gcaacctctg cctcccaggt
36781 tcaagtgatt cttctacctc agcctcccaa atagctggga ttacaggcgt gtgccaccac
36841 acccaactaa ttttcgtact tttagtagag acagggtttc accatgttgg ccaggctggt
36901 cactcctgac gtcaagtgat ccgcccacct tggcctccca aggtgctggg attacaggcg
36961 tgaaccacgg cacctggcca atactgctgt cttgagcaga tttcaggaaa agccttgaag
37021 caagaatctg gctctaaggg ccctctcttt cagggtaatt tgtatcttta accaaaccca
37081 gagggctagg cccgcctcag tggaatgggg tattggtggt ggaggttctg gcacctgata
37141 gcccaagttg gaatcagtat ctttgagagg ggtgagaagc caagaacggg ggcaggtctt

37201 tgcagatctt tttctggaac tgaggaaagc gggtgaaagg gaggagctga ccccggaggg
37261 gtggggcaga gaccctgtgt actttttggc ctgctttgat tcaggctgct gtgtggacac
37321 agccttgact ctctgggggc ctaagaaagg cagagctggc agccaggtcc ccagagagag
37381 agcctcacct accccctcac ctcatcccca ggtgaccatg ctccctgcca aacaggcaga
37441 actgacccgg cggagccaag cagagccccc tcatccttgg agtcctgaga agagacctga
37501 gggagatcgg cagctccagg ggtccccgct gccccccagg acatcagcca ggacccctga
37561 gagggagctg cggacacaga gacctctgga gagtggccaa gcaggcccaa gacagcctct
37621 gggggtgtgg cagagtcagg aggaaccgcc agggtcccag ggccctcata gacacctaga
37681 aaggagctgg agcagccagg agggaggcct gggccctggg ggctggtggg gatgtggaga
37741 gcccagcctg ggggcagcca aagccccgga gggagcatgg gggggcactt ccagggagta
37801 caaggagagc tgggggcagc cagaggcctg ggaggagaag cccactcatg agctccccag
37861 agaactagga aagagaagcc cactcacgag cccccctgag aactggggag gccccgcaga
37921 gtcctcacaa tcctggcact ctgggacacc cactgctgtg ggctgggggg cagagggagc
37981 gtgtccatac ccgcgtggct ctgagaggcg acccgagctt gactggaggg atctgcttgg
38041 ccttctccgg gcaccaggag agggggtctg ggcccgtgtc cccagcctgg actgggaggg
38101 cctcttggag ctcctgcagg ccaggctgcc ccgcaaggac ccagctggac acagggatga
38161 cctggccagg gctttagggc cagagctggg tcccccaggc acaaacgatg tccctgagca
38221 ggagtcacac agccagccag aaggctgggc cgaggccacc ccagtcaatg gacacagccc
38281 cgcactgcag tcccagagcc cggtccagct gcccagccct gcctgcacct ccacccagtg
38341 gccaaagatc aaagtgacaa gaggaccagc gaccgcaact ctggcaggcc tggagcagac
38401 gggccccctg gggagcagga gcactgcgaa gggccccagc ttgccagagc tgcaggtaag
38461 gaggtttcca ccctcccaag ggtagccaga aagtcagccc cactcagcca agtctccttg
38521 gaaaagcctc cccttggagt agcctaagct cctgcatccc cctccaggct cagacctcag
38581 cctccctgct gaccacaacc catcccgatc accaaactgg cccaacaatg atgatgagca
38641 acacagtttt aagcgtttat gggccaagcc ctgatctgaa tgtttgacgt gtgcttttcc
38701 catttagtct ccttagcaac caggtgagaa atacactatt tacagataag gaaactgatg
38761 cgcagggtga agccacttgc tggtggggca tgggcaaaac ccaggtctgc tcagtgcaga
38821 ccacactgag gcctcaagca agatgctctg gccagggcct attcccctcc tggctccacc
38881 atacctggtt tatgttgtgt ctggtggcac acacctgacc ccacctggga ctgtagccag
38941 tgtgggcatg ccccttcccc tggtggtact caggtacctg agtccaggac agcctcgctt
39001 accctctctg gcccctacag ctcctctcag acacagagaa gagggtgcat caatgctcac
39061 tgggttcagc tagctgccaa gacaggctgc ttaaatgagg gtcgtgtgca aagtgggccc
39121 agcgccgggc acacagtagg tgctcgccct tcccctgctt cactggtgct ttggtgctgc
39181 cttgaagccc agaacattct tcatctgaag atggtgggca gggagggtct tggaggccag
39241 aatcctgcac cctggcaata gctcattggg aggacggcat gtggttttga acaccagcct
39301 ctctctctct ctgtaagatg gaccaaccac ttctctgacg ggcagcgctg gggtgccgtg
39361 ggggttcctg cttgtttctc caaagcctcc tcatctccta gatgcaaagt ccaacgccac
39421 ctgccccagg atgagcaggg cttccaggca tcagggtgca ccccgtgctg ggcccagggc

39481 cttgccatat gtgcctcaag tggataggag gcagctgcca cttgacagat ggggaaactg
39541 aggctcagac tccttaggca tttgctgggg gtcacacatg cataggtcgt gagaaatgag
39601 atcaaagcat ccactccaaa gtttgtgctt ttatttttat ttatttattt acttattgag
39661 acggagtttc gctcttgttg cccagactgg agtgcaattg cacaatctca gctcactgca
39721 acctccgccc cccaggttca agctattctc ctgcctcagc ctcccgagta gctgggatta
39781 caggcatgtg ccaccatgcc cggctaattt tgtgttttta gtagagatgg ggtttctcca
39841 tgttggtcag gctggtcttg gactcctgac ttcaggtgat gcatccacct tggcctccca
39901 aagtgccggg attacaggtg cgagccacca tgcccggcct aagtctgtgc ttttagccac
39961 cacgtcacaa agctcctgag cccatgggag gactggagaa ccctctcccc tgtatttccc
40021 tgtggctttg atgagttgac cctccaatca actcaatgcg tggacatcag agaagcaggg
40081 ataagtcagc tcgtcctgcc ctcagattgc tgtcaggctt gttaggaagg cacacagaca
40141 gctcaaggag gacacggtgc tgtgtcccgg ggcatgctgg gtgccacagg ccggccaggt
40201 caagaaggaa gttagaaaga ttcttcagtt tcatggtgaa gctcagggtt ctcaggaggc
40261 taggagatgg tggctgggaa gggatgggag caccatgtac tgaaaggaat ttcacagatg
40321 acaaaacgag taaccaccag gtcctgctgg ggctccagct gctcctctct atgaaaatcc
40381 ttggcttggc acagctgggt gcagtggctc acatctgtaa tcccagcact ttgggaggct
40441 gaggcaggca aatcacgagc tcaggagatc gacaccagcc tggctaacac ggtggaaccc
40501 cgtctctact aaaaatacaa aaaattagcc gggcgtggtg gcgggtgcct gtagtcccag
40561 ctacttggga ggctaagaca ggagaatggt gtgaacccgg gaggtggaag ttgcagtgag
40621 ccgagatcac gccactgcac tccagcctgg gtgacagagc gagactccat ctcaaaaaaa
40681 aaaaaaaaaa aaatccttgg catggcactc aaggccggct tcccttcctt ccactccgga
40741 tttgcctcat tggtgctgag ctggctgcag ggcctgtgag cacatatccc tgcctttgca
40801 catgctcttc cctgttcagc caccaaacgc ctgttcattg tcgaagtctt aggtcaggct
40861 ccctgtcctg agaagccttc cctgaacagt cactcctagt aatggtcccc ttctgggctt
40921 ggcagttttg cttccatttg tccatgcaac atccatctaa tagggttgtc atttccccat
40981 aagctccaca caggcaggat ctctacctgc tttatctctg taactctagc tctggcccag
41041 ggcctgacat aaaggagggg acagctcagt ggatgcatgc aagatggagg acacaggggt
41101 cagtgggtga acaatgaatg aatggagggt gaatgtgtga atggatgatg gatcaatgat
41161 gcatgttgaa tgaataatag acatcttttg gatggatgac agatgggtga tgatggatgg
41221 atagctgggt gatgaatata tggtggtggt ggatatatga tggatgttgc ttaggtgatg
41281 gatggatgaa ctttgggtgg aatatgaatg gatagatgtg gcatgggtgg atgaatgtca
41341 gatgatagat gttggagata tgggtagatg gatgatggat ggagagaggg atatgtaggt
41401 attgggtaaa tggatatgtg atggatcaat ggagggtaga tacatgaatg ggtgatagac
41461 aatggatgat gggtacatga gagggtgagt catgtgatag acactggatg gttggatgga
41521 tacatggatc tactgatgct ggacgttaga tgggtgttgc atggacagat gatagaatgg
41581 atggatgagg tggacagatg catgcatcct ggagaataag ttgggctaag ctgtgttctt
41641 tttttaatct ccagttccaa ccagaggagc ctgaggagtc agaaccaagc agaggccaag
41701 accccctgac tgaccagaag caggcagact cggtagctgg gtcatgggtg tgggtacata

41761 cggtggggaa gggaggggga agcaggttgg agatgggctt cattcctcag aagagcccta
41821 gaatcaacca gaccaaagtt ggcattgcca tggggtcatc tatgtgcatg gtggggcctt
41881 aagaagaggg gctctgcagg agccaccttc tggcttgggg acctgggtag gtgggcgatc
41941 ctggggtcag aaccctaggc catgcttctc atttgctggg actgaaaagg tcaatgtctt
42001 tccctgcgca aatgatggtt tggccccagt ggtctctgtg ggaagagatc cttccggttt
42061 gaaagggcca ggccaggctg aaagagcagg gcttccacga gcaggagcca gagagctgag
42121 gatgtaccca gtcagccagg cctggagggt ctccagggtc ctggaggcgg gagagatatg
42181 tgcgggggtg ggacccacgg atgctgctct gaggcctggc tgcagctggt gagcacaact
42241 ctgcccgccc ctccccgtga cctaccaaac agactgttct cgtcaccctc agcctccaga
42301 gaggtgagat cagagttgag ggccgtgggg cagtgggagg tgggggagga ggagacagac
42361 tgtttggggc tgtgttttcc ggtcctgctc ttcctcccgt tgcctcctga agccaggggt
42421 ggcagggagc gtccattggg cagcatagcg ccccccaagt agggccctgc cgtgagtcaa
42481 agctggggga attctgccca aaccctcact gtgcagatga ggaaatggag gcaggggagt
42541 cgtcccaggt tatacagtca gtggctagct ggactgggac tagaacccag ggcccaggaa
42601 ccacccagcg ctctctctgc acctccacct ggcacccatg ctgcccccgt ccctgccccg
42661 atttcttgcg tggcttcgaa cggcagtgct gtaacttcga gcagtccagt cttcccatct
42721 gtggaatggg cggggacagg ggacactggt cgctaaaggc gctgccagcc ctcccaggga
42781 ctctgacaac tttgctcttc ctctttctgg gttatttctt catatcctgg agccagagta
42841 gtgaacgagg cggaaacagc aatgccgggg tgcccctcta gtctccatga aacgggcccg
42901 gcccatcaac gtggaaaata tctcaaccta cccccacccc taggcagcct tgactggtca
42961 gaactgataa ccacctcatt catgcattct ttcacacaca cttagtgagc accttctgta
43021 tacccaggtg tgggtcacag gtgagtcgga gcgccatcaa agagttcagt ctagtgagat
43081 ggttgcatgc ctgtggtcag gactgtgatg gggaatgtgg tagcagggtc ggggcaccc
43141 ctacccccaa agccctcctg gacctgtgct ttgatgcttc aagttccact tccaaagagt
43201 ctgcagattc cagagcgagg accctgggct ctctcctacc ttcccagtaa cccatgtgtg
43261 gctttggaca agtgcctctg cttcacaggg gccttggttt ccccaacgag gatgactcca
43321 aagagggagc atttgggggc tctgctagga atgtgtgtag aagtccacaa ctcattccct
43381 ttgcttaagc tggggtcaca aacctaacgc cctgaaggcc aggcaggtaa caatggtgag
43441 aaggtggggg ggcaccgagg gcacttgtgt gactcctcgg ttccagatag ctgttgccat
43501 ggggaagacg ggcccagagt ttccacatct ttcgatattt caagaaacac tggagatatg
43561 tatttatatg tgaaatctct aacctaatgt gaaatactct gaaaacacta tgtgggcaga
43621 aacatacctg tggtctgcac cccctccagc tggcgcccgt ggtttgactt tagggtgatg
43681 acgtgcacac cttcaaaggt gctgagccct gggagccttg tcagtccaac acgatgtagt
43741 gtgaacaagt taaccagctc gtctcggcag ttctgggagg agagaaagat gggaaccctg
43801 gggctccatg gtggggggca ccacagaatg ggcaggggag gctgtagcca ggggtgcccc
43861 catcctgcca aagggacctg gggccaacac tggaccctgt ttgacaggca gacaagaggc
43921 cagcagaggg caaggctggg agcccgctca agggccgact ggtgacctca tggcggatgc
43981 ccggggaccg gcccacgctg ttcaatccgt tcctgctgtc tctgggggtc ctcaggtggc

44041 gaagggtagg ctggctccag tggggactgg aggggtgagg gtggatagag acggggatgg
44101 gagggaggag ggggctgtta agcaaaggag agagagtggg ggctgaggag gaggcaccta
44161 gggccgtcgc atgacaggag aggtgggagc agagctctta agctcatcct ggtctcagca
44221 agcgagtggg tttcccactt ccggggcagg cttgggggct gctttcagct tttggagtga
44281 ggggtgatgg aataatctac acagctgaaa gcttccaatc agcagcctct cgccagccct
44341 gggccaacgg ggagccaggt atgggggaag ggaaggcgag cttataagtg gctctgggat
44401 tgcaaagtgt gtctcagacc tgggcctgag ccagtttcac aaacatctgg cgttacgtaa
44461 cccatcccct gcccagagag gcctggaaac actcaagcct ctggctgggt tttccacaag
44521 ggcaggcagc tgagacccgc tccctcccac ccgcttcaca gcaggacaga aggggcctgg
44581 ccatctccag cagccacagg ctggctcatg gggtctcttc tctctttgag aggctgactc
44641 tcttactcac cgctgttggc tctgagtcca gcactaggct gggcacctca gagacagtgt
44701 gcccatttta cagatgaagg gactgaggcc cagggaggtg agctgacttg cctgaggtcg
44761 cacagcttgg ggtataaagg agctgatttt cctcattctc agatgagaga agggttgtag
44821 ccaaacttac tgagcctcgg ttgccacatc tgtctattag gaaaaagtct gtggagactc
44881 agggctgcag agagggctca gggagatcag gatgcatgga ggttgcgggg ctgttgtcga
44941 aacactaact ttcccatcca cttcagaatt tccttttttt aaaaaattaa gacggagtct
45001 cgctctatcg cccaggctgg agtgcagtgg caccatctct actcactgca acctctgcct
45061 cccgggtcca agtgattctc ctgcctcagc ctccagagta gctggaatta taggcgtgcg
45121 ccactgcacc cggctaattt tttgtatttt tagtacagac agggtttcac catgttggcc
45181 aggctggtct tgaactcccg acctcaggta atccgcccac cttgacctcc aaagtgctag
45241 gattattggt gtgagccacc acgccaggcg tttttttttt ttttttttta aattaagata
45301 gagtctcgtt ctacccccag actggagtgc agtggcacca tctcggctca ctgcaacctc
45361 tgcctcccag gttcaagcga ttctgccatc tcagcctccc gagtatgtgg tactacaggc
45421 gtgtgccacc acacctggct aatttttata tttttagtag agatggggtt tcaccacgtt
45481 ggccaggctg gtttccaact cctgacttca agtgatccgc ccgccctggc ctcccaaagt
45541 gctgggatta caggcgtgag tcacccagct gagcccagaa tttcctttcg ctgctctttg
45601 tggttaaggc accgagggtg agcaggcaag catggttatc tgagtagtgt ggtaccttta
45661 agccatgggc aggaattacc ttgtctaact aaaggtcacc aacacacagc acgtctggca
45721 ggagggggca ggtaagtcct tggtagagcg aaggaggagc gaagcaggga aagcactctg
45781 gggtaatgat gctggctaac cagcacttat tgtatgccta ctacatgccc agctctgtgt
45841 tcactgcatg gcgccaccta attgcatttg atccacactc ccaggtgcag ttagacgcct
45901 actgtccatt ttacagatga tgaagtgaag ctcagagagg gagggtcact tgctccaggt
45961 cacacaggga gcaaggggca gaactgagat tcagaccctg ctctggctga cctcagagcc
46021 cagtgcttcc aacacgcccc ctacaccact gccttcacat cctgatctct ctgagccctc
46081 ctgcagctct gggagtcccc acagattttc cccattagaa agatgcggca accgaggctt
46141 ggtgaggttg gctacagtgg ttctctcatg tgggagtgag ggaaatgaag agctagggat
46201 ggaactgcca gcagggcctc ccagttacac agagcagatt taaacttctc ttgggctgaa
46261 gcagtgaaat tagcccaagg ccaagagcca gaaagcctgg agtcgagttc tggcttggca

46321 cctaacttgt gtgcccttct ctgagcctta ccttattatt ctcagtagag taacggtttg
46381 agactggata acctcgaagt ttggtagctc tggtctttgc cagtttaata actcacccct
46441 gggtccctga ggctgtgctg tctctgacct gccaggatga gttgtggttt cttcattggt
46501 gcattcattc attcgagccg tgcttacgga ggatgtgctg tgggcttagg ccctaggctg
46561 gtttccaaga tgtagacaaa aaacattcca gccctggcca ctggagccca gttaggtaag
46621 gacactgaca tgtaactgat gagaacagtg tggtcaccac tttagtgggg aaaggtacag
46681 atgtagggag cactgggcca gcagttccta agtctgctgg ggacactttg gcttcctttg
46741 gtctgagact tcaaggaaga aattttccag gccaacaagc attgggaggg ggaaggggag
46801 aaagagagag agaatgtgtg tgtgtgtgtg tgtgtgtgtg tgtgtgtgtg tgtgtgtgct
46861 gggtgtgtgt gagtgtgtgc tgggtgtgtg tgtgtgtgtg tgctggggta cagaagacag
46921 gaagagaagt gttccaggca cagcagagaa ccatcagggg gtagaaggac ctgtcttatc
46981 ctgggaattc ctagtagtcc agtgctgtgt atcttttttt tttttttttt ttctgagatg
47041 gagtctcgct ctgttgccta ggctggagtg gaatgcagtg gtgtgatctt cactcaccgc
47101 aacctacgcc tcccaggttc cagcgattct cctgcctcag cctcccaagt agctgggatc
47161 acagccatgt gccaccacgc ccggctaatt ttttgtattt ttaccagaga cggggtttca
47221 ccatgttggc cagcctagtc tcgaactcct gacctcaggt gatccacccg cctcggcctc
47281 ctaaagtgct gagattacag gcataaacca ccgcgcccag cctccagtgc tgtgcatctc
47341 tttatttact tattgtattt tgcaaaattg accaaagtat gtgtctctcc agcctgtcaa
47401 atgagaatct ggaaggccag gatgacaggc aggagccagg ttgtgatggt tcctgactgc
47461 ctagaagagg aatgtagcac tgatctgtca gcattggtga tgtgtggaag aactttgagc
47521 agggaagtgg tctggtcact gtgttgcggg tactaggagg agtcgcaggg gccagaaaag
47581 cctggaggag gctggtgctg ttaggtgccc agggcactgg accaggaccc tggtggtagg
47641 tgaggtctcc tgcgtgttca ttcactctcc caaggactga ctgcctgagt gaggtgcaag
47701 ggtagggtga gaaaactcag cccggggtgc cagaaggcca gtttctagag ttagatggac
47761 ttaaccttgg cctcattcct catggtggag agactcctgg cagcaaatgc tgggaaagaa
47821 cttcagctcc ctgggcctcc atttctttat ctgcaaaatg ggaacagtaa ttataaaagc
47881 aataattttt ttttttgaga gggagtctcg ctccgtctcc caaggtggag tgcagtggca
47941 caatctctgc tcactgtaac ctctgcctcc cgggttcaag cgattctcct gcctcagcct
48001 tttacatagc tggattacag gcgcctgaca ccacgcccgg ctaatttttg tatttttagt
48061 agagacgggg tttcaccatg ttggccagtc tgctcttgaa ctcctgatct caatgatcca
48121 cccacctcag cctcccaaag tgttgggatt acagacctga accaccgcgc ccggcaaggg
48181 caataatttt tgaaccccaa atcaagatga cagaataatt gaaatgccaa ttattccaag
48241 cttccctgcc tccttgccta aggatagcta atgagggata aagaggttgt cctaggtgcc
48301 ttacttgaat taacacttaa tccttccagc aacctcatga ggtaacagca ttaccccatt
48361 ttacagagag tgaaattgag caccgagagg tgccttgctt aaggtaacac taggctggaa
48421 tccccgtgcc acacgaactt cctagtgcat ttagcaacca attccaccac ggtccctact
48481 cacctgcagg aaaagagcat ctaacaaacg gcccagcgtt ctgggaacct gagcaacggg
48541 tgcacgccca cacctgaaaa accccaaacc aaaaaagacc catttgtgag gagtgggcat

48601 ttgggagaga ccctgccatc caagaactca ctttgtgtcc agcttactcg ctaggaattc
48661 cgcctcttcc tgtgtgacct cgggcaaatc ggctcacccc tctgagcctc cgttttctca
48721 ttagcaaaat aagacctccc tcccaagtct gtggtgagga cgaaaatcga ctcttggcga
48781 gtgggacccg ctgagtcgtt agtactgggt gctttgcccc ggtccccgcc aaatcacggc
48841 gccgagccct agcggaccca tcgctccggg gccgggtcgg gaagcgcagc ctgggcgagt
48901 cgtgcccacg cccggccgcc cgcatcctcc gcccccgcgc cattgccgga gtcacctgca
48961 ccctgggccc ttccttcctc ccagcgcgcc cgcggcccgg gcccgcccgc cctccggccg
49021 ccaccgccct cccttccctg cggccccatc cgcccacccc gccgtcccgc cgtcccgccg
49081 ccccgccgcc ctagcgcgcc cgtcccgttg cggcaggtcc cgcccccggc ccgtctcgcg
49141 cttccattgg cccgccgcgg cccccgccgc tgtttgtctc ggcttccccg ccacccattg
49201 gcccgctcgg gtcctcccag gaagtttgaa aaaaaaaaaa aaaaagtttt atggcggat
49261 ggaaggggcc ggggcagcgt cggggaaagg aagggccgga ggcgcggcgg cgggcggccg
49321 agaggggcgg cggcggcggc ggcggcgggg ttcccgcgcc gcggagcccg gcccgagagc
49381 cgcgtccacg ttcctgcctc ctgctcccgc cgccctgggg cgccgccatg acggtgagtg
49441 ccccagtcag ccgcccgcgc ccgaggccgg gagaagggcg acgacccgag gcagagccca
49501 gcctctcccc tccggggcag ccccctcctc atttcccgaa ggcgagagga gggctgggcc
49561 ggaagacggg gccacctccg aagcgctttc ggccagaccg gcctcaccct tcccgcaggc
49621 tcctcctcgc cttccctcgc ccactcgccc ccgggagccc aggcgaccct gaggggacgg
49681 aacgacccgt ctcgggcgcc ccctcctcac acttgcggca ccagtcacgg gtggattcgg
49741 ggcgaccctt tcccacctcc cctccagccc cctagacgaa gacccgattc cccaaggcgg
49801 ccaggggccc cagggaggag cggaggtggg cctgggctgg ggaggggcgc tcgtgggtgc
49861 ggatgggtcg cgctgacccg ggcctgggca agccagccct ccgtgtggcc gctggcccga
49921 ggtcgggtgg gcgagaagtg gccggaccgc cccacccaca gcccgggagg cagtcccggg
49981 acgtctgaag cagcgggata ccacgtcttc ctgggcttgc actttgcagc ggtcctgggg
50041 ccttgggaca agcctgcgag tctgagtagg gggcatgatg gcaggggttt gcctttctca
50101 tgggggtgag ctgtacccaa gccccatcaa ttacctgtgc catagttagc atattgagcc
50161 tcacagctgg gttcgctgga ctccccactt cacagccaga taaactgagg cttggggagg
50221 ggcgctggtg agttgcccga ggtcacccca agtataagag aaggagtagc tcctttccct
50281 gtccttctgg cccactgacc gtgatgttcg ggaagggagg ggtggggctg cttttggttt
50341 ctggctagtg cttcagggag gctctgacag cccagtggcc ctcctggggg aggatgagca
50401 gggtgtgtgt gtgcacaggc ggcagtaggc ctctttcagg ggagtggaga ccctgcggag
50461 gagtgggtgg gctgctgggg gcgggctggg ctgctggggg gcgtggcacg cctagcccct
50521 gccctgtgag tgtgatggcc tggacttcct cggcgtcctc ctggacatcc tgccacctcc
50581 ctgctggccc ttcctgtcgt cagcagcctc cccttttctg tcaggagcag aagggatgtg
50641 gggggaggag ctgagtcagt gtcctggggc caggaggaga gaagttgcct tgcccaccgg
50701 gtgcctgccc ggccctcagc cgggtgcttt cacgttacca gatttgatcc ttgaaggttg
50761 gtagtaacat cttaatcaaa cagatgagga aaccaaggtg tggcgaggtg aagtgtcatg
50821 ccccaggtaa ctcagtgagt cccaggctaa gccagaattt gtatccaggt ccgttccagc

50881 tttctttcac agttgcctgt cctgggggca gcacgtgctg agcaagggta aggctgccgg
50941 aagcagcgtg tggggtgctt ggaagatgga cagcacatcc ctgctggtgg cagcagcctt
51001 cctgagggag gtgtcctcct gtgattatag ggccttgtca ggtggagatg gactagcgag
51061 gagacagcag tgtggacaga aacgggtggt catgtttgag aagtagcaag agcgtaggga
51121 aggggtctgg ggaggagggc ctcactgcta gggcttggac aggggcaata gagagccatg
51181 aaagctttta gagcagagag gaggctatga tcaccattaa tagtgttcat tgagttcatc
51241 ctggctgcca tgcagacctc agcctgctct cagctggtgc tcctcctgta cccacctcac
51301 accctcctcc cctggccccc gtgccctgaa tcagggatcc atacagagca agtggcacag
51361 gtgtgtgagt gtgtgaggct ccggggcttc agggagttgg gattctcttc cacccctttt
51421 gcctttgcct gggtgcagct ttgctcccgg atcagccttc cccctcgcct gctgggcatc
51481 aaattatatt tggtcccttg tttactgtct ctttcctaca gtccaccagg gcaggggcca
51541 cagctcttgg tcacagctaa tttcctggcc tagaacagtg cctgataact ttaaaaaaaa
51601 attttttttt ctgcaaacac tccctgagcc cctatctgtg ctgggtcctg gcaggtaaac
51661 agtgtatgtc cctgactgtg agttgcatct aatctagcct gtgaaggata agtaaagggc
51721 ctcaagatag tcacggatgc aacagaggcc tctgagaggg gccatgggag ggcaggacgg
51781 gggcggctgg tggtgcggtg agtggcatga actgtgaagg gacctcagag gtggccatta
51841 agctgggtct taaggcctga gtagccgctt gcctagtcat cggaggagga ggaagcatag
51901 tgtttcaggc caaggacatg gcacatatga agggcccgga gtcctggtgt gttgtgatcg
51961 gcaaggaata tgcagtggta taaacagggg cccctcagca aggggaggcc aggctggaag
52021 agtgtgatga ggcttccctg ccatcctgag gcagctggac tttgttttaa agccacctgg
52081 agaccgggtg tagtggctta tgtgtgcctg taatcccagc attttgggag gctgaggtgg
52141 atggatcact tgaggtcagg agttcgagac cagcctggcc agcatggtga aatcccatct
52201 ctactaaaaa taaaaaaatt tgctggacat ggtggtgggt gcttgtaatc tcaggtattt
52261 gggaggctga ggcaggagaa acgcttcaac ccgggaggtg gaggttgcag taagccgaga
52321 tggcgccatt gcactccagc ctgggcagca gagactctgt ctcaaaaata aagcaagcca
52381 cctggagccg tccctggaac tgttaagcaa gcctgggata ccaagccttt ttgagtgagt
52441 cccgccagcc cgagggtgtg tagaggggtt gtgtgcctgg ccaggtagga ggcggggctg
52501 tgagaacctg ccagtactcc cgaaaggcgg aaggagaaac tgagcttcta gcaggtgaag
52561 aagaggtagc tgggcctgaa gagccctgta tgaaggggca tcgtgggaga tttagatgat
52621 cggacctttt gaccctgggg acaggtttcc ctgccagttt aaaaactcac attggttggc
52681 cgggcacatt ggctcacacc tataatccca gcatttttgg aggccgaggt gagcggatca
52741 cttgagctca ggagtttgaa accaacctgg gaaacatagg gagaccccat ctctacaaaa
52801 aaaaaaaaaa aaaaaaaatt agctgagcat ggtggagtgc ttatgtcgtc ccggctactt
52861 ggaagctgag gtggaaggat cgcttgagcc caggaggttg agtcagcagt aagctgtgat
52921 cacaccacca cactccagtc tgggcagcag agcagggccc tgtctcaaca aaacaaaact
52981 caaattggtg accttctatc cccaaggtca ctgtgtcatg gcgtgcctga catggtatgt
53041 gatgtgtctg tactgggctc tggaggatca ctaggtttct ccttgtacag acacgttcag
53101 tcctgtcgga agctgcggga ggggaggacc gccccgtcat ttgccacatc aggcagccag

53161 gactcagaga ggggatgtga cttaggaagg tcacagtgac tccacttccc aggcagccct
53221 agaggttcca gcctgacagc ttgtcattcc tgccccaatc tcccagccct ggcccaggga
53281 ctttcgcctg ggactgccca gtggtgccag ctctgtgggc ccaggaagcc tggctcctcc
53341 gggctaggtg ggggtagggg agatggccca aaaagcacag aaatttagac caggggtag
53401 atatttcctc agcaagtttt tcctgagcct tgctgtggtc aagccccctg caaggccctg
53461 gggccacagg aggtcccaga ctgaggggaa gtggagccag gtgtggtcac caatcttcat
53521 tcttagcttg ggatcgccct gggcagcctc cgtctcaggt ggaagacggg gtgggcagaa
53581 tacagagcag cgggacaaac gcagccccag gggttctcgg ccgtgagcag agcctgtgtc
53641 tctcccttag acaggtatac agggaggggc tgggatgcca acctactggg tcccccgact
53701 gtggctgtgg aaggttctgg atcttcagct gtcccaagcc ccggttggtc cacttcctat
53761 acctgagaag gggctccgtg ggtggagagt gggtcgggag aggtggccag gcagggtggc
53821 tgtggtttcc agctgccact gtcaccgccc actccactga gccatgccac ccgttctctg
53881 caggcaacca gacaccttgg cctggtgagg cctcgatgta ggggctgata ggtaggctca
53941 gagccatggc aaccggtggc cacctcctcc ctgccggcgc caggagcgga gactgcagag
54001 ggaccgggac ttagatgaga gaggtctccc cagcaggaag ggagggtctg gtggttggca
54061 cccactagat gagcatggtc agaaaaatat caggatgagc aacttcttta tctaggcctg
54121 ggtgggggtg cctggcacag gtctccatgg gacatgctgc agctggtagc ccccagaccc
54181 cggggctgtg cccccctggg cggcacccag aaggtagggc ttgccaggac tgacccttcc
54241 caaagagatc taggcgggga ccagagggtt tctgacccca gcctgtgact ggctccacct
54301 cccatagtct cctggagccg aggaaaggac aggactggcg ggcccatggg agggtccgtg
54361 gggcacttgg tcctcgtcag agggaggaac tggacagttc gcgcagtgct gctggtctgg
54421 actgccctct gagctctcag ggggtctcta tgcaggcaga gtcctcccag cccctctctc
54481 ccattcttgg gcgaggggtc ggctggagag aggcccctgg gacatctggg tgtgtgtgcc
54541 agccacccag cccaacttct tggctctctg agtgccacta tcaggaaagg ggccagggct
54601 gcctcagtgg ctgggaggcc tgcttggact tgggggttac ctagggcctt agccctcttg
54661 gctggcttcc caggagctcg gtcccacagg acttggggac agggtgggtg cagcacgctc
54721 ccctcaccag cccccattgg cttcctgctg gacccaactc acctccctcc tcatctcccc
54781 acagcccgat ctgctcaact tcaagaaggg atggatgtcg atcttggacg agcctggaga
54841 ggtaagagga ctgaggccgg aggggaggaa gctggcttgt gctgctgctg ggcccctggg
54901 cagcccagga gtgggggtgg ggctggggct ggtgtgagaa ccctgggggt ggggctggga
54961 ggggtggagg ctgcccatta ggcttctgcc tcctggagca gtctgcagtg tgtaaaaggg
55021 atcacagcaa ggggccgtct agcccctcac acccagtgcc ccatcccttg atctcatcaa
55081 gagtctcttg gccctgccac ctcagtagga gcctgggtgg gagtcaaggg tcttgggtgg
55141 aagggctcgt agaagctgtc tggcccgaga cacagattcc taaagcctgg tctgaggggg
55201 gtggggctca gggcgcaggt gctggggttt cacccacctg gatccccact ggttgagtca
55261 aagcctcctc tagtcctcag tggccacgtc tgtgacatgg gcataatgga taatggtgat
55321 tctttctaga gtggataaca aggtcagaga gatcaagcac gtacaggtgc cacaaagctc
55381 ctggcactta ctgtgggctc agctaaagac ccttcccttc ctctggcata tgtgtccttg

55441 gaaccagcag ggacagtggc tgtttcctca gctctgttct ttcggctgcc aggctggctc
55501 cctgcccaca ctgcctgggc aggagcgtct ccccacccct gccctgcatg cgcctgctgc
55561 tctggtctgc gtgcagcctg catgccggag agggctttga gccagagggg atttcattgt
55621 gtttccggcc cacatgtttt attccctcca gcccacagcc ttccctaccc gctgcctgcc
55681 cgctgtccct ggtggctgct ctctaggctc acacaaagcc cggcaccccg ccagtgaccc
55741 cttcctggcc tccccaccca gggtccccgg agggcagagg gattacaggc cccacccaga
55801 gctggccatt agtcatcctc tatcacaggg ttctggaatg tccgaactgg aagggccctg
55861 ggaattcagc cagcccaacc ccttggtgca cagacaggag acgggcccat gagggtgttg
55921 gtctcttcca ggcctaggag cacctggggc agacccaggc tcagaactca gcgcacctga
55981 ggcccatgga gctccaccca gcttccatct cagacagtca taggctaggc ctggaagagc
56041 taaagaaacc tcttggcata gaacccctgt cctactttac agagaggcaa ttgaggtctg
56101 cagcagcaac tccgtttact tagtgagagg aaaatgcttc taagaggcct ggcctcccag
56161 aacaaaccca gcaacctggt tggtgtccat ccctatgcca agggccatga cagccatcca
56221 cacaagagag gatgggcgcc gctcgcctca ccagaaggtc taggcacttc tttttttgtt
56281 ttttattttt gttttttttt ttctgaggca gagtttcact cttgttgccc aggctggagt
56341 gcagtggcgc aacctcggct cactgtgtct tccgcctccc gggttcaagc gattctcctg
56401 cctcagcctc ccgagtagcc gggattacag gcatgcgcca ccatgcccat ctaatttttt
56461 tttgtatttt tagtagagac agggtttctc catgttggtc aggctggtct cgaactcctg
56521 acctcagatg atccaccctt ctcagcctcc caaagtgctg ggattatagg cgcgagctac
56581 cgcgcccagc tatagggact tcttagtagc cctactttat ggttcagaag actgaagctc
56641 agaaaagtgg agacacttgc ctaaaggcac acagagagaa agggaactct tggatacaaa
56701 actccttttc tcagggaggc aatgtggaaa gaagcagaag gtatcgtcgt tgcatgcccc
56761 ttgagaactt accatcttct tgaggagaga aaactaagta cttgaaacaa ccaacagcac
56821 agacagggct tggtgatgtg agggaaccct gggagggctg gggaccgggt gatatgcact
56881 gaccctggca gtgggcgggc cgcgactggc attccagtgg agtgaccaga agcccagagg
56941 tgggctgggc ttggcatact atcaggctgt tctggcgccc ccaccccttt cctacatgtc
57001 ctgccaatct aaccccagct ggagcagaga caggcactgc aggaaggcac ccaatcacaa
57061 atcctaaact tacagcttag ggcgctctca cctgctgcgg atgtgggtag ccagcctaca
57121 gttcaagaag cagaccgtgc atctgggcgc ggtggctcac gcctgtaatc ccagcacttt
57181 gggaggccaa ggcaggtgaa tcacctgagg ccaggagttt gagaccagcc tggctgacat
57241 gatgaaaccc tatctctact aaaaatacaa gaaattagct gagcatgatg gcgggcgcct
57301 gtaatcccag ctactcggga ggctaagaca ggagaatcac ttgaactcag gaggaggggg
57361 aggttgcagt gagtggagat tgcaccattg cactccagcc tgggcaacaa gagcaaaact
57421 cctccatctc aaaaaaaaaa aaaaaaaaag gcagcagatt gtggctggca ctcactccta
57481 ctccagggct ccatccagtc ctacttccca ggggaccatt gtccctgacc atggtgatga
57541 tctgggggaa tatggaaaaa gacgtaacgt cctggccccg ctgagtccct gacttgctta
57601 cacaggtcat cacccctccc tgtctgcact gggtccgggg tgcctgagga gccttggcaa
57661 tcttaaaagg agctccaaga aggaaaggga gggtgtggtt acggagggga aaccaatcca

57721 tttcacagat gctacccctg aggctcagag aggaaatgga ggggccaggt ctcaaattgg
57781 cattttccgc ctccccaccc ctcctgtgtg cagcatagtg agtttggcag ccacacagga
57841 cacctgtaca atcacacaca ctggctcttt cattccatcc tccttgcagc ctccctcccc
57901 ctcgctcacc accacctcta cttcgcagtg gaagaaacat tggtttgtgc tgacagattc
57961 aagtctcaaa tattacagag actccactgc tgaggaggtg aggccatggg tgtactgatg
58021 aacccccgga agggcctggg gtggcctggc tgggttcact ggggtgggtc acaccagggc
58081 ccacacggac catagtgggc cctcttgctc caggcagatg agctggatgg tgagatcgac
58141 ctgcgttcct gcacggatgt cactgagtac gcggtgcagc gcaactatgg cttccagatc
58201 cacgtgaggc tgtgtgcagc ttgggggctg gtggtgggca gcatggctgg aggtccctgg
58261 gggaggtttt ggtgttgaac atggctcacc atggagactg gatcccttca ctcatttacc
58321 cagaggccct atcttcagag ccagagctgt gatgccaaca cttagcatat ctgggggaga
58381 cgctggagcc agcagggctc ttcccaggcc aatggaaggg aggttttgta ccccctgccc
58441 accccagact caagacctta gatgcatcct gggaaggaag ggccaccctc cctggggtcc
58501 atagtgggga gggagtcatg cggctggcca tgtggcaacc tacccatgcg gtggccttgc
58561 agaccaagga tgctgtctat accttgtcgg ccatgacctc aggcatccgg cggaactgga
58621 tcgaggctct gagaaagacc gtacgtccaa cttcagcccc agatgtcacc aagtacgtac
58681 taagctggac tggggccttg agggaggcac ttacccttgc gtccccgagt gggtttctag
58741 gtactcacct gagggctgca cctttctggg ccctcgtttc tctgtcaaca acatgggaag
58801 agagtagtga gcattctcgg tatcatccag aagtacactg agggtttgag gtttgatctg
58861 tcttctcaaa tgtgatgggg cctcttctct ttggaggaga tgcctgtgag tcaaaagccc
58921 ctgccaggtg gctgttctgt ctctgtgtct gtgccgtggg ggccgtgtct aagcctgtgt
58981 taggaagggg tggttaggta gttcagcctt ggcgcccaca cctcccggga tggggagctc
59041 actccttctg gaggctgcca cgggcactgg ggcaggaggg ctgagttacc gagtggcaga
59101 gccccccaca gtttataccc agatgcccat ggcgggctgg gacttggagc tgctgggcag
59161 cgggaacaga gtgtggtatt aaagggggct gggctgggtg tagtggctca cacctgtaat
59221 cccagcgatt tgggaggctg agatgggagg atcacttgag ctcaggagtt caagaccagc
59281 ctgggcaata tagtgagacc tcgtccctac aaaaaattta aaaatcagct ggccgtggta
59341 gggtgcgcct gtcagctgag gtgggaggat cacctgagcc cggaggccga gtcccccaga
59401 ctggccacac cgcggctatt cagccttagg ccagaggctt tcctgtctag gtttcagagt
59461 ccccatctgt cacgttagca tgtcatgtct gtacatgtgc caggatgtgc ccagagcact
59521 ttgtgaactc tgaagcactg tgcagttgga ctgttattta gcatctaggc ccagctgctt
59581 taagaaagaa accccgggca cagtaacttc atcgagatag atgttcatgt ctcccttcag
59641 aacagccccg aggtatgcag tcctggcctg gtggggctcg ctctgccgtt tcaggggagc
59701 cacctgccac acccactcgg cagcctatag tcacatggct acaagtagct gtgggaatac
59761 tgcgagtgta gtttttggca gggtgaccac gtacccagct atgactgtgg ataaagagaa
59821 ttgccatagt tgttcagagt ctctgcaact tgtgttctgt gcaccccgag gtcccgcctt
59881 caccccatca gcagtgactg ggcatccccc ttgccactgt gagcctcggg aacctgggac
59941 attggtctcc tacatcacct cattgatcac catttgcaaa aagccccctg cctgctttcc

60001 acgttcgccc accctgtcct cacatccatc ctgtgagagg cgtcgctgtc ccacattaca
60061 gatgaggtca ctgaagctca aagagggcaa agagaccagt gtggtggttc acgcctgtaa
60121 tcccagagct ttgggaggct gaggtgggag gagcacgtga gcccaggagt tcgagaccaa
60181 cctgggcaat atattgagac cccatctcta tcaaaaattt aaaaaattag ctgggcgtgg
60241 tggtgcatgt ctgtagtccc agctactcca gaggccgagg caggaggatc acttgagcta
60301 aggagtttaa ggctgcagtg aactatgatc acaccactct acttcagcct gggcagcaga
60361 gtgagaccct gtcccttgaa aaacaaaaac aagaacgggg gcgagggaag ccgggcatcc
60421 tggtgggtcc ctgggctgtc aagaggtgga gctgggatgt tcctggggtc tgttaaccag
60481 gaagctcagg tcgggctgct tccttccctg gggtctgtct ccagcccctg gcacagggcc
60541 tggcacacag gtgtcgctcc aaaagtgctc attgtgggac tgcaggggtg aggacccacc
60601 tgacgtggct ctgctggtgc cctaggctct cggactctaa caaggagaac gcgctgcaca
60661 gctacagcac ccagaagggc cccctgaagg caggggagca gcgggcgggc tctgaggtca
60721 tcagccgggg tggccctcgg aaggcggacg ggcagcgtca ggccttggac tacgtggagc
60781 tctcgccgct gacccaggct tccccgcagc gggcccgcac cccagcccgc actcctgacc
60841 gcctggccaa gcaggaggag ctggagcggg acctggccca gcgctccgag gagcggcgca
60901 agtggtttga ggccacagac agcaggaccc cagaggtgcc tgctggtgag gggccgcgcc
60961 ggggcctggg tgcccccctg actgaggacc agcaaaaccg gcttagtgag gagatcgaga
61021 agaagtggca ggagctggag aagctgcccc tgcgggagaa taagcgggtg cccctcactg
61081 ccctgctcaa ccaaagccgc ggagagcgcc gagggccccc aagtgacggc cacgaggcac
61141 tggagaagga ggtaggcacc acggctggct ctctaggagg ccccttgccc cagcgcccct
61201 ccagtccctg cattcatgcc ctggcatttg tcttgcactc ctacagtgat ggggagctca
61261 cccctgatct gctgtgagtg tgcaccccac actcctgcga actagaatct tcttccttag
61321 accttagtag tttggtttgg ttttgttttg ttttttccta gttctgccct tagcagccat
61381 agagaagaga agtgggaaga gaagggagag acctgccagg ctcacacctg taatcctaac
61441 attttgggag gccaaggtgg gagggtccct tgaagccaag agtttgagac cagtctgtgc
61501 gacatggcaa aaccccatgt ctacaaaaaa atacaaaatt tagctgggcg tgctggtgca
61561 tacctggagc cccagctact tgagaggctg aggtgggagg atggctggag cccgggaggc
61621 agaggatgca gtgagccaag atcaagccac tgcactccag cagtctggca atagagcccg
61681 actttgtctc agattaaaaa aaaaaaagaa gaagaagaag ggcgagaccg tttattgttc
61741 atcttctgcc cgtggcctcc ctgcctgcac tgactcgtgg aatcctcaca gcggcctcta
61801 cccctagggt ggacttcagc agttttactg acgggaaaac tgaggctcaa agatgtttcc
61861 tctgtcacct ccatgatgtg taaacatctg tgaccacagt ttccttttcc tatccctgtc
61921 tagccttctc tgggccaagg ctctcccagg tcattgacct ggccatccag gaaacagccc
61981 agcgtgtccc ctggctacat ctcactccag ggctgcttgc ccttctcaaa ccctgaggca
62041 ttcctaagcc ttccagggac gctgggcttg tatccgtgtg gagctggaag cctgcgggct
62101 cctcactgcc ttccaccagc cctgccccag cttccaggtc ccactgacca cccttctccc
62161 tcgtaggttc aggctcttcg ggcccagctg gaggcgtggc gtctccaagg ggaggctcct
62221 cagagtgcac tgagatccca ggaggatggc cacatccccc cgggctacat ctcacaggta

62281 aggccggggg gctgtttttc agggggaggg ggcagatttc tgcttgccgt gttatcagga
62341 aacagctgag attttgggag cccttcctgt gtgtgctgga acctgtgtgg ggcatttgac
62401 atgcgtcatc tcatttactc tccccacagc tggtgggcgt gatcactgtg cccgttttac
62461 agacaaggcc actgagctct gagaggttat gtgacttgcc caaggtcacc ccgcctgcag
62521 gtctcaaagg tgggatttga gcgagggtcc ggctgactgc agagcctgtg tgtgagtccc
62581 cgtgtgacac tctgcacttg gacccttgcc ccggggaagt ggggggctag tggccccgag
62641 agtcactccc tgaacacagg gaaatcctgc cgaggcccac tgggccaagg ccccacacaa
62701 ggaggtctgc aggacagggg agcctccagt agccctcaga gaatgcaccg tccgcctagg
62761 acagcggttc tcaacggggt ccattttgtc ccccttgggg acatttggct gtgtctggga
62821 atacttctgg ttgtcacaac taggacaaac taactctcta gaaaaaacgt gtgtgtgtac
62881 acatatataa gtttattata aattttacca ataaaaacaa tgcttagtac acaatttaca
62941 aataaaaata tataatttta ttgtgaattc cgtacagcaa atggattttt ctcagaatgc
63001 tttcagtgat tttttccata ctcttgtatc cacaactgat taacaaaggg ggttgttcta
63061 acgtgtgctg gttgacattt ttgtttatgg taatgagtaa gatgagaaga catttgtcga
63121 aactttactg gtccatgttg tcagctactt ctttgctaga tcggatagta gttttctatt
63181 gagaatattt gagaatattc tattaagaat atttttgcaa ttttttgttc tatttacaat
63241 gtaatggtta tagatgtgag atacttctaa gtttaatctg cattattagc attttcttat
63301 cacttgctta agtctagaca atcaacaaaa caataaatca agccctgatg tgtaatgttt
63361 accaattctg cagtgtaaat actcccgctg tggccaatgc tgcattacca gcacaaagtc
63421 cctggacaga gagttgggaa gagatgtcac actattcact aggatttcca ccatgcggat
63481 acagtagatg tgattggccc caagagcacg cataatacaa cacagtgaaa tatttaggaa
63541 gtaataaatt tgagtatcta ttgccttgtc tttaacatag ttaatttaaa gtatataaat
63601 ttaattgtta atggctgtgc ttaacaacta catcacagaa ttactaaaaa tttgacaatc
63661 agctgtctgt agttatcaca ccattatcta aagtagaaaa gaaggggcca ggcgcagtgg
63721 ctcacgcctg taatcccagc actttgggga ggccgaagtg ggcagatcac gaggtcagga
63781 gaacgagact aacctggcca acatggtgaa actccgtctc taataaaata caaaaaatag
63841 ccgggcgtgg tggtgcgcac tgtagtccca gctacttggg aggctgaggc aggggaccca
63901 ctggaaccca ggaggcgcag gttgcagtga gccgagattg cgccacggca ctccagcctg
63961 gcgacagagt aagactccat ctcaaaaaaa aaaaaaaaga aagaaaagaa aagaaggctg
64021 agatctaggc cctaggcagg aggagccttc taaaaatttc caaggggagg tcacgtcatg
64081 agggggctct caggaaggca ttggctgggg catgagaagg aaggctggtt ggtgcgtatt
64141 gtggatgcct ccagtgctga gctaatagtt agaagctctg gcctggtacg gtggctaatg
64201 cctgtatctc agcactttgg gaggtgaggt gggtggatca cttgaggtca aatgttcgag
64261 gccagtgtgg ccagagtggt gaaaccctgt ctctactaaa aaaaaaaaat acaaaactta
64321 gcagggcatg gtggtgggcg cctataatcc cagctactcc agtggctgag gcaggagaat
64381 cgcttgaacc cgggaggcag aggttgcagt gagctgagat cctgccactg cagcctgggt
64441 gacagtgaga ctctatctaa aaacagcaac aaaaaacaaa acaaacaaaa aaagagaagc
64501 tcattccaca gacttttgga ggtttggggt cagacagtga catcaggaaa gtgatgttgg

64561 agtggagggg gcttgggaga gggccaggtg taaggcatta ggaaatggct gaaggagggt
64621 gaatgtcaag ggtctggagg gaggagaatt tgggactgga tgaccagatc tacggaaggc
64681 agaggggcct ttgggggccc tgaggctggg gctgctgctc cagggaagcc cggagaccgt
64741 cctggggcca agtagtggat ggagtttcca ctgatagagg agcaggcagg ggaattgggg
64801 tgggacgcag agggtgaggc tgggggctta ccttgtcctg gggaggggca ggggaggagc
64861 cagtgttgtg ccctgtagcc tcccaccctg aggcagagaa gggagctggg ggcccaacac
64921 cccagctgag gcaggtggga gggcctccga ggcccccagc cagggggctt ctgcctgcgt
64981 gctctgctct aagaagctgc aggggctgct ttctgggtgg gtggggctgg gacccattcg
65041 tacccaccta taatcagagt ggcttcatct tcctccattt tattttattt taattttttt
65101 agagatgagg tcttttactg tcacccaggc tacagtgcag tggcacagag gctcactgca
65161 gcctcgaact ctagggctca agcagtcttc ccacctcagc ctcctgagta ggaaccacag
65221 gctacaccac catgcctggc taattaaaaa aatttttttg tagagacaag gtctcactat
65281 gttccctggg ctggtcttga actcctgggc tcaagtgctt ctctcatgtt ggcctcccaa
65341 agtgctgggg gtacaagtgt gaccactgca ctggcccacc tttgtccgtt ttactcatag
65401 agccagcttt agatttcctt tagataaagg ctctgccact acaatgacgt ttaaaaggca
65461 ctgctttggc ttctcagctt actggtggca aaactgaggc ccacagaagg gtgtgacctt
65521 tgagctatag gcagacctgt gtctggagtt caggcggtct ggtccccagg tgccccattg
65581 ggtgcaccaa agtctgtcag tgtttggtcc cgttagggtg ggatgggcac ggggagcagg
65641 tgggagggga gaagggaata actggggagc agagctgaag accaggcagg tggaggagtg
65701 accccagatg cggaaggcag cagagaagaa gagctgagtc atctgtatcg ccctcaggca
65761 cgggaggagg cggatggaaa ttcggtggac ctggaggagt gacagctggg agggattccc
65821 cagctggtat ctcgggctga gtgggtgccc ttccttcagg ggttgggggc ttgctgctgc
65881 acgacagctg tcgggtggtc tggggcttca cccactggac atctggtaaa gagaaccttt
65941 ccccactatt gcagtgggga cagaggcatt tctttatcta ctttgatgtc catgtcaaag
66001 gtggcagcca ggatcaggac accttgggcc actcaggaag gtaggtgaga gaggccctag
66061 gagcccctgt tcgtcagctg agtcccacct cctgatgtca gcccctgcac ccctgagctg
66121 ctgggtctgc ctgcctggtc tggccctctt cagaagagca gtgcccaccc ccacattgct
66181 gggctcccca ctggtggatg ctggcgtacc gtctccctct gtgcacaggg ttcccgtgcc
66241 cagggagcct cgggggctgg tcctgtgagg cctgctttac ggatgccaag actgaagctc
66301 agagggggcg aggggtttgt tcaggttcac tccaaggcgc agccagggtg aagaccgcct
66361 ctgaggtcca aagtcatgtt ctgttgtcct aagccaggag acacatagag ggcaggaaca
66421 gaagccaggg gacagacacc cctgtcgcca catgaagctc ttggccctcg ctcctccatc
66481 aaatcgtccc caggtgtggc tgctggagag gcggggaggt ggcctttgcg tgggggagac
66541 agtccctcat ctggtcctct gtgccttagt ttcttccctg gctcttcttc cccttccctt
66601 tttaatgacc ttaaacattt ttttaattat ataaaagaaa tgtttacatt ttcaaaaata
66661 atttaaacaa attgaaaggc tggaaatcaa caccctcaca ctccttctgc tgccagctgc
66721 caccccaggg tcctgtcggt ggctgctgcc gcctgcctat cagtttctgg tggcagctgt
66781 aacctgtaac cacagctacc taaataatgc acatgtatta cctcacgctt ctgtaggcga

66841 gaagtctaac gcgagtcttg ccgcgctgca gccaaggcgt gggcaggact gtgttcctct
66901 gcaggcaccc gttgtatccc ttgcctgctc cagcttctgg tggccgtccg cgtcccttgg
66961 ctctcggccc cttccttcat cttcaaagcc tgcagagggg aagtcgagtc cttctcctgc
67021 cacgtcactc tgaccatccc ttcctggtca cacctccctt gaactcttcg cttctgcccc
67081 cttcgtccac ttttaaggac ccttgtgatt acactgggct tgagggataa tctgggataa
67141 tctcccgatt ttaaggtcag ctgattagca accttgattc cctctgcagc ctgaattcct
67201 ctttgccatg taacatgatg tagtcacaga tcctgggagt aagatgtgga caactttgag
67261 gggctatcat tctgccccct acaatatcct ccctaccttt tctgtgtttt ctgtgttttc
67321 cattcactgg gacggacagg tctgcacgtt tgttcccgtg agtgagatca taccacgtgt
67381 atgtcagaag ccaactttca ccacctcatt ttgggatctt tttccacagc atccacacaa
67441 aaactctgtc tagcagctgc ctgtgtccca ttgtcaagct cgatcccatc atttattcaa
67501 gagttcccct gatagggatt tagattgttt ccagttttca tctgttacca acctggctgt
67561 agggaatacc cttgttccaa aagtctctgt acacatacga ctttctgtgg gatcatttct
67621 gggagtgaac ctactaggtc aaccagtggg gtcgcaaaat gtgatgatgc caccggacca
67681 cccccaacac acaccgttgt ggcggacact cccagcagtg ggtattatta gtctgagata
67741 cttgcccatc ttgtctacta agcattattt tgtattacat catcagtttc agctcattta
67801 ctgagcacct gccacatgtc aggccctggc ctagaccctg gggactgcgc cagtcagatg
67861 ttctcagtct tcagggaatt tatggtttgg cagaggtaga accttttgag gcaggggaga
67921 ggctgcccag agacagagct aggagagaca tttaaactgg gctgcaggtc agtaaattac
67981 ccagggaggt aaagttgcca aaaaaacatt ccaggccggg cacagtggct catgcctgta
68041 atcccagcat tttgggaggc caaggccggc agatcacaag gtcagaagtt caagaccatc
68101 ctggctaaca cagtgaaacc ccatctctac taaaaataca aaaattagct gggcgtggtg
68161 gtgggcacct gtagtcccag ctactcacga ggctgaggca gaagaatcac ttgaacccag
68221 aggtggaggt tgcagtgagc tgagattgct ccactgcact ccagcctggg tgacagaaca
68281 aaactctgtc tcaaaaacaa aaacaaaaac aaaaaaacca caaaattcca ggcagtggga
68341 atagctaaag ctcagaggtg tgaacgtgct gcaaccgtaa ctgtgagatt gcttggcaga
68401 gaaaggccag ggatgagaac gtaaaggtca gccatgggaa cggggacttg actctgtagg
68461 acgaggaatc cctggagggc ttgaggcagg agcgacaagg tgaggtttgt gaatgaggag
68521 ccgtggggtg ggggtgggag caggagcagg tgcagactgg aggtgctggg acccaggagg
68581 aggtggtgta cctgcccctg agccttctcc tctggaggct ggggaagggc cgctgtccag
68641 aggaacgggg cagcctgtga caccctggcg tccggcccac aggaggcatg tgagcgcagc
68701 ctggcagaga tggagtcctc gcaccagcag gtgatggagg agctgcagcg gcaccacgag
68761 cgggagctgc agcgcctgca gcaggagaag gagtggctcc tggctgagga gacggcagcc
68821 acggcctcag gtatggaccc tggggggggc acagtgggct gggctctgag cctctgtgct
68881 gaggttcctc ctagccaaac tggggaggat tttatcaaat aagatgtagg ggtctcagtg
68941 ccacatttgg ggtaaagggt cagcaggtgt tagaagctcc aagaactggc ctgtagccct
69001 acttaaaaag ggaaactgag gcagtaaaag ataccctccc cacccccaac accaccacta
69061 ccataggtgg tgactcttcc tgctcagggg cctgcagcca ccatagagtc tgcctaaagg

69121 cacatgctac ccacgtgcgt gggggagagg ctgatcagct gcatcccctg tcctcatagg
69181 agtgcctcct ccctgggcac atgggccaga ccctggggag agcaggggaa ggtgtgagca
69241 ttgtccatgc cagctgtgtg ccaggcactg ggccccacat gtttcccaca ggagcaccct
69301 tcatcctgga caattccctc ccctcctgcc accgcacacc tgctgtgtgc ctggcacatt
69361 cctaggcatc actaggggtt tgcagaacaa tagaaatgct ggccaatgtc acatgatctg
69421 aagctcacaa cctgccagtg ctgtgccaag tgctcatgca ctcgaaccca gccttggggc
69481 cagtgtgctc tgcatttcag gaagatcgcc tttgcagccg cgtgtgttag agaccccttc
69541 cccctcagca gagtctggac ttcgccctgt aatctggcac taaggtttct gctgtgaaat
69601 ggataaaaat cacacttaag tggaataata aagaccataa atagcctcat gtccgttcag
69661 ggtggggatt gccaccaaaa gatttaccag aggcccggtg cagtggctca tgcctgtaat
69721 cccagcactt tgggaggctg aggcgggcag atcacctgag gtcaggagtt cgagactagc
69781 ctggccaaca tggtgaaacc ccatctctac taaaaataca aaaattagct gggcgtgatg
69841 gtgggcagct gcaatcccag ctactcagga ggctaaggca ggagaatctc ttgaacccag
69901 gagacagagg ctgcagtgag ctgagattgc accactgcac tccagcctgg atgacagagt
69961 aagactccat caaaaaaaaa aaaggccagg cactgtggct catgcctgta atcccagcac
70021 tttgggaggc cgaggcaggt gcatcacgag gtcaggagtt caagaccagc ctggccaaga
70081 tggtgaaacc ctgtctctac tatgaataca aaaattagcc atgcgcggtg gcaggcgcgt
70141 gtaatcccag ctactcagga ggctgaggca ggagaatcgc ttgaaagtgg gcggcaaagg
70201 ttgtagtgaa ccaagattgt gccactgcac tccagcctgg gtgacagagt gagactccgt
70261 ctcctgggtg acagagtgag actccgtctc ctgggtgaca gagtgagact ctgtctcaaa
70321 aaaaaaaaga aagaaaaaaa ggagagaaac tttcaatgtt cagaagtttc ggattttaga
70381 attgtgggta aaaccatggg tggccctgag tgggaaaaaa aaagcctcgg tctcagtcct
70441 taggagctca caatctgaga ataaagccag caggagctaa tgttggggca tttactctga
70501 gccatgcttt gttctaaatt cttcacacac cttccaataa ccccatggag gaaaggtact
70561 gttgttatcg atccctgttt tccagataag ggatctgagg cacaaggggt taagtcacct
70621 gtgcaaagtc acatcggttt atgctgactg tggagcctga gctcttgacc acgaggctgg
70681 tcaaagcaga taggccttgt ttgagagccc tcagagtctg cagggggggt gtactaggtg
70741 gggtcctacc tggaaatcac tagaacagga ggtaccaatg gtcagaccct gagctgtctc
70801 cccagtaggg tccatccaac aggattatct tcccttaata gatgggctga gagaggttga
70861 gagcttaccc caagccactc agtttatgac acaacctggg ctactctaga ctcctggttc
70921 ggtgctcttt gcatagcact ccttggtcca agtccatagt acttgcatag tactatgtgg
70981 agtccacata gtactccacc agtacatgtg gcgtctcaga gctggtggca cttggtgctg
71041 ctgacccccc tccagtctct cttgtgcctc tctgccctgc ttccagccat tgaagccatg
71101 aagaaggcct accaggaaga gctgagccga gagctgagca aaacacggag tctccagcag
71161 ggcccggatg gcctccggaa gcagcaccag taagatgatg ccgggcccca actgcccacc
71221 ctgatggtta tgggttgagg aactttgctg aggccccttg gcagcggaca catcagtttg
71281 cccctagctg agattcctgc tccagtctca tggatgcaag aaacagaagc agaaggtcct
71341 caggcagacc ccatgccagt cctcacagac agtccttcag gcagcttctt gtcttggctg

71401 gtcccagcct ccacccacct cttcacccct ttagtctccc ctggagctgg gctgcagtgt
71461 cactgttgac cactaggcct ctccctgccc cagagcacag ttttggaatg ttgaggtttc
71521 aggggcttta actttaaaga tgggcccctc cagccgggca cattggctca tgcctgtaat
71581 cccagcactt tgggaggctg ggatcaggag ttcgagacta gcctggccaa catggtgaaa
71641 ccccatctct actaaaaata caagctagct gaatgtggtg gcgggagcct gcaattctag
71701 ctacttggga ggctgaggca ggagaatcgc ttgaacccag gaggcagagg ttgcagtgag
71761 ccaagattgt gccattgtat tccagcctgg gcaacaagag tgagagcaag actccatcta
71821 aaaaaaaaaa aaaagatggg ctcctccaag ccaccctcac ttagggtaaa cacctgtagc
71881 ccagagaagc tgtcactggt tcacagcccc acagcaggct aaaggcaaac ctagagaggg
71941 aaccccagag tcctgggatg ctttaagtct acctgactgg actccaggct tggcaggagc
72001 aagacaacct atgctctcct ctgctcctcc tctggggcag gtcagatgtg gaggcactga
72061 agcgagagct gcaggtgcta tcggagcagt actcgcagaa gtgcctggag attggggcac
72121 tcatgcggca ggctgaggag cgcgagcaca cgctgcgccg ctgccagcag gagggccagg
72181 agctgctgcg ccacaaccag gtgggcctgg ccccaggttg gggggacacg ggtgggtccc
72241 ggcacccctc ccctgaccac cgtgcctctc ccaggagctg catggccgcc tgtcagagga
72301 gatagaccag ctgcgcggct tcattgcctc gcagggcatg ggcaatggct gcgggcgcag
72361 caacgagcgg agttcctgcg agctagaggt gagtgtcctc actagcacca ggcagcccag
72421 tggttctcgg ggcccggggc tatggggcac ccccgccata ggctgggtat cacctgtggc
72481 aggaaagcca agtctcccag tggctgggcc agcctgacta ggctagaaca cctactgtgc
72541 atccagtagc cagcattctg ccctcccacc tgcattgtgt cacctacctg ccgtgtacca
72601 tagcaaatca ctccactttt ctgagtccca gtagctcatc tgtaaaatgg caccagcacc
72661 ttccttactg ggaaatattg ggtaagaaaa atatgtgtgt atgtgtgtga tgccagtatc
72721 actgtagtat tttctttctt tttctttctt tctttttttg agacagagtc tctctctgtc
72781 acccaggctg gagtgtagtg acacgatctc agttcactgc aacctctgcc tccctggttc
72841 aagcgattct cctgcctcag cctcccaagt agctaggatt acaggcctgc accaccacac
72901 ccagctaatt ttttatattt ttggtagaga caagtttcac tatgttggcc aggctggtct
72961 cgaactccgg acctcaagtg atctgcctac cttggcctca caaagtgctg ggattacagc
73021 atgagccacc acacccggcc ttagtattta tttctttact tttttttttt ttttgagaca
73081 gagttttgct gttgttgccc aggctacagt gcagtggcgc gatctcggct caccacaacc
73141 tccgcctccc aggttaaaac gattctcctg gccgggcgcg gtggctcacg cctgtaatcc
73201 cagcactttg ggaggccgag gcgggcagat cacgaggtag gagatcaaga ccatcctggc
73261 taacacagtg aaaccccgtc tctactaaaa atacaaaaaa attacccggg cgtggtggcg
73321 ggcgcggtgg cgggtgcctg tagtcccagc cactccggag gctgaggcag gagaatggca
73381 tgaacctggg aggcggagcg tgcagtgagc cgagatcgtg ccactgcact ccagcctggg
73441 tgacagagcg agactccgtc tcaaaaaaaa aaaaaatatt aaaaaaaaaa agcgattatc
73501 ctgcctcagc ctcccaagta gctaggatta caggcatgtg ccacctcgcc tggataattt
73561 tgtattttta gtagagacag ggtttttcca tgttggtcag gctcttctcg aactcccaac
73621 ctcaggtgat ccgcccactt cggcctctca aagtgttggg attacaggcg tgagccaccg

73681 tgcccagccg atattttatt tctctttttt tttttgagac ggagtcactc tgtcacccag
73741 gctggagtgc agtggcacca tctcggctca ctgcaagctc cgcctctcgg gttcatgcca
73801 ttctcctgcc tcagcctccc gagtagctgg gactacaggc acccgccacc acacctggct
73861 aattttttgt atttttagta gagacggggt ttcaccatgt tagccgggat ggtcccaatc
73921 tcctgacctc gtgatccgcc cgcctctgcc tcccaaagtg ctgggattac aggcgtgagc
73981 caccgctcct ggccccaata ttttatttct tcaacaaaca cttaggtgag gctcactctg
74041 tacctagaaa ttctggaagc cctttttgtc tgttaactca tctaatcttc ctgacagctc
74101 catacattag ctactatgac tgtccccatt tacagatgag gaaactgagg cacaggaagg
74161 tcaagtacct taccaaagct tttatagctg gactgggatt tgaaccaggc agtctggctc
74221 cagtctgagc ttttaagcct ccccagtcct ggctgaactt gtttcctcct tggtcaagtc
74281 agcagcacct agccctcggg gttggggagg ggttaatgag gtgccttgtg gagtggttca
74341 gcctgctcag gtttatgtag ggcttctttt catccatctc actcccgagg tgagagcctg
74401 gtgctgcatg agggctgcag cagacttgac cgtgagatat ccactcacac gggctggaca
74461 ggggtgagag taagggaagg caaagcaggg gaatcggaga gggcttcctg gaggagtccg
74521 cctccaggat gtagagggtt tttgtgcaga taggggctgc attctagggg cctgaggcag
74581 agagaacccg ggctgcaggg cactatgggc cagggtagct ctggcttctg gccctgggtc
74641 agtccagcac ctatatcccc aggtgctgct tcgcgtaaaa gaaaacgaac tccagtacct
74701 aaagaaggag gtgcagtgcc tccgggacga gctccagatg atgcagaagg taggtccttc
74761 cgctgggctg ggggccgtcg gggactctgg agccatctgg atgccatcct gtgagcacct
74821 gctctgtgcc aagccctcca ctcgcttcat ccttcctcag gctctcctgt gcttctccca
74881 tgtaggtgtc atcatcccca ttcactgaca gggaaactga gactaagaaa ggggaagtga
74941 cttccccaag gctgtacttg gcttctgttt atttagcatt atttaattta ttgattatta
75001 ttgagcacct actatatcct gggcactgtg ctaggtgctg ggaagacaga tgtgaacaaa
75061 ataaccccaa atctctgctg tggggagctt gcactccagt gagaataacc acgttgctag
75121 gagttgggtg ggtcagggtt cagcccaggt ctgagtctgc agcccgcctg gcccaccctt
75181 ggctgctatg tcactgcctg gtgcgctctg tcagtttggg gcaggaagtg gacatgttgg
75241 tggctctgta agtgcacgtg agccagaccg tgtttaccgc acttgaagaa caggtggcgt
75301 gaactcacag gaagggcccg agcacccagc caggctgacc tggttctcat cccagccact
75361 cactgccctg cggtcttggt caaaatcttc tcccccgaaa caggatgagg atgacaatga
75421 cgcctgtgtc cctgggtggt accagggagg tgggaggggt aagcccagaa cccacggcca
75481 tcttggggag ccacctggag ggatgaagcg aggtatctgc ggggaggtct ggctgctggt
75541 gctgcccatg tgcccggttg gcagggctgg agggagagac ttcatgccct catacctgcc
75601 tcctgccccc caggacaagc gcttcacctc gggaaagtac caggacgtct atgtggagct
75661 gagccacatc aagacacggt ctgagcggga gatcgagcag ctgaaggagc acctgcgtct
75721 tgccatggcc gccctccagg agaaggagtc gatgcgcaac agcctggctg agtagaggtg
75781 gatgccgagg cgtgtgccct gcagggtggg caggggctga ggctctccca cacccgggac
75841 tccctggacc accccagcca ggccacttcc ttcttctggc ctccaattcc cgttctgtaa
75901 aaaggacaat gaaaccagca acacggggtt gttgcagaga tcccatcaga cccgggccac

75961 aggctgcttc atcagcttaa gcctgttcct ccagagctgg gcggcacagt gcagagccac
76021 agcctactca gtgcaccccc cttcttgggg ctggaggcaa atgcagcgtg cacatccctg
76081 acccccacag tgaatctcca ctgagcttca cacacacaca tcgcacgctg aggccgggct
76141 tctatcccag ttagtcttaa cactccttct tgacttagca gggctcaaag tggttggttg
76201 gttggttggt tggttggttg gttggttggt tggttggttt tggttgcttt gagaaaggtc
76261 ttactctgac acccaggctg gagtgcagtg gtgcaatcac ggctcactgc agcctcaacc
76321 tcctaggctc aagggatcct cccacctcag ccttctgagt agctgggacc acaggccctc
76381 accaccatgc ccagataatt tttgcacttt ttgtataggt ggggtttcgc cgtgatttcc
76441 caggctggtc tcgaactcct gggctcaagc aatacacctg cctcagcctc ccaaaattct
76501 gagattacag gtgtgagccg ctgcacctgg ccaaagtgtt cttatttttg ctttttcaac
76561 gccacatcta cctggagcat cctctttctg ataagtcctc atggacttcc tatggcatgc
76621 aggagaggcc accctatggc tgagctgctt gggaaggagc caggaagagc ggatctggct
76681 gtaccttagg ggctgagagg tggtggaagg acccactcag cacccttgct tggccttggg
76741 gtggcctcgc agcccctcag cccaggcctc agccctgccg caccttgtct ttcaggtccc
76801 gcccagctgc agaccctcca ggctggagga ccagccgccc tccttccctc ctggatggaa
76861 gtaaaaagcc aagctttctc cccaccctct gtgggccaca cgtgcacttg cacccaccac
76921 acacacacac acacacacac acacacacac agacacacag acacatacgc acacacgtgc
76981 acacatgtac acacggatac acacacacac acacacactg catatctgag cgcgcccctc
77041 gcactgggtc tcaccttgca ccttcttcag gattttatat gtgaagagat tttatatag
77101 atttttttcc tttttttcca aaacacttta tactttaaaa aaaaaaaaaa aaagcaattc
77161 ctggtggctg tgtgcctcca accctggtcc ccctctgtct ccagccaccc tctgcttggg
77221 cttctgagct ggtggccctg gcccagaggt ctggcggagg cccaggcagc agccatggcg
77281 gggtgtctct acaggggaga ggcgggagcc tgccaccctc ttcctgccct acctcctact
77341 aacacttcct gccccatttg gacccgtacc atggggctca ggacagaggg agctagcagc
77401 tggcctccat ggccccacag cctccttcga ggctgtgctg ggtgcagaac cgccagagcc
77461 acccaaaagg tgtttctctt ctgctccctg aacctcttaa cttaataaaa cgttccagca
77521 gctctggtgt cctagatggc tggcagaaca ggaatggaga ttggggtttc ttgaagtggc
77581 ttccccactc acaccctacc cacaccccac ccttcctaaa gcagcggcct ctaggcttct
77641 gagggtgggg ctgaaaatcc aaggtctccc ttagtacaga ctgtgacgcc cccagtgtgg
77701 cttgcaggct agtggcagcg gaagcatgtg ggaaatagca agtccagtcc caccccaacc
77761 tactgaacca gcgtctgcat tttaacaggg tgccccagtg attcacgtga ccttcaaagt
77821 ctgaggagcc ctggagtagg gcccagggcc caggcctgag agagaggcct ggggctaaga
77881 ctagccctga ccatgacctt gggcaagtca cacccctcac tgagcctcag tttgcttctc
77941 catcaaatgg gtgtttttct ttcagtccca gtcctctcct gaggcttttc ccagaggtca
78001 tgcccagccc tgggcagggg tgagggcagg gttgtggctg ccaggcacag gtccagcggg
78061 ctgggagtcc tcaccatggc tggctgtgct gattatggtt gcatggggct gggaagaggt
78121 cccatttctg agccacagaa acaggccaca tccagtaggg gtcctggaag gctgtgagaa
78181 cccagaggag accctgaccc agcctgcagg tagaagactg cctttggagg cagctttgaa

78241 agatgactct tccagatgga agaatcagga agggccttcc cacgtggagg caccagcagg
78301 agcaaacgtc cctggtgtgt tcaggcaaag agccacagga gagtgacctc gtggagtggg
78361 aagagattgt ttcccattcc cttgccaggg attggttgag gaatggacaa ggcttgcagt
78421 tcacgcaagc cagtgaaatg cgaggggaag tcttgggagt ggggtggagg aggtcatctg
78481 ggaagggtgt ccttggtctt aaaaagaggc acaagaggcc gggcgcggtg gctcttgcct
78541 attatcctag cactttggga ggccaaggca gggggatcac ttgaggtcag gagttcgaga
78601 ccagcctggc caacatggtg aaaccccgtc tctactaaaa atacaaaaaa ttagccagac
78661 gtggtgccaa gcacctgtaa tcccagctac tcaggaggct gaggcaggag aatcacttga
78721 acccaggagg cagaagttgc agtgagccaa gatcgcgcca ctgcactcca gcctgtgtga
78781 caagggcgaa actccatctc aaaaaaaaag ggggagggtg gcgcaaggag aggacacctc
78841 tctcctggcc ctgtcgccat cttccctcat caatagctaa aaatggcaaa gaagaaagag
78901 ccaggacccc gtgccatcaa atcctccggc cttgtgctgc cctgtctcgg cactgcttgc
78961 cttaggagat caggagtgtt cctagccact gggagatggg atgcctgcca ctggcagctc
79021 tgccgtccat cctcaccatc ccaccgtccc cccaagacag ctctccatcc catcctctgc
79081 ctcccttcta gcctcaacct aactcagggc tcccccgtct cacacctggg ttgcaggaac
79141 atcctcttca ccaacccccc tcccatccct cctcagggct gcctagggca gctacaaaaa
79201 ccaggccagc ctctgaaagg tgctccacat ctcattaaaa accgtgaaat cgcactgccc
79261 cactatcaga atagctcaga ggagaaggtc tgccagtacc aagtgctggt ggggatacgg
79321 agaagagcag ctcccaggcg ctgctggtgc aaatgtgctt tgttgcaccc acggcaggaa
79381 actgaccagc ttctcatcaa gttaaatacg catctaccct atggcctagc atttccactc
79441 ttgcttacac atccaggaga aatgagtcct tacatgcaca aaaggatgag taccgtaata
79501 ctcacatcag caaataaaca atacagctgt gtgtcagtag tagaatgaat aaattgtggt
79561 cgattcata

### Stereocilin

The STRC gene encodes stereocilin, a protein that is normally expressed in the inner ear and is associated with the stereocilia of specialized hair cells in the inner ear (see, e.g., Verpy et al., Nature 456: 255-258, 2008; and Zhang et al., J. Med. Genet. 44: 233-240, 2007). Stereocilia, which are about 10-50 µm in length, are important for hearing and balance. Stereocilia play a mechanosensing role in hearing. By bending in response to sound, they form a structure for mechanoreception of sound stimulation.

The human STRC gene is located on chromosome 15q15. It contains 29 exons encompassing ~19 kilobases (kb) (Verpy et al., Nature Genetics 29(3): 345-349, 2001; NCBI Accession No. NG011636.1). The gene is tandemly duplicated on chromosome 15q15 in a telomere-to-centromere orientation, with the tandem copies located less than 100kb apart on the chromosome. The coding sequence of the second copy is interrupted by a stop codon in exon 20, meaning that it is a "pseudogene" that encodes a nonfunctional truncated protein. (Hereinafter, references to "the STRC gene" mean the gene that does not have that stop codon in exon 20, while references to the "pseudogene" or the "STRC pseudogene" denote the gene that does have that stop codon in exon 20.) In some examples, the full-length STRC protein is a full-length wildtype STRC protein. The full-length wildtype STRC protein expressed from the human STRC gene is 1775 residues in length, and contains a signal peptide and several hydrophobic segments. When the signal peptide is cleaved off during processing of the wildtype protein, the resulting mature wildtype stereocilin protein is 1719 residues in length. In some embodiments, a full-length STRC protein can include a heterologous signal peptide positioned N-terminal to an amino acid sequence of a mature wildtype stereocilin protein.

Various mutations in the STRC gene have been associated with hearing loss (e.g., non-symptomatic sensorineural hearing loss). For example, a homozygous 1-bp insertion was identified in exon 13 in a consanguineous Pakistani family with autosomal recessive non-syndromic sensorineural deafness-16 (DFNB16) (Verpy et al. (2001). Another example found in a family with DFNB16 includes both a 4-bp deletion in exon 5 and a larger deletion encompassing exons 17-29. The two deletions were inherited from the father and mother, respectively. The 4-bp deletion was predicted to result in the translation of 5 out-of-frame amino acids and a premature stop codon in exon 5.

Additional exemplary mutations in a stereocilin gene detected in subjects having non-symptomatic sensorineural hearing loss and methods of sequencing a nucleic acid encoding stereocilin are described in, e.g., Verpy et al., Nature 456:255-259, 2008; Verpy et al., Res. Systems Neurosci. 519:194-210, 2011; Hofrichter et al., Clinical Genetics 87:49-55, 2015; and Mandelker et al., J. Mol. Diagnostics 16:639-647, 2014. Methods of detecting mutations in a gene are well-known in the art. Non-limiting examples of such techniques include: real-time polymerase chain reaction (RT-PCR), PCR, sequencing, Southern blotting, and Northern blotting.

An exemplary human wildtype stereocilin protein is or includes the sequence of SEQ ID NO: 34. Non-limiting examples of nucleic acid encoding a wildtype stereocilin protein are or include SEQ ID NO: 35 and SEQ ID NO: 36. As can be appreciated in the art, at least some or all of the codons in SEQ ID NO: 35 can be codon-optimized to allow for optimal expression in a non-human mammal or in a human.

In some embodiments of any of the compositions described herein, the STRC protein comprises a sequence that is at least 75% (e.g., at least 80%, at least 85%, at least 90%, at least 91%, at least 92%, at least 93%, at least 94%, at least 95%, at least 96%, at least 97%, at least 98%, at least 99%) identical to SEQ ID NO: 34.
**Human Full-length Wildtype Stereocilin Protein (SEQ ID NO: 34) (Signal sequence shown in bold)** 
MALSLWPLLLLLLLLLLLSFAVTLAPTGPHSLDPGLSFLKSLLSTLDQAPQGSLSRSRFFTFLANISSSFE
PGRMGEGPVGEPPPLQPPALRLHDFLVTLRGSPDWEPMLGLLGDMLALLGQEQTPRDFLVHQAGVLGGLVE
VLLGALVPGGPPTPTRPPCTRDGPSDCVLAADWLPSLLLLLEGTRWQALVQVQPSVDPTNATGLDGREAAP
HFLQGLLGLLTPTGELGSKEALWGGLLRTVGAPLYAAFQEGLLRVTHSLQDEVFSILGQPEPDTNGQCQGV
FFLTLSLLGNLQQLLLWGVRHNLSWDVQALGFLSGSPPPPPALLHCLSTGVPLPRASQPSAHISPRQRRAI
TVEALCENHLGPAPPYSISNFSIHLLCQHTKPATPQPHPSTTAICQTAVWYAVSWAPGAQGWLQACHDQFP
DEFLDAICSNLSFSALSGSNRRLVKRLCAGLLPPPTSCPEGLPPVPLTPDIFWGCFLENETLWAERLCGEA
SLQAVPPSNQAWVQHVCQGPTPDVTASPPCHIGPCGERCPDGGSFLVMVCANDTMYEVLVPFWPWLAGQCR
ISRGGNDTCFLEGLLGPLLPSLPPLGPSPLCLTPGPFLLGMLSQLPRCQSSVPALAHPTRLHYLLRLLTFL
LGPGAGGAEAQGMLGRALLLSSLPDNCSFWDAFRPEGRRSVLRTIGEYLEQDEEQPTPSGFEPTVNPSSGI
SKMELLACFSPVLWDLLQREKSVWALQILVQAYLHMPPENLQQLVLSAEREAAQGFLTLMLQGKLQGKLQV
PPSEEQALGRLTALLLQRYPRLTSQLFIDLSPLIPFLAVSDLMRFPPSLLANDSVQQGYRGSEENSLEEDK
GLRPMTPRSLAAIRDYSPGMRPEQKEALAKRLLAPELFGEVPAWPQELLWAVLPLLPHLPLENFLQLSPHQ
IQALEDSWPAAGLGPGHARHVLRSLVNQSVQDGEEQVRRLGPLACFLSPEELQSLVPLSDPTGPVERGLLE
CAANGTLSPEGRVAYELLGVLRSSGGAVLSPRELRVWAPLFSQLGLRFLQELSEPQLRAMLPVLQGTSVTP
AQAVLLLGRLLPRHDLSLEELCSLHLLLPGLSPQTLQAIPRRVLVGACSCLAPELSRLSACQTAALLQTFR
VKDGVKNMGTTGAGPAVCIPGQQPIPTTWPDCLLPLLPLKLLQLDSLALLANRRRYWELPWSEQQAQFLWK
KMQVPTNLTLRNLQALGTLAGGMSCEFLQQINSMVDFLEVVHMIYQLPTRVRGSLRACIWAELQRRMAMPE
PEWTTVGPELNGLDSKLLLDLPIQLMDRLSNESIMLVVELVQRAPEQLLALTPLHQAALAERALQNLAPKE
TPVSGEVLETLGPLVGFLGTESTRQIPLQILLSHLSQLQGFCLGETFATELGWLLLQESVLGKPELWSQDE
VEQAGRLVFTLSTEAISLIPREALGPETLERLLEKQQSWEQSRVGQLCREPQLAAKKAALVAGVVRPAAED
LPEPVPNCADVRGTFPAAWSATQIAEMELSDFEDCLTLFAGDPGLGPEELRAAMGKAKQLWGPPRGFRPEQ
ILQLGRLLIGLGDRELQELILVDWGVLSTLGQIDGWSTTQLRIVVSSFLRQSGRHVSHLDFVHLTALGYTL
CGLRPEELQHISSWEFSQAALFLGTLHLQCSEEQLEVLAHLLVLPGGFGPISNWGPEIFTEIGTIAAGIPD
LALSALLRGQIQGVTPLAISVIPPPKFAVVFSPIQLSSLTSAQAVAVTPEQMAFLSPEQRRAVAWAQHEGK
ESPEQQGRSTAWGLQDWSRPSWSLVLTISFLGHLL
**Human Wildtype Stereocilin cDNA (SEQ ID NO: 35)** 
atggctctcagcctctggcccctgctgctgctgctgctgctgctgctgctgtcctttgcagtgactct
ggcccctactgggcctcattccctggaccctggtctctccttcctgaagtcattgctctccactctggacc
aggctccccagggctccctgagccgctcacggttctttacattcctggccaacatttcttcttcctttgag
cctgggagaatgggggaaggaccagtaggagagcccccacctctccagccgcctgctctgcggctccatga
ttttctagtgacactgagaggtagccccgactgggagccaatgctagggctgctaggggatatgctggcac
tgctgggacaggagcagactccccgagatttcctggtgcaccaggcaggggtgctgggtggacttgtggag
gtgctgctgggagccttagttcctgggggccccctaccccaactcggcccccatgcacccgtgatgggcc
gtctgactgtgtcctggctgctgactggttgccttctctgctgctgttgttagaggcacacgctggcaag
ctctggtgcaggtgcagcccagtgtggaccccaccaatgccacaggcctcgatgggagggaggcagctcct
cacttttgcagggtctgttgggtttgcttaccccaacaggggagctaggctccaaggaggctcttgggg
cggtctgctacgcacagtgggggccccctctatgctgcctttcaggagggctgctccgtgtcactcact
ccctgcaggatgaggtcttctccattttggggcagccagagcctgataccaatgggcagtgccaggagtt
ttcttccttactctttccctcctaggtaaccttcaacagctgctcttatggggcgtccggcacaacctttc
ctgggatgtccaggcgctgggctttctgtctggatcaccacccccacccctgccctccttcactgcctga
gcacgggcgtgcctctgcccagagcttctcagccgtcagcccacatcagcccacgccaacggcgagccatc
actgtggaggccctctgtgagaaccacttaggcccagcaccaccctacagcatttccaacttctccatcca
cttgctctgccagcacaccaagcctgccactccacagccccatcccagcaccactgccatctgccagacag
ctgtgtggtatgcagtgtcctgggcaccaggtgcccaaggctggctacaggcctgccacgaccagtttcct
gatgagtttttggatgcgatctgcagtaacctctccttttcagccctgtctggctccaaccgccgcctggt
gaagcggctctgtgctggcctgctcccacccctaccagctgccctgaaggcctgcccctgttcccctca
ccccagacatcttttggggctgcttcttggagaatgagactctgtgggctgagcgactgtgtggggaggca
agtctacaggctgtgcccccagcaaccaggcttgggtccagcatgtgtgccagggccccaccccagatgt
cactgcctccccaccatgccacattggaccctgtggggaacgctgcccggatgggggcagcttcctggtga
tggtctgtgccaatgacaccatgtatgaggtcctggtgcccttctggccttggctagcaggccaatgcagg
ataagtcgtgggggcaatgacacttgcttcctagaaggctgctgggccccttctgccctctctgccacc
actgggaccatccccactctgtctgacccctggccccttcctccttggcatgctatcccagttgccacgct
gtcagtcctctgtcccagctcttgctcaccccacacgcctacactatctcctccgcctgctgaccttcctc
ttgggtccaggggctgggggcgctgaggcccagggggatgctgggtcgggccctactgctctccagtctccc
agacaactgctccttctgggatgcctttcgcccagagggccggcgcagtgtgctacggacgattggggaat
acctggaacaagatgaggagcagccaaccccatcaggctttgaacccactgtcaaccccagctctggtata
agcaagatggagctgctggcctgctttagtcctgtgctgtgggatctgctccagagggaaaagagtgtttg
ggccctgcagattctagtgcaggcgtacctgcatatgcccccagaaaacctccagcagctggtgctttcag
cagagagggaggctgcacaggcttcctgacactcatgctgcaggggaagctgcaggggaagctgcaggta
ccaccatccgaggagcaggccctgggtcgcctgacagccctgctgctccagcggtacccacgcctcacctc
ccagctcttcattgacctgtcaccactcatccctttcttggctgtctctgacctgatgcgcttcccaccat
ccctgttagccaacgacagtgtacagcagggctacagaggtcagaggaaaacagtttggaggaagacaaa
gggttaagacccatgactcctcgcagcctggctgccatccgggattacagcccaggaatgaggcctgaaca
 
gaaggaggctctggcaaagcgactgctggcccctgaactgtttggggaagtgcctgcctggccccaggagc
tgctgtgggcagtgctgcccctgctcccccacctccctctggagaacttttgcagctcagccctcaccag
atccaggccctggaggatagctggccagcagcaggtctggggccagggcatgcccgccatgtgctgcgcag
cctggtaaaccagagtgtccaggatggtgaggagcaggtacgcaggcttgggcccctcgcctgtttcctga
gccctgaggagctgcagagcctagtgcccctgagtgatccaacggggccagtagaacgggggctgctggaa
tgtgcagccaatggaccctcagcccagaaggacgggtggcatatgaacttctgggtgtgttgcgctcatc
tggaggagcggtgctgagcccccgggagctgcgggtctgggcccctctcttctctcagctgggcctccgct
tccttcaggagctgtcagagccccagcttagagccatgcttcctgtcctgcagggaactagtgttacacct
gctcaggctgtcctgctgcttggacggctccttcctaggcacgatctatccctggaggaactctgctcctt
gcaccttctgctaccaggcctcagcccccagacactccaggccatccctaggcgagtcctggtcggggctt
gttcctgcctggcccctgaactgtcacgcctctcagcctgccagaccgcagcactgctgcagacctttcgg
gttaaagatggtgttaaaaatatgggtacaacaggtgctggtccagctgtgtgtatccctggtcagcagcc
tattcccaccacctggccagactgcctgcttcccctgctcccattaaagctgctacaactggattccttgg
ctcttctggcaaatcgaagacgctactgggagctgccctggtctgagcagcaggcacagtttctctggaag
aagatgcaagtacccaccaaccttaccctcaggaatctgcaggctctgggcaccctggcaggaggcatgtc
ctgtgagtttctgcagcagatcaactccatggtagacttccttgaagtggtgcacatgatctatcagctgc
ccactagagttcgagggagcctgagggcctgtatctgggcagagctacagcggaggatggcaatgccagaa
ccagaatggacaactgtagggccagaactgaacgggctggatagcaagctactcctggacttaccgatcca
gttgatggacagactatccaatgaatccattatgttggtggtggagctggtgcaaagagctccagagcagc
tgctggcactgacccccctccaccaggcagccctggcagagagggcactacaaaacctggctccaaaggag
actccagtctcaggggaagtgctggagaccttaggccctttggttggattcctggggacagagagcacacg
acagatcccccctacagatcctgctgtcccatctcagtcagctgcaaggcttctgcctaggagagacatttg
ccacagagctgggatggctgctattgcaggagtctgttcttgggaaaccagagttgtggagccaggatgaa
gtagagcaagctggacgcctagtattcactctgtctactgaggcaatttccttgatccccagggaggcctt
gggtccagagaccctggagcggcttctagaaaagcagcagagctgggagcagagcagagttggacagctgt
gtagggagccacagcttgctgccaagaaagcagccctggtagcaggggtggtgcgaccagctgctgaggat
cttccagaacctgtgccaaattgtgcagatgtacgagggacattcccagcagcctggtctgcaacccagat
tgcagagatggagctctcagactttgaggactgcctgacattatttgcaggagacccaggacttgggcctg
aggaactgcgggcagccatgggcaaagcaaaacagttgtggggtcccccccggggatttcgtcctgagcag
atcctgcagcttggtaggctcttaataggtctaggagatcgggaactacaggagctgatcctagtggactg
gggagtgctgagcaccctggggcagatagatggctggagcaccactcagctccgcattgtggtctccagtt
tcctacggcagagtggtcggcatgtgagccacctggacttcgttcatctgacagcgctgggttatactctc
tgtggactgcggccagaggagctccagcacatcagcagttgggagttcagccaagcagctctcttcctcgg
caccctgcatctccagtgctctgaggaacaactggaggttctggcccacctacttgtactgcctggtgggt
ttggcccaatcagtaactgggggcctgagatcttcactgaaattggcaccatagcagctgggatcccagac
ctggctctttcagcactgctgcggggacagatccagggcgttactcctcttgccatttctgtcatccctcc
tcctaaatttgctgtggtgtttagtcccatccaactatctagtctcaccagtgctcaggctgtggctgtca
ctcctgagcaaatggcctttctgagtcctgagcagcgacgagcagttgcatgggcccaacatgagggaaag
 
gagagcccagaacagcaaggtcgaagtacagcctggggcctccaggactggtcacgaccttcctggtccct
ggtattgactatcagcttccttggccacctgctatga
**Human Wildtype Stereocilin cDNA - Codon Optimized (SEQ ID NO: 36)**

atggctctgtctctgtggcctctgctgctgctcctgttgctgcttctgctgctcagcttcgccgtgacact
ggctccaacaggaccccactctctggatcctggcctgagctttctgaagtccctgctgagcaccctggatc
aggctcctcagggcagcctgagcagatccagattcttcaccttcctggccaacatcagcagcagcttcgag
cctggcagaatgggagaaggacctgtgggagaacctcctccactgcaacctccagctctgcggctgcacga
ttttctggtcacactgagaggcagccccgactgggaacctatgctgggactgctgggagatatgctggccc
tgctcggacaagagcagacccctagagatttcctggtgcatcaggctggcgtgctcggaggactggttgaa
gttctgcttggagcactggtgcctggcggacctcctacacctacaagacctccatgcaccagagatggccc
cagcgattgtgtgctggctgctgattggctgcctagcctgctcctgcttctggaaggcacaagatggcagg
ccctggtgcaggttcagccttctgtggatcctaccaatgccaccggcctggatggaagagaagccgctcca
cactttctgcagggactgcttggactgctcacacctactggcgagctgggctctaaagaagcccttgggg
aggcctgctgagaacagttggagcccctctgtacgccgcctttcaagagggactcctgagagtgacacaca
gcctgcaggacgaggtgttcagcatcctgggacagccagagcctgacaccaatggacagtgtcagggcgtg
ttctttctgaccctgagcctgctgggcaacctgcagcaactgcttctgtggggcgtcagacacaacctgag
ctgggatgtgcaggcactgggctttctgtctggaagccctccacctccaccagcactgctgcattgtctgt
ctactggcgtgccactgcctagagcctctcagcctagcgctcacatcagccccagacagagaagggccatc
accgtggaagctctgtgcgagaatcacctgggacctgctcctccatacagcatcagcaacttctccatcca
tctgctgtgtcagcacaccaagcctgccacacctcagcctcatcctagcaccacagccatctgtcagaccg
ccgtttggtacgccgtttcttgggctcctggtgctcaaggatggctgcaggcctgtcacgatcagttcccc
gacgagttcctggacgccatctgcagcaatctgagcttctctgccctgtccggcagcaatcggagactggt
caaaagactgtgcgccggactgctgcctcctccaacatcttgtcctgagggactgcctcctgtgcctctga
ctcccgatatcttttggggctgcttcctggaaaacgagacactgtgggccgagagactgtgtggcgaagcc
tctctgcaagccgtgcctccatctaatcaggcctgggtgcagcacgtttgtcagggccctacacctgacgt
gacagcctctcctccttgtcacatcggaccttgcggcgagagatgtcctgatggcggcagctttctcgtga
tggtctgcgccaacgacactatgtacgaggtgctggtgcccttctggccttggctggctggccagtgtaga
atctccagaggcggcaacgatacctgtttcctggaaggactcctgggaccactgcttccatctctgcctcc
gcttggaccctctcctctgtgtcttacccctggacctttcctgctggggatgctgtctcagctgcctagat
gccagtctagcgtgccagctctggcccatccaaccagactgcactatctgttgcggctgctgaccttcctc
cttggacctggcgctggcggagctgaagctcaaggcatgcttggcagagccctgctgctgtcatccctgcc
tgacaactgcagcttctgggacgccttcagacctgaaggacgcagaagcgtgctgaggaccatcggcgagt
acctggaacaggatgaggaacagcctacaccaagcggcttcgaacccaccgtgaatcctagcagcggcatc
tccaagatggaactgctggcctgcttcagccccgtgctgtgggatctgctgcagagggaaaaagcgtgtg
ggccctgcagatcctggtccaggcctatctgcacatgcctccagagaacctgcaacagctggtgctgtctg
ccgagagagaagctgcccaggattcctgactctgatgctgcagggaaagctgcagggcaaactccaggtg
ccacctagcgaagaacaggcccttggcagactgacagcactcctgctccagagataccccagactgacctc

tcagctgtttatcgatctgagccctctgatcccattcctggccgtgtccgacctgatgagattccctccta
gtctgctggccaacgactccgtgcagcagggatacagaggcagcgaggaaaacagcctggaagaggacaaa
ggcctgcggcctatgacacctagatctctggccgccatccgggattacagccctggaatgaggcccgagca
gaaagaggccctggctaagagactgctggctcccgagctgtttggcgaagttccagcctggcctcaagaac
tgctgtgggctgttctgcccctgctgccacatctgcctctggaaaacttcctgcagctgagcccacaccag
attcaggccctcgaggattcttggcctgccgctggactcggacctggacatgctagacacgtgctgagaag
cctggtcaaccagagcgttcaggacggcgaggaacaagtgcggagacttggacccctggcctgtttctga
gccccgaggaactgcagagtctggtgcctctgtctgaccctacaggccctgttgaaaggggcctgctggaa
tgtgccgccaatggaacactgagccctgagggcagagtggcctatgaacttctgggagtgctgagatctag
cggcggagccgttctgtcccctagggaacttagagtgtgggcacccctgtttagccagctgggcctgagat
tcctgcaagagctgtctgagccacagctgagggctatgctgcctgtgctccagggcacatctgtgacacca
gctcaagccgtcctgctgttgggcagactgctccctagacacgatctgtccctggaagaactgtgcagcct
gcacttgctgctgcctggactgtctcctcagacactgcaggccattcctcggagagtgcttgtgggagcct
gtagctgtctggcccctgagctgtctagactgagcgcctgtcaaacagccgctctcctgcagaccttcaga
gtgaaggatggcgtgaagaacatgggcaccacaggcgctggacctgccgtgtgtattcctggacagcagcc
cattcctaccacctggccagattgtctgctcccactgctgcccctgaaactgctgcagctggattctctgg
ctctgctggctaaccggcggagatattgggaactgccttggagcgaacagcaggcacagttcctgtggaag
aagatgcaggtccccaccaatctgacactgcggaatctgcaggctctcggcacacttgctggcggaatgag
ctgcgagttcctccagcagatcaacagcatggtggactttctggaagtggtgcacatgatctaccagctgc
caaccagagtgcggggaagcctgagagcttgtatttgggctgaactgcagcggcggatggccatgcctgaa
cctgaatggacaacagtgggccccgagctgaacggcctggactctaaacttctgctggatctccccatcca
gctgatggacagactgagcaacgagagcatcatgctggtggtggaactggtgcagagagccccagaacagc
tgctggcactgacacctctgcatcaagctgctctggccgaacgggcccttcagaatctggctcccaaagaa
acccctgtgtccggggaagtgctggaaacactgggacctcttgtgggcttcctgggcaccgagtctaccag
acagattcctctccagatcctgctgtcccacctgagccagctccagggattttgtctgggcgagacattcg
ccaccgaactcggatggttgctgctccaagagagcgtgctgggaaagcccgaactgtggtcacaggacgaa
gtggaacaggccggcagactggtgtttaccctgtctaccgaggccatcagtctgatccccagagaagcact
gggccctgaaacactcgagaggctgctggaaaagcagcagtcttgggaacagagcagagtgggccagctgt
gtagagaacctcagctggccgctaaaaaggccgcactggttgctggcgttgtcagacctgctgccgaggat
ctgccagagccagtgcctaattgtgccgatgtgcggggcacatttcctgccgcttggagcgctacacagat
cgccgaaatggaactgagcgacttcgaggactgtctgactctgtttgccggcgatcctggactgggaccag
aagaactgagagccgccatgggcaaagccaagcaactttggggacctccaagaggcttcagacccgaacag
attctgcagctcggccgcctgcttatcggactgggcgatagagagctgcaagaactgatcctggtggactg
gggcgtgctgtctactctgggacaaatcgacggctggtccaccacacagctgcggattgtggtgtccagct
tcctgaggcagtctggcagacatgtgtctcacctggacttcgtgcacctgactgccctgggctacacactg
tgtggactgcggcctgaggaacttcagcacatcagctcttgggagttcagccaggcagccctgtttctggg
aaccctgcatctgcagtgctccgaagaacaactggaagtgctcgcccatctgctcgtgctgccaggcggat
ttggccccatctctaattggggacccgagatcttcaccgagatcggcaccattgccgctggcatccctgat

ctggccctgtctgcactgctgagaggacagatccagggcgtgacaccactggccattagcgtgatccctcc
accaaagttcgccgtggtgtttagccccattcagctgtccagcctgacatctgcccaagccgtggctgtga
cccctgaacagatggcatttctgtctcccgagcagcggagagctgttgcttggctcaacacgagggcaaa
gagagtcctgagcaacagggaagaagcaccgcttggggactgcaggattggtccagaccttcttggtcact
ggtgctgaccatcagctttctgggccacctcctgtga

A non-limiting example of a human wildtype stereocilin genomic DNA sequence is SEQ ID NO: 37. The exons in SEQ ID NO: 37 are: nucleotide positions 1-142 (exon 1), nucleotide positions 445-1230 (exon 2), nucleotide positions 1319-1343 (exon 3), nucleotide positions 2111-3368 (exon 4), nucleotide positions 4325-4387 (exon 5), nucleotide positions 4489-4605 (exon 6), nucleotide positions 4748-4914 (exon 7), nucleotide positions 5570-5756 (exon 8), nucleotide positions 5895-6010 (exon 9), nucleotide positions 6292-6424 (exon 10), nucleotide positions 6777-6959 (exon 11), nucleotide positions 7264-7302 (exon 12), nucleotide positions 7486-7653 (exon 13), nucleotide positions 7817-7882 (exon 14), nucleotide positions 8364-8489 (exon 15), nucleotide positions 9467-9525 (exon 16), nucleotide positions 10598-10721 (exon 17), nucleotide positions 10826-10938 (exon 18), nucleotide positions 13402-13537 (exon 19), nucleotide positions 13955-14151 (exon 20), nucleotide positions 14350-14440 (exon 21), nucleotide positions 14649-14805 (exon 22), nucleotide positions 15390-15559 (exon 23), nucleotide positions 17250-17405 (exon 24), nucleotide positions 17787-17929 (exon 25), nucleotide positions 18119-18267 (exon 26), nucleotide positions 18509-18605 (exon 27), nucleotide positions 18694-18841 (exon 28), and nucleotide positions 19041-19238 (exon 29). The introns are located between each of these exons in SEQ ID NO: 37, i.e., at nucleotide positions 143-444 (intron 1), nucleotide positions 1231 to 1318 (intron 2), nucleotide positions 1344-2110 (intron 3), nucleotide positions 3369-4324 (intron 4), nucleotide positions 4388-4488 (intron 5), nucleotide positions 4606-4747 (intron 6), nucleotide positions 4915-5569 (intron 7), nucleotide positions 5757-5894 (intron 8), nucleotide positions 6011-6291 (intron 9), nucleotide positions 6425-6776 (intron 10), nucleotide position 6960-7263 (intron 11), nucleotide positions 7303-7485 (intron 12), nucleotide positions 7654-7816 (intron 13), nucleotide positions 7883-8363 (intron 14), nucleotide positions 8490-9466 (intron 15), nucleotide positions 9526-10597 (intron 16), nucleotide positions 10722-10825 (intron 17), nucleotide positions 10939-13401 (intron 18), nucleotide positions 13538-13954 (intron 19), nucleotide positions 14152-14349 (intron 20), nucleotide positions 14441-14648 (intron 21), nucleotide positions 14806-15389 (intron 22), nucleotide positions 15560-17249 (intron 23), nucleotide positions 17406-17786 (intron 24), nucleotide positions 17930-18118 (intron 25), nucleotide positions 18268-18508 (intron 26), nucleotide positions 18606-18693 (intron 27), and nucleotide positions 18842-19040 (intron 28).

**Human Wildtype Stereocilin Gene (SEQ ID NO: 37)** (NCBI Accession No. NG_011636.1)

1 gccctgccct cacctggcta tcccacacag gtgagaataa ccagaactca cctccggtac
61 cagtgttcac ttggaaacat ggctctcagc ctctggcccc tgctgctgct gctgctgctg
121 ctgctgctgc tgtcctttgc aggtaagaag aacagtgagc agaactgggg atgaggagga
181 gggtggctgg aaaaagactt taagaatatg gaggtgaacc tgttagatag aaggacaaag
241 gagagaggca gagacttgtg caaaagggaa aaatgagggt taagaaaagc aggccaagac
301 ttactgtagg ccagtgaaag gggttcagct caccatcccc tcacctcatc tttagatcca
361 ggtagggaac tgtgctcagg ggcagggttg agtttgggct ctgtgttcct ctccttcagt
421 gacctctggt ttctctcctt acagtgactc tggcccctac tgggcctcat tccctggacc
481 ctggtctctc cttcctgaag tcattgctct ccactctgga ccaggctccc caggctccc
541 tgagccgctc acggttcttt acattcctgg ccaacatttc ttcttccttt gagcctggga
601 gaatggggga aggaccagta ggagagcccc cacctctcca gccgcctgct ctgcggctcc
661 atgatttctt agtgacactg agaggtagcc ccgactggga gccaatgcta gggctgctag
721 gggatatgct ggcactgctg ggacaggagc agactccccg agatttcctg gtgcaccagg
781 caggggtgct gggtggactt gtggaggtgc tgctgggagc cttagttcct ggggcccccc
841 ctaccccaac tcggccccca tgcacccgtg atgggccgtc tgactgtgtc ctggctgctg
901 actggttgcc ttctctgctg ctgttgttag agggcacacg ctggcaagct ctggtgcagg
961 tgcagcccag tgtggacccc accaatgcca caggcctcga tgggagggag gcagctcctc
1021 actttttgca gggtctgttg ggtttgctta ccccaacagg ggagctaggc tccaaggagg
1081 ctctttgggg cggtctgcta cgcacagtgg gggcccccct ctatgctgcc tttcaggagg
1141 ggctgctccg tgtcactcac tccctgcagg atgaggtctt ctccattttg gggcagccag
1201 agcctgatac caatgggcag tgccagggag gtgagtgtgg ccagggctgg gactgggatg
1261 tggcagggca aggaaagtga aattggggta gttttcttcc ttactctttc cctcctaggt
1321 aaccttcaac agctgctctt atggtaagta acaggagacc agttctgagg gattgggcct
1381 ggaaaatctg gaggtgaaga gctgaagacc tcagcctcta gagaggaaaa ctgatgggag
1441 gagtgtagtt tagtggtttt ggggtgtgac tgtctgggtt ggtgtcccag ctccacctct
1501 tcctagccat atgaccttga gcaggttaca tagtctttct atacctcagt ttccccattt

1561 ataaaatgag aatgataata ttagttacca cagagttgtt gcacccggtt aaatgagttg
1621 atactgtgta tgcaaacgac ttaaaaccgt gctggcacat agcgcttaat aatgttagct
1681 agtaaagatg ggatttggaa aataaggaca cagctggatt cctctacccc cttactactt
1741 cagtacaaca atgccagaca gtagttagac atattgagtt gctgagcaga tttcctaaca
1801 tgaggcccgc tgagggttgt gtttaagcta tctaaaagca tacgaagaaa ggagacagaa
1861 gggggccagg tggacagaaa gaattccaac tggggcttct cctaggtgat tttggacctt
1921 ggcagggcag ctttctcttt tttgccccgt tgcagcattt caaccagtaa cgcctaaact
1981 ctcagggacc tcgcttgtag aaaagcctat gcttgccatg ccccttgagg gctctgagtc
2041 agggtcagaa tcttcagctg gaggaaatgt gaactgacca gatcctgcct gctcctccct
2101 ctgcacccag gggcgtccgg cacaaccttt cctgggatgt ccaggcgctg ggctttctgt
2161 ctggatcacc acccccaccc cctgccctcc ttcactgcct gagcacgggc gtgcctctgc
2221 ccagagcttc tcagccgtca gcccacatca gcccacgcca acggcgagcc atcactgtgg
2281 aggccctctg tgagaaccac ttaggcccag caccacccta cagcatttcc aacttctcca
2341 tccacttgct ctgccagcac accaagcctg ccactccaca gccccatccc agcaccactg
2401 ccatctgcca gacagctgtg tggtatgcag tgtcctgggc accaggtgcc caaggctggc
2461 tacaggcctg ccacgaccag tttcctgatg agtttttgga tgcgatctgc agtaacctct
2521 ccttttcagc cctgtctggc tccaaccgcc gcctggtgaa gcggctctgt gctggcctgc
2581 tcccaccccc taccagctgc cctgaaggcc tgccccctgt tcccctcacc ccagacatct
2641 tttggggctg cttcttggag aatgagactc tgtgggctga gcgactgtgt ggggaggcaa
2701 gtctacaggc tgtgcccccc agcaaccagg cttgggtcca gcatgtgtgc caggccccca
2761 ccccagatgt cactgcctcc ccaccatgcc acattggacc ctgtggggaa cgctgcccgg
2821 atgggggcag cttcctggtg atggtctgtg ccaatgacac catgtatgag gtcctggtgc
2881 ccttctggcc ttggctagca ggccaatgca ggataagtcg tgggggcaat gacacttgct
2941 tcctagaagg gctgctgggc ccccttctgc cctctctgcc accactggga ccatccccac
3001 tctgtctgac ccctggcccc ttcctccttg gcatgctatc ccagttgcca cgctgtcagt
3061 cctctgtccc agctcttgct caccccacac gcctacacta tctcctccgc ctgctgacct
3121 tcctcttggg tccaggggct gggggcgctg aggcccaggg gatgctgggt cgggccctac
3181 tgctctccag tctcccagac aactgctcct tctgggatgc ctttcgccca gagggccggc
3241 gcagtgtgct acggacgatt ggggaatacc tggaacaaga tgaggagcag ccaaccccat
3301 caggctttga acccactgtc aaccccagct ctggtataag caagatggag ctgctggcct
3361 gctttagtgt gagtgctctg ccagagggaa agctcctaga acagtgagaa ggccctccag
3421 gggaattcct cgaatactca gaggcagtag tgtggggtag tagttgaagc acacagctct
3481 agagtcagac aggcttggat tcatatcttg gttctgtgac cagccttgaa tgagttattt
3541 aacttctctg agcaatattt ttctcgtctc atttataaac tagggatgat aatggtatat
3601 gagataatac atgctgtggg cttagcacag tgcatgatac acaaacatgc aataaatatt
3661 accttgttat tcttttgggc tctttgactc tctcactttc tgcaccagaa agaaaaagga
3721 tcaagttaga ggactctaaa tttttcccct agagagtgag aattggaggc tggcagaata
3781 caggaagata aggtaggaat gagaaagatt cagggacact accaatcaga agactttggt

3841 tctaggttca actgtgccac aaattagtgt gatcttaggc aagcaatttc atttagtttt
3901 tctgggcttc agtttttagt ctgtagaatg gaggggtgag aatatgttaa acaccataat
3961 taattcactg agtgcctatt atatgcaagg cactttgcta ggttctgtag gatatataaa
4021 gatttcttac tccatgttgg ggccaccttt ttcaaaccct gggcccagta aaatggaatt
4081 agatagtctc atagtatttg gttcaggtct acaagtatta attgagccaa ctatggacct
4141 ggcatgggag agggtacaag agaaattaga gatatgatcc cggacctaaa agagcttaat
4201 atctgaagaa tcacacttga gatgatggac aagcatccca gcaagtggag ctggaatgcc
4261 tgggggagct gcaggagaga cagagaagac agctctgttg gcatattgtc tttcttccca
4321 ccagcctgtg ctgtgggatc tgctccagag ggaaaagagt gtttgggccc tgcagattct
4381 agtgcaggta acaggtggag ggcacatggg tgggctgggt gacagccatg gctggaggtc
4441 cctgccccgt gaggtgaggc catacccacc atgacctcct attcgcaggc gtacctgcat
4501 atgcccccag aaaacctcca gcagctggtg ctttcagcag agagggaggc tgcacagggc
4561 ttcctgacac tcatgctgca ggggaagctg caggggaagc tgcaggtgag cactgagaaa
4621 ggggagcaag ggcacctgga gcctagtgtt cagagggctt gctttagtgg gaggaggaac
4681 tccagagagg aaatggcagg gatactgagc atctccagag gcagaatcca ttcctgtgcc
4741 cctacaggta ccaccatccg aggagcaggc cctgggtcgc ctgacagccc tgctgctcca
4801 gcggtaccca cgcctcacct cccagctctt cattgacctg tcaccactca tccctttctt
4861 ggctgtctct gacctgatgc gcttcccacc atccctgtta gccaacgaca gtgtgtaagg
4921 ttcttgcact actcctcctg ctcctgtcac ggtcaggcca accgcatcca cctggagcag
4981 ccccttccgg agctcctctc tgtttttttc tttcatgcca gataggcaat gtgccaacat
5041 cgtagcaagg tttgagagag gcacatctca cgcctgagtg tgaaacccca atcattatgc
5101 taatgaacta caaaaggatc agagagctcc tctctattaa aaccagggag aggatgggcg
5161 tggtggctca tgcctgtaat cccagcacgt tgggagcccg aggcaggtgg atcactaggt
5221 ccgcctagtg agttcgagac cagcctggcc aatatggtga aaccccgtct ctattaaact
5281 acaaaaatta gccaggcatg gtggtgggcg cttgtagtcc cagttactct ggaggctgag
5341 gcaggaggat agcttgaacc tgggaggcag aggttgcagt gaaccaagat cgtgccactg
5401 cactccagcc tgggtgacag agcgagactc cgtcttaaaa aaaacaaaaa acaaaacaaa
5461 acaaaaaaac agggagagtc tccttcctat ctagacagca gggctacaga gggtcagagg
5521 aaaacagttt ggaggaagac aaagggttaa gacccatgac tcctcgcagc ctggctgcca
5581 tccgggatta cagcccagga atgaggcctg aacagaagga ggctctggca aagcgactgc
5641 tggcccctga actgtttggg gaagtgcctg cctggcccca ggagctgctg tgggcagtgc
5701 tgcccctgct cccccacctc cctctggaga actttttgca gctcagccct caccaggtat
5761 gagaatcatc ttctttactt gactggccca tcttctgcta gtggggacaa agagtcaatg
5821 gcatgtctct cagtggcccc tccctgcaag aaccctatag tgaccccagt gcgagctaac
5881 cttccccatc tcagatccag gccctggagg atagctggcc agcagcaggt ctggggccag
5941 ggcatgcccg ccatgtgctg cgcagcctgg taaaccagag tgtccaggat ggtgaggagc
6001 aggtacgcag gtgagttgtt gtgggatcag taaccaaggc aagagtggaa gaggtagaga
6061 gaggaaggca cagctgtcac gctgggtcgg tgttctagga agaaaggggc aagagagtag

6121 gcagtggcct caggcagcat agagttccag gagagaggtc tatagatggt gcccctgtgt
6181 agtggtgtag tgtcagagtg cccagtgtat gtacccatac catctgctgc caggcctgcc
6241 ttagtgctag tcttggggac cacacaaagg tcagcttcat gccctcctca ggcttgggcc
6301 cctcgcctgt ttcctgagcc ctgaggagct gcagagccta gtgcccctga gtgatccaac
6361 ggggccagta gaacgggggc tgctggaatg tgcagccaat gggaccctca gcccagaagg
6421 acgggtgagc ccctcagcac aagcctacaa gactttaggc ttcccctggg tctgtgtgga
6481 tggctttccc attgtgtcaa cttgagcaca gtggtgccag cccccatccc acttttgcaa
6541 cctccattcc ttactccatg gccattctta cctgttacca cctcttcctg gcccttctct
6601 atctggtctg tagcacccca aacataccct ttgccatttt gaacctaatc tactccagtc
6661 caatccctag ttccaaaccc tagcccaggc cctgggaaat tcagatgtgg gattagagag
6721 gaagttcaag gttcatctgt cttttctctc cagtcctaaa ccttctttgg ttacaggtgg
6781 catatgaact tctgggtgtg ttgcgctcat ctggaggagc ggtgctgagc ccccgggagc
6841 tgcgggtctg ggcccctctc ttctctcagc tgggcctccg cttccttcag gagctgtcag
6901 agccccagct tagagccatg cttcctgtcc tgcagggaac tagtgttaca cctgctcagg
6961 tttgcctgtc tcactccctg gcatgtaccc tccatccccg cttgagcccc agtcaagaga
7021 atcccattca gggataaaag cagcccctcc tttccctggg tgaacagtag aggtaaactc
7081 tgtctgcagg aggacgcctt cattcccttt cctcagatca agaagggacc tgagtcactg
7141 aggatggtta ctagggatgg ttaagaggca gcgggaagtt ttggagggtt tgccttagga
7201 acccacttag gacctggctg ctgggtcctg agagctgttg ttttcggtcc catcccaaca
7261 caggctgtcc tgctgcttgg acggctcctt cctaggcacg atgtgagtag cagcaacttc
7321 tcagcctccc gccagaggtc tctatcctct tttaacctgg ctcctgcatc tgcccctcct
7381 ctctctccgc tcccctcata cttactgcct tgctgcattg tgattgttgt cttccccaac
7441 acccttccct tcttcttcag gcctcttgtc tctcttgctc tttagctatc cctggaggaa
7501 ctctgctcct tgcaccttct gctaccaggc ctcagccccc agacactcca ggccatccct
7561 aggcgagtcc tggtcggggc ttgttcctgc ctggcccctg aactgtcacg cctctcagcc
7621 tgccagaccg cagcactgct gcagaccttt cgggtatgag agtggcaagg aggatgagat
7681 aatcagggat accggctctt tctggttggg aggaaggcat cttccctgag gccagggaag
7741 gcctttcata cctccccact tacacacaca cacacacaca cacacacaca cacacacaca
7801 accaattctc atgcaggtta aagatggtgt taaaaatatg ggtacaacag gtgctggtcc
7861 agctgtgtgt atccctggtc aggtaagtgt gagatctccc aactgagctc ctctccccat
7921 tctggggcag tttcatatgg ctggtgctac ctcccacact accctgcagt ggccctgaga
7981 gttctggtta gctctgtgcc cattagcagc cctccccagt gccagatgca ggacagcatg
8041 atccactcac attgtcctag actaatgtca aagctggaag ggcctgagaa atcttccagg
8101 ccacccaccc tgctttcaga tgaaaagacc aaggctggga gaagctaagg gactttgttt
8161 gcctggtgcc taactagcag caacacttga ccacagcagc ctgcagtgtg aggctcttag
8221 gcgtttattg ctacagtggc aaatgccatt ccacttctgt cctagctttg gtccctttcc
8281 acccccatgg ttccttttct ctgagtgcta agtacagact ctctcaccta tcactacact
8341 gctataccca tcaccgccag cagcctattc ccaccacctg gccagactgc ctgcttcccc

8401 tgctcccatt aaagctgcta caactggatt ccttggctct tctggcaaat cgaagacgct
8461 actgggagct gccctggtct gagcagcagg taattctccc cacttaattt cagaacttcc
8521 tccctcaatg tagtctacct tctttaccta tcccttagcc ctatttggcc agcttatccc
8581 tactatcctt tatttgattg tttgagatac agtctcactc tgttgcccag gctgcagtgc
8641 agtggcatga tcagagttcg ctgtaacctc aaactcctga gctcaggcaa tctttctgcc
8701 tcagcctcct gaatagctag gacgacaggt ggttaccacc atgcctggct aatttttaaa
8761 tttttttttt gttttttgag atgaagtctt gctctgtcac ccaggcttga gtacagtggc
8821 acaagcttgg ctcactgcaa cctctgtctc ccgggttcaa gcgattctcc tgcctcagcc
8881 tcccgagtag ctgggactac aggcactccc cacaatgcct ggctaatttt tttttgttt
8941 tagtagagac agggtttcac catattggcc aggctggtct cgaactgctg accttgtgat
9001 ctgcctgcct ctgcctctca aagtgctggg attacaggtg tgagccacca tgcccggcca
9061 atttttaaat tttttgtaga gacagacaat acaaaaatgt ggacactatg tggagacact
9121 atgttgaggt actatgctgt ccagattggt cttgaactcc tggcctcaag caatcctcct
9181 gccttggcct cccaaagtgc tgggattaca gacctgagcc actgcaccca gccccctagt
9241 atctcttata atgtgacttg cttttctttt tctttctcct tcccttttct ttcatttctt
9301 tctcactctc gagagaagag tgggcatctg ggagagtggg aggctggtgg gtcccacaga
9361 gtgaggaggc aggactgggt ccaaggcagt cctgcctctc cactctaggg ggtatccttg
9421 gacagtgtct cttctgggaa ggggctcgtc tttctttctc ttgtaggcac agtttctctg
9481 gaagaagatg caagtaccca ccaaccttac cctcaggaat ctgcagtgag taacttgtgt
9541 tgagcagtgc gctgaattcg accaacattt ttttgagtgc ttactatgtg ccaggcacca
9601 tgtgatatgg aatgggggat atagggatga atgatgcata gtccctgcct cgtggacgtt
9661 ctcctagcac ctccctttgc cctcctttcc ttccacagtg ccatgcctat cctgactaga
9721 gccaaaggac tcagaaaacc tggattcagg ttccagtcct gtcacctact tgtcctcttg
9781 ggcaagtcat ttaacgtccc tgtgtcagtt ttcccttctt taaatgagaa ttacaatggc
9841 accagcctca taggtagtta ctgtgaagat taaatgaggt aggtcatgta agatatttaa
9901 cacagtgttt ggtccattgt aaagtcccag tagtcatttg ctactgttag tttacttcag
9961 gatgacttca gaggcactgg ccaagcaaga ataaatagga ataagaaggt atcactttac
10021 ttacacccac attagaagaa caatgggctt cagaatcttt tttttttttt tttttcgag
10081 acagtcttgc tctgttgccc aggctggagt gcagtggcgc gatttcggct cactgcaacc
10141 tctgcctccc aggttcaagc gattctcctg tctcagcctc tggagtagct gggattacag
10201 gaatgtgcca ccatacccag ctaatttttg tatttttagt agagatgggg tttcaccatt
10261 ttggccaggc tggtctcaaa ctcctgacct caggtgatcc acccgcctca gcctcccaag
10321 ggcttcagaa tctaagacat ggctctagtt tcagtttacc acatttctag cagaatgatg
10381 ttgggaatgt cacctgactt ccataaatcc ttattttctc ctctgataaa cagcagtgat
10441 gttatgggga gctgatgaga tatctatgta aaaacatttc tcaaaccata aattacggtg
10501 gatgaacatc tgtacttgtg ttgagagtac tgatatcaag gagcaaacag gctgttgtat
10561 gtgttgaatg agcctctccc cactcacaca cccacagggc tctgggcacc ctggcaggag
10621 gcatgtcctg tgagtttctg cagcagatca actccatggt agacttcctt gaagtggtgc

10681 acatgatcta tcagctgccc actagagttc gagggagcct ggtgagaggg ggtgcctgga
10741 ctttagtggg agcagggagg ctgggaccct aggtatagaa cccagctcct atgttctgct
10801 ctggcctcac actgcttccc tacagagggc ctgtatctgg gcagagctac agcggaggat
10861 ggcaatgcca gaaccagaat ggacaactgt agggccagaa ctgaacgggc tggatagcaa
10921 gctactcctg gacttaccgt aagtactgca gctagagata ttggcccctc agaaagctca
10981 atctggggtg aagatctgcc cttagggaat gccctggagg aggtagtttt tctgtctggt
11041 agttccctga cataatttat agcccaaagc agaggatttt attcaaagtt gctctatgta
11101 ttgactggtt cccagaatat gctccagcac agggcagctg agggtggcaa cactgtattg
11161 aagcctgcca agtaatctta caataaccta gtccacatta attgagattg agacagagca
11221 tctgaagtga gggaggcaat gctccaaatc tgccccagag gattgtagtt tgctcagggc
11281 actgtgttct tagtgcattc agaggagtag atcgagagaa aaatatatga aaaatgtgat
11341 aaataccttc aaatacctga ggggctatca agtagaaatt agattgtcat atttatgagt
11401 ggccccattg ggcaagacta agagtagtta acggagatca gatttttaca tagtataaga
11461 aaaactaagg tagtgagttc ctggtccttg gagctgttcg agcctaagcc agatggcccc
11521 atggcaggaa tgttgtagag cacgttcata tacaggttgt gggaagaaaa ggctatagga
11581 acccaaggct cctccctacc catggagaaa tttattagta tgttactcat atgctgcttt
11641 tctcatttta cccctaccac caccccgttg ccatccgcac tgtaagtcag gataggaaaa
11701 tgctggtgtt acagtcttcc tggggaatat ggagctgaag tggagtaaaa gcagttgact
11761 tcattcctac tttttctttt tttttctttt tttttttttt tgagacagag ttttgctgtg
11821 tcaccaaggc tggagtgcag tgacgtgatc tcggctcact gcaacctcca tcttccaggt
11881 tcaagcaatt ctcctgcctc agtctcccga gtagctggga ctgtaggtgt gcaccaccat
11941 gccaggctaa tttttgtatt tgttgtaggg acgagctttc accatgttgg ccaggctggt
12001 cttgaactcc tggcttcaag tgatctgccc acctcggctt cccaacattc ttatattttt
12061 ataggccttt ccacagattt cagctcttgt atgacttagc ccagttccag aactggtaat
12121 cctaggtagg gtacaggtta tcacctctga tttcgggtaa aagggattta tttatttatt
12181 tgtttattta tttatatttt tgagacagag tctcgctctg tcacccaggc tggagtgcaa
12241 tggtgccatc tcggctcact gcaacctctc cctctggggt tcaagcaatt ctcctgcctc
12301 agcctgctga gtagctggga ttacaggcgc gtgccaccac acccggctaa tttttgcatt
12361 tttagtagag acggggtttc accatgttgc tcagggtggt ctcgaatttc tgaccctgtg
12421 atctgcctgc ctcggcctcc caaagtgctg ggattacagg catgagccac tgcgtccggc
12481 ctgtttttac ttttttttaa tgccattcag atctgtttaa atatgtgggt tctgtgagat
12541 aatttagaat cccaaggtta cagatgaggt gaaagatcct agaccatgca tcaaaaaact
12601 tgagtttctc atttgtgaaa gaaggataag agaaacacct attttgtctg ggtgcagtgg
12661 ctcatgccta taatcccagc atttggggag gccaaggtgg gtggatcacg gaggtcaggt
12721 gttcaagacc agactggcca acatggcaaa acaccatctc tactaaaaat acaaaagtta
12781 gctgggcgtg gtggcacgtg cgtgtaattc cagctattcg ggaggctgag gcacgagaat
12841 tgcttgaacc tgggaggtgc gggttgcagt gaactgagat cgcagcacca ctgtgctcca
12901 gcctgagtga tggagtgagg ccaggtcttg ttgtaggatc aaatgagata acacctgaaa

12961 gaactttgta aattgtatag cacgtacaaa caagaaggga cctcttcaca agcagaggaa
13021 gggtggtcct gtggaaaaaa acgggaattg ggagtgagag acctcaacat ttgatctctg
13081 tgaacctcag ttttttaatc tataaaatgg ggaaatgtta atggtactta atatttggag
13141 cttttgagtc cattagatca ggtaggattg ttcgttattt ttttttttta ggaagactag
13201 aaatatgttg ctcccttttt ctcccccact caagcttgat ggtgggaatt ggccctggag
13261 ctgtttacta tcagttcctg tccagcttca ctaaatttgg tctggggtca catcttagct
13321 gcggactgtg gggttttgtg gtcccttctc gacttggccc agctccacct gaatcctgtt
13381 gttgtcaaat tgctgtaata ggatccagtt gatggacaga ctatccaatg aatccattat
13441 gttggtggtg gagctggtgc aaagagctcc agagcagctg ctggcactga cccccctcca
13501 ccaggcagcc ctggcagaga gggcactaca aaacctggta agagtccacc ctaccagact
13561 cagatttgct gccctgggca attcttgctc ctcagacaat gctctctgac tgtcccccaa
13621 ccctctactt cttgctttct tgctgccaaa cagattcctg tctacaaggc ctggcccctg
13681 ttttgcctct gggttctgtt ccttgataat atgcttcacg ttacttgtcc atacctcttg
13741 gagtccgaga aatctcttgg agtccacctc tcagtctttc tgcctgctcc tatctgggct
13801 cattgcttaa ggaagtgaac aaaggtagtg agcatcatag ggtgctgagc tgggagcagg
13861 agggagggaa ggttaggggg cttggtgtct tgatcaaggt gtctggtatt ctgagtcaga
13921 agtgcattgt ccaagttctg atgctcttct ccaggctcca aaggagactc cagtctcagg
13981 ggaagtgctg gagaccttag gccctttggt tggattcctg gggacagaga gcacacgaca
14041 gatcccccta cagatcctgc tgtcccatct cagtcagctg caaggcttct gcctaggaga
14101 gacatttgcc acagagctgg gatggctgct attgcaggag tctgttcttg ggtatggacc
14161 ttcgagaact tcagattcta actcattcta tacccagtcc ctcagccacc atcatcagtg
14221 gcagcctgtt ccatattctt aaggtcccct ggagccctgt gtccgaaatc ctagcatgtc
14281 ctcttttccc cttccttttc ctcacagttc cctcagctcc ccagcccccg attttcttcc
14341 tgtccccagg aaaccagagt tgtggagcca ggatgaagta gagcaagctg gacgcctagt
14401 attcactctg tctactgagg caatttcctt gatccccagg gtgagatgaa ggaagaaggg
14461 aagggagtaa atgcatagag gggactggtg agctggttat ggggacccgt ggccaaagag
14521 ggcaaaggat atgaagccta gatctggggg gagactgcaa aacagagaca ggactttgga
14581 cttagagcta tagcagcagg tcctgatctg tccagatctc cccactctcc ttctaccttc
14641 tcatgcagga ggccttgggt ccagagaccc tggagcggct tctagaaaag cagcagagct
14701 gggagcagag cagagttgga cagctgtgta gggagccaca gcttgctgcc aagaaagcag
14761 ccctggtagc aggggtggtg cgaccagctg ctgaggatct tccaggtgaa actacccaaa
14821 tacttatatg tccagcagga tgtacaggga gtatcaaacg gtctgggttc tacatgtgct
14881 cttccctggg actgggtttt ctaatttata aagcaaagag tttagaggga tgatcttcaa
14941 gcctcttgta gttctagaat tctgtagttc tgggagtttg taaactatta agtttctttt
15001 tagcccagaa cttccatttt cctgctctct cgtgtctgct ctagactcag ctctagctcg
15061 gctaagtgtg gagctctctg ctggggagat ccctagaagc tttgaaggag acattgtgag
15121 gctggagaac tgggttcaaa ttcagtgcta ccattaaatc tctgaataac atcctcagtc
15181 ttccatctat aaaagtcttg gcatctccaa tcacttcttg ttctattatc tcctaagccc

15241 tatacatatt actctgtaat actcctttga tccctatttc tcacagtgct ctatcctcca
15301 aaggttggaa gactcactct atctacagat atctctctgg gcatatttta ttactgcgct
15361 gacctcctgg ccctgccttc ccccttcaga acctgtgcca aattgtgcag atgtacgagg
15421 gacattccca gcagcctggt ctgcaaccca gattgcagag atggagctct cagactttga
15481 ggactgcctg acattatttg caggagaccc aggacttggg cctgaggaac tgcgggcagc
15541 catgggcaaa gcaaaacagg ttagggatgg agagccaact ggggttggcc atgaggaagc
15601 tatttgggtg tgatgtagga cacaaagaga atggagagtt ggatgagagg tgggggaagc
15661 aagagataga agagttagaa gatttgggtc acaagtagga ggtgaaggga gataaatatt
15721 gaggaaagag agctagtata atgaatagag ggacgaaagc agtggttacc aaattttaat
15781 gcatatcacg atcatcaagg gaacagattt ttttctttat ttttttttct ttcttaaaaa
15841 aataatggca tgcttcggct gggtgcagcg gctcacgcct ataatctcag aactttggga
15901 ggccaaggcg ggcagatcac gaggtcagga gatcaagacc atcctgtcta acacggcgaa
15961 acacggtctc tactaaaaat acaaaaaagt tagccgggca tggtggtgca cacttgttgt
16021 cccagctact tgggaggctg aggcaggaga atggcgtgaa cctgggaggg ggagcttgca
16081 gtgagccgaa gtcaagccaa tgcactccat cctgggtgac agagcaagac tccatctcaa
16141 aaaaaaaaaa aaaaaaaaaa ggcatgcttc atgaatttgc gtgttatcct tgcacaggcg
16201 ccatgcaaat ctctgtatca ttccaatttt ttggggtatg tgctgctgaa ctgagcatgg
16261 gaacagtgcc agtgccagat taccatgctt cactgactta ataaaaacct ttggggaggc
16321 tgggcgcagt gactcatgcc tgtaatcaca gcactttggg aggcggaggc aggtggattg
16381 cttgagccca ggagttagag accagactgg gcaacatggt gaaaccctgt ctctactaaa
16441 aatagaaaaa acattagctg ggtgtggcgg cacatgcctg taatcccagc tactcaggag
16501 gctggggtag gagaatccca tgagtgcagg aggtggaggg tgcaatgtgc caagatcgca
16561 ccactgccct ccagcctggg tgtcagagca agaccctgtc tcataaatta aaaaataagc
16621 ctctggggga aagagtctag acatctgcat ctcctttttt tttttttttt tttttttttg
16681 agacagagtc tcactctgtc acccagcatc caggctggag tgcagtggtg tgatcttggc
16741 tcactgtaac ctctacatcc tgggttcaaa cgatcctcct gcctcagcct ctcaagtagc
16801 tgggactaca ggtgcaccac acctggctaa tttttgtatc tttggtagag atggggtttc
16861 actatgttgc ccaggatggt ctcgaacttc tgggctcaag caatcctccc acctcagcct
16921 cccaaagtgc tgggattaca gctgttagcc actgtgctgg gccctaggca tctgttttaa
16981 taagcgtctc tgtgtctgat gcacataaaa gtgtggaact catggactag agttagtttg
17041 ctcttctttt ccactgattg taatgtcttt caaaacacct tagaggaact gtaaggcaac
17101 ggtctcattt tatagtggag gaaactaaag aaaaggcaaa tgatttacct agagttatac
17161 agctaagggc agaggcaaga cttaaaaccc agcagtatga ctcccaatcc actgcttttc
17221 cactcacatt gttcctgtct ttctcctagt tgtggggtcc cccccgggga tttcgtcctg
17281 agcagatcct gcagcttggt aggctcttaa taggtctagg agatcgggaa ctacaggagc
17341 tgatcctagt ggactgggga gtgctgagca ccctggggca gatagatggc tggagcacca
17401 ctcaggtaac acttttcctc ctccctacgg cttcccaaac acccatccca cagacccagc
17461 cctatagatc atctaaagcc caaggaattt ttttcctgtg accctacctg gtccttcttt

17521 ctatcttttg ttgatacccc atactagtga ccttcaggac tctgatttat tcactctgag
17581 gccctggaca cataatactg tctcctacct cttttcctgg aggcttcctc tttttctttc
17641 cttttctttt ctgagtcctc agccttcccc atgactcctt aggtcttaat agtaacagaa
17701 tataacccag taacacctat cacttccctg tccattaatt ctccataact ttcctccttc
17761 ccctcttctc ccacccccca ccccagctcc gcattgtggt ctccagtttc ctacggcaga
17821 gtggtcggca tgtgagccac ctggacttcg ttcatctgac agcgctgggt tatactctct
17881 gtggactgcg gccagaggag ctccagcaca tcagcagttg ggagttcagg tcatttgtga
17941 aggggctgag ggtggtggtg ctgaggtaaa ggtggactta ctggggaaag aaggatcatg
18001 aaggtctggt cccatggagg aagggaactc atttgaagcc atctcttcct ttgtctcatg
18061 accacagccc ctttcactga agccgaattc ttcttccttc cttcctactg ttctacagcc
18121 aagcagctct cttcctcggc accctgcatc tccagtgctc tgaggaacaa ctggaggttc
18181 tggcccacct acttgtactg cctggtgggt ttggcccaat cagtaactgg gggcctgaga
18241 tcttcactga aattggcacc atagcaggtg gggagctggg ccactgctgg tgcaagttgg
18301 tttggtttct ataccatggg tggactggat ggaagactgc cctgcaattc ttaaggtggg
18361 ggcctgaggg tgtttaaata aggggctaga gacatattgg ggaaggtcta tgatagggca
18421 ctttgggagt agttagagaa ggtctatagg tttgaagaga gggaaggtca gtctaagaca
18481 atgtttggat gccacttgct tcaacagctg ggatcccaga cctggctctt tcagcactgc
18541 tgcggggaca gatccagggc gttactcctc ttgccatttc tgtcatccct cctcctaaat
18601 ttgctgtaag tattaatgga ctggggtgac cacaggagag ccagggccca atggggacta
18661 catgcatgca ctgattccta cccctgccct caggtggtgt ttagtcccat ccaactatct
18721 agtctcacca gtgctcaggc tgtggctgtc actcctgagc aaatggcctt tctgagtcct
18781 gagcagcgac gagcagttgc atgggcccaa catgagggaa aggagagccc agaacagcaa
18841 ggtgagttcc cagctgcaca gcttgatcct ccatctcctg acccagaatc aaacccctaa
18901 tttggtgctg tctggctctt agagtgcacc cagggagatc cctggagtga aggagtctac
18961 aggcagagcg ctaatttcca agtatcaatg ctcctggaga gctgagttgt gatattactc
19021 ccattccctg tctattatag gtcgaagtac agcctggggc ctccaggact ggtcacgacc
19081 ttcctggtcc ctggtattga ctatcagctt ccttggccac ctgctatgag cctgtctcta
19141 cagtagaagg agattgtggg gagagaaatc ttaagtcata atgaataaag tgcaaacaga
19201 agtgcatcct gattattttc agaagctgat gaggaata

The nucleotide sequence of the human STRC pseudogene is shown in SEQ ID NO: 38.

**Human Stereocilin Pseudogene 1 (STRCP1) (SEQ ID NO: 38)**

1 gccctgccct cacctggcta tcccacacag gtgagaataa ccagaactca cctccggtac
61 cagtgttcac ttggaaacat ggctctcagc ctctggcccc tgctgctgct gctgctgctg
121 ctgctgctgc tgtcctttgc aggtaagaag aacagtgagc agaactgggg atgaggagga
181 gggtggctgg aaaaagactt taagaatatg gaggtgaacc tgttagatag aaggacaaag
241 gagagaggca gagacttgtg caaaagggaa aaatgagggt taagaaaagc aggccaagac

301 ttactgtagg ccagtgaaag gggttcagct caccatcccc tcacctcatc tttagatcca
361 ggtagggaac tgtgctcagg ggcagggttg agtttgggct ctgtgttcct ctccttcagt
421 gacctctggt ttctctcctt acagtgactc tggcccctac tgggcctcat tccctggacc
481 ctggtctctc cttcctgaag tcattgctct ccactctgga ccaggctccc cagggctccc
541 tgagccgctc acggttcttt acattcctgg ccaacatttc ttcttccttt gagcctggga
601 gaatggggga aggaccagta ggagagcccc cacctctcca gccgcctgct ctgcggctcc
661 atgatttctt agtgacactg agaggtagcc ccgactggga gccaatgcta gggctgctag
721 gggatatgct ggcactgctg ggacaggagc agactccccg agatttcctg gtgcaccagg
781 caggggtgct gggtggactt gtggaggtgc tgctgggagc cttagttcct ggggggcccc
841 ctaccccaac tcagccccca tgcacccgtg atgggccgtc tgactgtgtc ctggctgctg
901 actggttgcc ttctctgctg ctgttgttag agggcacacg ctggcaagct ctggtgcagg
961 tgcagcccag tgtggacccc accaatgcca caggcctcga tgggagggag gcagctcctc
1021 actttttgca gggtctgttg ggtttgctta ccccaacagg ggagctaggc tccaaggagg
1081 ctctttgggg cggtctgcta cgcacagtgg gggcccccct ctatgctgcc tttcaggagg
1141 ggctgctccg tgtcactcac tccctgcagg atgaggtctt ctccattttg gggcagccag
1201 agcctgatac caatgggcag tgccagggag gtgagtgtgg ccagggctgg gactgggatg
1261 tggcagggca aggaaagtga aattggggta gttttcttcc ttactctttc cctcctaggt
1321 aaccttcaac agctgctctt atggtaagta acaggagacc agttctgagg gattgggcct
1381 ggaaaatctg gaggtgaaga gctgaagacc tcagcctcta gagaggaaaa ctgatgggag
1441 gagtgtagtt tagtggtttt ggggtgtgac tgtctgggtt ggtgtcccag ctccacctct
1501 tcctagccat atgaccttga gcaggttaca tagtctttct atacctcagt ttccccattt
1561 ataaaatgag aatgataata ttagttacca cagagttgtt gcacccggtt aaatgagttg
1621 atactgtgta tgcaaacgac ttaaaaccgt gctggcacat agcgcttaat aatgttagct
1681 agtaaagatg ggatttggaa aataaggaca cagctggatt cctctacccc cttactactt
1741 cagtacaaca atgccagaca gtagttagac atattgagtt gctgagcaga tttcctaaca
1801 tgaggcccgc tgagggttgt gtttaagcta tctaaaagca tatgaagaaa ggagacagaa
1861 gggggccagg tggacagaaa gaattccaac tggggcttct cctaggtgat tttggacctt
1921 ggcagggcag ctttctcttt tttgccccgt tgcagcattt caaccagtaa cgcctaaact
1981 ctcagggacc tcgcttgtag aaaagcctat gcttgccatg ccccttgagg gctctgagtc
2041 agggtcagaa tcttcagctg gaggaaatgt gaactgacca gatcctgcct gctcctccct
2101 ctgcacccag gggcgtccgg cacaaccttt cctgggatgt ccaggcgctg ggctttctgt
2161 ctggatcacc acccccaccc cctgccctcc ttcactgcct gagcacgggc gtgcctctgc
2221 ccagagcttc tcagccgtca gcccacatca gcccacgcca acggcgagcc atcactgtgg
2281 aggccctctg tgagaaccac ttaggcccag caccacccta cagcatttcc aacttctcca
2341 tccacttgct ctgccagcac accaagcctg ccactccaca gccccatccc agcaccactg
2401 ccatctgcca gacagctgtg tggtatgcag tgtcctgggc accaggtgcc caaggctggc
2461 tacaggcctg ccacgaccag tttcctgatg agtttttgga tgcgatctgc agtaacctct
2521 ccttttcagc cctgtctggc tccaaccgcc gcctggtgaa gcggctctgt gctggcctgc

2581 tcccaccccc taccagctgc cctgaaggcc tgccccctgt tcccctcacc ccagacatct
2641 tttggggctg cttcttggag aatgagactc tgtgggctga gcgactgtgt ggggaggcaa
2701 gtctacaggc tgtgcccccc agcaaccagg cttgggtcca gcatgtgtgc cagggcccca
2761 ccccagatgt cactgcctcc ccaccatgcc acattggacc ctgtggggaa cgctgcccgg
2821 atgggggcag cttcctggtg atggtctgtg ccaatgacac catgtatgag gtcctggtgc
2881 ccttctggcc ttggctagca ggccaatgca ggataagtcg tgggggcaat gacacttgct
2941 tcctagaagg gctgctgggc ccccttctgc cctctctgcc accactggga ccatccccac
3001 tctgtctgac ccctggcccc ttcctccttg gcatgctatc ccagttgcca cgctgtcagt
3061 cctctgtccc agctcttgct caccccacac gcctacacta tctcctccgc ctgctgacct
3121 tcctcttggg tccaggggct gggggcgctg aggcccaggg gatgctgggt cgggccctac
3181 tgctctccag tctcccagac aactgctcct tctgggatgc ctttcgccca gagggccggc
3241 gcagtgtgct acggacgatt ggggaatacc tggaacaaga tgaggagcag ccaaccccat
3301 caggctttga acccactgtc aaccccagct ctggtataag caagatggag ctgctggcct
3361 gctttagtgt gagtgctctg ccagagggaa agctcctaga acagtgagaa ggccctccag
3421 gggaattcct cgaatactca gaggcagtag tgtggggtag tagttgaagc acacagctct
3481 agagtcagac aggcttggat tcatatcttg gttctgtgac cagccttgaa tgagttattt
3541 aacttctctg agcaatattt ttctcgtctc atttataaac tagggatgat aatggtatat
3601 gagataatac atgctgtggg cttagcacag tgcatgatac acaaacatgc aataaatatt
3661 accttgttat tcttttgggc tctttgactc tctcactttc tgcaccagaa agaaaaagga
3721 tcaagttaga ggactctaaa tttttcccct agagagtgag aattggaggc tggcagaata
3781 caggaagata aggtaggaat gagaaagatt cagggacact accaatcaga agactttggt
3841 tctaggttca actgtgccac aaattagtgt gatcttaggc aagcaatttc atttagtttt
3901 tctgggcttc agtttttagt ctgtagaatg gaggggtgag aatatgttaa acaccataat
3961 taattcactg agtgcctatt atatgcaagg cactttgcta ggttctgtag gatatataaa
4021 gatttcttac tccatgttgg ggccaccttt ttcaaaccct gggcccagta aaatggaatt
4081 agatagtctc atagtatttg gttcaggtct acaagtatta attgagccaa ctatggacct
4141 ggcatgggag agggtacaag agaaattaga gatatgatcc cggacctaaa agagcttaat
4201 atctgaagaa tcacacttga gatgatggac aagcatccca gcaagtggag ttggaatgcc
4261 tgggggagct gcaggagaga cagagaagac agctctgttg gcatattgtc tttcttccca
4321 ccagcctgtg ctgtgggatc tgctccagag ggaaaagagt gtttgggccc tgcagattct
4381 agtgcaggta acaggtggag ggcacatggg tgggctgggt gacagccatg gctggaggtc
4441 cctgccccgt gaggtgaggc catacccacc atgacctcct attcgcaggc gtacctgcat
4501 atgcccccag aaaacctcca gcagctggtg ctttcagcag agagggaggc tgcacagggc
4561 ttcctgacac tcatgctgca ggggaagctg caggggaagc tgcaggtgag cactgagaaa
4621 ggggagcaag ggcacctgga gcctagtgtt cagagggctt gctttagtgg gaggaggaac
4681 tccagagagg aaatggcagg gatactgagc atctccagag gcagaatcca ttcctgtgcc
4741 cctacaggta ccaccatccg aggagcaggc cctgggtcgc ctgacagccc tgctgctcca
4801 gcggtaccca cgcctcacct cccagctctt cattgacctg tcaccactca tccctttctt

4861 ggctgtctct gacctgatgc gcttcccacc atccctgtta gccaacgaca gtgtgtaagg
4921 ttcttgcact actcctcctg ctcctgtcac ggtcaggcca accgcatcca cctggagcag
4981 ccccttccgg agctcctctc tgtttttttc tttcatgcca gataggcaat gtgccaacat
5041 cgtagcaagg tttgagagag gcacatctca cgcctgagtg tgaaaaccca atcattatgc
5101 taatgaacta caaaaggatc agagagctcc tctctattaa aaccagggag aggatgggcg
5161 tggtggctca tgcctgtaat cccagcacgt tgggagcccg aggcaggtgg atcactaggt
5221 ccgcctagtg agttcgagac cagcctggcc aatatggtga aaccctgtct ctattaaact
5281 acaaaaatta gccaggcatg gtggtgggcg cttgtagtcc cagttactct ggaggctgag
5341 gcaggaggat agcttgaacc tgggaggcag aggttgcagt gagccaagat cgtgccactg
5401 cactccagcc tgggtgacag agcgagactc cgtcttaaaa aaaacaaaaa acaaaacaaa
5461 acaaaaaaac agggagagtc tccttcctat ctagacagca gggctacaga gggtcagagg
5521 aaaacagttt ggaggaagac aaagggttaa gacccatgac tcctcgcagc ctggctgcca
5581 tccgggatta cagcccagga atgaggcctg aacagaagga ggctctggca aagcgactgc
5641 tggcccctga actgtttggg gaagtgcctg cctggcccca ggagctgctg tgggcagtgc
5701 tgcccctgct cccccacctc cctctggaga actttttgca gctcagccct caccaggtat
5761 gagaatcatc ttctttactt gactggccca tcttctgcta gtggggacaa agagtcaatg
5821 gcatgtctct cagtggcccc tccctgcaag aaccctatag tgaccccagt gcgagctaac
5881 cttccccatc tcagatccag gccctggagg atagctggcc agcagcaggt ctggggccag
5941 ggcatgcccg ccatgtgctg cgcagcctgg taaaccagag tgtccaggat ggtgaggagc
6001 aggtacgcag gtgagttgtt gtgggatcag taaccaaggc aagagtggaa gaggtagaga
6061 gaggaaggca cagctgtcac gctgggtcgg tgttctagga agaaaggggc aagagagtag
6121 gcagtggcct caggcagcat agagttccag gagagaggtc tatagatggt gcccctgtgt
6181 agtggtgtag tgtcagagtg cccagtgtat gtacccatac catctgctgc caggcctgcc
6241 ttagtgctag tcttggggac cacacaaagg tcagcttcat gccctcctca ggcttgggcc
6301 cctcgcctgt ttcctgagcc ctgaggagct gcagagccta gtgcccctga gtgatccaac
6361 ggggccagta gaacgggggc tgctggaatg tgcagccaat gggaccctca gcccagaagg
6421 acgggtgagc ccctcagcac aagcctacaa gactttaggc ttcccctggg tctgtgtgga
6481 tggctttccc attgtgtcaa cttgagcaca gtggtgccag cccccatccc acttttgcaa
6541 cctccattcc ttactccatg gccattctta cctgttacca cctcttcctg gcccttctct
6601 atctggtctg tagcacccca aacataccct ttgccatttt gaacctaatc tactccagtc
6661 caatccctag ttccaaaccc tagcccaggc cctgggaaat tcagatgtgg gattagagag
6721 gaagttcaag gttcatctgt cttttctctc cagtcctaaa ccttctttgg ttacaggtgg
6781 catatgaact tctgggtgtg ttgcgctcat ctggaggagc ggtgctgagc cccgggagc
6841 tgcgggtctg ggcccctctc ttctctcagc tgggcctccg cttccttcag gagctgtcag
6901 agccccagct tagagccatg cttcctgtcc tgcagggaac tagtgttaca cctgctcagg
6961 tttgcctgtc tcactccctg gcatgtaccc tccatccccg cttgagcccc agtcaagaga
7021 atcccattca gggataaaag cagcccctcc tttccctggg tgaacagtag aggtaaactc
7081 tgtctgcagg aggacgcctt cattcccttt cctcagatca agaagggacc tgagtcactg

7141 aggatggtta ctggggatgg ttaagaggca gcgggaagtt ttggagggtt tgccttagga
7201 acccacttag gacctggctg ctgggtcctg agagctgttg ttttcggtcc catcccaaca
7261 caggctgtcc tgctgcttgg acggctcctt cctaggcacg atgtgagtag cagcaacttc
7321 tcagcctccc gccagaggtc tctatcctct tttaacctgg ctcctgcatc tgcccctcct
7381 ctctctccgc tcccctcata cttactgcct tgctgcattg tgattgttgt cttccccaac
7441 acccttccct tcttcttcag gcctcttgtc tctcttgctc tttagctatc cctggaggaa
7501 ctctgctcct tgcaccttct gctaccaggc ctcagccccc agacactcca ggccatccct
7561 aggcgagtcc tggtcggggc ttgttcctgc ctggcccctg aactgtcacg cctctcagcc
7621 tgccagaccg cagcactgct gcagaccttt cgggtatgag agtggcaagg aggatgagat
7681 aatcagggat accggctctt tctggttggg aggaaggcat cttccctgag gccagggaag
7741 gcctttcata cctccccact tacacacaca cacacacaca cacacacaca accaattctc
7801 atgcaggtta aagatggtgt taaaaatatg ggtacaacag gtgctggtcc agctgtgtgc
7861 atccctggtc aggtaagtgt gagatctccc aactgagctc ctctccccat tctggggcag
7921 tttcatatgg ctggtgctac ctcccacact accctgcagt ggccctgaga gttctggtta
7981 gctctgtgcc cattagcagc cctccccagt gccagatgca ggacagcatg atccactcac
8041 attgtcctag actaatgtca aagctggaag ggcctgagaa atcttccagg ccacccaccc
8101 tgctttcaga tgaaaagacc aaggctggga gaagctaagg gactttgttt gcctggtgcc
8161 taactagcag caacacttga ccacagcagc ctgcagtgtg aggctcttag gcgtttattg
8221 ctacagtggc aaatgccatt ccacttctgt cctagctttg gtccctttcc acccccatgg
8281 ttccttttct ctgagtgcta agtacagact ctctcaccta tcactacact gctataccca
8341 tcaccgccag cagcctattc ccaccacctg gccagactgc ctgcttcccc tgctcccatt
8401 aaagctgcta caactggatt ccttggctct tctggcaaat cgaagacgct actgggagct
8461 gccctggtct gagcagcagg taattctccc cacttaattt cagaacttcc tccctcaatg
8521 tagtctacct tctttaccta tcccttagcc ctatttggcc agcttatccc tactatcctt
8581 tatttgattg tttgagatac agtctcactc tgttgcccag gctgcagtgc agtggcatga
8641 tcagagttcg ctgtaacctc aaactcatga gctcaggcaa tctttctgcc tcagcctcct
8701 gaatagctag gatgacaggt ggttaccacc atgcctggct aatttttaaa tttttttttt
8761 gttttttgag atgaagtctt gctctgtcac ccaggcttga gtacagtggc acaagcttgg
8821 ctcactgcaa cctctgtctc ccgggttcaa gcgattctcc tgcctcagcc tcccgagtag
8881 ctgggactac aggcactccc cacaatgccc ggctaatttt ttttttgttt tagtagagac
8941 agggtttcac catattggcc aggctggtct cgaactgctg accttgtgat ctgcctgcct
9001 ctgcctctca aagtgctggg attacaggtg tgagccacca tgcccggcca atttttaaat
9061 tttttgtaga gacagacaat acaaaaatgt ggacactatg tggagacact atgttgaggt
9121 actatgctgt ccagattggt cttgaactcc tggcctcaag caatcctcct gccttggcct
9181 cccaaagtgc tgggattaca gacctgagcc actgcaccca gccccctagt atcccttata
9241 atgtgacttg cttttctttt tctttctcct tccctttctt ttcatttctt tctcactctc
9301 gagagaagag tgggcatctg ggagagtggg aggctggtgg gtcccacaga gtgaggaggc
9361 aggactgggt ccaaggcagt cctgcctctc cactctaggg ggtatccttg gacagtgtct

9421 cttctgggaa ggggctcgtc tttctttctc ttgtaggcac agtttctctg gaagaagatg
9481 caagtaccca ccaacctgac cctcaggaat ctacagtgag taacttgtgt tgagcagtgc
9541 gctgaattcg accaacattt ttttgagtgc ttactatgtg ccaggcacca tatgatatgg
9601 aatgggggat atagggatga atgatgcata gtccctgcct cgtggacgtt ctcctagcac
9661 ctccctttgc cctcctttcc ttccacagtg ccatgcctat cctgactaga gccaaaggac
9721 tcagaaaacc tggattcagg ttccagtcct gtcacctact tgtcctcttg ggcaagtcat
9781 ttaacgtccc tgtgtcagtt ttcccttctt taaatgagaa ttacaatggc accagcctca
9841 taggtagtta ctgtgaagat taaatgaggt aggtcatgta agatatttaa cacagtgttt
9901 ggtccattgt aaagttccag tagtcatttg ctactgttag tttacttcag gatgacttca
9961 gaggcactgg ccaagcaaga ataaatagga ataagaaggt atcactttac ttatacccac
10021 attagaagaa caatgggctt cagaatcttt tttttttttt ttttcgagac agtcttgctc
10081 tgttgcccag gctggagtgc agtggcgcga tttcggctca ctgcaacctc tgcctcccag
10141 gttcaagcga ttctcctgtc tcagcctctg gagtagctgg gattacagga atgtgccacc
10201 atacccagct aatttttgta tttttagtag agatggggtt tcaccatttt ggccaggctg
10261 gtctcaaact cctgacctca ggtgatccac ccgcctcagc ctcccaaggg cttcagaatc
10321 taagacatgt ctctagtttc agtttaccac atttctagca gaatgatgtt gggaatgtca
10381 cctgacttcc ataaatcctt attttctcct ctgataaaca gcagtgatgt tatggggagc
10441 tgatgagata tctatgtaaa aacatttctc aaaccataaa ttacggtgga tgaacatctg
10501 tacttgtgtt gagagtactg atatcaagga gcaaacaggc tgttgtatgt gttgaatgag
10561 cctctcccca ctcacacacc cacagggctc tgggcaccct ggcaggaggc atgtcctgtg
10621 agtttctgca gcagatcaac tccatggtag acttccttga agtggtgcac atgatctatc
10681 agctgcccac tagagttcga gggagcctgg tgagagggga tgcctggact ttagtgggag
10741 cagggaggct gggaccctag gtatagaacc cagctcctat gttctgctct ggcctcactc
10801 tgcttcccta cagagggcct gtatctgggc agagctacag cggaggatgg caatgccaga
10861 accagaatgg acaactgtag ggccagaact gaacgggctg gatagcaagc tactcctgga
10921 cttaccgtaa gtactgcagc tagagatatt ggcccctcag aaagctcaat ctggggtgaa
10981 gatctgccct tagggaatgc cctggaggag gtagtttttc tgtctggtag ttccctgaca
11041 taatttatag cccaaagcag aggattttat tcaaagttgc tctatgtatt gactggttcc
11101 cagaatatgc tccagcacag ggcagctgag ggtggcaaca ctgtattgaa gcctgccaag
11161 taatcttaca ataacctagt ccacattaat tgagattgag acagagcatc tgaagtgagg
11221 gaggcaatgc tccaaatctg ccccagagga ttgtagtttg ctcagggcac tgtgttctta
11281 gtgcattcag aggagtggat cgagagaaaa atatatgaaa aatgtgataa ataccttcaa
11341 atacctgagg ggctatcaag tagaaattag attgtcatat ttatgagtgg ccccattggg
11401 caagactaag agtagttaac ggagatcaga tttttacata gtataagaaa aactaaggta
11461 gtgagttcct ggtccttgga gctgttcgag cctaagccag atggccccat ggcaggaatg
11521 ttgtagagca cgttcatata caggttgtgg gaagaaaagg ctataggaac ccaaggctcc
11581 tccctaccca tggagaaatt tattagtatg ttactcatat gctgcttttc tcatttacc
11641 cctaccacca ccccgttgcc atccgcactg taagtcagga taggaaaatg ctggtgttac

11701 agtcttcctg gggaatatgg agctgaagtg gagtaaaagc agttgacttc attcttactt
11761 ttttcttttt cttttttttt ttttttttga gacagagttt tgctgtgtca ccaaggctgg
11821 agtgcagtga cgtgatctcg gctcactgca acctccatct tccaggttca agcaattctc
11881 ctgcctcagt ctcccgagta gctgggactg taggtgtgca ccaccatgcc aggctaattt
11941 ttgtatttgt tgtagggacg agctttcacc atgttggcca ggctggtctt gaactcctgg
12001 cttcaagtga tctgcccacc tcggcttccc aacattctta tatttttata ggcctttcca
12061 cagatttcag ctcttgtatg acttagccca gttccagaac tggtaatcct aggtagggta
12121 caggttatca cctctgattt cgggtaaaag ggatttattt atttatttgt ttatttattt
12181 atatttttga gacagagtct cgctctgtca cccaggctgg agtgcaatgg tgccatctcg
12241 gctcactgca acctctccct ctggggttca agcacttctg cctcagcctc ctgagtagct
12301 gggattacag gcgcgtgcca ccacacccgg ctaatttttg catttttagt agagacgggg
12361 tttcaccatg ttgctcaggg tggtctcgaa tttctgaccc tgtgatctgc ctgcctcggc
12421 ctcccaaagt gctgggatta caggcatgag ccactgcgtc cggcctgttt ttactttttt
12481 ttaatgccat tcagatctgt ttaaatatgt gggttctgtg agataattta gaatcccaag
12541 gttacagatg aggtgaaaga tcctagacca tgcatcaaaa aacttgagtt tctcatttgt
12601 gaaagaagga taagagaaac acctattttg tctgggtgca gtggctcatg cctataatcc
12661 cagcatttgg ggaggccaag gtgggtggat cacggaggtc aggtgttcaa gaccagactg
12721 gccaacatgg caaaacacca tctctactaa aaatacaaaa gttagctggg cgtggtggca
12781 cgtgcgtgta attccagcta ttcgggaggc tgaggcacga gaattgcttg aacctgggag
12841 gtgcgggttg cagtgaactg agatcgcagc accactgtgc tccagcctga gtgatggagt
12901 gaggccaggt cttgttgtag gatcaaatga gataacacct gaaagaactt tgtaaattgt
12961 atagcacgta caaacaagaa gggacctctt cacaagcaga ggaagggtgg tcctgtggaa
13021 aaaaacggga attgggagtg agagacctca acatttgatc tctgtgaacc tcagtttttt
13081 aatctataaa atggggaaat gttaatggta cttaatattt ggagcttttg agtccattag
13141 atcaggtagg attgttcgtt attttttttt tttaggaaga ctagaaatat gttgctccct
13201 ttttctcccc cactcaagct tgatggtggg aattggccct ggagctgttt actgtcagtt
13261 cctgtccagc ttcactaaat ttggtctggg gtcacatctt agctggggac tgtggggttc
13321 tgtggtccct tctcgacttg gcccagctcc acttgaatga tattgttgtc aaattgctat
13381 aatagaatcc agttgatgga cagactatcc aatgaatcca ttatgttggt ggtggagctg
13441 gtgcaaagag ctccagagca gctgctggca ctgacccccc tccaccaggc agccctggca
13501 gagagggcac tacaaaacct ggtaagagtc caccctacca gactcagatt tgctgccctg
13561 ggcaattctt gctcctcaga caatgctctc tgactgtcct ccaaccctct acttcttgct
13621 ttcttgctgc caaacaggtt cctgtctaca aggcctggcc cgttttgcct ctgggttctg
13681 ttccttgata atatgcttca cgttacttgt ccatacctct tggagtccga gaaatctctt
13741 ggagtccacc tctcagtctt tctgcctgct cctatctggt ctcattgctt aagaaagtga
13801 acaaaggtag tgagcatcat agggtgctga gttgggagca ggagggaggg aaggtcaggg
13861 ggcttggtgt cttgatcaag gtgtctggta ttctgagtca gaagtgcatt gtccaagttc
13921 tgatgctctt ctccaggctc caaaggagac tccagtctca ggggaagtgc tggagacctt

13981 aggccctttg gttggattcc tggggacaga gagcacacga cagatccccc tacagatcct
14041 gctgtcccat ctcagtcagc tgtaaggctt ctgcctagga gagacatttg ccacagagct
14101 gggatggctg ctattgcagg agtctgttct tgggtatgga tcttcgagaa cttcagattc
14161 taactcattc tatacccagt ccctcagcca ccatcatcag tggcagcctg ttccatattc
14221 ctaagggccc ttggagccct gtgtccgaaa tcctagcatg tcctcttttc cccttccttt
14281 tcctcacagt tccctcagct ccccagcccc cgattttctt cctgtcccca ggaaaccaga
14341 gttgtggagc caggatgaag tagagcaagc tggacgccta gtattcactc tgtctactga
14401 ggcaatttcc ttgatcccca gggtgagatg aaggaagaag ggaagggagt aaatgcatag
14461 aggggactgg tgagctggtt atggggaccc gtggccaacg agggcaaagg atatgaagcc
14521 tagatctggg gggatactgc gaaacagaga caggactttg gacttatagc tatagcagca
14581 ggtcctgatc tgtccagatc tccccactct ccttctacct tctcatgcag gaggccttgg
14641 gtccagagac cctggagcgg cttctagaaa agcagcagag ctgggagcag agcagagttg
14701 gacagctgtg tagggggcca cagcttgctg ccaagaaagc agccctggta gcaggggtgg
14761 tgcgaccagc tgctgaggat cttccaggtg aaactaccca aatacttata tgtccagcag
14821 gatgtacagg gagtatcaaa cggtctgggt tctacatgtg cttttctctg ggactgggtt
14881 ttctaattta taaagcaaag agtttagagg gatgatcttc aagtctcgtc tagttctaaa
14941 attctatagc tctgggagtg tgtaaactat taagttttcc tttagcccag aacttccatt
15001 ttcctgctgt cttgtctctt taaacagctg actcagctct agctcggcta agtgtggagc
15061 tctctgctgg ggagatccct cgaaggagac attgtgaggc tagagaactg gtttcaaatt
15121 cagctctgcc atgaaatccc tgaccaacat cctcagtctt ccatctataa aatgagggac
15181 ttggcatctc caatcacttc ttgttcaatt cacttctaag ccctatacat cttaacctgt
15241 aatactccct cgatccctac ttcccacagt gctccatcct ccaaaggttg gaagagtcat
15301 tccatctaca gatacctcct ggccctgcct tcccccttca gaacctgtgc caaattgtgc
15361 agatgtacga gggacattcc cagcagcctg ctctgcaacc cagattgctg agatggagct
15421 ctcagacttt aaggactgcc tgacactatt tgcaggagac ccaggacttg ggcctgagga
15481 accacgggca gccatgggca aagcaaaatg ggtcagggac ggagagccaa ctgaggttgg
15541 ccatgaggaa gctgtgtggg tgtgatgtag gacacgaaga gaatagagag ttggatgaga
15601 ggtgggggaa gcagaagata aaagagttag aagatttggg tcacaagtag aaggtgagga
15661 gagataaata ttgaggaaag agagctacta taatgaatag agggactaaa gcagtgatta
15721 ccaaatttta atgcatatca caatcatcaa gggaacagat ttttttcttt tttctttttt
15781 tttctttctt aaaaaataat ggcatacttc atgaatttgt gtgttatcct tgcgcaggcg
15841 ccatgctaat ctctgtatca ttccaatttt tttttgtata tgtgctgctg aaccgagcac
15901 gggaacagtg ccaatgctgg attaccatgc tagactgact taataaaaac ctttgcggag
15961 gctggacgga gtggctcgtg cctgtaatca cagcactttg ggaggctgag gcaggtggat
16021 tgcttgagcc caggagtttg agaccagact gggcaacatg gtgaagccct gtctctacta
16081 aaaatagaaa aaaaattagc tgggtgtggc ggcacatgcc tgtaatccca gctactcagg
16141 aggctggggt aggagaatcc cctgagcgca ggaggtggag ggtgcaatga gccaagatca
16201 caccactgca ctccagcctg ggtgtcagag caagaccctg tctcataaat taaaaaataa

16261 gcctctggga gaaagtctag acatctgcat ctgctttttt tttttttttt tttttgaga
16321 cacagtctca ctctgtcacc cagcatccag gctggagtgc ggtggtgtga tcttggctca
16381 ctgtaacctc tacatcctgg gctcaaacga tcctcctgcc tcagcctctc gagaagctgg
16441 gactacaggt gcacaccact acacctggct aatttttgta tttttggtag agatgaggtt
16501 tcgctgtgtt gcccaggatg gtcttgaact cctgggctca agcattcctc ccacctcagc
16561 ctcccaaagt gctgggatta cagctgtgag ccactgtgct gggccccagg catctgcatt
16621 ttaataagcg tctctgtgtc tgatgcacat aaaagtgtgg aactcatgga ctagagttag
16681 tttgctcttc ttttccactg attgtaatgt ctttcaaaac accttagagg aactgtaagg
16741 caacggtctc attttatagt ggaggaaacc aaagaaaagg caagtgactt gcctagagtt
16801 atacagctaa ggtcagaggc aagacttaaa acccagcatt ctgactccca atctactggt
16861 tttccactca cattgttcct ctctttctcc tagttgtggg gtcccccccg gggatttggt
16921 cctgagcaga tcctgcagct cggtagactc ttaataggtc tgggagatca ggaactacag
16981 gagctgatcc tagtggactg gggagtgctg agcaccctgg ggcagataga tggctggagc
17041 tccactcagg taacactttt actcctccct accgcttccc aaacacccat cccacagacc
17101 cagccctata gatcatctaa agcccaagga attttttcct gtgaccctac ctggtcattc
17161 tttctgtctt ttgttaatat cccatactag tgaccttcag gactcttgat ttattcactc
17221 tgaggccctg gacacataat actgtctcct acctcttttc ctggaggctt cctctttttc
17281 tttccttttc ttttctgagt cctcagcctt ccccatgact ccttaggtct taatagtaac
17341 agaatataac ccagtaacac ctatcacttc cctgtccatt aattctccat aactttcctc
17401 cttcccctct tctcccaccc cccaccccag ctccgcattg tggtctccag tttcctacgg
17461 cagagtggtc ggcatgtgag ccacctggac ttcgttcatc tgacagcgct gggttatact
17521 ctctgtggac tgcggccaga ggagctccag cacatcagca gttgggagtt taggtcattt
17581 gtgaaggggc tgagggtggt cgtgttcagg taaaggtgga cttgctgggg aaaggaggat
17641 catgaagtta tagtcccatg gaggaaggga actcatttga agccatctct tcctttgtct
17701 catgaccaca gtccctttca ctgaagccga attcttcttc cttccttccc actgttctac
17761 agccaagcag ctctcttcct gggcaccctg catctgcagt gctctgagga acaactggag
17821 tttctggccc acctctttgt actgcctggt gggtttggcc caatcagtaa ctgggggcct
17881 gagatcttca ctgaaattgg caccatagca ggtggggagc tgggccactg ctggtgcaag
17941 ttggtttggt ttctatacca tgggtggact ggatggaaga ctgccctgca attcttcagg
18001 tgtgggcctg agagggtgtt taaataaggg gctagagaca tattggggaa ggtctatgat
18061 agggcacttt gggagtagtt agagaaggtc tataggtttg aagagaggga aggtcagtct
18121 aagacaatgt ttggatgcca cttgcttcaa cagctgggat cccagacctg gctctttcag
18181 cactgctgcg gggacagatc cagggcgtta ctcctcttgc catttctgtc atccctcctc
18241 ctaaatttgc tgtaagtatt aatggactgg ggtgaccaca ggagagccag ggcccaatgg
18301 ggactacatg catgcactga ttcctacccc tgccctcagg tggtgtttag tcccatccaa
18361 ctatctagtc tcgccagtgc tcaggctgtg gctgtcactc ctgagcaaat ggcctttctg
18421 agtcctgagc agcgacgagc agttgcatgg gcccaacatg agggaaagga gagcccagaa
18481 cagcaaggtg agttcccagc tgcacagctt gatcctccat ctcctgaccc agaatcaaac

18541 ccctaatttg gtgctgtctg gctcttagag tgcacccagg gagatccctg gagtgaagga

18601 gtctacaggc agagcgctaa tttccaagta tcaatgctcc tggagagctg agttgtgata

18661 ttactcccat tccctgtcta ttataggtcg aagtacagcc tggggcctcc aggactggtc

18721 acgaccttcc tggtccctgg tattgactat cagcttcctt ggccacctgc tatgagcctg

18781 tctctacagt agaaggagat tgtggggaga gaaatcttaa gtcataatga ataaagtgca

18841 aacagaagtg catcctgatt attttcagaa gctgatgagg aata

### Clarin 1 (CLRN1)

The CLRN1 gene encodes "clarin 1" (CLRN1), a protein that is expressed in hair cells of the inner ear (e.g., inner ear hair cells, outer ear hair cells) and in the retina.

The human CLRN1 gene is located on chromosome 3q25.1. It contains 7 exons encompassing ~ 47 kilobases (kb) (Vastinsalo et al. (2011) Eur J Hum Genet 19(1): 30-35; NCBI Accession No. NG_009168.1).

Various mutations in the CLRN1 genes have been associated with Usher syndrome type III (e.g., Usher syndrome type IIIA (MIM#606397) (see, e.g., Fields et al. (2002) Am J Hum Genet 71: 607-617, and Joensuu et al. (2001) Am J Hum Genet 69: 673-684) and retinitis pigmentosa (see, e.g., Khan et al. (2011) Ophthalmology 118: 1444-1448). Usher syndrome type III-causing mutations have been predominantly found in exon 3 of CLRN1. Usher syndrome type III-deafness can be modeled by generating CLRN1- deficient mice (see, e.g., Geng et al. (2017) Sci Rep 7(1): 13480). Exemplary mutations CLRN1-associated with Usher syndrome type III include: T528G, M120K, M44K, N48K, and C40G.

Exemplary mutations CLRN1-associated with retinitis pigmentosa include L154W and P31L (see, e.g., Khan et al. (2011) Ophthalmology 118: 1444-1448).

Additional exemplary mutations in a CLRN1 gene that have been detected in subjects having hearing loss and methods of sequencing a nucleic acid encoding CLRN1 are described in, e.g., Fields et al. (2002) Am J Hum Genet 71: 607-617, Joensuu et al. (2001) Am J Hum Genet 69: 673-684, Adato et al. (2002) Europ J Hum Genet 10: 339-350, Aller et al. (2004), Clin Genet 66: 525-529. Methods of detecting mutations in a gene are well-known in the art. Non-limiting examples of such techniques include: real-time polymerase chain reaction (RT-PCR), PCR, sequencing, Southern blotting, and Northern blotting.

An exemplary human wildtype CLRN1 protein is or includes the sequence of SEQ ID NO: 39, SEQ ID NO: 41, SEQ ID NO: 43 and SEQ ID NO: 45. Non-limiting examples of nucleotide sequences encoding a wildtype CLRN1 protein are or include SEQ ID NO: 40, SEQ ID NO: 42, SEQ ID NO: 44 and SEQ ID NO: 46.

In some embodiments of any of the compositions described herein, the CLRN1 protein comprises a sequence that is at least 75% (e.g., at least 80%, at least 85%, at least 90%, at least 91%, at least 92%, at least 93%, at least 94%, at least 95%, at least 96%, at least 97%, at least 98%, at least 99%) identical to SEQ ID NO: 39.

In some embodiments of any of the compositions described herein, the CLRN1 protein comprises a sequence that is at least 75% (e.g., at least 80%, at least 85%, at least 90%, at least 91%, at least 92%, at least 93%, at least 94%, at least 95%, at least 96%, at least 97%, at least 98%, at least 99%) identical to SEQ ID NO: 41.

In some embodiments of any of the compositions described herein, the CLRN1 protein comprises a sequence that is at least 75% (e.g., at least 80%, at least 85%, at least 90%, at least 91%, at least 92%, at least 93%, at least 94%, at least 95%, at least 96%, at least 97%, at least 98%, at least 99%) identical to SEQ ID NO: 43.

In some embodiments of any of the compositions described herein, the CLRN1 protein comprises a sequence that is at least 75% (e.g., at least 80%, at least 85%, at least 90%, at least 91%, at least 92%, at least 93%, at least 94%, at least 95%, at least 96%, at least 97%, at least 98%, at least 99%) identical to SEQ ID NO: 45.
**Human Full-length Wildtype CLRN1 Protein Isoform D (SEQ ID NO: 39)** 
MPSQQKKIIFCMAGVFSFACALGVVTALGTPLWIKATVLCKTGALLVNASGQELDKFMGEMQYGLFHGEGV

RQCGLGARPFRFSFFPDLLKAIPVSIHVNVILFSAILIVLTMVGTAFFMYNAFGKPFETLHGPLGLYLLSF

ISVALWLPATRHQAQGSCGCLVMILFASEVKIHHLSEKIANYKEGTYVYKTQSEKYTTSFWVIFFCFFVHF

LNGLLIRLAGFQFPFAKSKDAETTNVAADLMY
**Human Wildtype CLRN1 Isoform D cDNA (SEQ ID NO: 40)** 
atgccaagccaacagaagaaaatcatttttgcatggccggagtgttcagttttgcatgtgccctcggagt
tgtgacagccttggggacaccgttgtggatcaaagccactgtcctctgcaaaacggagctctgctcgtca
atgcctcagggcaggagctggacaagtttatgggtgaaatgcagtacgggcttttccacggagagggtgtg
aggcagtgtgggttgggagcaaggccctttcggttctcatttttccagatttgctcaaagcaatcccagt
gagcatccacgtcaatgtcattctcttctctgccatccttattgtgttaaccatggtggggacagccttct
 
tcatgtacaatgcttttggaaaaccttttgaaactctgcatggtcccctagggctgtaccttttgagcttc
atttcagttgccctttggctgccagctaccaggcaccaggctcaaggctcctgtggctgtcttgtcatgat
attgtttgcctctgaagtgaaaatccatcacctctcagaaaaaattgcaaattataaagaaggacttatg
tctacaaaacgcaaagtgaaaaatataccacctcattctgggtcattttcttttgctttttgttcatttt
ctgaatgggctcctaatacgacttgctggatttcagttccctttttgcaaaatctaaagacgcagaaacaac
taatgtagctgcagatctaatgtactga
**Human Full-length Wildtype CLRN1 Protein Isoform A (SEQ ID NO: 41)** 
MPSQQKKIIFCMAGVFSFACALGVVTALGTPLWIKATVLCKTGALLVNASGQELDKFMGEMQYGLFHGEGV
RQCGLGARPFFRFSFFPDLLKAIPVSIHVNVILFSAILIVLTMVGTAFFMYNAFGKPFETLHGPLGLYLLSF
ISGSCGCLVMILFASEVKIHHLSEKIANYKEGTYVYKTQSEKYTTSFWVIFFCFFVHFLNGLLIRLAGFQF
PFAKSKDAETTNVAADLMY
**Human Wildtype CLRN1 Isoform A cDNA (SEQ ID NO: 42)** 
atgccaagccaacagaagaaaatcatttttttgcatggccggagtgttcagttttgcatgtgccctcggagt
tgtgacagccttggggacaccgttgtggatcaaagccactgtcctctgcaaaacggagctctgctcgtca
atgcctcagggcaggagctggacaagtttatgggtgaaatgcagtacgggcttttccacggagagggtgtg
aggcagtgtgggttggagcaaggccctttcggttctcatttttccagatttgctcaaagcaatcccagt
gagcatccacgtcaatgtcattctcttctctgccatccttattgtgttaaccatggtggggacagccttct
tcatgtacaatgcttttggaaaacctttttgaaactctgcatggtcccctagggctgtaccttttgagcttc
atttcaggctcctgtggctgtcttgtcatgatattgtttgcctctgaagtgaaaatccatcacctctcaga
aaaaattgcaaattataaagaagggacttatgtctacaaaacgcaaagtgaaaaatataccacctcattct
gggtcattttcttttgctttttgttcattttctgaatgggctcctaatacgacttgctggatttcagttc
ccttttgcaaaatctaaagacgcagaaacaactaatgtagctgcagatctaatgtactga
**Human Full-length Wildtype CLRN1 Protein Isoform C (SEQ ID NO: 43)** 
MQALQQQPVFPDLLKAIPVSIHVNVILFSAILIVLTMVGTAFFMYNAFGKPFETLHGPLGLYLLSFISGSC
GCLVMILFASEVKIHHLSEKIANYKEGTYVY KTQSEKYTTSFWLTKGHS
**Human Wildtype CLRN1 Isoform C cDNA (SEQ ID NO: 44)** 
atgcaggccctgcagcagcaaccagtttttccagatttgctcaaagcaatcccagtgagcatccacgtcaa
tgtcattctcttctctgccatccttattgtgttaaccatggtggggacagccttcttcatgtacaatgctt
ttggaaaacctttttgaaactctgcatggtcccctagggctgtaccttttgagcttcatttcaggctcctgt
ggctgtcttgtcatgatattgtttgcctctgaagtgaaaatccatcacctctcagaaaaaattgcaaatta
taaagaagggacttatgtctacaaaacgcaaagtgaaaaatataccacctcattctggctgactaaaggcc
acagctga
**Human Full-length Wildtype CLRN1 Protein Isoform E (SEQ ID NO: 45)** 
MPSQQKKIIFCMAGVFSFACALGVVTALGTPLWIKATVLCKTGALLVNASGQELDKFMGEMQYGLFHGEGV
RQCGLGARPFRFSCYFLDPFMGLPTGVPHLLSLPCSTSCRREHTSERVQEPAGCFSAVRSKLHAGPAAATS
FSRFAQSNPSEHPRQCHSLLCHPYCVNHGGDSLLHVQCFWKTF
**Human Wildtype CLRN1 Isoform E cDNA (SEQ ID NO: 46)** 
atgccaagccaacagaagaaaatcatttttgcatggccggagtgttcagttttgcatgtgccctcggagt
tgtgacagccttggggacaccgttgtggatcaaagccactgtcctctgcaaaacggagctctgctcgtca
atgcctcagggcaggagctggacaagtttatgggtgaaatgcagtacgggcttttccacggagagggtgtg
aggcagtgtgggttggagcaaggccctttcggttctcatgctattttcttgaccccttcatggactccc
aacaggggtaccccatttactcagcctgccctgctcaacctcttgcaggagggagcacacgagtgaacgag
tgcaggaaccagctggctgctttagtgctgtgaggagtaaactccatgcaggccctgcagcagcaaccagt
tttccagatttgctcaaagcaatcccagtgagcatccacgtcaatgtcattctcttctctgccatcctta
ttgtgttaaccatggtggggacagccttcttcatgtacaatgcttttggaaaacctttga
**Human Wildtype CLRN1 Genomic sequence (SEQ ID NO: 47)** (NCBI Accession No. NG_009168.1)

1 aggagatact tgaaggcagt ttgaaagact tgttttacag attcttagtc caaagatttc
61 caattaggga gaagaagcag cagaaaagga gaaaagccaa gtatgagtga tgatgaggcc
121 ttcatctact gacatttaac ctggcgagaa ccgtcgatgg tgaagttgcc ttttcagctg
181 ggagctgtcc gttcagcttc cgtaataaat gcagtcaaag aggcagtccc ttcccattgc
241 tcacaaaggt cttgtttttg aacctcgccc tcacagaagc cgtttctcat catgccaagc
301 caacagaaga aaatcatttt ttgcatggcc ggagtgttca gttttgcatg tgccctcgga
361 gttgtgacag ccttggggac accgttgtgg atcaaagcca ctgtcctctg caaaacggga
421 gctctgctcg tcaatgcctc agggcaggag ctggacaagt ttatgggtga aatgcagtac
481 gggcttttcc acggagaggg tgtgaggcag tgtgggttgg gagcaaggcc ctttcggttc
541 tcatgtaagt agcaattgca tttgagttat ttaatgcttt aggcagactc ttcccagtgt
601 tgcgaggaat tatatttgag aattttgccg tgtttactgc aggacttttt aaatcggtgt
661 gaaccatatg aaaaacctat gactctgagc aatttcttct tcctagtttt tattatttta
721 tacttgcttt ttattataat atagagttaa ttcattgtta cataattaag gtttttggaa
781 atattggcaa ttaagatgct taagtattaa tatttatgta aaaaattatg gagtcttttt
841 aaaaaagtaa acttggggaa ataggaaagc tgtaaagaat gatctttatg ctttttgttc
901 tttataaaaa gaaccaaggt catgggctcc gtatttaacc aggttgccac ctttctcatg
961 attttgtttc ctgctcccca ctccctccca ttattcctgc taagaccttt cctgctgcta
1021 aatattcagt tttcattttt aactaatttg gaatcatttg gctatagaaa tttaaaatga
1081 tctgctgtgc taactgggaa agaaatggat gcctatttag tatagaacat tttaaactga
1141 ttgacctgca aatcatgtag agaatatgag agagattttc ttgttgtgat ttttgtgaaa

1201 tggaagtgta atccacagta tttataacct gtttatctta agaagagaat ttttaaaaat
1261 taccatgtga ataggcaact cattaaatga aaattaatag gaagtcattt gttatatctc
1321 ttacaacaca cattcagaag ttattattat ttcagaaggg ctggtttgga acaaccttat
1381 gaagacacag tcagtaaatt actgcataaa tcactcttca ggaaaggagg ttaccaactg
1441 aagcatttaa aatgaattat tattttgccc aggttttttt tttctttcta gtataggtag
1501 aaggctaaat taattgaatt tattattaac atatgcagtg cctaattaaa tttcagtgct
1561 ggtctattta tatttctgca acattcctta tatcttctta gcagtcattg gacaccaacc
1621 ttcagctcac ataggttact aagtgatatg aattttcata gggctccaga aaatttccaa
1681 gaattggttg ttagcttttt aattgatgaa gtggatacca gttcttttca ctgaatggct
1741 tttattcatt aaggtaatgg ggctgttaga gttgcttagt tttcctgggg aaggggaagg
1801 aagaaaacaa agcagaatgt catgtgatat gcaactgtat taaaaaaccg aaaaggaaaa
1861 aagttgagag agatgattta accgtgagtc accggcagcc aaagcgtgag taaagcttct
1921 cacagatgaa tttagacaaa agcggagaag gtactggtga atttctggga gcctttacat
1981 tttctacagt gaaatggaga taaactttac tcatgccata ggacatgttt caaaacaata
2041 ataagatgtt ttctgaacac ttactacata ctaagcactt tatatgcttt gtctcattta
2101 atccttacac agccacattc ttctggggtt tagcgaatga tttttgtggt tgtgtcctat
2161 gcttgtcctg tctaaggatg aagttgttct aattgggtgc ccctcctttt gctttctgtg
2221 aggacttgca gaactggtgg ggtttaaaca gtaccctcac ttatctcaca gaatttcatt
2281 agctcccaga tacccctgac attctccccc tagcctagtg aagaaaatct tccatttact
2341 tgttcattct gcagtgacag ctccatcaat atacaataga ctatacatat taagtgtact
2401 gtatatacta tacatgttaa aaatctcatt cattttggtg aggcccagct aagaatactt
2461 acagtagagc tttttttttt ttcctaagca taaaagtatc tttttcaatg cagcatgaga
2521 cagagttggg aaaaccaaaa taaatagatc caatggactc cccaaagagg ataatattca
2581 tttaaataaa cacccctctc agtgttaaaa ctttctaatc aacatgcctt tgggacacat
2641 tgcaccctca aagtttacac tcccattgca acgcagcttt gtggttcacg ttttttccat
2701 tcagaatgtc attaccctgt caatgatgtt tcatcaacgt ttgcttggat gagaatcctc
2761 tgatattctt cctgatagaa atgtataagc cctgttcata taaatgaata aaagatctaa
2821 ccttactttc tcagtagtgg cttccttgga gcaaaaagca gggacctcca gagagctcag
2881 gtggatgact cttttctgtt tcttccagag ctcaacttac aattagtgca caattcattt
2941 cccagaatgt cttctttctt attgtgcctt tagaaagtta ttaagcaaac atttgaattc
3001 acagaatctt accagtgtaa gaggaatgga aaaggtaact tatcaaggta acaatcactt
3061 cgtggccagt tttttcggct cactgcaact acccctcctg ggttcaagcg attctcctgc
3121 ctcagcctcc caagtagctg ggactacagg cgtgcaccac catgcccagc taattttttt
3181 tttgtatttt tagtaaagac agggtttcac catgtgggcc aggctggtct catggcaagt
3241 tttctttgtg ttgtcatgtt attatcaatt aataggaatt tatatttcag ttctgttagg
3301 tggataaaca ctattttgca tacctaaatg tttcatttat atcagcactg gccaataaaa
3361 atatactata agcaggccgg gtgcagtggc tcacgcctgt aatcccaact tttgggaggc
3421 caacactttg ggaggacaca gggtcaggag atcgagacca tcctggctaa cctggtgaaa

3481 tcccgtctct actaaaaata caaaaaatta gccgggcgtg gcgggggcgc ctgtagtccc
3541 agctacttgg gaggctgagg caggagaatg gcacgaaccc gggaggtgga gcttgcagtg
3601 agccgagatc tcgccactgc actccagcct tggtgacaga gcaagactct gtctcaaaaa
3661 aaaaaaaaaa aaaaaaaaaa aaaaaatata tatatatata tatatattac aagccacaag
3721 ccacatatgt acttttaaat gtcctagtag ccatattaga aaacaaaagt aaaaaggaat
3781 agatgaaatt aattttaatg atttttttaa acccaagata tccaaaatat tatcatttta
3841 acatataatt aaaaatttat tgagatgttt tacattgttt ttttttttct tgacgctgtc
3901 ttggaaatct agagtgtatt ttacatttac attacatcta attcagtcta gccacatttc
3961 acatgctcat ttttttgtgt gtggttttat ggagcagaga gtttaatagg caagaaagaa
4021 aagagaaggc agaagaaaat ggctcccctg tacagagacg cgggggtggg gcgctccaaa
4081 gccaaaagag gaggtcccta agtatggtag acaccagcca ggaatatatg cagtgtctgg
4141 aggaggggat gtctgatttg catagggtca catgctcatt tttatggcta ctgtattggt
4201 cagtacagat ttagatgggg atttgctctg aacaagttgg tgattgatgg tgttcatatt
4261 ttaattgaat ttttcctggg ttctgctgta cacttgtatg tgtgttagtt tcatgtgcaa
4321 tgcttgtgtc acttttaaaa cccagatata tggcataaca tgagaatgaa aaatggacca
4381 gaaaaatagt ttggcaatgt agtcatgttt gttcctatta aatgttccct attgaccact
4441 ctatctcttt taattataac aagaatctgc cctgccagca tgcccagtta cgctgggaaa
4501 acttctgcct catttactct ggctgattct catccactta tgggtcagtg gttcattttc
4561 tagaggtcac cagcattcat acctagcata caatttcatt cattataatg aaggaatgtt
4621 ttcccttcaa agagacacaa ctagtgggct taatttttct tgatatgtca cctgtaaaat
4681 tttaatgatg atgtttaaac tctaaatgta gccatcaaga caaaaactgc aaattttgag
4741 cctcagtgtg tgtgggtggg tttctgtttc ggtaatttga aacattgcag aatatcatca
4801 aaatatgata cccaagaatc atatggtatc aatcattcct agatatactg atctattcat
4861 tgccaagata gttcaatgag ctggcaaaaa catatggaat tattttctta aaatgtgaaa
4921 aataaaattt aaccaatcat gtatcacagc ttgcaacttt agtcatactt tgaaaagcat
4981 tttaatttgg acctcatgat tgaaaattta taaaaagctg aacagaaatt agttcacttc
5041 atattttaga aaagcagagt ttctttacta aatgaggcat ttgacccaaa ttggagagaa
5101 aatgttgaaa ctacttctgt gagcaagcag gtggcttctc aaacacatgt tgggatgaaa
5161 tggttgggcc tcagggtctc agtgcctgtc actgagagtt ggcactctct atctccatgg
5221 tctcctccaa gtgtgactct tgtctcttgc tgacctgacc tgccccaagt gactcactgg
5281 tcatgaccct gcacaccttg cgtctctcct atcaccctgc cgatggcaga gctacaaagg
5341 tctttgatgt agctctgtct gatatcctgt gtttccccct atggtctgtg tggaagcagg
5401 tatgggggtg tgtaaagggg aagcctatga agttcatctg caaagactac ctggttaggg
5461 gaggagagga agaagctata tgcaccattt caccagcaag catgggctct tctgcctttt
5521 agcttagggg tcctgttgtc tagtctcact cacctattaa aacagtccag caaatagagg
5581 ttttgtttac ctcccattaa aaaaaaagca attaatggaa tagaagataa taatgtatga
5641 gaagcactat tgtgaaagaa aaaccttcaa cttctctcag ccaaataatt gcttcctcct
5701 ctgtccttcc cagaccttga tgtttgctct attatttcaa aacaactata ttataaatat

5761 ttgagaatgt gtatttccct gcaaggagat cttaatcccc aagaaggcag gagctgtgta
5821 ttattcatcc cagtgctcct tcaaaccagg gcctcagaca gtgcatggcc caaagaggta
5881 cttaataaac gtttggtaaa ctgacactat tgaaattaag caacctggat ttgaggtggg
5941 tctctgccac tcacaagaga ttacgctttg agaaaattcc atcacttcat tgattttcag
6001 ttcttgcatc tgtatatggg agacgatact aggtgatttc tgacatctct caccgtttaa
6061 atgctctgtg atctatacaa cgaggggctc gctgttctag acaagttcct tccagcttta
6121 cagttgcata acccttctaa tcttagtcac atgatgactc cactgacaga tttttggcca
6181 ccatcattag acatgctgag ttacgtgtgc ctttgctctg atcctcaaaa ctcatgattt
6241 ttaaagtttt ctgaaatatc taccatttat caggatccag atggatttca tgaccaaagt
6301 ggatgtttct tttctctccc attacaatct tttacttttt gtgtgggaaa ttgcatgtta
6361 aagaaaggga aattgaagaa tgggatgctt tggaattctg gcaagatgga ttagtgggtt
6421 ccagaaagta ggggcagcca caaataccga aataaatgag atcgtattat tgagaaagca
6481 caaatggaag aaggtcaaaa gcaagaagaa gctgacatcc tgcttcctcc aatttttgct
6541 ttctctgttt ttccaagaaa ctcctcttcc aagccttgct gaaaaactcc actttcctaa
6601 atctaacttc ttaaactgat aatggcaaga agttaggaat gaccaatatg tttaacactc
6661 caagagtatt tgttttgttt tgttttgaga ctgggtctca ctctgtcgtc cagcctggag
6721 tgcagtggtg tgatcacagc tcactgcagc cttgacctct cctgcccaag gaatcctccc
6781 acctcagcct cctgagtaga agggaccaca ggcatgtgcc actacacctg gctaactttt
6841 aaaaaatttt tttgtagaga tggggttgcc caggcaggtc tcaaactgct gggctcaagc
6901 aatcctcctg cattggcctc ccaaagtgct ggaattatgg gcataagcca ctacgcctga
6961 cctctccaag ggcattcttt acccagaaga ggaacttggc agaacttatc ctccaattgg
7021 tgaggaatat ggagaaaatg actttaagca aaggaacttc tggttctgcc tacctaatcc
7081 agaaaaagaa gttttatttc tcccttcccc tagtaactat cttcccatat tcacataaaa
7141 aagtacagaa tcaacattgt tcaagaatta taattttact tgtaagcaca tgtgcacacg
7201 cacacccata taccttcctt ccctttaaat catcccacac cctaatagta gtaaaatcat
7261 tgacccgagc atacctggga gaggaagagg agtctgacag gggcaggttc taagtggcac
7321 tcctggaact taaccctggt gtatatgaac tttacctatt gaaggatgac tcctcaactg
7381 ttctcacaat ttgctgctct gctttctttt ctaatttctg aaggtgactc atcttcccca
7441 aggactttca gacttctcag aagaaaaaaa tattgggtgg gtctctgcca ctggcaaaag
7501 attagacttt gagaatcata aaagtatatc agtatatact cattaatatt gaattactat
7561 aattaatatt atgatattga tataatgata gaatgatatt gataaaagca atattcaata
7621 atgaatatta tttcagctgc ccacttattg ggtgcctcat aggtgccagg cattttgtat
7681 gtattatcta caacccttac atgggacata ttatgatgct gtttctcttg aagaaatatg
7741 gaaactggaa acagagaggt caccacaatt ttccaaagtc acatagctaa taggtagcag
7801 acttgggatt caaattcata tgcatatggt aaatcatgct cttcctctgc tacattttgc
7861 ccccttagaa tatgaaaaag ggatacaaag agatgaagaa aatatgtaag attatccttc
7921 aatttcacta tcttttttaa agtttttttt attatacttt aaattctgaa atacatgtgc
7981 agaacatgca ggtttgttac ataggtatac acgtgccatg atggtttgtt gcacccatca

8041 acctgtcatc tacattaggt atttttccta atgctatccc tcccctagtc ccccacccac
8101 cgacaagccc cggtgtgtga tattcccctc cctgtgtcca tgtgttctca ttgttcaact
8161 cccacttatg agtgagaaca tgcggtgttt ggttttctgt tcctgcgtca gtttgctgag
8221 aatgatggtt tccagcttca ttcatgttcc tgcaaaaggc atgaactcat tttatggctg
8281 cataccattc tatggtatac atgtgccaca ttttcttaat ctagtctatc attgatgggc
8341 atttggctta gttccaagtc cttgctattg tgaacagtgc tgcaataaac atatgtgggc
8401 atatgtcttt atagtagaat gatttataat cctttgggta tagacccagt aatgggattg
8461 ctgggtcaaa tggcatttct ggttctaggt ccttcaggaa ttgccacact gtcttccaca
8521 atagctgaac tagtttacac tcccaccaac agtgtaaaag cgttcctatt tctccacatc
8581 ctctccaaca tctgttgctt cctgactttt taatgattgc cattctaatt ggagtgagat
8641 ggtatctcag tgtggttttg attagcattt ctttaatgac aagtgatgat gagctttttt
8701 tcatgtttgt tggccgtata aatgtcttct tttgagaagt gtccgttcat atcctttgcc
8761 cactttttaa cggggttttt tcttgtaaat ttgtttaact tccttgtaga ttctggatat
8821 tagtcctttg tcagatgggt agattgcaaa aattttcttc cattctgcag gttgcccgtt
8881 cactctgata atagtttctt ttgctgcgca gaagtttttt tagtttaatt agatcccatt
8941 tgtcaatttt ggcttttgtt gccattgctt ttggtgttta gtcatgaagt ctttgcccac
9001 gcctatgtcc tgaatggtaa tgcctaggtt ttcttctagg atttttatgg ttttaggtct
9061 tatgtttaaa tctttaatcc atcttgagtt aatttttgta taaggtataa ggaaggggtc
9121 cagtttagtt ttctgcatat ggctagccag ttttcccaac accatttatt aaatagggaa
9181 tcctttcccc gttgcttgtt tttgtcaggt ttgtcaaaga gcagatggtt gtagatgtgt
9241 ggcattattt ctgaggcctc tgttctgttt cattggtctc tgtatctgtt ttgatacaag
9301 taccatgctg ttttggttac tgtagacttg tagtataatt tgaagtcagg tagcgtaata
9361 cctccagttt tgttcttttt gcttaggatt gtcttgacta ttcaggctct tttttggttc
9421 catatgaaat ttaaagtagt tttttctaat tctgtgaaga aagtcaatgg tagcttgatg
9481 ggaaaagcat tgaatctata agttactttg ggcagtatgg ccattttcat gatattaatt
9541 cttcctatcc gtgagcatgg aatgtttttc catttgtttg tgtcctttct tatttccttg
9601 agcagtagtt tgtagttctc cttgaagagg tccatcacat cccttgtaag ttgtattcct
9661 aggtattttg ttctctttgt agcaattgtg aatggaagtt cactcataag tttgctctct
9721 gtttgtctgt tattggtgta taggaatgct tgtgattttt gcacattgat tttgtatcct
9781 gagactttgc cgaagttgct tatcagctta aggagatttt gggctgagac gacaggtttt
9841 tctaaatata caatcatctc atctgcaaac agagacaatt tgacttcctc tcttcctatt
9901 tgaatacgct ttatttcttt ctcttgcctg tttgccctgg ccagaacttc caatactgtg
9961 ttgaatagga gtgttcacca cctattttaa gaatagtatt gaagcctcac aaaagctggt
10021 tctcatgtaa ccatctgaga atatttggcc ttatgacttg aattcattca ttgccttttt
10081 atttcacatt ttagtgatcc tgatgtctaa atcttaatct ttgatccttg caaggtaaaa
10141 tagccaagtc aagcctgttt aataatattg gttgaggaag tcacatgctt atgatcaatc
10201 tttgggttat gtaattatat taccttaatg ttggcagttt aggtgtaagg cagagatatc
10261 tgatcacatg tgtggttagc taatttaaga tcactgccaa ctaaaatctt catggtatga

10321 tcttcaaagt tagctacttt gaccacagca atgatttcac cacagcaatt aacaaaatgg
10381 cagactcttt cctgaggtgg catgaacagt tttaaaacaa agtcaaggac caaaagaaaa
10441 gcaggcacat ggcatttgat tcatttcaaa aactagtatt gtattaagag ccaaggggat
10501 agaattgtag catcaattaa aatcttgttt gaaaaaaaat aaaaacaaac gtccattttt
10561 atctctcaaa tatattaggg ttttcataaa gttataggtg tatttttaaa aaaacaaaac
10621 tcatatacat tagactgaaa aatttgcctg tcatctcatc atgcagctaa atgcaattgt
10681 ctatggcgag atacactctt attagaggta tgatagcact aactaatagc aaactttgta
10741 cctggtagtc taatttatgc agggttcata tttcgctccc tctcagcatg ctgtaactgt
10801 ggcaaagcca ttctcaggag ttattacccc aacataatca tacccctgtg gattaggagc
10861 agttaaatgg gtcctgttat cagagacaca tatgtgccac agccgctgtc atccctaagc
10921 caccgtgggt gattaagact cactgatggg actacctctg aataggcttg agtgaggtgg
10981 atacacttca gctgagagaa attcaggtaa ggggctgaga aaatcagatt ttgaatggtt
11041 ttatcatacc atcaggtctc cttttaagtg ctggggtcat ggatttcatt caacctgacc
11101 acatagcctt tggaagcttg gctcaatacc tgagtgtgag attatgggtg atattaaagg
11161 agatgaatgc attgagctga tgtcagagaa tgtcttttac tggattttca taatgactgg
11221 ctgcagatgg gctgagggga aagtcagatc aagagcattt ctgtaagaag aaagaaatct
11281 tccctcttat tctctttcaa gaaaatgaat agctgagcac caagaggcca aatacttttt
11341 taaaaaacac atccttttat gtagagaagg acaggttgag acgaaaaaca ggacctctga
11401 aactgtcttt atagctttaa tctaggaaga aattgcggca ccattgctga catcattatc
11461 agaggctgcc ttagttctga gagcttcaca gatggccttt tctgcatttt acatctggcc
11521 agatgaaggc aaaaaggttg atgaaaccaa aactattaga tcagtggtcc ataactctgg
11581 ctgcacttta gaatcatctg gggagtcctt aaaactactg atcctgaggc tccatcccag
11641 accaattgaa tcaggatctc tgggggtggg acctgagcat tggaatgctt taaaagttcc
11701 tcagggactc taatgtgcag ccaaggctga gaatgactga ggtggatggt ggccaagaca
11761 ggtgaggcca agagttagaa gcccttactg ttagggaagg cagaagccac aaggaggagg
11821 ggagggggaa ggagcagtat ttagcatttc ttccacacag ttggggggtt ttctgatcaa
11881 aattaggctg ggatctttcg cttccatttt catgaaggtc ttatgctttg tccacagcca
11941 cctggcctca gggcaatgag caatctgatt gatgaatttt cagtaagaaa ctgagcacac
12001 ttggctctca gccccagtgg cttcccctgt ttccaaatct gcccaccagt tacaggagcc
12061 tgctcaccaa ctggttgggt taaagaagtc ggctctgtcc ttggcaggga ggccttcagc
12121 tgtctggccc tgtctgtgac tgcgtgggtg aagctgccta atttggggaa cttgatggaa
12181 gatctaagct atgttctcta acagttttat cagaaataaa gttaactttt gacctccatc
12241 tgcctgtctc ctgtggaagg gctctgctcc ttccaaagag actctcaggg gttctcctta
12301 gaggtgtgtt atcagtccaa agatcatttt agaccagcca ttacagagga tgtccaagaa
12361 attgcacaag ggaaatagaa gtaagaatga gagcaatata tcagatagta aggaaagtat
12421 gtgactggtt tcaaataagt aattaaccat aaatccaaag ttcctgcatt agttatagca
12481 atagtcatcc acctggtcag gaaaaaaaaa gtaaaagact gagctgcaag atagaaagtt
12541 tgcctgaatt ccacgtactt caaggactac tatggaggat tccttggtcc caatgcagag

12601 acatgtactc tgaccaccca tgggccaaat ccctcttacc caccctcagt gattcctcct
12661 gggacctcac tacattgagt tcttacattc cccactcttt tcgggggaaa tataaccctt
12721 ctgctcttct catgatgtta aaaatattta gacaattact taaaatttac aacaatcttc
12781 cacataaatg atctcactta tgtttcttgt gagctctgtg atttatttct attatcatct
12841 atgctgatag ttaaggaaac tgagatatcg agaccaacat gcatagtaaa tggcaaaacc
12901 agatgaattt taaactttcc taactccaaa agccacatcc tgcccaaccc gccatgctgc
12961 cgctcagtta atgcctggct gtttgtctcc ccattggccc ctctacccat tgtgctttga
13021 tggcacactg tattccaact gcctgagact cctggttaat gccattacgt acagacttag
13081 gttgaattta ctaggatttt taagatttgt aggataagag atatgactgt tagactggaa
13141 tcagcaatag ataaaaggtt aacaaagttt cagaataaaa tataatagaa aaccccagca
13201 gatagaagta aaatgaatgg taagacaact gaaatgaaag ccatacattt agaaatatca
13261 aataaaacac agattaatgg cagacaataa aggaacatac ttagcagtta gtaaaaacac
13321 tttttacctt attcttatta ccctccagta accttttttt tttttttttt tgagacaaag
13381 tctcgctgtg tcgtccaggc tggagggtag tgatgtgatc tcggctcact gcaacttctg
13441 cctcttgggc tgaagagatt ctcctgcccc agcctcctga gtagctggga ctataggggc
13501 ccatcactgc acctggctaa tttttgtatt tttagtagag acggggtttc accatgttgg
13561 ccaggctggt cttgaactcc tggcctcagg tgatccaccc gcctcagcct cccaaagtgc
13621 tgagattaca agcgtgagcc accaagcccg gctgtaacct attgaaaata acattacttg
13681 acatgtgaga caaattattt gtaagttaaa gagtttatgt gccttcaatg ctcccaccct
13741 tccctcccct aaaaagatta ttgagtgccc atgatgtgcc taagccttct ggtagactct
13801 gagaatgtga agagagttag aggatacttg ttcaacaacc cagaatctag cagaagtgtt
13861 ttgaaatgac cagccacttg ggagagctat gctagcatat cattcaggat gggctgggtc
13921 atgttttaat aacaagtaag tttgaacttt ataggtttga aacaaaaaag atgtgtttct
13981 tgctcacagt tcatattttt tggagattgg ctggagcttc attccaggat actgccacca
14041 tctggaatgt tgacagttac cgtaacagag aaagagttgt ggaggctgtc acactggcaa
14101 ttaaattcgc tgacctggaa ctgacacatg ccacctccac ttatatttta ttggccaagg
14161 cttgttacac agccacatct aacttcagag aggtcaagat gagcaaatcc taccacgagc
14221 tatagatgga tgagtagcac tcattattat cacaattatt ttatttgtta taaaaactcc
14281 aagaaggaga ggtactctat gtgaggaata ggcataggaa aatcagaggg aagttcttaa
14341 agatgagcta gatttcacta gatggcattg aagaaacatt ccaagtaaat gaacagcata
14401 agcaaatgca tgaagaactt attgtggtcc tagtacagac gatgcgtgag agtgtggagg
14461 agggaaaagg taagactgga gaggaaggca ggaaccagaa caggacagac tgtgttcact
14521 gtcaggcagt taagtctatc ttgtaggcaa catgaagcct tttaaggaag gcaagcagct
14581 atgtgacagg acagaagatg gattggaaga aaatcaaaag agaagtgggg accagtcata
14641 aggctcctcc aatatttggg gatccaaacc aaagcactgg cagagaaata gaaaggaagg
14701 gaaatatttc caaaatattc aaaacagaaa ccaagagaac ttgatgacag aagatgaacc
14761 caggtttcta ctgaatggac agttgttaca ttctctgaga taaggaatac agaaggaaaa
14821 agttgagagg gaatatgaaa ttatttttaa tcatgtttaa tttgatcact tgtggaacat

14881 caaccagaga tgtccatcag ttacaccaat ttgtaggttt tgagagaggc ctgaggtaga
14941 ggcagagaag tgggaatcag cagattagcg gcagtagttg gaaccataac tgaagatgag
15001 atttcccagg gaggggagtt gaacaggaag gtagacaaag gctgagtgca gtggctcata
15061 cctgtaatcc cagcactttg ggaggctgca gtgggtagat cacaaacaag atcaggagtt
15121 caagaccagc ctggccaaca tggtgaaacc ccgtctctac taaaaaaaca caaaaattag
15181 ccgggcgtgg tggcacacac ttgtaatccc agcctcagga ggctgaggca ggagaattgc
15241 ttgaacccgg gaggcagagg ttgcagtgag ccaagatcgt gccattgcac tccagcctgg
15301 gcgacagagc gagactccgt ccccaccgac cccctaaaaa agaaagtaga caaagatgtg
15361 tcctagaaaa cactaatatg aatgggtaga gaggggagac ttgtcaagga gatcgagggg
15421 agagtcagtg aggtgaaacc ttagaggata gaccttcacc aaggaagtaa cagtcaacag
15481 gcctaaatgc cacagagatg tcaaatgaga gacactggaa atggttcttt gaatattaca
15541 gccagaacat cacaggagac catttccaaa gcagtcttga tgaagtggtg ggaagggag
15601 gtccctgaag gagctaagga gggactggga gatcatgacc cagagataag tgttgcaggt
15661 ataaaaggga aagactgaga tcaggaaata gccacaggat ccctaaggcc aggcttcggg
15721 tggggtggtg tgtgtgtgtg tgtgtgtgtg tgtgtgtgtg tataaaatgg gaagaccttg
15781 agtataattc tgtactaaga agatagagca gtagagagga aaaggctgaa gacagatggg
15841 agggtaaata gtgaattaga aaggtctgtg caaagatgag aaaggatgag tctgaaagca
15901 cagggagaag ggccagcctt ggacaggaga aaacatttct ttcactaaga ataaagggaa
15961 ggttgggtat acaccacaaa gaaggtgtag atgggtggca atggagttct gtcaagttga
16021 gggcattcca tgatagcctc acctttctct gtgaaatgag agagtttagt ttacaagata
16081 tttctgagca cttcataagc caggttcata gactgaaaga agtggaggtg gagtgtgata
16141 ggtccttaag aataggggaa gtttggaata tctgacaagg gacagagagg aaaaaactag
16201 aaaaggcttt gcagaatgtg ggcccacaga tcagaggcta aggggtcccc atttgtgcag
16261 gagagtaagg gcagggaggc aaggctcaca gcccaaaata tagagcccct caccaaatga
16321 ctgcaggagg gcagctttcc tatgagagca tccctatcac tgttttcact ccgagtcatt
16381 aacttacgac ttactcagct ctgtttcgta atagcagact cgagtaatga gggtatgaca
16441 gcctctctct gcatgccaag gtatgcagcg tggatttcct ttttcgcttt ctctctcctg
16501 tggcttaggt gccttctgtt ctgctaccag gatagagaac ccagtgacta gtttcttcta
16561 gctctctttt tctgactagg tatcttgtca gaaatttctg cttaccagac ttcatggaga
16621 gggaatcaag ctttgaatca gggttgaaaa agtagagctt aatatatata ttacaaaatg
16681 ccactcacgt tcttgaggtt accttgtatc tataccacaa ctagcattct tttagaaagc
16741 accattaccg aagtaatccc tttcctggga attcacccaa aaaggttatt cccacttatc
16801 ccccatctcc aaaataaaaa agaaaaatgt gtgtgcttag agatgttcct ggaagcatga
16861 gctgtaatac tgaaccaata gaagacgaac taacagattg cagggcatcc gtttggcaaa
16921 aaacttatgc agttatctaa atgatagtta tgaagacaat atgtataaca cattatattc
16981 tgagtgaaaa gaacagaagg tgatttcaaa actgcattgg gataatagta atataggaaa
17041 tagtgagatg aacaaagatt tcaaaggaac aaaaataaag aaaaattttg ctttttatat
17101 tggtaggcgt atgggtgaaa ttccaatttt aattttaatt tcatagttat aaaattgttc

17161 ataaaaaaag gtcccccata gacagttggg ctttgggaca aactaacaga aacagagtag
17221 gaagaaaatt catcttcctt caatcccctt tctctgctta aaacaaaaca aaaaagagct
17281 ttgtcatgtt caggtgtgca acgaattctt tttccaaatc tggaacttta catctgctat
17341 taaacaggag tcagtttcca tgtaacatgt tgacaatccc ccaagtgtgt tggaataatt
17401 ttttttaatg aggagatttg aaattccatt tcaattgcca acctgcctct ttcaacttct
17461 aaaaacaaag taaaacaaaa caaaaacaca ctgggtccta tcaccccctc ttgctactac
17521 tattttatct ccattgccct gaattctttc caaacttctt tccacccagc tttgatttgt
17581 tttcagtcgg gtttattgag gcataattta cgtacagtaa aattcatcct tcttagattt
17641 agaggtctat gcattttgac taatgcatat tggcttataa tgactaccac aacaaagata
17701 tagaacacac ccatcactcc cgggttctcc ctgtcccttt tttggtccat ctcctctcct
17761 acccccaacc ccttggcaac cactgatctg ttttctgtcc ttatcatttt gctttttcca
17821 ggatgttgta tataaggaat catgcagcat gcagcctctc gagtctgact tcttccagtt
17881 agcacagtta tttaagatct atccgagtta ttgtgagtag cagcatcttt tttattgctg
17941 actagtattt catcacatgg atgggccaca acttgtttat ctgttcacct gtcaatggat
18001 actaagttgt ttccagtttt tggcaaatat gaataaagta aacatttgca tacagatttt
18061 tgtgtggaca catgttttca attctcttag gtaaatactg agcaatggga ttgctgggtt
18121 atatgttaag tctatgttca gttttctaag ttctgaaaca attggatatc tatatgcaaa
18181 aatacaaaat taaccttgac tcaaacttgt accatacaca aaaaataacc tgaaagagat
18241 cacaggtgta aatgtaaacc tagaactaaa aacttcaagg agagaaacat agcagaaaat
18301 atttgtgacc ttgcattagg caaagacttc ttagatttga catttgaaac atgatacata
18361 aaaaatcttg ataaattgga gttcataaaa ataagaacta ctcttcaaaa gacactgata
18421 agagaatgaa aatacaagcc acagacagag aaaatatttg tgaatttctt atctgataaa
18481 gggtttgtat ccagcatgca taaagacttt tcaaaactca ataataaaca atccaataag
18541 aaatgcacaa aagatacaaa cacatcatcg aaaatgacct atgaaaggca aataagccca
18601 caaaaaaatt ctcaacatca ttagacactt gggaaatgca aattaaaaca acactgagat
18661 aaactacata tctattaaat ggctaccact ttaaaaacct gtcaagtgcc agccagaatg
18721 tggaacaagt aggactctct tacattgcta gtgggaaggt gaatggtaca gccactttgg
18781 aaaacactcc gcagcttctt atagttacac ataggacctg ggggtagagg atggattgac
18841 tgtaaagggg caaaacttcc tggtaggggt ggggaaagtt tttagaaggg tgaaattgtt
18901 ctatatcttg attattgtgg tggttacaca actgtctgca tttgtcaaaa ctcacagaac
18961 tgtacactaa aaaggtatat ttttatgctc tgctaatttt actttaatct taaaaatagg
19021 aaggaaaaaa taaaatcaat gccactgtgc gactttgggc aagttacttc acttctctgt
19081 gccttggtct tttgaaatct atacattaag gataaaataa taccttcctc atactgttag
19141 aattaaatgt gctaatttat gttttatata tataaagtac ttggccgggt gtggtgactc
19201 acacctgtaa tcccagcact gtgggaggcc gaggtgggca gatcacttga ggacaggagt
19261 tcaagactag cctggtcaac atggtgaaac cctgtctcta ctaaaaatac aaaaattagc
19321 tgggcttggt ggtgcgtgct tgtaatccca gctacttgag tggctgaggt gggaggatca
19381 cttgaactcc agaggtggag gctgcagtgg gctccaccca ctgcctgggt gacagagcta

19441 gactccatct cgaagaaaaa ataagtactt gaaacatagc aaatgtttta taattattgg
19501 cttttttttc ttattgttat tacttgcatt attgctgttt gaagaagttt ggtgataatg
19561 gagagaaaag ggcaattagg ggtctgggat ggtttaagta tgaggagacc gagacacatt
19621 gactcagagt gaagaaatca aagataagag aatgaaagaa agggagggta attctgaacg
19681 cacagacaaa gttatgttac taacgtggca tcggctgtgt gttgtaataa ataactccct
19741 ttcacttgtc aatagctaat caaatacttt ctggagacca gaagtgactt gctggatcaa
19801 tgacaactcc tccacagatc aaaatgttca aatccttttt ctgtgttgta atcctaaatc
19861 taaaagaaca gagagaccaa gcaaatctac ctcccaacat cattaaagtg acaactctca
19921 gtatttattt gaatggtctg ctctcagctt caaccaagga aaagtcaaat tagtgttggt
19981 cagaaaacag aagggtgtta cgagagttct ggctggtcat tacagacttg ggatttttg
20041 attaaaagaa gaagaagaag aagaaacctg ataaagtgta aatatagcaa gcagggatta
20101 gtgtcctgct gggtcatgtt ctcacaacag tgagaatttc agagatttca taagaattaa
20161 actgctccac atgacaattt attttacctt ctggcttttc caggaggcaa atcagtgcaa
20221 cttctttctg cctttgtttc aatttggtaa caaccctcaa ttttaggaca ggctaaacct
20281 agccacccta tcagagatga tgaagtagcc atctttttaa caggtgggga gatgaatgga
20341 atcagggttt gtttgtttgt ttgtttaata actgctagta aaaaccaagt caatagctga
20401 ctgagtgtaa gggaggctcc agaaggcagg ttattgtagt atagatgtga ctcgacttat
20461 gatgatgtta cttcccgata aacccgtcat aagttgaaat atcgttaaat tgaaaatgct
20521 tttaatacac cgaatctacc gaacatcata gcttagctca gcctacgtta aatgtgctca
20581 ggacgcacat tgcctacagc tgagccaaat cacctggcaa cacaggacac tgtagagtat
20641 cggttgctgg cccttgtgat gctgtgactg actgggagct gcgcttagtg cctctaccca
20701 gcattgagag tttcttatcg cttattacta gcctgggaaa agaccaaaat tcaaaactca
20761 aagtgcggtt tctaccgaat gcttataact ttcagaccat catgatgttg aaaaatcgaa
20821 ccatcgtagg ttgggatcca tcctataaga cgagctacac tgccggaagt gtaagactgc
20881 tatgctgccg gaagatgggg catagtggac aactgcaagt cctgacaaca ggaggtcagc
20941 atctgcgacc tttaacatcc acattgacac taccacagtc ttccaaacag agctgatgat
21001 atagtttgga tgtcgtccct gcccaaatct catgtcgaat cgtaatcccc agtgttggag
21061 gtggggcctg gtgggaggtg attgggtcat gggggcagag ttcttatgaa tggtttagca
21121 cggtcccccc ttggtactgt atagtgagtg agttctcatg cgatctggtt gtttaaaagt
21181 gtgtggcacc tcccctctct ctctttctcc tactctggcc atgtgaagtg ttggctcccg
21241 ctttgccttc caccatgatt gttaaattcc cagaggtctc cctagaagct aatgctgcca
21301 cgtacagcct ggagaactgt gagccaatta aacctcatct ctttttaaat tacccagtct
21361 caggccgggc gcggtgactg acacctgtaa tctcagcact ttgggaggct caggcaggaa
21421 gatgatttga ggtcaggagt tcgagaccag cctggccaac atggtgaaac cccatctcta
21481 ctgaaaatat aaaaattagc caggcatggt ggcgggtgcc tgtaatccca gctacttggg
21541 aggctgaggc aggagaatcg cttgaacctg ggagtcagag gttgcagaga gccaaaatgg
21601 agccactgta ctccagcctg ggcaatggag tgagaccctg tctcaaaaaa tatatatata
21661 ttacccagac tcaggtattt ctttacctga gactatgaga gaatggacta atatagctga

21721 agaattttat tttattttta aaaaactttt acgtttgggg gtacctgtaa aagtctgtta
21781 cataggtaaa ctcctgtcat gaggatttgt tgtacagatt ctttcctgct cctccccctc
21841 ctcccaccct ccatcctcaa gaagatccca gtgtctgttg tttccttctt tgtgttcgta
21901 agttctcatc atgtagctcc cacgtataag tgagaacatg cagtatttgg ttttctgtcc
21961 ctgtgttagt ttgctaagga tgatagcctc caactccatc tatcttcctg caaaagacat
22021 gatctcattc atttttattg ctgcatagta ttccatggtg tatatgtacc acatttcttt
22081 tatccagtct gtcattgatg ggcatttagg ttgattctgt gtcttcagaa ttgtgaatag
22141 tgctgcaacg aacattcgtg tgcttgtgtc tttatagtag aatgatttct attcttctgg
22201 tagtaatggg attgctgggt caaatggtcg ttctgctttt agctctttgc agaatcacca
22261 tactgctttc cacagtggtt gaactaattt acactcccac taacagtgta taagtgttcc
22321 cttttctctg caaccttgcc agcctctgtt atcttttgac tttttaataa aaaccattct
22381 aattagtgtg atggtatttc attgttgttt tgatttgcat ttctctaatg atcagtgatg
22441 ttgagctttt tttcatgttc gttggctgca ggtacatctt cttttgaaaa gtgtctgctc
22501 atgtcctttg cccacttttt aatggggttg tttttctctt gtaaatttaa gttcctcata
22561 gatgctgggt attagacctt tgtcagatgt atagcttgca aatattttct cccattctgt
22621 aggttgtctg cttactcttt tgattgtttc ttttaccatg cagaagctcc taagtttaat
22681 tagatcccat ttgtcaattt ttgcttttgt tgcaattgct tttggtgtct ttgtcatgaa
22741 atctttgcca ggtcctatgt ccagaatgat attgcctagg ttgtcttcta gggtttttat
22801 agttttgggt tttacattta aatctttaat ccatcttgag ttgatttttg tgtttggtgt
22861 aaggaagggg tccagtttca atattctgca tatggctagc cagttatccc agcattattt
22921 attgagtaag gagtatctcc tccgttgctt gtttttccca ggtttgttga agatcagatg
22981 gttgtaggtg tgtggcctta ttttggggct ctctatcctg ttcatttggt ctatgtgcct
23041 gtttttgtac cagtaccatt ctgttttggt tactgtagcc ctatagcata tttcaaagtt
23101 gggtaacatg atgcctccag ctttattctt tttgcttaga attaccttgg ccatttgggc
23161 tctttttggt accatatgaa gtttaaaata gttttttttc tagttatgtg aagaatgtcg
23221 ttggtaattt gataggaata acatgtaatg atattgattc ttcctatcca tgagcatggg
23281 atgtttttcc atttgtttgt gtcttctctg atttcttcaa gcagtgtttt gtaactcata
23341 ttgtagagat tattcacctc cttgcttagc tgtattccta ggtattgtat tctttctgta
23401 gtaattgtga atgggattgc ttttctgatt tggccctcag cttggtattg ttggtgtata
23461 ggaatgctag tgattttttg tatcctgaga ctttgctgaa gttatttatc agctgaagga
23521 gcttttgggc tgagactagg gggtttttta gatatagaat catgttctct gcaaacagat
23581 ttagtttgac ttcctctctt cctacttgga tgccctttat ttctttctct tgcctgattt
23641 ccctggccag gacttccagt accatgttga ataggcgtgg tgagagaggg cattcttgtc
23701 ttgtgccagt tttcagggag aatgcttcca ccttttgccc attcagtacg atgtttgtgg
23761 tggtttgtca tatatggcta ttattatttt gaggtgtgtt cctttaatac ctagtttatt
23821 gacagttttt aacatgaagc agtgtttaat tttattaaaa gtcttttctg cctctgttga
23881 gatagtcatg tggcttttgt ctttagttct gtttatgtga tgaatcacat ttgttgattt
23941 ccttatgttg gaccaacctt gcatcccagg gatgaagcct acttgattgt ggtggtttag

24001 ctttttgata tactactgga ttcagtttgc aagtattttg ttgaggattt ttgcattgat
24061 gttcatcacg gatatcggcc tgaagtttct ttttttgttg tgtctctgtc aggttttggt
24121 atcagaatga tgctggcctc ctagaatgag ttggggagga gttcctcctc ctcaattttt
24181 ttggaatagg ttctgtagga atggtaccag ctcttcttta tacatctggt agagtttggc
24241 tgtgaagcca tcaggtcctg ggatttttta gttggtaggg tatttattac tgattcccta
24301 aatagaccga taatgatttt agaagtggag tcggtttttt cctggtccag tcttgggaag
24361 gtgtatgtat ccaggaattt atttagctct tctaggtttt ctagtttgtg tgcatatggg
24421 tgttcatagt agtttctgat ggttgttttt atttccgtgg gatcagtggt aacattctct
24481 tcatcatttc tttttttttt tttttttttt ttttttgaga cggagtctcg ctctgtcgcc
24541 caggctggag tgcagtggcg cgatctcggc tcactgcaag ctccgcctcc cgggttcacg
24601 ccattctcct gcctcagcct cccgagtagc tgggactaca ggcgcccgct accacgtccg
24661 gctaattttt tgtattttta gtagagacgg ggtttcaccg tgttagccag gatggtctcg
24721 atctcctgac ctcgtgatcc gcccgcctcg gcctcccaaa gtgctgggat tacaggcgtg
24781 agccaccgcg cccggcccat ttctaattgt gtttatttga atcctctctc ttctcttctt
24841 tattaggcta gctagtggcc tatctatctt attaattttt tcaaaaaacc agctcctgga
24901 tttcttgatc ttttgaatgg tttttcatgt atcaatcctt cagttcagct ctgattttgg
24961 ttatttcttg tcttgtgcta gctttggggt tgacttgttc ttgcttctct aattctttca
25021 gttctgatgt tagtttgtta gtttgagatc taactttttg atgtggacat ttagtgctat
25081 aaatttaact cttaacactg ccttagctgt gtcccagaga gtctggtatg ttgtatcttt
25141 gttctcatta gtttgaaaaa acttcttgat ttctgtctta atttaattat ttatccaaag
25201 tcattcagga acatgttgtt taatttccat gtaattgcat ggttttgagc gatttcttta
25261 gtcttgactt ctatttttat tgtaccgtgg tctgagggtg tttgatatga ctttggttct
25321 tttgcatttg ctgaggattg ttttatgtcc aattatgtgg ttgattttag agtatgtgcc
25381 atgtggtgat gagaagaatg tatattctgt tggttttggg tacagagttc tgtagaggtc
25441 tattagatcc atttggtcca atgttgagtt cagatcctga atatctttgc taatttcctg
25501 cctccatgat ctaatactgt cagtaaagca ctgaagtctc ctactactat tgtgtgggag
25561 tctatgtctc tttataggtc tctaagaact tgctttatga atctgggtgc ttctgtgttg
25621 gatgcatata tatttaggat agttagatct tcttgttgaa ttgaaccctt taccattatg
25681 taacgccctt ctttgtcttt tttttctttt gttggtttga agtcttcttt gtctgaaatt
25741 aggattgcaa cccctgcttt tttctgtttt ctgtttgctt ggtagatttt cctccatccc
25801 tttattttgg acctatgggt gtccttacat attttatctt tatctatcca tccagccatc
25861 cagccatcca ttcatccgta tcatttttaa ccaataagga cttttaaaag cgcaaccaca
25921 acaccattaa cataaccaat aaaatctata acaatgataa aatatcatct aatactcagt
25981 ccatgtccaa ttttccctcg ctatctcaaa atcgtcttct tagaaatggt ctgttcaaat
26041 gagatcacat ggacagagga aggcgaacct cacactgtgg ggactgttgt ggggtggggg
26101 gaggggggag ggatagcatt gggagatata cctaatgcta gatgacaagt tagtgggtgc
26161 agcgcaccag catggcacat gtatatgtat gtaactaacc tgcacaatgt gcacatgtac
26221 cctaaaactt aaagtataat aataaaaaat aaaaataaaa aaataaaaag tgaaaaaaga

26281 aaaaaaaaaa aaaaagaaat ggtctgttca aatcacaaac cagattcaga aacaatagcc
26341 atacattaca ttttattaat atgtctctta aatttctttt aatctattac agtctttgga
26401 atttttatgt cttcgtttat ccttccaatt attaaaaaaa aagtattttt gtattcattg
26461 aatagacaat gcttgcagaa aagtaaaaaa aaaaaaattt agtacaaaaa ggtacatagt
26521 gagctgttcc ttagtctccc ttcccagaag caatgttacc acttttgtac aaatagtctc
26581 tgcctagaca cacatgccag tccctaaggt ggctgtaaca aggtggttaa gagtgagaac
26641 atgaattcaa attcctatta tgccactcac taagtataaa tcttggtcat ggtacatgcc
26701 tctgtgcctc agtttttaat aatggtacct acctcatagg gctgttgaga gaattaaatc
26761 agataagtgc ttaaataact attaatattt attattattc acattccctt ttggcttttt
26821 tcccaaatag cagagtggtg cacatatgtc ttcatttatt tggcttgttt tttcacctca
26881 catcacattt tgatgaataa ttccatacat gttgttatag atttgcttca ttctttgtaa
26941 tcattgacta atattccatt gtatgaatat gctactgcta aacatgtacg ttatttccaa
27001 cctcttatta tcaagaaatg ctgcaatgaa tatccttgta atactttagt ggattcatgt
27061 gcaaaaatat tcataggata aaatcctgaa agttaaattg ctgagctaaa gggtatgtgc
27121 attttaatgc tttatagatt gcccagctgc ctcaaaggag gttataacaa tttacactcc
27181 caagaaaaat gcacaagggt ccccatttcc ccatacccta gctaacacag gatattgcta
27241 aatgctttca tctttgtaaa catgatgtat tgaaaatggt atctcaaagt tttaatgtgc
27301 atttttctga ttgtgagaag ataaaggaaa tgtagtacaa ctaaacatca gcagtcaaat
27361 gacctggcca tgactcctga gtgaggacac tggtaaacac catcaggatc caaacacctc
27421 tgtatttacg aagaggatgc tccctattgg atagcactaa gcttatttca tgtatgtaca
27481 tatgtagtta gttaattaca tccagcggtg gcaaagggct tgttctgacc caatgaaact
27541 ttctctcctg gcccccttcc agcatgtggt caggagtaga gtgttgtggc catgaggcat
27601 gcatttgtac agatgactac ttactcctcc ttgaaacatt tttttccatt tgcttccctg
27661 ctgtctcact catgggtctg ctcctaattc acaaatcact cttttcccag tcttcttggt
27721 tgggttttct cctcttctgt gcttgtagac atgggggagc cccagggctt ctctcttgaa
27781 ctacagcttc tccctgggtt catctccttg ggatgtctgt tccaatgggt ttaaatacta
27841 gggctaggac tagggagagg tcattgaggc acttagcaca taaagtttaa ggaaacattc
27901 tttatcaggc ccatgcaagt gcaggactgg ccctggaggg tgactaccac cttacctttt
27961 ccaccctagg caccttgttt gccttaccct agccccagtc ctctttaaca ccccagtctt
28021 ctccctggac ctccagaaac ataaatccta tttgacattt ctacttggag gttttaaggt
28081 aactcaaacg taaaatatct aagacagaac tcttgcatca cttcccatcc tggggcccaa
28141 gcctgtctct tctactagtc tatctcagtt aacagcatca ccatttattc agttgctcag
28201 gacaaaaaat ttgaagtaat ccttgactct tctttttttt tttttgagac ggattctcac
28261 tctgttgccc aggctggagt gcagtggtgg gaccttggct cactgcaacc tctgcctcct
28321 gggttcaagc aattctcctg cctcagtctt ctgacctcgt gatccactcg cctcggcctc
28381 ccaaagtgct gggattacag gcgtgagcca ccgcacccgg cctggattct tttttttttt
28441 tttaaacaag tcctatcttc catctccaaa atgtatccca aatctgacaa cctctcccac
28501 cgtaggccag cccccatctc tcccctctga aaatagcctc ccttagatct ctggacattt

28561 gttcttcccc acccccttgt gatcactatt cagcattcag aatgatcttt taatattatg
28621 aaggagactg tgttcctctc ctacttaaaa ttctctagtg gcttcctaat aaatttagaa
28681 taaaaagcca actcctcgcc atggtcacca ggccaggatc cagtgggtac aacaatctgt
28741 tcccagcaca ataatcccac ttctgcctcc ccaccattca ccaggctcca ctgcactggc
28801 tcattcctgc ctcagttgtg cttcttttcc ctggaaagtt ctttctgtag atctttaaag
28861 ggtgatttcc ttctcaacat tcaggtgtca gctcgtttca ctttcctgac catgcccatc
28921 cactcagaga tcactcaaaa tcccattacc ctattttatt tctccatcat atgtatcact
28981 atctgaaact atcttgttgt tgatgcaggc tattttcttg accccttcat gggactccca
29041 acaggggtac cccatttact cagcctgccc tgctcaacct cttgcaggag ggagcacacg
29101 agtgaacgag tgcaggaacc agctggctgc tttagtgctg tgaggagtaa actccatgca
29161 ggccctgcag cagcaaccag gtaggggtgc ctgcaacccc agggccccag agggtgtgtt
29221 acaatgctct cgtagctctg ccatctgtgg acagcagtgt gttgtcagct cagtgggccc
29281 tttgcttcat catgtagggt ggctgccctc tgcctgtgag ggcaaagggc cagggtgaca
29341 gtctttttgg gtacccacaa tttgtgcatc ctgaattctt gtttggtgcc caagaagaat
29401 ggggtcacac agatgaactg aaggatggtg aatgcagaga attagcaatg aaagtggctc
29461 tcagcagaga gagaagctga aaagggaatg ggaagggcag gtcactctcc cctgaagtca
29521 agtcacatct ctccaatgtc cagccaccat ctctgaagtc aagtttcctc tctctgatgt
29581 ccagccactt ctcctctcta ctggctgagt ctggggtatt tataggcaga ggataggtgg
29641 tggggcaggc catacataat tttggaaaag gcaacattct attggtaaaa agacattatt
29701 cataaagaac caattgggaa agagcgggca cacagggatg gaagttctca ctttgggctg
29761 caggtttcag gcttttcagc tcaaaagtga ggtttttcca gggacctgcc ccgtctgcct
29821 aaaatttcta cacttctgtc attgataccg ctggataaca gctctcctat aaagttcagg
29881 ggcttaaaac aaaaatttac taattcaccc ggacagttct tgatagggtc tctcctaact
29941 gttgcagtta catagcaact gggttggagt catcttttct gggcttgaca tccaggacag
30001 cttcttccct tgtgtgtctg gtgcctcagt gctcctccag gcagcctttc tctccagaag
30061 agtagcctgg acttcttggc aactcaagct tccaaaagaa aaaaaagcag agactgctgg
30121 ttctcttatc aaacaggcct ggaactggca caatgtactt ctgctgccat attcaggagg
30181 tcaaagcaat cgaaggccaa tccaagttca aaggcattgg agaaaaatga gaagtgtcac
30241 ttaaatgaga agtgacacat gcgtaaaagg gggaaaagca ttgattgtgg ccatttttgg
30301 agataagcta tcacgtttat ttgtttgttt gctttctaat ggtctgtctt ctcccattag
30361 gttataagct ctgtgagaca acaggaatct tgtccatctt gttttatggc tctacttcca
30421 acacctagaa taatgcctgg cacatagtag gtgctcagtg aataacttaa gcacttgata
30481 catgtttggg gaactaaaat gaacagaatt aaacttcccc agattggtcc tgccagattt
30541 gctgatgcca agcatgctga tgcctcacca gatgaaagaa gccctaaaat gtagggtttc
30601 gctttctctg caaacaagaa aaacttgccc tgaacacaaa atctagaaat agatttggcg
30661 tgttttctac attgaaatat ttcccgtagt accagaaatt attttcccac agctttgtgc
30721 tacattaaaa tattgaagtt gactgaaaat atctccattc tttaatcttg gtgtagacta
30781 gaaacaattt ttttgtaaca aagtaaatat gaaaacttcc taatatttga actccccaga

30841 tatccccaga tatctccaaa cttaaaatat cattgcaagt taagataaat tttttaaat
30901 gactaccgag aaaggtcatt aaaggcttgg ttattaaaat gtacagattt gggttataaa
30961 gccagaactt atttgtttaa atcattacat atgaccaagc acagaaaata aattacctca
31021 aatctcctct ttgctaattt ttactggtaa actctataaa atgatcctat ctttaaacct
31081 ttttgtaaac cccttataag ttagtaagtg agaatgtatt catcagaagg attttagtga
31141 tgtttgaaat taaaaaagag agatttgatt tttaaaatta tacttgcaga ctactgctaa
31201 tgaaacttct tctaacccta gttttgtctt atcttcagtt tttccagatt tgctcaaagc
31261 aatcccagtg agcatccacg tcaatgtcat tctcttctct gccatcctta ttgtgttaac
31321 catggtgggg acagccttct tcatgtacaa tgcttttgga aaaccttttg aaactctgca
31381 tggtccccta gggctgtacc ttttgagctt catttcaggt aagtacaaaa ttctacctct
31441 gaagacaaat gtgcttttca atatgtcaaa aagaccgtct acctaaatat aaagttataa
31501 tcttaacata tatacatgga tgcacactgt agtattatac ataataacaa aaagtgtgga
31561 aataccacac ttgctcaaca gtagggaatt caataaatac tttatggaca tctatatgaa
31621 taactgtgat gctgacatta aattatattt ttgaagatgt aatcaagagg ataaacgctt
31681 gtctcaaaaa gttacatggg aaaagcagta tgtaaactta tataaacatt gtgaacctaa
31741 tttgattata tatataaaat atagggaata tacatataaa atacatatat gtatatatgg
31801 gggctatata tatatatata tgaaagagat agatagatag atagatagat agatagatag
31861 acagacagac agacaataca gactccaatc tgttggtcgt ggttgtctct gacccatgac
31921 actatggggg acttttattt ttgctcatac ttttcaatat ttcttagtgt tcaataatgt
31981 gctattattt atacataata ataaaaataa ataaatggca tcaaaaaaga gtaaagggcc
32041 agtgttccgc ccacatatga gcagccatat tcaagcctgt agacactttg tgtagcctaa
32101 tgctaggtgt atctgggcaa ggataaactc taaagccaga aattagttca tcaataaaca
32161 tgtgctactc aatagctagg gctgatggaa aagaatataa aacccagtct gtgccaaatg
32221 gtgcttacta tctgcaagtg ggagaaggag aaagacgaga aaatgaaaaa tgtgtgtata
32281 atttatatgt agctgttctg taggagatct ctgacttcac cccattctaa ctttgcaaaa
32341 agatccaaca ctttgtcaga ttcctgggag gcaagtaatt ttattgatgg tttcatggag
32401 ggatacagaa cgataacaac tcacacaaag caaacaatgt aatgaaaatc tctattcgac
32461 tgtttctttt tctcctgaag ttgccctttg gctgccagct accaggcacc aggctcaagg
32521 tactttcttg ctcttgacac tactcccttc tctcatacaa ttcaacccca accacaaacg
32581 tgtatagatc tctctctcta taaaacaaag gcctgtagtt aacaggaggt cacttgcagt
32641 gtagcctctg ttcattgtta cttgtgcaca ctgcttaggg tctcacccca tccacattct
32701 gctaatcaca ttattcaccc atccaatgta gatctctcca gtggagattc tgctaatatt
32761 ttctttagat ttgtcacaag tatataatac agttttaaat tgtacagatg attatcctat
32821 aacagaagag tctaagcctt ttacatcttt gtatctctaa cgaaagcatt cagcatgaag
32881 ctctgtacac agcagacaat tcaatatgaa tttgctgact tgaaacagca agcctagaaa
32941 ggagatgtta acttggtcac ttagacagaa caggtttcag caatcagaat tcagatgaca
33001 tggaactggt agaacaggcg ctttgaagca ataggacatg agccagtgag gagagggatg
33061 gaatatcata aacaaaggcc aagggctttg caatcagagc tgaagagcca agagcacagg

33121 ctcagggtgt gggcagactg aatgagaaag tgattcaatc acatgtgaaa gtccagatga
33181 gaagagagag ttgggattac ttctgctcac caaacatcca aaaccaaaca ggtggatccg
33241 ggtggtgtgc tgttttactg atgaccatta cacagaattt taacagaagg aatgtaaagc
33301 agtggttctc aaactggagt tcccagatga gcagcgtctg catcatctgg aaacctgata
33361 aagcagcaaa ttctcaggcc ctaccccaga cccactgaat cagaaacttg gggtctggg
33421 ggaagatggc catctgtatt ttaacaatct cccttcagga gattctgagg ctggctcaag
33481 tttgaactac aggtagttgg ttcaacaggt gttggtggac tgacaaacaa aaagagactc
33541 cgaggtaact ccaagatggt aatgtcagaa agcagctacc acccctaggg cttgggggaa
33601 ccaacaaaag agtttggcat tgccagaacc tagaatcttg aggagaggcc ccagagcatt
33661 gtgtctcaga ccttgaggac ttggcactgg gacaccatga ggggtttctg ggtgtgggaa
33721 agggctggaa actccccagt tgctgccacc agggagaact acaagtgaag tggaaggtgt
33781 gggcctttct ccctttcttt ttctcgtctt ctctctcccc ctggtgctca tgtttgacag
33841 aaaacagctg aaaaggcaga actagtttgg ggagtcttga cctggcatca taaagcagag
33901 aaaagcaaag ctggagtgaa ggtgagacac aacagctcat tagcagcaac agccgtctag
33961 cgctccagct tctgaatgaa attctgaaga acagcgcact tggaagacaa attatttgac
34021 agttctgaca gacgaccaaa ctaacagcat ttgaaaagca agatgactca gagaatacag
34081 aatttaatcc aaatcccaga tcctatctct gcctttggcc aggcctaatg caaggagaac
34141 ctgagcacac aaatatatgc aggaatgatc aggacctgtg cctgcattct attctgtctc
34201 acccaccttc aaatttgttg taaaaacatg ggctcaataa aggtttgtga atcagggaag
34261 gaagagaagg ggagaaagga agggaaggag ccagctccag atctgtgtct tgcagaggat
34321 aaaggccagt gtttttagat cacccagtgt ttttctaagc ccccaatact tattttgaaa
34381 tatcaaaatg ttcaataact aaaaaaaaaa ccgttacaac aataaaacat gtttgagggt
34441 cagatggact ggcagtttgt gacctctggg gataaacagg tcactttgga atcacagact
34501 tcctcattcc ccttaaatct catatggtac ccagaagccc ttggaacttt ggaaggtgtt
34561 tattcacagt tgtaatgtcc atgcagaccc tggctctaag acccaattgt gtaagggtag
34621 gtttgtagcc cttatcccaa acattctaag tgtgagccaa tgcgtcacac actcagaggc
34681 cagagactgt attggggtcc tttatttcac gtacgagtca cattccatta agagacccca
34741 gaagtcagct ctcttccact gactggttct cttcccttgt ttctcttgcc aatgtgtgct
34801 gcccaggtgg cagtgctcac tgtcagcaga gaagaaaaat gctttcctcc ttggacctct
34861 tttctctttt tctcctccct actcacattc aggttcccta agcttccccg ctccttgtgc
34921 tgaagtcatt ctatggtcat ttcttcaact gtctacttcc ctgctggatg ggcacccaag
34981 acttggcatc ctggggcatg tagaaagggg aaagggaagg gaagggaaaa gaagtcctcc
35041 caattgtcta tctggacctt tccacactgc ccagagtact gctatgggca tctccttatg
35101 tctcccgatg tggtgcatgc cagaccctgc aggtagaaaa ggaaagaaag caacccattg
35161 gaccaggcca gcaaaggctt cagtcacaca gctggctcat acttatggct tcatattctg
35221 ttgcctcttg aaccagacat ttcttccact ctcataacct ccagtttagc tcgtattcct
35281 cagcattctc catgtaatat tgttgcatga aacccatgca agtcagccaa tttgctcttt
35341 ctcatcttgt catttataaa ttgatgctga gaagtctttt tcccaaggtt tttagtaata

35401 ccttcatcat cccccatagt tcattttggg cagtgattcg cttctttgac tgtacattag
35461 aatcatctga agaactttct aaaactactg atctcaggtc tcacacaaca ctaattaaat
35521 tatagtctct ggtggggtag ggcttgggca ctgctatttt accttaagct ctctggagtc
35581 attctaattt gtagccagtg ctaagggttg caatataagt gaatatattt cacgtatttg
35641 tcaaacattg actgagtgcc cattatgtgc cagccattat aataggcact ggtgatccca
35701 cagtgaatca ggcacacaat gctgtcttca cggagcttgt tgtctagtgg gagagtcaaa
35761 caaaagtgta tatcaataat taagtgatta cagattgcaa taattacaat aagggtgata
35821 aacaggttgc tataatatac aatagtattg cctttcacca gacatttctt aaagaggtaa
35881 atcatctatc agacaccttt taaaaatctc atctaatttc aaaagtgtac ataaataatt
35941 aagtgattac agtttgcaat aattacaatg agggtgataa acaagttgct atgaaagaga
36001 aagatagcag agatctggct ttgagatggt ggtcagggaa gactgctcca atagctgagt
36061 tctaaagcta agaaggaact gagaaccttc acagaacgtc ccaagtagaa gagaaagcac
36121 actgaagact ctagggaaag aggtctgctt gttgtaggaa ccgaaagaag gccaatgtgg
36181 ctaggtgctg ggtagtgagg ggaaatggca caaggaaaaa atgaggttag agagatcagt
36241 tgataccagt taatgttgga ccctaaacat taaccatggt aagtctttta gaattgattc
36301 taattgcaat gaaaaccttt tgaaagattt taaaaagata tctgatagct gatttacctc
36361 tctaagaaat gtctggtgaa agacaatacc atcacattgg agatggaaaa agatgaatgg
36421 attctaaaaa cattctgaaa gtacattcaa aatgtttttc aggtagctta tgcaactaat
36481 aaatagtggt ggcattctgg gtaagacaag ggaggagcag gcttgcagtt tagggcaaga
36541 aaggggtggg gagaagagct cagcactaaa atcatgtgtt ccatttgggg cacatcgagt
36601 ctgagttgct atgagaccac caagtggaga tgccaagtaa atagtcagtt acatgaatct
36661 ggagttcagt gaagaggtct agaagaaaga tgtatatttg ggcattattc ggatatagat
36721 attatgtaaa gcaataaaat tggatgagat cacctaggga gagaatgcac atagataaaa
36781 actgacctag gaccacttca tgtctaaaac accaaaagca atgtcaacaa aagccaaaat
36841 tgacaaatgg gatctaatta aactaaagag cttctgcaca gcaaaagaat cagagtgaac
36901 aggcaaccca caaaatggaa gaaaattttc acaacctact catctgacaa agggctaata
36961 tccagaatct acagtgaact caaacaaatt tacaagaaaa aaacaaacaa ccccatcaaa
37021 aagtgggtga aggacatgaa cagacacttc tcaaaagaag acatttatgc agccaaaaaa
37081 cacatgaaaa aatgctcacc atcactggcc atcagagaaa tgcaaatcaa aaccacaatg
37141 agataccatc tcacaccagt tcaaatggca atcattaaaa agtcaggaaa caacaggtgc
37201 tggagaggat ctggagaaat aggaacactt ttacactgtt ggtgggacta taaactagtt
37261 caaccattgg ggaagtcatt gtggcgattc ctcagggatc tagaactaga aataccattt
37321 gacccagcca tcccattact gggtatatac ccaaaggact ataaatcatg ctgctataaa
37381 gacacatgca catgtatgtt tattgcggca ctattcacaa tagcaaagac ttggaaccaa
37441 cctaaatgtc caacaatgat agactggatt aagaaaatgt ggcacatata caccatggaa
37501 tactatgcag ccataaaaaa tgatgagttc atgtcctttg tagggacatg gatgaagctg
37561 gaaaccatca tcctcagcaa actatcgcaa ggacaaaaaa ccaaacaccg catgttctca
37621 cccataggtg ggaattgaac gatgagaaca catggacacg ggaaggggaa catcacacac

37681 tggggactgt tgtggggtgg gaagaggggg gagggatagc attaggagat atacctaatg
37741 ctaaatgacg agttattggg tgcagcacac cagcatggca catgtataca tatgtaccta
37801 acctgcacat tgagcacacg taccctaaaa cttaaagtat aataataata aaataaaata
37861 aaataaaaaa acaaaaattg atgtaggacc aattcctgaa gaacactgac agttaatttt
37921 ttggtttagg aggaggagaa gccagcaaac gacactgagt agcaatatcc aaagaaaaag
37981 aggaaaaagg aaaactggga gattatgagt gtcccagagg gaatgtttca agattaccat
38041 cagcagtgag ctttgtgtaa aggtggcctc ctgtaataga ggtgcgggca ggagaaggca
38101 gaatagggaa aagggggtga aaaagcttcc ctcaagattt ataatacagt ggaagagaga
38161 gacagagaga gagaaagaga aagagagaga gagaacttaa ggaggtagag gaagagagag
38221 aaccaaaaaa gagggagctg agtatagaag caattagatt catagttttt agttgcggca
38281 gtgatatttg agtgggggcc ttttatatat tccattctag gtgtttccca gttgatggga
38341 gagggtctta cctagatctg catgtaaaag ggagtaggcc agctggcaga cttgacatgg
38401 atcagtggta gaacatccta gcagttctgt gaatactctc tgagaatgac atgaaaggta
38461 ttggttcagg ccttttggag gtgataaaaa ccaccaaaat gtggacttat tgcaaattgt
38521 atttgttacc atttgcaatg attataatta ttctcttata tataggcttt cttacatata
38581 gcttctctta cacatagcgt catgagttat gccttctctt acatatagtg tctgctatga
38641 gttatgccaa gcagggcaaa caaaattgct gcctttcttt aaaaagagga cgctcctagt
38701 atgggcctaa ttaattatga taattacagc tatggcatgg aacataagca cattcatata
38761 cacaaagaca ataaaataaa gaacagttca aaaacagaac agttacatta tatatcagtt
38821 tcagtatgaa taataccctg gactctgaaa tatgtctggg gcacattatt ctgtaattgg
38881 tggtgaaaaa aaatctgcat cttatctcta cgccaatcct tatgaaggag ctgtttttca
38941 ggagttcgag aaagagacac agggatgtcc agtcatcaaa gccgcagagc ctgaggaaga
39001 ataaaggatt tgtggcagga aaacccagta atgaatgtat tctgctagtt tctcagcata
39061 gaacttagaa aagaggccac agaaggaaaa gagaagtaat gttagacagc tgatgttggc
39121 aatgggcaaa gaatttcatt ctcatatgca gggcagtggc taaaggggca gtgttgtgag
39181 gaatcacatt ccaggtcatt catgtccagg gtgtggtagg aggactgtgt ttcatttatt
39241 ttgtacatgg cccagctatg accttgtgca aggaaatgct taatcatttt ctatcacagt
39301 tgcaaagaga ttcttatcct actcagaatg tacgtcttct cttctgtttc atttacagtc
39361 acaacccaag tccttgcttt gacctccaaa gcaccacttg atgtatccac ttcagaaaca
39421 cacacagaca gctcacctct ttctatccct taccttcctc ccctcttcac tcaaaccacc
39481 tacttccttt gcattcactc ttccttcagc ctgaaataat cttcctccaa atatctacct
39541 tctcactccc tcacttctct caagatacac ttaaatgtta tattccctat gaggcctccc
39601 ctggccatcc ctccccagcc ttcctatccc ccctctctgc tttattttat tctccttaat
39661 atatatcaca ctctgataaa ccattgaatg tacttatcaa gttattgcct ctctctccct
39721 ttccattgtc tccatcactg agagctctgt gaagaaaagg atttgtacct gtttcattta
39781 gtgctgtacc cccagttccc acaacagcgc aacaggcact caataaatag ttgttgaata
39841 agtgaataaa acagaagtag ctgcatattt tctggtaaca aatgatattc ttctgaaaat
39901 gtcatatttt cagacatatt tccgaaaata aattcaaatt agataaacat tgtattttta

39961 gaccatttct tctttgcatt aatcatcctt ctcaataata taacatttgt aaaacttagg
40021 ttagatatgg gctcttcaac tttccattac agaagataaa gtgaaaaggc tagacccaat
40081 ggtgttattc cttcatctac atctatcctt tggaaacaca tgaccaaatt gcttgccatc
40141 acaatctcaa aatctaccct ttggtattaa ctcacttcac ttgtccctct gtcccttttt
40201 agatggtagc catcggtctc tggagcagtg tagagtcaga acaacttcta tttggggaag
40261 aaatcattgc tggtgacctt actttcaatt actaactttc tagtgacatt tacataattt
40321 tagagaaaat taacacctac acttgtaaag ttgtggcttt cccacaccta tttatcatct
40381 ctcaatattc cttgaaaagg aaattatcaa tttatcattc tatattggca atgaaatgcc
40441 cctaatatct gtcacctata agacaattga agatgatgtg ttgaaagctt tctgaaaatg
40501 ctgatcatta ctttaaatgg aattgaaatt ccagtttatt atttccaaaa atatgatctt
40561 actgatcata ggataacatt tcataacatt tcagagattt cttccccttc gaggagccaa
40621 acccatagga cctctggact cccacagatc ctggcaggga gttcccactc ataaaagcac
40681 aggtgccctc agagtcattc agggatgaag aagcaaccct cattggccat gtcctacgtt
40741 ccccatatag taaggactgg aggagaccag tgctcaattc tgcagtctca gacagctgtc
40801 agaggagagt catgaatgtg cagtgtctag cacattacaa acgtttgtta attgactgat
40861 cattcatggt gtgacagccc tataactcag ctatcctatt cagtcagaaa ttaactcagt
40921 aatcaaagtc attaaaagga agaaaaaaaa aacctacagt accagacaga tggtggggaa
40981 atcagacaga tgaaaggaaa aatggctgta ggtcattgag taagacactg ggcagcaaaa
41041 cctgggcctt gtgcctggtt atactccaca ttatagctcc agcaaggttt ggcaggattt
41101 ccacagtcct ggcttattct aacctttctt gggagcagga gcagtgttgg tcagatagac
41161 agacacataa ggaatctgtc caactggcac cgtgtgaatt tgggctcttg gtgtacatgg
41221 ataactggga aaaagaggag agagacatgt aggactgatc ctaccgtttg tgaagtcttg
41281 ggcaagagta tgaatgaaaa cccacttctc ttcccctgcc tggctccact gcacacagta
41341 aagagcctca agcataggtg tgtggacatt gcaccatgta tccaagctct gaccatgcct
41401 cttgaaacag ctattcctca gccaccctct gaccatggga ggaatgaccc aggagaaatg
41461 accacatagg tcttgaaaat gggctcaggg ctatttacga agtcaattcc ggggtcccag
41521 gagtatggac taaaatgtga gtcaggcatg ccagatgggt atgttctatt gacttcaagg
41581 attcctcatg ctgtgggaag gaacctctcc agaagagaga cagagcagaa ccctctaaat
41641 gtggggcaca aagcaggagc ccctcttgct ggattcaaag ggtcatactg gaagagtgta
41701 ggttgagtct tattctcaca tcactcatat cacttacaca cttcttttat agccttagca
41761 gccatgccac aaagagaaac tcttcatgca tatcttttgg tccataagtc ataaatagtt
41821 atccttgatc ccatgtcttt tttagagcca tggacagaga gaagcaaaaa tataccaagt
41881 tcacactgag ttgtctccct tcatatctct tagcagtcac tgaaaggtta tgagactcag
41941 gctgggtttc tatcctctgt ccctgaaacg acaacgttga cctcgtgatc aaccctagaa
42001 tccagagcaa gatctcagac tgtcctctct actaccagac agcacacttt gttttggggg
42061 ctgtgtgctt gaaatattag ctatggcaaa aggctttgag tcttatgaca ccccaagtaa
42121 cttttacttt aggaatttga aatacagcct tgctgtaatg ctgtctcctt aacaaagcag
42181 tacctttgaa atatttaaca acttgaaaag gaaaccgagc ttgaattttc ctttcaggtg

42241 ctcaggaaat aatgtttcac ttctgtctga aattcaccat ctcctcagac aaagaaggct
42301 cttatggtaa aaggaatggc attttctcca caattttcga ataaaagata aagagaaaac
42361 agcactgcag cctttttgtt aggatctaac aataaagaaa taatacggtt ttgccagggg
42421 agagctctgg ttttaagctc agaatacaaa aataggctga caaaatttta caaaggaata
42481 ttctcagcta ccactctgag gatggtagaa agtgaaattt caagaaaatt atattatttg
42541 attatttgat gatgattaag ctgattggcc agtcctatgt gaaattctaa agtagaagaa
42601 atgtgatgtt gtcttttctg ctcacctctc ccctcattcc tacccccaaa tctctgcctc
42661 taccccaaac ccagcctgac ctttgggaaa ggaatggggg ctgtcacttg caccgtagct
42721 cctcctgcct gcagtactct ccccccacag tccagcctcc ctctgctcag ctaacttttc
42781 ctatagctcc ttctggctac ttctaaggaa ccttccataa tgctgcccac cctctcagta
42841 acagcccctc tactatccct tgattacatg cactgtaatt ggttagtaat tgatttatg
42901 tcctctgcca aattatactc catgagagca aaaatcatga gtattatctt taatgtttaa
42961 atctccacaa ctatccccca tataagtcta gagaataaat gaatgagtga attaatgaat
43021 agaaactcaa gccattatgt tgccactcct aggatatttg gattaacttt aactgaagag
43081 taaaaagcat ttacctgtcc taaaggagac ataaaattag tgggagagta tttggagaaa
43141 aaaaagacac tgtaatacat tcttttgtgt tgcctaccct gatgtagtca ggtgtccctg
43201 atatggggtg ggctgaggat ttgaaataaa atacctaatt tgacattgta agtggaggaa
43261 tcaggatttg aaaccaaatt ggtttgacct aaatcaagct attatgataa ggacttgcaa
43321 gaaaaaagga atccataaaa accatatgaa tagctaccaa ttagtaagca tttatatgtg
43381 tcaattacaa agccaaatgc aaatcatgct ttatttatat tagccacctt ttatagatga
43441 agaaattgag acctgaatat taaaattgcc tacttttaca tagtaagtaa aggaatcagg
43501 gtttgaaccc aaattggttt cacctaaggt agaaaaccat cccagcaagt ctcctattaa
43561 ctggaaccct attgtggtgg cctgagatat aacagtagct gtggaagcgc tgtagagtcc
43621 tggccatcct atgtgctcct gatctggtcc ctcctgccac ctgcttctgc tccctgtgcc
43681 atccacccat ctggaagtct cccagtgtcc atcttcgggg gagacactca ccagagtttc
43741 cagcttccag ccagtatgga gtgcccctgt cccacagcaa tctcaccgaa atcacagcta
43801 catctgttaa aattaggcta ccaatgagtg atagatgagg gggaaaaata ataatagtgt
43861 actaaacaaa acaaatgttt atttttctca cacataaaaa tctagaggtt gaagtccagg
43921 gctggtccag aggctccaag gatctgggat ttagactccc tctttcttgt ttttccacag
43981 catatggctt ccatttctgg ggccacattg gtccaaaatg tatgctgggg ctccagccat
44041 tgcatccata tttcagccac aggaaggagg aagtggggaa gaaaggacag gcccctaata
44101 cctgtatagt tcaagaagac tatcccgccc atacttccca accaccctta gttgaacaat
44161 gctgtcttaa ttcaagacac tcacatgtct agccaaaaat ctgaattctg ttacaaacaa
44221 ggagaataga gatgtgcgcc acctcaatac ctcatccata gctacctttt cctttgtgca
44281 gctgtggcca agtgaaagct gaaggagctg tggtaaccct tctgaaggga ggctggggcc
44341 tttcacaaga ggctgcatga ttgacattta tcctgcatgg cctgtgaagt acagagaaat
44401 attttctctt gaagccacat catagcagtg gctgctttgt agcctgattc caccattatg
44461 cctttaaagt gcctagcaat tcagccttca catcatgcaa agaggaatat ctcccagtct

44521 ttgtaagatc agcttaattc taaccacctc cttacctccc actgcactcc tacacgcaca
44581 cacaaatctt cttcactcag agcagaacca taacccaagc cctaccacct agagactgaa
44641 gaatcaggct catgattaca aatatgcaat aattttttgt gtggataatg tcaatgggga
44701 tgatggtaag agaattcctt ggtttacaca ttgaccctct tccctgtccc ttacaatcag
44761 gaaatatttg tcccaacacc ttgtttcttc tgttgcaggc tcctgtggct gtcttgtcat
44821 gatattgttt gcctctgaag tgaaaatcca tcacctctca gaaaaaattg caaattataa
44881 agaagggact tatgtctaca aaacgcaaag tgaaaaatat accacctcat tctgggtcat
44941 tttcttttgc ttttttgttc attttctgaa tgggctccta atacgacttg ctggatttca
45001 gttccctttt gcaaaatcta aagacgcaga aacaactaat gtagctgcag atctaatgta
45061 ctgaaaggca aacctttcta taattttaca agggagtaga cttgctttgg tcacttttag
45121 atgtggttaa ttttgcatat ccttttagtc tgcatatatt aaagcatcag gacccttcgt
45181 gacaatgttt acaaattacg tactaaggat acaggctgga aagtaaggga agcagaagga
45241 aggctttgaa aagttgtttt atctggtggg aaattgcttg acccaggtag tcaaaggcag
45301 ttgactagaa tcgacaaatt gttactccat atatatatat gtgtgtgtgt gtgtgtgtgt
45361 gtgtgtgtaa gatgtcttcc tatcaaaaag atatcaaagg cacatggaat atattttaat
45421 aaaaacaaat aatatctcta atatatccac acatttgttg ccagatttca gaaaactgag
45481 ctgcaatcgc tttcctaaaa cagtagtgta ttaaatgaac atctataaaa tgtatcaaca
45541 cacattttaa aaaatttgtt taaagtatac tcttaggcca ggcgtggtga ctcacacctg
45601 taattccagc acttcaggag gccaaggtgg gaagatcatt tgagttcagg agttcgagtt
45661 acagcctggg caataaagtg agaccctgtc actaacaaaa ttaaaaaata aaataaatat
45721 aaaatatagg ctttaaaaaa gcatagtctt attaaccatg tctgttggtc aaaatctgca
45781 aactctaaaa gaagaaaaga agaaaaaacc aagcttaggg tatttttcct cccgtgcctg
45841 agtcccaatt acattcacga cagtactttc aatgaacata attgttagga ccactgagga
45901 atcatgaaaa atgatctctg cttagtacat ttgatgcaaa atgacttatt aggggctgtt
45961 tttctagcta tagtgtctcg agtactaata tgcaattatg aaaattatat taaatctggg
46021 attatgacgg tatcactgta tcatcttggt cttgttctgg ctgtcaccaa gcatgaccca
46081 ggtcaacttt ttttttcccc tgaattaccc atcaaattga tctgcagctg actaaaggcc
46141 acagctgagc ctggaactga cccttccttc atcctcaacc tgctgtcctc cagaaagcac
46201 caaggaaaaa gcagagaatg acagcaaaca gatcactagg cctctgacca caggtgctga
46261 gtactcagca gccctcatat aataggtttg aaagtactcc ttaaaataaa acactgtttc
46321 cctttggaac tatttacaag gatgaaacaa ccgtatacct gagaaataac ttgctctggt
46381 gtcaattcgc tattcgccag cagacatcag aacacaccga gtttccagat gctggttttt
46441 ccccttaaat caggaaatac acctggacaa tttctagaag actacaattc agtctagcca
46501 caaaggggat tttttttttt tggtaacagg ctagagcccg gttctgtaag tctttagctg
46561 aaatggtcca gtacaaaagc actggaaatg agtgggctag gaggacaagg accgtctcct
46621 gcgtgaggag ttggttggag gtccccaagg ccaggtaccc cctgcactct tattggattc
46681 ctctctgtct tcttggagtt ttgaaaaact ccttcgaaca ccaggctttt ttctttagaa

46741 aacaagtctc caatcgttct ctgttccgta gaaagagaaa gaaaacctgg agcagctgct
46801 gaaaaatcta atgaggaact aagaggcaaa cccacca

A non-limiting example of a human wildtype CLRN1 genomic DNA sequence is SEQ ID NO: 47. The exons in SEQ ID NO: 47 are: nucleotide positions 1-544 (exon 1), nucleotide positions 28764-29180 (exon 2), nucleotide positions 31239-31418 (exon 3), nucleotide positions 32481-32519 (exon 4), nucleotide positions 44799-46433 (exon 5), nucleotide positions 44799-44935 (exon 6), and nucleotide positions 46128-46837 (exon 7). The introns are located between each pair of these exons in SEQ ID NO: 47, i.e., at nucleotide positions 545-28763 (intron 1), nucleotide positions 29181-31238 (intron 2), nucleotide positions 31419-32480 (intron 3), nucleotide positions 32520-44798 (intron 4), and nucleotide positions 44936-46127 (intron 7).

### Transmembrane Channel-Like 1 (TMC1)

The TMC1 gene encodes "transmembrane channel-like 1" (TMC1). TMC1 is a calcium-dependent chloride channel and interacts with transmembrane O-methyltransferase homolog in the stereocilia of hair cells. Mutations in TMC1 may lead to non-syndromic sensorineural hearing loss. For example, a D572N mutation in TMC1 has been associated with deafness (Kurima et al., Nat Genet 30:277-284, 2002, and Wei et al., J Transl Med 12:311, 2014).

The human TMC1 gene is located on chromosome 9q21. It contains 24 exons encompassing ~314 kilobases (kb) (NCBI Accession No. NG008213.1). The full-length wildtype TMC1 protein expressed from the human TMC1 gene is 760 amino acids in length.

An exemplary human wildtype TMC1 protein is or includes the sequence of SEQ ID NO: 48. Non-limiting examples of nucleic acid encoding a wildtype TMC1 protein are or include SEQ ID NO: 49.

In some embodiments of any of the compositions described herein, the TMC1 protein comprises a sequence that is at least 75% (e.g., at least 80%, at least 85%, at least 90%, at least 91%, at least 92%, at least 93%, at least 94%, at least 95%, at least 96%, at least 97%, at least 98%, at least 99%) identical to SEQ ID NO: 48.
**Human TMC1 Protein (SEQ ID NO: 48)** (NCBI Accession No. NP-619636.2)
**Human TMC1 cDNA (SEQ ID NO: 49)**

atgtcacccaaaaaagtacaatcaaagtggaggaaaaagaagacgagactgaggaaagctcaagtgaaga
ggaagaggaggtggaagataagctacctcgaagagagagcttgagaccaaagaggaaacggaccagagatg
ttatcaatgaggatgacccagaacctgaaccagaggatgaagaaacaaggaaggcaagagaaaaagagagg
aggaggaggctaaagagaggagcagaagaagaagaaattgatgaagaggaattggaaagattgaaggcaga
gttagatgagaaaagacaaataattgctactgtcaaatgcaaaccatggaagatggagaagaaaattgaag
ttctcaaggaggcaaaaaaatttgtgagtgaaaatgaaggggctcttgggaaaggaaaaggaaaacggtgg
tttgcatttaagatgatgatggccaagaaatgggcaaaattcctccgtgattttgagaacttcaaagctgc
gtgtgtcccatgggaaaataaaatcaaggctattgaaagtcagtttggctcctcagtggcctcatacttcc
tcttcttgagatggatgtatggagtcaatatggttctctttatcctgacatttagcctcatcatgttgcca
gagtacctctggggtttgccatatggcagtttacctaggaaaaccgttcccagagccgaagaggcatcggc
agcaaactttggtgtgttgtacgacttcaatggtttggcacaatattccgttctcttttatggctattatg
acaataaacgaacaattggatggatgaatttcaggttgccgctctcctatttctagtggggattatgtgc
attggatacagctttctggttgtcctcaaagcaatgaccaaaaacattggtgatgatggaggtggagatga
caacactttcaatttcagctggaaggtctttaccagctgggactacctgatcggcaatcctgaaacagcag
acaacaaatttaattctatcacaatgaactttaaggaagctatcacagaagaaaaagcagcccaagtagaa
gaaaacgtccacttgatcagattcctgaggtttctggctaacttcttcgtgtttctaacacttggagggag
tggatacctcatcttttgggctgtgaagcgatcccaggaatttgcacagcaagatcctgacacccttgggt
ggtgggaaaaaaatgaaatgaacatggttatgtccctcctagggatgttctgtccaacattgtttgactta
tttgctgaattagaagactaccatcctctcatcgctttgaaatggctactgggacgcatttttgctcttct
tttaggcaatttatacgtatttattcttgcattaatggatgagattaacaacaagattgaagaggagaagc
tagtaaaggccaatattaccctttgggaagccaatatgatcaaggcctacaatgcatcattctctgaaaat
agcactggaccaccctttttgttcaccctgcagatgtacctcgaggaccttgctgggaaacaatggtggg
acaggagtttgtgaggctgacagtctctgatgttctgaccacctacgtcacaatcctcattggggactttc

taagggcatgttttgtgaggttttgcaattattgctggtgctgggacttggagtatggatatccttcatac
accgaattcgacatcagtggcaacgtcctcgctctgatcttcaaccaaggcatgatctggatgggctcctt
ctttgctcccagcctcccaggcatcaatatccttcgactccatacatccatgtacttccagtgctgggccg
ttatgtgctgcaatgttcctgaggccagggtcttcaaagcttccagatcaaataacttctacctggcatg
ctactgctcatcctcttcctgtccacaatgcctgtcttgtacatgatcgtgtccctcccaccatcttttga
ttgtggtccattcagtggcaaaaatagaatgtttgaagtcattggagagaccctggagcacgatttcccaa
gctggatggcgaagatcttgagacagctttcaaaccctgggctggtcattgctgtcattttggtgatggtt
ttggccatctattatctcaatgctactgccaagggccagaaggcagcgaatctggatctcaaaaagaagat
gaaaatgcaagctttggagaacaaaatgcgaaacaagaaaatggcagctgcacgagcagctgcagctgctg
gtcgccagtaa

A non-limiting example of a human wildtype TMC1 genomic DNA sequence is SEQ ID NO: 50. The exons in SEQ ID NO: 50 are: nucleotide positions 1-113 (exon 1); nucleotide positions 56102-56223 (exon 2), nucleotide positions 94568-94677 (exon 3), nucleotide positions 106121-106263 (exon 4), nucleotide positions 126797-126864 (exon 5), nucleotide positions 166909-166956 (exon 6), nucleotide positions 172743-172914 (exon 7), nucleotide positions 178718-178843 (exon 8), nucleotide positions 218319-218409 (exon 9), nucleotide positions 220644-220725 (exon 10), nucleotide positions 230050-230156 (exon 11), nucleotide positions 232986-233084 (exon 12), nucleotide positions 250613-250755 (exon 13), nucleotide positions 266539-266683 (exon 14), nucleotide positions 267323-267517 (exon 15), nucleotide positions 270086-270265 (exon 16), nucleotide positions 270391-270552 (exon 17), nucleotide positions 283582-283710(exon 18), nucleotide positions 294343-294410 (exon 19), nucleotide positions 299042-299281 (exon 20), nucleotide positions 305069-305194 (exon 21), nucleotide positions 308651-308729 (exon 22), nucleotide positions 308831-30882 (exon 23), and nucleotide positions 314151-314551 (exon 24). The introns in SEQ ID NO: 50 are: nucleotide positions 114-56101 (intron 1); nucleotide positions 56224-94567 (intron 2), nucleotide positions 94678-106120 (intron 3), nucleotide positions 106264-126796 (intron 4), nucleotide positions 126865-166908 (intron 5), nucleotide positions 166957-172742 (intron 6), nucleotide positions 172915-178717 (intron 7), nucleotide positions 178844-218318 (intron 8), nucleotide positions 218410-220643 (intron 9), nucleotide positions 220726-230049 (intron 10), nucleotide positions 230157-232985 (intron 11), nucleotide positions 233085-250612 (intron 12), nucleotide positions 250756-266538 (intron 13), nucleotide positions 266684-267322 (intron 14), nucleotide positions 267518-270085 (intron 15), nucleotide positions 270266-270390 (intron 16), nucleotide positions 270553-283581 (intron 17), nucleotide positions 283711-294342 (intron 18), nucleotide positions 294411-299041 (intron 19), nucleotide positions 299282-305068 (intron 20), nucleotide positions 305195-308650 (intron 21), nucleotide positions 308730-308830 (intron 22), and nucleotide positions 30883-314150 (intron 23).

**Human TMC1 Gene Sequence (SEQ ID NO: 50)** (NCBI Reference Sequence: NG_008213.1)

1 cagaaactat gagggcagaa cccagcaatc tgtgctttct ttcacaagcc ctccaggagt
61 tgctgaaatt taggaatcat tgccccaaaa agtggccctc ataatgatgc caggtaagga
121 aagacaacat tattacctat gtgtggtgtg ttaacattct ttatgaaaca tgtgtgaaga
181 cctaaatcga agactttctc catgagatat tccctaatca cattagtgaa ataaaactat
241 atttcctcta cttttctact gctacagaaa tctcccccag ttaggatagt cccagtttcc
301 tatttatctt tgtttttctt gcagtgcaca gcatgtcacc cagtaattga taagtttttt
361 tttttttgag acggagtctt gctctgtctc ccaggctgga gtgcagtggc gcgatctcgg
421 ctccccaagt agctgggact acaggcgccc gccatcacgc ccggctaatt ttttgtattt
481 ttagtagaga cggggtttcg ccgtgttagc caggatggtc tcgatttcct gacctcgtga
541 tccgcccgcc tcagcctccc aaagtgctgg gattacaggc gtgagccacc gcacctggcc
601 taattgatat gtttaaatgt taaaattaaa tgcaatgatt tcctaaaata aacaaatgtg
661 ggacaattta ttgcactctg ttcaaattcc cattttgaag tggaattcaa acaaacatga
721 cagtaaaggg ggacagtgag cacaggacaa agagaaaagg agaaagggaa gaaatacaga
781 gaactagata ggaggaagtg tcaaagactg atgggtgtaa gccaccttta cctctggata
841 ttggttttcc tttgaattgt ggtatgtggt catattgctt attagttata acatacagct
901 gtcagaagag tttttcaatt aaaatttcaa atgtaatatt aaaactgttt ggattccttc
961 ctactgggca ctggacactg tctgtggagc tgctggaaca gcgctgaata gggtcatatt
1021 ttaaaggaac tcagcctagt gcagtttctc aaccttagca ctattggcct ttggggcctg
1081 ataatccttt tttgtgaggg gctgtcctgt gcactgtagg atgtttagca gcatccttgg
1141 cttctactca gtagatgaca gtatcaaccc ccttccccag aagtgacaat taaaaatgtc
1201 tccagacttt tccaaatgtc ccctgaggag caaaattgtc tccagttgaa aaccactggg
1261 ttagtgggag aaacagataa gacagaagtt ctccatacac catgataatg ccatgatgcc
1321 atagggctgg caggagtgaa gggaagagca tctcacaaag actcgggggg atgcagggag
1381 gaggaaaaat taggaaatga agtgggctga gttgcagtta gcctgtgggg aaaatgaagg
1441 agaagggata aggaagaaga ggaggaagga gggaggaaga gtgaggagta atagggaatt
1501 atagttgacc ttgaacaact tggggtttag ggatgccgac ccttgcgtag tcaaaaatct
1561 gagcttaact tttgactacc ctcacacttt cctaacagcc tactgttgac cagatgcttt
1621 accaataaca taaacagttt attaacacat attttgtatg ttttatgtat tatacactgt
1681 attcttacaa taaggtaagc tagagaaaag aaaatgctat taagaaaatt gtaaggaaga
1741 aaaaatatat ttactgttca ttaaggggaa gtagatcatc ataaagctct tcatccttgt
1801 catcttcaag ttgaataggc tgaaggagga gggggagaga gaagagactg gccttgctgt
1861 cttaggggtg gcagggatgg tagaggtgca ggaggtggaa ggggaggcag gaaaggcagg
1921 cacactcggg gtagctttcg ttgaaagaaa tcttcatatg agtggaccca tgcagttcaa
1981 acctgacttg ttcagagtca actgtacttg ttccaggcta agaacagaga ataaaaaaaa
2041 aatgtataga ggcctgaaac agcatattat attaaaagaa ctcaaagccg gtaaatgctt
2101 tgctactgaa gcataaagtg agaaaggggc atcatgggct ctgaaattgc agccacaagt
2161 ctacagaaca catttttcat agcccaaggt cagtcttcta ttaccctttg cagtgaggtt
2221 atcatcatcc attttcactc gtcagtgctg gggagcccat taaatacttg ccactatact
2281 tggtcccaac agttaataga ctgtagcatc ttttatttct tcacctagcc tgaaatcagt
2341 tgagcttttg cagaatctta gatatgtcgt ttttttcttc aggtttagaa tggggcactt

2401 aaaaagatgc atagtacttg aaaatagaga aagaaaatag catgtagaaa tttgtgcctt
2461 tattctgccc aaacgtctca aaaaggaaaa aaataatttt aagggtaaat attgtcatca
2521 atttatctga gctcttttga cttcctttga gcctgaaaaa caggatcagt gtctttttag
2581 tatttaccat agtgcctgat atgtagaagg tgtttgacca atgtttatta gataaatgtt
2641 cacttttatt actagtaata ttaaccacat tttaatagag actaataata catgataaac
2701 ttctcaagga tgcgatctca tttgatcact ataacaaccc aatgaggtaa gcgaggaata
2761 gttgttaact ctaatacatg ttatatctta tccttgaccc agtattaccc cagtcatgcc
2821 ttcctttccc tgcaatgaaa gaaaaccgat aaacacaaaa tgttgtatcc catataagca
2881 ttcattctta tggtagataa gtaaatggaa aagataggta tgcattctca atttttttaa
2941 gtcctagaat tataaaacag taaagaagtg agaagagaga gaaatcttaa gggaccaacc
3001 tcttgatttt acaggtgaga aaactgaggc cacatagcca tttagtgcag agccaggatt
3061 ataaaccaga tgtttggaat cctatgtggt ggtggacacg aggatatact aattagcgtc
3121 gttaccggaa aattccacat atctctaagg agcagatcac cacacacctg cacagcccaa
3181 gcctggattg tggaggtttt tcttgtagag tttgtctttg atagaaagat acggtccaga
3241 aggccggctt caaggacata agaaagggca tattcaaaac aaatacatgt atgcctattc
3301 ttttaaaccg gtacttaaca atagtatcca ggtgtccaaa tataatagct tatctgtttt
3361 attttttcca gaaaaggaaa caaggtctta gagaggttac attttgcata ggattttgtt
3421 agccaagtca gcatctgttt gcatcttaaa aatgagtcat acctattcct tgtaagtagg
3481 acaggagggc agctggaatt tcccagctca ctccttgtca ctgcttcctg cctctgctgc
3541 tctttgtcct ccacagcacg cttcattggc taaaaagtga accagatact acattacctc
3601 agggcagttg taagtactga gtcagatgcc ttaggcttgt gtgatgggaa gtttagggcg
3661 agaccaatgt cttgctttaa caacagtgtg aataagttaa tttcttttga gatttatta
3721 ctcaatattc cacccagaaa gaaacacaga tgacactcag ccaatcaaag gccatctggg
3781 actgacaaac gcagagcttg ttgtagatga caaggctcag tggatggatc atcactgaaa
3841 gaaagggaaa cagtttgaac tgttgtatct ctctccttgc tcacatgaaa aacaaaaaaa
3901 tccttggcca gttgcggtgg ctcacgcctg taatcccagc actttgggag gcccaggcgg
3961 gcagatcact tgaggtcaga agttctggac cagcctggcc aacatggcaa aaccccgtct
4021 ctactaaaaa tacaaaaatt agccgggcgt ggtggcacac acctgtattc ccagctactt
4081 gggaggctga ggcatgagaa ttgcttgaac ctgggaggtg gaggttgcag tgagccaagg
4141 tcgtgccact gcactccagc ctggatgaca gagtgagact ccgtctcaaa aaaaaaaaaa
4201 aattccttaa ccacagacta agtaagcatt attgcacaga gtgtcattcg ctaatcttgc
4261 ttgtgcctga actttgaaag caccagcccc ttgctttggg ggctgggggc gtggggtgta
4321 agtgtgtgtg tcctctagaa gaagacaaca ctgagaacag aaggagagga tcttggagat
4381 ttgttaattt cctattgcag ttagccttgt ctggggctct cactttagca cttaaaataa
4441 catactcctt tgcacagatg ttattttttt ctcttcatgt gtacaacttg aattatccaa
4501 tggccttgct ttgaggatat tcatcctatc ttattgatcc tgtcactgtg taggacaatg
4561 gagaacacag taaaaaaccc tcaatggata ctacttacac taaattaact ctatgaattc
4621 aaaatttgaa gatgctactt aatcatgtaa gcctggttct ccaaaacttc atctgccatg
4681 ggaaaaatca aagcttgtaa aatgattaaa cttgtaaagg aattaaactc taggattcac
4741 tcattttata attggtattt tcaccctaag agctttcttt ttcctctctg gtgtttaaaa
4801 acaaagtaaa accagacaaa acaagattaa ctaaattaaa acccctgtga ctttaaaaaa
4861 tgtagccaag aataatgtgc aagtgtctag ttaggccaat agatatcctt aacatacgtg
4921 agctgggaac acaaatgagc atgaagggca aggaagagca ccaccccctg cttcttttcc
4981 tgctcctgtt cttgcagagg gtcaggcttg gagaccacat atggtcagct caacactcaa
5041 gtccaccaag cagcccatga ggaactcttc cttccagagt tgtgcacacc atgcagtgtg
5101 gtccacctgc agaagcacac aacaggcgct gcctgggcca gaggtccatt caggccatgg
5161 aattggtctc aatgccattt aggcatgaat ccagggtcct gggtaccaga cgcatggtct
5221 aggtaggggg tgtcatgtac taggtgagca tggggttccg ctgggcatgt tcccttgtct
5281 tgtaggatgt tagctctgag gggcgtggct ggaagaaggc tagccttgag ccctctaaaa
5341 ggcagggctc agggcaaagc tcccagttgc tcaggcctgt gggtaatact gcttaaaaat
5401 ccccttgaga acttctttaa aatgcaaata tctcgattct tttgtagttc aaaagcatga
5461 tgattgggtg ttcacaggca tgtgtaagac gtgacaccct ctgaacctcc ttatgacccg
5521 gtacattacc tgtctgacct gaaaaaaaaa tgcaaatttc tggattctac ccgcctcccc
5581 gcactccgac attacgatgt agcagctctg tggtaggatc caggaatctg cctgtgtaat
5641 cacaggttga tattctggtg tttcctggtt ttgctctgct gatttggggg atgtttacca
5701 gcaggagagc agcctctttc ctctgccctc ctcccttgct gccctcccca gaccccagtg
5761 actcacgctc ctcctgctcc tcctgttgct ggcctctctg ttgtggttgc cagcgctact

5821 ttgcagacgc tgcctgctcc ctgcacgcca acacagattc tgtttccctg cgaccgctgc
5881 tgctgttgtt tgttccgtcc ctggtttatt gtaggagagc tgattgttct gttgccactg
5941 ctaccaagct gcaaacacca ggctcacggt gcaaggggaa ggggcttctc ctctgggtgt
6001 tctgcctcta tcgcctaggc tctcaggctt tgttgccaat ggcactgcga gaccctaaac
6061 tatggccttg tgattcccac atctccattt agttctttct tttgttctct aagatctgag
6121 aaggagccca tgtctttcag tgttcctgtt ttcttgcgat acttcccccc tccccgactc
6181 cagaatttag ggcaaccttc tgctgtggtc tgaatgttta tttctctcca ccccgccaaa
6241 cttatatgtt gaaatcttaa cccctaaggt ggtggtatta gaaactgaag cctctgggag
6301 gtaattaagt tataaggaca aagccctcat gaatgggatt actgttctta taaaaaaaag
6361 gccccagaga gctagctagc tccttccacc atgtgaggac acaacgtgaa ggtgctgtct
6421 atgaggaagg gtccctcacc agacaccaat catctggcac attgatcttg aacttcctat
6481 cctccagaac tgtaaacaaa taaatttctc ttgcttataa accataagtt catgattttt
6541 tttttttttt tgagacggag tttcgctctt gttgcccagg ctggagtgca atggtgtgat
6601 ctcggctcac aacaaactcc gcctcccggg ttcaagcgat tctcctgcct cagcttccca
6661 agtagctggg attataggca tgcgccacca tgcctggcga attttgtatt tttggtagag
6721 acagggtttc tccatgttgg tcaggctggt ctcgaactcc tgacctcagg tgatccaccc
6781 gcctcggcct cccaaagtgc tagggttaca gatgtgagcc actgcacctg gccaagttca
6841 tgatttttgt aattagcatc caaaatggac taaaaccccc atcctcctga aggttctgaa
6901 tctgaggacc caagacttgg ctctcagcct taatgtcctg ctcccactat tttcaggggg
6961 cccaatataa atttcctgag tttttgaggt ggttgggctt gcagactgac ctttagtatg
7021 ccctttgtta catttacaaa atagattgct tacagggtta gacaaataca gttgaccctc
7081 tgtatccctg ccttccatat ccatgggtca aaattatttg gaaaaaaaaa tgtgtctgta
7141 ttgaacatgt atagattttt tttttgtata tttcctaaac tatatcgtat aataactact
7201 tacagagcat tcatattgta ttaggcatta taagtagtgt agttatgatt taaagtatgt
7261 gagagaatgt gcacaggtta tacagaaata ctacaccatt tttttttttt taatcaggga
7321 ctgaagcatc ttttcagcta tgactttggt atccggtggc gggtggggcg gtgggtgggt
7381 cctggaacca ttcctccatg aataccaagg gataactcta attgctaaat cgtttcctat
7441 ttttgtgaac tggtaaagga ctgcatgaga aaggccaaaa attaaagaat aatatgctta
7501 ttcaagcccg gtgtggtggc gggcacttgt agtcctagct acttggaagg ccgagatggg
7561 cccaggagtt tgaggctata gagggctatg gttgcacctg tgaatagtca ctgcacacta
7621 gctcgggcaa catagctaga ccctgtctct aaaaaagaaa aattctgctg taataaacct
7681 gcacgcataa aatgatgtct atgtgtctat atagtgtgta tatacatatg tgtttaggaa
7741 atgtgtatat atgtgtgtgt gtttgtgtgt gtggatactg tattagttaa gacaaaaaaa
7801 tctggcttca tatgttataa aataatgtct ggagctatcc actcaagtgt gctttcttgt
7861 ttctctgttt tttttttttt cctatttcat tcagtggttt tcactgcctt tgggaaaaag
7921 tgacacagca aaacacttag aagaatcaaa tgtttatcct ttgagaatgt ggtctgtaca
7981 taagagaaaa catttagaat caaattatga gactaaacca gtttccctca cctgcagcta
8041 gataaaaatt tctaagagga aggagagaat caggaaagat tgaagacttg actccctgat
8101 aagggttctg cccgagttct gagaatggag aataaaagac accctccccc tctctaatag
8161 attttgtcag tgaaggtcag agaggcccac gaaatcaggt ctggggagca aggtaaaacc
8221 cagaatttct acatgatcat gttaaagagg gaataaagga atatcattat agtgtatctg
8281 ggcaggtggg catgagacag cctgattgag ttcctgaaat cccaggtcgg tgtgggagca
8341 gcagaatatt ttaggtggct gagagctcca tagtttcttt ctttctttct ttctttgaga
8401 tggagtctca ctctgttgcc caggctggag tgtagtgacg cgatctctac tcactgcaac
8461 ctgtctccct ggttcaagta attatgtctc agcttcctga gtagctggga ttacagatgt
8521 gtgccaccac acctggctaa ttttttttta tttttagtag agatggattt ttatcatgtt
8581 gcccaggctg gtctcaaact cctgacctca agtgatccac ctgcctcagc ctcacaaagt
8641 gctgggatta caggtgtgag ccaccagcag tcccagctac tcaggaggct gaggcagaag
8701 aatcgcttga acccgggagg tggaggttgc agtggaccga gattgtgcca ctgcactcca
8761 gcctggtgac agagcaagac tccatcttaa aaaaaaaaaa accttctcaa ggtattttct
8821 gtgcttcttg ttttctcttt tgtatttttc atctttatat ctttgtgttt cattctggat
8881 aacttttagt gacctgtctc ctagttcact aattctcttt ttagctatgt ctagtattat
8941 aatttgttta ttgaattatt aagtttaggt atcaaatttt cagctttagg atatctaatt
9001 tgccacttgt tttctgccat gatttttttt ttttttttga ggtggagtct tactctgtca
9061 cccaggctgg agtgcactgg tgtgatcttg actcactgca agatctgcat cccgagttca
9121 catcattctc ctgcctcagc ctctcaagta gctgggacta caggcgcctg ccaccacgcc
9181 cagctaatta ttattattat tattattatt tgtattttta gtagagacag ggtttcacca

9241 tgttagccag gatggtctcc atctcctgac cccatgatcc acccaccttg gccttccaaa
9301 gtgctgggat tactggcatg agccactgca cctggcttct gccataattt ttaaccttga
9361 gttttaccta cttaaacata gcaaacacag ttgtttatat atttatctaa taattccagt
9421 atcaggagcc tctggagtct acttttgtct actttcattg ttttttgttt tttaaattta
9481 cattaacagt gtctagtctg tatttatgcc tggttacttt tgattgtgtg ctagacattg
9541 tttttggcaa ataatttttt aaacacaata tgtacaaatc atatgaagcc tagactggtg
9601 ttttgttcac caaaaaagat tttaaattac ttcagttacg ttcctaggga cactattaac
9661 tcaggatgat gttaattgaa gtttcagatt tgatattttc caggtcaact ggaccaactg
9721 tccctgctat atgatttatt tcctaaagca atatcaagtt cagctctgca cctctcttat
9781 atactatgga gctctcagtc acataaaata ttctgctgct ctcacactgc cctgtagttc
9841 ataagaggaa cagtttactg ttaatccgta ttactcttac atctagggtg tagctctttg
9901 agctaccagt ccaaagtgaa gaataattta ctgaattcct actttgtctg gctctaggca
9961 ctgatttttt ttaccctctt ggccacctat cttcaattgg aaaatgccct caattcaaaa
10021 atgctccaag tgccaagctt tcctatctga attcccattt tttcctgtat ctggctggca
10081 attcattaga atcctgctag ccctctgatg cttctaagat tttcatcttt tatttttatt
10141 tatttattcg agatggagtc ttgctctgct gcccaggctg gagtgcagtg gtgtgatctt
10201 ggctcactgc aacctccatc tcctagggtt aagtgattct cctgcctcag cctcccaagc
10261 agctgggact acaggcatcc accagcatgc ccggctaatt ttgtattttt agtagagatg
10321 gagtttcacc atgttggcca ggctgatctt gaactcctaa cctcaggtta tctgcctgcc
10381 ttggccttcc aaagtgttgg gtttataggc attagccacc atacccagcc tgaagttttg
10441 attaaaaatg ctttactggg ctgggcatgg cagctcacgc ctgtaatccc agcactttgg
10501 gaggttgagg cgggcagatc acttgaggtc aggagttcga gaccagcctg accaacatgg
10561 tgaaacccca tctctactaa aaatacaaaa attagccagg tatggtggtg catgcctgca
10621 atcccagcta ctcaggaggc tgaggcagga gaatcgcttg aatctgggag gcagaggttg
10681 cagtgagccg agatcgtgcc actgtactcc agcctggaca acaagagcga gactccgtct
10741 caaaaaaaaa aaaaaaaaaa aaaaaaaaaa aaaaaaaaaa aaaaaaactt taccggaatt
10801 aaagcccagt catcttgaag ccatgatcat gacgctgtgg aagaaccggt gaggtaggat
10861 gagacatatt ctcagatgct atgctgattt ggggctgtga tgattttgga ccatagacta
10921 tgatgaggct tcaaaaagag tgcaatagtg tattttaatt tctttggtta gattcaaatt
10981 aatcttcaga aagacaaaat gcagatgtag tcttgttaga gcttgctgat gagactaaat
11041 tttccaagtg tttgaggaat gcctaacagg ggaaaggcaa agcaaactat ttttaatgac
11101 cttatggctc tgaattgcta aaagggagtc agtccaccag gctcaggcta cttggagtag
11161 aggatcctgt ttcagtgaca aactggctca tcccttaatc tgccataaaa tgatggctta
11221 gcatacatgt aggagctggg gctaagggag aaggaggtgc tgtgttttgc agaagaagct
11281 acattctgta gtaaggcctg actcttccct ggccttggag gggaaatact tgtgagacag
11341 tggccaggat cagctgaagt gccctctctt cttgctttgc ctatgggaca ctgagcccct
11401 ttttgtggaa tatattcatc cagagaaccc tcacacctta taatgcataa agtgataaag
11461 gcttggagat ctggagaaac ccaaggtgcg atccctggct ctagaagaaa ctctgggcag
11521 ccagcatgat ttacttttct ctgctccaaa gactggagga taaattaaag gaaaagaggg
11581 tatgacccag caatccactg gtgcgagttg ttgctttaag caaagactac tgcttctctt
11641 cctgattttc ccctgaccat actctcccag aggtgtctcc cagcaaccac cagagttgtg
11701 gttaagtaca cctagccaat ccaataaatc taaaggagat gcagtccaca caaccttgca
11761 tgacttgtca tgtgagttac tgttctggag agacaaactc aggctggaaa cttatttgag
11821 atagcttatg aaaacaaaga taatttatga taaggataca ggcactctca tacaatctaa
11881 ggtagaaaaa cagctaagca tcagaacaaa ctggaccaaa gacttaaaca cttctgctcc
11941 tttgtgtaat tggtttcatg cgtttctctc tctcccttcc tctctctctt ttctctctgt
12001 ggactaggtt tttctacttt tcatatacaa attttaaaaa tacaatttct ggccaggtgc
12061 agtgactcac gcttgtaatc ccagcacttt gggaggccga gggaggtgga tcacttgagg
12121 ccaggacttt aagaccagcc tggccatcat ggtgaaactc catctctatt gcaaatacaa
12181 aaattagccg ggtgtgatgg tgcacgcctg taatctcagc tacttgggag gctgaggcag
12241 gagaatcgct taaacctggg aggcagaggt tgcagtgagc tgagattgtg tcactgcact
12301 ccagcctggg taacagagtg agaatctgtc tcgaaataaa tgaataaata agtaaaataa
12361 acaataaaaa taaataaaga caattttaaa aatttcccaa cattaagtgt cagcaatttg
12421 gagagggaca gctgtctctt gttctctctc ttctccaatt ctcaattcct aggtctgatg
12481 ctttccctgg ctcaactgac tatggttgtg gggtagggcc atgaaacaga aatatcacat
12541 taggatccaa cctttaatta tgtagatgga cagcagttta cagaaaaagt ggtggggagg
12601 tggagggagg tgctgaacag acattctaaa agggctccac caaagaaagc tatttgttat

12661 atttatgaac attctggaaa attctttcaa ttgtgttcat gaataaatta tgattggtta
12721 aaagctaaca cacatgtaga ttatataact tctaaaattt gtatttggta tctgaatcta
12781 tatattattt tggcatgtat agaaaaatcc cagatatttc aaaggctatc attagaaaaa
12841 cgttaatgaa aaggttaatg gaattctata tatttagttc ctgtgagaat gtccagttta
12901 actttcttaa tcaactgatg aagaaacaga actgaaatca caagattata aagatatttt
12961 aacgatgcat ttaggttttc attctaaatg ctgaaaaaag gtatccctgc aaatactggt
13021 taagaaatta agaagtccaa aacccaataa tgtagttggc agcctgttat aaagatttag
13081 agtattcaaa gataaaggca aataaaaaga ctacttttta taccagagta gaaacagttt
13141 attttcatga tcactactct tacttcaaat atatctatta tctaagttga tgcttgcaaa
13201 aagtcctgat tcttacacaa aggcagtcga ttcatggatc tcttagagag agagaaaaaa
13261 aaaaagctag ttgggtgtgt tacagttata tgaatcatgt aggatgctgc ttaacgctat
13321 atttatcatg gatgaaggca tgttttcagg ggaaactatg agtctaccta aagttaccca
13381 gttcacctct gttccaaggg actcatcttt tcagcatttt gtttagcatg ttacaaagta
13441 ccccgatgtc ttctaaacta ttaggattgc aggaggaaac tctcccagag ctgctagaag
13501 tcatccttga aagtttggat ttctcccgaa aacttgtatt tctatcattg ttttaactat
13561 cttcttgaat cctcttgatc ctgacctaca tgtcctcttc ccttgattgc atttaaattt
13621 agctgcatat aaccttgctc ttttccttct aatccaacct ataaaccata tcaggggagg
13681 atatgtgatt tgtcaatttt gttgcctcac ttcctcctca agaactttca gtggctccat
13741 tttacctctg agattaaatt gcagccactc atcctgagtt ttggtgttcc acaatctgac
13801 atatctagtc agctttgtgt tctagtgtgt ttgtgttgct ataaaggaag actgagacta
13861 gatagtttaa taagaaaaga ggttgatttg gctcacaatt ctgcttgctg gaagattggg
13921 catttggtaa aagccttagg ctgcttctat tcatggcaga agagtaagcg gagccttcat
13981 gtgtggaaat catagggcaa gggaggcagc aagggaggtg tggagaggtg ccaggctctt
14041 tttaacaacc acttctcatg ggaactaata cagtgagaac tcactgactc tttctacagg
14101 gagggcatta atttattcgt gaatgattct cccccatgac caaaatacct ctcattaggc
14161 cccacctcca acattgggat aaaatttcaa catgaggttt gaagggggga caaacatcca
14221 aactatagca ttctgctttg gccccctaaa acttatgtcc ttctcacatt caaatacaat
14281 catttcttcc taatagtccc taaaagtctt aacttgttcc agcataaact caaaagtcca
14341 aagtccaaag tatcacctgc gactcaaggc aacttcttta cagctgtgag cttgcaaaaa
14401 taaaaaatga attatattct tccatgatac aatggttgta caggcattga gtaaacattc
14461 ccatttccct aagggagaaa ctggccaaaa gaaagtggta acaggtccca tccaagtctg
14521 aaacccagtg cggcagacat tctttttttt gaggcagagt ctcgctccgt cacccaggct
14581 ggagtgcagt ggtgcgatct cggctcactg caagctctgc ctcctgggat cacgccattc
14641 tcctgcctca gcctcctgag tagctgggac tacaggcagc tgccaccatg cctggctaat
14701 tttttgtatt tttagtagag acggggtttc actgtgttag ccaggatggt cttgatctcc
14761 tgacctcgtg atccgcctgc ctcagcctcc caaagtgctg ggattacaga cgtgagccac
14821 cgcgcctggc cagacattaa ttcttaaaac tccaaaataa tttcctttga ttccatatac
14881 cacatcctgg gcatagtagt gtgaggggtg ggtcctaagg ccttaggcag ccccactctt
14941 tggctttgct gggtgcagcc cacgtgactg ctctcacaga ttgaagccca atgcctgtgc
15001 ctttttcagg ctgcagttgt atgttgctgg tggctctata attctggggt ctggagggtg
15061 gtagccctgc tcccacacct ccaatagata atgcccaagt agaggctgtg tgtggttgat
15121 ctgaccctat ggcagacttc tgcctgcctt cccaggtgtt ccaataaatc ctctgaaatc
15181 taggtggaag ctgccaagcc tccaacattc ttgcattatg ggcacctgca gatttcacac
15241 catgaagcca ccaaggctta ccacttacac ccacctaagc agtggctcaa acagtacctg
15301 gggccattgg agccatggat ggagccagag tggccaggat gcagacagca gcatcctgag
15361 gcagcacagg gcagtggtac cccaggcttg tcctgcaaaa ttattttgtc ctcctaggcc
15421 tctaggcttg tgatgggagg agcagcctca aaggtttctg aaatgccttt ggggcctttt
15481 ttttccaaag tcttggctat tagaacctag ctcccactta gtcatgctaa tatctctcta
15541 gcaagtgttt ggctgcatcc ttgaattcct ctcctgaaaa cactctttct tcatctacca
15601 cagggccagg ctgtaaatct tccaaatttt tatgctttac ttggttttta gttacaattt
15661 acaactttag gtcactcatt tgctgccata tctagtttac aatcatattt acaattaaaa
15721 gcagtgtaaa ccaaaaagta tctgagacag gtctcaatca atgtaagggt ttattttgcg
15781 atggttaagg accatggccc atgacacagc ctcaggaagt cctgagaaca tgtgcccaag
15841 gtagttttgt gtagcttggt tttatacatt ttagggagac agaagttaca gacaaaaaca
15901 taaatcaata catgcaaggt atacattggt ttggcccaga aaggcaagac atctacaagt
15961 gggggcttcc aggtcatagg tatattcaaa aatttcctga ttggcaatgg ttgaatgagt
16021 taagctctgc ctgaacagtt gaagtcagct tgagttaaga taaagaggtt ggctgggcac

16081 tgtggctcat gcctgtaatc ctagcacttt taggaggctg aggtggtcag attgccacct
16141 gggcaatctg tttgagatca gcctgggcaa catgatgaga ccctgtcttt ccaaaaaata
16201 aaaaaattag atgggcatgg tggagcacac ctgtagtcct tgctactcga gagactgggg
16261 caggaggatt acttgagccc aggaggcaga ggttgcagtg agctgagatt gcaccattgc
16321 attccagcct gggtgacaga gccagaccct gtctgaaaaa aaaaaaaaaa aaagtgaaag
16381 tcaaggttct tgtcatgtgg atgaagcctc caggtagcag gcttcagagt gaatagatga
16441 tgaatgtctc ttattagaac ttaaaaggtg tcagactctc tggaaaaaac ttggtaaggg
16501 agagattctc tacagaacac taacttcctc cacaagagac aacattgcag ggccttttca
16561 aaatgtcaaa taaatatatt ttgggataaa atacttgtat tgtattgtat tgtattgtat
16621 tgtattgtat tgtattgtat tgtatcgtat ttttgagaca gagccttgct ctgtcatcca
16681 ggctggagtg cagtggcacg atctcggctc actgtaacct ccgtctcccc agctcaagcg
16741 attctcctgc atcagcctcc tatgtagctg ggattacagg tgtccaccac catgcctggc
16801 taattttttt gtatttttag tagagatggg gtttcaccat gttggccagg ctggtcttga
16861 actcttgacc tcaagtgatc tgcctgcctt ggcctcccaa agtgctggga ttactgggat
16921 tacaggcatg agccaccaca cctggacaat aaaatacttt tatttccttt aggtcttgct
16981 atgtcatgtg atgctatatc agagtcgggt tcaaatttgg tatcttattg ctacaaagag
17041 tctgtcttgt cagtcatatg atctctgttt taatgttaat gctggtcatt tgtgtctaaa
17101 ctccaaaggg ggagggtgta ataaggcatg ttgggtaccc tctcatcttg ctatggcatg
17161 agctagtttt tcaggtttct ttgggatctc cttgaccaag gggtccattc agttgattgg
17221 gggagtttga attttaattt tgatttccag cagccacgcc acttcttgaa tgctgtgttg
17281 cttagaaatt tcttctgctg ctgggcatgg tgactcatgc ctgtgagagg caggcacttt
17341 ggaaggctga ggtgggcaga tcaattgggc tcaggagttt gagaccagcc tgggcaacat
17401 ggcaaaaccc catctctaca aaaaatacaa aaaaaaaaca acaacaaaaa aacaaatcag
17461 ccaggtgtgg tggcatgtgc ttgtagtccc agttactcgg gaggctgagg tgggagaatg
17521 acctcagcct aggagatgga ggtttcaatg atcctagatc gtgccactgc attccagcct
17581 gagtgacaga gcaagatcct gtctcaaaaa caaaacaaaa caaaacaaaa tttccgccag
17641 atatcctagg tcatcactct taactgcagc cttccacaaa tccatagggc atggacacaa
17701 cacagccaag tttttttttc taaagcataa catggaaccc tttgctctag ttcccagtaa
17761 gtttctcatt tccttctgag acctgctcag ccttgccttt actatctata tttctatcag
17821 cattttggtc aaaaccactt aactaatctc taagaagttc caaactttcc ctcatctgcc
17881 tgtcttcttc tgagccctcc aggttcttcc aacctctgtc caatgccctg ttccaaagtt
17941 gcttccacat tttcagttgt ctttacagca acatttcact acttggtacc agtattctga
18001 ctttgtccat tttgcattgc tttaaacgaa taccggagga tgggtcattt acaaagaaag
18061 gggtttattt ggctcacagt tctgcaagcc tgtacaagaa gcatggtgcc aggatttgct
18121 tttggtgaag gcaaggaagc ttccattcat ggcagaaagt gaaggggagc ctccatgtgc
18181 agagatcaca tggcgagaga ggaagagcgt gagaggaggg gaaggtacca tggtcttttt
18241 aacagaggta caggcttttg agggaactaa cagattgaga actcgctcac tcccacccct
18301 gccagggagg tcattaatct attcatgagg gatccaccca cataacacac acatctcact
18361 aggccccacc tccaacattg ggatcaaatt tcaacatgaa gtttgggagg gcgaacatcc
18421 aaagtacagc accttgtttc ttgttgtttt ctaatatttg ttctttatcc caggaaatgg
18481 ggggtaggaa ggtgagttct tggtgtactc taagcagtca tgctcagcct gtactatcaa
18541 attttattcc atttattctt ctgttgaggc ttgcatgact tccctaaggt acaatggcag
18601 agatagccac atgggtagta aaatcagggg cagacagacc agctttctgc caggaattta
18661 cctcagggaa attcctcatc tttgactttc ctcgttctga cttttttttc ttttaattcc
18721 acctcctctc caaacctggc actattcctt tccgatcttt atccttgtcc ttactgtttt
18781 gaagttggct cttatgcctg tttgtctttg caatctgtct ctgcgtcgcg tgttggtagt
18841 catttatttt tgtccctagt catgattccc agaaccttgc tgtggtcatt ttgatgcccc
18901 accctttgac tattacaaac caggagagct catgcaagtg gccacatgct gtagtggtta
18961 agagcacagg ccccgtagct gaacttgaaa ctcagcatga caacatgctg gctgtgtgac
19021 taaggcaaat taaatgacca ctctgtgtcc ttattttctt atacataaaa tggagctaca
19081 atagtacctc ctcataggga tgttgtgaag cttcagtgag ttaatacaga tataatgatt
19141 agttatctgc ctccattaaa tccaatcatc aatactaccc agctttatta aaacttctga
19201 cttttgtttt ggatgtggcc aggtcctgaa ctgaattgtt ttttccaggt caaatttaaa
19261 tctaccctcc tttccttttt ctcaatgcca aattgttctt cagtttccat ctctagcccc
19321 ataatctttg atttctcttg cccacatgga tggttacctt ttctgaaatg tcatagctct
19381 tacagtgcta gccacctatt ttgatgccaa atctttacac caaccagtgt tatcacttaa
19441 tcaattctta catctatgtc ttaacttctc caaccaaact taagacttgt tggtgccaag

19501 aatcaaatct catacttttg agttattcaa accatacaga acagtagata ctgaataagt
19561 atttgtctga atgaattgtt tgaataactt aaagagccta gaagcggctg ggtgcgatgg
19621 ctcatgccta taatccgagc actttgggag gctgaggcgg gccgatcacc tgaggtcagg
19681 agttcgagac cagcctgaca aatatgatga aaccccgtct gtactaaaaa tacaaaaatt
19741 agccgggcat ggtggcatgc acctgtaatc ccagctactc aggaggctga gacaggagaa
19801 tctcttgaac ccaggaggtg gaggttgcag tgagccaaga tcacaccatt gcactccagc
19861 ctgggcaata aaagcaaaac tccatctcaa aaaaaaaaaa agagcctaga agctttccag
19921 ggtggtcttg ggggtattgg ctttagaaga ggaagcttga tagatgaaaa tttccagatc
19981 aacggcattt tatgtacttc tcagaaacaa ggtatgacta gactgaaaga tctgaagtca
20041 ggaagaaact tcttggtatc agtctgtgcc cttaggaaga agtgaggtca taagctctat
20101 tagcacacat agctgaaaaa ctgaaagagg atgtaagaga aaggattgtt aaaaaacaaa
20161 aacaaaaaaa cacaaaaaac caaaaataaa aaatctgggt gaggtatttg caatactaga
20221 gccaaagaat ttgacctaag aggccgggcg tggtggttca catctgtaat cccagtactt
20281 tgggaggccg aggtgggtgg atcacttgag gccagaagtt tgaaactaac ctggccaact
20341 tgttgaaacc ccatgtctac taaatacaca caaaaaaggg cccggcgcgg tggctcgtgc
20401 ctgtaattcc agtactttgg gaggctgagg tgggaggatc acctgaggtc aggagatgga
20461 gaccatcctg gctaacatgg tgaaaccccg tctctgctaa aaatacacaa attagccggg
20521 tgtggtggca tgtgcctgaa gtcccagcta cttggagggt gaggcaggag aatcacttca
20581 acctaggagg tggaggttgc agtgagctga ggtagcgcca ctgcactcca gcctgggcga
20641 cagagcaaga ctctgtctca aaataaacaa ttaactgggt gtggtggcac acacctgtaa
20701 tctcagctgc tcctgagcct gaggtgggag aatcacttga acccgggagg cagaggttgc
20761 agtgagctga cattgtgcca ctgcgctcca gcctaggggg gagaaaaaga aaaaaaagaa
20821 ttctgatcca ggcactgact cattgagcaa gtcatcttta ctgttcaggc ctcagctttc
20881 ttcctttgtt tttttttgag acagagtctc attctattgc ccaggctgga gtgcaatggc
20941 atgatttcag ctcactgcaa cctctgccgc ccgggttcaa gcaattcttc tgcctcagcc
21001 tcccgagtag gtgggattac aggtgcctgc caccgcgccc agctaatttt tgtactttta
21061 gtagagatgg gggtttcact atcttggcca ggctggtctc gaactcctga cctcatgatc
21121 cacccacctt ggcctcccaa agtgctggga ttacagatat gagccaccgc acccagctag
21181 gcctcagttt tctaatgtgt aaaatgaaaa tatgcactag accttcactt gtttttttct
21241 atgtgataat tttttttaaa aaaagaaggt gtcatgttac attttatctt tatgtttaca
21301 tttcatcttg taaggttgcc tttgagcctt gacacagtga tttttctcat tcaactagtt
21361 tctctcccat ctcaactagt gacctcacca tccactaatt gtttaagcca gaaattggga
21421 acttttcctt ggttcctctt ccttgtttac ttcttccatc taccccatga gcaaatccta
21481 tctgttcttc cttaaattta gttcccagat ctatctcctg catctcaggt ctatgtactt
21541 cttcccatct cccctgctac tagtctgtcc agagctacaa ccacttccct tatagattat
21601 tggaataacc ttcttattgg tcttcatatt ttcactctga ctcccctctg atcaattttc
21661 tgcaatgcag ccagaattat cttttaaaaa tgtaagtttg agccaggcac agtggctcac
21721 gcctgtaatc ccagcacttt gggaggccaa ggtgggagga tcacctgagg tcaggagttt
21781 gagatcagcc tggccaacat ggtgaaactc tgtctctact aaaaatacaa aaattagctg
21841 ggcatggtgg tgggtgcctg taatcccagc tacttgggag gctgaggcag aactgcttga
21901 acctgggagg ccatatatat ataaagtttg atcacaccac tgtcctgctt aatctttttt
21961 tattttttaa cttttaaatt ttttaagaga agggtctctc tgtgttgccc agactggtct
22021 ctaattcgtg ggctcaagag atcctctcat cttagcctcc caagtagctg ggaatacagg
22081 catgtgccac cacacccagt gactttcctt cctaaaatgc ttcaatagtt tctttttctt
22141 tttctttctt tctttttttt tttttttttg agatggagtc tcgttctttc acccaggctg
22201 gagtgcagtg gcgtgatctt ggctcactgc aacctctgcc tcccgggtta aagtgattct
22261 tctgcctcag tcttccaagt agctgggact acagcatgtg ccaccacacc tcaataattt
22321 tttgtatttt tagtagaaat gggtttcacc atgttggtca ggctggtctc gaacagctga
22381 gcttgtgatc cacccgcctc ggcctcccaa agtgctggga ttacaggtgt gagccactgc
22441 acccagccac ttcaatagtt tctttttgtg cttaacataa agtttaaatt catgtccaga
22501 gcctataagg tgtcatatga ccaggcccct caaggtatat ccacttcact caagccatgt
22561 ttgtctcatc ttaattcttc aaacacccca cattctttcc taactcaagg tctttgagct
22621 tgctgcttct tccaggtgga ctgatcttag cccttccctt tgttgttgat tttttaactt
22681 tttttttttt ttttttgtga gaaagtcttg ctcttgtttt ccaggctgga gtgcagtggt
22741 gcaatctcgg ctcactgcaa cctccacctc ctgggttcaa gtgagtctcc tgactcagcg
22801 tcctgagtaa ctgggattac aggcacctgc caccacaccc ggctaatttt tgtattttta
22861 gtagaaatgg ggttttgccg tgttggccag gctgatcttg aactcctgac ctcaggtgat

22921 cctcccacct cggcctccca aagtgctggg attacaggca tgagccagcg ctcccagcct
22981 ttttaccttt ttttttttta atttcaatag gtttttgggg aacaggtagt ttttggttac
23041 gtgaaaagtt ctttagttgt gatttctgag attttagcac acccatcacc tgagcagcgt
23101 acactctacc caatgtgtag ctttttatcc ttcacccccc caagtctttc cctgagttcc
23161 caaagttcat tgtatcattc ttatgccttt gcattctcgt agcttagctc ccacttacga
23221 gtgagaacat acaatatttg gttttccatt cctgagtctc caattccacc caggttgctg
23281 tgaatgccat taatatgttc ctttttatgg ctgagtagta tttcatgata tatatataca
23341 cacacacaca cacacacaca catatataca cacacacatt tatatgtgta tatatacaca
23401 tatatatata tgtcacattt ctttatccac tcattgattg atgggcattt gtagccctgc
23461 cctttgaatg gctgacttct tcttatgctt cagaattaat actttgtaat tacattaata
23521 tgtttttttt aaaaaaaaac cttttacata tatattggtc tgtcacaata atgtataaca
23581 atattatagt ttctgtttta atgacttaac tttggcaaac tggtcaatga tttcattaaa
23641 actgatcttt ttaaaaataa tcagcttcaa tagacagttt aaccagattt gtcaactttt
23701 tgtttttggc acagtctcac tctgttgccc aggatggagt gcagtggtgc catcttggct
23761 cactgcaacc tctgcctcct gggttcaagt gattctcatg cctcagcctc ccgagtagct
23821 gggattagag gcacctgcca ccatgcccag ctaagttttg aatttttagt agagataggg
23881 tttcaccatg ttcgccaggc tggtctcgaa tgcctgacct caagtgatct ggctccctcg
23941 aactcccaga gtgctgggat tacaggcgtg agccaccaca cccggtctcc ataaaaaaat
24001 cttaaacaaa aggatatggt tggtgaaata caaatcctat tgcgcagtaa ttttcacaaa
24061 ttttaaaatt gacaatacct tcgcttcttt cagatcaatt atagtggctt tcaaatgtca
24121 tcgctcaaaa aagttcaaat gtcatcgctc aaaaaaaaaa caaaccttca ctagaaaatt
24181 aatcataaat tgatggtaca gactgggtgc agtggctcac aactgtaatc ccaccacttt
24241 gggaggccga ggtggatgga tcacctgagg tcaggagttc aagaccagca tggccaacat
24301 ggtgacaccc cagctctact aaaaaaaaaa aaaaatgaaa aaattagccc ggcgtggtgg
24361 tgcatgtctg taatcccagc tacttgagag gctgagacag gagaatcgct tgaatgcaga
24421 aggcggaggt tgcagtgagt cgagatcata acactgtaat ccagcctggg caacagaggg
24481 agactcctca aaaaatataa aaataaataa attgatgtta catttggaaa aactgctgaa
24541 attttcttct tctgcaaaaa gttagaaaaa accactgaat gattgaaaac attgacatta
24601 tttaattctt tgctttagag caagtactca gttcttgatt gaagtgattg agggattcaa
24661 acattgtcct tttgttttct tccggtaacg tcagaccagc atattttgtt attgctgctg
24721 gctgttttct tcaaaatttg ctttgaaaaa gatgtaaatg gccatttcct tatacttgtg
24781 attaaatatt tgttggcctt tccaccagtt ctctgcactg atcttcaaga aaaaatttta
24841 aaacctgcac acaaaatttt aaagactgaa cacagaattt taaagcatgc acattttca
24901 aagctgtttc tatctctttg gaagtttgtg tctgattaag ttcatctaag acttcactcc
24961 aggaaaaaag ttaacccaga aaagataaat agcctcagca gagagaaaaa tctactgatt
25021 ttcttgtgtc tacattttgt tttagatagc ataatacttc aaaagttggt actcatttct
25081 taaactttac tgtgtgcata gcttcaaaat gaatactttg ttgtgtgtgg gaaagccttt
25141 tcattgttat acttacaatc ttcagcatgg tccatccgtg aatctttttt aaaattgata
25201 aaaaggccag gcgcgatggc tcatgcccgt aatcccagca ccttgggagg ccaaggcaag
25261 tggatcacct gaggtcagga gtttgagacc agcctggcca acatggtgaa acctcgtctc
25321 tactgaaaat acaaaaatca gctgggtgcg ttggcgtgcg cctgtaatcc cagctactca
25381 ggaggatgag gcaggagagt ggtttaaacc tgggaggcgg aggttgcagt gagccgagat
25441 tgtgtcactg cactccagcc tgggcaacag agcgagactc cttctcaaaa aaaaaaaaaa
25501 aaatggttaa aaagatgaaa taaaaatttt ccaagttccc ctcaaccccc ctaaaaaaaa
25561 atcatacatg aagaaacatt tccaaaacag tggagtttgg agattgattt gcacaagcac
25621 aaatgaggat ttaggataaa tttctttgac ttttacctga acactacagt cagtcttggc
25681 catagtttca gcattgccat gtccttggtc atgatagagg ttcatgtcca gcatatcaca
25741 attcagtcat atggtgacta ttatgtgagt tcaggagtgt ttatcctaga aatagaaaga
25801 cacccaggaa tgactccttt ctttcaaatt tgctgttctc aaaatgaaca tacttaagga
25861 ctttacattt gatcagggta taaaccactg ggaagactaa gtgctcagaa aaagaaccaa
25921 agacttggag ataggaacag agcctgaaat gaacagaaac tattctatac cccccatgaa
25981 cctagaaaca aatgtgtata gctaagcgta ttagtccact cgggatgctt taacagaata
26041 tcaataaact ggaatgcttc acagtaaaca tttatttctc acagttctgg aggctgggaa
26101 gtccaagatc tggtatctgc tgaggttcct cttactggtt tgcagatggc cgtcttcttg
26161 ttctatcctc acatggcaga gagcagagaa cagaccatct cttgtgtctg ttcttagaag
26221 aacattaatc ctatttatga gcactctacc ttcgcaacca aaaggcttca tctccaaata
26281 ccatcacttg gagatttagc cttcaacaca ggaattttgg gggtacacaa acattcaatc

26341 catagcactg agtctgtatg tgttgggaac tgattgataa attggaattc ttcaaattaa
26401 cttacatgta gaatacagta tttattaaag gataagtaaa aagaaatgtg ttaatttata
26461 gtgtatttat tattattaaa agtctatagg gctgggcaca gtggctcatg cctgtaatcc
26521 cagcactttg ggaggccaag gtggggtgga tcacgaggcc aggagtttca agatcagcct
26581 ggccaagatg gtaaaattcc atctctacta aaaatacaaa aattaattgg gcatggtcag
26641 gcgcacgcct gtaatcccag ctacttggga ggctgaggca ggagaactgc ttcaatgaac
26701 ccgggaggtt cattcaagat cacgccactg cacttcagcc tgggtgacag agtgagactc
26761 tgtcttaaaa aaaaaaaaaa aaaagtctac agtaactgca attgtttaga atttaagcca
26821 acaagatatg ttttcttttc cgggagttgt attttttatc actgattgtc tacaattgac
26881 agtgcatgtc aaaaagcaga cttgtcgtgc tgcagtggct cacgcctgta atcccagaac
26941 tttgagaggg cgaggcagga ggattgcttg agcccaggag ttggagacca acctgggcaa
27001 catagtgaga tcccatctct aaaaaaataa aaataaaacc actcattttt gtcaatttta
27061 ttactgcttt taatttttct aaagacgatg tgctgctcca gctctgggta cacacatgcc
27121 cacgagagca agctcatgag agaagaaact acatcccctc tatccaccta tatttctaac
27181 gccttgcaca gtagctggca catagtggac atgaaataaa tatttgtgga ataaataagt
27241 gaagaagaat tttagaatac tttataagaa acaataaatc ttcagagatc aacatttcaa
27301 attagtaact ttatttacaa acctctcttc tgggttttat tatttactac aagtgcttgc
27361 aagacctgtt attgtacact catgtattcc tatcaaaggt aatcatcaga cattatattt
27421 cattaaataa tttaatggtg ctttctgttc tttattcatt aatttttttg tgtgtgtgtg
27481 agaatggagt ttcactatgt tgcccaggct ggccttgaac tccagggttc aagtggatcc
27541 tcctgccttg gcctcccaaa gtgctgggat tacaggcatg aaccaccatg tccaacaatg
27601 ttttctgctc tttagtaagt ctagccatat taaattttat gtgttttttt tttctttttt
27661 gagactgagt ctcactctgt cacccaggct ggagtgcatt ggcgcgatct ccactcagtg
27721 caacctccgc ctcccagttt caagcaattc tcttacttca gcctcctgag tagctaggat
27781 tacaggcacg taccaccaca tctggctatt tttttttttt ttttttttga gacgaagtct
27841 cactctgttg cccgagctgg attgcagtgg catgatcttg gctcactgca acctccacct
27901 cctgggttca agtgattctc ctgcctcagc ctcctgagta gctgggacta caggcatgtg
27961 ccaccatgcc cggctgattt ttgtatattt tttaacagag atggggtttc attatgttgg
28021 ccaggttggt ctcgaactcc tgacctcgtg atccacccac cttggcctcc cgaagtgctg
28081 ggattacaga tgtgagccac cgagcccggc ctaatttttg tgtttttggt ggagatgggg
28141 ttttaccatg ttggccaggc tggtctcgag ctccggacct caggccatcc acctacctcg
28201 gcttcccaaa gtgctaggat tacaggcgtg agccactgtg cccagctcat attacatttt
28261 aaatggaggt agaatatcaa cctgaattca ctaagagaat gcaggacaaa ttgccctgat
28321 atcctgacac aaaggtgaag caggactttt ttgtaatttg aggatttaaa agcatggttg
28381 ctccaggcca ggcgtggtgg ctcctgcctg taatcccagc actttgggaa gccgaggcag
28441 gcggatcatt tgaggtcagg agttggagac cagcctggcc aaaatggtga aaccctgtct
28501 ctactaaaaa tacaaaaatt agctgggcgt ggtcaggcgc ctgtaatccc agctacttgg
28561 gaggctgagg caggagaatt gcttcaagga acccaggaag cagaggttgc agagcccata
28621 tcgtgccact gcactctagc ctaggtgaca gagcgagacc agcctggcca acatggtaaa
28681 accccgtttc tactaaaaat acaaaaaatt agcagggcaa gctggcgggc gcctgtaatc
28741 ccagctacac gggaggctga ggcaggagaa tggcgtgaac ccaggaggcg gagcttgcag
28801 tgagctgaga tggcgccact gcactccagc ctgggcgaca gagggagact ccatctcaaa
28861 aaaaaaaaaa aaaaaaaaag tatggttgtt ccagtcctgg gaaattgaaa tagggggtaa
28921 aaaggctggt gggtgtggta ggcagaataa ttacttcttc cctctcccaa gatgttcagg
28981 tcctattccc catcctattc cctggaacct atagctagat tgccttatgg ggcaaaagga
29041 atttttcaag tgtgactaaa tttagaatca tgagttagag agattatcct ggattatctg
29101 tgtgggttta atgtaatcac aggggtccct agaaagaaaa gagggaagca gaaaagccaa
29161 aggagatgga atgatgggag ttgagatcag tcagatttac agatgcaata ttgtttccta
29221 taaattctgt ggtgaaagaa atgctatatt gttgctggct ttgaagatgg aggaaggggc
29281 tgagagtgaa ggaatgcaag tgaccttaaa ttggaaaaga caagaaaata attggccctg
29341 tagaggttat aaaagaaggg tggtccttct gacacactga ttttagtccg atgagattga
29401 ttttgaactt ctgacttcca aaaactgtaa gacaatacat tagtgttatt gtaaaccact
29461 acatttttgg taatttgtta cagcaatagg aaacttatat agtgggccct gggatccatc
29521 tgccctagcc gtttatctaa aacaaaagat cttataggga aagcaaacaa acaaacaaaa
29581 aatacagaaa caaagcaaaa caaagcaccc ctatgctctg agcttgggcc tggtaagggc
29641 actatgagcc catgatttag catgttgctg ggaaatctcc ttccccctgt tgagttgcca
29701 gaatcgtgga agcagatggt gtgggggatt caggctcctc cactgctttt ctcagccaga

29761 cctttgagga gagcttggct gaagtcttcc aagctttatc cccttgtagc cattgttttg
29821 agccatctca aacattaagt agctcttgag ggttgaattg agtccttcct aaagacagac
29881 taaagtccta gtctccagga cctgtaaatg ttcccttatt tgtaattagg atgtttacag
29941 atgtaatcaa attaaaatga ggtcataccg gactagggtg ggctctgatc caatatggcc
30001 ggcatcctca taagaagagg agaagagaca tagacacata gggaaaagac cgtgtgatga
30061 tggcagagat tagagtgatg tgtttacaag ccaaggaatg tcaaatattg ccggcaatat
30121 cagaaactaa gaaaaaggca tggcacagat tgtctctgag agcctctcag agagtatggc
30181 cctgctgaca ccttagtttc agaggtctag catccagaac tctgagagaa cagatttgtg
30241 ttgttttaag acacccacca tgtggtaatt tattacagta gcccttaaac taacacagtg
30301 accttgtgtc ttctgccaga gggatttgat cacactggtg aggaagggtc ctggtccata
30361 ataacccaaa tttctgccag ggaagaggag attgaaaggt cccagtgatc ttggcctatt
30421 tacttgggag ctcttccaca gaacaaatga actcctgaag atgagctgct acaaggattc
30481 tgtgctttgg acagaattgc acttgtctct tccccctgct gtcactccct gactaggaaa
30541 ggtccaagct gtgaaggaat cccccccaga agagacttgt aagcattaaa gacctgtgga
30601 aaactaagag ggagggtgga tagaggagtc tggagaccga ctaaccttct gagctgctta
30661 ttgcctgaag tgggggtgga gtgggctgca aacactgttg tatttggggg acacatcaca
30721 gatgcttatt tgcaccttgg actttgtcag gacactctgg ttacagcaca actttgtgaa
30781 catggatcac aagatacttg gaaaagcagt gtttgcatta atcagattag gattagctaa
30841 aattttaatt aaagaaccag agagtacttc agaggcaagg agatgatagt tacctgggat
30901 tacttgctat ttgtgaaacc aaccagctga ccacttgtgt tgtttgtccc aaggtataat
30961 aataactaga tgtttgcata ttgtgattgt ttctgttcgg atttaatggc tgatctattt
31021 atcatattaa aagagggatc aagttcaaag atttcaatgc tctttatctt ctttctatat
31081 atgatattta tttttaaatg tccatattaa atatacatat atacacacga ttctcagaat
31141 tacgaagaat gtgctgtgta gcattcaatt atatatataa tttttttttt gagatggagt
31201 ttccctcttg ttgcccaggc tggagtgcaa cggcatgatc tcgggtcaca gcaacctctg
31261 cctcccaggt tcaagtgatt ctcctacctc agcctcctga gtagctggga ttacaggcat
31321 gtgccaccat gctgggctaa ttttgtattt ttagtagaga cggggtttct ccatgttggt
31381 caggctggtc tcgaactccc aacctcaggt gatccaccgg cctctgcttc acaaagtgct
31441 gggattatag gcgtgagtca ccaagcctgg ctccaattct gtatattttt aaatgtaagt
31501 ataaagatca aagatacaaa gtatattcac atatcagttt ccatgttgtt attattatta
31561 ttatttgcca ttttaaccat ctccagatga agaaaactga atgtaaagtt aaatatctcc
31621 ttaactttta ctctatttaa aaagagactg aattatttgt gaggtaaccc agttgtagct
31681 gttctactct tctctactgc tagattttgg aagaaccaaa aaggaataaa gcaaagcatg
31741 acacagaaga gaaagcagaa ttatactgta gtagtatgct catattttat cagagtttgc
31801 ctgtactttt gctgattttt ttttctccta gcttggacat tttaaaggct tattttaaat
31861 ttatctcttc tgtattaaca ggtgagaagt ggacaaatca atgtataaaa actactatgg
31921 ctatcatcat ttagaatttt gtcttaaaag taattggatt tctggggtcc tagacacata
31981 atgattagag atcatgccac cagttctata tggagccatc tcaattcatc ctttctcaat
32041 ttatcctttc caggcatttt tcttgaaact gcctaagtac tggcgtctcc ctacgactat
32101 tatatctgaa aacacattca gttttcaaac cagctgaatt ttatgcctga ctattccttt
32161 taacttatac caatgaaaat cctttaataa attcactcac aattgctcca ctttctcaaa
32221 cagagcacaa agcatctaac aatatttctt catgattgat agcatacaca ctcttgactt
32281 catattcttc aaaatcttgg tatttttata agcattagtg cctttttttt ccccagtcag
32341 aagcactgac ggatttattc tattgggtgt tgtcttctgt ttggtcttta gcttttctta
32401 gctgtgatcg tttattgctg aataattaag ggcaattctt tcccactgaa atacttttca
32461 agttaaacat tttttcctta aatttttgcc ccaggctact tctagatcca gcgttgttcc
32521 agcttactga gcattccaga gggctaaggt ccaagttagc caaggtttta aaatgttgac
32581 catgtggacc tgaattcttt ccagttacat atttgcatac ttttaacagg agtggttatt
32641 tctcgtcaaa agacagaacg ctggaaatac cagggcaaaa ctatgtgttt ttttcttggt
32701 gatatcttga tcttagcatt ttactctctc ttcctggttt ctagcttgac ctttgtattt
32761 tagaggagag taatagagta acagtgcact gagaagcaat gggacatgac agaatagttt
32821 cagcagtgaa agtcagggaa gctggtttca accattcatt cacaagctta aaggactttg
32881 gtatctgact ttgtggatgc cactttctca tctgtaaaat aagggggttg gatcgtgtga
32941 tttataaggg gctttcttac tttctaacat tccatattct ataaagacag ttaaagtctc
33001 cagagagtaa agaatgtaga aaattcctga aatttactca gctcctttat tcctaaatat
33061 ttttgaattc tgaaattatt attattatta ttttttgaga tggagtctca ctctgctgcc
33121 caggccaaag tgcgatggca caatctcagc tcactgcaat ctctgctgcc caggttcaag

33181 tgattctcct gcctcagtct tctgagtagc tgggattaca ggcatgtgcc accatgcctg
33241 gctaactttt gtattttcag tagagaaggg gtttcaccat gttggacagg gtagtatcaa
33301 atttctgacc tcaagtgatc cacccgcctc ggcctcccaa agtgctggag ttacagccaa
33361 agcacctgac ctgaattctg agttttgatt ctgttttttt tttttttttt ttgagacaga
33421 gttttgctct tgtcacccag gctggagtgc aattgcacgt tctccgctca cagcaacctc
33481 catctcccgg gttcaagtga ttctcctgcc tcagcctccc aagtagatgg gactaccacc
33541 gtgcctagct aataattttg tgtattttag tagagatggg atttcactgt gttagccagg
33601 atggtcttga tctcttgacc tcgtgaccct ccctccttgg cctcccaaag tgctgggatt
33661 acaggcgtga gatactgcgc ccggcccata ctcacagatt tctttgtgat attatctcat
33721 ttgttttcac cacacccaga tgatgtggat ggtaggagaa atcccatgcg aataagtgga
33781 atatgttagt aactgcttcc ttgggtcctt ttgcctctgg acttaactct tttttttga
33841 gacggagtct tgctctgtca cccatgctgg agtgcagtgg cgcaatcttg gctcactgca
33901 acctctgcct cccagattca agcaattctc ctgcctcagc ctcccgagta gctgggatta
33961 caggcatgtg ctgccacgcc tggctaattt ttttgtattt ttagtagaga tggggtttca
34021 ccatattccc caggctggtc tcaaactcct gaccttgtga tccacccacc ttgaccaccc
34081 aaagtgctgg gattagaggc atgagccact gcacctggcc tggacttaac tcttaaatga
34141 tgttacccag gaaaaccaat ctatgactaa ggcaaaaaaa aaaaaaaaaa aaaaaaaaaa
34201 aaaagttgca cagagaagag tggaaatatc agtaaatcag aagctaggca ggcctaatcc
34261 ctaatccact tggtgacctt ctgcaaaaca cctttctgtt agccttttca cccgtgaatt
34321 tttttttttt tttgagacgg agtttcgctt tggttagcca gactggagta caatggtgcg
34381 atctcagctc actgcaacct tcgccttctg ggttcaaggg attctcctgc ctcagcctcc
34441 tgagtagctg ggattacagg aacctgccat cacacccaga taatttttgt attttagta
34501 gagatggggt ttcaccgtgt tggccaggct ggtcttgaac tcctgacctc aggtgatcca
34561 ccttcctcgg ctgcccaaag tgctgggatt acaggcgtga gtcactgtgc ctggcttact
34621 tgtgaaatta aagcttcaac ttttactcct taggaacgtc tcacaaattt ccacacataa
34681 atctaaagta gacactcctg cttgccaaaa gcatactctc taaaacattg gttctcagac
34741 ggacatagtg gctcatgcct gtaatcccag cactttggga ggccgagacg ggtggatcac
34801 ttgagatcag gagtttgaaa ccagcatggc caacatcgtg aaaccccatt tctactgaaa
34861 atacaaaaaa aattagccag gtatggtggt gcgtgcctgt aatcccagct actcgggagg
34921 ccgaagcagg agaatcactt gaacctggga ggcggaggtt gcagtgagcc agatcccgcc
34981 actgcactct agcctgggag atagagcgag actctgtctt ggaaacaaac aaacaaacaa
35041 acaaacaaaa aaattggttc ttgagcaggg caatttcacc tccaagggga catttgacaa
35101 tggctgggga catttttggt tgtcacatct ggggttgggg agtgaagtgc tattggcatc
35161 taatacgtag aggccaggga tgctgctgaa catcctgcag tgcacaggac agcccctgac
35221 agcattaaac agtccaaaat gtcaattgtt ttttgaggtt gagaaacgct ggtctaaaag
35281 aaatgacagg acaggtggct tggctcatgc ctatcatccc agcactttgg gaggctgagg
35341 cgagtggatc acctgaggcc agtagcttga gaccagcctg gccaacatgg tgaaacctct
35401 tctctactaa aaatacaaac aatagccggg cgtggtggcc cacacctgta atcccagcta
35461 ttcagaaggc caaggcagga gaatcgcttg aacccggggg gcaaagattg cagtgagcta
35521 agatcatgcc actgcactcc agcctgggtg acagagtgag actctcaaaa aaacaaacaa
35581 acaaaaaaac agaaatgact caaatgttta cagtgattac ttctggatgg gattatgata
35641 attgtttctc tactgttcta aaaaactttc tgttctttcc aaatgtttaa tattgagcac
35701 ttattttaca atcattaaac aaagaaatga caacaactgt taaaaaaaaa ttcttaagaa
35761 ctgaaaggca aacaccacca ccactaaagc catgtccccc agatctaggt tgtgagtcac
35821 atgataccga cttcagaggc agcatgtctc aaggcctctc caatgtgtgt cagggacagc
35881 agtgggagta gggccctctg agtcagcctc tttgacctgg gatgctgcag gagatcttag
35941 caaacattca agattttcac tgagtcattt gcctgtggca ttgatctgct cactcaagtc
36001 ttccggtgtt ttggtgtgag ggaggctaaa gaggaaaaac agctgtttag tttctcattg
36061 atttgtattg tcattgagca atacctggag cccaagtgga tgtcttttat tctgtggcct
36121 ccacagctgg gtcagtgtgg aaggcttcag accatgagaa attgttcctg ggagtcaatt
36181 attttaataa gtgagatatc agagtatatt cacagaaaga ggcttccctg gcactgtaag
36241 ggcagccagg aataaaataa aaatgcttct tcagtgaaga taattgtcca aattgaagac
36301 ataggaaata tttctaattt gcctgtgtat ttggtgaaca tttctctctc tctctctctt
36361 tctctctttc tctctctctc tctctttctg tctccataaa tgaagacatg ggaaaatcta
36421 ttaaaaatta aattaaaatg caacactttt acataaacaa aattcccaat cactctcctt
36481 actttacctc agcccaacca aatgtgagtt aaatctcttc cctttcccat gggacactag
36541 catcttggtg gcccagctca gtagctaagc atagagtaat gagtttgtta aataatttca

36601 attacattgt ttttgagatt ccagggtcac agggaaattc tgacctgaat tatctggatg
36661 aactctcaca attgtgcact gccttgttca aatttgggaa gtgctgagaa gcactcatcc
36721 aaggggtaga tctggacttc agctcatgca gcataataga atttcaggct cagtgatttc
36781 taatctgtta ccacttgcca ttttctactt gaagagggca gacaccttca ctgatctctg
36841 tttctatttc atcttttgaa aaatcgggat gataatggtt atctggttta catgctttta
36901 catgcttgtc aggagttatt ttaaagcact ttacaaatat aaaaatgtac aataaaattg
36961 cagagtcgga tgcagcaacc cctgtatcct ttgaggaatc tatcacaatt catacccctc
37021 ccaagccaac ttttctggta ctactctgga aaattgtgct tatacagtta aaatttttat
37081 tttcaaaaag ttgtgtaatt ccctttttca accatttttt tttgttataa gaaatgcaat
37141 taaaaacata cttttctttc caaatagaag agcctattct gccttataag aattgtttga
37201 aagattgaag gacaaatgaa gtcatacaac ttctctgata ccatattgaa ctcaaatgaa
37261 cacattttga ggtaagaaat gagcctggaa gatttttctt tttttttttt ttgagatgga
37321 gtcttgcttc gtcgcccagg ctggagtgca atggcgcaat cttggctcac tgcaacctcc
37381 gcctcctggg ttcaagtgat tctcctgcct cagcctcctg agtagctggg attacaggag
37441 tgctccacca ggcctcacta attttttgta tttttagtac agatgggttt tcaccatgtt
37501 ggccaggttg gtctcagact cctgacctca tgatccaccc gccttggctt cccaaagtgc
37561 tgggattaca ggcgtgagcc actgtgcctg gcaagagata gaatttttca caaaaataac
37621 tcataccaga aactttgtag gcccccaccg atgcccataa tctttaggaa acaaactcta
37681 gtgtcaccta tttcctgata ccttctgtaa cgaccaggtc atattctgag ttcctacagc
37741 actcagtgtt tctcatcgga gctgtagcaa tggagtaata aatggcactg ctcaggaaga
37801 gagaattcag aaagaaaaga gcagggccta gaaaaggaac aaacttatcg gatgtgtaga
37861 gaaagaatct gcaaagcaag taataaaatc cagaaaaacg tggtatcatg gtggtcacaa
37921 aaaaaagagt ggttaaattt tcgaaaatgg agacaaacca ctcaccatga tcgtgccatc
37981 tgaatagaaa cacagaaatg atgacaaaaa aatggctaaa gaaagaaggt caacaaccat
38041 gtccaatgct ttggatatta agaagtgttc agatggattt agcaagtaga aagtcactga
38101 cacttggtgc acaaccagac ctcaatgtat ttaggaggaa actgttattt tggaacaatt
38161 gaaagctttc atttggctga gaaatatcaa ctcattctta aataggtatg ttagaaggag
38221 cctctgggcc tgatatctcc ttgattacaa ggaaaatgaa taatagttgc gtctgataac
38281 catgacatat cacccctatg tttgcttgta gctttcagca gctcagaaca ttgttgaaaa
38341 cactcattat cccaatatcc ccaacaggga agtgttaaaa cctgccttgt gcaactgtca
38401 atctctgatg attagataaa aacaattatg tcatgagttg tagctattgt tatttacttc
38461 ccaagagcca gattacacag gtatctgatt gctgaagcaa tgtctggatg taccacgaaa
38521 ttccacatgc atgtacagta taaacacctc tggctatcac cttatctaat tttgatcaga
38581 tacaggttaa tcccctcctg tatctttata tgtcctgaat ttgcctcatc cacctgaact
38641 ttgccataat ttactctcaa tttgtatgtg tctgtgatgg agttgttgtc attttccttt
38701 tctttttctt ttttttgagg ccaagtctct ttctgtcacc cagactggag tgcaatgacg
38761 cgatctcagc tcactgcaac ctctgcctcc cgggttcaag taattcccct gcctcagcct
38821 cctaaatagc tgggactaca ggtgcatgcc accatgcctg gctaactttt tgtattttta
38881 gtagagacgg ggttttaccg tgttagccag gatggtctct tgatccgccc gcctccgcct
38941 cccaaagtgc tgggattaca ggcatgagcc accgcgcccg gcctcatttt ctctatctgt
39001 ctgcttaatc aggttttgca agtttcacat ttcctcaact aaacttcatt ttaccaccca
39061 agcaaacttc attataatta ttttattttt ctgatgacat cagatttctg taggggtacc
39121 tattagctag agatacttag attttatttt cccagcatgt gactatttcc cattctatac
39181 aacacaatgg caagagcagg agtatgtttt tggacaatta aagagagaaa aggccgggcg
39241 cggtggccca tgcctgtaat cccagcactt tgggaggctg aggtgggcag atcatgaggt
39301 caggagatca ggagattgag accatcctgg ctaacacggt gaaaccccat ctctactaaa
39361 aatgcaaaaa attagccggg cgtggtggtg ggcacctgta gtcccagcta ctctggaggg
39421 tgaggcagaa tggcgtgaac ccgggaggca cagcttgcag tgagccgaga tcgtgccact
39481 gcactccagc ctgggcggca gagcgagact ccgtctcaaa aaaaaaaaaa aaaaaaaaaa
39541 gagagagaga aaataactca gcttcattta gatttctatt attcttctct gatccatata
39601 gtggatgttc acagaagtgt aggaaccaaa actatgcaag ttaaactgtt aatttagaa
39661 gtttatagca gtgatgtgga aggctgacca aagttggcgc tttggtctca ctataagcca
39721 tagtaataaa tacattaaca atgtccacaa acaacagtag acaccattta gtaagggctt
39781 gctccaagcc aggcactgtt ttcagcttaa gtctcacagc cttgtgaagt agattctgtc
39841 ccatcttaga gcaagttggt ctgaggatcc tcaagggtaa ggtctcttgc caaaggtcag
39901 cagaggtagt gagttcagaa ctgggattta aatccaggct gttctatttc tattccacaa
39961 ttctgcctca actgcttctc tacattttag gcacacgttt gaatactgaa aatttgggtc

40021 acagagttgg agatgtttct tcttctaggc cccaaaccgg agcggggtgc agtggctcaa
40081 gcctgtaatc cgcactactt gggaagctga ggcgggagga tcgcttgagc tcaggagttt
40141 gaagatacag tcagctatga tcctgccact gcactctagc ctgggcaaca gaacaagatc
40201 ctatctctaa aaaaaaaaaa aaattaaaat tttaacacaa gggcccaaac aagtcaacag
40261 taattacaac atgatcttta ctactgtcta ataacatctc acagtgcttt aaacatacgt
40321 agttaccatt ctgtccactt ggttgcctgt aatattaact cagtaagtag gaatacttcc
40381 tgccctgact aatatactca taaatatatg ctctaagtca agggtcttgc aggttctgat
40441 ttaaaaaaca caattttttt gagggtcact ggctttacat tatctcattc ttttcaagtt
40501 cctttgtacg taaaacatcc tatgctcagt tatatgatct gttcaacctc ctgcttgaca
40561 gatatgaaaa cagtgctctc atgtaattta tgtaatttac atttttacat atttagcagt
40621 acacctttgt gctatgttat attgtttgag aactaatctg tttttctgat ggctggaagt
40681 ctaagtgaaa agattataca tgaaaataat tcatgtttat tatcactagg agaaagtaag
40741 acaatttagt ttgattcaca agcatttgaa tccctattgt gtcagacatt gtattggaaa
40801 ctggggatac aaggaagata taacctcatc tctcagagtt ccctagttat cccatacata
40861 tatacaatta ttatttgtca attaaatttt ttttaaaaat ttagaaagga gtttccaagt
40921 aagaaaaatc atagcgaaat ggtataattg gtataataac agttaattag ctatagtatg
40981 aaaatacaga ggaagatcta actaagttgg ggattattaa agaaaattta aattagctgg
41041 gtgtggcggc agacgcctgt aaccccagct acttgggagg ctgaggcagg agaattgctt
41101 gaactcaggt ggtggaggtt gcagtgagat gagatcactg ggtaacagaa caagactccg
41161 tctcaaataa aataaaataa aaaataaaga aaattggaca gagatgtggc atttgaactg
41221 tctttcagat taagaagaaa gttactagtt agccaattca aatataagat ttacaaacat
41281 agagtacagc atgagcaaag gtgtggaggt acaaaaagaa taatgttttt gaggggagaa
41341 tagcaagtac tctgggggtg gccaaagttt gagttgtgtg gttttggtgg tggatggaga
41401 tgcaggaaga ttaggaagta gattgagatc agattgagaa aggtcttcca gaccagatga
41461 aaaattttgc catatttagg caatagggag ccccagaaat tgttcaacca tcatcagctt
41521 tacatttcac tatggtaatt tggcttcata agaagcatgg gttggagatt gataagagca
41581 gactccaagg ggccaattca ttaaactcaa ctgccaataa tgcaagagac aatgaaagcc
41641 agaatgaaca cagtgaccat ggatgccaga ggcatttcta ggatttttgt agaggactta
41701 agagaccatt tgggagggga ggaaggcaga agataagggg aaagaaggag ttgatatctt
41761 tgatgactga tggcactttt gctaagatga agaataaatg gtagcaagaa taaattgact
41821 gggcgcagtg gctcatgcct gtaatccaac catttgggga ggctgagatg ggtggatcac
41881 ctgaggccag gagctcgaga ccagcctggc caacatggtg aaaccccatc tctactaaaa
41941 ataaaaaaaa ttagctgggc atggtggcgg gtgcctgtaa tcccagctat tcgggaggct
42001 gaggcaggag aatcgcttga acctcgaagg tggaggctgc agtgaacgag atcaagttat
42061 tgcattccag cctggacaac aggagcaaaa ctctggctca aaaaaaaaaa aaaaaaaaaa
42121 aaagtataaa ttgggggagg ggaggagata taggattcct attggaatgt attgagtttg
42181 agattccagc ctggacatat ggcactcttg aattgtttcc taggcgatca tatcactgtc
42241 ttgatgatat ctttaatcac acatatcact tgtggggaaa gaagaaactt caagagtttt
42301 acacaccaat cctttacttt gccatctaga caaattatat ttttaccaat gccatttttt
42361 gttgttattt tgtgggttct gcttaaaact tcccctatat ttttctaatt atggtaacct
42421 agggaaaaca tcaaattatg ttgataaaat tggctacaat ctggctctaa tgataatagc
42481 ttagaatcat gaagcagcaa tattttatcc tgagagcaaa caaactttat cctgaacaca
42541 tgtgttcttt tgccttattc atctaatatt acttgaaact tttatttttc cactttgaat
42601 agtagattct gccaagtgtt ttaaaagctt ttctcttttt aggggaggag gggaacagct
42661 tttctccctt ttgtctgata actgaggagt gaagatttaa ttggtgagaa aaatatacac
42721 actataaacc tcagaatgtt cccactgctt ttaatgtttg atgtgcctga cagattaccc
42781 tttcaaaaca gataatagga taggtggtgc tggaaaaaaa cttttccatc caagacaaaa
42841 ccacatggac attgaaaaga actggcattt gcctagacat ttagtggggg acatgtcctt
42901 agggcacaga ttccatagtg aaaggctctt ctcccccttc cacacactcc agctctctct
42961 agaaggtata atggaaagaa ctctgagcaa gaatcaggaa gttggccttt gttcctgact
43021 gtgccacttt gtggaactgg gcagaactta cctcattctg tgctttagca gcttcatttg
43081 caaaaattaa tgattacctc aaatgatttc tgattttagc caaaattaaa tacagtagtt
43141 ttaaaacaac tgctacatat taactactta cgtatgtaat tttgcactca aaataaatta
43201 tattatggaa aagaaactca acctatgtta accatccctg tactgatgag gaaactaaga
43261 cttaggaaaa agaagtgatt tactcagtat tgggcagtta gtgagcattt ttgagccaag
43321 gtcttacttt aaccagagac ctttgcataa tattgtactg ttactaaaca ctttctatta
43381 tttgactaga ggaacacaac atattgatgc atttgaaaca ataaaataat aaatattttt

43441 ccttaccctc ggcagaaaca ggatgatgag aaacattgac taaaaattta atcaagcctt
43501 aaggacaaat agtatcacag ttttgaatag gagattgggt accttggaag taattatgaa
43561 gaggtgtttt aactcagcag taggctcttt cttattcctg ctaggaatga agaagcacaa
43621 attgaaagtg aagaaggagg aaataaaaca ctgactctat gtgtaagaaa ggtgacttgc
43681 aaattatgag gcagttagga aaatgtttat tttttcttat tttataggct gtatatattt
43741 gagaattaat aattgtcctt tggagggtgt gtgtgtatgt gtatgttgga gctttgtgtg
43801 tgttttagtc cattttccta ctgctatgtg aagaaatgcc cgagacggta atttataaag
43861 aaaaagaggt ttaacggact caccgttcca catggctgtg gaggcctcac aatcatggtg
43921 gaaggtgaag gaggagcaaa ggcacatctt acatggtggc aggcaagagc gtgtgtgcag
43981 gggaattgcc ctttataaaa ccatcggatc tcacgagact tattcactat cacaaggaca
44041 gcatgggaaa aacttatccc catgattcaa ttacctccca ccagctcccc tcaagacaca
44101 tggggattat gggagctaca attcaagatg agatttgggt ggggacacag ccaaactgta
44161 tcagtatgcc tgtgtgtatg catgtgcacg tatataaatt ttttcactag tgactgatgg
44221 accatctata aaataaacaa aagagaagag acagggtctc actttgcctc ctaggctgga
44281 atacaatggc atgaccatag ctcattgcag cctctaattc ctgggctcaa gtgatcctcc
44341 cttctgagta gctgggatta caggcacgtg ccattatgcc caactaatgt tttgtttgtt
44401 tgttgtggag atgggagatg ggagtactgc tatgttactc aggctggtct tgaactcact
44461 cctggcctta agtgatcctc tggctttagc atcctaaacc actacaatct taaaaaggac
44521 cagtttcctg gattacctct ctccctccat catccactat ctgccgtcaa ctctatagag
44581 tcaagggcta tccctgcctt ttcaatagaa gccctcaaga agtcactcca tagggactat
44641 cttgggcttt gggaagtgta cagggttggg gtgggtggga aggtgaggag gaaaggccac
44701 tgtttcaggt ggggttggag ggagatggaa aatggaccac agaggttcag agaacaggcc
44761 ctttgtggaa agggggagac caaataatgc tgcgttgtga aaggtgaaac aggctgcaaa
44821 cagaaggagc ccacaggaag ccctgctctg agcagctgaa cattctgggg tcctcagact
44881 ccgataaaaa agcacattta gcctacagct ttatgccctt cctcctaccc ttgtggactc
44941 tgccacagac atgccctcca gattgattct atccacttgt ctctagtcta ctgctactat
45001 tttagtctaa ggccttctcc ccgctttcct gggttatggt cacagactcc tatctgagtt
45061 cccagcacca ggtgcccagt tgttttcatc tcttaacaca ttccacatat tatagcaaga
45121 gagatctttc tcaaatgcaa atcagacctt gttgcatccc aatctcaacc cctttagttg
45181 ctcttctctt tgccctcatg gtcaagttca agctctctag tgtgggggca gatgttcctt
45241 tataattttt ctgcttatgt ctcctgcctc aggcctcaca attctccacc tcacagatga
45301 tgtctcagcc atgatgagtt cctctcaaat cctttaggtt tcagagatgt catttcccct
45361 gggaacattt tcctgactcc tcgttccaag catatgtctg tctttatgtt cccatagcac
45421 ctgatcattg gatcatggta gggtttatca cggtctcgta tttgtctgct tacttgtctg
45481 tattcttcac ttctcaaata tgtcttcttt gttcatcact gtaactcctt tccccagaat
45541 gttctcagat atttacaccc atcttccccc tctgtgaagg gaataaatgg atatataaca
45601 ctgtattagt ccattctcat gccactatga agaaatatcc aagactgggt aatttataaa
45661 gaaaagaggt ttaaatgatt acagttctgc atggttgggg agaactcagg aaacttacaa
45721 ttatggcaga ggcacctctt cacggggtgg cgggagagag aataagtgca agtaggagaa
45781 atgccagatg cttctaaaac tatcagatct catgagactc actcactatc acaagaacag
45841 cacgggggaa accgcccccc atgatccgat tgcctctacc tggttccacc ctagacatgg
45901 tgattatggg aattaaaatc caaggtgaaa ttttggtggg gatacagagc caaaccatat
45961 taagcaccat cctcaaaata gccttccagc tgaagacagc taaggcactc atgtagtctt
46021 attttgtctc agacaaataa ttttaccttt gaatcattgt tcattaatct agattttaac
46081 ctgaggccct aaaaattgtt tatcatcttc tgggagatct tactcctaga tggtgtttta
46141 ttccacaggt taaatttaaa cttgggccaa atctccatcc ttgtggcctg tgagtactga
46201 atttgggact gaaagcctgg gactaactgg aagtgtggaa aaggaggagg gtgtggcgct
46261 gccctcaatg attttttttt ttttttagga tttctggtgg aaaaggagag acactcagga
46321 atccccagat aaacatggtt tagcttttgt agtcagatcc tttccccaaa tttactgctt
46381 ctttctccac cacttctcaa ccccccaaac tgaacttgcc tcttcaacac actcccttgt
46441 gccctataga cagtcacaag cttgacctat agagagcatg tatttcttga ggcaacgaag
46501 gtaggaaacc ctattctaaa atgtttctta cacttttggc tctgtctaaa ttctccattt
46561 ttcaattaaa atggaaacga gattagaatt ctattctagt tatgaatata atcagaacac
46621 aataaagcag aaagattgct tcaggctctt ctgttaacgt tacatttatt caacaactct
46681 ttatggggac ttgctatgag tgaggcacca ggctaggcac cgcagctaca aaggtgaaga
46741 gaacataggc tcttcttcaa agaaacttac actgtaagct ttatagttat gaaatcatga
46801 ttgcttacct taaatgttct tgacctacat tcttagatat tttgagtctt gagaaatttg

46861 tttatttttt tcattattat acaatgtcaa ttagctcagt ggaatccaag atgttcggct
46921 agccttctga tccaagatag tggaccaaac atttgtattt actcattgtg cccctccata
46981 cttctctgaa ataaaagtgt agatcttcaa aattaaacat atctataaag caaagggaac
47041 agaaggggac attatcagca gattagacat tccaaaaatt ttttagaaaa taaaagctaa
47101 attgaatagt aagacacgga attattagat gtgtttggaa aaccaacagc atgaacaaga
47161 taatccaaga aaaacaagtt gtcccttggt gaaaatggaa ggaatttgag aaatatagaa
47221 gtgaactaaa caataaatac agtcatccct tggtatctgt tggggactga ttccaggacc
47281 tccctcagat accaaaattt ggatgcccaa gtctctgata taaaatggta cagtatttgc
47341 atacagccta tgcacatcct actgtatact ttaaatcatc tttagattac ttataatacc
47401 taatacaatg taaatgcttt gtaaatactt gttatattgt attgtttagg gaataatgag
47461 aggaaaaaaa gtctgttcat gttcagtaca gatgcaactt tttttttttc cagaatcttt
47521 ttaacctatg gttggttgga atccacaaat gtagaacccg gctacagaga gctgactgta
47581 attaaaacca acagcttcta tttggtatac tgaaagagaa ttgggaaatc attaaatcca
47641 taaaataaga acaggatgga gtgaaaaaag ctggactctg tgacgaaatg gacatatgta
47701 tggataagaa tcaggaaagt tttcataaag gaagtgccaa actgagctag ttggccaaat
47761 gaactattct ccaggtggat ggggcagggc atttaggcag aggaagcaac aaagcacaga
47821 gtgaagccca ggaaactaca ggtagctgca gggagatgac ggggctacag tgtgcatgga
47881 gagaggccaa catggtgagg taggcaggag gagctcctga agggctccaa cctgggaagc
47941 caggctaaag agatggcatt ctatcttgtc agtaataaga agcattgaca ttttgtagag
48001 atgatgaatt gaggaaggga tttatgtggt catttgtggt tctggctgat ccccctgagt
48061 gctgtctgct agaaggattc caagggttaa gtcctgaggc gtgaagaaga accgatctgt
48121 aagatagctt aactaaggtg gtagcattgg gataagggtg tggggtgtgt gtgtgagggg
48181 gatacaaagt tattaaggtg gtagaattga tggcaacatt catcttgaca gagaatacag
48241 gatgaggagc agatttcaga ggaatataat gagccctttt ttagatcttt gtgaatgttc
48301 agaaatggca gctggtgttg cataattgca aaattagggc ttagttagtt attggcttct
48361 gagaacgtaa ctgcagtttt tttctttccc tctgccccac tctttgaaag gggtggggc
48421 tcaaagagta gtgtgtgtgt gtgtgtgtgt gtgtgtgtgt gtgtggtaaa tacttatata
48481 caaatttctt tagatttttc tttatctcct tcactccttt tcaacatttt ggctggttgt
48541 tctgatatta ccaaggtaat ccacaattat ggcaaatatt caaacaaaac aaattataga
48601 aagtccagaa aaaaaaactc tgaaatacct tttccagaga ccaccatcat gaacagtttg
48661 gtctacagaa ttacaaatct tttgaagtat atagtatatt cttttatgtg gccattcatt
48721 atattttatg ggatcttagt attattgctt ttaaattcat ttcttatatt ttaaatttga
48781 aatacttcaa aatatgcaga aaagtagaga aaataacatt aacaccatat acttaccatg
48841 tagatttaag agaaatctac actttgccaa atttcctttt tttttttttt tttttttttt
48901 ttttttgaga cagagtcttg ctctgtcacc caggctggag tgcagtggtg cgatctcggc
48961 ttactgcaac ctctgcctct tgggttcaag caattctcct gcctcagcct cctgagtagc
49021 tgggattaca ggtactcacc accatgcttg gctaattttt gtatttttag tagagatggg
49081 gttttgccat gttggccagg ctggtctcga actcctgacc tcaaatgatc cacctgcctc
49141 ggcctcccaa agtgctggga ttacaggcat gagccaccgc acccgaccaa atctccattt
49201 ttgtaaataa tttttcagat atagaaaact acccaattat ggctgggcat ggtggctcat
49261 ggctataatc ccagcacatt gggaggccaa ggcaggcgga tcatttgagg tcaggagttc
49321 gagaccagcc tggccaacat ggtgaaatcc cgcctctaca aaaaatataa aaattagccg
49381 ggcatggtgg caggtgcctg tagtcccagc tacttgggag gctgagggag gaaaatcact
49441 tgaacctggg aggcggaggt tgcagtgagc tgagatagca ccattgcact ccagactggg
49501 tgacagaaca agactctgtc tcaaaaaaaa gaaaagaaaa gtaagaaaac tacctgatta
49561 ctattacttc tctctttctc tctctctctc tgtgttctag aggtttgcag tatcctgaag
49621 ctggtgtgaa tcaacctcac gctttttttt tttttctttt tttttgaggt ggagtctcgc
49681 tctgtcgccc aggctggagt gcaatggtgc aatcttggct cactgcacct ccacctcctg
49741 ggttcaagtg attctcctgc ctcagcctcc cgagtggctg tgattacagg catacaccgc
49801 catgcccagc taattttttg tatttttagt aaagatgggg tttcaccatg ttggccaggc
49861 tggtctacaa atgtatctat ccataagcaa taccgtatat agtaatattt ttattattct
49921 attatttcta ttgaatgttt atattcatag tatcacactg tacacatcct tttgcaattt
49981 gctttcttta gaagcaacaa tatatttttg agatttatca tgttgctttg tgtagattgc
50041 ttactttttt tttttgatgg agtttccctc ttgttgctta ggctggagtg caatggcgta
50101 atctcggctc accgcaacct ctgcctcctg gattcaagag atcctcctgc ctcagcctct
50161 tgagtagctg ggattacagg catgtactac cacactcggc taattttgta tttttagtag
50221 agatggcttt tctccatgtt ggtcaagctg gtctcgaact cccaacctca ggtgatctgc

50281 ccgcctgggc ctcccaaagt gctgggatta caggtgtgag ccactgtgct tggccaatta
50341 tttacttttt aatttatttt atgatacaca atttgtctct ttatctcact ttattttttg
50401 attttttttt ttttaaacca ggatctcact tggtcaccca ggctggagtg cagtggcgtg
50461 atctcggctc actgcatccc ctgcctccca ggtgcaagca attctcctgc cttggcctcc
50521 tgagtagcta ggactatagg gtgtaccacc acatctggtt aatttttgta ttttagtaga
50581 gatggtattt tgtcatattg gccaggctgg tcttgaactg ctggcttcga gcaatttgcc
50641 cgccttggcc tccaaactct ctttattttt tagatattgt taacggtact atgggcagta
50701 tccttgaaca tctctctttg tataaatgtg aaagacattc atagacatgg gactaaagga
50761 ggtgcccata gtcaaccatg ctaggcattg acaaatggct tcctaaaggg gtaatacctg
50821 tgtacattgc cactagcaat ttgtacactt tcttgattcc tttcactctt gtcaaaactt
50881 actctttttt aaatttaaaa ttattggcca ggccatggtg gttcatgact gtaatctccc
50941 agcactttgg gaggctgagg caggtggatc acctgaaatc aggagtttga gaccagcctg
51001 gccaaacatg gtgaaaccct gtctctacta aaagtagaaa aattaactgg gcatggtggt
51061 gggtgcttgt aataccagat actcgggagg ctgaggcagg agaatcgttt gaacctggga
51121 ggtggaggct gtggtaagcc aagatcacac cattgcactc caacacaggg gacagagtga
51181 gactctgtct caaaaataaa ataaaataaa ataattgtca atattgttgg ttatgaaatg
51241 ctatctcatt attgtttaaa gaggcatttc ccttgataac tagtgaggtt gaattttgtt
51301 ttccagatgt gcatgagcca ttggtttttt tctgttatgc attttctatt catatacttt
51361 gaccacatga tatgtgcctg gtcataaccc atcaaactct tctttctctc agatacttca
51421 ctttatggaa atctttttca cgtttatccc tcaaacccag aattttgagt gtcatacctg
51481 ttctaacata aatttgagga aaatgaaggc taattcagaa taaagaacta tataagcaga
51541 tggacagtgt gttctcctct aaataggggt gctaggataa gaggcggtag gataaatgca
51601 cagcctcaaa atccaccacc cactaaagac ctcacagtac atgatgacat tagcatgctg
51661 tgccagcatt tctacctacc acgatgacct ggaatgaagg acaaaagggg caattgttaa
51721 gtcctgtgcg taaactttgg aagagaacat accttgcttt aaaccattaa gggggcaggc
51781 aatagattcc tgtacagggt ggcttagcca aaagtggctg gtcttgttct catttatatg
51841 gtttataatg attctaaatc attctcggag aaaaacagac tcatttcatc tccctttaat
51901 taggatttct gttactgcaa tattttagtg gcatttatgt caaatgtttt cttttgagaa
51961 cttctccctt ttactgtctt ttttctttta ttttaaactt ttattttagg ttagggggta
52021 catgtgaaaa ttggttacat aggtaaactc atgtcaagag ggtttgttgt acggatgatt
52081 catcacccag ggattaagct cagtactcaa tagttatctt ttctgctcct ctccctcctc
52141 ctactttcca ccctcaagta gaccccagag tctgttgttt tcttctttgt gttcataagt
52201 tctcataatt tagctcccac ttatatgtga gaacacgcag tatatgattt tctgttcctg
52261 cgttagtttg ctgatgataa tagcctccag ctccatccat gttcctgcaa aagccatgat
52321 cttgttcctt ttaatttact ttttgctatt ctgattagag agtggccatg ctccatgctc
52381 catatggata ttctttggaa acaaagcatt tttgaaatgg taggattggg gctagtaaaa
52441 gagaacagaa agaattgtga acagggtctc ctccagtaca gatggttaaa gaacacaaat
52501 agggaagtgt gatgagtgac ctccatttaa ttgggcgccc aagctcttgg ctgctccagc
52561 atgggttgcc atcctgactg atcccttggc tcagaaacag atatggtgct atcaggaaag
52621 caaggccaat atctgaatat tgcagcagag actgctccca tttcagaaag caacccagat
52681 taaagttgat gattcagttt caataaatgc ttcctttttt ataaaaggca tttaacatca
52741 ctctttcccc tggtaatatg gaattagctt ttgggatcaa ctggaaaatg tcagagggcg
52801 attgtaaaag gccacctgct aaccacagga agctgggctg atacagaatg ccaggttaac
52861 ttaaggtcga taatggttac ttccttgtca ctttttacat gcatccttgc tctgcagtgg
52921 gggtcataag aagtaagatg catgcaaggt gaatttctag aagtcagctt ctctgagaag
52981 tattctctga ccatacattc ctactagtcc ccacatacgc tgctgatctc cccatcttca
53041 cccccattgt tgaaattgct ccctcattgt aactgttcac tcatctgtct ccaccataaa
53101 gcttaggctc ctagaaagtc aggaccagag ctagtacagt gccccatgtt cttatggaat
53161 gattttagtg ccttgagttt ggtgtggtga ccagagctgg tggccaactg tcgtgaggcc
53221 ccagtccttc tttttgtcat gctttccctc ccagcccctc cttgaccact ctttcacctc
53281 catctttcta cttatataga gacaaataat aagatgcttg tacaaagggt gtacttgtat
53341 tatcccattt gtgttgatat tccacaacag caaattaggt ttttttgttt gtttgttttt
53401 tgatggagtc ttgctctgtt gcccaggctg gagtgcaatg cgtgatctcg gctcactgca
53461 acctctgcct cccgggttca agtgattctc ctgcctcagc ctcccgagta gctaggacta
53521 caggcaccca ccaccaagcc cggctaattt ttgtattttt agtactgacg gggtttcacc
53581 atattggcca ggctggtctt gaactcctga cctcaggtga tctgcccgcc tcggcctccc
53641 aaagtgctgg gattacacgt gtgagccact gtgcctggcc tgataaggtt taaagattaa

53701 ataccatgtc gttaaatttg caaatctctc gaataggaaa atcaggaaca atgacaacat
53761 tcggagcttc ccagaaggta acaactatgg ggagttttgt gggcatctga gagaagcaca
53821 cacacagatg gtttgccttt cattggtcca ttcttgccca gaaggcgaca ctaactgaaa
53881 caaagaagcc tcatatgaga aggacatggg gtcacagcag cttttggggt ggccctgggt
53941 ccttgatctg gtctttctct ggctcatttt gtattgctca ctttgttact tcacaatcca
54001 gggcttgtga catttttctt ctctggagtg gaaatgacag agctgcagaa cacaagtttc
54061 tgattggttc ctatgagtca ggaaagaggg ccagttgaaa ctgggctcac acacacacac
54121 acacacaaac taggctcaca tgtgttttcc catcagctaa acaagttttc agaagtacag
54181 aaataactcg ttggggaagg ggacccagga caatgacaat gtttttgatg gtaatgtctg
54241 cagaagtaag gcaggtttcc tctgtgcctg aaaagatggc accatttgag gagatggcat
54301 actcgtggtg gaatcttcat taagatgtca aactaagctg taggaaatca actctagaaa
54361 acagctctaa tgtgagaaaa catatatcca gttttataga gcaagagctg tgatcatggt
54421 gacatttaac aactgtatta gttggccaag agggtaaaaa tatacttgac ctcttagagc
54481 accataaata ctctcagctt gtgggccaac tgtgtcatgg tcttttttct agcccctcct
54541 ctttctccct tccttcttgt ctctctcctc ctctctcttc cctccccttt ctcctttctg
54601 accctctttt ctcttctcta tcttctcgaa ccttcccttc ctcctctcct ttaggtgctc
54661 agcacctctt tgattcaata aacatttacg gagctccttc ttgtgccaac gactgtacac
54721 agagctggga tgtcaagatg aatcagttac atttcctgcc caatgccaag ggatggcttg
54781 atgggttttg accagcccct ccctggactc ccaatttctt tttttttttt ttcttttttt
54841 tttttagacg gagtcttgct ctgttgccag gctggagtgc agtggcacga tctcggctca
54901 ctgcagtctc tgtctccctc ccaacttcaa gcgattctcc tgctcagcct ctcgagtatc
54961 tgggattgca ggcatgcgcc accacaccca gctaattttt ttgtattttt agtagagaca
55021 gggtttcacc atgttggcca gggtggtctg gatctcctga cctcgtgatc cacccgcctt
55081 ggcttcccaa agtgctggga atacagtcat gagccaccgc gcccggtgcc ctggactccc
55141 aatttctacc tcctggaacg tgctttactc accagccttg gtgttattct gcaatatggc
55201 caactgggat ttttttcaat cttacagaaa ttattctcgg aaatgtttgc tatttttttt
55261 taatgaagct acagcataca atttaaggat aaataatgta cttactctgc agctattgtt
55321 tcaagtcaaa tccgctgatt actcctccaa aattcttttc ttaataaaat ctctgaccct
55381 ttcctcttcc tgttagaact tctggagcta ccattgttcc tcctcttggg ctgctcataa
55441 cctaggggag tcacagtgaa attacttgct ttaaaattct ttaatcactc tgcgagttgt
55501 taggaaaagc tgacacatct gatttggaaa ctcccactcc agtggactgt tgtgcctcag
55561 cacacaggga cgggtggagc aaaggaacaa gaagccaaaa actctcaggg cacttactat
55621 gattgtgtaa aagagagact catcaattaa cacaggtact ggcaagtgca cttcataatg
55681 cctccatttc tagacagatc aaaaacagtg tgccctgtca ataaatgctg agtttctgta
55741 gcagcctgat tgagatcagc taacctttag gccaaaagcg ggattcttcc ttgctgaagc
55801 ttttggcaaa gcatattgag cttcatgcca gggattagat taactcttgc aaaactggtt
55861 caccaggaac aatgactcag tgtttttttt ttagtatccg gcatattgtg tggagcagct
55921 ataaatatat ttacaagctg gagtttcaag ggaggggagt aatagcttgc tgaaaaacat
55981 gccaactgag ttatgggtct tcacgcagcc agcatgctgt gttttgggct ctgcctcttg
56041 taatttttag aaattaatta gtttattctt tatttatatt tatttattta tttattttca
56101 gatgggatct tactctgttg cccaggctgg agtgcagtgg tgcgatctcg gctctctgca
56161 acctccgcct cccaggttca agtgattctc ctgcctcggc ctcctgagta gctgggattt
56221 caggtatgcc tcaccatgcc tggctgattt ttgtattttt agtagaggca gggtttcacc
56281 atgttggtca ggctggactt gaactcctga cctcaagtga tccacctaag ggtactggga
56341 ttacaggcat gaaccactgc acccggcctg ttggttgtaa ttttattttc ctcctcaagg
56401 actttgagca cattatcttc tctttaataa cttgtttcca ccctcctttg tgcacgcgcg
56461 cacgcgtgtg tgtgtgtgtg tgtgtgtgtg tgtgtgtgtg tgtgtatttt ctgatctccc
56521 tacgggggca gagagtctca agcaggtatg tccatgttat taacatactg tctgcttatt
56581 aactaactgc accaatgatg aaagccaact gacaaacaag tgatgctctt atccctcctt
56641 cgcaagcaat gaatctgaaa gccaagagaa gttttccttt caacaggaag tgttttggag
56701 tccgaacaac tctgcattta gacagtgcag cttttagtta cagatcttgg caatggggca
56761 ggcagaggga gcggcaacta cagactttgc cgtaggactg ttttctatgg agtgtgtgtg
56821 ctctgacgag ctagtgacag gagggaggtt acaaaagcat ttactgaaat aaggatttct
56881 aatagaggag ctttttaaaa gtattaaata tgctaatgtg cctagggcat agtcaacaaa
56941 gagttataat acatgtcatt tttcatattt atcaaggaaa ccatgtcacc cgcagccttt
57001 ctcaatttct ggcattctga gaaacacact gtagtggtgc ctgagggtct ggggaaagaa
57061 gaatagaatg aatggggcat ctcagaatcg taaactcccc cagttgtagt tcctttcgat

57121 gttggtcgtt ggatgaattt ccagtttcta ctattgtggt tggattgggg agagtagctc
57181 aaattgggaa ttgctttttg aggatctagg gcttctggaa ttctcccagt ttctttttac
57241 ctctttatgc tatcttgtct actttgaaaa atcagtagca tccatttgat tgcagagctg
57301 aatctttcta tacagtgagg agttgtaatc acattactta taacctgctt atttaacttt
57361 ttaaaggtgc aagtcattgt aacctaagta tttactcaca ctgatcctta atatactccc
57421 ctttcttatc tgcaagcttc tttctctggt ttctcctaac tctgttactg ctcatgagtt
57481 taaaactttg tcttctcagt tcctggcttc acctgtttgg tctctgctct ccaggactct
57541 gccttggaaa tctgactcac acttctatat gtccctgggg ctcaacttcg ggcctggcgc
57601 cagaatcacc caggctccaa gcaagccttg tagactccac tcagtccttt gactcaagga
57661 aatctccact tctaaatggc tctccggttg attctgatgc cagtccaggg ttgagaacca
57721 ccgctttagc ttctagacac acagccccct cccctttttc ctggagcccc tcttgggtca
57781 tgattctatt ttccctattt ctgtactcag aagggataaa aatggaagtg tatgtgaagt
57841 tgctactacg tcccaggtgc tttaaatatg ttattttatt caatcttcac aacagtctta
57901 tgcagtctct atttttattt taagaatgag gaaaacaata cttgggtgca gtggcttatg
57961 tttgtaatcc cagcactttg ggaggccaag gtgggtagat cacttgagcc caggagttca
58021 agaccagcct gggcaacatg gcataaccct gtctctacca acaatacgaa aattagccag
58081 tctcataacc tggtctataa ataaataaat agataaaaat ttaaaaataa aataatttaa
58141 aaagaaaaga atgaggaaaa caagattcag agagccttgg tcatcttccc aaaggtgcac
58201 aattaagaaa tggctaagca ggggttctga ctcagatctg tcagcatcta cagctttgct
58261 atttaaaaga gcgtggtccg tggactagca gaatcagtgt cacctgggat cttggtagaa
58321 acacaaatac ttgagtgcca tctcagacgt actgaatcag tttcttcagg ggtagggtcc
58381 aggaatatgc attttatgaa ggcctctaaa tgtttctcaa agtttaagaa gcatggggaa
58441 ctatatcttt tcctactata tagtcttatc tctttccttg tttctttact ccaaagtatt
58501 gccttatttc tttcccagaa acctcactat cagctcactg tgcagcctta ctcatatctt
58561 cctcaaccag cagttttatt acacacaccc ttctcatttc ctcacattgc tcatggtatc
58621 tgactgagta cagggatcca acctggcttt gggttttgca ctgtcttgcc tcagtctctt
58681 aatttcttcc tatgtacatt ttaagatatt gaagacacta acttccaaac ctcttctcta
58741 aatctgacag tgaagcagtg tgtgtgtgtg tgtgtgtgtg tgcatgccag tgcatacacc
58801 acacatacaa gcacatacac agagttaagt ttggtcacat ttacttcatt aattgatata
58861 taccacaact gagaaaaact tactggcatt ctgctccaag cttgaattcc cagacgaaaa
58921 acaaacaagc aaacaaacaa ctcctcctta cacaaaaata tctagtggcc ttggaaaatt
58981 ccatttaaca ttaaattgtg tctcttcaaa gtgaagcagg gtgggcagat ttttgaacat
59041 gaatctactg ataacacaac acctcttcaa aactagaatc tcattcaggg ttaaatgatg
59101 tccaagtcaa acctgagatg ctagcatgat gccccatcct cagtcagagg tttcagggtg
59161 gcaaacaact tttgctcact ctcctctgcc tctctctata tttgcagaaa gtccagcttt
59221 cactgcaaaa gttcgataac tctgtaaaac agtttgatcc taacttcaaa agaattttta
59281 tgctatttgg aatacataaa aaatgaattt attccaatac tttagcccca taaataatgc
59341 ataatgattg gtgaaagatt agcattttgg agttcaagta cttaaagctt tcttggatgg
59401 ataatgcatc acccttaact agcataaggg gaaatagaag gttgacaagg aaaacttaat
59461 ttagggttct ttatttattt ttctctagtt atgtttacct tcccaggttg ttttattaat
59521 gccacttttc acaaggatat ttcatgaata aatatcaggt tgtatctctg gccatctgtg
59581 atgatcacgt tttatttttt aatgagttgc tcatggatta tatcctaact ggaattgcag
59641 tcagtatatt gagatgcatt gactccattt ctgtctttca ttcttctttc tcaatgtatt
59701 ctcactggat tagcactctt agccctgagg actcttgggc cagttttatt actaaaagaa
59761 gaattacagt tttgctacac agtgaccttg caagaagtat cagcacagct caatttcaaa
59821 ttctcctcaa atatcaaata tgttcttggt atattttgct tagcagttta ttgccttctc
59881 atctccactg tttttcctga gtaacagctc agaaatctat aaataaaact taataatggg
59941 gagtgactca aaagtctggt tatttttatt agtaaactat tttaggggtg aacacttcac
60001 attttacatt ctcatgctcc acagtgctga aaatagtctg aatttttttt gtgtgttagg
60061 ttgatgagaa tatttttgag atgactagca gaaaacagaa tcattaaaac agtgctatca
60121 gttttcatgt tgaataactt tacaggaaat gatagcatct ttttttcttt ttttgagacg
60181 gagtttcgct cttgttgctc aggatgaagt gcaatggcgc gatctcggct caccgcaacc
60241 tccgcctcct gggttcaagc gattcttctg cttcagcctc ccgagtagct gggattacag
60301 gcatgcgcca ccatgcctgg ctaattttgt acttttagtg gagatggggt ttctccatct
60361 tggtcaggct ggtctctaat tcccgacctc aggtgatcca cccgcctcag tctcccaaag
60421 tgctgggatt acaggcgtga gccactgtgc tcagccgata gcatcttaac aataaacatg
60481 caaagatttg taaatgttgg caaacagcaa gaaaaacata tttagaaaat tgtgctatgc

60541 tttaatgtcc tgtctgtacg ataatttact tagctcccta ccttcccccc agcccaccag
60601 aatgtttaaa ctgaaattta aagcacagtt gatcttggct tggataggca gcctgcatat
60661 taactgagga tttagttagt atccaggagc agatctactt ctcaaagtga gattcacata
60721 gcccccttgg aagctttcaa atagattgac tagcctttta aaattcctct tctccccttt
60781 cttattgccc ccgccttgtc tagctgtatg ctaacttgct gcatagaatc ctgaatttct
60841 ggatttctac atttctagct caatgaatgg ggtaaatcaa aacttaataa cactcttgat
60901 tttgaaaagc ttcagtgctt catgtaagac tttttgagaa aagatcattt tcatttattt
60961 ctgttttatt tctagcttaa tgattaaatg taagctgttc aatacctttt tcaatatccc
61021 acttgttaaa cccacactaa acctcagaaa tattgagtca gtgatctaaa tttccattca
61081 aaatttccac aaagcagaag cataaatgtt ttcattgtca ttacaaataa acagacaggg
61141 ctgggcgcag tggttcatgc ctgtaatccc agcagtttgg gaagctgagg taggcggatc
61201 acttgaggtc aggagtttga gaccagcctg gccaacatgg tgaaacactg tctctactaa
61261 aaatacaaaa aaattagctg gattggtggt gtgtgcctgt gatcccagct actcaggagg
61321 ctgaggtagg agaatcactt gaacctggga ggcggaggtt gcaatgagct gagattgtgc
61381 cactgcactc cagcctgggt gacagagtga gacttcatct caaaaaacaa aaacaaaaac
61441 aaaaacaacc ccttcccctc cacgaaagaa aaccccaaat aaacagacaa atgaaagcaa
61501 tgaaaacagg aaaataattt tgacacctac ttctgggatg ttatgttatg ctttgcattg
61561 gttctattta ttagacatgt tcccatcttt attatttaag agcagataaa ataacctcac
61621 tacattaatc tcttttgctc tgcctctttc catatgattc ttcactagca gttcccaaaa
61681 caaaaaaatt gtgcaccaaa atcccaactg agactcaatt gtttgctgac tcaacaactc
61741 taatgagaaa tcttatacaa gagcttttgt ttcaaaagga agtgccagga aaattttgct
61801 tccctcaagc aatggcatgt tttctttttc tggacagtga gaaccatgta attaaacata
61861 ttttcctttg tgctttggct gtcaggaaac tcagagccaa acttgtagcc caagttttat
61921 ggctatgtaa gacattatgg cccatatatc tggactcttc ctttctcctt ggtattgatc
61981 ttcagtcttg gctttagttt acaaaatatg ccattataaa aaataacagt atctgtaggc
62041 tgtctgaaat tattatggaa aaagtagtgt acaaataaat gaataagtca attgtttgaa
62101 aaacaatgca tttgtatcag aaataacttt gtgagtggtc ttcaggttcc tagtctattt
62161 cattttgata tatatttctt gtgggtttac cacgtgccag acaaagatgt tcctggcagg
62221 ggggttaata atgttaacag tactcagtct ttctagtgcc ttacaggcta tagaaggaga
62281 aagaaataca aaacaaacta aaataggtat tcaattgcaa caaaagcaac ttgatgtagg
62341 ggtacagggg aaggaataat tcattctaca ggcctttaaa agataaagag gtatttcaaa
62401 ggcaaacaat ggagagaaga tcattctaag ctaaggaggt tgcatgaaga aagcataatg
62461 tcactacttt tttttttttt tttgagacag ggtctggctc tgttacccag gctgtagtgc
62521 agtgatgaga tcacaggtca ctgcaacctc caccttcagg gctcaaacca tccttccaca
62581 tcaacctccc aagtagctgg gactacagct gcatgccacc atacccagat aatttctttt
62641 attttttgtg gaaacagggt tttgccatgt tgctcaggct ggtcttgaac tcctgagctc
62701 aagtgatctg cccaccttgg cctcccaagg tgttgagatt acaggcatga gccaccgcac
62761 ccagcataat gtcatcaaat tgaagtgaga aatgggaatg tcactaccaa ccataagtaa
62821 taaacaatag attatcatta actttgctcc tagagaaaga caagtttatt tagaggtgag
62881 gcgagtagaa tttttttaag ggaacttctg ggtaatttaa caccatgatc cttctcgaat
62941 gccttacaac gtaaaaatgt agttctcctt tttcttttct tttctttttt tttttttttt
63001 ttgagacagg gtctcgcttc gttgcccagg ctggagtgca gtgacacaat cttggctcac
63061 tgcatcctcc gccgcctggg ttcaagtgat tctcctacgt cagcctcctg agtagctggg
63121 attacaggcg tgcactacca cgcctggcta atttttgaat ttttagtaga gatgaggttt
63181 tgccatgttg gccaggctgg tcttaaactc ctgacctcaa gtgatctgca cacctcgacc
63241 tcccaaagtg ctgggattac aggtgtaagc caccatgcct ggccattttt cttgataaag
63301 gggtgtcatt ggggcttctc ttactcctgt cactcatcat gggtgccatg tctttttttt
63361 tttttttgga gatggagtct tgctctgtcg cccaggctgg agtgcagtgg cgcgatcttg
63421 gctcactgca agctccacct cctgggttca cgccattctc cggccgcagc ctcctgagta
63481 gctgggacca caggtgcctg ccaccacgcc ctgctaattt tttgtacttt tagtagagac
63541 ggggtttcag cgtgttagcc aggatggtct caatctcctg acctcgtgat ctgcccgcct
63601 cagcctccca aagtgctggg attacaggcg ggagccactg cgcctggcct gggtgccatg
63661 tcttaattga caaccacaat gaagagagga aggaaagagg aaagaataag acaatacaag
63721 aagaaaaggg aaggaaaagc caatgacaga cattaactgg attttcccac aaagagaggt
63781 catgaggatc aagagcatac agaagtttgc aaaataaaga gtttctctgg cttggagttg
63841 accaaaataa gagaaaggtg aagcgagtat aatgtacgga gacttctcct gggagaagtt
63901 gtcagtggag aagttattgg gtcatatagt gatggtgtgc agataggacc tggtggtgag

63961 ttgacgagga gccattgcca ttcagccatc aagaatcatg atgtgtgttg tgtaggaatg
64021 gagaggtgag tgtggagtgg atggggcgta gatggagaag caggaggatt cgtcatgcac
64081 cctgaggctt gccagtattg atgtcagact tcctccgacc gtgttgccac cctggactgt
64141 ttgactttgg tagctatttt aaaacagcaa gaagagctgc ttgagaagaa cctgggggag
64201 atttcacaac cagcagctgc atcgaatctc cctggatgtg tgcttttgga aacaatttac
64261 acaagggctg atttcaacat gcactaatat atacagttta gctggcattt tatatggaaa
64321 ctttaataaa atggttctaa ttagtgctta atttacatag attagagagt tctacaggcc
64381 cacacagtag ggcttgggga ttttatgtca tttgatggag atgccagttt gattcagata
64441 aagaaccaat tttagggaat actgctgaag tttgcccgac acaaatgaag gagttgtagc
64501 ttgcatttct tttacccact cttgttcagt ggcttgaact agctttctgt ctcttccaca
64561 agatttagga caagatggat ttctgtgtca gcatgtagta ggagagcttt gattttctag
64621 ttctgtcctg tgtggatctg tatgtagctc agattccccc acaatctagg tcagagaagc
64681 tgctagcctt ggtgcaaagt tttcaaaaag gcttctattc agagagatga ataaggaaaa
64741 actcacaccc tacgtgcgaa ggttgcttaa tgagaggtgt gagatggagc tcagttgcca
64801 cttatctctg tagttgggta ccgttgtgca gtctgtgcac tgcccagcca tttgcagtga
64861 ccccaaagag gatcatctgg tcatcttgcg gggagagact gtgagttcct gccatgtcac
64921 ttggtataac atgagaaaac tcaaaactct aggcagatct ttgcacctgc cctacttttc
64981 tacctgctct cacaatcttc ttgagggtga ccagagtcca agtaactaag tgtaattcaa
65041 tgtggtgaat gatatgattg ctacacagga gtatctatta aggctttcaa aatgtaaaga
65101 ggaagtgact cattcttcct ggtggtggtg gttgcatcaa ttggtgtagt ctattgcctc
65161 tctcccatca acaagggaca tttggcaaag tctggagtgt gtcaactgtg ggtggtggt
65221 actaccggca tctagtggat agaggtcagg gatgctgctg aacattttac aatgcccagg
65281 acagccctcc cacaataaat tatctggccc aaaatatcag tagtgctgcc attgagaaat
65341 ctggttatgt aacagataac tttttttttt tttttgacat ggagtttcac tcttgttgcc
65401 caggctggag tgcaatggct tgatctcgac tcactgcaac ctctgcctcc tgggttcaag
65461 tgattctcct gcctcagcct cctgagtagc tgggattaca ggcatgcgcc accacgccca
65521 gctaattttg tatttttaat agagacgagg ttttaccatg ttggccgggc tggtcttgaa
65581 ctcctgacct cagatgatcc acccacctcg gtctcccaaa gtgctgggat tacaggcatg
65641 agccactgtg cccagccaac agataacttt ttaaattctc agctgtttaa cccaacaaat
65701 aattattttc ttcctcaagc tgtatgtcca gcatgggttg gcaacatagc cctgtttttc
65761 ctagtccctt caagaagtct attgatggaa gatgcatttt tatgtgggct tctaccacca
65821 ctatgagagg gggaaggaaa tttgttggat tgtaccctga attttaaaaa gcttctgccc
65881 agaattgaca tatattcttt ccaattgcat tttattggcc acaatccatc atatgaccat
65941 gcctgaattc agagggagta ggtggagaat acatttttaa tttttaattt tttttttttt
66001 tttgagacag agcctcactg tcttgcccag gctggagtgc agtggcgcca tctcggctca
66061 ctgcaacctt tgcttcccag gtttaagtga ttctcctgcc tcagcctcac aaatagccgg
66121 aattacaggc atgcaccccc atgcctggct aaggtttttc atatttttag tagaaatggg
66181 gtgtcaccat gttggccagg ctggtctcaa actcctggcc tcaagtgacc catcctcctc
66241 agccttccaa agtgctggga ttacaggtgt gagccattgt gcccagagag agtgcatttt
66301 tgtcttgtac ccagaaagag gagagagtga gaataatcat gaactaccct aatgactatc
66361 atgctgatgg taatggtggt tgtaggatgg tgtgagaatt taaagacggc ttcactcctg
66421 tcactttgct tacttactgc tgtataacaa actgccccaa acttcaggga tgaagctaac
66481 aaaaaaatca tttaatttgc tcatgaagtt gcaatttgga tagggctgtg tagaacagct
66541 cctctctata ccatggggca tcagttagga cagctcaaaa cctgggagta actcaaaggc
66601 tgggggctat aatcatctga atgcttgctc actcctgtgc tctcatgtca ggcagtggat
66661 gctggctgta gacagagacc tcagctgtgg ctgtcagctt gaccacctac tcccagcctc
66721 tacatgtggc tgtttggctc ccttacagta tggtggctgg gttccaaggc agaaagtaaa
66781 tgctgccagt ttcttaaagt ctgtctgcaa attggcacag cattctttct accatattct
66841 attcatcaag gagttgtaga ggccaatttt aaggggaaga aaaatagacc ctatgtttca
66901 atagcaggaa ggtcaaagaa tgtggaggcc ccagaaaact gcttcccagg taagtgacag
66961 gatagaaaaa caagattttt tttttttttt ttgagacagg gttttgctct tgttgcccag
67021 gctggagtgc aatggcgtga tctcggctca ccacgacctc tgcctcctgg gttcaagtga
67081 ttctcctgcc tcagcctccc gagtagctgg gattacaggc atgcgccacc cagttaattt
67141 tgtactttta gtagagacag ggtttctcca tgttggtcag gctggtcttg aactcccaac
67201 ctcaggtgat ccgcctgcct tggcttccca aagtgctggg attacaggtg tgagccacca
67261 tgccaggcca gaaaaacaag acttctttag gtggagtttg gggaatgagc tttctggaca
67321 agtaaacggc atgcatcaag gcacagaaac atcagagctg catttgaata aattctccta

67381 gctggtctta cctcctagct acttacttct actgttgaca tcaaccttat aaacttgcta
67441 gaggctcagt gtggggaaac aaacaaacaa agcctcatga aacgttttga tccaattaga
67501 aaatgtctat taaatacatt gtgtttcaca aatgtgaact attaatacaa tctgggtttg
67561 ttccgattta attgtctaaa attatacata attcatcttg ccaacaaatg aaattattga
67621 aaaattaaaa atatcaccac caattttttc tattaaatgt atatttttgt aatgatagct
67681 taagtttcat ctcagtttga gatttagtac actgtggcat tcactctggt tggggttcat
67741 ttggctctga gcttcaaggt tcaatttaga cttcaaagca tgtcggactt gacctgaggc
67801 acgtttttaa caatcaggtg cagtttgtaa ggttatgggt tgaggaatag cataaatggg
67861 ccatcctttt tctgattttg agaagagaat gactactaat acccaggtgt gttcttgtct
67921 ttcttgagtc aattctaaca gcattggctg tgggttataa aaccataaaa ttttacagcc
67981 aaaaacaact ttaaaggtcg tttagatgga acttcttcat tttgcagatg aggaaactga
68041 gaatcagaga caaagtgacc tcagggcatt acaacttact attactatat tgaaagagga
68101 agggaagctg gtgcaagttg agggacccac tgatgaactg cagggtatcc aatgttgccc
68161 ctgtgtttgg gctgagggag aagcagcttc ttagcttctg ctatgctgac ttatcttgtg
68221 atctctgtcc cagcccttgg ctgcctctcc tccttgtccc caggcctgca ccattccatc
68281 tgcctcctga ttattcactg acagctgcct tttccctcac cctttgttct cagtttttaa
68341 aaatccaatg gggagaagct tggattcaaa tgatagcatt ttcagctcat gaaaaattct
68401 ataacaagtg tatttgaccg tttgatctct ggcttctccc acacacttgc cctttatact
68461 ctgacagcca taagcttcat ttccttgaag cagattgcaa tttcaatgga aaggaaataa
68521 ttgtgtagct ttcaagctga gttcagttct aaaaaggact gagcaaagct caatcctggg
68581 ctattgtaga atcttagtca ctgaatcagg cagtgaaaga gctctctcca agcttcatgg
68641 ttatgatctt gaaaaacacc agagctaggt tcaaattcca gccataccat ttcctaactg
68701 tgtgattttg gcaaatcaca caaatactta aaattctttc gactcggttt cctcttctgt
68761 aaaatgggaa cactaattaa tgctcacctc aaaaggttgt tcattcattc aacacatatt
68821 cgttgggtgc ctcctgtgtg tcaaactgtt tcagtccagt gaggaagatg gacatgaatc
68881 caagagtcac caaaaaaatg taaagtggca cctgtgatga gtcacaaagg ggaggtatat
68941 gagaaagtgt gtgacagtat ctaacaggtg agggcattat ataatttatt gttgcaaaca
69001 ggacattttt gagcatgtaa gagggtgtgt taattgtcct ggaacaacag gcataaatca
69061 ggactttctg ggcaaactca ggccctaccc cactcttacc tacccttagt ataagaggga
69121 aggttgggaa aggctttcct ggggaaggga agaagaaact gagatccgcg gaataagtga
69181 ggattcctag gcaaaaagag gaagacagag tggtttaggc caagagagca aacagtggtg
69241 caagactcta aggcaggaca aagcatggag cctgcagaga acagacagaa ggtcagtgtg
69301 atgaagatgg gaggggtgcg tggctttaaa cacaggcccc ttgcttgttg gtttgccctg
69361 taggtgttaa gaaatgaggc tatatcaagg tgaattttaa gtagaggtgg cacctctact
69421 taagaccaga cttgcagctt taaacatcag tctggctgca aggcagaatg gattagcatt
69481 gagtagtagt tctggccttg gctttccaga ggaaacttct aaacaatatc aaagccttgg
69541 tgtcacccac agagatcctg atttaattag tttaggttgg ctcccagtag gttataaagt
69601 gtgtcaggat tgagattcac tgtagaagga agtgtgaata tggagaagaa taattacaag
69661 gctattgcag tagcccgggt aagaagtgat ggaataggtt catggcagtg ggtttctgaa
69721 gcagtggttc tcaaactgta gtggatttga gtatcacctg gaagtcttat ttatttatta
69781 gagatggtct cacactgttg cgtaggctag agtgcagtgg tataatcata gctcactgca
69841 accttgatca gccttcatct cctaggctca agcaatcctg ccacttcagc ttcctgagta
69901 gctgggacta caggcatgga ccaccatggc tatttgtttg tttgttttta atagagacag
69961 ggtctcactc tgttgcccag gctggtctca aattcctggg ctaaagcaag cctctctcct
70021 tggcctccca aagtgctggg attatagcca ctgcaccttg gcacggaaac cttgttaaaa
70081 tgcagatatc tgggtatcat tgatttagta ggtcgtggat ggggcctgag aatttgcatt
70141 tctgacaaat tacctggagg cgctagttgc tagtttagag atcacactta gcatctagtg
70201 ggtagtggtc agggatgctg gtgaatatca tacaatacac agaacagccc ttttgctgat
70261 cactgatcga gcaaaaaaac catctgatgt ggtagataac atcaacagga tttgatcagc
70321 agggtaaagg acaactccag gactcttaca gttttctgaa tttcacaact aaatgacgag
70381 tttcactatt cattgacatt caggagacag ccctacagac ctgagaagat ggtgaagtca
70441 aagctacaca tatatttgag ggttaccttt gaaacagcta agaggataca tcaagcagga
70501 acatatatgt ggaacctgga agggaagatc attacagata taaacttggg gatcattaaa
70561 gccaggaact tatcaatggc atcagtcagt tctcttttct tttactctta tcattggtgt
70621 caaagctctg gcctcttttt gttggttcca ctcaggtgcc gtgatcctcc agcctcagca
70681 ccttgctgtg agctctcctt gggaggctgc ctctccctcc tccttcagaa tttaccccct
70741 acttctcctt tgggtcccac ctctcaggct tggtcatgtg tggcattagc aagcaggtgt

70801 gcgtgtttcc ttctgaagca caaatcaaag ccatggttat gtatctgtgg gattatttgc
70861 taaatggcca cctctcctgc tagattctaa gttgcctgca gtgactggag aacatcccta
70921 ggtatcagat gctcaatgtc ttacacttat ggaaccgtca aagacagctg ttaaatgtcc
70981 aatgggtcag ttagggagat ggtggttgtt aaaactgtga tgagttctca ggaagaggga
71041 gaagcagtta cctttgtgaa gcagttgttg gtatttactt agtaatgaga ttaaatttcc
71101 taggacagtg ggtgtgtgaa ccagcaatag cgccatctgc tggaagtaag tgatacaaca
71161 actattatct tgactaggct cagggctgtt aaaacagtat tgctctggga gaaaagaatc
71221 atttttttta aggttaaaaa tgtgagtaaa attctttaga gtgaagatag aatttctttt
71281 gaaatggttg gaaaaattgg ataattattg aaaatagtgt tcagttttca aagaggagag
71341 aaaccttcaa gtcactgtaa ttaagagaag ttttattgaa agaacacatg gaataaggaa
71401 gttagatatc tccacccaaa accagcattt cagaaaacag aaacaggcaa aaatctggcg
71461 gagtgtggtg gctcatgcct gcaatcccag ccctttggga ggccatggtg gaaggatcgc
71521 ttgaagccag gagtttaaga atagcctggg caacatagta agatccccat tttctttttt
71581 cttttctttt cttttctttt tttcttttct tttctgagac agagtgttgc tcctttgcc
71641 caggctggag cgcaatggca tgatctcagc tcaccacaac ctcccgggtt caagaaattc
71701 tcctgccttg gcctcctgag tagctgggat tacaggcatg caccaccaca cctggctaat
71761 ttcgtatttt tagtagagac gtggtttctc catgttggtc aggctggtct tgaacgcccg
71821 atctcaggtg atctgcccac ctcggcctcc caaagtgctg ggattacagg catgagcccc
71881 cgtgcccagc cccaagaccc ccatttctat tttaaaaaaa aaaaagaaga agaagaagga
71941 aaagaaaaga aaaatggaaa caggcgaaaa cctaggcttc tcaaagactg aagggtattt
72001 tggaactgac tggatattgc tcatctctgg ctggttgtgt tgcagcctcg ttggtggcaa
72061 gtgcagaccc tcagtcagtg acctcctttg ttgctcttcc atcaaagggc cccttctcc
72121 tgcccaggca tggtgttact aatatggcga tggtaggggc aggggctgca tctatgacgg
72181 ggagaggtat gagagtatac tcatcattct gctgttgtct ttgttagttt gtttctaatt
72241 tctattgttg gggagaaaga taaattcttc cctaacctct tgggatcttc acctgggtct
72301 aagaattaaa tcactgtaag acaggttaac aggagaaaaa tatatgagtt ttattgattt
72361 ttacatgtac gcggagaccc tcatgagaca gcagaagacc tgaagaaatg gacaaagcag
72421 aaagctctaa taccttttag acagagaaac cataaattca tgaagagctg acaagacaaa
72481 ggggtttggg ccagtggcag taaattgtgg gaattcacta gggaggtgta aagatagagg
72541 aagataaggg atagtttaga aagtttattt gtacagctcc attgcagcat caactcccag
72601 tcaatggtga taagagttat tttcttattc tggtacaggg agggcaactt tcttaaataa
72661 atctttttgg cttacgacag gtagaagagg gcagatcaca tagccctttc cggcatctgc
72721 tgtttctcag gtgactttag ctcaaaataa tcaacatgcc acatcagcaa attttggggt
72781 gacatggcct gtttcgctat gataatggga aagaaatcag actttttaac tttgaattag
72841 aaaacacaaa ctttgactat gtcccacaat ttatgtgact gatttcatat tctatcacat
72901 ctattttata ttctctcata ttctcctcat gaattttccc ctagaatgtt ggggaattt
72961 tgaaatgact tggggggcat gctacaattc gaaaaggaga gagacatact ctccaattct
73021 ctccctgcct gcctcacaat gtgcagctct cctctgcttc ttcttcttct tttttttttt
73081 tttgagacag cgtctcactc tgtcacccag gcttgagtgc agtggcgcaa tcttggctca
73141 ctgcaagctc cgcctcccag gttcaagtga ttcttctgcc tcagccttcc gagtaggtgg
73201 tactagaggt gtgtgccacc acgcccagct aatttttgta tttttagtag agacggggtt
73261 tctttatgtt gaccaggcta gtctcgaact cctgacctca ggtgatctgc ccacctcagc
73321 ctcccaaaat gctggaatta caggtgtgag ccaacatgcc tggcctcctc tgcttctgat
73381 tcaactgctg ctaccgatag acttactgaa ggatttgaag ccaaatggta ttaattttgg
73441 tcagttactt ccctgtatct ggtgtcctca ctagacagaa cttctgtgta tggctacttc
73501 atagaccccg accagcctac atattgtttg ctctaaggtc tagtgccctc tcttatttca
73561 ctaagataca gccactgttg gtgggggagt cacataacat ggaacataat cactgtatca
73621 gttcaggtgc tcttgattca agtggattta attatgaggg aatttattcc ttacataatt
73681 actaagtcta gcattagagt ggcctgcaga tgagggttga tccagtggct cagtgatggc
73741 cagaacgctg tttacttttt ccattctgct atctatgggg ttggcttcat cctgaggttg
73801 ttttcctttc tgtcacaggt tttacatcaa tggggtaaac tagtttacat tgtggtaaca
73861 agcaactctc aacttttttt tttttttttt tttttagact gaatcttgct ctgttgccca
73921 ggctggagtg cagtggtaca gtcttggctc actgcaactt ccgcctcctg ggctcaagtg
73981 attctcctgc ttgagcttcc tgagtagctg ggattacagg catgcaccac cacgctggct
74041 aattttcgta tttttagtag agatggggtt tctttttttt ttttttttct ttttgagaca
74101 gaatcttgct ctgtcgccca ggctggagtg cagtggcgtg atctcggctc actgcaacct
74161 ctgcctcctg ggttcaagcg attcttccgc ctcagcctcc caagtagctg ggactacagg

74221 cgcatgccac cacgcccggc taatttttgt atttttagta aagacagggt ttcaccatat
74281 tgaccaggct ggtcttgaac tcctgacctc gtgatttgcc cgcctcagcc tcccaaagtg
74341 ctgggattac aggcgtgagc caccgcgccc agccaactct caactctagt agcttaagac
74401 aacaaagatt tatttcttgc tcttactgtc tgcccatctg tatcaccttc tctgcaggac
74461 ccagtttaat ggagcacaca cagaaaaagg tgctggtcac tagggccaag ggaacaatgg
74521 ctctggaagg tttttcttga acaattcagt gttccagtct tggagcaata catcttattt
74581 ctactgttaa atccatggcc agaactaatc atgttgtccc actcaccaca aagccatatg
74641 cagccccacc atgtgctcag gaaagaggaa tgggaagcat ttgatgaaca gaaataatga
74701 cttcaacaat tgggactatc agttcacagt ttcaattgta aaaaaaaaaa aaaaaaaaaa
74761 agacagaaat agaaaaagaa caattgaaag tacttgcttt ctcatccacg aaaaaagttg
74821 gtcaaagcct cttagtgtaa gtctatagca gatgatgcct gataaggaat tgtacatacc
74881 gttcttcgtg aaaaccaaga gtaccatgta tgtaacatag ttgctttggg cttcttatag
74941 cagttaagag tgtatcagca ggcctccatg gtatttgaat gttaaaagct ttcattcccc
75001 ctggaagaaa acacatagct aactaaagaa agaaaaaaga agggaaaaag aacaaagaaa
75061 aagaaccact agagtctctg aaaagagctt atgagatacc tgactgtctg cgccttctaa
75121 gtatgtacca tgaaacctta gattcctacc tcattttgtg atggtgtaga aggcacattg
75181 gccaccagca acccttgtaa atatttgcgg atttgaattt cacaaattga aacccagact
75241 gctgagggct ttgctgctaa cgtcaaagag ctggggcaac taccacaaaa ccacatgtgg
75301 gagacttttt agatgaggag ataggaaatt cctaataaga aaagtttttt tgtaaaatac
75361 tttctaatgc tggctcttta gagaagcaca ttttacatgt gcaatggtgc aaagtcctag
75421 cactaagatt tcttcactca cagaaagaaa gaatgatgca ggtgctgttc acatgcggcc
75481 ggaaccatgc ccttgccaag caggtcagcg gggcacaggg actgcgctcc tcatgatagt
75541 gcagattggc tcggaagtac tcaattctgt ggagtcacag cgccctctga cagatttcag
75601 tttctggaaa aggaagggcc cccttcaggg tacctgctgt tcaccttcca ttagattaag
75661 gttgaagagc gagtggccat ttgtaggtac tggtatgaga tgttcctagc tacccgcatg
75721 tcagccccgg gaaacggcaa catttcccac ccttcttacc ctgggtcctg agtaggaata
75781 aaagttagtg attcttaggg gcatattgag agctggcaat gatacctctc acaaatccat
75841 cccccagaga taaggggtcc cccatgcctt tgatgtattt gggagctgtg ctgtatgtgt
75901 tctctttggc tccccagtgg agaataataa caataacaac cgcgtactaa tacaacccag
75961 atgtcagcac agaccctctg ttctctgtct tccctgagat atctgtatct tctcagggat
76021 tactgtcggt tcaggcctgg ctcctgcttt cagcttgtgg gaaaactcac tccatcttca
76081 ttccataacc tcacatctcc tatgccagtt cccttctctg tgctccatgc tgaaccaaat
76141 gccacactca tcccctttgc tttgaattat atgaaaaaaa gaaaaaaccc tcatggggac
76201 tttatgtgta acagtcttca ctgcacattt gataacacag atgggggttt atatcctagc
76261 agagcagata ttgtcaatgg aaaatggaaa caaaacaaaa cacaaaagac aatcttcagc
76321 catcactatg tagcttggct atcatctttt gctttctcct ttacttctgg agtcccccca
76381 cataccctca gaaatggaga tagactctac atcaatctct gctctgggaa atctactcac
76441 atatccaaag gcaaactaac aagctcttgc tccgacaacc catcgacttg ggtctcatc
76501 cctccctgcc taccagtctc tgtgactgaa aggggcccat acagagactt attttcccag
76561 gaacgtgaat gatatccttc tcctgttcct tgaccttggg aggcagattt gattatggtc
76621 agctttgatt caaagtaagt tttttttttt ttctccctta ggagaatgtt gtgaggtgct
76681 gattgttctc tgcttcataa tcgtatcact tattactgat aatctttttc tgcccccaga
76741 ggttgctgaa gaatgtagca tatacagtgg acacagagcc ctgataatac aattactaaa
76801 ttctccaagg ttgagggcct ggtcttaggt catgagatta ctgatccttg ccttctgcct
76861 gctaggaagg cccaactaat ttcacagtct ttatcagtga accatagtaa agggggacca
76921 gaagagaaaa aggttatgtc ttttgggaaa atgccaaaat catattgtga aaatccttcc
76981 tactgttctt ctcatgggag ggggaggact ttggacttgt ttctctcctt ttcctgtaca
77041 tttggggatg tcagaaccgt ctgtttcctc atctacacgt ggttagatgc cgacaacaat
77101 ctcttctctt aaaattgctt actattagac cagcagagtg tttggtatta attgagcaag
77161 gaggaatttg taggaaaggc ttggcttaac taggagaggg aatgccaggg agaaatctgt
77221 atttcccact attttttttt ctttttttga ggcacagttt cactctgtcg cccaggctgg
77281 agtacagtgg tgcaatctca gctcactgca acctccgcct cctgggttca agcgattctc
77341 atgcctcagt ctcccgagta gctgggatta caggcgtgtg ccagcacacc cagctaattt
77401 ttgtattatt agtatagatg gggttttatc atgttggcca ggccggtctc aaactcctgg
77461 cctcaggtga tctgcccacc ttggcttccc aaagtgctgg gattacaggc atgaaccacc
77521 acacctggcc atttcccact attttgttga taaagacatt tggatttccc tgcattaggc
77581 agatgtggaa gaagatggaa gcgatgaaaa ccttcttgcc aaaatctccc aaaagacctg

77641 gccagtgggt gagcagatct cagttacaaa aagcctatag gttgaatttt ctaagaaaag
77701 gcctggggag gaggtaccag gagtccaaat tgaagttcga ttttcattat cacagatttt
77761 catatctctc tatctccctt tttttgtttt tggtgagaca gagttcaaga ccagcctggt
77821 caacatggtg aaaccccgtc tttactaaaa atacaaaaaa ttagccagcc gtagaggcgg
77881 gtgcctgtca tcccagctac tcgggaggct gaggcaggaa agtcacttga acccaggaga
77941 tggaggttgc agtgagccga gatcatgcca ttgcactcca gcctgggcga caagagccaa
78001 actccatttc aaaaaaaaaa agaaagaaaa agaaaaaaga aaaaaagaaa atgcactttt
78061 tgctgtcagt aaaagttaag caaaagtctt gctgagagac tgatatttaa ataactatta
78121 taatgcaatg taaaattact gcaagagata tgaacacagt gctcaggaag cttagaggaa
78181 ggagaaacca agtccttagt gatggaaggt gggctgtcaa ggaaggcttt gtggaggttg
78241 tgtaattgta ggatgagggc catgtagtag gtcgacgggg ccctcgtgtg ccaggtggaa
78301 ggaagccatt taagtctttt aagctgagtg gtggcatgat tacaagtgca ctttagaaaa
78361 atctctatgg cagcaatgtg gaggatgaag taacaaaaat atacactttg gttcattttg
78421 ttctaaatgg caactttacc tttaactgtg tacagagctt actgactata ctcaagcaac
78481 aaaatgcaca cttggaaatc ctgtgtcacg tgcctatact agctttgtcg ctgattcagc
78541 atatacacat cctccagtgt gacaggattg tgtatggaaa caggtaagac tggtgaatac
78601 taggctgtgt tcaagtgtag agtctcatga ttatcaccaa tactgtgggc tctccctctt
78661 ttagatgagt ttttctacca tatctttggc tttacaaaat atattaacac agtttcataa
78721 tagatctctt ggaatatggt cgtttggtgt taagcattct atctggcaaa atgaaaaata
78781 agatggtaag agttatccta caaaaatcct aatgttctca agctactgct gaaattcttt
78841 ttctagtaat ttacaaaaaa gtccccattt tcttgtagaa ataagccaat taaaaaaaga
78901 aaaaaattaa aataaaaaat taaagttctt aatgataaat tatattaaaa tttatttcct
78961 gttttaccct atatcctatg agcgtggttc tcaaacttcg ttcagtgcat accagaatca
79021 tcatgtgcgc ttgttaaaca gaggtctggg ttccaccccc agagcttctg attcacttca
79081 tggaatctga gaactggcat ttctgacaag ttcccagggg atgctgaggc tgctggtcca
79141 gggaccacac actgagaaat attgttctga aataataaca gctaattttt gttaggcagt
79201 ttctgtgttc caaatacagg ttgaatatcc cttatccaaa atgcttgggg caagctgggt
79261 gcagaggctt attcctgtaa tgtcagcact ctgggaggct gaggcaggag gattgcttga
79321 gaccaggaga ttaggaccaa cctaggcaac atagcaagac actgtttcta cacacacaca
79381 cacacacaca cacagacaca cacacacaca cacacacaca aattgccagg catggtggca
79441 tatccctata gtcccagcta cttgggaggc tgagggagga ggatcacttg aaacaaggag
79501 tttgagacta tcctgggcca catagtgaga ccctgtctct acaaaaaata tttgaaaaat
79561 tggccaggtg cggtggctca tgcctatagt cccagcactt tgggaggctg aggcaggtgg
79621 atcatttgaa gtcaggagtt caagaccagc ctggccaaca tggtgaaacc ccgtctctac
79681 taaaaacaca aaaattagct gggcagtagt ggtgggcgct tgtaatccca gctactcagg
79741 aggctgaggc cagagaatcg cttgaaccca agaggtggag gttgtagtga gctgagatcg
79801 tgctcctgca ctccagtctg ggtgacagag tgagaccctg tctcgaacaa aacaaaacaa
79861 aacaaaacca aaaaaaccaa aacaaaaaac atattagctt ggcatggtgg tgcattcctg
79921 tagtttcata tacttgggag gctgaggcag aagaattgct cacgcctggg ccttcaaggt
79981 tagaatgagc tgtgatctca ctactgcact ccaatgtggg tcacagagca ggactcaaat
80041 gaaaacaaaa ccaaaaccaa aattcttggg accagaagta tttcagattt cagattattt
80101 tggattttga aatattggca tgtatctaat gagacatctc agggatggga cccaagccta
80161 aacacaaaat tcatttattt ttcatataca ccttgtatac atagcctgaa ggtaatttta
80221 tataatattt taaataattt tgtgcacctg tcacgtgagg tcaggtgtgg aatttctac
80281 tgtggtgtca agtcaatgct caaaaaattg cagattttgg agaatttcag atttcagatg
80341 tttggattaa atgtgcttag cctggacctt acttgttgta gctcgtttaa tcctcatggc
80401 aatgctagga cacaggtggt tttagtataa tattattaca aatgaagagc ctgagacttg
80461 agaaagtaag acaacacact ccaggtcact cagccaatta gtggtgagca agaattgaac
80521 ccagatttga tatgcgaatg acttgctgtg attctcctat tggtgatttt tttttttttt
80581 tttttttttt ttgagacagg gtcttgctct gtcacccagg ctggagtgca gtggcatgat
80641 ctcagctcac tgcagcctct gcctcccagg ttcaagcgat tctcctgcct cagcctcctg
80701 agcaactggg actacaggca tgtgctacca tgcttggcta atttttgtat ttttagtaga
80761 gatggggttt caccatgttg gccatgtgga tctcgagctc ctgacctcaa gtgatccatc
80821 cgcctcggcc tcccaaaatg ttgggattat aggcgtgagc cactgcaccc ggccccttat
80881 tggtgtattt ttatttccca agtatgacac ctgttcagag actttggaac cctgagaaca
80941 ttttgttggt gtgttttgtg actaatattg cctttttctg agatgaatgc tgttctttga
81001 aaaaacattt gtatttttag atagaaatgt acttcatccc cagtgaatat ttctgacttt

81061 agagaaattg ccaaacctgc tgactcataa caaaaataaa gagcatctac tattcagttt
81121 atggctttta gttttgggag gtggtaggag gaaggataga ttaccaccca ctatagatca
81181 ttttattttc attttatttt aatgccattt accagtattg tgtatatgga ggtggtcttg
81241 agagctttgt ggagattatt ttttcaatag tacagatggc tctgataaat atttaaggca
81301 tcttgggaaa caagaatacc atcctgactc aggagaaatt ggtcacgtaa atcactttgt
81361 ttcaagtgct ttggcctctg agcatctcaa atggacacat acttcattgg cttattaact
81421 cattgccgga gtcactgaag gcccaaacta caacttcatg cctgaccaca caccaaggct
81481 tttttattgc ttagttttct ggctctttgc ctattctgct ctccctaccc ataaccatgt
81541 gagtatcatg tccagcaaac taacttactc cttctctctc cccactacac acacacacac
81601 acacacacac acacacacac accacactcc acacgcacac atacacagag catttttcat
81661 gggtatgttg tcagacagat ttctctgttc tgaaggctca tctccttaga gaaccttacc
81721 gtgtcaatgt tgcatttagt ttagtttagt gttgttgttg ttgttgttgt tgttgctgtt
81781 tttaaatctt gctctgttgc ccacagtggc gcgatttcag ctcactgcaa cctctgcctt
81841 ttgggttcaa gcaattctcc tgccgcagcc tgctgagtag ctgggattac aggcccccgc
81901 caccatgtcc agctagtttt tgtattttta gtagagacgg ggtttcacca tgttggctgg
81961 tcaagtcttg acttcctgac ctcgtgatcc acctggcttg gcttcccaaa gtgttgggat
82021 tacaggtgtg agccactgcg accggctgtt tttttttttt tttttttttt tttttcttt
82081 tttgagacgg aatttcgctc ttgttgccca ggctggagtg caatggtaca atcttggctc
82141 attgcaactt ctgcctcctg ggttcaagca attctcctgc ttcagcctcc caggtagctg
82201 agattgcagg tgcccaccac catgcctggc taatttttgt attttagtag agatgggatt
82261 tcaccatgtt ggtgttgtat ttagttttaa cactgttatc ttctcagatt gtaatgagag
82321 aacaggctat catcacttgg tgataaaatt tcctgtaatt ataatctcat tctcaaagat
82381 aaaacattga tacctaatgt gcttttaact ttaatgccaa tttatcacaa tggaagacat
82441 ttcaaataaa tactgttttt ttgttcataa ggtcttaaaa atcttgaata taactgcaaa
82501 aaagcattac atgttaaaag aaatcacaaa attagttaag aaaggagcca aactgaaaga
82561 agattatatt tctatgccct tgaatcttta caaaatttag taaaaactat taatatagaa
82621 ttatttacaa gagacttctc tatttaccag cccaatatct tcacatttct acaatctaag
82681 gggaggtgat tgtggccaga aaagtttctg ggctctggta ttgcagtttt cctgcactta
82741 ccaccatccg gcatgttata tattttactt atttttcttg cttattgctg tctctttcca
82801 caagttccat gagcagagac agtttatctg tgttttttac ggctattctc aatgtctgta
82861 acagtgtcta acgtactata gtcattaaag aaatgctggt tgggtgaatt cagaaataaa
82921 ttagtaaata ggtgaagcat gttggtttaa tattcatcat cagaacttaa aaacagttta
82981 aagaacctca actgagtttt gtacaataag tagtgacaag gtgggacaga ggtaatttct
83041 ttgtatctct gaggctttcc atttcatgag gaacatagaa taatatgtgt ttccaaatgc
83101 agtgttcaat atctgaagag tatatatcat cagtatagaa aatagacaac acatgttaaa
83161 gggaatgtaa aaattctaga tgatttttaa ggtatgtatg gtttaatcaa ttgtttatat
83221 gctattctct tctcttaaaa tgaaataatg cgaatgaaaa aataaacctt acaggtagtt
83281 tctgggaaat tcagacaaca aaattgtcta acatacgcca aaaacaatat agcatttaat
83341 gatgacatgg attccgaaag acaaatcact gctcatcata ttacaaggaa tccaaacaac
83401 tgaggggtta tcatttatct atagttgcat aacaaattac cctaaaattt agcagcttaa
83461 aacaacaatt tattatttca taaatttctg gggatgagga atccagaagc agattcactg
83521 ggtggttctg gctcaaagta tcttatttgt ttgctagggc tgctgtcaca gaacacaaca
83581 gcacaggtgc cttaaataac agaaacatgc attctggagg ccagaagtcc aagaccaagg
83641 tttcctctga ggtctccgtc cttggattgc agatgcttgt cctcttgctc cctcttcaca
83701 tggttgtccc tctgtgcaca tgcacttctg tgtctctttt tgagtgaaaa tttataagca
83761 ttccagtcag attaattttt tttttttttt tttgagatgg agtctcactc tgacacccag
83821 gctggagtgc agtggtgcaa tcttggctca ctgcagcctc cacctccctg gctcaagttc
83881 ttctcctgcc tcagcctccc aagtagctgg gattacaggc atacaccacc acgcctggct
83941 aatttttgta tttttagtag agacgtggtt ttgtcatgtt gcccaggctg gtctcgaact
84001 cctggcctca agttatccac ctgccttgtc ctcccaaaat gttgggatta caggtgtaag
84061 ccattgcacc ctgccccagt cagataaaat tttaatctca ctttaactta atcacttctt
84121 tgaaggccct gtctccaaat atagtcacat gaattagggc caagctgtat ggcttcattt
84181 aaccttaatt acttctttag aggctctatc tgcaaatgcc tccacattct gaggtggtgg
84241 gggggttagg atgtcaacat atgaatttga gggggcacaa tttagcccat agacacctgt
84301 catgaggttg tagtcagctg tcagttgggg ctgcaatctc atctgatgga ttaactgggg
84361 aaagatgggc ttcccagctc actggtgtag ttgttggcag gcctcagttt cttctcgctg
84421 gagacttcag ttcttgccat cggcttctcc acaggttgtc cagattacct aatgacatag

84481 cagctggctt ccccaaaagc atggaatggg ggttggggat gggataagag agagagaatc
84541 caagacagaa tgcacagtct tcttataact taggtttaga aataatatat cagtacctct
84601 gccacattct gttggtcaca cacactaacc ctggtaaaac atgggagtga accatacagg
84661 gggaactggg gtcattggtg gtcatctttg aggctggcta tggtaattag gtaaaaataa
84721 aaattcagtc tgcagaaacg tatgtactta tgttacttaa aatattttaa ccaattagtg
84781 aaaaatgact ttgaagtatt tttagctgct ataataattg agtaggaatt attttttctt
84841 ggattgagtt atatttacta taaaaatact tacttttttt gtcaaacaca tttataagtg
84901 ctgcaaatat tagtgaccaa tggtctattg ctctaaataa aaagcaccag gagttagtat
84961 agtagttgac tccagcctct ggtgctagac tgcctgggtt tgaatattta cttttgcact
85021 tacaacttgt aacaacttga acaatttatt caatctctct gttccctagt tttctcatat
85081 gtcaaatggg agaaacaaat aacatctgct tcatatggct gttatgaagg ctaagtaggt
85141 ttatatatat gtatgtatgt atgtatgtat gtgtgtgtgt gcatatatat atatatatat
85201 atagagagag agagagagag agagagaaag agaacttaga atagggcctg gcacagagaa
85261 tatgataaaa gaatattatt tgtttcctct ttttatcatc cagaacatgg tgtaatgccc
85321 aatacattgt agatactcaa taaatgtttt tgtatgatcc ctaaacattg tctgtaagga
85381 atttacaatg gagtttacag taaaaggtgg tacatggtag gtaccatgga agcattacaa
85441 acagatgacc atgtgaatct gaaagaagca cagattgatt tcttcgtcat aaaacatggt
85501 tgtatttgag ctaaagctta gaactaatga agattattgc caggcgtggt ggctcacgcc
85561 tgtaatccca gcactttggg aggccgaggt gggaggatca caaggtcaga agatcgagac
85621 catcctggct aacatggtga aaccccgtct ctactaaaaa tacaaaaaat tagctgggtg
85681 tggtggcggg cgcctgtagt cccagctact caggaggctg aggcagaatg gtgtgaaccc
85741 aggaggtgga gcttgcagtg agccgagatc gcgccactgc acttcagcct gggcgagaat
85801 gcaagagtct gcctcaaaaa aaaaaaaaaa aaaagagtat caatgtatca aaatcattat
85861 tttaggctgg gcatggtggc tcacatgtgt aatcccagca ctttgggagg ctgaggtgag
85921 agatcacttg agcccaggag ttcaaggcca gcctgggcaa aatggtgaga ccctgtctct
85981 acaaagaaaa aaaaaagatt attattcccc aaataggaaa taacataagc aaacgtcccc
86041 tatttccccc acgatggagc tggagcatgg tgtatgagaa ggagagtcag agggagcaag
86101 aatgaagggg tcaggttggt accaggtaat gaaatcattt taacataaga ctacatgatt
86161 tggacttcat tgtgtggtca cttcggagca ttttagcaac aaaataatgt cttaggaaga
86221 ttaatctggt ccaattgtaa agtaaattga gattcaggct tcaggcttgg cttgatccag
86281 gattcaaata aaatcaccaa gaatctgtat ttcagatctg ctttttttgt gtggctctgc
86341 ctgtggctcc ataagaagca cagaaaaaag agaaatggtc tgttaaacag aaaaagtgaa
86401 aaagcttcac tcccagaaaa tatcttcttg catctcatcg gcctgagttc actcatgttc
86461 gtattcttca acaaattatc atgtccagga tatgaaacgg ctgcttggat ccaggcacac
86521 tgataacgct gataccacac ctagaaaaag aaatgaattt cattcaaatt ccattctact
86581 attttaaggt ttcatgtcta gatggctgga gattaagtgt tgcagtccag gcgtggtggc
86641 ccacgcctgt aatcccagca ctttgggagg ccgaggtgga cagctcactt gaggacagga
86701 gtttgagacc agcctggcca acatgatgaa accccatctc tactaaaaat acaaaaaata
86761 gccaggcttg gtggtgcgtg cctgtaattc cagctactcg ggaggctgag gcaggagaat
86821 cacttgaatc caggaagtgg aggttgcggt gagccaagat ggcactactg cagtccagtc
86881 tgggtgacag agacttcatc tcaaaaaaaa aaaaaaaaag taagttttgc aattaggaga
86941 catgcctata atgtttattt agtggtgata cgtatggttt agtcctttaa ttatagtgtg
87001 gtttaaatgc aaatttaaag taactcaaat acagctattt tttctcacat catagggaga
87061 ctataaagtc cctcagtaat cattcttcaa atagatattt ttcttctttc atctactgcc
87121 ttcacgactg cagtcacttt ctccaacagt cttttctcca aataaaaaca aaaacaccac
87181 taccaccact accaaaaatc tcaagtacct gctatctctg atctttctca tctgctaata
87241 ttctcttcct cccttactcc cctcctcttt tcttcctttc ctccttcccc atctctccat
87301 ctcacacctc cctctcttcc tacctccctt tctttcaacc ttttcccctt ctattcttcc
87361 ttccctcgct tcctccttcc cttccttcct tccttccttc cttccttcct tccttccttc
87421 cttccttcct tttgctattt aaaaattcct caaactggct gggtgtggtg gctcatgcct
87481 gtaatcctag cactttggga tgccgaggtg ggtggatcac ttgaggtcag gagtttgata
87541 ccagcctggc caacatggtg aaaacccgtc tctactaaaa atacaaaaat tagtagtgta
87601 tggtggcacc gcacctgtag tcccagctac tcagaagggt gaggtgggcg aatcacttga
87661 acccaggagg cagaggttgt agtgagccga gatcgttcca ctgcattccg gcctgggtga
87721 cagagtgagt gagactgtct caaaataaca acaacaacaa aaacaaactg gcaatgcagg
87781 acttccttga aagacagagg aaaggaaaag attaagagaa gttataccaa aactcttttt
87841 aataggtagg cagggaaaaa attgggaacc tttatacatc aataatttta ttctcataat

87901 acttggaaat catgactagc tgttgctctt cttcaacatc taactattat catttccaca
87961 tctgattcaa gcacttcctt tttacatgtc tttttttttt ctttgcctga aatattattt
88021 ccttccatca aattaacctt tctgcccacc tggcaaccct ctcttcttca agccttactc
88081 atcaagaagc cttccctagc aacagggaag gctctgctac caatcccacc tttatgctct
88141 ctggactacc actttgtctt gtacatttct attaaattgt aacttttcat tttgttaca
88201 catctattga ctctactgct cagtgaacta tttgagacca ggacaccatt ttgcatacct
88261 agcacaatac ctaccacata gtagacattt cataaatcct tattgattga ataaatcaat
88321 gatgaataac atttttataa atttcatttt gtatttgagt tgaagctcca agtattacac
88381 cctgttagag attttaaact tgatttttaa aatattggac tagagcttaa aagaagatta
88441 tattagtcag gggtctcaag agaaacagaa tcaataaagt atacatttat tatataattc
88501 attataagaa attggctcac atgattgtgg aggctgacaa gtctcaatac ctacagtcgg
88561 caagctagag acccaggaga acctatggtg tagtccaggc tcattccaaa ggcctaagaa
88621 ccaagcgagc caatgatgta gttctagtgt gaaaacctgc aggctctaga cacaggaaga
88681 agccaatgtt tcagttaaag tccaagggca ggaaacacca aggtcccagc tcaaacatag
88741 tgagacagaa ggaattctct cttactcagc cttttgttct attcaggctt tcaacagatt
88801 ggatgaggcc cacccacaat ctgctttacc cagtctactg atttagatgt taatctcatc
88861 cagaaacacc ctcacagaca cacctagaat aatgtttgac caaatgtctg ggcaccctgt
88921 tacccagtca agtggatgca taaaattaaa caccataaag actaaagcct gtagatgaat
88981 atctggtaac acctcacaga ggagagacct aaagctgtga taacgtctct gaagccaaga
89041 gagaaaggct agaaaaagca agatctggga tatttcctta gaatccttgc ctgaaggcca
89101 agaagatgag gaagttaatt ttctcatcca gagtggtggt atcgagctag aaaattgtga
89161 agatctttct agttctaaaa catctgttac ctccttacct tggggtttca agactgggta
89221 aatggaaata ctaagataat tggatgatgt aagaagattt tggatgggaa caatatcttg
89281 ttatgcacca gttgtatgga aactacttat ttataagatt taaaattcta cgtatattcc
89341 ttcaggaaat gttgtcggct ttctacaaga tgcagcttta attgtattat tgggaaccat
89401 cattgattat taggttcttt ggagaaattt ctttccttca attagaagca cccatgaagg
89461 atatgaagaa gagttggtca aagaactata agaagatcat agaatgtact gcactataga
89521 agccaaagga agaggtaaat caagaggcaa gaaatgatgg aaaggtattt tgctatagag
89581 aggtaaaggt tgataagact tgggaaaaag gccatcggat tttacgatta ggatattaag
89641 gacaagtttc agccaagtca tgagaaactg agcatcagtt aggtccctgt tgtttgatat
89701 tatgacaatg tggtggattt agtaattaga gtctttcaaa attgtctgtt agtgctttgc
89761 agcttggggg catgagcaga gaaggtaggg ggcaggagga atggggagca tgtctgtggc
89821 tgagagttga aggcttgaat ggtaagagta ggcttgagga ttgtggcttg ttagaaaaga
89881 cagcattaaa atatctgaac atgggacttt gtttttagaa aggatacaaa tgaagttaaa
89941 agccagaaga aggtcttggg tgggattttc aagctgcatt ccttggagtc ttgaatcgtg
90001 gattagagaa aagaccaccc tgtgttgatc tcagtagctt ctcctgttaa acatcacagc
90061 acatgtgctt tgaccagttt tgtatattaa ggctctatgt ggctatcttc tgtcccgctc
90121 tgtcccccac ggccattctc tttccttcac catgctgctc tatgacccgg gaggctgacc
90181 tatatggacc atatcagtgg gctgctttgc cctcctgctt ctgtttggat tttgcccttg
90241 gggagcactt gcatgagctt gcagggaggg agaatgagag gcaaggtata atctgctgtg
90301 acctacaggg tcatcaggat cctttcccct tcaggcctag agttggtaat aatgcccagc
90361 tttcttaggc ccttgaaatt tctctatacc ctgctatatc tgttcaattg cctacataac
90421 ttaaccagtg tatgcttact actcactgca gccttgatct ccctggctta ggtgatcctt
90481 ccacctcagc ctcttgagta gctgggacta caggtgtgca ccaccatgcc tggttaattt
90541 tttgtatttt ttttgtagag acagggtctc accattttgc ccaggctggt cttgaactcc
90601 tgggctcaag caatcctccc accttggccc cccaaaatgc tgagattaca gacaagagcc
90661 accactcccg gcccaaacat tttttttttt tgtaaaaagc caggcagtaa atatttttga
90721 tctgtgagtt atacaatctg tgttaccacc acttaactct gctatctcag tgagaaaaca
90781 gccatagatg ttaagtaagt gaatgaatgt agctgtgttt caataaaact ttatttacaa
90841 aaacaggcag tgagctttgc ttttggccca taggctataa ttgaaagacc catgatctag
90901 gacaagaagt tttccatcta ctctatgaga gtggttctca atctgggata atttagctcc
90961 ctaagggaca tttgttaatt taagacattt ttggttgtca caactggagg agtagtgcta
91021 ctggcatcta gtggataaaa tccagggata ctgctaaaca tactgcaatg cacaagacag
91081 cccccacaac aaaaattacc ctgcccaaaa tatcaatagt gctgaggttg agaaatgctg
91141 gtcttgaaga tgttaaaaag attgtcagtt tgttcatcat atcgctgtgg cttggcgaaa
91201 agaatcacag agttaatttt ataactatga aggattgtta ggagctgagt tttttctag
91261 ttcttatttt ttaattattt tatgatactt ttcataaaaa gtggtgtaag ttgtgtttat

91321 tcagaaatgt gatttttttt tttttttttt gatggagttt cgctcttgtt tccgaggctg
91381 gagtgcaatg gcgggatctt ggctcactgc aacctccgcc tcccgggttc aagcgattct
91441 cctgcctcag cctcctgagt agctgggact actggccacc atgcctggct aatttctgta
91501 tttttagtag agatggggtt tcaccatgtt ggccaggctg gtcttcaact cccgaactca
91561 gatgatccac ctgcctcggc cttccaaagt gctgggatta caggtgtgag tcactgctcc
91621 cagcccagaa attgatggtt tctttagcaa ttttgtcacc catcaagtcg ttagtcatat
91681 taatcctcca taacttagtc tttttgcccc ctgattatct tttgggtggg cagtgaaagt
91741 attcatacct cttttagcaa aacctatctc ttcttggatt tcctttgctt gctgattaag
91801 aggaaggact atctgtgaaa gactcagatg tggctcaatg gaatgaatgt gagaatggta
91861 tgagtgtggg tggtgggtgc tgtgggggtc ggagatgagt ggctgaacca tccaaaaata
91921 agaatactga caagcgtgtt ttcttgtgtg tgtgtgcgtg ttgtgggagg agtgtaactg
91981 atgtggctag acaggattaa cgaaagtctc aggagttgag agagactggg gacattttac
92041 caatggcgag aaggaggaag gatgacagct gggaaaatta gtgattggaa aaggtaggct
92101 gggcagtgtg gccatgagct agtccttttc attatattct tctggggctc tatgtttaaa
92161 actagatact ttaacatttc agaatgagaa agagaggaaa ggatggtaac tgatatgggc
92221 tattatactg tgaattttaa aggagcctat atattatcct aaaactattg aacctgagca
92281 gaactgcaac gttggtattc ttgaactgaa ctgatatttc ctttgactaa tgtccttgtt
92341 caatgaccag agaccatgac actccccacc ttcaccccca cacattgttt gtttgtgtcc
92401 agtttgaaga acaatgacac atggaggtca gtgtttggat gatgtcataa ttttgatcag
92461 ctggaaagcc tggagtggcc ttgaagagat gcttaacttt tggaaatcac ttctttattg
92521 caaccaaagc tattaagttt aaaggtccat aaagatgaga taaacagtca taaaggcctt
92581 ttgaaattca tacttagtgt gttaaatgga tttaattgct gttagttaaa tgtggtgatg
92641 ttaaagcaat gtctatacac tggcaaaaat cagatcatca actgcttaat gagggacacg
92701 tcttgagaaa aacagtggaa gaaatgctgc ttatatgtaa tttgtgatta attttgcaa
92761 ggttttcaag ggaataactt ggccttaaaa tattacagtg aaaaacagct ttatacttgg
92821 gtccaatctt cttaaaatta gagttcattt ttgctattag acacctttta ttacaggata
92881 actttttctt cccccttgga gacagagtct tgctctgttg cccaggccag aatgcagtgg
92941 cgtgatctcg gttcactgta accaccgcct cccaggttca agtgattctt gtgtctcagc
93001 ctcctgagta gctgggatta cagtgcctgc caccatacct ggctaatttt ttgtatttta
93061 gtagagacag ggtttcatca tgttgctcag gctggtctga aactcctgag ctcaggcaat
93121 ccacccgcct tggcttccca atgtgctagg attacagatg tgagccactg agcccagcca
93181 gttataggat agcttctaaa cttacaatcc ttgaaatatc attttaggat aattctgatc
93241 ctgcatacca gtgaaataag tataattgaa atattaaacc aaatttggct tcaacattaa
93301 accaaaatta aaccaaatta agccagtatg ttaaatacat agtttaatct taattctagt
93361 taatattttt catttcctat gtgtcaggca atatactaaa caacttacat gtattactat
93421 tttaattctc ctggtaaact ggaggggtgt gcaattattg tcatctccat tttatagatg
93481 aagaaaactg agtcataaag aggttatgta cttggctgaa agtcacacag tcagtgatac
93541 tgttggaatt tgcacccaga caatttaatt ttagagccta ttttcaacca ctctctgaga
93601 ctgctttcct tttctgtact tttgatgata aatgtgaaaa gataattttg agggcattat
93661 ggtgtattgg atcttaaaaa cccaggtaat aagtcccatc tatagtacaa ataagctgcg
93721 tgatcttagg gaaatcacat aactgcttct gtaaaatgag agattacatt aagttatttc
93781 caaaatgtct tttcgacttc aatgtggtga ttccatagta atgtataggt ttttaaaaaa
93841 ccatatcctt caatcaaatg gtcatccttt agcattaagt tgtttggaat agatactccc
93901 caaattcaca gaataatttt ccagcctctc tttttgcttt gacacctttc ttattttttt
93961 tttttttctt cgagataggg tctcactcag tcgctcgggc tggagttcaa tggcacgatc
94021 ttggctcact gcaacctctg cctcccgggt ttaagcaatt cttgtgcctt agcctcctga
94081 gtagctgggc tacaggcgtc cgtcaccaca ctcagctaat tttttttaat agagatgggg
94141 tttcaccatg ttggtcaggc tggtctcgaa ctcttgacct caggtgattc ctcggcccta
94201 gcctcccaaa atgctgggat tacaggcgtg agccaccgtg cccgctattg accccatttg
94261 ttgatgctta gggatgaaag tggaaactcc actgctaaaa cagagaatat tacttgagtc
94321 attcctctgt catatatggg atgtttgcca gcatggaaca ttcacagaaa ttataactgt
94381 aaaggcccta gcaatgattt aatgccgtct cttcacatta tagttgggga agctcaaact
94441 gtgacagggt cagggacagg taaaattcac aaagttggtt tacagcagaa ctgtgctaca
94501 gttatctgga ttcagatcca agtcccctcc aaatgaatca caaccacttc tacctttata
94561 attccaggcc atgaaagatc actgttttag tctgcgtggt gcagtggaac agatagacct
94621 cggtttgaat ctcagctcta ctgtttacta gacatgaaat ggggaaatct aaaatgagta
94681 agtatacaca gaaacacaca ttacatacat atatgtaatg ttcaggatca ttacactgaa

94741 ctttagggct tagtactaat gtacaatcaa ggttgtgtta gtactgagca gtatatcttt
94801 tattgttgaa gtccaataag aacagagcag atatcaacct aatttgtaag tgatgcattt
94861 tcctaaacta aatgcccaat aatggaatgg gctagaatat tgcaggacag ggctgtgaat
94921 ggaggaaagc agttggaaaa tcaatccttt taaaggcaca aaaggatgaa tgaaccacat
94981 tcccaagaac ctgctcccac ccactgctgt tcccaccccc gctgtagttg gtagcatgtt
95041 cacaaggaag gagctggcta gctttcatgg gagccacatc atccatcaaa tcacattagt
95101 gttgttgatt tgaatgttgt ttcctttgta ggtctgcttg ctttgacttt gtaaaattgt
95161 ttagtcttgc cattattata taaggactat aatcataagg catttatatt tcatttgta
95221 ttatgtattt aagcaaaatt aagttaaaaa ttagacagag atatatagga tgattttcc
95281 ttcaagagtt attcatttat ttccttttgg atatgaaaaa cgcttatgca gtcctgtatg
95341 tggtctctct ctctttttat ttttatttat ttattttttg agatggagtc ttgctctgtt
95401 gcgcaggctg gagtgcaggg gtgctatctc ggctcactga aacctctgcc tcctgggttt
95461 aaatgattct cctgcttcag cctcctgagt agcttggatt acaggtgcca gccaccatgt
95521 ccggctaatt tttttgtatt tttagtagag acggggtttc accatgttgg ccaggctggt
95581 cttgatcctg acctcaagtg atccgcttgc ctcagcctcc caaaatgctg ggattacagg
95641 catgagccat tgtgcctggc ctgtggtctc tttttaaatg tttgagattt tctctgacca
95701 tcacatgatg gcagccgcaa gttgtaaata tattaaatga gataatacca aatggcataa
95761 gaccactgtg aattcagtga tgttccaaaa taacttgtat tttattcatc acacaggcat
95821 ttatataaat taaaaaataa tagcacctga aaacatgaaa ataattgtat tcaaggttat
95881 ttactgcagc atgattttta agagaaaaga ctggaaacaa cccaaatgtc cactaatcga
95941 gactgtgtga acccaagatg ttatagctgc atcacacagc tgagagaagg aataagatct
96001 atttctactc actgccagtg gtaatctcca ggaagcgctg ttaagtgaaa gagcaaacta
96061 gagaaaatgt gaatagtggt taccatttac ccaagaagtc tatgtgtggt gtgtgtgtgt
96121 gtgtgtgtgt gtgtgtgtgt gtgtgtgtgt atgtgtgatt tctagaggga cagaggaaaa
96181 aggtgaagac aaggatagga gcgagacttc tctgggtaca tctttttttt tttttttttt
96241 ttttttgaga caaagtctca ttttgttacc caggctggag tgcagtcgct tgatctcagc
96301 ttactgcaac ctctgcctcc ggggttcaag tgactctctt gcctcagcct ctcgagttgc
96361 tgggattaca ggtgcccacc accacgcccg gctaattttt gtatttttag tggagactgg
96421 gtttcaccat gttggccagg ctggtctcct gaccttaagt gatccaccta ccatggcctc
96481 ccaaagtgct gggattatag gtgtgaacta ccatgcctgc ctgtctggat ctcttttag
96541 tacatttgac tttgaagcca agttagtatt ttccataatt aagaaaaaaa tagaataaaa
96601 aaggaacaat ttctaaaaag gaagtcaaaa gaaacacatt aaactaactg taagttgagt
96661 tggtagcata accacacaga cagaaattat ttcaagtgac tttaacacat actgatgtga
96721 ctgtgcatcc ctatcgagat agaaataaag gtaaatataa ataaaggtaa aaataagcaa
96781 gatataaata aagttaaaat gaactgcaac aatcctgaat agttttcaat aattatatca
96841 taaatagtaa ttttggtatt gttattttaa aacagttata tttataatac atatgttaat
96901 gtaatcatgt ttatgcttat cataacatat ggatgcacag ctattaggat aatgtataaa
96961 catgattatg ttaatgccat tagggaccaa gatttcagag aaaaacaaag aaaaatataa
97021 aattaaagaa gccagtaaaa atgttatctt aaatttaaac tggaaacctc agtataaact
97081 cataatgtgt tttcttttaa aaaattattt tcaaattcag tctcctgaat agaactcgca
97141 acaattacca agccagtagc aacgacaata aacagcccta gtgcccagat ggtgctctcc
97201 aaatgccatt ttccactaaa aggaatgagg gctccttgga gaattggatg ttttcagata
97261 cagagcagaa cacatacaaa aagttcctgg aacatttgtc atactgaaaa gcagatacac
97321 tatgaaagac tatagtaatt gtatcaaaag aactcaggag ccaccatgaa gaggccccca
97381 gtgtccagaa atggaataat gtgaacatta agaataataa atcttaattt gtcaagatac
97441 aaatttaatc atatcgtaga tactcatagc agcattattt aaaataatga aatattagac
97501 acaacttaaa tattcaaaac tagcagtgtg cccttatata tagaaatgtc atttaaacct
97561 tgtagaagag aagtttatga tttaggaaga tgtttacaat atgctgtaaa acacacacaa
97621 caagtcataa aatattatgc atgcagtata ttccagttta ttttaaatga tctaggtata
97681 aatatccata aaaagaaaac tgaatgaata aatggtgaaa tgtgaatagt tgctatatct
97741 gtgtggcagg attacaggtg gttgtattct agtttctctt tatgttccaa tcttctacag
97801 ttagcatgta taattttata tttttttaat acaaaatatg ttatttaact agtatttact
97861 aaacaaaaaa ttaaaaatta atatatttta taaaaacaaa agcatatgaa tagaaaagta
97921 cattattttt taggttatag acaatgcttg tacctgtgtt tagggatttg atcctccagg
97981 taggaaacta cagatctaat ttaacttatt taattaatta attaattaat taatttattt
98041 atttatttac tttgagacag agtctcgctc tgtcaccagg ctggagtgca gtggtgtgat
98101 ctcggctcac tgcaacctcc acctcctggg ttcaagtgat tctcctgcct cagcctcctg

98161 agcagctggg actacaggca tgtgccacca tgcccagcta atgtttgtat ttttagtaga
98221 gacggggttt caccacgttg gccaggatag tctcaatctc ttgaccttgg aatccaccca
98281 cctcaccctc ccaaagtgct aggattacag gcgggagcca ctgtgcctgg cctaatttaa
98341 aaaattttta agtcaccaaa aagcaaagtg acatgctcaa gtccattatt gccaagcaaa
98401 gaaaaaaatg tcaaaagtac aaaatgtggt tatgttttaa aaattagatt attattatta
98461 agataggatc tggctctgtt gcccagacgg gagtgcagtg gtgtactcat ggctcactga
98521 agccttgact tcttgggctc aaatgatcct ttcacctcag cctcctgagt agccgggact
98581 ataaggcagg caccaccatg cctggctaat taaaaaaaga attttttttt tgtagcaatg
98641 aggtctcact atgttgtcca ggctgatctt gaatgcctgg actcaagtga tcctcctacc
98701 tcagcctctc aaagtgctgg cattacagtc ctgaaccacc atacctagcc agatttctat
98761 tactttgaca tggtcctgga tacttcatct atgtgtgcaa aatttcgtac ttatctctta
98821 aatctggaga aaaatggcca aatttggtat agttgaattt atcatggggt gattaataag
98881 tgactaaaga ttgaccttca aggatctgaa gatctgacca gtgtagattt atagtaatta
98941 tagtaattac atggtacctc caggtatttt ctatacattg ccgtagttag ctatgaaagg
99001 atgtaaagaa taaaggcaat tgactatttt aaagaaatta acctcagaag gagattcttc
99061 acctaaatgt ccctgcaaat attattgctt tatgctctga gttagctgaa aatgagttta
99121 ctatgcctgg tgtaaacact tgcaccaccc acctgtgtca aagaactttt cactaactgc
99181 atcatgactg attgacgtca gtctacttgg caaccaagtg agccacttag aagaaggctc
99241 cccttatgag gttaatttct ttaaaatggt gaattgccct tattccttat gtcttttcat
99301 aactgattac acaaatgtat agaaaatacc tcttgagata caatacaatc tgtaactagt
99361 catcagagaa taacacgtta ttgtaacaca gatttagctt ggccataaag ggcatattta
99421 catacacatt gaaaacaact ttaataaaat gaaaaacata tatccttgga ttcttagcaa
99481 tttaacagca atgctctcag gggatcaagc tactatttat tgactccccc tgtgtgcctt
99541 acacgtaatt actgaccctc acaacatccc tgggagttag tgtcttacag gagacctttg
99601 acatttgtga gaggacatcg tgaatatttt ccaagtcctc aatccaaaag tgtggacata
99661 gctgttggtt ttcctgaatt gtatttcttt caagaatcaa atgttgcttg tgaatattct
99721 catgtaagat taaaaaaaat cttttcctga ctcttagaac tttagtttta tctctgcttt
99781 gagagccact ttaataagca aactaaaatc actctacaga aaggctgtac agcaaatgag
99841 tagagaacaa aagctggcta aactgggcag tcagctaaga tcactcagtg tctgagaatt
99901 tgttctagaa aatcaccctc cgagaatcat gcagattgtg cttaggcttc cttcatatat
99961 tcatactctc ctcgggtgaa cataaattgg cacatttata aggaaatttt attattatat
100021 actcaaatat ttttaaacat ataaatagtt tgtcttgtta attctacctc ttggaatcta
100081 tcctaaggaa aataatcata agatatctgc aaagaattat ttgaatgttg ataataattt
100141 tatctataac aataaaaatt tgcaaggtgg cagatatcag tttatgtctc tgagctccaa
100201 atccaccctt gtttatcttg ctttgtgtga atggagctgg accctgtgga tctttctctt
100261 ttgcaaattt gggggatatt caactttgtc aattgagggc actggagaga ccctgcaatg
100321 gaatagaggc tccaggtgca cctacaggcc atctttccct accagcagcg atttcagagt
100381 aacttcagcc tgtccatacc ctccagcaat ggtgggtcac ttctatagat gaactctggc
100441 ccacccgcag caccaagcaa aggaaggtca ctcctgtaca ccagcttcca accactctca
100501 tccaccagtg atgatagatt gcttttatgg atcaatgcca gcctttgcac tcagtcgttt
100561 ctttgttcct gcaggctgcc tgtggtagtt actatctctc tgaagaggtc tgaatctcag
100621 ccttggggca aggagggctt gtcctcatcc ttagtgcctt tactgttctt cttcctcagc
100681 ctagaaataa tagctccttt taaaattctt gatgcctggg cctcttagag tcctccttta
100741 tatatctttt agtaggtaat ttttggaaca ttaaattttc cctgttcaaa taattatcat
100801 agtttttgtc ttccacttgg accttgaatg atacaaaagt taaaggctca acaataccag
100861 acttgttaaa taagtaggga gctatccaca cactggcata aaattgtttt gattatctat
100921 tattcccttg gtgaggtgac ccagaccctt gtttgaaagc gttttattgg ctgggtgtgg
100981 tggttcatgc ctgtaatccc agcactttgg gaggctgagg tgggcagatc acctgaggtc
101041 aggagtttaa gaccagcctg gccaatgtgg tgaaacccca tctctactca aaagtacaaa
101101 aattagctgg gtgtggtggc gggtgcctgt aatcccagct actcgggagg ctgaggcagg
101161 agaatcactt gaacccgggt gtcggaggtt gcagggagcc gagattgtgc cactgcactc
101221 cagcctgggt gacagagcga gactccatct caaaaaaaaa aacaaacaaa agaaaaacaa
101281 caacaacaac aaaagaaaaa acaaaaatga aagaaagggt tttatttgtc ttctctctct
101341 ctccctgttt tttttttttt tctttttttt ttttttgtct catctttgct gtaattgctc
101401 atttgctagg gtcattggac acagagatac caagatgtct tcagaaatca tgaatattcc
101461 ccaagtcctc tatcaaaaag tgtggacaga gctgtcggtt ttcctggggt ttagacatgt
101521 tctttcttgc ttctgttgtg tagtaaccct agctcctcaa ggaaatcgtg gtctatgggc

101581 tctgccaaac agtaattcct ttctttgctt gctggctcac tggcttgagg aactcaaagt
101641 aatcctttgg taatttcagc ttccaggtca gtggggacat taatgtgttt cttggtagaa
101701 atgtcactct actccaagaa ataagatctt taatccagaa aacctgagtt ttcaggaca
101761 gaatgcacaa acttttatgg tcattcactg ggagttctaa tgagcaggtt cgatcatatt
101821 tctattcttt agttcctcga cccatgcatt ttagctactg ggcggcgaac catgtatcag
101881 ctgttagtgc tgtgtgtatt ccatatcatg aaggtaattt cccaggccca caacgttttc
101941 ttcccaagct ggtgccgtaa ctgagccttt accaggctat tccatcattt taggtgtttc
102001 tagggtatag agtaaatgat aagaccagtg actcccatga taaatgtgca ccatggtgaa
102061 cttcctttgc tgtaaaatgt catcttggtc ctagtctata tcgtaagggg caccatactg
102121 atagaccagg tactggtgag cccttggatg gtggtactgg tagaggaact gcaggaagaa
102181 aaggtaaatc catactcaga atatgtgttg attctggtaa ggacacgttg cttttccctc
102241 caagacagat ggttccgata gatggttgtg tagctaccct ccctgaggaa cggtactatg
102301 tcaaggtctt ggcatcagat tccgtggctg acaggttgga aatttagcat tgacagtagc
102361 tgaattatct tttgtaagtg ggagctcaaa tatgaccatg catagcacca tagtgattgc
102421 agcactagag acactattta tgggctttcc tgcacaagaa ctggggtggc taaagacggc
102481 ccggctaaaa ggcaggatgg atcatcctga gcacatggtt gttgagagtc ttctttgcag
102541 tcgggcggta gtgtgagatc tgctttgtgt ttatttccgt agggttgtcc acatacattt
102601 ttttccccac accttttctc tgagcttcaa atcttttttt cttgtcctgt cgaaatggcc
102661 aagccattca ctactgctta gaagccagtg catacttaag gatctggtct tttcttccat
102721 atgaaatgaa taactaagtg tgttgctcat agttttcccc actgggagga tttcccttta
102781 cctctcttct ttggggccac ctcaagtgag gatgaaatgc aacagcagac tatctctggc
102841 tagaggcaat aaatcacact agcccaatta tgcaccaggc ctgagctgtt tcctccacta
102901 ctgattgttt gtaggtagca gttgtgggag cttctgtgga acagattcag gtggtacagc
102961 tgctcaaact cataactcac ctgcaatgtg ttctgaaaat gtgtggctta actgcatcta
103021 ccctaaaaga acaagttggt gagtgatgag atgagtggga agtagtgagg atgacctgga
103081 ttttccattg aggaagtggc caagtcctgc ggacaaattg tacctaggaa aaggtacaaa
103141 aacaggagaa tggatcatgc gttcagtatt ggaactgatg actattaagt tgctgtgggc
103201 atctgaatat agatgttgtg atggttttaa aacatgtctg aaaattcttt gacactgttt
103261 ccatcaagag gtgatgtctt tgtctcctcc ccttgaatct aaatgggcct ttgtgacaga
103321 tctgatgaat agaatgcagg aatgtgatgt ccaaggctag tttagaaaag accatgtttg
103381 tttctctgga gacacttgct ctgggagcta ttagtgtcct tccaccaaag ccccccagga
103441 acacccctgc agctaaacaa attggattta ttgcttattg tagcaagaca gaacattcac
103501 cagggggaac cataggttat ctcagtaaga gggttttaga aagaacctat tataagattt
103561 gggctttggt tgggtgattt gggggaaggt ttaaggaatc aggggtggcc aggcacggtg
103621 gctcacgcct gtaatcccag cactttggga ggccgaggca ggtggatcac gagatcagga
103681 gatctagacc atcctgacta acacggtgaa accccgtctc tactaaaaat tcaaaaaatt
103741 agccgggcgt ggtggcgggt gcctatagtc ccagctactc aggaggctga ggcaggagaa
103801 tggcgtgaac ccaggaggcg gagcttgcag tgagctgaga tcgcgccact gtgctccagc
103861 ctgggtgaca gagcgagact ctgtctcaaa aaaaaaaaaa agaaaaaaaa aaaggaatca
103921 ggggtttggt ctagattgaa tgctctcaga aaatgggagt aattctaggg ttgtatattt
103981 tataggtggc atagtgacct tgttttgtct gtccttacac aaatgatgaa gtaggctgat
104041 tttgtttcat tttatcttgg tcgcagagta actttgtctg aggttggtgt tctataagat
104101 tgtatatgtc caacaagaga atgacatggc ttagctgtga atgtcagatc agttctggat
104161 atgagaggct gatattttct ttttgagctt tcagttgtca tgtaaggagt gtggctacca
104221 tgaggccact gcatagagtg attgtatacc tatatataag agagatggtt tggagttcaa
104281 gcccccagct agtcaagtct tcccagccca ggagacagat gttcgaatga aggagactta
104341 ggtaatttca gccccagtca ctgtctgacc acaactgcat gagagaccct gagggagaac
104401 tgctttgctg aacccagtca acctccagat ttgtaagtaa aataaatggt tatcattgat
104461 tgaagtcact gttctagggt ggtttttcct acagtaatgt cattgataca ggtgtctggc
104521 aggcagttgg ctctagaggt ctggagctaa ggagaaagat ctggcttgga gatgtggatc
104581 aggaagtcct tttacaagga gaaaagtgct tgaagtcatt tgagtggatg acatcacctg
104641 aaagataata tacattgaga aagaaaaaaa taaaaagcca acaatagcac ctttagtaac
104701 aacactgaag agagcaggaa gaaataaaga aacatttgca aggagacaga gaaggagtga
104761 ataaggtgat caaatggcat ccatccaaga gatgatgtcc aggaagccaa gaatgagagt
104821 atttcagaaa gaagagtgtt gtccacagag ttaaatgcta tggagagaga tggagtaaga
104881 caagattgat tagcaccaat gagctttcat tgttgacaaa tgcagtttga gggaaactgg
104941 gggctaggga gtgatggggc agagaccaga atgcagtggg gtaaggaggg gtaagaagat

105001 gagaagaatg ccaaatctgt gctgtttgcc acatcatggg ccctctctcc atactattta
105061 tgtgtgtata atttttttct acaatttaat aatgcattaa accatcacat cacatcacat
105121 cacaaaccca acaattccac aatagcatgg gtagatcata ccctggtgta tttttaatgt
105181 tatactttaa ccggtagttt tgagtcataa atgtactact atgttgttgg tttttttttt
105241 ttttttgggt ctgtcattat agtaataact ttaaatctct tattgtttat ttctcttgca
105301 attaaaatag ttcatcattt aaaatagtac ccagtgttca gtgtccattg gtgccttgta
105361 attacttcag aaaaacagtc tcagtttaaa agttataaac tcttaatgcc tctctgcagg
105421 aggaatagct tccttctgcc agagcggtct ggggagcatt attctagtca taccctgcta
105481 attcattggc ttgattgtca ggggtcctga tctagttcac cctcctggga ggctggtgtg
105541 ggaccctgct tatcccagtg ggggtcaagg cctccttgac aacagtgaat gggttgctag
105601 gaggctgcgg aaagaaattt ctctagttcc atccacaagt agatcccact gcagtggcct
105661 catttggctc ctctaatcct ctctttccca ccttctttca gggcaagtct caccctattt
105721 tcttcattac ttgcttgtgg tttccttgcc ggagatgctt ggaggcaagc attatgacaa
105781 ggatgggcgc gggtgggagt aaggccttta aacgaagagc tacatttcaa agaacttcaa
105841 atgtgtattt caaatgtcac ttccaaatga ggttgtacac acagacagaa acgcaattaa
105901 cagatttgga agaggccacg tagcatgcat ttgaaactcc tgttgcattc cttggctatt
105961 cttgtctgtg gcatcttttt aacaaatcct gtagttacct tgtaaatacc atcatagaat
106021 atattgtatt taaaaaacat tctttactca gatatgaaag ttgaaaaggt actattattt
106081 ttaaaaatct gtattggatt tttttttttt catattttag gatgccagaa gcctcaaaaa
106141 tggaaaaccc cctgtgcttc acatctgaaa atctctgctg ggggcagcaa ctttgagcct
106201 gtggggaagg aactgtccac gtggagtggt ctggtgaatg cttaaggagc tgcagaaggg
106261 aaggtagtga ggagacagtt aaatattgag cacgttttgg tgcctagcat tgtactggcc
106321 tttcacatgc tatgtttaat cacgcaggat tgtatggaat gggattgtac agaatgaggc
106381 ctgggtttgc taaatcctgg gggagattgt ctaagctgtc acttgaaggg aaaactgtag
106441 catgtgggac ttaaaaacag cttgggatgg agtttgaggt agggcgagag ggaaaaagat
106501 cttggcagat acattctgaa caaatacttg tgtcatcctc aactgtatag tttttatgac
106561 ttttcctgtg ttcatctggt tggcagatga atgcaaaatg ccttttagct tcatggaagg
106621 cacacatgaa tcatggctat gtgataaaca gaagaagcca cgtttcggag ggtttctggt
106681 gtgtgtacgt gtgcacttgc atgtgtgtgg gcacaccctt gtgccaaatg gaccaccagt
106741 ttaaatcgat tttcaacagg gcagatttca tatgcttctt tagtgtggcc caagtcaatc
106801 tatttttaga aggggaaagt agagcatgtt ctgaaagttc ttttcttctg ccatcaggga
106861 ttgtagtact ttttatttat agtaatttca ggtctctaaa tacttcaagc aaaattaatg
106921 aggcaaacaa aaaaaattag gtaccagata attggatcat gtgttatttt atagagagct
106981 ttatttatct tgcctttgga aagtataatt tgtatttttc aaaattatga ttagatctaa
107041 ttctttttca agatttactt aaaataaata tttctgccta ggcacaagac tgtgtatagc
107101 acaactggcc ttgtattatt gacttgatga tgcaaagaaa aatatataag aatcagtttt
107161 ttttaaatgt gttaatattt atcataatgg aaatagtctt actaattagc aataactaga
107221 aaaatgtcta ctatttattg agccactact atatgccata ttctttacct atgttcattt
107281 tctcttctat attcaaaata atcctataag gtgtgcttta tgatcaccat ttgttccaat
107341 agggtctcaa ggacgttagg taattattcc tatgacattg agcctatgaa tggcagagct
107401 gggagtttga acctaagtct gatgactctg aagcttatag tctttctata agcttcttca
107461 gtctgaagct gcccctcaaa gtcatggcaa taactgagaa tagcattcag ttttacagct
107521 aggaaggtag ttgtagttgt gtttattatt caatactgtc catattcaaa gaaccattca
107581 ttgagcttct attttactcc agtcacggtg ataaataatg cagaatctag agatcaaaga
107641 taaaatggcc ttgccctcaa ggaacccatg agctagttgg gaaaactaat aggaatatta
107701 atgattataa tgcagaatgt taggtgcaat gagagaggta acaaggtact actggggcct
107761 gacaaaggcc accgtttctg tgtgtgtgtg tgtttatgtg tgtttgggaa aaggcaaaag
107821 acacttgaga taaagggaac aacatagaca aaatcaaagg cttgagagca tataacttat
107881 atgagacata gcaaattgtt tgctttaact agaaaatagt ggttatgtgt ggcagactat
107941 gagaaatgag aatgtaagtc cttgagaatg ataactttag aaattctaca tttgggatga
108001 tcataacctt ttaaaattgt ctcaatatga tagatatttt ccttctgttt tggggtatct
108061 gtctttttat tttttatttt tttgaggtgg agtctcactc tgtcacctag gctggagtgc
108121 aatggcgtga tcttggctca ctgcaacctt cacctcctgg gttcaagcaa ttctcctgcc
108181 tcagcctcct gagtagctgg gattacaggc gcctgccacc atgcctggct atttttgta
108241 tttttaatag agatggggtt tctccatctt ggccaggctg gtctcgaact cctgacctca
108301 gatgatccac ctgcctcagc ctcccaaagt gctgagatta caggcatgag ctaccacgcc
108361 tggcctgggg tgtccatctt ttaaagacac actgtaagaa tcataatatt ttcccaaaga

108421 agaaatgtaa ttagtacaca attacatgtt aaaactaggt gttattggta attaaaattt
108481 aaaaaaatta acccaaccaa ataataaaac taatgaaaat atttaatatt ggtaagaaca
108541 agatgcgata agcatatttg tgttttgctg attggtctat aaagtggttc aaaacttttg
108601 taaactatat ataagctatt ttaaatgttt atgccctttg attcaatagc actaggtttg
108661 gtactttata ataggaaagt tattagaaat atttactgca gggtttcttc aatgatgagt
108721 tgtaagaatc agcctaagta gctatggtac atcactttga tgcaacttaa tgtgtcatta
108781 aaaatcatgt ttatgaataa cattccagat gcatttaata caattctagc tgaaaaaaag
108841 atactgaaac atttgtgttt tatatatgtc tgcatatgta tctatatata tccctctccc
108901 tgtcaatcta taaattaaaa actatacatt aaaaactgca aattaaaact atatactaaa
108961 gaaagaaact ggaaagaaat ccacagaatg ttaataatca tggtcattgt gttgagtggt
109021 aggactatgg gtaatttttt ctttctattt ttatacattt ttcacttttt tttttttttt
109081 caatttgagg cagagtctca ctctgtcacc caggctggag tgcagtggaa cgatcttggc
109141 ttactgcagc ctctgcctcc gggttcaatc aattattcta cctcagcctc cagagtagct
109201 ggaattacag gtgcacacca tcacgcccgg ctaatttttg tatttttggt agagacaggg
109261 tttcactatg ttggtcaggc tagtctcaaa gtcctcccaa agtgctgggc ctccaaaagt
109321 gctgggattt ggcctcccaa aatgctggga ttacaggtgt gagcaccaca cccggcccat
109381 ttttcacatt tttaatgatg tttttaactt taaaagtaaa aatttgtaat ttaaaatttg
109441 tttaactttt acaaataaaa attacaaatg caaattaaaa ttgctatata tttttgttac
109501 tggacaagaa aagatgcatt atagagtctc gttttatttt gttttctgat aaatctattg
109561 ctggggagca tgtgcgtaat tcgttttaaa aattctcagt gttaactaga tttcttttgg
109621 gatagctctg ctgaactgga atgagatctt cttccccaga gccacttgct agtttgattt
109681 tctagaaaga gagaaattat agaaagctac aatctacaaa ttgctatgta ctacagtcta
109741 ctaaatagta taatacttta ttttgcaaac tatatctctc agagtacaag gctccacagc
109801 tagtcttgca ccaaatatat gaggttcttg aatagtcatt ttctcaaagt tctcatctct
109861 ttacaaagca agttgagggg ggaagcagag agagcactct ggtgcttcca aaagcatcag
109921 ccatccacac tctgcctatt gaaaacacca aatctgagca tgtgtgtcat tgtccaggaa
109981 taaacaatca caaaaaggca tcaatgaggc ctataaaaac aagagcgcat gcaagaagat
110041 gggattgtta tgaaacagga gcacaaggct actaaaagag ggcaagccta aaggaaaagt
110101 caccagggtg agatggaaat agatatagga agaatagtga acggtggcaa ggaagtaaag
110161 gaatacaaaa gtaataaaat atccattgca ttagtccgtc cttgcattgc tgtaaagaaa
110221 tacctgagac tggataattt ataaagaaaa gggtttattt ggtttatggt tctgcaggct
110281 gtatgagaag catggtgcca gcatctgctc ctggtgaggc ctcaggaagc ttctattcat
110341 ggaggaaggt gaagggggag ctagcatatc acatggtgag agatggagtg agagagagag
110401 agaaagagag agaggagatg ccagtctttt aaataaccag atcttatgtg aactcagagt
110461 aagaactcac ttatcatgag gacagcatca agacattcat gaggaatctg accccatgac
110521 ccaaacacct cccactaggc ccacctccaa catgggcagt cacatttcaa cataagactt
110581 ggagaggaca gaacatccaa accatatcac ccacattgag ggagcaagga gcagactcca
110641 tactgcagaa cacacatggg aaacaaactt gagaagtttc cccagggcat agggtaagag
110701 gacgaggggc tggaaaaaga aaggtaatgg atatagagga cagagaatgg agttcgaaga
110761 tacatctaag aactttacat attcctaagg aacccacaaa gataaaacaa acagagcaag
110821 tatgaaagat gtaatagaag caaatgaccc tgcattgaaa atgatcaaag tatgcagatt
110881 ggaaaggctc acaaattcca ataaaaagaa ttgaaaacaa aaaaaaatgc tgcaataaca
110941 ttctttaaac tatattaatt gtaaggtttt tacaaaaata agtactcagg cagtagtaaa
111001 aagtttgcga ataaagaaac caaaattaaa ctctaacttt tcttatgtaa taccaaattc
111061 cagaacttgg aaacatatct gcatgcttta ccagggacca gattatgagg cagttattta
111121 agattagaaa agttctcttt cattggtaaa ggcaacagaa agatatattt tagaggtaga
111181 agctctctaa catagacact attctgccaa aaaaaaaaaa taagataaat gtaacaaaat
111241 aaaatgagct tcaaggtgta ttttggtcac tgagagatga ttcaaaagaa gactttgaac
111301 aatgagaatg ttaacattta aaagaatagg tggaattttt ccttactcag tttgttcaat
111361 aaaatcatgg gtccactgta acctgatata tcttgttgct taaaggaaaa gcttggccct
111421 accttttctg ctttcttctg tgttcatggt gtagaaaatg tatttttgtt aaccaattat
111481 ttacttcaca cttgaggtag actttgttgc catgttggat ggctattttg ttaggtcaaa
111541 cagttttctg ccacatgcct atgtgatttt aaataatgta gccctcaccc tgagcttctg
111601 ctatagggac cacatgcttt gtggtaacca ggtgctgcca aaattggatg atagcaggct
111661 cctgacccat gcctgtcaag tcataggtgg ttaaggtcat gggagttgga gtcagactgt
111721 ctggttccaa atccaaggtc taccagttga ttttgacctt aattttctta ttctgtaaaa
111781 tgagtttatt gattgttccc actcagttcg tttgaggtga ggattaaatg agttaatacc

111841 tgtaactttt tgactggcac aagacacaat cctattacat gttctgaaaa aaaattctct
111901 acatgaacca aaggaatgaa ttctatttcc tccactgcta ctacaactca acacttctaa
111961 tcaccaaatg tgtggaggtt tttctacatc agtttgtcag acacctacta tgtgtcctat
112021 aattcagttc agttctgaca ccatctacat ggaaacaaca tccgatccca tggactaaaa
112081 gctcagtctc ataagactgt ctataatggc tcaagtgggt tcttcttgcc tgctgcacag
112141 aaaaaccaat acatcaaggc agtggtgttg cagtagagaa agcatttgat aatcacaagg
112201 ccagccaagt ggaagcatgg gagataattc ttaaatctgc ctccccaaga gctcaggggc
112261 tagggttttt aaggataatt tggcagacag gggactaggg aacgagtgct gctgatgagt
112321 tgtggatgaa atcataggag tatccaaact gtcttcgcat gctgagtcag tttctgggtg
112381 gaagtttctg cgtgggagtc aatttcttgg catgagtcac aggtccgggt ggagtcagtt
112441 agttgccaga atgcgaaagt ttaaaaatat ctcagagacc aatcttagat tttgacaata
112501 gtgattttat ctataggagc aagaggggaa gttaaaaatc ttgtgacctc tggctacatg
112561 attcttgagc agtaactatt atagaaaggt aagctaggga acaatgtctg gttatcattt
112621 aattacacct acatcttagc agaatttaag ttcttctcat aagcccaacc ttgtggactt
112681 tcattagtct tacaaaggca gtttcagtcc cccaccaagg agggcattag tttcaggtag
112741 ggactgttat catctttgtt ttaaagttaa aaaaggcagt tagcttgtga tgttagaaga
112801 aagatgtcag atttctctca ctgtcataat ttttgcaaag gtggtttcat ggccccactt
112861 gaaatgagaa tgggaagtcc aggttttcac ctgtgcttta gactgggtgg agataaatta
112921 aaggttcaca ggacctcctc ctcagatttg accatttgct agaatggttc acggaactca
112981 gaaaaacagt ttacttattt gattaccagt ttactttaaa aggaaattac aactcaggaa
113041 gagccagatg gaagagaggc atagggcaag gtatgtggga aggagcatga agcttccgtg
113101 cattcttggg ctcatcaccc tctcagcacc tccatgtgtt cagcaacctg gaagctcttc
113161 gaaaaatgtt cttaagccag gtgtggtggc tcacacctgt aatccccgca cattgggagg
113221 ctgaggtggg tggatcatct gaggtcagca gtttaagacc agcctggcca atgtggcgaa
113281 accccgtctt tactaaaaag agattagcca ggtgtggtgg cacatgcctg taatcctagc
113341 tacttgggag actgaggcag gagaattgct tgaactcagg aggcagaggt tgcagtaagc
113401 caagattgtg tcactgtact ctggcctagg tgacagagca ggactccgtc tccaaaaaaa
113461 aaaaaagaaa gaaaagaaaa agaaaaatgt tccttcagat tttatgaggg gcttcattgc
113521 atatacatgc ccctccctgg aggttggtct ggaggttggg ctgaaagttc caactctaac
113581 cacatggttg atttccatgg caacagccct gcttatcatt agggacttcc caaaagtcac
113641 ctcattaaca caaactctgg tgtgactgaa taacaaaaga tgctcctgtt acctttattg
113701 ctcttagaaa attccaaggg ctttaggggc tttgtgccaa aaacaaacac tatatatatc
113761 ttattataaa tcacactatc tcacattatt attatttaaa atgataagtc agtcaagctc
113821 tcttttcttg agaatttgat ttgggaaact tcgagaatga ggcaaggagc agtgggaata
113881 ggagtttaaa gtaagcatag aaagcataga aagaagctag ggaatagatt aaggttgcaa
113941 ggagggataa caaatggaga ttaggaaata gtggagactg tgattaagca gagcctgtca
114001 ttaggcattt ctgatataaa cccagatgct gtcatatcac ctcactgaat gcttgccctg
114061 aaacacttgt tttagtgcag ataaattcat cacctaagga tggcttatcc tttctatggc
114121 cattgaactt aagagctcca ctaaatagga taactccctt tatgctggga aatctaccaa
114181 aattacctgt gtcagaaggg taagaaaaga gaatccagga tccagaatgg gattgaagtg
114241 atctcccatt ctttttgtga tatttagact gccttctgcc tctccaatcg acagaataaa
114301 gaccaaactt ctacccgggt gggtagggct tatcctgagg atggagactt tctaagtata
114361 gatggtcaag aaactggtgc acatttaagt tagacgcatc tctaaattgt ctgggcttag
114421 actattctaa gaatgaataa ttatgaaaat tgaggcagct cagagactca ttttctaaga
114481 tttcaatata tgattagcaa agtgctatag aaaaaaataa aatggcacag tttggcagtt
114541 ggcatcttgt atctttctgt cccaggtctt tcttgtgctt gtaattcttt cattgataaa
114601 gaactcagct atctatccag gcgtgatggc tcatgcctgt tatctcagca ctttgggagg
114661 ctgaggcggg aggatcactt gaggtcagga gttcgagacc agcctggcca ccatggtgaa
114721 acccacatgg tgaaacatct gtactaaaaa tacaaaagtt agctgggtgt ggtggtggga
114781 gcctgcagtc tgagctactc aggaggctga ggcaggagaa ttcctcgaac ctgggacttg
114841 gaggttgcag agagccaaga ttgcgccact gcactccagc ctgggtgaca gagcaagact
114901 ccatctcaaa aaaaaaaaaa aaaaggattc agcaatgtgt ggttcttttg aatctatggt
114961 atgtattagc acatcacagc agagcagagg ttctcaacaa ttgattttat tccccagggg
115021 acatttggca atgtctggag acaattttgg aacttagagg ctgatgctat tttggaactt
115081 agaggcttag atgctattgg tgttgtgggc agatgacaag agagattgca aacgttctcc
115141 aattcagaag gcagcctcca caaaaagaat tattcagtcc aaagtgttaa tagtgctgtt
115201 attgagaaac catgccctag agaagcagcc tagaactttg agtttttcct tttttttttt

115261 tcaatcaact aatgagaata aatgtggagg agacgggcaa aaatgtcagc agccccgtat
115321 ggttttcaaa atcatttcag atatttgcta ttcaccagtg tcagccaatg tgtttatttt
115381 tgaaaggtca gaaatttcat agggccagat tactaaaatg gttattatta tttttttgtt
115441 ttcaagagct tatgttagtt atctagtata gagaagtcta ccatagagat ccctttgttc
115501 tgagtactgg actttttcac ttaccaaaac tctcctactc tatacgttct tcaccgtgat
115561 aatccaagaa gtctgaatcc tccccatggc tctcccctgt ttccatcccc agtcggtggg
115621 tttcccacag gtctccagtg tgctttggaa gtaggaactc ctaacagatt cacagtttga
115681 acatggtagt cctctgagct gttcagtatt ttaaatctct ctgcagttag ttataatcat
115741 tcaactcttt tagtcattct tctaatgtca gtatagataa ctaaaagatt gtaatattag
115801 aagacctatt tcccagagac cacaaagaca atctcatgga agtcccactg tagtaaatag
115861 agtctatttt ctacccagca tctggtagag taattcagac atgtgttttg acagtttgcc
115921 tgaaagtaac ttgacaaata attttactaa agaatcctag aaagtcaatg gcaacatttt
115981 gttcattcta agttgttcaa gccacgtcct tagatgtggt tgattcaaat ttcttcagaa
116041 ctggaaaagc acaagagaat tagtattcca gtaataagga atcatggatt gtggcagtct
116101 ttttaaaaca taaactagtt tagcacataa gtggtggcct agtacatttt aaagtggagt
116161 ccaagggaag tttcttgaat tgctgtagtt cttatggtgt tctgctgaag ccgtgtagat
116221 cttatggtgt tctgctgaag ccgtgtagat attgatcatg ctgcctccca ccaccacccc
116281 cttcattcca gatctgctca aagacaactt agaaggtcct atcctggtct cctgtctgaa
116341 acatatactt tctctcctcc cgcccccagc ctgcccatac tattccaatc cttctctacc
116401 tattattcct ttgttttgtt ttcaatataa aaatcttatc tatttcttta tacgttaatc
116461 atctatcact ccccacacac tacaatgtgt gcattcattc cctgagtaac tgaaacctag
116521 aggcccggga gtcatttttg aaacttcctt tccttggctt tctcctctaa ctgctcttcc
116581 aattcatcgg ctggatttga ctattctaat tccaaatctg tctggaactc gtgtacctct
116641 cgcagtgtcc actgctgttg ttctggtctg gggcacaatc gacactcatg ctcaccgaac
116701 tgcagcagcc cccttcctgc tgcttatttt gaccacacct ctttttgaac ctattctgta
116761 caaaggagct ggaaagagtc agtaaaaata cagagcatag tgttcattgc cttgcttgga
116821 caggacctgc catatgtgtc ttctctatag tattggctga atgcataaac agcgagaaat
116881 aaatgtgtag agagtaaatt gatagaaagc tgccctcagt catctcccat ggagagggct
116941 agtgatgact ggcgaaggtc acagagctac tatgtgttag attttggact aaaatccagt
117001 ttttctgaga acggtcaaga gtttatttat agaaaacatg ctgctttatc tcttaattcc
117061 ttctcactcc ctgcaaatta gtggcatttt agtttactta aatgcacaat tatttttaat
117121 tttgattgaa aaattaatac tcatttactt ctgaaaacta tttctttacc gaagagcata
117181 gagaaataag ggagtaattt atcaacctac cataggtgga atttttactt tcatctttat
117241 actttcttct tcattattgt tttttttaaa aaacgtaagt atgcatttat ttttataatc
117301 aggaaatatg tgaagttata taaatgtgta atgaatttca ttactgggaa ctaactgctg
117361 gtgatatttt acacttgatt ttagccaaaa ggccgagaag cgatgctggt gatattctaa
117421 tgtactataa cttattctaa tatttcatgg gatttatttg caattagtgt gatataaatt
117481 tccataatgt atggaaagat atattagaag atgctaacca tgggcttaag aaagtagatt
117541 tcataatttg aaacacggcc cacacaatta ataaatataa gacatttggc actctacttc
117601 taatctcttg atttttttta tagcaactgt ctttaagaac cccagcatat tcatggattt
117661 tttagaaacc taattatttc ctagaaaaat cctttgaaaa aggaaaatta tatttgacag
117721 ttttctaaag attttctgtg cagaagttgt atctggatat acatataaag ccctgaagta
117781 atctatacat tttatcgctg gtcttcagat atgttggcag agtcgctcgt gaaatatatg
117841 cttgaaatta atgtaattac tctgacattt gttattttaa taagcattct tagctaatac
117901 atagaggaat acattaaagg aactctaaca ccaaaattat cctttcttaa ttaaataaga
117961 aggactgcaa ttatttaaaa agtgtatgtc aaatctaagt tgccttcaaa tgtaatttaa
118021 ttttctgtta cttacatttt aaaatggcca tagctttttt cgttgttcat ctttcttaat
118081 tctttaggca ctacttaccc cccaactcca gcaaccaaaa ttaatcctaa tttgcatcac
118141 aataatttaa aatagatcaa agaaccaatt tcatagccta attcaaaagc atcatgcgac
118201 caatatgtag tattaataaa acatatttct ggttttgcca atccaatttt tttttaatt
118261 aagtgcttaa cacagtgccc agcacagaaa aagcacttga tacatgtttg gtaatgtctt
118321 tgtgactgtc atttttacta atgttgtata ttggtactac taaagtctaa tgggatgctt
118381 gtattatggt gtgataatat agttaggatg accacttgtc atggctggcc tgtgagaatc
118441 ctgtttaata tctattgttc cagttttctc caacttaaca ttttagcact caaaagtatg
118501 tgatgtggag gataaatttt atggtcgcca atcctttatt gtaaagaatc gtgatgcaaa
118561 ggctccagtg tagtcatcac tcatctcttg atttaggcat ctaagctatc caggcatcat
118621 catgggggtg agcagcctga atgacagtaa gctttccaag tatggggaca tgcacttaag

118681 ataggtatag ttattccagt gccaatctcc tcttacctgc tgtatgtcaa gaatcaggca
118741 acatttagga aatgaaacca caagtctttg tctctttttt taccctttat ctcaaaatgg
118801 tttttacttg ggatttgttt ttttgttttt tgttttttgt tttttttttt gagacggagt
118861 gccgctctgt cgcccaggct ggagtgcagt ggcgcgacct cggctcactg caagctctgc
118921 ctcccaggtt cacaccattc tcctgcctca cctcccgggt agctgggact acaggcgccc
118981 gccactacgc ctggctaatt ttttgtattt ttagtagaga cagggtttca ctgtgttagc
119041 caggatgatc tcaatctcct gacctcatga tccacccgcc tcggcctccc aaagtgctgg
119101 gattacaggt gtgagccacg gtgcctggcc tttagttgag atttgattat gaatgaaaat
119161 gtaaagaaat ttgctagatg tgtccatctt ttcatgtggc agacaatatg gactagatat
119221 atggtggtca aaattatgtt tgtgggtata gcctaatagc ctaaaccaat gacacttatg
119281 tggcagtttt atttatttat tttttctttt taagacaggg cctcactctg ttgcccagtc
119341 tggaatgcag tggtgcaatc tcagctcact gaaacctccg cctcctgggt tcaaatgatc
119401 ctcctgccta aacctcctga gtagctgaga ttacaggcat gagccaccat gcccaactaa
119461 tttttgtatt tttagtagag atggtgcttt accatgttgg ctaggcttgt tttaactcct
119521 gacctcaagt gatccacctg ccttggcctc ccaaaatgct gggatagcag gcgtgagcca
119581 ctacgcccag cctcaccctg gactttatga caatgccaaa tatatgcact gtgtcaaaaa
119641 cagtcctctt cactgtcctc cagtagaaaa agctcaataa tgggtctaca aactcctgga
119701 tttagtccag gctatgtaat ttgtggaact tagtgccaaa agtagggtcc tttgttcaaa
119761 aattattaag aatttccaga tggcaacagt ggaacagtca atgaagcaca ggatccttct
119821 gtccccaagg tcctgaacaa ctgcagaaac cttgtgcccg tgcattcatc cctccttctt
119881 ggatccttta cctgttgatc tccttcgttt tctttccaga caacaattat ggacaactta
119941 ttattaatat atgtgagcca ctgtgttcac tatcggaaat aaaaatatgc ttataaggta
120001 gtcccaaatc tttttttttt tttttttttt tttttgagag acagtctcgc tctattgccc
120061 aggctggagt gcagtggtgc gatctcggct cactgcactc tccacctccc gggttcaagt
120121 gattctcctc ccgcagcctc ctgagtagct aggattacag gcgtgagcca ccactcccgg
120181 ctaatttttt gtatttttag tagagacagg gtttcatgat agtggccagg ctggtttcga
120241 actcctgacc tcatgatccg cctgccttgg cctcccaagg tgctgggatt acaggagtga
120301 gccactgtgc ctggccccag atcttagatt ttggtgatta aggacttcac gaagtagatg
120361 aaataacacc cagaatttgg ctggtttatt ttctccttaa gatgctcagc tgtgatttc
120421 tgtatattag tctcaagctg caaattctct attccaggat cttatgatct gtgtcctaac
120481 tacatattat agggccccag tggtccctat gcctctggtt ctattgtcat cacacacttc
120541 ctgtttcatt cctatagaca gatttattca tgactaggga acagattatg ctagtaaagg
120601 tgttggttaa catgagttta aggcaagaac ttaaacgtaa atatctctag ggattggaca
120661 ggcaatatgc cttaaatagt aaagctatca ggtaggaatt gactgcatgc ctgcagagga
120721 aactgctgcc tatttagcca cagaagatta ttgccatgta aaaatgtgag tatggtttgt
120781 cacatatttt taaagaaaag tcagaaatcc aaaaatttgt atgaaatttt taaacatttt
120841 atattgtcaa ttcaattttt aaaaagactc aatataggca gagaaaaata actacagccc
120901 aaggttaagc caagaagtat tgtttttcta ctttttcttc gtagtcagat ttgttcagat
120961 ataattcaga tgtaataaat aaacccttta agttctatga gttttaacaa acacctacat
121021 tagtgtgaca atcactgtag acaagatata gaataattct ctcgtctccc ctccaaaatt
121081 ctctcaagcc tctttgtggt taattctcct agctcctgac aatcgctaat aaatttcctg
121141 tctatagttt tgccttttca agagtgtcaa ataaatggaa cactactgta tgtagccttt
121201 tgagacaggc ttctttcact gagcacaaat aatttgactt attcacgttg ttatatgtat
121261 ttagattgtt tctatctaat tttcaaaggt tttttccttc ctttcttctc ctttcctccc
121321 tccttccttc ctttcttttt ttcttcttcc tccttttatt ctgccttttt tctctccttc
121381 caactttccc gcttttttag atatcacatg tacagaagta gattaatctt gatgtttatt
121441 tcataacata ctctcatttg ggcccatgca tggcttgtat ttcaatagct ctattgagat
121501 aaaactcata tacattcaca tggagaaaca gccctgtgga gggaaagaaa ttgatatgca
121561 gtaaactaca catactcatt tacaacttga aaagtttgcc atatgtatac acaatgaaac
121621 catcaacact ttcaaggtaa taacatatct cagtttcctt gtgtctttat gttattcttc
121681 cctccttcca tctgatctgc tttgtgtcac tgcattttct agaattttat gtaaattaaa
121741 tcatatagca tgtgctcttt tttttgtctg gcttctttca tgcagcacaa ttattttgcc
121801 attcatatca gtttttgtgt gtatcaatag ttcattctgt attattacaa attagcattc
121861 aattacatga atacattgca atttattcat ttgtgaatgg acattgcatt atttccagtt
121921 ttggctgtga caaagattca tgtggaagtc tgtataggga catatgcttt catttctctt
121981 gggtaaatgt cttagagtag aatgcctaag tcatatggta gatgtatgtt taacttttaa
122041 agaaattgcc aaattgttgt ccaaagtgtg taccatttta cattcctggc agcagtgtat

122101 gagagttcta gttcttcacc atcccccttc caacacttgc tatagtcagt cttgcaattt
122161 tagccatttt aataaatgtg tagtgatatc tcactgttgt tttaatttgc atttccctag
122221 tgtctagtta tgttgtgtct ttacttttgt ttatttgcta tcattatctt ccttggtcaa
122281 atatctgttt aataattgat acatttttta ttggactgtt tgttttctta ttagtaactt
122341 tggagagtgc tatatatatt ctggatacaa atcctttaac aaatgtatga tttgcaaata
122401 ttttctccca gtctgtgatt gtcttttcat tctcctagca gtcttttgaa gagcagatgc
122461 tcttaatttt ataaagttca ctttatgttt ttttttattt tgtggattgg acatttggtg
122521 ttatatctaa aaaaaaatct gcctaaccca tagccttatg tgttttcttc tagaagtttt
122581 atagttttag gttttacatt tgggtctatg gtccattttg agttaatttt tgtatatggt
122641 ataagatgca gattgaagat ttttttttaa tatgtggaaa actgattgtt ccagcactat
122701 ttattgaaaa gactaatttc tccattaaat tgtctttgta cctttgttgt gaatcatttg
122761 cctatatatg tgtgagtcta tttctgaatt ctcttttctg tttcagtgat tcatctatgt
122821 tgatgccaat gctacactgt cctgataact gtagttaata aagtcttaaa gttaggtagt
122881 aaaagtctgc tattttcttc cttttcaagg ttgcattttc ctattccaag taatttgcat
122941 taatcaaaaa gtacatggaa aatgtgtatt ataacaaaag gtatggaatt ttaaaatttt
123001 caaaattctt ttgcatcaaa gtaaatgtgt actaacttgt tataatatgt gtgaacagga
123061 gctagtttga ggcactaaaa aggataaatc atcaatttaa aaacagcccc tatcagagca
123121 acatgaattc tgataaaatt gaagcaagaa caaatatcaa atttatcgtg aagcgtgggt
123181 gaaaaaaggg tgaaatcatt gatgctttat gaaaaattta tggggaaaat gtcacattat
123241 caaacagata actcatttta agaacagatg atatgatgtt gaagtcgaag cccacagcag
123301 cagaccatcc acattaattg ataggaaaaa aattcatctt gtttgtgtct taattgaaga
123361 gcactgatga ttaacagcac aagtaatagc cacaaccata tacacctcaa ctggctcagc
123421 ttacacaatt ctgactgaaa aattaaaatt ggccaaagtt tccactctat gggtgccaaa
123481 accattgtgc ccagatcagc tgcaggcaag agtagagctt ttgacggaaa ttttaaacaa
123541 gtgggatcaa ggtccagaaa gatttcttaa aagaacatta ataggagatg aaacatggat
123601 tatcagtatg accctgaagc caaagcacaa tcaaaacaat ggctaccaag aggtggaagt
123661 ggcccagtca aaataagagc agaccagtca agagcaaaga tcatggcaac agtttttgg
123721 atgttcaagg cacttttctt gactttctgg agggtcaaag aacaataata tctctcatta
123781 tgggagtgtt ttgagaaagt caaagcgtta gcagaaaaac gtctggcaaa gcctcaccac
123841 agagtccttc tccaccatga caatgttcct gttcattgtc acgaaacaag ctctcacgaa
123901 ataaagctct aattttgcaa gaatttcaat gggaaatcat taggcagcca ccttacagtc
123961 ttgatttggt ttcttctgac tttttgtttt ctaatcttaa aataatcttt aaggggcacc
124021 catttttcct tagttaataa tgtaaaaaag actgcattga cataattcaa ttcccagggt
124081 cttcatttct taaggaatgg actatttggc cggtatcatt gcttacaaaa gtgtcttaaa
124141 cttgatggag cttatattga gaaataaagt ttatattttt cattttaatc ttttaattat
124201 atttttccac aaaatttttg aagtcctctc acatatataa atttttggaa tcagcttatc
124261 aagtcatgta catttactgc tatatcacac tctgttttgt gaatattcca aaattatagt
124321 ctatgattta tattgttgtg actattgcat aatttacata tcaatgtact gatgatcata
124381 ttttgcaata tttttgttat tgttattcta ttatgaacag tgctactaat ataaatagta
124441 tttgccacct catacacagg tgcaagttta ttgtaatagt cttagtatta aatttctga
124501 gtcatagttt agcaaaagct caacttttca caggatattg ctggattgtt ctccaaagta
124561 gctaagttat actcctatta gccgtatatg agagattcta atcatcttca ttcttcaaca
124621 tttactattt tcagacttac taattttgcc agaaaaaata catgacaatt catacccgta
124681 cctgtagtgg tcttgatttc catttactca gaagacattg gacttttctt cttatgttta
124741 tgggctatat tttcccttct tttatgatat tactgttcat gtttttgctc atcttattgg
124801 gttatgggcc attttatttt atttttattt tttatttatt tatttttttt tgagatggag
124861 tctcgccccg tcctgcagac tggagtgcag tggcactatc tcgactcact gcaacctcca
124921 atgggccatt ttatatccta acatatgaag tatctgttca aatcttttac catttttttc
124981 ctgtaggtgt gcttgtcctt ttcttgttat aataaaacag ttctttattt ttgatactaa
125041 tcttttagaa agtgttctgt tgggctgggc atggtggttc atgcctgtaa tcccactctg
125101 ggaggctgag gtgggtggat tacctgaggt caggagttcg agaccaacct ggccaacatg
125161 gtgaaactcc atctctactg aaaatacaaa aattagcccg gtgtggtggc gggtgcctgt
125221 aatcccagct actcgggagg ctgaggcagg aaaattgctt gaacccagga ggtggaggtt
125281 gcagtgagct gagagcatgc cactgcactc tagcctgggt gacagagtga gactccatct
125341 caaaaaaaaa aaaaaaaaag agagagaaaa gaaaatgctc tgtcagtggc aaatatctct
125401 tacttttttt ttagttttct ttgaaacctc ttttaatgaa tagaaatttt tttattttaa
125461 tgtagtttaa ttcatctatt tttctttttt aattgtcttc agtttcttta gtcttgtttt

125521 cataatcttc tggatatgag tcttgcaaaa catcttttgt taattttctc atggtttttg
125581 ttataatagt atttagagtt attttatata tttatggtta atataaatca aattgcatgt
125641 tgttcttgct ctaaattaca gattcaataa tttgcaacaa aaccttatct ctttgtaagt
125701 ccttaagtta attaagtact ctggatatct tcacatataa atacttgtat cagctgtctt
125761 ctctcttact actccaatta aaatccagta tctagaatag ggaaaaacca ggtgaacatc
125821 acccttacag agatttattt taagacataa tttagggtgt gctccatact gccttggcta
125881 tgtactagtc tatatattct agaagggtca tatatgtgca caaattgatt tgtgtagcca
125941 aaagctgctc cagtataaat gtgccaccag gatccatttc aaaacattgc ttcctcttgc
126001 caaggttagt gcagctatgc ttgctatcac tcaagtaacc agagaatgcc agcgtccgta
126061 ctctcaaaaa gacagccttt tctgcaccat tacaattggt gtcttttgtc cctttctttt
126121 cactgatgga aatctttttt atcattccaa tcaaagatca ctaccttcct aggagaagtt
126181 tcaatagaaa gaaaaaaatg ttatacttcc tcagaaaaat agcactttct tcatatagca
126241 agttttactt tcagaaaagt tgagaaaata aatactatgc cccaggtttt cccgttattg
126301 gagattgatt gctatgttga atgtgctata gctttgctgc ttaaacccaa acgccaagtg
126361 caggagtatc ttctttacta acgcatttaa tttaccaagc acacttagtg gctggtattt
126421 cttacctttc taagggtgca ctcacttaca ctgccctcaa tatatgtaat taggataaaa
126481 ccatagaatg gaaggcatta aaaatgaggc aggaaagcac aggcagagtg ggatgaagcc
126541 agctttgctc tgaataggac ttatatgatc agtttagttt tattccaaac atgaacccca
126601 aattcaaccc ctttgacctg agtactccgt cccttaaatg gcaaactaaa cattcgtgaa
126661 aatttaatgg tgaggaaatg agtaccttct taagtttcag atggtgaaat ggcaccaggg
126721 gagcactttc agtggcaggc agaagtgtgt ggatgtgcta gatcaaacct gaaatatttg
126781 ttccttcatc ctgcagtccc tctccaaact agccagccac tgagaccttc tgacaggaca
126841 cccccaggat gtcacccaaa aaaggtattt acaaaatcaa gactgtctgt aggacactat
126901 gtctttctga gaggtataaa ggtatttgaa aactttggaa agtttgtttt acaattttg
126961 ttccctcttt tggggctttc cctttttcat ttttttctgt gggtagtttc cctttgtttc
127021 ctgagaaatt gagaagggtt taatatctct ccaccttctt ctcttttatg gatgaatgat
127081 ttcaccaact ggtaagtgct aaaactgacc tcgctttcac tcagaaagac aatggaggaa
127141 aggaaaatgc actgagattt cagcatagca actcaaatgc gggctttatt ccacagcagt
127201 gacctattgt cagattggtg tgtgagacag tttttgaaag ggatgtgttt ctgcttccgt
127261 ttatgagtta agcaaagttc taaattaagt tgagaagagg aagcaggtaa agtcagggat
127321 agcagagaaa ggagacatca aaccctattg ttgtaggata tttatgtgaa ggaatgagca
127381 acgtgataaa acacattttt gcttgctgtg acttgccata gggtgcattt tcagtactgt
127441 gtggataatg tgtggcatgt tcattcatca gtcattctgt gacatttatg tagcttacac
127501 ttggatctat tttcaacaac atttcagttc cctaaagaca aactggcaga attttcaaat
127561 aggaaaaacc tggacacaca catttgcttt tctgtataga ctgtctagtc cagataatct
127621 tgctatcagt cattttgtta gcttactcat ttaatcttag aatggaaagt ggaaacagag
127681 acctcattta gagataatct agtaaagcca tccagcttca ggctcatatc ttgggaatct
127741 gagcagtacc ctatttaata tgctcatttt attaacttta aacacctgga gaacatcatt
127801 attgagaagg tcattgcttt tgaaaaaaca aaaacaaaac aaccacttct tatgaggctc
127861 tacacatctt tccaaagaag ctattggaaa tggcccaaac tttcatatta actcttaaca
127921 gggtggcgtg gtttttcatt agtaagaaca ggattcaaat cttatggaag atggtagatt
127981 tcaacacctt agcatttttc tcaaccctgg gatcttcaag ttcctcaggc tatttaggta
128041 gttatagtgc tggcaaactt tttaagtgcc cagtagtttt tacaaaaact tgtaataaat
128101 ctgctcaggc tatcttaatt gccaaccatc tcattaagca agtcaaatta gaatggatta
128161 ttcctgctgt ttattagtgc tgcatattat ttatcattta ttaaccttat tgtgctcaca
128221 tagcaggtat tttaaagttt aacagcttta ttttaattat gaatgtaata tatgctgttg
128281 cagaaaatat gtaaacttta gagaaaatgt actaaatgat gcactgatga aaatacagaa
128341 tcccaccaca aaaatattat tatcaacatt tgggtattct cttccaaact ttttatatga
128401 gagttatgtt tggtttatac ataataaaat gggtaaaaat caataaatag aaagcaaatg
128461 tttttaaggc agtacttggg cttcataaga atggaaaaaa atgatagact tgcctggtgt
128521 ggtttgcata atgtcacaga atcataacaa tagctccatt attgagtgtc tattatgtgc
128581 caggttctgt acaatgttca gtgatgcgtc actgtgatga ctagaggtaa aggaaagata
128641 agtgatgcct tactatgatg acttccaaca agctatgaag ggacagaaca agcctttttt
128701 tttaactttt attttaggct tgggagtacg tatgaaagtt tgttacatag gtaaacatgt
128761 gtcacagggg tttgttgtgc atattatttc acacccaggt acctatgtcc agaacccaat
128821 agtttagttt tcttttctcc tcccctccct cattctatcc tcccccctca catagacccc
128881 agggtctgtt gtttccttct ttgttttcat aagttcttat cattatcata cttataaatg

128941 agaacgtggt atttggttgt ctgtttctgc attagttggc taaggataat gtcttccagc
129001 tccatccacg ttcccacata agatataatc tcgttctttt tttatggctg catggtattt
129061 catggtgtat tttatatata tagatatata gatatatata taaatatata tagatatata
129121 tataaatata tataggtata tatatgtcac atatatatat cacatataca aggatatgtc
129181 ctctagctcc atccatgttc ccacataaga cataatctcg ttcttgtttt atggctgcgt
129241 ggtatttcat ggtgtatttt atatatatat atctatataa aaatatatag atatatatat
129301 cacatatata tagcacatgt atctatatca catatatata tatatcacat tttctttatc
129361 cagttgggac agaacaagtc aactggcaag tccattgctc agcttgttga aatactctgc
129421 acagaatgca aggaaaaact gttttcatga ggtagcccat gaagcgatga agcaaggcaa
129481 atttatctgc ctggataacc tcctgtatgc tgttttcact ccatggagag tcaacacaac
129541 agacttctga gttgtattat cgagctcctt caacagggca ttgctgtaag atcctagatc
129601 ccacactcca tggtgtgatg ttttagccaa gtctggaagt agcacaggcc ctaaatctc
129661 atgattgcag gttgtcaact ctgctgtagg cttgccactt caagggcaag tggttggttg
129721 gggctgggat ttgggcagag ccatagaaag aaagggaaca ttcaaggaaa tttgatgatg
129781 tgcatgatgt ctctgtctga tacaaaaaga aaaaaaaact ctaaaaacat ttgatacata
129841 gaaatactag tatttggtga gattgaatgt gggaagatga gggataagaa ggtattaaga
129901 atgactccca agtttctgac ttggtcagtt gagtagaagg gaaattttta tttttatttt
129961 tatttttaaa atttcagtaa gtttttaggg aacaggtggt gtttggttac atgaataagt
130021 tctttagtgg tgatttctga gattttggtg cacctatcac ctaagcaggg tacactatgc
130081 ccaatgtgta gtctcttatc cctcaccccc catcctttcc cttgactccc caaagtccat
130141 tgtaacattc ttatgccttt gtgtcctcat aatttagctc ccaattgtga gtgagaacat
130201 atgatgtttg gttttccact cccgagttgc ttcacttaca ataatagtct ccaattcatg
130261 ccaacatcta ttattttttg attttttgat catgaccatt gttgtaggag ggaggtggta
130321 tcccattatg gttttgattt gtatttccct gataattagt gatgctgaac atctttccat
130381 atacttgttg gccatttgta tatctgagta gaaggtaatt ctaataaata aaacaggaag
130441 gcagcagaaa gaggtttgtt tgggatatgg cttgattatt tgcaagatat ttgctgtagc
130501 ttaggagaaa ggtggatgtt ggtgatactg attttgtcat tatctgtgag ttaaagtttc
130561 taaaatcacg tggcttccta gggaaagcat atagaatgac aagggaagat ggctagatgg
130621 ctagtaatgg caccctgaac aacttcagca tttagaggtg ggtggaggag gataagccca
130681 tggaagaaac agaaaggatg ggccagcgag atgagaggag aagctaaggg agtagacagt
130741 taccagaggg aaggtgggat cagttaagaa ccttagtatg ttgcaaagaa ataacctgca
130801 atgcggacca acccggggaa cgtctgtaaa aatgatgaca attcttaatt gtaatttaaa
130861 atgtaattgt attgagagct ctctctgtct ttgtctcatc ataatttgaa gcagattaac
130921 tacatattag ctagaaaaag ccctttgaaa ttttccttca atgtatcaat agattctatg
130981 actttttgta agtagaaact gcctattggt aaaatagcac ttaattgaga taatgcttgt
131041 gaaagccatt tctaaacttt gaattgacat acaaaataaa ggtggcaatc atcaccatgt
131101 tgctatactg tctttggcca tataaaattt ttttcaacaa tacaaagata agccttgtac
131161 aaattcttca aaattctaaa tttgtggtac ttgaataatg ttttactata tataaagatg
131221 aatagatagc atttcctaca ataaataaat aatttatata tcaatgtgtt gggattataa
131281 attctcatag aaattcactt tattaattca ttcattcatc tgatcacagt tcattcagca
131341 aataatttgt tttgttcttt ttaagtgctc tgggaagaag gtattgtgac agagtccctt
131401 ctctcaaagc gtttatagat tcatgtgttt ggggaaagta cagaacaatt attgaaataa
131461 aaccagagtc aggcacattg ctatgccagc tataaagagc ctatgaaaat taggaacatt
131521 gacacatttg ttcctaacct ggtttgtgct ttcaggattc ctctgaagtg gcagcactcc
131581 ttgggtttga aatgttcgtc agtaatgact gatggacatc aacaggtgaa tagacacaca
131641 aaaaacctat gaaacttaca taccataggt tactattcaa caataaaaag aaaaactgtt
131701 gatacatcta acaatgtaga tgaatctcaa aagcattatg ctattccttt ggctgataaa
131761 atggtgacac tcctttattt ctctttcttt catcccactt ttaaaatcag ccttagtata
131821 ctttaggcac aatatactat atacatttaa aatgacaatt tgacaagttt tgacaaatgt
131881 atatacatta taaaaatcat gataaagatt tccgccgcct caaaatgttt cctcatgcct
131941 tcttggaata aatccacaca ttccttcctt ctctacaccc caccacccat atactctacc
132001 tctagaccca agcaattgct agtctgcttt ttgtcagcgt aaattagttt gcattttcta
132061 gaatttcata ttaatggaaa tgtacagtaa gtactctttt gtgcctgact tcttacattt
132121 agtatagtgc ttttaagatt tatccaaatt gttgggtgta tcaataattt gtttcttttt
132181 attgctgagt agtattctat tgtatacgtg gttcacagtt tatccattca ccagttaatg
132241 gacattccag ttgttttata tttggggctg ttatgaataa agctgatgtg aacatttgaa
132301 tagaagtctg ttgtgaatgt ttggattgga attgctgggt tgtacagtaa gtgtttgatt

132361 aacttcataa gaaactgcca aatgattttc caaagtggct acatataatt ttgcattttc
132421 accagcagtg tatgagattt ccagttgatc ctcatcctca ttcaaaacct ggaattaaga
132481 gtgaatcttt taaattttag ccttttgaga aaatgtgtag tggtacttca ttgtagtttt
132541 agttcgcact tctctaatga ctaatgatgt ttaacatgct tttatatctt cttttgtctc
132601 catctattct ttgttcttca ttctcttttc ttcctttaga gtaagtattt tttattttat
132661 tccattttca ctattggtat attatctata aattctttgt tgttgttcca ttttagtggg
132721 tttacaatat aaatctttaa cttatcacag tctgtcatca aataatggta taccccttac
132781 atatgtttta ggaaccttac agtggtaaaa atatcagttc cccatcctgt acttgttgct
132841 attgttttca tgcattttat tctgtatata ttataaactc tacaatctat cattattact
132901 ttttgtttaa atattaaatt ctttaaaaga gatttttaaa aataagaaaa tagtctttca
132961 tattttctca tgtatttgcc atttttattg tttttttttt cttccatatt tgagatccaa
133021 atttctatct ggtatacttt tccttctgcc tgaataacta cctttagtat tttttgtag
133081 tacagatctg ttggcaatga ttatctgaac ttctgtatgt ctgaaaaagc ctttagctaa
133141 tttttgaaag ttttttgatg gacatatgat atggtttggc cctgtgtctc catctaaatc
133201 ttatcttgaa ttgtaatccc catgatcccc atgtgtcaag ggtgggacca ggtggaggta
133261 attggatcat gagggccgtt cgccccatgc tgttctccta ataatgagtg agtctcatga
133321 gatccgatgg ttttataagt gtctgacatt tcccctgctt gcacttctcc tttctgccac
133381 cttgtgaagc aggtgcctgt ttccccttca ccttctgcca tgattgtaag tttcatgagg
133441 tctccccagc cttgctgaac tgttagtcaa ttaaacctct ttcctttata aattacccag
133501 tctcaggtat ttcttcatag cagcatgaga atggactaac acggtacata caattacagg
133561 ttgctagtcc cttttttccc ttcagtgctt taaggatttc actcttccat agtttctgat
133621 gagtagtctg accattccta actttgtgcc attattttct catttttttc tgactctccc
133681 ttctgggact ccaattacaa atatcccaga cttcctaact ttggcaggtc aagagatcga
133741 gaccatcctg gtcaacatgg tgaaacccag tctctactaa atatacaaaa aattagccag
133801 gcatggtggt gggcgcctgt agtcccagct acttgtaagg ctgaggcagg agaatggctt
133861 gaacccggga gggggagcct gcagtgagct gagatcgcgc cactgcactc cagcccgggc
133921 gacagaccga gaatccgtat caaaaaaaaa aaatcattca aaagattact gatgcttgtt
133981 tcattttttt ttaatttttc aactggtaat taatttatta aaatagttga cttatacgtc
134041 tacaatggtg acttccatct caactcctgg ctcaatactg atggaagtaa tctgcttaac
134101 aatctcagaa ggactgagca agtcaatgag tcgcttgtgg attctcatct ggaaatgatc
134161 ccatggctta gaaccttcac cacatggagt tttttcttgt agtgattctc aaagtcttag
134221 taggcatttg aactggtctt ttcactttga gattcttttc ctttgctcct ctgatcaagt
134281 cagcacacac cttcgccagg gattttacat tgcagcttgt tagagtgatt cgaattcggt
134341 gaattgccat cttcggctcc gtgggtgttt ttccggtatc cttaaaagcc atcactgctg
134401 cgcggcttcc tgacctactt gttccggcaa gagtgaacag tggtgagtca ggagcaggtg
134461 cgggcggatc agagctccac aacacctacg accacctctt cttcaaagag gtgtttcatg
134521 ttttaaaatc ttaatttcct tctgtttcat tttggataaa atctgtcttc aagtttggtg
134581 cctttttttt tgggccaata tctaatctaa agttcatcct atccagtatg tttctcattt
134641 caaatattgt attttctatc tgcagagttt gatttgtatt tgaaaacgtt ttttaatctt
134701 tctcattgtc ttaaacattt ggagtatact tataataatt gttttactgt tgttatctgt
134761 tacttctatc atctatgaaa ttactatatc tgtttctatt gattgatttg attactattc
134821 tggttgggag gcaatatttt ccctacttct ttgcttactt ggtaattttt ttatcaatga
134881 aaatttataa aagtgaactt catttacaat tggatataat gaattttaca tttttgtgtc
134941 ctggattttt ctgtattcct ttaaatattg ttggatttct tttttttccc ccctccggga
135001 tgcagataag tttcttggaa ttagctggat gcttttgaga tttgctttta agatttgtta
135061 gggtgaattc agagcagggc tactttgatc taggaaatag ccctctgaag ggtctcatgt
135121 tctgtgagat aggaggtctt tctcctctga ctggttggta gcctaagcta ttcctgattc
135181 ttgtaaggtc tggacactgt tctgcatact cttttctgat agttctttcc ctggccttag
135241 tagttaccac acatctatgc acatagcggt attcagccaa agtttgaaag gattcaaaga
135301 tctccagagc tctctctgta tcactgtttc tgttattcta tcccacaaat tctaagtgtg
135361 tgaaactccc tgaaatcaga actatgtctc ctcaactcag tgagaccaca agccactgtt
135421 tgggtttccc ctgcccatgt tgcagcctag aaactctcct caggcaaaaa gatcaggcaa
135481 catcggggtc atctcatttc cttccctttc agagatcact gtaattgcct attgaacaat
135541 gcctgaagaa agatgtttca tatattttgt ctggctttct agttgcttaa ggtaagaagg
135601 taaatgcatt cctagttact gcattgtggc tgggagcaga attccaatac tgtttttttt
135661 ataatatgct tttacttaat tatttgagga ttccctttag ttatcataat tttacatgat
135721 gtaaacaggt aaatgagaat atcttcaaaa tatatttttc agttttgggg tggttctctc

135781 agttctggtt tttgccaaat ataatagatt tttagtatct gagttccata tgacaaaatt
135841 tataaatctt gggtgtatcc atatggtatg ttataatcgg aaaagataga tgaaattccg
135901 cttttgtcta atgtctttat aggtcttgga gaaacttgaa gaagggttgt aaagtccata
135961 actttacaat gagacaaaat taaaagaatc caagccaaaa ttgtcctaga caatgaaaag
136021 tatcgctatg cagaagccaa atctcttaag ctcctccacc atatcagtca tgacacagtc
136081 tatagtttaa caattgtttc aatttggaaa ttaattttta gtactaattt aaatatttca
136141 tattagtata tgagaattct tccttctgtt caggctgtgg tatggggaaa agtcagttcc
136201 catcaatgca gattcatatc taccagcaca gtgattaaat tatcctcata tcctcttgtg
136261 tttgaacaag ctgtgtactt ttaattttaa tggatttaac tcaatattca atatatgttg
136321 attactgaat acaacaaaaa gtgtaataga cactccttga gaagctcata atctatttgg
136381 gcagatggaa caaatactta gaaaaattga aaggtagttt aggaccacag taaaaaaaaa
136441 ttgggggtgc aggatataaa gtatcaattg aattgtagaa gcaatatata ctgtgtattt
136501 tttcacctgg gtgagagggt gagactccgt ctaaaaaaaa aaaaaaaact atggttgttc
136561 accataacta aacatttaat ctctttcagt ctatcttact catctacaaa atggagataa
136621 tacctgtttt gcagtgcagg ccctgacata cgggtagagt tccataaatc ctagcttcta
136681 ttccttaact cttttaactc ttttcttcct tccttctttc tagctaattt ttaactctct
136741 atcctagaca acttttgcta aacgtgtata acatgtagta cctacgcata ggaatatgtt
136801 ttatagaaca gaattattga aaacagcttc ttacatttct gttatttttt caaaagcaag
136861 aattcatgta ttccttctga gccaagtaca tgcaataaaa agtaagagag attcagaaat
136921 tctgatgatt attctcccaa gaagagtgct tgctagagtg agggtcttgc agacacagaa
136981 ggagctgatg gggtgaactg ctggggtgtt acaaaaggcc ttcgggaaaa tacgtaatct
137041 aagtcaataa atcatagagt gaagattccc tcagggaaaa agggagtggt tctgaagaac
137101 ttacaaattt ttccaggcaa tagccacaga tgctagcaca agggcattat ggccacatag
137161 tgtgagcaaa agtgcttaac tttttgccct tcttctaata tttactctat tagcccagac
137221 aacatgagat ccagaagcct catgatgagt ggagaggaaa tataaatcag aagaaatggt
137281 caaaaagatg cctatattca cttccgcact taggtctcca agcctcagtt cctggcatga
137341 gtaggtagag gagaggagct tggaatagga tgtgagatta cagttttaat atagactgga
137401 ctggatattt gaatacctga aaatgaatct gaattctcaa aatgaggtgg ttcctgcaac
137461 tgaaaataac aagaaagcta caggatcttc tgccatgtta tccaggggtg gaaaagtgaa
137521 attcagtgga gccaggttaa agatggagtt tgggccggga gtggtggttc atgcctataa
137581 tcccagaact ttgagaggcc aaggtgggca gattacttga ggccaggata tcaagaccag
137641 cctggccaac atggtgaaac cccatctcta ctaaaaataa aaaattagcc aggtgtggtg
137701 gcatgtgcct gtagtcccag ctactggggt gggtgaggca tgagaatcac ttgaacctgg
137761 aagggggagc ctgtggtgag tcaagattgc accactgcac tcccgcctgg gcaacagagc
137821 aagaccctgt gtgaccttgt gtccaaaaac agacaaacaa acaaatacca tgtggagttt
137881 gtttactctt taaacaaata tccttcatgt gttgaactga actataatta tagttagtag
137941 ggcacatagc aggttttaat aagatatcta gttcttcctc ttaaggtaac tcgttagaac
138001 ttttatgaga aagaactaaa gaatttctaa tggggcagct aatccactct tttgtgcact
138061 ctgttctgct gatgatctga tgttagtcgg ggtttctgtt tgagattcct gagccttaac
138121 tgctgatgac agagttcaaa atcattgctt tctaactttg agaatgactg cctaatatga
138181 catcattttc tacccctgag tgctaatagt ctaccttatt cctcctgcct gtccttctcc
138241 ttgaggagga ggatcatcaa tgcactgtgt ggattgaagt ggcttcatac ccttttgatg
138301 ttaatgttct cctttgctta aagtgtgaac tgaatatgga tccctcattt atgaagcacc
138361 atgacacttc ctgggtaatg aacaattaaa cagacttctc agcaatagtg actatttttc
138421 tgatttttca cttcatggac aatattgtat gcaagttaaa tttgcaacta gcttgaggct
138481 tcacacatgg tgagtttgag aaattgaatt ctctacctca aatgtatttc cttttccttg
138541 aactctacat tcaggtcagc tgtccagttc ttaaacaaca ggttttggag aggcaatgag
138601 taataaaata gcattttctc cttcaattct atctgcattt gaaaaaaata accttttatt
138661 tttgattcaa agaagagagg agttttcttg tctttctaca tctgttccaa ctcctccaaa
138721 tgcacaaaaa ttttttcaga gcattccaga gtttctcaga ctgcacatag aagattttgc
138781 atatagatac atcttggcca taataacctt ctactgaatt agtagaaaat agatgtattc
138841 taggttttca tatctttatg gcaatatgaa tagacaaggc tcttcttgca aaaatatctc
138901 ctgaactgtg cgggagatgc ttggaagacc cagtcaaaat tacgaagtat cccaagtttg
138961 tatcctcctg tggcttcccc gtattacaat aatgtcattc tgatccaggt tccagtactc
139021 tcaagccctt gtccttccct tttcatttta gcttgcattc atccaagtat ttggatggaa
139081 attatcaata ccttatacga gaacgtatgg gaggctgagg cgggcggatc acgaggtcag
139141 gagatcgaga ccatcctggc taacacagtt aaaccctgtc tctaccagaa aaatacaaaa

139201 aattagccat gcatggtggc acgcgcctgt agtcccagct acttgggagg ctgaggcagg
139261 agaatcgctt gaacctggga ggcagaggtt gcagtgagcc aagatcacgc caccgcactc
139321 cagcctgggt gacagagcga gactcatctc aaaaacaaac aaacaaaaag aatgtaaatg
139381 attgggagtt aaaattatgc ttttcagttt tcagccatgg atcatgtttt cttgccttgc
139441 ctgattccta gagtctcact ccatgatgtt tcctctttat tataaagatt agatggcttt
139501 gcattgctgc catgtggcat ttatacttct caagcagctg tgtcatctta ttctaccgtt
139561 taaacttagt tttactttaa gtccacatct aaactgtttg cctgcatgcc cagcactttt
139621 tccaccctct acattttatt tgactggagc aatcacctta gcctctctag gtctttgctt
139681 atcatcactc tgctcattct tcaagcctca cttgattact gcctcctctg tgttgctttc
139741 ctggatttcc tcagtaagac atagtttctc cctttcatac ttctgtggta ttaagtatgc
139801 accagttaca tgtgactcat ctccaagtgc tttggcatgg agatattatt tatgttcctt
139861 atatgactac tgaccacttt tttctagact aaatattgaa acacttggcc agataatttt
139921 gcttaatttt gagtttcctt ttatactttt taaaacaaat ttttgaaatt aaattacatt
139981 ttgttaggtt gcaaatctga aaaagtccaa ggatgaggtg agtttgttgt ggtcagttgg
140041 gtagttgcta aactcatagc tggtttgtgt ctgctgacca gcacattgtt agcctagaga
140101 aagtctacat ggggaaccag attatgatgc aagcgttgct tggagagccc ttgatcaaga
140161 gcaaaacagg ttggtagact cgagggatag ttcagctagg tctgatggaa cattccagtt
140221 ttgccaacag aaggaaaaac attggaaggg cttatcttcc ccaggatctg gatctgttcc
140281 actgggtaac atttaataag cactaacatg gtctcttctt tcacttgcca tttcctgtgc
140341 ttctttttcc aactactaca tctcctttga cacatttctt ttttcttttt cttttttttt
140401 ttttgagata gagtcttgct ctgtcaccca ggctggagta cagtggtacg atcttagctc
140461 accgcaacct ccacctcccc ggtccaagcg attctcctgc ctcagcctcc tgaattgctg
140521 ggatcacaga tgtgcgccac cacactcagc aatttttttt ttttttttgt atttttagta
140581 gaggcagggt ttcaccatgt tggccaggct ggtctcgaac tcctgacctc aggtaatcca
140641 ctggcctcgg cgtcctagag tgctggcatt acaggtgtga gccactgtgc ctggcctctt
140701 tttaagtgta tcaataccat cattttggct gcttattctg ccctgggagc cattctgtct
140761 ctttaggaag tttttgcgtg tatcagatct catcttttat attttgatgt ttaatagcat
140821 ttaaaggatt ttgttttgtg ttttttaaat gatttgaaaa caagtgtgct cactaattaa
140881 taaacagcag attcaaactt ttgttgcttt ttcaagaaaa aaatgaaaaa ttctgtaaca
140941 atatatctta gccataaaac acttgtaatc agcactcctc cagaaatttc aggatgtaag
141001 ttatcttaat gattatacta gaaaatcttg gaaagcaggg atatggctaa cttttataa
141061 catgtatccc tactgtcaca acaagagtta agacttaata aatacagtca tgtgttgctt
141121 aacaactggg atatattctt gtaaccaccc aaggggttca ttttgcctgc tacctggata
141181 cagccaattt atcaagaacc aggaattgca gtagagaaag ggtttaattc atacagagcc
141241 agctgaatgg gacactagag ttctattact gaagtcagtc tccctgaaaa ttcagagggt
141301 agggtttttc aaggatagtt tgaagggcca gggaatggtg ctgctgattg gttggagata
141361 caatcatagg gatgtggaaa atggtcctca tgcatttctc aagtccactc cttttcttag
141421 ttcctgctta gcggaggtgt tagaatttgc ttcttggtgg ggccacagga ctggttggtg
141481 ggtccaggtg gggccaccag ttgtcagaaa tgcaaaaacc tgaaaagaca tctcaaaaga
141541 ccaatcttag gttctgtaat agtgatgtta tttgcaggag taattgggga agttgcaaat
141601 cttgtggcta atttgttagt cctacaaagg cagcctagtc cccaggaaag aagggggttt
141661 atttaaggaa agggctgtta tcatctttgt ttcaaagtta aactataaac taagttcctc
141721 ccaaagttag ttggacctgt ccccaggaat ggacaagggc agcttggagg ttagaaacaa
141781 gatggaatct gttagtttag atctctttca ctgtcataat tttgtcactg ttaacgtttg
141841 caaaggtggt ttcactgtag actttataaa caccatatac ttagactata ctaagtttat
141901 taaaaatatt tttctttctt tagtaacaaa ttaaccttag cttactataa gttttttact
141961 ttttgaagtt ttttaatttt ttgaactttt gactcttgta ataacagcat aaaacacact
142021 ttgtacaact gtacaaacat attttcttta tatacttatt ctataagtgc tttctatttt
142081 cattttttcc ctttaaactt tttaatttaa atgaagacag aaacacaata ccctaggtct
142141 acacaggttc tggatcatca atatcattat ccaccacctc cacatcttgt cccactggaa
142201 gatcttcatg gacaataaca cgaagctctc gtctcctgtg ttaacaatgc cttcttctgg
142261 aatacctcct gaaagacctg cctgaggctg ttttatagtt aatttccttt tttacaagta
142321 gaaggagtac actctaaaat aacgattaag agtattgtac agtaaataaa tacagcagta
142381 acagagtcgg ttattatcat tatcaagtat tatgtactat acctaattgt gctacatttt
142441 tgtatgcctg gcagtgcagt aggtgtgttt caccaggatc accacaaaca tatgagtaat
142501 gtgttgatct atgacattac cagagctacc atgttgatat aaacaggaga cagggaaata
142561 ctgggtagaa gagggcagtt ccctggcaaa agccttactc ccaagcctgg aaacctgcag

142621 tcctaaatgg gaataggcat tcctgtttgt gcatccaaat gttgcctttt ggcctgccat
142681 gcccctctgt cctgtatcca tataaacccc aaatcccagg cttgatgagc agatgagcag
142741 acaaacagaa gagcagagga gcagaagagt ggtgctgcag agaaagagag aagagaagga
142801 gcatctgaat gtcaagagga gtttggatgg ggatggtcag agaggagatc agctgcagga
142861 cagccaaact ccaggggaag atcatctctc actccatccc ctttccagct ccccacccat
142921 cctgctgaga gccacctcca tcacccagta aaatcctcac attcaccatc cttcaagttc
142981 atgtgtgact tgattcttcc tggacactgg acaagaacct gggtaccaag agggcactga
143041 gctggttaac acttaagcca tctgcggatg gcggagctaa aagagcactg tagcacaccc
143101 actggggctt tgagagtcac aggcacacac ccctagatgc caccgtggga tgggaaccca
143161 aaagtgctcg ccctggctcc tgcacctatc tgtctacatg ctccctctcc tataacaggc
143221 ttgcacagca gtgactgcgc aacagatgag ccacacccct tccacatgtc tgcaaggggt
143281 atcagggaac tctccccttt caatgtcagt aggtaatagg aattttttag ctcctttata
143341 atcttatgat accacctttg tgtatgtggt ctgtcattga ctgaagtgtc gttatgtggt
143401 acgtgactgt atttattgag tggatggtca attgtatgag gtacttgaat aatgagtggt
143461 gtgaaccttg gttttatgac atcttctgag agctgctttc aggtgggtat aagaagagat
143521 atttctagtc taggtcccca ctccttttct tagttcctgc ttagtggagg tgttagaaaa
143581 aaaggttgga ctctggcact attttcctcc attggttata tggactcaaa gccataactt
143641 aaggaagaat gtctattttt agatttggtg actgatttag ataggttgct acagaaagtg
143701 gtacaaagat atttcacaca tagaatatca aatccagggt gctttagcac tgcattccaa
143761 tggcctggga tggatgggtg gaagccctgg caagctgaaa taaaccaggg caagctcctc
143821 tttggaattc acaagtctga caactggtga ctcaggctct agtcaggaga cctgaatgtt
143881 acgtctggca tcacgatacc ttagtttaca gagattaatc taacaggcct tcagttagtt
143941 tgctttattt taaaattctg cttagctttt tgttgcctac taaatttctc ctgatgtata
144001 tttagatgta ggtgaaggct gtagatgtat gcactaccaa caccagcttc tgggagggaa
144061 cattttccca ctcacaagca agtgaacttt gctttctcca tgaagaagaa tgacctgcat
144121 gatggctttc agaagtggag aacatggctc acagttccct ccacagttcc tccatggcct
144181 ttggttgcat cttctctgaa tggaggagct tgggttatgt gaaaagcaga gctaccagac
144241 tacataaatg ttttagtgag aaggcagcaa attaaaatat cagaaatttt gaaaggaaag
144301 gtttataata gttagattta aaacatactt tgaggccagg cagcgtggct tacgccagta
144361 atcccagcac tttgggaggc caaggcggga ggattgcttg agggcaggtg ttcgagacta
144421 gcctgcacta cataacaaga ccttgtcctg tctctattaa aaaaaaaaaa aggaaggaaa
144481 gggaagaaaa agtgctttaa tattcagagg catgagagat gcaatgagta atataggacc
144541 ttctctaagg acactggggg agcactctac agaggtggtt ctaatctagc ctggcaaatc
144601 tctgttccct tcatgccttg tacttccttc ttctttgtga ctttgctaat gttattcctt
144661 cctcttggac aaatttccta tactaaccaa aaggccccaa gacacagggg gaaatgtggt
144721 aaatctgaga ctggaaggaa attaccatct aatgtcactc ttgtacagta taactattgt
144781 taatattatt gctggggtca ctcatttatt catatcacaa acactgactg agtgcctatt
144841 atttgccaag catgggcaat aaagagttga agaccaacaa agcccctgcc ttcccagaag
144901 cttatttgct tgtggggaaa ataaacagtg gatcaattaa caaataagta gataacataa
144961 tgtcacggcc aggcgtggtg gcctcatgct ggtaatccca gcactttgga agactgaggt
145021 gggaggatca cttaggccca ggagtttgag accagcctgg gcagcatagg gaaatcccat
145081 ctctaccaaa acaaaacaaa acaaaacaaa acaaaacaaa acaaacaata aacgaaacac
145141 atggtggcgt gtgcctgtag tcccagctac tcaggaggct gaggtgggag gatcacttga
145201 gcccaggaca cagaggttgc agtgagccaa catctcccca ctgcactcca gcccgggtga
145261 cagggtgaga ccctgtctca aaacaaacaa acaaacaaaa caaccaaaaa ccaaaaaaag
145321 taatgtcaga tggtgactgt tttgaggaaa ataaagcagg gtaggggaac tagtgataac
145381 tggtagattt gggtttctcc atctctggct gcttttgcat cctcagtaag gtgagagtgg
145441 caaaggaaat tccaggttga ctttcgggtt cagtgaattt tctaccccgt gtctcttcac
145501 tgccctttcc ctccccagaa cctctaaaat ctgatcctta gattctgcaa tgtaagtcag
145561 cttccttctt cctggttgcc agctcactac attcttattc ctggataaac caaaaatctc
145621 ttccttgttt tcatattttc aaaaagatgt agaaatctct cacctgctca tgttccttcc
145681 tttttgtctt gttggtatat atacctttta caattctttc actaccaaat gtagttggat
145741 attgggatgt agtcacaaca aacacataaa tacaattcac catttttaaa tggacttatt
145801 catttagtct gcttaaaatt gagataataa cacacgcacc agttgggttt ctctgagaac
145861 tctgggcatt tttaatcctg gcaattttta aattgcttag cagtaaaaca gtcagacaac
145921 agcacctatt gttataaatc cctgcctctt cttacctcaa tgaggctatc ctttctctct
145981 atcaattctg tcagcagaat taagaagagc cattgatttc cagtttgttc agcgtttttc

146041 ttcttaggat gggagtgatg acttctaacc tctttacatc tagagactca atttaagtct
146101 acttctttca ttcagccttc tctcttcatt ttcagctgcc tggaatgtct tctttctctc
146161 agttcctatc tttgacatta aaaatcacag tacttagtta tattctacct cacaccaatc
146221 attttacttg ttaatatctt ttttcctagt tagattgtga cattttggaa taaaagaaaa
146281 atatcctttt ttactttata gctctccaga tttcattaga ttagtataga ttcgagtgcc
146341 ttttatatat caggcaatgc ctagtgtata atatttttac ttatgggttt tagcatttaa
146401 cttatttaat gatcacaaca ttcctatgat gttatttgtt ttcataattt gttgaaatga
146461 aagctgatgg tcagaaaggt taaaaagttg tctggtcata atttttgagg aggcagagct
146521 aggttttgaa cgcaggtctg tctgattctg agaataatcc tgtgctcctt cagctgaacc
146581 acacagcccc tcttagtgct ttgtctccat agacactaag tgtattcttg ttgattgagt
146641 gaattaacga gtgaattatc tggagctctt tctgcttctg ttttcttctc tgtacaatga
146701 ggatcatcac atgaacctga tctaagaata gaatagtcca actgagttcc cttaatttgg
146761 taaccaagag agagtagagt taattaacta gaaccctttg ccatcacttc tctctaaact
146821 tttcatcatt ttcctctaaa agtccggtcc tgagctctat tacaagatag aacattttag
146881 cctctatcga agagagcact attaatattt tatggcattg gctccttttt tcctaatact
146941 gtgggattaa aataatgttt actaaattct ggatgttcga ttcctgtttt ttcatgatat
147001 tttataatcc aggccttctt catagagaca cagggctatg ttttataact gccagggtca
147061 agcaaagaac agcttgtgtg agatacttaa agaaatagct aagcagtaac ttcagacctt
147121 ctgtatattt tcctcaaaat tttctctcag tttttgtctc cctacaactc attttagaa
147181 aacaaaaaca taacacaatt ttgccctttt aaaagataat acagctgggt gcagtggctc
147241 acgcttgtaa tcccagcact ttgggaggct gaggtggggc agatcacctg aggtcaggag
147301 ttcaagacca gcctggccaa tatggtgaaa ccctgtctct actaaaaata caaaaattag
147361 ctgggtgagg tggcggcagg cgcctgtaat cccagatacc cgggaagctg aggcaggagg
147421 atcgcttgaa cctgggaggc ggaggtagca gtgagctgga attgcaccac tgcactccag
147481 cctgggcgaa agagtaagac tctatctaaa gaaaaaaaaa tgtactataa taatttacaa
147541 agtacagaaa atatctaaag gaaaagaaat aacatctcac aacctcatca tctagatatg
147601 actgcaaaaa ttttggcata tttcatttca gcttttaatt aatttatttt tgcagaagac
147661 atttatttca gatattctaa ctttataatc tgtagaattt taatgatatt tttgtatgac
147721 atctatcata aacatttctg catgtcattg aaatatttat aaatgttatt tttatttaaa
147781 caatgcataa ttgatataac ccttctctta aaattggtga tttcacatac ctggggatga
147841 gccgatgtga aacaaaagag aaagggtaag agatagctag tctcttgact catttttttt
147901 tttccatatg acttgaaaaa aagaacttat cttagagacc tctagaatag ttagcacaaa
147961 tagatttctc aatgtggtta agatgaaata cagatactga atttactctt ttatgtgaaa
148021 taaataaaaa ctggataaga tatatatagc aacagttttc aagacattat acctcagaca
148081 acaaaggata atgacctctg agagagggaa aacaaattag gtgagcctgt gattgcccca
148141 gtttactgcc tggagagaga ttccaggcca gggtgcagag agaagggttg cgaatggaaa
148201 ctattgctct ttctgagtta aggagagaga cctgaaagtc tgcaggtaca aagatagctg
148261 caattcatga gaaagagttc cagagaaaag aaaactgaac agagagtgag ctctggattt
148321 ctgtagtgtt cctgctgact cttctgctga atcatgatca gtgcatgtgt gtggctggag
148381 aaagaacctc tgaaaaggtt tagaggacaa aatccttaga atcacatagg cctgggaata
148441 gctcctcttt ccaccagaaa gagtataaaa accatgaaat tcatgggaca ttaggtagag
148501 tacttagaag ggttttgctt cagcagtgag aaaaaattag tcctagacca aatgttgctc
148561 tgatcctaac aaaacttaaa aaatgagacc ctaaaggata aaactgtttc caggtaactt
148621 aaatatatac cagaacaaag ccgaagacta taggaatatg aaaatatcta gcagtcagta
148681 aggtaaaatt cataatgtct aatatgcaac caaatattac catgaatata aagaagcatg
148741 aaaataaata cccataattc caagaaaaat caattataca taattgcccc atcaatgaca
148801 cagatgatgt aattagtaaa caaagagcta attataactg ccaactctag ccattacaca
148861 tgttcaagtg gctagaggaa agatcaaatc acgacacaga agaaataaaa agacaataaa
148921 atatgtacag atgaaaatta caatatctga gatgaagcat aagcgagatg ggattcgcag
148981 cagattagat atagaagaag gattaatgaa catcaaaaca tgtatgacaa cagtagtata
149041 aagtctgtaa gaaaagaaat ggaaacatac tggtataagg ttttacacta tgtatgaaat
149101 ggtgtagtat cacttgaaga gagactgtga ctaaagacca ctgtaacctc ttagggaaag
149161 aaaaatatct ttttctaccc atcctgggtt cacagctgag gccactataa caaaaaacag
149221 attaataaca cagaagcata cacatttact taatataagt gttatgtgac acagtagcca
149281 tcataaggaa agaagactca aagaaacaga tctgagtatt tttatagtag gtttgatgaa
149341 gaatggatag tcatggagaa atataatggg gcatataata caatggggtt atgatctaat
149401 ggtagtaaac tgggggaaat ttagctgggt ttgcttgttc agatttttct ccttgacctt

149461 ttgtcgtgag agacaaggat gtttcttttc tctgggtata gggagggcac ctctcacatg
149521 agggacttat gatctgcttc agagaagtgc cagggaagaa cagagtgacc gtcctgcttc
149581 tgcagttttc tcaaattcat acagcttaaa atatttaaca tgctaaggtg ttacattttg
149641 gggtagtata tcttgatacc catcacctga aaccaaccac tttataatag taacacaaag
149701 agttgtacat aatataccat aaaggtggta aaatggaatc attggaaaag tcaattcatc
149761 caaaagaagg ctgaaagagg aaagcaaaaa caaagaagag atgggagaaa taaaaattaa
149821 atggtaggga catgagcatc cattaatttt ggtatacagg aggggcttga ggggtgtcct
149881 ggaatcaatc cccctcagat accaaaggat gactgtatat tttttaaatt aagagacttt
149941 cattctttaa atattcttga cttacagttt tttaaaaaga aaaaaaatca aagcatagta
150001 tagttgttaa atcattttaa tagtatattc agtggctgaa atgacaggga gtatcaatca
150061 ttatttattc aatataaagt tatccttact gaaaaaataa gatataactg ggaaatatgc
150121 ttggatccct cacacatcca cttaatttct taaaggaaga aagatgaaac acatgtaaga
150181 acagaaacaa ataaaccatt tactgtaaca ttccaggaat tgtgggatcc ttttcttccc
150241 aagtttattt atttgcttat ctttatctcc tagtggtagt ttgtttcctt tggtagaata
150301 tatggcagtc ccacattggt gacacctggt gctcccttca tccaactgga tcactttgag
150361 attcagtttt acaccttttt agcattttct ttgtgttcat atatcagtta tgatgagcca
150421 ctgataatat tttgttctaa aacgaaacat attacttagt acttttttta ttgtctcgct
150481 gacttttaaa atttgagtga tgtacagact ttgttttttt aatgaaatat aaatattttc
150541 tcatggtttc taaattcctt ataaaattaa attaattcaa tctgcaaaaa tattaaatgg
150601 taagagaata aactcatcac ttagatgttc atattaaatg taaatggcct aaataccca
150661 attaaaagtt aaaaatggtc taattgaata aaatacccat atccaactat atcttgccta
150721 taagaaaata ttgccacttg cagtataaaa atacaaatag gtaaaagata aaaggatgga
150781 aaaagacata ctattttaaa ctaataaaat attaattaat taattagtca gatttcagga
150841 cctttttctg aataattgat agaataaaca gacaaaaata tcagtaagga tacagaaaac
150901 ctccaaaata ttatcaatca ccttgaatta attaatattt atggggccag gcttggggct
150961 catgcctgta attccagcac tttggcaggc cttgagctca ggaattcgag acaagcctgg
151021 gcaacacaca cacacacaca cacacacaca cacacacaca cacgtgtata tatatatgca
151081 cacacacaca tcagctaaaa gcagcagaat acacattttc ttcaagagaa cacagaatat
151141 ttagcatgat atgctatatt ctaggccata aataaatctc aacagactta aaataattca
151201 gctaatacaa agtatattct gtaactacag tggaattaag tttgaaatta atagcaaaag
151261 atatctggca aatccccaaa tatttggtaa ctaatatcat acttctaaat gacccagggt
151321 caaataagaa atcaaaatag aaatttgaaa gtgtttgaac taaattaaac acacatttca
151381 aaacttttga gatgcaacaa cagcagtatt tagaggaaaa tttataatag tatagaccta
151441 tattagaaaa gaagatctca agccagtgat ctcagtttct acctgaagaa actagaaaaa
151501 caaaacaaat aaatccaaaa taagcagaag aaagtaagta ttaaagacca gagctgaaat
151561 caataaagta gaaaacagaa aaacaaaaca aaacaaaatt taataacacc caaaacttgt
151621 tattatacag atcagtaaga ttgataaacc actggtcaag actgattagg aatttatcaa
151681 gaagaaacga tttccttttt ttcatactgt atatgtagca cccaaaaata aataacttta
151741 gtataacatt tattcatcta atacagaaga cagaaatgag aaaagtggca tcattacaaa
151801 ttctacagat agtaaaagaa caataagata atatgataaa aaactcaggc caataaattt
151861 gacaacttag ataaattgca caaattactt atatgacaca atctaccaaa gttcattcag
151921 gaagaaattg ataatcttag tagctgtata tctattaaag aaattaagtt gatagttaaa
151981 aattatccta caaaacgtat tccaagctct gactgtttcc ctagagaatt gtacccaata
152041 tttaaggaga aagtaattct aattctgcgt aaacttttct aggaaattga agaggaggaa
152101 atatttccca actctccctg aggccagtgc tactctatcc aaaaccagaa aaagacattc
152161 caagaaaaca aaactacaga ccactgtatg caagaagcaa tcggaaatta aaaattgtaa
152221 aatatcattt aaaatatcag aaaaagtaat tatgaagtac ttaggaacaa acctgacaaa
152281 agtggctcaa gatttgaaca ctgaaaacta taaaacattg ctgtaagaaa ttaaagaggg
152341 cagaaataaa tggaaacctc atttgtgaat tagataaatt taacactgtg aagatattaa
152401 ttttccttaa attggtctat aattcaacag aatctcaatc aatacctcaa taggctttta
152461 aaaatgcttg caaaatttga caagctaatc ctaaaattta tatgaaaatg caaaggacct
152521 acaagaggca aactatcttt gaaaaagaag agtaagtttg gaagacatat acttgccctc
152581 agagctcatt ataaagctac agtaatcaag acagtgtgag actgaaataa agatagacaa
152641 agaaagacaa tttctaggtc atgagagtta tgtttcctaa aggaaggcag cagatgctta
152701 ttaactttcc ccgtgccctc aggatgcctt aaacagtggt tgccaaccac tagcagcttt
152761 agcaccactt ggaagctttt tagaaaagct aattactggg cccatcccag acctactgaa
152821 ccagaatctc tgtgggcaaa gcccaggaat ctgtgttttc acaagctctc caggtgataa

152881 tgatgtccat taaagtttga gatccattgc cttggccttt ccctgctaga aatcctccag
152941 gatttgggag acaggggaag aggtctggag agagtggcag aattcaacac cacagaatga
153001 agaggaaaat tgatggtctt tgtccatgga atccattaaa atatactctc attgctgcca
153061 ggctgagctg atgaataaaa aataagctaa tctcaaatca agccatcagg ctattgatga
153121 taattgtgtt ttagggtgag caaacagctt taccaagctc acacagttga gacaatttgt
153181 tgtttggaac tatcttactg taagatcctt aagaagaatg cagaagtatg aaaataaatc
153241 caggactcag aaatctttca actaaatctt taaaattctc tctctcatgc acacacacac
153301 atatgcacac atacacatat agtaagattt catgacacat gagacaggat gtgaagctga
153361 acagttcgat gaatgcctga gtggtaagat caccttgaag aaccagaagt gagggtctat
153421 tctagctggg tatgagctac aaactcattt cttggggaac acatgaacac atcggggagg
153481 ctaagtagct tctgaggcta ctaatagaat ttcagtcatt tctcctgtca tctgaaaaat
153541 atatgatatt gaatttctaa aaatgggttt agtgtagtct agtcttactg tctaaaacta
153601 agatgtggct tattccaata accaaagtga ttccatccca tgtgagggaa aaaaaggata
153661 atggtgaatt aaaagcacct gcgtgtattt gttgctcctt tagaggagtc tctcttcatc
153721 aacgttaact ttctttccta cttttgatct ggctgctaaa agtctaggtt tagatctcaa
153781 accattgtcc ttctctgcat gatgtaatag ttacaattac agtctcaacc ctctggcatt
153841 taatgaagat gaaattgatt tctttagact gaaaacaaaa aaatcactta caatcaggat
153901 actgaatatg gtagcaagta caaggtccca gcttctagta gacttggcag actgtcaagc
153961 tcttactaag tgaccccacc ccatgccttt ttgtcttttc tgttgccaca caattacaat
154021 aatttgttct ctgaatgcca gagtactgac aaggaaagga attctgaagc tggctgagag
154081 aagtcgtgta aatctcccaa tctaggctcc attgactctt gtctctttct ttgtatggaa
154141 ctgattttat aatttctgtg gcaatgaaag actagtaccc aaaatgactg ctggtccttt
154201 gtttattgat caatctgagc atggactaga attcatcctc caattggtaa caagaaaaaa
154261 ttggatgggg aagcatacca ttggctaagg cagcacagca agtttgaatt tggctgcctc
154321 ttctttcaat attggttgac atcatcaaga gcagacattg gcgttggagt taagagtatc
154381 aagagagtta ataaatacca atttagggta atattttatg cctagtcttt atcttttggt
154441 gaagtggttt ctaatatcat ctcagactaa atcttatggg atatttctac ttgtagatgc
154501 taaggtatta agaaaaagct caaagtagtt gtgttttcct tatcttccta aaagtttaga
154561 ttaaagatgc caaaaatttg tgtgttggta tgtgtcaaag catttgaatg cccttgtaga
154621 ctattaaaac ctttgttctg taccaggcac tgtattgagt atattgcatg tatttattat
154681 tatatttagt tctcacagca accgtacaca ataattacaa ttccttcctt tcactaaggc
154741 atagggagtt taaataaatt agccacagta caggatggca taaaactgta gcagagccaa
154801 aactcaaaca tgtctctgtc tgactcaaaa acataggctc attctcctag agccaggcaa
154861 caacctgaaa gctcttgagt tgacttttct agttctgtgg atgaatgcaa tcatctcagg
154921 acccagataa acagaagtcc ttctttctta ttcgtatttc tcagtactag gccagtcagg
154981 acaaactctg ttctccatgg ccgtgatctt gaagaaattg agatttcatc caaaggaaat
155041 caggtattga aacgtgtgtt tatgtgtatt gattaatctt ctgcctgttt ctggaaacat
155101 ttgaagaaat ataacaaaaa gcaaatatcc tgaaactgcc taatgaacct gatctctctc
155161 tctgtctttc cctttctctc tcataggtct tttgcctgta tttctctggt ggaatttact
155221 aaataattat atcaggtgtc ttcttattgt acttgactgc tttcatcaag gtaatcttga
155281 ggacagagac ttcctttgtt agccagggca ctctagggga acagaaatta cacacacaca
155341 cacacacaca cacacacaca cacaggaact ggttcatgga ggctaaaaag tctcaaaatc
155401 tgcaggccaa aggcttgaga acccggagtc ctcattgtgt aagttccact cagagtctga
155461 agaggggaga agacagatgt cccagctcag agacctttag gcagagagag aaaattctgt
155521 cttgctcagc ctttggttct tttcaggcct ccagtggatt agatgaggct cacccacata
155581 ggggaaggag tctgatttcc tcagtctacc aatgcaaagg ttaatctcat tcagcaacat
155641 cctcacagac acacccagaa taatctttca ccaaatatct aggcactttg gcccagccaa
155701 atagacacat aaaattcact tccacacttt cttactaatc cgtgcattct ctccaataca
155761 gggcacccgg cctggcaagg agtaagtgct ccatatatat ttactaaatg acaagagatg
155821 agattatcct tagactatga ctcagatata gacaatcaat tattaatacc ctatcatttt
155881 tccccactct gacaacccaa actgaaaaat gttgggattg ccttttttga caatatgatt
155941 ttctctgaat gcagactcat gttttgacct tccctgtctg ttcctgttta gtattcatgc
156001 tggcttaact cattgattga tcaagtaacc attgagcatc tgccgtgtta acactctgaa
156061 caagatcctg taatatgaat ggtgtttagc ctgatgaata aagtgtggtg tctgtgcatt
156121 catttttata tatttattgt ttttaaccaa actttaccca tgtaaagaat aacattgatg
156181 gcaggaactt gaatttggat ctacgtacat gttccatttc agactcacca tgaaatgagg
156241 tgcttgcatt tttcattgct gattttacaa tacactttca caccttttgg gtaaaatttt

156301 gcaatcaatg taactgaggc agcatggtat gaaagcaacc ctggaggtgg aaatagaaga
156361 ctagaattta aatcttggtt atgccactta ttggttgtat gatttttgtc aagtcatttg
156421 acccgtgtct attcagagtt tctttgctag aatgaaggag ttaactagaa tttattgatc
156481 tcagtagtca gtctcctcag gcacagataa atcttggata tgtatcctga gatagctttc
156541 ccatttccac attgaagaca acattctatt ttagagcaaa aatgaattat attatttaag
156601 aatatgagtt tcagaattca gactcatgct tacgggtttg aattctagtt ctgccacttt
156661 cttactatgc agactttagc aagtcagtta atgtttgtga gcctcaggtt tctcaccagt
156721 aaagcagtgt gagtaataac tatcatatta tattattaca agcataaaat gaaataagag
156781 tacttggcac aaagaaggat cttaattttt agctttttaa aaaagctgta tttgtttata
156841 tgcagtatgt atatatatat tctcaattct attattatag actatattta ttatgttttt
156901 cataaagtca ttagtttcac tcaggaatta gttgaccctt gtgagataca tggtttgacc
156961 acttttcact ttgatgttct tcagaaaata agacaattag aaatctctga ttgtaatctt
157021 catgtaaaat attttacagt catggctaaa atgatcccag gagtggaatc atacacataa
157081 attcttttct ttattctaat aatgttacct ttagtgtcta tgccaagcaa gtatgttatg
157141 tgttgagata aaaattctta gtatgtttgt atgtgacatt cccaactccc atcccactga
157201 agtctatatt tacctagttt ctttcttttg ttttcctcta acattcatgc tattttaatc
157261 aatgttcttt cattttgaag cccccaataa gtttgacccc aaagactttc agtttgcccc
157321 tgctattcat aagcatactt atgacaatgc atgtctccca ttcttttctc atatttacac
157381 cgatcttttt attgggacaa atgggtttgt tttttcatgc ttgtgtttaa tatctcactg
157441 agttcagcat aagttattaa acataatttt ctgttttctc tttctccctc aagactttct
157501 ttttctctag acctttattt cctaactcca gagtatttgc ccaggtctat aggcccctct
157561 ctcttttgct ttcttaccaa cccatccacc ttcttctgtt tcctattgcc caactggctt
157621 cacttaaaca agagcatttg ttatttgtac agagtgcaac atataattgt cctactctgt
157681 ttggttgcta taacaaaata tcatagacta ggcagcttat aaacaacaga tatttatttc
157741 tcatagttct ggaggctgga aaatccaata tccaggcact actggcagat ttggtgtctg
157801 gtgagagcct acactctggt tcatagacag ccatccattt gctgtgtctc catatggtag
157861 aaagaatgaa taaagtttct ggagtcttct ttacaatggc actaatccat tcatgaaggt
157921 ttcatcccat gacctaacaa taccattgta ttgggggtta tcttgcaaca tatgaatttt
157981 ggggaacata agctttcaat ctatagtaat gaagtaggaa attttccctg atcctttcat
158041 gggcaggaac tggagtatgc ctatttcaca gaggtcttat cagataaaaa aattctccat
158101 taaatggcaa aacaagtaac catttgggat tcctgggtgt acagtgccta ctattccaaa
158161 atgagattat tgaggctaac atgacccttt gtatacaaag acgggggcag aaataatatt
158221 tctagcaaat ccacacatct caatcacatt taatttcaca aaatttactt ctaataagca
158281 ttgttccaac taacaaacca tctggtgcta gtctaagata gagatggctg aagtgctcct
158341 gattccagta ttttaggtgt taaatctcca ttctaacatt gtcaatcctt aactggagta
158401 ctcaaatatt aactaacttc ctctaggata taaattaata tgtataccac attctatgtg
158461 aattatttta attatattac atgccatata caaattatat ataggtttag tcttcctgaa
158521 tattgcaagt tgagtctgtg agagaaaaaa ttaggatgaa ttgagaataa ctagaattca
158581 gaattctttt tcattaagtg ttgactgtgt taactcatat tcccggttag gtagcaagtc
158641 ctatgaagtg tgattttctc ctaacagcta aaaagcagaa aaaaaatgga tttattctag
158701 ttaatgactt gctttttcta tttttataga aaaaatgaag taaattattt cacttttctt
158761 gatgctatta ctattttgtc aggaagatag atcttctaaa ttttaggaaa ctatgaatct
158821 aagttctata ttgctctcat ttcctttttc tctttgtctc tcttctgttg tagcccctcc
158881 ctcaccgcta agccttcacc ctgactctac cctgccccca ctgccttttg ttctttggaa
158941 gacactcatt aacatttgct aatgtatgct aatgagcaat aattagcatc tctaatgtga
159001 acaaaagcag tgctacaaag gaagggtaaa gatgtgtaca gagttcagcc agccttttat
159061 gcttctttat gccttaggaa caatcctctt ctttcagttc tttcacaatt ctttagtgtt
159121 ttttgtttgt ttcattttgt ttgcttatcc atgaaccctg aaaaccattc ttcttcttga
159181 gttagaatat tgcaaaaatg ctacggagga cagaaagatt cctttttgtc ctaaataacc
159241 caaggatata agcacagctg ctaagaggtt ataatgcaca tttgtatttt catctggttg
159301 ttattttggt atcctattac tagaaaaggt tttaaccaga aaacctcaca caagataatg
159361 tatttcattt taccacactt atttacagtg gttgcccttt atggtggtaa ttgcaatgtg
159421 aaagcataca ctctgaatat atattatgac agcatggtta aataagaaat gttccctccc
159481 tcaaacaatc taaatgcaaa gaaaatgcaa gactcaccac ttactttttc ataaccacag
159541 agggctctaa atgtctttgt aaatagctat ttatttattg aatatgtata gttttttttt
159601 tgaaaaagcc agccctccaa accacataga gtaagagctg tttttgttct ttctgtagac
159661 tgatggtgtc gtgaaatacc taattgcagt ttctcacaaa tactccagca tgtaatttgc

159721 catttgcaga accaggcttc acatttcata tttgctcttc cagctcaggg gtggtagaat
159781 ttggaaacat accagcaacc cacttcactc agcctgtgtt taattataat ttcacgtaca
159841 ataaaagcag aaggaaaacg tgcaaaaaaa ttggtgtgca tccctttccc ctccttctta
159901 cgtatataaa aaagatttat ttccattgta aaaggataga atggggatct ttattttgag
159961 tagattttct atttttgttc tttctgtttg cctcagagtt agtgtcctct ctctgtgacc
160021 tcagagtaaa atgtgtttct ccataggctt catcctgttc tatttatcaa ctatattata
160081 tttatcaatt attttatatt gtgtttttaa taaagcagac atcagcagct atttattttc
160141 tacttctgag ggcctaaaga gtctcagaaa atgagtgtaa tctagaaggg aaaacattga
160201 agagtgacag ctattataac agttgtgatt tttgcctgcc tccctctttt ggattggtga
160261 gtcctaccaa aaaccaaaac tattattctc cttgtaattt tgaaaccaca gagattgaaa
160321 ataactattt agtatctcca agatattata atgtttgaaa atatgaccag ttttgggggc
160381 atttattaca aaagtttcca gtagggtgtt tttgaatgtc tgtgccctct ggttttcaca
160441 aaactggtct taaggaagat aagatactgg tacacacaga agaaatatca gcctacgtct
160501 gtctcattaa atcttatccc cagatacttt gatgatattt aagtgtaatg atctcattct
160561 gtgtttctca ctcctgataa ttttttaaat gtcttatagc ttacaggttt attaaggtat
160621 ttgatttatt taagaactac ataattattc tcttcattca atggcttctg aaatattatg
160681 tttaattcta tcatttgaag gacttgggat tgttataaac aaatggcacg agcttctttt
160741 tgtcctgacc tagaagtagc aaagataaag ggagctgctt agttaacctg agtgagattt
160801 aataatcttg ggtatatttt attctagagt aagaagaatt gcaccctatt actttattc
160861 agttttgtat gcagtagctt ctgttgtcta cttcacaacc accaatagtg ttatcataaa
160921 ttcagagtgt tgggctgtat tagcccattc ttgcactgct ataaagaact acctgagaca
160981 gggtaacctg taaagaaaat aggtttaatt ggctcatggt tctgcaggct gtacagaaag
161041 catggctgct ttctgctctc cagcctcctc atggaggcct caggaaactt acaatcatgg
161101 cagaaggcaa agttgaggaa ggaggtacct cctacatggc cggagcagga ggaagagaga
161161 gaaggggaag gtgctacaca cttttaaaca agatcttgtg agaactcact attccaggaa
161221 cagcaaaggg aaagtctgcg cccatgatcc agtcacctcc caccaggcac ctcttccaac
161281 actggggatt acaattagac atgagatttt ggcacaaatt tgaaccatat cctgggccat
161341 tctctatatg gagatgatgc ctgaatatga tttggtgact cagtctagca tccaagtggc
161401 tcattgttgg gcaattcttc tttagtcttc tttagggcta attttactca ttccttttga
161461 aaattggatt gatttacttt ttgtttggtt ttaagaaagc aaaatatcta aatgctgttc
161521 ttggcatagc ttatatacat atacatgcat acacatgtgt aattttcttc agatcaaaat
161581 atgcatcccc atcattcgta ttgctataat cagaaattag taatttgagg ggcaactgaa
161641 tgaccttata cctagagttt tcagagtata atattacaca gtgtgtattc ataagaatgc
161701 tgccttttaa agaggttttt ttttaaaaaa atcatagctt attttttata gtttcactgg
161761 tctgtcctcc cttttctctc tttttttccc ccttgagaag agagatttca catttttgtt
161821 aaggggctca taatttcctt atgaaatatg acaaatttca ctttatctta aaagcaaaat
161881 attaatagta cagacagctg aaggtggtct gagttaacct gagttacaca ttttatatat
161941 atatatatat atatatatat atatatatat atatgttaaa aatatttatt tgtcatatgt
162001 agcaagacta aaatgcaaaa ttttgcttta tgcttattct tttccagtat catttactgt
162061 aattctcacc ccatatttat ttttttactt ctctttctgt ttctctcctt tctcctctca
162121 ttctttttct ttcttttctt ctccctaccc catcttttag ttccatactt ttgaattgct
162181 atagtggact caaatctaag caagaggtat attagtaagt tgttcagtct tttaacaaag
162241 tatcagtcag agaagcacag cccagggata aaccaaaagc agaaataaaa ctgaggaatt
162301 aataatctaa ttcgtgttgt tttctttaca ttataggtgg ttcatttcat agcctacatc
162361 ttcaatttct caattctcat tagatctttc ttaacagcat ataagaccat tctaaatagc
162421 aaaaatagct gtatcctttt actcactgcc catcagttca taaagccact tgaattcagc
162481 tttttgccca cacaactgca ctaaacatat gcctgccaag atcatcaata tctctacatt
162541 gtcaaatcca gtggtcaatt ttcagtccac attgtactta gcaacttatg atacgtctct
162601 gatgcacatc ttctgccatc cacaatctct gactttcctt ctacttaaga gctgattgtt
162661 catttttgat ttcttcagtt tgttcttctc ctgttcctca ccattttgag tctccttttt
162721 ctgttttgtt taccctcatt ttctttgtta tctcatctgg tcttatggct ttaaatacaa
162781 tgtttttgct gataattccc taattcatat ttccagtcca gacctttctt ctgaactcca
162841 ggctcaaata ttcaactgcc tacttaatgt catgcttgga tgtctagaca gcacctgaaa
162901 tccaacggat ctgattaaag tcctgatctt taacagctcc tacctaagct attctcccta
162961 cagtcttcca tcttcttttc ctgtaattcc attcttcagg tgctcaagca aagaacctgt
163021 tgctatcctt gatttctcta tttctctcac atcctacagg gatgacatgg gttcatttat
163081 gcctgtcatg ccactgatta acatccctat caagaaagtt tagtctgagg taaaaacatc

163141 caatttgtat gagaagggcc aacattgaca ttcttcgtac acaaaaaaga atcctgagaa
163201 gaaacacaaa agagctaaat aatacattgc tgttgcagct taggtaggct ggagaagaaa
163261 cccatacata atctcaaacc ttactgttat aaagtcattc tttttttttt tttttttttt
163321 ttttgagacg gagtcttgct ctttcgccca ggccagagtg cagtggcgct atctcagctc
163381 actgcaagct ccgcctcccg ggttcatacc attctcctgt ttcagcctcc cgagtagctg
163441 ggactacagg cgcccaccac cacacccgga taatttttta tatttttagt agagacgggg
163501 tttcactgtg ttagccagga tggtctcgat ctcctgacct catgatccac ccgccttggc
163561 catttttttt tttttttttt taagagggag tcttattctg tggcccaggc tggagagcag
163621 tggcacaatc tcggctcact gcaacctctg cctcccgggt tcaggtgatt ctcctgactc
163681 agcctcctga gtagctggga ttacaggcgc ctgccaccaa gcccagctaa tttttgtgtt
163741 tgtagtagag gcggggtttc accttgctgt ccaggctggt cttaaactcc tgacctcaag
163801 tgattcatcc acctcagcct cccaaagtgc tgggattaca ggcgtgagcc actgcgcctg
163861 gcctataaag tcattctttt gcatcaaata gtaagactca ttaaaggcag tggtattttc
163921 ttttactgtc tacaaaccct tatccaagag cattttctct atattccttt gtgagcacac
163981 aataaatggt aattcaataa gtgaattttc agtttccaaa ataataacct ggagctataa
164041 acattgcttc aagaagtaaa tgtgggtcag agaaaaaggc aataccattc aactcttctg
164101 gccacttggt cactgtgtta tcctgaagaa gcataaacta ctttcagcat tatagattta
164161 aaaagaagcc ccagaacaac atccactttt ctgaacgact cctatgcctc tgaaatgtga
164221 cattttgctg ttgtttcttt ttactgtttt taattactga gtctatctgc atgaaatgag
164281 gatctgggtt gcctaggagt tcatctgctc ctattgcata ttcctctttt aactacacat
164341 acttcaatgc agttttgctt gctgcctcca acattttgac agacttcccc tcctcttcct
164401 tcactatcct cccatcactc tccaggagac aaggttattt ttaaagtgca tgatcaatgg
164461 gcattgggga gcagctgagg catgaagaga tcatacgatt gggaataagg tactagggac
164521 tgctgagaga aggaatggca acatgccatc tcagacattg tctgagaaag gaaagggttt
164581 gtagctccca ttcaagtatt ctgagctatc tcttcacaca ggtttttgtc ttggagggag
164641 ttcttaacat agttctttag cattgcttta agttgaagga gatataggcc agtgctgata
164701 gttacttttc aggccaaata atgttccatt aaaatcgagc ttgctctgag gatcttggta
164761 gtgtcttaaa tctcctggtc atagtgctgc tatttggttc ttgatgctta tgacaccttt
164821 gctgagggtt tgaatcaaga acaaaaacag tattatcaca atgactataa aacatgactg
164881 ttccacgatc agacaaagga atgctgttaa gcaaagaagg cacctaagga gaatgtggag
164941 atgaccatgc acagaccaga atcactattg gaaaaccaac tcaagaggag ggactgggtc
165001 atttctgcag cagtcttcat tgcatcactg agaaattgca gctctgtttt agttctctac
165061 ttgccaccac agggaatatc ttcccttatg gacttttagg ttttatttaa tggttattta
165121 taaactattc cttctgtgcc ggagaagatg attcatgact ctagccctgc tttttctctc
165181 ccgtggttat tagaggaaac tgagttgttg cttatctctt tagcttggga taccttattc
165241 tgctcttttt atttaatata taatctttcc taaagatttc cttatatttt tgttcattta
165301 gtaggttttt ctgttttatt taattaactt tatattatca cattctagtg aaataatcat
165361 cacaaatcat cctaatacat cgtgcaaatg ttttaaaaat gtgctttggt tgtgcatatc
165421 gcaaagtagg ctgtgggtca gcatttgcta tttgtcctca gctgtttctt ctttatatat
165481 aactgataac accctgctcc aatgttaatc ttatatcatt aaaattcttt cctagagaat
165541 ccttctttga ctgaagaagc ttagtggtga cattaattgc aaataaaaga ccatgaccta
165601 ttgtagaatg tggaatatct ccttagaagt gtctgccttc attattgcaa actttcagta
165661 gtgacataaa gatctttgtc ctgtgtagtt tcagttgcag ccagcaacag aaggcatatg
165721 ttcactttga acaaaagaac atttattttc acattctgtg gctgatgttg agtgataaca
165781 tttttgcata tcctaaaatt ttttacccag atgctgttta aaggcaaaaa gtgcttttac
165841 aataatattc tttgtgaatg gaatttacac tcttggcatg tgttgccttt tcaatatgcc
165901 catcaagttc tgaataataa cctttgacag tgagtgaaca gaaacaaaac cagaggttgt
165961 ttcatctgtt cacagcaaca ctgtttgtca tctttgcttt tttggaaaaa tgtcattcca
166021 ccttaaataa aaatatgata aaattcatgt tttataagca agttaataga cttcgattat
166081 taagaaatat tttaattttt ccaaggcaag aaacctgtga tgaatgaaca ttaagttgac
166141 aaaagaaaaa aatatgccaa taaatatatt ttttcatatt actgtagtgt caaaaatttt
166201 ataaaacttt tatatgcctt tataaaaatg aagcaaagct aagaagaaga tgctgtatgt
166261 gttatggggt gaggaataaa cagaaaaagt gtcctttgtc ttggaaagaa ctgtaaaatc
166321 acctatctcc atgttgccaa agggaatcac atattgggaa atttaggcta tattaatata
166381 atacgtataa aaataattga aatagaaaag atctggcttt aagcattttt ctaaagactt
166441 tttcatgctt ggtaaaagtg tttggaataa attattctct atttttaagt agttaatttg
166501 gttattaacc taattggtat gggcatgtgg atatagcttt attaacaatt caatttctca

166561 tgaaataaaa taacccgaca aaccccaagt ctacttattg caatacagaa tttattttgg
166621 ttgaagttat tttgaattga gctgatagct tataactttg aattgggctt ataactgtta
166681 tgttttgcaa atccctgaag attatgaaat agaaaaatct ttgcatggca ttgtataaga
166741 tgctgtagct gtatatcttt aataaatcgt gctcattttg gcaagtttat ggaaaaattg
166801 tattaactgc acaaaaacat tatgataaaa acaataaaag taaaaaccac ttactaacaa
166861 ttgtacaggc atttaaataa ttgctgtact gttttctttc ctcaacagta caaatcaaag
166921 tggaggaaaa agaagacgag actgaggaaa gctcaagtaa gtggtgatgg gccacttggg
166981 atacatttcc tatggaatac cagtaaactc aaagaattta tcctcttgtg gacttgagct
167041 accatatgta agcagaagtg tgtgtggata acttttcatg gtctcacagg aggaatcaag
167101 ttcagcaaac agaatcccca ctctagagtc tatccagttg gcactataag ggccaagact
167161 gtgttccctt gattgcttgc tgaaaaatca acaagcaaaa gacatgttaa taggagaaaa
167221 ggcatataat ttcatagaac ttgtattcat taaagcctta ggatgaagac ccaaagatag
167281 agaaaattgt ccatttttat gcttaggttc aacaaagtgt ggatagcagt gtagaaatat
167341 gattggacga aaagggtata atctaatgtt tacatagagt ggggaaacac aacaaggcct
167401 gtctgcctag attcttcttg gcctctctaa gcatgcattt ctttcttctg ggtatggggc
167461 agggaccctc tttggaatga gggtcttatg atctacactc aaacaaggta ggtcggatga
167521 tttcttgatg accagttttt acacagacag ggcagaggga atgttagagt agtattttta
167581 ggtttcatgc atttgagaag aatgtttatt ctgttgttat tgggtagaat gttctgtata
167641 tgtctgctag gtctgtttga tctataatgt tgttcatgac tgctgtttcc ttattgattt
167701 tctgtctaga tgttctatcc attatggaat gtaatgtagg gtattgaagt ttcctactat
167761 aattatattg ctgtatattt tctctcttga gtttggttaa tgtttatatt taggtgctct
167821 gatgttggat gcatatatac ttataattgt gatatcttcc tggtaaattg acccttttat
167881 tactatttaa tgcccttatt tgttgcttat gacagtttta tactaaaagc atattttgtc
167941 cgatgtaagt atagccaccc ctgttctttt ttagctacca tttgcatggc ctatcatttt
168001 ccgttccttc actttcaccc tatgtgtgtc cttaaatcta aagtgaatct tttatagtta
168061 gcatacagtt gtttcttttc tttggtttct tttttaattc attcagccac tttatgtctt
168121 ttgattgaga agttcaatcc acttaaattt aaagttatta ttgatagagc aagactaact
168181 tttgccattt tgttcattgt tttctgtctt aaagctcttt tgtccctttt ttatctcatg
168241 ttattctcct tcatatttta ttgattttta ttatagtgat atactttgat tcctttctct
168301 tttcttttgt gtgtcttcta tagaaatttt attgtggtta ccatgggact catgttcaac
168361 atcttataat tataaaagtc ctattttaag tagaaaacaa tttaacctca attgcatact
168421 aaaactctac acttctactt tccttgacac ttaatattat tgacatcttg agttacatat
168481 ttttatatta tgcatccatt aacgtatttt tatagttact gttaatactt ttttactttt
168541 ataatacaat taaaagttac caatatactg tcattaaagt attatagtat cctgtatttg
168601 tctacatatt tacctttacc agacagtttt atattttcat gtgattttgt gttgctcttt
168661 agtgtcctct cttttcaact tgaagaactt cctttagcat tacttgtaag aaaagtctag
168721 gggtgattaa cttaggtttt gtttgtctga gagtctttat ttctctgtca tttataaagg
168781 acaaacttgc tgagcatagc tttcttggtt ggcaggtttc ttctttccaa cacttttaaa
168841 atatcatcct actttcttct ggcctgcata atttctgcta agaaatgata gtcttatgaa
168901 ggtttccttg tacatgacaa gttgcttttc tgtttctttc aaaattctgt ctttgtcttt
168961 gacttctggc aatttaagta taatatgtat tggtgtggat ttatttgggt tcacataatt
169021 tagggtctgt gggcttcctg aatctgaggg tccattttct ttcccagatt tgggaagatt
169081 ttggttatta ttttctttga atagtctttc tggccttttc tttcttttat tctccttcag
169141 aactcccata gtgtatatat tggtctgctt gttgatccct cataaaactt tcttgacttt
169201 ttcttattct ttttatggtt ttgctcttct aactataatt tcaaacatcc tatttttcaa
169261 tttggtaatt ctttcttttg cttgatcgag tctgctgtta gacctctgta gtgaatttct
169321 gttactctat tcttcagtta ttgtgttcag ttattgtctt gtactcttca atatttatct
169381 ctggttattt ttatattttt atttctgttg aaactcatac tttgttcatg catttttctt
169441 ctgaatttgt tgagcatctt tatgagttat ttttaatagt ctgttaagta attcatatat
169501 ctccattata ttagggctgg tttctggagc tttatcttct gttgtttggg gtatgttttc
169561 ctgtttcttt atttttcttg attttgtttg tttgttttgg tatccatata ttagaaaaaa
169621 cagattcctt tcccagcctt caggagaagg attagccttg tacaggagaa gagtttcacc
169681 aatgaggcag gccagagatt cttgggcctc tcaaacattt cctgtgtatg catcgtctct
169741 ggacttgtgt gtgtaaattc tcaataagag ggatttttca gttgactttt tctttttaag
169801 aattcataat ctcttgttcc ctccagtgtc cttctgtcat aacatgtgtc ttctgaggaa
169861 gcagcatgtc ctgagttctt ttttttgttt ccagtagtcc ccaggcatca acagtatgct
169921 gggtcctgtc catgccctga gactggtcat tcccttaggc agcctcctga gaagtctgaa

169981 cacagggtat atatttcagt cttttatttc ccatttcagg ggcaagtcaa gggctggaat
170041 tcttgccaac tgtgtcatgc tgaactgggg gaaggggctc tggtgaatga gtacaaacta
170101 attcaaaact tcacctttgt tctccatggc cctgggccat ttagaatatg ctgggtccta
170161 ttagtgctct aggagacaag tgggacagaa gccagtccag tcatttttta gaaaagttgg
170221 agtgttagat gcactatcca actgtttttc tccccagaga ggtgcagatt tggtgtttat
170281 catctggtta ttctgtgcta aattggggga gtggctattt gagtgaggca tactattcaa
170341 acggctatct ttgctctcat tggttcctaa ccagaggtct tttcctgtca gtgctcagat
170401 tctggcaaga cagaaaccag tcctttggga acctccagaa aaatcagaat gttgaacaca
170461 tggttcagtt gttttctccc ctccccaggg agagactggg agatgatggt tttctcccag
170521 ttgtatggta ttgtgttagc agagagggat tatggtgtga gggtgtgtca gattttcta
170581 ccagctacct agctgatttt gtgttcatct agggttcagg cacctttcaa ctagtttccg
170641 aatttctcac gaagggagct tatctccaca ttgtagttga acttttatgt ccatggaggg
170701 aaggcgggtc cagggcttcc tattttgtca tcttgttgat gtcatcccag tagtttagtt
170761 ttaatacaaa aaatactaaa attcagagta ccaagtagct gcttgggttt gtgagttatt
170821 agtgggtaaa ttgtttcttg cctcctgtca agtttttgtc ctttctttaa ttagtgacta
170881 atgtggctaa taaaagcagg gtaaatattc agaatgccat gatggggttc aaataacata
170941 catttgccac aatattaaac aaacagaaac tcgttcttga tttggatgac agaatatata
171001 tacagttttc caagcccagt tagaaaagtt attatgggcc gggtgcgata cctcacacct
171061 gtaatcccag cactttggga ggccaaggca ggtgaatcac ttgaggccag gagttcacga
171121 ccagcctggc caacatggtg aaaccccatc tctactaaaa atacaaaaaa ttagctgggt
171181 atgatggcgc acacctgtat tctcaactac tcaggaggct gagacaagag aatcgtttga
171241 actctggagc tggaggttgc agtgagccga gactgtgcta ttgtactcca gcttgggtga
171301 tagaacaaaa ttgtgtctca aaaaaaaaaa aaaagtgaag ttattctgtt ttttactctc
171361 tgctacctga catttagtaa tatatgtaat atttagatct tatacctgaa atatcaataa
171421 aaaagtttta acaagtggta attaaagata attttgaaaa aataaaatga tgactcacac
171481 gtatgtgaaa agaacatatt taactaataa aacaacttgt gagatgctat aatcagttca
171541 aaggaacgtc caaagaatag atacatttat agataataaa tattgaaatg attgtacaaa
171601 tgaaggcaaa gttgtttttg aaagggggct tgtctctcct ttggaaagag ttcatttgga
171661 tgtctacaga taaaaagtgt tttgtattgc tatatatggt tatgtataat tttcaaagtt
171721 gtcatatagt tctcataaaa tcagaatcag ataacacaga aaggtatgct gcagataatt
171781 catagttttc cattgaaata taattttttc tctggggtag cctctattta agagagtaat
171841 gataactgta atatttaatt ttttacagta gtttatgcta aatgttagaa gttaggagca
171901 atagaaatga aactggaata gtaatgttat gttataaact ttgtgaagga aattttatct
171961 taattatctt aatttatctt aattatctct ctattcttta cctggaagca tcctaaaaag
172021 ttatgaacac atgaggcatt cattgttgtt gatgatgaaa caaaataatg ctgtcattat
172081 tctcaatttt acattaaatt tatgttacct atttctattc ataagaaata aatgatcaag
172141 caaatgtgta tactactttt ttttttaaag aaaagaacaa aaatattgag gaaaaaggag
172201 aaaaatattt agaatcaaga atgaagaaaa tttaaattga gaggaacctc aagggttgaa
172261 tgaatttctc caaaattatt ctagtttgta gcacctcttt aacgttcctc ttctttgttc
172321 taagatagaa gtagtaataa atatgaagtg gttaatcact caacaattat gtaatggaat
172381 ggtaatctct aaacttggac ttatttgaat ggtcttagag gtcatggtat gatattaacc
172441 accaagctat ttagttactc aagtaaatga agaccagaac ttcttcaaaa acttattaaa
172501 atttgggaat ccacattcac ctatttgagt cggtgaacca caatggaaat ggaaatctag
172561 atacatttat ttaaatgatg ggctatgaaa tatgtattcc atgatgcatc ccatcacgat
172621 gtggagaatt gctagactgt gctgtcagat gtgtaataat agttattaaa aactaagaat
172681 aagcttggta gtgggaggaa gcactttctg acattactca ttgaatcaag tgctatgttt
172741 aggtgaagag gaagaggagg tggaagataa gctacctcga agagagagct tgagaccaaa
172801 gaggaaacgg accagagatg ttatcaatga ggatgaccca gaacctgaac cagaggatga
172861 agaaacaagg aaggcaagag aaaaagagag gaggaggagg ctaaagagag gagcgtaagt
172921 tagttctgat attctttcaa aagttccaat gctgaagaag atcactcctt tcagatactc
172981 tttgacctta aaattggtaa ttaaagctac ttttagaaag agagccttaa tctgatgatg
173041 ctattttatt ctacttaatt aaatgtattt taatggttaa aatagaattt taaaatgaat
173101 atatattaaa aatggaccat gagaaattct cttatgaagg atctgaatta tcataggtcg
173161 ttgacatatt acagttaatt taaagatttt aaaatttccc cactaattgt atgcattgtt
173221 atctaggtta agtagaaact cctaaatagt gagcaaacat cactttatct tacagagcag
173281 gggttggaaa attatggcta aatccagccc aagaggtaag aatggttttt acagcttcaa
173341 agggtcatta ataaaaagca acaaatatgg gacagagttg tacatctcac tcaaagctta

173401 aaatattaac tatttggccc tttatagaaa gaaagtgtgc tagcccttgt tacagaggat
173461 ttgggttgtc attttctttt taatacaact caatatcttt tagttccacc aggaaaacat
173521 tttagtaaac gctgactttg tgtagaataa tgcattaaat gctgtttagg aaaaatccaa
173581 gaaaatactt ttcttctttt tgctttttgg aaatttgcag atagcaggca ggtaggaaaa
173641 atgtacgtgt aaaacacctc taattaaaga gaaaacctag accccttgga aagaatgcaa
173701 gagaacagcc aacctgttcc acaactcaca ggtaataggg ccatgcgagt cgccatcagg
173761 tggtggaatc tcctccaaac actcctagga ctttgaagca tgaggaactt tctaagatgt
173821 cctcccaaga tgacacactt ctatactcaa tagctctaac tattgtgtta ttccttataa
173881 atgattgtca ttaaaaacaa ctgaatgaaa aatttgtcaa gtagagaagc ttagcattaa
173941 caaaaaatgg tttcttttag aataagaata acaccatcta cttcatgtct aagttttcct
174001 cctcctcttc cccagctttt cctttctccc ttttgaatac caagacaaaa cttgctttta
174061 aattttgact ctagtatttt ttatttatat ctgcttttaa agtttcttta tgctttattc
174121 ctttcaaaaa taaaatgcaa cacattttat gtcccatgta ttactatgat tgttagttcc
174181 attattatgt agtaatagtt cctatttagc aaagcttatg ctttccaaca gtggtatttc
174241 attgagaaaa catctccaat tgatgtatgc atgatatagg ttgtgttcca aaatttgctt
174301 ttcctctcta tttatgccgc aaaaaaatcc ttttaaaaag atgagttgga caggaattcc
174361 tctgctctct tcgtctgctg atggccctca tggcaactgc tcaacttcta ggaaatgggg
174421 ccatgtgaag caactggatc ctgtggctat gacagtgtct cttcctagct ggcctcactt
174481 tcatttgatt cttgagaatc aagatccctt acctcccact cttccacata acccaactct
174541 ctccataggt tttgcaaatt gtatttttgc tgcgacaatt tgtgcatcta ttataaatag
174601 tatcagaaat tagttgtgat tattgatagt tctgttctct attttagagt ctattttcta
174661 ttgttcattt tgaagataaa catctttttt ttaaaagaaa atcttagata atatttttga
174721 gaaagattag tagccaaatc agggcacatt tatgtaggtt aattaattca gggtattttt
174781 tatttaacta ttcaagtaat gcctcaggga atttcccttg cattgacaca acatttgaaa
174841 ccaactaaaa ccaaggttgt aatgctaact atctttggag gcagttaaaa aaacactgcc
174901 ttgttagaca ttttggagta tttccagtaa tattacaggc ttttcctgac ccagttttaa
174961 gtggataagt aatatattca tagcagtgag aaattcagca tattatgcct tcttcttcct
175021 aacaactgtt gtttaaatat atcaaactta agatagaaaa ccattcttgc acagattagc
175081 ctgaaaattt agatgaggca tgcctatgcc ttttaatatg attagccctt tctttgagtg
175141 attctagaaa agcaggtttc ctgtaatgct gattagaaaa gtattttaat taccttcata
175201 tttgagggta acttatgtgt caacaactgt accctgtaac acagcctgat cctcctggtg
175261 aaataaacaa ttaacaacag agacttaaaa gatataccaa tctggggtca tttagtacag
175321 gaataaacgt ctttccatac cataataatc tcagaggtag ttgttatatt ttagtgtact
175381 ttgagcactg ggtttatgat gataaccctg agcaatccag aaaagttttg tatacatatg
175441 ttcattcaat aagttattta cccagctcta cttttgtgca acctaatttg cttatatctt
175501 tgggatactt ggatatgaac atgtaattgc ccacaatctt gcaatcttca aaagtccata
175561 atatagtaag ggataggcca cttagataca aattattatg acataagaaa gtatatgtgt
175621 cccagagaaa tacaaacaaa gtagcttgga gtctcagaga agtgaaaact gttctcacag
175681 ggatagttag aaaagggatt cttaaagaca tttcatatga tttttaaagg acttgaaatt
175741 tggacaagct gaacttataa actatttaat tagtattaga attgtttcta ttaaagggtc
175801 tctgaaagtt ggcctgtagg tttagttacc tggcaaatcc acacatcaaa tagataattc
175861 tttcattctt tggtgatgtt ggattagaga tgctgtttta catgtgattc ttaatggaag
175921 ttcagtagtg taaagcttta aggtaattat atgtaaatat catgaattat tttgaaatgg
175981 ttggcaagtt aaaggcttaa catatcaagc aattaaatta ttaatcataa agatttttc
176041 taattgactt tattactgat aatttcttgc catctgtttt attttatttt tgctagtttc
176101 tgaaaatatt tcaataaaac tgctatctat gtaatttaaa gttattccta tgtttgcatg
176161 gaaacaattg tgatcatttt aattaaaaca catctttacc tcctaagaca aactaaccca
176221 catatcattc taattggcaa attaaaaaaa aaaattgtgg atatagatag agtaaaaaaa
176281 atcctgaaat attttatgcc atggctacac agcatcatag gcttttatat gataaacacc
176341 aaataatgaa aatagaaaca acccaatggc tatcagagat gtatcttaga agagtgtact
176401 tccctggttc tcttgaacca aaatgattat tctaagcaaa atgtttgaat tgtccaaaaa
176461 acaaactgtc aataggaagt gttattttaa agggttaata tgaatttttg tttcagtaat
176521 catagcatga ttaaaaacta taaattggtg gcaaataaag gagattgaaa gtcattttgt
176581 aaggtgaact ataaaactgt ggatatgtcc ttttcctcaa ctagaacttt aaaaagcagc
176641 ttttgcctta tttctactgt atctagtcat aaattatttt tctgagtaat actgaattgg
176701 attacaattt ttgttattgg tcaatatcta gtgaggggag aaacttcaac agttaattga
176761 acagagagaa tttaataagt gaattgttag gaggtataaa tgttaattag actgcagaaa

176821 aggtaaaaag agaacactaa agtatcatag tgacggtaac tactggaagc agctaccatc
176881 ccaagggctg gaggaacaat ggaaagggtt gaaacgaaaa cactgtagcg tagaggaaag
176941 accctaggag ctaaaactca ggcctctgca gaggggctga tgctctgtgg tgctggtttc
177001 tgtgggagag gtgatgaagc tggttctgct agtgttggta atattggtga taaggattca
177061 cttgcagtat ggcaaggacg ctcagctcca ggagtgaaga agagatgcta aggaggaagt
177121 gtcaccccac aaagcaaata tgaagcagag aggaagccac aggaaagacg gagaccctct
177181 tcagtatccc ctattgtcag agtatatatt gacccagcta acaaagcaga aatatgactc
177241 acaaaatacc agccccagca ccacaatatt aaagtgtagg agggtgtgtt tgaagttggg
177301 acaatagctt aaaaactgaa ataatctatt accaaaaagc tgatctctac aatgtggtat
177361 tgcatgcctc gatttaaaat tatgttgctc ttgtttagat tttgtttcct ttgagttctg
177421 gaccgcatag tgttttcgta atactaggaa cacagaaaag aaaagctaca tctcatgctc
177481 agtaggaact aaattccttt gaagatctct tcaaaggatt cacttccacc aaatatattt
177541 caaggtgaat tttaccaaat gaagttataa gtttaattaa cagataacat taagttacct
177601 ttgagaaata gatttttatt tttcccgatt tcaatttttc tttctttctt tctcttgaga
177661 cagggctaac aaaattttta agggcaaaga catcttcacc cctaatttat tatccttatt
177721 gagaaataaa atatttaaac gaaatgtaga attgagggaa ggtattcaag ggaacaaaga
177781 agagagggaa atatcttgat atatctgtaa ttattctttc cacagatttt ctggttcaga
177841 gagtttgagg ctctagacat aaaagaattg cttagggcgg gtgcggtggc tcacacctgt
177901 aatcccagca cattgggagg ccggggaggg tggatcacct gaggtcagaa gttcaagacc
177961 agcctggcca acgtggtgaa actctgtctc taataaaaat acaaaaatta gccgggcaag
178021 atggcagatg cctgtaatcc cagctactgg ggagcctgag gcaggagaat tgcttgaacc
178081 tgagaggtgg aggttgcagt gagccgagat tgtcccactg cactccagcc tgggcaacag
178141 aacgagactc tgtctcaaaa aaaaaaaaaa aaaaaaaaaa gcccggctta tgcctgtaat
178201 cccagcactt tgggaggccg aggtgggcag atcacctgag gtcaggagtt cgagaccagc
178261 ctggccaaga tggtgaaacc cccatctcta ttaaaaatat aaaaattagc tgggctaggt
178321 ggtgggcgcc tgtaatccca gctactcggg aggctgaggt agattgcttg aacctgggag
178381 gcagaggttg cagtgagcca agatcgcacc attgcactac agcctgggtg acaagagcaa
178441 aactccatct caaaaaaaaa aaaagaaaag aaaagaaaaa aacagaatgg cttagattca
178501 gattcaggaa ggcaaacact actgcttaaa gttataatag ttccagtaat cctgcatcag
178561 tgttcagatt tgagttctca tgcttatggg tcctaatgtt gactgcatat ggttacattt
178621 aaaaaaaatg agctaaatat ctgatacctg ccttccttaa gttccaaagt caagcaaagc
178681 ttaactttat taaggtgttt cttttttact tttaaagaga agaagaagaa attgatgaag
178741 aggaattgga aagattgaag gcagagttag atgagaaaag acaaataatt gctactgtca
178801 aatgcaaacc atggaagatg gagaagaaaa ttgaagttct caagtatggt gccacttttt
178861 ataagtagaa acactttccc tgatattgat tttctaaatc ctctgaatat tccatatata
178921 tatatatata tatatatata tatacacaca cacacacaca tacacacaca cacacacaca
178981 tacactgtat atatagtata tgtaatacta tatatatcat ttatatatga ctcattatat
179041 ataatggtca cagtcataaa catgtatatc atatttctgc atatataccc aaatatatgt
179101 atatttacat atatctgttt cttatcacta gagagactgg gctaatgatt ataaattgga
179161 agcaactctg agaaattatt tagagtcttt taaattcttt ggtcatcttt aatcatttgt
179221 tccttaaact ggagtttcct tagcttaata catttgttac catctctttg aaagagataa
179281 tatatttgaa ttttacaagt ttgtgtggat tgtgcactct tgtttctctt cagggccctg
179341 gtctcaaatg aacttttcat gtgacagctt acattaaagc acagagaaga tgctaggcca
179401 acagaatagt aaataggatt ccaattaaag gttatgctaa tagctattcc ccgaacatgt
179461 cagtgaccca atttcagtct tatgttgatg ctaatgtgga cgcgaacgca gtcagagcac
179521 acagatttat aaactcttca tatgaaaagt caaatgatta tcctattctg gggatggga
179581 agattcaccc ctgaggatca cactatattt ccctgccctg gtggttggga gaggaggaaa
179641 gggcagggaa aagggaaact gtttgtgata acagccatgt ttgcatctcc tagttttgcc
179701 aaagattgca ttcttccata aatcattttt ataaatagta gtaagtttta aaaactatcc
179761 cttcacaagc ctatttaact agtaatgctg taaaaatgat agtatagtat tattgtctct
179821 gatgatgaaa acaagaactg aaatagctat caggataatt cacttctagt cttggttctg
179881 caagagcttg tgtgctattt aaaggtatcg cttaatctcc tgaagcctta gttacctcat
179941 atccaaaata aagaaagata ttctatatct tctctgtgcg cttgaagcgt agccatcatt
180001 tatagtattt ttgttttgtt ttgtagagta aggttttcca agttctaaac aatggtttc
180061 ccagaaaact tttgcaacac cacaaaattg cagtctggga actccctttg catgctcatg
180121 tacacatcca tcataaatga aggcaaaggc aagcagcatc ctacgttcca cagagctaca
180181 aattttgtgt atgcatgctc atgtgtctgt acattaaaat aattttcatt tagcgaagtg

180241 ataatgatgc taaattatag gttttaaaaa tattcttacc tggcaaaata gaagtttggc
180301 aaccctatgt gggcctctca aatatatttg aaatattaat gacccataaa gtccccaaac
180361 ttgggaactt cagataacta tatatagaaa gtttaaaatt ggagcaatat tgtgaacttc
180421 caaaatatat gattgaataa aaattccaag gactaccttt cctttccaac tgcatggagg
180481 tgtagaaata cccccctggt ctttgcccgt atctctcgtg caggatcgtg gaggagacag
180541 cagcttcaga tttgagttag gatctcagat cttccattta atagctgtgc acatttgatc
180601 gagttgtgta gtctctctac acaactcagg ttccttatga aattgtacct tattgacaga
180661 gtttttggaa ttataaaatg agataattta tgtaaaacat gttgccagaa cttggcacat
180721 agctgggaaa cctaagttgt agttattact tcatctcaga acagacttga tgcccaattc
180781 ctgaagggtg acaagagaga tggagagaat ttgggacctg gcaagtctag gtgggagcag
180841 acctagtcac aatcagaccc cagaagcctt gtgggatggt gacacattgg taccagtttg
180901 gataaacaat aagatttgga ggatgcaaca gaaggaagta aggcatttga caatttagca
180961 aaatccagga ccagaccgtt ctcagatctt ctaaaacata gccatagttc tgtggcagat
181021 ttcaaggtct caggccttgg tctggagtcc aagagttaag caaattttag caatggggag
181081 agataggcat tagcattgga aagaaggaca atacgagccc ttagtacgaa agcagacact
181141 gacaataagg agaccgcacc tgactctaat ctagacatag gtttttttat ttggcccaca
181201 tgtgttttgt ttctttttct tttatttttt ttaaccgtgg caatatatac aatacaaaat
181261 tcaccatttt aaccatgaaa tcgattttct ttatcataat ttttttcaat atttttaaaa
181321 atttaaatta cttttttttt ctcgagacag ggtcttaact ctgttgccca tgctggagtg
181381 cagtggtgct atcttagctc actgcagcct caatctcctg ggagcaaggg atccttctgc
181441 ctcagtctac cacgtacctg ggactatatg tgcacactac catgtttggc taattttttc
181501 tttttgtgta gggatggggt cttactatgt cgcccaggct gatctcgaac tcctggcctc
181561 aagccatctt cctacctcag cctcccaaag tgctgagatt ataggcatga gccactgcac
181621 ccagcctcaa tcttttaaaa gtgggagtaa aatattcaga tgttcagctt ttcatgaaca
181681 agtggaagat ttggtcctac attcttccat cacagtaaca gctaggacta agcacctggc
181741 ctctattaat ggggcattcc tttttcaatt tgcaccattc tcaaacaatc catattactt
181801 gtttatgctg cttggctgga ccctgtagac attcagattt gctgcctcct accagtgcag
181861 ctggggaaca agctaggcat gttaccaaga cggtgattca gatcttcaaa gccaaagtca
181921 gctcacagaa tggaagcaca aatttagtcc cagaaaagaa acaactgtgg cttctcacat
181981 gtcagcaagg ctcacagtct ggtgatgatt ttccatggcc agaagtggtt tctgccttgt
182041 ggccgcacct atgggttcca gctggtgcca tggccggggc cagcctaatt cttttggttt
182101 cagtctccgt gatgaagttg ttggtactta agtgcctgac ttatctctct ttgaatgctc
182161 tattgagctt tttttgaatg ttctattgag cttctcccca ccagattcca actacattat
182221 tagaacaagt ggtcagtact tcacagaacg tgcaggtaaa tgtttctcta ccccaatgcc
182281 accaatgctt gggagtgagg cagctttccc tctacccttt atttctttca gagacatcta
182341 actttgagag cgtgtttcta aggattatgt tattaatatt ccccaaatta gcctgcatca
182401 tcgctcctgg ctaagtctgt gaaacccaag atactaagct cagtgtaact gaaagagatg
182461 ttttgatcac agctttgaat ctgtgcctgg gttcgaagcc ttcttgttct ggacttggag
182521 aaagttttga aacttctaaa agatatttta gacagtgtca gggttttaag atatatttta
182581 gagtaagcct caggaatggc aacttatggt tctgagggtg ttatgggaga ctcagagata
182641 gttcccacat ttttaaagag catctaccat taggaacccg agcacactca gggcttagga
182701 tcaaaagatg gttaatataa gtggttgcta cataggcagg cagctctagg aattggttgt
182761 gaatccatat taatcctcaa acatggctca ggagctagag ttcagcattt tcaagatagc
182821 agaattggct ctatacagct gaaaggaagc catctccttt tcatcatcac tgtggaaatt
182881 gtgattttca ttctatgttt tattgtatat caatacaatt tcacacattc ctgtagcctt
182941 aaacctactg aatgataatc tcttggtgga aaggcaggtg tgtctggccc agaccagaaa
183001 tgtatatttt tattttttga tctaaaaatt actgatatat agtcagaatg aaagctgctg
183061 ttttcaattc tgagagacag aaagctccac taaacagaat actcagctct acagatgatt
183121 tatacaatga ggaagagtgt gtgtagggag ttggaggggg gtaagggaaa cagtgtcata
183181 ttttccaatt atttgaaagt caatttctat attttattaa tcacatcatt taaaaatatc
183241 cattaatgag aatactaagt ataatctgtt tttaagctgt tcatgggttt aattcagagg
183301 ctgtatgact agatctttcc tacctataag aggtcgcagc acttacctga tagcagccac
183361 cacaagtgaa ataaaatacc caggtagaaa gtaacaggtc tacttattac ttgtcttgaa
183421 acctaagact tttaactgta tcagaacatt atatgtggta gattgaaagt gctcatgact
183481 aaacctaaat aagcaaccac cttattttgt tacagtagaa taggttttag actgaaaatt
183541 gagacttgga ctttgagcta agtgcttcct ataacttctc tttgaacctc catttattca
183601 tttataaaac agataaattg gattgtggac catggtcttc aaactgctat cctccaaagt

183661 caagcagttt ggtgaaatca aatcagggca aaggaggcag aggtcgcagg gagagctact
183721 gggccacaat gccggcttca aaaacagacc tatggtttct ctcttctaca cagtgtactt
183781 ccatataaag cattgattgg aagaaagaat gcttctgtgg ctattaaaat aaaaaacagt
183841 tttaaaaacc cacttagctg gatatttaaa gaaacttatt ttggagaagg gacagttcta
183901 acatttcatg agtctacaaa ttaggataca gaaacactcc aagtatactt ttgcagggaa
183961 ttggcttttg gatagacacc attttaaaat gatttatttt gataacaaaa atgtttattt
184021 agctattata ctgactactt taatttctga gctttgtagt accctgacct attgtttctg
184081 ggagttgtcc attggctggc taggcctggt gatttattaa tgagacaccc agtaaagcca
184141 ggaaagttat cctcagattt cattcacact taaacaggtg cttcaaactg tcagctggcc
184201 attaaggtaa cattcctcct tactcaatct cccttgatct tttcacactt cttttcattt
184261 gtttgtttgt tttttatttt gttttatttt ttagttggga gaatgaatac attttagga
184321 aagtgaaaac agaccatggt tgatttcctc taatggagct gttgggaaaa ctttgggaaa
184381 actatttatt tggcttccat tctcaccctt tctctcctgt tttcttgtag cttaagattt
184441 tcacaggcaa gaatcatggt ttatacttta gtttttctct catctgtcat tgtgaaggaa
184501 taaaagatgc tatacaagag gacatttaag cgtgtgaaca cacccaccaa catacctca
184561 aaccattctg gattattttc ttttttaaat tttatatttc cataagtttt tggggaacag
184621 gtggtatttg gttaaatgag taagttcttg gggaacaggt ggtatttggt tacatgagta
184681 agttcttctg tgatgattta ttagattttg gtgcacccat cacctgagca gtatacactg
184741 aacccaattt gtagtctttt atccctcatc ccaatcccac cctttcccca gagtccccaa
184801 agaccactgt atcattctta tgcatttccg tcctcatagc ttagcttcca cttacgaatg
184861 agaacatatg atatttggtt ttccattcct gaattacttc acttagaata atagtctcca
184921 gttgtatcca ggttgctgtg aatgccaata tttctttctt tttctttttc tttctttctt
184981 tttttttttt ttgagatggg atcttgctct gtcacccagg ctgtagtgca gtggcgtgat
185041 ctcggctcac tgcaaccttt acctgccagg ttcaagcgat tttcctgcct cagcctcctg
185101 agtagctggg attacaggca tgtgccacaa cacccagcta atttttgtat ttttagtaga
185161 gatggggttt tgccatgttg gccaggctgg tcttgaactc ctgacttcgg gtgatctgcc
185221 caccttggcc tcccaaagtg ctgggattac aggcgtaagc caccatgcca ggcccaaatg
185281 ccaatatttc actccttttt atggctgagt agtattccat catgtatata taccacaatt
185341 tctttatcca ttcattgact gatggacatt tggtctggat tcatatttct gcaattgtga
185401 attctgctgc tataaacatg catgtgtaag tatccttttt gtttaatgac ttcttttcct
185461 ctgggtagat accctgtagt gggattgctg ggtcaaatgg tagttctact tttagttctt
185521 taaggaatct ccaccctgtt ttccatattg gttgtactag tttacattcc caccagcagt
185581 gtagaagtgt tcccttttca ccacatccat gccaacatct attatttttt gatgttttga
185641 ttatggccat tcttgcaggt gtaaggtggt attacattgt ggttttgatt tccattttcc
185701 tgatcattag tgatgttgag cattttttca taagtttttt ggtcgtttgt atatcttctt
185761 ttgaaaattg tctattcatg accttagccc actttttgat aggactgttt gtatttttct
185821 tgctaatttg tttgagttct ttgtagattc tggatattag tctttgtcag atgtagagat
185881 tgtgaagacc ttctccctct ctgtgggttg tctgtttact ctgctgactg ttccctttgc
185941 tgtgcagaag ctctttagtt taattaagtc ccacttattt atctttgttt ttgttgcatt
186001 tgctttgggg ttcttggtca tgaagtcttt gcctaagcca atgtctagaa ggtttttct
186061 gatgttatct tccagaattt ttatagtttc aagtcttaga tttaagtcct tgatccattt
186121 tgagttgatt tttgtataag gtgagagatg aagatccagt tttattctcc tgcatgtggc
186181 tagccaatta tcccagcaac atttgttgaa tgaatagggt gtcttttccc cacattatgt
186241 ttttgtttgc tttgtcaaag atcagttggc tgtaagtatt tggatttatt tctgggttct
186301 ctattctatt ccattggtct atgtgcctat ttttatacca gtagcctgct gttttggtga
186361 ccatagcctt atagtatagt ttgaaggaag gtaattggat gcttccagat ttgttctttt
186421 tgcttagtct tgatttggct atgtggcctc ttttttggct ccatatgaat tttagaattt
186481 tttttctagt tctatgaaga atgatggtgg tattttgatg agaattgcat tgaatttgta
186541 gattacttta ggtagtatgg tcattttcac aatagtgatt ctacccaacc acattagcat
186601 ggatgtattt ccatttgttt gtgtcatctg tgatttattt cagccatgtt ttgtagtttt
186661 ccttgtagaa gtatttcacc tccttggtta ggtataatcc caagtacttt tttttttttc
186721 ctgtggctat tgtaaaaggg gttgaatagt tgatttgatt ctcagcttgg tcactgttgg
186781 taaacagcag agctactgat ttgtgtacat taattttgta tcctgaaact ttgctgaatt
186841 catttattag ttccaggagc tttttggagg agtcttcagg gtttctgggg tatatgatca
186901 catcatcagc aaacagtgac agtttcacct ctttaccaat ctggatgccc tttatttctt
186961 tctcgtgtct gattggtctg gctagtacta tgttgaatag tagtgagagt gggcatcctc
187021 gtcttgttcc agttctcaga gggaatgctt tcaactattc cctgttcagt attatgttgg

187081 ctgtatgttt gtcatagatg gcttttatta cattgaggta cgtcccttgt atgccgattt
187141 tgctgagggt tttaatcaaa atgggatgct ggtttttgtc aaatgctttt tctgtgtcta
187201 tttttttttc gggggggtgg gtgtttaaga gcagaaagtt taataggcaa aagaaagaag
187261 agaaaaccac ccccatgcag agggaggggg atcctctctt gctgcatcta ttgagatgat
187321 catgtcattt ctgttttaat tctgtttatg tgttgtgtca catttattta caagtgtatg
187381 tcaaactatc cctgcatcca tggtatgaaa ttcacttgat catagtggat tatctttttg
187441 atatggtgtt ggatttggtt agctagtatt ttgttaagga tttttgcatc tatgttcatc
187501 agggatattg gtctgtggtt ttcttttttt gttttgttct ttcctggttt tggtattagg
187561 gtgatactgg tttcatagaa tgatttaggg agtactccct ctttctcttt cttgtggaat
187621 agtgtcaata ggattgaatg tctgatagaa ttcagctgtg aatctgtctg gtcctggact
187681 tttttttgtt ggtaattttt aaattactat ttcaatttca ttgcttgtta ttggtctgtt
187741 cagggtttct aattctgatt ctagctaaat cctcctgatt taagctagga ggattgtatc
187801 tttgcaggaa tttattcatc tcttctaggt tttctagttt atgtgcataa aggtgttcat
187861 agtagccttg agtgatcttt tgtatttctg tggtgttggc tgtaatatct cctatttcat
187921 gtctaataga gcttatttgg atcttctctc ttctttgctt cattaatctt gctaatcgtc
187981 tatcaatttt atttatcttt tcgaagaacc agctttttgt tcaatttatc ttttgtattt
188041 ttgttgttgt ttcaatttca tttagttctg ctctgatctt tgttatttct tttcttctac
188101 tatttgggtt tggtttgttt ttctagttcc ttgaggtgtt accttaggtt gtctatttgt
188161 gctctttcag actttttggt gtaggcattt aatgctatga acttttagca ccacctttga
188221 tgtatcccag cagttttgat aggttgtgtc actattattg ttcagttcaa agaatttttt
188281 catttccatc ttgatttcat tgttgaccca acaatcattc aggagcatgt actttaattt
188341 ccatgtattt gcatggattg gaggatttct tttgatttcc aattttattc cactgtggtc
188401 taagagagta cttgatacaa tttcagtttt cttaaattta ttgaaacttg ttttctggcc
188461 tgtcatatgg tttatcttgg agaaagttcc atgcactgat gaatggaatg tatactctgt
188521 ggttgttggg tagaatattc tgtaaatatc tgttgggtcc atttgttcta ggtatagttt
188581 taatccattg tttctttgtt gactttctgt cttgatgacc tgtctagtgc tgtcagtgga
188641 gtattgaagt cccccactat tattgtgttg ctgtctatct catttctgag gtctagtagt
188701 aattgtttta taaatttggg agctccagtg ttaggtgcat atttatttag gattatgttt
188761 tcctgttgaa caaagccttt tatcatgatt tagtgtccct ctttgtattt tttaactgct
188821 gttgctttaa agtttgtttt gtctgatata agaatagcta ctcctggtca cttttggtgt
188881 ccatttgcat ggaatgtaat tttccaccct tttacctcaa ctttatgtga gtccttatgt
188941 gttagttgag tctcttgaag acagcagata cttagttggt gaatactttt tttttaaatt
189001 atactttaca ttctggggta catgtgtgga atgtgcaggt ttgttacata ggtatacaca
189061 tgccatggtg gtttgctgca cctgtcaacc cgtcatccac attgggtatt tctcctaatg
189121 ctgtccctcc cttagctccg catccccaaa caggccacag tgtgtgtaca tgtgttctca
189181 ttgttcaact cccacttatg agtgagaaca tgtggtgttt agttttctgt tcttgtgtta
189241 gtttgctgag aatgatggtt ttcagcttca tccgtgtccc tgcaaaagac attaactcat
189301 ccttttttat ggctgcatag tattccatgg tgtatatgtg ccacattttc tttatctagt
189361 ctatcattga tgggcatttg ggttggttcc aagtctttgc tattatgaat agtgctgcag
189421 taaacatacg tgtgcatgtg tctttacagt aaaatgattt ataatccttt gggtatatac
189481 ccagtaatgg gatggctggg tcaaatggta tttctagttt cagatccttg aggaattgcc
189541 acactgtctt ccacaatggt tgaactgctt tagactccca ccaacagtgt aaaagcattc
189601 ctatttctcc acatcctctc cagcacctgt tgtttcctga ctttttaatg atcaccattc
189661 taactggcgt gagatggtat ctcattgtgg ttttgatttg catttctcta ataaccagtg
189721 atgatgagcc tttttttcat atgttggttg gctgaataaa tgtcttcttt tgagaaatgt
189781 ctgttcatgt cctttgccca ctttttgatg gggttgtttg atatttcttg taaatttgtt
189841 taagttcttt gtagattctg gatattagcc ctttgtcaga tggatagatt gcaaatattt
189901 tctcccattc tgtagattgc ctgtccaatc tgataatagt ttcttttcct gtgcagaagc
189961 tcttcagtgt aattagatcc catttgtaaa ttttggcttt tgttgccatt gcttttggtg
190021 ttttagtcat gaagtctttg cccatgccta tgtcctgaat ggtattgcct aggttttctt
190081 ctagggtttt tacggtttta ggttttaggt ttaagtcttt aatacatctt gagttaattt
190141 ttgtataagg tgtaaggaag gaatccagtt tcagctttct gcatatggct agccagtttt
190201 cccaacacca tttattgaat agggaatcct ttccccattg tttgtttttg tcaggtttgt
190261 caaagatcag atggttgtag atgtatggcg ttatttctga ggcctctgtt ctgttccatt
190321 ggcctatata tctgttttgg taccagtacc atgctgtttt tgctactgta gccttgtagt
190381 ataagtttga atcaggtagt gtgatgcctc cagctttgtt ctttatgctt aggattgtct
190441 tggctatgca ggatattttt tggttccata tgaaatgtaa agtagttttt ttcaagtctg

190501 tgaagaaagt caatggtagt ttgatgggga tagcattgaa cctacaaatt actttgggca
190561 gtatagccat tttcacgata ttgattcttc ctatccatga acatggaatg tttttccatg
190621 tttgtgtcct ctcttatttc cttgagcagt ggtttgtagt tctccttgaa gaggtccttc
190681 acatcccttg taagttagat tcctaggtat tttattcact ttgtagcaat tgtgaatggg
190741 agttcactca tgatttggct ctctgtttat ctgtttttga tgtataggaa tacttgtgat
190801 ttttgtacat tgattttgta tcctgagact ttgttgaagt tgctaatcag cttaaggaga
190861 ttttgggctg agacaatggg gttttctaaa tatataatca tgtcatctgc aaacagagac
190921 aatgtgactt cctcttttcc taactgaata ccctttattc ctttctcttg cctgattgcc
190981 ctggccagaa cttccaatac tatgttgaat aggagtggtg agagaagaca tccttgtctt
191041 atgctggttt tcaaagggaa tgcttatgct tccagttttt gcccattcag tatgatattg
191101 gctgtggatt tgtcataaat agctcttatt attttgagat acattccttc aatacctagt
191161 ttattgagag tttttagcat gaagggctgt tgaattttgt tgaaggcctt ttctgcatct
191221 attgagataa tcatgtggtt tttgtgattg gttctgttta tgtgatggat tacatttatt
191281 gattcgcata tgttgaacca gccttgcatc cccaggatga agccagcttg atcatgttgg
191341 ataagctttt tgatatgcta ctggattcag tttgccagta ttttattgaa gatctttgca
191401 ttgatgttca tcagggatat tggcctgaaa ttttcttttt ttgttgtgtc tctgccaggt
191461 tttgctatta ggatgatgct ggcctcataa aataagttaa ggaggattcc ctcttttat
191521 attgtttgga atagtttcag aaggaatgat accagatcct tttagtactt ctggtggaat
191581 tcatctgtga atttgtctgg tcctggactt tttggttggt aggctattaa ttgctgcctc
191641 aatttcagaa cttgttattg gtctagtcag ggattcaact tcttcctggt tcagttttag
191701 gagggtgtat gtggctagaa acttatccat ttcttctaga ttttctagtt tatttgcata
191761 gaggtattta tagtattctc tgatggtagt ttgtatttct gtgggatcgg cggtgatata
191821 ccctttatca ttttttattg catctacttg atttttctct cttttcttct ttattagtct
191881 ggctagttgt tgatctttca aaaaagccag ctcctggatt cattgatttt tttttttttt
191941 gaagggcttt ttgtatctct gtctccttca gttctgctct gctcttagtt acttcttgcc
192001 ttctgttagc ttttaaattt ttttgctctt gcttctctag ttcttttaat tgtgatttta
192061 gggtgtcgat tttagatctt tctttctttc tcttgtgggc ttttagtgct ataaatttcc
192121 ctcaacatac tgctttcaat gtgtcccaga gattctggta cattgtttct ttgttctcat
192181 tggcttcaaa gaacatcttt atttgtgcct taatttcatt atttacccag tagtcattta
192241 ggaacaggtt gttcagtttc gatgtagttg tgaagttttg agtgagtttc ttaatcctga
192301 gttctaattt gattgcactg tggtctgaga gactgttatg atttccattc ttttgcattt
192361 gctgaggagt gttttacttc caattacgtg gtcaatttta gaataagtgc gatgtggtgc
192421 tgagaagagt gtatattctg tttatttcgg gtggagagtt ctttagatat ccattaggtc
192481 tgcttggccc agagctaagt ttaagtcctg gatatccttg ttaattttct gtctcgttga
192541 tctgtctaat attgacagtg gggtgttaaa gtctcatgct attattgtgt gagggtctaa
192601 gcctctttgt aggtctctaa ggacttgctt tatgaatctg ggtgctcctg tattgggtgc
192661 atatatattt aggatagtta gctcttcttg ttgcattgat ccctttacca ttatgtaatg
192721 ctcttctttg tctttttttg atgtttgttg gtttaaagtc tgttttgtcg gagactagga
192781 ttggaacctc tgcttttttt tgctgtccat ttgcttggta aatattcctc catcccttta
192841 ttttgagcct atgtgtgtct ttgcacgtca gatgggtctc ctgaatacag cacaccaatg
192901 ggtcttgact cttaatccaa tttgccagtc tgtgtctttt aattggggca tttggcccat
192961 ttacatttaa ggttaatatt gttatgtgtg atttgatcct gccattatga tgctggatgg
193021 ttattttgct cattagttga tgcgatttct tcatagcgtt gatggtcttt acaatttagt
193081 atgtttttgc agcggctggt actagttgtt cctttccatg tttagtgctt ccttcatgag
193141 ctcttgtaag gcaggcctgg tggtgacaaa atctctcagc atttccttgt ctgtaaagga
193201 ttttatttct ccttcactta tgaagcttag tttggctgga tatgaaattc tgggttgaaa
193261 attattttct ttaagaatgt tgaatattgg cactgtcttc tggcttgtag ggtttctgca
193321 gagagatcta ctgttagtct gatgggcttc cctttgtggg taacccaacc tttctctctg
193381 gctgccctta atattttttc tttcatttca accttggtaa atctgatgat tacatgtatt
193441 ggggttgctc ttcttgagca gtatctttgt ggtgttctct gtatttcctg aatttgaatg
193501 ttggcttgcc ttgctagctt ggggaagttc tcctggataa tatcctgaag aatgttttcc
193561 aacttggttc cattctcccc atcactttca ggtacaccaa tcaaacatag atttggtctt
193621 ttcacatgat cccatatttc ctggaggttt tgttcatttc ttttcactgt tttttatcta
193681 atcttgtctt cttgctttat atcattgaat tgatctccaa tctctgatag cctttcttct
193741 gcttgatcga ttcggctatt catactcgtg tatgtttcat gaagttctcg tgctgtgttt
193801 ttcagctcca tcaggtcatt tatgtcctct aaactggtta tttcagttag caatttgtct
193861 aacctttttt caaggttctt agcttctttt cattgggtta gaacatggtc ctttagctcg

193921 gaggagtttg ttattaccca ccttctaaag cctacttctg tcaattcgtc aaactcattc
193981 tccatccagt tttgttcgct tgctggcaag gagttgtgat cctttggagg agaagaggca
194041 ttctggtttt tggaatgttc agccttttcg cactggtttc tccccatctt cgtggattta
194101 tctacctttg gtctttgaag ttggtgacct tcggctgggg tctctgagtg gtcctttttg
194161 ttgatgttga tactattcct ttctgtttgt tagttttcct tctaaccatc aggcccctct
194221 gctgcaggtc tgctggagtt tgctggaggt ccactccaga ccctgttcac ttgggtatca
194281 ccggcagagg ctgcagaaca gcaaagattg ctacctgttc cttcctctga aagctttgtc
194341 ccagagggac atccaccaga taccagccag agctctcctg tatgaggtgt ctgtcagccc
194401 ctactgggag gtgtttccca gtcagtatac ctgggggtga gggactcact tgaggagtca
194461 ctgtgtccct tatcagagct ggaacccagt gttgggaaat ctgctgctct cttcagagct
194521 gccacacagg gctgtttaag tctgctgagg ttgcacccac aaactcccct tcccccaggt
194581 gctctgtccc aggaaggtgg gggttttacc tatgagtccc tgactggggc tgctggcttt
194641 ttttcagaga tgccctggcc acagaggagg gaatctagag aggcagtttg ggtgcagtgt
194701 ccttactgag ctgtggtgag cttcgtccag tttgaacttc ctggcaactt tgtgtacact
194761 gtgagggtaa aaccccctac tcaagcctca gcaatggcag acgtccctcc ccccaccaag
194821 ctcgagcatg ccagatcgag ctcagattgc tgtgctggca gcgagaattt caagccagtg
194881 gatcttagct tgctgggctc cgtgggagtg ggacccactg agccagacca cttggctccc
194941 tggcttcagc cccctttcca ggggcgtgaa tggttctgtc tcactgacat tccaggcacc
195001 actggggtat gaaaaaacaa ctcctgcggc tagctctgtg tctgcccaaa cggcagccca
195061 gttttgtgtt ggaaacccag caccagggcc ctggtggcgt gggaactgga gggaatctcc
195121 tggtctgcag gttgtgaaga ccatgggaaa agtgcagtat ctgggccggt gtatgtggta
195181 cagtccatag tggcttccct tggctaggag agggagttcc ctgatccctt gcacttcctg
195241 ggtgaggcaa cgccccaccc tactttggct tgccctcctt gggctgcatc cactgtccaa
195301 ccagtcccaa tgaaatgaac tgggtacctc agttggaaat gcagaaatca cccaccttct
195361 gcatcgatct tgctgagagc tgcagactgg agctgttctt gttttgccat cttgccagca
195421 aatcggtgaa ttgttatgta ttctgcaatt ctatatcttt taagtggagc atttaggcca
195481 tctgcattca atgttagtat tgagatgtga ggtactattc tattcatcat actatctgtt
195541 gcctgaatac cttggttttt tatttattta cttatttatt gtatttttgt tttaatgggt
195601 cttgtgagac tcatgattta gagattctgt tttgatgtgt ttccatggtt cgtttcaata
195661 tttagagctc ctttcagcag ttcttatagt gctggcttgg tagtggcaaa ttctcttggc
195721 ctttgtctga aaaagactgt atctttcctt catttatgaa gcttaggata caaaactgtt
195781 ggctgataat tgttttgttt aaggaggata aaaataggac cccagtcccc tctagcctgt
195841 agggtttctg ctgagaaatc tgctgttact ctgatagatt ttcctttata ggctacctgg
195901 tgcttttgcc tcacagctct taagattttt ccctctgtct tgactttaga taacctgata
195961 actataggtt taggcaatga tctttttgca atgaatttcc caggtgttcg ttgaacttct
196021 tgtatttgga tgtctacatc tccagcaagg ctgaggaagt tttcctctat tattccccca
196081 aatatgtttt ccaaacttag atttctcttc ttccttaggg actccactta ttcttaggtt
196141 tggtcattta acataatccc aaacttcttg gaggctttat tcatttttta aaattctttt
196201 actttccagt acattttgca tttctctgtg ttctttactt cctgaagtag tgattgtttt
196261 atatttatgc tatctatttc actgaagatt tctcccctca tatcttgtat catatttttt
196321 tatttcctta agttggactt cacctttttc tggtgcctcc ttgattagct tactactcga
196381 ccttctgaat ttttttttct ggcaattcag ggatttcata ttggtttgga tccattgctg
196441 gtgagctatt gtgatttttt gatgatgtta aagaacatta ttttgtcata ttaccagaac
196501 tgtgtttctg gctccttctt atttgggtag gctatttcag aggaaagatc tgaagctcaa
196561 ggctgctgtt cagattcttt tgtcccatgg ggtgttccct tgatgtagta ctctccccct
196621 tttcttagga atgtggcttc ctgagagcca aactgtagtg attgttattt gtcttttgga
196681 tctagccacc cagtggagct actggtctct gggctggcac tgggggatgt ctgcacagag
196741 tcctgtgatg tgaaccatct ttaggtctct cagctgtgga taccagcatc tggtccagtg
196801 gaggtggcag aggagtaaaa cagactctgt gaggtgcctt agttgtagtt gtttaatgca
196861 ctagttttgt gctagttggc ctcctgccag gaggtggcgc tttcaagagt acgtcagctg
196921 tggtagtata ggggaggatc aggtggtggg tgggggccct agaactccca agaaaatatg
196981 aactttgtct ttagctacca gggtgggtag ggaaggacca tcaggtggga gcagggttag
197041 gtacgaatga cctcagactc tccttgtgtg gggcttgctg tggctgctgt ggggataggg
197101 gtgtggttct caggtcaatg gagttatgtt cccaggagga ttatggctgc ctctgctgtg
197161 tcatgcaggt tgtcaggaaa gtggagaaag ctgatagcct cacccagctc ccatgcaacc
197221 caaaaggctg gtctcattcc cactgtgccc cctccaacag cactgagatt gttttcaggc
197281 agtgggcaag tagggctgag aacttgcccc aggttaccag cctcctggtt gagaaagcaa

197341 gcggggcttt tgtgccacct ccctgcctgt caccggattc acatcctccc ccaagttctg
197401 gccaggaaac ttcacatttg gttggaatcg ttacagagtt cagctggagg tttccttctc
197461 cctgtggtct tttcccagtt cctctggcag ccctcctcaa ggactcctgt gagacaagtc
197521 agaaattgct tctctgggga cccggagaac ccacatggct tttcctgtgg cttcttctac
197581 ccttgtgttt cactcagctc tctaaattgt ctcagctcca ggtaaggtca aatctttctc
197641 ttgtgatctg gacctttagg ttccccagtg agtgtgtata ttcggggcag gggatgatct
197701 ccctttccca cttttgcagt ttgggcactc acagtatttg ggctgtctcc caagtcctgc
197761 aggagcaacc tgtttccttc aaatggtctg tggattctct tgactttccc agtatatttc
197821 tgcagcagtt cttggaacaa aagttcacaa tgcaagtctc cacattctat tctatccatc
197881 caagtggggg ctgcaattta gtcctgactc ctatctgcca ttttttccta gcagttcatt
197941 ctggattatt tttaaccaat aactcaacca aagcacaggc cttcagttct tgtactttac
198001 aggtatcttg agtatagcaa tttttcatct gcagaatgga gagtatatag gcaattaatg
198061 catctagatt ttgtccatca aaaggtaaca ttgacatccc atttttctgc tattttataa
198121 gttcatacca ggtgtcaagc attatgctgt gggcaagttt cttagtctct acatccttga
198181 gtctccttac tatgaatgga gataaaagta ctactgcata aagttattga gataattaaa
198241 taagctcata gatacaagca tgtaacatgg tacctagcat aataattttc tttatccctc
198301 ttaaccctcc ttcaaggtag gttttactat tttgacttta catataagga aactgaaact
198361 ctgagaggat aaataattgt cagacaatta ataagtgata gatccacaat ttaagcttgt
198421 tgtgtggaaa cttaccttgg tatgatggag gtctctaagt acactaccaa gcaagctctc
198481 ccagaagtca caaacctgaa gttcacatac caaattaaaa tgctcaaatc acatggctta
198541 gtaagcttat ttaaagcaca gtgagaagta aggggaaaga gatgggttat ggtaagacat
198601 gggataaagt agcaatatcg tgagatcatg ggcaacagga ccacacttct gtatcctgtg
198661 ttctgcagtg ttcgtgtgcc ctgtctctct catcctgctg atagtgtggc taaggggact
198721 gaggtcccaa tgggggccac ataataaaga ttacttgggc caatgatagg tacaagctcc
198781 attggaatga atgacacagg acaagtacgc ctgatcatag cagtagcttg tgattagcct
198841 gctgaaattg caggagttgt gtctgcattt cttggacaga gcagatgcaa gaggaacaaa
198901 ctggatgaca ccaatggctg gtcaagttag cagtgacagg tatctcagtc ccaaagtgtc
198961 aggataatga tttggtcttt tcatctcttt ctaactgtaa tgttcttgtt tgttcatcat
199021 atatattttt aaaaatttat tttctgtctt tctttggcag atattacagg ttactaattt
199081 acctgctatc tgtagtgaac acatcaaaca tgcctatgca tcatatgtac atgtttactt
199141 attagctata tttaacatat cctatgtgca ggaccagcta cataatttgc atgatgtaat
199201 gtaaaatgca aatggggggc cccttgttaa aaaatactta agaatttcta ttattaagaa
199261 ttttaagatg atgaaagaaa ccagaatatg ccagctctaa atatgcctca ttgacgtaaa
199321 tatttttgag ctgaaggaaa ttaagaagca catgcagaaa agctctctgc ctcttctatt
199381 tgcctaaaag caggacagag aattacaaaa acaaaaggtc ttttatgttc ctcctcccct
199441 ttcctgccta aagacaagat gtaaattctc ctttacaacc tttaccagct gagagcctgc
199501 ttcagaaaaa tctgggaaca gacttgactc tttcccacag ttctctggcc tttggaagct
199561 tagggctgtt ttcctctttg tcctgtcact tctctacaat gtattgatct tgttgaatac
199621 tttgtattag ctgggattct aagccacact gccttgagtt acttttcacc tgcgtgatgt
199681 tcatgtatta atagacttgt ttgtttttct cttgttaatc tgtcatttgt tacaggagac
199741 tgtctcaatt aaaaacttac gagggttcag aaaaattata atttcttccc aacaatgaca
199801 atagcagagc ataaaactaa gcacagggcc catgtgagta tggggtcctg taacagtgca
199861 ggtcacaggc ctatgaaatt gcccctgcct atgaatttca cctgactatt ccaagccaag
199921 agctacttat tatctatgct gaacatttct aatgatttcc atcttttcca atagcagaat
199981 tcagtggtct caaataattc agccaactca cattatagaa ctcaagtcta tgtaactcca
200041 agtcactgtt catgcactat agtcatggag ttctttttaa atacctgtca tccccatgtt
200101 caaacccttc aatgtctccc tagtaattaa caaaatttag tcaattttgc ctaaaaaata
200161 tcggtgagct ccacaacctg aattccaact tactttccaa agcaaactct cattaattac
200221 tgatgatgac catgaggttt tggagaggga caaaaccatc tccaaatgtt tttgagtgtc
200281 tcctgtgtat cgtgtcctgt actagatgtt ttacacatca actcatttaa tccttatgag
200341 aacattgaaa acaaagttat ccccatttta cagatgagaa aattgagatt gatagaggta
200401 aggtcacctt acacagttat taactgtgag ggtggattaa aatccagatt tctctgtctc
200461 taaaacctca ggctttttct actgtgccat actacttcta atttgaactg tattttccag
200521 tgaaatgaga ttaatcttcc ctatagacat gccaggtacc tgttatcact ttattctggt
200581 atccgctcca ttttttggca tcctatgctt cttccatgcc tgactttatc tcccgtttga
200641 gcctctttct ctgccaacgc ccatagaaat aaaattcaaa tgaattctct tgattattca
200701 tttggctctt ctgttaggca gcattgtgtt gcatttttaa aaattatatg taaatctcca

200761 tttcagaaga gattttgagc tcctcagggc acgaaacgta tcacatagct ctttattgtc
200821 tcaagtacta gcatagtgct ttgtatgcaa tatttaatta atattttctg tgtgggtgaa
200881 aatgttaatg tcttttttgt tggacattca tttccaaagc agaacaatta acatgactgg
200941 atgtttaaag ggtctggatg aataggtgag tgaaggacag aaatgtgctg gtaaaaagcc
201001 ctgatctata gtgcttgcca atttccgtgg tgtaaatact tccactatag ccaattttaa
201061 gctaccaaca gttagagcca actcacaaaa ttcttgaaaa tttcataatc agtggtttg
201121 tgtgagccag cataagtcag cttcggtatc tctttgaggg atttaataat gaactcaact
201181 aaggtaaaac tgatctggaa caactgaaag cagaggttaa agtaaaataa ctctgttatt
201241 taatctataa tcaactatga aatgcttgaa ttagagctgc ctgtgagaag gaaaaacaat
201301 gattccataa tgtgaaggct ccagacaaag gccaaaagtg catcaatgac ttcttatccc
201361 agtttttaa actaaaaaa ctgctagcta aatgattttt gtttatatg actttgttt
201421 tatatgactt ttgtttatat gactttcctc ccaaatctgt tagcatgcat agagtttcag
201481 tttttctttc atcttctcct cttctgctca tttctctctt tttaaatttt atcataaagt
201541 ggaacacttt ttattcaaga gaaatcaatt cttgattgag atcagaaatc tcttgccatt
201601 cacattagag ttgatctacc acaattgcct gcttcatcat tttcattcta ttggaataag
201661 tatttacagc tataaactat cctgcatctt acatatttct attaaacaag aacaatgtcc
201721 gtgacagttt tctgtctcag gatttatctt attaatattt attgtattgt tatgagtgct
201781 tagcaaatta ttgtggttaa ctttaccaat gcctttcaaa taatattttg aaattttccc
201841 tgaaaggata ttgagaaaag ccaaatgtaa aaattattgt ttttttccct tgggatttgc
201901 atgttttgaa aacgttctca attgttaggg aagtcagctg taaaacacag gctaaatata
201961 cttttgtcta ccaagaattc cctttacttg tctgtttcgt gatttgttta atctttctta
202021 gttatgcatg taccatttta ccatagttac ctggagatat tcttgtctct gccttagttt
202081 ggcaggcttt tcaagtggct cactgagcta ctgtttctat ttcttttaga aagttgtgga
202141 ggcatttatt actctcataa ctgcctcaaa atctcaagca catggcctag ttaaatcatc
202201 ttgaggttct gcccatatgg ttgcagaagg atattagtca ttggaaaaaa tgggtctaaa
202261 atgataatat tcaaagatgt tttccaatat tcttggcaat cttattcact aatttccacg
202321 gacatttaat tagcaagaat ttaaagaacc ttgttttagt ttcctgctgc tgtaatagaa
202381 tatcacagac tggataatat ataaagaaag gaaatttatt gggcttatgg ttctggaggc
202441 tgggaagtcc aagaggcatg acactggcac ctggcaaggg tcatccaatg gtggaaggca
202501 tcacatggtg agcaaggacg agagacaggg agaaatgggg ctgtatttat ctttttatca
202561 ggagcctact ctgtgatact agcccactct catggtaacc acattaatac attcatgagg
202621 atagagccct catggccgaa ttatctctta aaggtcccat ctcttactac tgttacaatg
202681 gcaattaagt ttccaataca taaactttag gagacatagt caaaccatag aaatcttaaa
202741 cttcaaataa acagggattt tttttagtag cacagtataa aatgataata ataaatatca
202801 gatataaaaa atttctgaag ttctttttag atagataaat catggttact attatcttct
202861 tagttacatt tcattgcacc ctattttctg tggtattatc agtcaacata ttatatatag
202921 taagacacat taaattttct gagcttgcca gggcatcctg tcccctaaca gtttttgata
202981 atgatttatc aaatgtgtac tctttagaat tttagctgtc agtaacagtt gattttcaca
203041 acttttatta gactatccat gatgtttttt ggaaaggtag ttaaaaggat aaatacatta
203101 attatcataa aattatctag gaatgctttt aaatacattt gacataatac attaacacta
203161 aaaggaaagc aaatatgcac ctaataagca caaaataaga ttaatcagag aaatgtgtcc
203221 ttgctgtttt ggggaagagt tatccagatc tcaggtaagg tactttttgt ggagataacc
203281 ttctgtttta tgaagcttgt aacaagataa caatcaaggc atttttgtgg ccatgtttat
203341 gtatatgagc aagcaatggc aagtgagata ctagtttggg ggcttaaata agataaaagt
203401 agttaaactt tattaaaatg cacatattaa aataaatttt gaaatacatt gtattcaaat
203461 attgtattag ggttctctta gacaactcat aggatacccc cacacacaca ttttatgtgt
203521 gtatgtatat atatatatat atatatatat atatatatat atatatatgt atatacacac
203581 acacatgcgt acatatatat acaggagttt attaagtatt aacttacatg atcacaaggt
203641 tccacaacgg ctgtctgcaa gctgaggagc aaggagagcc agtcccaaaa tcaaaactga
203701 agaacttgga gtccgatatt cgagggcatg aagcatccag cacaggagaa agatgttgtc
203761 ggggaggcta ggccagtctc tcattttcag atttttctgc ttggtttata gtcactggca
203821 gctgattaga ttgtgcccac cagattaagg gtgcatctgc cttccccagc ccactgactc
203881 aaatgttaat ctctctctct ttttttttga gacggagtct ggctctgtcg cccaggctgg
203941 agtgcagtgg cacgatctcg gctaactgca agctctgcct cccgggttca agccattctc
204001 ctgcctcagc ctcccgagta cctgggacta caggtggccg ccaccatgcc cggctaattt
204061 tttgtatttt tagtagagac agggtttcac tgtgttagcc aggatggtct cgatctcctg
204121 acctcgtgat ccacccgcct cagcctccca aagtgctggg attacaggcg tgagccacca

204181 cgcccggcct caaatgttaa tctcttttgg cagtacccac acagacatgc ctaggattaa
204241 ttctttgtac ccctcaatcc aatcaagttg acactcagta ttaaccatca caaatatcat
204301 aacaaatcgt gtgaaaggag gaaaggggaa tagttttaca tttcaaatat aaggggtcca
204361 agattccctt ttatagttat aaaaattaga aatttttaaa tggtatgaat cacttatttc
204421 aaatagttgt atttatgatg cttttgtaat atatatgcat aagtttacct atatatttat
204481 atatgtgtgc atgtacatat aatgtataaa tacacaaagg cactcatatc tacataatga
204541 tacatgattc tttggatgaa ttaactttta cattttatgc atacactctt attgtgtttc
204601 ttgaccagtg cttggtcaca gggatcagga caagttagaa gggaggctat gggaggacac
204661 tgtaaaatat taacacaact taattctgca gaaaggccag ttcacatagg aagatggtgt
204721 cctctgaggg tgcacttacc caagctgtgt tccccaattg cttaggacag tcttccatgt
204781 ctaagtttgt catgtggcaa acaatcaaat ttacctggta gtcctagtga tttgcagagc
204841 tcactgaaat gccatcgttt aaaagcatca ttttaaattt attgtccact gatagatcaa
204901 aagatagaat accctattcc aagagaaaaa ctaaagactt ctattagaaa aatttaggtt
204961 tagcacttct aaatatcact atacaacatt ttgaaaaatg ttttgaaaaa acagttataa
205021 tgcttagaaa catttagaag aatataagca cacaggtatt gtgttgaaac acattaccta
205081 cactttcaat gcaggatgct tcttctcact atggggcagc actttgtgaa tttttctgtt
205141 tcacagtatt cagaattcat tagaacatgt ttgattagaa tgcacatgag gcaagcaaaa
205201 ttagcacagt ctgtctatca agccacgtag atggccttgc aaagagctga atgctagtat
205261 atctaagaat atgagattat atgtatgatt catggtggaa atgtctcctt tgttagataa
205321 attcctgcaa accaatgcct ggaatgccct ctctgcctac tcgaattcta ttagctcttt
205381 aacatccatt tgaattctta tctcatctac aatatcttca gcaactctga agcctccatt
205441 gtcctctccc ttttcacttc aaggctgtct tgcattgtta tgtaactatt tcatgggcct
205501 agatctcatt tttcttaata aagtttaagc tgtagaaagg aattcatata tttccttgat
205561 ttcttttaaa cacttaacac attaagaaca taaaacatac ccagtaatta cttaaagaaa
205621 aaaaagacta agtaaaaggg acaatgaaag aataatagaa ataaaatgaa tttgtttatc
205681 agccagaatt ttgggtgggt cctcctcata gctatgtata gcaatctata atttcagtgc
205741 tatttctacc tacttctaat aaaaatattc ccacagtaaa ggggcaaggg gaaattccac
205801 cattttgcaa cattttggtg attagaattg ttgagatatg gttttagaca gagtttcctt
205861 cttattcttg ggctggtgat tttaagattc atttgtgtgt tttaggtcac attctcccca
205921 aatctcattt ttgctcaaca caagaacagt aagagtaagc tgggatgcct tagtgatatg
205981 gcctggaact ctgcagccta cataatacta tccctacctt caggtattag tgttgctaat
206041 aacatgctca actgattcat taaaacaaag gacaaaaaaa taaacaaaca taaaatcatt
206101 ttcatagagc ttaaagagac tatccagaga gacatttata aagttaccat tataaaatgc
206161 ttcacactaa aaaagaattc tttacaataa tttggttgta aattgtgtaa aattcttttt
206221 ccctccatca attttagatt tattggttgg agccttgtaa gtcagactga caaaagacag
206281 attaacaaga gaaaaacaag cagaagttta ttaccatgta tatccctctt attcatggga
206341 gatgagtaac tgaaagagga ggttagaact tgagctcaca tagcatctta acaaaagaac
206401 agtacatttt taaagaagtg acaagacaaa ggaaaaggac tttgagtttc taggatggca
206461 aatgatggga aagcaaatat atgggggaac taatggaaaa taaggactat ttagtaaagc
206521 ttgtcatgca gattcctctg gtgccctctc ttgactgata agtctggaat agtcttcagt
206581 gattaacttc tgtccttcca ggtaagaggg gaatgaaaat aataccttta tatatttatc
206641 tcctgctttt aggcaaaggg ggagagcaga gagcttttct tgtatctgct gcttcttgat
206701 tgctttcatc ttgaaataat ctttatacca tatttttggc acattttggc ataatatggc
206761 acataatcct gacatggcat attttggggt gccatattct gctaccctcc aaattgtctc
206821 tacccttcta ttatcttagc agataatagt acaacctccc aaattttggt ttccttctgt
206881 gaggaaaaca cagtttgtgc tgtgaccagt attgtagctc ctggggaaac ctgtgtggtc
206941 tcctctctct tctccctgct actgtaatac taacaaaaag aatgtttaaa ccataaattg
207001 aatcttaaag caatcaagta caaatcagag ttaggaaaag ttttaatcag caggtagaaa
207061 aaggatctag tgtgctttta tttttaaatt tttcattaat ccactaaagc ccagcttgtt
207121 atttttacct tgtttcatct aaaattaccc taagatgctg gcatgacctc tagggtgtga
207181 ctaaaatgtg tttgagtctt tatttgacat ttaagccagc catgaaaatt agcattggtt
207241 tagagctcca gggagcaaag aagtcatcct gagtctgaag aatttctgtg gctgtaactt
207301 ggaatttcat acaaatttga cctgtccaag acttgcttta attattcact aacaccagca
207361 tctggcttta acacatcttc atgagtatca tcagaactag agaaaaaaaa ttcccttaag
207421 aaatttttgt acataggtca ctctcatgtt tgttgctaat aaaaatttta gaggtcttag
207481 taatctaaaa aatatatatt ttacctgtaa agtggtacta tccaaaggaa actataccaa
207541 aatacgtcag acataaaagt taagaggtta tgaaaatgta catgcaattc tctttttgct

207601 tgttcttagt ttatttactg cttgttttca gatagtcagg tagttactct ttagagatgt
207661 aaaataaagt atggacatga aaagtagtta tttaccaaag gcaattatca agtatcaaaa
207721 catgcagagt acttactgcc agttcaggga gacaacaaaa caattatata taatatagta
207781 gctttttagc ccaatcttcc aataattgct aggccaacat gctaaattta agagttcttt
207841 acctcttaaa tttagtagtc tcagtaaata aaatttgttt agttctgcag gggcagaaag
207901 atgtgatact ttgtccctat cataaggtca aggacaacac ttctctaatg aaagagggtt
207961 cacaagagaa aaacataaca catttcttta accaaagttt tatatgacat ggaagccttc
208021 agaatgaaga tttgaagacc caaggaaaaa ctgtccattt ttatgcttag attcaataaa
208081 gtgtgaacag ccatgtagaa gtgttattag acaaacaggg aattatctaa tagtaatgaa
208141 ctgagtgggg aaacccagca atacctgtcc agattcttct tggcctcact gttgtaacat
208201 ctcttcaccc tcctggctta tttggtatag ttccttttag acagtactac ttcatagata
208261 aaatatatat attttgatta caaagactac aatttttgtc agcaacaaac atgagagtgt
208321 ttcttcctcc cttgacctac tgttgaccaa gactaggtca tagaatttct ttatggccac
208381 cttctagaca gaaaggtaga gaaggttaga gtaataattc ttgtttttat ggccggcttt
208441 ggggaaaagg gggttttagt ttctacggcc tgccctgggg aaggggaatt ctgaattcta
208501 tggctggttt tggggaagaa caaggggtga gaggtaggag agcaggagaa ggtcagagag
208561 accttggctc tgatgctgct actgaggcct tccagtgtcc tttagttcaa ggtgctcggc
208621 atgccaaagc agcacattca ggatatcatt ttctgagccc aacagttcca tattctgccc
208681 ccaacttgtt gcacattgga attgttcatg gacttacaaa aatattgatg ccatatccca
208741 gccctagaga ttgtgactta atttagctgt ccccaacctt tctgatacca gggaccagtg
208801 tcatggatga caatttttcc atatactggg gctgggtgag gagatggttt caggatgatt
208861 caagtgcatt acatttattc tgcacattgt aatatataat gaaataatta tacaactcac
208921 cataatgtgg aatcagtggg agccctgagc ttgttttcct gcaactatac gatcccatct
208981 gggggtgatg ggacacagtg acagatcatc aggaattaga ttctcataag gagttcacaa
209041 cctagatctc tcgcatgtgc agttcacaat agggtttgca cccctatgaa aatctaatgt
209101 gactgctgat ctgacagcag gaggagctca ggtggtaata tgagttagca atgggtagtg
209161 gctgtaaata cagatgaagc ttcactcact cgcccaccac ttgcctgctg tgtggcctgg
209221 ttcctgacaa tcatctggtc ttctcactgg tattcatttg ttcaattagc atttgttgaa
209281 attcacatct atgccaggtg ctaaacccat atctgtgcca gtggccacga cctgacagtc
209341 atctggtctt ctcactgatg tccattcatt caattagcat atgttgaaat tcacatctat
209401 gccaggtgct atattaagta ctgggaaatt agtacaaaaa atatagacct tggagattcc
209461 agtgcaatgg taaaaatgga ctagaaagca acacactggg ataactactg cagaaaggtc
209521 agaggttaag tcacactacc tagggggtgg aggagattgt tggaaagaaa gaaaggatca
209581 ggaaaggttt tgtggagaag ctgtagtgtt ggcctaactc tagaactgaa gataaagaaa
209641 aggaagaaaa taggtgtagg agaagagaaa gggtatctta gagaaatgaa acagcatatg
209701 taaaagcctg gatgcattag aaatcaactt gcagccagcc tctcatctta tgttttctct
209761 aacttatctt gtatatcatt gttgaaacat ttttctcaca gcactgtatt acacccatca
209821 atttcctgct caaaaatact ctggatcctt actaataaca gaatgaattc tgcactgcag
209881 acgtgacatt agggcatgcc agatcctcct ctaaagcatt taaactgtcc tcttcattgt
209941 ctatctgaag accccaggta tgttttcatc tcttcatttt tgcttatatc attcccctgc
210001 accagaatgt ccctcctact cctttgacca tgtaactgaa tcctcccttt gttcagggtt
210061 ccttgatacc actccatctc tcatctcaat cttttagcat ttatgtttac ttttactatt
210121 cttgtttgtt ggtaattaga tggtgcccta aatatttatc tatatcttta ctttttaatg
210181 aaattgtaaa ttccttacag aaaagcatag gaccacattc ctttatctct ctttctgcat
210241 atggtacaat gccttaaaat aaacaaaatt tatttttatt actgataaca aaactgaatg
210301 acaataatat aatgagtttg gaaaatggag aaaccaagca tttgcataat tgcctagtct
210361 ttgatcattg atgcacacta cttttacata attataatga gagtatacaa attgttctgt
210421 attctgaaat tttacttgac attgtatcat caacatttgc tatgaaaaat aataaatatc
210481 agctgggcaa ggtggctgac acctataatc ccagcacttt gagaggccaa ggtgggcgga
210541 tcacttgagg tcaggagttc gaaaacagcc tggccaaaag ggtgaaaccc tgtttctact
210601 aaaaatacac aaaaactagc tgggtgtagt ggcacaagcc tgtagtctta gctacttggg
210661 aggctgagac atgagaatct cttgaaccca ggaggcagag gttgatttga gccaagattg
210721 caccactgca ctccagcctg ggcagacaga gtgagtctct gtctcaaaaa aaaaaaaaaa
210781 aagataaata tctttggata ataactaaaa gcatcattta cctggtgtgc caggtactgc
210841 tttgcttaca ctgtttcaac ttttgtatgt atgtccttat tccaattcca tgaggtaaca
210901 ctggtgttat cctggggaaa ctgggagcac tgagctcaaa aagagtagag ctgtgctact
210961 tactaggtga ccttggatac ttattttact tagcgaatcc tcagtttcct tatctctgag

211021 atggggataa taatggttcc cacctttcaa gattttgagg ggatgaagtg agatgatgga
211081 cacgaatagt gcctggcaga tggccaacac tcagtgtgct ttagctcttg ctgccgtcac
211141 tgttgctggt cccattttac aagtcaggaa actggatcaa aggcatttaa taactctccc
211201 caaatcacac acctggtgtg tgacagagcc aggattcaaa cccctgtccc aattatgaca
211261 tgagataggg atcacagaat ttaatacgat gttgtccaag tgtaacattt tacaatttag
211321 tgctacagag aacatgtgca gagttttgag agaaaacaca gttggatatc tacagattat
211381 cttctgcttg gagagaaaga gaaattgaga gagatagaga gagagagatt cccaggggcc
211441 cgagctctct acagcagcac agcatggctg ggtcagggct caggatagaa ttcctgactg
211501 cctcctccca ggcggtgtca taactggggc ttctgaagcc caacacctgg gccctctcct
211561 tgggtctcaa atggagggtt gaatcctctc atgctcactt actaacaaga acaattccca
211621 ctttcaatgg ggtaacagtc tgaggggaaa tgggctaggg ttttgcctgg aagtacctag
211681 ttatttctta actttgtagt ttggagattc agcataaaaa ctgaagtcag ccctgttagt
211741 agtattgctg cttatacagc cgtccaccct tatcagaggg gaataccttc caaggctccc
211801 cgtggattcc taaaaccaag gatactactg aacctgattg ctgtcaatca gaacatattc
211861 ctgtttatgt cttccaccac acatttaatg ccttttctat cttaactaac cacttatcac
211921 acactgtggc cataactttt gcagtttgaa gtacaacaga aaaactagca agaatttctt
211981 ttcccttctt cacaatttca ggaagagaag acgtcttacc atagatctta gcaacctcag
212041 catacttttt tttttctgcc ctcattaggt tgagaacttt cccttattca cttaaaggag
212101 gcactttgtg gcttctcttt ggcatatcca aattgccagc atcactaaac ttatgctttg
212161 ggggcattat taagtagaat aagggttact ggaacataag cattgtgata ctgtgacagt
212221 caatctgaga acccagaggg ctactaacag gaggagagtg cagactgtgt ggatttgctg
212281 aacaaaggga agattcactt cccaggctgg acagcacagg atggcatgag atttcatcat
212341 gctactcagg acagcacaca atttaaaact tatgaattgt ttatttctgg aattttccat
212401 taaatatttt tggactgcag ttgactactt gtaacaaact gtggaaggca aaactgcaga
212461 taagggggaa ctactgtaat agttactgct tatactgctc ttgctatttg ccatggacta
212521 agctaaacac attacataca ttattttcta taatggatgt gacaatccta cagagtagct
212581 actattatct ccattttaca gatgaagcaa ctgggcatct taaccattcc agctactgcc
212641 atcagcattt gccttaggaa aaaaatgtgt tttgaataat cacttccagt atttgggatg
212701 attttcaata attctaccag cctaactatg aagttatatt atctaggagg cctctagttc
212761 ttatttttaa tctttgtacc ttcgagtagc ttcttacctc tgtaactcac tcccacagta
212821 atatttcatg agaagccaga ttctctggac tattggttaa attcttttag atggcagtgt
212881 ttgttatgaa aagacttttc attcttctgg tcccttcact ggaaacagct agaaagtaca
212941 agttttgatt acagttgcct aaacaccagt tttgaaatgt ctttcatttg cttgagcaat
213001 aaatttgttg ctcaatcata ctatcaaatt gaaaacaaga ggaaaggaaa gtcaacctaa
213061 cctaacccaa aaggggaaca tgtttcctgg gatgagaatg tgtcaaaggc agcatagcat
213121 gacctaacac ttctgtgtaa tcgtggttgt atcatgtgtt atgggtaaat tatctattct
213181 gtaagttagt ccagtgtgtg cttcttcaac taactgtgaa agcattcttg aaagaccatg
213241 aaaccttatt attctaatta ccaacacaac ggattgataa atgaatgatg aggccaggta
213301 ttattgtcat taaatagacc tcgctcaatt atttatcaat gagtctgggg ttacagctca
213361 gagagctgtg catattgtag cacggtttgc ttcagctctg agacctgaag accagattac
213421 agtcttggca ggtggctttt gttgaatgca attcaagggg gtggtacaca tttgtttcag
213481 tcagacattt ggatggtttg aggccaacag atattcaaag tttatccaca aaagcctatt
213541 tcattttaac catgtagaaa aatctcttta atttattcag ataaatacaa atgagtagga
213601 gaatggatta gctgtagaaa atcatacatg taaagatgtt ttcttgcatc ttagtgttct
213661 aaagactgtt ctttattttc ttagattctg ctatctgtca tggacacttc actgttctgt
213721 attgatcaat gtttatcttc atatgagatt tgtttttgta tcttctttct aattgtacaa
213781 tgaaatatgt tttctggtta agaaatacgt tatcaaggag gatagaattg ggtgatacta
213841 gtttttttcc atcctgaaat catgccagct cattgcaaag gtcagccctc aaaatagtat
213901 taaggcatgc cctctccttg agtctctgat accttgcagc ccaatgtccc ttcttgaagg
213961 cctgtcacct gcaagagtac tttcttacat cctatgagca tcagatatgg gggaatgaag
214021 aatctcttct tgctaaacaa atattcttgg tccaccttct gccccctggc tttattttaa
214081 acccatcccc aacatatctg atgagttgag tctagtaatt ctgtcttcag tgggctatgg
214141 ggagcaggga ttagctaaaa tggctgttca gggacctctt tggtataaaa cttggtgggg
214201 gtgctggcag ggatctctca gagaatgaag gaggggatag agcattagag aagaaaagaa
214261 attcatacct gagattatgt aatagataat agagtctgga ctttgtttaa aaaccagaat
214321 ctaagatata tatgttgagg ctagggcggg gtagtaattg aaagagaaat atgaaactat
214381 acctagcgtc aaggaaaact aacagtaatt gaagagacaa aaataaaact tgtttatatt

214441 gttcaaagga aaatgatatt tataacatct gacaaaaact ttattaatgc catgtagaca
214501 gggttttata ttattaactg agtttataat taattttgta ctgattttaa tttgactgtt
214561 accttggctt ccttctctgt ttcttaagtt ttgctagttt aatgttcacg agctcacaaa
214621 attaaaaatc ttggtagaaa tatggagtag ctatttcttt acatcctttt tagaacaaag
214681 ttgtgctttc gaattgacca cttatctaga aagtgatata aatctaataa gcaaaactgg
214741 atgtctttta aattatctgt taaataacta gagcagtgga gatgtcctga aagtgagcac
214801 tttattacat atgaaaaaca aattttaatt ttactactaa gaagaatttc catttctcta
214861 tttatatgtg ttcattattc tagagattaa tatgataact cctatgtata cttagttttg
214921 tgaatttcaa aattcttgat gccaaataac tgaagtatag taaattctgc cgtaacatga
214981 tgtatgggtt cctaaaaatc ccgacactat gcaaaattgg gcaataaaaa cacattttac
215041 taaaaaaatg gggataggca cacaatacct agaaatatca ctagtggcac attacaaaaa
215101 atatgggaat ttagtaacaa tgacagcaat atttacacaa tgttaatggt taagaaattt
215161 acaataaata tggcagttta ctttgaaaaa gacctaacgt tccctgtgga agttggcctt
215221 caaaagggtg cagcttgtga gtttttgaga agtgttggga ggagagtcgt tgttgtccca
215281 gttgtgagca ggtggggctt ttctacctgt gtggggaagt gtggtagctg atggatatgg
215341 tatgtggatt ttgtgtattc ctacgctgct tggtttagct gggtgcagtt ttctgcattt
215401 acctggcgtt tcttgtggat agaatcacat ataagcaaat cccagacttg tgttgtgatc
215461 aaattgttcc gtaatatatc aatcatgtta gaacaaggtc acggtttcaa aaccggcatt
215521 gccgtagaat tgactgtgta ataatccctt caacacgttc acatctatga tcccaccttg
215581 ttcttgccac catcttagca agcaggggtg cgattgcctg accatttgtt tgttttctgg
215641 gaaatcttga cctattaaaa gccacacagt tactgaggaa acctgtgtca gaccttgcct
215701 aggtctgtgt gagtctgagt caggtttgct ttcctctgaa aaatcagaca ctcatgtatg
215761 tgggacaaag agcatcagaa acatgggcaa ggtcatagag acagatccca gagactggat
215821 tagaacccaa cttgcttgat tttctgctta gaggcttcag ttcaccattt tagaaagtgc
215881 ctgctggctg ccaccctccc tcctgtctta tcacctctaa aagacattgt taatgcttag
215941 acaggatgtg ttttcttcat tgcatggatc taaaaaccca gccatgagaa cttagttttt
216001 aatttttgcc attgctttat tggatatttt agaatgtgca aaaggtttta gaaattgttt
216061 ttcaactaat tttaccagca cagaggtcat gtcaaatgtc agggtgtttg acaacaaggc
216121 caatctagtc tatcaattga gtttcagaga agattaatga taaagggtgc aatcccaaag
216181 tctgggaatg actttgcttt caagcattaa ttattgtgga aaataaaatt attagatact
216241 cccttgtcta catctacccc aaactatgtc aatcagctct atttctagaa ggtttacatt
216301 tattaaacac tccctgtggt caatgcaatg gtcaatgcta tggaagttac aacaagacaa
216361 gatattgtgt ccgctatcag gaaactgatt tgggagttct gtttgatgac cgaagagagt
216421 ctttaccact gtgcctatat ttttatgcca gaatatgggt gggtgcacat gtacaggtga
216481 gtaatgaggc cccactccct agtattagtt aagttagcag gtgtgtgctg tttttttggt
216541 cactgtgtat tgcaaaattc tgagtttggc actgatacta aggaacatct tacagaaaaa
216601 attccccact gacccttctt ttttttttct ttttgaaaca gaatctcact ctgttgcccc
216661 aggctagaat gcagtggcac aatcacagct cactgcaacc tccgcctccc aggttcaagt
216721 gatcctcctg cctcagcttc cccagtagct gggactacag gtacacacca ccacgcctgg
216781 tcgatttttg tatttttagt agaggcaggg tttcaccatg ttggccaggg tggtctcgaa
216841 ctcctgacct caagtgatcc acccaccttg gcttcccaaa gtgctgggat tacaggtatg
216901 agccactgtg cctagccaga cccttcattt ttaaaagttg actttatatg ttaataggag
216961 tttaattata gtcatatgtt gaagggtctt ttctgtaaaa ccacatattc caggagccat
217021 ccaactgagg acagcattgc acttgtggaa ccacaatcca ggcttgggtt atctgtttct
217081 atcatgacag aggctactcc atcacttaaa ctatatgttc tgcagccccc gagagctgaa
217141 gacaagcctg gattgcctct atttgtaatt ttcttttttg taaatttttt tttggtaaat
217201 tgtttttcaa acaaacaaca aaaatgactg tcccagtcag ttcaagctga tagaacaaat
217261 ggccatagac cgggtggctt aaacaacagc catttatttc tcacagtgct agaggctgaa
217321 agtctaaaat cgaagtgcca gcatggtcgg gctctggcaa gggccttctc ccaggttgca
217381 gatggctggc tccttgtgtc ttcatgtagt ggaagcaggg ctagctagct ctctggccac
217441 tacttacaag ggcactaatt ccattcatga gggttctacc cccatggcca attacctcct
217501 aaatacccca cctccaaata ccatcatgtt ggagattaga tttcaacata cagattttga
217561 tagggacaaa aacattcagt ctataacaat gatcaaataa ggttacaaaa attatagtgt
217621 atgtagcatg cttgcattat gttttgatga attaatgttt tttattatgc ataccaaaga
217681 aactaatgca actgctgtaa atgtataaat atttgggagt aatgttaaag tttacattta
217741 aagccctttc taaatgtgag gccgaaagta ttccttttct ctctctttct cttctcaacc
217801 ccatttactt tacagaatga tttttctgtt ttaagtcata attgcttttt ttttacagga

217861 ttaagtacca ggtcattaag tgtgttctat gtcagtttga cctaacctct ctgccctttt
217921 cttttttcta aaggaattct ttaaaaagaa gaagaggaag tattttaaaa taaacatttg
217981 actgccagaa tgtgatgtaa acttcaggtt caattctaca cttgatagaa atgaaaagaa
218041 ctagtgttct ctattttgac atttagcatc aattcccttt cagtgattcc agctgattag
218101 taagtgagac taaaatatct tactcttcct ggtcagcatt taaaaattta aacatggatt
218161 gaaaatttct aggttatgaa atacagtcta ggttcacctg tgaaggatct agaagaaata
218221 agactgcaga cctggtaaat tcaaatgtcc actacttctg tattctagtc attgcaactc
218281 acctcctttt atcccttatg ttatttttat tttctcaggg aggcaaaaaa atttgtgagt
218341 gaaaatgaag gggctcttgg gaaaggaaaa ggaaaacggt ggtttgcatt taagatgatg
218401 atggccaagg taggtatttt tatagttgtc tggtttatgg catattgcct ctaagaagaa
218461 cactggttct tttctaaagg ggtgaaaaaa atcttacatt ttgtatatat aaaacacaaa
218521 tactcataca gtatatacac agtatatctg tggtattgaa atttacaggg gagatttaaa
218581 aaattgttct tagaagagtt aaaatgaaaa ataagtcaag aaatacagat ttatggcaaa
218641 agacttactt gtgtgacttt agcacttcca aatataaaag attgcatcct ttttcatttt
218701 cattctctgg aagtgaaggg gttctggtca agttgttttt ctcaaagtat ctgtatagtt
218761 tttttctacc aggaacatca actatacatt ggacagaaaa taagaaaaca ataaatgata
218821 tactgataca aatatagatt ttggggaaag acagaaaagc aactaacatt tatttaacac
218881 ttactatgtg ccaagcactg aattgaattc ttttataaag ttatatttaa ttcttacagt
218941 aaagtaggaa catagatatt attatctcct ctttacaaat gttataccaa ctgtaagcta
219001 aatgtattcc cataaagttt tgtgaataaa gttcatatat cctaactttc atatatatat
219061 atactcacat acaatagatt aattttgtct gtttctacag tttaaataaa ttgaatcata
219121 tagtatctat gcatttgttt ctggcttctt tgatgcaatg ttatgttcac aagatatatc
219181 cacgttgttg tgggagttat acttcatttt tattcatgtg ttgtagtcta ttgtataaag
219241 atgctgtgac aatgtattta cccattctat tgtagatgga catttgggct gtttctagtt
219301 atgagtaatc tttttttttt ttgttcattt ttaaaattaa ctcatacaca attacttgtc
219361 ttctggtttg ttgaaacaat aatgcagaca tttgctacaa caatgactgt taaaatggct
219421 ggccacaaaa tctccagcaa tacattaatc tgaagggcat tcttgatgaa gttgactaat
219481 tttgccattt tcatccacct tatagtattt caggacagcc agcttaacct tccttttctt
219541 atgcttattc ttctttggag tggtgtaaga cttcttcctt ttcttagcac caccatgaag
219601 tctcaacaca agatgaagag tagactcctt ttgaatgttg tacccagaca aagcacatcc
219661 atcttccagt ttcttgccaa caaagatcag tctttgctga tcagtagaaa ttcctgtctt
219721 atcctagatc ttggacttcg cattttctat tgtatccaag ggttcaaact ccagagtgat
219781 ggtctttccc ataagggttt ttaaagaaaa tctgtgtttt ggtggtggtt ccactgcaga
219841 aggcagatgg gaaaaaaagc gtgggtgatc ttttgaactt cagacttgtc tcatgctatt
219901 atttagacaa tagtgcctgt ccagctccca cttcatccca gcatttgcat acatgcaaaa
219961 ttctctgcca cattctcatt cttcctggtg gtcctcttga ctatggatga cccaacattg
220021 gagttaaata gaaagaaggc cttttgcaaa atgattttat gacttgttgt cttctctttc
220081 aattaagctg accaatctgc ctctaccaga atgcagtgtc tcatgtcata caacttacca
220141 tcttgtattg aactcaccta tttgtacaca gactatcctg gtagatcaga aggctttcaa
220201 gacaggacct ggaaataaaa gacagaaaat aaaatttatt ttgtttatta tttttattat
220261 ttattttatt taattattca tattgagtcc ttcatacaat gaaaactaat taaacacata
220321 ctctgttcca atcaccctat tatgtgctaa tcccctgctt tcttattgtg ttatataata
220381 aatagctgtt tctgagaggc cagcatattt tccataaaac aggggatcga taaacatttt
220441 tttgttgaat taaattggtc tggggttagt ggagaacagg gaaacaagtg aaacctactg
220501 tttccccctt tgactagaaa gtagtatttg ctgccagaga gacatttcca agcttactgc
220561 attgtaatgt tttgaggtgg gggataaaca tcttgacaaa tcaagaggtt ggactttact
220621 tttgtatttg actttctttt cagaaatggg caaaattcct ccgtgatttt gagaacttca
220681 aagctgcgtg tgtcccatgg gaaaataaaa tcaaggctat tgaaagtaag tccttatcag
220741 atctcaggtg gagaaggttg tcttagagaa gtatctcata agcttatcag atgcctttgt
220801 cagtttctgg acttttggga agactagaaa caaacaaaca aacaaacaaa aaccaaatca
220861 acaaaaactt tgtttatgcc tgctttcgtc actgtgaaga tttgctagca cttaccaagg
220921 acaatactgg ggaccccact ctcgtgttct accttagtcc aaaacattac cattctaacc
220981 tggttttctc tcctatacag ggaagtcact attgtgcaca atgacattga atcatcttac
221041 ttggaaggca gagattttat tttggcaatg aacatgacat tgagaatata tactctcatg
221101 aatgttagac agtaatcaaa atgaacaatg ttttaacaag tgaagaagtc ttagtctggg
221161 cgcagtggtt cacgcctgta atcccagcac tttgggaggt tgaggtgtgc agatcacttg
221221 aggtcaggag ttcgagacca gcctggccag cgtggtgaat ccccgtctct actaaaagta

221281 caaaaggccg ggcgcggtgg ctcacgcctg taatcccagc actttgggag gccgaggcgg
221341 gcggatcacg aggtcaggag atcgagacca tcccggctaa aacggtgaaa ccccgtctct
221401 actaaaaata caaaaaatta gccgggcgta gtggcgggcg cctgtagtcc cagctacttg
221461 ggaggctgag gcaggagaat ggcgtgaacc caggaggcgg agcttgcagt gagccgagat
221521 cccgccactg cactccagcc tgggcgacag agcgagactc cgtctcacaa aaaaaaaaaa
221581 ataaaaaaaa taaaaaataa aaataaaagt acaaaaaatt agccaggcct ggtgcaaaag
221641 cctgtagtcc cagctactca ggaagctgaa gcaagagaat cacttgaacc cgagaggcgg
221701 aggttgcagt gagccgagat cacactactg cactccaacc tgggtgacac agccagactc
221761 tgtttcaaaa aaaaaaaaaa agaaagaaag aaagaaaaaa aacgaagtct taaaacctca
221821 ttttcaaatc tctgaagtag aataagcaat agtcatccag gagcttgcta tctgaatatt
221881 cacattgtgc ctttattttg tagaatattc ataggaagga aggaaggaag gaaggaagga
221941 gaatatagcc aatcaaaaac ctatttagtt tagtgcttta ggcttagaat aaggcaagta
222001 aacattttaa aatattgtaa ataaaaatag taaattgttt tttaaaataa taatctaaaa
222061 aaagggataa catttgaaac aattttgagc acaatctttc catattctgg tttctaacgg
222121 tttcaactag gatctgattt ccatccattc ctctctagat tggaggcagg ttttcctgct
222181 ggttaagtgt ggcgttgtat aattaaaatg gaggctccat agggagataa cacagctgat
222241 ggatagcttg ctgacatgat tgggcatgtt ttaggagtgg ctaaccctcg gttccctgtc
222301 tctcaatatc agatgggaat tattaagtgg caatgaatta aaatacatca gctcattaat
222361 tgtatcccct tccacacatt cccagtgcca tttctctctt gctttaattt cctccttaac
222421 acttaccgct atctaaaatg ctatatcttt aatttaattt tttgatgtaa tcttaattca
222481 ttttatgttc atttctccca atagatggga aatttagttt tgttcactcc tgcattccac
222541 ttggcactta ataaatatgt attgaataaa attacagaca aactcgctaa atgtctacca
222601 atctaggaaa aaaagaaagg aatttagata catcaaaaaa gagaagacat catggatttc
222661 ttaggtttca actgtacagc tcaaatgtgg taggttgaga ggcactgaaa aacaaaaggc
222721 catttggagc attacggtcc tgatacaata tatatagtaa gaactctgaa tttggtcttt
222781 gctttgctgc agattagctg tttgacttct agccagtcat ttaactcctc tggacctcag
222841 tctactaatt tataaaattg tagggctgag tcttgatcag ttgttagata aatcccaaga
222901 aatttgtaat taatgatagg taaatatata catttgaaaa ttatttttaa agcacactag
222961 agcaggttcc agcctatcct aatagtaata tcatgctcat ttgtgttcca ataggctata
223021 gccagtgaaa caaaaacaga cagaattatt tttattgcca gaaattgcat gcagcaagag
223081 ttacacatat gaccaccatc catcatgagt gtcacagtag aaaagacaga ttgagagcta
223141 tccaataata accttcagtt atctcctctc ttcttctctt cttctagtaa atttggaaag
223201 ccacgctgga aaattgaggc aaatgtagat tatttttctt tccatatata atttatgatt
223261 tgattagttt gtcatgactt cttcactgtt aatggtgctt tgtctcaact gtgaagaact
223321 gagctttatt ttttatttta tcaatattca ccacaccacc atttattgag taccaccatg
223381 agtcagatgc tctacaaaca ttgtttcatt taatcttcag tgttcctgca atgtgcacat
223441 taaccttcct attttatagt tgtgattaaa taactgtccc agaattagta gcagagctga
223501 aatataaggc aaaatctgtc tgggttcaaa acgtatgctt ttccatgtgt taggctgctt
223561 tctcctttag ggaaggtttt acatactttt tctttttaaa tatgttttgc accagttcat
223621 aaaaagtatt ctacgaggcc gatggcagga tgagtagtta tagattttct tgatcgttat
223681 caaggaatta tttttcattt tctcatgtat ttacttttta gtgagtgatt tatttaatta
223741 tatgttttgt attctatagg atgcttaatg tactgatttt aatgataacc atgaggcttt
223801 gggctatagt attagttgaa aaaatatgaa acctatgtaa gaatatatta ttagagcact
223861 ggattggact atcttataaa aagtacccta ctaaatatag gtaaagcatg tatctgtgat
223921 cacctttgaa actgaactta atgcaaaatg ggaagaaata ggacatgcaa tggattttct
223981 gagttgagga ctttttgacc cctaccacct ctgtagtttg gacaatctgt tgaaatctct
224041 ggttctgttt ccacatttgg ggctactaca atatctacaa catcttgggt ttaactagtc
224101 actatacaag atcatttcca gctcaacatt ctatgatgtg taagttacag tcagttttac
224161 atgtatatgt aggcatgtga aattcattaa tcatactttt gattgataaa tcctcaaaaa
224221 tttgaatgca aaacagattt atactaagag gggactatat aattgccttc tcaaattctt
224281 tttgaaataa agttggttaa gtgtgtaccc tacttataga atacatattt aattataaaa
224341 cggatataaa tgcttttctc ttaaataaat ggccatttaa tcttacaaaa ggttcagaag
224401 tatcattgta ttccatttcg acatcagaat catctaatat atatcttatt taatcctcag
224461 aataagcctg acattattga ttaactgagc aaatagtgga tgcctaacat gtttctaggc
224521 actattgcta ctgccaaaga cagcagagac tctaccctca ttgatcttac atccaaatgt
224581 gttaatgtat gtatgcttgg atggggaaga cagaatataa tataagcaaa taaataatga
224641 cggtaattgt ggattgtagt aagtggcatg gtcaaaagag gagctgttgc cttccattcc

224701 caacagcctt tctaatccac ttttccccac tctctgctca gtatctagat aataccaaat
224761 aagtattttt aaaatgtggc taaaggagtc caaaattaac agcctttact gagttttccc
224821 aaagctctgt ccaataattt gcagaatcaa gagcggaccc caaattagag tctaaaattc
224881 atactgtttc catcttgatt ccagtaagac ctagatctct ttattagtgt gaagcggatt
224941 ctcacctgat gactcccaac tctgtttcac ttaaaaaata gatttgataa ttcctgccac
225001 agaggatact gtgacgttat gcatcacttg ggaatactgt ggggttttac tgtcacgtca
225061 gccttaaggg gatcatgcct aaaactcgat ggttgctcct gtggacacat cttattcatc
225121 tcattgaact gtgtattttt ctgaagcaac tgcatgtttg tacccccagg aaatataatc
225181 tatacccatt gtgatgccaa cattattatt aagaccataa attaaataat tgaatgtatt
225241 tctagctggg gattacaaat ttttctcagg atcaaaatct actttttcct aatagtagat
225301 tgcatcatca aaattatagt ttcagaaacc tatttttttt cagacaggaa atagctttgg
225361 ataggcaagc caaagcacag ggcttaaacc ttatgtttcc ttgctgccct aaggattcta
225421 gaaaatacaa atcaaatttg tcacatttaa ttccctaccc ctaaatagtt cgaaaaaatt
225481 gtgcttcaca ttctagccta acatgatggt atttatattg tagtcacaca gcaggtcatt
225541 taattggtct tttcccattt agaaatactc atatttatct gcttgtctta ttacccatct
225601 attcaagagg aaatgatgtg ttatgacaga ctacaaatta ctttgcctta ataataaaca
225661 accagaaaga gagttgaaat tttaacttta atttgaaaga aacaagaata ttcaggaaca
225721 agaatatgct taagcataaa gcctcagttt aatacgtatc agagactctg tttttggtt
225781 gttgttgttg tttgtttgtt tgtttgtttt ttgagacaga gtcttgctct gttgcccagg
225841 ctggagtgca gtggcgcaat ctcggctcac tgcaaacctc tgcctcctgg gttcaagtga
225901 ttctcctgac tcagcctcct gagtaggtgg gattacaggc tcctgccact atgcccagct
225961 aatttttgta tttttagtag agacggggtt tcaacatgtt gaccaggcag gtcttgaact
226021 cctgacctcg tgatccacct ggctcagcct cccaaagtgc tgggattaca ggagtgagcc
226081 accgtgcctg gctgagactc tgtttttata cattaataat ttactatgga agcagcattt
226141 atcagtctga ttaaagaaat aagaattggt cattatgggc atcaaatttg tgaatctgac
226201 tggctttagc tgattgatgg atattatcta tacctattgt ttcagaaaca catattcatt
226261 gcatgtttat tgtatatatc atagtcttaa gaaaattaat tgaactatgt tttacttaaa
226321 gggccatata gcagtgtggt taagagagat ctagatttgg agtcaaacac ctgtagctca
226381 agactgcctg gtttagaagc ctggcttcct tgtttactag ctatgtagcc tatagatcta
226441 agtctctgta aaatgggaaa acaatagttc ccatttcgta gggatttgat gatcattaaa
226501 taagttaata catatagatc aaattggtgt ttgaaacagt gattcactaa atgttaccta
226561 ttattaatca acttttgatt cagtcaacaa ctgtgttaaa gtttttgttt cctgattcat
226621 agtgggacta ataatactta acctcattaa gttctagtgg cagcataatg agttgatgct
226681 gataaagttc ttggaacaca taactgacca tcaagatata ctagctatta ttattattat
226741 tactcatttt gcttccttac actgagagat taagtatgtt aaaattgcta ttcttttcat
226801 ttatgctctt ttagtcacac taatcaggca tttcttttta gtgaacatca gttttagtta
226861 acaacatccg gtaggataga accactagat aaaagggata aagagggaag atttcttctt
226921 ttttgttttt gtgttgagac aggaaaaact ggagttttat gacaacttgt tgtttcattt
226981 tctctacttt aagatcttca tttgttttgt tactgtgttt tctcctctgc tattgatagg
227041 tctagtgtgg aagaaacttt gtcttctgga tttcttagtc tcttccacct gtgtctgggt
227101 ctttcctata atcgttctag ttttaatgaa atctattggc ctgcaattca ttttctttac
227161 acttaagatt ctgtaaagac tcaacctcca aataaaatag ttgtgaagcc actctcaaat
227221 ctgagaactg gagtttggaa gttttggact ctaaagtata tttcattatg atgcttaagc
227281 atgttggagg aggggatact ttctgaaaca tcttaattca cttctgtctt ttgcatttaa
227341 gtatatttgt accctacacg caagaaattc atttgtcagg gattgtgtgt aggtgctgag
227401 aggatgggcc gtggcaataa ttagaaaaac aaaagcataa aaagtgagga aggaagaaag
227461 gaatggtggg ttagtatcct atatgcggga tttcatactg gaagatctta catgcgattc
227521 cagggaaaaa cttacattga tacctagtga ccaaggcttt cctccagtaa atgagtaact
227581 gtaatgagta acaaactgta agtccactgc cttcaataaa acatgatgag tgaacctggc
227641 tgagcagccc tcaggtagtc gttaccagag aatgtagata acggccatga aatggtatga
227701 agtcactggg agccaagtgg gtgatcagat gtcctaggag aagcagacca gtgcacctgg
227761 tgacagtgct gggctgggct gaggtagggt ggggtggggt tgtgtatcaa aaccacacac
227821 ccttagaaac cttatagaac tctgcacccc tcggcaatgt gaaatcaatc caaattctct
227881 tgtgagcata aagaaaaata ttgacttcaa aatcattgat tttttccaaa atatccattt
227941 aaagattgac ttcttaagtt tctaaaaaaa aacctgccac tgttttacag gaataagaaa
228001 gatggtaggt aaagaataga caaaggggcc aggtgcagtg gctcactcct gtaatcccag
228061 cactttggga ggctgaggcg ggtggatcac gaggtcagga gttgaagact agcctggcca

228121 acatggtgaa accctgtttc cactaaaatt acaaaaatta gccaggtgtg gtggtgggtg
228181 cctgtaatcc cagctactca ggaggctgag gcaggagaat tgcttgaaac tgtaaagcag
228241 aggttacagt gagccaagat tgcgccactg cactccaacc tgggtgacaa gggcaaaact
228301 ccatctcaaa acaaaacaaa aaaagaatag acaaaggtcc agaaatagag gtgttactgt
228361 cctgtgaggc cggcctcatc taagtaagta gaggctggaa tttgctaccc atgaaccttc
228421 cttttccatt gataatgctt ccccactgat aatgtttcaa agccagaagg ctccagaaaa
228481 tcattggtca atagagatgt ttagctgaat gttagctgaa tgggacagaa cacatgggct
228541 gatgcagcca gggactgaag gtgtttgttg ttttttcttg ggcaactgac aatgataatt
228601 tggaagattg ggaaagctag caacttggtg ctgaatctta aatgaccgtc aaagaaactt
228661 ttgcttttgt aaatctatga aatctgaacc taagacttaa cctcttcatt tttcttcttc
228721 cactctctgc tttaagaggg tgcttttttt ttctctctct cccaatgaga attaaccaat
228781 cactttactt ttcacttctg gggcaggggg tgagttgact gaacattctt gcctattgcc
228841 tattcattcc ccaaagctga agtggttttt taaaagtcat gctcatcacc ccttcctctg
228901 gccctaatgc ttaacacctg tcaatgactt ctccttacat tttaaataaa attctcactc
228961 tttaccatgg gttgagaggc cccacacact gtggtttggc ctctccatcc ttatctgtca
229021 ccactttccc tcattcatgt actccaactg cacaggcctg agttttattc ctcagtagtc
229081 ccaagccctt gccccttagg atctttgttc tggctttttc ctctgcttga gctgctcttc
229141 cccagctcag catgttgctg tcttttcttc aaacttcaga tctctgctta gatgacatct
229201 ctgatgttct ttccttgaca aaactttcta aagctacccc tttcaatact ttcccttccc
229261 tcctgtagga cctgctatta tctctatctc tatctcctgt catctacatc tatagctaat
229321 gtctatgtct gtatctctat ctctgtatct ttctatgtat cttacatatt tgttgtatcc
229381 atacctcact aaaatgtaag ttctattata gcagcacccg tatctatgac attgtgcttc
229441 acatctaaga taatttctgg cataagatgt gtggagtgaa tgaatgaatt tcagctttta
229501 taccatccct gtaaaattcc agtaatgggg gaaacagcac agaactgctt gtaagataac
229561 tatccgtaaa ggatgtggta aactcttttt gagaagaaca ttgtttgcct gagaaaagga
229621 agagaaaata gcagtaatta taattatagt gatagctaat tactagctag gcactgtagt
229681 tacctttaca tatatttgct cagttatcag tattaactca agaaataaaa tgcactctgc
229741 aggtggcaat catggctgct cagcaaaagg cagctttccc ttggatggac tatgatgggg
229801 taggtcacat gcagtctgca taactgcatt ctacgtacct tctacattcc acatgacagg
229861 gaatgcttcc atcatgatta acattttctc ttcctgaata gagagaaaag aatgctatag
229921 cctataaata taaaaaggac caatgcctca caattaatgg attcattttg aaggcaacca
229981 agacaaagct ctttttgttt gtttgtttgc tttgatctct cttcaaactt tttattttct
230041 ttgtaatagg tcagtttggc tcctcagtgg cctcatactt cctcttcttg agatggatgt
230101 atggagtcaa tatggttctc tttatcctga catttagcct catcatgttg ccagaggtga
230161 gatctgactt ccagtttaca aaacatgctg aaaaatgttt ctcctagaaa agtatttatc
230221 tcttctttaa agtagaataa ttaactggct attttcacgt cttagagtca taggatttgt
230281 aatgagatta accttcaaaa tggtgcccat gggccaaagc tcccaatcct tgcactaaag
230341 ggggtgtaag cctcttgaaa ttatataact tatgtataat ttcacatgta tgtttaaatg
230401 tgtattttta tgtggaaagt ttcatggctt ctttcagttt cccaaggggg tccctgttcc
230461 taaatgttaa aaactattgt tcaagaggaa gactttgaca ccttgtgagt tgtatttctt
230521 tattttgcca agttagcttt gaaagaaatg cttccttaat gcaactcaaa gcctccagat
230581 tttctatact gaagaaacac aaaatgaaca ttttccccct aagatacaaa gtaagataat
230641 gcccacaaca cacacacaca cacacacaca cacaaatagt gggatgggat gagagtggga
230701 tgggatgatg aggtccaaag tcagaaacta aaacacatag agggtccaaa tcaaagaaga
230761 caaagccctg agctaatgtg gggaaagggt cagctgaggc caaaaagcta gactgaggtc
230821 agagccttca ctcatatgaa gttaaatgat tctgacacct ttgaatgctt accttacaga
230881 agttggatta ggttttagtt aacctacaaa ggacctttta ggaccagtat ttcaaagcaa
230941 atttggtcca tacagcattg acagtggcca aaatttggtg gttttgggtg actttttatc
231001 ccgatagctt caaatcagtg ccagtttcta tactaagact cctattaaca taataataat
231061 aataatgctt tacattcata aagagatttg agcttttaat agtacaaagc actttcactt
231121 cttttactta ctgtgcgtca caacaaattt atgatgtaga ctgtagaaga cattttcttc
231181 acccatttaa aacatgtaaa tggagtcaca taatgctgat gtgacttagc taacatcttg
231241 gaatatattt ttttattaag ctgatctagg actagaaccc atgattcaat ccctttctct
231301 aagattttgc actttaatcc actagtttta taatccttga gataattcca gagcttctaa
231361 agttgtctgg ctcgtagaag acagtgacca aagatcagag gtcctgtgta gtttatttcc
231421 caagtgcaca ctttcaggta atgaagctga atgtgtgtat tttatctatt tcaaattaac
231481 cccagctttt tttttttttt ttttttttgc ttacctttct aaggaaatct gaaactcctc

231541 cattccatca ctggctacta tgacgttact tagtgtcaga tgcaggtata aatggagttt
231601 tatcagcagc atttattctt tcccccacac catgctgcct ccatgtggag aaaatgaaag
231661 gctgttgctg atttatttga ataatacgaa attacagaat gtctgctatg tagtggatgg
231721 agtgcctacg tgttaggttg gatgtattcc aagagctagg aagcattctg tctttggata
231781 cccttttata ggttgtatta ggtgctgagg ggcatcctgt tttctggcca atgattacca
231841 actccagcaa aagaacaaga gaatggacac cctgcatgct gactttgcag aaccaccaga
231901 ggccagggca gtcatctcct ggcagcaacg ctgtattttt cagctgcctc tacccgacgg
231961 gctcactcag tattcctcca aggcaaatgc ccttttgccc caaggtcctg tgtccctggg
232021 caagcctgag accttgatct cctttgtcct ctgtgccccc gaactttggg gcgagacagt
232081 gtgtctggaa cccaccttct attctgttcg gaccaggaag ccagatgggc caaaggccta
232141 ctttgggagg ttgtgcagct tgtgcacagg gagtgagcag agattgatgt ccagcccttg
232201 ttcagtcaag ctctgcaccc aggcttcaag ccatattcat cttctgttcc cagctttagg
232261 agggaagttt ccatctcaca gaagaagcaa catctgcttg gtaagctatg gggctagaa
232321 aggtgctctt tccagttctc accaaggcac cttcctatga ccctattctt gccctctcac
232381 tggcccactc ttcccagtac tattctccct accgtttttc tcttccaatc tggtagcatg
232441 aatttgaggc agcagtcctg aattagtgat gaagcagaca ccataatcag ggtgcaagtg
232501 ttctcacgtt gcagacaccc acaaattaca tgattggatt tccttgagtt ctctctcttg
232561 acatgaagga cagaatctcc ctccaccctc actcctaagg ggtattctct tatgaaagtg
232621 tgggtaagat tgttttgttg agtcctatgt gactcaaaag gctacctctc tcctttccag
232681 acttccaagt cagaacagtt tggtggtggt ggtggcaatg gggtcctcag aaagccctct
232741 ggggaatgtg gttgacctga ctgctggtgg tcccctgaac ttggaatgca gggtcctttg
232801 cactcccttt gttcttacct ctggatttag tagtcgattc ccagaaaatg ggaccccaca
232861 tggagaccca aagagtcccc ataaaaatgt ttcagaaggg cttttaaaca aaaccatttg
232921 tgatcacatg tgtggctcag actttgtttc taattgttca ctgctttctt tgcttcttca
232981 tacagtacct ctggggtttg ccatatggca gtttacctag gaaaaccgtt cccagagccg
233041 aagaggcatc ggcagcaaac tttggtgtgt tgtacgactt caatgtaagt gtctccacac
233101 aagtgtattg gtgggaggat ggatttttat ttagtttatt ttattctgaa acccacggcc
233161 tcctctcctg ggtcaggctg aatgtcttaa gaaagacgtc cctgctccct ttaagaaaga
233221 aaggaaagaa agaaagaaag agagagagag agagggaggg agggagggag ggagagagag
233281 agagagagag agagagaaag aaagaaagaa agagaaagaa agaaagaaag aaagaaacat
233341 gtccctttat ataaatgtga ctatgattta ggacaatggt aatatgatta tatgggaata
233401 tagactactg gataaaggtg agctcagagt tcaaagactt gaagcaaata ttcttatcac
233461 tgactttgga aaagaataaa ggaagagttt gcaaatgtgc tttttggaaa atcttgttgg
233521 attcctaggg aattaaaact tttacacttc ctattaacct ctttacttcc taaacaagcc
233581 tcctatttag tattactcaa tagttagtac taaataaagc taaatattga aaactaagaa
233641 atactaaatt ctaactttta aataatacta aaattagtat tattccattg tagtattcaa
233701 atgagcatta ctccttgagc tcccttaggt agagattatt actatggaaa ggagttattt
233761 ctgtccattg aaaaaccatt ttggactata tgattcaagt cttcgacaac aatgtaattc
233821 cacatattct ttttttaagt agtgactaga ctagaaatgt ctggaagcat atgcgtggga
233881 aagtagccag gttgaaattg aggaattgat aattcaagta atttttggag gctagacaat
233941 gagaataaaa ttttatacag attgcagaat gaaggaggga aaagcagact gcctgtttat
234001 agtgttaata tgtatatctc acaaacattt tgatttctac ttttaacctc cttaaattgt
234061 gagtttccca aagaagagat caggtctgct acatcttttc agcccgtaat ctagtcctca
234121 tccactacag gctaaatgaa tctcattgac tgaaaatcct gagtgagggg gatgattcat
234181 agagccttga agtaagaacc acaggaccag tttatgtgag gagtaccaga acatgcaaac
234241 cttaagaaaa gtttcccatc tccgaatgaa caaagcaagc tattgagtta gcatgaggca
234301 caatgatagc aacaaatatg tggcatttgt aatcttcaat cacccctgtc agttggactt
234361 tccatttcaa ttcagcattt tttaaactga attataaact tgcataggtt ttacaaagat
234421 gaatgtgaca tgattcttga cttcagggta ctcgtgatat tagaaggaag acacgtaagc
234481 aaccagtata caagatgctg tgcacaaagt tagcaaaatt taaaaaaagg aaaaaagatc
234541 ctggggaact gagcaagtgg ttcttatttc ttcttaggcg ggtctgggtt ttacagaaga
234601 gatggcctct gagttggatt ttacctctct tttctatcac ttctagattt tatttctctt
234661 ttctaccact tctaacttct tccacctctt cctcctcccc tccctttatc cctcccttta
234721 tttatctctc tcactttcaa atttcaaata atgcattact atgtcccttt accactgttt
234781 taatttctca ttctcttcct ctgattagca aaatgacaaa cttccagtct gcttaataag
234841 aacaaggacg aagatcactg actttttctg tttttaaggg catagcagag aaaggaagga
234901 ctaaaaatat gaacaaaagg tcttttgatt tttatatata gatttctgaa gtttaaatac

234961 ttctgaattt cccaatgcac tgaaatggaa aattttgggc aaaaatgtcc atttgagatt
235021 tgaaagttag tgactcagtt gaggaagaca aacagaaaag ttgctgtaag agagttacta
235081 atgagtggac aagagggtct gagaggcaaa ttcaggggaa agtagaggga aagggtttgg
235141 atgatgagtg tgcttcccaa gattgtatca ctgcccttga cagctctctc acatccagtt
235201 tggtgaaata tgatttgtcg gtcagcaggg gatacagaaa ccagagtgtt ctatgatagt
235261 tatttcaagg cctgcctgaa aacaatacta aggcaatgag agaaatttta aaggtcaaga
235321 aaatataaga aagccattac actgatttta ttcacttaca ggcttgctgc atgctggtat
235381 cagtcattaa tttggtatgc atcattgctg gggttagtag tgattttaat agctgtattt
235441 ctccaatgtc tgcatcatgc agtgaactgt gattggtaag taaggtccct cacttcacaa
235501 cccacttcat ggtgggtatt tatatctgag actaagctcg agtgttctta tatgtgaaat
235561 ggagaaaata atagtatcta cctcaaaaag catcaagtga gataatgcat gaaatagcag
235621 atagctcaga gtatgtagca cacagtacag gctgagtatt ccttatccaa aatgcttgag
235681 accagaagtg tttcatattt caaatttttt cagattttgg catatttccg tatacataat
235741 gagatatctt gaagatgaca ttgaagtcta aacacaacat tcatttagtt tcatatacac
235801 ctgtacatat agcctgaaag tgattttatc caatatttta agtagccttg ttcatgaaac
235861 aaagatttga tggtgttttg actgtgacct gtcacgtgag gtgaggtgtg gaatttttca
235921 cttgtggcat catatcggtg ctcgaaacat ttcatatctt ggagcattta agagtttaga
235981 ttttttttta ttaggaatgc tcaatttcta aatattcctt attacaagaa ccataacttg
236041 accaaaggca aacagctatt aggaaaccga attgggatgc agggctggtg gtattacaca
236101 cggtaatgta tgtggatagt ggcaagctag taaaaggact tttattataa caaagaagta
236161 aatacataag taaataaaag tattctgacc ttaatgagga gttcttgaaa gattcaagga
236221 aaggaagcaa taggattata tagtcattat caaattgttc attgaataac attattattt
236281 aaaagaaaat aatactgctt taaggaattt tatatgatat acaagttttc atgacttttg
236341 aagtttgaaa gatttgtctt ttgttttggc aatgattata ttctaaattc agcaggaaag
236401 ataggttgat tcatattaag ggacactttg cttttacatt tttgtaagaa aaaggaattc
236461 agcccatcct acaataagct aataattgag cacattctct atactagggt caaaataatt
236521 tcacaagtac atcaataaga gagagggaag gacctttaat tattaaactg aagataacaa
236581 caagaaaaaa atgtacaaag cttaatttaa aatataggag atttggtttc acagtaaaaa
236641 tccaaaagaa taaagccaag tagtcactgg taataagaca ttatagattc aaatgagatg
236701 ctgtcttggt caggacgtgg tgtcctccac ggcagaaggg aagaggctgc ctgagcacag
236761 attgtcttga gtatcgtgca tagatggtag agatcagacg ccacagacag ccgtgggctg
236821 tccctttaag aactcccata gaatgggcag agatgtccta gaattggaca ataaggaaag
236881 agtacaagga aggaaaaata aaatctagcc aagctcaagg acctgtatgt gttacctcag
236941 gttaaccaga cgtttctcag aagacagaat ctaaatttct acttacattg cttttgtttt
237001 tattatcatt tttgtgaata ataccaaaat taatgtagac taagtactta tatgggcagg
237061 cactgtgctt agtgctatga tatttctatt tatttatgaa tattatactc atatatttat
237121 agtgatttat actacttgtt tcagtatgca tcaagaaatg gaaggcacag atatcttgac
237181 agagataatt taatataaga aattaaacag gtattgggtg actaaaaagg caaaggggga
237241 cattgaggta acatagaaat ggtaacaggt aacacagacc agtttttacc cctactgttg
237301 ggagaacaaa agaggaagtg gggttattag aatctagggg cttggaggaa gggcgctgca
237361 tggccccaag aggtgtgatg agattggttt tgagtgtgct cgaacagact ggaaatggaa
237421 ccaaataccg ttaccagagt gaagaggcat tgctggagcc atgcattcag gagagagcaa
237481 atcccttctc ctgacttttt gtctaagtgc ctcctatagg aaaaatctgc aaggaatccg
237541 atctcaaagg aaaaaagtag ctccccaagt cccgtctcca gcatcacaaa gcagcatata
237601 caaggatggg tctgaagctg aaacagctta ctaactggca cattattcta tttatattat
237661 gacctattcc tcttcctagt ccagtaaggt agatatgact attcctaatt tacagatgga
237721 gagactgagt tttagtagga tttggtattt tcctccaagt tccgtggatg gcaagtggag
237781 aggctgggat gctcatctgg tctgcctgac atctctgtct gactctgaag cctaggacaa
237841 gctgcctcta catagggtac taggagccac tgagatggct acaggtttat actggaaatt
237901 cacaaaaata agagaatttt agaaatagag gtgctcttcg atatcttcca tcctatatct
237961 taccttataa agttacagaa attttgagtt actgaagcat gttcagggtc ttatagttgt
238021 taagaggctc ctaatctagt gttctcttca tgtcctgtat tccgcccgta attttcctgt
238081 tgtaatttcc agcaacattc tcactgactg ctcctggaac atttactgac tctgtttcat
238141 gatatatcta tttcacaagg ggaaatggag ttgaagcatt tgggccccat tttaatcttt
238201 cataataatc ttgtaattcc caaatcagct tctcaaatag aatgagagct ttcatgtttt
238261 tttttcctaa attctttttg tatcatttaa atattatgct ttattaaaat attcataatg
238321 gaaaatcata ctttgaaaat tgttattatt aattttgctt tggttggtta gcaaagttct

238381 atctttagtt tttaaaatta atttattttt gaaatagaca ataaaaatcg tatatttgca
238441 tgagttcttt gcttgatcct ctgaacaatg acttcataga ggtgctcact ctgtattgtc
238501 agatgatcta agtatacaac ccagtataca acccagtatt ccatctatca tggcaccatg
238561 gaggaaatca ttgacaaaat gtttttaaac tccttaaaaa ttcataatcc tgacttttgc
238621 aaagcccagg ggtgccaaag caggcatccc tgggcttttg aaaaatgcca ttatttaagc
238681 tagagccgtt atctaggcta tcattatcta ggtagagttt ggggtgtgtg tgacctcaat
238741 atgaaaacat ttggatccac tctcttggat ccaaagagat ggcatctgga ggctgtctct
238801 caaaggagat ctgagcagca cacccagcaa caggatcagc tgaccttgga cagactatat
238861 agaggtaagt tattccacat agaggcaggt gaacagaagc agaatagatt taagatctgc
238921 acttacagaa taatgtaaga taaacacaag catgatctct tatggtacag gagcctattg
238981 gatgggaagg atcagaaaat ctttggggta aggtagggga ggttgtgagg aactaccaat
239041 ctgtagagct tcatctggga accttggaac ctcctctcca tacccatgaa agcgcagtgc
239101 tctgtgactt agatgtatat atcacaactc atccaaattc tcttcagtta atagtagaca
239161 aacaccatac acaacatgca tgatcagcta gtctcagggc ttttcaaagt cagaatttac
239221 catttatgaa acactgtatt tttaaaatat tcttattgag tgcataatga tacatcttac
239281 tgatggtgct ggaaatctgt tttattaaaa tagtaagaaa cagagagatt attatgtatg
239341 caatgaatca aataagcaga aattgtatca tgtaaattaa tagaagcaga agaatgagtt
239401 taagtcagta ggaagaggat gtctttgttg gcaagatacc tggagagaca agggtgatat
239461 agaaatgata aagaagaaaa ggctacacag ttttaatatt cttgtgagga gaaagaagga
239521 ggaactaaag tggaatttgg aaggagcttc agctgatatc agacagtgag atcatatggg
239581 taaaggttta agttgtgagt aaagggaaat gcattctgct tttcaccttg agagcatctt
239641 gtacattgct gatggataaa tggtatgtct actttgcctc cccttttctg ctgattatca
239701 tagatgtggg agatagaaga tatcctacaa tgggcaggag aggagactta acacctatta
239761 tgtgttaagt aggtacatac attaaatttt atcctcataa caactgttta agatggaata
239821 tatcatccca gattattcct tctgccctgc ctgcctcaca tcccatcttg gaggtttgtc
239881 cttaaaggag acaggttaga cacatataga atgttaataa aaaggagata agttctttaa
239941 tcaacatcaa caactctttt cagtaataat gttccaaagt aaaggtgtct tctctcaaac
240001 atatctcagt tctcttaagt tccttcatct cttattgcac tggacacagg tagaggagat
240061 attttgtgat tcacacgtgg aataacacat cttttgtttt agaactattt cagtggttcg
240121 aatgcatctt tatttgaaaa ggtgaatcat tttaggtttc ctgaggtgta gacatcaaaa
240181 ttatgttaca tgcaggagat attgggggag ggaggcgtgt gaaggataaa ggagagagtg
240241 aacaagagta gacaggaaga gcttttagat ggtaatgcag gccctatacc tgtgaaagga
240301 gagagaaaag gaaggattta ggaagagtct cagattgtac tacagttttg aaagagtctt
240361 ggccaggcta acggtgagtc tctaaacaaa gtatgtccat tagataaatt ccatattggt
240421 tagcaataac ccagctccag tatttcccat catgctcagt cattatctag gtagtggttg
240481 gggtttgtgt gacctcaatg tgaaaacagt ttggtccact ctggtgggtc caaggaggtg
240541 gcatctggag gctctctctc aaaggagagc tgagcagcac gcccagctgc taccttgaaa
240601 atgtgactgg gctcctccac tttagatcat agctgttcat attgctgttg ttgctgttat
240661 ttagtttcaa caggtttcaa atgcagccag aatgacctgt ttcccacatc catcgtctgg
240721 gcctacctcc gtgctgggtc agcagaggct gaacttctag atcatgaata attcttcggt
240781 ggcatgaccc caagcgagac tgctaagcct cttgtggaca aatttagagt tataaatttt
240841 caaatccacc tagagatacg tctctttccc tagtagcaga gatcataact ttggaggggg
240901 tgaggaccct ctcatctgtg gatgtagtga gattcctcca atggctttgc tgagcagggt
240961 gggctgctgc tcaggcatgt aacgctggga ctggcagtat caaagggctc tgtcccttat
241021 ttacttcact tcctcaatgt ccagtgtggg tctctttggt tcaacaggct tattaagttt
241081 gattaaaaga agatgcattt catgttctat tctaggtcct agatcacttc ttttgctttg
241141 taagcttctt aatttgctac tcctgagtga ctttatgaca cagctctagc ttgtgacgag
241201 agagattcct gggtctgctt ttagcttcct tggtatttta aacttgggga actgatttct
241261 taaggtctga agtcctagcg atgccttttt tttttttttt tttttttgct ttagtaacac
241321 attttagttt tcatcacctc cctcaggcta cacttggcca aaccaaaagt gagcttttct
241381 tttgcaaata ttagggatgc atatgatcct aatgctatac ttacaatatg aaaacagcat
241441 tccttctttg gaagtgacca tagaaatgag tcttcttttc attaacatag taaacatttg
241501 ttgggcattt gccaggtgcc aggcattaag caaagatgag aaaacccaag gtctctgtcc
241561 tcaggaacat aaagtccgag ggagtagtca aataatcaca aaaataaatc caactgtaat
241621 tgtgccaagt gctatgaagg aaagctatat agtgttaaga gaacttatag aagacaacct
241681 gatgtggttt aggagctaaa ggaaggagac agataaaaga aaatattttt ctctgagaaa
241741 gtaatgttca aattgggctc taaggagtca ggacaacctc tgaatttgaa agaagggaga

241801 gatattctca gcagtaagaa catcaattac agagaacgtg tgatgataag aggtggcctt
241861 attcagggga ctgagtaaaa cgagagagct cagaatgtga agggacatca gaacacaagg
241921 cattggagag acagaaggga accaaagcac gtgggatcct ctaaaatcta gactgcacta
241981 attatttttt ttttttctca gagcaattaa aagtaactta agtggggggt aggtgggtag
242041 taacatatca gacttggatt ttgaggtaat caacagactg tagacgggca agatggctgt
242101 gaggagatca aggaagaatc tttcgatatg actaggttag agactaaggt tggaagagct
242161 aaatttacct ggcaggaatt ttctatatct aaatattggc tgccaaacta atagattaaa
242221 ggatgagcca ttgagttagg ttagaacttc acaatgcatt aaattatagt caataattta
242281 aacacatagc accaaatcat aaatctaata catctgtggt gacaagtaga ttatgtaaat
242341 ctacattcat ttttttccaa gtagctgcaa tatattcttt acattttaaa ttattttatt
242401 tgttcaaaaa ctccttttta aatacataat accaaggcat ctcccagtat aggctgtgtc
242461 cagatgacca cacacgtctc gtttcatgta tgaattcaca aagcttcact ctgaatattt
242521 taaagaacca gcagcaaatt tataactcca gaagtcaacc agccaacaaa accctctaat
242581 atgcttttaa tctcctcctt gcgaaatgaa agaagacaac ttttttttct ttgtggaaag
242641 aaattataca taaaagctca ttttatagca ctgagactgt ctcattttgt taagagtttt
242701 atgcaattag acagtaggaa aaattaaaga gtttttccag tttcagtgtc tctcagacac
242761 ttctttggtg ttttgattca ttaaatccca acataagatt attattgcca cgttactgtc
242821 ttggatgatt tactctattt tattaaaatg ggtagcagag agcagaagaa taaaaaaagt
242881 tggtgtacat gacaatttaa aggagcttat tctaactgag gattgctaat gattaccaga
242941 agcaattttt ttttgttcat tcaatcactc cacaaatata aattgaagtc agatgttggg
243001 aatacaaaga caactaacat atattccctg cccacaggga cattatgatc taattggata
243061 tgcaaacaaa caattgccat gcaaaatgac aatctgaggc tgatgtacat atgtcttagg
243121 cacttgggaa cataatggaa taagggttca gtgtccgggt taactggcat tcagatactg
243181 gactcttgag agagcccagt aaagagaacc tctatttccc atgtgagttc tgaataattt
243241 gtttaaaaag aagaggcaga tgggcaaaaa atttttccac attcattatt aatggaaatc
243301 gaattggagg aacaatccgg aattcttagc caaatggtct tgctgattta gaaccccaaa
243361 ttagccattt tacctctagg gcaacttatt tcctagaaga agaaagatat gtttgggctg
243421 ggccagccca gttaattctt tcccagttta ccaacagaaa tcactatgcc tttcctggtg
243481 tgcagtgagt atagggacag agaaaagctg aggcatattg catcaagtga agctccgtgc
243541 ctctgaagga aatgctgaga gaaaggaagg ggcattcatt acccctatca caactggctg
243601 tggttgagag gtcaacaaag gctttacaag tatcttgaac cagctttaaa gaaagagtag
243661 gaatagagca ggctctagaa tggggcaggc aattgggaag cgaattccaa gcagagagat
243721 cggcatgcac aaaggcatgg aggcatgaga tagtgctatg ctctcaacac ccaagagtga
243781 ttcagcaaag ttcatattta gggagaaagg agggcagcaa gaggtgagcc tgtcatacta
243841 aaaaaaaaaa aaaaaattca aaaaagagcc tgatcaggga gagaatttca ttatgctcaa
243901 gtcagacttt atcctatagg cagcggagcg cacctgagat aggaggaagg agcaggtttg
243961 gggaattttc ttgaatgtgc tatgttcatg cttcctatga gacatgcaag tggagtgaga
244021 tgtgtaatgg cattgggcat agggtctaga attcccactg ttagtgtcag tcgtggccca
244081 tggcacaaaa ggcaaatgga agtactttct atagaagcca gaagagttaa aaatgcagtg
244141 tagcataatg acaaaaacta ggcatgagat tccaacaaac ctgggtttga atcctgccac
244201 atacgaccta tgagatatta gacaatcatc ctgaaccttg tagttgccat gtataaaaca
244261 gcagtattaa tacctagcta ccaggcttgt ataaaaaata catgagatta tgtgtgtaaa
244321 gtattagcac atctcggctg ggcgtggtgg ctcacacctg taatcccagc actttgggag
244381 gccgaggtgg gtggatcaca aggtcaggag atcgagacca tcctggctaa catggtgaaa
244441 ccccgtctct actaaaaata caaaaaatta gctgggcgtg gtggcgggtg cctgtagtcc
244501 cagctactcg ggaggctgag tcagaagaat ggcatgaacc caggaggcgg agcttgcagt
244561 gagccgaaat tgcgccactg cactccagcc tgggtgacac ggtgagactc tgtctcaaaa
244621 aaaaaaaaaa aaaagtatta gcatggctct ggcacatagt agattcttct ttaaatgcct
244681 cttcaggata cttagctcat gaaaattccc atttgttgga gggacaagga aacagaagga
244741 aggaacttgt agccagagca agaaagtgga agtggggcag cagccatgtc ccctgtgtag
244801 gcaggaggca gggggacaag agtaccaatg caaggatgga gggatggata cccaaaggaa
244861 ctgcctcatt cacagctgct tgaggggcag agatcactgt ctacagtcct taacacaact
244921 tgattccctc tgctgaaaat tagatctgcc taggctcagc tcagcttcct ctttgtactc
244981 cacactgtct ctgttaagaa ccatagagca cctgtttcat gtctatgaat ctgggttgga
245041 gcaaaaggaa aataaaataa aaaaaactca gagatgagca aagtattagg cagaagccta
245101 atgggtctgg ctggagagga ggagtgtggc catgcgctgc tgtcagcatg cagctcacaa
245161 ggcccgggct ggctgctgtt cttgtgacgt tagttgaggt ttattctggg cataattagc

245221 cttttcactc cttgtttctt tcctgtgttg cgtcctactc catgaaatgt tgagtgccag
245281 tccttgtctt tcatccacac ttgaaacctt cgttcatgct aaggtgtaaa taaccagcaa
245341 caactcagag aaaagtagaa atacacccag acttgaatct tgcagtgtca cctcccctca
245401 cataccctca gcagatatgc ttaaaaaatt atatcttcct ttcctaccag ttttacttta
245461 tacattcttt caaaataatg gtctggcagc tattacaaat taaaaagagt tactaagaat
245521 gaagaaattc aatgatcatc ctggggatag agtgttatct ttcatggtga acatttcttg
245581 gtaatcatga gaaacaccaa agggatgcag ttttaatata atttcaggca acaaataatt
245641 atgaaggaga tgttttgcat gcagagcctt ttggggacct ctgaaatcct gtctcctaat
245701 ccatctatgt atattgggca aatagtaagt tctcaataag tgttagttag aaaagtaaat
245761 gcatgaattg agtcctggta accataataa aaacagtctt tcttgtggaa atgtgttggt
245821 gattatggac tggaccagct gcagcaatca aacagaaatt agagtcttaa tagacaacac
245881 acgagcatgg caccccagag ttggacagaa attttctgtg ctcactggga acttggctct
245941 gactctggtt tctctgtcac agattttggg gtgggagagt gctcccatgg ggaaagcagc
246001 acgtgctgat atcgaaattg aatagaacct tggctgggct aggagtcatt gctattttag
246061 tcacttccag atgtctgctg ctgtagtttt aagggacagg gaaagatgag tggagtgtgg
246121 catgccactg ggtaaaagat gaggagcgtt ctccttctaa gcccttcaag ttgtatttaa
246181 agccatccct caatatgcta caaagatgga ttggataaag cctttaaaaa ctgtgttctt
246241 ctattcacaa tagcaaagac ttggaaccaa cccaaatgcc catcaatgat agactggata
246301 aagaaaatgt ggcacatata caccatggca tactatgtag ccataaaaaa ggatgggttc
246361 atgtcctttg cagggacatg gatgaagctg gaaaccatca ttctcagcaa actaacacag
246421 gaacagaaaa ccaaacacca catgttctca ctcataagtg ggagttgaac aatgagaacg
246481 catggacaca aggaagggaa catcacacac tggggcctgt tggggggttg ggggctaggg
246541 gagggatagc attagaagga atacctaatg tagtgacagg ttgatgggtg cagcaaacca
246601 ccatggcata tgtataccta tgtaacaaac ctgcacattc tgcacatgta ccccagaact
246661 taaagtataa taaaaagtta attaattaaa aataaataaa caaaaactgt gctttttaac
246721 attcatttat agatttgact tcatatatat caaataggca aactgcagaa acctttgtga
246781 actaatcatg actctgcctg ccccaactag aaaaacaaaa caacacaaaa caaaacaaaa
246841 ccagaaccat tcttgggctt tggttatgat ctagtattta tgaagtgcca gccattaaac
246901 atatgttgaa tggtacctat gccagatgag ctggctgccc ccagtagcta ttgtgctaac
246961 ctaacaagct caccagatgc ttgttctata tctgctttct ccttttatga tattttcggt
247021 gtgtctttta atcccaaaat tgtcattatg aattagataa aagatatcag gtggggggtg
247081 accccctctg aaatattctc aaaagggatg cctggggttc tttccaagag gccctgtcac
247141 caccagggac cagtctaggg tatagatcaa agtctgtgat gggttcatag agtttcttgt
247201 ttctttcctt tctttcactg agaaggtatg gcactagcat gatataactc ttgctggata
247261 aagaaagcac aggagaagca gtatagtgca gtggttaagc atataggatt tggattagac
247321 tcaggtcaag ttcagacact gtcactcatt agtcatgtga acttggacaa attacttaac
247381 ctctctaagg ctcagtttct tcatctgtaa aataggtata gtgatagcag ctattgaatt
247441 tttgtgtgtg caggagaggg gaaaattatg gtaaataata attcctccct tgtccaacaa
247501 gattgtgagc tcaccggaaa tataacaaag ggtaagacca gtgaaggtca acatgccgcc
247561 tttattattg ttaaagctga cataaaagaa tgtagtgtct ttatggacaa gtgaacttca
247621 cagccagacc tctctgccag gacaggcctc tccttgcagg agcagagcac agcatgttcc
247681 ctctcaagag aaggaagaga tgtccgcatg tctccatcca tgctctactg tcaaatcagt
247741 cacttttccc cgaggctaga gtgaggcagt ggagggtggc tgggagtgtt atgttaatgg
247801 gtcttcactt ggaagggaac gtcaagagtt ggagatacga ttaccctagt agcagcaact
247861 tcatagtgtt ctcctgagga ttctgtgaga taaaataagg tgtttggcaa gtgctaaatg
247921 ttcacgaaat gtatacggtg atgttacttg tcacagcaac cattatcact gagtgtcaca
247981 tctgtggcaa ggaaataggg caatgaaaaa cagagacatt tggaagttgt ggtgttgtcc
248041 catgagggtt aaataaagag ttcaggatat aatctttaaa ccaaacaaga gattaagtgt
248101 ctatacttag catgttctgt tattgtcacc ctgggacaga ggaacaagca tgaaggttta
248161 agacatgata gaaggagatt ggatttaaca caagtgggaa gaagcagaga tcttaactag
248221 ataaatgtga aacaagaaga tgtcatgcac ctgggagctg ggagatcctc ccaagcatat
248281 tctcctggga tgaagatttc tgggtcttgg ggagttggtc aatccatgtc ttatcttaga
248341 catgagatta gctcacccac tccctctgct tgaggtttct actagctgtt tatccccaaa
248401 tgttctgttg gatggcatcc tagtcagggt tctccagaga aatataacca gtagaagata
248461 tgtatacaca catacacaca tatacatata tatatatata aaatttatta tatcaatcta
248521 gtatttatat aataaattta ttttgattta tcattattat tacttaaatt tattatataa
248581 atctttatat atatatatat gcatataaaa tttgttgggt tttttttttt tttttgaga

248641 cggggtctca ctttgtcacc taggctgcag tgcaatggtg tgatcttggg acactgcaac
248701 ctccacctcc tgggttctag caattctcct gcctcagcct cccaaagtgc tagaattaca
248761 ggtgtgagtc accatgtctg gctgatatat ataatttata agttaatata aataatagat
248821 ttactacaga tataaaagga gattcatttt gaggaatagg ctcacacaac catggaggct
248881 gagaaatctt ctaacctgac atctgcaagc tggagatcca gagaaaccag tgctgtaatt
248941 cagtctgaat ctgaaggcct gagaaccagg ggagccaatg gtgtgaatcc cactctgagg
249001 gcaggaggag atgagatgtc ccagctcaag cagtgagtga ggcaggagaa aaaggagcaa
249061 atccctcctt cttccttttg ttcttcctct ttttgtcctc agtggattaa atgatgtcca
249121 gccatgctgg ggagggcaat ctactttact gagtccacct atttaactgc tcatcttatc
249181 cagggacacc ctcccgggca tacccagaaa cagtgtttga tatggacacc ccatggccca
249241 ctcaagttgg cacatacaat taaacatcac aagttggctt tattttttct taaactgaaa
249301 aatgcagata caggcctgtg tggcccaggg aaacccagac ctttggtatt tacctgtgat
249361 tcattccgtg cagtcccgag ttaagcacac aagttcctct gagattattt ggagatttta
249421 ttttgttcta gttcttaaag agtacaatat ttaagaaaat ttgccccata ttttgaaagc
249481 aatagttttt cagatctctg ttggcctctg atttgagtta ggcatgatgt ttgtttaagg
249541 caattttcta aattacttct cttaattctt cctctaggaa ttagcatcta cgacatttct
249601 actttctttt ccacacctcc actctccccc acttcacttc ccttcttcag ctgtgagtgt
249661 caaagtccct gggttcgtat aaatgatgtc aaaggcaacc aattgggttt tctaagattc
249721 agggttgact cacctgatct caagaggaag ccccatattg acaagtgatt atcttctcat
249781 ttcacaaaac atagttattg tcagagagaa gtaagagggg agctggggag tggatagctg
249841 tagtgataca gtaacagtgc ttcaatgctg aaagtgaaaa acacagtgaa tgtgaggaag
249901 agaaaaggtg catattataa aggaagcagc atcagaatat gccaatgttc tgagaataaa
249961 gagggagtta ttcaggtggc tttgagagag ggtgatttca accctggcag ataattttta
250021 ggaacgagaa tctaatgcac aagtaaacat gcttgttata tgggaatgct gtgatagtct
250081 tgatgaatgg gaagtgtgca gatgagttcc aatgtcatcc cacctgctct ttgatatttg
250141 tgaagtgaga gctgagacat aacctctgtg gtttaagaag aactgatcta attatggtca
250201 ggtgattgga aatggaaatc tgccacagca ctttgaggaa gcatttttca gatccccatc
250261 ccctagtatt aagcaaactc ttaaaatcgt aaatctaatg ataccacgaa atgtgttttg
250321 atgtttaaat caaagggcat ttcacggtaa tggctccaca gctgagcaag cctgtatttg
250381 cttaaaaatt ttatctattt agaagtcact gattttcaaa taaactgaaa agctgccaat
250441 caacatggca gctgaaacat tcactttatg gagcaaaaca atagggctca tgtcagagct
250501 gtgacccaag tttgaaaatt aatgtcattt tgttatttct cttaagagtt gatcttttcc
250561 tttgagttgc tcttcacgac aactgctaag tggctttgtt gttggatttc agggtttggc
250621 acaatattcc gttctctttt atggctatta tgacaataaa cgaacaattg gatggatgaa
250681 tttcaggttg ccgctctcct attttctagt ggggattatg tgcattggat acagctttct
250741 ggttgtcctc aaagcgtaag tttcatttgt cttttgggaa gcaaataatg atttctggaa
250801 tcaaatggag ggattaattt ggggattgga tataattttg ttgctatttt atgtctaaag
250861 gaaacagagt ctgtaagaga tgaaatgtag ttttaatacc aacactaatg ttagactatt
250921 acatcttcaa aagaactgaa cataaacaca ggcctttgag tcatctcatg gggtcagacc
250981 aatgcttgca tgcctatctt gtgtttaaac atatttttta aatgtgtatt tttgtacttg
251041 aaggtgtgaa ttaatgttgc cagcaagaca ggaaaacagc tgctggtctc tcttttgcgt
251101 gcagagaggt ttgtaacttg cagaatcagc tcaataggca gaatctattg ttctctgctc
251161 attttttaaa aaaaccagaa taatcactca ttcaaaaaat tttagctgtg taagacagca
251221 aaaatgaatg gtccccaatg aaaccatcac aatcaactgt ataatcatgg tttgtcttgg
251281 gaaaagattt attggagtat ggggaagaat ttcactggag aactgatatt actgcagttc
251341 atttttctcc ctgcctaaaa ttatttataa tactccactt aaagactgca gaagcctagc
251401 agagttattt gggtttatat attaaaaaaa tgacccttta gccaattttt tttgaactag
251461 tttacgagaa gagttgaaac atctcaagta aggttgccat tcttttattt gactatgtgc
251521 aagttgaatg agcttttccc ctcattgtag gaggtccctc agcctgaggg tctaagaggg
251581 gaaaagtgca aagtcaccct aagcaggagc tctctgaagc agtctgtgac acactttgtg
251641 tcctaaacct gcctcactta cttgttcctc cagagaaaac tgccatattt catttttaag
251701 ctcttttagg acttaaaacc ttataggaaa tcctatatct aagatattta tcaacctgct
251761 aaagtgtaat cagagacctc aaatataaga gactgtccat cagataaact actatcacat
251821 tccaaaaatc gaagatattt tctagtcttt tgactatatt gctctggttg ggctgaagtc
251881 agaggtatca tttacattac tgaggcctca tgagtatcat ttctgtgttg ctcctttagt
251941 ttctgtggtc agtggagaag aatagtttct catgtctgat gactcagctt ttattccggt
252001 aactgtgcag gctgtgtacc ccacagccat tctgcctagg ttcaaatccc agctctacca

252061 tgtatgtatt tatacaatca ttaacagatg gctcaactgc tttgttctcc tgtttcccct
252121 tggtaataga ggtgtaatgg atgtgaggac taaatgaatt aatacaaaca aagcacttag
252181 aatatgcctg gatgagaaaa ttattcaaca aatgttaaca attttattag gctatatttt
252241 attaggctat attatatttt attagcctat aattcatagg cttatttaag aaaaaaagcc
252301 agcatggtag gagttgaggc cacattatag atccccaaaa caaggcagtc atgtatggtt
252361 atgcagttgt acatcagtag atttgtatat ttaataaatg attttctggc agatgaattt
252421 aaagtatcta gagcagcatt gtgccaggca agccacatat ataagtttaa aacttctagt
252481 agtcattaaa taaatagaca ggtacattta attttaataa taaatatatt tgctttaact
252541 caatatatca aaagtattat cattttaacg aacaatcaat ataaaattat tattgagata
252601 ttctctgcct ttcgaaaggt atcaagtctt tgaaatctgg tgtgtatttt ataccagtcg
252661 catttcaatt gttcagtagc cagatgttgc tggtggaaca ctctgttggg tagagtggct
252721 ctagaggaag tagtggcttt ttcaaagttg ctcgaagatg ccatttggga tagctgcagg
252781 gcatcaaatc cacattttcc ccctcaaagt cattcatgtt atatcatcca aaccagtgtg
252841 gccttgaacc ttccctctgg catggacctt tggctgctcc ttacctggtt aacaagtagt
252901 tcaaactcat tgcccaaaca gcaagacaac tcaaaaacaa tacaccactc tcactccacc
252961 accagcccat gggagaatat tcagtagaaa aagaattgat gagatataaa tctttgtaca
253021 gaatcattaa tcatctcttc ctactcctat cccagtcaaa tattctcttg gaataagttt
253081 gagcggattt atgacataca ttactcaaat ggaatagaag gtaacctaag ttctaaaatc
253141 gactaggcta catcaagatt ttttcactgt actcattctg ttagcgtctg catactcatc
253201 cacaaaatgg gagcaaatat ctatttttag ttacttaata gcaaaatcag aaaataaaaa
253261 gatagaaatc caagaaaata gtttgaaatt ttaagataag atgactaact ttgttggccc
253321 aggattctcc tggttttaga actgaaagtc ttgcatttga gaacctttca gtccccaaca
253381 aacatggatg gttggtcacc ttggttttaa gtccttccct tttcttcttc ctcccttcat
253441 tcatttcttt tatccttttt ttttttcctt ttctttccag gtcagtgtac attttcaacc
253501 acatcatatg cttagaaatc ttcatgataa atatagttac ataagtatta aaagcaaatt
253561 aaaacagtat aacataaagt tacacaacat ggaaaccatc agctcattta aatattgtcc
253621 tcattagttt gaagttttat atcataaaat aaaggtggct tgacatcaga acccaactgt
253681 tcccgttaaa aacaattctc taagctaaaa atttggggtt ttactggtct attttacagc
253741 agctaaacta ctggaagcag ttgaatcatt ttgagaatgc atgtcttaat aagaaactca
253801 tagctatttt atccttgtag gctttttcaa gactttttcc tgtttgtagg atgttttagt
253861 ccatttcgtg ttgctataaa ggaatacctg aggcttggta atttgtaaag aaaagaggtt
253921 tatttggctc acagtctaca gactgtacaa gaagcatgga gtcagcttct gcatcaagtg
253981 agtgattcag ctgtttccac tcctggtgga aggcaaaggg gaaccagcat gtgcagagat
254041 cacgatggtg agataggaag caaaagagag atggggaggt gctgggctct tttaaacagc
254101 cagctctcgt gggagctaat agaataagaa ctcacttact atcccctgca aaagaaggca
254161 ttaatctgtt cataaagaat cttcccctat gacccaaaca cctttcatta ggctctacct
254221 ccaacattga gatcaaatct ctacatgaca tttggggaga caaacagcga aaactcagca
254281 cagggctccc tgggattaga tataattttt ctgtttatga tcctggctat aataatactc
254341 atagaatgga ctaacaaata ttcttggata cttaaatcat gtgctatacg taacccttaa
254401 atggtgtcaa aatttttgtc tacatagact ataatataga ttatatatat atgagatata
254461 tattataaac tacatatatc acaaaccaac ctcaacacat gcagataaat attttaaat
254521 ttggcaaaat gacagtatgt tcatccatat ttctggagct gcaacaataa ttataaatta
254581 tacttgaaat aataataatt aacatctgtt gagcatctct catgaatcag tttaaattac
254641 attacttcta atccctaaac attgcaaagt agctattagt tttataattt tacagataat
254701 gaaataaaac tcagagatcc taaatgaact gcacaaggtc atttgactat aagacctctt
254761 tgaatttaga gttttctagt tcaaaagata tcttctaccc cttacacctt gccacctctc
254821 tgtactgtgc ttctccttac gtaggccaag gtgttcctcc caccaatctt atatactgtg
254881 ttgagtaccc attagttatt cctattggat ggatggagaa attaaggctc agcagagttt
254941 gaggatttca ctaagttcaa atgcccagta atttgcataa ctgaactttg aacaccttgc
255001 tttaatttca gcacttagct tttctaattt ctgatgtgga ttcatctata ttgatgataa
255061 caatcatcaa tatagactga tgtctctgtc tcgtcctggg tagccttttt atttttaaaa
255121 attctgtatt aaaatagttt cttgtctgtt gttaaattcc cttagagttt tatggccttg
255181 atgtgactca tgtgaccaaa cgttttgtta aaaaaaaaat taaacttatc aaaaccaagt
255241 ccttgttcta caagactctt gcatattcca agataaatca tggccagctt cacacacacc
255301 atttctaaat caacgagact ttattttctt ccttctactt agatgtcagt gcctttgtcc
255361 atttcttttt cacttctttg gtacatgttt acccagacag ttatgaccta cctgatactc
255421 tgcctaaata tcttccatgt ctcttgacat ccttaatttc ttaacattga aattgtttct

255481 ttttcctccc cattttccca cccccaatta atttttttct ttgtcgaagg aatttctcct
255541 tatctattta gtagaaagca gtcagagtgc ctcatagacc ccagtctaat attttacttc
255601 tcagagatat tttcattgga gatcataatg tttgatgtat gtaatgatga atttcagacc
255661 ctagagaaag ttatggaagg gtaagtggac ctggcctaca gggcatgata ttctggcaaa
255721 tcccacaaaa catatggtta tctcatcttc ctcaaactat agttgacctt atttcaaaaa
255781 tgcttggagc agaaaagcaa cgaggtatga aagacatgaa gtcaccacct tccattttat
255841 attactactc tatgaattgc atctagattt taaaactcaa cataaaaaac tgtcttcttg
255901 caagcatgga ctaataagga tagtagtagt tcacaaagaa tggcagctat ttacaaagtg
255961 aggtgatatt gtcaaaacct atcataatga caaagacaga actaaacagt gatagaataa
256021 caagcatatg tggtatgcat ttattatatg tattgtgcca tgcaaaaaag caatttatgc
256081 tgtatgtatt tacttggtga ggctctttcc agtgctaagt ttacttctag aatgacaatt
256141 catgtattca ttcatttgat tactatttgt ggaacaccta gaatgggtaa tctaagagaa
256201 ctatccagta cctgggtatt tcatggtgag caaaataaag tccttgcctc tgtggtgctc
256261 agttggggaa atattgtaaa caaagaaatg accagtggtt ttggccaatg tagtataatt
256321 ttatgttgtt gttatcctct gcagtcaaat atttgctaca gcttcttcat cctcaagaat
256381 tttaaatagt tgagaagagc caagatggct aacaagatgc agccaggaag agcttctccc
256441 aatgagaaac cagggtatta agaagattgg cacactccaa gtagatcttc agaagggagg
256501 cattgagagt ggatggagga aggatgtaga ctctgtgctg aagttagagg aagcttggaa
256561 tgctatgctc aggaatgagt tctaggactc attcctggct ccgagtgtct cctaaggaag
256621 ggagagttaa ataggcgtgg agtagcccac tctcaccatg gacctccata attctagctg
256681 caggagtctc aatgaccccc atggatattt gaactgatag ggggagctgc ttgaagagtt
256741 gacagggaca ggactgcaga ctgtgtggag ttcagaggat ttggcaaagg aaggactgca
256801 gtagagcaca gccaaggaca cccatccctg aagactcacc atgcccctgt aggctttggc
256861 ctttgttgac tgtcagacca atacagagca gggctgtctt gcccatggaa tgaggccatt
256921 ctgatctgag tgcctccctt gtctgccagc ctttcctgag gttcatgcca ggctgtgccc
256981 acttgcagca caggcttgga tgtccaactg gggcacttcc taaaggccat catcatagct
257041 tctttgttgg cagaccccac ccaactgttg gagagcttct gaagacaagc ctccacccgc
257101 acacaaccac ctgcagcctc ccccctctgc tttgccaaca cgcaggtatg catggaccct
257161 gctgctctgc tgccaccaac acatgagcac agaccccact gccactgccc cgacaaagca
257221 ctgttgccag caaccctcat ggagcattgt tgttaacaga ctggaaacac ctcagctcct
257281 tcagcacagc aggtgtttaa ccttgagagg ccagagaaca aagctgtgtg cctggtccta
257341 taccactggg ttacagcaca cagtccagga gtgctgagct gagcccaggt cccctgaaat
257401 cagccagaaa tgaagccagt tgactagacc taacttatat cacagtcaaa acctcaaagg
257461 catcaaagaa ttgcaacttt aaagattaaa gaaacatcag ctcacacaga tgagaaaaaa
257521 ccagcaaaag aactggggca actcaaaaag ccggagtgtg ttctcacttc caaatgacca
257581 tgctagctcc ccagcaatgg ttcttaatca gactgagatg gctgaaatga cagacataga
257641 attcagagtc tggatggcaa tgaagattgt tgagatttag taggaagtta aaacccaata
257701 cgatgaacct aagaatccag taaaacaata caagagctga aagacaaaat agtcatttta
257761 agaaccaagc tgatctgata gagctgaaaa ccccattaca ataatttcat aatacaattg
257821 gaagtatcag gagcagaaca gcccaagctg aggaaagaat ctcagagctc aaaagtggtt
257881 cttcaaattg actcagtcag acaagaataa aaaagaaaga ataaaaagga atgaacaaag
257941 cctccaagaa atacagaatt atgtaaaaag accaatccta tgactcactg acatccctga
258001 aagagagggg gagagagaaa gcaagttaga caacatattt gagggtactg cctatgaaaa
258061 tttccccatc ctcactagag aggtcaacat tcagattcag aaaattcaga gaacccctgc
258121 aagatcctat aaaagatgac catccccaag acacatagcc cacagattct ttaaggtcaa
258181 tgcaaaagaa aaaatattaa aggcagctgg agagaaggga aagacatcta taaaaagaac
258241 ccatcacact aatggcagac ctttcagcag aagcagtaca accaagaaga gattagggcc
258301 tatattcacc atcctaaaag aaaagaaatt tcaactaaga atttcatatc gagcctaact
258361 aagcttcata agcaaaggag aaataaaatc cttttcagac atgcaaatgc taaaggaatt
258421 cattactatc acacttgcct tacaagaggt ccttaaggaa gtgctaacat ggaaataaaa
258481 aactgttacc aactaccaca aaaacacaca taagtacata gaccactgac actataaagc
258541 aactacacaa tcaagtccac ataacaacca actaacaaca ctatgacagg atcaaatctt
258601 cacatatgaa tattaacctt aaatgtaaac gggctaaatg ccacattcaa aaggcacaga
258661 atggcaagtt ggatcaagaa gcaagaacca actgtatgtt gtcttcaagg gacccatctc
258721 acatgcagtg acacccacag gctcaaaata aagagattga taaaaatcca tcaagccata
258781 aagagattga taaaaatcta tcaagccata aagaaattga taaaaatcta tcaagcaaac
258841 agaaaacaaa aagagaagag cctgttattt ttattttaaa ccaaacagac tttaacctaa

258901 caataatcaa aaagggacaa agaagaggat tacataaaga taaaagattc aattcagcaa
258961 gaagacttaa ctgtcctaaa tgtatatgta cccaacactg gaacacccag gttcataaaa
259021 taagttcttg acccacaaaa agacttagat aactacacaa taatagcaag agagttctgc
259081 accttactga cagtattaga cagattattg aggcagaaaa ctaacaaagg tatttgggac
259141 ctgaactcaa cacatgacca aatggaccta acagacatct acagaacact ccacccaaca
259201 acaacagaat atacattctt cttatctgca aatgacacaa actataaaat caaccacatg
259261 ctcactcata aagcaattct cagcaaatta agaaaaactg aagtcacacc aacaacattc
259321 ttggatcaca gtgcaataaa aatagaaatc aataccaaga ggatctctaa aaatcacatg
259381 attacatgga aattaaatag cctgctcctg aatgactttt gggtaaacaa tgaaattaag
259441 gcaaaaatta agaaattatt tgaaactagt gagaacaaag acatgacata ccagaatctt
259501 tgggacccag ttaaagcagt ttaaaaggga agttatagca ctaaacaccc acatcaaaaa
259561 gttagaaaga tctcaaatta acaacttaac atcacatgta gagaaaccag agaaacaaga
259621 gcacaccaac cccaaagcta gcagaagata agaaataacc aaaatcagac ctgaactgaa
259681 tgaaattgag acacaaaaag ccatacaaaa ggtacataaa tccaagagtt gtctatttga
259741 aagtataaat aagattgata gaccactagc tagattaata aagaaaaaag aagattcaaa
259801 taaacacaat cagaaatgac aaaagggaca ttaccaacaa ctcaaaagaa acacaaaaaa
259861 ccctcagaga ttattacaaa cacctctatg cacacaaact agaacactta gaagaaatgg
259921 ataaattcct gaaaacatat aacctcccaa gattgaacca ggaagaaatt gaaaccctga
259981 acagaccaat aacaaattct agaattgaat cagtaataaa aagcctatca accagaaaaa
260041 gtcctggacc agacaaattc acagccaaat tctaccagat gtataaagaa gagttggtgc
260101 caatcctact gaaactattt caaaaacttg aggaggaggg atgcctcttt aactcattcc
260161 atgagaccag catcattctg ataccaaatc ctggcagaga cacaatgaga aaataaaact
260221 tcaggccaat atccttgata aacatacatg caaaaatcct caacaaaata atagcaaacc
260281 aaatccacca gcactcccaa aagtaattta ccatgatcaa gtaggcttta ttcctgggat
260341 gcaaggttgg ttcaacatac acaaataaat aaatgtgatt tatcacataa acagaatgaa
260401 aaacagaaac tacgtgatca tctcaataga cgcagaaaag gctttggata aaattcaaca
260461 tcctttcatg ttaaaaactc tgaacaaact aggcattgaa ggaacatacc tcaaaataat
260521 gagccatcta tgttaaactc acagccaaca tcatactgaa caggcaaaag ctggaagcat
260581 tatccttgag aactggaaca agacaaggat acccaccctc actattctta ttcaacatag
260641 tacgggaagt cctagccaga gcaattaggc aagagaaaga aataaaaggc acgcaaatag
260701 gaagagagga agtcaaacta tctctctttg tggacaatat aattctaaac ctagaaaacc
260761 acatagtctc tgcccaaagg ttcctagatc tgataaataa cttctgcaaa gtttcagaat
260821 aaaaaaccaa tgtacatttc tatacagcaa tattgtccaa gccaaaccag gaatgcaatt
260881 tcattcataa tagccacaaa aagaatataa tatctaggaa tatagctaac cagggaggtg
260941 aaagatctct acaatgagaa ttacagaaca tagcttaaag aaatcagaaa tgacacaaac
261001 aaatggaaaa attccatgct catagatggg aagaattaat attgttaaaa tggccatatt
261061 acccaaaaca ctttacagat tcaatgctat tcctatcaaa atacaaataa cagtttttag
261121 agttagaaga aactatttaa cattcatatg gaacccaaac accctgaata tccaaagcta
261181 tcacaagcta aaaattaaaa aaaatttaaa aagctggagg catcagaatc acactacctg
261241 acttcagact atgctacaag gctacagtaa ctaaaatctc atgatactga tagaaaaaca
261301 ggcacataga ccgtattagt cagggctctc tagagagaca gaactaatag gagatatata
261361 tatatatatg ggagcttatt agggagaaag gctcacatga tcacaaggtg aagtttcgtg
261421 ttaggccgtc tgcaagctga ggaaggaaga agccaagagt ggctcagtct gagtccaaaa
261481 gcctctaaaa cagggaaacc gacagtgtag ccttcaatct gtggctgaag gcctgagagc
261541 ccctggcaaa ccactggtgt aagtcccaga gtccagatgc tgaagaacct ggagtctgat
261601 atccaagagc aggaggatga aaggaagcat ctagtacagc aaaaagatga aagccagaag
261661 actcagcaag ccagcttacc ccactttctt ccacctcctt tgttttagcc actgtggcag
261721 ctgattagat gatgcccacc cacattaagg gtgggtcttc ccctcccagt ccactgactc
261781 aactgtcagt ctcctctggc aacaccttca cagacacacc aagaaacaat attttaccag
261841 ctatctaggc atccttcaat ccaatcaagt tgacacctaa tattaaccac cacatagacc
261901 aatggaataa gttagagaat ctagaaataa agctgcacat gtacaagaac ctgatttttg
261961 acaaagttaa caaaaacaag caatggggaa aggactccct gggtcataca cagcattgga
262021 ataactggat agccctatcc agaagatcaa agctggactc tttcctttca ccagatacaa
262081 aaatcaactc aaggtgggtt aaagacttaa atgtaaaatc gaaaactata aatttttagt
262141 ttttaaaact aatctataaa aactcttgaa gaaatcctag gaaatactat tctggatatc
262201 ggccctggca aatccttcat taagaagact ccaaaagcaa ttgcaataaa aaccaaaatt
262261 gataggtggg acctaattaa actaaagaac ttctgcacag caaaagaatt tatcaacaga

262321 ctaaacagac aaccaacaga atgagaaaaa tatttgcaaa ctatgcatcc aagaaaagtc
262381 taatatccag aatctataag gaacttaaac taaccagcaa aaacaaaaca aaacaaaacc
262441 ctaaaaaacc attaaaatgg gcaaaagaca tgaacagaca cttctcaaaa gaaggcacac
262501 acggccatta agcatataaa gaattactca acattactaa tcattagaga aatgcaaagt
262561 aaagccacaa tgagatacta tctcacacca gtcaaaatga ctattgttaa aaacaaacaa
262621 acaaacaaac aaacaaaaaa cagatgctgg catgactgtg gagaaaaggg aacacttaga
262681 tgctgttggt gggaatgtaa attagttcag ccactgtggc aagcaatttg aaaatttctc
262741 aaagaactca aaacagaact accatttagc caagcaatcc aattactggg taaatatcaa
262801 aggagtagaa atcattgtac tataaagaca catgtactca tatgttcacc acagcactat
262861 tcaaaacagc aaaaacatgg catcaaccta gatgtccgtc aacagtgtac tggatgaaga
262921 aaatgtagta tatatacacc atgcaatgct atgcagccat aaaaaagaat taaattatgt
262981 ctttgcagca acattgatga agctggaggc cattatccta agtgaattaa tgcaggaaca
263041 gaaaaccaaa taacctcata ttctcacttg taaatggaag ctaaacattg agtacacatg
263101 gatgcaaaga agagaacaat agacaccagg gcctatgtga gggtggagag tgggagcaag
263161 gtgaggattg aaaaactacc tattggttac tacgctcatc atctgagtaa tgaaataatt
263221 gatacaccaa acccccacaa catgcaattt acccatgtaa gcaacctgca catgcattcc
263281 ctgaacctaa aataaaaatt agaaagaaaa aaataataat taaaaaaatt atctggggga
263341 aaaataattt acattagtag caccctcttg tcttcagggg atatgttcta agacccccag
263401 tggatgccta aaactaagga tagcaaattc tattgctgtc agttggaaca tgtttctttt
263461 catgtcttca gtccacatat ttaatgactt ttccacttta actaaatttt aatcacacac
263521 tgtgaccatc acttttacag tttgaggtac aacagcaaaa ctatcattaa tttctttttt
263581 cttcttcaca atttcatgga tagaagattt gttcttactg tagatcttag caacctcagt
263641 gtatgatttt cttttccttc cttattaggt caaaaacttt tgccttttca cttaaaggaa
263701 gcactttaag tcttatctgt ggcatatctg aattgccagc atcactgctc ttgcactttg
263761 agagtattac atgaaataag ggttacttga acccaagcat tgccttactg taatagtaag
263821 tctgataact gagaggacta ttaacaacat gggtatacta ttgtatccac aggatgcatc
263881 cacactaaac aaagggagaa ttcacatcct gggcagggtg aagtgggaag gtgaggaatt
263941 tcatcacagt actcgaaaca tcataaaact taaaacatga attgtttatt tctagaattt
264001 tccatttaat atttttggaa ccatgaattg atcacaagta actaaaacca tggaaaagag
264061 agaactagtc cacatgttgt ttagtatcgc tattacagta atttcttttt tcttgtgtgg
264121 tcttatcctg tgggcttcta gaaaattgtt tcctacctgc ctctcaatcc ccttcctctt
264181 ctcaactttc attttagcag aggctacatt atagtgaccc tgttggttta ggatttaaag
264241 aggcatttat ttgatttgtt aaaaacctcc acatgcaccc atgaaataac caaatgacct
264301 cagggtataa atggagctgc aaacttgtcc aagtcaagaa attcagtata aaaaccttag
264361 gcaggccagg cgcggtggct cacgcctgta atcctagcac tttgggaggc cgaggtgggt
264421 ggatcacgag gcagaatatc gagacaatcc tggctaacat ggtgaaacct cgtctctact
264481 aaaaatacaa aaaattagcc aggcatggtg gcgggcgcct gtagtcccag ctactcagga
264541 ggctgaggca ggagaatggc gtgatcccgg gaggcggagc ttgcattgag ccgagatcgc
264601 gccactgcac tctagcctgg gcgacagaga agactcctcc tcaaacaaaa acaaaacaaa
264661 aacaaaaaca aaacaaacaa aaagaaacac cttaggcaac aacatatatg ctagaattgc
264721 ctttgctaac tcccctggca aagcaaatga tgtattgaga ttgcttctca gataaggtgg
264781 ttgaaaatga tagcaatctt aaaaaatgag cagttttcat aaatttgtat acattttgct
264841 ctatagacaa agagtagagc ttatagctat catggccata tctcaataaa aatgtgttgc
264901 aagcaggttt gccttgggca tctttcctta ttcctttcag tattccactt cttttcacac
264961 cctttcccac atgttagatg ttcccagtct gctcattcag ttcgggctcc cttccctggg
265021 aggctgtctt cagctcaaaa ctttgccagt gataaaactc cattcaaaac ttatctgcca
265081 agtggccatt tttacttgaa gccctgccct tggtcaatga cagtgcaaaa actggatagt
265141 ggcatattaa tcaaataagt cattttctcc ctaggttaaa ccatagggaa tatctgcttt
265201 caaccctttt ggcctccaaa aaaaaaaaaa aaagcatttt gtatggcttt tcatacctca
265261 tggtgtccta gcttgagcac tggagcaaag tcagaaaatc tgagcccttg acttatctct
265321 gtggttcact gctggataat gtccagaaaa ttatttcatg cctttgagct tcagcttcct
265381 tatctgtaaa aaggagagta actaccttga cttcttgctg agattttaaa gagaacaaat
265441 gtgaaagcac acacaatcac catttgcaaa gggtaaagtg gtagatggat gaaaggaata
265501 atgatctgta atttttcctt acatgctagc ctatttaaaa tgtatgagcc tccatgataa
265561 gtgtggctaa tagtggggga atgtagatga tctagagata ccggtcaacc tacaatgtca
265621 gctttatgtc attgtgcccc tttcttcttt ggggtttata tgactataat attttaaaaa
265681 ccaaaacctt atgggaccat tttataattt gtgtcatatg aaattttaat tttgctattt

265741 atagtgaaag ggaaaaaagg aagtggagta tataggatat agatctctaa ccactaaaat
265801 aaacctgtga gtaaatgcat tttaagctct tcatgcaaat acatatatat atatatatac
265861 acacatatat atacacacac acacatataa ttactgatta aaaatatata taaaatcaca
265921 tatatataca tataaaacca cttggcattc aggttgtata aaaatgttga tttttattca
265981 agaattgtag agtagatgga aatttaatgt actcaatgta atctctattt gggatgagat
266041 tctaggggga attaaaattt acataggagt ttctttgcct ggtttagtcc atccagaggc
266101 ttcagatggt ctgggaacca actaaagttg tgtggagcaa tgtaaaagtc agtatatatg
266161 tttcttttcc cctggggagg gattcaatag atttcatcgt attcttaaag aaattcttgt
266221 cctaccaaag gttaagaact aattatctca gttaaacaaa acacataaaa atatgttttt
266281 atatacatgt tgaattattc aaatcttcag tgcttagtag tttattaatc ttgaaggcct
266341 agaaagtgaa taatttcaac aactgaacaa aaataaaatt taaaaagaaa ctatttctg
266401 gtcattagaa caaacttgtt ataaaatata tgtctttttg ctattgcttc tccacttcaa
266461 cactcatgat catgttttgt tgctatttcc cctatctcat tttttctggc tgctgggtta
266521 aacttcctgt ttttgcagaa tgaccaaaaa cattggtgat gatggaggtg gagatgacaa
266581 cactttcaat ttcagctgga aggtctttac cagctgggac tacctgatcg gcaatcctga
266641 aacagcagac aacaaattta attctatcac aatgaacttt aaggtagagg caccaacttc
266701 aaaaacctgc tgtttgtatt tctaagagta aaattggagg cattccatct ggtccagtag
266761 tgcagtatct tgacaattta catgaaagat taactcatgg tttctgccta ccaagatctt
266821 gccatggttc tgattataac tggaaaatct tccatgaccc acaacagcac tattattcct
266881 taaacacttc tcttctctaa atctggtaat aaaataatgt tactggattg aaataggacc
266941 atgagtaatc agaataagat attttaaaaa ctagaacaat gttcactaat tatttacagg
267001 aataagcata atattgaagt gaccctaaaa cattattttg tagttttttt tttaattcac
267061 tggaatgaag atttttagag tgaagactat cctaatatca gagcaatcac agtttgtttg
267121 caagaattct tccttctgtc ttacttgcca tcgtttgttg gcaatattca cctggtttgt
267181 ggaatctcag atttagttta cattgtcatt cctcacattc tgatgattgt aaaaataact
267241 tttgaggttt tgatactttt aaaatgtagc taagatattc tattttgggt ttttgtttgt
267301 ttgtttgttt tcatggatac aggaagctat cacagaagaa aaagcagccc aagtagaaga
267361 aaacgtccac ttgatcagat tcctgaggtt tctggctaac ttcttcgtgt ttctaacact
267421 tggagggagt ggatacctca tcttttgggc tgtgaagcga tcccaggaat ttgcacagca
267481 agatcctgac acccttgggt ggtgggaaaa aaatgaagtt cgtctctgca tgctttttat
267541 gtgcttagaa cctgacattt gtttcttttg tgggttatgt ttctttgatg gaacatttaa
267601 aaaggccacc ctttacctat cccctatcct gcccttacag ttaaccaacc ctgttgctca
267661 ttttccattc tattctagtc ttatttctaa atctggactg tctgtttttc atttgtgac
267721 atgatggaaa gttagagtca gaaaagatat taaaaaacac atagtctaat cattatgttt
267781 tacagatggg aatgataaat ctcaagagat tgcattatat gtttatggtc acagagttag
267841 gagaagagtc agggtgagaa cctaggaatc ttgacttcga gtccaaggct cttgctgctt
267901 tctctgcaaa tccttcatct gtcattaaaa gatcaaattg ttgcctgaac tttttgaagc
267961 agccatgata aagccactga ttcctatgtt gatctcactg gaaacactca gattgcttct
268021 ccacctttgg gcatgtctgg gcttccctct gttttcatta gaagcctaac tgcagcatct
268081 ccatctccat ttcctcctgt cctgtacctt actttctctc cctctaaatg ctattcaaat
268141 gaagaaggcc agctcttttc caggaattaa acattatgac cgtaatttgc ctttagagta
268201 tctgcccatt tagtgaatca gagaaaggag gttagacata gatcatttag aatcagaaaa
268261 tcattggtca tgagttgttg ctagagtctg ttcccgttca taaaatgctt atgtgtatga
268321 tgtgacgtca ttttgagctt gggcattttc aaagtatgtg cagatttagg ctgtgggaag
268381 tgtagacttc ccacactgaa acttttgcag aacagtatag atcaaaagaa ttaaaatatg
268441 tctttataag catagaattt tctaaaaaat cttggacagc aaatgatata atctacttac
268501 agaaaaaggt tccaaataac attctgagta tttcagaagt ggtaccaata gagacgggac
268561 tagatatttt aaagacttta atatacagtc atgtcataaa aacttgtaaa atataagtaa
268621 tttagcagag ggcttttatt tatttaaaat gaaaaatgta ggaacacctc aaagcattta
268681 ctaagtttgc tttcaaaatg gtctcataat tgtaatagta catagaggta acattatcat
268741 gcagctttgg tccattattt atattggtta tgtttttgta taaataaacc atattaaagg
268801 atgggtagtt gattatcaat aaaataacta ttctttgtaa gaacctcttt aacatatatt
268861 ttagggaccc tctgagtcct aggggccaat gaaggtaata atggagattc atgagtaact
268921 ttacaaattt gataaaacca aatttaaatt tgccttgcat aagtttgcac aggataagcc
268981 aaattttctt tacattagtc taaaattata gcatgctgat tttgtattat gtcatgctga
269041 gatttggatc ctgttggtca tacttatata atgttttaat aaaaatgaca aaaatctaat
269101 agcaattgtt ttctaaatat agtctggtag ttattataat tattcacacc acgaagtcca

269161 caggagaaaa taggaaatgt ttagtggtga cagtttgagc cgggtatttg ggaaaggaga
269221 tagtcaccag agttacctaa aatcggtatt tccctattag aaaattagta catgttttgg
269281 attcttttta ttgacttcct ttagccaaat aatatcatca taaggttgcc aaacatccag
269341 gaaataaaaa gcacatgttt cctacttttc atgtttctat tccttcttct ttctctctcc
269401 cccctctccc cacccctgca ttattctctt gtctctgaaa ttatagccca ttctttcact
269461 tttgaaactt ttctacgttg gcttccataa cattatgtca tgtgttctcc atttgtttct
269521 ctgagttcct tctgtttcct ttaataaaac tcattgtatg tgtttatgtt ttcggcctta
269581 aaagtttaca tttgccaaaa ttcaatattt aggcctaggc ccatctcagg gaattcatcc
269641 attctaactg attaaactag cccatatgct gatgattgtc agattagaat tttcagtttg
269701 aacctcctcc tcaagcagat tattttccat ttcttttgga aatcttgttg tgaagtgcat
269761 caccatatca gaatcagtta ccacatagaa caatatgtgt tctctgtgta gagactgatg
269821 taaattgcca ccttcactat accaggaaac agactgttac tactggcaca tacacacact
269881 cagtcacatg cctctcttga gaatgtgaat tgaatatttt ccctgtcatt ttattgagat
269941 caaattctaa acgtgcataa aatagtcatt tttagtctta aaaaagatca aagaagccta
270001 gctcagaatc ttccaaaatt ctggcaaaaa gcaataataa ctttaaacac cattaaccta
270061 gtttctccct tgtgcctcct tgtagatgaa catggttatg tccctcctag ggatgttctg
270121 tccaacattg tttgacttat ttgctgaatt agaagactac catcctctca tcgctttgaa
270181 atggctactg ggacgcattt ttgctcttct tttaggcaat ttatacgtat ttattcttgc
270241 attaatggat gagattaaca acaaggtaag ccttgtttct ggattgtcct tgccataaga
270301 gtgttgttca attcccagtc tcaagtttgc cagaaatgcc tttgaaatct ctgttgtctt
270361 cattttagtt tctatctctt tgctttctag attgaagagg agaagctagt aaaggccaat
270421 attacccttt gggaagccaa tatgatcaag gcctacaatg catcattctc tgaaaatagc
270481 actggaccac ccttttttgt tcaccctgca gatgtacctc gaggaccttg ctgggaaaca
270541 atggtgggac aggtaatgcc accaacagaa gtgtatggca attagtagat taaaaaaaga
270601 gagtcaatat ctcttccata agattggctt ttaaaaaatt gcttattagt aaaaatagct
270661 aagatttgtt gaatgttgac tgtgtgctgg ctctgaattt catgtatatt atctcacact
270721 gtttgcaaat ctatcccttg aggtaaatac tatttgtatg tcccctttac agttgcatat
270781 aaaggaagga tagttgaaaa tggtgcaaag agatgtttac tatctgtatt ataataaagc
270841 actcgattta gatacaggcc aaagcaaata tttaaatgga actctattta gtgttacttg
270901 tttgctgtaa aacaaaacaa aacaaaacaa acaaacaaac aaaaacagag tcttggggt
270961 ggccaagaca gccaactaga ggcagctagt gtgcatggct ctcatggaga gaaatagagg
271021 ggttgagtaa atatagcacc ttcaaccgaa acatccaggt acatgcattg gatctaatca
271081 aggaaacaac ttgacccatg gagagcagag aaaagcaagg cagggtgaca gcccacctgg
271141 tagtgacatg aagccaaggg atccttctat gcccagggaa gtggtgagtg aatgcacgac
271201 cttgggaacc catacttctc ccgtggatct ttggaaccct tgtgtcagga gatatcctca
271261 tgaacccact ccacctgggc cttcagtccg acacacagag ctatgtggag tctcagcaga
271321 gcagccactt aagcacatgc agagacccgg gagccttaga tacctgggct tttctttttg
271381 ggcttcctgt caaaagtagc tatacctctg gcaaagcagg aggttagacc cccaaccaca
271441 ccccttagaa aaggggctga atccagtgac agtctgcagg ccccacttca cagcacctca
271501 cagaatgaaa cccactggct tggaactcca gccagttacc ggtagaaatg ttgcacctcc
271561 ctgagacaga gctcctgggg gaaagtgtgg gccatcatct ttgctgtttg gttgacttgg
271621 ccattccatc attcaggctt cagagagtcc aagccaacca gcggctgaag cagaccctga
271681 gcacagcata gctgttctat gaaaatgtgg ctagactact ttttcaaatg ggtctcctat
271741 ccctttcctc ctcactgggt aggacctcct aactggggtc tccaaccacc tcctacaggt
271801 gcgtttgggc cagcaacggg tctgtacatt tctgggatgg agcttccaga ggtgggggca
271861 ggctgccatc tttgctgttt cacagccttc actgttgcta cctccagata ataaaacatc
271921 tgaggtgacc agggacggat gtggggtccc aatatactgc agcagcccta tgaaaaagtg
271981 accagacttt tacatgggtg cccatcccca tatcggctca ccagacacat cctccaggcc
272041 taggcctcca gctaccctct gctagaatta tcgatcaagt agcagctctg taactccctg
272101 gacaggatcc ccaggggcaa ctgaaagttc ctctgccact acctctgcag tagaactgcc
272161 cttgctaccc ttggactaac aaaggaacaa agaccctaag cgcctcatcc atacctccaa
272221 taagctgcag ttgactccag gagagaaggc cagtctgtct ctcacaggtc ctacctaccc
272281 ccacccccct ccttgtcacc agacagggaa cccccagctc ggacccacaa catagaacat
272341 ccatcctggg ctaattgcaa ggagtaattg ctgacctgca tctcaatggg gtggagcccc
272401 caggagataa gcaaaagacc cttggccata accatgccaa ggtatcttcc tctgctgctg
272461 ccaagttgga gaagaaacat aacgctgaga ttgccccaga gctgaagggg cagcccagga
272521 gtgccaagcc gtaatctaca gccagctctc aagggggaaa gaaatctaca ctttcagagc

272581 attgagaggg aacatggctg caactgtgcg gcaacacagg ggagcctcac aaccaagcaa
272641 gagtctacca actgaccaat atgccgaagt gccacctact ggatcacatt ccccaaagca
272701 tcaacaccaa aaatatctca ctaacatacc cccctctgaa accaaagaca agtactcagc
272761 ttcaaataaa gaccctgtgc aaagccttgg cccaatgaaa acttccagga aagatgccta
272821 ttgactgtat tcaatctaca ttgcagttaa aggaacacct acacactgag atgagaaaga
272881 accaatgcaa gaactctggt aactcaaatg accagagtat tgtatgtcct ccaaacaacc
272941 acaccagttc tccaacaaga gttcttaacg aggctgagct agctgaaatg actgaaatag
273001 aattcagact gtggagagga atgaagatca ttgagattca gaagaatggc aaaacccaat
273061 ccaaggaaaa taagaatcac aataacacga cactggagct gaaggacaaa gtagctgtta
273121 taaaaaagaa gctaataggt ctcataaggc tgaataacac aatacaggaa tttcacaatg
273181 caatcacaag cattaacagt agaataaacc aagctgagga aagaatctta gaacttgaag
273241 actggctctc tgaaataaga cagtcagata aaaataaagg aaaaagaata aaacggaag
273301 aacaaaacct ccaagaagta tgggattatg taaatgccaa agctatgaat cactggcatc
273361 cctgaaaggg atggggaaaa agcaaacaac ttggaaaaca tattttagga tattatccat
273421 gaaaacttcc ccaaccttac tagagaggca aatttgctgc cagtctttgc taacagtcaa
273481 atttaggaaa tacagagaat tcctgcaaga ttctacacag gaagatcatc cccaaaacac
273541 acaattgtca ggttttctca ggtcaaaatg aaaaaatgtt aaagacagcc agagagaaag
273601 ggcaggtcac ctacagagag aacaccatta ggctaacagc agatgtctca gctgaaaccc
273661 tacaagccag aagcagtcag ggtcctatat tcaacattct taaagaaaaa aaatcttcaa
273721 ccaagaattt catatcaggc caaactaagc ttcctaagca aaggagaaat aagataattt
273781 ttgggcaagc aaatgttgag ggagtttgtt accaccagac ctgctttaca agacatcttg
273841 aaaggagcac taaatggagt aaggaaagac tatttgcagc taatacaaaa gcatacttaa
273901 atacacagac cagtgacact gtaaagcaac catacaaaca agccagcata ataatcagtg
273961 aacaacacaa tgacaggatt aaatccatac atattaatac taatcttgaa tgtaaatggg
274021 ctaaatgctc catttaaaag gcacagagta gcaagctgta ttaaaaaaaa agcaagaccc
274081 aatggtatgc tgtcttcaag agacccatct catgcttaat aacacccaca cgctaaaaat
274141 aaagggatgg aagaaaatct accaaccaag agaaaacaga aaaaaacatg ggttattgaa
274201 attaaaattc ccaatttcaa aaaaaaaatg caaaaaacaa aacacacaca tgcacacaca
274261 aatacacttc aaaccaacaa agataaaaaa aaggcaaaga agggcattac ataatggtaa
274321 agggttcaat gcaacaagaa gacctaacta tcctaaatat atatgcactc aatacaagag
274381 tacccagatt cataaagcaa gttcttagag acctacaaag agacataggc taccaccacc
274441 caataatagc ccaggccctg atgaattcac agctgaattc taccagatgt acaaagaaga
274501 actggtaaca ttcctactga agctatttca cgaaattgag gaggaggagg aacttcttcc
274561 cagctcatcc tatgaggcca gtatcattct gctaccaaaa cctggcagag acacaacaaa
274621 aaaagaaatc ttcaggccaa tatcatgagg cccatcaatt caaaaatctt caacaaaata
274681 cttgcaaacc gaattcagca gcacatcaaa tggctaatcc accacaatca agtaggcgtc
274741 atccttggga tggaaggtta gttcaacata tgcaaaacaa taaatgtgat tcatcatgta
274801 aaaagaaata aagagaaaaa ccacatgatt atctccatag atgcagaaaa agctttcaat
274861 aaaattcaac acccgttcat gttaaaaact ctcaataaac taggtattga aggaacatac
274921 ctcaaaataa taagagcctt ctatgacaaa cccacagcca aaatcataat gaacggacaa
274981 aagctgaaag cattcccctt gaaaaccagc acgagacaag gatgccctct ctcaccactc
275041 ctattcaaca tagtatttga agttctggcc agggcaatca ggcaagagaa agaaatgaag
275101 ggtatcaaaa taggaaaaga gggagtcaaa ttatctttgt ttgcagatga catgatccta
275161 tatctagaaa accccattac cccggcccaa aagcttctta agcggataag caacttcagc
275221 agtctcagga tatatcaatg tgcaaaattg ctcgcattct tatacaccaa caacaggcaa
275281 gcagagagcc aaatcatgaa tgaactcaca tctacaattg ctacaaagat aataaaatac
275341 ttaagaatgc agctaacaag ggaagtgaag gacctcttca tggagaacta caaactactg
275401 ctcaaggaaa tcagagagga cacacacaaa tgggaaaaca ttcaatgctc atacatggga
275461 agaatcaatt ttgttaaaat ggccatactg cccaaagtaa tttatagatt caatgctatt
275521 cccattaaac cacaactgaa attcttcaca gaattagaaa aaaactattt taaaattcct
275581 atggaaccaa aaaagagctc gtatagccaa gacaatccaa agaaaaaaga ataaagctgg
275641 aggaatcaca ctacctgact tcaaactata ctacagggcc acagtactca aaacagcatg
275701 gtactggtgc aagaacagac acatagacca atggaacaga atagataact tagaaataaa
275761 accacacaac tacaaccatc tgatctttga caaacctgac aaaaacaagc aatggggaaa
275821 atatttccta tttaataaat gttactggga gaactggtag ccatatgcag aaaattgaaa
275881 ctggacctct tctttacacc atgtataaaa attaactcaa gatggattaa aggcttaaat
275941 gtaaaaccca aaactatgaa aactctagga gaaaatctag gcaatatcat tcaggacata

276001 ggcacaagca aagatttcat gacaaaaatg ccagaagcga ttgcagtgaa agcaaaaatt
276061 aacaaatggg atctaactaa actaaagagc tccagcacag caaaagaaac tatcatcaga
276121 gtgaacagac aacctacaga atgggagaac atttttgcaa tctatccatc tgacaaaggt
276181 ctaatatgca gagtctacaa ggaaattaaa caaatttaca agaaaaaaac tggatcccat
276241 taaaaagtgg gcaaaggaca tgaacagaca cttctcaaaa gaagacatac atgcggcaaa
276301 caagcatgtg aaataaaact tcatatcact gatcattaga gaaatgcaaa tcaaaaccac
276361 aatgggatac cagctctcac cagtcagagt gattagtagt aaaaagtgaa aaaataatat
276421 gctggcaagg tcgcagagaa aaaggaacat ttatacactg ttagtgggaa tgtaaaatag
276481 ttcaaccatt gtggaaagca gtatggcgat tcctcaaaga gctaaaagca gaactatcat
276541 tcaacccagt catcccatta ctgggtatat acccagaggt atataaatca taaagacaca
276601 ggcatgtgaa tgttcattgc agcactattc acaatagcaa atacacggaa tcaacctaaa
276661 tgtccatcaa tgacagattg gataggaaaa atgtgatata ccatggaata ttatgcagcc
276721 ataaaaaaga atgagatcat gtctgttgca gaaaaatgga tctattatcc ttagcaaact
276781 catgtaggaa caccaaatac tgcatgttct tacttatatg ggagctaaat tatgataact
276841 catgaacgca aagaaggaaa caacagacac tggggtctac ttgagggtgg agggtgagag
276901 gagggagaag agcagaaaag ataactattg ggtactgggt ttaatacctg ggtgataaaa
276961 taatctgtac aacaaactcc catgacacaa atttacctat gtaacaaagc ttcacatgta
277021 ctactaaacc taaaataaaa gttttttttt aatggataat ttggggagca cacactaaga
277081 aattttaata atgtcaattt ttgttaaata attttccctt aaaagaaaaa aaatactatt
277141 ttaagttatt tgttttcatg atgattttgt aaataacccc agcccaagac tagccatacc
277201 ttaccagaga aaagaaataa tgtttcatat ggctttctat cccttaaggg aaaccaagac
277261 aaataaaacc aaacaaaacc caagaaaaat ctacttcaga tctcgtattt ttatacaaat
277321 ccaatttata ttttgaaaag cttttatgat ttcatttagt tagaattgat attcctaacg
277381 tgcagtccat aaattgattt tggggcttca gattatcatt gagaacagct tttgtcataa
277441 agcttctgta tgttagacaa cagttgagtt tagcactgaa tgtagtgttt gtgaaattat
277501 ttgaacaatt ttttactgca ttatttggta agataacata aaaattgaag gactagcatt
277561 agtcttataa aattcatata atttaaacaa atatgtaaaa atatatctta ggcttcaatc
277621 agatgctcat caaacttaag ccaatcaaga tttcaactgt gaaagaaaaa aataagataa
277681 atgtgtaaaa tgctagacat gtatcattaa tgattgagta caaaaattat ggtataattg
277741 aaaattctat tttattttat ttgttaagct ttcgggaacc agataaatcc cccctttgat
277801 gatatatgct attaatttga ttttcagaca tgcacatttc atacaactta caggaacata
277861 ttacatatga aataataaaa tcaaagtcat ctgcacttaa tacaggagtg cttgtgattc
277921 aggatgtggt agacagaata atggccctct ataacagtac acattctaat ctctggaacg
277981 ataaatatgt tacatggcaa aatggacttt gcagatgaaa ttaagttaaa gatctcagct
278041 tgaaagaatt attgtggatt atgtgagcag gcttaatgaa atcataagga tctttataag
278101 caggagataa agggaaaaag tcagaaaatt gagatgtaac agcagaagca gaggttggaa
278161 tgatgtgctt taaagatgga ggaaggagat taagagccaa ggaatgcagg tggcctcaga
278221 aactgaaaaa gaaaggaaac atattctctt cccaagtctg cactttgaat ttagacttct
278281 gacttccaga actgtaagaa gataaatttg cattgtttta aaccactaca tttatggcaa
278341 tttgttacag caacaatagg aaatgagtac atcatggtac ttgggtctcc ctgttccatg
278401 gctttttgaa gtgaagtctg gttgtggtgc ttaacacgag tctgttttat cacaactaag
278461 ccgctcccta aatcttcagc atacgattca agaaaggaaa atataggaag tacagtttta
278521 gaaactgaag gaatttgaga cgttctcttc tgcacatcct agaacacagg ttggcctgaa
278581 agctaagaaa cctgcacttt aacaagcaaa aacatctact gaatgggatt aatagaatta
278641 gctgtaaaga gtaggatctg ggctggccac ttctggagtg aaggaatgga ttaattagat
278701 aggaatgcga atgcaatcta ggagaatatt catgtttcag atgcagatgg ttctgcgata
278761 gtcccacaca tactgactta aaagagaaga aataccattt agttaatcct ccaccttgga
278821 agcccgtgga ttatgctgct tgtctttttt tttttttaaa cctgtctgta gtgattttcc
278881 ttgcttcatc atatttcttt actgccattt tagttggatt tccccttat ctttcacctt
278941 cctttctcca aggaagcatt aagctctagt gggaaagtct gcagaagcgg gccaagggaa
279001 aaggggttgt ggttctgact ggaatgttga ttaggcagat ggaatctctg ggaacaaaaa
279061 gaacatatag gacataacaa atctgagtgc tggcaaggtc tggcctttaa gagatcaata
279121 tctgtatcca tgcatctaaa atttctattc tttaattgct taaaaaatat gcctccaaaa
279181 atagtaaagg cagctgctct gtatgtaatg ttagatatgc accctcattc attgttctat
279241 gagagcatta cagatgtttc ctcttctcct ccatggatac cctctgcttt gatttctcc
279301 aaggatgcta tgagactcaa agcacttatt gttttgaata taggataatg tgattgctcc
279361 tttacttctg cagattccca agcaatcatt tagtttcatc cccaatggat ttgataggtg

279421 ggcagcagag ccattcctct ttgaagctat atcctgaggt cttcactttg gggaagggga
279481 gaaagcaaca gctgaagata aaatggctaa tgtgccaagt atttcctcct caaattttta
279541 cgtttcattc tgagatacgt tatattatta tttccgtttt atagatgaga agactgagac
279601 ttaggtaatt tgctcaagat ctctgagaaa gtcaatgaca ggagccccag ttcagcctac
279661 atagtctgct tacagagttt gtgtctttgt tcacagtgct aaattgtccc ccagttagca
279721 tcaacacaca ctccccaaac tcctgcctgc aatgtctata tttggtggcc ttttatgatg
279781 ttccttgcct attgttttcc cttcacctat taataaatta tttctttagc tttattgatc
279841 aaagctgccc aggcatttta actaacatca cgtgtgcaaa gtttttaggt gtggagtgtg
279901 gggtttagca ggaggcctgt gtgggggctg tgctccccat cctgacaagc acacaaggaa
279961 ctaggatgat atccttgaga ttggctgggc taccattgtt tggattctct ttcttttaaa
280021 atatattttt cctatggtat atgaataaac atttaaaatt tttaatacaa tgtttgaatc
280081 ttcttcagat attttatgtg aaagttaggt cactgcttgg gaaggaatag aatttcaacc
280141 tttgggatga ggatgcttaa gaggatgtgg aggacttcca aaccactctg aaatgttttc
280201 ttacctgagg aagtcttcta gacctggcag tagaaaccta aaaatatccc tcatagcctt
280261 cctctaattg atgctagtta ttagaatctg agctcaacat tgcctggaaa gggtgggcat
280321 ggaggcatgt gtctgtagtc ccagctactc aggtggctga gacaggagga ttgcttgagt
280381 ccaggagttc caggtcagcc tgggcaacat agcaagaccc ctgtctctac atacatacat
280441 acatacatac atatatacat acatacatac atacatacat acatacaaag aactgcctta
280501 ggaaaggaaa ggagacaaag agtaggaagg agaaaaatta tgtccaaaaa taaccacagg
280561 aatactatca ttcattcatt ttttatttat gagtgtacag aaattgctgg gcattgtgtg
280621 attatataat tggaagtaaa acacttctca gcaaatgcaa aataactgaa atcataacaa
280681 acagtatctc agacaacaga acaattagat tagaaatcaa aattaagaaa ctcactcaaa
280741 accacacaac tacatggaaa ttgaacaatt tgctcctgaa cgactcctga gtaaataatg
280801 gaatgaaggc agaaatcaag atgttccttg aaactaatga gaataaagag accatgtacc
280861 agaatctctg ggacatactt gaaggaactc ttcaaggaga actacaaacc actgctcagt
280921 gaaatcagag aggacacaaa catatgtgaa aacattccat gctcatggat aggaaaaatc
280981 aatattgtga aaacggccat actgcccaaa ataatttata gattcaatgc tatttccatt
281041 aaaccaccat tgacattctt cacagaatta gaaaaaaaac atttttaaat tcatatggaa
281101 ccaaaaaaga gtccttataa ctgagaaaat ccaaagcaaa tagaacaaaa caggaggtgt
281161 cacactacct gacttcaaac catactacaa ggctatagta atcaaaacag catagtactg
281221 gtgcaaaaac aggcagacag accagtggaa cagaataggg aacacagaaa taagatcaca
281281 gacctacagt catctgatct ttgacaaact tgacaaaaac aagcaatggg gaaaggattc
281341 gctatttaat aaatgatgct aagaaaactg gctagcctat gcagaaaatt gaaatgggac
281401 cccgtctata caccttatac aaaaattaac ttaggatgga ttaaatgctt atatataaaa
281461 gccaaaacta taaaaaccat agaagaaaat ctaggcaata ccatttagga catagggatg
281521 ggcaaagatt tcaagacgaa aacatcaaaa gcaattgcaa caaaaacaaa aattgacaaa
281581 tgggaactaa ttaaactaaa gagcttctgt acagcaaaag aaactctcat cagagtgaac
281641 agacaaccta cagaatggga gaaaattttt gcaatctatc catttgacaa agatctaata
281701 tccagaatct acaaagaact taaacaaatt tacgagaaaa aaacatacaa ccccattaaa
281761 aagtgggtga aggacatgaa cagacacttc tcaaagaaga gattcacgtg gccaacaacc
281821 atatgaaaaa acactcaaca tcactgatga ttagagaaat gcaaatcaaa accacaatga
281881 gataccatct cacaccagtc agaatggtga ttattaaaaa gtcaggaaac aacagatgct
281941 ggtgaggctg cagagagata ggaaggcttt tacacagtta gtgggaatgt aaattaattc
282001 aaacattgtg gaagacagtg tggcaattcc ttaaagacct agaaccagaa ataccatttg
282061 acccagcaat cccattactg ggtacatacc caaaggaaaa taaatcattc tgttacaaag
282121 atacatgccc acgtatattc attgcagcac tattcacaat agcagagaca tggaatcaat
282181 ccaaatgcct atcaatgata gactggataa agaaaatgtg ctatatgtac accatagaat
282241 actacgcagc aataaaaagg aatgagatca tgtcctttgc agagacatgg atggagctga
282301 aagccattat cctcagcaca ctaacacagg aacagaaaac caaacaccat atgttctcac
282361 tcataagtga gagctgaaca gtgagaaaac atggacacag ggaggggaac aacacacact
282421 ggagcctggt cgggggtaag ggagaaatga gaacatcagg aaaaatagct aatgattgca
282481 ggacttaata attaggtgat ggattgatag gtgcagcaaa ccaccatggc atgtgtttac
282541 ctatgtaata aaactgcacg tgtaccccag aacttaaaat aaaaaaagaa gacataaaaa
282601 atttaaaaaa atacacaatt caatggtttc taatatattc acatagttat gcatccagca
282661 tcacgatagc attttgtttt aggggcttac aatataccat tgtgcagaca tttaatttgt
282721 tttatttaat tagtctccca agcatattca aatttttccg tttttaatct ggaagaagca
282781 gtactttcat gattgtatgt atagatttgt gcacatgtgc aaatattgct gtcataggca

282841 tttctaaaag tggattttct tgatcaagct ccaaacactt taaaatgtga tggatcttaa
282901 caaatttctt cccaacgttg tgcccggttt aacttcccca tcagtttatg ctcatgctag
282961 ttcaccacac tcaccaatat gaagaagcat ggattttaca tttgtcattc gtgaggcaac
283021 aactcattgt tttcatttgt atttttatta tgtggggttt cggtattttt caattttaac
283081 ggaccgtttg tatatgttct tctgtgaatt tttaatttgt gacttttgct ggtttctttc
283141 tttattggat tgccaatcta tattcatact gatttgtaaa atcttttaat gtgttagcaa
283201 tactcaccct ctatcatata cgttgcaagc actttttcca catttgtctt tcaagtctgt
283261 aaatagtgct attcaccaca taaaatttta aactgctgtg tggccaatat tttcctgttt
283321 ttggctctcg tatcatgttt atgaaatttc tcagtaagaa gaaaactttt gatttagtaa
283381 acgggaaata attatttttt aagtaataaa gtcagggtga aatatttttt atttacttga
283441 tatgactagt tgttttcttc ttttgccatt aattgcagtc ttcaagccaa tacttcttta
283501 gaaatatgac agatttaata ccaacactag gaccatttga agacagaact gtgtgtttta
283561 atagagataa tatctcaaca ggagtttgtg aggctgacag tctctgatgt tctgaccacc
283621 tacgtcacaa tcctcattgg ggactttcta agggcatgtt ttgtgaggtt ttgcaattat
283681 tgctggtgct gggacttgga gtatggatat gtaagtatga tgttaatttt gctttttttt
283741 ttttttaaat ttatttattt tttattgata attcttgggt gtttctcaca gaggggatt
283801 tggcagggtc ataggacaat agtggaggga aggtcagcag ataaacaagt gaacaaaggt
283861 ctctggtttt cctaggcaga ggaccctgcg gccttccgca gtgtttgtgt ccctgggtac
283921 ttgagattag ggagtggtga tgactcttaa ggagcatgct gccttcaagc atctgtttaa
283981 caaagcacat cttgcaccac ccttaatcca tttaaccctg agtggacaca gcacatgttt
284041 cagagagcac agggttgggg gcaaggtcac agatcaacag gatcccacgg cagaagaagt
284101 tttcttagta cagaacaaaa tgaaaagtct cccatgtcta cttctttcta cacagacacg
284161 gcaaccatcc gatttctcaa tcttttcccc acctttcccg cctttctatt ccacaaagcc
284221 gccattgtca tccgggccca ttctcaatga gctgttgggc acacctccca gacggggtgg
284281 tggccgggca gaggggctcc tcacttccca gtaggggcgg ccgggcagag gcgcccctca
284341 cctcccgggc ggggcggctg gccgggcggg gggctgaccc cccacctccc ctcccggacg
284401 gggcggctgg ccgggcaggg ggctgaccac cccacctccc tcccggatgg ggcggctggc
284461 cgggcagagg ggctcctcac ttcccagtag gggcagccgg gcagaggcgc ccctcacctc
284521 ctggacgggg cggctggccg ggtggggggc tgaccccccc acctccctcc cggacggggc
284581 ggctggccgg gcggggggct gacgccccca cctccctccc ggacggggtg gctgccgggc
284641 ggagacgctc ctcagttccc agactgggtg gctgccgggc ggagaggctc ctcatttctc
284701 agacggggcg gctgccgggc ggaggggctc ctcacttctc agacggggca gttgccgggc
284761 agagggtctc ctcacttctc agatggggcg gccgggcaga gatgctcctc acctcccaga
284821 cggggtcgca gccgggcaga ggtgctcctc acatcccaga tgatgggcgg ccgggcagag
284881 gcgctcctca cttcctagat gtgatggtgg ccgggaagag gtgctcctca cttcctaggt
284941 gggatggcgg ccgggcggag acgctcctca ctttccagac tgggcagcca ggcagagggg
285001 ctcctcacat cccagacgat gggcggccag gcagagacgc tcctcacttc ccagacgggg
285061 tggcagccga gcagaggctg caatctcggc actttgggag gccaaggcag gcggctggga
285121 ggtggaggtt gtagcgagcc gagatcacgc cactgcactc cagcctgggc accattgagc
285181 actgagtgaa cgagactccg tctgcaatcc cggcacctcg ggaggctgag gctggcggat
285241 cacttgcggt ttggggctgg agactggcct ggccaacaca gcgaaacccc gtctccacca
285301 aaaccagtca ggcgtgtcgg cgcgagcctg caatcgcagg cactcggcat gctcagtcag
285361 gagaatcagg cagggaggtt gcagtgagcc gagatggcag cagtacagtc cagcttcggc
285421 tcagcatgag agggagaccg tggaaagaga gggagaggga gaccatgggg agagggagag
285481 ggagtaattt tgcgcttata aggtttttcc tttgtaattc gtcatttaca cacactatat
285541 aagagaaagg catcatagag tttagtggat gggatcaccg ttttaggtgc aataaatgaa
285601 gaccaatgag tacatttata atgcagagaa gttcagactc gagtatcatg gtttacaata
285661 ttctagtaaa attagcacaa gatagcaaaa gcaatggacc tcaacttgtc agctgaaatg
285721 ttgaactcag taaatcttgg ccctgtagcc caccttctcc ctataagttt actagcattt
285781 ctagaagcag ggaaagatgg ctaatatcaa ctgcatattc tatttatgcc tttgaattct
285841 actaaactcc tggatactat catatgccag tcactgtgca gtagatgggg tggataaaag
285901 gatttgataa atatggtact tgcttcacaa agttcacagt ctaataagag ctcgaaagag
285961 acacacacaa gaggctgcag aaggttcaag gaaggcctag tttgctgtca tgcatctggc
286021 aagtggtgga gctgaattct ggcaggggcg gcttcttttc ctgaaacaat ggctcaggtc
286081 tctcagaacc acactggtgc tgactccatc accagctttc cttctatggt catcctcagg
286141 tcccccaaca aagtctgagg gcaacagcac actcatcacc ctgtgattag cacattgcct
286201 ggtttccact gtcctccttt gagtcaaact ggtctcagtc atcctggaaa atatgacctg

286261 gggggttggt agtactcatt tgcttaaaat cttccagtgg cttctcaagt acttagaatg
286321 atttccaaat ccctttctag gccttcagta ccctggatga tctgctcctt ttctttctct
286381 ctcaagtcat ttgctacctc ttttgctctt gctttgtaaa gttcacatgg gcttcttttt
286441 atgctcaaac atgggctgct ttttatgctc aaacacaaga tggtccttgc atttgtaatt
286501 gctgtttctc ccgcaagggg tgctgttgcc ctagagctag gaatggctgg tttcttcttg
286561 tcattaaaga ctcagctatt atgttacctc cttagtgctc ttctctgacc acctattcta
286621 atgtagccaa tcaggcactg tttatatcaa cacactgttc tgttttcttc ataagatgtg
286681 catctatttg acattactct tgtaaaatgt actcatggtc tatctccctc tgttagaaag
286741 aatactctat gaaaatagag gccctgacct ctcttatttg aactgtgtga gtcacgtagc
286801 aggcagcatg tgttggctgg ctgtttgaat gttgaacctt ctcatttttg caccatcaag
286861 gcagcactgt ctattgccag aactctgcct ccttcgaagc atgtgcccat ggagtctcag
286921 tgtttgtaat gtttttgagt tattgagcca gtaatgcttc tctcctcacc agtgagggaa
286981 tatcctttat agctgatctt ctatctcttt tatctcctgc cctctagtct ctggatgagt
287041 ccaacagtta ggactgtttt caagatttca ggctagatct gtctggctgt ctgactttga
287101 gatggtacct tttataaggt cgtctccatc accctcattg aaagtgcctc ctcggactgg
287161 ccaaaagcag ttaggtaatg ctcttcggtg aacgacagtg aggtaatggc tacagataca
287221 ggatgctctt gtgttgcttt cccattcttt gctatgaggt attcagctgg cttggtttca
287281 tttctatttt ccttacctga ttcagctcct ctgaggcaga cataaaaatt gccatttctt
287341 ccttagacca ttgcttggct tgtacttacc ttttatccct tggctgcctt cttcttaagt
287401 ggcagaaaat ctgagtgcaa atatgaagtc aatattacat gacctggatg cataaaagct
287461 gtaattaaat gactgtaatt tattatttat tgactgctga cagtgttcct gggttagtag
287521 aaagtacagg aaaaaagaca agttcctgtt ttaggtcaca gagaaggaat tttctcccac
287581 gttacctttg gaaggctcaa aaaagctctg tcagaatcaa aacaagtttt aaaacattaa
287641 tgatggatga cgaatctttg atgtagatcc cattgtcatt gacttaccaa atggatagct
287701 tacacgggtg agggcctact aggtgcaagc cattatgaac aaactctgtg ctaggtgata
287761 aaggaataca gggatgtaga agatgtagac ttacgtagag gttgcaaatt tggcctgcag
287821 ggtagattca gctaatggat atttttgttt ggtatgccca gtggtggtgt ttgtttttgt
287881 ttcatttttt atgagctaat attttaaaat gagagctctt acataaaaac acatggattt
287941 attgtttttc ttgaaaggct ggcaacatta aatctgtatt cccactaggg agaaattggc
288001 tggagttgag aaaccactgc catctgcagt tgggatattc attctccagt tcctcatact
288061 cttcaccact cttttctctt acaagctccc gatataaata gagaaatcaa ttgtatggcc
288121 cttgaagaga aagtgtgtgt gtgtttgtgt gtgtgtgtga gagagagaga gacaggcgtg
288181 catcagtaga aatgcagaca cagaggagga gacatttgaa ctaagtcttg aagaatgatc
288241 aggatttcaa gagatgaaag aaagaattcc cggtggaggg acaagtgtaa gacagagcac
288301 cagagataga tagtgtgggc aatccaggca tagattaaaa aaaaaaaaaa aaaaaactaa
288361 aatgaggctg ttttagagca gaaaaggcta ataagaatgt ttctctgcta ttctcctaga
288421 cctgctaatc aataatatgg tttcagaaag tacctgtcat gaagggaagg acaacctgag
288481 ataggcagag gacatacttg agaattctgt cagaaataaa agataaaatc atggtccttt
288541 ctactgtatg catgcatgtg catgtataca cacacacaaa gcacatgatt tttttcttct
288601 gaattccaaa aataatgcca gtttattgta gaaaatttgg aaaatataac aaagctaata
288661 tgaaagtaaa aatcataggt aatctctgcc actaatgaga ttaattattt ggattgttga
288721 atcaaatagt gttagataac aagtgctaca tatttatata tagtaacaga tttttataaa
288781 tatacacata tatacttttg tatacgtata tatgtgtgta aacaggtagt agagtagact
288841 ggttacttgc ttttatttgt tttacctaac actgatcatt tcccaatgct attaactgtt
288901 gttttgtaac atacctttta aatggttgta taatattctg tatgcaaatg tagtataatt
288961 aatctaatta gtttttaatt tgctttaagt tgaactgtct aattattctt atgataataa
289021 gtgttgtgat gacatttaat tatatccatg tacataagta tcttcataga taaatcatag
289081 tggatgtaca aattattttc ttatgataaa tttctctgag ttgaattgct cagtcacagt
289141 agagggcttt aaaaaagtct attcactttt attacaacag cctctgtaga gactctgcag
289201 atttcagcat tagattcaga gatctataga atgctaaagt taggtggatt ttgacacaac
289261 cagttgtaat gcatgtattt aacatatgaa aaattgcatc tgtgaattga aatgaggttc
289321 ctaagtttat gtagccagag aataataagg acaatgaagt acacttctgg acgaaaatgt
289381 tatgttaaat gtattgattt ctgtactggc tctatctgag taagctgggt taaaagggat
289441 tgtgaatatt tttcaagaca caaatttatg gtcataatgt tatcacaaca aacctatgaa
289501 gattaactaa ggtggaaaaa ttactggctt ggagcagatt accagaccaa tgtaagttaa
289561 tgtaatgtaa gttaatgtct ccaaaggctt ctctgagcac agttctcagt gtttcagaag
289621 agttcatgag gagtagaata gtttgtccct aggaaggatt ttaactgctc taatttaata

289681 caaagtgtgc cctcttgtaa actttgggtt ttctttggca gttcaatgca gaatatcttt
289741 atcttcactt cagttttcat tgacaccatt cttactattt accagcaaag caagtaaaga
289801 atattcaaga agaaatagaa ttagattcag ggcatgatgg ggtaagggaa tgttcaataa
289861 actcagcatg gtgttcacag ttgtattttt aaggtgatca ttgctcactc gtaagtgaga
289921 gccgaacagc gatgggagac ttctttaaag tctgatgaag gatgctcagg attatccata
289981 aataagaggc atataattga ctgactacct tagtggattc ccaatcatat tatattgtat
290041 tatgtcacat ctcaatgtac tgcttatttt gtatcagaaa ctctacaaaa tactttacat
290101 gtattattcc atgtgatcgt accagcaatg ctgtgaggtg gtattattgt tcctacttta
290161 cagtaaactg aggcttagag gatgttaaat aattgctcaa agacacatta atggttagtg
290221 gtagagttag gcctgaaact cgagtctcat caccttcaaa gccatcaacc aaatttccat
290281 catgttgaat ttacaccgta atgcttgact ctagaaattt taaaaattct gccaaaactt
290341 gaataattca tacactaaac ctatggcttt tggaactcaa agtgctcata ggcttaggca
290401 ggctacctgt ggtagaatgg agttagcaaa tgaactggga tacagaatta gaagatcaac
290461 tgttctctag ctgggaacgt ggagatatta tatatcagtt cgggaaggta gaatgataaa
290521 gaatcaggaa gaaatcatca gcatcccata aggggttgag aacatgggaa cagggacaat
290581 gcagactgta ttttaagtgt tccttgaata tgtggacagg atctgtctgc agttaacagg
290641 atcctattct caactagggt tgaagtacaa agcctcaagt gctcaatcat cctagcagac
290701 gaggctagag tttgtcctag atcagtgctt ctggaatttg agcatacatc agaatcccct
290761 gaggacctgt gaaaacagca tttctgattc agtagatctg aggccaggcc cgagaatgtg
290821 cttttctgac aagttcccag gtgatgctgc tgactctcgt ctgaggacca cactcaaaag
290881 ccactgctct ggagccacag cccagactag cttgcagctc taagcagagt gattccagct
290941 gtgagaaatg ggatttatgt gcagaaggtc aggcaactca ttccacaaca gaattaattc
291001 tctctgagat cctgaattgc atttcatatt atccacaggt tggcaatctg cctcaaataa
291061 tgaaacttgc agagagagaa gagatcttcc caggcttttt ctttgtcaag tgagcaccat
291121 atgtcttccc tctgggtgga tgactgactt agcacatcac aagcaaaggt ccaaaaatta
291181 aacaacatca tttccaatcc ctcaactatg atggacttta gcccaaatcc acatgggctt
291241 ttctgatgct ggcccccaac agtcttacca ggaatgagca gtcattagtg agtttattac
291301 acttagctca ttcaaataat tttatgatct caaagttcaa gagaatctgt tttttttct
291361 ttttccctaa ccgtttttta cctttgagct ttttctattt tagagacaaa caaaatatat
291421 tgcaaattga tttatattta ttaaagtatt gctttaaaaa tttaaaatat aatataacct
291481 aatgtttata aaagcctacc tctaaaacaa tgagcactga cttttatact acatatgtgt
291541 agtttttctg ccttctcttt tgttttagtt ttaaagtgag agcttctgtg tttattgcat
291601 aagttaaatt ccatttctaa ggtgtttaca gagaagcaat tctttcataa ctggttctaa
291661 ttttcaacca ataacgtatt tttagactag aaagggatct tagaaatgaa ctagttcaga
291721 ggttggcaaa cttttgctgt aaagggctag atagtaagta ttttagcttt tgcaagccat
291781 ggtgtctcta ttataactgc taaatgctgt tgttttagta ccagtcatag ataatatata
291841 aatgaatgaa tatagctgta ttccaataaa atttatttgt taaaacaggt ggcagtccag
291901 atttggcaca tgggccatag tatgctgacc tttggtctag ttcatttttg atttatgtgt
291961 caggaaacta agcctaatga gataagtgac ctgcccagct tacctagcta agaaatggta
292021 gagtgaagtc taaaatgcag gtttgaatcc cagtcaaatg tctgttctac aaatccactt
292081 ggtccagtat ggccaccctg tatcctttct gtgcatcagg ccaatttata gatcaagggg
292141 gttgttcctc cagctgtccc tgctgcaaac acagcaggaa gctagggacc ctagggtgtc
292201 ctttatgctt ttctagtcaa ctagttataa aatgtctcat ctctaatgac cctcaggagc
292261 tctatgtgct tgtgagagag tgaagaaaac aggcatttaa aacgttgagt tttctccacg
292321 ttcagtagtt tggtgggcca cctcatgata ccattctaag gaggcgaagg cttaagattt
292381 gatttttaaa cagagacaga gaaaggctgt tccaaggcaa cagatgggta cgggccaacc
292441 gagtaagcat ttttcagatg tgttctgtga tcacacaggg ctccagggga aaagtgctga
292501 agatcatcca gagcagggca ctcccccaga aggaaaaaga ctcagagtta atgttctcca
292561 gataagaagc agttcatttt tctccatgaa ggacacattt tttttccatt gatgatttca
292621 gcacaaaaac tgtcaaccaa acctttttgc ctcttgtgaa cattgccctt ctagaaagtc
292681 ggcaaactaa cgaaacactt tgtagctgtc cttcaacagt atctagagtg atactgcagt
292741 tatccaggtc atctttgttc tgtttaatac acaggactct ttattatttc attatgtatc
292801 atcctatcat tttaccttga gggttaacca ttcctgttat gccatacttg ctcttctttc
292861 cctgaatctg ttacaaggga gattgtccct atgtttcagt tgaataggag agtaatactg
292921 ccttttagga aagtgatgaa gttgaggcct ggattcatga gactcttgag gccaagcatt
292981 tgaaaattga gaatggtttt gttggagttc caaatcagga accaattcta tctttttaca
293041 aatatatttt tgagttcttg ggtttgggag gaacagaggc tctcttggat catctttgtg

293101 tgtgtgtgtg tgtgtgtgtg tgtgtgtgtg tgtgtgtgtg tgtgtgttgg ggagggagct
293161 gttatgggga tatgtgtggt ctgaagcaca cctggagata ataagatctg agcagtgcta
293221 ggaacctgcc aatgtcttgg tgtctttttc gtggtcttct tggccctctc atttcgtatt
293281 tactgcattc ttattccctt attaccagtt ggtcagtctc ctctgattct ctatcccaaa
293341 ttaaataaga gccagtttta ttgcccttgc taattagcat ttactccatt gttagaatct
293401 cctgttagga tgcctaatgc atcaccagga agcctttaga tgggccaccc tatcacagtt
293461 aaccacttcc attctattgg ttttttgtgg ctcctctgta caaaaaacag cctagtgttg
293521 ctgccctgag catgaaggtt aatatccaag acatggagac tggacattga attgagtaac
293581 ttcttaagct ttttttatat ttcttttgct gtatttactt tatgttaaag ttaattcaca
293641 ttttcattga aaaaattttt aatatgcaaa acttgaaaaa gaaaaggaaa actatagttc
293701 atcacccgca tataaagtat gactatcttg ctgattattt tccagtcttt gtgcatagtt
293761 tcatatagtt gattaatgtg tggctttata ttttttgttt aacattagag cacagttttc
293821 ctcatgtaca taaatattct ttcaaaatga ttgtaattca ttgcaatata tcatagtata
293881 aatatactgt atatacataa ctaatgttca acacttagat ggtttcatca ttgcttgtta
293941 aagtttatca tcacatcaga taattttatt aagacagatt ctagaagttt tcttacagaa
294001 tcaaaaagaa taagattttt aaagctttta atatgtactg tcaaattgtt tttctgaaag
294061 gttgctacag tttttactgt caccagcagt gtataaggga gcctgtcttg ctgtactttc
294121 atagcattgg atgacagcat ttaaaaatga gtctcactgt gtttaaaaaa aaaaaggtgc
294181 tttctttgtt ttatttatat ctggttactt atcctagtga ttattctgct attgttgctg
294241 aagggaagta attgaaaccc tagccatgcc tatgcagaac aatttgcctt tcagttttga
294301 ccatgtgaga cgctaatcca atgaacattg tgtctcctct agccttcata caccgaattc
294361 gacatcagtg gcaacgtcct cgctctgatc ttcaaccaag gcatgatctg gtaggccagc
294421 tgttggacag cttatcactt acagaaaagc ctcccaggtt atttttgcat acagcattga
294481 atcctggtgt tagaggagaa tacaatcggt gtctaactcc atatttactt ttgcaaagtg
294541 gatgcctgta tatagtttcc ttttctttca tatcttaaat gtcagtcatc aaaggtgtct
294601 ctctgttttg caaagcactt cttcatcaaa taatgggagt ctgtgttctg gttagcagat
294661 ttatttgagg actatatttt tatattttgt gacgccagga tctaatatta aaacattaat
294721 aattacatta ccacatgata agtatgaaat aaattaacat aaggcctttc cgagagaccg
294781 agtttatttt gttgtcacta ctaaatatat ttaaaatgta acttatagct acttttaaaa
294841 ataatacctt atcaaatcca atagtttatc gagatgaaac gcatgtttta ggaaggtcga
294901 gttaaaatat gatcttcatt ttgtaacttg ttttataggg ccatggcaca tcagaattat
294961 tttttttgat gtgtgaaggg ttgtcatatc ttttatcaaa ttagagcatt ctcactgtaa
295021 acttgacagg ttaagatgag tcgatacagt agaaatcgtt tttgagttta aaaactgaat
295081 cacgacatcc gattcatctg gggaccatgt gtcttggaaa tctgtcactt cagatttcca
295141 gcctacaagc agttacaata aggctacttt atcctgggga catgtgccta agacaatatt
295201 atgctgccaa ccataccatt gtcctattca caagtgccgg tatagactca gtttttgaac
295261 aagtttcata tattgtacca atagatctgt cctgaattat taaacctttg ggtgctgaca
295321 gacaccggac ccctctgcaa atgctaaaac cgtcttgctt ctgtcacaca aagacaactc
295381 tccagggctc catcgtcctg ccatgggatg actgttaaat tcctgtgcct gactaggtga
295441 atcaatatgg aaaagatatt ataaaaatat tgtttcctcc tttgtcaggt aggtttaaaa
295501 atctaatttt tattcccttc ctttctaaat tttccaagta tgactcacat gtccagactc
295561 actaaggaag gaaatgcatc ctacattttc caagcaggga ggaaagtcaa ggtggcccga
295621 ggaacgtcta ctgacacaga agtaaaggga gcagcttctg tgggttctga gctttcattt
295681 cctggagttc aatttgctgt tatggcagaa aatggctaac acggctatgg gagtaatgag
295741 aaaccactgt tctatcagtg gcttttacac aaagtgtcat aaatccttca ggcctcatgg
295801 gtaccattca cgcagacata tgctgcacgt cgattgtgtt ctttcagagt taagacaaca
295861 gattggtcta gaaatttgtt tttattgtca gaaaagctgt tataatggct gtcctgcttg
295921 gtttcactac tgattttgca actatgttag tccactattg ttcatattga aattcttaaa
295981 tatctaacat gtttttccag ttctctcgtg ggcagatcca ttatcaaaat ctctgcactg
296041 tctttttctg tttattaatt cttcagacag tggtcaagct tgcttctggc taatttgaag
296101 agaatattga gttttttgag catacaaaga tattcacaca ctttccaaag ttccagaagc
296161 aaactgacca ttattctatt attcaccttc acgtcaatgc ttggcacaaa tattcttttc
296221 ctaaccctag gaggctgatg agggaggggg atgtcatgta gagggaggtg tggggcaggt
296281 gtaatgtaaa attagtaggc tgactctgca caaaggtcct cacacacaat cgttccatta
296341 ttttatgaag catgaggata taatgcctga gcttaaccat tttctacaga aacaattaaa
296401 aatgtacggg gatgttttat tcaattccat tggcttgtga ctggagatat tgaattactc
296461 ctttaggaat cgctgagtag tggaggttat ttacgcagat gttgttaaca tcaacattta

296521 tagataaaat ccattttgct atgatttggt tgaatgatta gtaaccttta tttacgcact
296581 tattttttga aatacacatg atagtttctg tgctaatatt tttaaatgat gaaagcacag
296641 aatatcttta tcatagcagg atattgagaa atggcagaga acacacagtg agagagaaaa
296701 tgagttttgg acattgctcc aacactgctt cactgggtac tctcccgcca ggtttatcac
296761 ctttttttct tcactttctc agttgatcag atgtgaataa tttacctgac atgtacatgt
296821 cgctgatgta ataaggagag ttaaataaaa gaagctttca ttttctttga tcttccagga
296881 agaaaagaat tttataaggc taaagtgtta caatgatttt tactgacaga tgatgggatc
296941 atgcctgggt aagggactta gtgagccgag ctgattagta atgcatgagt aaagatagga
297001 tgagcattaa cattcggata ttattatgcc aggacttatt tttctagctt atggctcttg
297061 agttatttgt ttaaaatgtc tttgtgatac ttattttgaa attttgaaat ttatttatag
297121 atgtatacac aaataataag catatacata caaacaatgt gcacgtatat aggtacaaat
297181 atatgtggtt ttaggcaagt ctcgattttg aattacgtgc aaaattatat tttagaattg
297241 atcaggttat gcagcaatga aacaacctca aaatcgtagc agctcataaa aggaacatgt
297301 tcaatgtggg gtggaagaag agctttgctc ttagaactta ccttcacttt agagacccag
297361 actgatggaa taggatccat ctgaaacatt gctatcacac tagcagagaa tgaagagaag
297421 gaaaaccaca caggactttt aaagtttcta cccagcagta aggacctaat ttctattcat
297481 actttattgg ccaaaacaag tgatgtcacc acctgatttt taggaagact caaagtgtat
297541 tacatcaaca tgtattggta ttggggagta taatctagtg ctttgcaatt ctaacccatt
297601 agctgggact atcacttgct tacttgctca cttttttggc gttccctctc tcttgctgtc
297661 ctgtaacaaa agtatactgt attgttttaa aatctgtctt ctcattagaa aaaaatagtt
297721 taacttccta caaagtcttt gttcaattca ttatggtaaa atatcattcg tatctattgt
297781 ccgaaatctt gtcaacaaga cagcccaaat tgtgacaata ttccactttt caaataaatg
297841 agttaacttt gggttggcaa aagttcaatt atagtggaaa ctaatttctt catctaaatt
297901 aaaaatgtct ctataatgaa ccaaatttac gttggattgg ctaaacaact attcagggag
297961 tacaaaatgc taaacctagc tataaatgtt gagggcttca ttatagatag gacagtttaa
298021 aagacagggc tccccttaca tttttaaaat aagtgcacat atatcttctt tttgattttg
298081 aaataataaa ttgctattta tattgattta tggtgctaat gaattttctc atccaagaag
298141 agtgaaactt attttgttga aataatcaac taagccatca agtaaaagtg accacaccta
298201 agttgttccc acctaaaagt agtattttaa aatgcttata aaaactcaga tgcacaaagc
298261 agagaattcc caagacaagc taaagtaaga aagtaaacag ataaactaaa tttaaataaa
298321 tgtgtggaat tttcatctca aaaacctata agctagttat aaattttcaa taaaacccta
298381 ttttaggagg cacaaacaca tcattgcaat tggctttaaa acaccatatc acctacaaag
298441 agaaaatgat caacatcaga aagagaaaat ttattttaat ttttttcttt cattaagcaa
298501 aaaacttttt tcatttgtac agtcgtggcc cactgcaacc ttgacctcct gtcattaagc
298561 aaaaactaat tattcatgac agaagtgatt aatgcaaatg tacagttttt ttgagtagaa
298621 gaagtgactt taagggttaa ttatttttaa caagttatat ttataggtgt attagtcaat
298681 gatataagtg ttggggaaag tttattttag aattagaaga aagaagtaaa acaattgtaa
298741 atattgttta ctctgttgta gtttaaattc actaatgata gtaggtatag ttagttacag
298801 catcctttct atgaaataat tatgatatgc aattcctaca ataatgggga aagtggcaaa
298861 atgtgtttag aagttggaaa tagataagtg cagtttcttt atgaaaaggg tttgtctggt
298921 tgaaagtggc agtgtatttt ctgcctttgg aaaagaagca tgatgtcaaa taaacaggtg
298981 cagtgtgact ttgttatgga gtaaagactc aaaactgagc agagttctgt ttctttcta
299041 ggatgggctc cttctttgct cccagcctcc caggcatcaa tatccttcga ctccatacat
299101 ccatgtactt ccagtgctgg gccgttatgt gctgcaatgt tcctgaggcc agggtcttca
299161 aagcttccag atcaaataac ttctacctgg gcatgctact gctcatcctc ttcctgtcca
299221 caatgcctgt cttgtacatg atcgtgtccc tcccaccatc ttttgattgt ggtccattca
299281 ggtctcttgc ttttgaaatt tgactcaggc atcgtgttct ttcgggggtg gaggtgggaa
299341 tggtcattca ttgtataagc tattttttcc ccccaaacaa tgacattttt ggttggtgga
299401 aatgagatcc cggctgggcg cagtggctca tgcctgtaat cccagcactt tgggaggctg
299461 aggcaggcag attacctgag gtcaggagtt cgagacaagc ctggccaaca cggtgaaacc
299521 tcgtctctac taaaaataca aagaaaaaaa aattagtcgc gtgtggtggt gggtgcctgt
299581 aatccctgct acttgagagg ctgaggcagg agaatcactt gagctcagga ggcggaggtt
299641 gcagtgagct gagatggtgc cattgcactc cagcctggag gacaagagca aaactctgtc
299701 tcaaaaaaaa aaaaaaagaa agaaatgaga tcctttgggt attcaggttc aataggtgac
299761 atggagattc ttaggcaagt tctgtctatt tcctcatttg ggaatcataa aagagaaaat
299821 actttatact tttccaggct ctgcctgggt aaaatatgta tggtgaatct gagctgtttc
299881 catgttagag gatagctaag agcaaggtct gatgacagtg gtctggatgt gagtttgcct

299941 gactctgggc cgtcacatcc agacgtggtc agctatcact acttctcttg tgcattacta
300001 ccttaaaaag agagagagag agacagacag agagagagag aataccccac actttaccta
300061 ggaaagagaa ttattactaa atactaatga gcataaggag ggccacccaa tttgttatcc
300121 aaaatatcac atgctagtga aaaaaattaa ggctgttcca catatatatt ctctcctaat
300181 tatcattcaa gtcaggaatc gagagtcaca acggctcctt gatccaattt agagatacac
300241 atttctacag ttctctatga cagagctctc ttctctctat gacacatatc agaaacctaa
300301 gtccctggga tctggaggat gaagtttctt tccctaaagg gccttagaaa tgaatctact
300361 acaagttctc ccaagaatct ctgcatgttg attcactggc cagcatgcac ttgagctgtt
300421 tctccttttc agtgaaggaa atgagactgt aaaaagccat gtgaatgata tgcatgatgc
300481 tgtaatgccc acatataatt cctccattat gttgtcagac ccaagttatt taattaaagt
300541 caaaaggttc attgctgaca gactaagtat ataagagaca ttttcactct taacagtagt
300601 ctgagttatt gctttgctgg aaaacaagaa aatgttaagc cagctgatcg agttaaacta
300661 gctggacctc tgtctactca gagaactgat gttgagaagc tctgttaatt ccgttgtgtg
300721 tgtgtgtgtg tgtgtgtgtg tgtgtgtgtg tgtgtgtgtg tttccaagtg ggctgaaata
300781 aataatgcag acagtgacat ctggagacta aatatgaaat tacagtcttg atgagacatt
300841 cgtctcaata aattccgaac caatctcaga gtacaaagtt caaggaatat gccattcaaa
300901 ggctttagtt aattgcaaat tattcacact acttattatc caggataagt ttaggtgtgc
300961 attaagctat taattggaag ttgatccttc tcagtaaaac atagcaaagg accctagctg
301021 cctgtaaggg aaaccacaca atgtaaacat ggatctccac agatgcattc agatttacaa
301081 tacgtcttag acatgaacaa tagttgcaca atttcagctt cttaagccct aactcttctg
301141 gctttcctaa ttggcaaact taggcaaaat tatggcatgt aaatcactat tttgaaatca
301201 gggaaattta ttttttattt ttaaaattat ttatttgttt tttaatttcc ataggttatt
301261 ggggaacaag tagtgtttgg ttacttgagt cagttcttta gggtgatttg tgagaaatca
301321 gggaatttta aatattttca atggagtact gggaagggtt atctctatgc tatttagatt
301381 ttttttttca tgtcagtttc ctcactttag agccaaagga tgtttgcatt gtttatttgt
301441 ctggacttaa aagtttgaaa ggattgtcct gctctagaaa tgaggtgaga atgcttgtat
301501 gcttgtacta gtgggtgcct catagcttgt catctttatg aaccccaagt tttaggtgat
301561 ttgcttaacc ttcctggtga aggttgtctg actctatcta gatcctgggc aagcaggaat
301621 ttaggaaata taaatttgaa ctaggttact ggaggtatca cagtgatggc tagttgctct
301681 ccagtgtagg gaagttttca ggtaagatgg ctatagtata tttatccatt cagtaaagca
301741 atttgatgag atattctagc ttgtctggtt ttataagacc tggctagaga gggtaaattt
301801 tccaagaaat tattccaata ggctagtaag tggatttatc gtaaatagtt gacaatttaa
301861 agatgtccag atgaaggcaa ttctaattgt tctatagcat ttcctgacac ctgatgtttc
301921 ctcccatcta tgtcaataat tctgaacaat ttatagttct ctaggttttt atatacatcc
301981 ctgcttacat tatgacaatt ttatttttca tatattttca tcttactgca aagataatat
302041 gtgtttatgc tagaaaacct aaacattgta gaaatatttt tacatgaaaa gaaacatggc
302101 ccataatctt gaaaacctga aataacaact ttttagcatt ttatattctg ttttgccttt
302161 catgcatgat ttttcataac tgagcttgta ctgacacagg atgtttctca gcctcttcac
302221 tggactcaca gaggggcacc ctgtctactc ggcctgctgt gcttaacccc ttgtgggagg
302281 aagcacgtgg gcaagtgagt gcgggatctg gcgagacaca ctgggcactg acacaggagc
302341 aagctccatg tggggcccgt ggccagacca ggtgtgtcac cttgaggaga atgcaatggt
302401 gcccaagtgc ctatgaccct aaagccccag agggggtgtt acaatgcttc ctgagttcca
302461 ccatcctcag cctgatggat ggcagtgtct tagcagctca gttggcccct tgcctcatca
302521 tgtggggcag ctgccctcca ctggagaggg caaaggccca gtgtgacagt ctttcctggg
302581 ttcccacaat cggtgggtcc caagctcttg tccagcatcc aagaagaatg aggtcacatg
302641 gattattgaa ggatggtgaa ggaggagaat tttattgagt aatgaaattg gctctcagca
302701 gagagggcag ttggagaggg gccaggaagg gcaggtcatc ttccccaaag tcaggttgtc
302761 tcttctccaa agtcaggccg actcttcccc aaagtcaggc cgtctctccc ctctaccaac
302821 tgagtctgtg gtctttatag gcacaggatg gaagtgtgtg ctgactggtt tgtgagtatg
302881 caaaaaaggc taaagtgaag gtgtcactca aaggtagaca cgacagtata gaaacccaac
302941 taggaaaggg taggtatatg taaaataggt gaagggtggg gatgaatcag aggaaagtgc
303001 gccaaacagg aagacaagtt ctcaatccgg tctgaggatt taacttggac cttggctttc
303061 aggctttaaa ctgtctttgg cttggaggtg gcatttcacc aggtacctgc ccctatctgc
303121 ctaggcattt ggctgcctcc tgttgctatc agtatactag gtgcaggttt tttagtatta
303181 aaagttaaaa ttatattgtg agactgctat tatattggtc atttgcaatc atgctttatg
303241 catctatttt tcttaatagt ttagctgact tctagagatt ggggatagag aagtttcctc
303301 tctgtggatc taggacacag taaagtgctg gtcaataatt tgaatgaatg aatgaataat

303361 gactgcttat tgtgcactaa gcactagaag agacacaggg ttaagtccat gcattaagca
303421 ttgattctgg tcctgtggca cttatcaccc agctgggaag acaggcctcc tcataaactg
303481 ttctaatcta gagtggcgct atgcctacag caggactatg gatagacaca gacaactgga
303541 aaacaattct gagaatattg tatgatagca tactttggct aacctggcag gagaaggcat
303601 tgccagggac atgtagatgc gtccataatt tgtacgtaat taagatatct ggactccttt
303661 ctttggtaaa agacctgacc tgaaaaaggt acacaggaat ccccagggac ttagcaactt
303721 tgctttgggt cacactggat gctgagaagg cctttgtgtg tagggataat ttcataaaat
303781 ttgaatgccc ccactctaac tacgttcatc agctatgacc tgaggtgctg gagaagggag
303841 gatagatggt cttatctttt ttggtacttt taatttatct gcttatcctg tattccatct
303901 ggctggggct gcattggggg gtatgaacaa tttggaatgg tgaaaaataa aacatattgg
303961 aatagggagt tataatctta tattgcttgt aagcttcatt ccctcttatg tatcataagt
304021 cttgaaaatt agagtgtaga tctttaattt gtgaagcaaa acttgtacct ctgtagtcca
304081 atgactactg acaaaatata tggagtcact atgtctgctg gaagatagta ttggttagac
304141 caggagaaaa aaaccataat ctgggacttg cgtgtcattg ttatttagta gttgaaaatg
304201 cttaaagatt aaaatacaaa agcaaaacta aacattgtat taaatgttgc accaacattt
304261 attctgatga tgcatacaat ttggtgaaac ccaacccaaa tctcttctga aaattacatc
304321 ttttcgtagt acaatcagta tgcatataca gagaaaataa tgatgtaatg aatattttga
304381 atattatggt acaattctag ctattcctta gtaagtcaag acatcctgtt ctcagttttc
304441 tagtttatag gagctgatac ttgccttcat ggagcttagc agtggagcta agctggtatt
304501 cattagggcc tgtccaggag aagaaactga gagggaaaaa acccatgatt cggtgtggtt
304561 accttaccta ttaacatatc tgatgaagga agtctggtgg aaaatgaggt ggaagctatt
304621 gcgtggaaag agatattgga ttatatgtgg attttattcc actgggctat tccttgctct
304681 catttccaag gctaattaaa aatacttagc tcaaagagag tgttctccta caagagcagc
304741 tgactgagag gcagacttat gagctattta caaaatatat ttcaatcaaa atcttagaaa
304801 tacagttttt aaaaatcttc tcctaaatat gtggttaatt ttctaactgc tttgactata
304861 tgtttatact gggatcatat ttctcgtatc ttacccatat attgactaca tgaggtcaaa
304921 gtgtcagcaa gttgtagcta aacatcagat tcctaccctg aaagaggacc taagcagtaa
304981 ttgagaaaga tttccagttt tcttgcttta accatggttt ttccatatgt tcttaataaa
305041 cttatctccc cctttttaat tcccccagtg gcaaaaatag aatgtttgaa gtcattggag
305101 agaccctgga gcacgatttc ccaagctgga tggcgaagat cttgagacag ctttcaaacc
305161 ctgggctggt cattgctgtc attttggtga tggtgtatgt gcttttcctc ctcatagaaa
305221 gagcctctgt gatgttcttc ctggctgttt ttacatttca gctttttcag ctctctcact
305281 ctccctaagg gttctgctgt aacctatcca aattcaacac tgattacatg gtggtgagag
305341 gctaaaattc atgagccata ttctgatcct attggatgta attcacaggc atctgcatct
305401 gtctttgctt ccccaggatt ataagatttg ttcccaagta accacaataa ttatccataa
305461 acagctaaag caagggaaac ataaatgcta aatgctaaat ctaatcagta cattgagctt
305521 ccccccaaca acccggcctc cacctctcgt tccataaagc agatggcaat gaatatacag
305581 aaataatgat cctctaagtt aatattcccc ttgggggccc tatagactat attttagggg
305641 caagggcttc aagggtctta aaaatttcac tgtccttgtt gttagagggc cgccaataac
305701 tgtgtgttca cgataataga atcagaggac cagaggatct tgggaagttc ctgtagtctg
305761 tcccttcaaa gtcagaagtg atttccaaag gggaacttgt cagatgactt ttgttttcac
305821 ataactttca ttggcttctg actcttggcc atcacagata aacttagatt ctaagtaaaa
305881 gtacagattt gctgtggaag agccaaaagc agcgtccatc tcttcctttc agtccctgag
305941 tgcctcctac atagaatgtc tggagctgcc acgactctca gtcagtgggt aaatattaaa
306001 cagccccagg gacactttaa gaacaaagtt gatgcaaagg atttggttca tttaaataaa
306061 ggtaaaacaa ggaaatctca gggagaaaac tcccagtggt ttgcagtaaa tgagcaggga
306121 ttgtgaccca gttattgtcg tgatcacaag ccacataaag tagtgagggc aggtttatct
306181 tcttcaattt tataacctaa gaaattgatt tacacaaaga aagtgatttt ccctagacaa
306241 gagagagagt aaaatggtca tagaaggacc tgaagcagat cctttcacag taaatccagt
306301 gctcacttct ttatactgcg aggctttctg tgagggagag ctaataacct gtagggataa
306361 ttaaacagta aatcagatta ccattatata ttacataacc ttgataatgg ggtgctatta
306421 agaatctgat agtaaatctc tggcacagat gcattagaaa atgacctact gaatgcagca
306481 tgtttcacca gatggccttt gaaacaattt tctttctcta catgtccatg atacagtggc
306541 ttctgagagc atgttaatca gtatcctgtg gcctttgaga gtcaaggccc tttagtggta
306601 tgaatgaggt gttgtttaaa acacacacac acacacacac acaatgtaaa tgtcagattt
306661 cgttaccata ttcttacttg attaggagac atgtttattt ttaagtgaag gagtggcatt
306721 gttcaaatgc cttaatccaa agatttagta atcagtccta agagggttta aaaggaattc

306781 acactgcctt tgtttcaggt actatttaca cactgactca tttaattccc ataacagctt
306841 tgagaagctg gcttcattaa tcatactatc aataacacat tgaaattctg ccccaagtca
306901 agtgactaag tggaactgaa actcatgtct tctccttatt tcctacactc acttgcagca
306961 ccaaacaggg atcagaatca gtgggaattt cagatgggct gaaagccact tgtccgaggt
307021 ggtatagacc cagggtcagc acacttttcc taattcgagg gccagaggca aattaggcca
307081 agaggcaaaa ttgaggctct taattaagta attactaata ataaccgttt aaaagacaga
307141 aaaaccattc ttaattcatg ggccatacaa aacagtctgg cagtcatctt catcctgcag
307201 gacctagttt gctttgatct ggacagcctg gaggaaagct ggccccatgc aaatgtcaaa
307261 tagatgtact gaaaggttga ttgtggctct gaggttctga tttaaaaccc agattttagc
307321 ttattactga gtatattttt tcaaattctg agatctcatc ttctattttt ggggcccata
307381 caattggctt agctttttat tacctttaat tagagaattg tactagtatg ttgaatttcc
307441 cccaatggat tatattaaat caacctggat ggctttcggg tatctaagca gagttcaggt
307501 tagttctctg caggcagttt cattcctttg gagactagaa aaagatttgt gaggaatact
307561 tatttaacgt gcattacaag gtagaaaatc atggtggata aatagagaat tccagaactt
307621 gaaaggactt gtgaaatctt tgagttcatc ttccaaatcc aggaactttt ctaagaaaat
307681 ccttataata cacaccacac acaaacacac acacacacaa acatttattg agcacctatt
307741 tcatgccaga tattgccctg catgcattta tgtatgtaat tttagtaaaa ctcccatcac
307801 tcatgtaaga gataaacaaa ctcagccaaa ccgtatcaat aattgtgttg ctagcaccag
307861 atggttcata aaggatactg tctttcattt tggggagtag aggaaatgtg ttcaatatcc
307921 agttatttgc agcaaagagt aatataagtt acctttttta ctgcatttct tctttgtttg
307981 tttgttcgtt ttgccttaag tgaagataag catatttatt tttacagaat tgtctggcta
308041 gaatttgccc tttctagaat gtaaatgtca tttaaaatcc aataatattc ctttatacaa
308101 cgttttcttt ttgagggaga aagaaatgca aacaatacat tctacggaaa aaattccatg
308161 ttcattatct atgcctatga ggattacatt cttagctgca aaggccaaag gacgaaattt
308221 aaggcagaaa gtcgttttcc tttttgttcg gcatgttccc taagctgcag taggttttcc
308281 attctctggg atgccattgc catccattta aaatgctact ttcaattata atttaaactt
308341 atgtgaatac atttccagga aaaagctaat attaggcagt gtgaaatagc agacagaaca
308401 ggctgtggat caggagcctt gggccccaac tttccaactt caccccagct tcatccctat
308461 gtcatcctga atgtatctct ttacctctct ggacctcagc ttttatctat aagacaagag
308521 atttagagta gaagatagct taatgttcat tcccatgctg atatttcata atttacttaa
308581 tgtaaattta agaagtatct tggggaactg aaatatacct tacactgtat tttttttctt
308641 tttaatttag tttggccatc tattatctca atgctactgc caagggccag aaggcagcga
308701 atctggatct caaaaagaag atgaaaatgg tatgatacaa tttatttcat agaaatatta
308761 tctttattaa tgtcaagcag tagaaaacac tgagaaatct acttttttcc ccttattttt
308821 tattgtgtag caagctttgg agaacaaaat gcgaaacaag aaaatggcag ctgcacgagc
308881 aggttggaga tacgtttatg tttgtaatgt ttgtaatttt tctttttctt tttctttctt
308941 tttttttttt ttttgctttt tctccattgg atggtgagtg gcattattgc tgtgagcgag
309001 tcattccaca atccttacct tccacctttt tagcccttct caggcatcac atccagggta
309061 tttggttgtt ttctagtggg aggaaaaaaa gagaaatgaa aatgtgggca tataaacttg
309121 gcagtagtga tttttaaaag tgaccttgaa aatgcgcaga aaatgaagaa atcccccata
309181 tagtcgtcta caaacagcag atacaaatgc agatgctctc ctgcccatgt tgactatttt
309241 catacatgga aggatatggc aagaagatcc caagagggtc ctctgggctt tccaccactg
309301 attgccagaa gccctttcta atccattaaa ctttcttttt ttaaaaacct ctttattttt
309361 taaagtatta gagtaaagcc aacttctagt tgccaaaatt aataatatgc tttgagttcc
309421 ccacggaaag gtgataccta agacctttag tgaaaatctt ttccaacttt gtccttactc
309481 ctaactttaa aactctttac attagtagga ctgtttcccc actgtaagta ataaaacact
309541 caaattggtt taattgagaa agagtaattt attcgaccac acaaatgcag ggaaatggta
309601 tgtatggctt caggccactt tcttttttct atatatatat atatttttta attatacttt
309661 aagttctagg gtacatgtgc acaacgtgca ggttagttac atatgtatac atgtgccatg
309721 ttggtgtgct gcacccatta actcatcatt tacattaggt atatgtccta atgctagccc
309781 tccctgctcc ccccaccgca cgacaggccc cggtatgtga tgttcccctt cctgtgtcca
309841 agtgttctca ttgttcaatt tccacctatg agtaagaaca tgcggtgttt ggtttttgt
309901 ccttgcgata gtttgctgag aatgatggtt tccagcttca tctatgtccc tacaaaggac
309961 atgaactcat cattttttat ggctgcatag tattccatgg tgtatatgtg ccacattttc
310021 ttaatccagt ctatcattgt tggacatttg ggttggttcc aagtctttgc tattgtgaat
310081 agtgccgcaa taaacatacg tgtgcatgtg tctttatagc agcatgattt ataatccttt
310141 gggtatatac ccaaaggatt ttttttcttt tttgagacct ggtctggctc tgtggcccag

310201 gctggagggc agtggtatga tctcggctca ctgcagctcc aacttcccag gctgaagtga
310261 tccttcctcc tcagcctcct gagtagtggg gacagcgcac accaccatgc ctggataatt
310321 tttaattttt tttttgaagt gaggtcttgc taagttgccc aggctgctct caaagtcttg
310381 ggctcaggtg atcttctctt tttggcctcc caaagtgttg ggattaaaag catgaaccac
310441 tgtgcccagc attcagggca ctttttatcc taagtttaga acagagtcag gtctgagccc
310501 acctctctgt gaatctctga agctaaactc ctttcacatt ggctgacctt tatggcagca
310561 ggaatggatg cagcaacttc caatgccatg tccttagact gtaaaacttc tttcctagaa
310621 attcctcaac ctctcctcct ttcagattgg ccccatatgg gtcatgtatc catccttgtg
310681 ccagtcactg tgtctgagta tggtaagtta ctgaagtcag tcaggaccca tccttgaagc
310741 tgggattggt ctgttttcct gacacatgtg aaatgcactg gggaggtcac acctacctaa
310801 gagaaggaga aaaggatgct gggtgtcccc aaacaacaaa tgttcaagac ttggttttgt
310861 tgccttcttt atgcttagcc ttttttcttt tccctctctc tatctagcat gcataaatgt
310921 aaagttgaat gctgggtgct gaattagatg attacagtga tgtacagtta tcagactagt
310981 ttttaatgaa atttttaatt ttttaaatca atactacatg tgtgcataat ttagagttcc
311041 atttagaatt tcataattct ataaggctta tcatgaaaaa cagcatcttc ttctgcctcc
311101 tacaccagtt ttgctctctt ctcctcccct gcactcaaat accctaagac aaacactttc
311161 aatttgttag ttgattttta gatatttatc tccaaattac ctaattttgt tgtaatttct
311221 tgacttttta gttttaagta ttatctctta aaattattaa ctacaatgga tataataatt
311281 ttaatagata atatttaaac gatttttata ctcttgcccc agtctttcta tgcattctac
311341 ctatgtagtt atattattat tttggttagg ccagtataca ttattatgga cacacagtct
311401 tcacagctaa gccaagtagt aaactatgat acttgtgcat actttaaaat tttctcagga
311461 ttcataattc ataatttctc tttttaaaca ttttcttatt tttatatatg tttcagtgat
311521 gcatttaatt tccaaatctc tgctatttat tgagttatac tctcaatata ttcagacata
311581 ttgggttatc tgtccatttc atttccttag agaaatcttt cccagaatct tctgcacttc
311641 tccaatttca actagtgtgt gtctgggata cagctgtcat cctttcattt ttcttcactg
311701 tcattcaaca ggatctctgt cactttatcc tgttttggat actgtgtttt ccattttccc
311761 caacttcctc tcttttggtc tgctcacatt ttgccagtaa gaaagggtat gtgagaggca
311821 aattttctga gaccatgcct atctgaaatt gttcttctct tactctccct tttgactgat
311881 agtttggggg tcaaaaatac tcatctaatt atattagaaa taatatccct tcaaaatagt
311941 attttttcag aggtttccca tgccttttag attccacaat tgctcttgag tagtttaaag
312001 ccattgtgat tcttagtctt tcttgagcaa cttgtatttt tgattatctg tttggtttc
312061 caatctggaa agttctaggg tctccttatc tgcatgttca gaatttcatg atggtgtatc
312121 ttgcatggat ctatttttat tccttgtgta ctcatcaatg gacttttgct ctttggttga
312181 agggaatttt cttgaattgt ttacaaattc tttccatcca ttttctgttt tttttttttt
312241 catgtagaat ttctaattta tttttcatcc ttaaattccc tttgtctctt tgctttacct
312301 atgaacattt ccttatcttt aaattttaac atttctcttg gccttttcac ttttgctatc
312361 ataattttag tttctgcaag ctgttttgtt ctttaattct tcttttttaa acagcatgct
312421 gtttttgttt aatggctgca atggcttgtt ttgtctttat gagaatatta ataactcttt
312481 ttgatgtttc tttgcctttg atagtctgtt ttctctaagc tgttttgttc ttctttctcc
312541 cctgcttggt aattcccctg ctttgtgacc ctcagctcat atttcagggt aggatgcaaa
312601 ggtgggtttc gaagctttaa gcacatggat ggggcttgtg ggcagatcct cattggggga
312661 ctctgcctgg gctgccgctg aacccccatg tcagtatttt taggcttctc ctcttaagct
312721 gatcagattc ctcagaaaaa ttacttccga actcctgccc agagagtaaa gatttgcctg
312781 gcaaatctgt agcacctgag tgggaaaaac aacacgggtg gggctcagct ttcaacctac
312841 aaatgctcac ttagccccct ctttttactg cggcactcct tgtggtcttc taacccagaa
312901 gcatctctgt tttactcttt ttagaaaaaa ttattccagt cttctgccag attaaaggta
312961 gtgggggttg gggagcaatt attcatttgt accaagtgaa gccgatctca gactctaact
313021 gctgctgttt cgttttttaa taacccccac ccctttgccc caccaagctt gactgcttct
313081 taacagctaa aacatcctct ttgtattggt tccttacttc cagaggtacc tgttgctgca
313141 aattactgaa cgtttcagag attttatgtg taaattatgt tgcttactgt ctttttcctc
313201 ccctggctta gcttttcagt ttgttagaat tgataagtca ttatcattaa tctgtctgct
313261 ttccagcttc taaaattttg tcattgatgt ctcttcccct attctccccc tccttgcagg
313321 tttatgcaac cttctccatc ttcctcccca accccctcaa aatttcttga ctgtcatgtt
313381 tgtgaaaggg tgaaagttca attcaatgta tttacctccg cagtttaggc aaacctttgt
313441 atttcctaca ctaggagaac aatggtttta tgtccatctt cactctgttc atcccacagt
313501 gcttacctga ggtattattt tgaaatagag tctgggatgg agtctacctt agtgagaatt
313561 tattagaatt gtttagtctc tgcagaagat gaggtggttg aagcacatac atcacatata

313621 ctagcagtgc ttttgtataa tttaatatta tcaaattagt acttaagaat ggaaaattct
313681 gacttataag atctaaattc taattaaggc ttattgttat ctttattgtt ttaaatgata
313741 atataatgct atagcatgct attcatctga tattttttcc ataatgtgta tggccaccca
313801 gtaaacacct actgaatatc tactgaaaaa tgaggaactc ttttgttgga aaaatataaa
313861 atattcatca cataagcaat aatgtataaa tgttacaaaa aagtagtaca ttcattctgc
313921 cttgtttaga atatgtgaag cctttgcaaa ctaagaaaca tggaaatatt ttgaacatta
313981 aaatggtaaa tattatggga aagtagagca catttggaca atacagattt ctggccacct
314041 cattcagaac cattaagcac actgaaagct acgtttttat ttgcctcctg ttcatcttct
314101 ctctctctct ccttgttttt tttttttttt tttttctgtt ttgtgaacag ctgcagctgc
314161 tggtcgccag taataagtat cctgagagcc cagaaaaggt acactttgcc ttgctgttta
314221 aaagtaatgc aatatgtgaa cgcccagaga acaagcactg tggaactgct attttcctgt
314281 tctacccttg atggattttc aaggtcatgc tggccaatta aggcatcatc agtcctacct
314341 gagcaacaag aatctaaact ttattccaag tcagaaactg tttctgcaga gccactctct
314401 cccctgctcc atttcgtgac tttttttttt tttttaacaa attgagttta gaagtgagtg
314461 taatccagca atacagttta ctggtttagt tggtgggtta attaaaaaaa atttgctcat
314521 atgaactttc attttatatg tttcttttgc c

### Pejvakin (PJVK)

The PJVK gene encodes "pejvakin" (PJVK). PJVK is a member of the gasdermin family, and is required for the proper function of auditory pathway neurons. Mutations in PJVK may lead to non-syndromic sensorineural hearing loss. For example, a v.930_931del AC frameshift mutation in PJVK has been associated with deafness (Zhang et al., Acta Otolaryngol 135(3): 211-216, 2015).

The human PJVK gene is located on chromosome 2q231. It contains 8 exons encompassing ~17 kilobases (kb) (NCBI Accession No. NG_012186.1). The full-length wildtype PJVK protein expressed from the human PJVK gene is 352 amino acids in length.

An exemplary human wildtype PJVK protein is or includes the sequence of SEQ ID NO: 51, SEQ ID NO: 53, SEQ ID NO: 55, SEQ ID NO: 57, SEQ ID NO: 59, or SEQ ID NO: 61. Non-limiting examples of nucleic acid encoding a wildtype PJVK protein are or include SEQ ID NO: 52, SEQ ID NO: 54, SEQ ID NO: 56, SEQ ID NO: 58, SEQ ID NO: 60 and SEQ ID NO: 62.
**Human PJVK Protein Isoform 1 (SEQ ID NO: 51)** (NCBI Accession No. NP_001036167.1)
**Human PJVK Isoform 1 cDNA (SEQ ID NO: 52)** 
atgtttgctgctgctaccaagagctttgtcaagcaagttggagatggagggagattagttcctgttccaag
cctcagtgaagctgacaaatatcaacctctaagtctggtggtaaaaagaagcgatgctttctgtttccta
gatataaatttacttcaacaccttttacactgaaagatattctcctaggagacagagaaatttcagctggt
atttcatcttatcaattactgaattatgaagatgaatcagatgtttcactctatggaaggcgaggtaacca
tattgtaaatgacgttgggattaacgttgctggatcagattccattgcagtgaaagcttcatttggtatag
taaccaaacatgaagtggaagtatcaacattactcaaagaaattactacacgaaaaattaactttgaccac
agcttgatacgtcagtcaaggagcagcagaaaggcagtattgtgtggtcatggagagcatccgaaccac
acgacagtgctcactgtctgtgcatgctggaattcgagggaagcaatgcggtttcactttatggatgaac
agaatcccaagggaagggacaaagctattgttttcccagcacatacaaccatagctttcagtgtttttgaa
ctcttcatatacctggatggtgcctttgacctttgtgtcacttcagtgtcaaaaggaggatttgaaaggga
agaaacggcaacatttgcactgctgtacaggttgagaaatatcctatttgaaagaaatagaagagtgatgg
atgtcatttctcgttcacagctttacttggatgatctttttctgactactatgacaaacctctcagcatg
actgatatttcactcaaagaagggacccatatccgagttaacttacttaatcacaacattcccaaagggcc
ttgcatactctgtggaatggggaacttcaaaaggagacagtttatgggtgctttcagtgttctgttgatg
gtcagaagtatgtgagacttcatgcagttccttgttttgatatttggcacaagaggatgaaataa
**Human PJVK Protein Isoform 2 (SEQ ID NO: 53)** (NCBI Accession No. NP_001340704.1) 
MAFAWDAAPLSFVKQVGDGGRLVPVPSLSEADKYQPLSLVVKKKRCFLFPRYKFTSTPFTLKDILLGDREI

SAGISSYQLLNYEDESDVSLYGRRGNHIVNDVGINVAGSDSIAVKASFGIVTKHEVEVSTLLKEITTRKIN

FDHSLIRQSRSSRKAVLCVVMESIRTTRQCSLSVHAGIRGEAMRFHFMDEQNPKGRDKAIVFPAHTTIAFS

VFELFIYLDGAFDLCVTSVSKGGFEREETATFALLYRLRNILFERNRRVMDVISRSQLYLDDLFSDYYDKP

LSMTDISLKEGTHIRVNLLNHNIPKGPCILCGMGNFKRETVYGCFQCSVDGQKYVRLHAVPCFDIWHKRMK
**Human PJVK Isoform 2 cDNA (SEQ ID NO: 54)** 
atggcgtttgcctgggatgcagcacctttgagctttgtcaagcaagttggagatggaggagattagttcc
tgttccaagcctcagtgaagctgacaaatatcaacctctaagtctggtggtaaaaaagaagcgatgctttc
tgtttcctagatataaatttacttcaacaccttttacactgaaagatattctcctaggagacagagaaatt
tcagctggtatttcatcttatcaattactgaattatgaagatgaatcagatgtttcactctatggaaggcg
aggtaaccatattgtaaatgacgttgggattaacgttgctggatcagattccattgcagtgaaagcttcat
ttggtatagtaaccaaacatgaagtggaagtatcaacattactcaaagaaattactacacgaaaaattaac
tttgaccacagcttgatacgtcagtcaaggagcagcagaaaggcagtattgtgtgtggtcatggagagcat
 
ccgaaccacacgacagtgctcactgtctgtgcatgctggaattcgaggggaagcaatgcggtttcacttta
tggatgaacagaatcccaaggaagggacaaagctattgttttcccagcacatacaaccatagctttcagt
gtttttgaactcttcatatacctggatggtgcctttgacctttgtgtcacttcagtgtcaaaaggaggatt
tgaaagggaagaaacggcaacatttgcactgctgtacaggttgagaaatatcctatttgaaagaaatagaa
gagtgatggatgtcatttctcgttcacagctttacttggatgatctttttctgactactatgacaaacct
ctcagcatgactgatatttcactcaaagaagggacccatatccgagttaacttacttaatcacaacattcc
caaagggccttgcatactctgtggaatggggaacttcaaaaggagacagtttatgggtgctttcagtgtt
ctgttgatggtcagaagtatgtgagacttcatgcagttccttgttttgatatttggcacaagaggatgaaa
taa
**Human PJVK Protein Isoform 3 (SEQ ID NO: 55) (NCBI Accession No.** NP_001340705.1) 
MYVYRLLSITVLMDILLEGLHSQYFTIYELRITPMAKHFSTQSFVKQVGDGGRLVPVPSLSEADKYQPLSL
VVKKKRCFLFPRYKFTSTPFTLKDILLGDREISAGISSYQLLNYEDESDVSLYGRRGNHIVNDVGINVAGS
DSIAVKASFGIVTKHEVEVSTLLKEITTRKINFDHSLIRQSRSSRKAVLCVVMESIRTTRQCSLSVHAGIR
GEAMRFHFMDEQNPKGRDKAIVFPAHTTIAFSVFELFIYLDGAFDRRVMDVISRSQLYLDDLFSDYYDKPL
SMTDISLKEGTHIRVNLLNHNIPKGPCILCGMGNFKRETVYGCFQCSVDGQKYVRLHAVPCFDIWHKRMK
**Human PJVK Isoform 3 cDNA (SEQ ID NO: 56)** 
atgtatgtatatagattactatccatcactgttttaatggatatcttattggaagggctacatagtcagta
tttcacaatatatgaactccgcataacacctatggcaaaacatttcagcactcagagctttgtcaagcaag
ttggagatggagggagattagttcctgttccaagcctcagtgaagctgacaaatatcaacctctaagtctg
gtggtaaaaagaagcgatgctttctgtttcctagatataaatttacttcaacaccttttacactgaaaga
tattctcctaggagacagagaaatttcagctggtatttcatcttatcaattactgaattatgaagatgaat
cagatgtttcactctatggaaggcgaggtaaccatattgtaaatgacgttgggattaacgttgctggatca
gattccattgcagtgaaagcttcatttggtatagtaaccaaacatgaagtggaagtatcaacattactcaa
agaaattactacacgaaaaattaactttgaccacagcttgatacgtcagtcaaggagcagcagaaaggcag
tattgtgtgtggtcatggagagcatccgaaccacacgacagtgctcactgtctgtgcatgctggaattcga
ggggaagcaatgcggtttcactttatggatgaacagaatcccaagggaagggacaaagctattgtttccc
agcacatacaaccatagctttcagtgtttttgaactcttcatatacctggatggtgcctttgatagaagag
tgatggatgtcatttctcgttcacagctttacttggatgatctttttctgactactatgacaaacctctc
agcatgactgatatttcactcaaagaagggacccatatccgagttaacttacttaatcacaacattcccaa
agggccttgcatactctgtggaatggggaacttcaaaaggagacagtttatgggtgctttcagtgttctg
ttgatggtcagaagtatgtgagacttcatgcagttccttgttttgatatttggcacaagaggatgaaataa
**Human PJVK Protein Isoform 4 (SEQ ID NO: 57) (NCBI Accession No.** NP_001340706.1) 
MESIRTTRQCSLSVHAGIRGEAMRFHFMDEQNPKGRDKAIVFPAHTTIAFSVFELFIYLDGAFDLCVTSVS
KGGFEREETATFALLYRLRNILFERNRRVMDVISRSQLYLDDLFSDYYDKPLSMTDISLKEGTHIRVNLLN
HNIPKGPCILCGMGNFKRETVYGCFQCSVDGQKYVRLHAVPCFDIWHKRMK
**Human PJVK Isoform 4 cDNA (SEQ ID NO: 58)** 
atggagagcatccgaaccacacgacagtgctcactgtctgtgcatgctggaattcgaggggaagcaatgcg
gtttcactttatggatgaacagaatcccaagggaaggacaaagctattgttttcccagcacatacaacca
tagctttcagtgtttttgaactcttcatatacctggatggtgcctttgacctttgtgtcacttcagtgtca
aaaggaggatttgaaaggaagaaacggcaacatttgcactgctgtacaggttgagaaatatcctatttga
aagaaatagaagagtgatggatgtcatttctcgttcacagctttacttggatgatctttttctgactact
atgacaaacctctcagcatgactgatatttcactcaaagaaggacccatatccgagttaacttacttaat
cacaacattcccaaagggccttgcatactctgtggaatggggaacttcaaaaggagacagtttatgggtg
ctttcagtgttctgttgatggtcagaagtatgtgagacttcatgcagttccttgttttgatatttggcaca
agaggatgaaataa
**Human PJVK Protein Isoform 5 (SEQ ID NO: 59) (NCBI Accession No.** NP_001340707.1) 
MESIRTTRQCSLSVHAGIRGEAMRFHFMDEQNPKGRDKAIVFPAHTTIAFSVFELFIYLDGAFDLCVTSVS
KGGFEREETATFALLYRLRNILFERNRRVMDVISRSQLYLDDLFSDYYDKPLSMTDISLKEGTHIRVNLLN
HNIPKGPCILCGMGNFKRETVYGCFQCSVDGQKYVRLHAVPCFDIWHKRMK
Human PJVK Isoform 5 cDNA (SEQ **ID** NO: 60) 
atggagagcatccgaaccacacgacagtgctcactgtctgtgcatgctggaattcgaggggaagcaatgcg
gtttcactttatggatgaacagaatcccaagggaaggacaaagctattgttttcccagcacatacaacca
tagctttcagtgtttttgaactcttcatatacctggatggtgcctttgacctttgtgtcacttcagtgtca
aaaggaggatttgaaaggaagaaacggcaacatttgcactgctgtacaggttgagaaatatcctatttga
aagaaatagaagagtgatggatgtcatttctcgttcacagctttacttggatgatctttttctgactact
atgacaaacctctcagcatgactgatatttcactcaaagaaggacccatatccgagttaacttacttaat
cacaacattcccaaagggccttgcatactctgtggaatggggaacttcaaaaggagacagtttatgggtg
ctttcagtgttctgttgatggtcagaagtatgtgagacttcatgcagttccttgttttgatatttggcaca
agaggatgaaataa
**Human PJVK Protein Isoform** 6 **(SEQ** ID **NO: 61)** (NCBI Accession No. NP_001356841.1)
**Human PJVK Isoform 6 cDNA (SEQ ID NO: 62)**

atgtttgctgctgctaccaagagctttgtcaagcaagttggagatggagggagattagttcctgttccaag
cctcagtgaagctgacaaatatcaacctctaagtctggtggtaaaaagaagcgatgctttctgtttccta
gatataaatttacttcaacaccttttacactgaaagatattctcctaggagacagagaaatttcagctggt
atttcatcttatcaattactgaattatgaagatgaatcagatgtttcactctatggaaggcgaggtaacca
tattgtaaatgacgttgggattaacgttgctggatcagattccattgcagtgaaagcttcatttggtatag
taaccaaacatgaagtggaagtatcaacattactcaaagaaattactacacgaaaaattaactttgaccac
agcttgatacgtcagtcaaggagcagcagaaaggcagtattgtgtgtggtcatggagagcatccgaaccac
acgacagtgctcactgtctgtgcatgctggaattcgaggggaagcaatgcggtttcactttatggatgaac
agaatcccaagggaagggacaaagctattgttttcccagcacatacaaccatagctttcagtgtttttgaa
ctcttcatatacctggatggtgcctttgacctttgtgtcacttcagtgtcaaaaggaggatttgaaggga
agaaacggcaacatttgcactgctgtacaggttgagaaatatcctatttgaaagaaatagaagagtgatgg
atgtcatttctcgttcacagctttacttggatgatctttttctgactactatgacaaacctctcagcatg
actgatatttcactcaaagaagggacccatatccgagttaacttacttaatcacaacattcccaaagggcc
ttgcatactctgtggaatggggaacttcaaaaggagacagtttatgggtgctttcagtgttctgttgatg
gtcagaagtatgtgagacttcatgcagttccttgttttgatatttggcacaagaggatgaaataa

In some embodiments of any of the compositions described herein, the PJVK protein comprises a sequence that is at least 75% (e.g., at least 80%, at least 85%, at least 90%, at least 91%, at least 92%, at least 93%, at least 94%, at least 95%, at least 96%, at least 97%, at least 98%, at least 99%) identical to SEQ ID NO: 51.

In some embodiments of any of the compositions described herein, the PJVK protein comprises a sequence that is at least 75% (e.g., at least 80%, at least 85%, at least 90%, at least 91%, at least 92%, at least 93%, at least 94%, at least 95%, at least 96%, at least 97%, at least 98%, at least 99%) identical to SEQ ID NO: 53.

In some embodiments of any of the compositions described herein, the PJVK protein comprises a sequence that is at least 75% (e.g., at least 80%, at least 85%, at least 90%, at least 91%, at least 92%, at least 93%, at least 94%, at least 95%, at least 96%, at least 97%, at least 98%, at least 99%) identical to SEQ ID NO: 55.

In some embodiments of any of the compositions described herein, the PJVK protein comprises a sequence that is at least 75% (e.g., at least 80%, at least 85%, at least 90%, at least 91%, at least 92%, at least 93%, at least 94%, at least 95%, at least 96%, at least 97%, at least 98%, at least 99%) identical to SEQ ID NO: 57.

In some embodiments of any of the compositions described herein, the PJVK protein comprises a sequence that is at least 75% (e.g., at least 80%, at least 85%, at least 90%, at least 91%, at least 92%, at least 93%, at least 94%, at least 95%, at least 96%, at least 97%, at least 98%, at least 99%) identical to SEQ ID NO: 59.

In some embodiments of any of the compositions described herein, the PJVK protein comprises a sequence that is at least 75% (e.g., at least 80%, at least 85%, at least 90%, at least 91%, at least 92%, at least 93%, at least 94%, at least 95%, at least 96%, at least 97%, at least 98%, at least 99%) identical to SEQ ID NO: 61.

A non-limiting example of a human wildtype PJVK genomic DNA sequence is SEQ ID NO: 63. The exons in SEQ ID NO: 63 are: nucleotide positions 5001-5334 (exon 1); nucleotide positions 6953-7185 (exon 2) and nucleotide positions 7897-8092 (exon 3); nucleotide positions 8281-9208 (exon 4); nucleotide positions 9575-9716 (exon 5); nucleotide positions 12075-12192 (exon 6); nucleotide positions 13913-14011 (exon 7); and nucleotide positions 14547-15131 (exon 8). The introns in SEQ ID NO: 63 are: nucleotide positions 1-5000 (intron 1); nucleotide positions 5335-6952 (intron 2); nucleotide positions 7186-7896 (intron 3); nucleotide positions 8093-8280 (intron 4); nucleotide positions 9209-9574 (intron 5); nucleotide positions 9717-12074 (intron 6); nucleotide positions 12193-13912 (intron 7); and nucleotide positions 14012-14546 (intron 8).

**Human PJVK Gene Sequence (SEQ ID NO: 63)** (NCBI Reference Sequence: NG_012186.1)

1 tttcttgccg ggagagaggg attagaggga ttgtccggcg tgggtctagg gtcgttgagg
61 cctaaagtta agcctttgtg gggaggacgg attgggggct gggtcacttt aagaaaaaga
121 ttctaaaatt acgaaggcag aattccaggg agtctcccgg gcctggtcct gaagcttgag
181 accccggata tatacgctcc agggggctgc ggtgcgctct tcgggtcccc gagccctgtg

241 tttaggaaca cgcggggacg tccaaacacc cgctcctctc ccgccgggcg ggctcctttg
301 tcttctgggc tttcgtcgcg agatggaacg ctgggtcagt gcatcccagc aaaacacgtt
361 aggagtaaac gaaggccttt ccggggacct gggtagtcgg ttgtaatggc gtttgcctgg
421 gatgcagcac ctttggtggg ccattagatg acgtgtcgct tctccagggg cttgctaggc
481 actagcagaa atgctcccct gtgcttactc ggtgttatgg aagattgctt gcacaatgct
541 attcgtacga aaggatatta ttaaaagtga tttatgggcg ggcgcggtgg ctcacgcctg
601 taatcccagc actttaggag gccgaggctg gcggatcgcc tgaagtaagc agttcgagac
661 cagcctggcc aacatggtga aaccctgtct ctactaaaaa tacaaaaatt agccgggcgt
721 ggtggcatgc gcctgtaatc ccagctactc gggaggctaa agcaggagaa tcgcttgaac
781 ccgggaggca aatgttgcag tgagccgaga tcgcgccact gcactccagc ctgggcgaca
841 gagtcagact ccctctcaaa aaaaaaaaaa aaaaaaaaag cgatttgttg ttttgttagc
901 atgtaagccc tggatgcttg aaacaagtga aagatgttca gaatctaatc aaattatatc
961 atctgatgct ttaattagag cctattctct cttcctctcc gtctatatat gtatgtatat
1021 agattactat ccatcactgt tttaatggat atcttattgg aagggctaca tagtcagtat
1081 ttcacaatat atgaactccg cataacacct atggcaaaac atttcagcac tcaggtaaaa
1141 ttcatttttt gccacaaaat tacttaaaaa caggccagaa ctttatatag ctcttgttaa
1201 aggttaagta gctgggataa taatgaaaca aaaagatgca gcaggactcc atttcaaaga
1261 gagagagtct gtgtgcccaa cttcagagta agaaaccact aggtaatagt ttatatagtt
1321 acttatatgt aatgtggctt tattttttaa ataaagattt gcatataatt gtttatatct
1381 gtgaatttat atttaatatt aaacacagtt ttgtaagcat ataagatgga aaatgaatga
1441 aatgctcaat caatgagctg tatgccatga ggattcaaaa cagcactttg gtgttgccaa
1501 aattccggtg gtggacttgt gaaggaattc cctttggact aggtccactg tagattgtat
1561 ccgggactat cgttagtgcc taaattgcat tgcttcccac agcactatat gcttccctta
1621 aaattggccc attgccagtg gcaaatcact atctactgtg atccttatca tagacattgg
1681 tcatattttt acgttattag ttttctgtgt atattttagt gctatagcct gccattccaa
1741 gttaaacatg ctcttagcat atcattttac accgttgatc tataattcat tacctgagca
1801 catgagcaga ggcagggaat tatacagtgg taaactactt ggtgaaacca tgctttgtat
1861 ttttaagatg tataattata tttaaaaaca agcaatgctt aattttgtgc ctgattttcc
1921 tctttaaaaa tggatttatc tgggggttgc agttgatgac gttttgattt taatatgttt
1981 gctgctgcta ccaagagctt tgtcaagcaa gttggagatg gagggagatt agttcctgtt
2041 ccaagcctca gtgaagctga caaatatcaa cctctaagtc tggtggtaaa aaagaagcga
2101 tgctttctgt ttcctagata taaatttact tcaacacctt ttacactgaa agatattctc
2161 ctaggagaca gagaaatttc agctggtaag tttaaatgtt tgggagtgcc aactcattca
2221 tctgtattat tggcaaccta aacataggtc atatatgcta cttatgttag taatacacat
2281 tttcaatgat taaagcaact tttattatga tgttagtgta ataacattat taagattaac
2341 aatgccaaaa gtttgtataa tatcaaatct cttattggtc aaataatttt caaatctatc
2401 aattatttta taaattgttt taaccaaatc aacttagaat atgacatatt tcttcatcta
2461 taaaatggga tattatttgg gcttacctat gttacatggt caaatgagat gaaaataatt

2521 tgtatttatt ttttacaatt caattattac aattggtaaa ctgaaatgta ttgaagggta
2581 ggttatataa taagctgata ttttgatgag ttactactta taaacgctta tttttctttt
2641 taattgcccg gtggatcttg atgcttaaga agttctgttg gctgaataga aaatttgagt
2701 tccttttaag aattctggct atgtgtttct ttttgttgta agattaattg aattactact
2761 ttaagtcttg gtgagtcatg ttgcctttct ctaacatttg ggtatttgag tcttcaattg
2821 ttaatttaac atgttactat atataatcta cataagataa agatgtttaa aaaatactga
2881 gtttcttctt ataaaggtat ttcatcttat caattactga attatgaaga tgaatcagat
2941 gtttcactct atggaaggcg aggtaaccat attgtaaatg acgttgggat taacgttgct
3001 ggatcagatt ccattgcagt gaaagcttca tttggtatag taaccaaaca tgaagtggaa
3061 gtatcaacat tactcaaaga aattactaca cggtcagtat aataatccta atatatttca
3121 gtgttttcat taaatacttt aggtatataa acttcattac aaagaatgct taaaacattg
3181 aggttgtatt ttactagtgt aatttgctag atatgtttga aatacattgg tagtatatcc
3241 aaactttgaa taaggataga tatcaaaaat cacttggctg aagtcagaga cctcagggcc
3301 ccagatcaag gagctaactg atgaagaggc agagaggctg cagctagaga ttgaccagaa
3361 ataggatgca gagaatcatg aggcccagat caagaatggc agccttgact ccccagggaa
3421 gcagaagact gaggaagatg aggaggagga agatgagaag gacaaaggaa aactgaagcc
3481 caacctaggc aacagggcag acctgcctga ttaccgctgg accaagaccc tgtcggagct
3541 ggacctggcg gtccctttct gtgtggactt ccagctgaaa gggaaggacg tggtggtggc
3601 atccaacggc ggcacctcca ggtggggctc aaggggcagc cagcgatcat cgatggggag
3661 ctctacaacg aagtgaaggc ggaggagagc tcgtggctca ttgaggacgg caaggtggtg
3721 actgtgcatc tggagaagat caagaagatg gagtggtgga gccgcttggt gtccagtgac
3781 cctgagatca ataccaagaa gattaaccct gagaattcca agctgtcaga cctggacagt
3841 gagactcaca tcatggtgga aaagacaatc tatgaccagt aacagaagtc catggggctg
3901 ccaacctcag acgaacagaa gaaacaggag attctgaaga agttcatgga tcagcatccg
3961 gagatggatt tttccaaggc taaattcaac tagcccctgt ttttccctcc ctgaactctt
4021 ggggctgagc tgcaaccacc caactttctt tcccactctt ctctgggact tgtgggcctc
4081 agggcttggg gcaggcatgg gactggccca ggcacacagg tcccagggca tcaggagaaa
4141 ggctggggct tggggtcttg tcctccccag ttggcctact gttacacatt aaaatgattt
4201 gcccagctaa aaaaaaaaaa aaaccacttg gcttaaacaa aacttttaat tgttccacaa
4261 aattactttg gctctacaca catttgctta attattagat ttaaataata cagcaatctg
4321 tctgcaacaa atagagtccc cttcaacaga ctttcaacag acagagtgaa acattctgca
4381 taagcatgtt ttaagaaaat agagaggaga gacaaattat tacatttctt ttgggtgtat
4441 tttctgacta ttaggattgc cttgatttac tattaggtga actatgaatg aataacacat
4501 gatagcaaaa aatgtatttg attatgtgta attagatgtt atagagtctt gtgaatgtat
4561 cttctttatt ttagaaaaat taactttgac cacagcttga tacgtcagtc aaggagcagc
4621 agaaaggcag tattgtgtgt ggtcatggag agcatccgaa ccacacgaca gtgctcactg
4681 tctgtgcatg ctggaattcg aggggaagca atgcgggtaa accacacttg ttgggttctc
4741 ttactaacta aagtgttgcc atgggaattt ttttaagtat tgaatttcta gtcaggcttg

4801 tgtattttgt gaaatgttct gatgaaagag ctgcagttgt ataggatttt gcacagcaat
4861 gtctgttcta gaatatgaaa aaaaaaaaca aatcttggga ttctaaacat gtgtaatata
4921 atgcaaaaac agtaatcctt gtgaaattac gtgcatactg gagtccctca agttactatt
4981 taagtgttgt tttaagaata aaaattgagt gagttgaatt gattttattt ttaagtgata
5041 cagtttttga ctgggcacag tggctcacgc ctgtaatcct agcattttgg gaggccgagg
5101 tgggcggatc acttgcagtc aggagttgga gatcaagttg ggcaacacag agaaacccca
5161 tctctactga aaatacaaaa attagccagg tgtggtggta cacccctgta atcccaagcc
5221 actcaggaag ctgaggcaca agaatcactt gaacctggga ggtggaggtt gcagtgagcc
5281 gagattgtgc cattgcactc taacctgggt ggcagagtga gactccgtct caaaaaaaca
5341 aaacaaaaca aaaaccccaa ccaaaaaaaa aatgtgtaac ataatgcaaa aacagtaatc
5401 cttgtgaaat tacatgcata ctggagtccc tcatattact atttaagtgc tgttttaaga
5461 ataaaaattg agtgcttaag ttgaattgat ttaagtgata gagtttttac actttgaatt
5521 ttttaaagct gaatttatag attttttttt tgagacggag tctcgctctg tccccgaggc
5581 tggagtgcag tggcgcgatc ttggctcact gcaaactccg cctcccgggt tcatgccatt
5641 cttctgcctc agcctcccga gtagctggga ctacaggcac ctgccaccac gcccggcagt
5701 gttatccagg atggtttcga tctcctgacc tcgtgatccg ctagcctcgg cctcccaaaa
5761 tgctgggatt acaggtgtga gccaccgcgc ctggcctgaa tttatatttt tatattcaaa
5821 tagtttttca aagttaatat tatcaatggc catacatgct ttattcaagg ttagttaaat
5881 cctttaaaaa atattagcgg caaggtgcag tggatcatgc ctgtaatccc agtgcttagg
5941 aagactgaga taggaagaca gcttgggacc aggagttcaa gaccaacttg agcaaaataa
6001 tgagaccttg tctctacaaa aaataaaaga attagctggt tgtggtggca catgcctata
6061 gtcccagcta ctcggaaggc tgaggcaggg ggctgcctgc ggggtttggg tcattgtctt
6121 ttagaatttg ggaggctttg aaaagccgtg cttttgtgct ggggaagatg tagcagtctc
6181 ttctccaaac cacccatttg ccttcggact tctctggggc cagcagccat ccaactaggg
6241 gcccggggcc acgggctcag ctgacgacca tgggcttcgc gtccaaccag cagtttgcag
6301 gttcgagctc aggacttggc ggggacacct taggatgcag gagtggccag gagttcaagg
6361 ttacagtagg ctatgattgt gccactgctc tccagcctgg gtgacagaga gagaccctgt
6421 ctctgaaaaa aaaattggaa aagtatctgt gtttaatttt cagctcaatt atgatattgt
6481 ttatctttgt cattcagtga cacctaataa tgacttgatt ggattaaaac caattgttag
6541 aaagataggg ttcaaggata atcttttcaa gtctttgcaa ataaaagtta ttttcgcata
6601 ttcagctgcc tgtcaaattc tgattaacat tggatcattt ttgaggcaca aactgaaaca
6661 tttacttgat tgttagcgat tgctttagaa agctactaaa agctctggag ggctttggaa
6721 tggtagacag gctaggctga aaactttaac tctggcactg ctttctaaga tttcctgtta
6781 aaaaaaaaaa aaaggcttct gctccagagc attatggttg tggtagcact aatcatgggc
6841 tgtaatacca aagtaagtga aaagtgcaga ggtatttcct aatatagacc aggagtttct
6901 gttggaccaa ttggatctct gctataaaaa atggtttaat tcaagcagaa taatccttta
6961 cttgggaatt ttttgttttg tttttggtag gattatagga aatgtttcat ttctccaaaa
7021 agctatcctt acatgttatg atccttaatt atgttattta tttattcaat atagtttcac

7081 tttatggatg aacagaatcc caaggaaggg gacaaagcta ttgttttccc agcacataca
7141 accatagctt tcagtgtttt tgaactcttc atatacctgg atggtgcctt tggtgagtta
7201 agggtctgca tgaaatacaa atgattatat ttttaaggag cattcattag ggcatgtgct
7261 ctctctctct cttttctctc atttctcgtg tctaacaatt atagaagatg ctctcagcac
7321 tgtcaatctt atgctttggc aaaatagctg agaaagaccc tccgttcgct gcttccccat
7381 tccacttttc tgcttcttgg tgaaggttgt aaactctcca ctaccccatt taaaattata
7441 aataagagct aaaatgccat ctactgacaa agtattcctt agcttacatg gggagataat
7501 aaatgacctt cttataggta gtttttgatg gtggcaaaca gaggagtagg tagatagtag
7561 gatgcctgaa gtgttaggat gcccaaacca gggcctctaa agacagcttt tcttacttca
7621 gttttactta tcatagaccc agcttcccct tgcactcttc ctaaaaaata actttaaaaa
7681 gtgaaaatac ttcctccaaa attgtcacct tgtataatac ctaagtgcag ctcactttag
7741 gtctctacat ttcacaggtc tttaaggtct gttggccaac tggaacaagt ccaggtgtag
7801 cccaagggat caggaaaggc agcccctagc catggtaatt tacaaatgtt tttagacctg
7861 ttcttgttca tttctgcaat tttgtaggag gagggaatag ggagtccaga aacaaaacaa
7921 aactacatgt taagtaaatt gtctttttaa aaaatatttt taatttttgt ggctatatag
7981 tagattatat atatatttat gtatgggata catgaggtat tttgatacgg gcatacagtg
8041 cataataatc acatcatgga aaatggggta tccatcccct caagcattta tcctttgtgt
8101 tacaaacaat ccaattatac tcttttagtt atttaaaaat gtacaaattc tcagtctaag
8161 atggagaaaa aactgtacaa ttacattatt ttgactgtag tcaccctatt atgcttccaa
8221 atactaggtc ttattaagta aattctaatt ggataacttt aggtttttac agttttattg
8281 atggttggga ggaatgtaaa tggactgata tatccacaag aatttaaaat atatatataa
8341 tttggacttc cacttctagg aatttatcct acagatatat ttacacatat atgaaatgat
8401 atgagtgcaa gtatattcag tcctcaataa caagagattg gaaacaacat taaggagact
8461 aaataatggt gcatccttgc aagagaatac tatgaagcca ataagaatat aatactttta
8521 atagatattt ttcaagagct gagtataata tgtaactatt tgggcataaa taattttata
8581 cacatacatg tatatatgca tctttcttta tgcttataaa atatatggag tatttctgga
8641 agcaaacata agaaacttaa accacgggtg cccctaagcc cctgaggtag aagactgtgg
8701 ctggatacag ggtgggccag agacctattt gtcactgtat ctcctttctg tatgaatttt
8761 gtaccatgtg aatgttacct tttccaaaag tatgtaaaag tcaactttta aaaacaatac
8821 aatgaatgat ttgtactaga attcatcacc ccatcaaaca ataaaatatg aattattcaa
8881 gttataacat cttctaacaa ttttttttgt agacctttgt gtcacttcag tgtcaaaagg
8941 aggatttgaa agggaagaaa cggcaacatt tgcactgctg tacaggttga gaaatatcct
9001 atttgaaaga agtatgttta ttgaagagta ctgtgaatgt cttttttttt tctttcctgg
9061 cttaacacat gaggttttct attcagtaat ctcatctgtt aaggaaaaat ttaaatttaa
9121 aagcctggct ggtatggtgg ctcatgccta taatcccagc actttgggag gccagggcag
9181 gcagattgct agcactcaga agtttgaaac acttgggcaa tgtgagaaaa ccccatctgt
9241 acaaagaata caaaagaaaa aaaattagtt ggttgcagtg gtgtgcacct gtagtcccag
9301 ctacttgaga ggctgaggta ggaggatcac ttgagcgtgg gaggcggagg ttgtagtaag

9361 tggagattgt gccactgccc tccagcttgg gtgacagagc cagaccctgt ctcaaaaaaa
9421 aaaaaaaaaa aaaaaaagcc aaattatttc atgactagtg gattcacata tttattaatg
9481 ctgtttgcat tatgtatttt tcattttcac ttctaacaca tttcttttct gtttttgtcc
9541 ttttagatag aagagtgatg gatgtcattt ctcgttcaca gctttacttg gatgatcttt
9601 tttctgacta ctatgacaaa cctctcagca tgactgatat ttcactcaaa gaaggaccc
9661 atatccgagt taacttactt aatcacaaca ttcccaaagg gccttgcata ctctgtggaa
9721 tggggaactt caaaagggag acagtttatg ggtgctttca gtgttctgtt gatggtcaga
9781 agtatgtgag acttcatgca gttccttgtt ttgatatttg gcacaagagg atgaaataaa
9841 atgaaaaatg aatacaccgt gttggtgttt taggtgcagt tgtgccacaa accttcccta
9901 aattatctag gtttgctttg atgaattaaa ttaaaatgag aaaagcaaaa agaaattaac
9961 ctgtctgggt atcatattcc ctatctataa agtagcaatt ataacagtag tgtctatttc
10021 ttaagttgtc atgaaaatta ataacattgt ttatatcaaa aaagatttac atataaaatt
10081 cagagattat gaatcatgcc agtcacttta gaatcatttt tgggatgtag t

### Heat Shock Protein Family A (Hsp70) member 1A (HSPA1A)

The HSPA1A gene encodes "heat shock protein family A member 1A" (HSPA1A). The human HSPA1A gene is located on chromosome 6p21.33. It contains 1 exon, encompassing ~ 2400 bp (NCBI Accession No. NC_000006.12). HSPA1A encodes a 70kDa heat shock protein, which stabilizes proteins against protein aggregation and is involved in protein folding.

Exemplary wildtype HSPA1A protein sequences are or include SEQ ID NO: 64, SEQ ID NO: 66, SEQ ID NO: 68, SEQ ID NO: 69, SEQ ID NO: 70, and SEQ ID NO: 93. Exemplary DNA sequences that encode a NDP protein are shown below.
**Human Mature HSPA1A Protein (SEQ ID NO: 64)** (NCBI Accession No. NP_005336.3) 
MAKAAAIGIDLGTTYSCVGVFQHGKVEIIANDQGNRTTPSYVAFTDTERLIGDAAKNQVALNPQNTVFDAK
RLIGRKFGDPVVQSDMKHWPFQVINDGDKPKVQVSYKGETKAFYPEEISSMVLTKMKEIAEAYLGYPVTNA
VITVPAYFNDSQRQATKDAGVIAGLNVLRIINEPTAAAIAYGLDRTGKGERNVLIFDLGGGTFDVSILTID
DGIFEVKATAGDTHLGGEDFDNRLVNHFVEEFKRKHKKDISQNKRAVRRLRTACERAKRTLSSSTQASLEI
DSLFEGIDFYTSITRARFEELCSDLFRSTLEPVEKALRDAKLDKAQIHDLVLVGGSTRIPKVQKLLQDFFN
GRDLNKSINPDEAVAYGAAVQAAILMGDKSENVQDLLLLDVAPLSLGLETAGGVMTALIKRNSTIPTKQTQ
IFTTYSDNQPGVLIQVYEGERAMTKDNNLLGRFELSGIPPAPRGVPQIEVTFDIDANGILNVTATDKSTGK
ANKITITNDKGRLSKEEIERMVQEAEKYKAEDEVQRERVSAKNALESYAFNMKSAVEDEGLKGKISEADKK
 
KVLDKCQEVISWLDANTLAEKDEFEHKRKELEQVCNPIISGLYQGAGGPGPGGFGAQGPKGGSGSGPTIEE

VD
**Human Mature HSPA1A cDNA (SEQ ID NO: 65)** 
atggccaaagccgcggcgatcggcatcgacctgggcaccacctactcctgcgtgggggtgttccaacacgg
caaggtggagatcatcgccaacgaccagggcaaccgcaccaccccagctacgtggccttcacggacaccg
agcggctcatcggggatgcggccaagaaccaggtggcgctgaacccgcagaacaccgtgtttgacgcgaag
cggctgatcggccgcaagttcggcgacccggtggtgcagtcggacatgaagcactggcctttccaggtgat
caacgacggagacaagcccaaggtgcaggtgagctacaagggggagaccaaggcattctaccccgaggaga
tctcgtccatggtgctgaccaagatgaaggagatcgccgaggcgtacctgggctacccggtgaccaacgcg
gtgatcaccgtgccggcctacttcaacgactcgcagcgccaggccaccaaggatgcgggtgtgatcgcggg
gctcaacgtgctgcggatcatcaacgagcccacggccgccgccatcgcctacggcctggacagaacgggca
aggggggagcgcaacgtgctcatctttgacctgggcggggcaccttcgacgtgtccatcctgacgatcgac
gacggcatcttcgaggtgaaggccacggccggggacacccacctgggtggggaggactttgacaacaggct
ggtgaaccacttcgtggaggagttcaagagaaaacacaagaaggacatcagccagaacaagcgagccgtga
ggcggctgcgcaccgcctgcgagagggccaagaggaccctgtcgtccagcacccaggccagcctggagatc
gactccctgtttgagggcatcgacttctacacgtccatcaccagggcgaggttcgaggagctgtgctccga
cctgttccgaagcaccctggagcccgtggagaaggctctgcgcgacgccaagctggacaaggcccagattc
acgacctggtcctggtcggggctccacccgcatccccaaggtgcagaagctgctgcaggacttcttcaac
gggcgcgacctgaacaagagcatcaaccccgacgaggctgtggcctacggggcggcggtgcaggcggccat
cctgatgggggacaagtccgagaacgtgcaggacctgctgctggacgtggctcccctgtcgctggggc
tggagacggccggaggcgtgatgactgccctgatcaagcgcaactccaccatccccaccaagcagacgcag
atcttcaccacctactccgacaaccaacccggggtgctgatccaggtgtacgagggcgagaggccatgac
gaaagacaacaatctgttggggcgcttcgagctgagcggcatccctccggcccccaggggcgtgccccaga
tcgaggtgaccttcgacatcgatgccaacggcatcctgaacgtcacggccacggacaagagcaccggcaag
gccaacaagatcaccatcaccaacgacaagggccgcctgagcaaggaggagatcgagcgcatggtgcagga
ggcggagaagtacaaagcggaggacgaggtgcagcgcgagagggtgtcagccaagaacgccctggagtcct
acgccttcaacatgaagagcgccgtggaggatgaggggctcaagggcaagatcagcgaggcggacaagaag
aaggttctggacaagtgtcaagaggtcatctcgtggctggacgccaacaccttggccgagaaggacgagtt
tgagcacaagaggaaggagctggagcaggtgtgtaaccccatcatcagcggactgtaccagggtgccggtg
gtcccgggcctgggggcttcggggctcagggtcccaagggaggtctgggtcaggccccaccattgaggag
gtggattag
**Human Mature HSPA1A cDNA (SEQ ID NO: 94)**

atgtaccggatgcagctgctgagctgtatcgccctgtctctggccctggtcaccaattctgccaaagccgc
ggcgatcggcatcgacctgggcaccacctactcctgcgtgggggtgttccaacacggcaaggtggagatca
tcgccaacgaccagggcaaccgcaccacccccagctacgtggccttcacggacaccgagcggctcatcggg

gatgcggccaagaaccaggtggcgctgaacccgcagaacaccgtgtttgacgcgaagcggctgatcggccg
caagttcggcgacccggtggtgcagtcggacatgaagcactggcctttccaggtgatcaacgacggagaca
agcccaaggtgcaggtgagctacaagggggagaccaaggcattctaccccgaggagatctcgtccatggtg
ctgaccaagatgaaggagatcgccgaggcgtacctgggctacccggtgaccaacgcggtgatcaccgtgcc
ggcctacttcaacgactcgcagcgccaggccaccaaggatgcgggtgtgatcgcggggctcaacgtgctgc
ggatcatcaacgagcccacggccgccgccatcgcctacggcctggacagaacgggcaagggggagcgcaac
gtgctcatctttgacctgggcgggggcaccttcgacgtgtccatcctgacgatcgacgacggcatcttcga
ggtgaaggccacggccggggacacccacctgggtggggaggactttgacaacaggctggtgaaccacttcg
tggaggagttcaagagaaaacacaagaaggacatcagccagaacaagcgagccgtgaggcggctgcgcacc
gcctgcgagagggccaagaggaccctgtcgtccagcacccaggccagcctggagatcgactccctgtttga
gggcatcgacttctacacgtccatcaccagggcgaggttcgaggagctgtgctccgacctgttccgaagca
ccctggagcccgtggagaaggctctgcgcgacgccaagctggacaaggcccagattcacgacctggtcctg
gtcgggggctccacccgcatccccaaggtgcagaagctgctgcaggacttcttcaacggcgcgacctgaa
caagagcatcaaccccgacgaggctgtggcctacggggcggcggtgcaggcggccatcctgatgggggaca
agtccgagaacgtgcaggacctgctgctgctggacgtggctcccctgtcgctggggctggagacggccgga
ggcgtgatgactgccctgatcaagcgcaactccaccatccccaccaagcagacgcagatcttcaccaccta
ctccgacaaccaacccggggtgctgatccaggtgtacgaggcgagaggccatgacgaaagacaacaatc
tgttggggcgcttcgagctgagcggcatccctccggcccccagggcgtgccccagatcgaggtgaccttc
gacatcgatgccaacggcatcctgaacgtcacggccacggacaagagcaccggcaaggccaacaagatcac
catcaccaacgacaagggccgcctgagcaaggaggagatcgagcgcatggtgcaggaggcggagaagtaca
aagcggaggacgaggtgcagcgcgagagggtgtcagccaagaacgccctggagtcctacgccttcaacatg
aagagcgccgtggaggatgaggggctcaagggcaagatcagcgaggcggacaagaagaaggttctggacaa
gtgtcaagaggtcatctcgtggctggacgccaacaccttggccgagaaggacgagtttgagcacaagagga
aggagctggagcaggtgtgtaaccccatcatcagcggactgtaccagggtgccggtggtcccggcctggg
ggcttcggggctcagggtcccaaggagggtctgggtcaggccccaccattgaggaggtggattaa

In some embodiments of any of the compositions described herein, the HSPA1A protein comprises a sequence that is at least 75% (e.g., at least 80%, at least 85%, at least 90%, at least 91%, at least 92%, at least 93%, at least 94%, at least 95%, at least 96%, at least 97%, at least 98%, at least 99%) identical to SEQ ID NO: 64.

In some embodiments of any of the compositions described herein, the HSPA1A protein comprises a sequence that is at least 75% (e.g., at least 80%, at least 85%, at least 90%, at least 91%, at least 92%, at least 93%, at least 94%, at least 95%, at least 96%, at least 97%, at least 98%, at least 99%) identical to SEQ ID NO: 66.

In some embodiments of any of the compositions described herein, the HSPA1A protein comprises a sequence that is at least 75% (e.g., at least 80%, at least 85%, at least 90%, at least 91%, at least 92%, at least 93%, at least 94%, at least 95%, at least 96%, at least 97%, at least 98%, at least 99%) identical to SEQ ID NO: 68.

In some embodiments of any of the compositions described herein, the HSPA1A protein comprises a sequence that is at least 75% (e.g., at least 80%, at least 85%, at least 90%, at least 91%, at least 92%, at least 93%, at least 94%, at least 95%, at least 96%, at least 97%, at least 98%, at least 99%) identical to SEQ ID NO: 69.

In some embodiments of any of the compositions described herein, the HSPA1A protein comprises a sequence that is at least 75% (e.g., at least 80%, at least 85%, at least 90%, at least 91%, at least 92%, at least 93%, at least 94%, at least 95%, at least 96%, at least 97%, at least 98%, at least 99%) identical to SEQ ID NO: 70.

In some embodiments of any of the compositions described herein, the HSPA1A protein comprises a sequence that is at least 75% (e.g., at least 80%, at least 85%, at least 90%, at least 91%, at least 92%, at least 93%, at least 94%, at least 95%, at least 96%, at least 97%, at least 98%, at least 99%) identical to SEQ ID NO: 94.
**Mouse Mature HSPA1A Protein (SEQ ID NO: 66)** (NCBI Accession No. NP_034609.2) 
MAKNTAIGIDLGTTYSCVGVFQHGKVEIIANDQGNRTTPSYVAFTDTERLIGDAAKNQVALNPQNTVFDAK
RLIGRKFGDAVVQSDMKHWPFQVVNDGDKPKVQVNYKGESRSFFPEEISSMVLTKMKEIAEAYLGHPVTNA
VITVPAYFNDSQRQATKDAGVIAGLNVLRIINEPTAAAIAYGLDRTGKGERNVLIFDLGGGTFDVSILTID
DGIFEVKATAGDTHLGGEDFDNRLVSHFVEEFKRKHKKDISQNKRAVRRLRTACERAKRTLSSSTQASLEI
DSLFEGIDFYTSITRARFEELCSDLFRGTLEPVEKALRDAKMDKAQIHDLVLVGGSTRIPKVQKLLQDFFN
GRDLNKSINPDEAVAYGAAVQAAILMGDKSENVQDLLLLDVAPLSLGLETAGGVMTALIKRNSTIPTKQTQ
TFTTYSDNQPGVLIQVYEGERAMTRDNNLLGRFELSGIPPAPRGVPQIEVTFDIDANGILNVTATDKSTGK
ANKITITNDKGRLSKEEIERMVQEAERYKAEDEVQRDRVAAKNALESYAFNMKSAVEDEGLKGKLSEADKK
KVLDKCQEVISWLDSNTLADKEEFVHKREELERVCSPIISGLYQGAGAPGAGGFGAQAPKGASGSGPTIEE
VD
**Mouse Mature HSPA1A cDNA (SEQ ID NO: 67)** 
atggccaag aacacggcga tcggcatcga cctgggcacc acctactcgt gcgtgggcgt

gttccagcac ggcaaggtgg agatcatcgc caacgaccag ggcaaccgca cgacccccag

ctacgtggcc ttcaccgaca ccgagcgcct catcggagac gccgccaaga accaggtggc

gctgaacccg cagaacaccg tgttcgacgc gaagcggctg atcggccgca agttcggcga

tgcggtggtg cagtccgaca tgaagcactg gcccttccag gtggtgaacg acggcgacaa
 
gcccaaggtg caggtgaact acaagggcga gagccggtcg ttcttcccgg aggagatctc
gtccatggtg ctgacgaaga tgaaggagat cgctgaggcg tacctgggcc acccggtgac
caacgcggtg atcacggtgc ccgcctactt caacgactct cagcggcagg ccaccaagga
cgcgggcgtg atcgccggtc taaacgtgct gcggatcatc aacgagccca cggcggccgc
catcgcctac gggctggacc ggaccggcaa gggcgagcgc aacgtgctca tcttcgacct
ggggggcggc acgttcgacg tgtccatcct gacgatcgac gacggcatct tcgaggtgaa
ggccacggcg ggcgacacgc acctgggagg ggaggacttc gacaaccggc tggtgagcca
cttcgtggag gagttcaaga ggaagcacaa gaaggacatc agccagaaca agcgcgcggt
gcggcggctg cgcactgcgt gtgagagggc caagaggacg ctgtcgtcca gcacccaggc
cagcctggag atcgactctc tgttcgaggg catcgacttc tacacatcca tcacgcgggc
gcggttcgaa gagctgtgct cagacctgtt ccgcggcacg ctggagcccg tggagaaggc
cctgcgcgac gccaagatgg acaaggcgca gatccacgac ctggtgctgg tgggcggctc
gacgcgcatc cccaaggtgc agaagctgct gcaggacttc ttcaacgggc gcgacctgaa
caagagcatc aacccggacg aggcggtggc ctacggggcg gcggtgcagg cggccatcct
gatgggggac aagtcggaga acgtgcagga cctgctgctg ctggacgtgg cgccgctgtc
gctgggcctg gagactgcgg gcggcgtgat gacggcgctc atcaagcgca actccaccat
ccccaccaag cagacgcaga ccttcaccac ctactcggac aaccagcccg gggtgctgat
ccaggtgtac gagggcgaga gggccatgac gcgcgacaac aacctgctgg ggcgcttcga
actgagcggc atcccgccgg cgcccagggg cgtgccacag atcgaggtga ccttcgacat
cgacgccaac ggcatcctga acgtcacggc caccgacaag agcaccggca aggccaacaa
gatcaccatc accaacgaca agggccgcct gagcaaggag gagatcgagc gcatggtgca
ggaggccgag cgctacaagg ccgaggacga ggtgcagcgc gacagggtgg ccgccaagaa
cgcgctcgaa tcctatgcct tcaacatgaa gagcgccgtg gaggacgagg gtctcaaggg
caagctcagc gaggctgaca agaagaaggt gctggacaag tgccaggagg tcatctcctg
gctggactcc aacacgctgg ccgacaagga ggagttcgtg cacaagcggg aggagctgga
gcgggtgtgc agccccatca tcagtgggct gtaccagggt gcgggtgctc ctggggctgg
gggcttcggg gcccaggcgc ccaagggagc ctctggctca ggacccacca tcgaggaggt
ggattaga
**Rhesus Monkey Mature HSPA1A Protein (SEQ ID NO: 68)** (NCBI Accession No. XP_014991489.2) 
MAKAAAIGIDLGTTYSCVGVFQHGKVEIIANDQGNRTTPSYVAFTDTERLIGDAAKNQVALNPQNTVFDAK
RLIGRKFGDPVVQSDMKHWPFQVINDGDKPKVQVSYKGETKAFYPEEISSMVLTKMKEIAEAYLGYPVTNA
VITVPAYFNDSQRQATKDAGVIAGLNVLRIINEPTAAAIAYGLDRTGKGERNVLIFDLGGGTFDVSILTID
DGIFEVKATAGDTHLGGEDFDNRLVNHFVEEFKRKHKKDISQNKRAVRRLRTACERAKRTLSSSTQASLEI
DSLFEGIDFYTSITRARFEELCSDLFRSTLEPVEKALRDAKLDKAQIHDLVLVGGSTRIPKVQKLLQDFFN
GRDLNKSINPDEAVAYGAAVQAAILMGDKSENVQDLLLLDVAPLSLGLETAGGVMTALIKRNSTIPTKQTQ
IFTTYSDNQPGVLIQVYEGERAMTKDNNLLGRFELSGIPPAPRGVPQIEVTFDIDANGILNVTATDKSTGK
 
ANKITITNDKGRLSKEEIERMVQEAEKYKAEDEVQRERVSAKNALESYAFNMKSAVEDEGLKGKISEADKK
KVLDKCQEVISWLDANTLAEKDEFEHKRKELEQVCNPIISGLYQGAGGPGPGGFGAQGPKGGSGSGPTIEE
VD
**Rat Mature HSPA1A Protein (SEQ ID** NO: **69)** (NCBI Accession No. NP_114177.2) 
MAKKTAIGIDLGTTYSCVGVFQHGKVEIIANDQGNRTTPSYVAFTDTERLIGDAAKNQVALNPQNTVFDAK
RLIGRKFGDPVVQSDMKHWPFQVVNDGDKPKVQVNYKGENRSFYPEEISSMVLTKMKEIAEAYLGHPVTNA
VITVPAYFNDSQRQATKDAGVIAGLNVLRIINEPTAAAIAYGLDRTGKGERNVLIFDLGGGTFDVSILTID
DGIFEVKATAGDTHLGGEDFDNRLVSHFVEEFKRKHKKDISQNKRAVRRLRTACERAKRTLSSSTQASLEI
DSLFEGIDFYTSITRARFEELCSDLFRGTLEPVEKALRDAKLDKAQIHDLVLVGGSTRIPKVQKLLQDFFN
GRDLNKSINPDEAVAYGAAVQAAILMGDKSENVQDLLLLDVAPLSLGLETAGGVMTALIKRNSTIPTKQTQ
TFTTYSDNQPGVLIQVYEGERAMTRDNNLLGRFELSGIPPAPRGVPQIEVTFDIDANGILNVTATDKSTGK
ANKITITNDKGRLSKEEIERMVQEAERYKAEDEVQRERVAAKNALESYAFNMKSAVEDEGLKGKISEADKK
KVLDKCQEVISWLDSNTLAEKEEFVHKREELERVCNPIISGLYQGAGAPGAGGFGAQAPKGGSGSGPTIEE
VD
**Cattle HSPA1A Protein (SEQ ID NO:** 70) (NCBI Accession No. NP_976067.3) 
MAKNMAIGIDLGTTYSCVGVFQHGKVEIIANDQGNRTTPSYVAFTDTERLIGDAAKNQVALNPQNTVFDAK
RLIGRKFGDPVVQSDMKHWPFRVINDGDKPKVQVSYKGETKAFYPEEISSMVLTKMKEIAEAYLGHPVTNA
VITVPAYFNDSQRQATKDAGVIAGLNVLRIINEPTAAAIAYGLDRTGKGERNVLIFDLGGGTFDVSILTID
DGIFEVKATAGDTHLGGEDFDNRLVNHFVEEFKRKHKKDISQNKRAVRRLRTACERAKRTLSSSTQASLEI
DSLFEGIDFYTSITRARFEELCSDLFRSTLEPVEKALRDAKLDKAQIHDLVLVGGSTRIPKVQKLLQDFFN
GRDLNKSINPDEAVAYGAAVQAAILMGDKSENVQDLLLLDVAPLSLGLETAGGVMTALIKRNSTIPTKQTQ
IFTTYSDNQPGVLIQVYEGERAMTRDNNLLGRFELSGIPPAPRGVPQIEVTFDIDANGILNVTATDKSTGK
ANKITITNDKGRLSKEEIERMVQEAEKYKAEDEVQRERVSAKNALESYAFNMKSAVEDEGLKGKISEADKK
KVLDKCQEVISWLDANTLAEKDEFEHKRKELEQVCNPIISRLYQGAGGPGAGGFGAQGPKGGSGSGPTIEE
VD
**Human Mature HSPA1A Protein (SEQ ID NO: 93)**

AKAAAIGIDLGTTYSCVGVFQHGKVEIIANDQGNRTTPSYVAFTDTERLIGDAAKNQVALNPQNTVFDAKR
LIGRKFGDPVVQSDMKHWPFQVINDGDKPKVQVSYKGETKAFYPEEISSMVLTKMKEIAEAYLGYPVTNAV
ITVPAYFNDSQRQATKDAGVIAGLNVLRIINEPTAAAIAYGLDRTGKGERNVLIFDLGGGTFDVSILTIDD
GIFEVKATAGDTHLGGEDFDNRLVNHFVEEFKRKHKKDISQNKRAVRRLRTACERAKRTLSSSTQASLEID
SLFEGIDFYTSITRARFEELCSDLFRSTLEPVEKALRDAKLDKAQIHDLVLVGGSTRIPKVQKLLQDFFNG
RDLNKSINPDEAVAYGAAVQAAILMGDKSENVQDLLLLDVAPLSLGLETAGGVMTALIKRNSTIPTKQTQI
FTTYSDNQPGVLIQVYEGERAMTKDNNLLGRFELSGIPPAPRGVPQIEVTFDIDANGILNVTATDKSTGKA
NKITITNDKGRLSKEEIERMVQEAEKYKAEDEVQRERVSAKNALESYAFNMKSAVEDEGLKGKISEADKKK

VLDKCQEVISWLDANTLAEKDEFEHKRKELEQVCNPIISGLYQGAGGPGPGGFGAQGPKGGSGSGPTIEEV
D

A non-limiting example of a human wildtype NDP genomic DNA sequence is SEQ **ID** NO: 71.
**Human HSPA1A Gene Sequence (SEQ ID NO: 71) (NCBI Reference Sequence** NC_000006.12) 
1 aacggctagc ctgaggagct gctgcgacag tccactacct ttttcgagag tgactcccgt 61
tgtcccaagg cttcccagag cgaacctgtg cggctgcagg caccggcgcg tcgagtttcc 121
ggcgtccgga aggaccgagc tcttctcgcg gatccagtgt tccgtttcca gcccccaatc 181
tcagagcgga gccgacagag agcagggaac cggcatggcc aaagccgcgg cgatcggcat 241
cgacctgggc accacctact cctgcgtggg ggtgttccaa cacggcaagg tggagatcat 301
cgccaacgac cagggcaacc gcaccacccc cagctacgtg gccttcacgg acaccgagcg 361
gctcatcggg gatgcggcca agaaccaggt ggcgctgaac ccgcagaaca ccgtgtttga 421
cgcgaagcgg ctgattggcc gcaagttcgg cgacccggtg gtgcagtcgg acatgaagca 481
ctggcctttc caggtgatca acgacggaga caagcccaag gtgcaggtga gctacaaggg 541
ggagaccaag gcattctacc ccgaggagat ctcgtccatg gtgctgacca agatgaagga 601
gatcgccgag gcgtacctgg gctacccggt gaccaacgcg gtgatcaccg tgccggccta 661
cttcaacgac tcgcagcgcc aggccaccaa ggatgcgggt gtgatcgcgg ggctcaacgt 721
gctgcggatc atcaacgagc ccacggccgc cgccatcgcc tacggcctgg acagaacggg 781
caagggggag cgcaacgtgc tcatctttga cctgggcggg ggcaccttcg acgtgtccat 841
cctgacgatc gacgacggca tcttcgaggt gaaggccacg gccggggaca cccacctggg 901
tggggaggac tttgacaaca ggctggtgaa ccacttcgtg gaggagttca agagaaaaca 961
caagaaggac atcagccaga acaagcgagc cgtgaggcgg ctgcgcaccg cctgcgagag 1021
ggccaagagg accctgtcgt ccagcaccca ggccagcctg gagatcgact ccctgtttga 1081
gggcatcgac ttctacacgt ccatcaccag ggcgaggttc gaggagctgt gctccgacct 1141
gttccgaagc accctggagc ccgtggagaa ggctctgcgc gacgccaagc tggacaaggc 1201
ccagattcac gacctggtcc tggtcggggg ctccacccgc atccccaagg tgcagaagct 1261
gctgcaggac ttcttcaacg ggcgcgacct gaacaagagc atcaaccccg acgaggctgt 1321
ggcctacggg gcggcggtgc aggcggccat cctgatgggg gacaagtccg agaacgtgca 1381
ggacctgctg ctgctggacg tggctcccct gtcgctgggg ctggagacgg ccggaggcgt 1441
gatgactgcc ctgatcaagc gcaactccac catccccacc aagcagacgc agatcttcac 1501
cacctactcc gacaaccaac ccggggtgct gatccaggtg tacgaggcgg agagggccat 1561
gacgaaagac aacaatctgt tggggcgctt cgagctgagc ggcatccctc cggcccccag 1621
gggcgtgccc cagatcgagg tgaccttcga catcgatgcc aacggcatcc tgaacgtcac 1681
ggccacggac aagagcaccg gcaaggccaa caagatcacc atcaccaacg acaagggccg 1741
 
cctgagcaag gaggagatcg agcgcatggt gcaggaggcg gagaagtaca aagcggagga 1801
cgaggtgcag cgcgagaggg tgtcagccaa gaacgccctg gagtcctacg ccttcaacat 1861
gaagagcgcc gtggaggatg aggggctcaa gggcaagatc agcgaggcgg acaagaagaa 1921
ggtgctggac aagtgtcaag aggtcatctc gtggctggac gccaacacct tggccgagaa 1981
ggacgagttt gagcacaaga ggaaggagct ggagcaggtg tgtaacccca tcatcagcgg 2041
actgtaccag ggtgccggtg gtcccgggcc tgggggcttc ggggctcagg gtcccaaggg 2101
agggtctggg tcaggcccca ccattgagga ggtagattag gggcctttcc aagattgctg 2161
tttttgtttt ggagcttcaa gactttgcat ttcctagtat ttctgtttgt cagttctcaa 2221
tttcctgtgt ttgcaatgtt gaaatttttt ggtgaagtac tgaacttgct ttttttccgg 2281
tttctacatg cagagatgaa tttatactgc catcttacga ctatttcttc tttttaatac 2341
acttaactca ggccattttt taagttggtt acttcaaagt aaataaactt taaaattcaa
**Human HSPA1A Gene Sequence (SEQ ID NO: 94)**

atgtaccggatgcagctgctgagctgtatcgccctgtctctggccctggtcaccaattctgccaaagccgc
ggcgatcggcatcgacctgggcaccacctactcctgcgtgggggtgttccaacacggcaaggtggagatca
tcgccaacgaccagggcaaccgcaccaccccagctacgtggccttcacggacaccgagcggctcatcggg
gatgcggccaagaaccaggtggcgctgaacccgcagaacaccgtgtttgacgcgaagcggctgatcggccg
caagttcggcgacccggtggtgcagtcggacatgaagcactggcctttccaggtgatcaacgacggagaca
agcccaaggtgcaggtgagctacaagggggagaccaaggcattctaccccgaggagatctcgtccatggtg
ctgaccaagatgaaggagatcgccgaggcgtacctgggctacccggtgaccaacgcggtgatcaccgtgcc
ggcctacttcaacgactcgcagcgccaggccaccaaggatgcgggtgtgatcgcggggctcaacgtgctgc
ggatcatcaacgagcccacggccgccgccatcgcctacggcctggacagaacgggcaaggggggagcgcaac
gtgctcatctttgacctgggcgggggcaccttcgacgtgtccatcctgacgatcgacgacggcatcttcga
ggtgaaggccacggccggggacacccacctgggtggggaggactttgacaacaggctggtgaaccacttcg
tggaggagttcaagagaaaacacaagaaggacatcagccagaacaagcgagccgtgaggcggctgcgcacc
gcctgcgagagggccaagaggaccctgtcgtccagcacccaggccagcctggagatcgactccctgtttga
gggcatcgacttctacacgtccatcaccagggcgaggttcgaggagctgtgctccgacctgttccgaagca
ccctggagcccgtggagaaggctctgcgcgacgccaagctggacaaggcccagattcacgacctggtcctg
gtcgggggctccacccgcatccccaaggtgcagaagctgctgcaggacttcttcaacgggcgcgacctgaa
caagagcatcaaccccgacgaggctgtggcctacggggcggcggtgcaggcggccatcctgatgggggaca
agtccgagaacgtgcaggacctgctgctgctggacgtggctcccctgtcgctggggctggagacggccgga
ggcgtgatgactgccctgatcaagcgcaactccaccatccccaccaagcagacgcagatcttcaccaccta
ctccgacaaccaacccggggtgctgatccaggtgtacgagggcgagagggccatgacgaaagacaacaatc
tgttggggcgcttcgagctgagcggcatccctccggcccccaggggcgtgccccagatcgaggtgaccttc
gacatcgatgccaacggcatcctgaacgtcacggccacggacaagagcaccggcaaggccaacaagatcac
catcaccaacgacaagggccgcctgagcaaggaggagatcgagcgcatggtgcaggaggcggagaagtaca
aagcggaggacgaggtgcagcgcgagagggtgtcagccaagaacgccctggagtcctacgccttcaacatg

aagagcgccgtggaggatgaggggctcaagggcaagatcagcgaggcggacaagaagaaggttctggacaa
gtgtcaagaggtcatctcgtggctggacgccaacaccttggccgagaaggacgagtttgagcacaagagga
aggagctggagcaggtgtgtaaccccatcatcagcggactgtaccagggtgccggtggtcccgggcctggg
ggcttcggggctcagggtcccaaggagggtctgggtcaggccccaccattgaggaggtggattaa

### Vascular Endothelial Growth Factor (VEGF)

The VEGF gene encodes vascular endothelial growth factor (VEGF), formerly known as fms-like tyrosine kinase (Flt-1). The VEGF protein is a heparin-biding protein that induces migration and proliferation of vascular endothelial cells.

The human VEGF gene is located on chromosome 6p21. It contains 8 exons encompassing ~16 kilobases (kb) (NCBI Accession No. NG_008732.1). The full-length wildtype VEGF protein expressed from the human VEGF gene is 412 amino acids in length.

An exemplary human wildtype VEGF protein is or includes the sequence of SEQ ID NO: 72, SEQ ID NO: 74, SEQ ID NO: 76, SEQ ID NO: 77, SEQ ID NO: 78, or SEQ ID NO: 79. Non-limiting examples of nucleic acid encoding a wildtype VEGF protein are or include SEQ ID NO: 73 and SEQ ID NO: 75.

Non-limiting examples of protein and nucleotide sequences encoding a wildtype VEGF protein are shown below.
**Human VEGF Transcript Variant 1 Protein Sequence (SEQ ID NO: 72)** 
MTDRQTDTAPSPSYHLLPGRRRTVDAAASRGQGPEPAPGGGVEGVGARGVALKLFVQLLGCSRFGGAVVRA
GEAEPSGAARSASSGREEPQPEEGEEEEEKEEERGPQWRLGARKPGSWTGEAAVCADSAPAARAPQALARA
SGRGGRVARRGAEESGPPHSPSRRGSASRAGPGRASETMNFLLSWVHWSLALLLYLHHAKWSQAAPMAEGG
GQNHHEVVKFMDVYQRSYCHPIETLVDIFQEYPDEIEYIFKPSCVPLMRCGGCCNDEGLECVPTEESNITM
QIMRIKPHQGQHIGEMSFLQHNKCECRPKKDRARQEKKSVRGKGKGQKRKRKKSRYKSWSVYVGARCCLMP
WSLPGPHPCGPCSERRKHLFVQDPQTCKCSCKNTDSRCKARQLELNERTCRCDKPRR
**Human VEGF Transcript Variant 1 cDNA (SEQ ID NO: 73)** 
ctgacggacagacagacagacaccgcccccagccccagctaccacctcctccccggccggcggacagt
ggacgcggcggcgagccgcgggcaggggccggagcccgcgcccggaggcggggtggaggggtcgggctc
gcggcgtcgcactgaaacttttcgtccaacttctgggctgttctcgcttcggaggagccgtggtccgcgcg
ggggaagccgagccgagcggagccgcgagaagtgctagctcgggccgggaggagccgcagccggaggaggg
ggaggaggaagaagagaaggaagaggagaggggggccgcagtggcgactcggcgctcggaagccgggctcat
 
ggacgggtgaggcggcggtgtgcgcagacagtgctccagccgcgcgctccccaggccctggcccgggcc
tcgggccggggaggaagagtagctcgccgaggcgccgaggagagcgggccgccccacagcccgagccggag
agggagcgcgagccgcgccggccccggtcggcctccgaaaccatgaactttctgctgtcttgggtgcatt
ggagccttgccttgctgctctacctccaccatgccaagtggtcccaggctgcacccatggcagaaggagga
gggcagaatcatcacgaagtggtgaagttcatggatgtctatcagcgcagctactgccatccaatcgagac
cctggtggacatcttccaggagtaccctgatgagatcgagtacatcttcaagccatcctgtgtgcccctga
tgcgatgcgggggctgctgcaatgacgagggcctggagtgtgtgcccactgaggagtccaacatcaccatg
cagattatgcggatcaaacctcaccaaggccagcacataggagagatgagcttcctacagcacaacaaatg
tgaatgcagaccaaagaaagatagagcaagacaagaaaaaaatcagttcgaggaaagggaaaggggcaaa
aacgaaagcgcaagaaatcccggtataagtcctggagcgtgtacgttggtgcccgctgctgtctaatgccc
tggagcctccctggcccccatccctgtgggccttgctcagagcggagaaagcatttgtttgtacaagatcc
gcagacgtgtaaatgttcctgcaaaaacacagactcgcgttgcaaggcgaggcagcttgagttaaacgaac
gtacttgcagatgtgacaagccgaggcggtga
**Human VEGF Transcript Variant 3 Protein Sequence (SEQ ID NO: 74)** 
MTDRQTDTAPSPSYHLLPGRRRTVDAAASRGQGPEPAPGGGVEGVGARGVALKLFVQLLGCSRFGGAVVRA
GEAEPSGAARSASSGREEPQPEEGEEEEEKEEERGPQWRLGARKPGSWTGEAAVCADSAPAARAPQALARA
SGRGGRVARRGAEESGPPHSPSRRGSASRAGPGRASETMNFLLSWVHWSLALLLYLHHAKWSQAAPMAEGG
GQNHHEVVKFMDVYQRSYCHPIETLVDIFQEYPDEIEYIFKPSCVPLMRCGGCCNDEGLECVPTEESNITM
QIMRIKPHQGQHIGEMSFLQHNKCECRPKKDRARQEKKSVRGKGKGQKRKRKKSRPCGPCSERRKHLFVQD
PQTCKCSCKNTDSRCKARQLELNERTCRCDKPRR
**Human VEGF Transcript Variant 3 cDNA (SEQ ID NO: 75)** 
ctgacggacagacagacagacaccgcccccagccccagctaccacctcctccccggccggcggcggacagt
ggacgcggcggcgagccgcgggcaggggccggagcccgcgcccggaggcggggtggaggggggtcggggctc
gcggcgtcgcactgaaacttttcgtccaacttctgggctgttctcgcttcggaggagccgtggtccgcgcg
ggggaagccgagccgagcggagccgcgagaagtgctagctcgggccgggaggagccgcagccggaggaggg
ggaggaggaagaagagaaggaagaggagaggggggccgcagtggcgactcggcgctcggaagccgggctcat
ggacgggtgaggcggcggtgtgcgcagacagtgctccagccgcgcgcgctccccaggccctggcccgggcc
tcgggccggggaggaagagtagctcgccgaggcgccgaggagagcgggccgccccacagcccgagccggag
agggagcgcgagccgcgccggccccggtcggcctccgaaaccatgaactttctgctgtcttgggtgcatt
ggagccttgccttgctgctctacctccaccatgccaagtggtcccaggctgcacccatggcagaaggagga
gggcagaatcatcacgaagtggtgaagttcatggatgtctatcagcgcagctactgccatccaatcgagac
cctggtggacatcttccaggagtaccctgatgagatcgagtacatcttcaagccatcctgtgtgcccctga
tgcgatgcgggggctgctgcaatgacgagggcctggagtgtgtgcccactgaggagtccaacatcaccatg
cagattatgcggatcaaacctcaccaaggccagcacataggagagatgagcttcctacagcacaacaaatg
tgaatgcagaccaaagaaagatagagcaagacaagaaaaaaaatcagttcgaggaaagggaaaggggcaaa
 
aacgaaagcgcaagaaatcccgtccctgtgggccttgctcagagcggagaaagcatttgtttgtacaagat
ccgcagacgtgtaaatgttcctgcaaaaacacagactcgcgttgcaaggcgaggcagcttgagttaa
acgaacgtacttgcagatgtgacaagccgaggcggtga
**Mature Human VEGF-A (SEQ ID NO: 76)** 
APMAEGGGQNHHEVVKFMDVYQRSYCHPIETLVDIFQEYPDEIEYIFKPSCVPLMRCGGCCNDEGLECVPT
EESNITMQIMRIKPHQGQHIGEMSFLQHNKCECRPKKDRARQEKKSVRGKGKGQKRKRKKSRYKSWSVYVG
ARCCLMPWSLPGPHPCGPCSERRKHLFVQDPQTCKCSCKNTDSRCKARQLELNERTCRCDKPRR
**Mature Human VEGF-B (SEQ ID NO: 77)** 
PVSQPDAPGHQRKVVSWIDVYTRATCQPREVVVPLTVELMGTVAKQLVPSCVTVQRCGGCCPDDGLECVPT
GQHQVRMQILMIRYPSSQLGEMSLEEHSQCECRPKKKDSAVKPDRAATPHHRPQPRSVPGWDSAPGAPSPA
DITHPTPAPGPSAHAAPSTTSALTPGPAAAAADAAASSVAKGGA
**Mature Human VEGF-C (SEQ ID NO: 78)** 
AHYNTEILKSIDNEWRKTQCMPREVCIDVGKEFGVATNTFFKPPCVSVYRCGGCCNSEGLQCMNTSTSYLS
KTLFEITVPLSQGPKPVTISFANHTSCRCMSKLDVYRQVHSIIRR
Mature Human VEGF-D (SEQ **ID** NO: 79)

FAATFYDIETLKVIDEEWQRTQCSPRETCVEVASELGKSTNTFFKPPCVNVFRCGGCCNEESLICMNTSTS
YISKQLFEISVPLTSVPELVPVKVANHTGCKCLPTAPRHPYSIIRR

In some embodiments of any of the compositions described herein, the VEGF protein comprises a sequence that is at least 75% (e.g., at least 80%, at least 85%, at least 90%, at least 91%, at least 92%, at least 93%, at least 94%, at least 95%, at least 96%, at least 97%, at least 98%, at least 99%) identical to SEQ ID NO: 72.

In some embodiments of any of the compositions described herein, the VEGF protein comprises a sequence that is at least 75% (e.g., at least 80%, at least 85%, at least 90%, at least 91%, at least 92%, at least 93%, at least 94%, at least 95%, at least 96%, at least 97%, at least 98%, at least 99%) identical to SEQ ID NO: 74.

In some embodiments of any of the compositions described herein, the VEGF protein comprises a sequence that is at least 75% (e.g., at least 80%, at least 85%, at least 90%, at least 91%, at least 92%, at least 93%, at least 94%, at least 95%, at least 96%, at least 97%, at least 98%, at least 99%) identical to SEQ ID NO: 76.

In some embodiments of any of the compositions described herein, the VEGF protein comprises a sequence that is at least 75% (e.g., at least 80%, at least 85%, at least 90%, at least 91%, at least 92%, at least 93%, at least 94%, at least 95%, at least 96%, at least 97%, at least 98%, at least 99%) identical to SEQ ID NO: 77.

In some embodiments of any of the compositions described herein, the VEGF protein comprises a sequence that is at least 75% (e.g., at least 80%, at least 85%, at least 90%, at least 91%, at least 92%, at least 93%, at least 94%, at least 95%, at least 96%, at least 97%, at least 98%, at least 99%) identical to SEQ ID NO: 78.

In some embodiments of any of the compositions described herein, the VEGF protein comprises a sequence that is at least 75% (e.g., at least 80%, at least 85%, at least 90%, at least 91%, at least 92%, at least 93%, at least 94%, at least 95%, at least 96%, at least 97%, at least 98%, at least 99%) identical to SEQ ID NO: 79.

A non-limiting example of a human wildtype VEGF genomic DNA sequence is SEQ ID NO: 80. The exons in SEQ ID NO: 80 are: nucleotide positions 1-1104 (exon 1); nucleotide positions 4133-4184 (exon 2) and nucleotide positions 7261-7457 (exon 3); nucleotide positions 8252-8328 (exon 4); nucleotide positions 8681-8710 (exon 5); nucleotide positions 10524-10646 (exon 6); nucleotide positions 11748-11879 (exon 7); and nucleotide positions 14333-16279 (exon 8). The introns in SEQ ID NO: 80 are: nucleotide positions 1105-4132 (intron 1); nucleotide positions 4185-7260 (intron 2); nucleotide positions 7458-8251 (intron 3); nucleotide positions 8329-8680 (intron 4); nucleotide positions 8711-10523 (intron 5); nucleotide positions 10647-11747 (intron 6); and nucleotide positions 11880-14332 (intron 7).
**Human VEGF-A Gene (SEQ ID NO: 80)** (NCBI Accession No. NG_008732.1) 
1 cgcctgtaat cccagcactc tgggaggcag aggtgggccg atcacttgag gtcaggagtt

61 cgagaccagc ctgggcaaca tggtgaaaca ccatctctac taaaaacaca aaaattagcc

121 aggtgtggtg gcaggcacct gcagtcccag ctactccgga ggctgaggca ggagaattgc

181 tcgaacctgg gaggcagggg ttgcagtgag ccgacatggc gccactgcac tccagtctgg

241 gcgacagagt gagaccctat ctcaaaaaaa aaaaaaaaaa aaaaagaccc aactcaagta

301 tcatctccag gaagccttcc cctactccca gcaattaaat gctcctcaga gaattcccat
 
361 ttttggttta ctctttggtt tacctccaga caggaagccc ccactgacac tgttgtagtc
421 ccagggtgca acacaaagca gagatcacaa gctgagttta ataattgctt gtggaataca
481 tgtcccaagc cacctcctgc aggaagccct tccagatgcc cattctagcc agtctggctc
541 tttgcttcca taccttcaca acacttgtgc ctcccccagg gcctctttct catcttgctt
601 tctggggcag ctgtgtgcac atttgtctgt gtgcagcaac tctctaaggc agggattttt
661 actcctattt ttgatgaggg gagctgtggc tcagagaggt tgaataacct aaggccacac
721 agtgagtggc agagccagga atgtgacttg ggtccatttg aatccaaagt ccctgtactt
781 tccactgccc tacctagatg tccctgtacc tcctataaaa tcagcatgga gcctggtgcc
841 tggtagtccc tacaaatatt cacaaattgg agcttagctc agctctcagg caaggcccag
901 gtcaaaaggg cagatacagc tttgggacct tagttgccac cacatgccat accttcttcc
961 cagcagaagg actccctcca agacagggta ggggtggagg atgtgaacag gggcagaaat
1021 gggcatgttt tggggtcaga cttggaggaa tagcagagat tggagtgtca gaaggtgagc
1081 atgcctgggg gtgttgggga gatgcaattc atcagggaca gcttagtgtc aggggattag
1141 actggggccc atgaaggaga ggcagaggct gatgggccta ggggtggtgt gggtaggtga
1201 gcttccccag acagtgactc tgccctgccc tctctccagc taggtcctct tccccattcc
1261 ttcccccttt cctgactgga tcctcttggg agagttaccc tccttggctt cctctgctcc
1321 aatcttttta tcagttggcc atcattactt atcattacct caagtcaaac ctccagatcc
1381 acatggggct aggacattgg cactggacca aagaggccct ttcctttgct ttctctttgt
1441 ctttttaatg ctttgttgca aagacctagg cggggagaga gagagagaga gagagacaga
1501 gattgaccca cagtcagggt cagggaattg aggggaacca acccaattct ctctccttca
1561 attcaccagg tttgtatcct gcccttcctg cagatcagtg tcctgctagt cacctggggg
1621 tcaggggatg gagtgaagga caagacctcc ttccattgca gtgaagccac ttggagaaat
1681 gtgtggaaaa cagcaagacc cagtgactct ctcctcacct tctttccaat ctcaggagag
1741 attttgtccc ttcatccacc ggcttctaga ttaaccaccc acacccacac aggcgagagt
1801 ttccctgaat attggaggtg acaggacatc aggacaaagt acaactattg tgccttggcc
1861 caatcactac tttctttgtc tggggccgcc gctggctcct ggctgccttt cgcacttttc
1921 tccaccccca cccctttctc cccttccccc tcacctggaa caccttcccc ttcctccttg
1981 gctccttctg aactgccttc agagccacag actgtgggga gtggccactg cgctcccaag
2041 gtgaggccct ccaagcgggg ccgagtttgc ccctcaactg ggagccagca tgacctctgt
2101 gtgggctgct ctctgcttca ctgccccttc ccccaatctg ctaggtgacc ctgggcccct
2161 ttgtgccctc tctgggcctt cggaggattc tttggggaga cagtctgctc tgacgcccct
2221 tcccctgcag caagcagcct ggggagggag gtgaggataa gtgaagtcaa gttgttcagg
2281 gggctaagcc catggaaggg aagatgccac agagatacat gtggtcttgt gattgttgtt
2341 ttgtgctttt tccccttttt tgaaagctca ggtgactagg tgacttgagc ttttaatttg
2401 gtgacaatgt gggcactggc tgagtcctta agagtacatt gttgtaaatg ccggtgacaa
2461 cacactgggg catgggatcc agagttaacc cctccaggtc acagccaggt tatatctcca
2521 caatgaaggg gggaggtggg ccatactttc tcgccctaat gaaggtagct caaaaacccc
2581 taggccaggt tgtaatccta gccttatata aaaggaattc tgtgccctca ctcccctgga
 
2641 tccctgggca aagccccaga gggaaacaca aacaggttgt tgtaacacac cttgctgggt
2701 accaccatgg aggacagttg gcttatgggg gtggggggtg cctggggcca cggagtgact
2761 ggtgatggct atccctcctt ggaacccctc cagcctcctc ttagcttcag atttgtttat
2821 ttgtttttta ctaagacctg ctctttcagg tctgttggct cttttagggg ctgaagaagg
2881 ccgagttgag aagggatgca agggaggggg ccagaatgag cccttagggc tcagagcctc
2941 catcctgccc caagatgtct acagcttgtg ctcctggggt gctagaggcg cacaaggagg
3001 aaagttagtg gcttcccttc catatcccgt tcatcagcct agagcatgga gcccaggtga
3061 ggaggcctgc ctgggagggg gccctgagcc aggaaataaa catttactaa ctgtacaaag
3121 accttgtccc tgctgctggg gagcctgcca agtggtggag acaggactag tgcacgaatg
3181 atggaaaggg agggttgggg tgggtgggag ccagcccttt tcctcataag ggccttagga
3241 caccataccg atggaactgg gggtactggg gaggtaacct agcacctcca ccaaaccaca
3301 gcaacatgtg ctgaggatgg ggctgactag gtaagctccc tggagcgttt tggttaaatt
3361 gagggaaatt gctgcattcc cattctcagt ccatgcctcc acagaggcta tgccagctgt
3421 aggccagacc ctggcaagat ctgggtggat aatcagactg actggtccca ctcttcccac
3481 aggcctcaga gccccaactt tgttccctgg ggcagcctgg aaatagccag gtcagaaacc
3541 agctaggaat ttttccaagc tgcttcctat atgcaagaat gggatggggc ctttgggagc
3601 acttagggaa gatgtggaga gttggaggaa aagggggctt ggaggtaagg gaggggactg
3661 ggggaaggat aggggagaag ctgtgagcct ggagaagtag ccaaggatcc ctgagggaat
3721 gggggagctg agacgaaacc cccatttcta ttcagaagat gagctatgag tctgggcttg
3781 ggctgataga agccttggcc cctggcctgg tgggagctct gggcagctgg cctacagacg
3841 ttccttagtg ctggcgggta ggtttgaatc atcacgcagg ccctggcctc cacccgcccc
3901 caccagcccc ctggcctcag ttccctggca acatctgggg ttgggggggc agcaggaaca
3961 agggcctctg tctgcccagc tgcctccccc tttgggtttt gccagactcc acagtgcata
4021 cgtgggctcc aacaggtcct cttccctccc agtcactgac taaccccgga accacacagc
4081 ttcccgttct cagctccaca aacttggtgc caaattcttc tcccctggga agcatccctg
4141 gacacttccc aaaggacccc agtcactcca gcctgttggc tgccgctcac tttgatgtct
4201 gcaggccaga tgagggctcc agatggcaca ttgtcagagg gacacactgt ggcccctgtg
4261 cccagccctg ggctctctgt acatgaagca actccagtcc caaatatgta gctgtttggg
4321 aggtcagaaa tagggggtcc aggagcaaac tccccccacc ccctttccaa agcccattcc
4381 ctctttagcc agagccgggg tgtgcagacg gcagtcacta gggggcgctc ggccaccaca
4441 gggaagctgg gtgaatggag cgagcagcgt cttcgagagt gaggacgtgt gtgtctgtgt
4501 gggtgagtga gtgtgtgcgt gtggggttga gggcgttgga gcggggagaa ggccaggggt
4561 cactccagga ttccaataga tctgtgtgtc cctctcccca cccgtccctg tccggctctc
4621 cgccttcccc tgcccccttc aatattccta gcaaagaggg aacggctctc aggccctgtc
4681 cgcacgtaac ctcactttcc tgctccctcc tcgccaatgc cccgcgggcg cgtgtctctg
4741 gacagagttt ccgggggcgg atgggtaatt ttcaggctgt gaaccttggt gggggtcgag
4801 cttccccttc attgcggcgg gctgcgggcc aggcttcact gagcgtccgc agagcccggg
4861 cccgagccgc gtgtggaagg gctgaggctc gcctgtcccc gcccccgggg gcgggccggg
 
4921 ggcggggtcc cggcggggcg gagccatgcg cccccccctt ttttttttaa aagtcggctg
4981 gtagcgggga ggatcgcgga ggcttggggc agccgggtag ctcggaggtc gtggcgctgg
5041 gggctagcac cagcgctctg tcgggaggcg cagcggttag gtggaccggt cagcggactc
5101 accggccagg gcgctcggtg ctggaatttg atattcattg atccgggttt tatccctctt
5161 cttttttctt aaacattttt ttttaaaact gtattgtttc tcgttttaat ttatttttgc
5221 ttgccattcc ccacttgaat cgggccgacg gcttggggag attgctctac ttccccaaat
5281 cactgtggat tttggaaacc agcagaaaga ggaaagaggt agcaagagct ccagagagaa
5341 gtcgaggaag agagagacgg ggtcagagag agcgcgcggg cgtgcgagca gcgaaagcga
5401 caggggcaaa gtgagtgacc tgcttttggg ggtgaccgcc ggagcgcggc gtgagccctc
5461 ccccttggga tcccgcagct gaccagtcgc gctgacggac agacagacag acaccgcccc
5521 cagccccagc taccacctcc tccccggccg gcggcggaca gtggacgcgg cggcgagccg
5581 cgggcagggg ccggagcccg cgcccggagg cggggtggag ggggtcgggg ctcgcggcgt
5641 cgcactgaaa cttttcgtcc aacttctggg ctgttctcgc ttcggaggag ccgtggtccg
5701 cgcgggggaa gccgagccga gcggagccgc gagaagtgct agctcgggcc gggaggagcc
5761 gcagccggag gagggggagg aggaagaaga gaaggaagag gagagggggc cgcagtggcg
5821 actcggcgct cggaagccgg gctcatggac gggtgaggcg gcggtgtgcg cagacagtgc
5881 tccagccgcg cgcgctcccc aggccctggc ccgggcctcg ggccggggag gaagagtagc
5941 tcgccgaggc gccgaggaga gcgggccgcc ccacagcccg agccggagag ggagcgcgag
6001 ccgcgccggc cccggtcggg cctccgaaac catgaacttt ctgctgtctt gggtgcattg
6061 gagccttgcc ttgctgctct acctccacca tgccaaggta agcggtcgtg ccctgctggc
6121 gccgcgggcc gctgcgagcg cctctcccgg ctggggacgt gcgtgcgagc gcgcgcgtgg
6181 gggctccgtg ccccacgcgg gtccatgggc accaggcgtg cggcgtcccc ctctgtcgtc
6241 ttaggtgcag gggagggggg cgcgcgcgct aggtgggagg gtacccggag agaggctcac
6301 cgcccacgcg ggccctgccc acccaccgga gtcaccgcac gtacgatctg ggccgaccag
6361 ccgagggcgg gagccggagg aggaggccga gggggctggg cttgcgttgc cgctgccggc
6421 tgaagtttgc tcccggccgc tggtcccgga cgaactggaa gtctgagcag cgggggcggg
6481 agccagagac cagtgggcag ggggtgctcg gaccttggac cgcgggaggg cagagagcgt
6541 ggagggggca gggcgcagga gggagagggg gcttgctgtc actgccactc ggtctcttca
6601 gccctcgccg cgagtttggg aaaagttttg gggtggattg ctgcggggac cccccctccc
6661 tgctgggcca cctgcgccgc gccaaccccg cccgtccccg ctcgcgtccc gctcggtgcc
6721 cgccctcccc cgcccggccg ggtgcgcgcg gcgcggagcc gattacatca gcccgggcct
6781 ggccggccgc gtgttcccgg agcctcggct gcccgaatgg ggagcccaga gtggcgagcg
6841 gcacccctcc ccccgccagc cctccgcggg aaggtgacct ctcgaggtag ccccagcccg
6901 gggatccaga gaaccatccc taccccttcc tactgtctcc agaccctacc tctgcccagt
6961 gctaggagga atttcctgac gccccttctc ttcacccatt tcctttttag cctggagaga
7021 agcccctgtc accccgctta ttttcatttc tctctgcgga gaagatccat ctaacccctt
7081 tctggcccca gagtccaggg aaaggatgat cactgtcaga agtcgtggcg cgggagccca
7141 ctgggcgctt tgtcacattc caccgaaagt cccgacttgg tgacagtgtg cttcccttcc
 
7201 ctcgccaaca gttccgagtg agctgtgctt tagctctcgt gggggtgggt caagggagga
7261 tttgaagagt cattgcccca ctttaccctt ttggagaaat ggcttgaaat ttgctgtgac
7321 acgggcagca tgggaatagt ccttcctgaa ccctggaaag gagctcctgc cagccttgca
7381 cacactttgt cctggtgaaa ggcagccctg gagcaggtgt ttttttggaa ctccaaacct
7441 gcccacccaa cttgcttctg aaagggactc taaagggtcc ctttccgctc ctctctgacg
7501 ccttccctca gccagaattc ccttggagag gaggcaagag gaaagccatg gacaggggtc
7561 gctgctaaca ccgcaagttc ctcagaccct ggcacaaagg ccttggctac aggcctccaa
7621 gtagggagga gggggaggag tggctgcctg gccacagtgt gaccttcaga ggcccccaga
7681 gaaggacacc tggcccctgc ctgcctagaa ccgcccctcc tgtgctccct ggccttggaa
7741 ggggtatgaa atttccgtcc cctttcctcc ttggggccca ggaggagtgg agggtcccgg
7801 gagaatattg tcagggggaa ggcagggggt gtcatgggaa tgggtgaggg ggctgaggtg
7861 cagaatccag ggggtccctg caggagccgc agtggtaagc tgtccagctg gaagcctggt
7921 aactgttgtt ttctcttgag aggggcttcc tgtgaccttg gctgtctctg ggagcagggc
7981 tggggtacct gagtggggtg catttggggt gtgtgggaag gagagggaaa gaaagatgga
8041 cagtgggact ctcccctagc agggtctggt gttccgtagg ctagagtgcc cctctgctct
8101 gcgagtgctg ggcgggaggg gagttggtga gagctggaga cccccaggaa gggctggcag
8161 aagcctttcc ttttgggtgc tgtcaggtcc gcatgtcttg gcgtgttgac cttcacagct
8221 tctggcgagg ggaggaatga tctgatgcgg gtggggaggg ttagaggagg cctcaggcct
8281 aaggtggtgc agggggcccc ctaggggctg ggcagtgcca aggcataaaa gccttccctg
8341 gtccctggtg gcatttgaag gtgcccaggt gagaggggct tggcacctcc tcaccctggg
8401 agggagaaga aaccagggaa caggtaggag tgggagacag gtgaggcttt ggaaatctat
8461 tgaggctctg gagagatttg tgtagagagg aaaatgtggt tctcccccag ggtctcctcc
8521 tgggttttta ccctctaagc aacctgtggg catgctgggt tattcctaag gactagaaga
8581 gcttggatgg gggagggtgg ttggtgccct tcggtcctcg gcacccccct ccgtctccaa
8641 caccagctca ccctggtatt tgtcatgtca gcaggagaag gtcaccatgt tgtttttctc
8701 gcccctagtc cttccttcct gccccagtcc aaatttgtcc tcctatttga ccttaatact
8761 taccatggct ttggaccagg gaactagggg gatagtgaga gcagggagag ggaagtgtgg
8821 ggaaggtaca ggggacctcg acagtgaagc attctggggt tttcctcctg catttcgagc
8881 tccccagccc ccaacatctg gttagtcttt aacttcctcg ggttcataac catagcagtc
8941 caggagtggt gggcatattc tgtgcccgtg gggacccccg gttgtgtcct gttcgactca
9001 gaagacttgg agaagccaga ggctgttggt gggagggaag tgaggaggga ggaggggctg
9061 ggtggctggg cctgtgcacc ccagcccctg cccatgccca tgccttgctc tctttctgtc
9121 ctcagtggtc ccaggctgca cccatggcag aaggaggagg gcagaatcat cacgaaggtg
9181 agtccccctg gctgttggat ggggttccct gtcctctcag gggatgggtg gatggcctaa
9241 ttcctttttc ttcagaactg tggggaggaa ggggaagggg cacaggaata taaggatcaa
9301 gaaagaaaga gctgggcacc acgaggttca ccctcagttt cgtgaggact ctccgctgtt
9361 caggtctctg ctagaagtag gacttgttgc ctttttcttc tgctctttcc agtaaaattt
9421 tatttggaga aggagtcgtg cgcacagagc aggaagacag tgttcaggga tcctaggtgt
 
9481 tgggggaagt gtcccttgtt tcccctagct cccaggggag agtggacatt tagtgtcatt
9541 tcctatatag acatgtccca tttgtgggaa ctgtgaccct tcctgtgtga gctggaggca
9601 cagagggctc agcctaatgg gatctctcct cccttccctg gtttgcattc ctttgggggt
9661 ggagaaaacc ccatttgact atgttcgggt gctgtgaact tccctcccag gccagcagag
9721 ggctggctgt agctcccagg cgccccgccc ccctgcccaa ccccgagtcc gcctgccttt
9781 tgttccgttg tggtttggat cctcccattt ctctggggac accctggctc tccccaccac
9841 tgactgtggc ctgtgctctc cacctctggg gagggaaggc cctggggtct tccttcccgc
9901 gagtttccct gacctaaatc tggcgtggct gggtagtggc cagcagtggt gatgcccagc
9961 ctgttctgcc tcctccttcc ccaccccagg agccctttcc ttggcctagg acctggcttc
10021 tcagccactg accggccccc tgcttccagt gcgccactta ccccttccag cttcccagtg
10081 gtctctggtc tgggagaggc aggacaaagg tctttgtttg ctggagaaaa ggttgtctgc
10141 gataaataag gaaaaccacg aaagcctggt tgttggagtg tacgtgtgtg ctcccccagg
10201 cagtggaggc cagccctcct tggaggggcg gctgcctgat gaaggatgcg ggtgaggttc
10261 cccgcctcca cctcccatgg gacttgggga ttcattccaa ggggaagctt tttgggggaa
10321 ttcctacccc aggtcttttt accctcagtt accaacccct tgcccaggcc agaccttcct
10381 gctatcccct cctgggccac aagcctggcc ctcctctgtc ccaattgtga tgaaggggca
10441 gttcaaaact tcttgattag tcatcttctc ccctatcgac ttggctttaa aaaatgacct
10501 tttcagactt ctagtctcgt tcactctttt tgatgatgct ttgccgtaac ccttcgtggg
10561 tagagaagga ttctgtgccc attggtggtc tggataaaag aaatagagac ctcacaggaa
10621 gcagtggact ggcctgtttc cccactgttc tttctgtttt cacacctgtg gccttctccc
10681 caccttcttc ccaatcaacc tattgtgtac atagcccccc tcattgtcct ttattcttct
10741 ggaaagcaga ccttggaggg aggagtgagg gggaggctca gctgtggtct ctgggggtg
10801 ggggttggga gctggggtgg aagtccacga agcatacact taagatgctt tggtgaagtt
10861 ctaaacttca tattacccag gctgaaaaaa gagcacttgt tcctagggct ggaaatggaa
10921 gccaaaacac cacctttttc agcctgtttc agcatcttta gagatcagcc caacccactt
10981 acacagttga gcagagttgg aggcctagag aggggaggga ctggcccaag gtcataccaa
11041 ctcatggcca gagcctgggc ctcctcactg gccaggtgtt atttcttccc tctgggtagg
11101 gaacctattt cagggacagg attgctatgt ggtagtggtg gtggggtgcg ataggcgtgg
11161 caggctgggc cacaatttgg agtagtcatg ccagagtcct gcatttattt attctcaagg
11221 gccccgcctc tgtggcccag aattacccct tcatgctcca gtgcacccca ggcttcgtgg
11281 ccagcctggg aaactgtctc taccctggtc tcccttcaga tcagcttcta gaaatgtttc
11341 gtggctacag tggcagcact gttttttcca tgatgcaagc agtttgccct cttgggcggg
11401 gttatcagtg gctggcaggg ctggcacagc gtgtccgccc actgccacct gtgggttcca
11461 ggagggccca gcccctgtgc tgatgcccac caccttctca gctcatgtct ggggaagagg
11521 actggcaggg ggaaaggtgc ctcctcctga aaggtgcctc ctctgttttt gcctaatata
11581 ggcttgggaa cactttgatg tcagctaatt ctgactcctt tacttactag ctgtgcggcc
11641 ttggggcaac ttacttagcc tctttgagcc tcctgttccc catctgtaaa atggaatctc
11701 aatagtgtct aatagtacca tgtggagaaa cttgtgtgaa atgatagctg tggactactg
 
11761 tacacagtac tcaggatgta gtaagtgctc aataaacagc tgttggtatg gttgacgtta
11821 tggtagtggt tgtggggagg acgtaggaaa ctggagacta gcttggcaaa gctggctctt
11881 cctcctttta gggaaagctt agagcatccc catggggtat acccatactc agactgtcct
11941 ctggcatcga ggttggccca ggattcagtt cagctgtcac agtgaggtgg cgggatcaga
12001 tgtggcaggc catgtccctt ggaacttgag tacatcgtgt gatctctgga atgaaaacag
12061 gccttcacca gtgttgatgg tggaaagctt agggaagtgc ttcaaacaca gtaggaggga
12121 cttacgttag attttggaag gacttgcctg attcggaagc tccaaagagt ggcattacag
12181 agctgggtgg agagaggggc tagccatctt ttgtgtcgcc caccgggctc atgtgtcatc
12241 gcctctcatg cagtggtgaa gttcatggat gtctatcagc gcagctactg ccatccaatc
12301 gagaccctgg tggacatctt ccaggagtac cctgatgaga tcgagtacat cttcaagcca
12361 tcctgtgtgc ccctgatgcg atgcgggggc tgctgcaatg acgagggcct ggagtgtgtg
12421 cccactgagg agtccaacat caccatgcag gtgggcatct ttgggaagtg gggcaagggg
12481 gggataggga ggggggtaac actttgggaa caggtggtcc caggtcgttt cctggctaga
12541 tttgccttgt ctggctcctg cccctgagtt gcacagggga ggtatggtgg ggtcttgcct
12601 tctgtggaga agatgcttca ttcccagccc aggttcccag caagccccaa ccatctcctt
12661 ctccctgatg gttgcccatg ggctcaggag gggacagatg gatgcctgtg tcaggagccc
12721 ctctctccct ctcttggaga gagtcctgag tgccccccct tcttgggggc tttgtttggg
12781 aagctggatg agcctggtcc atggagagtt taaaaagtct tttggtgtta cctggtaatg
12841 gggcacatct cagcccagat agggtgggag ggagctgtga aacacaggga gggggttgct
12901 ttcgggtatc tactaggagt cagggtgaag cctagagagg atgaaagaag gggaggggat
12961 ggggagtggt aagaacctag gatttgaatt cccagcctgg ccaacccttg cagccatgtc
13021 ttggcctcaa gtggaacaag ggctccttga ggccagcagg gttgggggag ttgggtggg
13081 cctgagcctc tttcctgcta gagctcttgg tcctccctgc ctccaccacc catccctgct
13141 ctgcagaacc cctgggtgct gagtggcagg agccccaggg ttgtcccatc tgggtatggc
13201 tggctgggtc actaacctct gtgatctgct tccttccttt ccagattatg cggatcaaac
13261 ctcaccaagg ccagcacata ggagagatga gcttcctaca gcacaacaaa tgtgaatgca
13321 ggtgaggatg tagtcacgga ttcattatca gcaagtggct gcagggtgcc tgatctgtgc
13381 cagggttaag catgctgtac tttttggccc ccgtccagct tcccgctatg tgacctttgg
13441 cattttactt caatgtgcct cagtttctac atctgtaaaa tgggcacaat agtagtatac
13501 ttcatagcat tgttataatg attaaacaag ttatatatga aaagattaaa acagtgttgc
13561 tccataataa atgctgtttt tactgtgatt attattgttg ttatccctat cattatcatc
13621 accatcttaa cccttccctg ttttgctctt ttctctctcc ctacccattg cagaccaaag
13681 aaagatagag caagacaaga aaagtaagtg gccctgactt tagcacttct ccctctccat
13741 ggccggttgt cttggtttgg ggctcttggc tacctctgtt gggggctccc atagcctccc
13801 tgggtcaggg acttggtctt gtgggggact tgtggtggca gcaacaatgg gatggagcca
13861 actccaggat gatggctcta gggctagtga gaaaacatag ccaggagcct ggcacttcct
13921 ttggaaggga caatgccttc tgggtctcca gatcattcct gaccaggact tgctgtttcg
13981 gtgtgtcagg gggcactgtg gacactggct cactggcttg ctctaggaca cccacagtgg
 
14041 ggagagggag tgggtggcag agaggccagc ttttgtgtgt cagaggaaat ggcctctttt
14101 ggtggctgct gtgacggtgc agttggatgc gaggccggct ggagggtggt ttctcagtgc
14161 atgccctcct gtaggcggca ggcggcagac acacagccct cttggccagg gagaaaaagt
14221 tgaatgttgg tcattttcag aggcttgtga gtgctccgtg ttaaggggca ggtaggatgg
14281 ggtgggggac aaggtctggc ggcagtaacc cttcaagaca gggtgggcgg ctggcatcag
14341 caagagcttg cagggaaaga gagactgaga gagagcacct gtgccctgcc ctttccccca
14401 caccatcttg tctgcctcca gtgctgtgcg gacattgaag cccccaccag gcctcaaccc
14461 cttgcctctt ccctcagctc ccagcttcca gagcgagggg atgcggaaac cttccttcca
14521 ccctttggtg ctttctccta agggggacag acttgccctc tctggtccct tctccccctc
14581 ctttcttccc tgtgacagac atcctgaggt gtgttctctt gggcttggca ggcatggaga
14641 gctctggttc tcttgaaggg gacaggctac agcctgcccc ccttcctgtt tccccaaatg
14701 actgctctgc catggggaga gtagggggct cgcctgggct cggaagagtg tctggtgaga
14761 tggtgtagca ggctttgaca ggctggggag agaactccct gccaagtacc gcccaagcct
14821 ctcctcccca gacctcctta actcccaccc catcctgctg cctgcccagg gctccaggac
14881 acccagccct gcctcccagt ccaggtcgtg ctgagcaggc tggtgttgct cttggttccg
14941 tgccagctcc caaggtagcc gcttccccca caccgggatt cccagaggtt ctgtcgcagt
15001 tgcaaatgaa ggcacaaggc ctgatacaca gccctccctc ccactcctgc tccccatcca
15061 ggcaggtctc tgaccttctc cccaaagtct ggcctacctt ttatcacccc cggaccttca
15121 gggtcagact tggacagggc tgctgggcaa agagccttcc ctcaggcttt gccccctgcc
15181 ggggactggg agccactgtg agtgtggaga cctttgggtc ctgtgccctc cacccagtct
15241 cggcttccca ccaaagcctt gtcaggggct gggtttgcca tcccatggtg ggcagcgtga
15301 ggagaagaaa gagccatcga gtgcttgctg cccagacacg cctgtgtgcg cccgcgcatg
15361 cctccccaga gaccacctgc ctcctgacac ttcctccggg aagcggccct gtgtggcttt
15421 gctttggtcg ttcccccatc cctgcccacc ttaccacttc ttttactccc cccaccgccc
15481 ccgctctctc tctgtctctg tttttttatt ttccagaaaa tcagttcgag gaaagggaaa
15541 ggggcaaaaa cgaaagcgca agaaatcccg gtataagtcc tggagcgtgt acgttggtgc
15601 ccgctgctgt ctaatgccct ggagcctccc tggcccccag tacaacctcc gcctgccatt
15661 ccctgtaacc ctgcctccct cccctggtcc ttccctggct ctcatcctcc tggcccgtgt
15721 ctctctctca ctctctcact ccactaattg gcaccaacgg gtagatttgg tggtggcatt
15781 gctggtccag ggttggggtg aatgggggtg ccgacttggc ctggaggatt aagggagggg
15841 accctggctt ggctgggcac cgattttctc tcacccactg ggcactggtg gcgggcccat
15901 gttggcacag gtgcctgctc acccaactgg tttccattgc tctaggcttc tgcactcgtc
15961 tggaagctga gggtggtggg gagggcagac atggcccaag aagggctgtg aatgactgga
16021 ggcagcttgc tgaatgactc cttggctgaa ggaggagctt gggtgggatc agacaccatg
16081 tggcggcctc ccttcatctg gtggaagtgc cctggctcct cacggaggtg gggcctctgg
16141 aggggagccc cctattccgg cccaacccat ggcacccaca gaggcctcct tgcagggcag
16201 cctcttcctc tgggtcggag gctgtggtgg gccctgccct gggccctctg gccaccagcg
16261 gcctggcctg gggacaccgc ctccgggctt agcctcccat cacaccctac tttagcccac
 
16321 cttggtggaa gggcctggac atgagccttg cacggggaga aggtggcccc tgattgccat
16381 ccccagcagg tgaagagtca aggcgtgctc cgatgggggc aacagcagtt gggtccctgt
16441 ggcctgagac tcacccttgt ctcccagaga cacagcattg ccccttatgg cagcctctcc
16501 ctgcactctc tgcccgtctg tgcccgcctc ttcctgcggc aggtgtccta gccagtgctg
16561 cctctttccg ccgctctctc tgtcttttgc tgtagcgctc ggatccttcc agggcctggg
16621 ggctgaccgg ctgggtgggg gtgcagctgc ggacatgtta gggggtgttg catggtgatt
16681 ttttttctct ctctctgctg atgctctagc ttagatgtct ttccttttgc ctttttgcag
16741 tccctgtggg ccttgctcag agcggagaaa gcatttgttt gtacaagatc cgcagacgtg
16801 taaatgttcc tgcaaaaaca cagactcgcg ttgcaaggcg aggcagcttg agttaaacga
16861 acgtacttgc aggttggttc ccagagggca agcaagtcag agaggggcat cacacagaga
16921 tggggagaga gagagagaaa gagagtgagc gagcgagcga gcgggagagc gcctgagagg
16981 ggccagctgc ttgctcagtt tctagctgcc tgcctggtga ctgctgcctt ctctgctttt
17041 aaggcccctg tggtgggctg caggcactgg tccagcctgg cggggcctgt tccgaggttg
17101 ccctggttgc ctgagtggta ggctggtgtg gcttagtgta gtggtgtgga cgcaagctgt
17161 gtgttgtgtc ctgtggtcct tctgctcata gtggctgttg gtcctgatgt tattactacc
17221 tctggtagta atgctgagaa gctgaaagcc gattccaggt gtggacaatg tcaacaaagc
17281 acagatgctc tcgctggggc cttgcctcgg ccctttgaag tctgcatggc tgggcttctc
17341 actcactcag tgtttcttgc tgggggaagg aattgagtct cccacttcag actgggcctc
17401 cctgaggaaa gggttgtgtc tccccactca gactgaggtt ccctgagggt agggctgtgt
17461 ctctcccctc cgacctgggc tccctgatag ggctgtctcc ccgctcagac tgaggctccc
17521 tcaggccagg gctatgtctc cctcctcaga ctggggctct gagggcaagg ggtctggctg
17581 ttcgtttagg atggggcact tttgcctaca cactgaagga gctgtagcat ccaagaatac
17641 tagatacctt taatcctcca ccagtcatgg tgacaacccc aagcagccca cacattttca
17701 agtgccccca ggatgcgtgg agggaggggt ctgtgcccat tctcctgaca ttagcctgtg
17761 agctccgtaa gcccgggcct cgtttacgta cctttgtgag ccccgggcat ctgtacctct
17821 ttcctttgcc catactgggg accaaggaag tgtcaagtgc atgagtgaat gtgtgactca
17881 gttcagaggg tgaggtcagg agcacagggt cgggacaggt ggctggcatc ttttaatgcc
17941 ttagcttatg ttctttatac caacttggcc tgtgctcaga gtgagggagg ccctgggggt
18001 cagggtaagc gtcagtcagg gaggcaagac tttgtgggga tttcctagac agggccaagg
18061 cacccccagc tcaccccgag gctgtgttag ggaagtcctt ggagtgtctc ccctccccca
18121 gcaatgttct tgtggcttgt gtgtgctcag gggatgctgg gaaccaggcc tgggtagttg
18181 gtgtggggtg ctgtctgtct tggccctatg tgaaaccaag agggcgtata ttagtgctgg
18241 ggtgggggct ctgcctaact tcagggctgg atgaggggag tctcagttcc ccaggggtcc
18301 ttgggaaaga taaggacttt gacattttag ggtttttagg tgattattct gctgatgggg
18361 gtttgtgtga agtgacctgg gagctaactg aagttactct aacctcccaa tacctttacc
18421 caacccccaa gctggctgta tctgggaata tcagtttcca aaattggagg cttaggactc
18481 cgtttcgggg ctccccagaa gggtagggcc tgttctgcct ccttctcaca atcacccagg
18541 ggcaggggca tgctgagaaa gttcttggag gccccctttg cttcagctgg agtagtgaag
 
18601 ccgccgaatt gtctctcccc atcctaagtg aagcagcata tttgaaagga aagacaacct
18661 gttacctggg cctgcaacct ccaggcagct caagagagat gaggcctaca gccacagtgg
18721 gaggggacat ggggaatgga gatggtccct caccttcctg gggcctcctg ctctacgcta
18781 ccccctcggg agcctcctgt ccccagggca ggcccttgcc attgttggtc acccggccaa
18841 gcctctctgc ctcaggcgtt ctcccagaag atctgcccac tctcttcccc acaccagccc
18901 ctagagactg aactgaaaac cctcctcagc agggagcctc ttctgattaa cttcatccag
18961 ctctggtcac ccatcagctc ttaaaatgtc aagtggggac tgttctttgg tatccgttca
19021 tttgttgctt tgtaaagtgt tcccatgtcc ttgtcttgtc tcaagtagat tgcaagctca
19081 ggagggtaga ctgggagccc ctgagtggag ctgctgctca ggccggggct ccctgagggc
19141 agggctgggg ctgttctcat actggggctt tctgccccag gaccacacct tcctgtcctc
19201 tctgctctta tggtgccgga ggctgcagtg acccaggggc ccccaggaat ggggaggccg
19261 cctgcctcat cgccaggcct cctcacttgg ccctaacccc agcctttgtt ttccatttcc
19321 ctcagatgtg acaagccgag gcggtgagcc gggcaggagg aaggagcctc cctcagggtt
19381 tcgggaacca gatctctcac caggaaagac tgatacagaa cgatcgatac agaaaccacg
19441 ctgccgccac cacaccatca ccatcgacag aacagtcctt aatccagaaa cctgaaatga
19501 aggaagagga gactctgcgc agagcacttt gggtccggag ggcgagactc cggcggaagc
19561 attcccgggc gggtgaccca gcacggtccc tcttggaatt ggattcgcca ttttattttt
19621 cttgctgcta aatcaccgag cccggaagat tagagagttt tatttctggg attcctgtag
19681 acacacccac ccacatacat acatttatat atatatatat tatatatata taaaaataaa
19741 tatctctatt ttatatatat aaaatatata tattcttttt ttaaattaac agtgctaatg
19801 ttattggtgt cttcactgga tgtatttgac tgctgtggac ttgagttggg aggggaatgt
19861 tcccactcag atcctgacag ggaagaggag gagatgagag actctggcat gatctttttt
19921 ttgtcccact tggtggggcc agggtcctct cccctgccca ggaatgtgca aggccagggc
19981 atgggggcaa atatgaccca gttttgggaa caccgacaaa cccagccctg gcgctgagcc
20041 tctctacccc aggtcagacg gacagaaaga cagatcacag gtacagggat gaggacaccg
20101 gctctgacca ggagtttggg gagcttcagg acattgctgt gctttgggga ttccctccac
20161 atgctgcacg cgcatctcgc ccccaggggc actgcctgga agattcagga gcctgggcgg
20221 ccttcgctta ctctcacctg cttctgagtt gcccaggaga ccactggcag atgtcccggc
20281 gaagagaaga gacacattgt tggaagaagc agcccatgac agctcccctt cctgggactc
20341 gccctcatcc tcttcctgct ccccttcctg gggtgcagcc taaaaggacc tatgtcctca
20401 caccattgaa accactagtt ctgtcccccc aggagacctg gttgtgtgtg tgtgagtggt
20461 tgaccttcct ccatcccctg gtccttccct tcccttcccg aggcacagag agacagggca
20521 ggatccacgt gcccattgtg gaggcagaga aaagagaaag tgttttatat acggtactta
20581 tttaatatcc ctttttaatt agaaattaaa acagttaatt taattaaaga gtagggtttt
20641 ttttcagtat tcttggttaa tatttaattt caactattta tgagatgtat cttttgctct
20701 ctcttgctct cttatttgta ccggtttttg tatataaaat tcatgtttcc aatctctctc
20761 tccctgatcg gtgacagtca ctagcttatc ttgaacagat atttaatttt gctaacactc
20821 agctctgccc tccccgatcc cctggctccc cagcacacat tcctttgaaa taaggtttca
 
20881 atatacatct acatactata tatatatttg gcaacttgta tttgtgtgta tatatatata
20941 tatatgttta tgtatatatg tgattctgat aaaatagaca ttgctattct gtttttata
21001 tgtaaaaaca aaacaagaaa aaatagagaa ttctacatac taaatctctc tcctttttta
21061 attttaatat ttgttatcat ttatttattg gtgctactgt ttatccgtaa taattgtggg
21121 gaaaagatat taacatcacg tctttgtctc tagtgcagtt tttcgagata ttccgtagta
21181 catatttatt tttaaacaac gacaaagaaa tacagatata tcttaaaaaa aaaaaagcat
21241 tttgtattaa agaatttaat tctgatctca aagctcctct tggtttctcc ttctccattg
21301 aatccttgct ctagacttcc tcccgccccc tttccctctt cctctgggga acatggcatt
21361 tgtcttgggt ctgggaaagg tacgtctaat gtgtaggata tggggtgacc caccttgttg
21421 tgctgggggc aaagtccttc cattttggct gagctggtcc tgggggaacc catccatcct
21481 gtcttgatat agaaggtggg aagctctggg aatgggtgga gggggagaaa cagcttagga
21541 gccaagggcc cttgcaattg gtagtgctgc cttcaggaat tagatgatcc aggcccctgt
21601 cccttagcca gggaaagaac tggccatgtc tccaagcttg ctgcccagga gacaggagag
21661 agctgttttt gtctgtgggg gtcttgttgg ctgcaacagg ctgggagtgg gagggggatg
21721 ctgctggagg gctgtgactc cagggtgtaa cttaatttta catagtttta caccctggag
21781 ttccttgagc ctctggaaga tggacccata ggtttggtca ccactgatgg gacactccct
21841 gccccagctt gccataagct cttctctcac gtcctgctcc tgggtaaggt ggcacctatc
21901 caggtctttg acactgaagg gcagtgcttc caaattcact tcctctagcc tctcatttat
21961 tcttgcaaac catagatatg gcttgaataa atattgagcc agtcattgtg ctgctatgaa
22021 taagacacaa ttccacccct caaggatctg gtgaggatgg gtgggtgggg agacccaaaa
22081 ctataaatcc atgagcagaa aaatacataa aatgtgctgg gggcatctga tctagcttgg
22141 gagtgggagt tgtattgggg gtggtggctg gggggtggtg tcttatgaca ttatctctag
22201 gctgccactt aaagtatggt ttgaagacag ggagaacggg gcggcggagt gaaagggttg
22261 aggacatccc aggcagaagg gatagtgtga gcaaggcatg aaggtggcac ttgggcaggg
22321 gcagggagtg ggtgggcagg gtggaggctg gagacacggg caagggttag ctccaaggga
22381 gcctgcagca ccatgccaag agcctagggc ctgtcaggga tgagcagcag ccgaggggtt
22441 ttaaacactg agcggctcag tcagatagac ttttccatag gtgcctggca acagtatggg
22501 aggcagagag gccggtagag gctgttgtgg tcttccagga ggaattgtgc tggtggtggg
22561 gaagcacctt tggtgactgg cagtgccggc cattgcaagg tctcagcctc aggcagcttt
22621 tggtctagta agtgctcctt tcccaatcac tgaccacgtg ccgaccccta cattggttaa
22681 cactgaatct gcacagcagc cctgggaggt aggtgctttt tttttttttt tttttttttt
22741 gagatggagt cttgctctgt cacccaggct ggagtacaat ggtgcgatct cgggtcactg
22801 caacctccgc ctcccaggtt caagggattc tccctcctca gcctcccgag tagctgggat
22861 tacaggcacg caccatcatg cccgactaat ttttgtgttt tcgtagagac ggggtttcac
22921 catgttggcc aggctggcct caaacccttg acctcaggtg atctgcccgc cttggcctcc
22981 caaagtgctg gcattacaga tgtgagccac cgcgcccggc aggtaggggc tttttcatc
23041 ccatctggat ttttgctgga gcctttaaat tggccgcctg gcttctcctc tggccttcct
23101 ttagttgaat ctcacacaac tgccagagtg agcctgttaa aatgtgagtt aagtttggc
 
23161 accttttgac tcaaaacctt ccagagatag ttttactcgg agaaaaaccc aaatggcctt
23221 aaaaggcctg caccatttgg gctgcctccc tgactactca atgacttcgg gc
**Human VEGF-B Gene (SEQ ID NO: 81)** (NCBI Accession No. NG_029823.1) 
1 gccgtccccg ccgccgctgc ccgccgccac cggccgcccg cccgcccggc tcctccggcc
61 gcctccgctg cgctgcgctg cgctgcctgc acccagggct cgggaggggg ccgcggagga
121 gtcgcccccc gcgcccggcc cccgcccgcc gcgcccgggc ccgcgccatg gggctctggc
181 tgtcgccgcc ccccgcgccg ccgggctagg gcgatgcggg cgcccccggc gggcggcccc
241 ggcgggcacc atgagccctc tgctccgccg cctgctgctc gccgcactcc tgcagctggc
301 ccccgcccag gtacgtgcgg cccgacagcg cgcccgcccg cccgccgtgc cctctctcaa
361 ggttggcgga agtgaggagg cgacccgcgg cctcggccga ggggatctgc ggggtccggc
421 cttggacgcg ggcgtctcgg gggcccggcg cgtgcatccc ctgggaggtg cccctccccg
481 ctggcccgcc agccccagtc tggggccact cctccgcccc accccgtccc ctgggcaccg
541 ccgggggcgg ggctccctcg gtgcctgcag gacctgcttc ggtgggcact gtgcgcgctc
601 ctgatctgca tgcagccccc aaacatgctc ggctccgcgg cctggagatt tggcgcggcc
661 accagagcac ctgttgcgtt ttgggccagg gcaggtcccg ggtggccagg agaggacagg
721 taaagctaga gcagtggccg caggaacaga gcaatctgga aagatgtcag agaggtggtg
781 aagcctactg atgcaagggc aaagccccag ggacggtgac agggccacac cctcctaaag
841 tgtaccttgg gtacaggtct tttctctccc acaggcccct gtctcccagc ctgatgcccc
901 tggccaccag aggaaaggta atacttacaa aaactcggca ctagccagca ctaagagggt
961 ttgtcatggg ccctgattcc aggtgtccat catcttgtgt aactccccta gaagattcaa
1021 actattattc tacccatttc acagaggagg aaattgaggc agtggggtta ctgggatctg
1081 gataaacagg gcaggggaag acaggttgtg agggcagagt ggggagggct agtggagggt
1141 ggctgtgact tggggactgg ggaggacaga tgctgggagc agctgcagga aaaccagtga
1201 ggacttaacc cctaccggtc tgctcccagt ggtgtcatgg atagatgtgt atactcgcgc
1261 tacctgccag ccccgggagg tggtggtgcc cttgactgtg gagctcatgg gcaccgtggc
1321 caaacagctg gtgcccagct gcgtgactgt gcagcgctgt ggtggctgct gccctgacga
1381 tggcctggag tgtgtgccca ctgggcagca ccaagtccgg atgcaggtac tgggcaggtg
1441 gggcaacggg caggggatgc aggtactggg caggtggggc agcgggcagg gtggatgctg
1501 gctggctctg gggctggggc agcctggaga ctgatgcaca ggagacaggg ttggggggag
1561 ggctggtggc cagcctcccg gctgttgggt gagcttttct cccttcagtc ttagagcatc
1621 ccctttctct ctctccctca ctgtcccccc tgttcttctc ctgagcacag atcctcatga
1681 tccggtaccc gagcagtcag ctgggggaga tgtccctgga agaacacagc cagtgtgaat
1741 gcaggtgcca gccaggccca acttctgagc tcgcagaggc caggcttggg gggtgctggg
1801 tatggtggtc cacagaactg gggaccaggt tcctaagagt agaaccaagg ggccgggcgt
1861 ggtagctcac gcctgtaatc ccagcacttt gggaggccga ggcgggcgga tcatgaggtc
1921 aggagatcga gaccatcctg gctaacatgg taaaaacctg tctctactaa aaaaatacaa
1981 aaaattagcc gggcgtggtg gcgggcacct gtagtcccag ctactccgga ggctgaggca
 
2041 ggagaatggc atgaacctgg gaggcgtagc ttgcagtgag ccaagatcgt gccactgcat
2101 tccagcctgg gcgacagagc aagactctgc cacaaaaaaa aaaaaaaaaa aaaaaaaaaa
2161 agagtagagc caagggtagg ccttggatct ggggcaacaa agtaggatgc ttgggccggg
2221 ggtagtggct cacacctata atcccagcac tttgggaggc ccaggcaggc agatcacctg
2281 aggtaaggcg tttgagacca gcctggccaa catggtgaaa ccccatctct actaaaaaaa
2341 aatacaaaaa ttagccgcgc atggtggcag gtgcctgtaa tcccagctac ttgggaggct
2401 gagacaagag aatagcttga acctgggagg cggaggttgc agtgagccga gaccacgcca
2461 ctgcactcca gcctgggcaa gaagagggaa acacagtctc aaagagagag agagagagag
2521 agagagagag agagagagag taggatgctg ggatttcctg atcttcctct tgtttgtctg
2581 tgtctgtcta tcttactttt cagacctaaa aaaaaggaca gtgctgtgaa gccagacagg
2641 tgagtctttt ggactccagc tgagtagggg tatggggagt acaagtgagt ccagaagctg
2701 ttgctcctct ttctcctccc acccgtcccc cactttccct tttcctctgc tccccaagcc
2761 tgtgttctct gccccgaccc cagctctagg gaagactctt ccttcccacc ccagacatgt
2821 cgcttctcct ccctagggct gccactcccc accaccgtcc ccagccccgt tctgttccgg
2881 gctgggactc tgcccccgga gcaccctccc cagctgacat cacccatccc actccagccc
2941 caggcccctc tgcccacgct gcacccagca ccaccagcgc cctgaccccc ggacctgccg
3001 ctgccgctgc cgacgccgca gcttcctccg ttgccaaggg cggggcttag agctcaaccc
3061 agacacctgc aggtgaggcg tctgtggggt ggtgtttgtt tgggaaggca ccaaggccct
3121 gtcctggagc aggtccaggg tgagcctgcc agtaggagga gggccaggga aggggaaact
3181 gttgaatgtg ttgaacaaat atttaccaaa cagctgctgg cacctggagc tgtataagca
3241 agtccaacat tctaactcag gagtcagata caggagggac aatgccagca gaggatgtca
3301 agggctgtgg tgaggggtac ctggcctcgt ctttcagagg tcagagatct cttggaggag
3361 gtgacatctg cccggagtag aaatgtagga cacagctttt ccagcttgca gttcgggcct
3421 tcccagaagc ccctgtggta gacatctgcc cttacctacg ggcttcaaga ggccagggga
3481 catagtggaa cccttgtcca cccttacctg atgtgtttct caccatccgt gtcccgcagt
3541 gaggccatgg ggtgaaatca tatgggcagg gccagtgggg gctcccgagg agtgtcagga
3601 ggggatgacc tttgcaaaag gttcatcctt cgccctgcag cctccagtgc ccagggccga
3661 gtggtgtggc aggatgctca ggttgtgatc ttagtgggcc cgaggcacac atgagtccag
3721 ggctggggct gcgctccagc cagcatgaac agatcacttc ctccctcctc aggtgccgga
3781 agctgcgaag gtgacacatg gcttttcaga ctcagcaggg tgacttgcct cagaggctat
3841 atcccagtgg gggaacaaag aggagcctgg taaaaaacag ccaagccccc aagacctcag
3901 cccaggcaga agctgctcta ggacctgggc ctctcagagg gctcttctgc catcccttgt
3961 ctccctgagg ccatcatcaa acaggacaga gttggaagag gagactggga ggcagcaaga
4021 ggggtcacat accagctcag gggagaatgg agtactgtct cagtttctaa ccactctgtg
4081 caagtaagca tcttacaact ggctcttcct cccctcacta agaagaccca aacctctgca
4141 taatgggatt tgggctttgg tacaagaact gtgaccccca accctgataa aagagatgga
4201 aggagctgtc cctgcctgtg tcactgtttg tcactgtcca ggctggctgg tttgggcatg
4261 aatgtctgca tcactaaatc cagagcttgt cttgctccct cattgtgcag atggaggaaa
 
4321 tgaggactaa ggccccacag cagatcccag gcagggccag aattatgtat tcatcacttt

4381 caagttattg ccacgcatgg gagtcaggga tagcccagtc aatacagact gcctgccctc

4441 ctgctcttca ccagggttct tttctagaag gagacagcct tctgtggcca gagagcttgg

4501 ggtaggaccc agatctactg agtgaccttg cttgtcacta cccctgcctc tctgagcagc

4561 agtttccaca tgtgcacata gagggaacag aagattgctg tggttggcgt cctcggcccc

4621 cagagaagtt tgagactatc tttacgtaat agaaaagaac acttgttctt cctgccaggc

4681 a
**Human VEGF-C Gene (SEQ ID NO: 82)** (NCBI Accession No. NG_034216.1) 
1 acttcgggga aggggaggga ggagggggac gagggctctg gcgggtttgg aggggctgaa
61 catcgcgggg tgttctggtg tcccccgccc cgcctctcca aaaagctaca ccgacgcgga
121 ccgcggcggc gtcctccctc gccctcgctt cacctcgcgg gctccgaatg cggggagctc
181 ggatgtccgg tttcctgtga ggcttttacc tgacacccgc cgcctttccc cggcactggc
241 tgggagggcg ccctgcaaag ttgggaacgc ggagccccgg acccgctccc gccgcctccg
301 gctcgcccag ggggggtcgc cgggaggagc ccgggggaga gggaccagga ggggcccgcg
361 gcctcgcagg ggcgcccgcg cccccacccc tgcccccgcc agcggaccgg tcccccaccc
421 ccggtccttc caccatgcac ttgctgggct tcttctctgt ggcgtgttct ctgctcgccg
481 ctgcgctgct cccgggtcct cgcgaggcgc ccgccgccgc cgccgccttc gagtccggac
541 tcgacctctc ggacgcggag cccgacgcgg gcgaggccac ggtaggtctg cgttagggtt
601 tgcggagaac ccgagagttt gcgttagggt ctgcggcgga cccgagaacc tgcgcggggg
661 aaagtgtgtg tgctttaagc ttgtgtacgt gggatccaaa gttactgagc tcagtgcacg
721 ctgctttgga gaaaaatctt cttcttttta aatagaaagt tgttactaga gaggcaagca
781 agttacacga gtgaagggcc cggagaggtg cccagtgaga gatcccgaaa tctatttcag
841 actggtttcc tctggggcaa ccaaggggtc ttgaaccctg cccagtcagc ggggctctgg
901 agagtatgag ttcattttgg tcgggaaatg ctcgtttctt tccccagctg attcatggga
961 ctccaaacag attctgggac actggtgatc agtcaaccca gccttacttt cctggagtgt
1021 tcatagtctg cagagcacca ggcgctgtga gcgactttag aaaaaaagtg tcagggactt
1081 tagtaaccag gctccagagc tttcagagtt cacttgaagt tggtcagact tgagatgtaa
1141 cgggagatta gagtcagtta gatcacaatc aagcaatgca aggcttgttt caactttata
1201 agctcttcat tcctaaaatc tggtcatgag atactgcaaa ctaagttttt tttttcttg
1261 tttgggtttt ttgtttgttt tgttttcttt taaataagag gtgtttaatc ctttgcctga
1321 aaatgttgcc aaaatacttt tgaggcatcc tgattttgaa aaaggatttg tgtgtgttcc
1381 tacttcttac tggctcttcc aaaagagtat atctttatct aaaaagttca tacctgccat
1441 aaccatatag agtatgctta agagagttcc taaagaagtt atattcgaca tttcagttca
1501 aacacttgca gtaccccttg ctggaactat actgcggtag tttatttcaa ctggtggcaa
1561 gtggagaggt acctgtggtg tgttacagtc aactttaatt tgactgttga ttaacacata
1621 cacaatgtgt agaaaatagc cttatatttt gaaatatttt atttatattt atattttgaa
1681 atacacttaa agattgagaa gactaatttt tggaaatcaa actacctttg catttaaatt
 
1741 ttgggaaaac attaaaatgt tggaatgtgt aataatttaa tataggggtt aaaggaatgc
1801 ctcttgagta aaaacaatat acatgaaata gaacagactg cattctgtga atcacaaaaa
1861 taattttcag tgcctatact tacattgccg taactatagt gatgaaaata tctttctgtt
1921 cttaaatacc gcagaaatgt aataatcggc tcaataacgc ttctgataat ttgagtcctt
1981 gattttgcag tacctatttg ctatttctgc aaagtcaaaa ctagtaaaag tatgatttag
2041 taaggcaaca tctctgtatt ggttacggtc acttactttg gtttacagta agaaaatgtg
2101 cagtagagag taaaatgaga aacattccct gataagccaa tagcctgtat gagagtcact
2161 cactgctggg tgagtctaga attaatgtac cttgagtgtg tgtgtgtgtg tgtttacgtg
2221 tgttatgtat aggtatggga ttggaggatt aaaaaagtta acttattttt aactttcaaa
2281 agtgtcttcg ttggtgatgt aaaatttgcc attggtgata tggcaaattt taccattgct
2341 gtgaaaatca ctatgcattt ttgtttgcaa atgtcgcttt tcatatttaa ccatttttat
2401 attctgtatg gtgaatatgg gttagtattc tgcatgtgat gtgggttcct ttcctatgtg
2461 atgaaacatc atttcccata tgaaacatca tctctgagtt ccatagacca caaagcagta
2521 tttcattgct ccattaaagt gtcgttcccc agtgggtgaa tagagccttg acagggcatg
2581 tgtcattaat gcattcagtg aaactgctgt gtccttgcta tgaaacatgt cacacaactg
2641 ccaagaaact gatatgaaga ctctatgtct ctccctagtg gcctgtacaa gcaccacatt
2701 tagatttaag cactagagaa gaactgggga gaaatcaatt gttaattttc tataactaca
2761 tgaaccccaa gaaaatattt cagaaggtaa acagaagatg ctaaatatct ataatcccaa
2821 tagatgcaat gttttcataa ctttcaccta ggaacttttt taatatttaa aaaatcaaaa
2881 atagcacaca aactatgaac caaggttagc catccagccc ttcagcccac ccgtaaagcc
2941 atgcaaaaga ttgtaggtaa aaatcacaaa tagaatgaag atctccttat tcctcgactt
3001 atatttgcat ttactggatt cctatttcta gttatttgtg gttgcatgac ttactgaaaa
3061 gtcttaaaat gtttctctga aggatgttaa cacaaacaag acagtgtcaa aaaagtaagg
3121 caggcaaaag taaaattaac tggacatgat gggaacaaaa gagaatagca agtctgaagc
3181 tgttctattt acaatttttc ttatagtata tgattatgtt tgtcaacatc tactcttata
3241 agagatttct gacattcctc tgagtgatgc tacttatatg aggttttaga tatagaaagt
3301 tcaattcatc tttcattttg atttccactc cattatagtc ttctgaacta tgaatatctg
3361 attgatagct atttattcac aagacagtta tcaaaattgc ttcatagaaa gcagttaatt
3421 actgatctta gtggcatatg tctaagaccc gtagatctct aatcctgtcc ttttcagttg
3481 agttgagaaa gacggagggt agtggattta gatttcattt tggacttcca gttctggttc
3541 cggttcctct tgagaggaag gtcctggcct cctagttatc tccatgtgtc caacccagaa
3601 tgctgtttct cagctcttgc tggggttttc accaacagag gaactggcgt gagactaatg
3661 aaggtttccc gaatccctgg gactgtgctt gcctgcttgc ctttggcagt cctggggttg
3721 ttattgagag taggagttta taatcattat ctgtgatgtt cataacgtgt gttaagcact
3781 tcgcaaagag ctttgcaagt tagttttgtt tttcactaca cctccataat atggacatta
3841 ttttatttca ttaaggatga tgaacccaag atctgaatga cttaaccagg aaaaagaata
3901 gatgtttttt tccttttttt cttttaaaaa aagttttatg gcatatacca acagtccccg
3961 acttagaatg gttctattta ggacttttgg actttacaat ggtgcaaagt cgatagacat
 
4021 tcagtagaaa ccatacttca aggtttgaat tttgatcttt tcctgggcta ataatatgca
4081 gtaaaatact ttctctggat gctggacaat ggaagtgagc tgtgaccagt cagccatgtg
4141 atcgtgagag taaacaacag gtactctact gtatattaaa tatattttca actcaggagg
4201 aatttgtcag gatgtagccc catcgtaaat cgagaagcat gtatgtttta gagaaatgtg
4261 gtaatgggtt tgggcctttg ggcctgtgat tcagactcct ttcagcccct ctgctggtct
4321 cctttggcac actatagcag cgtgcaggaa ggcagagaaa agcactgcgt gtgactttgc
4381 agttaggggc accttgttgc atttatttag gtcctaattc cccacaacgg gacaaacgga
4441 aataaaccca gactttcttc caggctttac cacatggtgt tcgtttgact aaagataaat
4501 tataaatgac ctaatccatg gtaatattat aagaacgact gcttaactgg aggcttaaca
4561 agattttctg acaaacggtt tttcaaagtt gtgctcaagt aacttcagtg aaagattgtg
4621 attttacctc tgccaaatga cctttcacaa ataagtggat ttggaaagaa caaatttaag
4681 ttggcagaag cgttatactt cctctgactt ctgagcatat attgtttcac ctttcaccag
4741 ttacattatt ccttgtagat gtgcatttta taaatactat atgggtctac cttttgttac
4801 aaacacatat ttgtatgtac agatttgtat aaattgtgga ttaagttatc attgattttt
4861 ctcattagaa aaaacagata tagcagagaa ctttaaacaa ggagagatgc tgatgagttt
4921 gagttatagt gtaattataa aatttactat aaatatttta ggtttgtgta attgtttttc
4981 tgaaacttac tagtgtaaat ttttggggtg gcttctttcc tcggttgatt aatttttttt
5041 ttcagagttt atgtttaacc atgagaaagc agagactgtt tttatttaat ttgctttatt
5101 gggcatccat catggaatta atcaaattgt acattaaatg ctttgtcagt ggattagtac
5161 tgtttcctta tactgctcgc gccctagtgg ctttcaggtg ttaggagtta tccacgatag
5221 tttttttttcc aattttgaaa tccaaaaact catttctttt ttctttcttt cctttttttt
5281 tttttttttg agacagggtc atgctctatt gcccaggctg gagtgcagtg gcatgatgtt
5341 ggctcactgc aacctctgcc tcgtgggttc aagcgattct cgtgcctcag cctcccaagt
5401 agctggaatt acaggcatgt gccactagag caggctaatt tttgtatttt tcatagagag
5461 agggttttgt cttgtaggcg aagctggttt caaactcctg acttcaggtg atccacttga
5521 tctcctaaag tgctgggatt acaggcatga gccactgcgc ccggccccaa aagctaattt
5581 tcttaaatta tttggtgcta taatcctgat ctgaactgac ataaggctat ttatagtcat
5641 tattatctaa tttaaatatt tctaatcttc actgggaaaa tagtaatgta tttatttaca
5701 tgatgctgcc atcagtcctg cttgtttttg gggtgtgagt attaatatgt ctatgcactt
5761 ggtttccaga aaaattccag gttcagagac tcttgcagtc tccaggcatt ttggataagc
5821 agatggattg tagccctgtt ccacaggcct tcctccattg ctttattgcc ttggggctgc
5881 ctggaagagg cagggaaagg taatccagga attcaagaaa gaagccatgt ggatgctcct
5941 aattcctttt tacccattag taccaggttt atttgctctt taatctactt caactcatgt
6001 taatcagttc ttactagtgg cagcttcagt tctaagcagg aaattacagt tcagtgtgtg
6061 atgcaaaaaa tgtagagtga gtgatcttgt ctataacaag cttataatca ttttggagaa
6121 aataaattta ctcattaaaa atactacaag tgaattaaac tataaatcca gaccatgtaa
6181 gtggttttgt aagagagtct tgtgagttac caccagttgt tggctagcct tctgggagtt
6241 gcatgggcag gcctgggaaa ggtggaatga tatgagaccc aatgttaaaa atgagtagat
 
6301 gctaattaca gaaatggggg agagagggat gttttgaata ttgaagaatg acagcaaagt
6361 acagaggaag taaaggtcag ggatatttgg ggaactagaa gcaaaccctt tggtgcaaaa
6421 gtcaagagct ttaagggaaa ctgaagggag catggtggga agtgtgaaaa ccaaagagaa
6481 agtctgagtt tgagttgaaa gcagcaggag ccaatgaaga ttctttagtg gtatgctgaa
6541 agtgaggttt ttaaggagct taacctggca ttgttgcaca gaagtgtcta agaaagctga
6601 ccagaagctt ctcagaagct tctcagaagc cattttcaac tgcagggttt cttaaagtgt
6661 ctggcagtga ctctgccttt ctcattacag tggccaccca tatatggtat tcgaactcct
6721 ctatatgctc ctgattgctg gcgatgatag tggtgattac ttttgactta ctgtaggcac
6781 ccatcattgt gctatctgga tacatttaca aaagtataga aatagacatc tctgccatta
6841 tggtttgcat tccaaacgaa gcaagcagtg taaacagtgg tcatgcttag atacacatac
6901 acagaacccc ccacacacat attcatgcat acaccgctta atagttcttc agactgccaa
6961 gtgtgactta cgtgttcttt cttgcatggt taagaatttg tagacagttc attgaaatag
7021 aatatttaga caatcagaag tgtacaagat tagaatctct atatttgcac acacatttat
7081 ttgtaggtca gtgggcaatt gaaaaaaaag agaaggagag aaccctaaag tgtcctggaa
7141 attcctgctt tttaaaatct gctaaaaatg agaccagaaa ggtgggagtg gggatgtgag
7201 gaggtgggta aactaccaaa taaacaatat aaatactttg gctgttctta taaaaggttt
7261 ttttaaatat ggtggaataa tttactcaaa ctcaagagat gccacctact agagaaagga
7321 catactgaaa gaggaaatat tcaaatgcac aactttgtgt aaaaggtaat acttacaagt
7381 ttaaaagact cacttctaaa gaagtttggc ttcaacctgt cctcatttgg acaggtggaa
7441 agatgtatat tttggggtag accagaaaga tgtggtttga cttctatagt tgaaaggttt
7501 ctatttagac atttattttg taatttaatt tacctaaaac ttcatcccta aattaccatt
7561 ttctcttact tttatgcaat agtaaagtat gcagtcataa gtgataaagg ctgggatcaa
7621 agctagctat tttgctaact gcagaaccta tgcactctgt ttacactgcc tgtgatagcg
7681 cctggaagaa ctcactgcca atatttctgc ttgtttcagt cacataactg ttgcttgtgt
7741 cagtgacgta acttgtttag agatgcagtg cagtggataa gagcacaggg tatggacatg
7801 cctttctgta ttcatgtcct gaggatgcct cttagtacct gtgtgacctt ggacaagtta
7861 cttagtttct ctctgcttct ttgtagcaca atgagtagat gctaattttt aatttttaat
7921 tttttctttt tctttgtgtc tgttttgtgt atttagttac tttatctttt tttttttttt
7981 tttttttttg agacagactc tggctctgta gcccaggctg gatggaatgc agtggtgcta
8041 tctcagctca ctgcaagctc cgcctcccag gttcacgcca ttctcctgcc tcagcctcct
8101 gagtagctgg aactacaggt gcccaccacc acacccagct aatttttttt ttatattttt
8161 ttagtagaga cggggtttga ccatgttagc cagaatggtc tcaatctcct gacctcgtga
8221 tctgcccgcc tcggcctccc aaagtgctgg gattacaggt gtgaaccacc acgcccagcc
8281 tagttacttt atccttttaa aagtcctctt ccaaaatgta gaatataatt aatgcctgct
8341 taatcaggtt gttttgagga taagaatgtg tatataggcc aggtgtggtg gctcacgtct
8401 gtaatcccag cactttggga gtctgagtca ggtagatcac ttgaacccag gagtttgaga
8461 ccagcctgag caacatgggg aaaaccctat ctctacaaaa aatagaaaaa ttaactgggc
8521 atggtgacac gtgcctgtat tcacagctac tctggaggct gaggtgagaa gatggcttga
 
8581 gcccaggagg cggaggttgc agtgagtgag ctgagatcac cccactgcac tccagcctgg
8641 gcaacagagt gagactctgt ttcaaaaaaa aaaaaaaaaa aacagcatgt gcatacatat
8701 aaagcccttg gaaccatgat tggcatatag taagctttct gtaaatttgt gttaaataga
8761 tagtcttcat gatcagaaaa caaacaaaga atatagcatg taagtactat gtcctagaca
8821 tatgacataa ctgtgattct tcagcacctt gcaactaatt tattaaattt ttaaaattat
8881 aaattactga caattttccc aatttctaaa atatagcaag aggatataat actgatgttt
8941 gattttgaaa caggtgaagg aaatgcaggg tgtttaatca ttcagtgtct ggaggctaca
9001 tactgtagtt ctgcctataa tatgttccca cttcccctgg gtgccggggt ttgttcctct
9061 cctggagggc agcacaggtg tccatagacc acaccaaacc aaagaatctg ggatgatgct
9121 gtctccttat gtgcttcatt catacttagg tcatttggct gtaaaggatt ttggtacaca
9181 taagcttttt ataatgccat ataatttaat atgaactcat cttgaatata ttcagagcat
9241 atacattttc aagtttaaat gtttattaat gaaacatgct aatgttgcta ctttgatgtg
9301 taaatatctc atctatatta ttgccacaaa aaataaatat gcattctaaa tttacaatga
9361 aatcatcaca gaatacctaa aatatcaaca atttcactga ggaaaatata aagaaaactt
9421 aaggggagaa tgctatatgc cagagttaaa cttgtgtgaa tttgtttagg aaaatgtgat
9481 tgcactgttc ctaaggaccc ctcttccaga atggtaacta gtacagcgtg tgctgtctga
9541 gatgtgcgga cactgggaca ctgggatgaa ttatggagta gatactttcc ttctctgaga
9601 gtaagctgat ttgcttgagt actctagttt attccaaata gttgattttg tttggtggtt
9661 ctctttgtca atttgccagt cagtgctgtg acttgcgtgg ttaccctgtg ctgaagacta
9721 cagtgctggg ggcattggag gcccgtgagc agacaggaaa aagggaggaa aaatcagtgg
9781 tggacacact tcccagctct tgaacagctg acagcatatt tttttgattc agtaacatcc
9841 agaaagtatt cacttcccta ggattccagt gcagatgtta gttggagtat tttagaactt
9901 ttgaccagat accacatttt taaacagaat tttttttctg actggaactg gaacaaagca
9961 ctaaacacta aatagaccct caggtagtat gtaagtacat taaaaattag ataatactat
10021 attattcatt cacatctcta tggagatact gtatcatctt tttaaaagca attttcgtat
10081 taatatttgt atttcagaat tagaattgta tgtaagcata taataaaatg aagctttata
10141 gttcagtgct attatgtttt cccaattata gaattcaaat taacatttta atgtgatttt
10201 cttcatagta atttttcatt gtcacactaa tttatttttc tctcaacttt tttttattta
10261 aaaatttttt ttttttttaa tttgagacag gcctcactct atttcccagg ctgcagtgca
10321 gtggcatgaa cgtggctcgt tgcagcctcc gcctcctgta ctcaagtgat ccacctacct
10381 cagcttccca agtagctggg atcacaggca cgtgccaccc cacccagcta ttttttaaaa
10441 ttttttgtag aaatgaggtc tcactaagtt gcccaggctg ctctcaaact cctgggctca
10501 agtgatctgc cttggcctcc caaagtgctg ggattacagg catgagccgc tgttccagcc
10561 taatttcttt ttctcttaca agtgctttgc ttacattttt attgttagaa acaggattca
10621 aataactgct tatttaatat tctttttatg tagaagaaac agtcttgaac ttgataatac
10681 ttttggttcc aacttgttaa aatattggaa aaggatgaaa catttgtgaa aataatcaga
10741 ccttggcact gcggtagttg aggaagagtc atgcgtgtga gtgagtgcca ctctgagaca
10801 ggcagcactt tctcccttgt ggtaattgaa tagggtatgg gaagccgact acttaatctg
 
10861 cttcaggcaa gcagtgttca aagtttaacg cccttttact gaaatgacaa acataatcac
10921 cttacctaaa aggatgtgtg ggtaatgatt ttaccaaata gttcacttgg tgaacaatag
10981 tgaaaacagt tcaccttccc ttgtctttgt ttacaattaa atgagcccct ccaggtccct
11041 ccctgcccca agagtggatg aatttggcaa atcagactga aatgttaaag aaaatggtct
11101 tgactactct gttttaactg aagtgaccac acagctgcca tgaatcctgt gattgaattc
11161 ctataacctg taaaagggat tgttatttcc actcactcca aatttcttgg aagtagctag
11221 ttgatatcag ataccagatg cgaggctatt ttaaatcact gtgatttaat ttactataat
11281 atcattgcct acatggaata tttttaattc tttctttttg tttgtgtctg ttttgtgtat
11341 ttagttactt tatcctttta aaagtcctct tccaagttta tgttttgaaa aaagagaaca
11401 cttactgaga ttcatgtttg cttacaggtc atgacattta tgacttccct aaaaaacaaa
11461 ctgatccctt tgtctcagag ccaaatttta gttttaacat aatagtgatt cattgtttgt
11521 tctctctgag tcctagatct ctcttttaat tttggtgtct gtccatgcca aattagaatt
11581 ttattaaggc caaaattatt catgagtcat gaccatcaca cttaacaatt gggtaatttg
11641 aagctgcttc agatacacat taaagcaaat aatgatgtca taaattttaa tgcaatattt
11701 tttttccttt aatattggta gcagtgaagc ctgctctgtt agagaccaat tctgttttac
11761 tttagaaaaa tgtacctttg tgctcatgtc catgagagca cagtgtagtt attttattac
11821 tataatatag cacacagtga cacataactt agtaaattaa agattgaaaa tatttaaaat
11881 tggtgatttt ttgtgccatg ttgatttgac atttattctt ctatctgtct aaaactagga
11941 gcagtgctag tgtgacagcc tcaagtcatg ttaacacgac acacgtagaa gactgacgct
12001 cgggcttcct ctagtgcaaa acacctgaaa ttcaaatttt gcttttctgt gctattttcc
12061 taattaaatg tatccttgat gttgagttta actttattac acccctgtta aatatctgca
12121 atgtgttggg aaggaggagg acacatatgc ctgatttaaa tttaatataa ctgtatagat
12181 ataatttcat tttccattca atcattgaaa tgatataaat taatatatgt acagctaaaa
12241 ttaaatctgt gatgtcgaga tgaactccat gctgtccttg gtgcactgtg tattacacat
12301 gggtattaaa tgcgtgctta gtgcttagtg aatgggttag aaggagtttt tttttttttt
12361 ttttgagatg gagtctcgct ctgtcgccca ggctggagtg cagtggggta atctcggctc
12421 actgcaacct ccgcctcccg ggttcaagca attctccctg cttcagcctt ctgagtagct
12481 gggattacag gcacgcacca ccatgccagg ctaatttttg tattatttag tagagactgg
12541 gtttcaccct gttggccagg ctggtcttga acacctgacc tcaggtgacc tgcccgcctc
12601 tgcctcccaa agtgttggga ttacaggcgt gagccaccac acctggccta gaaaagagtt
12661 ttaatagata cccctgtaaa atgccaagca atcagttgcc attactaaga actgcaacaa
12721 gttccagtta aagatgtaca tgtttaagag ctgtcgttct ttaataaaat atgaccaaga
12781 tgtcttaaac atgggcttta atacatgtta ctgtgggttt cactccaact taatagcaca
12841 caaagttctg aagacacatg atttcactgg tggtacattc gcacattgtt ttctgtccat
12901 gtttaagttc attcttgtcc agaacagttt ctggttttct aagcctaacg tattaacatt
12961 tgagttaatt aaatcgatag ggagcctttt atcttttttt ttttcctatc tgggggcttt
13021 gctttcgcac tgacattagc agttcccttc ctatgtgcaa taggaaaatg tgcagtcatt
13081 ccaggagcat ggtctcctca ggaggttaaa agtgattgta acttagaggt taatgtttca
 
13141 ctccagagga aagtgtttca ccccacttga tgatgtgccc ctgggtgctc acagaaagag
13201 tagtactctt ttttaaacca aaattttaga cacgtgttat gcaaatcttc tttataaatt
13261 acacattctt ttctttcctt ttattctttt actccagtat actattttgc aattaaaaca
13321 ttttaaactt cttccagaaa aaaacttctt ccaataactt cttacttcaa actactttta
13381 ctaatgacaa aataactatg tccatcacag tgaggaatta ttcattgttt ataccacctt
13441 gtattactta tataaatatc atcctataaa aaatactata tattgggact agatttcttt
13501 aaatttaaat ttaaattctt ttggaaaatc aggttctatc agctctgttg tcttttcatc
13561 tctaattgta catcttccca ctattttgat ttatacatga tgttattata ttaggatagc
13621 tttgatttta atgtaatatt agctagtttt atggcttgaa aaacttagta catagactat
13681 attcataaat acagcactat acattctagc atatggcgtg tgcacttaat ctgaagaatt
13741 agacattttt tcctgttctg tgatgattga ttgattggtg gtgggggcag aaggtggcca
13801 gtctttggca acatgtaaaa ttttgttaaa attttgtcaa actgatttat tccattgcta
13861 aattttgaaa tcagcatttt tttaggggga atatctcgag tttatgagga cagaaagaaa
13921 tattcttaga aaattgagaa ggttttgtcc ctgtacatgc aaactagggg gtgtttagat
13981 cagagcaaat gtcttgcagc aggggttgac gccgggccat gtttagtcat caaagcatga
14041 cttctattct gggaatgtgg taattgagag gtaaatatta tttacaatag cccttataaa
14101 tctttagttg ttcaacaaaa taacttgcca tcagagttag atgatttagc taaaattatt
14161 taattgggtt attcctactt gatccaacat ttgattataa tatatgtagt agcgttaaat
14221 aatttgggat ttctggcctc tggctggctg ttgaagactt tttgttggtg gcgggggca
14281 tcctaaggaa caacttgccc tctactagca ttctttacta gaaaaagctc ctatttactt
14341 gttttttttt tcctaaatat gtgttttatt tccttttggc attgtctttc ttgctcccaa
14401 ttataattgt gagcaaatag caatcgaaat tctgtgtaac ttccagcaca gtttagtcat
14461 cagagcttgc cagcaagtat gtctattgtg aggaaactgc atttgccaca gctagaccat
14521 cagcaagtga agtcgtattg tcctctgttt tgtagttgtt ttttcctatt tatgctttac
14581 tctgattcaa gttcagcatc tcagtgatca taattgtggg gaaaaaaatt catttgaac
14641 tataaacaga aaagatacca agttgttgta tttgaaagat tattttagaa tgtggttata
14701 ccttcaagta aaataagctg taaaataagc tgaagaacca tatatttagg cacattttga
14761 ctataaaata gatcctctac atataacaag aatagctttt tttttttttt tttttttttt
14821 tttttttgag acaaagtctt gctctgtcgc ccaggctgga gtgcagtggc acaatcttgg
14881 ctcactgcaa gctccacctc ctgggttcat gccattctcc tgccccagcc tcccgagtag
14941 cagggactac gggtgcccac caccatgccc agctaagttt ttgtattttt aatagagaca
15001 gggtttcaat gttaaccagg atggtctcga tctcctgacc ttgtgatccg cctgcctcgg
15061 cctcccaaag tgctgggatt acaggtgtga gccacggagc ctggcctata tataacaaga
15121 attgctttta tatgtctata tgtcacttca taatatttgt ttattattta gctagttatt
15181 gcctataagc cagtatttag gtattatata aaatctgagg aaaacagttc tataagattc
15241 agtaaaattc caattctata agattccaag atgtaaagtg ttttaggtat ttaaaagttt
15301 tatatagaca atagtgactc atttgttatg gtatatgaat atagacaaga acttagactg
15361 aagcaaaaaa taacaattag ttacatagct atttcaaagc ccatgcagta gttttttgtt
 
15421 tgtttgtttg agacggagtc tcgctgtctc ggccaggctg gagtgcagtg gtgcgatctc
15481 ggctcactgc aagctccgcc tcctgggttc aagccattgt cctgcctcag cctcctgagt
15541 agctgggact acaggcgccc gccaccacgc ccggctaatt ttttgtattt tttttagta
15601 gagatggggt ttcaccatgt tagccaggat ggtctggatc tcctgacctt gtgatccacc
15661 cacctcggcc tcccaaagtg ctgggattac aggcgtgagc taccgcaccc ggccagtagt
15721 ttttaatgtt cagtgaagtt aaaacatctc ccgtccagtt atgctgtgta ataaatatta
15781 aagcaggaga tattagaaca atacaacact tactgtgcta tttataattt aaaatattaa
15841 agataaataa tggaataact ataaatattg atactatgat aaaactttta gcatgtctaa
15901 gttgttcatt ttgatagata caaaaaacca aagccaagca aaataaaaca cacctaaaac
15961 tttatgaatg ttaacaattt tctatttttg ccataaatct ttggccgttg caccatattt
16021 tattgaataa aggacagttg ttaagctagg gcttggtcca ctgtgtgtca gtatcaggat
16081 gtgatgattc ttttctgacc catattcttc tctctctttg gctttggttc tcttgtacga
16141 ctcttctgta ggtggccttg gccacacctg tgaaatatag ggcacagttg tgctgcttct
16201 actcattgtg aacttgatca tatgttttgt attgtgcctc tcttcattgg ctcctttta
16261 ttttagtgta attcaaagtg ttaattggag gttttaaatg tagttttgac caaattgtgt
16321 ttgaggtcac atgtacccag tttaaaaaac gaagaaaaaa attcaactca cttcccagcg
16381 tcctttgcag ctaatgttgc cctcgaatag ttcttgctca taagagaaaa tctagcaggg
16441 atttatggga aaacttttgc ttttttttgc taaggcactg cttttcttct tgttccctta
16501 tttccttcca tggaaatcag aaaatgtttt ataattttaa aactttacct ttgtttctca
16561 aaataacact ttcagactta tgtaaagcaa gattttaata attaatatgt tgcctcgatt
16621 atggagtaac tttccaaagt tgtactatca tgatcttaat ttattgaccc ttagggttaa
16681 gataatttgt gtgtttataa cctaacattc tttgcttggc tgatagattc ctttaaaatg
16741 ttatgtgttt atattttgta catactttaa gaagctttat tcaattgcca catttcaaat
16801 aaataaaggt tctttttttt ttggtaagcc tatacctttg tttcttctag cttttaaaag
16861 tgttaatagc gtgggaatga cccttgacca tctctgattg tttgggaaat ggtaactaga
16921 aagaactgag agaggacaaa cttggatgag aagtctcaga actgatagta aaaacaaatg
16981 caaacaagaa aatatattac gtacttcatt cagattaata agtaattaaa cacttgaact
17041 attcaaaaat ctagtttgag tctaggcttt tgcaagcctg aatttgtcat cataatcatc
17101 aaaattagaa attacgaaag aatgttaatt taaatttaaa acagctggaa atacaataca
17161 tggcctataa gcttatcttc tcatctgtca acaactgctt gcatatgtca catgaactgt
17221 tttaactgca attacaaaag tcaatggaat gtttagaggt gattcaaaaa atgtgattgg
17281 ctgacatata gagttatcct aagaaacagg ctgtccaaca caattttaaa tatactcaag
17341 ctcttttata ctgattaaga gtttcaagtt aaggttttgg gttaaaaaat caacatcaaa
17401 tgataaataa gaaatataat cttgtttaaa ttatttattc taatagttta tttatgaaca
17461 caacatattt ctctttcatt actttttttt tctcagttgt aaaattctag agctggagcc
17521 cataggccaa tccgtagtat atctgaaatg attaaaggtt ttatacttga caagccctct
17581 gtctgcaggt tacttgcttc tacttggagc agtggattta tcatcctcca ctccagggta
17641 aatatctaaa agtcacttct gccacgttat ttcccatctg gtagattgtt ataatacctg
 
17701 tgagtcagta ttatctgcac agtgtatttc ctctgctcaa ggcactttga ccatatgagg
17761 tgttcacatc tgatcaatcg ggctgaagct tgccctagtc tgaggctgga ccaattgatc
17821 tctaacgata ttcttacctg acaatctaga ggatacaaat caagatgttt caagtgcaga
17881 aagcaacagt aaagcataag ttcttttttt ttttttgcat tctttttttt tttattatta
17941 tactttaagt tttagggtac atgtgcacaa tgtgcaggtt agttacatat gtatacaagt
18001 gccatgctgg tgcgctgcac ccactaacaa gcatatgttc taatgtgtaa tatatcttca
18061 agacagtctg gaccatgaag tgtattttta cttattgagt gtatatggga ggatcatttt
18121 ataggattgg ttatattttt ccatttcttg attctttgat acttaaattt ttggtttcat
18181 attctcctct ttcacatgtt gtccccattt tacatatgca aagatagttg tttgccacaa
18241 aaataaacat ttaacagact ccaaacacat agactgtgta attagcataa cgaaataata
18301 gtggctgagt gacgagcaga agtgattcac tcatttggct ctgtggcttt caaagttatt
18361 aatgatgtta catattcttc tctattttat ttaaaaacta acactcataa agttaattct
18421 cataattttt gctgggttta ataattcaaa atatgaatca aaatttttat ttattatgca
18481 gtttcattct attaaaatta tctgctaaat taatattaag tagtcctata gcatatatta
18541 tttaataatt gcaagtagtg acatatcata aataaactgt ataatatgta ttattgattc
18601 tgttattttt cctagcaatg cacagggaac cagtaaattt cacaagcaga gaatactaac
18661 ttgtcattta tttaatattc taaacaaatg aagccgcctc tataagtgaa ttttctggac
18721 ttctaaagat gagcattgtt gagtttaata actcaaattt ttattgtgtt aagtaaagta
18781 tattaaatat aacctcaccc taatgactca gctgtaatta aaaaagagtt cacgaccagc
18841 ctgggtaaca cggtgagacc ccatctctac aaaaataaaa aataaaaatg aaaattaaaa
18901 aaaattagcc aggcatggtg gcatataccc aagtactccg aaggccgagg gtggaggatc
18961 gctcaaacct aggagtccaa ggctgtagtg acctgtgata gtgccactgt actccagcct
19021 gggaaacaga gcaagaccct gtctcttaaa aaacaacaac aaacctacac atgaaaatta
19081 ttgctgcttc ccatcaattt tcagtgctat ctttatttaa cctctctact atttgccttc
19141 actttttaaa attttgcttt tattcttctt tctttggtct ccttctctaa aatttttccc
19201 ctgcttctgt ttttttgtat actaggaaga catttattat ttttataggt atgatcaaat
19261 tgtaaaataa gtttatttca ttctctggtg atttcactaa ctattttgag agtatcagtt
19321 aaaatgggat ttgtttgtgt gtaacaaaat ctaactgtag tggctaaagc aaattggagt
19381 ttatttttct ctagtaacta aaaggcccca gtgggcgttt aagggctggt accacatgga
19441 gcaacatcat atcatcaatg tccagactcc tgtgcttttt tcacttgatc atttttagag
19501 tttggccttc tgtgcttcat tcctcgtggt cacaacatgg ctgattgttg tgcagcttta
19561 tgtctgattt caaggaagaa agaatagtga gagaggaggt tgttgcctta acacattcaa
19621 accaaagcaa aagttttctc tgaaatcccc agtagatttg cccttacatc ttctcaggta
19681 gaagtgcatt tttgccattc ctggctgtaa gagaagccga taaagaaaat acatgcactt
19741 tctaacaata ttgaaattct tttcacaaag aaggaaagga gaatgaatgt actttgggta
19801 ggtatcgtgg tctgttttat gttgctgtaa atcacaaact ggatgattta taataaaaag
19861 aaattagttt tcctcacaat tctgaagact ggaaagtcca gggttgcagg gctgtgtctg
19921 gtgagggcct tcttactgtg tcttctcatg gtggaaggca tcacatcagg agagatttct
 
19981 ccagagtgca agaacaggtg gaactttata taacaaactc attcctgtga taaggagccc
20041 actcccaaga taataccatt aatccattca ggtgggcaga gcctcatgac ctaatcacct
20101 tctgaaggtc ccacctttca atgcttttgc attggggatt aaatttccaa cacatgaagt
20161 ttggggaaca cattcacgct gtagcaggag gcaaccacat ttgtgacatt gcgtcattgt
20221 actttctaga tttgttttct ccaggttgtt tgcaacgttg atgtactctt tgtataccca
20281 cctgaaagat taatgttact gaagtattgt gtttaagaaa gaaagatgaa tgctaccatg
20341 taagaccttt tgactgtcca aaagattaaa attccagttt ggagaggtag cacttacatg
20401 aaaaccttat aaaacatgag aacttaaact tgctaatttc attgtgtcaa atcgcataac
20461 agagaaagga aaaacttgaa ggccactcct cttgaaaaag tatttgagta gtgtcgattt
20521 cagtgtaaga ccaattaaat aatgtcaaat taagtaaata taacattaaa atatttcttt
20581 taacatattg gtgttgattt cacttttact ctgtaatttt tgtgacattg gaaacaggaa
20641 atattcaaat tttgccttct tttggatcgt ttcctctccg tttcttaatg tgtaaaccgt
20701 aatagggctt tctgcagcca acgtgctcat tcttttcttt ctgttaggct gtgccttttc
20761 tgagaacagt tggtactgat ggagatttct gttctttttc ttcacacaag tgtcatatga
20821 aaattggggg atcagctatt ttcagatcac aggctcctct gataacagaa taccataatt
20881 ccaactttcc tgtgttgatt tgtagatccc acactgtgtg atcatgacaa acatctaagc
20941 ataagagggc gatgaatgat taaaatttat tcactcttcc cataaaacac ctggtacaga
21001 tgcagtgtgt tgtcctcaca gaggaaaaac agtggttcct ccttccaagt ggcgtgtcca
21061 cagcactggt accagataac aagaacctta gactttcttt gttgctgcag tgtgtctacc
21121 tgtgcctctc ttaagtgtct tacacgttac tcacagagaa agaaatacaa aaatggaaaa
21181 cataagctca caaaaatgca tttagtgcat ttatgatgtt aatggaaacc ttggatacag
21241 tcctctgtta tttccttact actcttgata ttttacaata ttgctttatt attcaaattc
21301 ataatcatat atgtgttaaa caattttttt tggtaagtag actgtgtatc aaacttaaga
21361 gggatttgac atggaaagac atttaaaaga tggcagaaaa cagctattat aagggcattt
21421 gaactttaga tattttccgt tggtcagttt ccatagatca tcatctgatt cactagggtt
21481 tggccagttt atattattta aattgtttcc cctgcaactg tagtattttc tttaatgttt
21541 tgaattattc ttaatgtgac ttgaataagg gtaattgtca catatgctga tgttttccca
21601 ttaggatgaa aatatttaga aaagctgtac tagcaaaaac caggttaact tgttttcaaa
21661 gtagaattta attattttat ttcaagcttt gattcctttt ttaaaaatta agtaaatgtc
21721 cttgagagca atacctttgt gtgtgtgtgt gtgtgtgtgt gtgtgtatca gttacttgca
21781 ccttcaattg aatgaaagac agtagtaggt tgtattatgg cagaggggta gaagaatgga
21841 cattctcatg ggtatctcaa aggtcaagtc agaattggat tccctgtgga tggaagagag
21901 gagtggagaa ggtgcttctc atgtggaaaa acaagaaaag tctatgacag gcctcagtgt
21961 ttgccagctg cagtggaatt ctcatagctt gtattgaact ctgtgtccta atcttctgaa
22021 accacctcaa gagagtatat attgctaaaa taggagctca gctatttttc aggggcagtg
22081 atccagagga cactataaca gaaatagata atttcaaagt cgaaaatata ttagatacta
22141 tttttattct ctatctggaa agtctgtata gaggctttat tctggtctgc tttctcagat
22201 aaatatccta ttcaggtcac tggaaataat atgtcaggtt ttaaaagaga cttcatatgt
 
22261 agccaaataa acctgctaag catatttaga aaatcatgct agtgactagt gtcttaaact
22321 gtttccacaa aaacaatgct gttaatagta ggatttaccc ttttgcattg gaaatgagtt
22381 gtcaaggtta aaaagatgag tagccagata ttcttgctaa acatgttttg agaagaaatt
22441 taataatata aagtttttgt taaatgtggc ctattaattt taaacaataa tactgactga
22501 agtcattagg tagctgtgga aagaagttag cctggttttt actaatgtag cttttctaaa
22561 tattttctgc ctaatgagaa aagagggata agatccaact acaattggaa gcttaaatat
22621 ttaatcaagt ttgcagaact actattaaga tcatatgtaa ctatcagata tttttaatc
22681 tgaggacttc tttctagtaa atagaaattt cttcccaagc attataggaa tatgtcttat
22741 tgacttattt ttttggcatg ggttactagg aaataatatt ttcagggagt tcagggaccc
22801 agatttgatt cccagataat tcagattaac cttctttaat cattctattt tatgttatta
22861 ctggagagta cagggtaggt tgaaacagca taaactgcca aatcagaact ggatttgaat
22921 ttagtgatgg gcctcaggag ctgtatgacc ttaattctgt gagtcaggct ttctgagtca
22981 ggctgtgaac tgggcaaaat agtgtatacg ttacaacatt gtaacagagc tcatttaaag
23041 cattgtctca tgttagctac tgataaagca ttagttccca ctccagggcc tgttagaatt
23101 ctaactccat ccctgtgagt catgttcatg ctttcgtggt tctcatttga ggtttgtga
23161 ccaaaggccc cacagatgga ataaaattaa tctaaatagc catttattaa tgtaatatct
23221 gctagctaat tacatattta ttaaatataa ataacaaata gctccaagat accagtgacc
23281 tccatgttgc taaagccaac agttattaga acttctctgt tctgtgagct tttctgtgat
23341 ggtcacaggt gagaccgcaa gaggacacac aggagaggca caatgaaaca aagtttatta
23401 tactcatagg tcttagacag gaggtcaccg catgccacac agggtcacag ggtgtcttag
23461 tttgttcagc ctgctataac aaagtgtctt agactggata atttataaag aacagaaaat
23521 tcattgttca tagttctgga ggttgggaag tccaagatca aggagccagc agattgggca
23581 tctgctgagg gcccactctt catagatggt gccttgttgc acgtccccct cacatggtga
23641 taggggtaag gaggaagcag gctctcccaa gcctctttta taagggtacc aatttattct
23701 tgagggctcc accctcatga cctcatcatc tcccaaaggt ctggcctctt aataccaaaa
23761 cgctggggtt gatattttaa catgtgaatt ttggagggac attgacatta cagaccacag
23821 cacggggggt ggggggtggt tctccatgtg gcagaagaca ggaaagagga gagagcttat
23881 gccacagcct ttactggagt ttcagggtaa aggcaaggca gagtagagta gggtaaacag
23941 tttagaactg gctggtttga atactttcag taggctctaa ggtataggat taatccctag
24001 ttgcttgata cctgacagtg ggaaattaag gcagaggaat attgtctcct ggggtatata
24061 gccagataaa ggaggtatgg ctctggattg ggtaatttgc ataccaaagg tgttctccct
24121 gctgaggttt ttgctgtctg taaggattgg cagcccctgg agggccatct ctcccaagcc
24181 ataaactttt ttttaagatg tcgaaatatc atagtactca gaaaaatttg aaatatatat
24241 atatatatat atatgtataa aacatcctct tacttggcag catttacaat gtttgatcat
24301 tatttttcag ctttgtcttc aatgcactca ttttacctca gtggtggtta ctccatccca
24361 ttctccactg ctggctactc tccttgaact tttaatgtcg aagtgttcag ggctcagccc
24421 actgtcctct attatttttc atttctattc actctcttag tgatttaacc cagtcccatg
24481 gttctagata tcttcatatc tgtatcttta gtccaaatct gtcttcttaa ctttaaaata
 
24541 taactagctg cctttgtcat ttccactcag atggctaaca gacacttcaa atttaaacat
24601 tcccagttga aatttttatc tctccgtttc tgatcaaatt tgctctagct ctacctatag
24661 ccttccctaa ctcagtcagt ggcaacaaat tgctccgagt tggtcaagtc aaaattcatg
24721 gtatcatcct tgacttcttt ctttcctacc ctacattcag actcatcagg gaatccaatc
24781 ctcttggctg tgtcttcaaa aatataacca gagtctaacc acttgctaca tctctgacct
24841 gagccaccat cgtcttccac ttggatcagg gcagcgttct gtttaccagt ctcactggtt
24901 cttatcttca cctccctaga gtgtattctc aactgagcag aaagaagaat cctttagga
24961 tgtaagtcac attatattcc tctcctgtta tatgtaaggc cctagaatag ttccctactt
25021 cacaaaaagt aaaaaccaaa atctctatca agtaatagta gtcattgccc tggcagtgtt
25081 tcaattaaaa gagtttaacg actggttcat tttatagagg tgtggacagt gtcaaggaaa
25141 cagaaaagaa caaaagaaca tgaagcatcc aggtactatc aacagtggga agtcctaact
25201 accctagtcc tgcaggggca aggtgagcga atagtgctat cagccctgga aattgctggg
25261 cactgtagga gaggggctgt caatagaagc tctaactgta gagtgcacac acttctctca
25321 ctatggtgag gcaccataaa tgtggtgagg cagagggaga gtgaggagaa tgaatatcca
25381 acctattttt tcttcacatc ccagccaaat acttggcatt gttagtcttt tttattttag
25441 ctattgtggt ggtgatgtaa tagcatccca ttgtggtttt attttgcctt ttcatggaaa
25501 ctgatgaagt tggacatctt ttgacaagct tgttgcccat ttggatatcc tcttttataa
25561 aatgcctgtt taagtgtttt gttcattttt ctttttggtt gttttttttt tttttttttt
25621 ttagatttct acaattcctt tacatgattt ggttgccagg ctttttaaag ctgtgtgtat
25681 tgcagatatc ttcttccatc tcttactggt cttttttctt tctttctttc ttttttttta
25741 ctggccagat ttctttaatt ttaatgtaag gtgatttttt cttgtttttc ctctatgatt
25801 aatatttttt atgtcttgtt taagaaacat ttgtctatcc aatgttatga aaatagtctt
25861 gtagaagctt tattgctttt tcactttcag atttatattt acgatcaacc tggaattgat
25921 atttgtgtag tggggagggt agaaatcaag actagttatt tttaatttgg ataactaggt
25981 aacctagcat atttctctgc tgctctgtag cattaccctt gtaaataaat catgtctcta
26041 tacggtttct ttctggactc tgttttacga ttgtatgctc tctggcatta gtatcacact
26101 gcctgaaatt ctgaagttcc atagtatctt ttcttttcct tgtttttttt tttttttttg
26161 agacagagtg tcattctgtc acacaggctg aagtgcaata gtgcaataat ggctcaccac
26221 agccacaatc tctcagggtc cagcagtcct cccacctcag tctcctgagt acctgggact
26281 acaggtactc accactacac ttggctaatt tttgtgttct tctgtagaga taaggcttca
26341 ctatgttgcc cagactggcc ttgaactcct gggttcaatt gatcctcctg catcagcctc
26401 tgaaagtgct gggattatag ctgtgagcca ccatgcacag cttcattgta ccttaatatt
26461 tgacagaaat acctacagtt tgttctttaa tatcaccttg aatatagata ttgagattcc
26521 attgaatcta aaaattgatt tggggaaaac tgatatatta gcaataatgg gctttctatt
26581 ttggccttat agcatttatc ctattatata atcattttta agtttttctt tttgtgtaga
26641 gaccttgcat atcttccttt tcggtaccta ggtatgagac attttggaag ctatggcaaa
26701 ggatatttgt tttgcatttt cattgtcttt ttttttctgt gtatagtatt ctaggttggc
26761 atttattttc tatattctaa agattttttt ccatattacc ctctgttttt tattacatcc
 
26821 actatcaatc ttattgttgc ttctttgaac aagaaatgat tttcactgtt agcatttaaa
26881 attctgtctt tggggccggg cacggtggct cactcctgta atcccagcac tttgggaggc
26941 caaggcaggg ggatcacgag gtcaggagat tgagaccatc ctggctaaca cggtgaaacc
27001 ccgtctctac taaaaataca aaaattagcc gggtgtggtg gcgggcacct gtagtccagc
27061 tactcgggag gctgaggcag gagaatggcg tgaacccagg aggcggaggt tacagtgagc
27121 cgagatcacg ccactgcact ccagcctggg cgacagagcg agactctgcc tcaaaaaaaa
27181 aaaaaaaaaa aaaaattctg tctttggatt tcagtagttc tattatggtg tagtatttta
27241 gatgtagctt tctttgtgtt taatctgttt gatgatcata gcaatttttg aatctacagc
27301 ttgatgcttt ttgttatctt ggaaaatcct agaaaactac ttctttaaat atgccttttt
27361 ctttatagtc tctatcctcc agttaaatat atattaaaca tttgcagtat ctgctctata
27421 tctctatgtc acttttccaa tcatctatgt ctattatgct tttttttttc tagtttctc
27481 tggatgtttt ctactgatct gtcttccagt tcactaatcc tttctttagc tctaatatta
27541 ttaattccat atattgagac ctattttcag ttattacatt ttacagttct caaatttctg
27601 ttttgtaaaa taaattctat tctttgctca aattcttcat cttcctttct gttttctcaa
27661 acatactagt tgtagtactt taacaatacc tatttctttt ttttgaggca gagtcttgct
27721 atgttgctga aggtggtctt gaatttctgg gctcaagaga tcctcccagt tcagcttcct
27781 gagtatacaa tacctatttt aatatcaagc acaactgaag tttcattttt attgtctatt
27841 ttttctattt ttttgttagt cttatttgtg gatatttttc ttatttgctg aaaacttttg
27901 attgaatgtt tgatgtttta tttaaaaatt ataactgtct agactatgca gttttgcctt
27961 tctagagagg attcagtgta tcctctggta atcagccaaa aggcagacag atcactccag
28021 ttccattaga atatgacatg gctagaagct tttctgaagc ctgtgtgtat cagtctggta
28081 tggcctaccc actttccttg ggagttgtct ttcaggatac ccattcagag cctatgaatt
28141 tatgagattt tttttctttg gtgggttctg aacttcaatt tttgtaacct ctgcactatg
28201 agactgttgc atacattctt gtgcttttca gaagatattt gcttggcttc tttgcttctt
28261 accatggata atttgaaaac ttagtgcctc aaaaggaaaa ccttaagtat cagggaacct
28321 catttctctc ttgttttgtg taccctctgt ccgtgatctt agcccctcaa atctgactgc
28381 ctttaaaggc ccaaacttta atgttttgtc tctccagctc tgtgaaaatg ctggcagtct
28441 cacttttgtt aactctgttg ttttctgcct ggattttcag cctcttacct atccaaggac
28501 tatcatatta tattacccaa gggaaaatgt tgctctcata atgtctgctc acctctctta
28561 ctttatgttt ctgcccagga tcttggcaac tgaaagcttg gctgtctcta tggttatatc
28621 ttcaaatagg tgtttgttgc ttttcgtttc atctcatatt ttctagttgt tctcagtggg
28681 aacattgttt tgtcacaagc tattccatca tggtcagaag tggaagttgc taagtcaact
28741 accttgataa ttattgtgcc gttagtatgg cttagtgact aagggaacac attttgttt
28801 ggataggcct ttggtttaaa ttcctcttac tatgtttgag ataccgttcc attgaaaact
28861 tagttttatc acctgtaatc agaaaaaaaa agaccttttt ttagaggact cttaggagga
28921 ttaattggaa aatattcacc taaagtactg attacaataa ccatcacata gtaaatttag
28981 taaatatgtc tcaaatgata cagaaacctt atggaacctg taaggttaaa tcatgcattt
29041 ctgaaagtag taataatatt tactttactt ccagtgggtt aatatgaacc aaattatttg
 
29101 aagatatttc tattaaatat tgattacgtg aaagaatata acatatactt aagatctata
29161 acaataaatg aatgccagtg taccgctact caagttaaaa ataaaatact accagtccca
29221 ttgagtttta tgtatatgta ttccttataa atatttcatt gagtttttgc ttttgaaatt
29281 tatgtaattg gaatgatgtt ctatgcacct ctttgcaact tgcattatta taacttgctt
29341 tatcttaata tcatctctgt gattcattca tgttaactat agtttttttt cattttctct
29401 tattttcaat gtggtgagat tatgttgtat aatatctccc aaatatgcat ttattattaa
29461 tagaaatggc gtgctttttg atctttcact tttggtcctt tttccttgga attgtatttg
29521 ggaattagtt gaagatgaat ttctctagag cagctttttg tttgcttgtg ctccgcacct
29581 gtggacacta taaatagaga ccactttcaa aggtattttt ggcttaagtc tgctcgaacc
29641 acacagatag tgttacttca gaccacaact aggatgagag ctttctcctg attatatccc
29701 aaggaggaga tttctttctt ccttttaccc agagcaaatg ttgagacagg caaatttcct
29761 tgcagtctcc ttctgtagat cagagttatt tttctagttt gcccttatat tgagaggaca
29821 ctatagattt gttctagctt aacttaggga actactatta gactctttaa gagagctctg
29881 ggctctgttg cctgttaaac tgtcaagttc aaagcccagg ttcaccagct ggagcaactg
29941 ttccagggtg aatatgactt agtgttgaat actctctgaa ttatttcact ttatttttgc
30001 acccctagga atactttacc ttcttaatag atctttatat gcagacacac atatgtttaa
30061 acatatggaa gtacatatgt gtgcatgtat gtagataaat agatagatgc actttttcag
30121 aaagagtctt ctaccgaact gttagaaaca gaaatgtctt aagtctgatt tgtgctgctg
30181 tgacagaata cctaaaactg ggtacatgat gaaaaataga aatctagttt tcacagttct
30241 agagattgaa acgttcaaga tcaagttgct ggcaggtcag gagcctggtg agggccccgt
30301 ctctgcttct aaggtggcgc tttgaaggct gcgtcctctg gaggggagga atgttgctcc
30361 tcacatggca gacggacaga agagcaaaga gatagaaccc atgatacatg aaagcccttt
30421 tgtaaaggca ttaaacccac caatctgggc aaaggcttca tgacctaatc acctcttaaa
30481 ggccccacct ccccccatat tcttacattg gcaattagaa ttcaacatga gttttggagg
30541 ggacattcaa actataccaa gaaataactg attcagtttt tataagaaat tttagaactg
30601 tccctaagac ctggtgagtt tcaggcctca tgttctgagc ctacatctga catcagctac
30661 catagtatcc gctgtgcagg cagagcctct tcagtcatag gtatggggtg aaacagattt
30721 ccacctcttc cctgagcttc gctggcacag aataaatgca ttctaaagaa aactgatacg
30781 tcagagacat ctgagcttac tcttaattgc tttcttacct ttctctgtct tgggccagat
30841 tttcttccag gctgatggct tcttccaact ctgaattcta gcactctctg ctgtttcatt
30901 ttgtattcca tgccttcgtc tcttctctga tcccttccat ctgcctgaaa gcacttcctt
30961 tttattattt ctgtgtccta taataataaa gttaacaaaa gtgttaagta tatgtttatt
31021 tttccaaggt tacataggca catgtataat gtagggaaac tggctgacaa tcagagacag
31081 aagacacata cacgtgggcc cacacacatt cgttcactgt tgtccttaat gcagtattca
31141 acattacaaa ttactgtaat cttaataaaa ctgcgacatt gttttccttg tttatttagg
31201 gagcaccttt aaaaatcatg tatttgctga tttattcatt cactattgtc cttaatgtag
31261 tattcaacat tccaaattcc tgtaatctta ataaaactgt gacatagttt ccttgtttct
31321 ttatttaggg agcatctata aaactcatta tttactcctc agaaaacttg catgggcata
 
31381 tagttatgaa ccagtttgaa tattatgatt ttatattcac aatattttta tacttatgat
31441 cctaggctaa ggatatttat attagaatgt tcgctgagga cttaaataag ttctcactga
31501 tacttacact gaatattatc tcaatgtact tgagagaaaa ggtattgcaa gtaaaggtaa
31561 atatatgagt acatcaattg tataatttaa ttttctcaca attttaggac aattttgtat
31621 aggatctata atcatatact tttttaaata ttcacttgct tttattaaat caatcacaag
31681 aaaatacaga acgttttccc cttccacttc tgaagtggag gaaatcactt caaattcatg
31741 ggatctttga actttgccca ttgggccaga cttgcagtat cataaacttc ttttcctac
31801 ctatgtatga ttttgccatg tgcgaaacaa tcattgctag taccacgagt gagtgatgat
31861 aagcttagaa tacatccagc taagatcagt gccatttgaa gctccctcaa tatcgcattt
31921 cttcggttct ttaaaaaaaa ttcattgttt tggagtcagc atattattaa tgaatggagt
31981 atttgagggg aataaatatg actttggcaa tatcacatgc atgggaaaaa ttggctagag
32041 aaagggctaa gtctttcatt ctgtgtttta gagaacagct ttagctcgta tgaaaaaatt
32101 ctggcataga tatgtgaggg ggagtgttgg ataagaaaag gaaagagaaa aagcttgctg
32161 tggagtggaa ttctcatcac atttctttct tacctactac tctggggaaa cttcagttac
32221 agggctgata aatatttatt aagaatatat agacagtata acctaaaaac attggaagat
32281 attttagcga ggcttgagtc agattaaatc taccaaccgc taaaagatgg ctttgcctag
32341 agaaattctt ttttctttgg gagcagctat tagggaaaag cctattgcct aatatcttaa
32401 ttgaaaccta tgcataaaat tcaattaaaa atggcctata ttttgaattg tttttcaagt
32461 tacagaagaa ctttaagatc tttgtaggta atttctaaaa agcataaata tcttaaaata
32521 tatatctgta ctatcaccat taaaacttac cattctttgc tctatatttt ccaaagctat
32581 attcagagaa aataaaacct attatttcat agcctgcttt aaaaatatct cttaaaatac
32641 gctatttttc atctcctgat ctatccctca agaatatgat tttaaaactg cattacctca
32701 tttcgactta tttgttttat gtcctccttg ttattaacta tttttaattt tttctgtgta
32761 aaaacactta tttctttaca tattttctct aaatttggaa gctatgcctt cgtcaatgat
32821 accatgttaa cattttaaat ttttaaaaat attttaatat attttattca aacagtccca
32881 ataattaaat ttatttcact ggcaaatgcg atataaacca attgctgcat ctctcctctc
32941 caaaaagcat gtcttgttct agtgatgtat ttggagatag aagacttaaa ttattttatt
33001 tttttttact ttttaaaaat gagaaatgaa aactcggcta tatgcatggt gtacactgtg
33061 acgttttgat tgaagtacat tgtggaatga ttaaatcaaa ctgattagca cattatcatc
33121 tcacatactt atcatttttt gtgatgagaa tgtttgaaat ctactctatt attatttacc
33181 atagtcacca tgagtatatt acatcttcag aaattatccc ttctgtctaa ttgaaactgt
33241 accctgtaca caacatctcc ccgttgacca ccccaccagt ccccgagccc ctggtatcca
33301 ccattctact ctctgcttct atcatttgat ttttttaaat ttctacattt aagcgtgatc
33361 atgcatattt gtctttttgt gcctggctta tattgctaag cataatgccc tttagtttta
33421 ttcatctttt tgcaaatggc cggatttcct tcttttttat ggttgaacaa agtacctttt
33481 ttccccccac tgtcccaaat atatgggctc aacccccaaa ttggcaggtc taagaagtct
33541 ttccaaaggg tagtctatta catttaattt ctaaacataa gttgaagaaa agaaaattta
33601 tcctgacagt tccaaagttc aataaaaagt atagtttttg agtttttact tcagtaaggt
 
33661 attattttag tttcttagct tttcagaaag tctcagtctt ttcaatggga aactctctta
33721 gggcatttaa aatataattt gctaacaatg aacttttaga tgagtaatcg tggctgctat
33781 cactggccct ggatggcagt gggatgagct tgcaatgcca aggatacagc tgagcaaatg
33841 ccactggaag ctaaataaca tgaaagaaac cagggttttg ttgtttacaa tggccatgtg
33901 atggtatgag tacaggaaag ggctagaacc tgggcatctg aggagaaaaa gcattgacag
33961 catggatgta aatgacgact ggtatttgaa tgcaatgtgg ggccagaggt agacatcatg
34021 gaaaagtatc agactttgca tacaggacac aaactaaagt aatcactagg gttagaagag
34081 gagcccactt tctggagata ggttactcag agcaagaaat gcagcaagtc agaaagctgt
34141 cgatttggga agctacttat taatgtacct tacatcctcc ctgacaacaa tcgagattga
34201 taaccacatt taattcagtt ctagtcagta agacggaggt tttctagcac cagctgccaa
34261 gattaaaaaa tgtgcatgtc ctagctctgg gaagtgtctg gtatgattga tgcttggtac
34321 ttgttcatct aaatatgaat ttactactgg gaaagaaatt aaacttctag atattcattt
34381 ggtagatcgg tgaggtgact gaagtgtggc aaacctcaca atgattatga ataaacttta
34441 ttaagaaaaa ctgaggcaat taacttattt gttcttcaaa actgaggaaa tatttgtcct
34501 cacatatatg atagaatatt gtttgctgct ggtaaccaat ctttgctttc ttctactaac
34561 aacttgtttg tcaatatgtc ttatttctca ctctaataag actatgtcta aataacttct
34621 ctcctaaggg ttaaaaatac aaagaatttt cacacataac caagtaataa agaatgtata
34681 ttattgaatg aatgagtaat ttacaaaaga aagtgttgag aagtagaata cataatttca
34741 gatttatgaa atatgaaatt tcagattcag gatctgtcca gtggagttgt gtttttactt
34801 ttatggggat taagtcaaag gcagtatatt aaatataatg gaataatatt cccccaaaac
34861 atggtaaatc tatactttcc ctgaatataa attttccttt aggacaccaa atctttaatt
34921 aggttaatta taatttcagg tagattaaag aggactattt ctctttaaac ataaggataa
34981 ttatatgaga attaaatctg tgagaaagaa ttggaacctg aaaaagacaa tggagaaaac
35041 tagcagacgg tatgttcaac tagaagaaag agaatttaaa agttaaaccc aaggtggtga
35101 ataatcacaa aaattgttaa tgaagagcac tttttttttt ttgctgatgt tctttaaagt
35161 tttttagccc tggtaataca tatttagaat tattttagaa tttgattatc tatgactgta
35221 ccaatattat tgtgttagca gtatgagtct tgctttctat tattgaaatc gttgttaaca
35281 gatcaaatta cccattatag ttttatgctc tgtgcataca gtggcatgac ttagattaga
35341 agaggtggat aatgttgttc tcatattgat attataagct tttatcacag atctagggac
35401 ctttgtcttc taattcttgt gtgtttatta ttgcttattc cattcaattg gcatgtaatt
35461 gaaatggaat tgaagacaag tacacatgtg tgttccttta tggcccctaa cattcactgt
35521 ttagttatat aggtctagat tatgaatggt gcctagaaaa aaaagtatga ataaggaata
35581 attaataatg aatacttatt tccatcacct gttaacatac tctgtccatt tattcctcat
35641 tttaccgtct acatttaagt tctcttttct agccaccaag cattattgtc tagctaccat
35701 ctactgagtg tttaatatat gtccagaatt gagcagagtg ctttgtatgt aattcatata
35761 accaatctag caaacatcct tgatttttca ttttacctag ttgcctaaac cttccagcaa
35821 tctattagga agtcaaaaag cttctcttcc tctgaaatat attccaaatc catccatttc
35881 acctgttatt cttctaggct aagctgtcat catcctttgc tctggtgatt gcaatagaac
 
35941 ctaattgtcc tatctttttc ttgctttaca cccttaaaat ctaatctact tcttgaaatc
36001 agggtgtgtt tcattgtaaa tgagataata tcatttctac atcttaacct agcagtgact
36061 tctttccatc atatctggac taaaatccaa actctgtatg gatgataaga cctatcagaa
36121 ttggagtacg tttttttcat ccccatcaca tgccatgccc gttcttttca aaaggctacg
36181 gctctttctt gtctaacatg taagcacatt cttttcaaag agcctggaat gttcttccct
36241 agatgggcat gactggctct tttttgtgga ggtcccagct accaattcct taaggaatc
36301 tacccttcct tatctgttct aaagtagcca cccagtttct atcatgaccc tcagtttatt
36361 gtcatcaaca cttcattttc ttcatttatt gtttattcct taattgattc tacttcttta
36421 gtagaaattc cagaagcttt aaagtgtttt gttcatagct gaatgcccag aacctagact
36481 aatttttgac attcaaaaaa tagcttataa gcatttagta cattttatta aataagaaaa
36541 tagtatctcc tctaatctca gaatattata aatttatatt gttattatca cttaatagat
36601 ttaaaaagtc atgaatgtgg gagataagcc cagggttatc cagttatttc agtggcagag
36661 tcagaatttg agctcatgtg tttcagacct tagaacttta cctatagtct ttgatgtcta
36721 catcacatta tgctccctcc catgtaccaa attattctct taacttacta tatcaactaa
36781 tatatctata aaataatctc tggcatagga atatagcatt catataataa atcattactg
36841 taatgcgttg acaatgcatt acagtaaata atgcattact taacctgata cattattcag
36901 ctgtttagat gagtcatcaa cctaaaaata tctgactggc tgtacagtaa tgtggtaata
36961 cattcatcat agagaaatta acgtatgggt tgaatattca tagtgatatg aatacaaata
37021 tgtgtatcag taggattatg gatgtatgat agtttagtat ttacctagca tgtcaatgga
37081 tgataacaat tatctgcaaa tatttctgag tgttttaatt tgggtttccc tagaagtaga
37141 ctttgagaca gggaattgag gcaaggagtc tatctgggag gcaatcccag ggcactttgg
37201 caggaaaatg gtaaaatgag acaggatggg agaggaccca ataaatggta tgttatcaag
37261 cgagctatcg ttatgggtat ctggagttta attctacagg ggactctatg aaacagtgta
37321 gaacacacaa ctcagactta tcctgaccaa gatgcaaggg agctgggtat ttatacaaaa
37381 agtcttgtta aactttggct tagggctgct ctggggagca taattacttg tctcctgtct
37441 gctgtaagtg ggcagcatgg gttctggcag taagagcaaa aactcaggga ataaagtgga
37501 gatgataaca gttgaaagtt ggactggtat ctactgaaat actgagacca tggacagctc
37561 gcaggctatt aacaaggtct tctaaactac tcatatatgc atggtaatct gcatgcattt
37621 ctgaatgtat acatctatac gaattggaac caacagatga aagtaactac atgaaaactg
37681 tgaaatatag aaattactta cctgtaatag gacagataga atatacagac aatgctctta
37741 agttcatggg tttctatttg aatgtatatg ttggtgcaga ccctgtgaca atctgtattt
37801 tcaggaattg taggtatatt tgagaaagtt atatttttat gtaatatata acagttctct
37861 ttaggactta tcagtggata tcctaagaaa cttttaaata ttttcctatg tttattcatc
37921 ccacttcaca ctggtataga tagatagttt tttgataact caaatctacc tgtggtaagc
37981 agaataatgg cccccaaaga tgtccacaac ctactccctg gaacctgtgg acttgttacc
38041 tacatggcag aaggggtttt gcaggtgtgg ttaaattaag gatcttgaga tggaaagatt
38101 atgatcctgg attatctaca tggttccagt gtctttacag gctctttata agtgaaagag
38161 agaggatgtg gagtcagatt cagaatggtg tgatgtgaaa aagactcatc tggtcattga
 
38221 tggctttgaa tgtggaagga agccatgagc caaggagagc atggagcttc tagaagctag
38281 aaaagggaag aaagtgtatt cttctccaga gccccttaca ggctttgcca gacaccttga
38341 tcgtagccca gtgaaaacca ttttggacat ttcacctgca aagctgtaaa aaaaaatgca
38401 tttctaatgt tttaagcaac taaacttgta gtactttaca ccagcagcaa tgagaaacta
38461 atgagctacc taataaacac agaggtaaag tcacctcaag taaaaaaaaa aaaaacaagt
38521 taagacctat gttttaaagg tcatagcagt atttccactg tccgtatgtc actgcagtga
38581 taaatgctgt gttttgacca ttgaaagaat tcgaaattcg agtaagcaaa actaagttgg
38641 aattctgcct aattactctc tatttaacct cagataactt ttaatttctc tttgctttat
38701 gttcatcatc tggaatatgg ttacagtaat aatattctga cttccctcat gagttcattg
38761 taagtattaa tttgaataac ctaggcgaaa ggacgttgta gtctgcaaca tagtacatgc
38821 aattttattt ggctttgtta gcaaataata gtgctaaaag aaatcagata tccaaaagac
38881 ataaattaaa ccacataatc ctagtcaatt tgtagatttc tgagtatttc tgtatcaaac
38941 tggcaagtat atgctatatt tatgatgagt aagtcataac agttaaaaga ttgactatta
39001 attgagagta aaaattgtcc ttgttattgt cttatgcatt ttaatttgca ttttctgtgc
39061 cttaaataga aatattttaa aacaattcat gtatgcagga ataaaagcac tttagtggac
39121 tattaaatgg taaacttctc cacatcattc atgagggagg agttataaaa ctgttctaac
39181 tctcaatttc ctaaaacttt ggtaaaataa tattctatct catattctcc cttctgtatg
39241 tctttatgtt tgcaaagatt ctttggggca cagaaataaa caaatcatcc ctatgaaagg
39301 gtcatcgtag ctgccagtga ccaaaagagc tgcctgctga gcagtttcat actgttactc
39361 cagctgccat ggactcatgc gtgaggaatt cacacagcta atcaattcca taaggtgcag
39421 ttgctggctt ctctcttctt aagcctgtac ataaggaatt ggcacttgag tgtttcatcc
39481 tgtggactct acatcattcc ttcacaccct ctgccatttc ctggccctcc ccaaaataga
39541 cccaaagcaa aacaataata aacaatttgc tgtttgattt ttagtcagca ctaccattca
39601 ccagccctta agaatgccac agcagctgcc tgtcactgac tgtttcagca ggatcaggct
39661 agaggtagga gaggcggggc aaaatttctc ctccttgtag gttttagtta attgctgtca
39721 atgacaacta gcagttgtgc acctgtttcc atgcatttag tttctaccct ccatcaataa
39781 attctaacaa ttatttattg aacatattct atagttttgg tccttaaaat aacatctgat
39841 tataggtact gttgttatca aatgaggaag cagacttcac aaatagcaac tgggccacaa
39901 atcttgctac caagtgtcct agccagtggt cttggaagtc taggctcttt ccctatagcc
39961 cccctgattt tattagccaa gttctaattc taacatggga aagtcctatt tcttagttat
40021 gtgtgatgta acttagattt gtgtgtaagt tattatttct tagttttgtg tgatataact
40081 tacattatca tgtacatgtg cacatttaca tcttacatgg gaaattttaa tcagaggatg
40141 tcttcatctt tgcgtcacta ctgaattgac taaagactca ctcaaagaca tttccgtttt
40201 aatagtgaag agaaggaagt gattctcttt gcatcttatc ccttcatcct acaggcattg
40261 tgctcctact gaacactgaa gagtagtttt gttggcagta accttagaac gtttggagtc
40321 aaaagttgtt gtctctatta cactcagatt gtagaaagtc agcaggccat tcggagtgcc
40381 aatatgagct atgtaaaagt catctcaaga ctggaggaag agtgactctt gaggccttta
40441 ttttccatat aaattgtgcc ctctgtttcc tagcaccttt caggatccgg ggcagcctgt
 
40501 ctgccatttt ccacttgttt agggctttaa aatcgtaaag aacttcatta actccatgaa
40561 ggccttcaat ttaatattga ggatactgga gtatttatca cagaaaagat acagctcaaa
40621 aattttaacg caatttcatg gaaaattaaa aaaataacta ttacattttg gtaagacttg
40681 tatactgaat ttttcagaat gtaagctaaa ttgggtttaa tcttatattt tataaaaaca
40741 aagaaatatg aaatttatcc atttattttt tttttcttta tgacatattc caaatataca
40801 gaggaggaca gagacctaaa agcctcaggg acccatggtg gggctctatc aaattctttc
40861 attttgcctt atgtttccag atattgttat gagaaaaaaa cttacaggta aattgaagac
40921 ccccatataa gccttcagaa atctcattcc ccttcctcca ccaagttaac caatattctg
40981 aatttagtat ttatattttt atgctttttt tttacgttta cacatatcat tgatctatcc
41041 aactttttgt aaattatgtt gtgctgaaca tacattcttc aacatgttat tttctgtttt
41101 tgtttatgtt ttattttagg gattaagcct tgttgatgcc tatagcttta atatatatgt
41161 agaaatctgt tgtatgaccc atgggttagc aattctctgg taaaggatat tccattttct
41221 tcaaacattt tgctgctgca attagtactg tagtgaacat tttgatgttt ccttataaaa
41281 agaatggtgg tctttttagt ttgtaggaat tctttgatca ttctattaat cctttgtcaa
41341 ttatgtggtt gaaatgcctt gaattttcaa atttatttgt atgaaaatac tgacagtagt
41401 cccttatgat ttttgccatc cattctataa agagctattc gcgtttcaat tctaaaattc
41461 tttgtaagtt ttctctttca ttcttggaag actgtttgtt tactctttag tctcttcaaa
41521 gaattttgtt tggttttttg gctctctcta tttctgtgtt tatatttcac tattttatac
41581 tgttgctttg attaaatcct tccttttaga ttgtttaagt acattttcat tctttttcta
41641 acttcttgag ttgaattacc ttaaatttat tattctcagt cttatagttt ttaataaatg
41701 tatttcattt attaaataaa tgtatccatt tctcttttaa cttcttccca caagtttgga
41761 aaagtattcc ttttattgta aagttctggt tctctttctt ttttttcttt ttgtcctatt
41821 gggatttcct gttataaccc atgggctact gaagttttca aacgtataga aattttttgc
41881 agaaagtttt tgcagtatct attaattaac gatttctatt gtaacatcat ggtaaaaaaa
41941 cattggttct gtaagatacc aattctttga aattgtttgt gatcttttgg aacccagctg
42001 gtggtcagtt ttcataaata gtttttgtgt atctgaaaaa gatgtcaatc cagacaggat
42061 tttatatgta tacctatgag aagaaacttg ttaaatttat gttagatctt ttctatttta
42121 ctaaattcct gcttcccctg aagggtttag gtatgcaagt cctttagaaa tctaaaggta
42181 gtaatgttag gtttataatt gaaatggctt ctcagtggac tctacgtctt ttactgtatt
42241 gtgaaccctt tgatgcattg ttttacattt ttttcttact ggcattagta ttactacata
42301 tataaatttt tttcaagtta gtatctatat agtatatttt cttcagtctc tattttaaac
42361 tttcaaaaca aatatagttt tgttttaggt tattcttgta atcatcatgt agttttataa
42421 aaatctaatt tgattatcac taatatgtga gtttgataaa tccacatagt cataattact
42481 gatatgtcac aacacagtaa gggctgtggt cttcatttat ctgaacaact gctttacatt
42541 tagtttcaga gatcggagta ataatgaacc tttctgcatg tacccagtgt tatgactcct
42601 gtaaggatta atttaaaaat gttcaatatt tattttcctt ttggattttg ctgaaaattt
42661 aattttagaa ataaaaaaaa tctctagttc tttcaaattt tccatgaatt gaagattgtt
42721 taccgatcta tttttttaat actgcatttt ggcataattt tttaataaat gtttacactg
 
42781 taattttcag taattcagtg taaagctaac aggttttttt caaagatgaa gagacacaat
42841 atgttataga atatagtaca tggtgggaca gttaagtagg ggttctggag tttatttttg
42901 cttctgctac ttaactatgt tattacaaag aaaaaaatca tgtaagagac tccgccaaat
42961 tctcttctgt ataacaagag ggagtgacat actatctcaa tttcagtttt catatttaat
43021 gactcacaat tggatttttg gtatatattt gaccactttc taagatttta ttttttatgt
43081 tcttgaaaat tttatattct ataaatattt ttgtatacat atgtacctca cattcaaggt
43141 aagaaattct taagtaattg aactttgtgg ctataattaa atatatacac taatagatga
43201 cagaataaag aaaatatttt tattctgaga gcctaatttt aggtaatgaa aatcatttta
43261 tgggtaaata aagtacacat tttatagtgt gctaaaaaat acacaaaatt tgacatttaa
43321 aacaataaaa aatattaaat aacttggaat gaaaggagca tatcgaagta tttgtgaata
43381 aataatctca aattgatttt ttccaacact ataacatctc ttttctgttt aaaagaagtt
43441 taatagttgg ttcaatcttt tctttgagtt tgttttttat attttccaag cttttaattg
43501 tcagtggagg gagaggactt gagtgttttt aaggtttcca tcaacatcca ttctcctgta
43561 ttcattgtta ggccaaaagg actcaacatc tttgagtctt ccctatttga gaccaagtct
43621 atactcagct tctaatttca gctctagctt ccattcactc tcagtctttg ttagcattat
43681 ctaagtttag gttgagatta ttgcattatt gacttgcatt ataaacttct tctttcaaga
43741 ataatatgac cttttaatct tgttatatat gtttacccat catttacata aattttatt
43801 ttatattcca tatggtttta ttccaacagt cttttgtagt taaatatttt atactcattc
43861 atcataaatc ttcaagtaat ttttagaaga attttgtaaa agtatatgac ttttttaaat
43921 tttgtgggta gactatatca ataaaatatt taaatatatt cagaaacttc ttttaaagtt
43981 catatcactt tactttggta aagagtttta gcaaaagctt ataattaaaa tatgcgacag
44041 tgctcacaga gcatttctga ttactatcag aaataggggt ttattctgga aagttgtttt
44101 cctgaaagtc ttaattgtaa aaagtgcagt ttaataccta ttatattctt atgtgtatat
44161 taatgccaaa ttaagcaatc ttcctccctt tctttcttcc ttctttcctt tcctctttcc
44221 ttcttttctt cgtttctata tttatgctgt atacgtacta cttgaaatat ccagacacta
44281 ctatttgttt aattcacaag ccatatctgg tgatatggat attattgttc ttgtttcata
44341 gttgaagaaa ctgaaacttt aatgaggttg gataagagcc acaagtatag cagattttat
44401 agtgtataac cctgcaaagc tcaaatttgg attgatatct ctgtttattc tagaaccctc
44461 ctctgtgtca ttctatgtca gtttgctttg ttgtttatta tggtaaagta atcattcttc
44521 acttcaacag ttatctgtga gttgattcta aaacatagaa aaattttgtt ttaaaataaa
44581 ttgtatgtat gtcgtcacct aataatgggc ctatgtgggg tccacacaaa accagtttgc
44641 tgcagtcttg gcatagtgta aaattcccaa agtttttata tacatattga atgcctcaag
44701 gtatatttta aaagcccttg ctaatcccaa aggaattgtt ctaaaaattt gtttaaatga
44761 aatttttata tattttaata tttaaatttt aaataatatt tttaaaaatc ttaggctgaa
44821 ctttcagttt gtaattagca aagtaattat tatttgcccc aagccagctt cctcctattt
44881 cctgaccctc atttgtcttc cctccttata agatgcttaa tgaaagagaa cttgattttt
44941 gttcatttgt ttttgagaca gggtctggct ctgttgccca ggatagagtg cagtggcaca
45001 attgtagctc actgcagcct tgaacctggg ctcaagggat cctcctgcct cagtgtcctg
 
45061 agtagctggg actacaagct catggctaat gagcctggct aatttttaaa tttttgtag
45121 agatgggggt ctcgctgtat tgcccaggat ggtcttgaac tcctaggctc aagcagtcct
45181 ccagcttggc ctcccaaagt actgggattg caggtgtgag gaccacgccc agccttgaaa
45241 cttgattgtt atttacatgg tgcacttaag aagcctaatc ttaccttctg acccagggtt
45301 tgcagtcaca gtccttagag tttttgaata aagactgtgc tatttctttt ttttaaaagg
45361 gatttgtttt tatctaattt atttctgcct ttatgtttac tcattattaa ttattcaaaa
45421 taagattggg atcatcattt atgtatgaat aactcaaatt agtttattgt aatacatagt
45481 cctggtgaag aagaacatat tcagttcatg ccttcaattt gcatttatct tgattagtgt
45541 ttaagtgcat ctcatttccc tatgcttctg ttttcatagc ttatataact acactgttag
45601 attttatgca tgatgtaaca ggatacaatc ccatgatata tttccatgat gtcaaagctt
45661 tgaattatgt aaataggaaa catgaagcaa aagcatacta cttattcatt atgcattgct
45721 atgcggcaga cgcacagtca agtgatgcta tttaatctcc ctatctgcat attcttaaag
45781 tgcatatctc tgtgatatcg aagcactcaa gcaatgagta aatttctgca tctggttaat
45841 aaattcagca aacaggagtt catcaacatc tggcatctgc cttattttac tggctttata
45901 tgaataactg atggatggcg acctttgata aatttagtgg ttgcttggaa ttttagtagg
45961 ggtttgatat tgtttgtgtg ctattgtgaa tttaatatgg caatcaactt tctcttggtt
46021 gctttactca tactagaaac cacagcatca accaatcaaa aggctgttta gcatttggtg
46081 ttatcacata taccctaatt tgcacaactt tccaaaatgt aagtaccaag agagaagaca
46141 catcaataaa atataggaat tataaggaag aggaagaggt ttttaaaagt atattattat
46201 tcagcaaata atggaaactg gtgctcacaa actactaaat taagagggct tttttccttt
46261 aaaaattaat gcaatgatag tgcagaacac caaataaaaa ctgcatagcc aaggctctgg
46321 aaggttgctg gtggctcctg gacatcagtg ggatttcaca tctttctctc tgacatatca
46381 tttgttcatt tccagagaat gcccagctct gtgttttctt aaaaataagt ttagaattct
46441 cataaaggtg gacattattg gcctagcatg agttaatttt ctctacccct ggacctggac
46501 caatcatata tataagcaga gagcagaaag gtagcacaga ctgaggcaag gtgaggataa
46561 ggtatttttt gtgtgtcaca gagacacctt gcttgtctca tttttgaaaa tgatgaaaac
46621 aaatggtgac taataaatag aaaatgctct gtggtattat cttctagtac taatgttttc
46681 ttcactgtcc ctggcctggt atctttcttg gcatacctca caaattactt gacttctgtt
46741 ttatgttgac ttcactcctt tacattacat tattgggaag ccctaggtct tagaaaacta
46801 tttcttaaac tgaattaaaa ttttgccctt gtacactggt actggctctg tcctctcagt
46861 aatagaaaag aagtcttcca tatgtccaca gccattattt gcaaaaagat atctctttct
46921 tcaggcttat gtttcccatg ctacatctct cttgttctct gaaccacact ttatacagcc
46981 tgactttcca tattctcact gttgctgtca tccccatctg atcattcccc tctgtcttaa
47041 aatgtgacaa ttacaactgg acataatact ccatctgtga tcttctcagg acagacccaa
47101 gcaggtccca gtatagtgtt agcaggacat cacatttttg gcttcagatg aacctgtaaa
47161 tacctaaagt acctggactt ttcccatgaa ttgttggtag gccaggatgt gtgccccatt
47221 agttggggac actttctagt gacagttcct ggaactctat ctggctattt ctctagtcag
47281 gcagtgaagg cagataaatc ctcggttcct ggcaggttca ggtggaacac gcagcacagt
 
47341 gtgcgctgtt gaatgagtgt tcccgagatc taatcacatg gaatgcctga ttgtcaaatg
47401 atgaacaaat aattcaggag atggacctgg gagttgaaaa tttttttaac aaaaaagaaa
47461 ttacatatat gcatttttaa caaaagtttt ataatcattg atatagtgat ggtagcacca
47521 gtagatattc aggcaagtaa ttatcaactg gatgacttat aaaggaattg ataaagcctc
47581 ccaagggatt caagaaaagg gtaaagcttg gaagtcccta aggataagtg gctgagatgt
47641 ttgattcagg acttcctgtc cttgaaacct tggtcagtta ctagcacgta ggacagtctc
47701 tattgtggac cagtaaaaag ctggttgtct ggagaattca gacagaaaag gtaaggctgg
47761 gacccagtta tcaaggctgc actacaacta gggcttgtaa aagttagggg attttggaat
47821 tagcgtagaa ataatgtaat atggtgtaag taacacaggc tttgtaatta gtgcaaaaat
47881 cataagcata tgggcttgag tcagtcttct ctttcctact tactggctgc ataccttttg
47941 tcagctattt aacctctctc taagcctcag cttcttcatt tataaaacaa attaatatta
48001 tctacactga agtattatag gagagatgat aatgtatttt ttaaaaacta gcacagaccc
48061 tggtcaacaa taataaacat actaataatt gttactatta taataaacgt gttgatatct
48121 ttttcttcat caatgacttt ataaaaatat tgggatggag atgccaaaat ctcctctgct
48181 aaatgccatc tttcatccta aggctataca ggaacaggcc ttgcctactg aactttgtag
48241 ctataggcct actacattgc ctcccacttg aaccctcggt tataaacgga atggagagtt
48301 acttttccat tcagaaaaga accttgatat ttcagaagga aaagttagcc ctggacactt
48361 gagcatttct aagaaaatag gttttttttt tctttttaaa ttgtgtacct gctgccctga
48421 attctctaga tgttctgagc actctgtata ttcttactca tagcttagta ataaattatg
48481 tttataaatt ggagactggt attgtataat ttagttcatt gaaataattt gatctggtga
48541 cttcatagcc agacagagtc aatggattca tataaatgag aatccagggt taatcaccat
48601 caaacatttt caacataaag tgacattttt ttccttagaa tgggattttt actacattca
48661 aattttacta atgctgtaaa catgaaaatt ttgagtcagg taatgaattt gagaatggat
48721 agctaatctt taaaaatcta gtaacaaatg tttctgggaa ttgtggctat tgttcccttt
48781 tggaatgatg accaacattt ttggaaatga aaatattgat aaatttgtgt ggacttaaat
48841 atggagtcca cacaagattg gggtcatagg acagaaacaa tatttgtttc acatttacat
48901 tgctgaggta ttatgaagag gttagaaagg tgatttttat ttagcttgca gaataaatat
48961 tttaaaatca cagaataaca tatactatta aatgcctaca tacatttata tccatatgaa
49021 taaaacagta gaagctgaaa tataaaataa catgccactg ctgctggaga gatttaagcc
49081 aattatctgc tatcctggag catcagacat atatatctat atctatctat aatattatat
49141 atttcataat ttatatattt atatatcggt ataatatatt gttttataca gattatatta
49201 ttatatgctg tttttcacca atgttgttta catatattat acattattta tatccggtga
49261 ctacataatt gtgcatggta catcccgtct ctagtcagtt gtcatgacaa gagttttaat
49321 catttatgct tactttatga ttctattgta caatttttat tggacattat ttcagttttt
49381 attaattaac agggcatatc aaatacctcc atacacactt tattacagat tttttgtcc
49441 caaatatttt tagcattttt ttaaatatga aaatagatag tttggaaaca agcttctatc
49501 atctactatt tcttcagtta tttgaatgca gatggcctat cacaggacac aagaagttga
49561 gaatgtgcta ggagaattgc cagcactgct caatacaggt gtttgcccag gtctaccttt
 
49621 tgactctctc acttgatctg ttcttgtgct tctcactttc ctgggcagcc agtcccttac
49681 tgagtatttc tggccttcag tatctgcttc ttccggttcc tttgatgcag cacccacttg
49741 ggctcgcttc tgtagttcgc taaactcagt tgcttatcat tgtaatattt ccgtaatatc
49801 agaatgacta ctgtctcaac ttaggagaca tccacttgtc ttctccagca cctgactgag
49861 ttcttgccat ataaattttt gttgaataaa ttcaatgttt ggcttttcac atcatcataa
49921 acaagactta caccaacaat catgggggaa tttgaataaa attaagatta ggtctgactt
49981 ttgcaatttt acaacttaca agttagtgtt aagaaaatgt ataaagatgg tgatatgcca
50041 ttttgaaaca tattaagcat tttcatcttt agaacagcct tcaagagagt tattatgaaa
50101 cacatttaag cttaagaaag ctgagaatca gaatgataat taataattat ttgagggtgg
50161 atgaaaaagt aaagaacatt tacacagttt tagtgagaat gtaaattagt tcaatcctaa
50221 ggaaaacaat atgaaaattt ctcaaagaac taaaaataga actacagttt gacccagcaa
50281 ccccattact ggtatgcggg tggtactata taagaggaaa agaaacatcc tgtcaaacag
50341 acatctgcac tcatgtttat cgcagcacta tttttaatag caaagtcatg gaatcagcct
50401 aagttcctat cagcggggga ctggatacag aaaatgtggt acatacacac catggaatac
50461 tatgcgtccg taaaaggatg aaatgatgtg ctttgcagca acatggactc tgctggaggc
50521 cgttttccta agtgaattaa cacagaaaca gaaaatcaaa tacttcatgt tctcacttat
50581 acgggagcta aacagtggtc atgtaagcac ataaagatgg aaacagtaga cactggggac
50641 tccaaaatgg ggggaaagag ggaggagaac aagggttgta gaagccaaat tgaagcctga
50701 aacaatacac ctttgtaacc cacctgcaca tgtggcccct gaatctaaaa taaatcaaac
50761 aaaaaattaa caagcaaaca aaaaacagag gaactagggt ttgaattaat atttctgatc
50821 tgcatatcgc tattttcctc ttaaattatg tgcttaaata catgaagaag gaggaggaga
50881 aaaaaacaac ttaattagga gacttacttt aataatgtat catttataac ttttcttcca
50941 aaatctggtt acatgtcaag aaactgtata atgtgaatct ctctagttca cctttccttt
51001 cctttatgta gatcttggtt gcaattcaga ttttcttttt tctaatgaac agtacaatca
51061 gtaacagttg caccaaacat ttcaatttgc aatatacatg taatggtttt tcaagtgaag
51121 gttacatttt atcttttttt attatccata gaataatgtg ttttaggatg aaagagacac
51181 tgtgtgtgtg tactccgttt aaaattaaga tctgacggtc tgagacatca catacaaagc
51241 ataattcagt agctatctga cagtttccca attgctatga cactaaaaat aaaccaaacc
51301 agataaatca accccctaag tggactctct ggcctgaaat gttcgggttt taatgtgcct
51361 gtggaatgtt ccaatataat tttcatcaat gttgattgtc cagctcacac agccctgggc
51421 actagagtga ttgacataaa ccccgtcatt attccaacaa ttaaatcagc aggaattagc
51481 acaaacattt ttttcctgtt ccgtacacta aaaatgtttc catgctgcaa tgttaggatt
51541 taaatgtaac aaacataatc ttccctgtgt tgtataactt tccgatctac taagaaagcc
51601 taccaaaatg gagcatttgg tttaaaaata aatacctgga ctcttccatg tggcaacaaa
51661 caatacataa gagatattgg acaatgaaca attaattaca ttgataaaaa taattgatga
51721 attaagaatg taaatctaaa gatactttat attcctcttt ttggtttaac tttcaaatcc
51781 agaaagctct taacctggtt aactaatcga tgataacgtt atacaacaaa cttttgttga
51841 gtacttgcta ttcattcctt gaacgttatt tattgtaata aacgtgttaa ttgaggttct
 
51901 acatatataa aactgtttaa ggtgcaatga aacaaaaatc acctgaactt cggacaaatg
51961 tgatttttgt ctttgcttag gacgtttttg gaatatttac ttttgaaaat gtctccatga
52021 cctgtggcag tgttttcttc tgaatagtat aaatgacgat ggcaaatcta aaataagtta
52081 tttggagcca catctagaaa ataatgtgag tattccagtt attagggaag gctagatttg
52141 atcaaatata tatatagtta tatgtattct ctctctatat atagaatata tagttacata
52201 tgttctatat atagttatat atactctcta tatatagaat atatagttat atatactctc
52261 tatatataga atatatagtt atatatactc tatatactct atatagaata tatatataac
52321 tatatataga atatatataa ctatatataa atatatagtt atatataact atatatagaa
52381 tatatataac tatatataaa tatatagtta tatatattta tttataaata tagttatata
52441 tactctatat agatatatac tctatataga gaatatatag ttatatatat tctatatata
52501 tattctatat atagttatat attctatata tagatatata ttctatatat agaatatata
52561 gttatatata gttatatata ttctatatat ataacttcta tatatagtta tatattctat
52621 atatagaata tatatataga atatatgtat agaatatata gaatatataa tatatagtta
52681 tatatattct atatatcgaa tagttatata tattcgatat atcgaatata tagttatata
52741 tattcgatat atcgaatata tagttatata tattcgatat atcgaatata tagttatata
52801 tattctatag ccataatgcc tattagcctg gcatttagac ttgcaagtgt caacttatta
52861 cttaccttgt tttattttat ttctttaatg atctcaaaac cttcctctct cgatttcaat
52921 ctgtggtttg ttcccaccat ttgccgaggt ctagcatctt attctttgat tttctttttt
52981 cttttcttca acttttaagt tcaggggttc atgtgcagga tgtgtaggtt tgttacgtag
53041 gtaaatgtgt gccatggtgg tttgctgcac agatcatcct atcacatagg tattaaaccc
53101 agcatccatt aactattctt ccgatgcgtt ccctaccctc atacgctccc cccacaacag
53161 gccccagtgt gtgttgttcc ctcccatgtg tgcatgtgtt ctcatcattc agctcccact
53221 tataagtgag aacatgcagt gtttgctttt ctgctcctgc attagtttgc tcaggatgat
53281 ggcttccagc tccatccatg tccctacaaa ggacatgatc tcgttccttt ttatggctac
53341 atagttttcc atagtgtata tgtaccacat tctctttatc cagtctatca ttgatgggca
53401 atgattttgt aatataatga tttatattcc tttgggtata tacccagtaa tgggattgca
53461 gggtcaagtg gtatttctgc ctctaggtat ttggggaatc accacactgt ctttcagaat
53521 ggttaaacta attaatactc ccacaaacag tgtaaagggt tcctttttct ccacaacacc
53581 accagcatgt gttgtttttt gacttcttaa taatagccat tctgattgga ataagatggt
53641 atctcattgt ggttttgatt ttgcatttct ctaatgatca gtgatattga gcttttattt
53701 catatgtttg tcagctgcat gtatgtcttc ttttgagagg cagaaatacc attcgaccct
53761 gcaatcccat tactgggtat atacccaaag gaatataaat cattatatta taaaatcatt
53821 gcccatcaat gatagactgg ataaagagtc tctgttcatg ttctttgccc actttttaat
53881 gggatttttt tttcttgtaa atttaagttc cttgtagact ctggatatta gacttttgtc
53941 agatggatag attgcagaag ttttctccca ttctgtaggt tgtctgttaa ctccgatgat
54001 agtttctttt gctgtgaaga agctctttag tttaattaga tcccattcgt caatctttgc
54061 ttttgttgca atcgcttttg gcgtcttcgt catgaaatct ttgcctatgc ctgtgtcctc
54121 aatggtattg cctagattat cttctaggat ttttgtagtt ttgagtttta catttaaatc
 
54181 tttaatccat cttgagttaa tttttgcata tgatgtaagg aaagggtcaa gtttcaattt
54241 tctgcatgtg gctagccagt tctcccagca ccatttatta aatagggagt cctttctcta
54301 ttgtttattt ttgtcaggtt tgtcaaaagt cagatggttg taggtgtgtg gctttattcc
54361 tgggttctct gttctgttcc attggtctat gtgtctcttc ttgtaccagt atcctgctgt
54421 tttggttact gttgacttgt agtatagttt gaaatcaggt agtgtgatgc ctccagcttt
54481 gtgctttttg cttaggattg tcttggctat ctgggctctt ttttgattcc atatgaattt
54541 tttaaaagtt tttctaatgc tgtgaagaat accaatggta gtttaatgga aatagcattg
54601 aatctatcaa ttactttggg cagtatggcc attttcatga tattgattct tcctatccat
54661 gagcatgaaa tgcttttcca tttgcttgtg tcctctctga attttttatc agtggtttgt
54721 acttctcctt gaagaggtct ttcacttccc ttgttagctg tattagcatc ttattctttc
54781 aagaggacag tttgttcagt ttgtttaaat tattatcacc atcacaacca ttaaaccttt
54841 attcagcaac tgaagaatgt accacaatat ctctcatttg caagacatat tctgttcatc
54901 ttctgctttc tgcttctgta gcttataaag atatataaat atattttaaa cccaatttgt
54961 aattatatat gatgggaatt gaaaacgtgg gtgattttca aataatcact aaggaataaa
55021 atatgctaaa aataaattgt gaagaattga tatgattttc taaggatggc tttactggag
55081 aaaagcagtt atcctacaat agttaatata taactatttt tatgattatt ctgttcagaa
55141 tctgaattta tattattatt tggggccttt tttaatcaag tgaagaaagg tatattttct
55201 tttcaaactt tttattttga acattcataa attacatgaa cactaaaaga cataaattat
55261 ctttcaggaa acattgaata aattctggat tcgtctatat tttattgatt tgtaaaacat
55321 ctcaaattta acatagaaat gttaaaaaga gattatatta taacgtttgt tggaaaatat
55381 gctatattta taatatttat tctgtattta tatattataa atatattcta tatttatata
55441 ttataaacat attctatata tattataaac atattctata tttattaaca tttaacatta
55501 atgttaataa atatagaata tttttcaaca aactttataa tataattccc tccttcaatg
55561 aactaatttt tatggtggtt tacatttctt tttcaatgtt ggtttacatt tgacgttagg
55621 atgtttatta gttaatacaa taaagtgggt ggtcttttca ttaatattat cagtcaggtt
55681 tgtaaatagt gtatgttttg tataaccact taaaatttat atactgatat atatttgtt
55741 taagaatact taaaataaaa acattagtta tcatttgttg aactggaagg atcattactt
55801 aaaaatccat ccttaaaatt aaaatgactt atgcttcact gtgtagtgca ttgataaata
55861 tttagtctat agttttttct attgctatgt gtttagccaa gaattgtttt taatttggtg
55921 aacagtacct tattaggaat atgaaaattt ttgaagcagt catgaaatat ttaacaatta
55981 attatttaac aaacaattat gcaaaattaa ttttactaaa gaaatatttt taaatcactg
56041 caagaaatat tttcattcaa atgagtactt acaaccaaga aagagtcttt tattttttaa
56101 atgtgtataa atttaaactt caaaatactt aaatcactta atgcctcaga gaaaatatct
56161 taatacttat gaagtgttac gtgtactaag cattatgtta aattctataa agacatcttt
56221 ttaaatcatc acaagaaatt acaaggcagg tgcatctatt atatttttta tagtagaact
56281 aattgaggca cagtgagagg aaatgatgta ctcaagttcc acaggtgata agtggtacag
56341 ctgagtaaaa ttccaagtct aaaagctagt ctcttaacta ttatatatcg gcctatatgc
56401 ataggccgac aagtagttat ttaaagttat ataaatcaag agaaaacact acaaaattta
 
56461 cctataggat atttatacaa aatatgggca ttttattagg aaagctttct agaaatatgg
56521 gggaacttta agaaacagtg aaattcaaag ctatcattgt ccaaaagcat gtggtcttct
56581 gtttttgcac tagtgactta ggtgcaaatt acttccaaga tttttctcct aattgagaag
56641 tactttttag gtctagattc catggaggag gtgggtcttc gaggtggggc ttcaaggtgg
56701 agatagtgtc ttttttctag catgtaatga ggtaaatttg cttttgaatg aacaggtgca
56761 cctatttcaa ttaaggcaaa ttagtttcca aaggctttgc tatttaagat attatagaag
56821 gctatttaaa tgtgccaatt tacataaaat acattttgaa agattacagg aaacaatttt
56881 atataattca ctgaaacaat ttcacagtcc tctcaaatga tgattttgaa aatgacctaa
56941 atggcaggca cctctttgaa gcagctggca tgatgttatg tggtcccatg gccccttaca
57001 ttttcaggtc aatggtaaat tctccagagt ccaacttcaa ccctgcagta ttctgttcaa
57061 aattgcccct gttcagaagc ctgcatatgt aacctcttac attttgtgtt tcacaatttc
57121 cattgaactc ttcctacaca ccagaatggg caaaaatagg aatttaattt ctgggaagat
57181 ttcctctaag agcctctgtc caaaacatcc agtgctgaaa caagaaaagg tggtcctggc
57241 atatatccac tagtaatttt tttgtcttct tcttttcttt cctgttttat gtgtgaatag
57301 tgaaacttat cggttcaaag accgatgagt cagaatgctg tcattttaca tccaaacagt
57361 ggaaatttaa attgttaaaa taaaaataca caatcactaa cattaccctg atgaaataat
57421 gataagcaaa gtagtcttaa attacaggct ataagtgctg gataaagaac aatattttaa
57481 aaatatctaa agtcatataa tgaacagaag acagatttcc atttatacct gaaagactgg
57541 ttttcagaaa ttataataga acatggggat ttaaaaattg tatttggatc atttatttta
57601 cagttagaaa gtgatgtagg aaggagttcc cagaggagat gaaacagatt gaaaacagca
57661 aaattccatg acagaggtgg cttgaaatag accctttttt caaagcaata actggcctct
57721 tcatcatcta tagatagcat ttttggcaaa atcagaaaca tttgctttgc cagtcattga
57781 aggtaaaaag aaaaaaaaaa tggctgaatg cttttaaatt acatttattt aatttctgct
57841 ttcatacaaa ggcataaaat tatcagttta aattttaaaa aatattatta taaaattaca
57901 tgcttagaag tctgttgatt gttttcccac agccatttta taactgaaac cctcaatttt
57961 ggatatgaat gtataataaa cgcacatgtt cattctaaac attttctttt caaatacttt
58021 tacattcctt taggtttcct aaagaaatgt aaattccttc tgccacttag tttttgctat
58081 ttttagcagt ttcttaaagt ctgcaaattc ctttcactca gtttttgcac ttagttcata
58141 atgacttcac atttgacatt gattgtccac aaaaggggtt atttttagtt tcaaaaggtg
58201 aacgttattt gttttagaaa ttctcgcgta tatttcttag tctaaagata ggtttctaa
58261 cataaaaatg tgatagtgta gtctgtttgc tgagcacttg tagtggaaca aactcagtgc
58321 taaattcttg gtttttaata actaatttat ttattacagt aactgctaaa tatgcttcaa
58381 attataaact atttgccaaa attacagata ataatataac caaacaccat gtggatttca
58441 cctaaactag aagtaacttg gtgactttct tatttaatct tttatttatt gtacttggcc
58501 atacttattg tatgtttatg tataaaaaca taaagcaata gaagttttta cagttattat
58561 actcactgtc ttataaatgg agaaactgag acccagagaa ttttagtaac ttattcaaag
58621 ttacctagct ggtgactaga agcaaaacac aaataagacc acagattact agtttgtca
58681 agatcttcaa cataatttaa tattgctgtc aactcagagg tctcagaaga taaggtctta
 
58741 ccatctgaaa acaacaaaaa agaaatttca taaaatcctg tttgtaccaa gcaatttatt
58801 tctagaatga aagcaaaatt atttttttaa agttctttag attgctgaga aatcaagagc
58861 attagggttg tgtctcccct ttaaatttac cttttagttt ctttgggttt aagtttttag
58921 atgaaatttt aaattctatt tagctctcag tttatgtgta tttaaaattt agatttagga
58981 tctattcttt ttaataaggg tgaaattaca tatggagaaa tttactgttt atagcgagtt
59041 ttgacaacta gagttgtata accaccatcc ccacagtcaa gatacatcac ttctcccctt
59101 cccaaacata ttccctggtg acactttgtg gtcatactca ttctccgcct ctagcctttt
59161 acaatgactt ctgttactcc ccctgtagat ttgcctatta aagaatgtta tataaaggaa
59221 tcacacatca tgtagcattt tgaatctggc ttgctttact tgttttaatg catttgagat
59281 ttgttcctat tgtggcagat atcagtagta gtcttctttt cattgttgga tattccatta
59341 catgaatgta caatcacatt ttatccattt gcccagctaa agaacatttg ggtgttttca
59401 gttttcatta ttatgagtaa accctccata aacatattgc atacaaattt ttatgtgaac
59461 ataagttttt gtttcaccta aacagatact atacaggact gaaattttag taagtatatg
59521 tttaactttg tgagaaactg cccaactgtt tttgcaaagt ggctgtacag atttacattt
59581 tcaacagcaa catgtgagag ttccagttgc tgcacgtcct tgtgagcact tagggttgta
59641 tttttaattt tatccactct aatgggtgtg gagtggtgtc ttattaaggt tttaataagc
59701 atttctctaa tttaatgcat tatattactt ctttttgttg gtttgtaaac tgcttatctt
59761 ctttggtgag tttgatcaaa tcatttgtcc atgtttgtgt ttttgattat atttcagata
59821 caagtcttta ccagttatgt gctttgtaaa tgttttctca ctgtttatca tttgtgtgtt
59881 ttatggaaag cagaatattt cgattttctg gaaatataat ttgtcacttt aaaaattatg
59941 taaattgcta ttggtattaa gtgtaagaaa actttgcttc atctatgatc acaaatattt
60001 tcttctgaaa aaaaagctct ctgaagagtt tttttttttc aatgaatgtg ttttctttct
60061 tctctccata tgttatcttc tgaatagcta agcaatgttt acattattta ttcaggcctt
60121 tccatccact ttcacggatt ccactgaagg agaaattggg tttgaaatcc tcttttctca
60181 aaaactaatg agtcatgcat cttctcattg atattatctg gctcccctga aaacctctag
60241 tttggtccat ttttagtgat atctttctac tcaggttcct caaaccaatc tgtacctaag
60301 agacccccag attaaaattc aagttcattc ttttaacaaa ttaggtgcta attgctacaa
60361 atgcaagaaa acttcaggtg ctaatttgtt aaaagaatga acttgaactt tagtctgggg
60421 gtctcttaga tatagattga aaaggattga aaagaattat agtttctttc acttttgcca
60481 tcatacaata tgtattattt atatttcttc ttcgttaata ttcagttcac cttctttgtc
60541 catctttcta ttgaagcacc agtattcatg cagtgtttta agaatattag tccattttta
60601 tccattatgt atattgcaaa taatttcgta ctttattgtc ttttaatttg tttgcagtgt
60661 tttctgccat acaaaaattt tcattttatt ttatattcac tttaaaaagg cctttttcag
60721 cctaagagta taaatataat agcttttcta cttttttcat tttttaatat tgcattttct
60781 ctattgtgaa tgttatgctt atgtttttct ccagctgatc tattgaggaa cagtgttttt
60841 ttaataaata taaatattgt tatagttatt tctcatttcg aagttttacg gtactaattt
60901 gtctgtctta aaacttcaaa taatacttaa ttgaaagtct ctgtctacca tcatcccttt
60961 cttaacagtt cggtgaatat cttttattat attgtgtaag gatgttgtat tgcagctcct
 
61021 cttgctaggc tgaacatgca gacctgttct ggaaatagat gtgatcagtt taggtcacaa
61081 cctgtcttga atcacagctg acttgtagtg gagagttcca ggaaccttcc aatcacacac
61141 aggtccctat tgctcctttt cttctagggc tattcaacaa gcagagacaa acaaaaactg
61201 atctgtcaca ttttactgca ggtaacagtg accacaaata cacctgtaca ctgttttttt
61261 tccaacatta ttgaggtgtg actgacaaat aaaaatggta tatatttaag gtatgtgatg
61321 cgatgttttg ctgtacatgt acattgtgaa atgattatca taatcatgtt aacatatcca
61381 tcacctcaca tattgttatt tttataatca cttttataaa ttttcccttc agtagtaacc
61441 ttttgagatt ctgaaacact tttgagtttt gaatgttgtg tcataagtga gtttttttgg
61501 caaatatttt ataaacatct aattgggaaa ctgtgagagg atgttttgta actttcattt
61561 aaactaagat ctcctctcca cctccaccct tcccatattc agtagatttt ctatggctgt
61621 atggtgtatc aggaatcatc ccatatactt cagaaagtaa tggaataata taaatatttt
61681 tctaacatag acctacagag attaagtgat atactcaaat cataccataa atggtagtga
61741 aagggtaaac tcaacattca cagccaactt cattatctaa tagggaacaa cacatttcat
61801 attaataatt gacttttatt tttaggaggc tctgttataa tgaaattaaa aaatgagatt
61861 agattatgtt tctcaatata tggtacaaaa atcacttaca tagaataact tcgattatta
61921 aaaatagaag tgaaaaatac ttttcagatt aaataagatt tcaattttaa ggcaaatatg
61981 ttcattttgt gctcaaaaat tagacaggaa aactataatc tgttaaaatg taggttcttg
62041 atccacccaa gaaaccatgg agaggtgggg gacagttacc tgcatttctg acagttagtt
62101 cattgactct tattcgacta aactttgaga accagtgtcc aaagtattta cttacatatt
62161 tatatttaaa agctgggata atcatgacaa tataaaataa acaattgatg ttgtatttta
62221 cttggcagat agataaagct atacaagttt cctttttcct aattctaaag cataattaga
62281 aaaaaattgt atttatttct tagatatggt aactactaaa atataatttg gaatgttcta
62341 cttaaatgag tatacttatg tatgcagtta gccgtcaata agatttagtg ttctctgaac
62401 tatttcattt ctatgaaggt ggtattaatc ttgatgataa aattttcttt taaatattga
62461 gagattctgc caaaatataa atcttactgt gacaatgttg ttgaaattcc tttcttcata
62521 tgattatttg ttctaaaata gctcccatga aagtaaaatt cctactctca agcatcctgc
62581 ttaagcatat tgtattgttg ctggtaacta aaaataaagc aaagcattag ggtggatttc
62641 agtgtaattt tatctgttta gtagagtgat acaaatgaat aaattttcaa acatacaaat
62701 tgaccaatta tttgaaagag ctatgaataa aataaataag ctaccttaaa attattttaa
62761 aattatatca caaaattttt ttcctaacaa caaaatatgt atttttaatt gttttggttc
62821 atacaaatta aaatacaaat ataattttac caaggaaagt gaagtaattt aggagtaatt
62881 aataatattt gtgttaggga acggagcata gatactatta aacctaaccg ttttatagtg
62941 catttgtttc taaatccctt aagctggtaa gtcattgatc ttgcattttt ctttgacagg
63001 cttatgcaag caaagatctg gaggagcagt tacggtctgt gtccagtgta gatgaactca
63061 tgactgtact ctacccagaa tattggaaaa tgtacaagtg tcagctaagg aaaggaggct
63121 ggcaacataa cagagaacag gccaacctca actcaaggac agaagagact ataaaatttg
63181 ctgcagcaca ttataataca gagatcttga aaagtaagta tgggaaataa aatgtatagt
63241 aagccttata tatcaaacca gtagaagttg ccagcctggt tctttaattt cagtgtttgt
 
63301 atcaccgaat acacctttag ggcggaaagc aaatagaaaa taaagaatta cttcaaaata
63361 caacatttta tgatttcact ttgagtatta agatgacttc ataaaataca ggatagatag
63421 gtccatgcat aaacaacaca catttatttt ttcaggtgat agccattgga aaatttttaa
63481 aacagatggc tatattttac atactatatc tgtttaataa cttaaacatt ctttatctta
63541 tcaaataatt ataccttaat ttcctatctg atttggattg agaaatgctt catttgaaat
63601 caccttttca gatgtcactt gttgctttgc tgcttcacag ataactggct cagagcggga
63661 cttaagctga tgaggtatct agcataaagc ttcacaaaaa atgatcatgt tgcataaaat
63721 gtcatagcat caattggtgg ggtatgactg ggcacagagg ttcatgtctg taatccgaga
63781 ccattgggag gccaaagtgg gaggattgct tgaagccaga gtcagagacc agcctgaaca
63841 acacagcaag acctcatctc tattaaaaaa aattaaaaag tggtggagta atttaaaaac
63901 atatttgcct tcagttggta gctcttaatc gctataaaag tttgatattg gaaagtattt
63961 tgagaacaga ggcaaacaaa atggcttcat ggttgtataa ccaatagttt atgtcctttt
64021 ccaaaaattc tattttaggg gaaatatggg ataagtaaac tagtatactt aggaagtaca
64081 ttatttgtaa tgcagaattt aaaaagtcaa cagtattata ggtaagctac taactcatca
64141 gaaacaaaat tccagaggag agttaaaagg gataggaaat attgactttg ctgaagaaag
64201 ttcagaatga gtcccagttg gtgaaaaata gccttgtatt ttatgtagct gcagccacga
64261 cattactcaa ggttgccaaa tagtccgcct gaaaaggacg tttgacttga atctaaaacc
64321 atagctgtct tctcatatgc aactcagttt taaagaaaaa taaaattata tttatatttg
64381 cattgagagc acaaaaatgg cccatactat agagaatgaa catgactctc tctgcacatc
64441 ttataaacat tcatatatta aattaccatt ttcctaagaa agaaaaacca aacattagtg
64501 ttgtgggata ttggtgtact tttaataaag cataatgcta atttttctta ttgcctagct
64561 atagaaatga gtttaagaaa tatccttagc ttgtcttttc aatgttatat cattctcatt
64621 cacaagtcaa tttaggatcc catcagctgt ctctttagtt atatattgaa cttaaatgtt
64681 aaaatctgaa atgaatgtga gtgatttgca gctgctgtgg gcttttttta tcttggtggt
64741 ttccgtaaaa cttactgatt tccctatgac ttgacaggta ttgataatga gtggagaaag
64801 actcaatgca tgccacggga ggtgtgtata gatgtgggga aggagtttgg agtcgcgaca
64861 aacaccttct ttaaacctcc atgtgtgtcc gtctacagat gtgggggttg ctgcaatagt
64921 gaggggctgc agtgcatgaa caccagcacg agctacctca gcaagacggt gggtattgcc
64981 cattcctgct acctgcgaga gcccccttaa tcagaagctt taactttggg gatgatgtgt
65041 ttaaacttct ggttgcttta aactaacttg gttcaactca gggtctcata ttttctttag
65101 gggaatgaag tcagtttttc agtgttttgt gtgtttgaca acacagtgtc acaaagaaag
65161 taattgatgc tttattactt tgcaaactct tcatatagtg gagtcaaagc ctcatgcaga
65221 aaacaataaa tatacgtatg tatgtgtgta catgtacgtg tgtatgtgtg tgtgttgatc
65281 ttcaagaata aacatgttca tttggcaata aaatactttt aaatgggtga gttttcagat
65341 ggtggagata acagtgcaaa cagtattcaa agtgtaaaat taaatgcgtg tgttctgtga
65401 aacagtggta tagtattgaa ataagccatc tcactgaaga aatttattgt cttcctgtcg
65461 aaagcaaagg aaataactct gacactaaat ctcagatgtt ttgaataaac tgggaagttt
65521 atctcattaa atggtaagct tctgataacc aggaaatgtc tcttaaggtt ggctttggat
 
65581 cagggatttt cagaccagac acataaaccc gctttgtggg ccttacgtgg tagaggaggc
65641 tggctgtgga acacgcgtat gtgctggcga taaccatgaa agtgttgttt gaaattgtgt
65701 gcacctctgg cctctgaaca gaaatgtttg gagaaatcca gtcccgtggg ctggactgaa
65761 gcaaacttct cccgaaagac aaaactgtgg ttgtcctaat gacctttcta agctattttt
65821 ggagttagac tcacacacgt aaatctggct aaattcagaa ctgttctttg acctggagaa
65881 tgaaaaatga aattgaaaga tgtggtgtta gatttgattt gtatgatgtg gtatgacaga
65941 aaattcacat tttaatttta tgcaaacttt agctgatcta catggttttc agtaataaaa
66001 cctactctta tttcaatata gtattaatag aaatcagagt taagattact atctcaattc
66061 catgaactac attttttcag ctagctgaat atttccaaaa tttttactgt tacgatcatt
66121 tggcctctga gtttggagac attgtcttat tatacaaccc aaattaattg ctttcttttc
66181 aaaaattgag tttctttata ttgctcaccc acttgataaa gaaacaagcc attttagatt
66241 aagacttgca agaagaaaca aaaaataaat tataaagcta aattttctat tcagcttgcc
66301 tctgcctcaa ctctaaaggt taaaaaaaat taaaaggtga tgctttgggg cctgctatca
66361 tttcttttag tatcatagtt agttaagctt agccagatgc aattcattct gattatttcc
66421 taaaagcaca gcaaaataat gtaggggtcg ttccaacatg tgttctcaag gaatacaatg
66481 tgactctaaa catagtcctg catttagaaa ctttaccaga aaaatgtaca gttgtaggaa
66541 catgacaata tttcagtggt tgccaaaatt ataatatttt tgatagacta acatataatt
66601 atactattgg acatagcaaa agttatcctt agttcattta tcctccctga gtctttatca
66661 aacaacaata aacaataaga cgttatagtt ttgtacttac tattctaaat aaattaatac
66721 tactttaaca ggttttattg gtattatgta ttttatcata accctgaaat caaacaggat
66781 tctttaagat cctctaaatt ttagaactgc atactttaaa ataaattaaa tagaaatact
66841 taggccaagc agatattctt tgaaaggtaa tattcatttt taaaacatat taataattaa
66901 aatgttgttt ttatcttcaa gccaaacctc ttaatgggaa ttttggtata ctgagtctct
66961 tgagaaatat gtcatttcct tttaaagtta gagaaaataa atatggatga tttaggaatt
67021 gaaatataca atttttggaa tgccgttaat gatttatatt ttaaaataat tcagttaagc
67081 aatatgatgt attctagatt atattcctaa tatggtattt gatttaaatc aaatagtaaa
67141 ccaaggtact ttggatactg tttccaacta ataagataac cttctctctg ctcttcctcc
67201 ttccatttcc tttcttcata atcttttcta taaattactt tcaagcatat tttttaaaaa
67261 catagatcca aatctatggc taggcatggt ggctcacgcc tgtggtctca gctgctgagg
67321 aggcagaggc aggaggatcg ctggagccca ggagttcagg gctgcagtgt gctaggattg
67381 caccactgca ctcccagcct aggtgacaga gcatgaccct gtttcaaaaa caaaaaaacc
67441 ccacagagct gatgtcagtc tccagcataa aggcctttca gtgatttccc attgcttgta
67501 aaataagaca aaaatctttt ttgaaggtta tatttattga catatcatag ctgtacatat
67561 tttggggtca tgtgttatat gttgatacct gtacaaaatg tgtaatgatc aaatcagggt
67621 aattgggata tccatcatct caaacattta tcttttcttt ctattgggaa caatacagta
67681 cttctcttgc agctttttaa aaaatataca atatatcgtt attcactcca atttccctga
67741 tgtactattg aacactaaaa cttactcctt ctatttaact gtgcttttgt acccctcaac
67801 catcctctct ccatcctccc tacccttgcc cctcccagcc tctgataatc agtattcttc
 
67861 tctctacttc catgagatcc actcgtttag ctcccacata aatgagaaca tgcaggattt
67921 tcaagaccac atctttatca caacctgcaa tctctgcccc atagcccctg ttcagcatca
67981 tctaatgtaa tgattcttga tcttgtgctt ccagtgctct agactttctc caattcctgg
68041 gcatcatcgt gtttcctctg ctcctgggcc cttgcacaca cacctttagg ttaacctggc
68101 cttcctgtct cctctttcct gaggtgctag ctcttcagat cttaatttgg gtgtcaagtg
68161 gctttggcaa ggcctcaggc aagactttct gacctatttg gattagaccc aatatttgta
68221 gtaaatgctt tatagtttcc catattttta aaatagtatt tattacagcc tataatcaca
68281 tagtttatag ttatgtagtc attgttactt gattaatgtc tccctcctcc acaaaggcaa
68341 aaacagatcc gttcactact tatcccagtg cctaccacag aatcagtgca caaatctctt
68401 ttagtatcac ataaaaactg tggacccttt catgagaaca aatgaaaata tagacacatc
68461 cacagcattt ccccccacag ttttagggag tttgttccct gacccctagc cttttccctt
68521 taaaccaggc tttaaaactg ctgccttgaa gattattaat aataaaatag ctgattaaca
68581 cacttgtgga attttcaaat aattggtctt tttgctaaac aaacgtttat gctatcaatt
68641 tagcttgatc ttttaaaaaa tatctgtttc tggagaggaa cagaaaaggt aactattggg
68701 tactgggctt aattcctggg tgatgaaata atctgtgcaa caacccccat gacatgagtt
68761 tacctatgtc acaaacctgc acatgcaccc ccaaacctaa aataaaagtt tacctattta
68821 acaaacctgc acatggaccc ccaaacctaa aataaaagtt tacctatgta acaaacctgc
68881 acatggaccc ccaaacctaa aataaaaccc caaacctaaa ataaaagttt atgtcacaaa
68941 cctgcacatg cacccccaaa cctaaaataa aaccccaaac ctaaaataaa agtttaccta
69001 tgtcacaaac ctgcacatgc acccccaaac ctaaaataaa agtttacctg tgtcgcaaat
69061 ctgcatatgc accgcgaaac ctaaaataaa agtttaccta tgtcacaaat ctgcacatgg
69121 acccccaaac ctaaaataaa agtttaccta tgtcacaaac ctgcacatgg acccccaaac
69181 ctaaaataaa accccaaact taaaataaaa gtttacctat gtcacaaacc tgcacatgga
69241 cccacaaacc tgcacatgga cccccaaacc taaaataaaa ccccaaacct aaaataaaag
69301 tttacctatg tcacaaacct gcacatgcac ccccaaacct aaaataaaag tttacgtatg
69361 taacaaacct gcacatggac ccccaaacct aaatataaac tttatatatg tatgtatata
69421 tatacatatg tatatacaga tatacatata tgtatctgta aaagaaacat atctatctat
69481 atctatatct agatatccag atgactagat gagatataga tatctgtttc tcttacagat
69541 aaggaagaaa attcttcttt attctctcat tcattagtta atatgagcaa aagccaacat
69601 tatatctttc ttactattgg tcaaccaaat aaatgcatac tttacatatg ggccaaatag
69661 atacctaagc ataccttagg taaaatctaa acttggaaaa cagaaaatca gtacattgtt
69721 agtaagcaaa gtaaataaat ttagtcttct attaaccatt tgagtttttt ctgtgaccat
69781 tgcatattca ttttctgcat tggtctaatt aggtgtcaca ttcacccagt gggtattgga
69841 gtgaaagtat ttattcaagg gtaggtgtgt atgctcagta gcataaaaat tgctctatca
69901 agagggaggt gttggagagg tttaggcttt agaaattgca aggcttcgct gggcactgtg
69961 gcttacacct gtaatcccag cactttggga tgccaagaca ggcagatctc ttgagcttag
70021 gaattggaga ccagcctggg caacatagtg aaaccctgtc tccccaaaaa agtataaaaa
70081 ttagccaggc atggtgttgt gcacctgtgg tcccagctac gcgggaggta gctgaggtgg
 
70141 gaggattgct ggatcctgga aggcagaggc tgcagtgagt aaagatcgca gcactgcact
70201 ccagcctggg tgacagagcc agaccctgcc tcaaaaaaaa aaaaaacaaa aaaaaaacaa
70261 aagaaaaaaa ttacaagggg ccaggggtaa gatgagcatt tagtgctaag tgtaagctgc
70321 atataggtga aataattttg cccagtctac ttagtactta aacatcagat atttgctata
70381 agaaaattgt ctactttaga tcacatggaa tcttttcttg ctgtatttca tctctttcac
70441 ctcccagctt tgcagaattt aggcttactt ttataataga tttacaatgt tgatatgagt
70501 tttgtcaact tcttcccaat catttgcttg tgtgaaaaat caggacttgc ccatgaaatt
70561 tctgaagttt gtctgctttt ttgttattgg ttgatttttt tttcagtttt ttttctatcc
70621 tattggtggg ctatatattt acaataatca caatatttat acattctgat ctgtaatgca
70681 gtcatatttt aaaatttata tagcattagt ttttattttg tcattttaat tttttaaaga
70741 tttgaagatt ttgagaatta aaaatttgta gttcatcaaa aatatggtat ttctcctgag
70801 aattctgacc taagacacct tctctgcaga ctgtaactct ccttgtattt tctcatctac
70861 ttttttttac ttttcccaaa cctttccctc caattcaggc ttctttcctg atttctatac
70921 tgtccactca accttgtgac ttggctgtcc ctcagacagg tcagtctgtc tcacttaaca
70981 gttagaacct attccctacc accttgccca aataagcaag taagtgaatc ctattctttt
71041 tccagtgttc tgtgtcttag taaatgacat catcattcag ccagtcggtc aagccaagtt
71101 attcttcctt tcaccaaaac taattgctat tcaatctacc ttgcaaacat ctctaggacc
71161 tatccagtgt ctccacgctg ctaatcccgc tctgatctag gtcacattta ccccttacct
71221 agaccattcc tcttggacaa gcctaataac tggtctccta cctccattct tcctttcctc
71281 caatctattt gccacttttt agcagtgatt tttttttctt ttcttaatgc taagtcagat
71341 actgctaaaa actccttgat aacattgcct tattgggatg gagccagcat tcaactagtg
71401 tggtgataag cttatctttt ctaattgggt tgcttaggcc agcatctggt cagattgatt
71461 ggtacaaatg ttctaagttg ttagtgtttt gaatggaact cttctttcag acaaaatccc
71521 ttaaacataa tttcctgggc cctcaatgat ctggatgcta cctaagtgtc cagcctcaaa
71581 tgtcaatgct tttccccaca aacactactt accactaaga cccaaattct tttcacttct
71641 tgggttatgt caatatctgt cacctccagt attgcaatgc tgtactctgt gtttgaaaca
71701 tctccctaca catgatcccg tatctcgatg tatctagtta atgtctactc atccttaggc
71761 ctaaacataa atgtgacttc caggacattc tgttatgtgc ttccacagca tccattgttg
71821 ctgtatctta gcatcattac cttatctgtc agattgtttt atctttgttt aattccctag
71881 gttttttttt tttttttttt tttagatgga gtctcactct tgttgcccag gtgggggagt
71941 gcagtggcag gaactcagct cactgtgacc tctgcctcct gggttcaagt gattatcttg
72001 cctcagcctc ccaagtagct gggattgcag gtgtgcacca ccacacctgg ctaatttttt
72061 gtatttgtag tagagatggg gtttcaccat gttagccagg ctggtctaga aactcctgac
72121 ctcaggtgat cctcccacct ctgcctccca aagtgctggg attacaggtg tgagccactg
72181 cacccagtct tattccatag gttctagact ccatgaaaac aaagattgcg tctgcttgtt
72241 acactgaaca atagcacagt agttggcaca tagcaaacac ttagaagtga ataaaatgaa
72301 tatttgaata aaaagaaaca aatttggggt caaaggtcaa attttgaata tgctgatttg
72361 aaagagtctg aatggtgttt aggtggagat accagaaggt ttgatgtaca attctaaaat
 
72421 tttagaacaa taactgggtc tttaaatata gatttgggag gaagcatcat tctgataaat
72481 gtcatttgaa gcctggtgtg cttattaatt cttagggtat tgatgtatca caagagaaga
72541 gcaaacatca gtaccaaggg aaagccaaca ttcatcatta gctgtgccct gcatttccct
72601 ctttgatttt gtattatcat gcccttttcc tatccaaagg attcgcttat attttgttt
72661 atagtataag gaattttttt ttttttgaaa cggagtctca ctcttgtcac ccaggctgga
72721 gtgcaatggt gtgatcttgg ctcactgcaa cctccacctc ctgggttcaa gcgattctcc
72781 tgcctcagcc tcccgagtag ctgggattac aggtgtccac caccacgccc agctgatttt
72841 gtattttaat agagataggt tttcaccacg ttggccaggc tggtctcgaa ctcctgactt
72901 caagtgatcc actcgcctcg gccacccaat gtgctgggat tacaggcatg agccaccgca
72961 cccggcagat agttttaatt taaattcagt tagacaagtg catgtggtac acccattatg
73021 tgacgcttca tattatgtac taaaagcatg ctatgcacaa agtgtaggtg tacagtgatg
73081 gatagatcct gccctatgga actaacaaat tagcaagcga aatcaacatt tatcaagtaa
73141 ttaaaagtag tttaagtgtt gtgaaggaaa agtacaaagg cttatggaag tgcatatcca
73201 ggatgtttat aaaccaaggg aggtcgaggc ataaaatttc tgaaagcaag aatgcagtgt
73261 tcctacattt ttactaatat tccaagttct gaaaacgtgt acttaagtag cagcttttcc
73321 tgagtttatt caatttattg cattatcagt gtagtatgaa tgggagtgtg tgatgatttc
73381 cttcaaatat aaaaggaaat ttcttaatgc tgtgttttct taaggtaaaa aaaaacaaaa
73441 aaaagacttt tcgttttgtt aaaagagttc caggcacatt ttgtagcaaa gccaccaatt
73501 ggcatgatgt aaattgtaag gacaatgtac cttaggtaat gttaaactca agtttttat
73561 agatacttca tagaaatgct tttttaaaat gggactcaat gaaggggagg ttatcagggc
73621 gtgtgaggaa gccagctgtt cattgaaaag taaaagcata tgcactgtgc attccaaagt
73681 atactaatgg ataaaaactg cttcaaagtt ctccacacaa gtatctaagt ggctttgaaa
73741 agttaagtag attggatgtc ttttcaacct ctttcataaa catacacttt gagtatattt
73801 tcttatgaga agttgtatgt ttaagagata taaccaaaaa gtagaaaatg aatttgcag
73861 ggggtaaatt atttggtgat gaaagcagaa gcagaagaaa gtcatctgct tttgaggcac
73921 acatttggat taaaagttac ttgaatgtta gtatttacct tatatatttt actaaatcta
73981 ggaaagaata aagaaaaaca gctcaatctc tagagtcttc ctgaaaatgg tggggttaaa
74041 tcaagctcaa ggaactagaa gttctttgct gtgttcatta gtgccagact aaagaagcca
74101 gtttagttaa gaagctagct tgctatgagc taattctgat acatagaagc agcattattt
74161 taaattctac attgccatct taaaaacaag cagctacttc tttttcatta ttttataaag
74221 caataatatg agcaatcaat aatatgagca tgtatattaa caaagacatg ctcgattata
74281 tttaagttgg atgtttcata aaattttaca acaaaaaata tttaaaccta agtaaaatat
74341 gcaactatat aatgaaatta gaggtattat tttgcactgc agtcagagat atttcttcca
74401 ttaattaaac agaatactgc agcagtctgg aagattaatt tattacattc ctttgtattc
74461 agcaagtgga acgtgatttg tattgtatct ttagcaagtt aatatttggc aagcacagtt
74521 tttggaaatc aggttttatc ctgatgtgga aaaagaactc tatgccagta ccagttatca
74581 gaactttctt taagaataac atttgcctta agaacataat taatgcatct gtgaaaaata
74641 ccactttttt ccaacccaag gtacaatgag attcacgtat tttattcagt atttctaaaa
 
74701 ctcttcaatt gaggttttta ttttaattgt ggtactgtgt atacttttta tgcataacaa
74761 tatgttaaaa ttaattcagt accagattta aatctgttag gctatctgtt attccatttt
74821 ctttcccaaa cccaatttaa gttccacagg ccttttatag ctggccatgt ggttgcaatg
74881 ctaagtcaaa atgctaagac cttttcccat tttggggcat tatttcttta aattcgttgt
74941 agttttaaaa tctgatttta tagttattaa tcatattctt ctgggggtac atatatctgt
75001 acttaatgga ctaaagtttg gaatatttaa atttaaatta aatatggtag tgagatttgt
75061 tctgaagaga aactggttat atttaggata taatggtgtt ttaaaggaag gcatttttg
75121 tgtcatattt gtatccacac aaacattact tataatttag tactaaatta taactcaaga
75181 tcctctcagt aaggaagttc tacacctttt tcttgaatag ccagcaagat aagtacgatt
75241 aggaattgtt gctttatagc cgttgctatg ctgctgtaaa caacctattg tttcaaaatg
75301 tggctttatt tgaagtccat tcttgttggt gagaattgac tattaaatat tcctattaaa
75361 gaatgccagt agattacaac tgggaacctc tcagttggaa acacttgcca tttcctcttg
75421 tttattatag ttttacagaa tatacagtca gatttttttt tcagtttcca accttgctta
75481 aatgcctttt tttatatgtc cttttgatct tctccaaaac caacaactgg tattatatgc
75541 ctaaagtata tgtcaggctt cataatgaat tgtattatta caactttttt tgtgtcctac
75601 attggacaca agttaatatt acttattggt tcagaacaat tcactttatt gtataaaact
75661 gttttagaaa aatcataatt ttcaaaatat caaattataa tagatacatt atcagttaaa
75721 tacatcatca atcagtattt actaaataaa catacttgga gctattagct agtgctgaat
75781 aatacataaa ttttatgtgc tatgaactta aatgtagaac agaaaatact attatataca
75841 caataaagca caaaatagtc tgacaatagt atcatgattc atattttctt aaaataatct
75901 acttactgtt tcaaacattg atatgtttgg atagatctaa actcttttga gaggaaattc
75961 tagttactat gttagttcag atcattatta atggcaagaa caaattgcat aagggaatta
76021 aactccagtg tttacggtaa gctatttggt atttcagaaa ttctaccata gtatctctct
76081 ctgtgtatat gtatgttctc attcaccctc accgtgaggt ctcttttttt ttcttttct
76141 tttttttttt tttttttttt tcggagaggg agtccacctc tgttggccag gctggagtgc
76201 aggtgcagtg gtgcaatctg ggctcactgc aacctctgcc tcccgggttc aagaaactct
76261 cctgcctcag cctcccaagt agctgggatt acaggcatcc gccacgccac ccagctaatt
76321 ttagtatttt tagtagaaac gggatttcac catgttgggc cggctggtct tgaactcctg
76381 acctcaagtg atcctcccac ctcagcctcc caaagtgctg ggttacaggc cgtgagtcac
76441 catgccccgc cctcattgtt aggtttctga tcagcataga ggcaaggtca ttactgatga
76501 ctgttatcct gagattctag caaggtgctt ggtatcatgt aggtactgaa taaatagtta
76561 ctgaaaacta aacaaggaaa gattttaaat aaaaatttta aaatttagaa tttctttaac
76621 aaaaatttaa ctaaatcttg gctgatgtga atgatgataa agaacaagag aaagaagagg
76681 aggaagagaa atccaaacat tacactgaaa tcaccattat tattatccct attttatagg
76741 taatgaaatg gaggcttggg gaagttaggt aatttttcca agtctcagaa gtagtaaatt
76801 gaagatccag gactctaatt actgtctgtt tctcaagact aagtaaactc ttgtttattc
76861 ttacatactt cgtggagtag ggaagttgcc agtgatttga aaatgttatg tatttatttt
76921 gggagggcgc catgcaagtt tataatgcac ttctaattat tattttttca gttcaaaaaa
 
76981 tgtatatttt ctaagcacat actatctagg aaaaaaagct tacggaaatt cttagtaacg
77041 tagggattaa atatagctat gtaaaaattc acaaagtggt ctgtaaacaa gaaaatagaa
77101 tttactatat ttattgaaaa ggcagtagaa actgtaatca tgtctaactg ataagagtaa
77161 gtcagtcaat atacacacat gagaccagca cagaaagaat tagaaaaatt aaagaggcct
77221 ctttagtatg tgacaagagc tggtataggt acaattatat cttaatacat acccagatgg
77281 catattccca gtagatatta ctaattttcc tgaagttgat agataaataa aactcaatta
77341 tcaagactct ttctactttt aatattatca aaggtatctt tctctggact cttaaaattt
77401 tttcacactg gtatcatcaa gtagcaggtc aaaatttagg gatagaggat gtgtttcaag
77461 agaagaacaa aattttttaa ctttaatttt tcaatctctg ttccttttga caaaattaac
77521 tgaattcagc ctgccaattg ctacttagac acaaaaaaga gaaatgagac attagttatt
77581 aagattttga ttttgattcc tttatgttgt gatttccttt atttccttta agtagttaaa
77641 ctgcaacaaa gatttttttc tctccaactg taaaaaagtc ttccatttta ccacttataa
77701 aagtcttcaa cttagaggaa aagacagttc tctaaacagt gatttggttt tagtgctgtt
77761 ttacctgtct ccttattctt ttagtcagtg tattcaaaaa cacatttctt gcagcctttg
77821 aaagcataga gccacactta gttttccagg aagatgcttt cttaatgtga attagctatt
77881 cttcatctca ccagaacatt cttcaaagct gctgttcctg aattcatgct tgatacccat
77941 gggtggatcc tgatttgtga acattatcta cctaggcatg gatgaggtct ctccatgatc
78001 actgatactg ctcacggcaa tctgtctacc atttggaaaa caggctgggg cagaatctgg
78061 aagggcagcc atcagtggga gcaggaatga tgctgccaag ggttaggggt gggaaaacat
78121 aaactcttct acaatagctg ttagttatct caaatagtgc cttaccttca ctatcaggct
78181 ctgtcctgcc tggtttcact ggagacaatg ccaaatcctg ctgtgcttac ttattacttt
78241 gtttttgcat tgctataaag gaatacctaa gactgggtag tttataaaga aaataggctt
78301 aattggctta tggttctgca ggctgtacag gaagcatgat gctggcattt gctcaccttc
78361 tggtgatgcc tcaggaagct tgcaatcttg gtggaagggg gcgctggtgt gtcatatggc
78421 cagagtgtga gcaagagagg gaagggagag gtctcagact cctttaaaca accagctcta
78481 gcatgaacta acaggaaaat ctcacttctt accgaaggat ggtgctaagc cattcatgag
78541 ggatctggtc ccatgaccca ttcatctccc gccagctccc acctctaaca ttgggaatca
78601 catttcaaca tgagatttgg aggagacaca gatccaaacc atatcagatt acaaacactg
78661 tgcgtgtaag caaaattgtt tctgctggtc tgagccactt gagccctttc ccactagtca
78721 catggagcac ctcttcttgt cccccgtctc cactgctgct tccttcctac agcccctgcc
78781 ctgccacagc ccttggaacc acatcgggtg ctggttatct cctgtcaatt cttgttacat
78841 ttgacttccc gaagacatgt ggccctgtga ccacacacac ctgtttgaag ctctcccctc
78901 tttctctggc acccgttggc ttgacagtct cccctcctga tgctccatca ttgttatgct
78961 tcttcccaga catattcact agttcctttg cctcttccca cctcatagat gttcctgtgc
79021 cccaggtttc tattcttaga cattttctct tttcatttag agtgctcatc aagtaatctc
79081 atcaatgtcc aagactccaa ttacagctta aatgttcatg acttttcaat ggctctccta
79141 aactctagac acacatatgt gactgcctac tagacctaaa ttcttaggta caccttccta
79201 ccatctacat tattgcagag cttaaataat caaattataa attatacaaa tataaaaaat
 
79261 aagtaatttt ataaatttat taaaaattgc tttttaaaac cactttgtat gtcttctgta
79321 gtgctagttc ttaatataga ttccatagat gtcttaaaag atagaaaaat tgtagcttat
79381 gaagacactg cagaattctc tcttaaaata ggtatatata gtgcaaagga ttactgccat
79441 tgcaatattt cacctaatca aagattttat ccaacgtgta taaacgggta tctttttaga
79501 taagaaactg gtcaaaatca taatagtggc atatgttgag gtcacatagt gaagttatct
79561 atgcagatca gacagtattg tggagaatac ctagaatctt gagaatttac acaaatagta
79621 ataacttaga tctgtatagc aaataatata catatcagat tcatttagtt tatgtaatct
79681 ttataatctg agaataatga gtcaagtgtt atccttagtt tactgattat caaagaaaaa
79741 tacagcttgg agaagtcaaa tgaattttcc aaatttatgc tacttataaa tgggagagct
79801 aataaaagcc acctgtctgg gtataaccct gtacctattt cttctaaagc atattgtttt
79861 ctaaagaaag tagttacatt ttaaatgaat atataaggaa tacatcttga atgaagacaa
79921 tatgcatttt ataatttaca tatttctgta tttcattatt cagaattcat ttaaaaagtc
79981 tccttgttgg catggacaaa attggcagct aatcctatcc cataaatatt ttcaatggtc
80041 ttcagctgat ggcgtgaaag ttcagcaaag gccaagtcac aatgctttga agtttgtcat
80101 cttttttatc actgctcaga ttgtcatgaa gacaccaagt aatacatgga tcaattatag
80161 ttaaatgact gcaagttgat cttatcaagc agttagtggg caagatttca cttttaccat
80221 tttttctggt aatgatttag agtgtaagaa cactaggaat ttaaatctac ttagttttac
80281 ttaatcaatg atgtatttgt ttatttaatt taaaacattg tttttggaga aggaagagaa
80341 cacaagaagt tcttgatcca taaataaggc gttcagaagg agaccagtta aactaaattt
80401 tgactgtaat tcagaattgt gtaaaatgcc ctactttatt ttaagctcat tattttcata
80461 attcctttac caagatagca gaaacatggc tttatctatt aaattataaa tgtagttttt
80521 tttgttttaa acataatcaa tttataagag atctaagaca tataaaacta cattgaggta
80581 gaaatttaat aaaaattaac tttacatcta tgtaacttac gtctatgtat tcagtttatt
80641 taaagtaggt cataaccaag tatttctggg ttgtgtggcc aatagctttg ctcctaaatc
80701 acaaagatgt ttctgtgtgt gacacttttt aaatccctgg aatattttaa atgttcattt
80761 caaataatct ataactttga aacagttctt ttttcttgaa cacattgttc tgtcacgtct
80821 aataggataa ccaattagtt ggttatccta attctaatat gcttcataac tagtgacata
80881 agataaacat atggagtatt tattactctg ataaaagata ctggttagga gcaagacagt
80941 ttttactttg taatcataaa aacatagcgt cctgcgtaca ctaggcagct acttatatag
81001 agctaatctc actctttttc ggactccaat atttaccata aaagtgcttt acagcatcta
81061 tataattttt tcttcttttt gtcccttctt tgtagttatt tgaaattaca gtgcctctct
81121 ctcaaggccc caaaccagta acaatcagtt ttgccaatca cacttcctgc cgatgcatgt
81181 ctaaactgga tgtttacaga caagttcatt ccattattag acgttccctg ccagcaacac
81241 taccacagtg agtatgaatt aaatctattt tttctgcatc ctctactaat attaaattat
81301 tttcaagtaa acataagtta aacaaataac ctctgtgaaa tttactgtaa tatactctgc
81361 ttcaaataca aaacaaagac aaaattttgt atagtataca atgaaaataa actgaaaatt
81421 aaaattcagt gggaagcgta ttttagaggc aaagtaattc acaatataaa agaagtatgt
81481 ctgtgaaata aaacacattc caccagtgtt cataaaatat cagtaaggta gcttaattga
 
81541 tttaacattc ctgaaatcag gtatgctaac tcataaatca tattaaagtt caaaacattt
81601 cagccttgat aagattttag gccaaaacaa aaagtaaata taaataattt tatatctatt
81661 agcattcagt tgctttcttc aatctcaaac ataaaaatat atatgtgtgt gtgtgtatat
81721 gtgtatatat gtatctccat gtttcttatt tcagcaatat ttgaatagtg aaatagtgct
81781 taattacttg aaaaaaattt ctgttaatat catttcaaat tgacattcaa accattattt
81841 gttattcagg tttcctttcc atcctatttt tttggagggg caaatggcag catatacctc
81901 atgtgatcat ataggagaac ttatcacgtt ctcctgtatg atcatgttct gaccctgtga
81961 atagctgata atgaattaat atgcattact gcctaagatc tgagagtttt ctgccccaaa
82021 gtagggatgc aaccctttgt tccttaggat atccggtgca tatggatgaa acttacataa
82081 ctctagcatt tgtaacaaat gtataaataa attgatcaaa atacatatat attacaaaaa
82141 agaaaacctt acatttttaa aagactcaac aataaatgta atatgcaaac catggttttc
82201 cccactgaat cacgttgtac tactctgctt agtgtggcag atagggggac cccttgtttg
82261 tccagggaca gtttaggaaa acctggaaga tagcctaatt tgagtgacag agatctgtcc
82321 ccttttatgt gagaaagcaa agatcttaca gacatttgtg aataaatgta tgctaataca
82381 agcaagagac cactaccatc tgaactcaag aggatgccgt atgtgcaatt tcatccccgc
82441 aggctagaga gttctgttca ccttcctcag ccctgagcag aagccctcag tgactcctcc
82501 attctgagag ctttagccat tatctccata ctcatggctt ccaaaatgac atttgaagat
82561 ctaaccatat ttttgactta taatatttta tctaaatcta tcttctattg ctttgcttgt
82621 aattatatta gctgtaagta caaagagaaa gacttgttaa atcctttatt tctacattat
82681 tcatggcagt gttttctcat tttctttgag gccttgaaat cacagagttg tcattacttc
82741 ttcttcttct tctatcccat ctaaacacaa ttgattattt tggaggaggg ggagatatgt
82801 gttccaagct aaacttatat ttcatttcca tcactgccat tatcatccac ttcattatgc
82861 atgtctctga tctaatgcag tgatattaat actagttttc aagcatcaag tctttctcct
82921 gttgtataat gtcactgttt tatgaagtaa gctttaaaag gcatcgtttc attcatctca
82981 ccagaaacag tgactttcag ttgctcaaag aactgagcac aaatttctca ccaccctatt
83041 ctggtgtcag ccttcctttc ttgttttgtc ttccattatt cacccagtct gtgtttcacc
83101 aagtccgatt cccagaacgc caagctcatt tcctgctctg tatcttgttt ctattctggc
83161 cctggcttgt gttctgcctt tccaaagccc tctccatttc tcagagttca ggtaaaggtc
83221 tttctcctgc atttgtcttg tccgaatacc acggccaact ttgcttcttg aatacccata
83281 gcactcactg gattaacttc atcctcacat ggctttatca gggtctgcaa atgttgcatg
83341 tggaactgga ttatgagagt gtttccagta agtttatatt gtctgcaaca ttcagctaac
83401 aacactcaaa aatacttgag ccattcctgt gctctcagcc cctttacctg cattgtctga
83461 tttaaccctt aacaaaatca tctagaaggt cagcagcagc atttgcctta ttttacagat
83521 aaaggtggaa atcacactcc aaagctgttg gctaaataat gtacataaat gcttgttaaa
83581 taaaatgact catttcctgg atgtatttta tagcatttgc catgttttca tcattactta
83641 ttcatataac tactatccta tacactgttt gaggtcaaga agagacagga agctacagca
83701 aaaagagcct gagtcttaga gtaagccata cctgattctg aattcagatt tgccactaat
83761 tcattctctg tgatcaggta ggtgttttat gtctctgaaa ctcagtttac ttatatataa
 
83821 actgtaaaga tgtaaagagg attgaatgaa gtactgaatg cttatgcaat gcttagcaga
83881 gtgtctggta tccagaaggt agtcattaaa gggcaaattt attattacca ttaatatcta
83941 agtctgattc atttcctcac ccatgtgttt ttcagtattt ttaaaaccac tatctacagt
84001 aagaaaaaca ttttatattc caaaccagga tgtgtggtat acacacacat agtgtgtctc
84061 tgtgtatata tatatgtcga tattagagaa ttttaaatta gctagataac ttcacatgta
84121 attaattatg tataagcata aaactaaaac aaattaccat ttaaaaatat tgtccttaat
84181 tatggtgctg ctcaagcttt ctcattatca caaccccaag gatcctttta taaacatttt
84241 ttccctaatc actttcccca taaaatttta atgtaacaaa tatactctgt ctttatacta
84301 tatgtatctc tgtgctttat acataaaaag gaagtgtaaa gtttactttt ttgttcaatg
84361 tagtgttaaa taacaaataa aatgtttaag ttaacattta aattaaaaat ttttaatctc
84421 ttaacagcta acataaattt ataattgtat ttgccaggaa acttttaagg taatctgttt
84481 acttatataa attgtaatat atcaaattac atatgcaaag taacaattga tatgaatata
84541 taatacaaat ataataattt ataaaaacat tcaaaatcac tatttttcca gcaaaagtaa
84601 aataattttt aaaataactt ctttaaaaat atttggagag cttaactatt aactttgctc
84661 agtatgagaa aacttttaac ttatatagat gataaatatt aatttagata ttgtcaactt
84721 tttcttttca tcatttgaca tgtaatggta tgactaacat ttgtagaatt aataataaaa
84781 tataaattat tattatttaa ttctcaaagc tcaagtcaca catgaaaaca ccaaggacca
84841 agcataagca atatccacag ggcaaccaat ggcagccaat aggcccacat aggtaccact
84901 cagtatatgt ttgaaatcac tcagtcaatt aagagaaaat cctccagtca tagccatcta
84961 gtcaccataa tttcatagct catatattgc aaacacttgg ggtgtgttcc gttgccaatg
85021 ctgttcatgt aatggccaat aaaaccaaat agaatttaag tattaaggaa tattattttc
85081 ttgggttttg gagagccaaa aatcattgag atattaagat tttagcccca tccaggcctc
85141 atacccaatt tttaccccct tagagatgat tttactccct ttatgaatat ttgccttatt
85201 attacgtgtg atatacccaa ataatttttc tattctgttt ttctttgtga aaatattggt
85261 ctcaaccctt tatgaattat ggcccacaat tcaataaaag ctacactagt gataatatat
85321 atatgatgga catgtgacta tgttataaaa ttctaacatt tgttaagatt taagaggaac
85381 aatcaccacc acggtggcta aaattaaaaa cactgacaaa aacaagtgtt agaatgtggt
85441 gcaaccaaaa ctcctatgtc accaatgggc attaaaattg ttcaatcatt tttcaaaaca
85501 gttgacagtt tcttatgaat ttaacatgta catatttcca cacaataact tgtgtacaaa
85561 tgttctttat tagtcaataa ctggaaacaa ctcaaatgcc catgaaagat gagtggatta
85621 acacatctta gtatatgcat acagtggaac attgctgagc aatgtaaaaa gaatgaacta
85681 ctgatacaca caagaacata aaaggatctc agagacatta agttgagtga aataagcttg
85741 gctaaaaaga gtacattctc tatgatttga tttacatgag gcccttaaaa gacgcaaatg
85801 tactctagag tgagagaacg caattcagtg tttgtttgag gatttggatc aggattgact
85861 gacaatgggc aagagagaca ttgtgaggtg ttagaaacat tctttatttt gattgtgaca
85921 gtagttaatg tggatacata aacttacgaa aagatgataa attcaaaata ggagcatgtt
85981 attgtatgca aattattccc caataaaatt gatttttgaa aagcctagaa aagaattcct
86041 ttattaagta caatttttta agtagcttag gtttcaaata gtagatttct cttttttttt
 
86101 tttttttttt tttttttttt gagacagatt ctcactctat cacccaggct ggagtacaat
86161 gacgtgatct cagctcactg caacctcctc ctcctgggtt caagcaattc tctgcctcat
86221 cctcccgagt agctgtgatt acagttgccc gccaccatgc ctggttaatt tttgtatttt
86281 tagtagagat ggggtttcac cattctggcc aggctggtct tgaactcctg accttgtgat
86341 ctacccgcct cggcctccca aagtgctggg attgcaggca tgagccacca cgcccagcca
86401 aaatagtaga ttttttaaaa cctttacaat aacattttaa gtcaaatctt tgattaggga
86461 gggagatttc ccacagtcat ccaaaaacta tacgttgagt cactaagata aagaatgctt
86521 ctagatccta ttagagaaaa caaagatgcg cttggctggc tctgaactta gagcacttat
86581 agtcaagtaa gaacaacaca tattatagtt acattattat tgttttattt tactaaatat
86641 tacaaagcac caagcatctg ccacacattg tctcatgttc atacaaatac tgccggtggc
86701 tccattcgta atacacatca catagtacaa ggctgaagag tgtggatgct gtagggagct
86761 tccagttttg agaatttagt ctctgatttc tctttgagtt tgagtaggtt ctgtaaactt
86821 tcttgtgaca catttttttt aatctacaaa agagcacata agtaagaatg ttacatataa
86881 catcatacag aatatttata agctaatctt cactgaaatc aatctgttca atagcattat
86941 accatatttg acataccata gccatgttaa tctgatattg tagaatagca tagtataata
87001 ataataactc ctaactcaag gatgttgtga tctttataac cagcaatcca tgttaaatat
87061 tagcacagtg cctaaaacat attaagcatt caataaatga tcgctactat ttttactaac
87121 atcctacaga tttggaaatt gagtcttaga aatgttaatg tgtaaaatgc taaagagcca
87181 aaaaaactgc caggaaaata taaaaattaa aatcatttta tttctgaaac ccatgtgttc
87241 cccccaatat cttctaacat ttctagtatt tacagaaaaa ctttcaagtc tcaatatcag
87301 aaagtttcat aaaagccaga ggaagtagca attctcttta gcagacagag ttagatacca
87361 attttcatat tggtgttctc acagattatt ttttccaatt atttcctctc atattttctt
87421 caaatttaac tccatgtatt tccatgggcc ctttagtaga gactttcttt tcatggaggg
87481 agtcatttgt gattaagtag cgatccccta gattttcctc cttggtagta gtgtttgttt
87541 attcattcat gagacattta tgtcaggtct tctatctcta taatactgta ttaggaaatg
87601 tggggtattt ttaaatgaat aatataattc tttagctaaa ataactcatg tactagagta
87661 caaacaaaca atatttctag tatagtaagt gtctaaaaca ctatgctgta cttcattcac
87721 tcattcaaca aatagcattt gtgtttctgc tagatactaa taaggtggtt agaccacaga
87781 gatagaaaga tacatagaat tgttactcca gaagaaattc cattcttgaa ccagacatgt
87841 aaacatatga ctgaaatcag tgaaatcagt gtggtgagga agcatcttgg agacatggct
87901 acagtagaga ggggatacat gagtctggag aggtcaacag aatcacatat gcccagccaa
87961 ggatgggctg tttcttaaag tctcttgaga actactaaaa gtatatatgc agagatgtgg
88021 tatctgcagt tttgtattat agaaagaaca gcttggtggc aagggagtgt gtggattaga
88081 ggaagcaaaa cgggaggtgg aaaaattcgg gagtaatcat ttgaaataat ctgagtgaaa
88141 aataataaag atggtggcca cagcagtgga tatggggata gaaagaaaag aggtggaaaa
88201 gacgtttgaa atacaagaaa gcatatgact tgggaaccgc atatggggga tggtaaagtg
88261 ggagaagttg aggatgaaac tgtatggatg acattcaaaa gacaaggagc acctgttcat
88321 agtaggaact tacctgaaaa ggagaactga gtttttaaca catgttagct tgagtgcttg
 
88381 taatatttgt aagacatcag agtggtagag taattggaag agatgacctc aaccaaaaga
88441 aatatagcaa gtaagaagac aagatgagcc acagatgaaa ctgtggggaa atcaatattt
88501 aagaaacagt tatctgtaga gcagacacaa taatctggag aaacagtaat ctgtacggga
88561 gactgagaag aagcaatcag aacaatcgga ataacaacag agagagagac cacaaaagcc
88621 agtggagaca tgagttcaag gaaggaggaa gtggtaagag gtgccaaata ttagaaaggt
88681 tcaagaataa ctgggactta ctctgttagc tgaacagcat gaaatagaga tgtttattta
88741 ctaataactt accatactct gggcacagag agggagaatg tgttttgaga aagggggaga
88801 gaaagcaaga cagtgaaaga aattcacatt tacacatgag aaactgaatc agaaagggta
88861 agtaaatttc cccaatgtca cacaactaga aaagtagcaa agcagagatt caaactaaat
88921 ttgtttggat ataatccatt tcttttgagg tgtggctatg actgcatttt tattgttttt
88981 tagttgatac ataattgtac atatttatgg ggtatatgtg atattttgat acatacatat
89041 aatgcgtaat ggtcagctca ggatattttg gatatccatc acctcaaaca tttatcattt
89101 ctttgtgttg taaacatttt aaatcttcta gctattttga aatatataat aaaatattat
89161 taactattgt taccctactg tgctgtggaa tactagaact tgtttcttct atataactgt
89221 actttggaat gcattaccaa cctctcttca ttccccccac aactgaccca cacttcccag
89281 actctagtaa ccatcattct actctctact tccgtgagat tcactttttg gctctcataa
89341 atgagtgaga acatgagata tgtgtctttc tgtgcccagc ttatttcact taacataata
89401 gcctccagtt ccctccatgt tgatgcaaat gacaggattg cattcttttt atggctaaat
89461 agtactccgt tgtgtatata taacagattt tctttatttt taacttctat aaattaaaaa
89521 atatcaaaca tcaaggacaa aacaaaaaca aaacaaaaaa gttcccttct accccaattt
89581 gaaatgtaac aactattttt ttgtatttac taaagataca acccaaatcc ccagtcatca
89641 ttcttcactc ttcattccca gaagtaacca ccactcaaaa attgtattat tctcactctt
89701 ctgtattttt atggcatctg gatttattaa cataccagaa gatattatac agtgcagctt
89761 tgagtgtttt gtattttaag ttaaaatgtt ggcattactt tttttctctt gacattatgt
89821 tttacagata tagtcatatt gctatctgta cacctggtgt attcatttaa taactatata
89881 gcattccatt aaataaacac aagtttttat gtttttttta ttctgtgacg atgtactata
89941 gggaagtcaa tatacactac agattagcaa gagagtacaa tcaaagagga gagagtgatg
90001 tcaaatagac aaggaccttg aagttaatag tgctaaattt attagttatt tggttagcat
90061 ggacaatgtc cctaaagatg taataaaata acacgttctg tgcacagtta ataaaccagg
90121 ttgatttcag tcttaagctg tgaaagcaat tggatattaa atgaaaacac aatactgtga
90181 ttctaaagct ttgagcagca gcctagggaa tttcacctat tctgtagagg tattagggga
90241 gcagaggttg tttctgggta gaggaatgat tgacaagaca atgttcagca tagccaagaa
90301 atcttgaatt atttttaatg gataataaat atagaagata ggtattattt caatttggaa
90361 gacagtcgcc tcgtggtgaa aaagagaaaa gggatgaaaa aagatgagga aaaaaaatgg
90421 ataaatctag cctgaaggct aatttgcata tttatttatt tgctgtcttt tatttctttt
90481 attaaaatgt tgacattgtt tactcagtaa aacttatttg cttgcaagaa gttacactta
90541 ctagggttta tttaagaaga ttttattctt atgagaacat gtcttatttc caaggaatag
90601 agtaagtaga tttatagcca aggaaacatt tgatttggaa cttgaggtct ccgtgttttt
 
90661 ggtctattaa tatttaacag atcctcagtc caaatgccta gatgcttaaa tatccataag
90721 ttgtactttt agccataatg cttcactctt tcctttttaa tttgaaggtt atacattatt
90781 gctttaccaa aagacaatga agcaaaatag cctaataaca taaatttaat aaactgtatc
90841 agagcagaaa tggagagata gcttagcttc cagttaattg gaccacagtc tcgtactaga
90901 tcatcagaaa ctactgtttt tcagcatttt aaaacatgca tctttagtct cccctgaaat
90961 tatcctcttt gccagttcaa aatctttatg tccatatacg gttatttttg aaatcgtaag
91021 tcatatgtct gttcatcccc tttcctataa agctcattat ctaaacattg tctcacactt
91081 caaaggatca cctgttgatg tgaatggatt taccagtgga actcaggcaa gagattccag
91141 gctgtgagcg ttactgtttt tgtaaaggga aggtaacttt tatatatagt ttgcctgcct
91201 ttggacagtc ttgtttctac cttctaaaat acgataattg aaagcacagc gttcctatgg
91261 cttgctgata gattgtgaga aataacagca aaatatatac agctgaggag attgcatcaa
91321 tacatctctg caccgaaatt atggcagtga tttgcctaga attgggggct attagatcaa
91381 agattgtcct aaagtttaaa gttacattca accaaacaca tggacaattt ttgttaatat
91441 catattagac cagatttacc agttgttttc taaaatttaa tatacatttt ttaaccattt
91501 taggttacag aaaaagcaga aagtatagag agttttcata taacatcctc tttcacgagt
91561 ttcctctagt tttaacatct tgcattagca tggtacattt gtaacagttg aggagccgat
91621 actgacatgt gatcgttaac taaaatccat agcttacatt agggttccct cttttttggg
91681 tgtaagttct gtggattttg acaaaggtat aaatgatgtg tcaatcatta cagtatcatg
91741 cagaatactt ttactgccct aaaagtctcc gtgttccgcc tgttcatttc tcctcccctg
91801 tattcctggc atcaaccagc cactttgatg tcatatcaac tcttataccc tcataacgtg
91861 ttttttattg tcatcattgt tttgtttttg tttttctgtt aatgacgtat ggctgtgaca
91921 aggtaatgag accagcggct gttgttggag gacctttttt cttaattcct ggttacttat
91981 ccttgaatta tgtgacaagc tgataaatcc aataaccatg gattttccat tgtttcaacc
92041 tgccaatact tctctcaagc ttgtatatcc aagaaaatat aaaaaatagt agagcagaaa
92101 attacaagct tccatactta tcctactctc cctgtgtccc catccccact tctggtggct
92161 ttccaactca gttaagtcct gtacttagcc agttggtgac cacacaatgt gacatgtttt
92221 atatccaagt cagtctcaga ctaggtgagt tttttatgtg gcagattact gcttagttta
92281 tactcagaca aaaaggaaaa aattaaacat ataaacaccc tttttttttt ttcgagagtc
92341 tcatgctgtt gcccaggcta gagtgcagtg gcgtgatctc agctcactgc atcctctgcc
92401 tcccgggttc cagcgattct cctgcctcag cctcctgagt aactgggatt acaagcacgt
92461 gccaccacac ctggctaatt tgtatatttt tagtagagat ggggtttcgc catattggcc
92521 aggcttgtct caaactcctg acctcaggtg atctgcccat ctcgacctcc caaaatgctg
92581 ggattacagg cgtaagccac cgtgcccagc ctaaacccat gttttaaatt aatgcaatga
92641 ttgtattagc ctttcaaaca ttaagactgg caaaattgtt acaactatgg agtttcatt
92701 gattcatcct actcaccatc ttttccttta atatctgaac aaaccccaac tctgttcact
92761 gttctccctg tgagtagact aatggaacaa gagcaaacag taacacaaac agacgctaac
92821 acaagttcat gcagaatcaa taaaaatcag tacaacagaa gataagtcta tgtgtttttg
92881 atacatacga ttaagcatgt gcctttttaa caatttatat aaaacctaaa atatatgtat
 
92941 ttctgatttt tacctgtagt actgaagaag ttacttaata acgttgaata taaaggccac
93001 ttttacttaa ccaccttcca ttcactatta actgctgttt ccaaagtgta agcaaatcag
93061 tctctgtgta catagtcaag tgtatacaag cgtcaggcgt aacaaactca agatgaacat
93121 gacagttcaa agatatttgg gacaaaattg ttgaagcatt tttacccagg gctctgtagc
93181 tctgacaatg aaagaatata gttgctcttc cagctgctat tcagacagaa agcttgggca
93241 agaagggtct gtatctatgt tcttcataat acaattacaa gtttgaactt cagataacat
93301 cagcagttgg catgtggaaa accaaaaccc tattttggta tttatcaaga ttgttaatgg
93361 agtcaggttt cccttatttg tttctttaat ggggtacaga acatcctgtt ggataacccg
93421 ctgagtgaca tgacgatgct ctgaaggaat gcatgagagc ttgtggtacc tgccttgaaa
93481 catgggtttc actaatgctg gtggctcaca cttcccattg aacaagacta gagataggaa
93541 ggctatttga gggacacagc tatggaacca taggtgccac atggtaagcc aaattttat
93601 ttgttgtgtt gttgtgaaaa accttattaa aagagcttcc aatgagagta cttgattaat
93661 aacacagttc gtatctatag aaataatttg cttttcaaga aaatcatcat gtgctacagt
93721 tgaattgaca ttaatgttat cattcatttt gaatgatctg tgaagtattt taagagagat
93781 ctggggaagt aaatcaataa gtagtttaac ataaatcaag gtgcagttaa tttttttcaa
93841 ttagaaatat attacaaaga ttctgtcatt tccaacaacg tgaaataacc tggaggaaat
93901 tgtgctgagt gaaataagcc agacacagaa agatgcatat tactgatctc ccttatatat
93961 ggaatctaga aaagtagaat tcatagatac agaatagaag agtggtcacc acggtcttgg
94021 gggtggggga catggggaaa tgttagagtg tgacaacttg cagttacaag gtgaatatgc
94081 tctggagacc taacgtatag tacagcatag ttactctact caatagtatt ctcactgcac
94141 cccccaactc ccccacacac acagtaactc tttgaggtgt ggatatgtta attagcttga
94201 ttttggtaat catttcacag tgtatacata taccaaaaca tcactttgta taccttatat
94261 atgtacaata tttatcaatc atacttcaat aaagctggaa aaatgcatga atattatata
94321 tatgtatatg tatatacaaa tgtataagag attatagcag attatagctc tttgaaaaag
94381 aataacattt cagcccagtt ctgactaaga ccaaacaaaa ggtgatagca tgttttagtt
94441 ccttaaatgt ggatttgagg agtcaagaaa tctccaagtg taggaaaacc tccgtggcaa
94501 agagtgtaga atatgagaat cacaaaaata gcatgcacaa aatagcctgc tgtgatgttg
94561 aaagtataag agctagtaat tattattatt attattattt ttatttttat tattatactt
94621 taagttttag ggtacatgtg cacattgtgc aggttagtta catatgtata catgtgccat
94681 gctggtgtgc tgcacccact aacttgtcat ctaccattag gtatatctcc caatgctatc
94741 cctccccctc cctccacccc acaacagtcc ccagagtgtg atgttcccct tcctgtgtcc
94801 atgtgatctc attgttcaat tcccacctat gagtgagaat atgcggtgtt tggtttttg
94861 ttcttgtgat agtttactga gaatgatgac ttccaatttc atccatgtcc ctacaaagga
94921 catgaactca tcatttttta tggctgcata gtattccatg gtgtatatgt gccacatttt
94981 cttaatccag tctatcattg ttggacattt gggttggttc caagtctttg ctatcgtgaa
95041 taatgccgca ataaacatac gtgtgcatgt gtctttatag cagcatgatt tatagttctt
95101 tgggtatata cccagtaatg ggatggctgg gtcaaatggt atttccagtt ctagatccct
95161 gaggaatcgc cacactgact tccacaatgg ttgaactagt ttacagtccc accaacagtg
 
95221 taaaagtgtt cctatttctc cacatcctct ccagcacctg ttgtttcctg acttttgaat
95281 gattgccatt ctacgtggtg tgagatggta tctcattgta gttttgattt gcatttctct
95341 gatgaccagt gatggtgagc attttttcat gtgttttttg gctgcataaa tgtcttcttt
95401 tgagaagtgt ctgttcatgt ccttcgccca ctttttgatg gggttgtttg tttttttctt
95461 gtaaatttgt ttgagttcat tgtagattct ggatattagc cctttgtcag atgagtaggt
95521 tgtgaaaatt ttctcccatt ttgtaggttg cctgttcact ctgatggtag tttcttttgc
95581 tgtgcagaag ctctttagtt taattagatc ccatttatca atttgtcttt ttgttgccat
95641 tgcttttggt gttttagacg tgaagtcctt gcccatgcct atgtcctgaa tggtaatgcc
95701 taggttttct tctagggttt ttatggtttt aggtctaacg tttaagtctt taatccatct
95761 tgaactgatt tttgtataag gtgtaaggaa gggatccagt ttcagctttc tacatatggc
95821 tagccagttt tcccagcacc atttattaaa tagggcatcc tttccccatt gcttgttttt
95881 ctcaggtttg tcaaagatca gattgttgta gatatgcggc gttatttctg agggctctgt
95941 tctgttccat tgatctatat ctctgttttg aaccagtacc atgctgtttt ggttactgta
96001 gccttgtggt atagtttgaa gtcaggtagg gtgatgcctc cagctttgtt cttttggctt
96061 agggttgact tggtgatgca ggctcttttt tggttccata tgaactttaa agtagttttt
96121 tccagttctg tgaagaaagt cattggtagc ttgatgggga tggcattgaa tctgtaaatt
96181 accttgggca gtatggccat tttcacgata ttgattcttc ctacccatga gcatggaatg
96241 ttcttccatt tgtttgtatc ctcttttatt tccttgagca gtggtttgta gttctccttg
96301 aagaggtcct tcacatccct tgtaagttgg attcctaggt attttattct ctttgaagca
96361 attgtgaatg ggagttcact catgatttgg ctctctgttt gtctgttgtt ggtgtataag
96421 agtgcttgtg atttttgtac attgattttg tatctggaga ctttgctgaa gttgcttatc
96481 agcttaagga gatttttggc tgagacaatg gggttttcta gatatacaat catgttgtct
96541 gcaaacaggg acaatttgac ttcctctttt cctaattgaa taccctttat ttccttctcc
96601 tgcctaattg ccctggccag aacttccaac actatgttga atagtagtgg tgagagaggg
96661 catccctgtc ttgtgccagt tttcaaaggg aatgcttcca gtttttgccc attcagtatg
96721 atattggctg tgggtttgtc atagatagct cttattattt tgaaatatgt cccatcaata
96781 cctaatttat tgagagtttt tagcatgaag ggttgttgaa ttttgtcaaa ggctttttct
96841 gcatctattg agataatcat gtggtttttg tctttggctc tgtttatatg ctggattaca
96901 tttatttatt gatttgcata tattgaacca gtcttgcatc ccagggatga agcccgcttg
96961 atcatggtgg ataagctttt tgatgtgctg ctggattcgt tttgccagta ttttattgag
97021 gatttttgca tcaatgttca tcaaggatat tggtctaaaa ttctcttttt ttgttgtgtc
97081 tctgcctggc tttcatatca gaatgatgct ggcctcataa aatgagttag ggaggattcc
97141 ctctttttct attgattgga atagtttcag aaggaatggt accagttcct ccttgtacct
97201 ctggtagaat tgggctgtga atccgtctgg tcctggactc tttttggttg gtaagctatt
97261 gattattgcc acaatttcag atcctgttat tggtctattc agagattcaa cttcttcctg
97321 gtttagtctt gggagagtgt atgtgtcgag gaatttattc atttcttcta gattttctag
97381 tttatttgca tagaggtgtt tgtagtattc tctgatggta gtttgtattt ctgtgggatc
97441 ggtggtgata tcccctttat cattttttat tgcgtctatt tgattcttct ctcttttttt
 
97501 ctttattagt cttgctagca gtctatcaat tttgttgatc ccttcaaaaa accagctcct
97561 ggattcatta atttttgaag ggttttttgt gtctctattt ccttcagttc tgctctgatt
97621 ttagttattt cttgccttct gctagctttt gaatgtgttt gctcttgctt ttctagttct
97681 tttaattgtg atgttagggt gtcaattttg gatctttcct gctttctctt gtgggcattt
97741 agtgctataa atttccctct acacactgct ttgaatgcgt cccagagatt ctggtatgtt
97801 gtgtctttgt tctcgttggt ttcaaagaac atctttattt ctgccttcat ttcgttacgt
97861 acccagtagt cattcaggag caggttgttc agtttccatg tagttgagca gttttgagtg
97921 agattcttaa tcctgagttc tagtttgatt gcactgtggt ctgagagata gtttgttata
97981 atttctgttc ttttacattt gctgaggaga gctttacttc caagtatgtg gtcaattttg
98041 gaataggtgt ggtgtggtgc tgaaaaaaat atatattcca ttgatttggg gtggagagtt
98101 ctgtagatgt ctattaggtc cgcttggtgc agaactgagt tcaattcctg cgtatccttg
98161 ttgactttct gtctcgttga tctgtctaat gttgacagtg ggtgttaaag tctcccatta
98221 ttaatgtgtg ggagtctaag tctctttgta ggtcactcag gacttgcttt atgaatcttg
98281 gtgctcctgt attgggtgca tatatattta ggatagttag ctcttcttgt tgaattgatc
98341 cctttacgat tatgtaatgg ccttttttgt ctcttttgat ctttgttggt ttaaagtctg
98401 ttttatcaga gactaggatt acaacccctg cctttttttg ttttccattt gcttggtaga
98461 tcttcctcca tccttttatt ttgagcctat gtgtgtctct gcacgtgaga tgggtttcct
98521 gaatacagca cactgatggg tcttgactct ttatccaatt tgccagtctg tgtcttttaa
98581 ttggagcatt tagtccattg acatttaaag ttaatattgt tatgtgtgaa tttgatcctg
98641 tcattatgat gttagctggt tattttgctc attagttgat gcagtttctt cctagtctcg
98701 atggtcttta cattttggca tgattttgca gcggctggta ccagttgttc ctttccatat
98761 ttagcgcttc cttcaggagc tcttttaggg caggcctggt ggtgacaaaa tctctcagca
98821 tttgcttgtc tgtaaagtat tttatttctc cttcacttat atgaagctta ttttggctgg
98881 atatgaaatt ctgggttgaa aattcttttc tttaagaatg ttgaatattg gcccccactc
98941 tcttctggct tgtagggttt ctgccgagag atcctctgtt agtctgatgg gcttcccttt
99001 gagggtaacc agacctttct ctctggctgc ccttaacatt ttttccttca tttcaacttt
99061 ggtgaatctg acagttatgt gtcttggagt tgctcttctc gaggagtatc tttgtggcat
99121 tctctgtatt tcctgaatct gaacgttggc ctgccttgct agattgggga agttctcctg
99181 gataatatcc tgcagagtgt tttccaactt ggttccattc tccccatcac tttcaggtac
99241 accaatcaga catagatttg gtcttttcac atagtcccat atttcttgga ggctttgctc
99301 atttcttttt attctttttt ctctaaactt cccttctcgc ttcatttcat tcatttcatc
99361 ttccattgct gatactcttt cttccagttg attgcatcgg ctcctgaggc ttctgcattc
99421 ttcacgtagt tctcgagcct tggttttcag ctccatcagc tcctttaagc acttctctgt
99481 attggttatt ctagttatac attcttctaa atttttttca aagttttcaa cttctttgcc
99541 tttggtttga atgtcctcct gtagctcaga gtaatttgat cgtctgaagc cttcttctcg
99601 cagctcgtca aagttattct ccatccagct ttgttccatt gctggtaagg aactgtgttt
99661 ctttggagga ggagaggcac tctgcttttt agactttcca gtttttctgt tctgtttttt
99721 ccccatcttt gtggttttat cgacttttgg tctttgatga tggtgatgta cagatgggtt
 
99781 ttcggtgtgg atgtcctttc tgtttgttag ttttccttct aacagacagg accctcagct
99841 gcaggtctgt tggaataccc tgctgtgtga ggtgtcagtg tgcccctgct gggggtgcc
99901 tcccagttag gctgctcggg ggtcaggggt cagggaccca cttgaggaag cagtctgccc
99961 gttctcagat ctccagctgc gtgctgggag aaccactgct ctcttcaaag ctgtcagaca
100021 gggacattta agtctgcaga agttacttct gtctttttgt ttgtctgtgc cctgccccca
100081 gaggtggagc ctacagaggc aggcaggcct ccttgagctg tggtgggctc cacccagttc
100141 gagcttcccg gctgctttgt ttacctaatc aagcctgggc aatggcgggc gtccctcccc
100201 cagcctcatt gccgccttgc agtttgatct cagactgctg tgctagcaat cagcgagact
100261 ccgtgggcgt aggaccctcg agccaggtgc gggtataatc tcgtggtgtg ccgttttttg
100321 tttttttttt gtttgtttgt ttgtttgttt ttgagacgga gtctcgctct gtcgcccagg
100381 ctggagtgca gtggcgggat ctcggctcac tgcaagctcc gcctgccggg ttcacgccat
100441 tctcctgcct cagcctccca agtagctggg actacaggcg cccgccacta cgcccggcta
100501 attttttgta tttttagtag agacggggtt tcaccgtttt agccgggatg gtctcgatct
100561 cctgacctcg tgatccgccc gcctcggcct cccaaagtgc tgggattaca ggcgtgagcc
100621 accgcgccgg gccggtgcgc cgttttttaa gcccgtcgga aaagcgcagt attcgggtgg
100681 gagtgacccg attttccagg tgctgcccct cacccctttc tttgactagg aaagggaact
100741 ccctgaccac ttgtgcttcc caagtgaggc aatgcctcgc cctgcttcgg ctcgcgcacg
100801 gtgcgtgcac ccactgacct gcgcccactg tctggcactc cctagtgaga tgaacccggt
100861 acctcagatg gaaatgcaga aatcacccgt cttctgcgtt gctcaggctg ggagctgtag
100921 accggagctg ttcctatttg gccatcttgg ctcctcccca agagctggta attattaccc
100981 aacgtttaga atataatgta acagaacgta tttggaatac atttatcttt taatatttca
101041 aaggagagat aaatgcaaac taagatttta tttatcatta aattcattgt tctttagttt
101101 gaaaaacaca tatactcata tttttgtcac gtgattttaa ccagcataaa gcaatagctg
101161 ccacaccaaa gtcaagtgaa gctgtgaaaa taattaatga aacaattctg ataaaagcat
101221 ctttatcctt catacaggtt ataggcagga ttgtggttgt gcctgtgtgt gtgtgtggtg
101281 gggtcggtgg ggggatagga aagttagttt attcacatga ttctcaacat agacatacct
101341 tgcaaataat tgtgcaattc cctgctttgc tttgcagaca gaatgtcttt gaatcaatgt
101401 cttttataaa tacactatac aaaaaacata attataaacc tttaaagtat ttatgcatac
101461 tttaatattc agaactagaa gagaaatttc tgtaaggaga aatgctttgg agattaaaac
101521 gtaaaggaga taaccaaatt ttactaaacc tctatccatc atgaccacat ttcctttcat
101581 tctaaaataa aaatattttg gaaatgttga ccccctgccc accatttttg cctgagaagt
101641 tttaaatgcg aagaacttgc tcctcacttt tgaagcacgg tgctttcttt aggaaccaat
101701 ctgtatcaca tgcattctat tggtttgtat tttattacat atcattctat tacattttaa
101761 aatgcatttc tgagcccatg tcattgattt catgattcat taatgggttg caagcagcag
101821 ctggaataac actggcttag gtaatggcag tataaatcac atgttggtaa aatgaaagat
101881 gcctttctac caacacgtta taatggccac tcgatgccaa catggcagtg tgttacttaa
101941 accatatatc aatcaacaca gaagcgacat aaccaaaatg tttttaccat catttataga
102001 agattatgag tttcatgcag ccaggattaa ttgcattgct attatagaac ttaatataag
 
102061 ttgtatcata tttgatggaa ggatgctaac tatcaagagg atataaattt taaaaacaat
102121 gatactgttt tggataagtc ctgactgtct gcattttatc tagaacaata tatgttcatg
102181 attaacctag aaaataatga caaaaatgcg caatgagtag agacttgccc ttggaattcc
102241 taaaattatt aggaaggtac aatgttctcc aagaaatatt ggtaagtgaa aaaaacaagg
102301 tttacaataa tgtgtaacgg atgctatctg tgtaacaaaa tgtgaaaaaa catatatgca
102361 attttttggc aagaagatac cctgaaaaca taaaccaaaa agccaaaaaa aaaaaaagaa
102421 acttgctgat gatacagaac ttctctgact gtacattttt ctcatagtct taactctgat
102481 aattacatac atgtttgaca ttctcaaaag taagaacata gaagtacata atatatgcag
102541 gataaagcaa ataacagttt atgtgaagat tgttagaagc caaggttttc atggaagtta
102601 ttagtatgaa gtcaaagtag tacattaaaa tccagtaatg ttaattggat ttggaagtaa
102661 cagtatgaaa ttttttctct aaaaatgtgt gcaggaatct cttgcttaag atcagtaatc
102721 tattcccaaa aatagatttt gagagcacat ttctgttcta aatgggaaca attatagtga
102781 atggaaggta aaccaaaaca tagctatttc ctacaaaata attgatacct aataatgcct
102841 gtagaaaagc atagaaaagt gacaaactct cttgataaaa tctaaaatgt atattgtttc
102901 ctaatcagca attattgatg ttgctaacat acagttgctt tgtatctagt tacatggacc
102961 gtgtatatgc tgtatgagaa attgtcactt tgtggtatag catagcaaat aacacagcaa
103021 tcaaaaagtt aaaagtcaaa attaaaataa ataaaagacc cataatagac tgtttttcac
103081 cttgctaaag gagaaatgac tcttgaagaa actcaagaag ggacaaagaa aggtcaaatc
103141 agtcattgct atttgcagaa ggtgatgggt ggatggagga caagggggca atcttcgaca
103201 aactgtcata tgaagaggaa tgaagagacc ttccccatgt ctgtgtctag tagatgctgt
103261 agagacttgc tggacaggaa agctttggta aatcttcaga aatgatgttc aagctgaagg
103321 aagatgttca agctgcattt tggttgcaaa atgcagattt gcttgttttt ctgttgaaaa
103381 attcctctat gtgatgctgt aagagttacc aggctattat taaaagaaag atgtattgtc
103441 acactgagta tgtctgcaaa actttcttag taactggcct gtttgtttat actcaatgca
103501 catgttaaga tggctacata ggattcttac tatgttctat caatttggaa tgtaactcaa
103561 cattcttcca gcacataaag ttgtttacaa tatataatga gtgtgaaaga tataaataaa
103621 tacttctatt tgatgtctac accaacatta gatgaaaaaa tgaggtagtg agaagttgat
103681 atatatcctg taaattagca tatttaaaac caaaaatcct tctactgggt aaatattaac
103741 taggaaaaca ttctacagga tataatgcat ttttctagct ctgccccctg aaaaggtcta
103801 gtagtaatgt gatctgatgg cactaaacac ttctgatctc aaatgatagc ctctaaaatg
103861 ctgttttcca acaaagagaa ttagggctcc tgaagaaatg gctgatgcca ggtgtggagg
103921 agaaaatgag caagctgagc ttgtgatata tgttcacccc agagagggct catgtcacgt
103981 gggcaccaac agagaggggt ctcaggtagc aacagtgggg ccattggacc atcaaaggag
104041 caattagggt acttgactga agcctgtagg ttcataattt ttactctaaa acaaggaagg
104101 aaggtgggaa gaaaggggaa aggaggggaa gggaaggaag aaaggaggga gggagggaag
104161 gaaggaagta aagagctaga acaacaatga aacaaaatat ctttgataac tttttggggg
104221 agtacagagt ggaaattttg catttctgga ggctttacaa gatggggatc tgtgcttcag
104281 aaataagtca gtaaagatac atttacatag atgtttcctc cagtattgct tggaaaagcc
 
104341 aaaatcaaag tgatgtagat agttacctaa gagatgaata aaataaataa tggtacatct
104401 tttgtatgga atattacata gccactaaaa ataatggagc agaactctac tcaatgacat
104461 ataaaaatgt ccatgatata ttgtataatg gaaaaaaatt ttaaagcaaa aggttttacc
104521 ttatttattt cacaagctaa aaccctatat gattatacaa accaatgtgt ttgccttaaa
104581 aagataaaag tctggtgtcc tctaagacca taccacaaac agtgatcacc tctgggattg
104641 gaattttata gcagttgtgg gtagagagag tttgggagaa aacattcaca ttttatctca
104701 taattcttat atttttaaag cttttctatg agcattattt tgatacatat gtgagcagag
104761 atggtccctt ctaggtacca gttacaccat tgctaaacct cgtctgtttc ttccaaaggt
104821 gtcaggcagc gaacaagacc tgccccacca attacatgtg gaataatcac atctgcagat
104881 gcctggctca ggaagatttt atgttttcct cggatgctgg agatggtaag cagaatgatg
104941 ctttaaagac taaacgccag gggtctatat tgtgattaac atactctaat attaaaacca
105001 cacatataat attcatcatt cttctaaata agtgactaaa aaatagtaca ttttctactt
105061 cacgttggta ttattttgaa ctcctttata aaggaacata ctcatgtttg tacccaatcc
105121 tttgatggaa aacatacaaa aggaagatgc ttttaaaagc ttttgtttca tgccacaccc
105181 agaaccaagt aaggtatagt aaagattctc tgactatttg tctagactca acagatggat
105241 tccatgacat ctgtggacca aacaaggagc tggatgaaga gacctgtcag tgtgtctgca
105301 gagcggggct tcggcctgcc agctgtggac cccacaaaga actagacaga aactcatgcc
105361 agtgtgtctg taaaaacaaa ctcttcccca gccaatgtgg ggccaaccga gaatttgatg
105421 aaaacacatg ccagtgtgta tgtaaaagaa cctgccccag aaatcaaccc ctaaatcctg
105481 gaaaatgtgc ctgtgaatgt acagaaagtc cacagaaatg cttgttaaaa ggaaagaagt
105541 tccaccacca aacatgcagg taagagatcc tcatgaagaa tattaattta tcttactaga
105601 aatatgctct aaaccaaaag cagttttctt aaccaaagta acaattgctt gttagaatga
105661 ggccaataca ttctgaaaga caaaaatctt atattttttt aaccaatgct aaacttgcat
105721 gagactggat ttgcttccaa aaaagcaaga caggagcctt gaggatcaaa attagatcca
105781 agctcagaat atactctaaa ttagtggttc ctgtgggatg ttttcaaaat acctaaaggc
105841 aatccacttc acccccacca aaaagtagga tgccttcaga cataatttag gacaactgtt
105901 ctaaatgcag acaattttta tgcaaaactt acaaacaaag gacaggagag taccactggt
105961 atagaattat atctggaaat gaaaatacat agctaagtat ttgatagaat tagagaaata
106021 aatgaatatg ttgttaaagg tacccaagac tcatgtctta aacttggttt tgactcttct
106081 gcatcaagaa aaaaaaaatg aagttattcg actcaagcac attcagtgtt aatcccgaaa
106141 cctcttcctc tttaatctca gagaataaat tgatcctact atttaagcca gacctgtccg
106201 tttgacattt tctccacatt ccttcaacat tgcttcatgc cattccttct ttcttctgta
106261 acttgaacat cttcaactct actttttttc cccttagttt ataaaagttc ccaaatttct
106321 tctattttat tagcctctta ctgtgaagtt gagcaaactt tagcaaccca ggtctgttct
106381 cttcaaagct gctcaaaaac gaggcatttg ttttcacctc tttcacctac catgtattca
106441 accaactaaa acctggttta tggcctacag tgctatgtta aacctctgtt taacgtttca
106501 ttggcctcat agttgccaaa tccaacagag aattttgtcc ttaggtaacc tgtcctttcc
106561 atggcctttt tctttaagct cacctcttct atgacatgac attgtacttt cctagttgtc
 
106621 tacttaattt tcagaccatt tgtttttaat cactttcact ggattctctt tttacatcca
106681 ttctttacat atgggtagat aatgttcttt ttaacagaaa aaagcttgta tttcatttat
106741 ttactatctt cactaatgtt cattattatt tttccacagt atataacgag ttcatctttt
106801 tattctcaag agttttgtgt ccttatttta atgtatattt ttggtggttt tcctaaacta
106861 atattttagc tactggataa aaaggcttta gtggatctgg ctatctcatt gattatattt
106921 ttcttccctt atactgcaaa gtacatcata ggtataattt tattttgaga aagaactata
106981 ttccaatttt acctatatat tcctactgta ataaccaaga ggacatttca aaaatgtata
107041 tgctactatt ttcagaagga tatagatact aaatcttttt aagccttatt aaactatgtt
107101 ttatattgaa ttcttattga gtgaatttag tatacattga aaaatctgaa attcttatgt
107161 ctgcatacct acttttctac agaattatat gaacacagac aatatttcta tcattgcatt
107221 tctcctaata ctatacattc tttcaaaaag actctgttta ctgcaactca tcctagattc
107281 atttttctaa gtgtataatt ccaagctttg tgatataaat tggagcaaaa gagaaaatat
107341 catattttga aaatatatat tctcctcatt ctctcttaat atctattaca aagtgcagta
107401 ggcagagtat atattctttg gttcattgcc ttaattaaca gcttttatgg atcattaagt
107461 atcatatccc tttttagtag aggaatacca gtagtcataa ctatgaattt gcatttgcaa
107521 agtttaagaa aaattaacta gacatgcttt cttttctaga gccaaataaa tgaaatgtca
107581 aaaccaaatt gtgtagggtt ttttatagca cacttttgat ttccacctca ggtcatcttg
107641 cacttttccc tttacaccta ttcccacaca ttaggcatgc tcatactcca aaatgttttt
107701 aaaagatagc tccaattctc accaggcgtg gtggctcaca cctgtaatcc tagcactttg
107761 gaaggccaag gcgggcagat cacttgaggc caggagttca agaccagccc agccaacatg
107821 gcaaaaccct ggctctatga aaaatacaga aacttagcca ggcgtggcca cacatacctg
107881 taatcccagc tactcgggag gctgagacac aagaatggct tgagcccagg aggcggaggt
107941 tgcagtaagc tgagattgcc ccactgcgct ccagcctggg cgacagaaca agactctgtc
108001 tcaaaaaatg aagcctcaat tcactggagc aacagggcag gagacacact gaccaattga
108061 taatttgtcc tctcaccgct actggaaact ggaagtctag gatgggagga aggggaccct
108121 cgctgctgag tattgtcctt ctcacctccc tgccccagaa tgcctctgtt tcacctggag
108181 ccaaggtgga aaggaagagt tagtcaccgt catgcagcca gctgccaagt agagacacaa
108241 gagtgagggt ggggctgctc actgaggacc acagttctgc caacatggct gggtttaatt
108301 gcctggaagt ctgggtccca tacaagtccc agactcatgt gaattctgcc atgcttatgt
108361 gatcacagta agtcagcaga ccttcagtgt tcactttcag aatttctgtt ttatgtttct
108421 ttcacccatg aagcaaagag cacttcaaag gaactaatgc ttacaatacc tcatccttcc
108481 atttgttata tcagcaagca agacttttat tttaaggctt ttccattcct cttaaatgtt
108541 gtaattctaa gcagaaacaa acttttttaa acctatgaac aatttcgtca taaaattagt
108601 aattttattc cagtctgaaa tttaaaaaca cagaaatacc ttggtagcat gatgaatcca
108661 ttgccttgat ctttaacgta agtgtgttct tgtttgttct cctagctgtt acagacggcc
108721 atgtacgaac cgccagaagg cttgtgagcc aggattttca tatagtgaag aagtgtgtcg
108781 ttgtgtccct tcatattgga aaagaccaca aatgagctaa gattgtactg ttttccagtt
108841 catcgatttt ctattatgga aaactgtgtt gccacagtag aactgtctgt gaacagagag
 
108901 acccttgtgg gtccatgcta acaaagacaa aagtctgtct ttcctgaacc atgtggataa
108961 ctttacagaa atggactgga gctcatctgc aaaaggcctc ttgtaaagac tggttttctg
109021 ccaatgacca aacagccaag attttcctct tgtgatttct ttaaaagaat gactatataa
109081 tttatttcca ctaaaaatat tgtttctgca ttcattttta tagcaacaac aattggtaaa
109141 actcactgtg atcaatattt ttatatcatg caaaatatgt ttaaaataaa atgaaaattg
109201 tattataagc tg
**Human VEGF-D Gene (SEQ ID NO: 83)** (NCBI Accession No. NG_012509.1)

1 aagacacatg cttctgcaag cttccatgaa ggttgtgcaa aaaagtttca atccagagtt
61 gggttccagc tttctgtagc tgtaagcatt ggtggccaca ccacctcctt acaaagcaac
121 tagaacctgc ggcatacatt ggagagattt ttttaatttt ctggacatga agtaaattta
181 gagtgctttc taatttcagg tagaagacat gtccaccttc tgattatttt tggagaacat
241 tttgattttt ttcatctctc tctccccacc cctaagattg tgcaaaaaaa gcgtaccttg
301 cctaattgaa ataatttcat tggattttga tcagaactga ttatttggtt ttctgtgtga
361 agttttgagg tttcaaactt tccttctgga gaatgccttt tgaaacaatt ttctctagct
421 gcctgatgtc aactgcttag taatcagtgg atattgaaat attcaaaatg tacagagagt
481 gggtagtggt gaatgttttc atgatgttgt acgtccagct ggtgcagggc tccagtaatg
541 aacatggacc agtgaaggta ggtgagctca atttttaatg tgatgaagtt ctttttattg
601 gctcatacag aggactttct ttgccctcac tatttttgaa acaatataaa aatgcagaac
661 catcctagta gtggactgga caggatatat cctgcatttc ttggtgactg ttctgtttct
721 tagtcttatt aaacttctgg ccatgagtat ttgaaaatgc tggggtgggg gttggttgtt
781 ttttgaagtt atggtagaac atgagcagtc tggaatcttt ttggatggag aagtttctcc
841 tcggttgcat atgcagatgg cacagtggtt ttttaatgaa acattgattt gtttttatga
901 ctgataactt tgtgggccat ttattttcag gtctttgtga gcagcagagg ttagtttatt
961 cgtttggtaa attgaaaatt gcagtgcttt ctatgaacat tttctatctg cttttgcaaa
1021 ttaccaattg ctgccatcca gaaaaatgtt ttctcagaga gtgtttggtt ttttcgcttc
1081 ttgaaaggtg taaaattcaa cactaatcaa atctttggtt ctgtgagcaa gcatcaaata
1141 gagcttaacg aacagagagt aatgcaaaaa attaaccaga atgttgctga ctgctggaac
1201 aatacttagc ccaattttat gcatttcatg gattatatat attttaagct tctattttga
1261 aaatgattcg gatgtaattg ttttgactga agtggtgcta aatcattatt gatttaggaa
1321 cttctggcct tgaaaagaca attaaatgtg agtttgagtt tttgtagctg ttgtttagga
1381 cgtgcatttt caacaggggt gatatcgccc ccagcagggg aaaattggtt cgtggaggga
1441 tgaaaaagtt ttaccctttt gatacatcac gcacagacgt acatacagta catatggtat
1501 gtagtttaaa atacgttata cattcaaatt gcataggggg aaagtgtctt caaaggctcc
1561 tggctggtgt tggtggcctg gcgagggtgg gggctgatat ttaaaaatgt tgaacactga
1621 gctggaggtg agtgggaagt caccttgtct ggcagccagc acttttctaa cttctcaaat
1681 tccaaattta catgggagaa atcacagtgt ttggctccat tttactgcca ccagccttga
1741 tattggatct caaggccaga ttgtaggtaa atgggaatct gactgtaagc ataggtaaca

1801 ggagaaaacg gtctgacttg tttgaagcaa gtaagacttt tccgactgtc cagcttttc
1861 gtaagttcat gaaacagaaa tcctttagat gtgctgggct cctcgagttt taaaatattt
1921 ccattttcct tgtaaaacat cactttatct gatggtataa taaaggagac tgaattgttg
1981 gacataagtc atattcttta ggttgctcag ctctttcaaa gagcacaagc accaagaatg
2041 tctcaggtgt cctgggcact gtgaagtttt atttatttat ttatttattt atttgagacg
2101 gagtctcact ctgtcaccca ggctggagtg tagtgatgca atctcagctc gctgcaacct
2161 ctgcctcccg ggttcaagtg attcccctgc ctcagcctct cgagtagctg gaattatagg
2221 cacgtgccac catgccgggc taattttttg tatttttagt agagacaggg tttcactgtg
2281 ttagccagga tggtctcgat ctcctgacct gatgatctgc ccgccttggc ctcccatagt
2341 gctgggatta caggcgtgag ccaccgcgcc cggctggcac tgtgaagttt taaatgcttt
2401 gaatgtactg ttaatatata aatttgttga atggagtaac gaaggaaagc agctgaattt
2461 gcatgtgaca gaaaacaagt tgttacacgt ggaggatgca gcattatata cttactaata
2521 cgattagcaa ttttttttca gaatatatca gaatttcctt taaaaagtga agctacatat
2581 actgaactta tatccaccct acccctcgct caattaaatt atttctgata tgaaggaaag
2641 gattgcctat agctaaacag aagttttggc atagatatga ctgagtggta agggataatg
2701 agatatataa tattggttat agttttcttt tttccccaac ttttaagaaa agaaaatcta
2761 agaggaaata atgtttctaa atgttaaagt tcttcttgtt aaaattaaaa tgtagtgtga
2821 aaacatattc atggtggtaa tggtttaggg tggtggtatt tgaatttttt gttttttttt
2881 ttttactttt tgaaataaaa tgttttattt attataagaa attcatggtc ttcctcggta
2941 cttggtccat atgtatttct ttctcctcac cctcccctct tctaaccacc tcttttccag
3001 aatggatggt gatgccaatt ttgctcctag gttgtcctcc agccaccccc agcccagttt
3061 tcactttcta gctttgaaga ttgtccagtt ttccagctta gactagcatt gcctctacga
3121 tagttctagg tatctgtgaa gatttttttt tttcctgtag caatcatgaa atagctacta
3181 gtgctctgga cgattgtgct taatgccatt tgggtgtaag gcctcctaat ttcattagtc
3241 ttaccacttg tgtttgcaag gatagctgtt tggagtcagc agtgcatgga acatgtgaac
3301 tgagacaaat ctgtttgaaa atctcaactt aagatttcaa atccactgtg tatactattt
3361 agctgttaag tttctcagtc taagaagaac acgttttcct ctagaaataa gcttaaccca
3421 gtacctatgc atcgttcatt aatataatca gattacggcc tagatgtgga atttatttct
3481 gtttctattt tctccagctt tttaatacag ctcagacagt catatacagt tgtacacgat
3541 gtgcactgca caactctagg gtgctccatg catatagaca tatctgtatg tgacagccct
3601 aagtacaatg tggtagacac tctggacatt tggtaaataa gtatctacat cctcactctt
3661 ctctcattca cattggcctt ctggccagca ctatttctgg tttatttaat aaacaattct
3721 tgtcccccag aaattctgtg agtcttctga agatgctggg gaaagccagc cttcataaat
3781 cttttcaatg tcactaatga agacatgtca ctattgcaac aaaggaatac caggccatag
3841 taggctttca caactttttt ctgacagaat ttgtttcttt ctgctttatc ttccctgtgt
3901 actgtctttt tttatccacc ctctttctta attacactgg tagaagaata ttggtgggtg
3961 atggagtcat tcacattctg tgtaagacta gaatcagtcc aagatccaca ggaatgtggg
4021 atacttcttt catgagaagc cacatggctc acaagacttc tttacatttg tgttacttgc

4081 cttctcctga aattgtctct acagtgaatc aaataacaag gactgaggtg tattttccat
4141 atttaattcc aggaagcagt cccactcctt ctccctgccc gatttccagt tccaaagtca
4201 tgtacaggga agtccattga ctttgatgtt ggtcaaatgg cccagcaagc ttttgtccac
4261 ttttctgtca gacacagtcc ttggcacaaa agacatctga aatacttccc attaatttct
4321 tctttattta tgaccttgta gttaagtgtg ttgaattata tgcttctata cacatctgtt
4381 ttaatccagt tactcactta taactggtga ctgaaacttt tatatcacta tagcgtatta
4441 ttggtaccca ggatacaata gaatcctcaa tatcttacat cttcaatata gtacttcctt
4501 gtatgactta tttcttaaca taaaaatcct gttttagacc tgtggataaa cctttactat
4561 aaatcccaga gacaatttct ggaatgagaa tatagtattt gtccacacac atgattccat
4621 gtagaacttg aggtaataag atctgggact cccagataca tttattatta ccacaattgg
4681 atagaggtgc tacagaaata ggcacacaaa tgacagtaaa attgtttctg agaacatttc
4741 taggctcaac ttttttctac aacttaggat gaacagagag agtggaaact gtatataatt
4801 gggaatgtga ctgagatgag acaaagatga catggaagca tttcttcttc ccttgctttt
4861 ctggtggtaa gttcactctt ctgtcttcct tgccactagt tcttgcagtc cctaccaaga
4921 cactagggca ttatacagca aacacaccct gaggccccct gctactgcat catgaaagcc
4981 ttagctggtt gtgatgtcct agttcataaa gaatctgcct agtctctcat tagagctttc
5041 caatatttga caattcattc atttattttt ttcaacaggt atttattggg caccaatata
5101 tctaggcatt gatctagggg ctgggaatga agcaggaaat aagtctaggg gctgggaatg
5161 cagcaggaaa taagacaatg tttcaattct cttggaattt gcattccaag tgtgatgggt
5221 agaggcagac aatatgcaaa tgtgtaacat gtcaggtgtg ataaatgcta tgaagaaaat
5281 caagtagggg gagcggatac agtactgaat aaatcagagg agacctttct gattcagtta
5341 tgtctctaca gagtcctgaa ggtaataaga gagacagagg caaccatcag tgcagtggcc
5401 atggtgggcg cagcaagttg gcttcgatgt ctgggtatta gccagggagc cggtgcagct
5461 ggaggtgggg tgagtggagg ccagaagtgc agttgttgag gttggagaag tagccagaga
5521 agatcatgta aggtgtgact gtgctcccat aaaactttat ggacactaaa aattgaattt
5581 tatgtaattt tcctgcatca tgaaattaaa aaaaaaattg tttcaaccat tggaaaatgt
5641 aaaaaattgt tcttagctaa caggccatac aaaaacaggc tgcaggttag atttggccca
5701 tgggtcatag tttggcaatt gtgttatata taaattatta cactgtttta tcttcatcat
5761 ggtacacatc catacctgac atgttttatt tgcctgtgta tacatttatt gctttgtcac
5821 cgtctactag aatacaagtt ttttgagaaa aggagctctt aattgtcttg tttactgcag
5881 catcccaatc accagtgaaa agcctgtatg taatagagta gatactaaaa acagtatttg
5941 ttgactgatt gatggactag acctcaggat ttttagatac ccaggatcag gatatgaact
6001 actgcattaa atcatctcca acatctactc tcatgatggc ttgtctgact tcatgattta
6061 aaaaaaattc taaagcattt ttaaggcagt atattcaccc tgtgctattt tcctgtgggc
6121 ataggatagg gtttgtgtgg atgtatcaaa gacttctcca ctgtccttcc aggcacaagc
6181 catcctataa ggcgttccct tttaggagcc agaggtatag aaggaagtca tctgagccca
6241 ttattccagc ctgttggtaa cagaaagaga gatgagcaaa gtagcatttg gctgcacagg
6301 ggtctggaca aaacttcaac atcttgatgt tttccttcat ccctgagagc cttagaaatg

6361 ctacctatgc tttcatttta tgttaggttg tttgtaaaat catggtaaat tatggcaaaa
6421 tactctggtc agatacttta tcaggttagg ccaggatatg ctgtgaaaac aaacaccccc
6481 gaaatcttgg tggttcataa acaacagaga cttatttatt gcttatacca catgaccagc
6541 ctggttcatc tggggtcaaa tccacatggg gatgaagctg gctgagtatc cgatcttgaa
6601 tgttaggaat cactatgaca gagggaggga gagcctgaga gcgcctcacc ctggcactta
6661 aattctctgg cccagaagtg acacatatta cttctagttg cagccccttg acccactgcc
6721 ccatctagcc acaaagggcc aggaagtgca atcccaccat gcgctcagaa ggcagagaga
6781 tggaaatatt tgacaaaccg tattaatgac tgtgacacat ggtgagttct tcacattggt
6841 gacttctaag tgcttctgaa cacacctttg actgtagaat gtcaaaaaca ttctcaattt
6901 tacagcagtg ctggaaaatg aattgtattg agacaacttg ggaggagtag aaggagatgg
6961 aataaaaaaa attcactgat ttctgtcatg tgtcatgcct cctttaatgc acggaagaac
7021 gtatactttg cttaggaaat cacattacca cattcagaac aactttagtg aagatgggaa
7081 acaaccatgg ctggtcaatg gggtaacgtg tgctgaagga agacaggttt atttctcagg
7141 aggcatcttc tccctgggaa aaggaaagac acatattcat ctcagtaaat atttgtggaa
7201 tgtctaccat gtgccaggca tatgagtgga aatgatgttt ttataaaata aagatgtctt
7261 aagtatttgc atgtcttact aacagtcagg gagttcaact tggccagtgg gaaggataat
7321 tttatgcctg aaactttagt aatgtaaatt tttatgctga ttctcttttt gtcattcact
7381 gagtattcct aagtaactat aacactcaca tattggagat aagtattggt ttcctgaaat
7441 ctaggtttgg ctaacattgc ttctggaaag agaaaataac atgtgttgaa tgtccctatg
7501 gcaaattctt gctgttactt actgttccat tatggagagt gttagaaaga gtgtaatttt
7561 gattgaatga atatgctaaa aaaaacaccc taaataagaa actcacaaaa agaacagagt
7621 ggaatgaaaa agtgaaaaca actttgcaat ggtgtgcttc gtttataagt acattgtcac
7681 agcaagacta gggatgattg ggcatcttag agagtgcaga ggatgttgat aaggaagagg
7741 aagaggagat aagtggattt tgatatggga cactagatcc ttagggaaga aatgtgaaag
7801 agggtattct gggctgactg ccgctggcat agcaaggctg ctttgtagaa gggacaataa
7861 agttttgcat aaggatcctt tttcccatcc aatggattat gctatacttt agctggaata
7921 gggctgtagc agttagctat tgccacaata atgctgtgta acaaagcagt ccaaatttca
7981 gtggcttaaa acatcactac cttattctca cttatgtctc caagtcagct gggagtcaga
8041 ggattaaggc tcaaggctta ggtttggtgc aggtcagccc caggtgtctc tcatcctcct
8101 ttgatcagtg ggctagcagg agcatgtgct tgcaatgatg gtggaaatag aaaaacgtgc
8161 aatcatatgt gagtttcaag cctctccttg catcatgtct tgtaatgtcc tattggtcag
8221 tagggctggg aaatatatcc ctccctcaga gagtcaggag tggaaatgaa aatgttttta
8281 acaatagcct aatcaccata aaggctttgg gtggcacact gggcccttga tcttggagat
8341 cagtgataga ctgtaacaga cctcagaaac cccttggggt tattcagagc atacttctag
8401 agggtctggg gttgtcttgc cattcaagcc catcccacac ccatctgccc ccacatatcc
8461 taagcacagt tatgtcaagt gagaacagag gcatttgagt ggagcacaga tttcaaacac
8521 ataaaggcag ggcatggcca tctcccgcag tgctagatga tctggctggg agaaaaccac
8581 aagcagtctt catcaaattg gaggatgtgg aggtcccagg catttagtcc cctagtatgt

8641 ccttaaaaaa cccagacagc catgcacaca cactcacaca aatgcacacg cacacacaca
8701 tacattcaca cacacactct tttcctgcat ttgtagttcc tttcctcagt ctaagtttaa
8761 ttaaacaact tggatttcat atatattcat ttaaatatag aattgcataa agaaaagagc
8821 caccagaacc accaatggta gcatgaggcc tctgaaatga aagatgagcc caccccaacc
8881 ttaaatggcc aggtttctgg atttcagaga agcaagatca gacccttggc aggttccatc
8941 tgagcagcca cagcagccat agctacagca ataattatta gcaagtgtgc gttctccctg
9001 ccctagttag tacaccctat accatcaggt gtcatctgtt ctagttaaag tcagaccgta
9061 ataacaaaac atcaaaatgt aaaatgtggg ttgttttttt ctttacacac ttctaaagac
9121 atttaaagcc tgagcagata ccttaaaatt catcttctat ctgaaaatac ctcctagaac
9181 cattttaaaa aggagatgga ggcaaggtgg agagaaaaag tttgtctttg gttgacactg
9241 ggaaagttgt cttttaaaaa attttttaat gtttatttac tatggagttg ccgtgtcttg
9301 ctcttttcaa gctcacctgt ccggcttcta aggaatatca ggggccctaa aacacagact
9361 tctaaacccc aacacatagg acacacctaa gtgtggaaag aaaggaaaga ggcaggaaag
9421 aaaggaaaga ggaaggaaag aaaggaatgc aacatttatt gtgtgcctac catatacctg
9481 accctttcct acatatgacc acatatgatc tcttcatgag ctttatgagg tgacctatta
9541 ttaacaatgc tgctttataa atgaggaaac caagccttaa ggaagtcaaa taccatatcc
9601 tcagtcaaat agctaaaaaa aaaaaaagaa aagaaaaaga aaaggtagtg cctggattca
9661 aagacagctt atttttttaa cttatagagt aaaatggatt ttttcccttt tggtgtgtag
9721 ttctatgaat tctaacacat gtatagattt atataaccac aaccataatt aggatacgga
9781 actattccat caccccccaa aaattcactt acgttatcct tttacagtca gaccttcctc
9841 ccagcctccc aacctctggc aatcactgat ctgttctcct tcactgtcgt tttgtatttt
9901 tggttcgctg tcattttggt tttttgggaa tgtcatataa atggaatcat acagtatgta
9961 gcagtttgag attggcttct ttcactcata ctgcctttga gattctacca gtgtcccagt
10021 agttcattcc tttttattgc tgagtagtat tacatcgatt gctgaaccgt tcacccattg
10081 aatgacacat aagttgtttc tagttttttg gctattgtga ataaagctac tacaaacatt
10141 catgtacatg tatttatgtg aacataaggc ttcatttatc aagggtaaaa tccccagaag
10201 taggactgtt gggtcatatg gtgagcatat gtttataaga aactgcaaac acattttcca
10261 gagtgttata ccattcaaag ccagttttga acaattcaaa tctatgcttt ttctttccag
10321 taagctgttt ctccagacga cagagtggtt tactatatta ggcaaagctt catcttgggg
10381 aatgttattt ccaactgaga gctatgggag gcaaagtcac aaaccttcag ctcagactca
10441 ccaatctaac ttgatctggg aagatacctc gacaggtggg ggagggagaa catctgctga
10501 ggaaggccgg gagtggagtg agacattgct acttggaccc caaaaccagg aaaaatttgg
10561 tggttttggt tgagcttgaa ccatcaggtt aaacaggaaa tgcaaaatgc tcttccagtg
10621 aaacagaaga agctgctaag aggtagaggg aacatggaac tatttgtatt gcaaccgaat
10681 ccacctcccc aaacccactg caaaggaaaa ttgctgactg caatgtccct gaactccagg
10741 gtgagtaaga agagaaccac gccccccagg gaacacttgt aaatttttaa gcaaatctag
10801 ggaaagttgt gtaaaatgtg tcccttccat aactcaccct aaagcttctg ggtattctaa
10861 atatacaaat tctattcatc acaccgtttt atcaccaagc ttcctgaagg catattgcca

10921 acacaatttg acaaagaaaa tagcaccaac agctttttaa agccttggaa ggatagtctc
10981 agacccatgg ctctctagag gtccagggat gaatctagca aagctggcct ccttttccat
11041 atatatagaa caagaggatt gttaatagca tgggattgga atggctcaag aaaagctttt
11101 tgaagctctt tgagaaaagt agccagggtc cccccttaaa ccttctttgc tgatggcctt
11161 acagctcaaa accagcattg gaaccggcta ggtactccca aaccaaagcc tcttccactt
11221 ggcatgtata tttatattct catatcttct ttcaaatacc tttcagtgtt gccttttgaa
11281 aagattataa tttgtacttt tttgttgaat tcagtaggtt aaagcagtac ctattatctt
11341 taattttatt ttgaaaaatt ggttggtttg gcttttgggg aatttgagag gcaatttagt
11401 ctgtcattta ggatggcatc atataaggca gccaacttca tcatgctatt tagcctgaaa
11461 agcgatattg ttgatcttca accttgaaaa agtcgtacag gttgggagct gacctgcaaa
11521 tggtcaggta tcttgagata ggaaaaggat ttacaaatcc aggtcagcaa gatactcata
11581 caaatatttt gaacccaagg tatacatcaa atagatttaa agactaagac tatttctgag
11641 catgcatcga ctgtttgaag actagtggac ttgtatgaag ttcaggagtt aacctgggtc
11701 ttgtaactta aaaacagcat ttctcatgcc ctaaattagt agtagagaga caatatagag
11761 agttcctata tagccctcac ctatcttcct ctaaggctaa cacctgggaa gttttttttt
11821 aaaaaatcct gatggcaaag ctgcagccca gacccaaaaa atgagaatct gtgagattta
11881 agattcgagc atcagtattt ctttgaagct tattcccagg tagattcaag aagaagccaa
11941 gattttgaat gcctgcttta ttattttttt ttttgtgaga gctttaatgg cacaaaatgt
12001 ttatagctac aagttataca tgtattgtaa attgtatata acgatacaaa gtgctaacta
12061 aatagatgga gaacgcataa ccactttggc tcagttaatc caataatttc cacaatgggt
12121 tcatgaaaaa cctcatctga ctggctaagt cttcaattct cattttttta gaatagtaaa
12181 tatttttact gcaatcacag gaacttgttg cgctttccaa tgtgcaagtt tctgcagttt
12241 tgctcttgat tctatttaca aatatcttaa gccagttttc atcaaacata tgaaaaatga
12301 agcctgcctt tttgtcccca aattgtaaat aggtgtaaat ctttttaagc atcaatctca
12361 caatttcaac agttaattgt tgaattctcc tttccaatac acgatgtttt cgttcaacac
12421 aaatctgggt agtagtaaaa tgttcatccc gtgtgttagg gctacagata catactacgt
12481 gctctcataa taaattttac tgttatgatg tattcatttt ttaagtaatt ttccataatt
12541 tcactgatgc tttaagaaaa agagcaacac tatgtttctt tttaacgttg aaagaaatca
12601 taaatatacc caggttcaat ggcgctactg atagaactgc tgctgaattt ggaatgagca
12661 caatgcacta gctgtaagaa gtcagttttg ggtaatgaat aattttactt ttccctcctg
12721 taataccaat ttcatactgt agcagtttat ctagtcagtt ctcctaactt tctgtccttt
12781 ccaattttat tcaggctttt caaaaatcag tgagccatca gatttacttt cttcccaaag
12841 aaattcaaat atgaatgctt gaaataaata ggttatagaa ggcaaatcaa gtgttatcag
12901 gtttaagatt aagacagaaa catggtctaa taaaaagatg tccaaaatta tctagacatc
12961 aaatgctaga aagaaaaaag aaaattactt gcaaaactat ttaggatata tcatctttct
13021 taaagattgc cagctatatt cttgaaaaaa agataaaaat tttcttgttt aacctgcatg
13081 ttattttcca ctataacatt aattttacaa atgttgatgt gagagagaag agttagatag
13141 tttctgcaaa ctggcaaacc tggctgtgat agtgttttta ttcaagttaa cctggtgcac

13201 tttatagatt gctagcttta aaattaacag tgtgaacccc tgctttaaac ccttctcaaa
13261 gtgcagtcag ccaatcaaca gtattgccat cacctgggaa cttgttagac cccactttgt
13321 caaactcatt tcctggtatg acaatgaatc tggctacagc aacagggtgg tggacctcat
13381 ggtccacatg gcctccaagg agtaacaccc caggaccatc agccccagtg agagcacgag
13441 agaaagagag aggccctcag ctgcttggga atccctgcca cgctcagtcc ccaccacact
13501 gaggatctcc tctcctcaca atttccatgc agaccccctg aagagggaag ggcctaggga
13561 gccccacttt gtcatgtacc accaataaag ttccctgtac tcagccaaaa aaaaaagaaa
13621 gaaagaaaaa agaaaaagaa atgcagcatc ctgtcttgcc ccaaacccac tgcgtcagaa
13681 tctgcatttt aacaagatcc ccaggtatgc acattaagtt ttgagaggca gtactacata
13741 taaaatatgc aacagaccac acaaacctgg gcccaattgc tatttgtcag atacatgtat
13801 gtatttatta cttataaatt ccttcatcag atatgtatta cgtgcttatt actttaaaaa
13861 gatgaatctg acacatatcc taccctcaaa gagatctcag tctaggaagg gaaatacaat
13921 gtgttcataa ataatatagt ctaagttagg gacttcataa aatgcttgat ggaagaagtg
13981 gcatttattt tggacctgga atgagaactg aaagaagata ttcaaaagac aagtgatgta
14041 gtgatgtagt tttgaataca cgaataatct cagggttact ttttctatag cttttggtga
14101 atcaagttgt ttattcatac atttaataaa tatttattca gctcttacta ttgctataag
14161 acagatacca ttactgctca ggtggttttt acaacctagt ggggaagaca agcagataac
14221 aagaaatgac acaattcatt agtgttactg gtagcgttgt gaaggacaag gatggggtgc
14281 tagaaatcca tgtgacggac cagacctagg caggggtggg agttatggag gaggtttccc
14341 tgagtaggta acatctaagc tgagaaagat ttagttagac aaagagtgaa tggcattcca
14401 ggcaaaggaa acagcatgga ctaaggtcaa aacaagaaga aatgaacgaa agagcaagtt
14461 gataaaatgg atgtccaata atattccatt gttcaactat gcaacatttt tcattgcctc
14521 ccaaattgga tcaaagtagc ctggtcttca tgcctccagt ccttcctctt tttaaaaatt
14581 ttatacaact atattaatat agaatttaca tactataaaa tttcattcaa gtatattagg
14641 aaatttactg agttgtatag ctatcatcca tataaggtcc gttatgtccc tttgcaatga
14701 accctccatt ctcactccca gcctcaggca gccaataatc tattttctgt ctctaggggc
14761 tggaaactta ggattggaat tagttggttg tataataaat ttagcttatt agagaactgc
14821 tgaactcttt tccaaagcaa caatacaatt ttacagtttc atcagccttg tgtgagggtt
14881 cctgttgctc catatccatt acaacacttg gtattgtcag tccttttgtt tatagccatt
14941 ctactgggtg tgaagtggta tctcatttca ccagcccctc ttcctaactt atttgccaga
15001 ttccccactt ctaaacttgc tctatgccca accaaagtga accccttgct gttttttttt
15061 tcccctgagg ctcgttgata tccccaaatt atgcctttct tcattctttg aatgtgttac
15121 ttgactggat atgtattttt ggtgaaggag aggggctgga aattgagaga tgaaattggt
15181 aggagtacag aaggccgttg acttcctcac aacgtgggac acttccaagt ttctcctaca
15241 aaccccacca ctataccttt ccaaatatct tgcctgccat cttggtcacc cgtgacctaa
15301 attaatactt aggaagctat ggagatgctc atgcttcctc aggggagaat gctgtgtgaa
15361 cttagaaatt agcaaaaggg gaaatgacta cccttgaaat tgtaccacag aaaggagcat
15421 acattcaaat gatggtgaag tcttattaaa aagactttaa gcctcccaat ttagaagctg

15481 gcttgtggta gccaagaaat tagagaatga tttgttattt aagagtgaaa ttgctagtag
15541 tcttgaatta attaccataa aaaagctgcc gctgtaataa aaagcagaga tctggtaacc
15601 ctgcttcatt cactttcttc ctcttgatga aaacctttta attattttga ttttgattag
15661 cacttccatc agaaaatagg aagaaattag gagcaattta cttggcaaag gtttagtaat
15721 taaaaccact gaaatcaatg gtttaagtat gtatttggga taaacacatt tttaattgtt
15781 atttttgtct tacccaagtg gataacataa agttcattat acaaaaatca accagtgcag
15841 aaagccgatt gggaacaaac aaccattctt gaaccctttg aaaatgtatg cccttgatga
15901 attacttgtc aggtttctac ttaagtttaa ttgttagtac ttccacttaa aatagcaaaa
15961 tgactgtctg caatttctgt tatttcaaac taggttttaa attctggggt tgaaagtaag
16021 cttaaataac tctgagtact tgagtaaaca ctgcattttt tatgatgagg caagttgtat
16081 gagccattta attactttaa ttcaatccag agatttgata ttttctcctg agttctcaca
16141 gttggatagt ttaaaaatat ttctaagatt gaattgaatc ctgtaagaac tataataaaa
16201 ccatacatct gcctgttttc tttctttctc tttctctcct ttcttttctt tctttctttc
16261 tctttctctc ctttctttct ttctctttct ctcctttctt tctttccttc ttttctttct
16321 ttctttcttt tctttctttc tttctttctt tcctttcttt cttctttctt tctttcttct
16381 ttcctttctt tctttctttc tttctttctt tctttctttc tttctttttc tttttctttc
16441 tttctttctt tctttctttt tcaagatcgt gctccattgc ccaggctgga gtgcaagtgg
16501 cgagatcatg gctcactgca accttgacct ccccaggctc aggtgattct cccacctcag
16561 tctcctgagt agctgggagt acaggcatgc accagcatgt ctggctaatt tttgtatttt
16621 tagtagagac agggtctcac catgtttcca gtctgatctt gaactcttgg gctcaggcaa
16681 tcctcccacc tcggcttccc aaagtgctgg gattacaagt gtaagccacc atgcctggcc
16741 ttgtattttt ggataatatt gagtgaaatt tgcctcaata cataaaaatc ctttcaatat
16801 gcttcaactc taaaatcttt aaaaagctgc ttggtttcat tattttcacg tggaagcagc
16861 attgtcacaa agttttattt tgaagactga attgtatatg gagtactgtc caactagaag
16921 agaatgaata atcaggaaaa gagagactat tgacaaataa gacttaatgg agaagcattg
16981 aatttcaaag ctgccagaga cattagcaat tgttcagttt catctccaga tttcttagat
17041 gaagaagctg tggcaagcca atttcacaaa cccgatctat gatcctcatg ctccccactc
17101 ctctcactcc cacaattttg ccttgattca aactttatcg aaaagtgaga aatcacaggt
17161 atgagctcac tgagcttccc aggcagtctt gccatctaca aacccatgtc attctgtatt
17221 ttcctcttgt ttctgacagt gatcctctca tctagtcaca ctacatccac ttatctctgg
17281 cacttatgtc aaaacaaccc ctctaggtcc tgccttagct ctcctgcata tgcttttct
17341 ttcttcctgt ctgttgcctc tttcccccag cttataattc tcccgctttt attaaaaaca
17401 tgcacccaca gacacacact cacatctgga ttcccaagtg cgataacctt gtagattcat
17461 attttcagat cgttcttagt ccacagaata taaaattgat tgaatgatgg caaacatctg
17521 cagatgacaa tcatttacag aaatggcact aaagccatga agctgaagct gcaggactta
17581 tggatctggg ctagaagaca ggggccatca aaaatttagc ttcagttgga aatcggctct
17641 tttagtgcac tacatcagat ccatgaaaga ggaaggaaga gttactgctc attgctgcct
17701 ggagctgtga cttctatgaa attacttaac ggggcaaagg aggaagcaga tacttccttg

17761 caactaggac gtgagctgag ctgatccctg aatcataaat actcaacaag attcggattc
17821 aggacccagg gtattatggg gtacaaccat aaaacttcta aatgttggag aaagaataat
17881 gagaaagaaa aatgaatctc tgaatgaaaa tggaactact aaccaaaatt ccaaatcacg
17941 ttaatataat tattactaag aatgacagca acaaaatgaa taatcagtat atgtcatacc
18001 tataaagtta aaatctaaaa taatttgaag aaatacttaa aaatatttgc aaccctcaaa
18061 gagataaaga aataatattc ataaaattga aatgacaaac taggggcagg gcgcagtggc
18121 tcacgcctgt aatcccagca ctgtgggagg ccgaggtggg tggatcacaa agtcaggaga
18181 tcgagaccat cctggctaac acggtgaaac cctgtctcta ataaaaatac aaaaacaaaa
18241 ttagccgggc gtggtggtgg gcgcctgtag tcccagctac tcaggaggct gaggagggag
18301 aatggcgtga acccgggagg tggagcttgc agtgagccga gatcgcgcca ctgcactcta
18361 gcctgggcaa cagaatgaga ctgggtctca aaacaaacaa acaaacaaac aaacaaacta
18421 ggaagaatat acagaaatgt aacagaaagt agataggaag aagaatgaag tagaaatgct
18481 tgaaattaga aatacggtga ttgaaattaa aaccttaaca aagtaaactc tagactcaca
18541 acaattggag agttggtgat tggaaagatg aaccaattaa tttacccata acacattaga
18601 gagaggtgaa gaggaaaaaa tagctaaata taaaagcaca tttaagagac atgtaagatg
18661 attaaaagcc tccaacattt ttcaaaaaag agagtagagg caatattata ggtggaataa
18721 tgaaaatgat aatggctgag aatattaaag aattgagaat aaaaaagaca agtggcctca
18781 gattgaaact gccatgaagg tctctgtctc aaattctcaa ctctgctttt gtagtatgaa
18841 agcagccgta gacaataagt aaatgaatgg acatggctgt gtgtcaatac aacttgattt
18901 acaaaaacag gcaggcagca ggctaaatta ggctgggagg tcatagtttg acagctcttg
18961 atctaaagaa taaccattgt tggccaggcg cagcggctca tgtctgtaat cacaacactt
19021 tgggcggcca aggcgagcag atcacttgag cccaggagtt caagaccagc ctgcgcaaca
19081 tggcgaaacc ccatctctac taaaagtaca aaaattagcc gggcatggtg gcaggctcct
19141 gtagtcccag ctacttagga ggctgaggca ggagaatcgc ttgaacctgg gaggctgagg
19201 ttgcagtgag cggagatggt gccactgcac tccagcctgg atgacagagc cagactcggt
19261 ctcgaaaata aataaagaat aacctttgtt tacattttca tgtatatgtc tccagatgtt
19321 cctcatatat ccatataagt atgtatatat gatgtacata agtgcataag taaaaataca
19381 taagtaattt taaaagaggt tatatcatag ctgaatcaca catgacttag accacacttg
19441 ctcttctgag tacccatttt tggacaactc tcagtgtcca ctagcatgtc taaaaaaaat
19501 ttctcctcct aaaatgctaa tgcatgatgt agattttaac tacagctttt gaggctcagt
19561 ggaggagagt gtacatcaca ctacccaatg caaagccttt taacagcccc ttttgctcat
19621 ctttgttttc cttcactgtt cctcgccctg cagcaggcag ctgacattca cctttccctt
19681 ccaccctcct ctctcagcac tggtcctttg cctcctccct catcccagct ctacaaatgg
19741 cctcagcctg catattccaa ccacccagca aaacaaacat ctagtaacta ccgggtcagc
19801 aagagaaaat cggaggccct agccatagat agatcacctt atcatttctc cagcacattc
19861 caggaaggat ctcaattcgt tctgtcccaa tatacctcta catcctttgt tttgatgatc
19921 acatagaatt cattgtatgc atatatcatt atttatataa ctaagccccc aatttgaac
19981 aagaggctac atactgaaaa tcttttatct accaagtctt ttttttctta caaacccatc

20041 caatacagtt gcactgtatc cgccaataca gttcatgcaa ctgtatggaa agcaaactct
20101 agaagagtat ttgatagctc cataagaata ttaaactctt atacttctag agcgtaaaac
20161 cccagaaaag tattttgtca gaaaaaaatc atgtttttga aatagtaggt tttaaaagaa
20221 aacaaagaga gttagaagta agaaaattca ggttatcagg ggcttgatat cttttttttg
20281 tgactgtagt caggttaccc aggcctttgt ggatcaattt ctcaaacaaa taacagagat
20341 gaaaacaact gtcagtagct actgtcacaa aaataatatg ataaacatct tttcgtttcc
20401 tatggctgcc ataactaatt atcacaaact tggtggctta aaataacaga aatgtatttt
20461 ctcacagctc cagagtccag aattccaaaa tggaagggtc agcaaggctg cacgccctca
20521 aaacactctt ggagagaatc ttttcttgct tcatccagct tctgatggct ctaggcaatc
20581 ccccgcttgt cactgcatca ctccggtctc tgcccccatc ttcaaatgac cttcctctgt
20641 gtccctgtct tctcttctgt ctcttataag gacacttgtc attgtacagt atttagggtc
20701 cacatagata atccaggagg atctcatttt aagatcctta acttcattac atctgtacag
20761 atcctttgtc caaattaagg taatgttcac aggttctgtg ggttaggatg tggacatatc
20821 attttgaggc ccaccattca atgcactaca attttctttg taagtgttct gagaacccag
20881 gaagaataag taaatcctct gattcaatca aaaatgagca gaatgtaaac caaaaatact
20941 agaagatatc ctttttccta ggcactggca tacgtttcaa tagtatctct aattacagat
21001 gtatttacac ttctaagtgt cagaaatgaa gattattatg acaaatttaa aatttagtaa
21061 cttgttttta aactggtatt tttgtttttt taagcgatca tctcagtcca cattggaacg
21121 atctgaacag cagatcaggg ctgcttctag tttggaggaa ctacttcgaa ttactcactc
21181 tgaggactgg aagctgtgga gatgcaggct gaggctcaaa agttttacca gtatggactc
21241 tcgctcagca tcccatcggt ccactaggtt tgcggcaact ttctatgaca ttgaaacact
21301 aaaaggtagg tgtgtggatg tggagacaaa ttctttctct ttattagata gtgacaaaca
21361 cacttacttt ctccaaaagt ctgcgtaggg cagacacgtg tcagagggag aatgaaagtc
21421 ggtctttgat ttccagtatt agtgcagaat cttgattttt taatgtttac ttcacagaga
21481 aatatattca atatgatttt gagataaaaa acacaaaaga acatctcaaa atatagaaat
21541 aaatttaact caaggatgtg ggggcaaaga aaacccagga agactattgt tctcaataga
21601 aaaacaatca tcaggtttac ttagaaaata aaacctgggg attcatccac aaggagtatt
21661 ctactgccat acatacatag acaaaaacca aagatggcca ggcatggtgg ctcatgcctg
21721 taatcccagc actttgggag gccaagaacg gcagatcgct tgagcccagg agtttgaaac
21781 cagcctgggc aacatgacga gaccccatct ctacaaaaaa atagaaaaaa atagctgaac
21841 attgtggtac gtgcctgtat tcccagctac ttgggaggct gaggtgggag gatcggctga
21901 gcccaggagg tcgaggctgc agtgagccat gatcatgcta ctgcactcca gcctgggcaa
21961 cagagcaaga tcctgtctca aaaaaaccac cccctccccg aaccaataga ttaggtatag
22021 cttttttttt tatttctgca aagttgtgtg attttttagt aaaagtgaaa agtaaaagtt
22081 agacactgag ttgtaatatt gggtactggt attgctagga gaataatgaa gggagaaaat
22141 tctcaagaca aaatgatcaa aactccagac tagaaaataa cataatttga aataaggata
22201 atgatcgttc cctagtaacg tatgaagatt ctttaaaaga aaatcaggcc aggtgtggtg
22261 gatgacacct gtaatcccag cactttggga ggttgaggca ggtggatcac ctgaggtcag

22321 gagttcgaga ccagcctgac caacatggtg aaaacccatc tctactaaaa atacaaaatt
22381 agccaggcat ggcggtgcat gcctgtaatc ccaactactt gggaggctga ggcaggagaa
22441 tcacttgaac ccaggaggcg gaggttgcag tgagccaaga ttgcaccatt gcactccaac
22501 ctgggcaaca agagtgaaac tccatcttaa aaaacaaaaa aacaaaacaa aacaaaacaa
22561 aaaaacaaag aaagaaagaa agaaaaccag tgtgagtagg tactgtcata atgaataaaa
22621 tgcttgtcaa gtaaagttct gtggtttctg attaggcact taattttttg atgtcatgtc
22681 aaaacaacac aagaagctgc tacagcattg ctgatctagc attctagatt tgattaacat
22741 gattacctgg taactgacta tatatatagc ataattcaaa ttaaccacta ggccaaaact
22801 atttttttct ttctacctaa aattgtattt gttttttccc atttgtaaaa agatgttggg
22861 gttgcacaga cctctgttgt tggctgtggg gagagctgtg catagatggg gacatattct
22921 ttggtagcac cttgggcatg agtgtgaaga ggattcttag tgttcatgtg tatgactgtg
22981 tgtctaagtg agttgatgtg gtaggaacca tagcctcgag ctcacttcag acagaaattt
23041 gaagctctag atgtttctat gtggtgaatt aatatagcct tcatgagaac aaagggttgg
23101 cacctcatct agaggtcaaa ggccttaacc agattacctg actgaaggcc tgaggtgaac
23161 ctggagctat ttcatgaaca tgaacgttac cataaaacca agactaggtg atctaaaaaa
23221 agtcaatgac attgacattt tctaaggaga aaaccattaa aatatcacgg aatttgcaaa
23281 ccctatgaat ttatctcgta atttctaggg gatcatctga agttatttac taagttagaa
23341 gcagtcaata atgaaatcag catgttacac caattagaat ttgttatcaa ctctcccttg
23401 tggatttcaa ggtaatttcc tgaggcagag cagtcctgct gggaagcagt aaagaatagt
23461 agagttatga agcctttaat cagtgatgtt ggtcataatt gcatatgatt ttgattaaaa
23521 gcttaccact ccaatttcaa aacttagttt tataaaaaga gaaaaagcaa atggaaagca
23581 acctttaagt agaattttaa attgggaata ttttcatagg ttggtttctt gttttttcaa
23641 ggattcatga ttaattatag tgaattaaat aataaccata ataatactta attgagtaat
23701 ttatctctca tgtgttcagc cttcaggctt tttatataaa tattaataat cagaaatttt
23761 taaaaaggtt tcttgggggc tgggcacagt ggctcacgcc tttaatccca gcactttggg
23821 aggctgaggt gggcggatca caaggttaag agatcaagac cagcctggcc aacatggtga
23881 aactccgtct ctactaaaaa tacaaaaatt agctgggcat ggtggcgcat acctgtaatc
23941 ccagctactt gggaggctga ggcaggagaa tcgcttgaac ccaggaggtg gaggttgcag
24001 tgagccgaga tcgcgccgct gcactccagc ctggcaacag agtgagaccc cgtctcaaaa
24061 aaaaaaaaaa aagtttctca ggatagaatg ttttattatg aggaaaaaaa taaaaaaaaa
24121 caaagtttag gtagactgtg tagtataatg cagtgctcca gacaactcat attatgtttt
24181 cagtgattga agaagcaaca tatgaaaaaa attggtatta agggatgaaa aaggagaaag
24241 gaatggagaa gatgttataa gggaataatt tagggactag ggtcacatct caaatggtaa
24301 tattgtatca gtatacttca agcagacgtt ttctctattc cttctattcc cctatatggg
24361 aattcaggat aatctcagga gagttcttga aaatgcagta aaaataaagg agagtttgcc
24421 tattgttgca taacacatta ccacaaaact tagctactta aaacaatatt tatctcagtt
24481 tctgtgggcc agaaatgctg acatggccta gatcagtttt tctgcttcag atgtctctgg
24541 aggctgcagt taagatgttg gcttgggctg caggcatctt agggcacagc tgaagcagag

24601 tgtgctttca agttcagtca gctgttgttt gcaggactca attcctcaca ggctgttgga
24661 ctgagggtct tagttcctcg ctggctattg gccagaggct gcctcaagtt ccttgtcgtg
24721 tggacctttc taacatggcg gcaaactaac acaagaactg aaaaccaaac accgcatgtt
24781 ctcactcata agtgggagtt gaacaatgag aacacatata cacaaggagg ggaacatcac
24841 aaaccagggc ctgttggtgg gtggggggca aagggaggga gagcattagg acaaatacct
24901 aatgcatgcg gggcttgaaa cctagatgac gggttgacgg gtgcagcaaa ccaccgtggc
24961 acatgcatac ctgtgtaaca aacctgcacg ttctgcacat gtatcccaga acttaaagta
25021 aaataagcta aaaaaaaaaa aaaaaaaaaa agcctgcaag tggagaagga ggagagagag
25081 agagagagtg tgtgtgagaa agtgagacag agtaagaaag aagggagctt gcaataagta
25141 gaaacaagac tgcaatcttt tttaacttca tcatggaagt ggtatcccat cacttttgct
25201 gtattctctt cataagaagc aagttactag gttcagctca cacacgaggg gaaggcattt
25261 acacaagggc atgactatca ggagatgggg accactgggg gttagtttca aagctgccta
25321 ccacaagaga ttttcaccag caccacaacc ccacacataa taaataaact ctacagttcc
25381 atggcaagct acaaggaagt ctttgtgttg tctttatcat aaaattgaat ctttccccca
25441 aaacacctgt agaaaggcta tgcagcatta agtatcaaag ggtcaaaatc taatttaaaa
25501 aataaattat gagagaaaag caagtactgt tttacaagaa agtatataat tttttattc
25561 ccataggctt ctttttttgt taccttcaaa ttaactatgg tcaaaggatt gctcattcca
25621 gtaataagaa caggcttatt tcatcattta cataaatatc agtatcgggc acttgcaaat
25681 gagaaaactc tgatttgagt aaggattagt ttcacttcaa tttccattta tgcgtttgcc
25741 aagtttaaat cctactgtag ttacttaagc aaccaacaga aacatatttc ttgatgatgc
25801 taaaacctac ctacacctgg ctttaaagca gaagaaatgc tatccagctg tatcctataa
25861 attgcttgat aaataatatt tacatgttat ttctttacac gcttctattt acttccctat
25921 taaccagctt ctccagatgt attcagtgta attatgtaca ggacatggtg ttctacctca
25981 aagggaaata aaatacttat attctacaag gtatggtata aatgggaaag agcacagaga
26041 ttggttgtaa cttctctgca aacaatcttt ggttaaagga ctgacaaaaa atatccatct
26101 agtaaaagaa acaagcagta gaaatgtgaa aatgactgtg ttcagcataa ccctggcaca
26161 tgttggcaca ccaggaccat tccacctgct agccccagtg agctttcttt ctctctgcag
26221 ttatagatga agaatggcaa agaactcagt gcagccctag agaaacgtgc gtggaggtgg
26281 ccagtgagct ggggaagagt accaacacat tcttcaagcc cccttgtgtg aacgtgttcc
26341 gatgtggtgg ctgttgcaat gaagagagcc ttatctgtat gaacaccagc acctcgtaca
26401 tttccaaaca ggtataagga catcggcaag cctgccgtct ctaaagccct gatgataata
26461 gtgttgatag ctacaacaac cttgttgcta aacactgtac taaatgcttt atgtgcataa
26521 tcgcatttaa tctttgaaac aaatcaagag ggtattttat gctcctttat agatgaggaa
26581 actgaggctc agagaggtaa cttacttatc caagtttaca cggctgataa atagcagagc
26641 tgggattcaa acccaggtct gtgggaggcc agagcctgaa ctcttatcag ttattgtctc
26701 tctgatgcta tgatgtcaag actcagaaaa ccatatggaa gttactagga caggatgtta
26761 aaaggaatga agatagctga tggcaggttc tgtgttgctc aaatggagtg attgagaaaa
26821 atttaatgaa ggttgtgggc agaattgggg aaagagggat gggacagtga agcacctgaa

26881 gctagcaaaa agggtgagat ttccccaccc ttcgactgag gagacaaggg gagggagcag
26941 ttatggaacc ccagagggaa agcggagaag agggtttctg gactggccac tgtcaactct
27001 tagcctggca gggagtaggg gcaggggctg ggagaggcaa gagggtttat agatgtagcc
27061 catggagggc agcgtcctgg ggcacagagc ctgatgggga aggaagggga gaggatggtg
27121 tctctggagg ggcaaagaga gaatatctag cacaaaagac atttggaaat aattctgcag
27181 tccccatgaa agaaataatt cagccctctt catcttataa atttcccttt gcttagtagg
27241 taagttttcc tttctctggt taccatgttt ttaataaatg tcgcatatga gagcagtagt
27301 aacaaacttg ggaaagcaat actaaaaaac tgtacatcaa actacattat ttttggtatc
27361 tgttgcaaat tctttgaatg ttgatatact tatttaattg tcagtttgcc tatatggttt
27421 attctccttt taactgcaaa aagaggtagt acaagaaaat tacatgcact gaatatatcc
27481 atgttaggta attttcttaa tgctgatttg ttttgttaat agatttactt atccttaatt
27541 ttgttcctcc accctctaaa atatccttta aactgcagag tttctttttc taaattcttt
27601 tagagaatct tggagcttgt gtgtatttta acctctacta tttgacaatt aggaaaaaaa
27661 aatgacccaa gtagggctaa gcatcaattc tgagtagaaa aaaagcttgc ataacgtaag
27721 agggtttagt tcatgatcat gtgattccct agagcatagc caaaaaattg tgcaagaaga
27781 tgaagtttat gcaaagtaaa cactacaaaa acactctcac tgagactcat ggaagttcca
27841 ttacatattg agatgtcaga ataaacacaa ccttaatgga ttgcttccac ttaatagata
27901 gttgagtact ttattttgac tgctgtactc aacaaatgat taatttcata caatgtactt
27961 aagtgtaagt gtacagcagt ttttcttcca aatatgcatt tttgaacatt ttaatagtag
28021 ttttttaaaa actatctctc gccccaaagt tatggactat ttggcaaacc acctcttaga
28081 aacaaagtga acaaatgaat actctatgta gtaaggacta catggaatgg tgattgaata
28141 gacaatgcat taaaatcaac ttcaaactca tccacacatc atagaaccac tcaagaaagt
28201 tgtgtaatct ctctgtacat ctgtttcttc acctgtaaga tggggataat aaaaacccac
28261 cccataatgt tgttataagg ggtaaatgag ttaacatgaa agcacttaca acagggcctg
28321 gcctacggaa agcgtagaaa gaatgatata catgttagct ataattattt gtttgggcat
28381 gatgatcttg tatcagtcag ctattaatac acagtgctac gtaacaaaca cactaaaact
28441 cagtggttcc aaacaacaat gatttactct cacttatgta tctgaggttc agttagttta
28501 gggtggattc agctgggctt gactctaagc tgcagtgtga gtccaagtca acttctatgt
28561 ttcccatcgc ttggaccagc aagcctgccg gattatgttc ttttcatggc tatagcataa
28621 gtttgagaaa acaagcccca taaaacatgc acagttcaaa tttgtacttc tgtcatgtct
28681 gctaacatcc cactagccaa agaaagtgac atggccaaat aaaaagtcaa gagacaggaa
28741 actatatttc ttcaatagag atggtgtgag aagggatgaa taaatagttt gaataataat
28801 ctattctaat aaaatcctaa agaaactgca tagctttaat ctcatttaac ccttacgatt
28861 tacactcaaa tttaaagcag gagcagggat taacagtacc attttacaag agtatcaatg
28921 atcaaaaagt taaaagattt gaccaaactt aaacacttca taaagttaga tgagcctgtt
28981 tagttccatt cttaatggac aacttggcta aagtcattgc cgtaaatgac aaaggcaaaa
29041 attcagacta gaacaacttt aaaaatgata tataaaatta aaaaaatata ttgtggttat
29101 ctttctctgc tgtagctctt tgagatatca gtgcctttga catcagtacc tgaattagtg

29161 cctgttaaag ttgccaatca tacaggttgt aagtgcttgc caacagcccc ccgccatcca
29221 tactcaatta tcagaagatc catccagatc cctgaagaag atcggtaagc actttgttt
29281 ttttttttat actggattat gcttattttt gccttgccaa aactgtgtgt agaggatttt
29341 tcattaatgt agagttatca atttatcttc aaattattca tattttagct tctaacctga
29401 acctactggt aataactggc aaatacatat ttttattaga tgttagatat atattgtggt
29461 aggaccaaaa cgctcagtgg tatgagcttg tcatgtctac ttgtagacac gtttgttctt
29521 aatatgcata gtctactgca ggtgtaaaac cgctgtaacc tcaaatttag cacatgttcc
29581 agaacaagca ttaaataaaa cttatttcag gctgtttgta tatttacatc ctgaaatgag
29641 agaaaacaaa gaagattcct ggggtcattg gggagtgaca aaactagcag cacgtgagtt
29701 ttgcaacttt ctaatctttc ccccttaaga ttaccaaatg gaaaaccctt attttaaatt
29761 aaaatgactt aaaattaatt tggctttgaa attctcttga gattaatttt gtgattattt
29821 ataagacaaa cgacaaaacc aaaggtaagg atcacttatt tgaagaaaaa aaaggattta
29881 atataaagaa acaagtaaag ggtaactgaa tgaaaagatc attagttaga tacttagact
29941 ttgcaaaagt aaagcaataa atatattgtg tgagtggcaa gtaaaagagt aagctggtga
30001 ttagttaaca ttgaaccaag aagacacctc ccgcctctct ccagcagaaa agaaaacttg
30061 tagaaaagca acatttattc tcaagatgtt cctgctgttc tggccaatgc ttttatcttt
30121 attttaaaga aaataatctt aggggccggg cacggtggct cacacctgta atcccagcag
30181 tatgggaggc tgaggcgggc agatcacgag gtcaggagat tgagaccatc ctggctaaca
30241 cggtgcaact ccgtctctac taaaaatata aaaaaaaatt agccgggtgt ggtggtgggt
30301 gcctgtagtc ccagttactt gggaggctga ggcaggagaa tggcatgaac ctgggaggtg
30361 gagcttgcag tgagccgaga tcgcgccact gcactccagc ctgggcaaca gagggagact
30421 ccgtcaaaaa aaaaagaaaa gaaaataatc ttaggaggta tgttagaaca tatttaattt
30481 tcgtaaataa actgttgaca gctataattt gcatagtttt aacatatacc ttcttaagaa
30541 gattacaatt tccaaaatta cagtttagtt tctaaacatt tccacgtaga atatttataa
30601 ttacagaatc actatctagt aagatgcagt tcataaatac aaattaagct cctttaaaat
30661 cttttttttt tttgagacgg agtttcgctc ttgttgcccc ggctggagtg caatggcgag
30721 atctcggctc actgcaacct ccgcctccca ggttcaagtg attctcctgc ctcagccttc
30781 caagtagctg ggattacagg catgtgccgc cacgcccggc taattttgta tttttcagta
30841 gagatggggt ttctccatat tagtcaggct ggtctcgaac tcccgacctc aggtgatctg
30901 cccgcctcgg actcccaaag tgctgggatt acaggcgtga gccaccgcac ctggcccgct
30961 cctttaaaat cttaactctt ctcacatcct tatcattttg tggtgagaac atttgaaatt
31021 tactctctta gcaatttgga aatataaaat acattattat tattgactat agttaccatg
31081 ctgtgcaata gatctcaaaa ccttatttct ccaaactgag tataaaaagc aaaaaataaa
31141 tttaaaaaat cttaactatt atctactcag gaatttattt aatgttccaa agtttgatt
31201 atccttgaaa gcttttaaat ctggttcatt gttttctttt cctagctgtt cccattccaa
31261 gaaactctgt cctattgaca tgctatggga tagcaacaaa tgtaaatgtg ttttgcagga
31321 ggaaaatcca cttgctggaa cagaaggtaa tgatagtagt agtaaaataa taacagtaat
31381 aataaaagta gcaacaatag cagcaaccgt tattgagtac ttagtatgac cagacaatgt

31441 tgtaaatctt gtatatgttt taactcattt aattctcaca acaaccctgt aatgtactat
31501 tgttatttct ttttttacaa ttacaaaatg ggcacaccaa gatatcgagt aatttcctca
31561 aggtcacaca gctgagagtg atagaagtgg tcagattcca gaaccctctg taccataatc
31621 atatagtaag tccatcaatg catcactctg ctttttaatc tcaaagaaaa gcccagagtg
31681 gttggagcat cctgaaacac tgaaattgaa aggggatgtt aaacttgaat gaaagctgct
31741 tcagaactaa atactagtca actgcagttt actaatttat ttgtataaaa gtctcacagg
31801 aacataaagt aattttggac ttggtttctg tcagctgcat catatgaaaa cacgccagta
31861 atttgaaaaa agaaatggag aataagagaa aatttttctg gaattagggc aaagcaaaag
31921 caaaatatgg ctattggtct aaggggagct gggagcaaaa tgagatatag tcaaattgtc
31981 attgctcata atccgtaatt ctgtgacttt gggtgatgaa catttgcttg aaaatgaatg
32041 cattttaatg tattttccag agagctttac atggttccac caaaaaaaaa aaagaaagaa
32101 aaaaaaagtc acaagtagtc atgcagtttt aaccttgctt gttttaaact ataatctata
32161 agcaagaaca gtaagggcta aactctaaga aatctgtcct ataaactgta aatagcaacc
32221 tactctccat tcttctgcct ctcagcttcc ttctaaatgt tttcacgaaa gctcagccca
32281 gagcatgtgg ggaaatttaa tatttatgtg gtttaaatgc ccatgccgat cttgaatgtt
32341 ctttacaccc ttcccaccaa gtgttcagcc atcctctacc agaacataca tcagttattt
32401 ggagactcgc catccagtga ggcagcacca aggggtgggc acactcctgt tgttatactg
32461 aaatgaaatc tctctccaga acagaccaga ctgcccaatg gaatacaata agagtcatat
32521 atgtaactca cattttacat taaaaagata aaaagaaaca agtgaaattt atcttaatat
32581 attttattta atccaatcta tccaatgtac tatcatttca acatgcaatc aatataaaaa
32641 atttatgagc tagcctacat tctttgtgct aagtctttga aatccagtgt gtgttgtaca
32701 cttacagcac atctcaattt agactctcta tttcatgccc aatagtcaca tatggcccat
32761 agctaccttg ttggacagcc cagctcaaga gcatcaactc attagttcag gttttaccct
32821 atagaaccat gtaaaacaaa tctaattctt tctccatgtg actcatgttc taaaagtgtt
32881 agaaaatttt tgtggggttg gccgggaatg gtggctcatg cctgtaatcc tagcactttg
32941 ggaggccgag atgggaggat cacttgaggt tgggagtttg aaaaccagcc gggccggccg
33001 ggcgcggtgg ctcacgcctg tagtcccagc actttgggag gccgaggcgg gcggatcacg
33061 aggtcaggag attgagacca tcctggctaa aacggtgaaa ccccgtctct actaaaaata
33121 caaaaaaaaa attagccggg cgtagtggcg ggcgcctgta gtcccagcta cttgggaggc
33181 tgaggcagga gaatggcgtg aacccgggag gcggagcttg cagtgagccg agatcccgcc
33241 actgcactcc agcctgggca acagagcgag actccgtctc aaaaaaagaa aaaaaaaaaa
33301 aagaaaacca gccgggccaa catggcgaaa tcccatctct actactacta aaaaaaaaaa
33361 aaaaaaaaaa aaaaaaaaaa aaaaaatagc tgggcatggt gatgtgcgcc tgtagtccca
33421 gctactcagg aggctgaggc atgagaatct cttgaacctg ggaggcagag gttgcagtga
33481 gccgaggtca tgccaccgca ctcgggcctg gatgacagag tgagactctg cctccaaaaa
33541 aagaaagaaa gaaagaaaga gagaaagaga aaaagaaaag aaaagaaaga gagagagaga
33601 gaaagaaaga aagaaaagaa aagaaagaga gagaaagaaa gaaagaaaag aaagaaaaag
33661 aaaggaagga aggaaggata gaaagaaaga aagagaaaga aagtaagtta ctgtgggaag

33721 ggcaatatac ttagagctta aaggctctag ctgaggattt cctggcacaa agcctctgag
33781 acagttttgt acggaggagg tttgtaggca ggcacaaggc attaaagggt aaagagagag
33841 gcagcagacc acagcgcagt tgctaagagg catcagccta agccaatggg aagttgaagc
33901 tgggaacccc tgcgggggta agtcttaaat ctaggtgaag aggctgagcc ctgtagaccg
33961 ttcattggct gccttggaga ggggatgtag ccttaggaga ggcagcttat ttctgctgga
34021 gataattctt gggaaggcac tcagccagta cacgtgtgta gagggtggta agaagaatag
34081 ggaagaagag aagtgctgtt ctaagcagcg ggtatatgag cactcagggc tggaggtgag
34141 agaacatact ggaagaagtt cagcatggtg gggatataat gggtggggac ataatgggga
34201 aggggctggt tagatgtgag agaagaagct ggacaggctc acaggggcaa agcagaagga
34261 ccttgtaatt cttgcatggg agcttggatg tgatactaag gacaaaggga aaccacacaa
34321 ctggctaaag caggagagtg acagtaccca ttagaatttt aacaaaatca tatcagcaac
34381 agtgtagaag aggatgtgtt gactcggggc agggaaggag gcaggaaacc ttttgggaag
34441 ttgtagcaat gatgtaagtg agaggcgcag ggtggactgg atcacaattg tttctcattc
34501 tcatctccta taagtcccca atgtggatca agttcacatt catttctcca gtggccccat
34561 caaattgatg ggatttactg agcttactgc tcactatact ttttttccgc acatgtgact
34621 gttaagccaa ctgccttttc tacagtgact tctcttgccc agatgagcac atttaggtta
34681 gtgaatccaa gtctaattta attgagtctt gttagagtca ggtcattgct ctaaattgtt
34741 aaaacaaacg atatacacaa acacacacac acacaaaaga ataaattgag gaggtcccaa
34801 ttctgtattt taaaatactt aatatatccc ttccttcttg aagagagcta caatttcaag
34861 aatttatttt gaaaaaattc tatcagttta ttgataaaaa tggtaaaagg aacaaagctg
34921 agaacagaac atatcatcag actctctagg gacggaaatc aatacataat acttcaagat
34981 atcaccctca gctacttgct gttccaccac cagttctctt gcatccagtt tttgtttctt
35041 ttaacttggc tataaggatg tgagcacaga ctctgccaaa cagcctgctg aagttctgca
35101 aacaccttgt ggattgtgtt tccctgaaca accctgtcca acaatggcct tgggcctgtc
35161 tgaagtattc ttagtgaact tctgctgcca cctagtgatc attgcgaatt tttaaaaagc
35221 ctctttatcc tttaacaagt tactcttgtc ttaccccaaa gcggcatctg cctcactggc
35281 atatgactta cagagctcac attttccctc tccacaaagt tcaaaatgga gacttctcag
35341 atttagcctt ccagcaccca ttctcaaacg tcgtgcatta cagaaaagtt taaactataa
35401 agttgaaccg cacgagattc tcattttgat atgtcattaa agtcagatat tggcaagaac
35461 atatggttta acccaatggc ttggaatgct ggatgggatt cccagaagct ccatacatcg
35521 gcctgcttca ggaccctttt attacaccaa ataaacccaa caaacatcca tcgagtgcct
35581 attttatatc agaacaatga gacctgctcc ttctccttca ggacatttta cttaatgggg
35641 gaaacataca agtaaacaga caaatgtaat cagtgcaaga aagaatcgga cagcaatatg
35701 cacaggataa ggacacttag ctaaacctgc agacattggg aaagatgtca gggaaaaggg
35761 atggcagaag gaaagacaag gccaaaggca tgggactggg aggcactgga atatcagggc
35821 aacagaaagt gctgtcattt ggtataaatg gaacacacag gatatatgaa agcaagaaac
35881 agaagatgag tctgtgaggt tactatgatc cagttcatgg agggccttgt atgtcctggt
35941 gggaaaattg gtttttattg tgggaaggga accttttaat gcattcaaag cagtggaatg

36001 aagtgatttg gtttgcattt gttaatcatg tgaagaagaa taggttggag ggataaaact
36061 tgtggtggtg agatgagtga cagagcgaca gccatctaca aaagaaacac aaggtacaga
36121 ctgaattgtg ggagtggtag tggggatgga aaataaagaa caaatttaga gaggcagaac
36181 tgagtggaca tgatgctcag acttaccaga gcaggtgagt caagaatgat gtcaccattc
36241 cctaaatgag gcaaactggg agagaagcag atttggggag aaaactacta aacatgtacg
36301 tccctccaaa accttcaaga atgctaaagg gacagagtca agttcttcca aagtttctgt
36361 ctcttccatt tattttgcaa tgaatttcaa cttgttaatc agaaatggat ttggagtagt
36421 agctattcaa ttgttttata caccttctag aagaaatatt tgtcagatga gtatgtcaaa
36481 aacttctcag accctcccct ttacttgaat gtatcttcca gcagatgttg gtggaatcaa
36541 agttcattat tactcctata tcttttatac tagtgttatg atctgcagtg aggacatgca
36601 ttgctattgc tacgcctggc ctggaatgtc tgaggtgtat ttctacaaca ccaattattt
36661 tgtgtgtctc tccttttaat ctccaccaaa aaatggagac agctagattc ataaccctta
36721 gacatcctat attttattac taatacttgg ttgtggaagt ttgcattcta aacaagtcat
36781 caacataaaa atctctaaaa atattttatg aaaaatattc agcttatgat attttaaaaa
36841 tcacaaacct aagaaaacac acaaacctgt gtttgtattg tgaaatcaga ggttaatctt
36901 cctcaccctg tgaaaaaatt aggggcataa atctatagga aggagattgg gtgaatccaa
36961 gttacttacg gatgacataa aagatacaat caactttggt gatccaattg ccacactaca
37021 actatctgac caacacgttt ctgtttctct ttagaccact ctcatctcca ggaaccagct
37081 ctctgtgggc cacacatgat gtttgacgaa gatcgttgcg agtgtgtctg taaaacacca
37141 tgtcccaaag atctaatcca gcaccccaaa aactgcagtt gctttgagtg caaagaaagt
37201 ctggagacct gctgccagaa gcacaagcta tttcacccag acacctgcag gtaaatgcct
37261 tgagttgtct tgcctttact catgacattt aacgtagatg ccattttaat ttggtgggat
37321 tccttagtaa gctaattttt ccccttggtg cctgagtccc ttttggataa tagaacagag
37381 gtttagcagc taaggaataa gcaagagtat accgagtcat gattaatcat gaaaataaga
37441 aaatcaatcc tgcctgttat tatgtgtgta gagctttttt tttttagacg gagtctcgct
37501 ctgtctccag gctggagtgc agtggcgtga tctgggctca ctgcaacctc cgtctctctg
37561 gttcaaggga gtctcctgcc tcagcctccc aagtagctgg gattacaggc acgtgccacc
37621 acacccagct aatttttgta tttcagtaga gacggggttt caccatgttg gccaggatag
37681 tcttgatctc ctgacctcat gatccacccg tctcggcctc ccaaagtgct gggattacag
37741 gcatgagcca ccgcgcccac ccggggttta gagctcttaa acaacaatga aattggtaaa
37801 atttaagcag ttattaggat attgcttaat aataatattt taggaagggg aggtttcaga
37861 tcatcaacct gtttagttca atctctcatt gtttatgtct atatatgttt gtttttttct
37921 ttaatggagg gaatatcatt ggatttatat agcaagtaat ttattcatga atagaacact
37981 tggaggttac tgattcactt cagtagattt tgaatccatg tgggataacc atgcaggatt
38041 ttatactgac attttaaata catgtctgga aaacttaatc aaatcagagc agagttattc
38101 ttttgaatcc agtcattgat tctgaattct catctttatt ttcttttgca acagctgtga
38161 ggacagatgc ccctttcata ccagaccatg tgcaagtggc aaaacagcat gtgcaaagca
38221 ttgccgcttt ccaaaggaga aaagggctgc ccaggggccc cacagccgaa agaatccttg

38281 attcagcgtt ccaagttccc catccctgtc atttttaaca gcatgctgct ttgccaagtt
38341 gctgtcactg tttttttccc aggtgttaaa aaaaaaatcc attttacaca gcaccacagt
38401 gaatccagac caaccttcca ttcacaccag ctaaggagtc cctggttcat tgatggatgt
38461 cttctagctg cagatgcctc tgcgcaccaa ggaatggaga ggaggggacc catgtaatcc
38521 ttttgtttag ttttgttttt gttttttggt gaatgagaaa ggtgtgctgg tcatggaatg
38581 gcaggtgtca tatgactgat tactcagagc agatgaggaa aactgtagtc tctgagtcct
38641 ttgctaatcg caactcttgt gaattattct gattcttttt tatgcagaat ttgattcgta
38701 tgatcagtac tgactttctg attactgtcc agcttatagt cttccagttt aatgaactac
38761 catctgatgt ttcatattta agtgtattta aagaaaataa acaccattat tcaagccata
38821 taa

In some embodiments of any of the vectors described herein, the vector includes a sequence encoding an antibody or antigen-binding antibody fragment that can bind specifically to VEGF. In some embodiments, an antibody or antigen-binding antibody fragment can include a human IgG Fc domain (e.g., SEQ ID NO: 113). In some embodiments, the sequence encoding an antibody or antigen-binding fragment can further include a sequence encoding a secretion signal sequence (e.g., SEQ ID NO: 92). In some embodiments of any of the vectors described herein, the vector comprises a sequence of a T2A peptidase (e.g., SEQ ID NO: 99).

In some embodiments, the antibody can be a humanized antibody, a chimeric antibody, or a multivalent antibody. In some embodiments, an antibody or an antigen-binding antibody fragment can be a scFv-Fc, a V_{H}H domain, a V_{NAR} domain, a (scFv)₂, a minibody, or a BiTE. In some embodiments, an antibody or an antigen-binding antibody fragment can be a DVD-Ig, and a dual-affinity re-targeting antibody (DART), a triomab, kih IgG with a common LC, a crossmab, an ortho-Fab IgG, a 2-in-1-IgG, IgG-ScFv, scFv₂-Fc, a bi-nanobody, tanden antibody, a DART-Fc, a scFv-HAS-scFv, DNL-Fab3, DAF (two-in-one or four-in-one), DutaMab, DT-IgG, knobs-in-holes common LC, knobs-in-holes assembly, charge pair antibody, Fab-arm exchange antibody, SEEDbody, Triomab, LUZ-Y, Fcab, kλ-body, orthogonal Fab, DVD-IgG, IgG(H)-scFv, scFv-(H)IgG, IgG(L)-scFv, scFv-(L)-IgG, IgG (L,H)-Fc, IgG(H)-V, V(H)-IgG, IgG(L)-V, V(L)-IgG, KIH IgG-scFab, 2scFv-IgG, IgG-2scFv, scFv4-Ig, Zybody, DVI-IgG, nanobody, nanobody-HSA, a diabody, a TandAb, scDiabody, scDiabody-CH3, Diabody-CH3, Triple Body, miniantibody, minibody, TriBi minibody, scFv-CH3 KIH, Fab-scFv, scFv-CH-CL-scFv, F(ab')₂-scFV₂, scFv-KIH, Fab-scFv-Fc, tetravalent HCAb, scDiabody-Fc, diabody-Fc, tandem scFv-Fc, intrabody, dock and lock bispecific antibody, ImmTAC, HSAbody, scDiabody-HAS, tandem scFv, IgG-IgG, Cov-X-Body, and scFv1-PEG-scFv2.

Additional examples of an antibody or an antigen-binding antibody fragment include an Fv fragment, a Fab fragment, a F(ab')₂ fragment, and a Fab' fragment. Additional examples of an antibody or an antigen-binding antibody fragment include an antigen-binding fragment of an IgG (e.g., an antigen-binding fragment of IgG1, IgG2, IgG3, or IgG4) (e.g., an antigen-binding fragment of a human or humanized IgG, e.g., human or humanized IgG1, IgG2, IgG3, or IgG4); an antigen-binding fragment of an IgA (e.g., an antigen-binding fragment of IgA1 or IgA2) (e.g., an antigen-binding fragment of a human or humanized IgA, e.g., a human or humanized IgA1 or IgA2); an antigen-binding fragment of an IgD (e.g., an antigen-binding fragment of a human or humanized IgD); an antigen-binding fragment of an IgE (e.g., an antigen-binding fragment of a human or humanized IgE); or an antigen-binding fragment of an IgM (e.g., an antigen-binding fragment of a human or humanized IgM).

Any of the antibodies or antigen-binding antibody fragments described herein can bind specifically to VEGF.

A V_{H}H domain is a single monomeric variable antibody domain that can be found in camelids. A V_{NAR} domain is a single monomeric variable antibody domain that can be found in cartilaginous fish. Non-limiting aspects of V_{H}H domains and V_{NAR} domains are described in, e.g., Cromie et al., Curr. Top. Med. Chem. 15:2543-2557, 2016; De Genst et al., Dev. Comp. Immunol. 30:187-198, 2006; De Meyer et al., Trends Biotechnol. 32:263-270, 2014; Kijanka et al., Nanomedicine 10:161-174, 2015; Kovaleva et al., Expert. Opin. Biol. Ther. 14:1527-1539, 2014; Krah et al., Immunopharmacol. Immunotoxicol. 38:21-28, 2016; Mujic-Delic et al., Trends Pharmacol. Sci. 35:247-255, 2014; Muyldermans, J. Biotechnol. 74:277-302, 2001; Muyldermans et al., Trends Biochem. Sci. 26:230-235, 2001; Muyldermans, Ann. Rev. Biochem. 82:775-797, 2013; Rahbarizadeh et al., Immunol. Invest. 40:299-338, 2011; Van Audenhove et al., EBioMedicine 8:40-48, 2016; Van Bockstaele et al., Curr. Opin. Investig. Drugs 10:1212-1224, 2009; Vincke et al., Methods Mol. Biol. 911:15-26, 2012; and Wesolowski et al., Med. Microbiol. Immunol. 198:157-174, 2009.

A "Fv" fragment includes a non-covalently-linked dimer of one heavy chain variable domain and one light chain variable domain.

A "Fab" fragment includes, the constant domain of the light chain and the first constant domain (C_{H1}) of the heavy chain, in addition to the heavy and light chain variable domains of the Fv fragment.

A "F(ab')₂" fragment includes two Fab fragments joined, near the hinge region, by disulfide bonds.

A "dual variable domain immunoglobulin" or "DVD-Ig" refers to multivalent and multispecific binding proteins as described, e.g., in DiGiammarino et al., Methods Mol. Biol. 899:145-156, 2012; Jakob et al., MABs 5:358-363, 2013; and U.S. Patent Nos. 7,612,181; 8,258,268; 8,586,714; 8,716,450; 8,722,855; 8,735,546; and 8,822,645, each of which is incorporated by reference in its entirety.

DARTs are described in, e.g., Garber, Nature Reviews Drug Discovery 13:799-801,2014.

Additional aspects of antibodies and antigen-binding antibody fragments are known in the art.

In some embodiments, any of the antibodies or antigen-binding antibody fragments described herein has a dissociation constant (K_{D}) of less than 1 × 10⁻⁵M (e.g., less than 0.5 × 10⁻⁵ M, less than 1 × 10⁻⁶ M, less than 0.5 × 10⁻⁶ M, less than 1 × 10⁻⁷M, less than 0.5 × 10⁻⁷ M, less than 1 × 10⁻⁸ M, less than 0.5 × 10⁻⁸ M, less than 1 × 10⁻⁹ M, less than 0.5 × 10⁻⁹ M, less than 1 × 10⁻¹⁰ M, less than 0.5 × 10⁻¹⁰ M, less than 1 × 10⁻¹¹ M, less than 0.5 × 10⁻¹¹M, or less than 1 × 10⁻¹² M), e.g., as measured in phosphate buffered saline using surface plasmon resonance (SPR) for a VEGF protein (e.g., any of the VEGF proteins described herein, e.g., one or more of mature human VEGF-A, mature human VEGF-B, mature human VEGF-C, and mature human VEGF-D).

In some embodiments, any of the antibodies or antigen-binding antibody fragments described herein has a K_{D} of about 1 × 10⁻¹² M to about 1 × 10⁻⁵ M, about 0.5 × 10⁻⁵ M, about 1 × 10⁻⁶ M, about 0.5 × 10⁻⁶ M, about 1 × 10⁻⁷M, about 0.5 × 10⁻⁷ M, about 1 × 10⁻⁸ M, about 0.5 × 10⁻⁸ M, about 1 × 10⁻⁹ M, about 0.5 × 10⁻⁹ M, about 1 × 10⁻¹⁰ M, about 0.5 × 10⁻¹⁰ M, about 1 × 10⁻¹¹ M, or about 0.5 × 10⁻¹¹ M (inclusive); about 0.5 × 10⁻¹¹ M to about 1 × 10⁻⁵ M, about 0.5 × 10⁻⁵ M, about 1 × 10⁻⁶ M, about 0.5 × 10⁻⁶ M, about 1 × 10⁻⁷ M, about 0.5 × 10⁻⁷ M, about 1 × 10⁻⁸ M, about 0.5 × 10⁻⁸ M, about 1 × 10⁻⁹ M, about 0.5 × 10⁻⁹ M, about 1 × 10⁻¹⁰ M, about 0.5 × 10⁻¹⁰ M, or about 1 × 10⁻¹¹ M (inclusive); about 1 × 10⁻¹¹ M to about 1 × 10⁻⁵ M, about 0.5 × 10⁻⁵ M, about 1 × 10⁻⁶ M, about 0.5 × 10⁻⁶ M, about 1 × 10⁻⁷M, about 0.5 × 10⁻⁷ M, about 1 × 10⁻⁸ M, about 0.5 × 10⁻⁸M, about 1 × 10⁻⁹ M, about 0.5 × 10⁻⁹ M, about 1 × 10⁻¹⁰ M, or about 0.5 × 10⁻¹⁰ M (inclusive); about 0.5 × 10⁻¹⁰ M to about 1 × 10⁻⁵ M, about 0.5 × 10⁻⁵ M, about 1 × 10⁻⁶ M, about 0.5 × 10⁻⁶ M, about 1 × 10⁻⁷ M, about 0.5 × 10⁻⁷ M, about 1 × 10⁻⁸ M, about 0.5 × 10⁻⁸ M, about 1 × 10⁻⁹ M, about 0.5 × 10⁻⁹ M, or about 1 × 10⁻¹⁰ M (inclusive); about 1 × 10⁻¹⁰ M to about 1 × 10⁻⁵ M, about 0.5 × 10⁻⁵ M, about 1 × 10⁻⁶ M, about 0.5 × 10⁻⁶ M, about 1 × 10⁻⁷M, about 0.5 × 10⁻⁷ M, about 1 × 10⁻⁸ M, about 0.5 × 10⁻⁸ M, about 1 × 10⁻⁹ M, or about 0.5 × 10⁻⁹ M (inclusive); about 0.5 × 10⁻⁹ M to about 1 × 10⁻⁵ M, about 0.5 × 10⁻⁵ M, about 1 × 10⁻⁶ M, about 0.5 × 10⁻⁶ M, about 1 × 10⁻⁷M, about 0.5 × 10⁻⁷ M, about 1 × 10⁻⁸ M, about 0.5 × 10⁻⁸ M, or about 1 × 10⁻⁹ M (inclusive); about 1 × 10⁻⁹ M to about 1 × 10⁻⁵ M, about 0.5 × 10⁻⁵ M, about 1 × 10⁻⁶ M, about 0.5 × 10⁻⁶ M, about 1 × 10⁻⁷M, about 0.5 × 10⁻⁷ M, about 1 × 10⁻⁸ M, or about 0.5 × 10⁻⁸ M (inclusive); about 0.5 × 10⁻⁸ M to about 1 × 10⁻⁵ M, about 0.5 × 10⁻⁵ M, about 1 × 10⁻⁶ M, about 0.5 × 10⁻⁶ M, about 1 × 10⁻⁷ M, about 0.5 × 10⁻⁷ M, or about 1 × 10⁻⁸ M (inclusive); about 1 × 10⁻⁸ M to about 1 × 10⁻⁵ M, about 0.5 × 10⁻⁵ M, about 1 × 10⁻⁶ M, about 0.5 × 10⁻⁶ M, about 1 × 10⁻⁷M, or about 0.5 × 10⁻⁷ M (inclusive); about 0.5 × 10⁻⁷ M to about 1 × 10⁻⁵ M, about 0.5 × 10⁻⁵ M, about 1 × 10⁻⁶ M, about 0.5 × **10⁻⁶** M, or about 1 × 10⁻⁷ M (inclusive); about 1 × 10⁻⁷ M to about 1 × 10⁻⁵ M, about 0.5 × 10⁻⁵ M, about 1 × 10⁻⁶ M, or about 0.5 × 10⁻⁶ M (inclusive); about 0.5 × 10⁻⁶ M to about 1 × 10⁻⁵ M, about 0.5 × 10⁻⁵ M, or about 1 × 10⁻⁶ M (inclusive); about 1 × 10⁻⁶ M to about 1 × 10⁻⁵ M or about 0.5 × 10⁻⁵ M (inclusive); or about 0.5 × 10⁻⁵ M to about 1 × 10⁻⁵M (inclusive), e.g., as measured in phosphate buffered saline using surface plasmon resonance (SPR), for a VEGF protein (e.g., any of the VEGF proteins described herein, e.g., one or more of mature human VEGF-A, mature human VEGF-B, mature human VEGF-C, and mature human VEGF-D).

A variety of different methods known in the art can be used to determine the K_{D} values of any of the antibodies or antigen-binding antibody fragments described herein (e.g., an electrophoretic mobility shift assay, a filter binding assay, surface plasmon resonance, and a biomolecular binding kinetics assay, etc.).

In some embodiments of any of the antibodies and/or antigen-binding antibody fragments described herein, the half-life of the antibody and/or the antigen-binding antibody fragment in a subject (e.g., a human) is decreased about 0.5-fold to about 4-fold (e.g., about 0.5-fold to about 3.5-fold, about 0.5-fold to about 3-fold, about 0.5-fold to about 2.5-fold, about 0.5-fold to about 2-fold, about 0.5-fold to about 1.5-fold, about 0.5-fold to about 1-fold, about 1-fold to about 4-fold, about 1-fold to about 3.5-fold, about 1-fold to about 3-fold, about 1-fold to about 2.5-fold, about 1-fold to about 2-fold, about 1.5-fold to about 4-fold, about 1.5-fold to about 3.5-fold, about 1.5-fold to about 3-fold, about 1.5-fold to about 2.5-fold, about 1.5-fold to about 2-fold, about 2-fold to about 4-fold, about 2-fold to about 3.5-fold, about 2-fold to about 3-fold, about 2-fold to about 2.5-fold, about 2.5-fold to about 4-fold, about 2.5-fold to about 3.5-fold, about 2.5-fold to about 3-fold, about 3-fold to about 4-fold, about 3-fold to about 3.5-fold, or about 3.5-fold to about 4-fold) as compared to the half-life of a control antibody and/or a control antigen-binding antibody fragment (e.g., any of the control antibodies and control antigen-binding antibody fragments described herein) in a similar subject. See, e.g., Leabman et al., MAbs. 5(6): 896-903, 2013. In some embodiments, an antibody or antigen-binding antibody fragment described herein has one or more amino acid substitutions in the Fc region that decrease its half-life in a mammal, and a control antibody lacks at least one (e.g., lacks all) of these one or more amino acid substiutions in the Fc region.

In some examples of any of the antibodies and antigen-binding fragments thereof described herein, the antibody and antigen-binding fragment can bind to a VEGF antigen (e.g., any of the exemplary VEGF proteins described herein, e.g., one or more of mature human VEGF-A, mature human VEGF-B, mature human VEGF-C, and mature human VEGF-D) (e.g., any of the binding affinities described herein).

In some embodiments described herein, an antibody or antigen-binding antibody fragment can decrease an activity of a VEGF (e.g., one or more of any of the exemplary VEGF proteins described herein, e.g., one or more of mature human VEGF-A, mature human VEGF-B, mature human VEGF-C, and mature human VEGF-D). In some embodiments, an antibody or antigen-binding antibody fragment can block a VEGF (e.g., one or more of any of the exemplary VEGF proteins described herein, e.g., one or more of mature human VEGF-A, mature human VEGF-B, mature human VEGF-C, and mature human VEGF-D) from binding to one or more of its receptors (e.g., one or more VEGF receptors) See, e.g., WO 1998/045331, US 9,079,953, US 2015/0147317, US 2016/0289314, Plotkin et al., Otology & Neurotology 33: 1046-1052 (2012); and Ferrara et al. (2005) Biochem Biophys Res Commun 333(2): 328-335. In some embodiments, an antibody or antigen-binding antibody can decrease downstream signaling (e.g., signaling downstream of a VEGF receptor, e.g., one or more of any of the exemplary VEGF receptors described herein, e.g., one or more of human VEGFR-1 (SEQ ID NO: 111), human VEGFR-2 (SEQ ID NO: 112), and human VEGFR-3). In some embodiments, a decrease in an activity of a VEGF can be detected indirectly, e.g., through an increase in hearing (e.g., a 1% to about 400% increase (or any of the subranges of this range described herein) in hearing) or a decrease (e.g., a 1% to 99%, a 1% to 95%, a 1% to 90%, a 1% to 85%, a 1% to 80%, a 1% to 75%, a 1% to 70%, a 1% to 65%, a 1% to 60%, a 1% to 55%, a 1% to 50%, a 1% to 45%, a 1% to 40%, a 1% to 35%, a 1% to 30%, a 1% to 25%, a 1% to 20%, a 1% to 15%, a 1% to 10%, a 1% to 5%, a 5% to 99%, a 5% to 95%, a 5% to 90%, a 5% to 85%, a 5% to 80%, a 5% to 75%, a 5% to 70%, a 5% to 65%, a 5% to 60%, a 5% to 55%, a 5% to 50%, a 5% to 45%, a 5% to 40%, a 5% to 35%, a 5% to 30%, a 5% to 25%, a 5% to 20%, a 5% to 15%, a 5% to 10%, a 10% to 99%, a 10% to 95%, a 10% to 90%, a 10% to 85%, a 10% to 80%, a 10% to 75%, a 10% to 70%, a 10% to 65%, a 10% to 60%, a 10% to 55%, a 10% to 50%, a 10% to 45%, a 10% to 40%, a 10% to 35%, a 10% to 30%, a 10% to 25%, a 10% to 20%, a 10% to 15%, a 15% to 99%, a 15% to 95%, a 15% to 90%, a 15% to 85%, a 15% to 80%, a 15% to 75%, a 15% to 70%, a 15% to 65%, a 15% to 60%, a 15% to 55%, a 15% to 50%, a 15% to 45%, a 15% to 40%, a 15% to 35%, a 15% to 30%, a 15% to 25%, a 15% to 20%, a 20% to 99%, a 20% to 95%, a 20% to 90%, a 20% to 85%, a 20% to 80%, a 20% to 75%, a 20% to 70%, a 20% to 65%, a 20% to 60%, a 20% to 55%, a 20% to 50%, a 20% to 45%, a 20% to 40%, a 20% to 35%, a 20% to 30%, a 20% to 25%, a 25% to 99%, a 25% to 95%, a 25% to 90%, a 25% to 85%, a 25% to 80%, a 25% to 75%, a 25% to 70%, a 25% to 65%, a 25% to 60%, a 25% to 55%, a 25% to 50%, a 25% to 45%, a 25% to 40%, a 25% to 35%, a 25% to 30%, a 30% to 99%, a 30% to 95%, a 30% to 90%, a 30% to 85%, a 30% to 80%, a 30% to 75%, a 30% to 70%, a 30% to 65%, a 30% to 60%, a 30% to 55%, a 30% to 50%, a 30% to 45%, a 30% to 40%, a 30% to 35%, a 35% to 99%, a 35% to 95%, a 35% to 90%, a 35% to 85%, a 35% to 80%, a 35% to 75%, a 35% to 70%, a 35% to 65%, a 35% to 60%, a 35% to 55%, a 35% to 50%, a 35% to 45%, a 35% to 40%, a 40% to 99%, a 40% to 95%, a 40% to 90%, a 40% to 85%, a 40% to 80%, a 40% to 75%, a 40% to 70%, a 40% to 65%, a 40% to 60%, a 40% to 55%, a 40% to 50%, a 40% to 45%, a 45% to 99%, a 45% to 95%, a 45% to 90%, a 45% to 85%, a 45% to 80%, a 45% to 75%, a 45% to 70%, a 45% to 65%, a 45% to 60%, a 45% to 55%, a 45% to 50%, a 50% to 99%, a 50% to 95%, a 50% to 90%, a 50% to 85%, a 50% to 80%, a 50% to 75%, a 50% to 70%, a 50% to 65%, a 50% to 60%, a 50% to 55%, a 55% to 99%, a 55% to 95%, a 55% to 90%, a 55% to 85%, a 55% to 80%, a 55% to 75%, a 55% to 70%, a 55% to 65%, a 55% to 60%, a 60% to 99%, a 60% to 95%, a 60% to 90%, a 60% to 85%, a 60% to 80%, a 60% to 75%, a 60% to 70%, a 60% to 65%, a 65% to 99%, a 65% to 95%, a 65% to 90%, a 65% to 85%, a 65% to 80%, a 65% to 75%, a 65% to 70%, a 70% to 99%, a 70% to 95%, a 70% to 90%, a 70% to 85%, a 70% to 80%, a 70% to 75%, a 75% to 99%, a 75% to 95%, a 75% to 90%, a 75% to 85%, a 75% to 80%, a 80% to 99%, a 80% to 95%, a 80% to 90%, a 80% to 85%, a 85% to 99%, a 85% to 95%, a 85% to 90%, a 90% to 99%, a 90% to 95%, or a 95% to 99% decrease) in the size or the severity of one or more symptoms of an acoustic neuroma, vestibular schwannoma, or neurofibromatosis type II in a mammal as compared to the level of hearing or size of an acoustic neuroma, vestibular schwannoma, or neurofibromatosis type II in the mammal, respectively, before administration of any of the AAV vectors described herein. In some embodiments, a decrease in a VEGF activity can be detected in an in vitro assay.

In some embodiments, the antibody that specifically binds to a VEGF is bevacizumab (Avastatin^{®}) (SEQ ID NO: 103) or an antigen-binding fragment thereof. Bevacizumab (full size antibody ~150 kDa) inhibits all isoforms of VEGF-A. Bevacizumab received Food and Drug administration (FDA) approval in 2004 for colon cancer for intravenous (IV) dose of 4.0-7.5 mg/kg at 2-3 weeks (plasmatic half life 21 days), for intravetrial (IVT) dose 1.25 mg in 0.05 mL (half-life 5.6 days). Bevacizumab has a K_{D} for VEGF 165 (VEGF-A) of 58 pM. *See, e.g.,* WO 2017/050825. The leader sequence of bevacizumab is SEQ ID NO: 104, the heavy chain variable domain of bevacizumab is SEQ ID NO: 105, the heavy chain constant region of bevacizumab is SEQ ID NO: 106, the light chain variable domain of devacizumab is SEQ ID NO: 107, and the light chain constant region of bevacizumab is SEQ ID NO: 108. An exemplary cDNA sequence encoding bevacizumab is SEQ ID NO: 109.

In some embodiments, the antibody that specifically binds to a VEGF is ranibizumab (Lucentis^{®}) (SEQ ID NO: 95), or an antigen-binding fragment thereof. Ranibizumab (~50 kDa) inhibits all isoforms of VEGF-A. Ranibizumab received FDA approval in 2006 for ocular use for intravenous (IV) dose of 4.0-7.5 mg/kg at 2-3 weeks (plasma half life of 0.5 days), for intravetrial (IVT) dose 0.5 mg in 0.05 mL (half-life of 3.2 days). Ranibizumab has a K_{D} for VEGF 165 (VEGF-A) of 46 pM. *See, e.g.,* WO 2014/178078. The leader sequence of ranibizumab has a sequence of SEQ ID NO: 96, the heavy chain variable domain of ranibizumab has a sequence of SEQ ID NO: 97, the heavy chain constant region of ranibizumab has a sequence of SEQ ID NO: 98, the light chain variable domain of ranibizumab has a sequence of SE QID NO: 100, the light chain contant region of ranibizumab has a sequence of SEQ ID NO: 101. An exemplary cDNA sequence encoding ranibizumab is SEQ ID NO: 102.

In some embodiments, the antibody that specifically binds to VEGF is sevacizumab (APX003/ SIM-BD0801), or an antigen-binding fragment thereof.

In some embodiments, the antibody or antigen-binding fragment thereof that specifically binds to VEGF is aflibercept (SEQ ID NO: 110). An exemplary cDNA sequence encoding aflibercept is SEQ ID NO: 114.

In some embodiments of the antibodies that specifically bind to VEGF and antigen-binding fragments thereof described herein, the antibody or antigen-binding fragments thereof includes a variable light chain domain that is or includes a sequence that is at least 80% identical (e.g., at least 82%, at least 84%, at least 86%, at least 88%, at least 90%, at least 92%, at least 94%, at least 96%, at least 98%, or at least 99%) identical to the variable light chain domain of bevacizumab, and/or includes a variable heavy chain domain that is or includes a sequence that is at least 80% identical (e.g., at least 82%, at least 84%, at least 86%, at least 88%, at least 90%, at least 92%, at least 94%, at least 96%, at least 98%, or at least 99%) identical to the variable heavy chain domain of bevacizumab.

In some embodiments of the antibodies that specifically bind to VEGF and antigen-binding fragments thereof described herein, the antibody or antigen-binding fragments thereof includes a variable light chain domain that is or includes the variable light chain domain of bevacizumab, and/or a variable heavy chain domain that is or includes the variable heavy chain domain of bevacizumab. In some embodiments of the antibodies that specifically bind to VEGF and antigen-binding fragments thereof described herein, the antibody or antigen-binding fragments thereof includes a variable light chain domain that is or includes the sequence of variable light chain domain of bevacizumab, except that it includes one, two, three, four, five, six, seven, eight, nine, ten, eleven, twelve, thirteen, fourteen, or fifteen amino acid substitutions, and/or includes a variable heavy chain domain that is or includes the sequence of variable heavy chain of bevacizumab, except that it includes one, two, three, four, five, six, seven, eight, nine, ten, eleven, twelve, thirteen, fourteen, or fifteen amino acid substitutions. In some embodiments the first antigen-binding domain includes the three CDRs in the light chain variable domain of bevacizumab, and/or the three CDRs in the heavy chain variable domain of bevacizumab.

In some embodiments of the antibodies that specifically bind to VEGF and antigen-binding fragments thereof described herein, the antibody or antigen-binding fragments thereof includes a variable light chain domain that is or includes a sequence that is at least 80% identical (e.g., at least 82%, at least 84%, at least 86%, at least 88%, at least 90%, at least 92%, at least 94%, at least 96%, at least 98%, or at least 99%) identical to the variable light chain domain of ranibizumab, and/or includes a variable heavy chain domain that is or includes a sequence that is at least 80% identical (e.g., at least 82%, at least 84%, at least 86%, at least 88%, at least 90%, at least 92%, at least 94%, at least 96%, at least 98%, or at least 99%) identical to the variable heavy chain domain of ranibizumab.

In some embodiments of the antibodies that specifically bind to VEGF and antigen-binding fragments thereof described herein, the antibody or antigen-binding fragments thereof includes a variable light chain domain that is or includes the variable light chain domain of ranibizumab, and/or a variable heavy chain domain that is or includes the variable heavy chain domain of ranibizumab. In some embodiments of the antibodies that specifically bind to VEGF and antigen-binding fragments thereof described herein, the antibody or antigen-binding fragments thereof includes a variable light chain domain that is or includes the sequence of variable light chain domain of ranibizumab, except that it includes one, two, three, four, five, six, seven, eight, nine, ten, eleven, twelve, thirteen, fourteen, or fifteen amino acid substitutions, and/or includes a variable heavy chain domain that is or includes the sequence of variable heavy chain of ranibizumab, except that it includes one, two, three, four, five, six, seven, eight, nine, ten, eleven, twelve, thirteen, fourteen, or fifteen amino acid substitutions. In some embodiments the first antigen-binding domain includes the three CDRs in the light chain variable domain of ranibizumab, and/or the three CDRs in the heavy chain variable domain of ranibizumab.

### Vectors

The compositions provided herein include at least two (e.g., two, three, four, five, or six) nucleic acid vectors, where: each of the at least two different vectors includes a coding sequence that encodes a different portion of a target protein (e.g., a supporting cell target protein or a hair cell target protein), each of the encoded portions being at least 30 amino acids (e.g., about 30 amino acids to about 800 amino acids, about 30 amino acids to about 750 amino acids, about 30 amino acids to about 700 amino acids, about 30 amino acids to about 650 amino acids, about 30 amino acids to about 600 amino acids, about 30 amino acids to about 550 amino acids, about 30 amino acids to about 500 amino acids, about 30 amino acids to about 450 amino acids, about 30 amino acids to about 400 amino acids, about 30 amino acids to about 350 amino acids, about 30 amino acids to about 300 amino acids, about 30 amino acids to about 250 amino acids, about 30 amino acids to about 200 amino acids, about 30 amino acids to about 150 amino acids, about 30 amino acids to about 100 amino acids, about 30 amino acids to about 50 amino acids, about 50 amino acids to about 800 amino acids, about 50 amino acids to about 750 amino acids, about 50 amino acids to about 700 amino acids, about 50 amino acids to about 650 amino acids, about 50 amino acids to about 600 amino acids, about 50 amino acids to about 550 amino acids, about 50 amino acids to about 500 amino acids, about 50 amino acids to about 450 amino acids, about 50 amino acids to about 400 amino acids, about 50 amino acids to about 350 amino acids, about 50 amino acids to about 300 amino acids, about 50 amino acids to about 250 amino acids, about 50 amino acids to about 200 amino acids, about 50 amino acids to about 150 amino acids, about 50 amino acids to about 100 amino acids, about 100 amino acids to about 1600 amino acids, about 100 amino acids to about 800 amino acids, about 100 amino acids to about 750 amino acids, about 100 amino acids to about 700 amino acids, about 100 amino acids to about 650 amino acids, about 100 amino acids to about 600 amino acids, about 100 amino acids to about 550 amino acids, about 100 amino acids to about 500 amino acids, about 100 amino acids to about 450 amino acids, about 100 amino acids to about 400 amino acids, about 100 amino acids to about 350 amino acids, about 100 amino acids to about 300 amino acids, about 100 amino acids to about 250 amino acids, about 100 amino acids to about 200 amino acids, about 100 amino acids to about 150 amino acids, about 150 amino acids to about 800 amino acids, about 150 amino acids to about 750 amino acids, about 150 amino acids to about 700 amino acids, about 150 amino acids to about 650 amino acids, about 150 amino acids to about 600 amino acids, about 150 amino acids to about 550 amino acids, about 150 amino acids to about 500 amino acids, about 150 amino acids to about 450 amino acids, about 150 amino acids to about 400 amino acids, about 150 amino acids to about 350 amino acids, about 150 amino acids to about 300 amino acids, about 150 amino acids to about 250 amino acids, about 150 amino acids to about 200 amino acids, about 200 amino acids to about 800 amino acids, about 200 amino acids to about 750 amino acids, about 200 amino acids to about 700 amino acids, about 200 amino acids to about 650 amino acids, about 200 amino acids to about 600 amino acids, about 200 amino acids to about 550 amino acids, about 200 amino acids to about 500 amino acids, about 200 amino acids to about 450 amino acids, about 200 amino acids to about 400 amino acids, about 200 amino acids to about 350 amino acids, about 200 amino acids to about 300 amino acids, about 200 amino acids to about 250 amino acids, about 250 amino acids to about 800 amino acids, about 250 amino acids to about 750 amino acids, about 250 amino acids to about 700 amino acids, about 250 amino acids to about 650 amino acids, about 250 amino acids to about 600 amino acids, about 250 amino acids to about 550 amino acids, about 250 amino acids to about 500 amino acids, about 250 amino acids to about 450 amino acids, about 250 amino acids to about 400 amino acids, about 250 amino acids to about 350 amino acids, about 250 amino acids to about 300 amino acids, about 300 amino acids to about 800 amino acids, about 300 amino acids to about 750 amino acids, about 300 amino acids to about 700 amino acids, about 300 amino acids to about 650 amino acids, about 300 amino acids to about 600 amino acids, about 300 amino acids to about 550 amino acids, about 300 amino acids to about 500 amino acids, about 300 amino acids to about 450 amino acids, about 300 amino acids to about 400 amino acids, about 300 amino acids to about 350 amino acids, about 350 amino acids to about 800 amino acids, about 350 amino acids to about 750 amino acids, about 350 amino acids to about 700 amino acids, about 350 amino acids to about 650 amino acids, about 350 amino acids to about 600 amino acids, about 350 amino acids to about 550 amino acids, about 350 amino acids to about 500 amino acids, about 350 amino acids to about 450 amino acids, about 350 amino acids to about 400 amino acids, about 400 amino acids to about 800 amino acids, about 400 amino acids to about 750 amino acids, about 400 amino acids to about 700 amino acids, about 400 amino acids to about 650 amino acids, about 400 amino acids to about 600 amino acids, about 400 amino acids to about 550 amino acids, about 400 amino acids to about 500 amino acids, about 400 amino acids to about 450 amino acids, about 450 amino acids to about 800 amino acids, about 450 amino acids to about 750 amino acids, about 450 amino acids to about 700 amino acids, about 450 amino acids to about 650 amino acids, about 450 amino acids to about 600 amino acids, about 450 amino acids to about 550 amino acids, about 450 amino acids to about 500 amino acids, about 500 amino acids to about 800 amino acids, about 500 amino acids to about 750 amino acids, about 500 amino acids to about 700 amino acids, about 500 amino acids to about 650 amino acids, about 500 amino acids to about 600 amino acids, about 500 amino acids to about 550 amino acids, about 550 amino acids to about 800 amino acids, about 550 amino acids to about 750 amino acids, about 550 amino acids to about 700 amino acids, about 550 amino acids to about 650 amino acids, about 550 amino acids to about 600 amino acids, about 600 amino acids to about 800 amino acids, about 600 amino acids to about 750 amino acids, about 600 amino acids to about 700 amino acids, about 600 amino acids to about 650 amino acids, about 650 amino acids to about 800 amino acids, about 650 amino acids to about 750 amino acids, about 650 amino acids to about 700 amino acids, about 700 amino acids to about 800 amino acids, about 700 amino acids to about 750 amino acids, or about 750 amino acids to about 800 amino acids), where the amino acid sequence of each of the encoded portions may optionally partially overlap with the amino acid sequence of a different one of the encoded portions; no single vector of the at least two different vectors encodes the active target protein (e.g., an active supporting cell target protein (e.g., a full-length supporting cell target protein (e.g., a full-length wildtype supporting cell target protein)) or an active hair cell target protein (e.g., a full-length hair cell target protein (e.g., a full-length wildtype hair cell target protein)); and, when introduced into a primate cell, the at least two different vectors undergo homologous recombination with each other, where the recombined nucleic acid encodes an active target protein (e.g., an active supporting cell target protein (e.g., a full-length supporting cell target protein) or an active hair cell target protein (e.g., a full-length hair cell target protein). In some embodiments, one of the nucleic acid vectors can include a coding sequence that encodes a portion of a target protein (e.g., a supporting cell target protein or a hair cell target protein), where the encoded portion is, e.g., about 30 amino acids to about 800 amino acids, about 30 amino acids to about 750 amino acids, about 30 amino acids to about 700 amino acids, about 30 amino acids to about 650 amino acids, about 30 amino acids to about 600 amino acids, about 30 amino acids to about 550 amino acids, about 30 amino acids to about 500 amino acids, about 30 amino acids to about 450 amino acids, about 30 amino acids to about 400 amino acids, about 30 amino acids to about 350 amino acids, about 30 amino acids to about 300 amino acids, about 30 amino acids to about 250 amino acids, about 30 amino acids to about 200 amino acids, about 30 amino acids to about 150 amino acids, about 30 amino acids to about 100 amino acids, about 30 amino acids to about 50 amino acids, about 50 amino acids to about 800 amino acids, about 50 amino acids to about 750 amino acids, about 50 amino acids to about 700 amino acids, about 50 amino acids to about 650 amino acids, about 50 amino acids to about 600 amino acids, about 50 amino acids to about 550 amino acids, about 50 amino acids to about 500 amino acids, about 50 amino acids to about 450 amino acids, about 50 amino acids to about 400 amino acids, about 50 amino acids to about 350 amino acids, about 50 amino acids to about 300 amino acids, about 50 amino acids to about 250 amino acids, about 50 amino acids to about 200 amino acids, about 50 amino acids to about 150 amino acids, about 50 amino acids to about 100 amino acids, about 100 amino acids to about 1600 amino acids, about 100 amino acids to about 800 amino acids, about 100 amino acids to about 750 amino acids, about 100 amino acids to about 700 amino acids, about 100 amino acids to about 650 amino acids, about 100 amino acids to about 600 amino acids, about 100 amino acids to about 550 amino acids, about 100 amino acids to about 500 amino acids, about 100 amino acids to about 450 amino acids, about 100 amino acids to about 400 amino acids, about 100 amino acids to about 350 amino acids, about 100 amino acids to about 300 amino acids, about 100 amino acids to about 250 amino acids, about 100 amino acids to about 200 amino acids, about 100 amino acids to about 150 amino acids, about 150 amino acids to about 800 amino acids, about 150 amino acids to about 750 amino acids, about 150 amino acids to about 700 amino acids, about 150 amino acids to about 650 amino acids, about 150 amino acids to about 600 amino acids, about 150 amino acids to about 550 amino acids, about 150 amino acids to about 500 amino acids, about 150 amino acids to about 450 amino acids, about 150 amino acids to about 400 amino acids, about 150 amino acids to about 350 amino acids, about 150 amino acids to about 300 amino acids, about 150 amino acids to about 250 amino acids, about 150 amino acids to about 200 amino acids, about 200 amino acids to about 800 amino acids, about 200 amino acids to about 750 amino acids, about 200 amino acids to about 700 amino acids, about 200 amino acids to about 650 amino acids, about 200 amino acids to about 600 amino acids, about 200 amino acids to about 550 amino acids, about 200 amino acids to about 500 amino acids, about 200 amino acids to about 450 amino acids, about 200 amino acids to about 400 amino acids, about 200 amino acids to about 350 amino acids, about 200 amino acids to about 300 amino acids, about 200 amino acids to about 250 amino acids, about 250 amino acids to about 800 amino acids, about 250 amino acids to about 750 amino acids, about 250 amino acids to about 700 amino acids, about 250 amino acids to about 650 amino acids, about 250 amino acids to about 600 amino acids, about 250 amino acids to about 550 amino acids, about 250 amino acids to about 500 amino acids, about 250 amino acids to about 450 amino acids, about 250 amino acids to about 400 amino acids, about 250 amino acids to about 350 amino acids, about 250 amino acids to about 300 amino acids, about 300 amino acids to about 800 amino acids, about 300 amino acids to about 750 amino acids, about 300 amino acids to about 700 amino acids, about 300 amino acids to about 650 amino acids, about 300 amino acids to about 600 amino acids, about 300 amino acids to about 550 amino acids, about 300 amino acids to about 500 amino acids, about 300 amino acids to about 450 amino acids, about 300 amino acids to about 400 amino acids, about 300 amino acids to about 350 amino acids, about 350 amino acids to about 800 amino acids, about 350 amino acids to about 750 amino acids, about 350 amino acids to about 700 amino acids, about 350 amino acids to about 650 amino acids, about 350 amino acids to about 600 amino acids, about 350 amino acids to about 550 amino acids, about 350 amino acids to about 500 amino acids, about 350 amino acids to about 450 amino acids, about 350 amino acids to about 400 amino acids, about 400 amino acids to about 800 amino acids, about 400 amino acids to about 750 amino acids, about 400 amino acids to about 700 amino acids, about 400 amino acids to about 650 amino acids, about 400 amino acids to about 600 amino acids, about 400 amino acids to about 550 amino acids, about 400 amino acids to about 500 amino acids, about 400 amino acids to about 450 amino acids, about 450 amino acids to about 800 amino acids, about 450 amino acids to about 750 amino acids, about 450 amino acids to about 700 amino acids, about 450 amino acids to about 650 amino acids, about 450 amino acids to about 600 amino acids, about 450 amino acids to about 550 amino acids, about 450 amino acids to about 500 amino acids, about 500 amino acids to about 800 amino acids, about 500 amino acids to about 750 amino acids, about 500 amino acids to about 700 amino acids, about 500 amino acids to about 650 amino acids, about 500 amino acids to about 600 amino acids, about 500 amino acids to about 550 amino acids, about 550 amino acids to about 800 amino acids, about 550 amino acids to about 750 amino acids, about 550 amino acids to about 700 amino acids, about 550 amino acids to about 650 amino acids, about 550 amino acids to about 600 amino acids, about 600 amino acids to about 800 amino acids, about 600 amino acids to about 750 amino acids, about 600 amino acids to about 700 amino acids, about 600 amino acids to about 650 amino acids, about 650 amino acids to about 800 amino acids, about 650 amino acids to about 750 amino acids, about 650 amino acids to about 700 amino acids, about 700 amino acids to about 800 amino acids, about 700 amino acids to about 750 amino acids, or about 750 amino acids to about 800 amino acids in length.

In some embodiments of these compositions, at least one of the coding sequences includes a nucleotide sequence spanning two neighboring exons of target genomic DNA (e.g., supporting cell target genomic DNA or hair cell target genomic DNA), and lacks the intronic sequence that naturally occurs between the two neighboring exons.

In some embodiments, the amino acid sequence of none of the encoded portions overlaps even in part with the amino acid sequence of a different one of the encoded portions. In some embodiments, the amino acid sequence of one or more of the encoded portions partially overlaps with the amino acid sequence of a different one of the encoded portions. In some embodiments, the amino acid sequence of each of the encoded portions partially overlaps with the amino acid sequence of a different one of the encoded portions.

In some embodiments, the overlapping amino acid sequence is between about 30 amino acid residues to about 800 amino acids (e.g., or any of the subranges of this range described herein) in length.

In some examples, the vectors include two different vectors, each of which includes a different segment of an intron, where the intron includes the nucleotide sequence of an intron that is present in a target genomic DNA (e.g., a supporting cell target genomic DNA (e.g., SLC26A4 genomic DNA or a GJB2 genomic DNA) (e.g., any of the exemplary introns in SEQ ID NO: 3 described herein) or a hair cell target genomic DNA (e.g., an OTOF genomic DNA, a TRIOBP genomic DNA, a PJVK genomic DNA, a NDP genomic DNA, a CLRN1 genomic DNA, a TMC1 genomic DNA, a VEGF genomic DNA, a HSPA1 A genomic DNA, or a STRC genomic DNA (e.g., any of the exemplary introns in SEQ ID NO: 19, SEQ ID NO: 22, SEQ ID NO: 33, SEQ ID NO: 37, SEQ ID NO: 47, SEQ ID NO: 50, SEQ ID NO: 61, SEQ ID NO: 71 or SEQ ID NO: 80 described herein), and where the two different segments overlap in sequence by at least 100 nucleotides (e.g., about 100 nucleotides to about 3,000 nucleotides, about 100 nucleotides to about 2,500 nucleotides, about 100 nucleotides to about 2,000 nucleotides, about 100 nucleotides to about 1,500 nucleotides, about 100 nucleotides to about 1,000 nucleotides, about 100 nucleotides to about 800 nucleotides, about 100 nucleotides to about 600 nucleotides, about 100 nucleotides to about 400 nucleotides, about 100 nucleotides to about 200 nucleotides, about 200 nucleotides to about 3,000 nucleotides, about 200 nucleotides to about 2,500 nucleotides, about 200 nucleotides to about 2,000 nucleotides, about 200 nucleotides to about 1,500 nucleotides, about 200 nucleotides to about 1,000 nucleotides, about 200 nucleotides to about 800 nucleotides, about 200 nucleotides to about 600 nucleotides, about 200 nucleotides to about 400 nucleotides, about 400 nucleotides to about 3,000 nucleotides, about 400 nucleotides to about 2,500 nucleotides, about 400 nucleotides to about 2,000 nucleotides, about 400 nucleotides to about 1,500 nucleotides, about 400 nucleotides to about 1,000 nucleotides, about 400 nucleotides to about 800 nucleotides, about 400 nucleotides to about 600 nucleotides, about 600 nucleotides to about 3,000 nucleotides, about 600 nucleotides to about 2,500 nucleotides, about 600 nucleotides to about 2,000 nucleotides, about 600 nucleotides to about 1,500 nucleotides, about 600 nucleotides to about 1,000 nucleotides, about 600 nucleotides to about 800 nucleotides, about 800 nucleotides to about 3,000 nucleotides, about 800 nucleotides to about 2,500 nucleotides, about 800 nucleotides to about 2,000 nucleotides, about 800 nucleotides to about 1,500 nucleotides, about 800 nucleotides to about 1,000 nucleotides, about 1,000 nucleotides to about 3,000 nucleotides, about 1,000 nucleotides to about 2,500 nucleotides, about 1,000 nucleotides to about 2,000 nucleotides, about 1,000 nucleotides to about 1,500 nucleotides, about 1,500 nucleotides to about 3,000 nucleotides, about 1,500 nucleotides to about 2,500 nucleotides, about 1,500 nucleotides to about 2,000 nucleotides, about 2,000 nucleotides to about 3,000 nucleotides, about 2,000 nucleotides to about 2,500 nucleotides, or about 2,500 nucleotides to about 3,000 nucleotides), in length.

The overlapping nucleotide sequence in any two of the different vectors can include part or all of one or more exons of a target gene (e.g., a supporting cell target gene (e.g., a SLC26A4 gene or a GJB2 gene) (e.g., any one or more of the exemplary exons in SEQ ID NO: 3 described herein) or a hair cell target gene (e.g., an OTOF gene, a TRIOBP gene, a PJVK gene, a NDP gene, a CLRN1 gene, a TMC1 gene, or a STRC gene) (e.g., any one or more of the exemplary exons in SEQ ID NO: 19, SEQ ID NO: 22, SEQ ID NO: 33, SEQ ID NO: 37, SEQ ID NO: 47, SEQ ID NO: 50, SEQ ID NO: 63, SEQ ID NO: 71 or SEQ ID NO: 80 described herein).

In some embodiments, the number of different vectors in the composition is two, three, four, or five. In compositions where the number of different vectors in the composition is two, the first of the two different vectors can include a coding sequence that encodes an N-terminal portion of the target protein (e.g., an N-terminal portion of the supporting cell target protein or an N-terminal portion of the hair cell target protein). In some examples, the N-terminal portion of the target gene (e.g., the N-terminal portion of the supporting cell target gene or the N-terminal portion of the hair cell target gene) is between about 30 amino acids to about 800 amino acids (e.g., between about 30 amino acids to about 790 amino acids, between about 30 amino acids to about 780 amino acids, between about 30 amino acids to about 770 amino acids, between about 30 amino acids to about 760 amino acids, between about 30 amino acids to about 750 amino acids, between about 30 amino acids to about 740 amino acids, between about 30 amino acids to about 730 amino acids, between about 30 amino acids to about 720 amino acids, between about 30 amino acids to about 710 amino acids, between about 30 amino acids to about 700 amino acids, between about 30 amino acids to about 690 amino acids, between about 30 amino acids to about 680 amino acids, between 30 amino acids to about 670 amino acids, between about 30 amino acids to about 660 amino acids, between about 30 amino acids to about 650 amino acids, between about 30 amino acids to about 640 amino acids, between about 30 amino acids to about 630 amino acids, between about 30 amino acids to about 620 amino acids, between about 30 amino acids to about 610 amino acids, between about 30 amino acids to about 600 amino acids, between about 30 amino acids to about 590 amino acids, between about 30 amino acids to about 580 amino acids, between about 30 amino acids to about 570 amino acids, between about 30 amino acids to about 560 amino acids, between about 30 amino acids to about 550 amino acids, between about 30 amino acids to about 540 amino acids, between about 30 amino acids to about 530 amino acids, between about 30 amino acids to about 520 amino acids, between about 30 amino acids to about 510 amino acids, between about 30 amino acids to about 500 amino acids, between about 30 amino acids to about 490 amino acids, between about 30 amino acids to about 480 amino acids, between about 30 amino acids to about 470 amino acids, between about 30 amino acids to about 460 amino acids, between about 30 amino acids to about 450 amino acids, between about 30 amino acids to about 440 amino acids, between about 30 amino acids to about 430 amino acids, between about 30 amino acids to about 420 amino acids, between about 30 amino acids to about 410 amino acids, between about 30 amino acids to about 400 amino acids, between about 30 amino acids to about 390 amino acids, between about 30 amino acids to about 380 amino acids, between about 30 amino acids to about 370 amino acids, between about 30 amino acids to about 360 amino acids, between about 30 amino acids to about 350 amino acids, between about 30 amino acids to about 340 amino acids, between about 30 amino acids to about 330 amino acids, between about 30 amino acids to about 320 amino acids, between about 30 amino acids to about 310 amino acids, between about 30 amino acids to about 300 amino acids, between about 30 amino acids to about 290 amino acids, between about 30 amino acids to about 280 amino acids, between about 30 amino acids to about 270 amino acids, between about 30 amino acids to about 260 amino acids, between about 30 amino acids to about 250 amino acids, between about 30 amino acids to about 240 amino acids, between about 30 amino acids to about 230 amino acids, between about 30 amino acids to about 220 amino acids, between about 30 amino acids to about 210 amino acids, between about 30 amino acids to about 200 amino acids, between about 30 amino acids to about 190 amino acids, between about 30 amino acids to about 180 amino acids, between about 30 amino acids to about 170 amino acids, between about 30 amino acids to about 160 amino acids, between about 30 amino acids to about 150 amino acids, between about 30 amino acids to about 140 amino acids, between about 30 amino acids to about 130 amino acids, between about 30 amino acids to about 120 amino acids, between about 30 amino acids to about 110 amino acids, between about 30 amino acids to about 100 amino acids, between about 30 amino acids to about 90 amino acids, between about 30 amino acids to about 80 amino acids, between about 30 amino acids to about 70 amino acids, between about 30 amino acids to about 60 amino acids, between about 30 amino acids to about 50 amino acids, between about 30 amino acids to about 40 amino acids, between about 50 amino acids to about 800 amino acids, between about 50 amino acids to about 790 amino acids, between about 50 amino acids to about 780 amino acids, between about 50 amino acids to about 770 amino acids, between about 50 amino acids to about 760 amino acids, between about 50 amino acids to about 750 amino acids, between about 50 amino acids to about 740 amino acids, between about 50 amino acids to about 730 amino acids, between about 50 amino acids to about 720 amino acids, between about 50 amino acids to about 710 amino acids, between about 50 amino acids to about 700 amino acids, between about 50 amino acids to about 690 amino acids, between about 50 amino acids to about 680 amino acids, between 30 amino acids to about 670 amino acids, between about 50 amino acids to about 660 amino acids, between about 50 amino acids to about 650 amino acids, between about 50 amino acids to about 640 amino acids, between about 30 amino acids to about 630 amino acids, between about 50 amino acids to about 620 amino acids, between about 50 amino acids to about 610 amino acids, between about 50 amino acids to about 600 amino acids, between about 50 amino acids to about 590 amino acids, between about 50 amino acids to about 580 amino acids, between about 50 amino acids to about 570 amino acids, between about 50 amino acids to about 560 amino acids, between about 50 amino acids to about 550 amino acids, between about 50 amino acids to about 540 amino acids, between about 50 amino acids to about 530 amino acids, between about 50 amino acids to about 520 amino acids, between about 50 amino acids to about 510 amino acids, between about 50 amino acids to about 500 amino acids, between about 50 amino acids to about 490 amino acids, between about 50 amino acids to about 480 amino acids, between about 50 amino acids to about 470 amino acids, between about 50 amino acids to about 460 amino acids, between about 50 amino acids to about 450 amino acids, between about 50 amino acids to about 440 amino acids, between about 50 amino acids to about 430 amino acids, between about 50 amino acids to about 420 amino acids, between about 50 amino acids to about 410 amino acids, between about 50 amino acids to about 400 amino acids, between about 50 amino acids to about 390 amino acids, between about 50 amino acids to about 380 amino acids, between about 50 amino acids to about 370 amino acids, between about 50 amino acids to about 360 amino acids, between about 50 amino acids to about 350 amino acids, between about 50 amino acids to about 340 amino acids, between about 50 amino acids to about 330 amino acids, between about 50 amino acids to about 320 amino acids, between about 50 amino acids to about 310 amino acids, between about 50 amino acids to about 300 amino acids, between about 50 amino acids to about 290 amino acids, between about 50 amino acids to about 280 amino acids, between about 50 amino acids to about 270 amino acids, between about 50 amino acids to about 260 amino acids, between about 50 amino acids to about 250 amino acids, between about 50 amino acids to about 240 amino acids, between about 50 amino acids to about 230 amino acids, between about 50 amino acids to about 220 amino acids, between about 50 amino acids to about 210 amino acids, between about 50 amino acids to about 200 amino acids, between about 50 amino acids to about 190 amino acids, between about 50 amino acids to about 180 amino acids, between about 50 amino acids to about 170 amino acids, between about 50 amino acids to about 160 amino acids, between about 50 amino acids to about 150 amino acids, between about 50 amino acids to about 140 amino acids, between about 50 amino acids to about 130 amino acids, between about 50 amino acids to about 120 amino acids, between about 50 amino acids to about 110 amino acids, between about 50 amino acids to about 100 amino acids, between about 50 amino acids to about 90 amino acids, between about 50 amino acids to about 80 amino acids, between about 50 amino acids to about 70 amino acids, between about 50 amino acids to about 60 amino acids, between about 80 amino acids to about 800 amino acids, between about 80 amino acids to about 790 amino acids, between about 80 amino acids to about 780 amino acids, between about 80 amino acids to about 770 amino acids, between about 80 amino acids to about 760 amino acids, between about 80 amino acids to about 750 amino acids, between about 80 amino acids to about 740 amino acids, between about 80 amino acids to about 730 amino acids, between about 80 amino acids to about 720 amino acids, between about 80 amino acids to about 710 amino acids, between about 80 amino acids to about 700 amino acids, between about 80 amino acids to about 690 amino acids, between about 80 amino acids to about 680 amino acids, between 30 amino acids to about 670 amino acids, between about 80 amino acids to about 660 amino acids, between about 80 amino acids to about 650 amino acids, between about 80 amino acids to about 640 amino acids, between about 30 amino acids to about 630 amino acids, between about 80 amino acids to about 620 amino acids, between about 80 amino acids to about 610 amino acids, between about 80 amino acids to about 600 amino acids, between about 80 amino acids to about 590 amino acids, between about 80 amino acids to about 580 amino acids, between about 80 amino acids to about 570 amino acids, between about 80 amino acids to about 560 amino acids, between about 80 amino acids to about 550 amino acids, between about 80 amino acids to about 540 amino acids, between about 80 amino acids to about 530 amino acids, between about 80 amino acids to about 520 amino acids, between about 80 amino acids to about 510 amino acids, between about 80 amino acids to about 500 amino acids, between about 80 amino acids to about 490 amino acids, between about 80 amino acids to about 480 amino acids, between about 80 amino acids to about 470 amino acids, between about 80 amino acids to about 460 amino acids, between about 80 amino acids to about 450 amino acids, between about 80 amino acids to about 440 amino acids, between about 80 amino acids to about 430 amino acids, between about 80 amino acids to about 420 amino acids, between about 80 amino acids to about 410 amino acids, between about 80 amino acids to about 400 amino acids, between about 80 amino acids to about 390 amino acids, between about 80 amino acids to about 380 amino acids, between about 80 amino acids to about 370 amino acids, between about 80 amino acids to about 360 amino acids, between about 80 amino acids to about 350 amino acids, between about 80 amino acids to about 340 amino acids, between about 80 amino acids to about 330 amino acids, between about 80 amino acids to about 320 amino acids, between about 80 amino acids to about 310 amino acids, between about 80 amino acids to about 300 amino acids, between about 80 amino acids to about 290 amino acids, between about 80 amino acids to about 280 amino acids, between about 80 amino acids to about 270 amino acids, between about 80 amino acids to about 260 amino acids, between about 80 amino acids to about 250 amino acids, between about 80 amino acids to about 240 amino acids, between about 80 amino acids to about 230 amino acids, between about 80 amino acids to about 220 amino acids, between about 80 amino acids to about 210 amino acids, between about 80 amino acids to about 200 amino acids, between about 80 amino acids to about 190 amino acids, between about 80 amino acids to about 180 amino acids, between about 80 amino acids to about 170 amino acids, between about 80 amino acids to about 160 amino acids, between about 80 amino acids to about 150 amino acids, between about 80 amino acids to about 140 amino acids, between about 80 amino acids to about 130 amino acids, between about 80 amino acids to about 120 amino acids, between about 80 amino acids to about 110 amino acids, between about 80 amino acids to about 100 amino acids, between about 100 amino acids to about 800 amino acids, between about 100 amino acids to about 790 amino acids, between about 100 amino acids to about 780 amino acids, between about 100 amino acids to about 770 amino acids, between about 100 amino acids to about 760 amino acids, between about 100 amino acids to about 750 amino acids, between about 100 amino acids to about 740 amino acids, between about 100 amino acids to about 730 amino acids, between about 100 amino acids to about 720 amino acids, between about 100 amino acids to about 710 amino acids, between about 100 amino acids to about 700 amino acids, between about 100 amino acids to about 690 amino acids, between about 100 amino acids to about 680 amino acids, between 30 amino acids to about 670 amino acids, between about 100 amino acids to about 660 amino acids, between about 100 amino acids to about 650 amino acids, between about 100 amino acids to about 640 amino acids, between about 30 amino acids to about 630 amino acids, between about 100 amino acids to about 620 amino acids, between about 100 amino acids to about 610 amino acids, between about 100 amino acids to about 600 amino acids, between about 100 amino acids to about 590 amino acids, between about 100 amino acids to about 580 amino acids, between about 100 amino acids to about 570 amino acids, between about 100 amino acids to about 560 amino acids, between about 100 amino acids to about 550 amino acids, between about 100 amino acids to about 540 amino acids, between about 100 amino acids to about 530 amino acids, between about 100 amino acids to about 520 amino acids, between about 100 amino acids to about 510 amino acids, between about 100 amino acids to about 500 amino acids, between about 100 amino acids to about 490 amino acids, between about 100 amino acids to about 480 amino acids, between about 100 amino acids to about 470 amino acids, between about 100 amino acids to about 460 amino acids, between about 100 amino acids to about 450 amino acids, between about 100 amino acids to about 440 amino acids, between about 100 amino acids to about 430 amino acids, between about 100 amino acids to about 420 amino acids, between about 100 amino acids to about 410 amino acids, between about 100 amino acids to about 400 amino acids, between about 100 amino acids to about 390 amino acids, between about 100 amino acids to about 380 amino acids, between about 100 amino acids to about 370 amino acids, between about 100 amino acids to about 360 amino acids, between about 100 amino acids to about 350 amino acids, between about 100 amino acids to about 340 amino acids, between about 100 amino acids to about 330 amino acids, between about 100 amino acids to about 320 amino acids, between about 100 amino acids to about 310 amino acids, between about 100 amino acids to about 300 amino acids, between about 100 amino acids to about 290 amino acids, between about 100 amino acids to about 280 amino acids, between about 100 amino acids to about 270 amino acids, between about 100 amino acids to about 260 amino acids, between about 100 amino acids to about 250 amino acids, between about 100 amino acids to about 240 amino acids, between about 100 amino acids to about 230 amino acids, between about 100 amino acids to about 220 amino acids, between about 100 amino acids to about 210 amino acids, between about 100 amino acids to about 200 amino acids, between about 100 amino acids to about 190 amino acids, between about 100 amino acids to about 180 amino acids, between about 100 amino acids to about 170 amino acids, between about 100 amino acids to about 160 amino acids, between about 100 amino acids to about 150 amino acids, between about 100 amino acids to about 140 amino acids, between about 100 amino acids to about 130 amino acids, between about 100 amino acids to about 120 amino acids, between about 100 amino acids to about 110 amino acids, between about 120 amino acids to about 800 amino acids, between about 120 amino acids to about 790 amino acids, between about 120 amino acids to about 780 amino acids, between about 120 amino acids to about 770 amino acids, between about 120 amino acids to about 760 amino acids, between about 120 amino acids to about 750 amino acids, between about 120 amino acids to about 740 amino acids, between about 120 amino acids to about 730 amino acids, between about 120 amino acids to about 720 amino acids, between about 120 amino acids to about 710 amino acids, between about 120 amino acids to about 700 amino acids, between about 120 amino acids to about 690 amino acids, between about 120 amino acids to about 680 amino acids, between 30 amino acids to about 670 amino acids, between about 120 amino acids to about 660 amino acids, between about 120 amino acids to about 650 amino acids, between about 120 amino acids to about 640 amino acids, between about 30 amino acids to about 630 amino acids, between about 120 amino acids to about 620 amino acids, between about 120 amino acids to about 610 amino acids, between about 120 amino acids to about 600 amino acids, between about 120 amino acids to about 590 amino acids, between about 120 amino acids to about 580 amino acids, between about 120 amino acids to about 570 amino acids, between about 120 amino acids to about 560 amino acids, between about 120 amino acids to about 550 amino acids, between about 120 amino acids to about 540 amino acids, between about 120 amino acids to about 530 amino acids, between about 120 amino acids to about 520 amino acids, between about 120 amino acids to about 510 amino acids, between about 120 amino acids to about 500 amino acids, between about 120 amino acids to about 490 amino acids, between about 120 amino acids to about 480 amino acids, between about 120 amino acids to about 470 amino acids, between about 120 amino acids to about 460 amino acids, between about 120 amino acids to about 450 amino acids, between about 120 amino acids to about 440 amino acids, between about 120 amino acids to about 430 amino acids, between about 120 amino acids to about 420 amino acids, between about 120 amino acids to about 410 amino acids, between about 120 amino acids to about 400 amino acids, between about 120 amino acids to about 390 amino acids, between about 120 amino acids to about 380 amino acids, between about 120 amino acids to about 370 amino acids, between about 120 amino acids to about 360 amino acids, between about 120 amino acids to about 350 amino acids, between about 120 amino acids to about 340 amino acids, between about 120 amino acids to about 330 amino acids, between about 120 amino acids to about 320 amino acids, between about 120 amino acids to about 310 amino acids, between about 120 amino acids to about 300 amino acids, between about 120 amino acids to about 290 amino acids, between about 120 amino acids to about 280 amino acids, between about 120 amino acids to about 270 amino acids, between about 120 amino acids to about 260 amino acids, between about 120 amino acids to about 250 amino acids, between about 120 amino acids to about 240 amino acids, between about 120 amino acids to about 230 amino acids, between about 120 amino acids to about 220 amino acids, between about 120 amino acids to about 210 amino acids, between about 120 amino acids to about 200 amino acids, between about 120 amino acids to about 190 amino acids, between about 120 amino acids to about 180 amino acids, between about 120 amino acids to about 170 amino acids, between about 120 amino acids to about 160 amino acids, between about 120 amino acids to about 150 amino acids, between about 120 amino acids to about 140 amino acids, between about 120 amino acids to about 130 amino acids, between about 150 amino acids to about 800 amino acids, between about 150 amino acids to about 790 amino acids, between about 150 amino acids to about 780 amino acids, between about 150 amino acids to about 770 amino acids, between about 150 amino acids to about 760 amino acids, between about 150 amino acids to about 750 amino acids, between about 150 amino acids to about 740 amino acids, between about 150 amino acids to about 730 amino acids, between about 150 amino acids to about 720 amino acids, between about 150 amino acids to about 710 amino acids, between about 150 amino acids to about 700 amino acids, between about 150 amino acids to about 690 amino acids, between about 150 amino acids to about 680 amino acids, between 30 amino acids to about 670 amino acids, between about 150 amino acids to about 660 amino acids, between about 150 amino acids to about 650 amino acids, between about 150 amino acids to about 640 amino acids, between about 30 amino acids to about 630 amino acids, between about 150 amino acids to about 620 amino acids, between about 150 amino acids to about 610 amino acids, between about 150 amino acids to about 600 amino acids, between about 150 amino acids to about 590 amino acids, between about 150 amino acids to about 580 amino acids, between about 150 amino acids to about 570 amino acids, between about 150 amino acids to about 560 amino acids, between about 150 amino acids to about 550 amino acids, between about 150 amino acids to about 540 amino acids, between about 150 amino acids to about 530 amino acids, between about 150 amino acids to about 520 amino acids, between about 150 amino acids to about 510 amino acids, between about 150 amino acids to about 500 amino acids, between about 150 amino acids to about 490 amino acids, between about 150 amino acids to about 480 amino acids, between about 150 amino acids to about 470 amino acids, between about 150 amino acids to about 460 amino acids, between about 150 amino acids to about 450 amino acids, between about 150 amino acids to about 440 amino acids, between about 150 amino acids to about 430 amino acids, between about 150 amino acids to about 420 amino acids, between about 150 amino acids to about 410 amino acids, between about 150 amino acids to about 400 amino acids, between about 150 amino acids to about 390 amino acids, between about 150 amino acids to about 380 amino acids, between about 150 amino acids to about 370 amino acids, between about 150 amino acids to about 360 amino acids, between about 150 amino acids to about 350 amino acids, between about 150 amino acids to about 340 amino acids, between about 150 amino acids to about 330 amino acids, between about 150 amino acids to about 320 amino acids, between about 150 amino acids to about 310 amino acids, between about 150 amino acids to about 300 amino acids, between about 150 amino acids to about 290 amino acids, between about 150 amino acids to about 280 amino acids, between about 150 amino acids to about 270 amino acids, between about 150 amino acids to about 260 amino acids, between about 150 amino acids to about 250 amino acids, between about 150 amino acids to about 240 amino acids, between about 150 amino acids to about 230 amino acids, between about 150 amino acids to about 220 amino acids, between about 150 amino acids to about 210 amino acids, between about 150 amino acids to about 200 amino acids, between about 150 amino acids to about 190 amino acids, between about 150 amino acids to about 180 amino acids, between about 150 amino acids to about 170 amino acids, between about 150 amino acids to about 160 amino acids, between about 180 amino acids to about 800 amino acids, between about 180 amino acids to about 790 amino acids, between about 180 amino acids to about 780 amino acids, between about 180 amino acids to about 770 amino acids, between about 180 amino acids to about 760 amino acids, between about 180 amino acids to about 750 amino acids, between about 180 amino acids to about 740 amino acids, between about 180 amino acids to about 730 amino acids, between about 180 amino acids to about 720 amino acids, between about 180 amino acids to about 710 amino acids, between about 180 amino acids to about 700 amino acids, between about 180 amino acids to about 690 amino acids, between about 180 amino acids to about 680 amino acids, between 30 amino acids to about 670 amino acids, between about 180 amino acids to about 660 amino acids, between about 180 amino acids to about 650 amino acids, between about 180 amino acids to about 640 amino acids, between about 30 amino acids to about 630 amino acids, between about 180 amino acids to about 620 amino acids, between about 180 amino acids to about 610 amino acids, between about 180 amino acids to about 600 amino acids, between about 180 amino acids to about 590 amino acids, between about 180 amino acids to about 580 amino acids, between about 180 amino acids to about 570 amino acids, between about 180 amino acids to about 560 amino acids, between about 180 amino acids to about 550 amino acids, between about 180 amino acids to about 540 amino acids, between about 180 amino acids to about 530 amino acids, between about 180 amino acids to about 520 amino acids, between about 180 amino acids to about 510 amino acids, between about 180 amino acids to about 500 amino acids, between about 180 amino acids to about 490 amino acids, between about 180 amino acids to about 480 amino acids, between about 180 amino acids to about 470 amino acids, between about 180 amino acids to about 460 amino acids, between about 180 amino acids to about 450 amino acids, between about 180 amino acids to about 440 amino acids, between about 180 amino acids to about 430 amino acids, between about 180 amino acids to about 420 amino acids, between about 180 amino acids to about 410 amino acids, between about 180 amino acids to about 400 amino acids, between about 180 amino acids to about 390 amino acids, between about 180 amino acids to about 380 amino acids, between about 180 amino acids to about 370 amino acids, between about 180 amino acids to about 360 amino acids, between about 180 amino acids to about 350 amino acids, between about 180 amino acids to about 340 amino acids, between about 180 amino acids to about 330 amino acids, between about 180 amino acids to about 320 amino acids, between about 180 amino acids to about 310 amino acids, between about 180 amino acids to about 300 amino acids, between about 180 amino acids to about 290 amino acids, between about 180 amino acids to about 280 amino acids, between about 180 amino acids to about 270 amino acids, between about 180 amino acids to about 260 amino acids, between about 180 amino acids to about 250 amino acids, between about 180 amino acids to about 240 amino acids, between about 180 amino acids to about 230 amino acids, between about 180 amino acids to about 220 amino acids, between about 180 amino acids to about 210 amino acids, between about 180 amino acids to about 200 amino acids, between about 180 amino acids to about 190 amino acids, between about 200 amino acids to about 800 amino acids, between about 200 amino acids to about 790 amino acids, between about 200 amino acids to about 780 amino acids, between about 200 amino acids to about 770 amino acids, between about 200 amino acids to about 760 amino acids, between about 200 amino acids to about 750 amino acids, between about 200 amino acids to about 740 amino acids, between about 200 amino acids to about 730 amino acids, between about 200 amino acids to about 720 amino acids, between about 200 amino acids to about 710 amino acids, between about 200 amino acids to about 700 amino acids, between about 200 amino acids to about 690 amino acids, between about 200 amino acids to about 680 amino acids, between 30 amino acids to about 670 amino acids, between about 200 amino acids to about 660 amino acids, between about 200 amino acids to about 650 amino acids, between about 200 amino acids to about 640 amino acids, between about 30 amino acids to about 630 amino acids, between about 200 amino acids to about 620 amino acids, between about 200 amino acids to about 610 amino acids, between about 200 amino acids to about 600 amino acids, between about 200 amino acids to about 590 amino acids, between about 200 amino acids to about 580 amino acids, between about 200 amino acids to about 570 amino acids, between about 200 amino acids to about 560 amino acids, between about 200 amino acids to about 550 amino acids, between about 200 amino acids to about 540 amino acids, between about 200 amino acids to about 530 amino acids, between about 200 amino acids to about 520 amino acids, between about 200 amino acids to about 510 amino acids, between about 200 amino acids to about 500 amino acids, between about 200 amino acids to about 490 amino acids, between about 200 amino acids to about 480 amino acids, between about 200 amino acids to about 470 amino acids, between about 200 amino acids to about 460 amino acids, between about 200 amino acids to about 450 amino acids, between about 200 amino acids to about 440 amino acids, between about 200 amino acids to about 430 amino acids, between about 200 amino acids to about 420 amino acids, between about 200 amino acids to about 410 amino acids, between about 200 amino acids to about 400 amino acids, between about 200 amino acids to about 390 amino acids, between about 200 amino acids to about 380 amino acids, between about 200 amino acids to about 370 amino acids, between about 200 amino acids to about 360 amino acids, between about 200 amino acids to about 350 amino acids, between about 200 amino acids to about 340 amino acids, between about 200 amino acids to about 330 amino acids, between about 200 amino acids to about 320 amino acids, between about 200 amino acids to about 310 amino acids, between about 200 amino acids to about 300 amino acids, between about 200 amino acids to about 290 amino acids, between about 200 amino acids to about 280 amino acids, between about 200 amino acids to about 270 amino acids, between about 200 amino acids to about 260 amino acids, between about 200 amino acids to about 250 amino acids, between about 200 amino acids to about 240 amino acids, between about 200 amino acids to about 230 amino acids, between about 200 amino acids to about 220 amino acids, between about 200 amino acids to about 210 amino acids, between about 250 amino acids to about 800 amino acids, between about 250 amino acids to about 790 amino acids, between about 250 amino acids to about 780 amino acids, between about 250 amino acids to about 770 amino acids, between about 250 amino acids to about 760 amino acids, between about 250 amino acids to about 750 amino acids, between about 250 amino acids to about 740 amino acids, between about 250 amino acids to about 730 amino acids, between about 250 amino acids to about 720 amino acids, between about 250 amino acids to about 710 amino acids, between about 250 amino acids to about 700 amino acids, between about 250 amino acids to about 690 amino acids, between about 250 amino acids to about 680 amino acids, between 30 amino acids to about 670 amino acids, between about 250 amino acids to about 660 amino acids, between about 250 amino acids to about 650 amino acids, between about 250 amino acids to about 640 amino acids, between about 30 amino acids to about 630 amino acids, between about 250 amino acids to about 620 amino acids, between about 250 amino acids to about 610 amino acids, between about 250 amino acids to about 600 amino acids, between about 250 amino acids to about 590 amino acids, between about 250 amino acids to about 580 amino acids, between about 250 amino acids to about 570 amino acids, between about 250 amino acids to about 560 amino acids, between about 250 amino acids to about 550 amino acids, between about 250 amino acids to about 540 amino acids, between about 250 amino acids to about 530 amino acids, between about 250 amino acids to about 520 amino acids, between about 250 amino acids to about 510 amino acids, between about 250 amino acids to about 500 amino acids, between about 250 amino acids to about 490 amino acids, between about 250 amino acids to about 480 amino acids, between about 250 amino acids to about 470 amino acids, between about 250 amino acids to about 460 amino acids, between about 250 amino acids to about 450 amino acids, between about 250 amino acids to about 440 amino acids, between about 250 amino acids to about 430 amino acids, between about 250 amino acids to about 420 amino acids, between about 250 amino acids to about 410 amino acids, between about 250 amino acids to about 400 amino acids, between about 250 amino acids to about 390 amino acids, between about 250 amino acids to about 380 amino acids, between about 250 amino acids to about 370 amino acids, between about 250 amino acids to about 360 amino acids, between about 250 amino acids to about 350 amino acids, between about 250 amino acids to about 340 amino acids, between about 250 amino acids to about 330 amino acids, between about 250 amino acids to about 320 amino acids, between about 250 amino acids to about 310 amino acids, between about 250 amino acids to about 300 amino acids, between about 250 amino acids to about 290 amino acids, between about 250 amino acids to about 280 amino acids, between about 250 amino acids to about 270 amino acids, between about 250 amino acids to about 260 amino acids, between about 280 amino acids to about 800 amino acids, between about 280 amino acids to about 790 amino acids, between about 280 amino acids to about 780 amino acids, between about 280 amino acids to about 770 amino acids, between about 280 amino acids to about 760 amino acids, between about 280 amino acids to about 750 amino acids, between about 280 amino acids to about 740 amino acids, between about 280 amino acids to about 730 amino acids, between about 280 amino acids to about 720 amino acids, between about 280 amino acids to about 710 amino acids, between about 280 amino acids to about 700 amino acids, between about 280 amino acids to about 690 amino acids, between about 280 amino acids to about 680 amino acids, between 30 amino acids to about 670 amino acids, between about 280 amino acids to about 660 amino acids, between about 280 amino acids to about 650 amino acids, between about 280 amino acids to about 640 amino acids, between about 30 amino acids to about 630 amino acids, between about 280 amino acids to about 620 amino acids, between about 280 amino acids to about 610 amino acids, between about 280 amino acids to about 600 amino acids, between about 280 amino acids to about 590 amino acids, between about 280 amino acids to about 580 amino acids, between about 280 amino acids to about 570 amino acids, between about 280 amino acids to about 560 amino acids, between about 280 amino acids to about 550 amino acids, between about 280 amino acids to about 540 amino acids, between about 280 amino acids to about 530 amino acids, between about 280 amino acids to about 520 amino acids, between about 280 amino acids to about 510 amino acids, between about 280 amino acids to about 500 amino acids, between about 280 amino acids to about 490 amino acids, between about 280 amino acids to about 480 amino acids, between about 280 amino acids to about 470 amino acids, between about 280 amino acids to about 460 amino acids, between about 280 amino acids to about 450 amino acids, between about 280 amino acids to about 440 amino acids, between about 280 amino acids to about 430 amino acids, between about 280 amino acids to about 420 amino acids, between about 280 amino acids to about 410 amino acids, between about 280 amino acids to about 400 amino acids, between about 280 amino acids to about 390 amino acids, between about 280 amino acids to about 380 amino acids, between about 280 amino acids to about 370 amino acids, between about 280 amino acids to about 360 amino acids, between about 280 amino acids to about 350 amino acids, between about 280 amino acids to about 340 amino acids, between about 280 amino acids to about 330 amino acids, between about 280 amino acids to about 320 amino acids, between about 280 amino acids to about 310 amino acids, between about 280 amino acids to about 300 amino acids, between about 300 amino acids to about 800 amino acids, between about 300 amino acids to about 790 amino acids, between about 300 amino acids to about 780 amino acids, between about 300 amino acids to about 770 amino acids, between about 300 amino acids to about 760 amino acids, between about 300 amino acids to about 750 amino acids, between about 300 amino acids to about 740 amino acids, between about 300 amino acids to about 730 amino acids, between about 300 amino acids to about 720 amino acids, between about 300 amino acids to about 710 amino acids, between about 300 amino acids to about 700 amino acids, between about 300 amino acids to about 690 amino acids, between about 300 amino acids to about 680 amino acids, between 30 amino acids to about 670 amino acids, between about 300 amino acids to about 660 amino acids, between about 300 amino acids to about 650 amino acids, between about 300 amino acids to about 640 amino acids, between about 30 amino acids to about 630 amino acids, between about 300 amino acids to about 620 amino acids, between about 300 amino acids to about 610 amino acids, between about 300 amino acids to about 600 amino acids, between about 300 amino acids to about 590 amino acids, between about 300 amino acids to about 580 amino acids, between about 300 amino acids to about 570 amino acids, between about 300 amino acids to about 560 amino acids, between about 300 amino acids to about 550 amino acids, between about 300 amino acids to about 540 amino acids, between about 300 amino acids to about 530 amino acids, between about 300 amino acids to about 520 amino acids, between about 300 amino acids to about 510 amino acids, between about 300 amino acids to about 500 amino acids, between about 300 amino acids to about 490 amino acids, between about 300 amino acids to about 480 amino acids, between about 300 amino acids to about 470 amino acids, between about 300 amino acids to about 460 amino acids, between about 300 amino acids to about 450 amino acids, between about 300 amino acids to about 440 amino acids, between about 300 amino acids to about 430 amino acids, between about 300 amino acids to about 420 amino acids, between about 300 amino acids to about 410 amino acids, between about 300 amino acids to about 400 amino acids, between about 300 amino acids to about 390 amino acids, between about 300 amino acids to about 380 amino acids, between about 300 amino acids to about 370 amino acids, between about 300 amino acids to about 360 amino acids, between about 300 amino acids to about 350 amino acids, between about 300 amino acids to about 340 amino acids, between about 300 amino acids to about 330 amino acids, between about 300 amino acids to about 320 amino acids, between about 300 amino acids to about 310 amino acids, between about 350 amino acids to about 800 amino acids, between about 350 amino acids to about 790 amino acids, between about 350 amino acids to about 780 amino acids, between about 350 amino acids to about 770 amino acids, between about 350 amino acids to about 760 amino acids, between about 350 amino acids to about 750 amino acids, between about 350 amino acids to about 740 amino acids, between about 350 amino acids to about 730 amino acids, between about 350 amino acids to about 720 amino acids, between about 350 amino acids to about 710 amino acids, between about 350 amino acids to about 700 amino acids, between about 350 amino acids to about 690 amino acids, between about 350 amino acids to about 680 amino acids, between 30 amino acids to about 670 amino acids, between about 350 amino acids to about 660 amino acids, between about 350 amino acids to about 650 amino acids, between about 350 amino acids to about 640 amino acids, between about 30 amino acids to about 630 amino acids, between about 350 amino acids to about 620 amino acids, between about 350 amino acids to about 610 amino acids, between about 350 amino acids to about 600 amino acids, between about 350 amino acids to about 590 amino acids, between about 350 amino acids to about 580 amino acids, between about 350 amino acids to about 570 amino acids, between about 350 amino acids to about 560 amino acids, between about 350 amino acids to about 550 amino acids, between about 350 amino acids to about 540 amino acids, between about 350 amino acids to about 530 amino acids, between about 350 amino acids to about 520 amino acids, between about 350 amino acids to about 510 amino acids, between about 350 amino acids to about 500 amino acids, between about 350 amino acids to about 490 amino acids, between about 350 amino acids to about 480 amino acids, between about 350 amino acids to about 470 amino acids, between about 350 amino acids to about 460 amino acids, between about 350 amino acids to about 450 amino acids, between about 350 amino acids to about 440 amino acids, between about 350 amino acids to about 430 amino acids, between about 350 amino acids to about 420 amino acids, between about 350 amino acids to about 410 amino acids, between about 350 amino acids to about 400 amino acids, between about 350 amino acids to about 390 amino acids, between about 350 amino acids to about 380 amino acids, between about 350 amino acids to about 370 amino acids, between about 350 amino acids to about 360 amino acids, between about 380 amino acids to about 800 amino acids, between about 380 amino acids to about 790 amino acids, between about 380 amino acids to about 780 amino acids, between about 380 amino acids to about 770 amino acids, between about 380 amino acids to about 760 amino acids, between about 380 amino acids to about 750 amino acids, between about 380 amino acids to about 740 amino acids, between about 380 amino acids to about 730 amino acids, between about 380 amino acids to about 720 amino acids, between about 380 amino acids to about 710 amino acids, between about 380 amino acids to about 700 amino acids, between about 380 amino acids to about 690 amino acids, between about 380 amino acids to about 680 amino acids, between 30 amino acids to about 670 amino acids, between about 380 amino acids to about 660 amino acids, between about 380 amino acids to about 650 amino acids, between about 380 amino acids to about 640 amino acids, between about 30 amino acids to about 630 amino acids, between about 380 amino acids to about 620 amino acids, between about 380 amino acids to about 610 amino acids, between about 380 amino acids to about 600 amino acids, between about 380 amino acids to about 590 amino acids, between about 380 amino acids to about 580 amino acids, between about 380 amino acids to about 570 amino acids, between about 380 amino acids to about 560 amino acids, between about 380 amino acids to about 550 amino acids, between about 380 amino acids to about 540 amino acids, between about 380 amino acids to about 530 amino acids, between about 380 amino acids to about 520 amino acids, between about 380 amino acids to about 510 amino acids, between about 380 amino acids to about 500 amino acids, between about 380 amino acids to about 490 amino acids, between about 380 amino acids to about 480 amino acids, between about 380 amino acids to about 470 amino acids, between about 380 amino acids to about 460 amino acids, between about 380 amino acids to about 450 amino acids, between about 380 amino acids to about 440 amino acids, between about 380 amino acids to about 430 amino acids, between about 380 amino acids to about 420 amino acids, between about 380 amino acids to about 410 amino acids, between about 380 amino acids to about 400 amino acids, between about 380 amino acids to about 390 amino acids, between about 400 amino acids to about 800 amino acids, between about 400 amino acids to about 790 amino acids, between about 400 amino acids to about 780 amino acids, between about 400 amino acids to about 770 amino acids, between about 400 amino acids to about 760 amino acids, between about 400 amino acids to about 750 amino acids, between about 400 amino acids to about 740 amino acids, between about 400 amino acids to about 730 amino acids, between about 400 amino acids to about 720 amino acids, between about 400 amino acids to about 710 amino acids, between about 400 amino acids to about 700 amino acids, between about 400 amino acids to about 690 amino acids, between about 400 amino acids to about 680 amino acids, between 30 amino acids to about 670 amino acids, between about 400 amino acids to about 660 amino acids, between about 400 amino acids to about 650 amino acids, between about 400 amino acids to about 640 amino acids, between about 30 amino acids to about 630 amino acids, between about 400 amino acids to about 620 amino acids, between about 400 amino acids to about 610 amino acids, between about 400 amino acids to about 600 amino acids, between about 400 amino acids to about 590 amino acids, between about 400 amino acids to about 580 amino acids, between about 400 amino acids to about 570 amino acids, between about 400 amino acids to about 560 amino acids, between about 400 amino acids to about 550 amino acids, between about 400 amino acids to about 540 amino acids, between about 400 amino acids to about 530 amino acids, between about 400 amino acids to about 520 amino acids, between about 400 amino acids to about 510 amino acids, between about 400 amino acids to about 500 amino acids, between about 400 amino acids to about 490 amino acids, between about 400 amino acids to about 480 amino acids, between about 400 amino acids to about 470 amino acids, between about 400 amino acids to about 460 amino acids, between about 400 amino acids to about 450 amino acids, between about 400 amino acids to about 440 amino acids, between about 400 amino acids to about 430 amino acids, between about 400 amino acids to about 420 amino acids, between about 400 amino acids to about 410 amino acids, between about 450 amino acids to about 800 amino acids, between about 450 amino acids to about 790 amino acids, between about 450 amino acids to about 780 amino acids, between about 450 amino acids to about 770 amino acids, between about 450 amino acids to about 760 amino acids, between about 450 amino acids to about 750 amino acids, between about 450 amino acids to about 740 amino acids, between about 450 amino acids to about 730 amino acids, between about 450 amino acids to about 720 amino acids, between about 450 amino acids to about 710 amino acids, between about 450 amino acids to about 700 amino acids, between about 450 amino acids to about 690 amino acids, between about 450 amino acids to about 680 amino acids, between 30 amino acids to about 670 amino acids, between about 450 amino acids to about 660 amino acids, between about 450 amino acids to about 650 amino acids, between about 450 amino acids to about 640 amino acids, between about 30 amino acids to about 630 amino acids, between about 450 amino acids to about 620 amino acids, between about 450 amino acids to about 610 amino acids, between about 450 amino acids to about 600 amino acids, between about 450 amino acids to about 590 amino acids, between about 450 amino acids to about 580 amino acids, between about 450 amino acids to about 570 amino acids, between about 450 amino acids to about 560 amino acids, between about 450 amino acids to about 550 amino acids, between about 450 amino acids to about 540 amino acids, between about 450 amino acids to about 530 amino acids, between about 450 amino acids to about 520 amino acids, between about 450 amino acids to about 510 amino acids, between about 450 amino acids to about 500 amino acids, between about 450 amino acids to about 490 amino acids, between about 450 amino acids to about 480 amino acids, between about 450 amino acids to about 470 amino acids, between about 450 amino acids to about 460 amino acids, between about 480 amino acids to about 800 amino acids, between about 480 amino acids to about 790 amino acids, between about 480 amino acids to about 780 amino acids, between about 480 amino acids to about 770 amino acids, between about 480 amino acids to about 760 amino acids, between about 480 amino acids to about 750 amino acids, between about 480 amino acids to about 740 amino acids, between about 480 amino acids to about 730 amino acids, between about 480 amino acids to about 720 amino acids, between about 480 amino acids to about 710 amino acids, between about 480 amino acids to about 700 amino acids, between about 480 amino acids to about 690 amino acids, between about 480 amino acids to about 680 amino acids, between 30 amino acids to about 670 amino acids, between about 480 amino acids to about 660 amino acids, between about 480 amino acids to about 650 amino acids, between about 480 amino acids to about 640 amino acids, between about 30 amino acids to about 630 amino acids, between about 480 amino acids to about 620 amino acids, between about 480 amino acids to about 610 amino acids, between about 480 amino acids to about 600 amino acids, between about 480 amino acids to about 590 amino acids, between about 480 amino acids to about 580 amino acids, between about 480 amino acids to about 570 amino acids, between about 480 amino acids to about 560 amino acids, between about 480 amino acids to about 550 amino acids, between about 480 amino acids to about 540 amino acids, between about 480 amino acids to about 530 amino acids, between about 480 amino acids to about 520 amino acids, between about 480 amino acids to about 510 amino acids, between about 480 amino acids to about 500 amino acids, between about 480 amino acids to about 490 amino acids, between about 50 amino acids to about 800 amino acids, between about 50 amino acids to about 790 amino acids, between about 50 amino acids to about 780 amino acids, between about 50 amino acids to about 770 amino acids, between about 50 amino acids to about 760 amino acids, between about 50 amino acids to about 750 amino acids, between about 50 amino acids to about 740 amino acids, between about 50 amino acids to about 730 amino acids, between about 50 amino acids to about 720 amino acids, between about 50 amino acids to about 710 amino acids, between about 50 amino acids to about 700 amino acids, between about 50 amino acids to about 690 amino acids, between about 50 amino acids to about 680 amino acids, between 30 amino acids to about 670 amino acids, between about 50 amino acids to about 660 amino acids, between about 50 amino acids to about 650 amino acids, between about 50 amino acids to about 640 amino acids, between about 30 amino acids to about 630 amino acids, between about 50 amino acids to about 620 amino acids, between about 50 amino acids to about 610 amino acids, between about 50 amino acids to about 600 amino acids, between about 50 amino acids to about 590 amino acids, between about 50 amino acids to about 580 amino acids, between about 50 amino acids to about 570 amino acids, between about 50 amino acids to about 560 amino acids, between about 50 amino acids to about 550 amino acids, between about 50 amino acids to about 540 amino acids, between about 50 amino acids to about 530 amino acids, between about 50 amino acids to about 520 amino acids, between about 50 amino acids to about 510 amino acids, between about 550 amino acids to about 800 amino acids, between about 550 amino acids to about 790 amino acids, between about 550 amino acids to about 780 amino acids, between about 550 amino acids to about 770 amino acids, between about 550 amino acids to about 760 amino acids, between about 550 amino acids to about 750 amino acids, between about 550 amino acids to about 740 amino acids, between about 550 amino acids to about 730 amino acids, between about 550 amino acids to about 720 amino acids, between about 550 amino acids to about 710 amino acids, between about 550 amino acids to about 700 amino acids, between about 550 amino acids to about 690 amino acids, between about 550 amino acids to about 680 amino acids, between 30 amino acids to about 670 amino acids, between about 550 amino acids to about 660 amino acids, between about 550 amino acids to about 650 amino acids, between about 550 amino acids to about 640 amino acids, between about 30 amino acids to about 630 amino acids, between about 550 amino acids to about 620 amino acids, between about 550 amino acids to about 610 amino acids, between about 550 amino acids to about 600 amino acids, between about 550 amino acids to about 590 amino acids, between about 550 amino acids to about 580 amino acids, between about 550 amino acids to about 570 amino acids, between about 550 amino acids to about 560 amino acids, between about 580 amino acids to about 800 amino acids, between about 580 amino acids to about 790 amino acids, between about 580 amino acids to about 780 amino acids, between about 580 amino acids to about 770 amino acids, between about 580 amino acids to about 760 amino acids, between about 580 amino acids to about 750 amino acids, between about 580 amino acids to about 740 amino acids, between about 580 amino acids to about 730 amino acids, between about 580 amino acids to about 720 amino acids, between about 580 amino acids to about 710 amino acids, between about 580 amino acids to about 700 amino acids, between about 580 amino acids to about 690 amino acids, between about 580 amino acids to about 680 amino acids, between 30 amino acids to about 670 amino acids, between about 580 amino acids to about 660 amino acids, between about 580 amino acids to about 650 amino acids, between about 580 amino acids to about 640 amino acids, between about 30 amino acids to about 630 amino acids, between about 580 amino acids to about 620 amino acids, between about 580 amino acids to about 610 amino acids, between about 580 amino acids to about 600 amino acids, between about 580 amino acids to about 590 amino acids, between about 600 amino acids to about 800 amino acids, between about 600 amino acids to about 790 amino acids, between about 600 amino acids to about 780 amino acids, between about 600 amino acids to about 770 amino acids, between about 600 amino acids to about 760 amino acids, between about 600 amino acids to about 750 amino acids, between about 600 amino acids to about 740 amino acids, between about 600 amino acids to about 730 amino acids, between about 600 amino acids to about 720 amino acids, between about 600 amino acids to about 710 amino acids, between about 600 amino acids to about 700 amino acids, between about 600 amino acids to about 690 amino acids, between about 600 amino acids to about 680 amino acids, between 30 amino acids to about 670 amino acids, between about 600 amino acids to about 660 amino acids, between about 600 amino acids to about 650 amino acids, between about 600 amino acids to about 640 amino acids, between about 30 amino acids to about 630 amino acids, between about 600 amino acids to about 620 amino acids, between about 600 amino acids to about 610 amino acids, between about 650 amino acids to about 800 amino acids, between about 650 amino acids to about 790 amino acids, between about 650 amino acids to about 780 amino acids, between about 650 amino acids to about 770 amino acids, between about 650 amino acids to about 760 amino acids, between about 650 amino acids to about 750 amino acids, between about 650 amino acids to about 740 amino acids, between about 650 amino acids to about 730 amino acids, between about 650 amino acids to about 720 amino acids, between about 650 amino acids to about 710 amino acids, between about 650 amino acids to about 700 amino acids, between about 650 amino acids to about 690 amino acids, between about 650 amino acids to about 680 amino acids, between 30 amino acids to about 670 amino acids, between about 650 amino acids to about 660 amino acids, between about 680 amino acids to about 800 amino acids, between about 680 amino acids to about 790 amino acids, between about 680 amino acids to about 780 amino acids, between about 680 amino acids to about 770 amino acids, between about 680 amino acids to about 760 amino acids, between about 680 amino acids to about 750 amino acids, between about 680 amino acids to about 740 amino acids, between about 680 amino acids to about 730 amino acids, between about 680 amino acids to about 720 amino acids, between about 680 amino acids to about 710 amino acids, between about 680 amino acids to about 700 amino acids, between about 680 amino acids to about 690 amino acids, between about 700 amino acids to about 800 amino acids, between about 700 amino acids to about 790 amino acids, between about 700 amino acids to about 780 amino acids, between about 700 amino acids to about 770 amino acids, between about 700 amino acids to about 760 amino acids, between about 700 amino acids to about 750 amino acids, between about 700 amino acids to about 740 amino acids, between about 700 amino acids to about 730 amino acids, between about 700 amino acids to about 720 amino acids, between about 700 amino acids to about 710 amino acids, between about 750 amino acids to about 800 amino acids, between about 750 amino acids to about 790 amino acids, between about 750 amino acids to about 780 amino acids, between about 750 amino acids to about 770 amino acids, between about 750 amino acids to about 760 amino acids, or between about 780 amino acids to about 800 amino acids) in length. In some examples, the first vector further includes one or both of a promoter (e.g., any of the promoters described herein or known in the art) and a Kozak sequence (e.g., any of the exemplary Kozak sequences described herein or known in the art). In some examples, the first vector includes a promoter that is an inducible promoter, a constitutive promoter, or a tissue-specific promoter. In some examples, the second of the two different vectors includes a coding sequence that encodes a C-terminal portion of the target protein (e.g., a C-terminal potion of the supporting cell target protein or a C-terminal portion of the hair cell target protein). In some examples, the C-terminal portion of the target protein (e.g., the C-terminal portion of the supporting cell target protein or the C-terminal portion of the hair cell target protein) is between about 30 amino acids to about 800 amino acids (e.g., between about 30 amino acids to about 790 amino acids, between about 30 amino acids to about 780 amino acids, between about 30 amino acids to about 770 amino acids, between about 30 amino acids to about 760 amino acids, between about 30 amino acids to about 750 amino acids, between about 30 amino acids to about 740 amino acids, between about 30 amino acids to about 730 amino acids, between about 30 amino acids to about 720 amino acids, between about 30 amino acids to about 710 amino acids, between about 30 amino acids to about 700 amino acids, between about 30 amino acids to about 690 amino acids, between about 30 amino acids to about 680 amino acids, between 30 amino acids to about 670 amino acids, between about 30 amino acids to about 660 amino acids, between about 30 amino acids to about 650 amino acids, between about 30 amino acids to about 640 amino acids, between about 30 amino acids to about 630 amino acids, between about 30 amino acids to about 620 amino acids, between about 30 amino acids to about 610 amino acids, between about 30 amino acids to about 600 amino acids, between about 30 amino acids to about 590 amino acids, between about 30 amino acids to about 580 amino acids, between about 30 amino acids to about 570 amino acids, between about 30 amino acids to about 560 amino acids, between about 30 amino acids to about 550 amino acids, between about 30 amino acids to about 540 amino acids, between about 30 amino acids to about 530 amino acids, between about 30 amino acids to about 520 amino acids, between about 30 amino acids to about 510 amino acids, between about 30 amino acids to about 500 amino acids, between about 30 amino acids to about 490 amino acids, between about 30 amino acids to about 480 amino acids, between about 30 amino acids to about 470 amino acids, between about 30 amino acids to about 460 amino acids, between about 30 amino acids to about 450 amino acids, between about 30 amino acids to about 440 amino acids, between about 30 amino acids to about 430 amino acids, between about 30 amino acids to about 420 amino acids, between about 30 amino acids to about 410 amino acids, between about 30 amino acids to about 400 amino acids, between about 30 amino acids to about 390 amino acids, between about 30 amino acids to about 380 amino acids, between about 30 amino acids to about 370 amino acids, between about 30 amino acids to about 360 amino acids, between about 30 amino acids to about 350 amino acids, between about 30 amino acids to about 340 amino acids, between about 30 amino acids to about 330 amino acids, between about 30 amino acids to about 320 amino acids, between about 30 amino acids to about 310 amino acids, between about 30 amino acids to about 300 amino acids, between about 30 amino acids to about 290 amino acids, between about 30 amino acids to about 280 amino acids, between about 30 amino acids to about 270 amino acids, between about 30 amino acids to about 260 amino acids, between about 30 amino acids to about 250 amino acids, between about 30 amino acids to about 240 amino acids, between about 30 amino acids to about 230 amino acids, between about 30 amino acids to about 220 amino acids, between about 30 amino acids to about 210 amino acids, between about 30 amino acids to about 200 amino acids, between about 30 amino acids to about 190 amino acids, between about 30 amino acids to about 180 amino acids, between about 30 amino acids to about 170 amino acids, between about 30 amino acids to about 160 amino acids, between about 30 amino acids to about 150 amino acids, between about 30 amino acids to about 140 amino acids, between about 30 amino acids to about 130 amino acids, between about 30 amino acids to about 120 amino acids, between about 30 amino acids to about 110 amino acids, between about 30 amino acids to about 100 amino acids, between about 30 amino acids to about 90 amino acids, between about 30 amino acids to about 80 amino acids, between about 30 amino acids to about 70 amino acids, between about 30 amino acids to about 60 amino acids, between about 30 amino acids to about 50 amino acids, between about 30 amino acids to about 40 amino acids, between about 50 amino acids to about 800 amino acids, between about 50 amino acids to about 790 amino acids, between about 50 amino acids to about 780 amino acids, between about 50 amino acids to about 770 amino acids, between about 50 amino acids to about 760 amino acids, between about 50 amino acids to about 750 amino acids, between about 50 amino acids to about 740 amino acids, between about 50 amino acids to about 730 amino acids, between about 50 amino acids to about 720 amino acids, between about 50 amino acids to about 710 amino acids, between about 50 amino acids to about 700 amino acids, between about 50 amino acids to about 690 amino acids, between about 50 amino acids to about 680 amino acids, between 30 amino acids to about 670 amino acids, between about 50 amino acids to about 660 amino acids, between about 50 amino acids to about 650 amino acids, between about 50 amino acids to about 640 amino acids, between about 30 amino acids to about 630 amino acids, between about 50 amino acids to about 620 amino acids, between about 50 amino acids to about 610 amino acids, between about 50 amino acids to about 600 amino acids, between about 50 amino acids to about 590 amino acids, between about 50 amino acids to about 580 amino acids, between about 50 amino acids to about 570 amino acids, between about 50 amino acids to about 560 amino acids, between about 50 amino acids to about 550 amino acids, between about 50 amino acids to about 540 amino acids, between about 50 amino acids to about 530 amino acids, between about 50 amino acids to about 520 amino acids, between about 50 amino acids to about 510 amino acids, between about 50 amino acids to about 500 amino acids, between about 50 amino acids to about 490 amino acids, between about 50 amino acids to about 480 amino acids, between about 50 amino acids to about 470 amino acids, between about 50 amino acids to about 460 amino acids, between about 50 amino acids to about 450 amino acids, between about 50 amino acids to about 440 amino acids, between about 50 amino acids to about 430 amino acids, between about 50 amino acids to about 420 amino acids, between about 50 amino acids to about 410 amino acids, between about 50 amino acids to about 400 amino acids, between about 50 amino acids to about 390 amino acids, between about 50 amino acids to about 380 amino acids, between about 50 amino acids to about 370 amino acids, between about 50 amino acids to about 360 amino acids, between about 50 amino acids to about 350 amino acids, between about 50 amino acids to about 340 amino acids, between about 50 amino acids to about 330 amino acids, between about 50 amino acids to about 320 amino acids, between about 50 amino acids to about 310 amino acids, between about 50 amino acids to about 300 amino acids, between about 50 amino acids to about 290 amino acids, between about 50 amino acids to about 280 amino acids, between about 50 amino acids to about 270 amino acids, between about 50 amino acids to about 260 amino acids, between about 50 amino acids to about 250 amino acids, between about 50 amino acids to about 240 amino acids, between about 50 amino acids to about 230 amino acids, between about 50 amino acids to about 220 amino acids, between about 50 amino acids to about 210 amino acids, between about 50 amino acids to about 200 amino acids, between about 50 amino acids to about 190 amino acids, between about 50 amino acids to about 180 amino acids, between about 50 amino acids to about 170 amino acids, between about 50 amino acids to about 160 amino acids, between about 50 amino acids to about 150 amino acids, between about 50 amino acids to about 140 amino acids, between about 50 amino acids to about 130 amino acids, between about 50 amino acids to about 120 amino acids, between about 50 amino acids to about 110 amino acids, between about 50 amino acids to about 100 amino acids, between about 50 amino acids to about 90 amino acids, between about 50 amino acids to about 80 amino acids, between about 50 amino acids to about 70 amino acids, between about 50 amino acids to about 60 amino acids, between about 80 amino acids to about 800 amino acids, between about 80 amino acids to about 790 amino acids, between about 80 amino acids to about 780 amino acids, between about 80 amino acids to about 770 amino acids, between about 80 amino acids to about 760 amino acids, between about 80 amino acids to about 750 amino acids, between about 80 amino acids to about 740 amino acids, between about 80 amino acids to about 730 amino acids, between about 80 amino acids to about 720 amino acids, between about 80 amino acids to about 710 amino acids, between about 80 amino acids to about 700 amino acids, between about 80 amino acids to about 690 amino acids, between about 80 amino acids to about 680 amino acids, between 30 amino acids to about 670 amino acids, between about 80 amino acids to about 660 amino acids, between about 80 amino acids to about 650 amino acids, between about 80 amino acids to about 640 amino acids, between about 30 amino acids to about 630 amino acids, between about 80 amino acids to about 620 amino acids, between about 80 amino acids to about 610 amino acids, between about 80 amino acids to about 600 amino acids, between about 80 amino acids to about 590 amino acids, between about 80 amino acids to about 580 amino acids, between about 80 amino acids to about 570 amino acids, between about 80 amino acids to about 560 amino acids, between about 80 amino acids to about 550 amino acids, between about 80 amino acids to about 540 amino acids, between about 80 amino acids to about 530 amino acids, between about 80 amino acids to about 520 amino acids, between about 80 amino acids to about 510 amino acids, between about 80 amino acids to about 500 amino acids, between about 80 amino acids to about 490 amino acids, between about 80 amino acids to about 480 amino acids, between about 80 amino acids to about 470 amino acids, between about 80 amino acids to about 460 amino acids, between about 80 amino acids to about 450 amino acids, between about 80 amino acids to about 440 amino acids, between about 80 amino acids to about 430 amino acids, between about 80 amino acids to about 420 amino acids, between about 80 amino acids to about 410 amino acids, between about 80 amino acids to about 400 amino acids, between about 80 amino acids to about 390 amino acids, between about 80 amino acids to about 380 amino acids, between about 80 amino acids to about 370 amino acids, between about 80 amino acids to about 360 amino acids, between about 80 amino acids to about 350 amino acids, between about 80 amino acids to about 340 amino acids, between about 80 amino acids to about 330 amino acids, between about 80 amino acids to about 320 amino acids, between about 80 amino acids to about 310 amino acids, between about 80 amino acids to about 300 amino acids, between about 80 amino acids to about 290 amino acids, between about 80 amino acids to about 280 amino acids, between about 80 amino acids to about 270 amino acids, between about 80 amino acids to about 260 amino acids, between about 80 amino acids to about 250 amino acids, between about 80 amino acids to about 240 amino acids, between about 80 amino acids to about 230 amino acids, between about 80 amino acids to about 220 amino acids, between about 80 amino acids to about 210 amino acids, between about 80 amino acids to about 200 amino acids, between about 80 amino acids to about 190 amino acids, between about 80 amino acids to about 180 amino acids, between about 80 amino acids to about 170 amino acids, between about 80 amino acids to about 160 amino acids, between about 80 amino acids to about 150 amino acids, between about 80 amino acids to about 140 amino acids, between about 80 amino acids to about 130 amino acids, between about 80 amino acids to about 120 amino acids, between about 80 amino acids to about 110 amino acids, between about 80 amino acids to about 100 amino acids, between about 100 amino acids to about 800 amino acids, between about 100 amino acids to about 790 amino acids, between about 100 amino acids to about 780 amino acids, between about 100 amino acids to about 770 amino acids, between about 100 amino acids to about 760 amino acids, between about 100 amino acids to about 750 amino acids, between about 100 amino acids to about 740 amino acids, between about 100 amino acids to about 730 amino acids, between about 100 amino acids to about 720 amino acids, between about 100 amino acids to about 710 amino acids, between about 100 amino acids to about 700 amino acids, between about 100 amino acids to about 690 amino acids, between about 100 amino acids to about 680 amino acids, between 30 amino acids to about 670 amino acids, between about 100 amino acids to about 660 amino acids, between about 100 amino acids to about 650 amino acids, between about 100 amino acids to about 640 amino acids, between about 30 amino acids to about 630 amino acids, between about 100 amino acids to about 620 amino acids, between about 100 amino acids to about 610 amino acids, between about 100 amino acids to about 600 amino acids, between about 100 amino acids to about 590 amino acids, between about 100 amino acids to about 580 amino acids, between about 100 amino acids to about 570 amino acids, between about 100 amino acids to about 560 amino acids, between about 100 amino acids to about 550 amino acids, between about 100 amino acids to about 540 amino acids, between about 100 amino acids to about 530 amino acids, between about 100 amino acids to about 520 amino acids, between about 100 amino acids to about 510 amino acids, between about 100 amino acids to about 500 amino acids, between about 100 amino acids to about 490 amino acids, between about 100 amino acids to about 480 amino acids, between about 100 amino acids to about 470 amino acids, between about 100 amino acids to about 460 amino acids, between about 100 amino acids to about 450 amino acids, between about 100 amino acids to about 440 amino acids, between about 100 amino acids to about 430 amino acids, between about 100 amino acids to about 420 amino acids, between about 100 amino acids to about 410 amino acids, between about 100 amino acids to about 400 amino acids, between about 100 amino acids to about 390 amino acids, between about 100 amino acids to about 380 amino acids, between about 100 amino acids to about 370 amino acids, between about 100 amino acids to about 360 amino acids, between about 100 amino acids to about 350 amino acids, between about 100 amino acids to about 340 amino acids, between about 100 amino acids to about 330 amino acids, between about 100 amino acids to about 320 amino acids, between about 100 amino acids to about 310 amino acids, between about 100 amino acids to about 300 amino acids, between about 100 amino acids to about 290 amino acids, between about 100 amino acids to about 280 amino acids, between about 100 amino acids to about 270 amino acids, between about 100 amino acids to about 260 amino acids, between about 100 amino acids to about 250 amino acids, between about 100 amino acids to about 240 amino acids, between about 100 amino acids to about 230 amino acids, between about 100 amino acids to about 220 amino acids, between about 100 amino acids to about 210 amino acids, between about 100 amino acids to about 200 amino acids, between about 100 amino acids to about 190 amino acids, between about 100 amino acids to about 180 amino acids, between about 100 amino acids to about 170 amino acids, between about 100 amino acids to about 160 amino acids, between about 100 amino acids to about 150 amino acids, between about 100 amino acids to about 140 amino acids, between about 100 amino acids to about 130 amino acids, between about 100 amino acids to about 120 amino acids, between about 100 amino acids to about 110 amino acids, between about 120 amino acids to about 800 amino acids, between about 120 amino acids to about 790 amino acids, between about 120 amino acids to about 780 amino acids, between about 120 amino acids to about 770 amino acids, between about 120 amino acids to about 760 amino acids, between about 120 amino acids to about 750 amino acids, between about 120 amino acids to about 740 amino acids, between about 120 amino acids to about 730 amino acids, between about 120 amino acids to about 720 amino acids, between about 120 amino acids to about 710 amino acids, between about 120 amino acids to about 700 amino acids, between about 120 amino acids to about 690 amino acids, between about 120 amino acids to about 680 amino acids, between 30 amino acids to about 670 amino acids, between about 120 amino acids to about 660 amino acids, between about 120 amino acids to about 650 amino acids, between about 120 amino acids to about 640 amino acids, between about 30 amino acids to about 630 amino acids, between about 120 amino acids to about 620 amino acids, between about 120 amino acids to about 610 amino acids, between about 120 amino acids to about 600 amino acids, between about 120 amino acids to about 590 amino acids, between about 120 amino acids to about 580 amino acids, between about 120 amino acids to about 570 amino acids, between about 120 amino acids to about 560 amino acids, between about 120 amino acids to about 550 amino acids, between about 120 amino acids to about 540 amino acids, between about 120 amino acids to about 530 amino acids, between about 120 amino acids to about 520 amino acids, between about 120 amino acids to about 510 amino acids, between about 120 amino acids to about 500 amino acids, between about 120 amino acids to about 490 amino acids, between about 120 amino acids to about 480 amino acids, between about 120 amino acids to about 470 amino acids, between about 120 amino acids to about 460 amino acids, between about 120 amino acids to about 450 amino acids, between about 120 amino acids to about 440 amino acids, between about 120 amino acids to about 430 amino acids, between about 120 amino acids to about 420 amino acids, between about 120 amino acids to about 410 amino acids, between about 120 amino acids to about 400 amino acids, between about 120 amino acids to about 390 amino acids, between about 120 amino acids to about 380 amino acids, between about 120 amino acids to about 370 amino acids, between about 120 amino acids to about 360 amino acids, between about 120 amino acids to about 350 amino acids, between about 120 amino acids to about 340 amino acids, between about 120 amino acids to about 330 amino acids, between about 120 amino acids to about 320 amino acids, between about 120 amino acids to about 310 amino acids, between about 120 amino acids to about 300 amino acids, between about 120 amino acids to about 290 amino acids, between about 120 amino acids to about 280 amino acids, between about 120 amino acids to about 270 amino acids, between about 120 amino acids to about 260 amino acids, between about 120 amino acids to about 250 amino acids, between about 120 amino acids to about 240 amino acids, between about 120 amino acids to about 230 amino acids, between about 120 amino acids to about 220 amino acids, between about 120 amino acids to about 210 amino acids, between about 120 amino acids to about 200 amino acids, between about 120 amino acids to about 190 amino acids, between about 120 amino acids to about 180 amino acids, between about 120 amino acids to about 170 amino acids, between about 120 amino acids to about 160 amino acids, between about 120 amino acids to about 150 amino acids, between about 120 amino acids to about 140 amino acids, between about 120 amino acids to about 130 amino acids, between about 150 amino acids to about 800 amino acids, between about 150 amino acids to about 790 amino acids, between about 150 amino acids to about 780 amino acids, between about 150 amino acids to about 770 amino acids, between about 150 amino acids to about 760 amino acids, between about 150 amino acids to about 750 amino acids, between about 150 amino acids to about 740 amino acids, between about 150 amino acids to about 730 amino acids, between about 150 amino acids to about 720 amino acids, between about 150 amino acids to about 710 amino acids, between about 150 amino acids to about 700 amino acids, between about 150 amino acids to about 690 amino acids, between about 150 amino acids to about 680 amino acids, between 30 amino acids to about 670 amino acids, between about 150 amino acids to about 660 amino acids, between about 150 amino acids to about 650 amino acids, between about 150 amino acids to about 640 amino acids, between about 30 amino acids to about 630 amino acids, between about 150 amino acids to about 620 amino acids, between about 150 amino acids to about 610 amino acids, between about 150 amino acids to about 600 amino acids, between about 150 amino acids to about 590 amino acids, between about 150 amino acids to about 580 amino acids, between about 150 amino acids to about 570 amino acids, between about 150 amino acids to about 560 amino acids, between about 150 amino acids to about 550 amino acids, between about 150 amino acids to about 540 amino acids, between about 150 amino acids to about 530 amino acids, between about 150 amino acids to about 520 amino acids, between about 150 amino acids to about 510 amino acids, between about 150 amino acids to about 500 amino acids, between about 150 amino acids to about 490 amino acids, between about 150 amino acids to about 480 amino acids, between about 150 amino acids to about 470 amino acids, between about 150 amino acids to about 460 amino acids, between about 150 amino acids to about 450 amino acids, between about 150 amino acids to about 440 amino acids, between about 150 amino acids to about 430 amino acids, between about 150 amino acids to about 420 amino acids, between about 150 amino acids to about 410 amino acids, between about 150 amino acids to about 400 amino acids, between about 150 amino acids to about 390 amino acids, between about 150 amino acids to about 380 amino acids, between about 150 amino acids to about 370 amino acids, between about 150 amino acids to about 360 amino acids, between about 150 amino acids to about 350 amino acids, between about 150 amino acids to about 340 amino acids, between about 150 amino acids to about 330 amino acids, between about 150 amino acids to about 320 amino acids, between about 150 amino acids to about 310 amino acids, between about 150 amino acids to about 300 amino acids, between about 150 amino acids to about 290 amino acids, between about 150 amino acids to about 280 amino acids, between about 150 amino acids to about 270 amino acids, between about 150 amino acids to about 260 amino acids, between about 150 amino acids to about 250 amino acids, between about 150 amino acids to about 240 amino acids, between about 150 amino acids to about 230 amino acids, between about 150 amino acids to about 220 amino acids, between about 150 amino acids to about 210 amino acids, between about 150 amino acids to about 200 amino acids, between about 150 amino acids to about 190 amino acids, between about 150 amino acids to about 180 amino acids, between about 150 amino acids to about 170 amino acids, between about 150 amino acids to about 160 amino acids, between about 180 amino acids to about 800 amino acids, between about 180 amino acids to about 790 amino acids, between about 180 amino acids to about 780 amino acids, between about 180 amino acids to about 770 amino acids, between about 180 amino acids to about 760 amino acids, between about 180 amino acids to about 750 amino acids, between about 180 amino acids to about 740 amino acids, between about 180 amino acids to about 730 amino acids, between about 180 amino acids to about 720 amino acids, between about 180 amino acids to about 710 amino acids, between about 180 amino acids to about 700 amino acids, between about 180 amino acids to about 690 amino acids, between about 180 amino acids to about 680 amino acids, between 30 amino acids to about 670 amino acids, between about 180 amino acids to about 660 amino acids, between about 180 amino acids to about 650 amino acids, between about 180 amino acids to about 640 amino acids, between about 30 amino acids to about 630 amino acids, between about 180 amino acids to about 620 amino acids, between about 180 amino acids to about 610 amino acids, between about 180 amino acids to about 600 amino acids, between about 180 amino acids to about 590 amino acids, between about 180 amino acids to about 580 amino acids, between about 180 amino acids to about 570 amino acids, between about 180 amino acids to about 560 amino acids, between about 180 amino acids to about 550 amino acids, between about 180 amino acids to about 540 amino acids, between about 180 amino acids to about 530 amino acids, between about 180 amino acids to about 520 amino acids, between about 180 amino acids to about 510 amino acids, between about 180 amino acids to about 500 amino acids, between about 180 amino acids to about 490 amino acids, between about 180 amino acids to about 480 amino acids, between about 180 amino acids to about 470 amino acids, between about 180 amino acids to about 460 amino acids, between about 180 amino acids to about 450 amino acids, between about 180 amino acids to about 440 amino acids, between about 180 amino acids to about 430 amino acids, between about 180 amino acids to about 420 amino acids, between about 180 amino acids to about 410 amino acids, between about 180 amino acids to about 400 amino acids, between about 180 amino acids to about 390 amino acids, between about 180 amino acids to about 380 amino acids, between about 180 amino acids to about 370 amino acids, between about 180 amino acids to about 360 amino acids, between about 180 amino acids to about 350 amino acids, between about 180 amino acids to about 340 amino acids, between about 180 amino acids to about 330 amino acids, between about 180 amino acids to about 320 amino acids, between about 180 amino acids to about 310 amino acids, between about 180 amino acids to about 300 amino acids, between about 180 amino acids to about 290 amino acids, between about 180 amino acids to about 280 amino acids, between about 180 amino acids to about 270 amino acids, between about 180 amino acids to about 260 amino acids, between about 180 amino acids to about 250 amino acids, between about 180 amino acids to about 240 amino acids, between about 180 amino acids to about 230 amino acids, between about 180 amino acids to about 220 amino acids, between about 180 amino acids to about 210 amino acids, between about 180 amino acids to about 200 amino acids, between about 180 amino acids to about 190 amino acids, between about 200 amino acids to about 800 amino acids, between about 200 amino acids to about 790 amino acids, between about 200 amino acids to about 780 amino acids, between about 200 amino acids to about 770 amino acids, between about 200 amino acids to about 760 amino acids, between about 200 amino acids to about 750 amino acids, between about 200 amino acids to about 740 amino acids, between about 200 amino acids to about 730 amino acids, between about 200 amino acids to about 720 amino acids, between about 200 amino acids to about 710 amino acids, between about 200 amino acids to about 700 amino acids, between about 200 amino acids to about 690 amino acids, between about 200 amino acids to about 680 amino acids, between 30 amino acids to about 670 amino acids, between about 200 amino acids to about 660 amino acids, between about 200 amino acids to about 650 amino acids, between about 200 amino acids to about 640 amino acids, between about 30 amino acids to about 630 amino acids, between about 200 amino acids to about 620 amino acids, between about 200 amino acids to about 610 amino acids, between about 200 amino acids to about 600 amino acids, between about 200 amino acids to about 590 amino acids, between about 200 amino acids to about 580 amino acids, between about 200 amino acids to about 570 amino acids, between about 200 amino acids to about 560 amino acids, between about 200 amino acids to about 550 amino acids, between about 200 amino acids to about 540 amino acids, between about 200 amino acids to about 530 amino acids, between about 200 amino acids to about 520 amino acids, between about 200 amino acids to about 510 amino acids, between about 200 amino acids to about 500 amino acids, between about 200 amino acids to about 490 amino acids, between about 200 amino acids to about 480 amino acids, between about 200 amino acids to about 470 amino acids, between about 200 amino acids to about 460 amino acids, between about 200 amino acids to about 450 amino acids, between about 200 amino acids to about 440 amino acids, between about 200 amino acids to about 430 amino acids, between about 200 amino acids to about 420 amino acids, between about 200 amino acids to about 410 amino acids, between about 200 amino acids to about 400 amino acids, between about 200 amino acids to about 390 amino acids, between about 200 amino acids to about 380 amino acids, between about 200 amino acids to about 370 amino acids, between about 200 amino acids to about 360 amino acids, between about 200 amino acids to about 350 amino acids, between about 200 amino acids to about 340 amino acids, between about 200 amino acids to about 330 amino acids, between about 200 amino acids to about 320 amino acids, between about 200 amino acids to about 310 amino acids, between about 200 amino acids to about 300 amino acids, between about 200 amino acids to about 290 amino acids, between about 200 amino acids to about 280 amino acids, between about 200 amino acids to about 270 amino acids, between about 200 amino acids to about 260 amino acids, between about 200 amino acids to about 250 amino acids, between about 200 amino acids to about 240 amino acids, between about 200 amino acids to about 230 amino acids, between about 200 amino acids to about 220 amino acids, between about 200 amino acids to about 210 amino acids, between about 250 amino acids to about 800 amino acids, between about 250 amino acids to about 790 amino acids, between about 250 amino acids to about 780 amino acids, between about 250 amino acids to about 770 amino acids, between about 250 amino acids to about 760 amino acids, between about 250 amino acids to about 750 amino acids, between about 250 amino acids to about 740 amino acids, between about 250 amino acids to about 730 amino acids, between about 250 amino acids to about 720 amino acids, between about 250 amino acids to about 710 amino acids, between about 250 amino acids to about 700 amino acids, between about 250 amino acids to about 690 amino acids, between about 250 amino acids to about 680 amino acids, between 30 amino acids to about 670 amino acids, between about 250 amino acids to about 660 amino acids, between about 250 amino acids to about 650 amino acids, between about 250 amino acids to about 640 amino acids, between about 30 amino acids to about 630 amino acids, between about 250 amino acids to about 620 amino acids, between about 250 amino acids to about 610 amino acids, between about 250 amino acids to about 600 amino acids, between about 250 amino acids to about 590 amino acids, between about 250 amino acids to about 580 amino acids, between about 250 amino acids to about 570 amino acids, between about 250 amino acids to about 560 amino acids, between about 250 amino acids to about 550 amino acids, between about 250 amino acids to about 540 amino acids, between about 250 amino acids to about 530 amino acids, between about 250 amino acids to about 520 amino acids, between about 250 amino acids to about 510 amino acids, between about 250 amino acids to about 500 amino acids, between about 250 amino acids to about 490 amino acids, between about 250 amino acids to about 480 amino acids, between about 250 amino acids to about 470 amino acids, between about 250 amino acids to about 460 amino acids, between about 250 amino acids to about 450 amino acids, between about 250 amino acids to about 440 amino acids, between about 250 amino acids to about 430 amino acids, between about 250 amino acids to about 420 amino acids, between about 250 amino acids to about 410 amino acids, between about 250 amino acids to about 400 amino acids, between about 250 amino acids to about 390 amino acids, between about 250 amino acids to about 380 amino acids, between about 250 amino acids to about 370 amino acids, between about 250 amino acids to about 360 amino acids, between about 250 amino acids to about 350 amino acids, between about 250 amino acids to about 340 amino acids, between about 250 amino acids to about 330 amino acids, between about 250 amino acids to about 320 amino acids, between about 250 amino acids to about 310 amino acids, between about 250 amino acids to about 300 amino acids, between about 250 amino acids to about 290 amino acids, between about 250 amino acids to about 280 amino acids, between about 250 amino acids to about 270 amino acids, between about 250 amino acids to about 260 amino acids, between about 280 amino acids to about 800 amino acids, between about 280 amino acids to about 790 amino acids, between about 280 amino acids to about 780 amino acids, between about 280 amino acids to about 770 amino acids, between about 280 amino acids to about 760 amino acids, between about 280 amino acids to about 750 amino acids, between about 280 amino acids to about 740 amino acids, between about 280 amino acids to about 730 amino acids, between about 280 amino acids to about 720 amino acids, between about 280 amino acids to about 710 amino acids, between about 280 amino acids to about 700 amino acids, between about 280 amino acids to about 690 amino acids, between about 280 amino acids to about 680 amino acids, between 30 amino acids to about 670 amino acids, between about 280 amino acids to about 660 amino acids, between about 280 amino acids to about 650 amino acids, between about 280 amino acids to about 640 amino acids, between about 30 amino acids to about 630 amino acids, between about 280 amino acids to about 620 amino acids, between about 280 amino acids to about 610 amino acids, between about 280 amino acids to about 600 amino acids, between about 280 amino acids to about 590 amino acids, between about 280 amino acids to about 580 amino acids, between about 280 amino acids to about 570 amino acids, between about 280 amino acids to about 560 amino acids, between about 280 amino acids to about 550 amino acids, between about 280 amino acids to about 540 amino acids, between about 280 amino acids to about 530 amino acids, between about 280 amino acids to about 520 amino acids, between about 280 amino acids to about 510 amino acids, between about 280 amino acids to about 500 amino acids, between about 280 amino acids to about 490 amino acids, between about 280 amino acids to about 480 amino acids, between about 280 amino acids to about 470 amino acids, between about 280 amino acids to about 460 amino acids, between about 280 amino acids to about 450 amino acids, between about 280 amino acids to about 440 amino acids, between about 280 amino acids to about 430 amino acids, between about 280 amino acids to about 420 amino acids, between about 280 amino acids to about 410 amino acids, between about 280 amino acids to about 400 amino acids, between about 280 amino acids to about 390 amino acids, between about 280 amino acids to about 380 amino acids, between about 280 amino acids to about 370 amino acids, between about 280 amino acids to about 360 amino acids, between about 280 amino acids to about 350 amino acids, between about 280 amino acids to about 340 amino acids, between about 280 amino acids to about 330 amino acids, between about 280 amino acids to about 320 amino acids, between about 280 amino acids to about 310 amino acids, between about 280 amino acids to about 300 amino acids, between about 300 amino acids to about 800 amino acids, between about 300 amino acids to about 790 amino acids, between about 300 amino acids to about 780 amino acids, between about 300 amino acids to about 770 amino acids, between about 300 amino acids to about 760 amino acids, between about 300 amino acids to about 750 amino acids, between about 300 amino acids to about 740 amino acids, between about 300 amino acids to about 730 amino acids, between about 300 amino acids to about 720 amino acids, between about 300 amino acids to about 710 amino acids, between about 300 amino acids to about 700 amino acids, between about 300 amino acids to about 690 amino acids, between about 300 amino acids to about 680 amino acids, between 30 amino acids to about 670 amino acids, between about 300 amino acids to about 660 amino acids, between about 300 amino acids to about 650 amino acids, between about 300 amino acids to about 640 amino acids, between about 30 amino acids to about 630 amino acids, between about 300 amino acids to about 620 amino acids, between about 300 amino acids to about 610 amino acids, between about 300 amino acids to about 600 amino acids, between about 300 amino acids to about 590 amino acids, between about 300 amino acids to about 580 amino acids, between about 300 amino acids to about 570 amino acids, between about 300 amino acids to about 560 amino acids, between about 300 amino acids to about 550 amino acids, between about 300 amino acids to about 540 amino acids, between about 300 amino acids to about 530 amino acids, between about 300 amino acids to about 520 amino acids, between about 300 amino acids to about 510 amino acids, between about 300 amino acids to about 500 amino acids, between about 300 amino acids to about 490 amino acids, between about 300 amino acids to about 480 amino acids, between about 300 amino acids to about 470 amino acids, between about 300 amino acids to about 460 amino acids, between about 300 amino acids to about 450 amino acids, between about 300 amino acids to about 440 amino acids, between about 300 amino acids to about 430 amino acids, between about 300 amino acids to about 420 amino acids, between about 300 amino acids to about 410 amino acids, between about 300 amino acids to about 400 amino acids, between about 300 amino acids to about 390 amino acids, between about 300 amino acids to about 380 amino acids, between about 300 amino acids to about 370 amino acids, between about 300 amino acids to about 360 amino acids, between about 300 amino acids to about 350 amino acids, between about 300 amino acids to about 340 amino acids, between about 300 amino acids to about 330 amino acids, between about 300 amino acids to about 320 amino acids, between about 300 amino acids to about 310 amino acids, between about 350 amino acids to about 800 amino acids, between about 350 amino acids to about 790 amino acids, between about 350 amino acids to about 780 amino acids, between about 350 amino acids to about 770 amino acids, between about 350 amino acids to about 760 amino acids, between about 350 amino acids to about 750 amino acids, between about 350 amino acids to about 740 amino acids, between about 350 amino acids to about 730 amino acids, between about 350 amino acids to about 720 amino acids, between about 350 amino acids to about 710 amino acids, between about 350 amino acids to about 700 amino acids, between about 350 amino acids to about 690 amino acids, between about 350 amino acids to about 680 amino acids, between 30 amino acids to about 670 amino acids, between about 350 amino acids to about 660 amino acids, between about 350 amino acids to about 650 amino acids, between about 350 amino acids to about 640 amino acids, between about 30 amino acids to about 630 amino acids, between about 350 amino acids to about 620 amino acids, between about 350 amino acids to about 610 amino acids, between about 350 amino acids to about 600 amino acids, between about 350 amino acids to about 590 amino acids, between about 350 amino acids to about 580 amino acids, between about 350 amino acids to about 570 amino acids, between about 350 amino acids to about 560 amino acids, between about 350 amino acids to about 550 amino acids, between about 350 amino acids to about 540 amino acids, between about 350 amino acids to about 530 amino acids, between about 350 amino acids to about 520 amino acids, between about 350 amino acids to about 510 amino acids, between about 350 amino acids to about 500 amino acids, between about 350 amino acids to about 490 amino acids, between about 350 amino acids to about 480 amino acids, between about 350 amino acids to about 470 amino acids, between about 350 amino acids to about 460 amino acids, between about 350 amino acids to about 450 amino acids, between about 350 amino acids to about 440 amino acids, between about 350 amino acids to about 430 amino acids, between about 350 amino acids to about 420 amino acids, between about 350 amino acids to about 410 amino acids, between about 350 amino acids to about 400 amino acids, between about 350 amino acids to about 390 amino acids, between about 350 amino acids to about 380 amino acids, between about 350 amino acids to about 370 amino acids, between about 350 amino acids to about 360 amino acids, between about 380 amino acids to about 800 amino acids, between about 380 amino acids to about 790 amino acids, between about 380 amino acids to about 780 amino acids, between about 380 amino acids to about 770 amino acids, between about 380 amino acids to about 760 amino acids, between about 380 amino acids to about 750 amino acids, between about 380 amino acids to about 740 amino acids, between about 380 amino acids to about 730 amino acids, between about 380 amino acids to about 720 amino acids, between about 380 amino acids to about 710 amino acids, between about 380 amino acids to about 700 amino acids, between about 380 amino acids to about 690 amino acids, between about 380 amino acids to about 680 amino acids, between 30 amino acids to about 670 amino acids, between about 380 amino acids to about 660 amino acids, between about 380 amino acids to about 650 amino acids, between about 380 amino acids to about 640 amino acids, between about 30 amino acids to about 630 amino acids, between about 380 amino acids to about 620 amino acids, between about 380 amino acids to about 610 amino acids, between about 380 amino acids to about 600 amino acids, between about 380 amino acids to about 590 amino acids, between about 380 amino acids to about 580 amino acids, between about 380 amino acids to about 570 amino acids, between about 380 amino acids to about 560 amino acids, between about 380 amino acids to about 550 amino acids, between about 380 amino acids to about 540 amino acids, between about 380 amino acids to about 530 amino acids, between about 380 amino acids to about 520 amino acids, between about 380 amino acids to about 510 amino acids, between about 380 amino acids to about 500 amino acids, between about 380 amino acids to about 490 amino acids, between about 380 amino acids to about 480 amino acids, between about 380 amino acids to about 470 amino acids, between about 380 amino acids to about 460 amino acids, between about 380 amino acids to about 450 amino acids, between about 380 amino acids to about 440 amino acids, between about 380 amino acids to about 430 amino acids, between about 380 amino acids to about 420 amino acids, between about 380 amino acids to about 410 amino acids, between about 380 amino acids to about 400 amino acids, between about 380 amino acids to about 390 amino acids, between about 400 amino acids to about 800 amino acids, between about 400 amino acids to about 790 amino acids, between about 400 amino acids to about 780 amino acids, between about 400 amino acids to about 770 amino acids, between about 400 amino acids to about 760 amino acids, between about 400 amino acids to about 750 amino acids, between about 400 amino acids to about 740 amino acids, between about 400 amino acids to about 730 amino acids, between about 400 amino acids to about 720 amino acids, between about 400 amino acids to about 710 amino acids, between about 400 amino acids to about 700 amino acids, between about 400 amino acids to about 690 amino acids, between about 400 amino acids to about 680 amino acids, between 30 amino acids to about 670 amino acids, between about 400 amino acids to about 660 amino acids, between about 400 amino acids to about 650 amino acids, between about 400 amino acids to about 640 amino acids, between about 30 amino acids to about 630 amino acids, between about 400 amino acids to about 620 amino acids, between about 400 amino acids to about 610 amino acids, between about 400 amino acids to about 600 amino acids, between about 400 amino acids to about 590 amino acids, between about 400 amino acids to about 580 amino acids, between about 400 amino acids to about 570 amino acids, between about 400 amino acids to about 560 amino acids, between about 400 amino acids to about 550 amino acids, between about 400 amino acids to about 540 amino acids, between about 400 amino acids to about 530 amino acids, between about 400 amino acids to about 520 amino acids, between about 400 amino acids to about 510 amino acids, between about 400 amino acids to about 500 amino acids, between about 400 amino acids to about 490 amino acids, between about 400 amino acids to about 480 amino acids, between about 400 amino acids to about 470 amino acids, between about 400 amino acids to about 460 amino acids, between about 400 amino acids to about 450 amino acids, between about 400 amino acids to about 440 amino acids, between about 400 amino acids to about 430 amino acids, between about 400 amino acids to about 420 amino acids, between about 400 amino acids to about 410 amino acids, between about 450 amino acids to about 800 amino acids, between about 450 amino acids to about 790 amino acids, between about 450 amino acids to about 780 amino acids, between about 450 amino acids to about 770 amino acids, between about 450 amino acids to about 760 amino acids, between about 450 amino acids to about 750 amino acids, between about 450 amino acids to about 740 amino acids, between about 450 amino acids to about 730 amino acids, between about 450 amino acids to about 720 amino acids, between about 450 amino acids to about 710 amino acids, between about 450 amino acids to about 700 amino acids, between about 450 amino acids to about 690 amino acids, between about 450 amino acids to about 680 amino acids, between 30 amino acids to about 670 amino acids, between about 450 amino acids to about 660 amino acids, between about 450 amino acids to about 650 amino acids, between about 450 amino acids to about 640 amino acids, between about 30 amino acids to about 630 amino acids, between about 450 amino acids to about 620 amino acids, between about 450 amino acids to about 610 amino acids, between about 450 amino acids to about 600 amino acids, between about 450 amino acids to about 590 amino acids, between about 450 amino acids to about 580 amino acids, between about 450 amino acids to about 570 amino acids, between about 450 amino acids to about 560 amino acids, between about 450 amino acids to about 550 amino acids, between about 450 amino acids to about 540 amino acids, between about 450 amino acids to about 530 amino acids, between about 450 amino acids to about 520 amino acids, between about 450 amino acids to about 510 amino acids, between about 450 amino acids to about 500 amino acids, between about 450 amino acids to about 490 amino acids, between about 450 amino acids to about 480 amino acids, between about 450 amino acids to about 470 amino acids, between about 450 amino acids to about 460 amino acids, between about 480 amino acids to about 800 amino acids, between about 480 amino acids to about 790 amino acids, between about 480 amino acids to about 780 amino acids, between about 480 amino acids to about 770 amino acids, between about 480 amino acids to about 760 amino acids, between about 480 amino acids to about 750 amino acids, between about 480 amino acids to about 740 amino acids, between about 480 amino acids to about 730 amino acids, between about 480 amino acids to about 720 amino acids, between about 480 amino acids to about 710 amino acids, between about 480 amino acids to about 700 amino acids, between about 480 amino acids to about 690 amino acids, between about 480 amino acids to about 680 amino acids, between 30 amino acids to about 670 amino acids, between about 480 amino acids to about 660 amino acids, between about 480 amino acids to about 650 amino acids, between about 480 amino acids to about 640 amino acids, between about 30 amino acids to about 630 amino acids, between about 480 amino acids to about 620 amino acids, between about 480 amino acids to about 610 amino acids, between about 480 amino acids to about 600 amino acids, between about 480 amino acids to about 590 amino acids, between about 480 amino acids to about 580 amino acids, between about 480 amino acids to about 570 amino acids, between about 480 amino acids to about 560 amino acids, between about 480 amino acids to about 550 amino acids, between about 480 amino acids to about 540 amino acids, between about 480 amino acids to about 530 amino acids, between about 480 amino acids to about 520 amino acids, between about 480 amino acids to about 510 amino acids, between about 480 amino acids to about 500 amino acids, between about 480 amino acids to about 490 amino acids, between about 50 amino acids to about 800 amino acids, between about 50 amino acids to about 790 amino acids, between about 50 amino acids to about 780 amino acids, between about 50 amino acids to about 770 amino acids, between about 50 amino acids to about 760 amino acids, between about 50 amino acids to about 750 amino acids, between about 50 amino acids to about 740 amino acids, between about 50 amino acids to about 730 amino acids, between about 50 amino acids to about 720 amino acids, between about 50 amino acids to about 710 amino acids, between about 50 amino acids to about 700 amino acids, between about 50 amino acids to about 690 amino acids, between about 50 amino acids to about 680 amino acids, between 30 amino acids to about 670 amino acids, between about 50 amino acids to about 660 amino acids, between about 50 amino acids to about 650 amino acids, between about 50 amino acids to about 640 amino acids, between about 30 amino acids to about 630 amino acids, between about 50 amino acids to about 620 amino acids, between about 50 amino acids to about 610 amino acids, between about 50 amino acids to about 600 amino acids, between about 50 amino acids to about 590 amino acids, between about 50 amino acids to about 580 amino acids, between about 50 amino acids to about 570 amino acids, between about 50 amino acids to about 560 amino acids, between about 50 amino acids to about 550 amino acids, between about 50 amino acids to about 540 amino acids, between about 50 amino acids to about 530 amino acids, between about 50 amino acids to about 520 amino acids, between about 50 amino acids to about 510 amino acids, between about 550 amino acids to about 800 amino acids, between about 550 amino acids to about 790 amino acids, between about 550 amino acids to about 780 amino acids, between about 550 amino acids to about 770 amino acids, between about 550 amino acids to about 760 amino acids, between about 550 amino acids to about 750 amino acids, between about 550 amino acids to about 740 amino acids, between about 550 amino acids to about 730 amino acids, between about 550 amino acids to about 720 amino acids, between about 550 amino acids to about 710 amino acids, between about 550 amino acids to about 700 amino acids, between about 550 amino acids to about 690 amino acids, between about 550 amino acids to about 680 amino acids, between 30 amino acids to about 670 amino acids, between about 550 amino acids to about 660 amino acids, between about 550 amino acids to about 650 amino acids, between about 550 amino acids to about 640 amino acids, between about 30 amino acids to about 630 amino acids, between about 550 amino acids to about 620 amino acids, between about 550 amino acids to about 610 amino acids, between about 550 amino acids to about 600 amino acids, between about 550 amino acids to about 590 amino acids, between about 550 amino acids to about 580 amino acids, between about 550 amino acids to about 570 amino acids, between about 550 amino acids to about 560 amino acids, between about 580 amino acids to about 800 amino acids, between about 580 amino acids to about 790 amino acids, between about 580 amino acids to about 780 amino acids, between about 580 amino acids to about 770 amino acids, between about 580 amino acids to about 760 amino acids, between about 580 amino acids to about 750 amino acids, between about 580 amino acids to about 740 amino acids, between about 580 amino acids to about 730 amino acids, between about 580 amino acids to about 720 amino acids, between about 580 amino acids to about 710 amino acids, between about 580 amino acids to about 700 amino acids, between about 580 amino acids to about 690 amino acids, between about 580 amino acids to about 680 amino acids, between 30 amino acids to about 670 amino acids, between about 580 amino acids to about 660 amino acids, between about 580 amino acids to about 650 amino acids, between about 580 amino acids to about 640 amino acids, between about 30 amino acids to about 630 amino acids, between about 580 amino acids to about 620 amino acids, between about 580 amino acids to about 610 amino acids, between about 580 amino acids to about 600 amino acids, between about 580 amino acids to about 590 amino acids, between about 600 amino acids to about 800 amino acids, between about 600 amino acids to about 790 amino acids, between about 600 amino acids to about 780 amino acids, between about 600 amino acids to about 770 amino acids, between about 600 amino acids to about 760 amino acids, between about 600 amino acids to about 750 amino acids, between about 600 amino acids to about 740 amino acids, between about 600 amino acids to about 730 amino acids, between about 600 amino acids to about 720 amino acids, between about 600 amino acids to about 710 amino acids, between about 600 amino acids to about 700 amino acids, between about 600 amino acids to about 690 amino acids, between about 600 amino acids to about 680 amino acids, between 30 amino acids to about 670 amino acids, between about 600 amino acids to about 660 amino acids, between about 600 amino acids to about 650 amino acids, between about 600 amino acids to about 640 amino acids, between about 30 amino acids to about 630 amino acids, between about 600 amino acids to about 620 amino acids, between about 600 amino acids to about 610 amino acids, between about 650 amino acids to about 800 amino acids, between about 650 amino acids to about 790 amino acids, between about 650 amino acids to about 780 amino acids, between about 650 amino acids to about 770 amino acids, between about 650 amino acids to about 760 amino acids, between about 650 amino acids to about 750 amino acids, between about 650 amino acids to about 740 amino acids, between about 650 amino acids to about 730 amino acids, between about 650 amino acids to about 720 amino acids, between about 650 amino acids to about 710 amino acids, between about 650 amino acids to about 700 amino acids, between about 650 amino acids to about 690 amino acids, between about 650 amino acids to about 680 amino acids, between 30 amino acids to about 670 amino acids, between about 650 amino acids to about 660 amino acids, between about 680 amino acids to about 800 amino acids, between about 680 amino acids to about 790 amino acids, between about 680 amino acids to about 780 amino acids, between about 680 amino acids to about 770 amino acids, between about 680 amino acids to about 760 amino acids, between about 680 amino acids to about 750 amino acids, between about 680 amino acids to about 740 amino acids, between about 680 amino acids to about 730 amino acids, between about 680 amino acids to about 720 amino acids, between about 680 amino acids to about 710 amino acids, between about 680 amino acids to about 700 amino acids, between about 680 amino acids to about 690 amino acids, between about 700 amino acids to about 800 amino acids, between about 700 amino acids to about 790 amino acids, between about 700 amino acids to about 780 amino acids, between about 700 amino acids to about 770 amino acids, between about 700 amino acids to about 760 amino acids, between about 700 amino acids to about 750 amino acids, between about 700 amino acids to about 740 amino acids, between about 700 amino acids to about 730 amino acids, between about 700 amino acids to about 720 amino acids, between about 700 amino acids to about 710 amino acids, between about 750 amino acids to about 800 amino acids, between about 750 amino acids to about 790 amino acids, between about 750 amino acids to about 780 amino acids, between about 750 amino acids to about 770 amino acids, between about 750 amino acids to about 760 amino acids, or between about 780 amino acids to about 800 amino acids) in length. In some examples, the second vector further includes a poly(A) sequence.

In some examples where the number of different vectors in the composition is two, the N-terminal portion encoded by one of the two vectors can include a portion including amino acid position 1 to about amino acid position 800, about amino acid position 790, about amino acid position 780, about amino acid position 770, about amino acid position 760, about amino acid position 750, about amino acid position 740, about amino acid position 730, about amino acid position 720, about amino acid position 710, about amino acid position 700, about amino acid position 690, about amino acid position 680, about amino acid position 670, about amino acid position 660, about amino acid position 650, about amino acid position 640, about amino acid position 630, about amino acid position 620, about amino acid position 610, about amino acid position 600, about amino acid position 590, about amino acid position 580, about amino acid position 570, about amino acid position 560, about amino acid position 550, about amino acid position 540, about amino acid position 530, about amino acid position 520, about amino acid position 510, about amino acid position 500, about amino acid position 490, about amino acid position 480, about amino acid position 470, about amino acid position 460, about amino acid position 450, about amino acid position 440, about amino acid position 430, about amino acid position 420, about amino acid position 410, about amino acid position 400, about amino acid position 390, about amino acid position 380, about amino acid position 370, about amino acid position 360, about amino acid position 350, about amino acid position 340, about amino acid position 330, about amino acid position 320, about amino acid position 310, about amino acid position 300, about amino acid position 290, about amino acid position 280, about amino acid position 270, about amino acid position 260, about amino acid position 250, about amino acid position 240, about amino acid position 230, about amino acid position 220, about amino acid position 210, about amino acid position 200, about amino acid position 190, about amino acid position 180, about amino acid position 170, about amino acid position 160, about amino acid position 150, about amino acid position 140, about amino acid position 130, about amino acid position 120, about amino acid position 110, about amino acid position 100, about amino acid position 90, about amino acid position 80, about amino acid position 70, about amino acid position 60, about amino acid position 50, or about amino acid position 40 of a wildtype hair cell target protein (e.g., SEQ ID NO: 8, SEQ ID NO: 9, SEQ ID NO: 10, SEQ ID NO: 11, SEQ ID NO: 12, SEQ ID NO: 20, SEQ ID NO: 23, SEQ ID NO: 24, SEQ ID NO: 25, SEQ ID NO: 26, SEQ ID NO: 27, SEQ ID NO: 28, SEQ ID NO: 30, SEQ ID NO: 31, SEQ ID NO: 32, SEQ ID NO: 34, SEQ ID NO: 39, SEQ ID NO: 41, SEQ ID NO: 43, SEQ ID NO: 45, SEQ ID NO: 48, SEQ ID NO: 51, SEQ ID NO: 53, SEQ ID NO: 55, SEQ ID NO: 57, SEQ ID NO: 59, SEQ ID NO: 61, SEQ ID NO: 64, SEQ ID NO: 66, SEQ ID NO: 68, SEQ ID NO: 69, SEQ ID NO: 70, SEQ ID NO: 72, SEQ ID NO: 74, SEQ ID NO: 76, SEQ ID NO: 77, SEQ ID NO: 78, or SEQ ID NO: 79).

In some examples where the number of different vectors in the composition is two, the N-terminal portion of the precursor hair cell target protein can include a portion including amino acid position 1 to amino acid position 300, amino acid position 1 to amino acid position 310, amino acid position 1 to about amino acid position 320, amino acid position 1 to about amino acid position 330, amino acid position 1 to about amino acid position 340, amino acid position 1 to about amino acid position 350, amino acid position 1 to about amino acid position 360, amino acid position 1 to about amino acid position 370, amino acid position 1 to about amino acid position 380, amino acid position 1 to about amino acid position 390, amino acid position 1 to about amino acid position 400, amino acid position 1 to about amino acid position 410, amino acid position 1 to about amino acid position 420, amino acid position 1 to about amino acid position 430, amino acid position 1 to about amino acid position 440, amino acid position 1 to about amino acid position 450, amino acid position 1 to about amino acid position 460, amino acid position 1 to about amino acid position 470, amino acid position 1 to about amino acid position 480, amino acid position 1 to about amino acid position 490, amino acid position 1 to about amino acid position 500, amino acid position 1 to about amino acid position 510, amino acid position 1 to about amino acid position 520, amino acid position 1 to about amino acid position 530, amino acid position 1 to about amino acid position 540, amino acid position 1 to about amino acid position 550, amino acid position 1 to about amino acid position 560, amino acid position 1 to about amino acid position 570, amino acid position 1 to about amino acid position 580, amino acid position 1 to about amino acid position 590, amino acid position 1 to about amino acid position 600, amino acid position 1 to about amino acid position 610, amino acid position 1 to about amino acid position 620, amino acid position 1 to about amino acid position 630, amino acid position 1 to about amino acid position 640, amino acid position 1 to about amino acid position 650, amino acid position 1 to about amino acid position 660, amino acid position 1 to about amino acid position 670, amino acid position 1 to about amino acid position 680, amino acid position 1 to about amino acid position 690, amino acid position 1 to about amino acid position 700, amino acid position 1 to about amino acid position 710, amino acid position 1 to about amino acid position 720, amino acid position 1 to about amino acid position 730, amino acid position 1 to about amino acid position 740, amino acid position 1 to about amino acid position 750, amino acid position 1 to about amino acid position 760, amino acid position 1 to about amino acid position 770, amino acid position 1 to about amino acid position 780, amino acid position 1 to about amino acid position 790, amino acid position 1 to about amino acid position 800 of a wildtype hair cell target protein (e.g., SEQ ID NO: 8, SEQ ID NO: 9, SEQ ID NO: 10, SEQ ID NO: 11, SEQ ID NO: 12, SEQ ID NO: 20, SEQ ID NO: 23, SEQ ID NO: 24, SEQ ID NO: 25, SEQ ID NO: 26, SEQ ID NO: 27, SEQ ID NO: 28, SEQ ID NO: 30, SEQ ID NO: 31, SEQ ID NO: 32, SEQ ID NO: 34, SEQ ID NO: 35, SEQ ID NO: 41, SEQ ID NO: 43, SEQ ID NO: 45, SEQ ID NO: 48, SEQ ID NO: 51, SEQ ID NO: 53, SEQ ID NO: 55, SEQ ID NO: 57, SEQ ID NO: 59, SEQ ID NO: 61, SEQ ID NO: 64, SEQ ID NO: 66, SEQ ID NO: 68, SEQ ID NO: 69, SEQ ID NO: 70, SEQ ID NO: 72, SEQ ID NO: 74, SEQ ID NO: 76, SEQ ID NO: 77, SEQ ID NO: 78, SEQ ID NO: 79).

In some examples where the number of different vectors in the composition is two, the N-terminal portion encoded by one of the two vectors can include a portion including amino acid position 1 to about amino acid position 800, about amino acid position 790, about amino acid position 780, about amino acid position 770, about amino acid position 760, about amino acid position 750, about amino acid position 740, about amino acid position 730, about amino acid position 720, about amino acid position 710, about amino acid position 700, about amino acid position 690, about amino acid position 680, about amino acid position 670, about amino acid position 660, about amino acid position 650, about amino acid position 640, about amino acid position 630, about amino acid position 620, about amino acid position 610, about amino acid position 600, about amino acid position 590, about amino acid position 580, about amino acid position 570, about amino acid position 560, about amino acid position 550, about amino acid position 540, about amino acid position 530, about amino acid position 520, about amino acid position 510, about amino acid position 500, about amino acid position 490, about amino acid position 480, about amino acid position 470, about amino acid position 460, about amino acid position 450, about amino acid position 440, about amino acid position 430, about amino acid position 420, about amino acid position 410, about amino acid position 400, about amino acid position 390, about amino acid position 380, about amino acid position 370, about amino acid position 360, about amino acid position 350, about amino acid position 340, about amino acid position 330, about amino acid position 320, about amino acid position 310, about amino acid position 300, about amino acid position 290, about amino acid position 280, about amino acid position 270, about amino acid position 260, about amino acid position 250, about amino acid position 240, about amino acid position 230, about amino acid position 220, about amino acid position 210, about amino acid position 200, about amino acid position 190, about amino acid position 180, about amino acid position 170, about amino acid position 160, about amino acid position 150, about amino acid position 140, about amino acid position 130, about amino acid position 120, about amino acid position 110, about amino acid position 100, about amino acid position 90, about amino acid position 80, about amino acid position 70, about amino acid position 60, about amino acid position 50, or about amino acid position 40 of a wildtype supporting cell target protein (e.g., SEQ ID NO: 1 or SEQ ID NO: 4).

In some examples where the number of different vectors in the composition is two, the N-terminal portion of the precursor supporting cell target protein can include a portion including amino acid position 1 to amino acid position 300, amino acid position 1 to amino acid position 310, amino acid position 1 to about amino acid position 320, amino acid position 1 to about amino acid position 330, amino acid position 1 to about amino acid position 340, amino acid position 1 to about amino acid position 350, amino acid position 1 to about amino acid position 360, amino acid position 1 to about amino acid position 370, amino acid position 1 to about amino acid position 380, amino acid position 1 to about amino acid position 390, amino acid position 1 to about amino acid position 400, amino acid position 1 to about amino acid position 410, amino acid position 1 to about amino acid position 420, amino acid position 1 to about amino acid position 430, amino acid position 1 to about amino acid position 440, amino acid position 1 to about amino acid position 450, amino acid position 1 to about amino acid position 460, amino acid position 1 to about amino acid position 470, amino acid position 1 to about amino acid position 480, amino acid position 1 to about amino acid position 490, amino acid position 1 to about amino acid position 500, amino acid position 1 to about amino acid position 510, amino acid position 1 to about amino acid position 520, amino acid position 1 to about amino acid position 530, amino acid position 1 to about amino acid position 540, amino acid position 1 to about amino acid position 550, amino acid position 1 to about amino acid position 560, amino acid position 1 to about amino acid position 570, amino acid position 1 to about amino acid position 580, amino acid position 1 to about amino acid position 590, amino acid position 1 to about amino acid position 600, amino acid position 1 to about amino acid position 610, amino acid position 1 to about amino acid position 620, amino acid position 1 to about amino acid position 630, amino acid position 1 to about amino acid position 640, amino acid position 1 to about amino acid position 650, amino acid position 1 to about amino acid position 660, amino acid position 1 to about amino acid position 670, amino acid position 1 to about amino acid position 680, amino acid position 1 to about amino acid position 690, amino acid position 1 to about amino acid position 700, amino acid position 1 to about amino acid position 710, amino acid position 1 to about amino acid position 720, amino acid position 1 to about amino acid position 730, amino acid position 1 to about amino acid position 740, amino acid position 1 to about amino acid position 750, amino acid position 1 to about amino acid position 760, amino acid position 1 to about amino acid position 770, amino acid position 1 to about amino acid position 780, amino acid position 1 to about amino acid position 790, amino acid position 1 to about amino acid position 800 of a wildtype supporting cell target protein (e.g., SEQ ID NO: 1 or SEQ ID NO: 4).

As used herein, the term "vector" means a composition including a polynucleotide capable of carrying at least one exogenous nucleic acid fragment, e.g., a plasmid vector, a transposon, a cosmid, an artificial chromosome (e.g., a human artificial chromosome (HAC), a yeast artificial chromosome (YAC), a bacterial artificial chromosome (BAC), or a PI-derived artificial chromosome (PAC)) or a viral vector (e.g., any adenoviral vectors (e.g., pSV or pCMV vectors), any retroviral vectors as described herein) and any Gateway^{®} vectors. A vector can, e.g., include sufficient cis-acting elements for expression; other elements for expression can be supplied by the host primate cell or in an *in vitro* expression system. The term "vector" includes any genetic element (e.g., a plasmid, a transposon, a cosmid, an artificial chromosome, or a viral vector, etc.) that is capable of replicating when associated with the proper control elements. Thus, the term includes cloning and expression vectors, as well as viral vectors (e.g., an adeno-associated virus (AAV) vector, an adenovirus vector, a lentivirus vector, or a retrovirus vector).

Vectors include all those known in the art, including cosmids, plasmids (e.g., naked or contained in liposomes) and viruses (e.g., lentiviruses, retroviruses, adenoviruses, and adeno-associated viruses) that incorporate the recombinant polynucleotide. Skilled practitioners will be capable of selecting suitable vectors and primate cells for making any of the nucleic acids described herein.

In some embodiments the vector is a plasmid (i.e. a circular DNA molecule that can autonomously replicate inside a cell). In some embodiments, the vector can be a cosmid (e.g., pWE and sCos series (Wahl et al. (1987), Evans et al. (1989)).

In some embodiments, the vector(s) is a viral vector (e.g., adeno-associated virus, adenovirus, lentivirus, and retrovirus). Non-limiting examples of viral vectors are described herein. In some embodiments, the vector(s) is an adeno-associated viral vector (AAV) (see, e.g., Asokan et al., Mo/. Ther. 20: 699-7080, 2012). "Recombinant AAV vectors" or "rAAVs" are typically composed of, at a minimum, a transgene or a portion thereof and a regulatory sequence, and optionally 5' and 3' AAV inverted terminal repeats (ITRs). Such a recombinant AAV vector is packaged into a capsid and delivered to a selected target cell (e.g., an outer hair cell).

The AAV sequences of the vector typically include the cis-acting 5' and 3' ITR sequences (See, e.g., B. J. Carter, in "Handbook of Parvoviruses", ed., P. Tijsser, CRC Press, pp. 155 168, 1990). Typical AAV ITR sequences are about 145 nucleotides in length. In some embodiments, at least 75% of a typical ITR sequence (e.g., at least 80%, at least 85%, at least 90%, or at least 95%) is incorporated into the AAV vector. The ability to modify these ITR sequences is within the skill of the art. (See, e.g., texts such as Sambrook et al., "Molecular Cloning. A Laboratory Manual", 2d ed., Cold Spring Harbor Laboratory, New York, 1989; and K. Fisher et al., J Virol. 70:520 532, 1996). In some embodiments, any of the coding sequences described herein are flanked by 5' and 3' AAV ITR sequences in the AAV vectors. The AAV ITR sequences may be obtained from any known AAV, including presently identified AAV types.

AAV vectors as described herein may include any of the regulatory elements described herein (e.g., one or more of a promoter, a polyA sequence, and an IRES).

In some embodiments, the AAV vector is selected from the group consisting of: an AAV1 vector, an AAV2 vector, an AAV3 vector, an AAV4 vector, an AAV5 vector, an AAV6 vector, an AAV7 vector, an AAV8 vector, an AAV9 vector, an AAV2.7m8 vector, an AAV8BP2 vector, and an AAV293 vector. Additional exemplary AAV vectors that can be used herein are known in the art. See, e.g., Kanaan et al., Mol. Ther. Nucleic Acids 8:184-197, 2017; Li et al., Mol. Ther. 16(7): 1252-1260; Adachi et al., Nat. Commun. 5: 3075, 2014; Isgrig et al., Nat. Commun. 10(1): 427, 2019; and Gao et al., J. Virol. 78(12): 6381-6388.

In some embodiments, the vector(s) is an adenovirus (see, e.g., Dmitriev et al. (1998) J. Virol. 72: 9706-9713; and Poulin et al., J. Virol 8: 10074-10086, 2010). In some embodiments, the vector(s) is a retrovirus (see, e.g., Maier et al. (2010) Future Microbiol 5: 1507-23).

The vectors provided herein can be of different sizes. The choice of vector that is used in any of the compositions, kits, and methods described herein may depend on the size of the vector.

In some embodiments, the vector(s) is a plasmid and can include a total length of up to about 1 kb, up to about 2 kb, up to about 3 kb, up to about 4 kb, up to about 5 kb, up to about 6 kb, up to about 7 kb, up to about 8kb, up to about 9 kb, up to about 10 kb, up to about 11 kb, up to about 12 kb, up to about 13 kb, up to about 14 kb, or up to about 15 kb. In some embodiments, the vector(s) is a plasmid and can have a total length in a range of about 1 kb to about 2 kb, about 1 kb to about 3 kb, about 1 kb to about 4 kb, about 1 kb to about 5 kb, about 1 kb to about 6 kb, about 1 kb to about 7 kb, about 1 kb to about 8 kb, about 1 kb to about 9 kb, about 1 kb to about 10 kb, about 1 kb to about 11 kb, about 1 kb to about 12 kb, about 1 kb to about 13 kb, about 1 kb to about 14 kb, or about 1 kb to about 15 kb.

In some embodiments, the vector(s) is a viral vector and can have a total number of nucleotides of up to 10 kb. In some embodiments, the viral vector(s) can have a total number of nucleotides in the range of about 1 kb to about 2 kb, 1 kb to about 3 kb, about 1 kb to about 4 kb, about 1 kb to about 5 kb, about 1 kb to about 6 kb, about 1 kb to about 7 kb, about 1 kb to about 8 kb, about 1 kb to about 9 kb, about 1 kb to about 10 kb, about 2 kb to about 3 kb, about 2 kb to about 4 kb, about 2 kb to about 5 kb, about 2 kb to about 6 kb, about 2 kb to about 7 kb, about 2 kb to about 8 kb, about 2 kb to about 9 kb, about 2 kb to about 10 kb, about 3 kb to about 4 kb, about 3 kb to about 5 kb, about 3 kb to about 6 kb, about 3 kb to about 7 kb, about 3 kb to about 8 kb, about 3 kb to about 9 kb, about 3 kb to about 10 kb, about 4 kb to about 5 kb, about 4 kb to about 6 kb, about 4 kb to about 7 kb, about 4 kb to about 8 kb, about 4 kb to about 9 kb, about 4 kb to about 10 kb, about 5 kb to about 6 kb, about 5 kb to about 7 kb, about 5 kb to about 8 kb, about 5 kb to about 9 kb, about 5 kb to about 10 kb, about 6 kb to about 7 kb, about 6 kb to about 8 kb, about 6 kb to about 9 kb, about 6 kb to about 10 kb, about 7 kb to about 8 kb, about 7 kb to about 9 kb, about 7 kb to about 10 kb, about 8 kb to about 9 kb, about 8 kb to about 10 kb, or about 9 kb to about 10 kb.

In some embodiments, the vector(s) is a lentivirus and can have a total number of nucleotides of up to 8 kb. In some examples, the lentivirus(es) can have a total number of nucleotides of about 1 kb to about 2 kb, about 1 kb to about 3 kb, about 1 kb to about 4 kb, about 1 kb to about 5 kb, about 1 kb to about 6 kb, about 1 kb to about 7 kb, about 1 kb to about 8 kb, about 2 kb to about 3 kb, about 2 kb to about 4 kb, about 2 kb to about 5 kb, about 2 kb to about 6 kb, about 2 kb to about 7 kb, about 2 kb to about 8 kb, about 3 kb to about 4 kb, about 3 kb to about 5 kb, about 3 kb to about 6 kb, about 3 kb to about 7 kb, about 3 kb to about 8 kb, about 4 kb to about 5 kb, about 4 kb to about 6 kb, about 4 kb to about 7 kb, about 4 kb to about 8 kb, about 5 kb to about 6 kb, about 5 kb to about 7 kb, about 5 kb to about 8 kb, about 6 kb to about 8kb, about 6 kb to about 7 kb, or about 7 kb to about 8 kb.

In some embodiments, the vector(s) is an adenovirus and can have a total number of nucleotides of up to 8 kb. In some embodiments, the adenovirus(es) can have a total number of nucleotides in the range of about 1 kb to about 2 kb, about 1 kb to about 3 kb, about 1 kb to about 4 kb, about 1 kb to about 5 kb, about 1 kb to about 6 kb, about 1 kb to about 7 kb, about 1 kb to about 8 kb, about 2 kb to about 3 kb, about 2 kb to about 4 kb, about 2 kb to about 5 kb, about 2 kb to about 6 kb, about 2 kb to about 7 kb, about 2 kb to about 8 kb, about 3 kb to about 4 kb, about 3 kb to about 5 kb, about 3 kb to about 6 kb, about 3 kb to about 7 kb, about 3 kb to about 8 kb, about 4 kb to about 5 kb, about 4 kb to about 6 kb, about 4 kb to about 7 kb, about 4 kb to about 8 kb, about 5 kb to about 6 kb, about 5 kb to about 7 kb, about 5 kb to about 8 kb, about 6 kb to about 7 kh, about 6 kb to about 8 kb, or about 7 kb to about 8 kb.

In some embodiments, the vector(s) is an adeno-associated virus (AAV vector) and can include a total number of nucleotides of up to 5 kb. In some embodiments, the AAV vector(s) can include a total number of nucleotides in the range of about 1 kb to about 2 kb, about 1 kb to about 3 kb, about 1 kb to about 4 kb, about 1 kb to about 5 kb, about 2 kb to about 3 kb, about 2 kb to about 4 kb, about 2 kb to about 5kb, about 3 kb to about 4 kb, about 3 kb to about 5 kb, or about 4 kb to about 5 kb.

In some embodiments of any of the compositions, kits, and methods provided herein, the at least two different vectors can be substantially the same type of vector and may differ in size. In some embodiments, the at least two different vectors can be different types of vector, and may have substantially the same size or have different sizes.

In some embodiments, any of the at least two vectors can have a total number of nucleotides in the range of about 500 nucleotides to about 10,000 nucleotides, about 500 nucleotides to about 9,500 nucleotides, about 500 nucleotides to about 9,000 nucleotides, about 500 nucleotides to about 8,500 nucleotides, about 500 nucleotides to about 8,000 nucleotides, about 500 nucleotides to about 7,800 nucleotides, about 500 nucleotides to about 7,600 nucleotides, about 500 nucleotides to about 7,400 nucleotides, about 500 nucleotides to about 7,200 nucleotides, about 500 nucleotides to about 7,000 nucleotides, about 500 nucleotides to about 6,800 nucleotides, about 500 nucleotides to about 6,600 nucleotides, about 500 nucleotides to about 6,400 nucleotides, about 500 nucleotides to about 6,200 nucleotides, about 500 nucleotides to about 6,000 nucleotides, about 500 nucleotides to about 5,800 nucleotides, about 500 nucleotides to about 5,600 nucleotides, about 500 nucleotides to about 5,400 nucleotides, about 500 nucleotides to about 5,200 nucleotides, about 500 nucleotides to about 5,000 nucleotides, about 500 nucleotides to about 4,800 nucleotides, about 4,600 nucleotides, about 500 nucleotides to about 4,400 nucleotides, about 500 nucleotides to about 4,200 nucleotides, about 500 nucleotides to about 4,000 nucleotides, about 500 nucleotides to about 3,800 nucleotides, about 500 nucleotides to about 3,600 nucleotides, about 500 nucleotides to about 3,400 nucleotides, about 500 nucleotides to about 3,200 nucleotides, about 500 nucleotides to about 3,000 nucleotides, about 500 nucleotides to about 2,800 nucleotides, about 500 nucleotides to about 2,600 nucleotides, about 500 nucleotides to about 2,400 nucleotides, about 500 nucleotides to about 2,200 nucleotides, about 500 nucleotides to about 2,000 nucleotides, about 500 nucleotides to about 1,800 nucleotides, about 500 nucleotides to about 1,600 nucleotides, about 500 nucleotides to about 1,400 nucleotides, about 500 nucleotides to about 1,200 nucleotides, about 500 nucleotides to about 1,000 nucleotides, about 500 nucleotides to about 800 nucleotides, about 800 nucleotides to about 10,000 nucleotides, about 800 nucleotides to about 9,500 nucleotides, about 800 nucleotides to about 9,000 nucleotides, about 800 nucleotides to about 8,500 nucleotides, about 800 nucleotides to about 8,000 nucleotides, about 800 nucleotides to about 7,800 nucleotides, about 800 nucleotides to about 7,600 nucleotides, about 800 nucleotides to about 7,400 nucleotides, about 800 nucleotides to about 7,200 nucleotides, about 800 nucleotides to about 7,000 nucleotides, about 800 nucleotides to about 6,800 nucleotides, about 800 nucleotides to about 6,600 nucleotides, about 800 nucleotides to about 6,400 nucleotides, about 800 nucleotides to about 6,200 nucleotides, about 800 nucleotides to about 6,000 nucleotides, about 800 nucleotides to about 5,800 nucleotides, about 800 nucleotides to about 5,600 nucleotides, about 800 nucleotides to about 5,400 nucleotides, about 800 nucleotides to about 5,200 nucleotides, about 800 nucleotides to about 5,000 nucleotides, about 800 nucleotides to about 4,800 nucleotides, about 800 nucleotides to about 4,600 nucleotides, about 800 nucleotides to about 4,400 nucleotides, about 800 nucleotides to about 4,200 nucleotides, about 800 nucleotides to about 4,000 nucleotides, about 800 nucleotides to about 3,800 nucleotides, about 800 nucleotides to about 3,600 nucleotides, about 800 nucleotides to about 3,400 nucleotides, about 800 nucleotides to about 3,200 nucleotides, about 800 nucleotides to about 3,000 nucleotides, about 800 nucleotides to about 2,800 nucleotides, about 800 nucleotides to about 2,600 nucleotides, about 800 nucleotides to about 2,400 nucleotides, about 800 nucleotides to about 2,200 nucleotides, about 800 nucleotides to about 2,000 nucleotides, about 800 nucleotides to about 1,800 nucleotides, about 800 nucleotides to about 1,600 nucleotides, about 800 nucleotides to about 1,400 nucleotides, about 800 nucleotides to about 1,200 nucleotides, about 800 nucleotides to about 1,000 nucleotides, about 1,000 nucleotides to about 10,000 nucleotides, about 1,000 nucleotides to about 9,000 nucleotides, about 1,000 nucleotides to about 8,500 nucleotides, about 1,000 nucleotides to about 8,000 nucleotides, about 1,000 nucleotides to about 7,800 nucleotides, about 1,000 nucleotides to about 7,600 nucleotides, about 1,000 nucleotides to about 7,400 nucleotides, about 1,000 nucleotides to about 7,200 nucleotides, about 1,000 nucleotides to about 7,000 nucleotides, about 1,000 nucleotides to about 6,800 nucleotides, about 1,000 nucleotides to about 6,600 nucleotides, about 1,000 nucleotides to about 6,400 nucleotides, about 1,000 nucleotides to about 6,200 nucleotides, about 1,000 nucleotides to about 6,000 nucleotides, about 1,000 nucleotides to about 5,800 nucleotides, about 1,000 nucleotides to about 5,600 nucleotides, about 1,000 nucleotides to about 5,400 nucleotides, about 1,000 nucleotides to about 5,200 nucleotides, about 1,000 nucleotides to about 5,000 nucleotides, about 1,000 nucleotides to about 4,800 nucleotides, about 1,000 nucleotides to about 4,600 nucleotides, about 1,000 nucleotides to about 4,400 nucleotides, about 1,000 nucleotides to about 4,200 nucleotides, about 1,000 nucleotides to about 4,000 nucleotides, about 1,000 nucleotides to about 3,800 nucleotides, about 1,000 nucleotides to about 3,600 nucleotides, about 1,000 nucleotides to about 3,400 nucleotides, about 1,000 nucleotides to about 3,200 nucleotides, about 1,000 nucleotides to about 3,000 nucleotides, about 1,000 nucleotides to about 2,600 nucleotides, about 1,000 nucleotides to about 2,400 nucleotides, about 1,000 nucleotides to about 2,200 nucleotides, about 1,000 nucleotides to about 2,000 nucleotides, about 1,000 nucleotides to about 1,800 nucleotides, about 1,000 nucleotides to about 1,600 nucleotides, about 1,000 nucleotides to about 1,400 nucleotides, about 1,000 nucleotides to about 1,200 nucleotides, about 1,200 nucleotides to about 10,000 nucleotides, about 1,200 nucleotides to about 9,500 nucleotides, about 1,200 nucleotides to about 9,000 nucleotides, about 1,200 nucleotides to about 8,500 nucleotides, about 1,200 nucleotides to about 8,000 nucleotides, about 1,200 nucleotides to about 7,800 nucleotides, about 1,200 nucleotides to about 7,600 nucleotides, about 1,200 nucleotides to about 7,400 nucleotides, about 1,200 nucleotides to about 7,200 nucleotides, about 1,200 nucleotides to about 7,000 nucleotides, about 1,200 nucleotides to about 6,800 nucleotides, about 1,200 nucleotides to about 6,600 nucleotides, about 1,200 nucleotides to about 6,400 nucleotides, about 1,200 nucleotides to about 6,200 nucleotides, about 1,200 nucleotides to about 6,000 nucleotides, about 1,200 nucleotides to about 5,800 nucleotides, about 1,200 nucleotides to about 5,600 nucleotides, about 1,200 nucleotides to about 5,400 nucleotides, about 1,200 nucleotides to about 5,000 nucleotides, about 1,200 nucleotides to about 4,800 nucleotides, about 1,200 nucleotides to about 4,600 nucleotides, about 1,200 nucleotides to about 4,400 nucleotides, about 1,200 nucleotides to about 4,200 nucleotides, about 1,200 nucleotides to about 4,000 nucleotides, about 1,200 nucleotides to about 3,800 nucleotides, about 1,200 nucleotides to about 3,600 nucleotides, about 1,200 nucleotides to about 3,400 nucleotides, about 1,200 nucleotides to about 3,200 nucleotides, about 1,200 nucleotides to about 3,000 nucleotides, about 1,200 nucleotides to about 2,800 nucleotides, about 1,200 nucleotides to about 2,600 nucleotides, about 1,200 nucleotides to about 2,400 nucleotides, about 1,200 nucleotides to about 2,200 nucleotides, about 1,200 nucleotides to about 2,000 nucleotides, about 1,200 nucleotides to about 1,800 nucleotides, about 1,200 nucleotides to about 1,600 nucleotides, about 1,200 nucleotides to about 1,400 nucleotides, about 1,400 nucleotides to about 10,000 nucleotides, about 1,400 nucleotides to about 9,500 nucleotides, about 1,400 nucleotides to about 9,000 nucleotides, about 1,400 nucleotides to about 8,500 nucleotides, about 1,400 nucleotides to about 8,000 nucleotides, about 1,400 nucleotides to about 7,800 nucleotides, about 1,400 nucleotides to about 7,600 nucleotides, about 1,400 nucleotides to about 7,400 nucleotides, about 1,400 nucleotides to about 7,200 nucleotides, about 1,400 nucleotides to about 7,000 nucleotides, about 1,400 nucleotides to about 6,800 nucleotides, about 1,400 nucleotides to about 6,600 nucleotides, about 1,400 nucleotides to about 6,400 nucleotides, about 1,400 nucleotides to about 6,200 nucleotides, about 1,400 nucleotides to about 6,000 nucleotides, about 1,400 nucleotides to about 5,800 nucleotides, about 1,400 nucleotides to about 5,600 nucleotides, about 1,400 nucleotides to about 5,400 nucleotides, about 1,400 nucleotides to about 5,200 nucleotides, about 1,400 nucleotides to about 5,000 nucleotides, about 1,400 nucleotides to about 4,800 nucleotides, about 1,400 nucleotides to about 4,600 nucleotides, about 1,400 nucleotides to about 4,400 nucleotides, about 1,400 nucleotides to about 4,200 nucleotides, about 1,400 nucleotides to about 4,000 nucleotides, about 1,400 nucleotides to about 3,800 nucleotides, about 1,400 nucleotides to about 3,600 nucleotides, about 1,400 nucleotides to about 3,400 nucleotides, about 1,400 nucleotides to about 3,200 nucleotides, about 1,400 nucleotides to about 3,000 nucleotides, about 1,400 nucleotides to about 2,600 nucleotides, about 1,400 nucleotides to about 2,400 nucleotides, about 1,400 nucleotides to about 2,200 nucleotides, about 1,400 nucleotides to about 2,000 nucleotides, about 1,400 nucleotides to about 1,800 nucleotides, about 1,400 nucleotides to about 1,600 nucleotides, about 1,600 nucleotides to about 10,000 nucleotides, about 1,600 nucleotides to about 9,500 nucleotides, about 1,600 nucleotides to about 9,000 nucleotides, about 1,600 nucleotides to about 8,500 nucleotides, about 1,600 nucleotides to about 8,000 nucleotides, about 1,600 nucleotides to about 7,800 nucleotides, about 1,600 nucleotides to about 7,600 nucleotides, about 1,600 nucleotides to about 7,400 nucleotides, about 1,600 nucleotides to about 7,200 nucleotides, about 1,600 nucleotides to about 7,000 nucleotides, about 1,600 nucleotides to about 6,800 nucleotides, about 1,600 nucleotides to about 6,400 nucleotides, about 1,600 nucleotides to about 6,200 nucleotides, about 1,600 nucleotides to about 6,000 nucleotides, about 1,600 nucleotides to about 5,800 nucleotides, about 1,600 nucleotides to about 5,600 nucleotides, about 1,600 nucleotides to about 5,400 nucleotides, about 1,600 nucleotides to about 5,200 nucleotides, about 1,600 nucleotides to about 5,000 nucleotides, about 1,600 nucleotides to about 4,800 nucleotides, about 1,600 nucleotides to about 4,600 nucleotides, about 1,600 nucleotides to about 4,400 nucleotides, about 1,600 nucleotides to about 4,200 nucleotides, about 1,600 nucleotides to about 4,000 nucleotides, about 1,600 nucleotides to about 3,800 nucleotides, about 1,600 nucleotides to about 3,600 nucleotides, about 1,600 nucleotides to about 3,400 nucleotides, about 1,600 nucleotides to about 3,200 nucleotides, about 1,600 nucleotides to about 3,000 nucleotides, about 1,600 nucleotides to about 2,800 nucleotides, about 1,600 nucleotides to about 2,600 nucleotides, about 1,600 nucleotides to about 2,400 nucleotides, about 1,600 nucleotides to about 2,200 nucleotides, about 1,600 nucleotides to about 2,000 nucleotides, about 1,600 nucleotides to about 1,800 nucleotides, about 1,800 nucleotides to about 10,000 nucleotides, about 1,800 nucleotides to about 9,500 nucleotides, about 1,800 nucleotides to about 9,000 nucleotides, about 1,800 nucleotides to about 8,500 nucleotides, about 1,800 nucleotides to about 8,000 nucleotides, about 1,800 nucleotides to about 7,800 nucleotides, about 1,800 nucleotides to about 7,600 nucleotides, about 1,800 nucleotides to about 7,400 nucleotides, about 1,800 nucleotides to about 7,200 nucleotides, about 1,800 nucleotides to about 7,000 nucleotides, about 1,800 nucleotides to about 6,800 nucleotides, about 1,800 nucleotides to about 6,600 nucleotides, about 1,800 nucleotides to about 6,400 nucleotides, about 1,800 nucleotides to about 6,200 nucleotides, about 1,800 nucleotides to about 6,000 nucleotides, about 1,800 nucleotides to about 5,800 nucleotides, about 1,800 nucleotides to about 5,600 nucleotides, about 1,800 nucleotides to about 5,400 nucleotides, about 1,800 nucleotides to about 5,200 nucleotides, about 1,800 nucleotides to about 5,000 nucleotides, about 1,800 nucleotides to about 4,800 nucleotides, about 1,800 nucleotides to about 4,600 nucleotides, about 1,800 nucleotides to about 4,400 nucleotides, about 1,800 nucleotides to about 4,200 nucleotides, about 1,800 nucleotides to about 4,000 nucleotides, about 1,800 nucleotides to about 3,800 nucleotides, about 1,800 nucleotides to about 3,600 nucleotides, about 1,800 nucleotides to about 3,400 nucleotides, about 1,800 nucleotides to about 3,200 nucleotides, about 1,800 nucleotides to about 3,000 nucleotides, about 1,800 nucleotides to about 2,800 nucleotides, about 1,800 nucleotides to about 2,600 nucleotides, about 1,800 nucleotides to about 2,400 nucleotides, about 1,800 nucleotides to about 2,200 nucleotides, about 1,800 nucleotides to about 2,000 nucleotides, about 2,000 nucleotides to about 10,000 nucleotides, about 2,000 nucleotides to about 9,500 nucleotides, about 2,000 nucleotides to about 9,000 nucleotides, about 2,000 nucleotides to about 8,500 nucleotides, about 2,000 nucleotides to about 8,000 nucleotides, about 2,000 nucleotides to about 7,800 nucleotides, about 2,000 nucleotides to about 7,600 nucleotides, about 2,000 nucleotides to about 7,400 nucleotides, about 2,000 nucleotides to about 7,200 nucleotides, about 2,000 nucleotides to about 7,000 nucleotides, about 2,000 nucleotides to about 6,800 nucleotides, about 2,000 nucleotides to about 6,600 nucleotides, about 2,000 nucleotides to about 6,400 nucleotides, about 2,000 nucleotides to about 6,200 nucleotides, about 2,000 nucleotides to about 6,000 nucleotides, about 2,000 nucleotides to about 5,800 nucleotides, about 2,000 nucleotides to about 5,600 nucleotides, about 2,000 nucleotides to about 5,400 nucleotides, about 2,000 nucleotides to about 5,200 nucleotides, about 2,000 nucleotides to about 5,000 nucleotides, about 2,000 nucleotides to about 4,800 nucleotides, about 2,000 nucleotides to about 4,600 nucleotides, about 2,000 nucleotides to about 4,400 nucleotides, about 2,000 nucleotides to about 4,200 nucleotides, about 2,000 nucleotides to about 4,000 nucleotides, about 2,000 nucleotides to about 3,800 nucleotides, about 2,000 nucleotides to about 3,600 nucleotides, about 2,000 nucleotides to about 3,400 nucleotides, about 2,000 nucleotides to about 3,200 nucleotides, about 2,000 nucleotides to about 3,000 nucleotides, about 2,000 nucleotides to about 2,800 nucleotides, about 2,000 nucleotides to about 2,600 nucleotides, about 2,000 nucleotides to about 2,400 nucleotides, about 2,000 nucleotides to about 2,200 nucleotides, about 2,200 nucleotides to about 10,000 nucleotides, about 9,500 nucleotides, about 9,000 nucleotides, about 8,500 nucleotides, about 8,000 nucleotides, about 7,800 nucleotides, about 7,600 nucleotides, about 7,400 nucleotides, about 7,200 nucleotides, about 7,000 nucleotides, about 6,800 nucleotides, about 6,600 nucleotides, about 6,400 nucleotides, about 6,200 nucleotides, about 6,000 nucleotides, about 5,800 nucleotides, about 5,600 nucleotides, about 5,400 nucleotides, about 5,200 nucleotides, about 5,000 nucleotides, about 4,800 nucleotides, about 4,600 nucleotides, about 4,400 nucleotides, about 4,200 nucleotides, about 4,000 nucleotides, about 3,800 nucleotides, about 3,600 nucleotides, about 3,400 nucleotides, about 3,200 nucleotides, about 3,000 nucleotides, about 2,800 nucleotides, about 2,600 nucleotides, about 2,400 nucleotides, about 2,400 nucleotides to about 10,000 nucleotides, about 2,400 nucleotides to about 9,500 nucleotides, about 2,400 nucleotides to about 9,000 nucleotides, about 2,400 nucleotides to about 8,500 nucleotides, about 2,400 nucleotides to about 8,000 nucleotides, about 2,400 nucleotides to about 7,800 nucleotides, about 2,400 nucleotides to about 7,600 nucleotides, about 2,400 nucleotides to about 7,400 nucleotides, about 2,400 nucleotides to about 7,200 nucleotides, about 2,400 nucleotides to about 7,000 nucleotides, about 2,400 nucleotides to about 6,800 nucleotides, about 2,400 nucleotides to about 6,600 nucleotides, about 2,400 nucleotides to about 6,400 nucleotides, about 2,400 nucleotides to about 6,200 nucleotides, about 2,400 nucleotides to about 6,000 nucleotides, about 2,400 nucleotides to about 5,800 nucleotides, about 2,400 nucleotides to about 5,600 nucleotides, about 2,400 nucleotides to about 5,400 nucleotides, about 2,400 nucleotides to about 5,200 nucleotides, about 2,400 nucleotides to about 5,000 nucleotides, about 2,400 nucleotides to about 4,800 nucleotides, about 2,400 nucleotides to about 4,600 nucleotides, about 2,400 nucleotides to about 4,400 nucleotides, about 2,400 nucleotides to about 4,200 nucleotides, about 2,400 nucleotides to about 4,000 nucleotides, about 2,400 nucleotides to about 3,800 nucleotides, about 2,400 nucleotides to about 3,600 nucleotides, about 2,400 nucleotides to about 3,400 nucleotides, about 2,400 nucleotides to about 3,200 nucleotides, about 2,400 nucleotides to about 3,000 nucleotides, about 2,400 nucleotides to about 2,800 nucleotides, about 2,400 nucleotides to about 2,600 nucleotides, about 2,600 nucleotides to about 10,000 nucleotides, about 2,600 nucleotides to about 9,500 nucleotides, about 2,600 nucleotides to about 9,000 nucleotides, about 2,600 nucleotides to about 8,500 nucleotides, about 2,600 nucleotides to about 8,000 nucleotides, about 2,600 nucleotides to about 7,800 nucleotides, about 2,600 nucleotides to about 7,600 nucleotides, about 2,600 nucleotides to about 7,400 nucleotides, about 2,600 nucleotides to about 7,200 nucleotides, about 2,600 nucleotides to about 7,000 nucleotides, about 2,600 nucleotides to about 6,800 nucleotides, about 2,600 nucleotides to about 6,600 nucleotides, about 2,600 nucleotides to about 6,400 nucleotides, about 2,600 nucleotides to about 6,200 nucleotides, about 2,600 nucleotides to about 6,000 nucleotides, about 2,600 nucleotides to about 5,800 nucleotides, about 2,600 nucleotides to about 5,600 nucleotides, about 2,600 nucleotides to about 5,400 nucleotides, about 2,600 nucleotides to about 5,200 nucleotides, about 2,600 nucleotides to about 5,000 nucleotides, about 2,600 nucleotides to about 4,800 nucleotides, about 2,600 nucleotides to about 4,600 nucleotides, about 2,600 nucleotides to about 4,400 nucleotides, about 2,600 nucleotides to about 4,200 nucleotides, about 2,600 nucleotides to about 4,000 nucleotides, about 2,600 nucleotides to about 3,800 nucleotides, about 2,600 nucleotides to about 3,600 nucleotides, about 2,600 nucleotides to about 3,400 nucleotides, about 2,600 nucleotides to about 3,200 nucleotides, about 2,600 nucleotides to about 3,000 nucleotides, about 2,600 nucleotides to about 2,800 nucleotides, about 2,800 nucleotides to about 10,000 nucleotides, about 2,800 nucleotides to about 9,500 nucleotides, about 2,800 nucleotides to about 9,000 nucleotides, about 2,800 nucleotides to about 8,500 nucleotides, about 2,800 nucleotides to about 8,000 nucleotides, about 2,800 nucleotides to about 7,800 nucleotides, about 2,800 nucleotides to about 7,600 nucleotides, about 2,800 nucleotides to about 7,400 nucleotides, about 2,800 nucleotides to about 7,200 nucleotides, about 2,800 nucleotides to about 7,000 nucleotides, about 2,800 nucleotides to about 6,800 nucleotides, about 2,800 nucleotides to about 6,600 nucleotides, about 2,800 nucleotides to about 6,400 nucleotides, about 2,800 nucleotides to about 6,200 nucleotides, about 2,800 nucleotides to about 6,000 nucleotides, about 2,800 nucleotides to about 5,800 nucleotides, about 2,800 nucleotides to about 5,600 nucleotides, about 2,800 nucleotides to about 5,400 nucleotides, about 2,800 nucleotides to about 5,200 nucleotides, about 2,800 nucleotides to about 5,000 nucleotides, about 2,800 nucleotides to about 4,800 nucleotides, about 2,800 nucleotides to about 4,600 nucleotides, about 2,800 nucleotides to about 4,400 nucleotides, about 2,800 nucleotides to about 4,200 nucleotides, about 2,800 nucleotides to about 4,000 nucleotides, about 2,800 nucleotides to about 3,800 nucleotides, about 2,800 nucleotides to about 3,600 nucleotides, about 2,800 nucleotides to about 3,400 nucleotides, about 2,800 nucleotides to about 3,200 nucleotides, about 2,800 nucleotides to about 3,000 nucleotides, about 3,000 nucleotides to about 10,000 nucleotides, about 3,000 nucleotides to about 9,500 nucleotides, about 3,000 nucleotides to about 9,000 nucleotides, about 3,000 nucleotides to about 8,500 nucleotides, about 3,000 nucleotides to about 8,000 nucleotides, about 3,000 nucleotides to about 7,800 nucleotides, about 3,000 nucleotides to about 7,600 nucleotides, about 3,000 nucleotides to about 7,400 nucleotides, about 3,000 nucleotides to about 7,200 nucleotides, about 3,000 nucleotides to about 7,000 nucleotides, about 3,000 nucleotides to about 6,800 nucleotides, about 3,000 nucleotides to about 6,600 nucleotides, about 3,000 nucleotides to about 6,400 nucleotides, about 3,000 nucleotides to about 6,200 nucleotides, about 3,000 nucleotides to about 6,000 nucleotides, about 3,000 nucleotides to about 5,800 nucleotides, about 3,000 nucleotides to about 5,600 nucleotides, about 3,000 nucleotides to about 5,400 nucleotides, about 3,000 nucleotides to about 5,200 nucleotides, about 3,000 nucleotides to about 5,000 nucleotides, about 3,000 nucleotides to about 4,800 nucleotides, about 3,000 nucleotides to about 4,600 nucleotides, about 3,000 nucleotides to about 4,400 nucleotides, about 3,000 nucleotides to about 4,200 nucleotides, about 3,000 nucleotides to about 4,000 nucleotides, about 3,000 nucleotides to about 3,800 nucleotides, about 3,000 nucleotides to about 3,600 nucleotides, about 3,000 nucleotides to about 3,400 nucleotides, about 3,000 nucleotides to about 3,200 nucleotides, about 3,200 nucleotides to about 10,000 nucleotides, about 3,200 nucleotides to about 9,500 nucleotides, about 3,200 nucleotides to about 9,000 nucleotides, about 3,200 nucleotides to about 8,500 nucleotides, about 3,200 nucleotides to about 8,000 nucleotides, about 3,200 nucleotides to about 7,800 nucleotides, about 3,200 nucleotides to about 7,600 nucleotides, about 3,200 nucleotides to about 7,400 nucleotides, about 3,200 nucleotides to about 7,200 nucleotides, about 3,200 nucleotides to about 7,000 nucleotides, about 3,200 nucleotides to about 6,800 nucleotides, about 3,200 nucleotides to about 6,600 nucleotides, about 3,200 nucleotides to about 6,400 nucleotides, about 3,200 nucleotides to about 6,200 nucleotides, about 3,200 nucleotides to about 6,000 nucleotides, about 3,200 nucleotides to about 5,800 nucleotides, about 3,200 nucleotides to about 5,600 nucleotides, about 3,200 nucleotides to about 5,400 nucleotides, about 3,200 nucleotides to about 5,200 nucleotides, about 3,200 nucleotides to about 5,000 nucleotides, about 3,200 nucleotides to about 4,800 nucleotides, about 3,200 nucleotides to about 4,600 nucleotides, about 3,200 nucleotides to about 4,400 nucleotides, about 3,200 nucleotides to about 4,200 nucleotides, about 3,200 nucleotides to about 4,000 nucleotides, about 3,200 nucleotides to about 3,800 nucleotides, about 3,200 nucleotides to about 3,600 nucleotides, about 3,200 nucleotides to about 3,400 nucleotides, about 3,400 nucleotides to about 10,000 nucleotides, about 3,400 nucleotides to about 9,500 nucleotides, about 3,400 nucleotides to about 9,000 nucleotides, about 3,400 nucleotides to about 8,500 nucleotides, about 3,400 nucleotides to about 8,000 nucleotides, about 3,400 nucleotides to about 7,800 nucleotides, about 3,400 nucleotides to about 7,600 nucleotides, about 3,400 nucleotides to about 7,400 nucleotides, about 3,400 nucleotides to about 7,200 nucleotides, about 3,400 nucleotides to about 7,000 nucleotides, about 3,400 nucleotides to about 6,800 nucleotides, about 3,400 nucleotides to about 6,600 nucleotides, about 3,400 nucleotides to about 6,400 nucleotides, about 3,400 nucleotides to about 6,200 nucleotides, about 3,400 nucleotides to about 6,000 nucleotides, about 3,400 nucleotides to about 5,800 nucleotides, about 3,400 nucleotides to about 5,600 nucleotides, about 3,400 nucleotides to about 5,400 nucleotides, about 3,400 nucleotides to about 5,200 nucleotides, about 3,400 nucleotides to about 5,000 nucleotides, about 3,400 nucleotides to about 4,800 nucleotides, about 3,400 nucleotides to about 4,600 nucleotides, about 3,400 nucleotides to about 4,400 nucleotides, about 3,400 nucleotides to about 4,200 nucleotides, about 3,400 nucleotides to about 4,000 nucleotides, about 3,400 nucleotides to about 3,800 nucleotides, about 3,400 nucleotides to about 3,600 nucleotides, about 3,600 nucleotides to about 10,000 nucleotides, about 3,600 nucleotides to about 9,500 nucleotides, about 3,600 nucleotides to about 9,000 nucleotides, about 3,600 nucleotides to about 8,500 nucleotides, about 3,600 nucleotides to about 8,000 nucleotides, about 3,600 nucleotides to about 7,800 nucleotides, about 3,600 nucleotides to about 7,600 nucleotides, about 3,600 nucleotides to about 7,400 nucleotides, about 3,600 nucleotides to about 7,200 nucleotides, about 3,600 nucleotides to about 7,000 nucleotides, about 3,600 nucleotides to about 6,800 nucleotides, about 3,600 nucleotides to about 6,600 nucleotides, about 3,600 nucleotides to about 6,400 nucleotides, about 3,600 nucleotides to about 6,200 nucleotides, about 3,600 nucleotides to about 6,000 nucleotides, about 3,600 nucleotides to about 5,800 nucleotides, about 3,600 nucleotides to about 5,600 nucleotides, about 3,600 nucleotides to about 5,400 nucleotides, about 3,600 nucleotides to about 5,200 nucleotides, about 3,600 nucleotides to about 5,000 nucleotides, about 3,600 nucleotides to about 4,800 nucleotides, about 3,600 nucleotides to about 4,600 nucleotides, about 3,600 nucleotides to about 4,400 nucleotides, about 3,600 nucleotides to about 4,200 nucleotides, about 3,600 nucleotides to about 4,000 nucleotides, about 3,600 nucleotides to about 3,800 nucleotides, about 3,800 nucleotides to about 10,000 nucleotides, about 3,800 nucleotides to about 9,500 nucleotides, about 3,800 nucleotides to about 9,000 nucleotides, about 3,800 nucleotides to about 8,500 nucleotides, about 3,800 nucleotides to about 8,000 nucleotides, about 3,800 nucleotides to about 7,800 nucleotides, about 3,800 nucleotides to about 7,600 nucleotides, about 3,800 nucleotides to about 7,400 nucleotides, about 3,800 nucleotides to about 7,200 nucleotides, about 3,800 nucleotides to about 7,000 nucleotides, about 3,800 nucleotides to about 6,800 nucleotides, about 3,800 nucleotides to about 6,600 nucleotides, about 3,800 nucleotides to about 6,400 nucleotides, about 3,800 nucleotides to about 6,200 nucleotides, about 3,800 nucleotides to about 6,000 nucleotides, about 3,800 nucleotides to about 5,800 nucleotides, about 3,800 nucleotides to about 5,600 nucleotides, about 3,800 nucleotides to about 5,400 nucleotides, about 3,800 nucleotides to about 5,200 nucleotides, about 3,800 nucleotides to about 5,000 nucleotides, about 3,800 nucleotides to about 4,800 nucleotides, about 3,800 nucleotides to about 4,600 nucleotides, about 3,800 nucleotides to about 4,200 nucleotides, about 3,800 nucleotides to about 4,000 nucleotides, about 4,000 nucleotides to about 10,000 nucleotides, about 4,000 nucleotides to about 9,500 nucleotides, about 4,000 nucleotides to about 9,000 nucleotides, about 4,000 nucleotides to about 8,500 nucleotides, about 4,000 nucleotides to about 8,000 nucleotides, about 4,000 nucleotides to about 7,800 nucleotides, about 4,000 nucleotides to about 7,600 nucleotides, about 4,000 nucleotides to about 7,400 nucleotides, about 4,000 nucleotides to about 7,200 nucleotides, about 4,000 nucleotides to about 7,000 nucleotides, about 4,000 nucleotides to about 6,800 nucleotides, about 4,000 nucleotides to about 6,600 nucleotides, about 4,000 nucleotides to about 6,400 nucleotides, about 4,000 nucleotides to about 6,200 nucleotides, about 4,000 nucleotides to about 6,000 nucleotides, about 4,000 nucleotides to about 5,800 nucleotides, about 4,000 nucleotides to about 5,600 nucleotides, about 4,000 nucleotides to about 5,400 nucleotides, about 4,000 nucleotides to about 5,200 nucleotides, about 4,000 nucleotides to about 5,000 nucleotides, about 4,000 nucleotides to about 4,800 nucleotides, about 4,000 nucleotides to about 4,600 nucleotides, about 4,000 nucleotides to about 4,400 nucleotides, about 4,000 nucleotides to about 4,200 nucleotides, about 4,200 nucleotides to about 10,000 nucleotides, about 4,200 nucleotides to about 9,500 nucleotides, about 4,200 nucleotides to about 9,000 nucleotides, about 4,200 nucleotides to about 8,500 nucleotides, about 4,200 nucleotides to about 8,000 nucleotides, about 4,200 nucleotides to about 7,800 nucleotides, about 4,200 nucleotides to about 7,600 nucleotides, about 4,200 nucleotides to about 7,400 nucleotides, about 4,200 nucleotides to about 7,200 nucleotides, about 4,200 nucleotides to about 7,000 nucleotides, about 4,200 nucleotides to about 6,800 nucleotides, about 4,200 nucleotides to about 6,600 nucleotides, about 4,200 nucleotides to about 6,400 nucleotides, about 4,200 nucleotides to about 6,200 nucleotides, about 4,200 nucleotides to about 6,000 nucleotides, about 4,200 nucleotides to about 5,800 nucleotides, about 4,200 nucleotides to about 5,600 nucleotides, about 4,200 nucleotides to about 5,400 nucleotides, about 4,200 nucleotides to about 5,200 nucleotides, about 4,200 nucleotides to about 5,000 nucleotides, about 4,200 nucleotides to about 4,800 nucleotides, about 4,200 nucleotides to about 4,600 nucleotides, about 4,200 nucleotides to about 4,400 nucleotides, about 4,400 nucleotides to about 10,000 nucleotides, about 4,400 nucleotides to about 9,500 nucleotides, about 4,400 nucleotides to about 9,000 nucleotides, about 4,400 nucleotides to about 8,500 nucleotides, about 4,400 nucleotides to about 8,000 nucleotides, about 4,400 nucleotides to about 7,800 nucleotides, about 4,400 nucleotides to about 7,600 nucleotides, about 4,400 nucleotides to about 7,400 nucleotides, about 4,400 nucleotides to about 7,200 nucleotides, about 4,400 nucleotides to about 7,000 nucleotides, about 4,400 nucleotides to about 6,800 nucleotides, about 4,400 nucleotides to about 6,600 nucleotides, about 4,400 nucleotides to about 6,400 nucleotides, about 4,400 nucleotides to about 6,200 nucleotides, about 4,400 nucleotides to about 6,000 nucleotides, about 4,400 nucleotides to about 5,800 nucleotides, about 4,400 nucleotides to about 5,600 nucleotides, about 4,400 nucleotides to about 5,400 nucleotides, about 4,400 nucleotides to about 5,200 nucleotides, about 4,400 nucleotides to about 5,000 nucleotides, about 4,400 nucleotides to about 4,800 nucleotides, about 4,400 nucleotides to about 4,600 nucleotides, about 4,600 nucleotides to about 10,000 nucleotides, about 4,600 nucleotides to about 9,500 nucleotides, about 4,600 nucleotides to about 9,000 nucleotides, about 4,600 nucleotides to about 8,500 nucleotides, about 4,600 nucleotides to about 8,000 nucleotides, about 4,600 nucleotides to about 7,800 nucleotides, about 4,600 nucleotides to about 7,600 nucleotides, about 4,600 nucleotides to about 7,400 nucleotides, about 4,600 nucleotides to about 7,200 nucleotides, about 4,600 nucleotides to about 7,000 nucleotides, about 4,600 nucleotides to about 6,800 nucleotides, about 4,600 nucleotides to about 6,600 nucleotides, about 4,600 nucleotides to about 6,400 nucleotides, about 4,600 nucleotides to about 6,200 nucleotides, about 4,600 nucleotides to about 6,000 nucleotides, about 4,600 nucleotides to about 5,800 nucleotides, about 4,600 nucleotides to about 5,600 nucleotides, about 4,600 nucleotides to about 5,400 nucleotides, about 4,600 nucleotides to about 5,200 nucleotides, about 4,600 nucleotides to about 5,000 nucleotides, about 4,600 nucleotides to about 4,800 nucleotides, about 4,800 nucleotides to about 10,000 nucleotides, about 4,800 nucleotides to about 9,500 nucleotides, about 4,800 nucleotides to about 9,000 nucleotides, about 4,800 nucleotides to about 8,500 nucleotides, about 4,800 nucleotides to about 8,000 nucleotides, about 4,800 nucleotides to about 7,800 nucleotides, about 4,800 nucleotides to about 7,600 nucleotides, about 4,800 nucleotides to about 7,400 nucleotides, about 4,800 nucleotides to about 7,200 nucleotides, about 4,800 nucleotides to about 7,000 nucleotides, about 4,800 nucleotides to about 6,800 nucleotides, about 4,800 nucleotides to about 6,600 nucleotides, about 4,800 nucleotides to about 6,400 nucleotides, about 4,800 nucleotides to about 6,200 nucleotides, about 4,800 nucleotides to about 6,000 nucleotides, about 4,800 nucleotides to about 5,800 nucleotides, about 4,800 nucleotides to about 5,600 nucleotides, about 4,800 nucleotides to about 5,400 nucleotides, about 4,800 nucleotides to about 5,200 nucleotides, about 4,800 nucleotides to about 5,000 nucleotides, about 5,000 nucleotides to about 10,000 nucleotides, about 5,000 nucleotides to about 9,500 nucleotides, about 5,000 nucleotides to about 9,000 nucleotides, about 5,000 nucleotides to about 8,500 nucleotides, about 5,000 nucleotides to about 8,000 nucleotides, about 5,000 nucleotides to about 7,800 nucleotides, about 5,000 nucleotides to about 7,600 nucleotides, about 5,000 nucleotides to about 7,400 nucleotides, about 5,000 nucleotides to about 7,200 nucleotides, about 5,000 nucleotides to about 7,000 nucleotides, about 5,000 nucleotides to about 6,800 nucleotides, about 5,000 nucleotides to about 6,600 nucleotides, about 5,000 nucleotides to about 6,400 nucleotides, about 5,000 nucleotides to about 6,200 nucleotides, about 5,000 nucleotides to about 6,000 nucleotides, about 5,000 nucleotides to about 5,800 nucleotides, about 5,000 nucleotides to about 5,600 nucleotides, about 5,000 nucleotides to about 5,400 nucleotides, about 5,000 nucleotides to about 5,200 nucleotides, about 5,200 nucleotides to about 10,000 nucleotides, about 5,200 nucleotides to about 9,500 nucleotides, about 5,200 nucleotides to about 9,000 nucleotides, about 5,200 nucleotides to about 8,500 nucleotides, about 5,200 nucleotides to about 8,000 nucleotides, about 5,200 nucleotides to about 7,800 nucleotides, about 5,200 nucleotides to about 7,600 nucleotides, about 5,200 nucleotides to about 7,400 nucleotides, about 5,200 nucleotides to about 7,200 nucleotides, about 5,200 nucleotides to about 7,000 nucleotides, about 5,200 nucleotides to about 6,800 nucleotides, about 5,200 nucleotides to about 6,600 nucleotides, about 5,200 nucleotides to about 6,400 nucleotides, about 5,200 nucleotides to about 6,200 nucleotides, about 5,200 nucleotides to about 6,000 nucleotides, about 5,200 nucleotides to about 5,800 nucleotides, about 5,200 nucleotides to about 5,600 nucleotides, about 5,200 nucleotides to about 5,400 nucleotides, about 5,400 nucleotides to about 10,000 nucleotides, about 5,400 nucleotides to about 9,500 nucleotides, about 5,400 nucleotides to about 9,000 nucleotides, about 5,400 nucleotides to about 8,500 nucleotides, about 5,400 nucleotides to about 8,000 nucleotides, about 5,400 nucleotides to about 7,800 nucleotides, about 5,400 nucleotides to about 7,600 nucleotides, about 5,400 nucleotides to about 7,400 nucleotides, about 5,400 nucleotides to about 7,200 nucleotides, about 5,400 nucleotides to about 7,000 nucleotides, about 5,400 nucleotides to about 6,800 nucleotides, about 5,400 nucleotides to about 6,600 nucleotides, about 5,400 nucleotides to about 6,400 nucleotides, about 5,400 nucleotides to about 6,200 nucleotides, about 5,400 nucleotides to about 6,000 nucleotides, about 5,400 nucleotides to about 5,800 nucleotides, about 5,400 nucleotides to about 5,600 nucleotides, about 5,600 nucleotides to about 10,000 nucleotides, about 5,600 nucleotides to about 9,500 nucleotides, about 5,600 nucleotides to about 9,000 nucleotides, about 5,600 nucleotides to about 8,500 nucleotides, about 5,600 nucleotides to about 8,000 nucleotides, about 5,600 nucleotides to about 7,800 nucleotides, about 5,600 nucleotides to about 7,600 nucleotides, about 5,600 nucleotides to about 7,400 nucleotides, about 5,600 nucleotides to about 7,200 nucleotides, about 5,600 nucleotides to about 7,000 nucleotides, about 5,600 nucleotides to about 6,800 nucleotides, about 5,600 nucleotides to about 6,600 nucleotides, about 5,600 nucleotides to about 6,400 nucleotides, about 5,600 nucleotides to about 6,200 nucleotides, about 5,600 nucleotides to about 6,000 nucleotides, about 5,600 nucleotides to about 5,800 nucleotides, about 5,800 nucleotides to about 10,000 nucleotides, about 5,800 nucleotides to about 9,500 nucleotides, about 5,800 nucleotides to about 9,000 nucleotides, about 5,800 nucleotides to about 8,500 nucleotides, about 5,800 nucleotides to about 8,000 nucleotides, about 5,800 nucleotides to about 7,800 nucleotides, about 5,800 nucleotides to about 7,600 nucleotides, about 5,800 nucleotides to about 7,400 nucleotides, about 5,800 nucleotides to about 7,200 nucleotides, about 5,800 nucleotides to about 7,000 nucleotides, about 5,800 nucleotides to about 6,800 nucleotides, about 5,800 nucleotides to about 6,600 nucleotides, about 5,800 nucleotides to about 6,400 nucleotides, about 5,800 nucleotides to about 6,200 nucleotides, about 5,800 nucleotides to about 6,000 nucleotides, about 6,000 nucleotides to about 10,000 nucleotides, about 6,000 nucleotides to about 9,500 nucleotides, about 6,000 nucleotides to about 9,000 nucleotides, about 6,000 nucleotides to about 8,500 nucleotides, about 6,000 nucleotides to about 8,000 nucleotides, about 6,000 nucleotides to about 7,800 nucleotides, about 6,000 nucleotides to about 7,600 nucleotides, about 6,000 nucleotides to about 7,400 nucleotides, about 6,000 nucleotides to about 7,200 nucleotides, about 6,000 nucleotides to about 7,000 nucleotides, about 6,000 nucleotides to about 6,800 nucleotides, about 6,000 nucleotides to about 6,600 nucleotides, about 6,000 nucleotides to about 6,400 nucleotides, about 6,000 nucleotides to about 6,200 nucleotides, about 6,200 nucleotides to about 10,000 nucleotides, about 6,200 nucleotides to about 9,000 nucleotides, about 6,200 nucleotides to about 8,500 nucleotides, about 6,200 nucleotides to about 8,000 nucleotides, about 6,200 nucleotides to about 7,800 nucleotides, about 6,200 nucleotides to about 7,600 nucleotides, about 6,200 nucleotides to about 7,400 nucleotides, about 6,200 nucleotides to about 7,200 nucleotides, about 6,200 nucleotides to about 7,000 nucleotides, about 6,200 nucleotides to about 6,800 nucleotides, about 6,200 nucleotides to about 6,600 nucleotides, about 6,200 nucleotides to about 6,400 nucleotides, about 6,400 nucleotides to about 10,000 nucleotides, about 6,400 nucleotides to about 9,500 nucleotides, about 6,400 nucleotides to about 9,000 nucleotides, about 6,400 nucleotides to about 8,500 nucleotides, about 6,400 nucleotides to about 8,000 nucleotides, about 6,400 nucleotides to about 7,800 nucleotides, about 6,400 nucleotides to about 7,600 nucleotides, about 6,400 nucleotides to about 7,400 nucleotides, about 6,400 nucleotides to about 7,200 nucleotides, about 6,400 nucleotides to about 7,000 nucleotides, about 6,400 nucleotides to about 6,800 nucleotides, about 6,400 nucleotides to about 6,600 nucleotides, about 6,600 nucleotides to about 10,000 nucleotides, about 6,600 nucleotides to about 9,500 nucleotides, about 6,600 nucleotides to about 9,000 nucleotides, about 6,600 nucleotides to about 8,500 nucleotides, about 6,600 nucleotides to about 8,000 nucleotides, about 6,600 nucleotides to about 7,800 nucleotides, about 6,600 nucleotides to about 7,600 nucleotides, about 6,600 nucleotides to about 7,400 nucleotides, about 6,600 nucleotides to about 7,200 nucleotides, about 6,600 nucleotides to about 7,000 nucleotides, about 6,600 nucleotides to about 6,800 nucleotides, about 6,800 nucleotides to about 10,000 nucleotides, about 6,800 nucleotides to about 9,500 nucleotides, about 6,800 nucleotides to about 9,000 nucleotides, about 6,800 nucleotides to about 8,500 nucleotides, about 6,800 nucleotides to about 8,000 nucleotides, about 6,800 nucleotides to about 7,800 nucleotides, about 6,800 nucleotides to about 7,600 nucleotides, about 6,800 nucleotides to about 7,400 nucleotides, about 6,800 nucleotides to about 7,200 nucleotides, about 6,800 nucleotides to about 7,000 nucleotides, about 7,000 nucleotides to about 10,000 nucleotides, about 7,000 nucleotides to about 9,500 nucleotides, about 7,000 nucleotides to about 9,000 nucleotides, about 7,000 nucleotides to about 8,500 nucleotides, about 7,000 nucleotides to about 8,000 nucleotides, about 7,000 nucleotides to about 7,800 nucleotides, about 7,000 nucleotides to about 7,600 nucleotides, about 7,000 nucleotides to about 7,400 nucleotides, about 7,000 nucleotides to about 7,200 nucleotides, about 7,200 nucleotides to about 10,000 nucleotides, about 7,200 nucleotides to about 9,500 nucleotides, about 7,200 nucleotides to about 9,000 nucleotides, about 7,200 nucleotides to about 8,500 nucleotides, about 7,200 nucleotides to about 8,000 nucleotides, about 7,200 nucleotides to about 7,800 nucleotides, about 7,200 nucleotides to about 7,600 nucleotides, about 7,200 nucleotides to about 7,400 nucleotides, about 7,400 nucleotides to about 10,000 nucleotides, about 7,400 nucleotides to about 9,500 nucleotides, about 7,400 nucleotides to about 9,000 nucleotides, about 7,400 nucleotides to about 8,500 nucleotides, about 7,400 nucleotides to about 8,000 nucleotides, about 7,400 nucleotides to about 7,800 nucleotides, about 7,400 nucleotides to about 7,600 nucleotides, about 7,600 nucleotides to about 10,000 nucleotides, about 7,600 nucleotides to about 9,500 nucleotides, about 7,600 nucleotides to about 9,000 nucleotides, about 7,600 nucleotides to about 8,500 nucleotides, about 7,600 nucleotides to about 8,000 nucleotides, about 7,600 nucleotides to about 7,800 nucleotides, about 7,800 nucleotides to about 10,000 nucleotides, about 7,800 nucleotides to about 9,500 nucleotides, about 7,800 nucleotides to about 9,000 nucleotides, about 7,800 nucleotides to about 8,500 nucleotides, about 7,800 nucleotides to about 8,000 nucleotides, about 8,000 nucleotides to about 10,000 nucleotides, about 8,000 nucleotides to about 9,500 nucleotides, about 8,000 nucleotides to about 9,000 nucleotides, about 8,000 nucleotides to about 8,500 nucleotides, about 8,500 nucleotides to about 10,000 nucleotides, about 8,500 nucleotides to about 9,500 nucleotides, about 8,500 nucleotides to about 9,000 nucleotides, about 9,000 nucleotides to about 10,000 nucleotides, about 9,000 nucleotides to about 9,500 nucleotides, or about 9,500 nucleotides to about 10,000 nucleotides (inclusive).

A variety of different methods known in the art can be used to introduce any of vectors disclosed herein into a primate cell (e.g., a supporting cochlear outer hair cell or a hair cell (e.g., an inner hair cell or an outer hair cell). Non-limiting examples of methods for introducing nucleic acid into a primate cell include: lipofection, transfection (e.g., calcium phosphate transfection, transfection using highly branched organic compounds, transfection using cationic polymers, dendrimer-based transfection, optical transfection, particle-based transfection (e.g., nanoparticle transfection), or transfection using liposomes (e.g., cationic liposomes)), microinjection, electroporation, cell squeezing, sonoporation, protoplast fusion, impalefection, hydrodynamic delivery, gene gun, magnetofection, viral transfection, and nucleofection.

Skilled practitioners will appreciate that any of the vectors described herein can be introduced into a primate cell by, for example, lipofection.

Various molecular biology techniques that can be used to introduce a mutation(s) and/or a deletion(s) into an endogenous gene are also known in the art. Non-limiting examples of such techniques include site-directed mutagenesis, CRISPR (e.g., CRISPR/Cas9-induced knock-in mutations and CRISPR/Cas9-induced knock-out mutations), TALENs, and base editing (Rees & Lui, Nature Rev. Genetics 19: 770-788, 2018; Eid et al., Biochem J. 475(11): 1955-1964, 2018). These methods can be used to correct the sequence of a defective endogenous gene present in a chromosome of a target cell.

Any of the vectors described herein can further include a control sequence, e.g., a control sequence selected from the group of a transcription initiation sequence, a transcription termination sequence, a promoter sequence, an enhancer sequence, an RNA splicing sequence, a polyadenylation (polyA) sequence, and a Kozak consensus sequence. Non-limiting examples of these control sequences are described herein. In some embodiments, a promoter can be a native promoter, a constitutive promoter, an inducible promoter, and/or a tissue-specific promoter.

Some embodiments of any of the compositions and kits described herein can include any combination of the AAV vectors described herein. Some embodiments of any of the methods described herein can include the use of any combination of the AAV vectors described herein.

### Promoters

The term "promoter" means a DNA sequence recognized by enzymes/proteins in a primate cell required to initiate the transcription of a specific gene (e.g., a supporting cell target gene or a hair cell target gene). A promoter typically refers to, e.g., a nucleotide sequence to which an RNA polymerase and/or any associated factor binds and at which transcription is initiated. Non-limiting examples of promoters are described herein. Additional examples of promoters are known in the art.

In some embodiments, a vector encoding an N-terminal portion of a hair cell target protein (e.g., a human hair cell target protein) or a hair cell target protein can include a promoter and/or an enhancer. The vector encoding the N-terminal portion of the hair cell target protein or a hair cell target protein can include any of the promoters and/or enhancers described herein or known in the art.

In some embodiments, a vector encoding an N-terminal portion of a supporting cell target protein (e.g., a human supporting cell target protein) or a supporting cell target protein can include a promoter and/or an enhancer. The vector encoding the N-terminal portion of the supporting cell target protein or a supporting cell target protein can include any of the promoters and/or enhancers described herein or known in the art.

In some embodiments, the promoter is an inducible promoter, a constitutive promoter, a primate cell promoter, a viral promoter, a chimeric promoter, an engineered promoter, a tissue-specific promoter, or any other type of promoter known in the art. In some embodiments, the promoter is a RNA polymerase II promoter, such as a primate RNA polymerase II promoter. In some embodiments, the promoter is a RNA polymerase III promoter, including, but not limited to, a HI promoter, a human U6 promoter, a mouse U6 promoter, or a swine U6 promoter. The promoter will generally be one that is able to promote transcription in cochlear cells such as hair cells. In some examples, the promoter is a cochlea-specific promoter or a cochlea-oriented promoter.

A variety of promoters are known in the art that can be used herein. Non-limiting examples of promoters that can be used herein include: human EF1a, human cytomegalovirus (CMV) (US Patent No. 5,168,062), human ubiquitin C (UBC), mouse phosphoglycerate kinase 1, polyoma adenovirus, simian virus 40 (SV40), β-globin, β-actin, α-fetoprotein, γ-globin, β-interferon, γ-glutamyl transferase, mouse mammary tumor virus (MMTV), Rous sarcoma virus, rat insulin, glyceraldehyde-3-phosphate dehydrogenase, metallothionein II (MT II), amylase, cathepsin, MI muscarinic receptor, retroviral LTR (e.g. human T-cell leukemia virus HTLV), AAV ITR, interleukin-2, collagenase, platelet-derived growth factor, adenovirus 5 E2, stromelysin, murine MX gene, glucose regulated proteins (GRP78 and GRP94), α-2-macroglobulin, vimentin, MHC class I gene H-2κ b, HSP70, proliferin, tumor necrosis factor, thyroid stimulating hormone α gene, immunoglobulin light chain, T-cell receptor, HLA DQα and DQβ, interleukin-2 receptor, MHC class II, MHC class II HLA-DRα, muscle creatine kinase, prealbumin (transthyretin), elastase I, albumin gene, c-fos, c-HA-ras, neural cell adhesion molecule (NCAM), H2B (TH2B) histone, rat growth hormone, human serum amyloid (SAA), troponin I (TN I), duchenne muscular dystrophy, human immunodeficiency virus, and Gibbon Ape Leukemia Virus (GALV) promoters. Additional examples of promoters are known in the art. See, e.g., Lodish, Molecular Cell Biology, Freeman and Company, New York 2007. In some embodiments, the promoter is the CMV immediate early promoter. In some embodiments, the promoter is a CAG promoter or a CAG/CBA promoter.

The term "constitutive" promoter refers to a nucleotide sequence that, when operably linked with a nucleic acid encoding a target protein (e.g., a supporting cell target protein, a hair cell target protein), causes RNA to be transcribed from the nucleic acid in a primate cell under most or all physiological conditions.

Examples of constitutive promoters include, without limitation, the retroviral Rous sarcoma virus (RSV) LTR promoter, the cytomegalovirus (CMV) promoter (see, e.g., Boshart et al, Cell 41:521-530, 1985), the SV40 promoter, the dihydrofolate reductase promoter, the beta-actin promoter, the phosphoglycerol kinase (PGK) promoter, and the EF1-alpha promoter (Invitrogen).

Inducible promoters allow regulation of gene expression and can be regulated by exogenously supplied compounds, environmental factors such as temperature, or the presence of a specific physiological state, e.g., acute phase, a particular differentiation state of the cell, or in replicating cells only. Inducible promoters and inducible systems are available from a variety of commercial sources, including, without limitation, Invitrogen, Clontech, and Ariad. Additional examples of inducible promoters are known in the art.

Examples of inducible promoters regulated by exogenously supplied compounds include the zinc-inducible sheep metallothionine (MT) promoter, the dexamethasone (Dex)-inducible mouse mammary tumor virus (MMTV) promoter, the T7 polymerase promoter system (WO 98/10088); the ecdysone insect promoter (No et al, Proc. Natl. Acad. Sci. U.S.A. 93:3346-3351, 1996), the tetracycline-repressible system (Gossen et al, Proc. Natl. Acad. Sci. U.S.A. 89:5547-5551, 1992), the tetracycline-inducible system (Gossen et al, Science 268:1766-1769, 1995, see also Harvey et al, Curr. Opin. Chem. Biol. 2:512-518, 1998), the RU486-inducible system (Wang et al, Nat. Biotech. 15:239-243, 1997) and Wang et al, Gene Ther. 4:432-441, 1997), and the rapamycin-inducible system (Magari et al. J. Clin. Invest. 100:2865-2872, 1997).

The term "tissue-specific" promoter refers to a promoter that is active only in certain specific cell types and/or tissues (e.g., transcription of a specific gene occurs only within cells expressing transcription regulatory proteins that bind to the tissue-specific promoter).

In some embodiments, the regulatory sequences impart tissue-specific gene expression capabilities. In some cases, the tissue-specific regulatory sequences bind tissue-specific transcription factors that induce transcription in a tissue-specific manner.

Exemplary tissue-specific promoters include but are not limited to the following: a liver-specific thyroxin binding globulin (TBG) promoter, an insulin promoter, a glucagon promoter, a somatostatin promoter, a pancreatic polypeptide (PPY) promoter, a synapsin-1 (Syn) promoter, a creatine kinase (MCK) promoter, a primate desmin (DES) promoter, an alpha-myosin heavy chain (a-MHC) promoter, and a cardiac Troponin T (cTnT) promoter. Additional exemplary promoters include Beta-actin promoter, hepatitis B virus core promoter (Sandig et al., Gene Ther. 3:1002-1009, 1996), alpha-fetoprotein (AFP) promoter (Arbuthnot et al., Hum. Gene Ther. 7:1503-1514, 1996), bone osteocalcin promoter (Stein et al., Mol. Biol. Rep. 24:185-196, 1997); bone sialoprotein promoter (Chen et al., J. Bone Miner. Res. 11:654-664, 1996), CD2 promoter (Hansal et al., J. Immunol. 161:1063-1068, 1998); immunoglobulin heavy chain promoter; T cell receptor alpha-chain promoter, neuronal such as neuron-specific enolase (NSE) promoter (Andersen et al., Cell. Mol. Neurobiol. 13:503-515, 1993), neurofilament light-chain gene promoter (Piccioli et al., Proc. Natl. Acad. Sci. U.S.A. 88:5611-5615, 1991), and the neuron-specific vgf gene promoter (Piccioli et al., Neuron 15:373-384, 1995).

In some embodiments, the tissue-specific promoter is a cochlea-specific promoter. In some embodiments, the tissue-specific promoter is a cochlear hair cell-specific promoter. Non-limiting examples of cochlear hair cell-specific promoters include but are not limited to: a ATOH1 promoter, a POU4F3 promoter, a LHX3 promoter, a MYO7A promoter, a MYO6 promoter, a α9ACHR promoter, and a α10ACHR promoter. In some embodiments, the promoter is an outer hair cell-specific promoter such as a PRESTIN promoter or an ONCOMOD promoter. See, e.g., Zheng et al., Nature 405:149-155, 2000; Tian et al. Dev. Dyn. 231:199-203, 2004; and Ryan et al., Adv. Otorhinolaryngol. 66: 99-115, 2009.

In some embodiments of any of the AAV vectors described herein, the AAV vector includes a human ATOH1 enhancer-promoter (SEQ ID NO: 84).

**Human ATOH1 enhancer-promoter (SEQ ID NO: 84)**

ctatggagtttgcataacaaacgtttggcagctcgctctcttacactccattaacaagctgtaacatatag
ctgcaggttgctataatctcattaatatttggaaacttgaatattgagtatttctgagtgctcattcccc
atatgccagccacttctgccatgctgactggttcctttctctccattattagcaattagcttcttaccttc
caaagtcagatccaaggtatccaagatactagcaaaggaatcaactatgtgtgcaagttaagcatgcttaa
tatcacccaaacaaacaaagaggcagcatttcttaaagtaatgaagatagataaatcgggttagtcctttg
cgacactgctggtgctttctagagttttatatatttaagcagcttgctttatattctgtctttgcctccc
accccaccagcacttttatttgtggagggttttggctcgccacactttgggaaacttatttgatttcacgg
agagctgaaggaagatcatttttggcaacagacaagtttaaacacgatttctatgggacattgctaactgg
ggcccctaaggagaaaggggaaactgagcggagaatgggttaaatccttggaagcaggggagaggcagggg
aggagagaagtcggaggagtataaagaaaaggacaggaaccaagaagcgtgggggtggtttgccgtaatgt
gagtgtttcttaattagagaacggttgacaatagagggtctggcagaggctcctggccgcggtgcggagcg
tctggagcggagcacgcgctgtcagctggtgagcgcactctcctttcaggcagctccccggggagctgtgc
ggccacatttaacaccatcatcacccctccccggcctcctcaacctcggcctcctcctcgtcgacagcctt
ccttggcccccaccagcagagctcacagtagcgagcgtctctcgccgtctcccgcactcggccggggcctc
tctcctcccccagctgcgcagcgggagccgccactgcccactgcacctcccagcaaccagcccagcacgca
aagaagctgcgcaaagttaaagccaagcaatgccaaggggaggggaagctggaggcgggctttgagtggct
tctgggcgcctggcgggtccagaatcgcccagagccgcccgcggtcgtgcacatctgacccgagtcagctt
gggcaccagccgagagccggctccgcaccgctcccgcaccccagccgccggggtggtgacacacaccggag
tcgaattacagccctgcaattaacatatgaatctgacgaatttaaaagaaggaaaaaaaaaaaaaacctg
agcaggcttgggagtcctctgcacacaagaacttttctcggggtgtaaaaactctttgattggctgctcgc
acgcgcctgcccgcgccctccattggctgagaagacacgcgaccggcgcgaggaggggggttgggagaggag
cggggggagactgagtggcgcgtgccgctttttaaagggcgcagcgccttcagcaaccggagaagcatag
ttgcacgcgacctggtgtgtgatctccgagtgggtgggggaggtcgaggagggaaaaaaaaataagacgt
tgcagaagagacccggaaagggcctttttttttggttgagctggtgtcccagtgctgcctccgatcctgagc
ctccgagcctttgcagtgcaa

### Enhancers and 5' Cap

In some instances, a vector can include a promoter sequence and/or an enhancer sequence. The term "enhancer" refers to a nucleotide sequence that can increase the level of transcription of a nucleic acid encoding a protein of interest (e.g., a supporting cell target protein or a hair cell target protein). Enhancer sequences (50-1500 basepairs in length) generally increase the level of transcription by providing additional binding sites for transcription-associated proteins (e.g., transcription factors). In some embodiments, an enhancer sequence is found within an intronic sequence. Unlike promoter sequences, enhancer sequences can act at much larger distance away from the transcription start site (e.g., as compared to a promoter). Non-limiting examples of enhancers include a RSV enhancer, a CMV enhancer, and a SV40 enhancer.

### Destabilizing Domain (DD)

Any of the AAV vectors provided herein can optionally include a sequence encoding a destabilizing domain ("a destabilizing sequence") for temporal control of protein expression. Non-limiting examples of destabilizing sequences include sequences encoding: a FK506 sequence, a dihydrofolate reductase (DHFR) sequence.
**Exemplary DHFR destabilizing sequence (SEQ ID NO: 85):** 
MISLIAALAVDYVIGMENAMPWNLPADLAWFKRNTLNKPVIMGRHTWESIGRPLPGRKNIILSSQPSTDDR
VTWVKSVDEAIAACGDVPEIMVIGGGRVIEQFLPKAQKLYLTHIDAEVEGDTHFPDYEPDDWESVFSEFHD
ADAQNSHSYCFEILERR
**Exemplary DHFR destabilizing domain sequence (SEQ ID NO: 86)**

ggtaccatcagtctgattgcggcgttagcggtagattacgttatcggcatggaaaacgccatgccgtggaa
cctgcctgccgatctcgcctggtttaaacgcaacaccttaaataaacccgtgattatgggccgccatacct
gggaatcaatcggtcgtccgttgccaggacgcaaaaatattatcctcagcagtcaaccgagtacggacgat
cgcgtaacgtgggtgaagtcggtggatgaagccatcgcggcgtgtggtgacgtaccagaaatcatggtgat
tggcggcggtcgcgttattgaacagttcttgccaaaagcgcaaaaactgtatctgacgcatatcgacgcag
aagtggaaggcgacacccatttcccggattacgagccggatgactgggaatcggtattcagcgaattccac
gatgctgatgcgcagaactctcacagctattgctttgagattctggagcggcgataa

In some embodiments of any of the AAV vectors described herein, the AAV vector includes a destabilizing domain (SEQ ID NO: 87).

**Exemplary destabilizing domain (SEQ ID NO: 87)**

atcagtctgattgcggcgttagcggtagattacgttatcggcatggaaaacgccatgccgtggaacctgcc
tgccgatctcgcctggtttaaacgcaacaccttaaataaacccgtgattatggccgccatacctgggaat
caatcggtcgtccgttgccaggacgcaaaaatattatcctcagcagtcaaccgagtacggacgatcgcgta
acgtgggtgaagtcggtggatgaagccatcgcggcgtgtggtgacgtaccagaaatcatggtgattggcgg
cggtcgcgttattgaacagttcttgccaaaagcgcaaaaactgtatctgacgcatatcgacgcagaagtgg
aaggcgacacccatttcccggattacgagccggatgactgggaatcggtattcagcgaattccacgatgct
gatgcgcagaactctcacagctattgctttgagattctggagcggcga

Additional examples of destabilizing sequences are known in the art. In some embodiments, the destabilizing sequence is a FK506- and rapamycin-binding protein (FKBP12) sequence, and the stabilizing ligand is Shield-1 (Shld1) (Banaszynski et al. (2012) Cell 126(5): 995-1004).

**Exemplary FKBP12 destabilizing sequence (SEQ ID NO: 88)**

MGVEKQVIRPGNGPKPAPGQTVTVHCTGFGKDGDLSQKFWSTKDEGQKPFSFQIGKGAVIKGWDEGVIGMQ
IGEVARLRCSSDYAYGAGGFPAWGIQPNSVLDFEIEVLSVQ

In some embodiments, the destabilizing sequence is a DHFR sequence, and the stabilizing ligand is trimethoprim (TMP) (Iwamoto et al. (2010) Chem Biol 17:981-988).

In the absence of a stabilizing ligand, the protein sequence operatively linked to the destabilizing sequence is degraded by ubiquitination. In contrast, in the presence of a stabilizing ligand, protein degradation is inhibited, thereby allowing the protein sequence operatively linked to the destabilizing sequence to be actively expressed. As a positive control for stabilization of protein expression, protein expression can be detected by conventional means, including enzymatic, radiographic, colorimetric, fluorescence, or other spectrographic assays; fluorescent activating cell sorting (FACS) assays; immunological assays (e.g., enzyme linked immunosorbent assay (ELISA), radioimmunoassay (RIA), and immunohistochemistry).

In some embodiments, the destabilizing sequence is a FKBP12 sequence, and the presence of an AAV vector carrying the FKBP12 gene in a primate cell (e.g., a supporting cochlear outer hair cell) is detected by western blotting. In some embodiments, the destabilizing sequence can be used to verify the temporally-specific activity of any of the AAV vectors described herein.

### Poly(A) Sequences

In some embodiments, any of the vectors provided herein can include a poly(A) sequence. Most nascent eukaryotic mRNAs possess a poly(A) tail at their 3' end which is added during a complex process that includes cleavage of the primary transcript and a coupled polyadenylation reaction (see, e.g., Proudfoot et al., Cell 108:501-512, 2002). The poly(A) tail confers mRNA stability and transferability (Molecular Biology of the Cell, Third Edition by B. Alberts et al., Garland Publishing, 1994). In some embodiments, the poly(A) sequence is positioned 3' to the nucleic acid sequence encoding the C-terminus of the hair cell target protein. In some embodiments, the poly(A) sequence is positioned 3' to the nucleic acid sequence encoding the C-terminus of the supporting cell target protein or a protein of interest (e.g., a Cas9 endonuclease, e.g., a SaCas9 endonuclease (e.g., any of the SaCas9 endonucleases described herein), a reporter protein (e.g., a GFP protein, a mScarlet protein)).

As used herein, "polyadenylation" refers to the covalent linkage of a polyadenylyl moiety, or its modified variant, to a messenger RNA molecule. In eukaryotic organisms, most messenger RNA (mRNA) molecules are polyadenylated at the 3' end. The 3' poly(A) tail is a long sequence of adenine nucleotides (e.g., 50, 60, 70, 100, 200, 500, 1000, 2000, 3000, 4000, or 5000) added to the pre-mRNA through the action of an enzyme, polyadenylate polymerase. In higher eukaryotes, the poly(A) tail is added onto transcripts that contain a specific sequence, the polyadenylation signal or "poly(A) sequence." The poly(A) tail and the protein bound to it aid in protecting mRNA from degradation by exonucleases. Polyadenylation is also important for transcription termination, export of the mRNA from the nucleus, and translation. Polyadenylation occurs in the nucleus immediately after transcription of DNA into RNA, but additionally can also occur later in the cytoplasm. After transcription has been terminated, the mRNA chain is cleaved through the action of an endonuclease complex associated with RNA polymerase. The cleavage site is usually characterized by the presence of the base sequence AAUAAA near the cleavage site. After the mRNA has been cleaved, adenosine residues are added to the free 3' end at the cleavage site.

As used herein, a "poly(A) sequence" is a sequence that triggers the endonuclease cleavage of an mRNA and the additional of a series of adenosines to the 3' end of the cleaved mRNA.

There are several poly(A) sequences that can be used, including those derived from bovine growth hormone (bgh) (Woychik et al., Proc. Natl. Acad. Sci. U.S.A. 81(13):3944-3948, 1984; U.S. Patent No. 5,122,458), mouse-β-globin, mouse-α-globin (Orkin et al., EMBO J. 4(2):453-456, 1985; Thein et al., Blood 71(2):313-319, 1988), human collagen, polyoma virus (Batt et al., Mol. Cell Biol. 15(9):4783-4790, 1995), the Herpes simplex virus thymidine kinase gene (HSV TK), IgG heavy-chain gene polyadenylation signal (US 2006/0040354), human growth hormone (hGH) (Szymanski et al., Mol. Therapy 15(7):1340-1347, 2007), the group of SV40 poly(A) sites, such as the SV40 late and early poly(A) site (Schek et al., Mol. Cell Biol. 12(12):5386-5393, 1992).

The poly(A) sequence can be a sequence of AATAAA. The AATAAA sequence may be substituted with other hexanucleotide sequences with homology to AATAAA which are capable of signaling polyadenylation, including ATTAAA, AGTAAA, CATAAA, TATAAA, GATAAA, ACTAAA, AATATA, AAGAAA, AATAAT, AAAAAA, AATGAA, AATCAA, AACAAA, AATCAA, AATAAC, AATAGA, AATTAA, or AATAAG (see, e.g., WO 06/12414).

In some embodiments, the poly(A) sequence can be a synthetic polyadenylation site (see, e.g., the pCl-neo expression vector of Promega which is based on Levitt el al, Genes Dev. 3(7):1019-1025, 1989). In some embodiments, the poly(A) sequence is the polyadenylation signal of soluble neuropilin-1 (sNRP) (AAATAAAATACGAAATG, SEQ ID NO: 90) (see, e.g., WO 05/073384). Additional examples of poly(A) sequences are known in the art.

### Internal Ribosome Entry Site (IRES)

In some embodiments, a vector encoding the C-terminus of the hair cell target protein or a hair cell target protein can include a polynucleotide internal ribosome entry site (IRES). In some embodiments, a vector encoding the C-terminus of the supporting cell target protein or a supporting cell target protein can include a polynucleotide internal ribosome entry site (IRES). An IRES sequence is used to produce more than one polypeptide from a single gene transcript. An IRES forms a complex secondary structure that allows translation initiation to occur from any position with an mRNA immediately downstream from where the IRES is located (see, e.g., Pelletier and Sonenberg, Mol. Cell. Biol 8(3):1103-1112, 1988).

There are several IRES sequences known to those in skilled in the art, including those from, e.g., foot and mouth disease virus (FMDV), encephalomyocarditis virus (EMCV), human rhinovirus (HRV), cricket paralysis virus, human immunodeficiency virus (HIV), hepatitis A virus (HAV), hepatitis C virus (HCV), and poliovirus (PV). See e.g., Alberts, Molecular Biology of the Cell, Garland Science, 2002; and Hellen et al., Genes Dev. 15(13):1593-612, 2001.

In some embodiments, the IRES sequence that is incorporated into the vector that encodes the C-terminus of a hair cell target protein or a hair cell target protein is the foot and mouth disease virus (FMDV). In some embodiments, the IRES sequence that is incorporated into the vector that encodes the C-terminus of a supporting cell target protein or a supporting cell target protein is the foot and mouth disease virus (FMDV) 2A sequence. In some embodiments, the IRES sequence that is incorporated into the vector that encodes the C-terminal portion of a protein of interest (e.g., a Cas9 endonuclease, e.g., a SaCas9 endonuclease (e.g., any of the SaCas9 endonucleases described herein)) is the FMDV 2A sequence. The Foot and Mouth Disease Virus 2A sequence is a small peptide (approximately 18 amino acids in length) that has been shown to mediate the cleavage of polyproteins (Ryan, M D et al., EMBO 4:928-933, 1994; Mattion et al., J. Virology 70:8124-8127, 1996; Furler et al., Gene Therapy 8:864-873, 2001; and Halpin et al., Plant Journal 4:453-459, 1999). The cleavage activity of the 2A sequence has previously been demonstrated in artificial systems including plasmids and gene therapy vectors (AAV and retroviruses) (Ryan et al., EMBO 4:928-933, 1994; Mattion et al., J. Virology 70:8124-8127, 1996; Furler et al., Gene Therapy 8:864-873, 2001; and Halpin et al., Plant Journal 4:453-459, 1999; de Felipe et al., Gene Therapy 6:198-208, 1999; de Felipe et al., Human Gene Therapy 11:1921-1931, 2000; and Klump et al., Gene Therapy 8:811-817, 2001).

### Reporter Sequences/Detectable Marker Genes

Any of the vectors provided herein can optionally include a sequence encoding a reporter protein or a detectable marker ("a reporter sequence" or "detectable marker gene"). Non-limiting examples of reporter sequences or detectable marker genes include DNA sequences encoding: a beta-lactamase, a beta-galactosidase (LacZ), an alkaline phosphatase, a thymidine kinase, a green fluorescent protein (GFP), a red fluorescent protein, an mCherry fluorescent protein, a yellow fluorescent protein, a chloramphenicol acetyltransferase (CAT), and a luciferase. Additional examples of reporter sequences and detectable marker genes are known in the art. When associated with regulatory elements which drive their expression, the reporter sequence or detectable marker gene can provide signals detectable by conventional means, including enzymatic, radiographic, colorimetric, fluorescence, or other spectrographic assays; fluorescent activating cell sorting (FACS) assays; immunological assays (e.g., enzyme linked immunosorbent assay (ELISA), radioimmunoassay (RIA), and immunohistochemistry).

In some embodiments, the reporter sequence or detectable marker gene is the LacZ gene, and the presence of a vector carrying the LacZ gene in a primate cell (e.g., a supporting cochlear outer hair cell or a hair cell (e.g., a cochlear outer hair cell or a cochlear inner hair cell)) is detected by assays for beta-galactosidase activity. In other embodiments, the reporter or detectable marker is a fluorescent protein (e.g., green fluorescent protein) or luciferase, the presence of a vector carrying the fluorescent protein or luciferase in a primate cell (e.g., a supporting cochlear outer hair cell or a hair cell (e.g., a cochlear outer hair cell, a cochlear inner hair cell)) may be measured by fluorescent techniques (e.g., fluorescent microscopy or FACS) or light production in a luminometer (e.g., a spectrophotometer or an IVIS imaging instrument). In some embodiments, the reporter sequence or detectable marker can be used to verify the tissue-specific targeting capabilities and tissue-specific promoter regulatory activity of any of the vectors described herein.

### Flanking Regions Untranslated Regions (UTRs)

In some embodiments, any of the vectors described herein (e.g., any of the at least two different vectors) can include an untranslated region. In some embodiments, a vector can includes a 5' UTR or a 3' UTR.

Untranslated regions (UTRs) of a gene are transcribed but not translated. The 5' UTR starts at the transcription start site and continues to the start codon but does not include the start codon. The 3' UTR starts immediately following the stop codon and continues until the transcriptional termination signal. There is growing body of evidence about the regulatory roles played by the UTRs in terms of stability of the nucleic acid molecule and translation. The regulatory features of a UTR can be incorporated into any of the vectors, compositions, kits, or methods as described herein to enhance the stability of a supporting cell target protein or a hair cell target protein or of a protein of interest (e.g., a Cas9 endonuclease, e.g., a SaCas9 endonuclease (e.g., any of the SaCas9 endonucleases described herein), a reporter protein (e.g., a GFP protein, a mScarlet protein).

Natural 5' UTRs include a sequence that plays a role in translation initiation. They harbor signatures like Kozak sequences, which are commonly known to be involved in the process by which the ribosome initiates translation of many genes. Kozak sequences have the consensus sequence CCR(A/G)CCAUGG, where R is a purine (A or G) three bases upstream of the start codon (AUG), which is followed by another "G". The 5' UTR have also been known, e.g., to form secondary structures that are involved in elongation factor binding.

For example, in some embodiments, a 5' UTR is included in any of the vectors described herein. Non-limiting examples of 5' UTRs including those from the following genes: albumin, serum amyloid A, Apolipoprotein A/B/E, transferrin, alpha fetoprotein, erythropoietin, and Factor VIII, can be used to enhance expression of a nucleic acid molecule, such as a mRNA.

In some embodiments, a 5' UTR from a mRNA that is transcribed by a cell in the cochlea can be included in any of the vectors, compositions, kits, and methods described herein.

3' UTRs are known to have stretches of adenosines and uridines embedded in them. These AU-rich signatures are particularly prevalent in genes with high rates of turnover. Based on their sequence features and functional properties, the AU-rich elements (AREs) can be separated into three classes (Chen et al., Mol. Cell. Biol. 15:5777-5788, 1995; Chen et al., Mol. Cell Biol. 15:2010-2018, 1995): Class I AREs contain several dispersed copies of an AUUUA motif within U-rich regions. For example, c-Myc and MyoD mRNAs contain class I AREs. Class II AREs possess two or more overlapping UUAUUUA(U/A) (U/A) nonamers. GM-CSF and TNF-alpha mRNAs are examples that contain class II AREs. Class III AREs are less well defined. These U-rich regions do not contain an AUUUA motif. Two well-studied examples of this class are c-Jun and myogenin mRNAs.

Most proteins binding to the AREs are known to destabilize the messenger, whereas members of the ELAV family, most notably HuR, have been documented to increase the stability of mRNA. HuR binds to AREs of all the three classes. Engineering the HuR specific binding sites into the 3' UTR of nucleic acid molecules will lead to HuR binding and thus, stabilization of the message *in vivo.*

In some embodiments, the introduction, removal, or modification of 3' UTR AREs can be used to modulate the stability of an mRNA encoding a hair cell target protein. In some embodiments, the introduction, removal, or modification of 3' UTR AREs can be used to modulate the stability of an mRNA encoding a supporting cell target protein. In other embodiments, AREs can be removed or mutated to increase the intracellular stability and thus increase translation and production of a hair cell target protein. In other embodiments, AREs can be removed or mutated to increase the intracellular stability and thus increase translation and production of a supporting cell target protein.

In other embodiments, non-UTR sequences may be incorporated into the 5' or 3' UTRs. In some embodiments, introns or portions of intron sequences may be incorporated into the flanking regions of the polynucleotides in any of the vectors, compositions, kits, and methods provided herein. Incorporation of intronic sequences may increase protein production as well as mRNA levels.

### Primate Cells

Also provided herein is a cell (e.g., a primate cell) that includes any of the nucleic acids, vectors (e.g., at least two different vectors described herein), or compositions described herein. In some embodiments, the primate cell is a human cell (e.g., a human supporting cell or a human hair cell). In other embodiments, the primate is a non-human primate (e.g., simian cell (e.g., a monkey cell (e.g., a marmoset cell, a baboon cell, a macaque cell), or an ape cell (e.g., a gorilla cell, a gibbon cell, an orangutan cell, a chimpanzee cell). Skilled practitioners will appreciate that the nucleic acids and vectors described herein can be introduced into any primate cell (e.g., a primate supporting cell or a primate hair cell). Non-limiting examples of vectors and methods for introducing vectors into primate cells are described herein.

In some embodiments, the primate cell can be a supporting hair cell of the inner ear of a mammal. For example, a supporting cell can be Hensen's cells, Deiters' cells, inner pillar cells, outer pillar cells, Claudius cells, inner border cells, inner phalangeal cells, or cells of the stria vascularis.

In some embodiments, the primate cell is a specialized cell of the cochlea. In some embodiments, the primate cell is a hair cell. In some embodiments, the primate cell is a cochlear inner hair cell or a cochlear outer hair cell. In some embodiments, the primate cell is a cochlear inner hair cell. In some embodiments, the primate cell is a cochlear outer hair cell.

In some embodiments, the primate cell is *in vitro.* In some embodiments, the primate cell is present in a primate. In some embodiments, the primate cell is autologous cell obtained from a primate and cultured *ex vivo.*

### Methods

Also provided herein are methods of correcting a hair cell target gene defect in a hair cell of an inner ear in a primate that include: administering to the inner ear of the primate a therapeutically effective amount of any of the compositions described herein, where the administering repairs the hair cell target gene defect in the hair cell of the inner ear of the primate.

Also provided herein are methods of increasing the expression level of a hair cell target protein in a hair cell of an inner ear of a primate that include: administering to the inner ear of the primate a therapeutically effective amount of any of the compositions described herein, where the administering results in an increase in the expression level of the hair cell target protein in the hair cell of the inner ear of the primate.

Also provided herein are methods of treating hearing loss (e.g., non-syndromic sensorineural hearing loss or syndromic sensorineural hearing loss) in a primate identified as having a defective hair cell target gene that include: administering to the inner ear of the primate a therapeutically effective amount of any of the compositions described herein.

In some embodiments of any of these methods, the primate has been previously identified as having a defective hair cell target gene (e.g., a hair cell target gene having a mutation that results in a decrease in the expression and/or activity of a hair cell target protein encoded by the gene). Some embodiments of any of these methods further include, prior to the introducing or administering step, determining that the primate has a defective hair cell target gene. Some embodiments of any of these methods can further include detecting a mutation in a hair cell target gene in a primate. Some embodiments of any of the methods can further include identifying or diagnosing a primate as having non-syndromic sensorineural hearing loss. Some embodiments of any of the methods can further include identifying or diagnosing a primate as having syndromic sensorineural hearing loss.

Also provided herein are methods of correcting a supporting cell target gene defect in a supporting cell of an inner ear in a primate that include: administering to the inner ear of the primate a therapeutically effective amount of any of the compositions described herein, where the administering repairs the supporting cell target gene defect in the supporting cell of the inner ear of the primate.

Also provided herein are methods of increasing the expression level of a supporting cell target protein in a supporting cell of an inner ear of a primate that include: administering to the inner ear of the primate a therapeutically effective amount of any of the compositions described herein, where the administering results in an increase in the expression level of the supporting cell target protein in the supporting cell of the inner ear of the primate.

Also provided herein are methods of treating hearing loss (e.g., non-syndromic sensorineural hearing loss or syndromic sensorineural hearing loss) in a primate identified as having a defective supporting cell target gene that include: administering to the inner ear of the primate a therapeutically effective amount of any of the compositions described herein.

Also provided herein are methods of restoring hearing in a primate identified or diagnosed as having hearing loss that include: administering to the inner ear of the primate a therapeutically effective amount of any compositions described herein.

Also provided herein are methods of restoring synapses and/or preserving spiral ganglion nerves in a primate identified or diagnosed as having an inner ear disorder that include: administering to the inner ear of the primate a therapeutically effective amount of any of the compositions described herein.

Also provided herein are methods that include administering to an inner ear of a primate a therapeutically effective amount of any of the compositions described herein.

In some embodiments of any of these methods, the primate has been previously identified as having a defective supporting cell target gene (e.g., a supporting cell target gene having a mutation that results in a decrease in the expression and/or activity of a supporting cell target protein encoded by the gene). Some embodiments of any of these methods further include, prior to the introducing or administering step, determining that the primate has a defective supporting cell target gene. Some embodiments of any of these methods can further include detecting a mutation in a supporting cell target gene in a primate. Some embodiments of any of the methods can further include identifying or diagnosing a primate as having non-syndromic sensorineural hearing loss. Some embodiments of any of the methods can further include identifying or diagnosing a primate as having syndromic sensorineural hearing loss.

In some embodiments of any of these methods, two or more doses of any of the compositions described herein are introduced or administered into the cochlea of the primate. Some embodiments of any of these methods can include introducing or administering a first dose of the composition into the cochlea of the primate, assessing hearing function of the primate following the introducing or the administering of the first dose, and administering an additional dose of the composition into the cochlea of the primate found not to have a hearing function within a normal range (e.g., as determined using any test for hearing known in the art).

In some embodiments of any of the methods described herein, the composition can be formulated for intra-cochlear administration. In some embodiments of any of the methods described herein, the compositions described herein can be administered via intra-cochlear administration or local administration. In some embodiments of any of the methods described herein, the compositions are administered through the use of a medical device (e.g., any of the exemplary medical devices described herein).

In some embodiments, intra-cochlear administration can be performed using any of the methods described herein or known in the art. For example, a composition can be administered or introduced into the cochlea using the following surgical technique: first using visualization with a 0 degree, 2.5-mm rigid endoscope, the external auditory canal is cleared and a round knife is used to sharply delineate an approximately 5-mm tympanomeatal flap. The tympanomeatal flap is then elevated and the middle ear is entered posteriorly. The chorda tympani nerve is identified and divided, and a currette is used to remove the scutal bone, exposing the round window membrane. To enhance apical distribution of the administered or introduced composition, a surgical laser may be used to make a small 2-mm fenestration in the oval window to allow for perilymph displacement during trans-round window membrane infusion of the composition. The microinfusion device is then primed and brought into the surgical field. The device is maneuvered to the round window, and the tip is seated within the bony round window overhang to allow for penetration of the membrane by the microneedle(s). The footpedal is engaged to allow for a measured, steady infusion of the composition. The device is then withdrawn and the round window and stapes foot plate are sealed with a gelfoam patch.

In some embodiments of any of the methods described herein, the primate has or is at risk of developing non-syndromic sensorineural hearing loss. In some embodiments of any of the methods described herein, the primate has been previously identified as having a mutation in a hair cell target gene. In some embodiments of any of the methods described herein, the primate has been previously identified as having a mutation in a supporting cell target gene. In some embodiments of any of the methods described herein, the primate has any of the mutations in a hair cell target gene that are described herein or are known in the art to be associated with non-syndromic sensorineural hearing loss or syndromic sensorineural hearing loss. In some embodiments of any of the methods described herein, the primate has any of the mutations in a supporting cell target gene that are described herein or are known in the art to be associated with non-syndromic sensorineural hearing loss or syndromic sensorineural hearing loss.

In some embodiments of any of the methods described herein, the primate has been identified as being a carrier of a mutation in a hair cell target gene (e.g., via genetic testing). In some embodiments of any of the methods described herein, the primate has been identified as being a carrier of a mutation in a supporting cell target gene (e.g., via genetic testing). In some embodiments of any of the methods described herein, the primate has been identified as having a mutation in a hair cell target gene and has been diagnosed with hearing loss (e.g., non-syndromic sensorineural hearing loss or syndromic sensorineural hearing loss). In some embodiments of any of the methods described herein, the primate has been identified as having a mutation in a supporting cell target gene and has been diagnosed with hearing loss (e.g., non-syndromic sensorineural hearing loss or syndromic sensorineural hearing loss). In some embodiments of any of the methods described herein, the primate has been identified as having hearing loss (e.g., non-syndromic sensorineural hearing loss or syndromic sensorineural hearing loss). In some embodiments, successful treatment of hearing loss (e.g., non-syndromic sensorineural hearing loss or syndromic sensorineural hearing loss) can be determined in a primate using any of the conventional functional hearing tests known in the art. Non-limiting examples of functional hearing tests are various types of audiometric assays (e.g., pure-tone testing, speech testing, test of the middle ear, auditory brainstem response, and otoacoustic emissions).

In some embodiments of these methods, the primate cell is *in vitro.* In some embodiments of these methods, the primate cell is originally obtained from a primate and is cultured *ex vivo.* In some embodiments, the primate cell has previously been determined to have a defective hair cell target gene. In some embodiments, the primate cell has previously been determined to have a defective supporting cell target gene.

Methods for introducing any of the compositions described herein into a primate cell are known in the art (e.g., via lipofection or through the use of a viral vector, e.g., any of the viral vectors described herein).

An increase in expression of an active target protein (e.g., an active supporting cell target protein (e.g., a full-length supporting cell target protein) or an active hair cell target protein (e.g., a full-length hair cell target protein) as described herein is, e.g., as compared to a control or to the level of expression of an active target protein (e.g., an active supporting cell target protein (e.g., a full-length supporting cell target protein) or an active hair cell target protein (e.g., a full-length hair cell target protein) prior to the introduction of the vector(s).

Methods of detecting expression and/or activity of a target protein (e.g., a supporting cell target protein or a hair cell target protein) are known in the art. In some embodiments, the level of expression of a hair cell target protein can be detected directly (e.g., detecting hair cell target protein or detecting hair cell target mRNA). In some embodiments, the level of expression of a supporting cell target protein can be detected directly (e.g., detecting supporting cell target protein or detecting supporting cell target mRNA). Non-limiting examples of techniques that can be used to detect expression and/or activity of a target protein (e.g., a supporting cell target protein or a hair cell target protein) directly include: real-time PCR, Western blotting, immunoprecipitation, immunohistochemistry, or immunofluorescence. In some embodiments, expression of a hair cell target protein can be detected indirectly (e.g., through functional hearing tests). In some embodiments, expression of a supporting cell target protein can be detected indirectly (e.g., through functional hearing tests).

### Pharmaceutical Compositions and Kits

In some embodiments, any of the compositions described herein can further include one or more agents that promote the entry of a nucleic acid or any of the vectors described herein into a primate cell (e.g., a liposome or cationic lipid). In some embodiments, any of the vectors described herein can be formulated using natural and/or synthetic polymers. Non-limiting examples of polymers that may be included in any of the compositions described herein can include, but are not limited to, DYNAMIC POLYCONJUGATE^{®} (Arrowhead Research Corp., Pasadena, Calif.), formulations from Mirus Bio (Madison, Wis.) and Roche Madison (Madison, Wis.), PhaseRX polymer formulations such as, without limitation, SMARTT POLYMER TECHNOLOGY^{®} (PhaseRX, Seattle, Wash.), DMRI/DOPE, poloxamer, VAXFECTIN^{®} adjuvant from Vical (San Diego, Calif.), chitosan, cyclodextrin from Calando Pharmaceuticals (Pasadena, Calif.), dendrimers and poly (lactic-co-glycolic acid) (PLGA) polymers, RONDEL^{™} (RNAi/Oligonucleotide Nanoparticle Delivery) polymers (Arrowhead Research Corporation, Pasadena, Calif.), and pH responsive co-block polymers, such as, but not limited to, those produced by PhaseRX (Seattle, Wash.). Many of these polymers have demonstrated efficacy in delivering oligonucleotides *in vivo* into a primate cell (see, e.g., deFougerolles, Human Gene Ther. 19:125-132, 2008; Rozema et al., Proc. Natl. Acad. Sci. U.S.A. 104:12982-12887, 2007; Rozema et al., Proc. Natl. Acad. Sci. U.S.A. 104:12982-12887, 2007; Hu-Lieskovan et al., Cancer Res. 65:8984-8982, 2005; Heidel et al., Proc. Natl. Acad. Sci. U.S.A. 104:5715-5721, 2007).

Any of the compositions described herein can be, e.g., a pharmaceutical composition. A pharmaceutical composition can include any of the compositions described herein and one or more pharmaceutically or physiologically acceptable carriers, diluents, or excipients. Such compositions may include one or more buffers, such as neutral-buffered saline, phosphate-buffered saline, and the like; one or more carbohydrates, such as glucose, mannose, sucrose, and dextran; mannitol; one or more proteins, polypeptides, or amino acids, such as glycine; one or more antioxidants; one or more chelating agents, such as EDTA or glutathione; and/or one or more preservatives.

In some embodiments, the composition includes a pharmaceutically acceptable carrier (e.g., phosphate buffered saline, saline, or bacteriostatic water). Upon formulation, solutions will be administered in a manner compatible with the dosage formulation and in such amount as is therapeutically effective. The formulations are easily administered in a variety of dosage forms such as injectable solutions, injectable gels, drug-release capsules, and the like.

As used herein, the term "pharmaceutically acceptable carrier" includes solvents, dispersion media, coatings, antibacterial agents, antifungal agents, and the like that are compatible with pharmaceutical administration. Supplementary active compounds can also be incorporated into any of the compositions described herein.

In some embodiments, a single dose of any of the compositions described herein can include a total sum amount of the at least two different vectors of at least 1 ng, at least 2 ng, at least 4 ng, about 6 ng, about 8 ng, at least 10 ng, at least 20 ng, at least 30 ng, at least 40 ng, at least 50 ng, at least 60 ng, at least 70 ng, at least 80 ng, at least 90 ng, at least 100 ng, at least 200 ng, at least 300 ng, at least 400 ng, at least 500 ng, at least 1 µg, at least 2 µg, at least 4 µg, at least 6 µg, at least 8 µg, at least 10 µg, at least 12 µg, at least 14 µg, at least 16 µg, at least 18 µg, at least 20 µg, at least 22 µg, at least 24 µg, at least 26 µg, at least 28 µg, at least 30 µg at least 32 µg, at least 34 µg, at least 36 µg, at least 38 µg, at least 40 µg, at least 42 µg, at least 44 µg, at least 46 µg, at least 48 µg, at least 50 µg, at least 52 µg, at least 54 µg, at least 56 µg, at least 58 µg, at least 60 µg, at least 62 µg, at least 64 µg, at least 66 µg, at least 68 µg, at least 70 µg, at least 72 µg, at least 74 µg, at least 76 µg, at least 78 µg, at least 80 µg, at least 82 µg, at least 84 µg, at least 86 µg, at least 88 µg, at least 90 µg, at least 92 µg, at least 94 µg, at least 96 µg, at least 98 µg, at least 100 µg, at least 102 µg, at least 104 µg, at least 106 µg, at least 108 µg, at least 110 µg, at least 112 µg, at least 114 µg, at least 116 µg, at least 118 µg, at least 120 µg, at least 122 µg, at least 124 µg, at least 126 µg, at least 128 µg, at least 130 µg at least 132 µg, at least 134 µg, at least 136 µg, at least 138 µg, at least 140 µg, at least 142 µg, at least 144 µg, at least 146 µg, at least 148 µg, at least 150 µg, at least 152 µg, at least 154 µg, at least 156 µg, at least 158 µg, at least 160 µg, at least 162 µg, at least 164 µg, at least 166 µg, at least 168 µg, at least 170 µg, at least 172 µg, at least 174 µg, at least 176 µg, at least 178 µg, at least 180 µg, at least 182 µg, at least 184 µg, at least 186 µg, at least 188 µg, at least 190 µg, at least 192 µg, at least 194 µg, at least 196 µg, at least 198 µg, or at least 200 µg, e.g., in a buffered solution.

The compositions provided herein can be, e.g., formulated to be compatible with their intended route of administration. A non-limiting example of an intended route of administration is local administration (e.g., intra-cochlear administration). In some embodiments, the therapeutic compositions are formulated to include a lipid nanoparticle. In some embodiments, the therapeutic compositions are formulated to include a polymeric nanoparticle. In some embodiments, the therapeutic compositions are formulated to include a mini-circle DNA. In some embodiments, the therapeutic compositions are formulated to comprise a CELiD DNA. In some embodiments, the therapeutic compositions are formulated to comprise a synthetic perilymph solution. An exemplary synthetic perilymph solution includes 20-200 mM NaCl; 1-5 mM KCl; 0.1-10 mM CaCl₂; 1-10 mM glucose; 2-50 mM HEPES, having a pH of between about 6 and about 9.

Also provided are kits including any of the compositions described herein. In some embodiments, a kit can include a solid composition (e.g., a lyophilized composition including the at least two different vectors described herein) and a liquid for solubilizing the lyophilized composition. In some embodiments, a kit can include a pre-loaded syringe including any of the compositions described herein.

In some embodiments, the kit includes a vial comprising any of the compositions described herein (e.g., formulated as an aqueous composition, e.g., an aqueous pharmaceutical composition).

In some embodiments, the kits can include instructions for performing any of the methods described herein.

### Devices and Surgical Methods

Provided herein are therapeutic delivery systems for treating hearing loss (e.g., non-syndromic sensorineural hearing loss or syndromic sensorineural hearing loss). In one aspect, the therapeutic delivery systems include i) a medical device capable of creating one or a plurality of incisions in a round window membrane of an inner ear of a primate in need thereof, and ii) an effective dose of a composition (e.g., any of the compositions described herein). In some embodiments, the medical device includes a plurality of micro-needles.

Also provided herein are surgical methods for treatment of hearing loss (e.g., non-syndromic sensorineural hearing loss or syndromic sensorineural hearing loss). In some embodiments, the methods include the steps of: introducing into a cochlea of a primate a first incision at a first incision point; and administering intra-cochlearly a therapeutically effective amount of any of the compositions provided herein. In some embodiments, the composition is administered to the primate at the first incision point. In some embodiments, the composition is administered to the primate into or through the first incision.

In some embodiments of any of the methods described herein, any of the compositions described herein is administered to the primate into or through the cochlea oval window membrane. In some embodiments of any of the methods described herein, any of the compositions described herein is administered to the primate into or through the cochlea round window membrane. In some embodiments of any of the methods described herein, the composition is administered using a medical device capable of creating a plurality of incisions in the round window membrane. In some embodiments, the medical device includes a plurality of micro-needles. In some embodiments, the medical device includes a plurality of micro-needles including a generally circular first aspect, where each micro-needle has a diameter of at least about 10 microns. In some embodiments, the medical device includes a base and/or a reservoir capable of holding the composition. In some embodiments, the medical device includes a plurality of hollow micro-needles individually including a lumen capable of transferring the composition. In some embodiments, the medical device includes a means for generating at least a partial vacuum.

The invention is further described in detail by reference to the following experimental examples. These examples are provided for purposes of illustration only, and are not intended to be limiting unless otherwise specified. Thus, the invention should in no way be construed as being limited to the following examples, but rather should be construed to encompass any and all variations that become evident as a result of the teaching provided herein.

Without further description, it is believed that one of ordinary skill in the art can, using the preceding description and the following illustrative examples, make and utilize the compounds of the present invention and practice the claimed methods. The following working examples specifically point out various aspects of the present invention, and are not to be construed as limiting in any way the remainder of the disclosure.

### EXAMPLES

### Example 1. AAV single vector injection into the inner ear

Immunofluorescent staining was performed on cochlear tissue of a cynomolgus macaque (non-human primate) following administration of a single Anc80-GFP AAV vector directly into the inner ear through the round window.

The cochlear tissue from the treated macaque was processed for immunofluorescence analysis using Myo7a as a marker for hair cells and Iba-1 as a marker for macrophages. The middle turn is representative of the entire sensory epithelium. The data in Figures 1A-1C show clear GFP expression in both the hair cells and the supporting cells, including the following supporting cell subtypes: Hensen's cells (HC), Claudius cells (CC), Dieter cells (DC), inner and outer pillar cells (OPC/IPC), inner border cells, and inner phalangeal cells (IPHC/IBC). These data demonstrate successful Anc80-GFP AAV vector transduction into different cell types of the inner ear sensory epithelium, and the resulting expression of the encoded reporter gene (GFP) in these different cell types. These data indicate the present claimed compositions including a single AAV vector or two or more AAV vectors can be used to express a gene in hair cells and supporting cells, and can be used to repair a mutation in a gene in hair cells and supporting cells.

### OTHER EMBODIMENTS

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

All publications, patent applications, patents, and other references mentioned herein are incorporated by reference in their entirety. In case of conflict, the present specification, including definitions, will control. In addition, section headings, the materials, methods, and examples are illustrative only and not intended to be limiting.

**In view of the foregoing, it will be appreciated that the invention decribed herein inter alia relates to the following items:**
1. A composition comprising at least two different nucleic acid vectors, wherein:
   each of the at least two different adeno-associated virus (AAV) vectors comprises a coding sequence that encodes a different portion of a supporting cell target protein, each of the encoded portions being at least 30 amino acid residues in length, wherein the amino acid sequence of each of the encoded portions may optionally partially overlap with the amino acid sequence of a different one of the encoded portions;
   no single vector of the at least two different vectors encodes the full-length supporting cell target protein;
   at least one of the coding sequences comprises a nucleotide sequence spanning two neighboring exons of the supporting cell target genomic DNA, and lacks an intronic sequence between the two neighboring exons; and
   when introduced into a primate cell the at least two different vectors undergo concatamerization or homologous recombination with each other, thereby forming a recombined nucleic acid that encodes a full-length supporting cell target protein.
2. The composition of item 1, wherein the amino acid sequence of one of the encoded portions overlaps with the amino acid sequence of a different one of the encoded portions.
3. The composition of item 2, wherein the amino acid sequence of each of the encoded portions partially overlaps with the amino acid sequence of a different encoded portion.
4. The composition of item 2, wherein the overlapping amino acid sequence is between 30 amino acid residues to about 390 amino acid residues in length.
5. The composition of any one of items 1-4, wherein each of the at least two different vectors comprises a different segment of an intron, wherein the intron comprises the nucleotide sequence of an intron that is present in a supporting cell target genomic DNA, and wherein the two different segments overlap in sequence by at least 100 nucleotides.
6. The composition of item 5, wherein the two different segments overlap in sequence by about 30 nucleotides to about 390 nucleotides.
7. The composition of any one of items 1-6, wherein the nucleotide sequence of each of the at least two different vectors is between about 30 nucleotides to about 390 nucleotides in length.
8. The composition of item 7, wherein the nucleotide sequence of each of the at least two different vectors is between 30 nucleotides to 340 nucleotides in length.
9. The composition of any one of items 1-8, wherein the number of different vectors in the composition is two.
10. The composition of item 9, wherein a first of the two different vectors comprises a coding sequence that encodes an N-terminal portion of the supporting cell target protein.
11. The composition of item 10, wherein the N-terminal portion of the supporting cell target protein is between about 30 amino acids to about 390 amino acids in length.
12. The composition of item 11, wherein the N-terminal portion of the supporting cell target protein is between about 30 amino acids to about 340 amino acids in length.
13. The composition of any one of items 10-12, wherein the first vector further comprises one or both of a promoter and a Kozak sequence.
14. The composition of item 13, wherein the first vector comprises a promoter that is an inducible promoter, a constitutive promoter, or a tissue-specific promoter.
15. The composition of any one of items 10-14, wherein the second of the two different vectors comprises a coding sequence that encodes a C-terminal portion of the supporting cell target protein.
16. The composition of item 15, wherein the C-terminal portion of the supporting cell target protein is between 30 amino acids to about 390 amino acids in length.
17. The composition of item 16, wherein the C-terminal portion of the supporting cell target protein is between 30 amino acids to about 340 amino acids in length.
18. The composition of any one of items 15-17, wherein the second vector further comprises a poly(dA) sequence.
19. A composition comprising two different nucleic acid vectors, wherein:
   a first nucleic acid vector of the two different nucleic acid vectors comprises a promoter, a first coding sequence that encodes an N-terminal portion of a supporting cell target protein positioned 3' of the promoter, and a splicing donor signal sequence positioned at the 3' end of the first coding sequence; and
   a second nucleic acid vector of the two different nucleic acid vectors comprises a splicing acceptor signal sequence, a second coding sequence that encodes a C-terminal portion of a supporting cell target protein positioned at the 3' end of the splicing acceptor signal sequence, and a polyadenylation sequence at the 3' end of the second coding sequence;
   wherein each of the encoded portions is at least 30 amino acid residues in length, wherein the amino acid sequences of the encoded portions do not overlap, wherein no single vector of the two different vectors encodes the full-length supporting cell target protein, and, when the coding sequences are transcribed in a primate cell, to produce RNA transcripts, splicing occurs between the splicing donor signal sequence on one transcript and the splicing acceptor signal sequence on the other transcript, thereby forming a recombined RNA molecule that encodes a full-length supporting cell target protein.
20. The composition of item 19, wherein at least one of the coding sequences comprises a nucleotide sequence spanning two neighboring exons of a supporting cell target genomic DNA, and lacks an intronic sequence between the neighboring exons.
21. A composition comprising:
   a first nucleic acid vector comprising a promoter, a first coding sequence that encodes an N-terminal portion of a supporting cell target protein positioned 3' of the promoter, a splicing donor signal sequence positioned at the 3' end of the first coding sequence, and a first detectable marker gene positioned 3' of the splicing donor signal sequence; and
   a second nucleic acid vector, different from the first nucleic acid vector, comprising a second detectable marker gene, a splicing acceptor signal sequence positioned 3' of the second detectable marker gene, a second coding sequence that encodes a C-terminal portion of a supporting cell target protein positioned at the 3' end of the splicing acceptor signal sequence, and a polyadenylation sequence positioned at the 3' end of the second coding sequence;
   wherein each of the encoded portions is at least 30 amino acid residues in length, wherein the respective amino acid sequences of the encoded portions do not overlap with each other, wherein no single vector of the two different vectors encodes the full-length supporting cell target protein, and, when the coding sequences are transcribed in a primate cell to produce RNA transcripts, splicing occurs between the splicing donor signal on one transcript and the splicing acceptor signal on the other transcript, thereby forming a recombined RNA molecule that encodes a full-length supporting cell target protein.
22. The composition of item 21, wherein at least one of the coding sequences comprises a nucleotide sequence spanning two neighboring exons of a supporting cell target genomic DNA, and lacks an intronic sequence between the neighboring exons.
23. The composition of item 21, wherein the first or second detectable marker gene is alkaline phosphatase.
24. The composition of item 21 or 23, wherein the first and second detectable marker genes are the same.
25. A composition comprising:
   a first nucleic acid vector comprising a promoter, a first coding sequence that encodes an N-terminal portion of a supporting cell target protein positioned 3' to the promoter, a splicing donor signal sequence positioned at the 3' end of the first coding sequence, and a F1 phage recombinogenic region positioned 3' to the splicing donor signal sequence; and
   a second nucleic acid vector, different from the first nucleic acid vector, comprising a second F1 phage recombinogenic region, a splicing acceptor signal sequence positioned 3' of the second F1 phage recombinogenic region, a second coding sequence that encodes a C-terminal portion of a supporting cell target protein positioned at the 3' end of the splicing acceptor signal sequence, and a polyadenylation sequence positioned at the 3' end of the second coding sequence;
   wherein each of the encoded portions is at least 30 amino acid residues in length, wherein the respective amino acid sequences of the encoded portions do not overlap with each other, wherein no single vector of the two different vectors encodes the full-length supporting cell target protein, and, when the coding sequences are transcribed in a primate cell to produce RNA transcripts, splicing occurs between the splicing donor signal one transcript and the splicing acceptor signal on the other transcript, thereby forming a recombined RNA molecule that encodes a full-length supporting cell target protein.
26. The composition of item 25, wherein at least one of the coding sequences comprises a nucleotide sequence spanning two neighboring exons of a supporting cell target genomic DNA, and lacks an intronic sequence between the two neighboring exons.
27. A composition comprising a single adeno-associated virus (AAV) vector, wherein the single AAV vector that comprises a nucleic acid sequence that encodes a supporting cell target protein; and
   when introduced into a primate cell, a nucleic acid encoding a full-length supporting cell target protein is generated at the locus of the supporting cell target gene, and the primate expresses the supporting cell target protein.
28. The composition of any one of items 1-27, wherein the supporting cell target gene is gap junction protein beta 2 (GJB2).
29. The composition of any one of items 1-27, wherein the supporting cell target gene is solute carrier family 26 member 4 (SLC26A4).
30. A composition comprising at least two different nucleic acid vectors, wherein:
   each of the at least two different adeno-associated virus (AAV) vectors comprises a coding sequence that encodes a different portion of a hair cell target protein, each of the encoded portions being at least 30 amino acid residues in length, wherein the amino acid sequence of each of the encoded portions may optionally partially overlap with the amino acid sequence of a different one of the encoded portions;
   no single vector of the at least two different vectors encodes the full-length hair cell target protein;
   at least one of the coding sequences comprises a nucleotide sequence spanning two neighboring exons of the hair cell target genomic DNA, and lacks an intronic sequence between the two neighboring exons; and
   when introduced into a primate cell the at least two different vectors undergo concatamerization or homologous recombination with each other, thereby forming a recombined nucleic acid that encodes a full-length hair cell target protein.
31. The composition of item 30, wherein the amino acid sequence of one of the encoded portions overlaps with the amino acid sequence of a different one of the encoded portions.
32. The composition of item 31, wherein the amino acid sequence of each of the encoded portions partially overlaps with the amino acid sequence of a different encoded portion.
33. The composition of item 31, wherein the overlapping amino acid sequence is between 30 amino acid residues to about 390 amino acid residues in length.
34. The composition of any one of items 30-33, wherein each of the at least two different vectors comprises a different segment of an intron, wherein the intron comprises the nucleotide sequence of an intron that is present in a hair cell target genomic DNA, and wherein the two different segments overlap in sequence by at least 100 nucleotides.
35. The composition of item 34, wherein the two different segments overlap in sequence by about 30 nucleotides to about 390 nucleotides.
36. The composition of any one of items 30-35, wherein the nucleotide sequence of each of the at least two different vectors is between about 30 nucleotides to about 390 nucleotides in length.
37. The composition of item 36, wherein the nucleotide sequence of each of the at least two different vectors is between 30 nucleotides to 340 nucleotides in length.
38. The composition of any one of items 30-37, wherein the number of different vectors in the composition is two.
39. The composition of item 38, wherein a first of the two different vectors comprises a coding sequence that encodes an N-terminal portion of the hair cell target protein.
40. The composition of item 39, wherein the N-terminal portion of the hair cell target protein is between about 30 amino acids to about 390 amino acids in length.
41. The composition of item 40, wherein the N-terminal portion of the hair cell target protein is between about 30 amino acids to about 340 amino acids in length.
42. The composition of any one of items 39-41, wherein the first vector further comprises one or both of a promoter and a Kozak sequence.
43. The composition of item 42, wherein the first vector comprises a promoter that is an inducible promoter, a constitutive promoter, or a tissue-specific promoter.
44. The composition of any one of items 39-41, wherein the second of the two different vectors comprises a coding sequence that encodes a C-terminal portion of the hair cell target protein.
45. The composition of item 44, wherein the C-terminal portion of the hair cell target protein is between 30 amino acids to about 390 amino acids in length.
46. The composition of item 45, wherein the C-terminal portion of the hair cell target protein is between 30 amino acids to about 340 amino acids in length.
47. The composition of any one of items 44-46, wherein the second vector further comprises a poly(dA) sequence.
48. A composition comprising two different nucleic acid vectors, wherein:
   a first nucleic acid vector of the two different nucleic acid vectors comprises a promoter, a first coding sequence that encodes an N-terminal portion of a hair cell target protein positioned 3' of the promoter, and a splicing donor signal sequence positioned at the 3' end of the first coding sequence; and
   a second nucleic acid vector of the two different nucleic acid vectors comprises a splicing acceptor signal sequence, a second coding sequence that encodes a C-terminal portion of a hair cell target protein positioned at the 3' end of the splicing acceptor signal sequence, and a polyadenylation sequence at the 3' end of the second coding sequence;
   wherein each of the encoded portions is at least 30 amino acid residues in length, wherein the amino acid sequences of the encoded portions do not overlap, wherein no single vector of the two different vectors encodes the full-length hair cell target protein, and, when the coding sequences are transcribed in a primate cell, to produce RNA transcripts, splicing occurs between the splicing donor signal sequence on one transcript and the splicing acceptor signal sequence on the other transcript, thereby forming a recombined RNA molecule that encodes a full-length hair cell target protein.
49. The composition of item 48, wherein at least one of the coding sequences comprises a nucleotide sequence spanning two neighboring exons of a hair cell target genomic DNA, and lacks an intronic sequence between the neighboring exons.
50. A composition comprising:
   a first nucleic acid vector comprising a promoter, a first coding sequence that encodes an N-terminal portion of a hair cell target protein positioned 3' of the promoter, a splicing donor signal sequence positioned at the 3' end of the first coding sequence, and a first detectable marker gene positioned 3' of the splicing donor signal sequence; and
   a second nucleic acid vector, different from the first nucleic acid vector, comprising a second detectable marker gene, a splicing acceptor signal sequence positioned 3' of the second detectable marker gene, a second coding sequence that encodes a C-terminal portion of a hair cell target protein positioned at the 3' end of the splicing acceptor signal sequence, and a polyadenylation sequence positioned at the 3' end of the second coding sequence;
   wherein each of the encoded portions is at least 30 amino acid residues in length, wherein the respective amino acid sequences of the encoded portions do not overlap with each other, wherein no single vector of the two different vectors encodes the full-length hair cell target protein, and, when the coding sequences are transcribed in a primate cell to produce RNA transcripts, splicing occurs between the splicing donor signal on one transcript and the splicing acceptor signal on the other transcript, thereby forming a recombined RNA molecule that encodes a full-length hair cell target protein.
51. The composition of item 50, wherein at least one of the coding sequences comprises a nucleotide sequence spanning two neighboring exons of a hair cell target genomic DNA, and lacks an intronic sequence between the neighboring exons.
52. The composition of item 50, wherein the first or second detectable marker gene is alkaline phosphatase.
53. The composition of item 50 or 52, wherein the first and second detectable marker genes are the same.
54. A composition comprising:
   a first nucleic acid vector comprising a promoter, a first coding sequence that encodes an N-terminal portion of a hair cell target protein positioned 3' to the promoter, a splicing donor signal sequence positioned at the 3' end of the first coding sequence, and a F1 phage recombinogenic region positioned 3' to the splicing donor signal sequence; and
   a second nucleic acid vector, different from the first nucleic acid vector, comprising a second F1 phage recombinogenic region, a splicing acceptor signal sequence positioned 3' of the second F1 phage recombinogenic region, a second coding sequence that encodes a C-terminal portion of a hair cell target protein positioned at the 3' end of the splicing acceptor signal sequence, and a polyadenylation sequence positioned at the 3' end of the second coding sequence;
   wherein each of the encoded portions is at least 30 amino acid residues in length, wherein the respective amino acid sequences of the encoded portions do not overlap with each other, wherein no single vector of the two different vectors encodes the full-length hair cell target protein, and, when the coding sequences are transcribed in a primate cell to produce RNA transcripts, splicing occurs between the splicing donor signal one transcript and the splicing acceptor signal on the other transcript, thereby forming a recombined RNA molecule that encodes a full-length hair cell target protein.
55. The composition of item 54, wherein at least one of the coding sequences comprises a nucleotide sequence spanning two neighboring exons of a hair cell target genomic DNA, and lacks an intronic sequence between the two neighboring exons.
56. The composition of any one of items 30-55, wherein the hair cell target gene is an OTOF gene, a TRIOBP gene, or a STRC gene.
57. A composition comprising a single adeno-associated virus (AAV) vector, wherein the single AAV vector that comprises a nucleic acid sequence that encodes a hair cell target protein; and
   when introduced into a primate cell, a nucleic acid encoding a full-length hair cell target protein is generated at the locus of the hair cell target gene, and the primate expresses the hair cell target protein.
58. The composition of item 57, wherein the hair cell target gene is a PJVK gene, an OTOF gene, a NDP gene, a CLRN1 gene, a TRIOBP gene, a STRC gene, a TMC1 gene, a VEGF gene or a HSPA1A gene.
59. The composition of any one of items 1-58, further comprising a pharmaceutically acceptable excipient.
60. A kit comprising a composition of any one of items 1-59.
61. A kit of item 60, further comprising a pre-loaded syringe comprising the composition.
62. A method of correcting a supporting cell target gene defect in a supporting cell of an inner ear in a primate, the method comprising:
   administering to the inner ear of the primate a therapeutically effective amount of a composition of any one of items 1-29,
   wherein the administering repairs the supporting cell target gene defect in the supporting cell of the inner ear of the primate.
63. A method of increasing the expression level of a supporting cell target protein in a supporting cell of an inner ear of a primate, the method comprising administering to the inner ear of the primate a therapeutically effective amount of a composition of any one of items 1-29,
   wherein the administering results in an increase in the expression level of the supporting cell target protein in the supporting cell of the inner ear of the primate.
64. The method of item 63, wherein the primate has previously been determined to have a defective supporting cell target gene.
65. A method of treating non-syndromic sensorineural hearing loss in a primate identified as having a defective supporting cell target gene, the method comprising:
   administering to the inner ear of the primate a therapeutically effective amount of a composition of any one of items 1-29.
66. The method of item 65, further comprising, prior to the administering step, determining that the primate has a defective supporting cell target gene.
67. A method of restoring hearing in a primate identified or diagnosed as having hearing loss, the method comprising:
   administering to the inner ear of the primate a therapeutically effective amount of a composition of any one of items 1-29.
68. The method of item 67, further comprising, prior to the administering step, determining that the primate has a defective supporting cell target gene.
69. A method of restoring synapses and/or preserving spiral ganglion nerves in a primate identified or diagnosed as having an inner ear disorder, the method comprising:
   administering to the inner ear of the primate a therapeutically effective amount of a composition of any one of items 1-29.
70. The method of item 69, further comprising, prior to the administering step, determining that the primate has a defective supporting cell target gene.
71. A method comprising administering to an inner ear of a primate a therapeutically effective amount of a composition of any one of items 1-29.
72. The method of item 71, wherein the primate has been previously identified as having a defective supporting cell target gene.
73. A method of correcting a hair cell target gene defect in a hair cell of an inner ear in a primate, the method comprising:
   administering to the inner ear of the primate a therapeutically effective amount of a composition of any one of items 30-59,
   wherein the administering repairs the hair cell target gene defect in the hair cell of the inner ear of the primate.
74. A method of increasing the expression level of a hair cell target protein in a hair cell of an inner ear of a primate, the method comprising administering to the inner ear of the primate a therapeutically effective amount of a composition of any one of items 30-59,
   wherein the administering results in an increase in the expression level of the hair cell target protein in the hair cell of the inner ear of the primate.
75. The method of item 74, wherein the primate has previously been determined to have a defective hair cell target gene.
76. A method of treating non-syndromic sensorineural hearing loss in a primate identified as having a defective hair cell target gene, the method comprising:
   administering to the inner ear of the primate a therapeutically effective amount of a composition of any one of items 30-59.
77. The method of item 76, further comprising, prior to the administering step, determining that the primate has a defective hair cell target gene.
78. A method of restoring hearing in a primate identified or diagnosed as having hearing loss, the method comprising:
   administering to the inner ear of the primate a therapeutically effective amount of a composition of any one of items 30-59.
79. The method of item 78, further comprising, prior to the administering step, determining that the primate has a defective hair cell target gene.
80. A method of restoring synapses and/or preserving spiral ganglion nerves in a primate identified or diagnosed as having an inner ear disorder, the method comprising:
   administering to the inner ear of the primate a therapeutically effective amount of a composition of any one of items 30-59.
81. The method of item 80, further comprising, prior to the administering step, determining that the primate has a defective hair cell target gene.
82. A method comprising administering to an inner ear of a primate a therapeutically effective amount of a composition of any one of items 30-59.
83. The method of item 82, wherein the primate has been previously identified as having a defective hair cell target gene.
84. The composition of any one of items 1-26 or 30- 56, wherein the second nucleic acid vector further comprises a destabilizing sequence.
85. The composition of item 84, wherein the second nucleic acid vector further comprises a FKBP12 destabilizing sequence.
86. The composition of any one of items 27-29 or 57-59, wherein the nucleic acid vector further comprises a destabilizing sequence.
87. The composition of item 86, wherein the nucleic acid vector comprises a FKBP12 destabilizing sequence.

## Claims

1. A recombinant adeno-associated virus (rAAV) vector for use in treating hearing loss in a primate, wherein the rAAV vector is to be administered to the inner ear of the primate, wherein the rAAV vector comprises a promoter, a nucleic acid sequence that comprises a supporting cell target gene, and a destabilizing sequence.

2. The rAAV vector for the use of claim 1, wherein the supporting cell target gene is selected from the group consisting of gap junction protein beta 2 (GJB2) and solute carrier family 26 member 4 (SLC26A4).

3. The rAAV vector for the use of claim 2, wherein the supporting cell target gene is GJB2.

4. The rAAV vector for the use of claim 1, wherein the destabilizing sequence is selected from the group consisting of a dihydrofolate reductase (DHFR) sequence and a FK506-and-rapamycin-binding protein (FKBP12) sequence.

5. The rAAV vector for the use of claim 1, wherein the primate has previously been determined to have a defective supporting cell target gene.

6. The rAAV vector for the use of claim 1, wherein the rAAV vector is to be administered to the cochlea of the primate.

7. The rAAV vector for the use of claim 1, wherein the primate is a human.

8. The rAAV vector for the use of claim 1, wherein the hearing loss is non-syndromic sensorineural hearing loss or syndromic sensorineural hearing loss.

9. The rAAV vector for the use of claim 1, wherein the rAAV vector further comprises a polyadenylation sequence.

10. The rAAV vector for the use of claim 1, wherein the promoter is a native promoter, a constitutive promoter, an inducible promoter, or a tissue-specific promoter.

11. The rAAV vector for the use of claim 10, wherein the promoter is a tissue-specific promoter.

12. The AAV vector for the use of claim 11, wherein the tissue-specific promoter is a cochlea-specific promoter.

13. The rAAV vector for the use of claim 1, wherein the rAAV vector further comprises 5' and 3' AAV inverted terminal repeats.

14. The rAAV vector for the use of claim 1, wherein the rAAV vector is packaged in an AAV capsid.

15. The rAAV vector for the use of claim 14, wherein the rAAV capsid is an Anc80 capsid.
